# EUROPEAN PATENT SPECIFICATION

(11) **EP 2 227 556 B1**
(45) Date of publication and mention of the grant of the patent: **27.08.2014**
(21) Application number: 08852690.0
(22) Date of filing: 19.11.2008
(51) Int. Cl.: C12Q 1/68, G01N 33/574

(54) **LUNG CANCER MARKERS AND USES THEREOF**
LUNGENKREBS-MARKER UND DEREN VERWENDUNGEN
MARQUEURS DE CANCER DU POUMON ET LEURS UTILISATIONS

(30) Priority: 19.11.2007 US 3767 P
(43) Date of publication of application: 15.09.2010
(60) Divisional application: 13192146.2
(73) Proprietor: Celera Corporation, Alameda, CA 94502 (US)
(72) Inventor: BIRSE, Charles, Alameda, CA 94502 (US); RUBEN, Steve, Alameda, CA 94502 (US); LEWIS, Marcia, Alameda, CA 94502 (US); MESRI, Mehdi, Alameda, CA 94502 (US)
(74) Representative: Jones Day
(86) International application number: PCT/US2008/084077
(87) International publication number: WO 2009/067546

(56) References cited:
- WO-A1-2006/080597
- US-A1- 2005 272 061
- US-A1- 2007 264 193
- KASAMATSU A ET AL: "Identification of candidate genes associated with salivary adenoid cystic carcinomas using combined comparative genomic hybridization and oligonucleotide microarray analyses", INTERNATIONAL JOURNAL OF BIOCHEMISTRY AND CELL BIOLOGY, EXETER, GB, vol. 37, no. 9, 1 September 2005 (2005-09-01), pages 1869-1880, XP004974399, ISSN: 1357-2725, DOI: DOI:10.1016/J.BIOCEL.2005.04.004
- AFFYMETRIX: "SLPI", INTERNET CITATION, 1 September 2005 (2005-09-01), page 1, XP007915926, Retrieved from the Internet: URL:https://www.affymetrix.com/analysis/ne taffx/showresults.affx [retrieved on 2010-11-23]
- AFFYMETRIX: "Timp1", INTERNET CITATION, 1 September 2005 (2005-09-01), page 1, XP007915927, Retrieved from the Internet: URL:https://www.affymetrix.com/analysis/ne taffx/showresults.affx [retrieved on 2010-11-23]
- AFFYMETRIX: "SCC", INTERNET CITATION, 1 September 2005 (2005-09-01), page 1, XP007915928, Retrieved from the Internet: URL:https://www.affymetrix.com/analysis/ne taffx/showresults.affx [retrieved on 2010-11-23]
- AFFYMETRIX: "TFP1", INTERNET CITATION, 1 September 2005 (2005-09-01), page 1, XP007915929, Retrieved from the Internet: URL:https://www.affymetrix.com/analysis/ne taffx/showresults.affx [retrieved on 2010-11-23]
- AFFYMETRIX: "CEACAM5", INTERNET CITATION, 1 September 2005 (2005-09-01), page 1, XP007915930, Retrieved from the Internet: URL:https://www.affymetrix.com/analysis/ne taffx/showresults.affx [retrieved on 2010-11-23]
- AFFYMETRIX: "MMP2", INTERNET CITATION, 1 September 2005 (2005-09-01), page 1, XP007915931, Retrieved from the Internet: URL:https://www.affymetrix.com/analysis/ne taffx/showresults.affx [retrieved on 2010-11-23]
- AFFYMETRIX: "MDK", INTERNET CITATION, 1 September 2005 (2005-09-01), page 1, XP007915932, Retrieved from the Internet: URL:https://www.affymetrix.com/analysis/ne taffx/showresults.affx [retrieved on 2010-11-23]
- AFFYMETRIX: "OPN", INTERNET CITATION, 1 September 2005 (2005-09-01), page 1, XP007915933, Retrieved from the Internet: URL:https://www.affymetrix.com/analysis/ne taffx/showresults.affx [retrieved on 2010-11-23]
- AFFYMETRIX: "Cyfra", INTERNET CITATION, 1 September 2005 (2005-09-01), page 1, XP007915934, Retrieved from the Internet: URL:https://www.affymetrix.com/analysis/ne taffx/showresults.affx [retrieved on 2010-11-23]
- GIOVANELLA L ET AL: "Evaluation of the serum markers CEA, NSE, TPS and CYFRA 21.1 in lung cancer", INTERNATIONAL JOURNAL OF BIOLOGICAL MARKERS, WICHTIG EDITORE, MILAN, IT, vol. 10, no. 3, 1 July 1995 (1995-07-01), pages 156-160, XP009141563, ISSN: 0393-6155
- MARGOLIS M L ET AL: "Serum tumor markers in non-small cell lung cancer. A comparative analysis", CANCER, AMERICAN CANCER SOCIETY, PHILADELPHIA, PA, US, vol. 73, no. 3, 1 February 1994 (1994-02-01), pages 605-609, XP009141562, ISSN: 0008-543X
- MIZUSHIMA Y ET AL: "Clinical significance of the number of positive tumor markers in assisting the diagnosis of lung cancer with multiple tumor marker assay", ONCOLOGY, S. KARGER, BASEL, CH, vol. 47, no. 1, 1 January 1990 (1990-01-01), pages 43-48, XP009141585, ISSN: 0030-2414
- STOLL D ET AL: "Protein microarrays: Applications and future challenges", CURRENT OPINION IN DRUG DISCOVERY AND DEVELOPMENT, CURRENT DRUGS, LONDON, GB, vol. 8, no. 2, 1 January 2005 (2005-01-01) , pages 239-252, XP008081397, ISSN: 1367-6733
- PASTOR ET AL.: 'Diagnostic value of SCC, CEA and CYFRA 21.1 in lung cancer: a Bayesian analysis.' EUR RESPIR JOURNAL vol. 10, 1997, pages 603 - 609
- OERNTOFT T F ET AL: "GENOME-WIDE STUDY OF GENE COPY NUMBERS, TRANSCRIPTS, AND PROTEIN LEVELS IN PAIRS OF NON-INVASIVE AND INVASIVE HUMAN TRANSITIONAL CELL CARCINOMAS", MOLECULAR & CELLULAR PROTEOMICS, AMERICAN SOCIETY FOR BIOCHEMISTRY AND MOLECULAR BIOLOGY, INC, US, vol. 1, no. 1, 1 January 2002 (2002-01-01) , pages 37-45, XP008015037, ISSN: 1535-9476, DOI: 10.1074/MCP.M100019-MCP200
- GREENBAUM DOV ET AL: "Interrelating different types of genomic data, from proteome to secretome: 'Oming in on function", GENOME RESEARCH, COLD SPRING HARBOR LABORATORY PRESS, WOODBURY, NY, US, vol. 11, no. 9, 1 September 2001 (2001-09-01), pages 1463-1468, XP009088703, ISSN: 1088-9051, DOI: 10.1101/GR.207401
- GREENBAUM D ET AL: "COMPARING PROTEIN ABUNDANCE AND MRNA EXPRESSION LEVELS ON A GENOMIC SCALE", GENOME BIOLOGY (ONLINE), BIOMED CENTRAL LTD, GB, vol. 40, no. 9, 1 January 2003 (2003-01-01), pages 117.01-117.08, XP008036618, ISSN: 1465-6914

## Description

### FIELD OF THE INVENTION

This invention relates to the field of disease assessment and therapy. Disclosed herein are compositions and methods for assessing and treating diseases, especially cancer, and particularly lung cancer.

### BACKGROUND OF THE INVENTION

Cancer is one of the leading causes of death worldwide, and cancer, especially lung cancer, is difficult to diagnose and treat effectively. Accordingly, there is a need in the art for new compositions and methods for assessing and treating various cancers. particularly lung cancer.

### Lung cancer

Lung cancer is the second most prevalent type of cancer for both men and women in the United States and is the most common cause of cancer death in both men and women. The five-year survival rate for lung cancer continues to be poor at only about 8-15%. This low survival is because lung cancer is commonly not detected until it has spread beyond the lungs. Only 16% of new lung cancer cases in the United States are detected at the earliest stage, when the cancer is still localized to the lungs. At this early stage, survival is considerably higher, with estimates as high as 70-80%. Therefore, procedures for detecting lung cancer are of critical importance to the outcome of a patient since these procedures have the potential to reduce mortality. Thus, there is a need for new diagnostic compositions and methods that are more sensitive and specific for detecting early lung cancer.

Furthermore, there is also a need for new diagnostic compositions and methods for determining the stage of a patient's disease. Stage determination has potential prognostic value and provides criteria for designing optimal therapy. Biomarkers that are indicative of different stages of lung cancer would be useful to facilitate the staging of lung cancer.

Lung cancer patients are typically monitored following initial therapy and during adjuvant therapy to determine their response to therapy and to detect persistent or recurrent disease or metastasis. Thus, there is clearly a need for lung cancer markers that are more sensitive and specific in detecting lung cancer, its recurrence, and progression.

Although imaging modalities, such as computed tomography (CT) screening, are being studied to aid in the early detection of lung cancer, controversy remains as to the ability of these methods to impact mortality (I-ELCAP Investigators, NEJM 2006 (355): 1763-71 and Bach et al. 2007. JAMA 297:953-961). In addition, the most advanced imaging technologies under study are expensive and not widely available. These CT imaging tests may lead to over-diagnosis of lung cancer, resulting in significant expenses to the health care system to manage patients with pulmonary nodules observed through these CT imaging tests. Furthermore, there is significant morbidity associated with the management of the pulmonary nodules in an effort to ascertain whether the nodules are malignant or benign. It is estimated that 10-50% of smokers in a high risk group have pulmonary nodules upon imaging studies *(*CHEST 2007 Supplement - Evidence for the Treatment of Patients With Pulmonary Nodules: When Is It Lung cancer?: AACP Evidence-Based Clinical Practise Guidelines*).* Thus, there is a significant need for novel diagnostics that can be used either independently or with imaging modalities for early diagnosis and improved management of patients with lung cancer. For example, a blood test for biomarkers that has high performance (e.g., high sensitivity and specificity) for detecting lung cancer could provide a low cost complement to CT testing for early detection of cancer. If the performance of a biomarker test were sufficiently high, such a test could serve as a lower cost alternative to CT or X-ray testing. For example, only those patients that tested positive in a biomarker test may then need to undergo more expensive imaging tests. Furthermore, a biomarker test could be used, for example, in a yearly screening regimen for lung cancer.

Although there have been reports of circulating tumor markers and antigens with potential use in lung cancer (see Schneider, J. 2006. Advances in Clin Chem, 42: 1-41 for a review), markers currently used generally suffer from low sensitivity and less than desirable specificity, especially among smokers (Schneider, 2006), and are typically only used to monitor for recurrence of lung cancer. Thus, there is a need in die art for a panel of markers with high sensitivity (and varying specificities, depending on the clinical indication), such as for detecting lung cancer. Furthermore, there is also a need for novel markers that are useful individually or as part of a panel for detecting lung cancer. Such markers, and panels of markers, would facilitate management of patients with lung cancer, for example.

For a further review of lung cancer diagnostics, including the use of tumor biomarkers as well as CT screening, see the following citations: Schneider, "Tumor markers in detection of lung cancer", Adv Clin Chem. 2006;42:1-41; Bach et al., "Computed tomography screening and lung cancer outcomes", JAMA. 2007 Mar 7;297(9):953-61: and International Early Lung Cancer Action Program Investigators et al., "Survival of patients with stage I lung cancer detected on CT screening", N Engl J Med. 2006 Oct 26;355(17):1763-71. Also see Pepe et al., "Phases of biomarker development for early detection of cancer", J Nat'l Cancer Inst. 2001. 93(14):1054-1061

### DESCRIPTION OF TABLES 1-2

Tables 1 and 2 provide further information for lung cancer markers ("LCM"), including their names, symbols (alternative symbols are indicated in parentheses), Genbank protein accession numbers, and an exemplary protein sequence for each marker (except for the carbohydrate antigens CA 242, CA 19-9, and CA 72-4, for which representative journal citations are provided for each). Exemplary LCM protein sequences are provided as SEQ ID NOS:1-65 (additionally, the carbohydrate antigens CA 242, CA 19-9, and CA 72-4 are also provided). Nucleic acid sequences (e.g., mRNA transcript sequences and genomic DNA) and alternative protein sequences for each marker are well known in the art and can readily be derived using the information provided in Tables 1-2, for example.

The LCM provided in Table 1 are as follows (alternative names/symbols are indicated in parentheses): SLPI, MIF, TIMP1, TFPI, ENO2 (NSE), CEA (CEACAM5), MMP2, AMBP, Cyfra 21-1 (Cyfra, KRT19), SCC (SERPINB3), OPN, defensin (DEFA1, HNP-1, HNP1-3), CA 242, CA 19-9, CA 72-4, MN/CAIX (CA9), ProGRP (GRP), KRTI8 (TPS), ECAD (CDH1), TIMP2, CD44, LGALS3BP, ERBB2 (HER-2), UPA (PLAU), DKK (DKK1), CHGA, VEGF. KITLG, PBEF (visfatin), SORT1 (sortilin), MDK (midkine), IGFBP3, IGFBP4, CTSC, ICAM3, CTGF, LCN2, EGFR, BGN, TIMP3, HGF, MUC16 (CA125), NCAM, CRP, SERPINA1 (ATT), PKM2, RBP, KLK11, KLK13, SAA, and APOC3.

The LCM provided in Table 2 (which are particularly useful as autoantibody markers) are as follows (alternative names/symbols are indicated in parentheses): TP53 (p53), KLKB1, CFL1 (CFLN), EEFIG, HSP90α (HSP90AA1), RTN4, ALDOA, GLG1, PTK7, EFEMP1, SLC3A2 (CD98), CHGB, CEACAM1, ALCAM, HSPB1 (HSP27), LGALS1, and B7H3.

Elevated levels of each of these LCM are indicative of lung cancer, except for sortilin (SORT1), for which low levels are indicative of lung cancer.

### DESCRIPTION OF TABLES 3-12

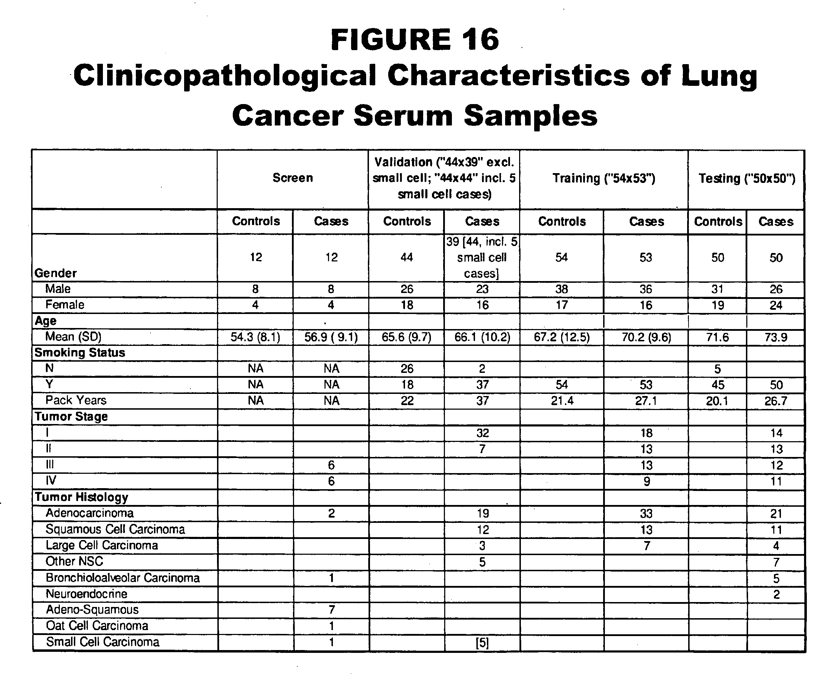

**Table 3** provides 35 different panels of 11 markers (each row of 11 markers represents a panel) that have at least 98% specificity and 82% sensitivity for detecting lung cancer. The total number of occurrences of each marker in these 35 11-marker panels is indicated at the bottom of Table 3. Seven markers (SLPI, TIMP1. TFPI, SCC, OPN, CEA and CA242) appear in all 35 of these panels, GRP appears in 33 of these 35 panels, MIF appears in 29 of these 35 panels, and NSE and HNP-1 each appear in 15 of these 35 panels. AMBP, Cyfra, MMP2, Ca72-4, Cal9-9, and CAIX each appear in 7-9 of these panels, as indicated in Table 3.
**Table 4** provides markers the can be included in any of the panels disclosed herein. For example, the markers in Table 4 can be added to any of the panels disclosed herein and/or can replace one or more members of any of the panels disclosed herein. As a specific example, the markers in Table 4 can be added to any of the panels disclosed in Table 5 and/or can replace one or more members of any of the panels disclosed in Table 5. The markers disclosed in Table 4 are also disclosed in Table 2.
**Tables 5-12** provide data for the analysis of various panels in various lung cancer uses, such as distinguishing lung cancer samples versus normal samples such as for diagnosing/detecting lung cancer (Tables 5-6 and 11-12, for example), as well as certain specific uses (these specific uses, which may be referred to herein as "indications" or as determining or assessing lung cancer "characteristics", are provided in Tables 7-10, for example). In Tables 5-12, each row represents a panel (a panel may comprise an individual marker). For each panel in Tables 5-12, data are presented based on logistic regression and/or split point analysis (as indicated in each table). Area under the curve (AUC), sensitivity at 95% specificity, and specificity at 95% sensitivity are indicated. "Size" (second column) indicates the number of markers in the given panel. Further information regarding characteristics of the sample sets (the "54x53", "50x50", and "44x44" sample sets) used in each of the analyses is provided in Figure 16 (the "104x 103" sample set used in Table 8 is the "54x53" and "50x50" sample sets combined). In Tables 5-12, and elsewhere herein, "trained" refers to the sample set (which may be referred to as the "training set") which was used to formulate cutoff levels, and "tested" refers to the sample set (which may be referred to as the "testing set") to which these cutoff levels were applied (such as to classify a sample as a lung tumor or normal sample, or other specific use, based on whether marker levels were above or below the cutoff levels established from the training set).
**Table 5** provides data for logistic regression and split-point analysis of the 9-marker panel of Cyfra, SLPI, TIMP1, SCC, TFPI, CEACAM5. MMP22, OPN, and MDK, and all subcombinations thereof (including individual markers), in distinguishing lung tumor samples versus normal (i.e., control/healthy) samples, such as for diagnosing/detecting lung cancer. For each panel in Table 5, data are presented based on logistic regression and split point analysis and based on analysis of either training and testing on the same 54x53 (54 controls and 53 cases) sample set, or training on the 54x53 sample set and testing on the 50x50 (50 controls x 50 cases) sample set (see Figure 16 for characteristics of these sample sets). Area under the curve (AUC), sensitivity at 95% specificity, and specificity at 95% sensitivity are indicated. The panels are sorted based on the AUC indicated in the third column. "Size" (second column) indicates the number of markers in the given panel. Thus, Table 5 provides the 9-marker panel of Cyfra, SLPI, TIMP1, SCC, TFPI, CEACAM5, MMP2, OPN, and MDK, and all panel subcombinations thereof, including each of these nine markers individually (each row represents a panel).
**Table 6** provides data for split-point analysis of panels (including individual markers) that include any of the nine markers in the panels provided in Table 5 and/or various other markers (which are not in the panels provided in Table 5) in distinguishing lung tumor samples versus normal (i.e., control/healthy) samples, such as for diagnosing/detecting lung cancer.
**Table 7** provides data for logistic regression analysis of the 9-marker panel of Cyfra, SLPI, TIMP1, SCC, TFPI, CEACAM5, MMP2, OPN, and MDK, and subcombinations thereof (including individual markers), in distinguishing adenocarcinoma versus squamous cell carcinoma types of lung cancer.
**Table 8** provides data for split-point analysis of the 9-marker panel of Cyfra, SLPI, TIMP1, SCC, TFPI, CEACAM5, MMP2, OPN, and MDK, and subcombinations thereof (including individual markers), in distinguishing stage I versus stage III lung cancer. In addition to their utility in distinguishing between early and late stage lung cancer (e.g., stage I or II versus stage III or IV), the panels provided in Table 8 are also useful for distinguishing between any other stages of lung cancer (e.g., any of stages I, II, III, and IV).
**Table 9** provides data for split-point analysis of various panels in distinguishing small cell lung cancer (SCLC) versus other types of lung cancer (e.g., non-small cell lung cancer, NSCLC). In the left-side of Table 9, marker levels are higher in NSCLC (as compared to SCLC). In the right-side of Table 9, marker levels are higher in SCLC (as compared to NSCLC).
**Table 10** provides data for split-point analysis of the 9-marker panel of Cyfra, SLPI, TIMP1, SCC, TFPI, CEACAM5, MMP2, OPN, and MDK, and subcombinations thereof (including individual markers), in distinguishing malignant lung tumors versus benign lung lesions.
**Table 11** provides data for split-point analysis of various panels in distinguishing small cell lung cancer (SCLC) versus normal (i.e., control/healthy) samples.
**Table 12** provides data for split-point analysis of various panels in distinguishing lung cancer (including both small cell lung cancer (SCLC) and non-small cell lung cancer (NSCLC)) versus normal (i.e., control/healthy) samples.

### BRIEF DESCRIPTION OF THE FIGURES

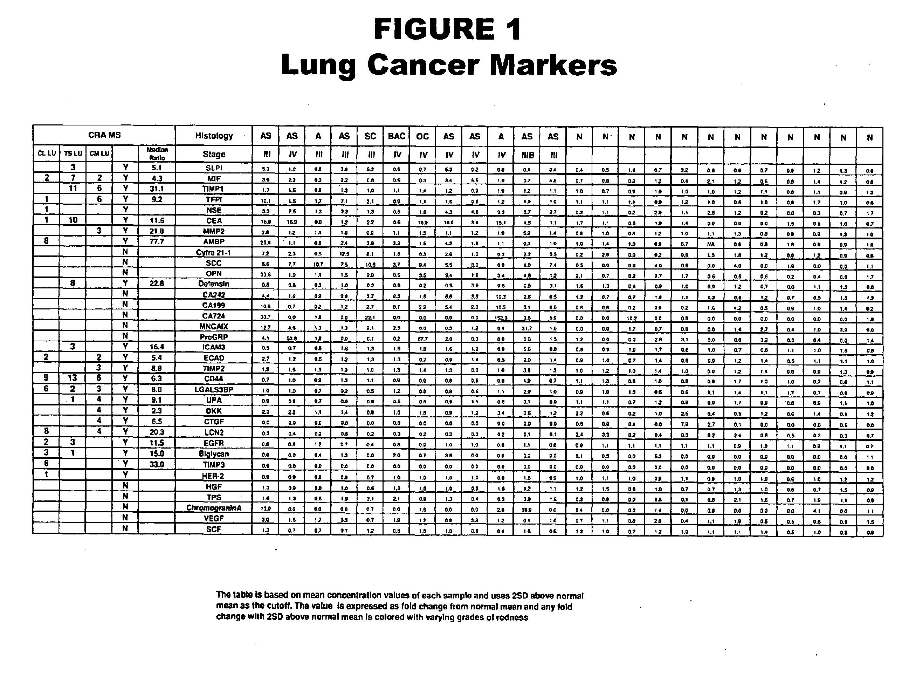

**Figure 1****.** Shows relative expression levels for exemplary lung cancer markers (LCM) screened by ELISA in a sample set of 12 lung tumor and 12 normal serum samples. The left portion of the table shown in Figure 1 provides tumor samples identified by histology and tumor state (histology abbreviations for tumor samples are "AS" = adenosquamous, "A" = adenocarcinoma, "SC" = squamous cell carcinoma, and "BAC" = bronchioalveolar carcinoma), and the right portion of the table shows normal samples (identified as "N" for histology). The table is based on mean concentration values of each sample and uses 2 standard deviations (2SD) above normal mean as the cutoff: the value is expressed as fold change from normal mean (thus, any fold change with 2SD above normal mean is above the cutoff). The column labeled "CRA MS" is a summary of mass spectrometry data that indicates the number of differentially expressed lung tumor samples and the median mass spec ratio of these samples (numerical representation of over-expression is indicated by 2.0 or more, whereas numerical representation of under-expression is indicated by 0.5 or less) (lung tumor sample abbreviations for mass spectrometry are "CL LU" = lung cancer cell lines, "TS LU" = lung cancer tissues, and "CM LU" = lung cancer conditioned medium).

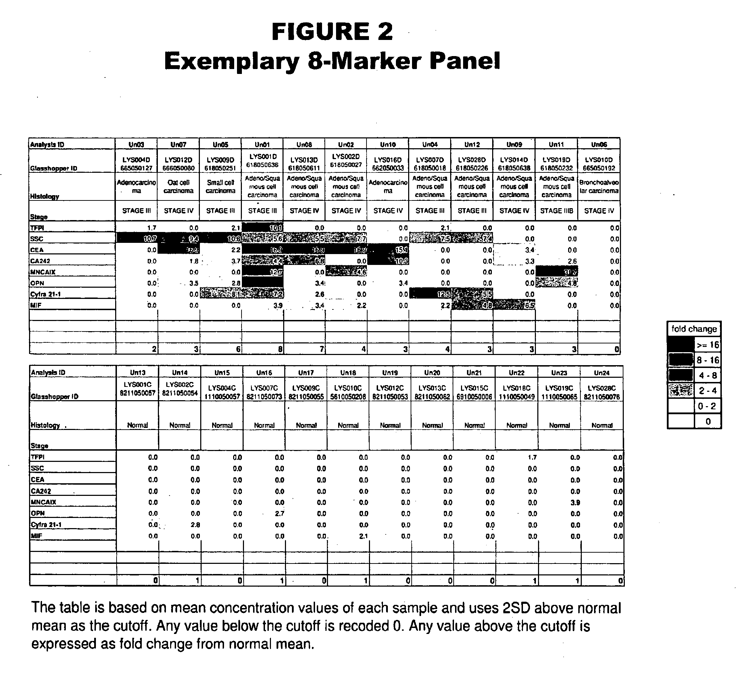

**Figure 2****.** Shows relative expression levels based on ELISA screening of a sample set of 12 lung tumor (upper section) and 12 normal (lower section) serum samples for the eight markers TFPI, SCC (interchangeably referred to as SSC), CEA, CA242, MNCAIX, OPN, Cyfra 21-1, and MIF (as also shown in Figure 1). The table is based on mean concentration values of each sample and uses 2 standard deviations (2SD) above normal mean as the cutoff; the value is expressed as fold change from normal mean (thus, any fold change with 2SD above normal mean is above the cutoff). Any value below the cut-off is recoded as 0. Any or all of these eight markers may be used in combination as a panel for lung cancer assessment, and the panel may optionally include additional markers.

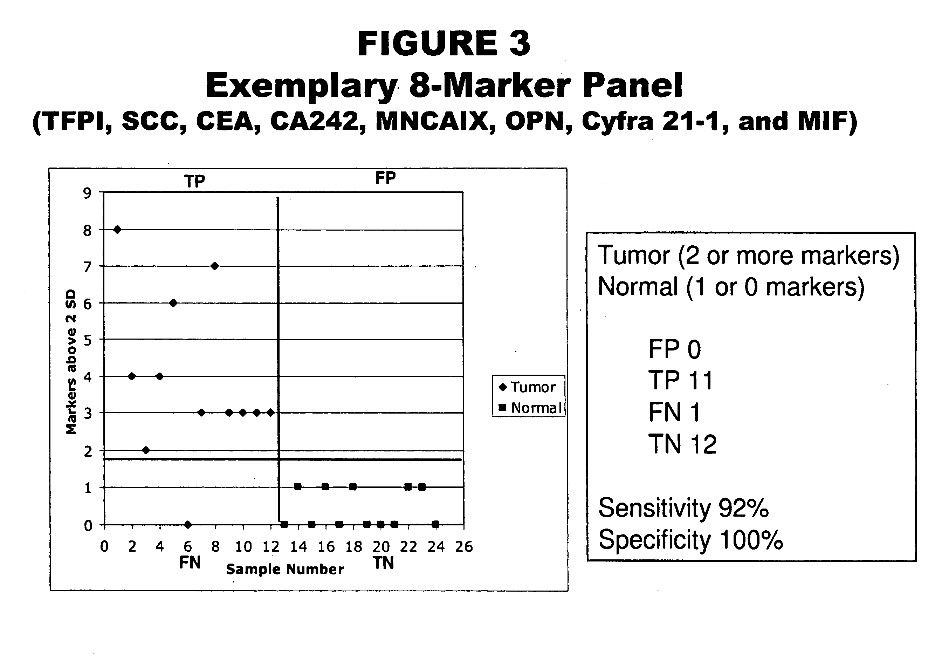

**Figure 3****.** Shows the performance of the eight marker panel of TFPI, SCC, CEA, CA242, MNCAIX, OPN, Cyfra 21-1, and MIF. Using an algorithm in which markers greater than or equal to two standard deviations were scored "positive", this panel of eight markers had a sensitivity of 92% and specificity of 100% among the 12 sera from lung cancer patients and 12 sera from healthy controls ("FP" = false positives, "TP" = true positives, "FN" = false negatives, and "TN" = true negatives)

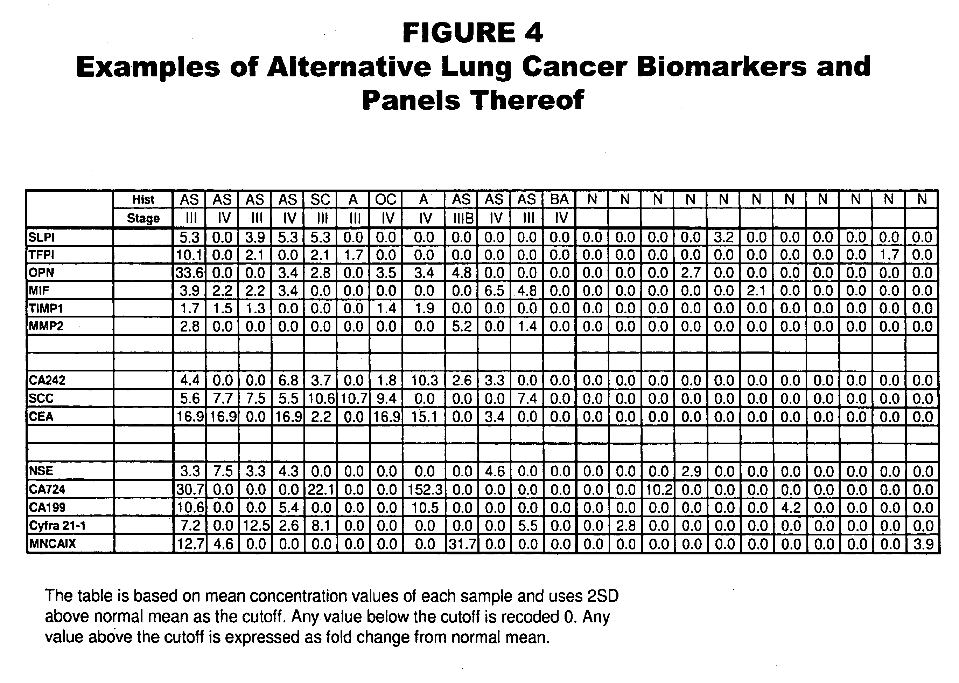

**Figure 4****.** Shows relative expression levels based on ELISA screening of a sample set of 12 lung tumor (left portion; histology ("Hist") abbreviations are "AS" = adenosquamous, "A" = adenocarcinoma, "SC" = squamous cell carcinoma, "OC" = oat cell carcinoma, and "BAC" = bronchioalveolar carcinoma) and 12 normal (right portion: identified as "N" for histology) serum samples for alternate panels of LCM, including a panel of the markers SLPI, TFPI, OPN, MIF, TIMP1, and MMP2. Any or all of these markers can also be used in any combination with any or all of the following markers: CA242, SCC, CEA, NSE, CA72-4, CA19-9, Cyfra 21-1, and MN/CAIX, as shown in Figure 4. The table is based on mean concentration values of each sample and uses two standard deviations (2SD) above normal mean as the cut-off; the value is expressed as fold change from normal mean (thus, any fold change with 2SD above normal mean is above the cutoff). Any value below the cut-off is recoded as 0.

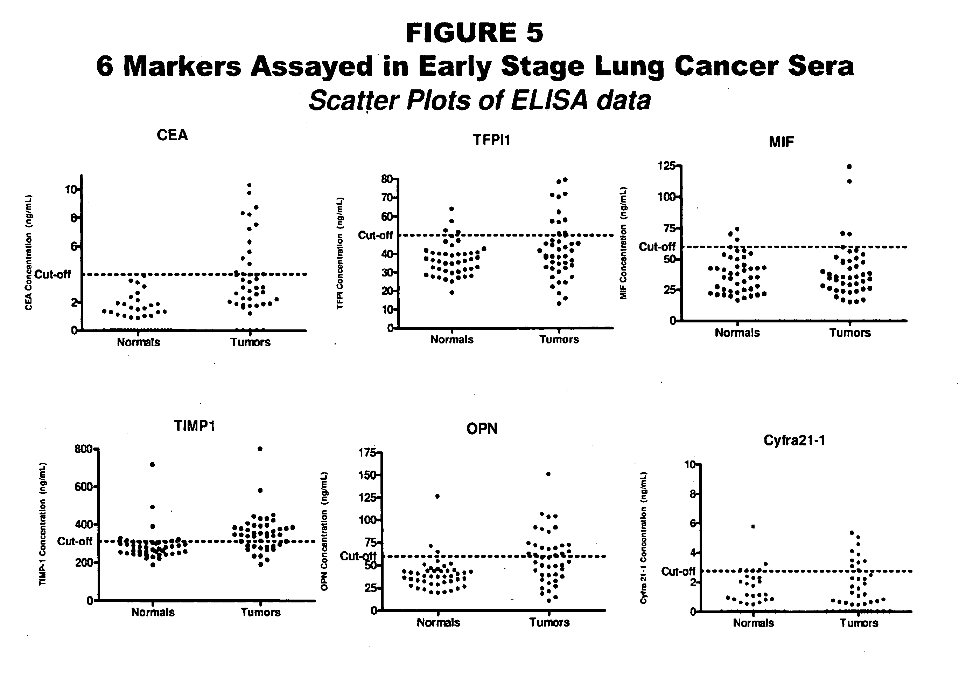

**Figure 5****.** Shows scatter plots of ELISA data for the six markers CEA, TFPI, MIF, TIMP1, OPN, and Cyfra 21-1 in 44 normal and 44 lung tumor samples, with exemplary cut-offs indicated (dotted lines). Cut-offs can be applied that maximize sensitivity while not compromising specificity of the panel, for example.

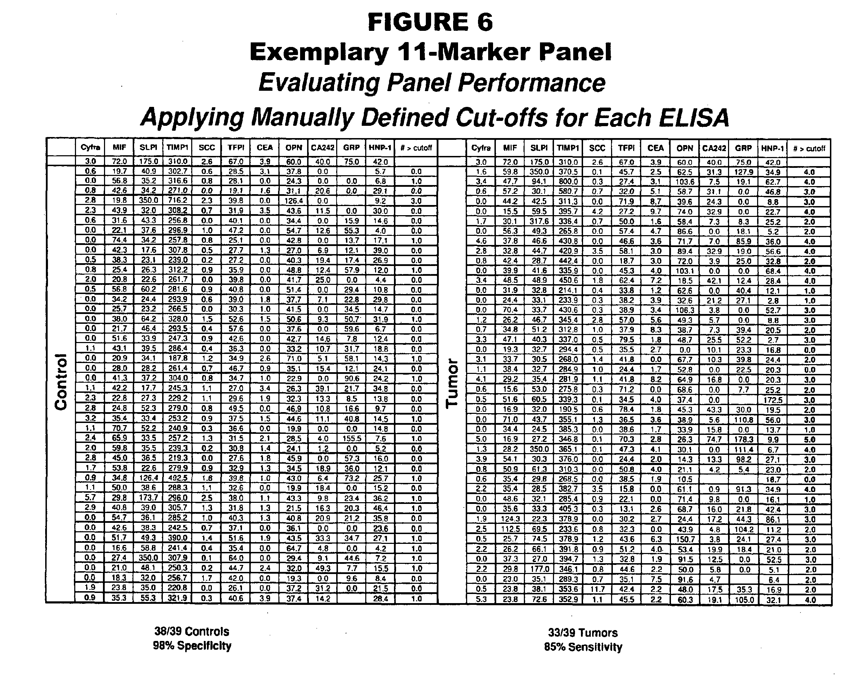

**Figure 6****.** Shows results of ELISA analysis for the 11 markers Cyfra 21-1, MIF. TIMP1, TFPI, CEA, OPN, SCC, SLPI, HNP-1, GRP, and CA242 in 39 control (normal) samples (left portion, labeled "Control") and 39 lung tumor samples (right portion, labeled "Tumor"). Values shown are concentration (ng/mL). Manually defined cut-offs are indicated immediately below each marker name. The columns labeled "# > cutoff" indicate the total number of markers with elevated expression (i.e., a concentration greater than the manually defined cut-offs) in a given serum sample. "Stage" indicates lung cancer stage, and "Hist Type" indicates histology type. Any or all of these 11 markers may be used in combination as a panel for lung cancer assessment, and the panel may optionally include additional markers.

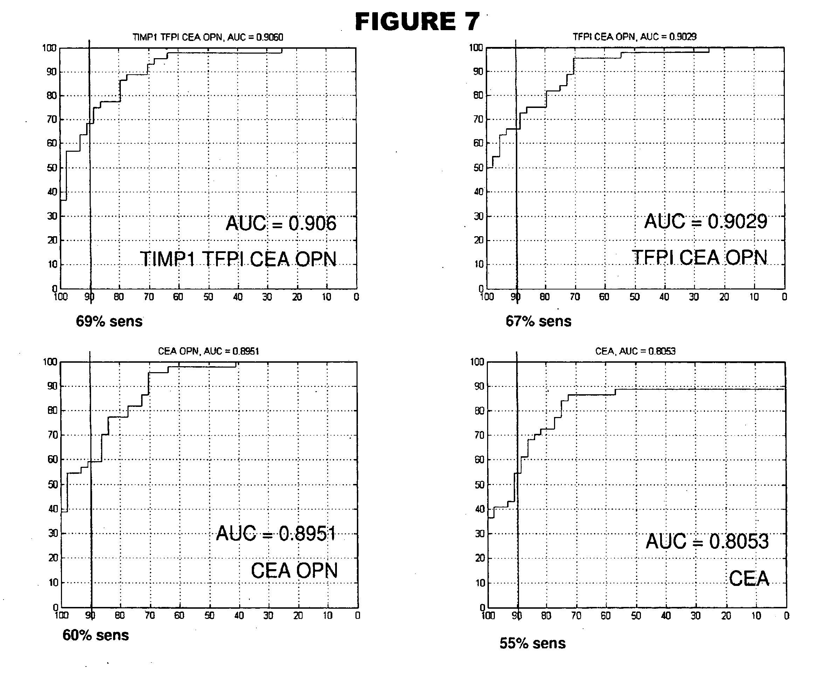

**Figure 7****.** Shows performance of exemplary panels of markers, demonstrating that increased sensitivity can be achieved by including additional markers. The marker CEA provides 55% sensitivity and 90% specificity, the two markers CEA and OPN provide 60% sensitivity and 90% specificity, die three markers TFPI, CEA, and OPN provide 67% sensitivity and 90% specificity, and the four markers TIMP1, TFPI, CEA, and OPN provide 69% sensitivity and 90% specificity. The score is the sum of the log₂ of the ratios of the tumor concentration to the mean concentration in normal serum. ROC curves can be constructed by varying the cut-off of the score needed to call a sample a tumor.

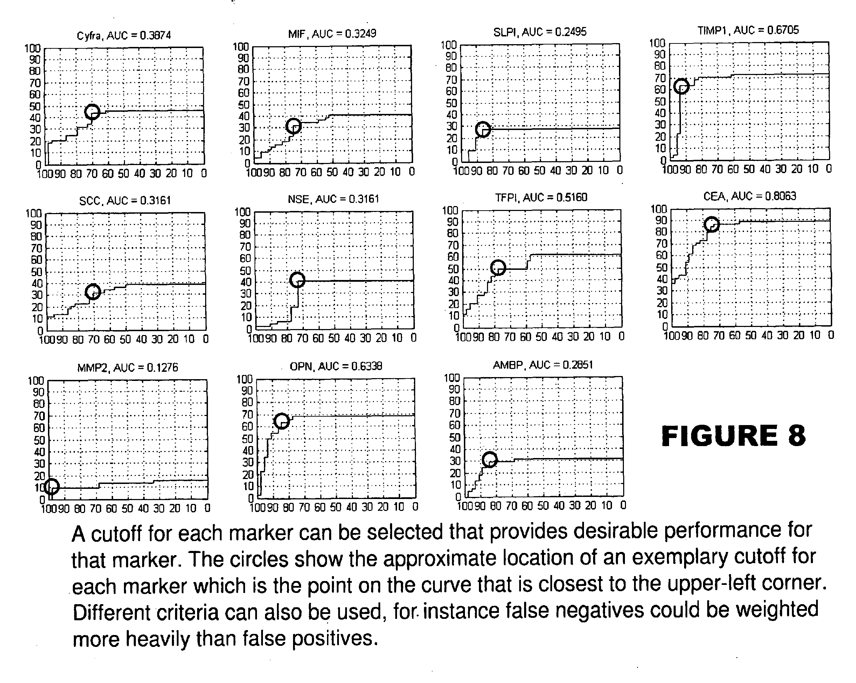

**Figure 8****.** Shows examples of applying a cut-off for various markers (Cyfra 21-1, MIF, SLPI, TIMP1, SCC, NSE, TFPI, CEA, MMP2, OPN, and AMBP are shown) that provides desirable performance for that marker. The circles show the approximate location of an exemplary cut-off for each marker which is the point on the curve that is closest to the upper-left corner. Different criteria can also be used, for instance false negatives could be weighted more heavily than false positives.

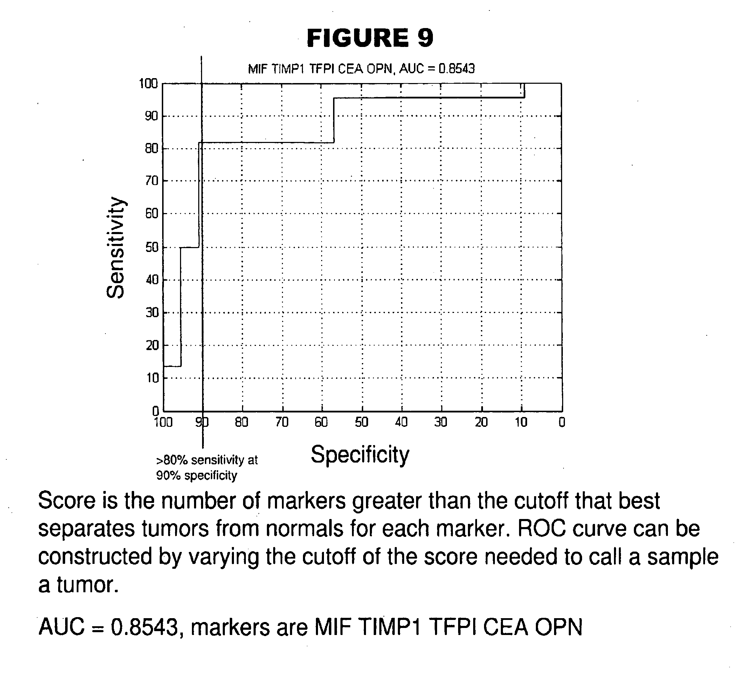

**Figures 9-10****.** Shows AUC (area under curve) = 0.8543 for markers M IF, TIMP1, TFPI, CEA, and OPN (Figure 9), and AUC = 0.8518 for markers TIMP1, NSE, CEA, and OPN. Score is the number of markers greater than the cutoff that best separates tumor samples from normal samples for each marker. ROC curve can be constructed by varying the cut-off of the score needed to call a sample a tumor.

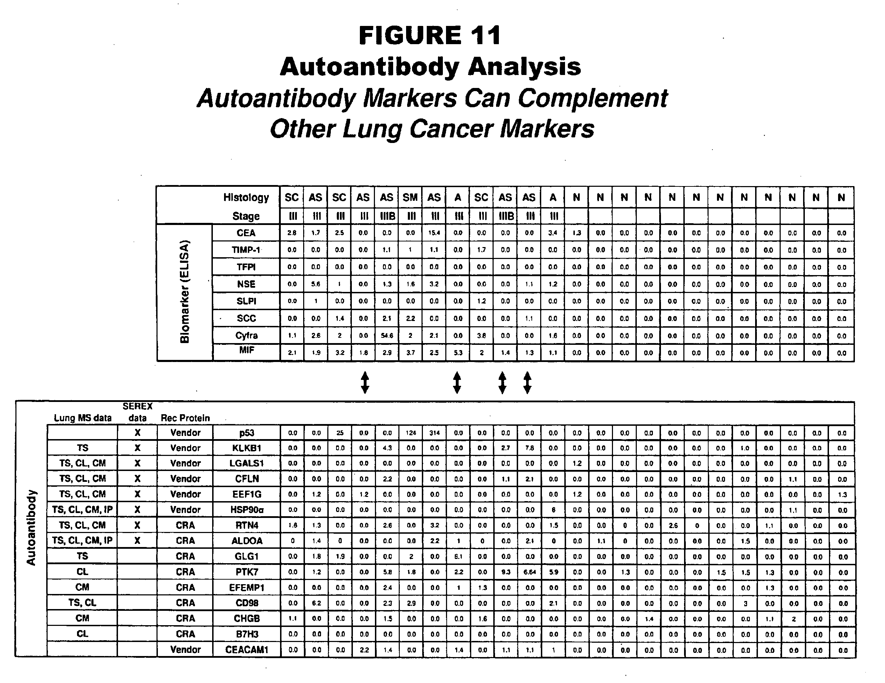

**Figure 11****.** Shows results of analysis for certain autoantibody markers (bottom table), as well as certain other lung cancer markers (top table). In the bottom table (autoantibody markers), the column labeled "Lung MS data" indicates a summary of where differential expression has been observed by mass spectrometry (CL=cell lines, TS= tissues, CM= conditioned medium, and IP=immunoprecipitation), the column labeled "SEREX data" indicates autoantibody markers that overlap with the Serological Expression (SEREX) database which identifies markers that elicit a high-titer IgG antibodies, and the column labeled "Rec Protein" indicates the source of recombinant protein used for autoantibody analysis ("vendor" indicates an external commercial source and "CRA" indicates an internal source). Histology abbreviations for tumor samples in the top table are "AS" = adenosquamous, "A" = adenocarcinoma, "SC" = squamous cell carcinoma, and "SM" = small cell carcinoma.

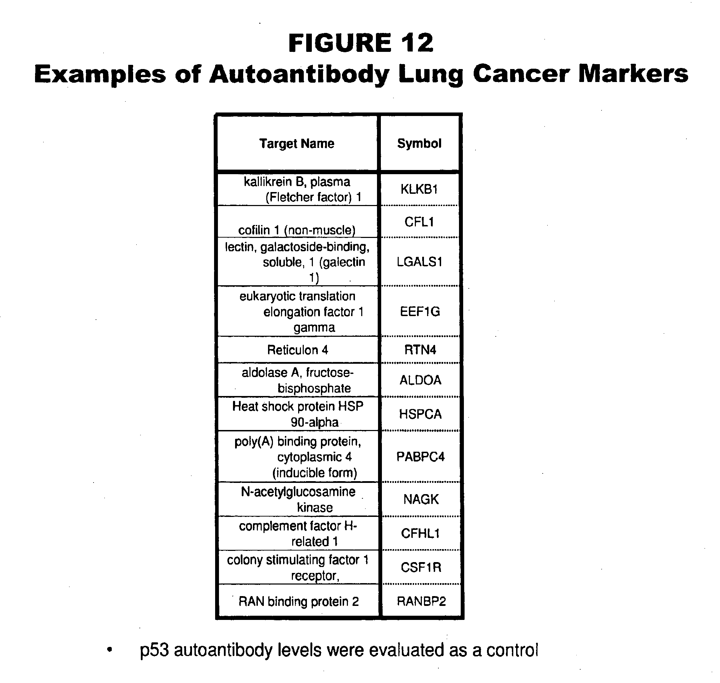

**Figure 12****.** Shows exemplary autoantibody LCM, which can be used alone or in combination with other LCM. Certain of these autoantibody LCM are also provided in Table 2 long with other autoantibody LCM.

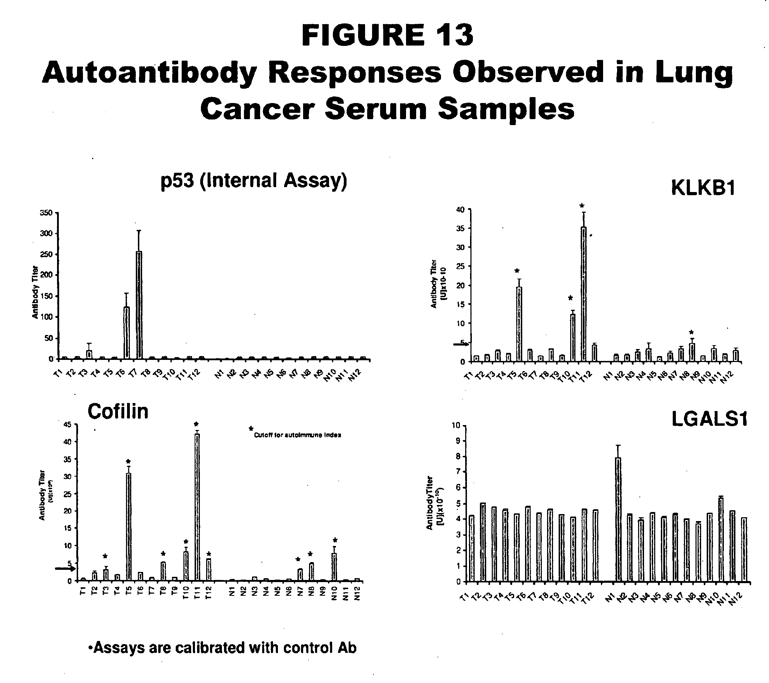

**Figure 13****.** Shows autoantibody responses observed in lung cancer and normal serum samples for the autoantibody markers KLKB1, cofilin, and LGALS1, as well as p53. Along the horizontal axis, T1 through T12 indicate tumor samples and N through N12 indicate normal samples.

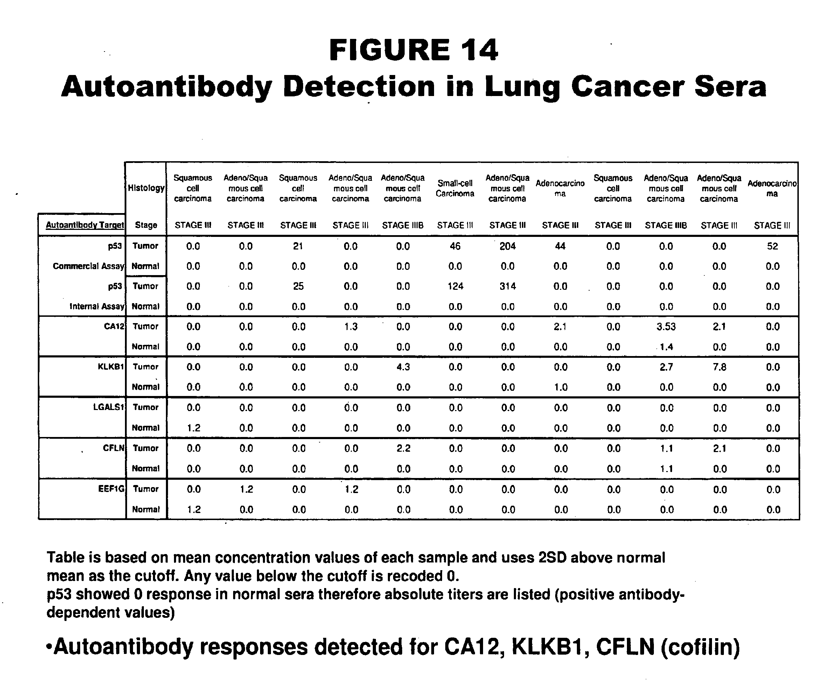

**Figure 14****.** Shows autoantibody detection in lung cancer and normal serum samples for the autoantibody markers CA12, KLKB1, CFLN, LGALS1, and EEFIG, as well as p53. The table is based on mean concentration values of each sample and uses 2SD above normal mean as the cut-off. Any value below the cutoff is recoded as 0.

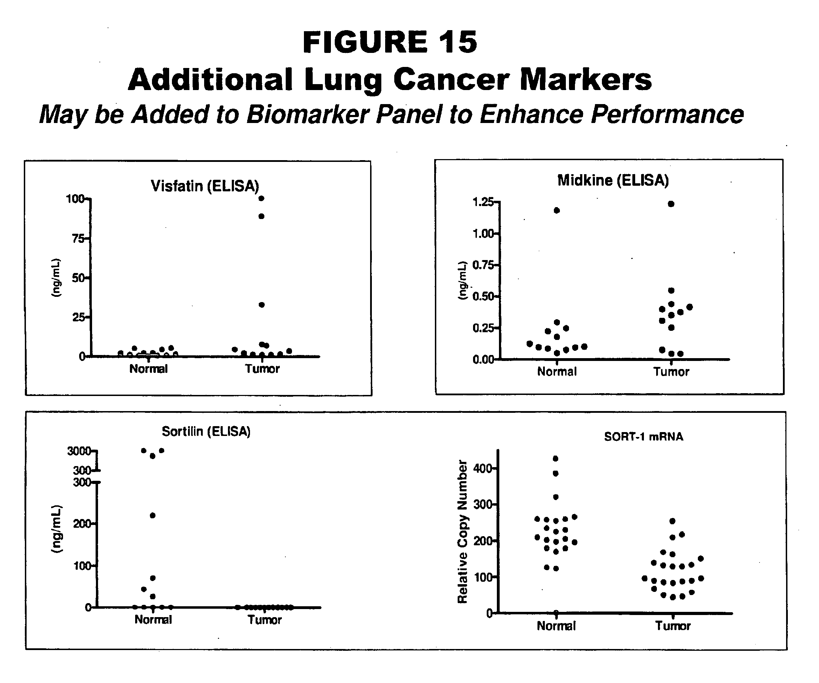

**Figure 15****.** Shows three additional LCM: visfatin (PBEF), sortilin (SORT-1), and midkine (MDK). Any or all of these three LCM can be implemented in a panel of markers for lung cancer diagnosis, for example. Figure 15 shows abundance levels (in ng/mL) of these three markers in 12 normal lung and 12 lung tumor samples based on ELISA analysis. For sortilin (SORT-1), abundance levels (by relative copy number) of this marker based on mRNA expression analysis of 22 normal lung and 23 lung tumor samples is also provided. For sortilin, lung tumor samples have a decreased abundance level of this marker compared with normal lung samples.
**Figure 16****.** Shows clinicopathological characteristics of lung cancer serum samples used in various analyses disclosed herein.

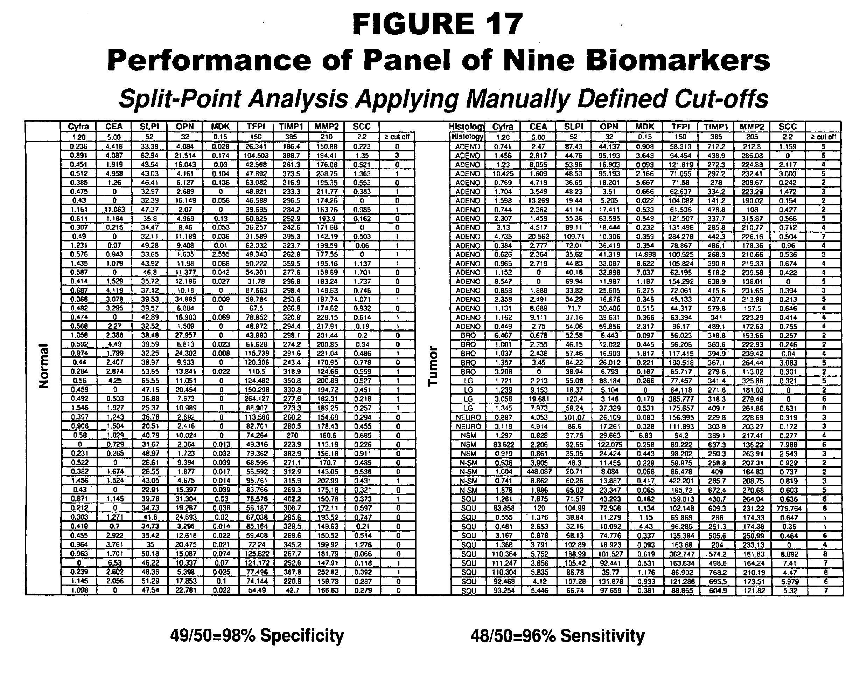

**Figure 17****.** Shows results of ELISA analysis for the 9-marker panel of Cyfra, SLPI, TIMP1, SCC, TFPI, CEACAM5, MMP2, OPN, and MDK in 50 control (normal) samples (left portion, labeled "Normal") and 50 lung tumor samples (right portion, labeled "Tumor"), using split-point analysis applying manually defined cut-offs. The manually defined cut-offs are indicated immediately below each marker name. Values shown are concentration (ng/mL). The columns labeled "≥ cut off' indicate the total number of markers with elevated expression (i.e., a concentration greater than or equal to the manually defined cut-offs) in a given serum sample. "Histology" indicates histology type ("adeno" = adenocarcinoma, "squ" = squamous cell carcinoma, "nsm" or "n-sm" = non-small cell carcinoma, "bro" = bronchioloalveolar carcinoma, "LG" = large cell carcinoma, and "neuro" = neuroendocrine). Any or all of these nine markers may be used in combination as a panel for lung cancer assessment, and the panel may optionally include additional markers.

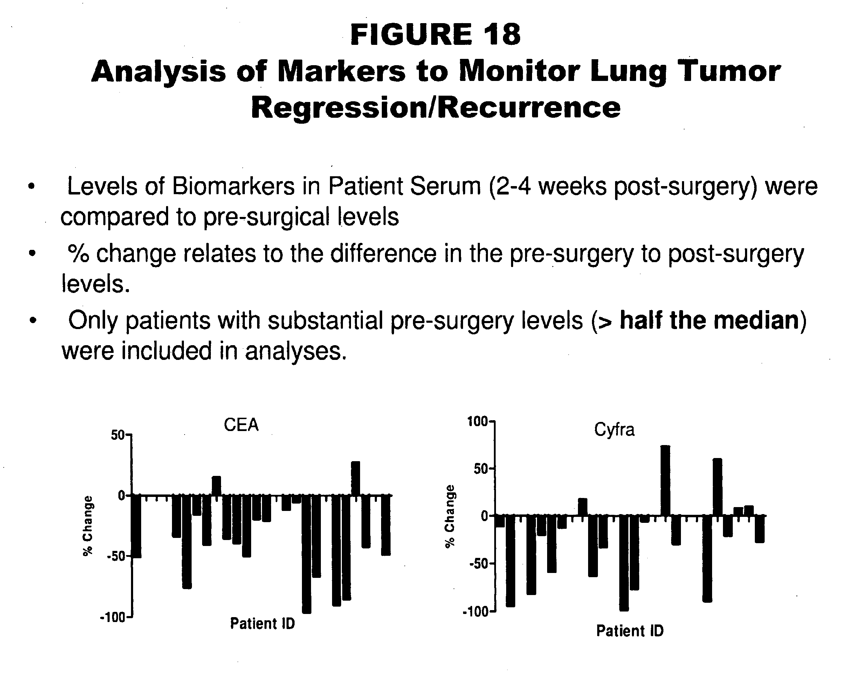

**Figure 18****.** Describes the analysis of markers to monitor lung tumor regression/recurrence, with CEA and Cyfra as examples. In particular, levels of biomarkers in patient serum 2-4 weeks following surgery were compared to pre-surgical marker levels.

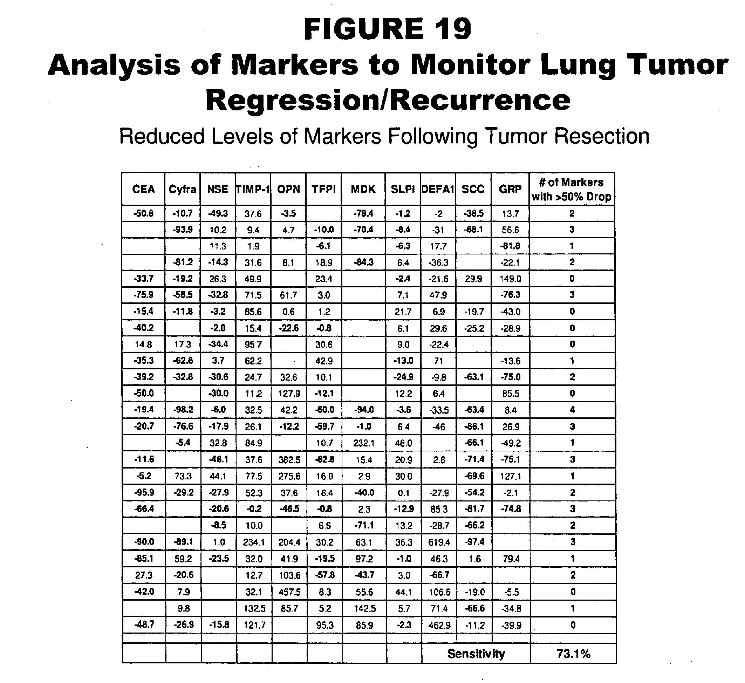

**Figure 19****.** Shows an analysis of markers to monitor for lung tumor regression/recurrence. Percentage change in levels of biomarkers in patient serum 2-4 weeks post-surgery as compared to pre-surgical levels is indicated.

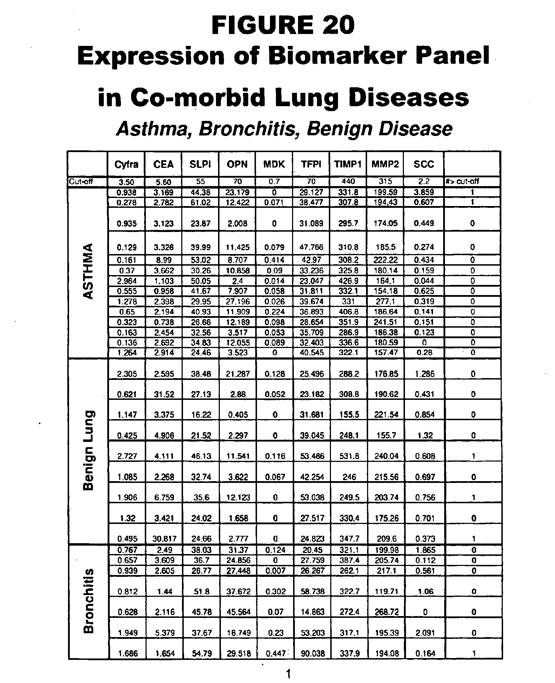

**Figure 20****.** Shows an analysis of the expression levels of the 9-marker panel of Cyfra, SLPI, TIMP1, SCC, TFPI, CEACAM5, MMP2, OPN, and MDK in the following co-morbid lung diseases: asthma, bronchitis, and benign lung diseases. Values shown are concentration (ng/mL). The column labeled "# > cut off' indicates the total number of markers with elevated expression (i.e., a concentration greater than the manually defined cut-offs) in a given serum sample. The manually defined cut-offs are indicated immediately below each marker name (in the row labeled "Cut-off").

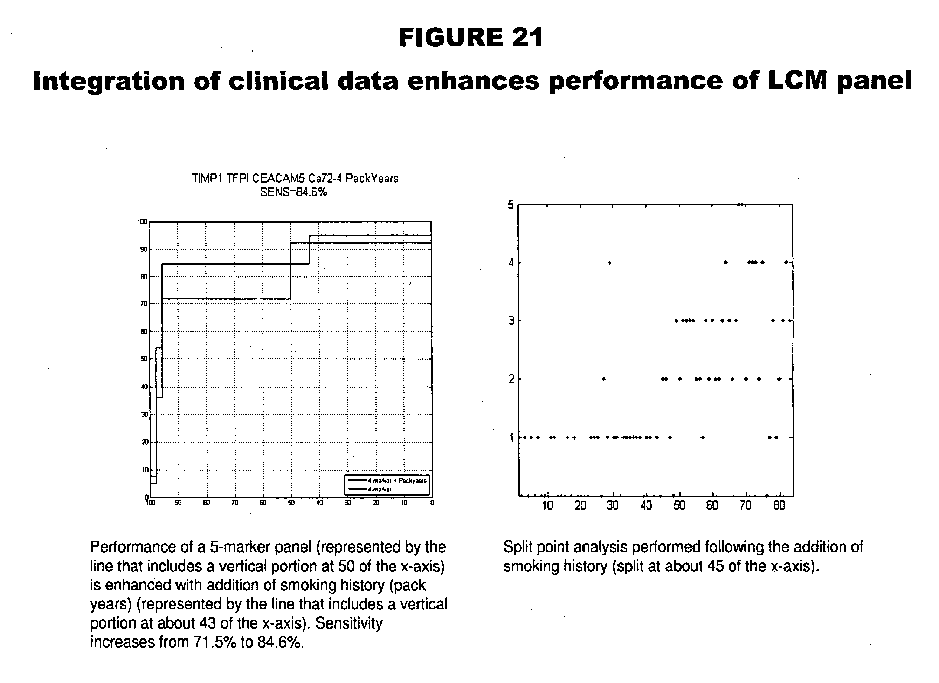

**Figure 21****.** Shows an analysis of integrating an exemplary supplemental biomedical parameter (smoking history) with an exemplary LCM panel (TIMP1, TFPI, CEACAM5, and Ca72-4) plus pack years ("pack year": number of cigarettes smoked per day multiplied by number of years of smoking at this rate).

### DETAILED DESCRIPTION OF EXEMPLARY EMBODIMENTS OF THE INVENTION

The present invention relates to a method for detecting lung cancer in an individual, the method comprising detecting the levels of protein markers in the panel comprising TFPI, SCC, OPN, MDK, CEACAM5, and Cyfra in a sample from said individual, wherein elevated levels of at least one of said protein markers indicate that said individual has lung cancer.

Further, the present invention relates to the use of a composition comprising a substrate and detection reagents for detecting a plurality of lung cancer protein markers comprising Cyfra, SCC, TFPI, CEACAM5, OPN, and MDK, wherein the detection reagents are coupled to the substrate for detecting lung cancer in sample from an individual.

Finally, the present invention relates to the use of a kit comprising at least one container and detection reagents for detecting a plurality of lung cancer protein markers comprising Cyfra, SCC, TFPI, CEACAM5, OPN, and MDK, wherein the detection reagents are stored in one or more containers for detecting lung cancer in a sample from an individual.

The invention will best be understood by reference to the following detailed description of the exemplary embodiments, taken in conjunction with the accompanying table(s) and/or figure(s).

Exemplary aspects disclosed herein relate to the following markers (see Tables 1-2). combinations of these markers, and methods of using these markers. particularly for lung cancer-related uses, and especially for lung cancer diagnostics (alternative names/symbols are indicated in parentheses): SLPI, MIF, TIMP1, TFPI, ENO2 (NSE), CEA (CEACAM5), MMP2, AMBP, Cyfra 21-1 (Cyfra, KRT19). SCC (SERPINB3), OPN, defensin (DEFA1, HNP-1, HNP1-3), CA 242, CA 19-9, CA 72-4, MN/CAIX (CA9), ProGRP (GRP), KRT18 (TPS), ECAD (CDH1), TIMP2, CD44, LGALS3BP, ERBB2 (HER-2), UPA (PLAU), DKK (DKK1), CHGA, VEGF, KITLG, PBEF(visfatin), SORT1 (sortilin), MDK (midkine), IGFBP3, IGFBP4, CTSC, ICAM3, CTGF, LCN2, EGFR, BGN, TIMP3, HGF, MUC16 (CA125), NCAM, CRP, SERPINA1 (ATT), PKM2, RBP, KLK11, KLK13, SAA, APOC3, TP53 (p53), KLKB1, CFL1 (CFLN), EEFIG, HSP90α (HSP90AA1), RTN4, ALDOA, GLG I, PTK7, EFEMP1, SLC3A2 (CD98), CHGB, CEACAM1, ALCAM, HSPB1 (HSP27), LGALS1, and B7H3, which are collectively referred to herein as "LCM" ("lung cancer makers"). Elevated levels of each of these LCM are indicative of lung cancer, except for sortilin (SORT1), for which low levels are indicative of lung cancer. Tables 1 and 2 provide further information for each of these LCM, including their names, symbols, Genbank protein accession numbers, and an exemplary protein sequence for each marker (except for the carbohydrate antigens CA 242, CA 19-9, and CA 72-4, for which representative journal citations are provided for each). Exemplary LCM protein sequences are provided as SEQ ID NOS:1-65 (additionally, the carbohydrate antigens CA 242, CA 19-9, and CA 72-4 are also provided). Nucleic acid sequences (e.g., mRNA transcript sequences and genomic DNA) and alternative protein sequences for each marker are well known in the art and can readily be derived using the information provided in Tables 1-2, for example. The markers provided in Table 2 are particularly useful as autoantibody markers.

Certain aspects of the description disclose combinations comprising, consisting of, and consisting essentially of the following nine LCM, and subcombinations thereof (these nine LCM may be referred to herein as the "9-marker panel", which is shown in Figure 17 and Table 5, for example): Cyfra, SLPI, TIMP1, SCC, TFPI, CEACAM5, MMP2, OPN, and MDK, Certain embodiments of the invention provide the use of compositions based on this 9-marker panel and subcombination thereof, and methods for detecting using this 9-marker panel, particularly for uses related to lung cancer (such as detecting lung cancer). In certain aspects disclosed herein, one or more members of this 9-marker panel is replaced by one or more markers shown in Table 4 and/or one or more markers shown in Table 4 is added to this 9-marker panel. With respect to the nine markers Cyfra, SLPI, TIMP1, SCC, TFPI. CEACAM5, MMP2, OPN, and MDK, elevated levels are indicative of lung cancer (for all of the LCM disclosed herein, elevated levels are indicative of lung cancer, except for sortilin (SORT1), for which low levels are indicative of lung cancer). In certain embodiments, if the levels of two or more markers (i.e., a "plurality") of the 9-marker panel are elevated in a sample (e.g., a serum sample) from an individual, this indicates that the individual has lung cancer. In various other embodiments, if the levels of one or more, three or more, four or more, five or more, six or more, seven or more, eight or more, or all nine markers of the 9-marker panel are elevated in a sample from an individual, this indicates that the individual has lung cancer. In certain embodiments, a marker is classified as being elevated if its level is greater than (or greater than or equal to) a predetermined cutoff level.

Furthermore, disclosed herein are combinations comprising, consisting of, and consisting essentially of the following six LCM (each of which is also contained in the above 9-marker panel), and subcombinations thereof (these six LCM may be referred to herein as the "6-marker subset of the 9-marker panel", which is shown in Table 5): Cyfra, SLP1, TIMP1, TFPI, CEACAM5, and MDK. Certain aspects of the description relate to compositions based on this 6-marker subset of the 9-marker panel and subcombination thereof, and methods of using this 6-marker subset of the 9-marker panel, particularly for uses related to lung cancer (such as detecting lung cancer). In certain aspects, one or more members of this 6-marker subset of the 9-marker panel is replaced by one or more markers shown in Table 4 and/or one or more markers shown in Table 4 is added to this 6-marker subset of the 9-marker panel. With respect to the six markers Cyfra, SLPI, TIMP1, TFPI, CEACAM5, and MDK, elevated levels are indicative of lung cancer (for all of the LCM disclosed herein, elevated levels are indicative of lung cancer, except for sortilin (SORT1), for which low levels are indicative of lung cancer). In certain aspects of the disclosure, if the levels of two or more markers (i.e., a "plurality") of the 6-marker subset of the 9-marker panel are elevated in a sample (e.g., a serum sample) from an individual, this indicates that the individual has lung cancer. In various other aspects of the disclosure, if the levels of one or more, three or more, four or more, five or more, or all six markers of the 6-marker subset of the 9-marker panel are elevated in a sample from an individual, this indicates that the individual has lung cancer. In certain aspects, a marker is classified as being elevated if its level is greater than (or greater than or equal to) a predetermined cutoff level.

Exemplary aspects of the present disclosure are LCM and combinations of LCM (combinations of LCM may be interchangeably referred to herein as panels), and uses thereof, particularly uses related to lung cancer. For example, Exemplary aspects of the present disclosure are methods and compositions for assessing (e.g., diagnosing/detecting, prognosing, or predicting drug response), treating, and preventing diseases, especially cancer, and particularly lung cancer, using LCM. Furthermore, the compositions and methods disclosed herein may be suitable for other types of cancer, particularly other epithelial cell-related cancers and solid tumors, as well as other lung diseases.

LCM proteins and fragments thereof (LCM peptides), LCM carbohydrate antigens and fragments thereof, and LCM nucleic acid molecules and fragments thereof encoding LCM proteins and peptides, are collectively referred to as "LCM" or "markers" (which may be interchangeably referred to as "biomarkers", "antigens", or "targets").

The terms "protein" and "polypeptide" are used herein interchangeably. Furthermore, references herein to proteins/polypeptides may also typically encompass carbohydrate antigens ("CA"): for example, references to LCM proteins/polypeptides may also typically encompass the carbohydrate antigens CA 242, CA 19-9, and CA 72-4. Exemplary LCM protein/polypeptide sequences are provided as SEQ ID NOS: 1-65 (additionally, carbohydrate antigens CA 242, CA 19-9, and CA 72-4 are also provided). A "peptide" typically refers to a fragment of a protein/polypeptide. Thus, peptides are interchangeably referred to as fragments. References herein to proteins, peptides, carbohydrate antigens, nucleic acid molecules, and antibodies typically are not limited to the full-size or full-length molecule, but also can encompass fragments of these molecules (unless a particular sequence or structure is explicitly stated).

As used herein, a "lesion" (e.g., a lung lesion) may be interchangeably referred to as a "nodule" (e.g., a lung nodule), and "lung" may be interchangeably referred to as "pulmonary".

As used herein, "subcombinations" (of LCM) may be interchangeably referred to as "subsets" (of LCM).

"Abundance level" may be interchangeably referred to herein as "expression level", or just "level" or "abundance". Determination of LCM levels may be referred to herein as "quantifying" LCM, or "quantification" of LCM.

A "differential" abundance level is a level of a marker (e.g., LCM protein or nucleic acid) in a test samples (e.g., a disease sample) either above or below the normal abundance level of the same marker in a corresponding control or normal sample or group of control/normal samples (e.g., a sample set or population). Thus, for example, a "differential" abundance level can encompass either a "high" (or "increased") or "low" (or "decreased") abundance level. An example of a normal abundance level for a LCM is the mean abundance level of the marker in individuals who do not have lung cancer, which may be the mean abundance of the marker in, for example, a particular control samples set or population of individuals who do not have lung cancer. The normal abundance may also be the typical abundance level of a marker in a normal cell (e.g.. a normal lung cell) compared with the typical abundance level of the marker in a corresponding disease cell (e.g., a lung cancer cell).

An example of a "high", "increased", or "elevated" (these terms are used herein interchangeably) abundance level for a LCM is an abundance level that is at least two standard deviations above the normal abundance level of the marker (e.g., the mean abundance level of the marker in individuals who do not have lung cancer). An example of a "low" or "decreased" abundance level for a LCM is an abundance level that is at least two standard deviations below the normal abundance level of the marker (e.g., the mean abundance level of the marker in individuals who do not have lung cancer). Thus, in this particular example, an abundance level that is between 2 standard deviations above and 2 standard deviations below the mean abundance level of the marker in individuals who do not have lung cancer may be considered within a normal abundance level range. These are merely exemplary cut-offs which can be used to label an abundance level of a marker as "high"/"increased" or "low"/"decreased".

In alternative aspects of the disclosure, the cut-offs for a "high"/"increased" or "low"/"decreased" abundance can be an abundance level that is greater that one standard deviation above or below the normal abundance level, or greater that three standard deviations above or below the normal abundance level, or any other desired standard deviation. In further alternative aspects of the disclosure, the cut-offs for a "high"/"increased" or "low"/"decreased" abundance can be based directly on the expression ratio or fold difference, for example, a 2-fold increase/decrease, 3-fold increase/decrease, or 4-fold increase/decrease, or any other desired degree of increase/decrease. Further, the normal abundance level can be based on, for example, either the mean or median abundance level (e.g., of a given control sample set). Other exemplary methods for developing cut-offs for "high"/"increased" or "low"/"decreased" abundance levels include determining a normal abundance level range (such as by testing a panel of markers in a control sample set of normal lung tissue samples), and classifying any test samples above or below this normal range (or above/below a desired threshold relative to this normal range, such as outside a particular percentage of samples within this normal range such as above or below 95% of samples within the normal range) as "high"/"increased" or "low"/"decreased", respectively.

A wide variety of further cut-offs for classifying the abundance level of a marker as "high"/"increased" or "low"/"decreased", and methods for formulating these cut-offs, are known in the art and/or can be implemented by one of ordinary skill in the art. For a given marker or panel of markers, various cut-offs can be applied, such as cut-offs that maximize sensitivity while maintaining a desired specificity, for example, or that maximize specificity while maintaining a desired sensitivity. For example, the classification of a sample as a tumor sample or normal sample can be accomplished using a variety of methods that may involve using a set of training data to produce a model that can then be used to classify a test sample (such as to diagnose lung cancer, for example). Tumor/normal cut-offs can be selected by manual inspection of multiple markers from the training data set, and these cut-offs can be applied to classifying test samples (such as to characterize patient samples with respect to lung cancer). Exemplary methods include, but are not limited to, split-point analysis (e.g.. Mor et al., "Serum protein markers for early detection of ovarian cancer", Proc Natl Acad Sci USA. 2005 May 24; 102(21):7677-82), logistic regression analysis (e.g., Planque et al., "A multiparametric serum kallikrein panel for diagnosis of non-small cell lung carcinoma", Clin Cancer Res. 2008 Mar 1;14(5):1355-62), Naïve Bayes, multivariate analysis, decision tree modeling (e.g., Patz et al., "Panel of serum biomarkers for the diagnosis of lung cancer", J Clin Oncol (2007), 25, 5578-5583), and other classification methods (see, for example, Dudoitet al., "Classification in Microarray Experiments", Statistical Analysis of Gene Expression Microarray Data, 2003, Chapman & Hall/CRC: 93-158).

The terms "sensitivity" and "specificity" are used herein with respect to the ability of one or more markers to correctly classify a sample as a tumor sample or a non-tumor sample (a non-tumor sample may be interchangeably referred to as a "normal", "control", or "healthy" sample), respectively. "Sensitivity" indicates the performance of the marker(s) with respect to correctly classifying tumor samples. "Specificity" indicates the performance of the marker(s) with respect to correctly classifying non-tumor samples. For example, 98% specificity and 85% sensitivity for a panel of markers used to test a set of control and tumor samples indicates that 98% of the control samples were correctly classified as control samples by the panel, and 85% of the tumor sample were correctly classified as tumor samples by the panel.

Area under the curve (AUC) refers to the area under the curve of a receiver operating characteristic (ROC) curve, which are well known in the art (see, e.g.. Planque et al., "A multiparametric serum kallikrein panel for diagnosis of non-small cell lung carcinoma". Clin Cancer Res. 2008 Mar 1;14(5):1355-62). AUC measures are useful for comparing the accuracy of a classification algorithm across the complete data range. Classification algorithms with a greater AUC have a greater capacity to classify unknowns correctly between two groups of interest (e.g., lung cancer samples and normal samples). ROC curves are useful for plotting the performance of a particular feature (e.g., an LCM and/or a supplemental biomedical parameter) in distinguishing between two populations (e.g., cases having lung cancer and controls without lung cancer). Typically, the feature data across the entire population (e.g., the cases and controls) are sorted in ascending order based on the value of a single feature. Then, for each value for that feature, the true positive and false positive rates for the data are calculated. The true positive rate is determined by counting the number of cases above the value for that feature and then dividing by the total number of cases. The false positive rate is determined by counting the number of controls above the value for that feature and then dividing by the total number of controls. Although this definition refers to scenarios in which a feature is elevated in cases compared to controls, this definition also applies to scenarios in which a feature is lower in cases compared to the controls (in such a scenario, samples below the value for that feature would be counted). ROC curves can be generated for a single feature as well as for other single outputs, for example, a combination of two or more features can be mathematically combined (e.g., added, subtracted, multiplied, etc.) to provide a single sum value, and this single sum value can be plotted in a ROC curve. Additionally, any combination of multiple features, in which the combination derives a single output value, can be plotted in a ROC curve. These combinations of features may comprise a test. The ROC curve is the plot of the true positive rate (sensitivity) of a test against the false positive rate (specificity) of the test.

The present disclosure discusses in greater detail different aspects thereof related to antibodies, proteins, carbohydrate antigens, immunogenic peptides (e.g., peptides which induce a T-cell response), or other biomolecules, as well as small molecules, nucleic acid agents (e.g., RNAi and antisense nucleic acid agents), and other compositions that modulate the markers (e.g., agonists and antagonists), such as by binding to or otherwise interacting with or affecting the markers. These compositions can be used for assessing, treating, and preventing diseases, especially cancer, and particularly lung cancer, as well as other uses. Moreover, disclosed herein are methods for assessing, treating, and preventing diseases such as lung cancer, particularly by using these compositions. Further provided are methods of screening for agents that modulate LCM. such as by affecting the function, activity, and/or expression level of LCM, and agents identified by these screening methods.

Disclosed herein are also methods of modulating cell function, especially lung cell function. In particular, disclosed herein are methods of modulating cell proliferation and/or apoptosis. For example, for cancer/tumor cells, disclosed herein are methods of inhibiting cell proliferation and/or stimulating apoptosis. Such methods can be applied to the treatment of diseases, especially cancer, and particularly lung cancer. In certain exemplary aspects, disclosed herein are methods of treating lung cancer by targeting LCM to thereby inhibit proliferation of lung cancer cells and/or stimulate apoptosis of lung cancer cells.

Disclosed herein are also methods of determing or predicting effectiveness or response to a particular treatment, and methods of selecting a treatment for an individual, particularly a lung cancer treatment. For example, markers that are differentially expressed by cells (e.g., lung cancer cells) that are more or less responsive (sensitive) or resistant to a particular treatment, such as a cancer treatment, are useful for determining or predicting effectiveness or response to the treatment or for selecting a treatment for an individual.

Further, disclosed herein are also methods of selecting individuals for a clinical trial of a therapeutic agent, particularly a clinical trial for lung cancer or other cancer. For example, the markers can be used to identify individuals for inclusion in a clinical trial who are more likely to respond to a particular therapeutic agent. Alternatively, the markers can be used to exclude individuals from a clinical trial who are less likely to respond to a particular therapeutic agent or who are more likely to experience toxic or other undesirable side effects from a particular therapeutic agent. Furthermore, such individuals who are determined to be less likely to respond to a particular therapeutic agent can be selected for inclusion in a clinical trial of a different therapeutic agent that may potentially benefit them.

In certain exemplary aspects disclosed herein, the various individual LCM and LCM panels described herein are provided as compositions. For example, in certain aspects of the disclosure, each of the members of an LCM panel, and/or reagents for detecting each of these members, are provided as individual compositions, such as in the form of reagents for detecting each member of an LCM panel by ELISA assays (which may be referred to herein as "ELISA reagents"). Furthermore, in certain aspects, compositions that comprise multiple members of a panel or an entire panel (and/or reagents for detecting each of these multiple members), are provided, such as in the form of kits that contain reagents (such as ELISA reagents) for detecting multiple members of a panel or an entire panel. Other compositions include arrays or other platforms that have multiple LCM, or multiple reagents (e.g., antibodies) for detecting multiple LCM, coupled to a substrate. In various compositions disclosed herein, the LCM. or reagents for detecting LCM (e.g., antibodies), are labeled with a detectable moiety (such as a fluorescent label).

### Exemplary LCM Combinations/Panels

For example, using a panel of sera from 12 lung cancer patients and 12 healthy control individuals, a group of 8 markers made up of TFPI, SCC, CEA, CA242, MN/CAIX, OPN, Cyfra 21-1, and MIF (Figure 2) detected all the cancer samples except a bronchioalveolar cancer sample (which is biologically distinct from other samples in the panel), and only a few of these markers were detected at levels above the threshold in the healthy control samples. When a simple algorithm was applied (i.e., markers greater than or equal to two standard deviations were scored "positive", using the criterion stated above), this group of eight markers had a sensitivity of 92% and specificity of 100% among the 12 sera from lung cancer patients and 12 sera from healthy controls (no false positives, 11 true positives, 1 false negative, and 12 true negatives) (Figure 3).

An alternate panel was configured that was made up of following markers: SLPI, TFPI, OPN, MIF, TIMP1, and MMP2 (Figure 4). Any or all of these markers can also be used in any combination with any or all of the hollowing markers: CA242, SCC, CEA, NSE, CA72-4, CA19-9, Cyfra 21-1, and MN/CALX (Figure 4).

Further, a six-marker panel made-up of Cyfra 21-1, TIMP-1, MIF, TFPI, CEA, and OPN was also configured (Figure 5). This six-marker panel, when tested on a larger group of 44 lung tumor sera and 44 normal sera, resulted in 75% sensitivity at 95% specificity.

Further, an 11 -marker panel made-up of Cyfra, MIF, TIMP1, TFPI, CEA, OPN, SCC, SLPI, HNP-1, GRP, and CA242 was also configured (Figure 6). This 11-marker panel, when tested on a group of 39 lung tumor sera and 39 normal sera, resulted in 98% specificity for controls (38/39 controls) and 85% sensitivity for tumor sera (33/39 tumors) (Figure 6).

Table 3 shows further examples of various 11-marker panels. Specifically, Table 3 provides 35 different panels of 11 markers (each row of 11 markers represents a panel) that have at least 98% specificity and 82% sensitivity for detecting lung cancer. Seven markers (SLPI, TIMP1, TFPI, SCC, OPN, CEA and CA242) appear in all 35 of these panels, GRP appears in 33 of the 35 panels, MIF appears in 29 of the 35 panels, and NSE and HNP-1 each appear in 15 of the 35 panels. AMBP, Cyfra, MMP2, Ca72-4, Ca19-9, and CAIX each appear in 7-9 of the panels, as indicated in Table 3.

Further, a 9-marker panel made-up of Cyfra, SLPI, TIMP1, SCC, TFPI, CEACAM5, MMP2, OPN, and MDK was also configured (e.g., Figure 17 and Table 5). This 9-marker panel demonstrated 98% specificity (49/50 controls) and 96% sensitivity (48/50 tumors) (Figure 17). Additionally, a 6-marker subset of this 9-marker panel was also configured that was made-up of Cyfra, SLPI, TIMP1, TFPI, CEACAM5, and MDK (Table 5).

Other markers, which are also referred to herein as LCM and which may be used either alone or in combination with any of the other LCM described herein in any combination, include a group of antigens to which "self-made" or "autoantibodies' are often found in the circulation of patients with various diseases, particularly cancer (Table 2 and Figures 11-14). Examples of these autoantibody markers include the following: KLKB1, CFL1, LGAGS1, EEF1G, RTN4, ALDOA, HSPCA, PABPC4, NAGK, CFHL1, CSF1R, and RANBP2 (Figure 12), and other autoantibody markers as shown in Table 2. Detection of autoantibody LCM such as these may complement other LCM and enhance the performance of LCM panels, particularly for assessing lung cancer.

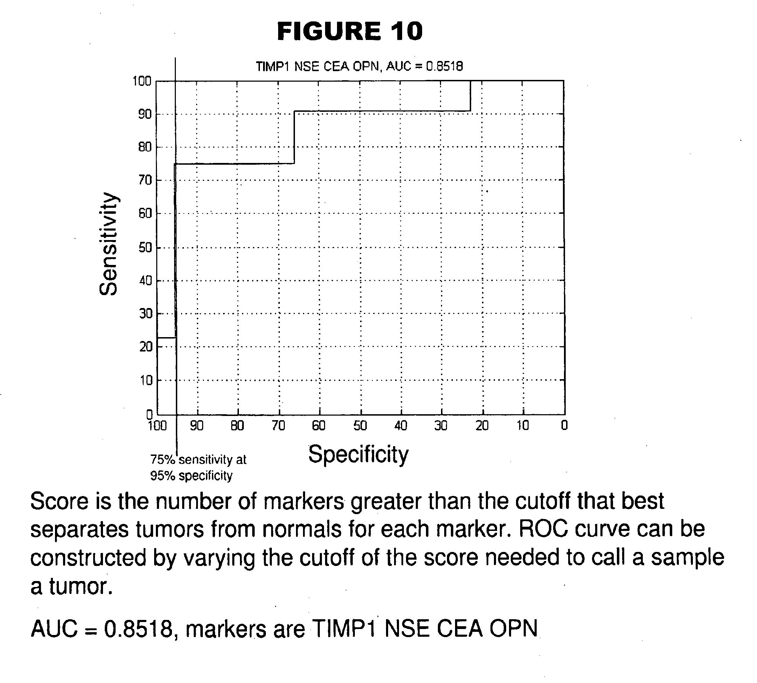

The following are exemplary panels of LCM. Various exemplary aspects disclosed herein are , for example, compositions based on these panels and methods of using these panels, particularly for uses related to lung cancer such as diagnosis of lung cancer (e.g., differential levels, such as elevated or low levels as compared to control/normal levels, of a plurality of markers in a panel, or all markers in panel, can indicate the presence of lung cancer). These exemplary panels may consist of, consist essentially of, or comprise the following combinations of markers:
1) Cyfra, SLPI, TIMP1, SCC, TFPI, CEACAM5. MMP2, OPN, and MDK (which may be referred to herein as the "9-marker panel" and is shown in Figure 17 and Table 5).
2) Cyfra, SLPI, TIMP1, TFPI, CEACAM5, and MDK (which may be referred to herein as the "6-marker subset of the 9-marker panel" and is shown in Table 5).
3) Cyfra, SLPI, TIMP1. SCC, TFPI, CEACAM5, MMP2, OPN, and MDK (the "9-marker panel"), which may optionally be in combination with one or more other markers (which may be added to this 9-marker panel and/or replace one or more members of this 9-marker panel), wherein these other markers may optionally be selected from the group consisting of the markers shown in Tables 1-2 (SEQ ID NOS:1-65 and the carbohydrate antigens CA 242, CA 19-9, and CA 72-4), Table 4, Figure 1, and Figure 12, particularly those markers that are shown in Table 4.
4) Cyfra, SLPI, TIMP1, TFPI, CEACAM5, and MDK (the "6-maker subset of the 9-marker panel"), which may optionally be in combination with one or more other markers (which may be added to this 6-marker subset of the 9-marker panel and/or replace one or more members of this 6-marker subset of the 9-marker panel), wherein these other markers may optionally be selected from the group consisting of the markers shown in Tables 1-2 (SEQ ID NOS:1-65 and the carbohydrate antigens CA 242, CA 19-9, and CA 72-4), Table 4, Figure 1, and Figure 12, particularly those markers that are shown in Table 4.
5) Any of the panels (which may include single markers) provided in Table 5 (which provides the 9-marker panel and all subcombinations thereof; each row of Table 5 represents a different panel), which may optionally be in combination with one or more other markers (which may be added to any panel in Table 5 and/or replace one or more members of any panel in Table 5), wherein these other markers may optionally be selected from the group consisting of the markers shown in Tables 1-2 (SEQ ID NOS:1-65 and the carbohydrate antigens CA 242, CA 19-9, and CA 72-4), Table 4. Figure 1, and Figure 12, particularly those markers that are shown in Table 4.
6) Any of the panels provided in Table 5 or Table 6 (particularly the 9-marker panel of Cyfra, SLPI, TIMP1, SCC, TFPI, CEACAM5, MMP2, OPN, and MDK, as well as subsets thereof, and panels comprising this 9-marker panel or subsets thereof that further include one or more additional markers such as those panels set forth in Table 6), particularly for use in methods for distinguishing lung tumor samples versus normal (i.e., control/healthy) samples. These panels are particularly useful for determining whether an individual has lung cancer (i.e., detecting lung cancer), for example.
7) Any of the panels provided in Table 7 (particularly the 9-marker panel of Cyfra, SLPI, TIMP1, SCC, TFPI, CEACAM5, MMP2, OPN, and MDK, as well as subsets thereof), particularly for use in methods for distinguishing adenocarcinoma versus squamous cell carcinoma. These panels are particularly useful for determining whether an individual's lung cancer is adenocarcinoma or squamous cell carcinoma, for example.
8) Any of the panels provided in Table 8 (particularly the 9-marker panel of Cyfra, SLPI, TIMP1, SCC, TFPI, CEACAM5, MMP2, OPN, and MDK, as well as subsets thereof), particularly for use in methods for distinguishing between any stages of lung cancer (e.g., any of stages I, II, III, and IV), particularly between early stage (stage I or II) and late stage (stage III or IV) lung cancer, and especially between stage I and stage III lung cancer. These panels are particularly useful for determining the stage of an individual's lung cancer, for example.
9) Any of the panels provided in Table 9, particularly for use in methods for distinguishing SCLC versus other types of lung cancer (e.g., NSCLC). These panels are particularly useful for determining whether an individual's lung cancer is SCLC or NSCLC, for example.
10) Any of the panels provided in Table 10 (particularly the 9-marker panel of Cyfra, SLPI, TIMP1, SCC, TFPI, CEACAM5, MMP2, OPN, and MDK, as well as subsets thereof), particularly for use in methods for distinguishing malignant lung tumors versus benign lung lesions. These panels are particularly useful for determining whether a lung lesion identified in an individual (such as by CT screening) is a malignant tumor or a benign lesion, for example.
11) Any of the panels provided in Table 11, particularly for use in methods for distinguishing SCLC versus normal (i.e., control/healthy) samples. These panels are particularly useful for determining whether an individual has SCLC, for example.
12) Any of the panels provided in Table 12, particularly for use in methods for distinguishing lung cancer (including both SCLC and NSCLC) versus normal (i.e., control/healthy) samples. These panels are particularly useful for determining whether an individual has lung cancer such as SCLC or NSCLC, for example.
13) Panels that include any or all of the 11 markers provided in Figure 19 (and subsets thereof, as well as panels comprising these 11 markers or subsets thereof that further include one or more additional markers), particularly for use in methods of monitoring for lung tumor regression and/or recurrence. These panels are particularly useful for monitoring for lung tumor regression and/or recurrence, for example.
14)Cyfra 21-1, MIF, TIMP1, TFPI, CEA, OPN, SCC, SLPI, HNP-1, GRP, and CA242 (which may be referred to herein as the "1-marker panel" and is shown in Figure 6).
15)TFPI, CEA, MIF, TIMP1, OPN, and Cyfra 21-1 (which may be referred to herein as the "6-marker panel" and is shown in Figure 5).
16)TFPI, SCC, CEA, CA242, MN/CAIX, OPN, Cyfra 21-1 and MIF (which may be referred to herein as the "8-marker panel" and is shown in Figures 2-3).
17)TFPI, SLPI, OPN, MIF, TIMP1, and MMP2, any or all of which can optionally be used in combination with any or all of the following additional markers: CA242, SCC, CEA, NSE, CA724, CA199, Cyfra 21-1, and MN/CAIX (see Figure 4).
18)TFPI, TIMP1. CEA, and OPN (see Figure 7).
19) TFPI, CEA, and OPN (see Figure 7).
20) CEA and OPN (see Figure 7).
21) TFPI, MIF, TIMP1, CEA, and OPN (see Figure 9).
22) TIMP1, NSE, CEA, and OPN (see Figure 10).
23) TFPI, CEA, TIMP-1, NSE, SLPI, SCC, Cyfra 21-1, and MIF, any or all of which can optionally be in combination with any or all of the following autoantibody markers: p53, KLKB1, LGALS1, CFLN, EEF1G, HSP90α, RTN4, ALDOA, GLG1, PTK7, EFEMP1, CD98, CHGB, B7H3, and CEACAM1 (see Figure 11).
24) TFPI, SLPI, TFPI2, CEA, and TIMP1.
25)TFPI, SLPI, and TIMP1.
26) KLKB1 and cofilin (CFLN) (see Figure 13).
27) KLKB1, cofilin (CFLN), and CA12 (see Figure 14).
28) TFPI, either alone or in combination with one or more other markers, which may optionally be selected from the group consisting of the markers shown in Tables 1-2 (SEQ ID NOS: 1-65 and the carbohydrate antigens CA 242, CA 19-9, and CA 72-4), Table 4, Figure 1, and Figure 12.
29) One or more markers selected from the group consisting of defensin (DEFA1, HNP-1), ICAM3, CTGF, LCN2, biglycan, and HGF, either alone or in combination with one or more other markers, which may optionally be selected from the group consisting of the markers shown in Tables 1-2 (SEQ ID NOS: 1-65 and the carbohydrate antigens CA 242, CA 19-9, and CA 72-4), Table 4, Figure 1, and Figure 12.
30) Two or more markers selected from the group consisting of TFPI, defensin, ICAM3, CTGF, LCN2, biglycan, and HGF, either alone or in combination with one or more other markers, which may optionally be selected from the group consisting of the markers shown in Tables 1-2 (SEQ ID NOS: 1-65 and the carbohydrate antigens CA 242, CA 19-9, and CA 72-4), Table 4, Figure 1, and Figure 12.
31) One or more markers shown in Table 1 (SEQ ID NOS:1-38 and 56-65 and the carbohydrate antigens CA 242, CA 19-9, and CA 72-4) and/or Figure 1, in combination with one or more autoantibody markers shown in Table 2 (SEQ ID NOS:39-55) and/or Figure 12.
32) Any of the 11-marker panels provided in Table 3 (each row of Table 3 represents a different 11-marker panel).
33) SLPI, TIMP1, TFPI, SCC, OPN, CEA, and CA242, which may optionally be in combination with GRP and/or MIF (see Table 3).
34) SLPI, TIMP1, TFPI, SCC, OPN, CEA, and CA242, which may optionally be in combination with GRP and/or MIF, and which may optionally further be in combination with HNP-1 and/or NSE (see Table 3).
35) SLPI, TIMP1, TFPI, SCC, OPN, CEA, and CA242, which may optionally be in combination with any or all of GRP, MIF, HNP-1, and NSE, and which may optionally further be in combination with any or all of CAIX, Ca19-9, Ca72-4, MMP2, Cyfra 21-1, and AMBP (see Table 3).
36)SLPI, TIMP-1, TFPI, SCC, OPN, CEA, and CA242, which may optionally be in combination with any or all of GRP, MIF, HNP-1, NSE, and Cyfra 21-1.
37) One or more markers selected from the group consisting of visfatin, sortilin, and midkine, either alone or in combination with one or more other markers, which may optionally be selected from the group consisting of the markers shown in Tables 1-2 (SEQ ID NOS: 1-65 and the carbohydrate antigens CA 242, CA 19-9, and CA 72-4), Table 4, Figure 1, and Figure 12.

### Exemplary Uses of LCM

Certain aspects of the present disclosure relate to methods of detecting the presence of lung cancer in an individual by measuring the amounts of circulating LCM, such as in serum, by immunological methods or other methods. These LCM are, for example, differentially expressed (over- or under-expressed) in individuals with lung cancer as compared to individuals without lung cancer (individuals without lung cancer are interchangeably referred to herein as "normal", "control", or "healthy" individuals). Detection of variation from a "normal" expression level, or differential expression, can be used for, for example, early diagnosis of lung cancer, distinguishing between a benign and malignant lung lesion (such as a lesion observed on a CT scan), monitoring lung cancer recurrence, or other clinical indications.

LCM may be used in a variety of clinical indications for lung cancer, including, but not limited to, detection of lung cancer (such as in a high-risk individual or population), characterizing lung cancer (e.g., determining lung cancer type, sub-type, or stage) such as distinguishing between non-small cell lung cancer (NSCLC) and small cell lung cancer (SCLC) and/or between adenocarcinoma and squamous cell carcinoma (or otherwise facilitating histopathology), determining whether a lung lesion is a benign lesion or a malignant lung tumor, lung cancer prognosis, monitoring lung cancer progression or remission, monitoring for lung cancer recurrence, monitoring metastasis, treatment selection, monitoring response to a therapeutic agent or other treatment, stratification of patients for computed tomography (CT) screening (e.g., identifying those patients at greater risk of lung cancer and thereby most likely to benefit from spiral-CT screening, thus increasing the positive predictive value of CT), combining LCM testing with supplemental biomedical parameters such as smoking history, etc., or with nodule size, morphology, etc. (such as to provide an assay with increased diagnostic performance compared to CT testing or LCM testing alone), facilitating the diagnosis of a pulmomary nodule as malignant or benign, facilitating clinical decision making once a lung cancer lesion is observed on CT (e.g., ordering repeat CT scans if the lesion is deemed to be low risk, such as if an LCM-based test is negative, with or without categorization of lesion size, or considering biopsy if the lesion is deemed medium to high risk, such as if an LCM-based test is positive, with or without categorization of lesion size), and facilitating decisions regarding clinical follow-up (e.g., whether to implement repeat CT scans, fine needle biopsy, or thoracotomy after observing a non-calcified lesion on CT). LCM testing may improve positive predictive value (PPV) over CT screening alone. In addition to their utilities in conjunction with CT screening, LCM can also be used in conjunction with any other imaging modalities used for lung cancer, such as chest X-ray. Furthermore, LCM may also be useful for enabling certain of these uses to be achieved before indications of lung cancer are detected by imaging modalities or other clinical correlates, or before symptoms appear.

As examples of how LCM may be useful for diagnosing lung cancer, a high or low abundance level (i.e., a "differential" abundance level) of one or more LCM in an individual who is not known to have lung cancer may indicate that the individual has lung cancer, thereby enabling early detection of lung cancer at an early stage of the disease when treatment is most effective, perhaps before the lung cancer is detected by other means or before symptoms appear. An increase in the abundance of one or more LCM during the course of lung cancer may be indicative of lung cancer progression, e.g., a lung tumor is growing and/or metastasizing (and thus a poor prognosis), whereas a decrease in the abundance of one or more LCM may be indicative of lung cancer remission, e.g., a lung tumor is shrinking (and thus a good prognosis). Similarly, an increase in the abundance of one or more LCM during the course of lung cancer treatment may indicate that the lung cancer is progressing and therefore indicate that the treatment is ineffective, whereas a decrease in the abundance of one or more LCM during the course of lung cancer treatment may be indicative of lung cancer remission and therefore indicate that the treatment is working successfully. Additionally, an increase or decrease in the abundance of one or more LCM after an individual has apparently been cured of lung cancer may be indicative of lung cancer recurrence. In a situation such as this, for example, the individual can be re-started on therapy (or the therapeutic regimen modified such as to increase dosage amount and/or frequency, if the patient has maintained therapy) at an earlier stage than if the recurrence of lung cancer was not detected until later. Furthermore, a differential abundance level of one or more LCM in an individual may be predictive of the individual's response to a particular therapeutic agent. In monitoring for lung cancer recurrence or progression, changes in LCM levels may indicate the need for repeat imaging (e.g., repeat CT scanning), such as to determine lung cancer activity, or the need for changes in treatment.

Detection of LCM may be particularly useful following, or in conjunction with, lung cancer treatment, such as to evaluate the success of the treatment or to monitor lung cancer remission, recurrence, and/or progression (including metastasis) following treatment. Lung cancer treatment may include, for example, administration of a therapeutic agent to a patient, surgery (e.g., surgical resection of at least a portion of a lung tumor), radiation therapy, or any other type of lung cancer treatment used in the art, and any combination of these treatments. For example, LCM may be detected at least once after treatment or may be detected multiple times after treatment (such as at periodic intervals), or may be detected both before and after treatment. A differential abundance level of LCM, such as an increase or decrease in the abundance level of LCM after treatment compared with the abundance level of LCM, before treatment, or an increase or decrease in the abundance level of LCM at a later time point after treatment, compared with the abundance level of LCM at an earlier time point after treatment, or a differential abundance level of LCM at a single time point after treatment compared with normal levels of LCM, may be indicative of lung cancer progression, remission, or recurrence.

As a specific example, ELISA analysis of LCM levels in pre-surgery and post-surgery (e.g., 2-4 weeks after surgery) serum samples can be carried out. An increase in the level of LCM in the post-surgery sample compared with the pre-surgery sample can indicate progression of lung cancer (e.g., unsuccessful surgery), whereas a decrease in the level of LCM in the post-surgery sample compared with the pre-surgery sample can indicate regression of lung cancer (e.g., the surgery successfully removed the lung tumor). Similar analyses of LCM levels can be carried out before and after other forms of treatment, such as before and after radiation therapy or administration of a therapeutic agent or cancer vaccine.

In addition to the utilities of testing LCM levels as stand-alone screening tests, testing of LCM levels can also be done in conjunction with CT screening. For example, LCM may facilitate the medical and economic justification for implementing CT screening, such as to screen large asymptomatic populations at risk for lung cancer (e.g., smokers). For example, a "pre-CT" test of LCM levels could be used to stratify high-risk individuals for CT screening, such as to identify those who are at highest risk for lung cancer based on their LCM levels and who should be prioritized for CT screening. If a CT test is implemented, LCM levels (e.g., as determined by immunoassay of serum samples) of one or more LCM can be measured and the scores added to scores for supplentental biomedical parameters (e.g., tumor parameters determined by CT testing) to create a combined score, such as to enhance positive predictive value (PPV) over CT or LCM testing alone. A "post-CT" immunoassay panel for determining LCM levels can be used to determine the likelihood that a pulmonary lesion observed by CT (or other imaging modality) is malignant or benign.

Detection of LCM may be useful for post-CT testing. For example, LCM testing may eliminate a significant number of false positive tests over CT alone. Further, LCM testing may facilitate treatment of patients. As an example, if a lung tumor is less than 5 mm in size, results of LCM testing may move patients from "watch and wait" to biopsy at an earlier time, if a lung tumor is 5-9 mm, LCM testing may eliminate biopsy or thoracotomy on false positive scans, and if a lung tumor is larger than 10 mm, LCM testing may eliminate surgery for sub-population of these patients with benign lesions. Eliminating the need for biopsy in some patients based on LCM testing would be beneficial because there is significant morbidity associated with nodule biopsy and difficulty in obtaining nodule tissue depending on location of nodule. Similarly, eliminating the need for surgery in some patients, such as those whose lesions are actually benign, would avoid unnecessary risks and costs associated with surgery.

In addition to testing LCM levels in conjunction with CT screening (e.g., assessing LCM levels in conjunction with size or other characteristics of a lung nodule observed on a CT scan), information regarding LCM can also be evaluated in conjunction with other types of data, particularly data that indicates an individual's risk for lung cancer (e.g., patient clinical history, symptoms, family history of cancer, risk factors such as whether or not the individual is a smoker, and/or status of other biomarkers, etc.). These various data can be assessed by automated methods, such as a computer program/software, which can be embodied in a computer or other apparatus/device.

The various methods described herein, such as correlating the level of LCM in an individual with an altered (*e.g.*, increased or decreased) risk (or no altered risk) for lung cancer, can be carried out by automated methods such as by using a computer (or other apparatus/devices such as biomedical devices, laboratory instrumentation, or other apparatus/devices having a computer processor) programmed to carry out any of the methods described herein. For example, computer software (which may be interchangeably referred to herein as a computer program) can perform the step of correlating the level of LCM in an individual with an altered (e.g., increased or decreased) risk (or no altered risk) of lung cancer for the individual. Accordingly, disclosed herein is a computer (or other apparatus/device) programmed to carry out any of the methods described herein.

LCM may also be used in imaging tests. For example, an imaging agent can be coupled to an LCM, which can be used to aid in lung cancer diagnosis, to monitor disease progression/remission or metastasis, to monitor for disease recurrence, or to monitor response to therapy, among other uses.

LCM can be detected using a variety of platforms. For example, LCM may be detected using singleplex ELISAs, ultrasensitive detection technologies, multiplex formats, and/or automated immunoanalyzers.

In addition to detecting LCM in serum, LCM may also be detected in, for example, plasma and bronchial lavage.

LCM may be used for pharmacoproteomic or pharmacogenomic applications; for example, detection of LCM may be used for treatment selection or stratification. Differential expression of LCM in, for example, tumor cells that are resistant to a treatment (e.g., a particular therapeutic agent) and tumor cells that are sensitive to a treatment can be used to predict resistance or sensitivity of an individual's lung cancer to the treatment. As specific example, CTGF is secreted at elevated levels by cell lines that are resistant to the chemotherapeutic agent Topotecan. In contrast, TIMP1, TFPI, and TIMP2 are secreted at elevated levels by cell lines that are sensitive to the chemotherapeutic agent Iressa. LCM may also be used as treatment response markers for a particular therapeutic agent. For example, certain LCM may be used as surrogate markers of cisplatin or Iressa treatment response.

Thus, the LCM profile of an individual having lung cancer can be used to determine which treatment(s) are best suited for that particular individual. For example, treatments to which an individual's lung cancer is predicted to be sensitive can be selected for the individual rather than treatments to which the individual's lung cancer is predicted to be resistant. As a further example, LCM levels can be used by a medical practitioner to distinguish between types of lung cancer (e.g., non-small cell lung cancer (NSCLC) versus small cell lung cancer (SCLC), adenocarcinoma versus squamous cell carcinoma, different stages of lung cancer, or other lung cancer characteristics) in order to adjust therapy options (e.g., to select a particular therapeutic agent or a particular form of treatment, such as chemotherapy, surgery, or radiation therapy, that is best suited for that particular subtype of lung cancer).

Tables 5-6 and 11-12 provide panels that are particularly well-suited for diagnosing/detecting lung cancer, among other lung cancer-related uses. For example, Tables 5 and 6 provides LCM panels that are particularly well-suited for distinguishing lung tumor samples versus normal (i.e., control/healthy) samples, Table 11 provides LCM panels that are particularly well-suited for distinguishing SCLC versus normal samples, Table 12 provides LCM panels that are particularly well-suited for distinguishing lung cancer (including both SCLC and NSCLC) versus normal samples.

Any of the LCM and the various exemplary LCM panels disclosed herein (such as any of the panels provided in Tables 5-6, such as the 9-marker panel or the 6-marker subset of this 9-marker panel, as well as any LCM provided in Tables 1-2 (SEQ ID NOS:1-65 and the carbohydrate antigens CA 242, CA 19-9, and CA 72-4), Table 4, Figure 1, and Figure 12, and any panels that include one or more of these LCM. particularly panels that include one or more markers provided in Table 4) may be used for any of the various lung cancer-related uses disclosed herein. However, certain LCM panels are particularly well-suited for certain specific lung cancer-related uses ("indications"); these specific uses may be referred to herein as determining or assessing various "characteristics" of lung cancer. Examples of such LCM panels that are particularly well-suited for certain specific lung cancer-related uses are provided in Tables 7-10 and Figure 19. For example, Table 7 provides LCM panels that are particularly well-suited for distinguishing adenocarcinoma versus squamous cell carcinoma types of lung cancer, Table 8 provides LCM panels that are particularly well-suited for distinguishing between different stages of lung cancer (such as between early-stage and late-stage lung cancer such as stage I versus stage III lung cancer, or between any other of stages I, II, III, and IV) such as to determine the stage of lung cancer in a patient, Table 9 provides LCM panels that are particularly well-suited for distinguishing SCLC versus other types of lung cancer (e.g., NSCLC), Table 10 provides LCM panels that are particularly well-suited for distinguishing malignant lung tumors versus benign lung lesions, and Figure 19 provides LCM that are particularly well-suited for monitoring for lung tumor regression and/or recurrence.

Tables 5-12 provide a variety of exemplary LCM panels, together with performance characterisitics for each panel (AUC, sensitivity, and specificity). In certain aspects of the disclosure, LCM panels are provided that have at least 70% sensitivity at 95% specificity, or at least 70% specificity at 95% sensitivity, In certain aspects disclosed herein, LCM panels are provided that have at least 85% sensitivity at 95% specificity, or at least 85% specificity at 95% sensitivity, In further aspects of the disclosure, LCM panels are provided that have at least 90% sensitivity or at least 90% specificity, or that have at least 95% sensitivity or at least 95% specificity. In yet further aspects of the disclosure, LCM panels are provided that have at least 70, 75, 80, 85, 90, 91, 92, 93, 94, 95, 96, 97, 98, or 99% (or any other percentage in-between) sensitivity and 70, 75, 80, 85, 90, 91, 92, 93, 94, 95, 96, 97, 98, or 99% (or any other percentage in-between) specificity. In yet further aspects of the disclosure, LCM panels are provided that have at least 0.7, 0.75, 0.8, 0.85, 0.9, 0.91, 0.92, 0.93, 0.94, 0.95, 0.96, 0.97, 0.98, or 0.99 (or any other value in-between) AUC values. Any of these panels are particularly useful for lung-cancer related uses such as those described herein (e.g., diagnosing lung cancer), such as in clinical practice. However, the desired performance for clinical use of an assay may vary depending on such factors as the particular use, point of implementation, or other factors.

### Distinguishing NSCLC and SCLC

The following panels of LCM are particularly useful for distinguishing (which may be interchangeably referred to as "resolving") non-small cell lung carcinoma (NSCLC) and small cell lung carcinoma (SCLC) from each other and/or from normal (i.e., control/healthy) samples. These panels may consist of, consist essentially of, or comprise the following combinations of markers:
1) Any of the panels provided in Table 9, particularly for distinguishing NSCLC versus SCLC.
2) Any of the panels provided in Table 11, particularly for distinguishing SCLC versus normal samples.
3) Any of the panels provided in Table 12, particularly for distinguishing SCLC and NSCLC versus normal samples.
4) OPN (either alone or in combination with one or more other markers). particularly for distinguishing NSCLC from SCLC and for distinguishing SCLC from NSCLC.
5) SCC, OPN, AMBP, and Ca72-4, particularly for distinguishing NSCLC from SCLC (levels of these LCM are higher in NSCLC as compared to SCLC).
6) ENO2, MMP2, Ca19-9, CAIX, and GRP, particularly for distinguishing SCLC from NSCLC (levels of these LCM are higher in SCLC as compared to NSCLC).
7) Cyfra, SLPI, TIMP1, TFPI, CEACAM5, MMP2, and CA242, particularly for distinguishing SCLC from normal samples (particularly by using split-point analysis).
8) SLPI, TIMP1, TFPI, CEACAM5, MMP2, OPN, and CA242, particularly for distinguishing SCLC from normal samples (particularly by using split-point analysis).
9) Cyfra, TIMP1, ENO2, TFP1, CEACAM5, MMP2, OPN, and DEFA1. particularly for distinguishing NSCLC and SCLC from normal samples (particularly by using split-point analysis).
10) Cyfra, MIF, TIMP1, SCC, TFPI, CEACAM5, OPN, and DEFA1, particularly for distinguishing NSCLC and SCLC from normal samples (particularly by using split-point analysis).
11) TIMP1, ENO2, TFP1, CEACAM5, MMP2, OPN, AMBP, and DEFA1, particularly for distinguishing NSCLC and SCLC from normal samples (panicularly by using split-point analysis).

### Supplemental Biomedical Parameters

The term "supplemental biomedical parameters" refers to one or more assessments of an individual, other than LCM, that are associated with lung cancer risk. "Supplemental biomedical parameters" include, but are not limited to, physical descriptors of a patient, physical descriptors of a pulmonary nodule observed by CT imaging, the height and/or weight of a patient, the gender of a patient, smoking history, occupational history, exposure to carcinogens, exposure to second-hand smoke, family history of lung cancer (or other cancer), the presence of pulmonary nodules, size of nodules, location of nodules, morphology of nodules (e.g., nodules may be observed by CT imaging), etc. Smoking history is usually quantified in terms of "pack years", which refers to the number of years a person has smoked multiplied by the average number of packs smoked per day. For example, a person who has smoked, on average, one pack of cigarettes per day for 35 years is referred to as having 35 pack years of smoking history. Supplemental biomedical parameters can be obtained from an individual using routine techniques known in the art, such as from the individual themselves by use of a routine patient questionnaire or health history questionnaire, etc., or from a medical practitioner, etc. Alternately, supplemental biomedical parameters can be obtained from routine imaging techniques including CT imaging (e.g., low-dose CT imaging) and X-ray.

Testing of LCM in combination with an assessment of supplemental biomedical parameters may, for example, improve sensitivity, specificity, and/or AUC for detecting lung cancer (or other lung cancer-related uses) as compared to LCM testing alone or assessing supplemental biomedical parameters alone (e.g., CT imaging alone).

Accordingly, any of the LCM, and panels of LCM, can be used in combination with supplemental biomedical parameters. Furthermore, supplemental biomedical parameters may serve to replace one or more markers of a panel, such as to enable the use of smaller panels (i.e., panels with fewer biomarkers) while retaining similar performance (e.g., sensitivity, specificity, and/or AUC for detecting lung cancer). Thus, supplemental biomedical parameters can be used in addition to a panel, or in addition to one or more markers of a panel, or as a substitute for one or more markers of a panel. As a specific example, one or more supplemental biomedical parameters can be used in addition to the 9-marker panel of Cyfra, SLPI, TIMP1, SCC, TFPI, CEACAM5, MMP2, OPN, and MDK or the 6-marker subset of this 9-marker panel (Cyfra, SLPI, TIMP1, TFPI, CEACAM5, and MDK). As another specific example, one or more supplemental biomedical parameters can replace one or more members of the 9-marker panel of Cyfra, SLPI, TIMP1, SCC, TFPI, CEACAM5, MMP2, OPN, and MDK or the 6-marker subset of this 9-marker panel (Cyfra, SLPI, TIMP1, TFPI, CEACAM5, and MDK). Furthermore, one or more supplemental biomedical parameters can be used in addition to any of the panels provided in Table 5 and/or can replace one or more members of any of the panels provided in Table 5. Moreover, one or more supplemental biomedical parameters can be used in addition to any of the markers or panels provided herein and/or can replace one or more members of any of the panels provided herein, including the markers provided in Tables 1-2 (SEQ ID NOS:1-65 and the carbohydrate antigens CA 242, CA 19-9, and CA 72-4), Table 4, Figures 1, 12, and 19, and the panels provided in Tables 3 and 6-12. Furthermore, supplemental biomedical parameters can be incorporated into algorithms and scoring systems/classifiers, together with biomarker assessments (e.g., biomarker levels), for assessing lung cancer (e.g., diagnosing lung cancer).

Examples of supplemental biomedical parameters include, but are not limited to, any of the following. Any or all of these supplemental biomedical parameters can be used, in any combination, with any of the LCM and LCM panels disclosed herein. For example, any of the LCM and LCM panels disclosed herein can be assessed alone (without considering supplemental biomedical parameters) or can be assessed in combination with CT results (for example), or can be assessed in combination with any other supplemental biomedical parameters, or can be assessed in combination with CT results plus any other supplemental biomedical parameters, or any other combination of supplemental biomedical parameters can be assessed in combination with any of the LCM and LCM panels disclosed herein. Any of these supplemental biomedical parameters can be assessed as part of an algorithm or scoring system/classifier, together with biomarker assessments (e.g., biomarker levels), such as for assessing lung cancer (e.g., diagnosing lung cancer).
1) age, gender, and/or ethnicity;
2) family history of lung cancer or other type of cancer;
3) smoking history (e.g., whether or not an individual previously and/or currently smokes);
4) smoking level (e.g., "pack year": number of cigarettes smoked per day multiplied by number of years of smoking at this rate);
5) size of lesion;
6) location of lesion;
7) lesion morphology (ground glass opacity (GGO), solid, non-solid);
8) edge characteristics of lesion (smooth, lobulated, sharp and smooth, spiculated, infiltrating);
9) any other parameters determined from computed tomography (CT) screening;
10) exposure to second-hand smoke: and
10) any known carcinogen exposure (including, but not limited to, exposure to any of asbestos, radon gas, chemicals, smoke from fires, and air pollution, which can include emissions from stationary or mobile sources such as industrial/factory or auto/marine/aircraft emissions).

Exemplary methods of combining LCM with supplemental biomedical parameters can comprise the steps of obtaining a value for at least one supplemental biomedical parameter (e.g., smoking history) from an individual, comparing the value of each of the supplemental biomedical parameter(s) to one or more predetermined cutoffs, assigning a score for each supplemental biomedical parameter based on said comparison, combining the assigned score for each supplemental biomedical parameter with the assigned score for each LCM to obtain a total score for said individual, comparing the total score with a predetermined total score cutoff, and classifying said individual as having or not having lung cancer (or the likelihood thereof) based on whether the individual's total score is above or below (or equal to) the predetermined total score cutoff. In certain embodiments, if the individual's total score is above (or equal to) the predetermined total score cutoff, then the individual is classified as having lung cancer.

Further exemplary methods can comprise the steps of:
a) obtaining a value for at least one supplemental biomedical parameter of an individual;
b) comparing the value of each supplemental biomedical parameter against one or more predetermined cutoffs and assigning a score for each supplemental biomedical parameter based on said comparison;
c) quantifying in a test sample obtained from the individual, the levels of one or more LCM or LCM panels (e.g., the 9-marker panel of Cyfra, SLPI, TIMP1, SCC, TFPI, CEACAM5, MMP2, OPN, and MDK, or the 6-marker subset of Cyfra, SLPI, TIMP1, TFPI, CEACAM5, and MDK);
d) comparing the amount of each LCM quantified to a predetermined cutoff and assigning a score for each LCM based on said comparison;
e) combining the assigned score for each supplemental biomedical parameter determined in step b with the assigned score for each LCM determined in step d to obtain a total score for said individual;
f) comparing the total score determined in step e with a predetermined total score cutoff; and
g) classifying the individual (or the test sample from the individual) as having or not having lung cancer (or the likelihood thereof) based on whether the individual's total score is above or below (or equal to) the predetermined total score cutoff (in certain embodiments, if the individual's total score is above (or equal to) the predetermined total score cutoff, then the individual is classified as having lung cancer).

In the above exemplary methods, the supplemental biomedical parameter obtained from the individual can be, for example, the individual's smoking history, age, carcinogen exposure, gender, nodule size, nodule morphology, and/or nodule location (nodule characteristics, such as size, morphology, and/or location, may be determined by CT imaging, as well as X-ray or other imaging methods). Preferably, the supplemental biomedical parameter is related to nodule mophology.

### Exemplary Scoring Systems and Cutoffs

A variety of methodologies can be used to classify a sample based on assaying one or more LCM disclosed herein. Classifying a sample can be based on a score derived from assessing one or more LCM disclosed herein, optionally in combination with one or more supplemental biomedical parameters (including, but not limited to, the supplemental biomedical parameters disclosed in the preceding section). A score or other classification system can be based on, for example, determining whether the level of one or more LCM is above or below a cutoff level (which may be referred to as a "cutoff value" or just "cutoff"), or is above or below a cutoff value by a certain amount (e.g., by a certain number of standard deviations such as two standard deviations), or the magnitude/extent of how high or low the level of one or more LCM is (which may optionally be in relation to a cutoff value). A wide variety of scoring systems and methodologies for establishing cutoff values are known in the art, and one of ordinary skill in the art would know how to implement a known scoring system or method of establishing cutoff values (or devise a new scoring system or method for establishing cutoff values) that is best suited for the intended use, such as assessing lung cancer based on one or more LCM or LCM panels (optionally in combination with one or more supplemental biomedical parameters). Accordingly, one of ordinary skill in the art could establish and adjust cutoff values to suit the intended use, and could incorporate these cutoff values into any desirable scoring system. For example, cutoff values can be adjusted based on whether increased sensitivity (for detecting tumor samples and avoiding false-negatives) or increased specificity (for avoiding false-positives) is considered more important. For example, cutoffs can be selected such as to achieve at least 70% sensitivity at 95% specificity, or at least 70% specificity at 95% sensitivity, or at least 85% sensitivity at 95% specificity, or at least 85% specificity at 95% sensitivity, or at least 90% or 95% sensitivity, or at least 90% or 95% specificity, or any other desired sensitivity and/or specificity (such as the sensitivity and specificity values described above). As another example, cutoffs can be set lower while requiring more markers in a panel to be above the cutoff levels in order to classify a sample as a tumor sample, or cutoffs can be set higher while requiring fewer markers in a panel to be above the cutoff levels in order to classify a sample as a tumor sample. When a cutoff value is set and applied to testing, it may be interchangeably referred to herein as a "predetermined" or "established" cutoff value. Furthermore, various analysis methods can be applied, including, but not limited to, split-point analysis (such as for setting discrete cutoffs), logistic regression analysis (such as for factoring in the magnitude/extent by which a marker level is elevated or low), Naïve Bayes, multivariate analysis, decision tree modeling, etc.

A representative example is shown in Figure 17 for the 9-marker panel of Cyfra. SLPI, TIMP1, SCC, TFP1, CEACAM5, OPN, and MDK. In Figure 17, exemplary cutoffs for each of these 9-markers are shown just below each marker symbol, as follows (levels/concentrations, in ng/ml, were determined by ELISA): Cyfra = 1.20 ng/ml, CEA = 5.00 ng/ml, SLPI = 52 ng/ml, OPN = 32 ng/ml, MDK = 0.15 ng/ml, TFPI = 150 ng/ml, TIMP1 = 385 ng/ml, MMP2 = 210 ng/ml, and SCC = 2.2 ng/ml. These cutoff values were established by examining the levels of these markers in both normal (control) samples and lung tumor samples in the 50x50 samples set and determining appropriate cutoff values that would best distinguish lung tumor versus normal samples (e.g., cutoff values were selected for which the levels of a majority of lung tumor samples are above and the levels of a majority of normal samples are below, so as to maximize sensitivity and specificity). These cutoffs were then applied to the same 50x50 sample set (i.e., the 50x50 sample set was used for both training and testing in this example). In this example, if the levels of two or more of the nine markers was greater than or equal to the established cutoff value for each marker, then the samples was classified as a lung tumor sample (thus, if the levels of none, or only one, of the nine markers was greater than or equal to the established cutoff value for each marker, then the sample was classified as a normal sample). Using this exemplary scoring system in this exemplary sample set, 48 out of 50 tumor samples were correctly classified, whereas the 42^{nd} and 43^{rd}-listed samples were mis-classified as not being tumor samples since the level of only one of the nine markers (rather than the minimum of two or more) in each of these two samples sets was greater than or equal to the cutoff level (96% sensitivity; right-side of Figure 17). Similarly, using this exemplary scoring system in this exemplary sample set, 49 out of 50 normal (control) samples were correctly classified, whereas the 2^{nd}-listed sample was mis-classified as being a tumor sample since the levels of three of the nine markers (which meets the minimum of two or more) in this samples set were greater than or equal to the cutoff levels (98% specificity; left-side of Figure 17). However, one of skill in the art would appreciate that no assay would be expected to correctly classify every single sample; rather, some misclassification is expected in the art. The goal is generally to minimize, rather than eliminate, misclassifications. Further, an assay (such as an assay of LCM levels) can be combined with other types of tests (such as CT screening) to further minimize misclassifications.

In other exemplary scoring systems, if the levels of one or more, three of more, four or more, five of more, six or more, seven or more, eight or more, or all nine markers of the 9-marker panel of Cyfra, SLPI, TIMP1, SCC, TFP1, CEACAM5, MMP2, OPN, and MDK are greater than or equal to the established cutoff value for each marker, then the sample can be classified as a lung tumor sample. In certain exemplary scoring systems, if the level of a marker is greater than or equal to the established cutoff value for that marker, it can be assigned a value of one (for example) and if the level of a marker is below the established cutoff value for that marker, it can be assigned a value of zero (for example). However, any desired values can be assigned to the various outcomes. Furthermore, these values can be added together (or otherwise combined) to determine a total score for a sample, and a classification of the sample as a tumor or normal sample (for example) can be assigned based on this total score. For example, in the example described above and depicted in Figure 17, a score of two or greater can be used to classify a sample as a tumor sample (and a score below two could be used to classify a sample as a normal sample) if the level of each marker that is greater than or equal to its established cutoff value is assigned a value of one. Any other scoring system can be used, and one of ordinary skill in the art would know how to select or devise a scoring system best suited for the intended use.

A scoring system and cutoff values such as those exemplified in Figure 17, or any other desirable scoring system and cutoff values, can be applied to any of the other LCM and LCM panels disclosed herein, such as the 6-marker subset of the 9-marker panel (Cyfra, SLPI, TIMP1, TFPI, CEACAM5, and MDK) and any of the other panels provided in Table 5, and can optionally incorporate any supplemental biomedical parameters. For example, any supplemental biomedical parameters can be assigned a value in a scoring system (e.g., a history of smoking can be assigned a value of one or other value, and no smoking history can be assigned a value of zero, negative one, or other value), and such values can be combined with the values assigned to marker levels being above a predetermined cutoff level (for example), such as to generate a total score for classifying a sample as a lung tumor or normal sample. Furthermore, these various scoring systems and cutoff values can be applied to any of the lung cancer-related uses disclosed herein, including specific uses such as those disclosed in Tables 7-10.

Any of the scoring systems disclosed herein or known in the art, or which may be devised by one of ordinary skill in the art, can be incorporated into a computer program, and such a computer program can be embodied on computer readable medium. For example, a computer program can generate a total score from a sample based on, for example, the number of markers in a panel for which the levels are above predetermined cutoff levels, together with parameters from CT screening (e.g., tumor volume/size, tumor morphology, tumor location, and/or other tumor characteristics, etc.) and/or other supplemental biomedical parameters (e.g., smoking history, age of the individual, etc.), and this total score can be used to classify a sample as a lung tumor or normal sample, for example. A single total score can be generated that represents the combination of multiple different types of assessments (e.g., a combination of LCM levels and supplemental biomedical parameters), or multiple individual scores can be generated for evaluation individually (e.g., a score based on assessment of LCM levels, and one or more separate scores based on supplemental biomedical parameters). An example of this type of integrated approach of combining LCM levels (using the exemplary panel of TIMP1, TFP1, CEACAM5, and Ca72-4) with a supplemental biomedical parameter (smoking history, as indicated by "pack years") is shown in Figure 21.

### Kits

Any combination of LCM and LCM panels (as well as supplemental biomedical parameters) can be provided in the form of kits, such as for use in performing the methods disclosed herein. Furthermore, any kit can contain one or more detectable labels (e.g., detactably labeled reagents such as antibodies), such as a fluorescent moiety, etc..

For example, a kit can comprise (a) reagents comprising at least one antibody for quantifying one or more LCM in a test sample, wherein said LCM comprise: Cyfra, SLPI, TIMP1, SCC, TFP1, CEACAM5, MMP2, OPN, and MDK (or just Cyfra, SLPI, TIMP1, TFPI, CEACAM5, and MDK; or any other LCM or LCM panels disclosed herein, such as the panels disclosed in Tables 5-12), and optionally (b) one or more algorithms or computer programs for performing the steps of comparing the amount of each LCM quantified in the test sample to one or more predetermined cutoffs and assigning a score for each LCM quantified based on said comparison, combining the assigned score for each LCM quantified to obtain a total score, comparing the total score with a predetermined total score, and using said comparison to determine whether an individual has lung cancer. Alternatively, rather than one or more algorithms or computer programs, one or more instructions for manually performing the above steps by a human can be provided.

In certain embodiments, a kit can contain: (a) reagents comprising at least one antibody for quantifying one or more LCM in a test sample, wherein said LCM are Cyfra, SLPI, TIMP1, SCC, TFP1, CEACAM5, MMP2, OPN, and MDK, and (b) reagents containing one or more LCM for quantifying at least one antibody in a test sample; wherein said antibodies are: TP53 (p53), KLKBI, CFLI (CFLN), EEFIG, HSP90α (HSP90AA1), RTN4, ALDOA, GLG1, PTK7, EFEMP1, SLC3A2 (CD98), CHGB, CEACAM1, ALCAM, HSPB1 (HSP27), LGALS1, and B7H3, and optionally (c) one or more algorithms or computer programs for performing the steps of comparing the amount of each LCM and antibody quantified in the test sample to one or more predetermined cutoffs and assigning a score for each LCM and antibody quantified based on said comparison, combining the assigned score for each LCM and antibody quantified to obtain a total score, comparing the total score with a predetermined total score, and using said comparison to determine whether an individual has lung cancer. Alternatively, rather than one or more algorithms or computer programs, one or more instructions for manually performing the above steps by a human can be provided.

### Translating LCM Assessments to Lung Cancer Assessments, and Systems Therefor

An assessment of LCM in an individual, such as LCM levels determined by assaying a serum samples (or other sample) from the individual, can be translated to an assessment of lung cancer for the individual. For example, the levels of multiple LCM (such as each of the LCM in the 9-marker panel of Cyfra, SLPI, TIMP1, SCC, TFP1, CEACAM5, MMP2, OPN, and MDK) can be translated to a score or other identifier that indicates whether an individual has lung cancer (or that indicates the likelihood that the individual has lung cancer), for example. Similarly, the score or other identifier may indicate a specific type of lung cancer assessment, such as the assessments of various lung cancer characteristics described herein, including (but not limited to), determination of whether an individual's lung cancer is adenocarcinoma or squamous cell carcinoma, determination of the stage of an individual's lung cancer (such as distinguishing between stage I and stage III lung cancer), determination of whether an individual's lung cancer is SCLC or NSCLC, determining whether a lung lesion identified in an individual (such as by CT screening) is a malignant tumor or a benign lesion, and determining lung tumor regression and/or recurrence. Any of these determinations may be expressed in a discrete (e.g., absolute) or continuous (e.g., likelihood) manner, for example.

Furthermore, the assessment of LCM in an individual, such as LCM levels, can be translated to a tangible report. Thus, a score or other identifier that indicates the lung cancer assessment can be provided in the form of a tangible report.

Additionally, the translation, such as the translation of LCM levels to a lung cancer assessment (such as a score or other identifier), can be performed by a computer. Furthermore, certain aspects of the disclosure relate to computer readable medium having a computer program code embodied thereon for translating LCM levels to a lung cancer assessment.

In certain aspects, disclosed herein are systems for assessing one or more LCM, particularly the levels of multiple LCM, and translating this LCM assessment to an assessment of lung cancer, such as a determination of whether an individual has (or is likely to have) lung cancer (which may be indicated by a score or other identifier). In certain aspects of the disclosure, these systems include one or more computers to receive an LCM assessment, translate the LCM assessment to a lung cancer assessment, and output the lung cancer assessment (e.g., as a score or other identifier). These systems may optionally comprise multiple computers that communicate via the internet (or any other mode of communication used in the art for inter-computer communication).

Accordingly, disclosed herein are methods of translating an assessment of LCM (e.g., LCM levels) to an assessment of lung cancer (e.g., a score or other indication of whether an individual has lung cancer, or their likelihood of having lung cancer, or other specific lung cancer assessment). In certain embodiments, this assessment of lung cancer is provided in the form of a tangible report. In certain embodiments, the translation is performed by a computer. Furthermore, disclosed herein are computers programmed to translate an LCM assessment to a lung cancer assessment. Certain aspects of the disclosure provide computer readable medium having a computer program code embodied thereon for translating LCM levels to a lung cancer assessment. In certain aspects of the disclosure, a system is provided for receiving an LCM assessment, translating the LCM assessment to a lung cancer assessment, and outputing the lung cancer assessment (e.g., as a score or other identifier). In various aspects of the disclosure, the system comprises one or more computers (which may optionally communicate via the internet or other mode of communication).

### Reports, Transmission of Reports, and Programmed Computers

The results of a test (*e.g.,* a diagnosis of lung cancer for an individual based on the level, or other assay, of one or more LCM disclosed herein, or assessment of tumor progression/regression/recurrence, lung cancer stage, type of lung cancer such as NSCLC versus SCLC or adenocarcinoma versus squamous cell carcinoma, malignant tumor versus benign lung lesion, *etc*.), and/or any other information pertaining to a test *(e.g.,* the levels of one or more LCM disclosed herein in a sample from an individual, which may optionally be provided in the absence of explicit disease or diagnostic information), may be referred to herein as a "report". A tangible report can optionally be generated as part of a testing process (which may be interchangeably referred to herein as "reporting", or as "providing" a report, "producing" a report, or "generating" a report).

Examples of tangible reports may include, but are not limited to, reports in paper (such as computer-generated printouts of test results) or equivalent formats and reports stored on computer readable medium (such as a CD, USB flash drive or other removable storage device, computer hard drive, or computer network server, etc.). Reports, particularly those stored on computer readable medium, can be part of a database, which mary optionally be accessible via the internet (such as a database of patient records or biomedical information stored on a computer network server, which may be a "secure database" that has security features that limit access to the report, such as to allow only the patient and the patient's medical practioners to view the report while preventing other unauthorized individuals from viewing the report, for example). In addition to, or as an alternative to, generating a tangible report, reports can also be displayed on a computer screen (or the display of another electronic device or instrument).

A report can further be "transmitted" or "communicated" (these terms may be used herein interchangeably), such as to the individual who was tested, a medical practitioner *(e.g..* a doctor, nurse, clinical laboratory practitioner, etc.), a healthcare organization, a clinical laboratory, and/or any other party or requester intended to view or possess the report. The act of "transmitting" or "communicating" a report can be by any means known in the art, based on the format of the report. Furthermore, "transmitting" or "communicating" a report can include delivering a report ("pushing") and/or retrieving ("pulling") a report. For example, reports can be transmitted/communicated by various means, including being physically transferred between parties (such as for reports in paper format) such as by being physically delivered from one party to another, or by being transmitted electronically or in signal form (*e.g*., via e-mail or over the internet, by facsimile, and/or by any wired or wireless communication methods known in the art) such as by being retrieved from a database stored on a computer network server, etc.

The present disclosure describes computers (or other apparatus/devices such as biomedical devices or laboratory instrumentation) programmed to carry out the methods described herein. For example, disclosed herein is a computer programmed to receive (*i.e*., as input) the levels of one or more LCM disclosed herein and provide (*i.e*., as output) a lung cancer diagnosis or other result (*e.g*., assessment of tumor progression/regression/recurrence, lung cancer stage, type of lung cancer such as NSCLC versus SCLC or adenocarcinoma versus squamous cell carcinoma, malignant tumor versus benign lung lesion, *etc.*) based on the levels of one or more LCM. Such output (*e.g*., communication of lung cancer diagnosis, *etc.*) may be, for example, in the form of a report on computer readable medium, printed in paper form, and/or displayed on a computer screen or other display.

Further exemplary aspects of the disclosure are described in greater detail below.

### 1. LCM Proteins

The present disclosure describes LCM proteins that consist of, consist essentially of, or comprise the amino acid sequences of SEQ ID NOS:1-65 (additionally, carbohydrate antigens CA 242, CA 19-9, and CA 72-4 are also provided, which may also be encompassed by references herein to proteins/polypeptides), as well as all known variants and fragments of these proteins, and nucleic acid molecules that are within the an to make and use. Examples of such obvious variants include, but are not limited to, naturally-occurring allelic variants, pre-processed or mature processed forms of a protein, non-naturally occurring recombinantly-derived variants, orthologs, and paralogs. Such variants can readily be generated using art-known techniques in the fields of recombinant nucleic acid technology and protein biochemistry.

A protein is said to be "isolated" or "purified" when it is substantially free of cellular material or free of chemical precursors or other chemicals. LCM proteins can be purified to homogeneity or other degrees of purity. The level of purification can be based on the intended use. The primary consideration is that the preparation allows for the desired function of the protein, even if in the presence of considerable amounts of other components.

In some uses, "substantially free of cellular material" includes preparations of a protein having less than about 30% (by dry weight) other proteins (i.e., contaminating protein), less than about 20% other proteins, less than about 10% other proteins, or less than about 5% other proteins. When the protein is recombinantly produced, it can also be substantially free of culture medium, i.e., culture medium represents less than about 20% of the volume of the protein preparation.

The language "substantially free of chemical precursors or other chemicals" includes preparations of a protein in which the protein is separated from chemical precursors or other chemicals that are involved in the protein's synthesis. In one aspects of the disclosure, the language "substantially free of chemical precursors or other chemicals" includes preparations of a LCM protein having less than about 30% (by dry weight) chemical precursors or other chemicals, less than about 20% chemical precursors or other chemicals, less than about 10% chemical precursors or other chemicals, or less than about 5% chemical precursors or other chemicals.

Isolated LCM proteins can be purified from cells that naturally express it, purified from cells that have been altered to express it (recombinant), or synthesized using known protein synthesis methods (e.g., Sambrook et al., Molecular Cloning: A Laboratory Manual. 3rd. ed., Cold Spring Harbor Laboratory Press, Cold Spring Harbor, NY, (2001)). For example, a nucleic acid molecule encoding a LCM protein can be cloned into an expression vector, the expression vector introduced into a host cell, and the protein expressed in the host cell. The protein can then be isolated from the cells by an appropriate purification scheme using standard protein purification techniques.

A LCM protein or fragment thereof can be attached to heterologous sequences to form chimeric or fusion proteins. Such chimeric and fusion proteins comprise a protein operatively linked to a heterologous protein having an amino acid sequence not substantially homologous to the protein. "Operatively linked" indicates that the protein and the heterologous protein are fused in-frame. The heterologous protein can be fused to the N-terminus or C-terminus of the protein.

In some uses, the fusion protein does not affect the activity of the protein *per se.* For example, the fusion protein can include, but is not limited to, beta-galactosidase fusions, yeast two-hybrid GAL fusions, poly-His fusions, MYC-tagged, HI-tagged, and Ig fusions. Such fusion proteins, particularly poly-His fusions, can facilitate the purification of recombinant LCM proteins. In certain host cells (e.g., mammalian host cells), expression and/or secretion of a protein can be increased by using a heterologous signal sequence.

A chimeric or fusion LCM protein can be produced by standard recombinant DNA techniques. For example, DNA fragments coding for different protein sequences can be ligated together in-frame in accordance with conventional techniques. In another aspects of the disclosure, a fusion gene can be synthesized by conventional techniques including automated DNA synthesizers. Alternatively, PCR amplification of gene fragments can be carried out using anchor primers that give rise to complementary overhangs between two consecutive gene fragments that can subsequently be annealed and re-amplified to generate a chimeric gene sequence (Ausubel et al., Current Protocols in Molecular Biology, 1992-2006). Moreover, many expression vectors are commercially available that already encode a fusion moiety (e.g., a GST protein). A LCM-encoding nucleic acid can be cloned into such an expression vector such that the fusion moiety is linked in-frame to the LCM protein.

To determine the percent identity of two amino acid sequences or two nucleic acid sequences, the sequences can be aligned for optimal comparison purposes (e.g., gaps can be introduced in one or both of a first and a second amino acid or nucleic acid sequence for optimal alignment and non-homologous sequences can be disregarded for comparison purposes). In an exemplary aspects of the disclosure, at least 30%, 40%, 50%. 60%, 70%, 80%, or 90% or more of the length of a reference sequence can be aligned for comparison purposes. The amino acid residues or nucleotides at corresponding amino acid positions or nucleotide positions can then be compared. When a position in the first sequence is occupied by the same amino acid residue or nucleotide as the corresponding position in the second sequence, then the molecules are identical at that position (as used herein, amino acid or nucleic acid "identity" is equivalent to amino acid or nucleic acid "homology"). The percent identity between the two sequences is a function of the number of identical positions shared by the sequences, taking into account the number of gaps, and the length of each gap, that are introduced for optimal alignment of the two sequences.

The comparison of sequences and determination of percent identity and similarity between two sequences can be accomplished using a mathematical algorithm. (Computational Molecular Biology, Lesk, A.M., ed., Oxford University Press, New York, 1988; Biocomputing: Informatics and Genome Projects, Smith, D.W., ed.. Academic Press, New York, 1993; Computer Analysis of Sequence Data, Part I, Griffin, A.M., and Griffin, H.G., eds., Humana Press, New Jersey, 1994; Sequence Analysis in Molecular Biology, von Heinje, G., Academic Press, 1987; and Sequence Analysis Primer, Gribskov, M. and Devereux, J., eds., Stockton Press, New York, 1991). For example, the percent identity between two amino acid sequences is determined using the Needleman and Wunsch (J. Mol. Biol. (48):444-453 (1970)) algorithm which has been incorporated into the GAP program in the GCG software package, using either a Blossom 62 matrix or a PAM250 matrix, a gap weight of 16, 14, 12, 10, 8, 6, or 4, and a length weight of 1, 2, 3, 4, 5, or 6. In another example, the percent identity between two nucleotide sequences can be determined using the GAP program in the GCG software package (Devereux et al., Nucleic Acids Res. 12(1):387 (1984)) using a NWSgapdna.CMP matrix and a gap weight of 40, 50, 60, 70, or 80, and a length weight of 1, 2, 3, 4, 5, or 6. In another example, the percent identity between two amino acid or nucleotide sequences is determined using the algorithm of E. Myers and W. Miller (CABIOS, 4:11-17 (1989)) which has been incorporated into the ALIGN program (version 2.0), using a PAM120 weight residue table, a gap length penalty of 12, and a gap penalty of 4.

The sequences of the proteins and nucleic acid molecules disclosed herein can further be used as a "query sequence" to perform a search against sequence databases to, for example, identify other protein family members or related sequences. Such searches can be performed using the NBLAST and XBLAST programs (version 2.0) of Altschul et al. (J. Mol. Biol. 215:403-10 (1990)). BLAST nucleotide searches can be performed with the NBLAST program, score = 100, wordlength = 12, to obtain nucleotide sequences homologous to the query nucleic acid molecule. BLAST protein searches can be performed with the XBLAST program, score = 50, wordlength = 3, to obtain amino acid sequences homologous to the query proteins. To obtain gapped alignments for comparison purposes, Gapped BLAST can be utilized as described in Altschul et al. (Nucleic Acids Res. 25(17):3389-3402 (1997)). When utilizing BLAST and gapped BLAST programs, the default parameters of the respective programs (e.g., XBLAST and NBLAST) can be used.

As used herein, two proteins (or a region or domain of the proteins) have significant homology/identity (also referred to as substantial homology/identity) when the amino acid sequences are typically at least about 70-80%, 80-90%, 90-95%. 96%, 97%. 98%, or 99% identical A significantly homologous amino acid sequence can be encoded by a nucleic acid molecule that hybridizes to a LCM protein-encoding nucleic acid molecule under stringent conditions, as more fully described below.

Orthologs of a LCM protein typically have some degree of significant sequence homology to at least a portion of a LCM protein and are encoded by a gene from another organism. Preferred orthologs are isolated from mammals, preferably non-human primates, for the development of human therapeutic markers and agents. Such orthologs can be encoded by a nucleic acid molecule that hybridizes to a LCM protein-encoding nucleic acid molecule under moderate to stringent conditions, as more fully described below, depending on the degree of relatedness of the two organisms yielding the proteins.

Non-naturally occurring variants of the LCM proteins can readily be generated using recombinant techniques. Such variants include, but are not limited to, deletions, additions, and substitutions in the amino acid sequence of the LCM protein. For example, one class of substitutions is conserved amino acid substitutions. Such substitutions are those that substitute a given amino acid in a LCM protein by another amino acid of like characteristics. Typically seen as conservative substitutions are the replacements, one for another, among the aliphatic amino acids Ala, Val, Leu, and Ile; interchange of the hydroxy residues Ser and Thr; exchange of the acidic residues Asp and Glu; substitution between the amide residues Asn and Gln; exchange of the basic residues Lys and Arg; and replacements among the aromatic residues Phe and Tyr. Guidance concerning which amino acid changes are likely to be phenotypically silent are found in Bowie et al., Science 247:1306-1310 (1990).

Variant LCM proteins can be fully functional or can lack function in one or more activities, e.g.. ability to bind substrate, ability to phosphorylate substrate, ability to mediate signaling, etc. Fully functional variants typically contain only conservative variations or variation in non-critical residues or in non-critical regions.

Non-functional variants typically contain one or more non-conservative amino acid substitutions, deletions, insertions, inversions, or truncations, or a substitution, insertion, inversion, or deletion in a critical residue or critical region.

Amino acids that are essential for function can be identified by methods known in the art, such as site-directed mutagenesis or alanine-scanning mutagenesis (Cunningham et al., Science 244:1081-1085 (1939)). The latter procedure introduces single alanine mutations at every residue in the molecule. The resulting mutant molecules are then tested for biological activity or in assays such as *in vitro* proliferative activity. Sites that are critical for binding partner/substrate binding can also be determined by structural analysis such as crystallization, nuclear magnetic resonance, or photoaffinity labeling (Smith et al., J. Mol. Biol. 224:899-904 (1992); de Vos et al., Science 255:306-312 (1992)).

LCM, disclosed herein include fragments of LCM, and peptides that comprise and consist of such fragments. Such fragments of LCM may be naturally-occurring in the human body. An exemplary fragment typically comprises at least about 5, 6, 8, 10, 12, 14, 16, 18, 20 or more contiguous amino acid residues of a LCM protein. Such fragments can be chosen based on the ability to retain one or more of the biological activities of LCM or can be chosen for the ability to perform a function, e.g., bind a substrate or act as an immunogen. Particularly important fragments are biologically active fragments, such as peptides that are, for example, about 8 or more amino acids in length. Such fragments can include a domain or motif of a LCM, e.g., an active site, a transmembrane domain, or a binding domain. Further, possible fragments include, but are not limited to, soluble peptide fragments and fragments containing immunogenic structures. Domains and functional sites can readily be identified, for example, by computer programs well known and readily available to those of skill in the art (e.g., PROSITE analysis).

Proteins can contain amino acids other than the 20 amino acids commonly referred to as the 20 naturally-occurring amino acids. Further, many amino acids, including the terminal amino acids, can be modified by natural processes, such as processing and other post-translational modifications, or by chemical modification techniques well known in the art. Common modifications that occur naturally in proteins are well known to those of skill in the art.

Known modifications include, but are not limited to, acetylation, acylation, ADP-ribosylation, amidation, covalent attachment of flavin, covalent attachment of a heme moiety, covalent attachment of a nucleotide or nucleotide derivative, covalent attachment of a lipid or lipid derivative, covalent attachment of phosphotidylinositol, cross-linking, cyclization, disulfide bond formation, demethylation, formation of covalent crosslinks, formation of cystine, formation of pyroglutamate, formylation, gamma carboxylation, glycosylation, GPI anchor formation, hydroxylation, iodination, methylation, myristoylation, oxidation, proteolytic processing, phosphorylation, prenylation, racemization, selenoylation, sulfation, tRNA-mediated addition of amino acids to proteins such as arginylation, and ubiquitination.

Such modifications are well known to those of skill in the art and have been described in the scientific literature. Several particularly common modifications, glycosylation, lipid attachment, sulfation, gamma-carboxylation of glutamic acid residues, hydroxylation and ADP-ribosylation, for instance, are described in most basic texts, such as Proteins-Structure and Molecular Properties, 2nd Ed., T.E. Creighton, W. H. Freeman and Company, New York (1993). Many detailed reviews are available on this individual, such as by Wold (Posttranslational Covalent Modification of Proteins, B.C. Johnson, Ed., Academic Press, New York 1-12 (1983)); Seifteret al. (Meth. Enzymol. 182: 626-646 (1990)); and Rattan et al. (Ann. N.Y. Acad. Sci. 663:48-62 (1992)).

Accordingly, exemplary LCM proteins and fragments thereof can also encompasses derivatives or analogs in which, for example, a substituted amino acid residue is not one encoded by the genetic code, in which a substituent group is included, in which a mature LCM is fused with another composition, such as a composition to increase the half-life of a LCM (e.g., polyethylene glycol or albumin), or in which additional amino acids are fused to a mature LCM, such as a leader or secretory sequence or a sequence for purification of a mature LCM or a pro-protein sequence.

### 2. Antibodies to LCM Proteins

The present disclosure describes antibodies to LCM proteins, including, for example, monoclonal and polyclonal antibodies; chimeric, humanized, and fully human antibodies; and antigen-binding fragments and variants thereof, as well as other examples.

Antibodies that selectively bind to a LCM protein can be made using standard procedures known to those of ordinary skills in the art. The term "antibody" is used in the broadest sense, and specifically covers, for example, monoclonal antibodies, polyclonal antibodies, multispecific antibodies (e.g., bispecific antibodies), chimeric antibodies, humanized antibodies, fully human antibodies, and antibody fragments (e.g., Fab, F(ab')₂, Fv and Fv-containing binding proteins), so long as they exhibit LCM-binding activity. Antibodies (Ab's) and immunoglobulins (Ig's) are glycoproteins typically having the same structural characteristics. Antibodies can be of the IgG, IgE, IgM, IgD, and IgA class or subclass thereof (e.g.. IgG1, IgG2, IgG3, IgG4. IgA1 and IgA2). Antibodies may be interchangeably referred to as "LCM-binding molecules".

The term "monoclonal antibody", as used herein, refers to an antibody obtained from a population of substantially homogeneous antibodies, i.e., the individual antibodies comprising the population are substantially identical except for possible naturally occurring mutations that may be present in minor amounts. Monoclonal antibodies are highly specific and are typically directed against a single antigenic site. Furthermore, in contrast to polyclonal antibody preparations, which typically include different antibodies directed against different determinants (epitopes), each monoclonal antibody is typically directed against a single determinant on an antigen. In addition to their specificity, monoclonal antibodies are advantageous in that substantially homogenous antibodies can be produced by a hybridoma culture which is uncontaminated by other immunoglobulins or antibodies. The modifier "monoclonal" antibody indicates the character of the antibody as being obtained from a substantially homogeneous population of antibodies, and is not to be construed as requiring production of the antibody by any particular method. For example, monoclonal antibodies can be made by hybridoma methods such as described by Kohler and Milstein, Nature 256: 495-497 (1975), by recombinant methods (e.g., as described in U.S. Pat. No. 4,816,567), or can be isolated from phage antibody libraries such as by using the techniques described in Clackson et al., Nature 352: 624-628 (1991) or Marks et al., J. Mol. Biol. 222: 581-597 (1991).

"Humanized" forms of non-human (e.g., murine or rabbit) antibodies are chimeric immunoglobulins, immunoglobulin chains or fragments thereof (such as Fv, Fab, Fab', F(ab')₂ or other antigen-binding subsequences of antibodies) which contain minimal sequence derived from non-human immunoglobulin. Typically, humanized antibodies are human immunoglobulins (a recipient antibody) in which residues from a complementarity determining regions ("CDR") of the recipient are replaced by residues from a CDR of a non-human species (a donor antibody) such as mouse, rat, or rabbit having the desired specificity, affinity, and capacity. In some instances, Fv framework residues of the human immunoglobulin are replaced by corresponding non-human residues. Furthermore, a humanized antibody may comprise residues which are found neither in the recipient antibody nor in the imported CDR or framework region (FR) sequences. These modifications can be made to further refine and optimize antibody performance. In general, a humanized antibody can comprise substantially all of at least one, and typically two, variable domains, in which all or substantially all of the CDRs correspond to those of a non-human immunoglobulin and all or substantially all of the FRs are those of a human immunoglobulin consensus sequence. A humanized antibody can also comprise at least a portion of an immunoglobulin constant region (Fc), typically that of a human immunoglobulin. For further details concerning humanized antibodies, see: Jones et al., Nature 321:522-525 (1986); Reichmann et al., Nature 332:323-327 (1988); Presta, Curr. Op. Struct. Biol. 2:593-596 (1992); Queen et al., U.S. Patent Nos. 5,530,101; 5.585,089; 5,693,762; and 6,180,370; and Winter, U.S. Patent No. 5,225,539.

Antibodies, as used herein, include antibody fragments, particularly antigen-binding fragments, as well as other modified antibody structures and antigen-binding scaffolds (such as modified antibody structures that are smaller or have less than all domains or chains compared with a typical naturally occurring, full-size human antibody). Examples of antibody fragments and other modified antibody structures and antigen-binding scaffolds are known in the art by such terms as minibodies (e.g., U.S. Patent No. 5,837,821), Nanobodies (llama heavy chain antibodies; Ablynx, Ghent. Belgium), Adnectins (fibronectin domains; Adnexus Therapeutics, Waltham, MA), Affibodies (protein-binding domain of *Staphylococcus aureus* protein A; Affibody, Stockholm, Sweden), peptide aptamers (synthetic peptides; Aptanomics, Lyon, France), Avimers (A-domains derived from cell surface receptors; Avidia, Mountain View, CA (acquired by Amgen)), Transbodies (transferrin; BioRexis Pharmaceuticals, King of Prussia, PA (acquired by Pfizer)), trimerized tetranectin domains (Borean Pharma, Aarhus, Denmark), Domain antibodies (heavy or light chain antibodies; Domantis, Cambridge, UK (acquired by GlaxoSmithKline), Evibodies (derived from V-like domains of T-cell receptors CTLA-4, CD28 and inducible T-cell costimulator; EvoGenix Therapeutics, Sydney, Australia), scFV fragments (stable single chain antibody fragments; ESBATech, Zurich, Switzerland), Unibodies (monovalent IgG4 mAbs fragments; Genmab, Copenhagen, Denmark), BiTEs (bispecific, T-cell activating single-chain antibody fragments; Micromet, Munich, Germany), DARPins (designed ankyrin repeat proteins; Molecular Partners, Zurich, Switzerland), Anticalins (derived from lipocalins; Pieris, Freising-Weihenstephan, Germany), Affilins (derived from human lens protein gamma crystallize; Scil Proteins, Halle, Germany), and SMIPs (small modular immunopharmaceuticals; Trubion Pharmaceuticals, Seattle, WA) (Sheridan, Nature Biotechnology, 2007 Apr;25(4):365-6).

An "isolated" or "purified" antibody is one that has been identified and separated and/or recovered from a component of the environment in which it is produced. Contaminant components of its production environment are materials that would interfere with diagnostic or therapeutic uses for the antibody, and may include enzymes, hormones, and other proteinaceous or nonproteinaceous solutes. For example, the antibody can be purified as measurable by any of at least three different methods: I) to greater than 95% by weight of antibody as determined by the Lowry method, preferably more than 99% by weight; 2) to a degree sufficient to obtain at least 15 residues of N-terminal or internal amino acid sequence by use of a spinning cup sequenator; or 3) to homogeneity by SDS-PAGE under reducing or non-reducing conditions using Coomasie blue or silver stain. Isolated antibody can include an antibody *in situ* within recombinant cells since at least one component of the antibody's natural environment will not be present. Ordinarily, however, an isolated antibody can be be prepared by at least one purification step.

An "antigenic region", "antigenic determinant", or "epitope" includes any protein determinant capable of specific binding to an antibody. This is the site on an antigen to which each distinct antibody molecule binds. Epitopic determinants can be active surface groupings of molecules such as amino acids or sugar side chains and may have specific three-dimensional structural characteristics or charge characteristics.

"Antibody specificity" refers to an antibody that has a stronger binding affinity for an antigen from a first individual species than it has for a homologue of that antigen from a second individual species. Typically, an antibody "binds specifically" to a human antigen (e.g., has a binding affinity (Kd) value of no more than about 1 x 10⁻⁷ M, preferably no more than about 1 x 10⁻⁸ M, and most preferably no more than about I x 10⁻⁹ M) but has a binding affinity for a homologue of the antigen from a second individual species which is at least about 50-fold, or at least about 500-fold, or at least about 1000-fold, weaker than its binding affinity for the human antigen. The antibodies can be of any of the various types of antibodies as described herein, such as humanized or fully human antibodies.

An antibody "selectively" or "specifically" binds a marker protein when the antibody binds the marker protein and does not significantly bind to unrelated proteins. An antibody can still be considered to selectively or specifically bind a marker protein even if it also binds to other proteins that are not substantially homologous with the marker protein as long as such proteins share homology with a fragment or domain of the marker protein. In this case, it would be understood that antibody binding to the marker protein is still selective despite some degree of cross-reactivity.

For reference only, disclosed herein is an "antibody variant", which refers to an amino acid sequence variant of an antibody wherein one or more of the amino acid residues have been modified. Such variants necessarily have less than 100% sequence identity with the amino acid sequence of the antibody, and have at least 75%, 80%, 85%, 90%, 95%, 96%, 97%, 98%, or 99% amino acid sequence identity with the amino acid sequence of either the heavy or light chain variable domain of the antibody.

The term "antibody fragment" refers to a portion of a full-length antibody, including the antigen binding or variable region or the antigen-binding portion thereof. Examples of antibody fragments include Fab, Fab', F(ab')₂ and Fv fragments. Papain digestion of antibodies typically produces two identical antigen binding fragments, called the Fab fragment, each with a single antigen binding site, and a residual "Fc" fragment. Pepsin treatment typically yields an F(ab')₂ fragment that has two antigen binding fragments which are capable of crosslinking antigen, and a residual other fragment (which is termed pFc'). Examples of additional antigen-binding fragments can include diabodies, triabodies, tetrabodies, single-chain Fv, single-chain Fv-Fc, SMIPs, and multispecific antibodies formed from antibody fragments. A "functional fragment", with respect to antibodies, typically refers to an Fv, F(ab), F(ab')₂ or other antigen-binding fragments comprising one or more CDRs that has substantially the same antigen-binding specificity as an antibody.

An "Fv" fragment is an example of an antibody fragment that contains a complete antigen recognition and binding site. This region typically consists of a dimer of one heavy and one light chain variable domain in a tight, non-covalent association (V_{H}-V_{L} dimer). It is in this configuration that the three CDRs of each variable domain interact to define an antigen-binding site on the surface of the V_{H}-V_{L} dimer. Collectively, the six CDRs confer antigen-binding specificity to the antibody. However, even a single variable domain (or half of an Fv comprising only three CDRs specific for an antigen) has the ability to recognize and bind antigen.

An "Fab" fragment (also designated as "F(ab)") also contains the constant domain of the light chain and the first constant domain (CH1) of the heavy chain. Fab' fragments differ from Fab fragments by the addition of a few residues at the carboxyl terminus of the heavy chain CH1 domain, including one or more cysteines from the antibody hinge region. Fab'-SH is the designation for Fab' in which the cysteine residue(s) of the constant domains have a free thiol group. F(ab') fragments are produced by cleavage of the disulfide bond at the hinge cysteines of the F(ab')₂ pepsin digestion product. Additional chemical couplings of antibody fragments are known to those of ordinary skill in the art.

A "single-chain Fv" or "scFv" antibody fragment contains V_{H} and V_{L} domains, wherein these domains are present in a single polypeptide chain. Typically, the Fv polypeptide further comprises a polypeptide linker between the V_{H} and V_{L} domains that enables the scFv to form the desired structure for antigen binding. For a review of scFv, see Plückthun in The Pharmacology of Monoclonal Antibodies, vol. 113, Rosenburg and Moore eds., Springer-Verlag, New York, pp. 269-315 (1994). A single chain Fv-Fc is an scFv linked to a Fc region.

A "diabody" is a small antibody fragment with two antigen-binding sites, which fragments comprise a variable heavy domain (V_{H}) connected to a variable light domain (V_{L}) in the same polypeptide chain. By using a linker that is too short to allow pairing between the two domains on the same chain, the domains are forced to pair with the complementary domains of another chain and create two antigen-binding sites. Diabodies are described more fully in, for example, EP 0 404 097; WO 93/11161; and Hollinger et al., Proc. Natl. Acad. Sci. USA 90:6444-6448 (1993). Triabodies, tetrabodies and other antigen-binding antibody fragments have been described by Hollinger and Hudson, 2005, Nature Biotechnology 23:1126.

A "small modular immunopharmaceutical" (or "SMIP") is a single-chain polypeptide including a binding domain (e.g., an scFv or an antigen binding portion of an antibody), a hinge region, and an effector domain (e.g., an antibody Fc region or a portion thereof). SMIPs are described in published U.S. Patent Application No. 20050238646.

Many methods are known for generating and/or identifying antibodies to a given marker protein. Several such methods are described by Kohler et al., 1975, Nature 256: 495-497; Lane, 1985, J. Immunol. Meth. 81:223-228; Harlow et al., 1988. Antibodies: A Laboratory Manual. Cold Spring Harbor Laboratory Press; Harlow et al., 1998, Using Antibodies. Cold Spring Harbor Press; Zhong et al., 1997, J. Indust. Microbiol. Biotech. 19(1):71-76; and Berry et al., 2003, Hybridoma and Hybridomics 22(1): 23-31.

Polyclonal antibodies can be prepared by any known method or modifications of these methods, including obtaining antibodies from patients. In certain exemplary methods for generating antibodies such as polyclonal antibodies, an isolated protein can be used as an immunogen which is administered to a mammalian organism, such as a rat, rabbit, or mouse. For example, a complex of an immunogen such as a LCM protein (or fragment thereof) and a carrier protein can be prepared and an animal immunized by the complex. Serum or plasma containing antibodies against the protein can be recovered from the immunized animal and the antibodies separated and purified (in the same manner as for monoclonal antibodies, for example). The gamma globulin fraction or the IgG antibodies can be obtained, for example, by use of saturated ammonium sulfate or DEAE SEPHADEX, or other techniques known to those skilled in the art. The antibody titer in the antiserum can be measured in the same manner as in the supernatant of a hybridoma culture.

A marker such as a full-length LCM protein, an antigenic peptide fragment, a fusion protein thereof, or a carbohydrate antigen or fragment thereof, can be used as an immunogen. A marker used as an immunogen is not limited to any particular type of immunogen. In one aspect, antibodies can be prepared from regions or discrete fragments, (e.g., functional domains, extracellular domains, or portions thereof) of a LCM. Antibodies can be prepared from any region of a marker as described herein. In particular, the markers can be selected from the group consisting of SEQ ID NOS:1-65, the carbohydrate antigens CA 242, CA 19-9, and CA 72-4, and fragments thereof. An antigenic fragment can typically comprise at least 8, 10, 12, 14, 16, or more contiguous amino acid residues, for example. Such fragments can be selected based on a physical property, such as fragments that correspond to regions located on the surface of a marker (e.g., hydrophilic regions) or can be selected based on sequence uniqueness.

Antibodies can also be produced by inducing production in a lymphocyte population or by screening antibody libraries or panels of highly specific binding reagents, such as disclosed in Orlandi et al. (Proc. Natl. Acad. Sci. 86:3833-3837 (1989)) or Winter et al. (Nature 349:293-299 (1991)). A protein can be used in screening assays of phagemid or B-lymphocyte immunoglobulin libraries to identify antibodies having a desired specificity. Numerous protocols for competitive binding or immunoassays using either polyclonal or monoclonal antibodies with established specificities are well known in the art (e.g., Smith, Curr. Opin. Biotechnol. 2:668-673 (1991)).

Antibodies can also be generated using various phage display methods known in the art. In representative phage display methods, functional antibody domains are displayed on the surface of phage particles which carry nucleic acid molecules that encode the antibody domains. In particular, such phage can be utilized to display antigen-binding domains expressed from a repertoire or combinatorial antibody library (e.g., human or murine). Phage expressing an antigen binding domain that binds an antigen of interest can be selected or identified with the antigen, e.g., using labeled antigen or antigen bound or captured to a solid surface or bead. Phage used in methods such as these can typically be filamentous phage including fd and M 13 binding domains expressed from phage with Fab, Fv, or disulfide stabilized Fv antibody domains recombinantly fused to either the phage gene III or gene VIII protein. Examples of phage display methods that can be used to make antibodies include methods described in Brinkman et al., J. Immunol. Methods 182:41-50 (1995); Ames et al., J. Immunol. Methods 184:177-186 (1995); Kettleborough et al.; Eur. J. Immunol. 24:952-958 (1994); Persic et al.. Gene 187:9-18 (1997); Burton et al., Advances in Immunology 57:191-280(1994); PCT application No. PCT/GB91/01134; PCT publications WO 90/02809; WO 91/10737; WO 92/01047; WO 92/18619; WO 93/11336; WO 95/15982; WO 95/20401; and U.S. Pat. Nos. 5,698,426; 5,223,409; 5,403,484; 5,580,717; 5,427,908; 5,750,753; 5,821,047; 5,571,698; 5,427,908; 5,516,637; 5,780,225; 5,658,727; 5,733,743 and 5,969,108.

Antibodies, antigen binding fragments, and/or antibody variants can be produced by recombinant and genetic engineering methods well known in the art. For example, methods of expressing heavy and light chain genes in *E. coli* are described in PCT publication numbers WO901443, WO901443, and WO9014424, and in Huse et al., 1989 Science 246:1275-1281. When using recombinant techniques, such as to produce an antibody variant, the antibody variant can be produced intracellularly, in the periplasmic space, or directly secreted into the medium. If an antibody variant is produced intracellularly, as a first step, the particulate debris, either host cells or lysed fragments, can be removed, for example, by centrifugation or ultrafiltration. Carter et al. (Bio/Technology 10: 163-167 (1992)) describe a procedure for isolating antibodies that are secreted to the periplasmic space of *E. coli.* Briefly, cell paste can be thawed in the presence of sodium acetate (pH 3.5), EDTA, and phenylmethylsulfonylfluoride (PMSF) over about 30 minutes. Cell debris can be removed by centrifugation. Where an antibody variant is secreted into the medium, supernatants from such expression systems can first be concentrated using a commercially available protein concentration filter (e.g.. an Amicon or Millipore PELLICON ultrafiltration unit). A protease inhibitor such as PMSF can be included in any of the foregoing steps to inhibit proteolysis, and antibiotics can be included to prevent the growth of contaminating microorganisms.

An antibody composition prepared from cells can be purified using, for example, affinity chromatography, hydroxylapatite chromatography, gel electrophoresis, and/or dialysis. The suitability of protein A as an affinity ligand typically depends on the species and isotype of the immunoglobulin Fc domain of an antibody. Protein A can be used to purify antibodies that are based on human delta1, delta2, or delta4 heavy chains (Lindmark et al., J. Immunol Meth. 62: 1-13 (1983)). Protein G can be used for all mouse isotypes and for human delta3 (Guss et al., EMBO J. 5: 1567-1575 (1986)). The matrix to which the affinity ligand is attached can be, for example, agarose or mechanically stable matrices such as controlled pore glass or poly(styrenedivinyl)benzene. Where the antibody comprises a CH3 domain, the BAKERBOND ABX™ resin (J.T. Baker, Phillipsburg, N.J.) can be used for purification. Other exemplary techniques for antibody purification include, but are not limited to. fractionation on an ion-exchange column, ethanol precipitation, reverse phase HPLC, chromatography on silica, chromatography on heparin hepharos, chromatography on an anion or cation exchange resin (such as a polyaspartic acid column), chromatofocusing, SDS-PAGE, and ammonium sulfate precipitation.

Following any preliminary purification step(s), contaminants in a mixture containing an antibody of interest can be removed by low pH hydrophobic interaction chromatography using an elution buffer at a pH between about 2.5-4.5, preferably performed at low salt concentrations (e.g., from about 0-0.25M salt).

Full-length antibodies, as well as antibody fragments, can also be expressed and isolated from bacteria such as *E. coli,* such as described in Mazor et al., "Isolation of engineered, full-length antibodies from libraries expressed in Escherichia coli", Nat Biotechnol. 2007 May;25(5):563-5 and Sidhu, "Full-length antibodies on display", Nat Biotechnol. 2007 May;25(5):537-8.

Further details regarding antibodies are set forth in the following U.S. patent numbers: 6,248,516 (Winter et al.); 6,291,158 (Winter et al.); 5,885,793 (Griffiths et al.); 5,969,108 (McCafferty et al.); 5,939,598 (Kucherlapati et al.); 4,816,397 (Boss et al.); 4,816,567 (Cabilly et al.); 6,331,415 (Cabilly et al.); 5,770,429 (Lonberg et al.); 5,639,947 (Hiatt et al.): and 5,260,203 (Ladneret al.), and in the following published U.S. patent applications: US20040132101 (Lazar et al.), US20050064514 (Stavenhagen et al.), US20040261148 (Dickey et al.), and US20050014934 (Hinton et al.). Antibody engineering is further described in Jain et al., "Engineering antibodies for clinical applications", Trends Biotechnol. 2007 Jul;25(7):307-16.

### 3. Antibody-Drug Conjugates to LCM Proteins

An antibody against LCM can be coupled (e.g., covalently bonded) to a suitable therapeutic agent (as further discussed herein) either directly or indirectly (e.g., via a linker group). A direct reaction between an antibody and a therapeutic agent is possible when each possesses a substituent capable of reacting with die other. For example, a nucleophilic group, such as an amino or sulfhydryl group, on one molecule may be capable of reacting with a carbonyl-containing group, such as an anhydride or an acid halide, or with an alkyl group containing a good leaving group (e.g., a halide) on the other molecule.

Alternatively, it may be desirable to couple a therapeutic agent and an antibody via a linker group. A linker group can function as a spacer to distance an antibody from an agent in order to avoid interference with binding capabilities. A linker group can also serve to increase the chemical reactivity of a substituent on an agent or an antibody, and thus increase the coupling efficiency. An increase in chemical reactivity may also facilitate the use of agents, or functional groups on agents, which otherwise would not be possible.

A variety of bifunctional or polyfunctional reagents, both homo- and hetero-functional (such as those described in the catalog of the Pierce Chemical Co., Rockford, 111.), can be employed as the linker group. Coupling can be effected, for example, through amino groups, carboxyl groups, sulfhydryl groups, or oxidized carbohydrate residues (e.g., U.S. Pat. No. 4,671,958).

Where a therapeutic agent is more potent when free from the antibody portion of an immunoconjugate, it may be desirable to use a linker group that is cleavable during or upon internalization into a cell. A number of different cleavable linker groups have been described. Mechanisms for the intracellular release of an agent from these linker groups include cleavage by reduction of a disulfide bond (e.g., U.S. Pat. No. 4,489,710), by irradiation of a photolabile bond (e.g., U.S. Pat. No. 4,625,014), by hydrolysis of derivatized amino acid side chains (e.g., U.S. Pat. No. 4,638,045), by serum complement-mediated hydrolysis (e.g., U.S. Pat. No. 4,671,958), by protease cleavable linker (e.g., U.S. Pat. No. 6,214,345), and by acid-catalyzed hydrolysis (e.g., U.S. Pat. No. 4,569,789).

It may be desirable to couple more than one agent to an antibody. Multiple molecules of an agent can be coupled to one antibody molecule, and more than one type of agent can be coupled to the same antibody. For example, about I, 2, 4, 6. 8, 10, 12, 14, 16. 18, 20, or 22 (or any other number in-between) molecules of therapeutic agents can be coupled to an antibody. The average number or quantitative distribution of therapeutic agent molecules per antibody molecule in a preparation of conjugation reactions can be determined by conventional means such as mass spectroscopy, ELISA, or HPLC. Separation, purification, and characterization of homogeneous antibody-drug conjugates having a certain number of therapeutic agents conjugated thereto can be achieved by means such as reverse phase HPLC or electrophoresis (see, e.g., Hamblett et al., Clinical Cancer Res. 10:7063-70 (2004).

Examples of suitable therapeutic agents that can be conjugated to an antibody include, but are not limited to, chemotherapeutic agents (e.g., cytotoxic or cytostatic agents or immunomodulatory agents), radiotherapeutic agents, therapeutic antibodies, small molecule drugs, peptide drugs, immunomodulatory agents, differentiation inducers, and toxins.

Examples of useful classes of cytotoxic or immunomodulatory agents include, but are not limited to, antitubulin agents, auristatins, DNA minor groove binders, DNA replication inhibitors, alkylating agents (e.g., platinum complexes such as cis-platin, mono(platinum), bis(platinum) and tri-nuclear platinum complexes and carboplatin), anthracyclines, antibiotics, antifolates, antimetabolites, chemotherapy sensitizers, duocannycins, etoposides, fluorinated pyrimidines, ionophores, lexitropsins, nitrosoureas, platinols, pre-forming compounds, purine antimetabolites, puromycins, radiation sensitizers, steroids, taxanes, topoisomerase inhibitors, vinca alkaloids, and the like.

Examples of individual cytotoxic or immunomodulatory agents include, but are not limited to, androgen, anthramycin (AMC), asparaginase, 5-azacytidine, azathioprine, bleomycin, busulfan, buthionine sulfoximine, calicheamicin or calicheamicin derivatives, camptothecin or camptothecins derivatives, carboplatin, carmustine (BSNU), CC-1065, chlorambucil, cisplatin, colchicine, cyclophosphamide, cytidine arabinoside (cytarabine), cytochalasin B, dacarbazine, dactinomycin (formerly actinomycin), daunorubicin, decarbazine, docetaxel, doxorubicin, etoposide, estrogen, 5-fluordeoxyuridine, 5-fluorouracil, gemcitabine, gramicidin D, hydroxyurea, idarubicin, ifosfamide, irinotecan, lomustine (CCNU), maytansine, mechlorethamine, melphalan, 6-mercaptopurine, methotrexate, mithramycin, mitomycin C, mitoxantrone, nitroimidazole, paclitaxel, palytoxin, plicamycin, procarbizine, rhizoxin, streptozotocin, tenoposide, 6-thioguanine, thioTEPA, topotecan, vinblastine, vincristine, vinorelbine, VP-16, and VM-26.

Examples of other suitable cytotoxic agents include, but are not limited to, DNA minor groove binders (e.g., enediynes and lexitropsins, a CBI compound; see also U.S. Patent No. 6,130,237), duocarmycins, taxanes (e.g., paclitaxel and docetaxel), puromycins, vinca alkaloids, CC-1065, SN-38, topotecan, morpholino-doxorubicin, rhizoxin, cyanomorpholino-doxorubicin, echinomycin, combretastatin, netropsin, epothilone A and B, estramustine, cryptophysins, cemadotin, a maytansinoid, discodermolide, eleutherobin, and mitoxantrone.

Examples of other suitable agents include, but are not limited to, radionuclides, differentiation inducers, drugs, toxins, and derivatives thereof. Exemplary radionuclides include ⁹⁰Y, ¹²³I, ¹²⁵I, ¹³¹I, ¹⁸⁶Re, ¹⁸⁸Re, ²¹¹At, and ²¹²Bi. Exemplary drugs include methotrexate, and pyrimidine and purine analogs. Exemplary differentiation inducers include phorbol esters and butyric acid. Exemplary toxins include ricin, abrin, diptheria toxin, cholera toxin, gelonin, *Pseudomonas* exotoxin, *Shigella* toxin, and pokeweed

In some aspects of the disclosure, the therapeutic agent used in an antibody-drug conjugate is an anti-tubulin agent. Examples of anti-tubulin agents include, but are not limited to taxanes (e.g., Taxol® (paclitaxel), Taxotere® (docetaxel)), T67 (Tularik) and vinca alkyloids (e.g., vincristine, vinblastine, vindesine, and vinorelbine). Other antitubulin agents include, for example, baccatin derivatives, taxane analogs (e.g., epothilone A and B), nocodazole, colchicine and colcimid, estramustine, cryptophysins, cemadotin, maytansinoid, combretastatins, discodermolide, and eleutherobin.

In certain aspects of the disclosure, the cytotoxic agent is a maytansinoid, another group of anti-tubulin agents. For example, in specific examples, the maytansinoid is maytansine. DM-1 (ImmunoGen, Inc.; see also Chari et al., Cancer Res. 52:127-131 (1992)) or DM-4.

In some aspects of the disclosure, the therapeutic agent is an auristatin, such as auristatin E (also known in the art as dolastatin-10) or a derivative thereof. Typically, an auristatin E derivative is, e.g., an ester formed between auristatin E and a keto acid. For example, auristatin E can be reacted with paraacetyl benzoic acid or benzoylvaleric acid to produce AEB and AEVB, respectively. Other typical auristatin derivatives include AFP, MMAF, and MMAE. The synthesis and structure of auristatin derivatives are described in U.S. Patent Application Publication Nos. 2003-0083263, 2005-0238649 and 2005-0009751; PCT Publication Nos WO 04/010957 and WO 02/088172, and U.S. Patent Nos. 6,323,315; 6,239,104; 6,034,065; 5,780,588; 5,665,860; 5,663,149; 5,635,483; 5,599,902; 5,554,725; 5,530,097; 5,521,284; 5,504,191; 5,410,024; 5,138,036; 5,076,973; 4,986,988; 4,978,744; 4,879,278; 4,816,444; and 4,486,414.

### 4. LCM Nucleic Acid Molecules

Exemplary isolated LCM nucleic acid molecules disclosed herein consist of, consist essentially of, or comprise a nucleotide sequence that encodes a LCM protein disclosed herein, an allelic variant thereof, or an ortholog or paralog thereof, for example. As used herein, an "isolated" nucleic acid molecule is one that is separated from other nucleic acid present in the natural source of the nucleic acid. Preferably, an "isolated" nucleic acid is free of sequences which naturally flank the nucleic acid (i.e., sequences located at the 5' and 3' ends of the nucleic acid) in the genomic DNA of the organism from which the nucleic acid is derived. However, there can be some flanking nucleotide sequences, for example up to about 5 kilobases (KB), 4KB, 3KB, 2KB, or 1KB or less, particularly contiguous protein-encoding sequences and protein-encoding sequences within the same gene but separated by introns in the genomic sequence, and flanking nucleotide sequences that contain regulatory elements. The primary consideration is that the nucleic acid is isolated from remote and unimportant flanking sequences such that it can be individualed to the specific manipulations described herein such as recombinant expression, preparation of probes and primers, and other uses specific to the nucleic acid molecules. Moreover, an "isolated" nucleic acid molecule, such as a transcript/cDNA molecule, can be substantially free of other cellular material, or culture medium when produced by recombinant techniques, or chemical precursors or other chemicals when chemically synthesized.

A nucleic acid molecule can be fused to other coding or regulatory sequences and still be considered isolated. Isolated nucleic acid molecules can include heterologous nucleotide sequences, such as heterologous nucleotide sequences that are fused to a nucleic acid molecule by recombinant techniques. For example, recombinant DNA molecules contained in a vector are considered isolated. Further examples of isolated DNA molecule include recombinant DNA molecules maintained in heterologous host cells, or purified (partially or substantially) DNA molecules in solution. Isolated RNA molecules include *in vivo* or *in vitro* RNA transcripts of isolated DNA molecules. Isolated nucleic acid molecules further include such molecules produced synthetically.

Isolated nucleic acid molecules can encode a mature protein plus additional amino or carboxyl-terminal amino acids, or amino acids interior to the mature protein (when the mature form has more than one peptide chain, for instance). Such sequences may play a role in processing of a protein from precursor to a mature form, facilitate protein trafficking, prolong or shorten protein half-life, or facilitate manipulation of a protein for assay or production, among other things. As generally is the case *in situ,* additional amino acids may be processed away from the mature protein by cellular enzymes.

Isolated nucleic acid molecules include, but are not limited to, sequences encoding a LCM protein alone, sequences encoding a mature protein with additional coding sequences (such as a leader or secretory sequence (e.g., a pre-pro or pro-protein sequence)), and sequences encoding a mature protein (with or without additional coding sequences) plus additional non-coding sequences (e.g., introns and non-coding 5' and 3' sequences such as transcribed but non-translated sequences that play a role in transcription, mRNA processing (including splicing and polyadenylation signals), ribosome binding, and/or stability of mRNA). In addition, nucleic acid molecule can be fused to a marker sequence encoding, for example, a peptide that facilitates purification.

Isolated nucleic acid molecule can be in the form of RNA, such as mRNA, or in the form of DNA, including cDNA and genomic DNA obtained by cloning or produced by chemical synthetic techniques or by a combination thereof. Nucleic acid molecules, especially DNA, can be double-stranded or single-stranded. Single-stranded nucleic acid can be the coding strand (sense strand) or the non-coding strand (anti-sense strand).

Further, disclosed herein are isolated nucleic acid molecules that encode fragments of a LCM protein as well as nucleic acid molecules that encode obvious variants of a LCM protein. Such nucleic acid molecules may be naturally occurring, such as allelic variants (same locus), paralogs (different locus), and orthologs (different organism), or can be constructed by recombinant DNA methods or by chemical synthesis. Such non-naturally occurring variants can be made by mutagenesis techniques, including those applied to nucleic acid molecules, cells, or organisms. Accordingly, nucleic acid molecule variants can contain nucleotide substitutions, deletions, inversions, and/or insertions. Variations can occur in either or both the coding and non-coding regions, and variations can produce conservative and/or non-conservative amino acid substitutions.

A fragment of a nucleic acid molecule typically comprises a contiguous nucleotide sequence at least 8, 10, 12, 15, 16, 18, 20, 22, 25. 30. 40, 50, 100, 150, 200, 250, 500 (or any other number in-between) or more nucleotides in length. The length of a fragment can be based on its intended use. For example, a fragment can encode epitope bearing regions of a protein, or can be used as DNA probes and primers. Isolated fragments can be produced by synthesizing an oligonucleotide probe using known techniques, for example, and can optionally be labeled and used to screen a cDNA library, genomic DNA, or mRNA, for example. Primers can be used in PCR reactions to clone specific regions of a gene.

A probe/primer typically comprises substantially a purified oligonucleotide or oligonucleotide pair. An oligonucleotide typically comprises a nucleotide sequence that hybridizes under stringent conditions to at least about 8, 10, 12, 14, 15, 16, 17, 18, 19, 30, 21, 22, 23, 24, 25, 30, 40, 50 (or any other number in-between) or more contiguous nucleotides.

Allelic variants, orthologs, and homologs can be identified using methods well known in the art. These variants can comprise a nucleotide sequence encoding a protein that is typically 60-70%. 70-80%, 80-90%, 90-95%, 96%, 97%, 98%, or 99% homologous to the nucleotide sequence. Such nucleic acid molecules can readily be identified as being able to hybridize under moderate to stringent conditions, to a nucleotide sequence shown in the Sequence Listing or a fragment thereof.

As used herein, the term "hybridizes under stringent conditions" is intended to describe conditions for hybridization and washing under which nucleotide sequences encoding a protein at least 60-70% homologous to each other typically remain hybridized to each other. The conditions can be such that sequences at least about 60%, at least about 70%, or at least about 80% or more homologous to each other tyically remain hybridized to each other. Such stringent conditions are known to those skilled in the art and can be found in, for example, Current Protocols in Molecular Biology, John Wiley & Sons, N.Y. (1989-2006). 6.3.1-6.3.6. One example of stringent hybridization conditions is hybridization in 6X sodium chloride/sodium citrate (SSC) at about 45°C, followed by one or more washes in 0.2 X SSC, 0.1% SDS at 50-65°C. Examples of moderate to low stringency hybridization conditions are well known in the art.

Disclosed herein are also kits for detecting the presence of LCM nucleic acid (e.g., DNA or mRNA) in a biological sample. For example, a kit can comprise reagents such as a labeled or labelable nucleic acid and/or other agents capable of detecting LCM nucleic acid in a biological sample; means for determining the amount of LCM nucleic acid in the sample; and means for comparing the amount of LCM nucleic acid in the sample with a standard. The nucleic acid and/or other agent can be packaged in one or more suitable containers. The kit can further comprise instructions for using the kit to detect LCM nucleic acid.

### 5. Vectors and Host Celts

The present disclosure also describes vectors containing LCM nucleic acid molecules. The term "vector" refers to a vehicle, such as a nucleic acid molecule, which can transport the LCM nucleic acid molecules. When the vector is a nucleic acid molecule, the LCM nucleic acid molecules are covalently linked to the vector nucleic acid. A vector can be, for example, a plasmid, single or double stranded phage, a single or double stranded RNA or DNA viral vector, or artificial chromosome, such as a BAC, PAC, YAC, OR MAC.

A vector can be maintained in a host cell as an extrachromosomal element where it replicates and produces additional copies of the LCM nucleic acid molecules. Alternatively, a vector can integrate into a host cell genome and produce additional copies of the LCM nucleic acid molecules when the host cell replicates.

Disclosed herein are vectors for maintenance (cloning vectors) and vectors for expression (expression vectors) of the nucleic acid molecules, for example. Expression vectors can express a portion of, or all of, a protein sequence. Vectors can function in prokaryotic or eukaryotic cells or in both (shuttle vectors). Vectors also include insertion vectors, which integrate a nucleic acid molecule into another nucleic acid molecule, such as into the cellular genome (such as to alter *in situ* expression of a gene and/or gene product). For example, an endogenous protein-coding sequence can be entirely or partially replaced via homologous recombination with a protein-coding sequence containing one or more specifically introduced mutations.

Expression vectors can contain cis-acting regulatory regions that are operably-linked in the vector to the nucleic acid molecules such that transcription of the nucleic acid molecules is allowed in a host cell. The nucleic acid molecules can be introduced into the host cell with a separate nucleic acid molecule capable of affecting transcription. The separate nucleic acid molecule may provide, for example, a trans-acting factor interacting with the cis-regulatory control region to allow transcription of the nucleic acid molecules from the vector. Alternatively, a trans-acting factor may be supplied by a host cell. Additionally, a trans-acting factor can be produced from a vector itself. It is understood, however, that transcription and/or translation of nucleic acid molecules can occur in cell-free systems.

Regulatory sequences to which LCM nucleic acid molecules can be operably linked include, for example, promoters for directing mRNA transcription. These include, but are not limited to, the left promoter from bacteriophage, the Iac, TRP, and TAC promoters from *E. coli,* the early and late promoters from SV40, the CMV immediate early promoter, the adenovirus early and late promoters, and retrovirus long-terminal repeats.

In addition to control regions that promote transcription, expression vectors can also include regions that modulate transcription, such as repressor binding sites and enhancers. Examples include the SV40 enhancer, the cytomegalovirus immediate early enhancer, polyoma enhancer, adenovirus enhancers, and retrovirus LTR enhancers.

In addition to containing sites for transcription initiation and control, expression vectors can also contain sequences necessary for transcription termination and, in the transcribed region, a ribosome binding site for translation. Other regulatory control elements for expression include initiation and termination codons as well as polyadenylation signals. Numerous regulatory sequences useful in expression vectors are well known in the art (e.g., Sambrook et al., Molecular Cloning: A Laboratory Manual. 3rd. ed., Cold Spring Harbor Laboratory Press, Cold Spring Harbor, NY (2001)).

A variety of expression vectors can be used to express a nucleic acid molecule. Such vectors include chromosomal, episomal, and virus-derived vectors, for example vectors derived from bacterial plasmids, from bacteriophage, from yeast episomes, from yeast chromosomal element, including yeast artificial chromosomes, from viruses such as baculoviruses, papovaviruses such as SV40, Vaccinia viruses, adenoviruses, poxviruses, pseudorables viruses, and retroviruses. Vectors may also be derived from combinations of these sources such as those derived from plasmid and bacteriophage genetic elements, e.g. cosmids and phagemids. Appropriate cloning and expression vectors for prokaryotic and eukaryotic hosts are described in Sambrook et al., Molecular Cloning: A Laboratory Manual. 3rd. ed., Cold Spring Harbor Laboratory Press, Cold Spring Harbor, NY (2001).

A regulatory sequence can provide constitutive expression in one or more host cells (e.g., tissue specific) or can provide for inducible expression in one or more cell types such as by temperature, nutrient additive, or exogenous factors such as a hormone or other ligand. A variety of vectors providing for constitutive and inducible expression in prokaryotic and eukaryotic hosts are well known in the art.

Nucleic acid molecules can be inserted into vector nucleic acid by well-known methodology. For example, the DNA sequence that will ultimately be expressed can be joined to an expression vector by cleaving the DNA sequence and the expression vector with one or more restriction enzymes and then ligating the fragments together. Procedures for restriction enzyme digestion and ligation are well known in the art.

A vector containing a nucleic acid molecules of interest can be introduced into an appropriate host cell for propagation or expression using well-known techniques. Bacterial cells include, but are not limited to, *E. coli, Streptomyces,* and *Salmonella typhimurium.* Eukaryotic cells include, but are not limited to, yeast, insect cells such as *Drosophila,* animal cells such as COS and CHO cells (e.g., DG44 or CHO-s), and plant cells.

As described herein, it may be desirable to express a protein as a fusion protein. Accordingly, disclosed herein are fusion vectors that allow for the production of fusion proteins. Fusion vectors can, for example, increase the expression of a recombinant protein; increase the solubility of a recombinant protein, and/or aid in the purification of a protein such as by acting as a ligand for affinity purification. A proteolytic cleavage site can be introduced at the junction of the fusion moiety so that the desired protein can ultimately be separated from the fusion moiety. Proteolytic enzymes include, but are not limited to, factor Xa, thrombin, and enteroenzyme. Typical fusion expression vectors include pGEX (Smith et al., Gene 67:31-40 (1988)). pMAL (New England Biolabs, Beverly, MA) and pRIT5 (Pharmacia, Piscataway, NJ), which fuse glutathione S-transferase (GST), maltose E binding protein, or protein A, respectively, to a recombinant marker protein. Examples of suitable inducible non-fusion *E. coli* expression vectors include pTrc (Amann et al., Gene 69:301-315 (1988)) and pET 11d (Studier et al., Gene Expression Technology: Methods in Enzymology 185:60-89 (1990)).

Recombinant protein expression can be maximized in host bacteria by providing a genetic background wherein the host cell has an impaired capacity to proteolytically cleave the recombinant protein (Gottesman, S., Gene Expression Technology: Methods in Enzymology 185, Academic Press, San Diego, California (1990), pp. 119-128). Alternatively, the sequence of a nucleic acid molecule of interest can be altered to provide preferential codon usage for a specific host cell, such as *E. coli* (Wada et al., Nucleic Acids Res. 20:2111-2118 (1992)).

LCM nucleic acid molecules can, for example, be expressed by expression vectors in a yeast host. Examples of vectors for expression in yeast (e.g., ***S.** cerevisiae*) include pYepSecI (Baldari, et al., EMBO J. 6:229-234 (1987)), pMFa (Kurjan et al.,Cell 30:933-943 (1982)), pJRY88 (Schultz et al., Gene 54:113-123 (1987)), and pYES2 (Invitrogen Corporation, San Diego, CA). Nucleic acid molecules can also be expressed in insect cells using, for example, baculovirus expression vectors. Baculovirus vectors available for expression of proteins in cultured insect cells (e.g., Sf 9 cells) include the pAc series (Smith et al., Mol. Cell Biol. 3:2156-2165 (1983)) and the pVL series (Lucklow et al., Virology 170:31-39 (1989)). Nucleic acid molecules can also be expressed in mammalian cells using mammalian expression vectors. Examples of mammalian expression vectors include pCDM8 (Seed; B. Nature 329:840 (1987)), pMT2PC (Kaufman et al., EMBO J. 6:187-195 (1987)), and CHEF (U.S. Pat. No. 5,888,809).

The expression vectors listed herein are provided by way of example only of well-known vectors available to those of ordinary skill in the art that would be useful to express LCM nucleic acid molecules. The person of ordinary skill in the art would be aware of other vectors suitable for maintenance, propagation, and/or expression of LCM nucleic acid molecules (e.g., Sambrook et al. Molecular Cloning: A Laboratory Manual. 3rd. ed., Cold Spring Harbor Laboratory Press, Cold Spring Harbor, NY (2001).

Further, disclosed herein are also vectors in which LCM nucleic acid molecules are cloned into a vector in reverse orientation, but operably linked to a regulatory sequence that permits transcription of antisense RNA. Thus, an antisense transcript can be produced to all, or to a portion, of a LCM nucleic acid molecule, including coding and non-coding regions. Expression of this antisense RNA may be individual to each of the parameters described above in relation to expression of the sense RNA (e.g., regulatory sequences, constitutive or inducible expression, tissue-specific expression).

Disclosed herein are also recombinant host cells containing the vectors described herein. Host cells include, for example, prokaryotic cells, lower eukaryotic cells such as yeast, other eukaryotic cells such as insect cells, and higher eukaryotic cells such as mammalian cells.

Recombinant host cells can be prepared by introducing vector constructs, such as described herein, into cells by techniques readily available to a person of ordinary skill in the art. These techniques include, but are not limited to, calcium phosphate transfection. DEAE-dextran-mediated transfection, cationic lipid-mediated transfection, electroporation, transduction, infection, lipofection. microinjection, and other techniques such as those found in Sambrook, et al. (Molecular Cloning: A Laboratory Manual. 3rd. ed., Cold Spring Harbor Laboratory Press, Cold Spring Harbor, NY (2001).

For example, using techniques such as these, a retroviral or other viral vector can be introduced into mammalian cells. Examples of mammalian cells into which a retroviral vector can be introduced include, but are not limited to, primary mammalian cultures or continuous mammalian cultures, COS cells, NIH3T3, 293 cells (ATCC #CRL 1573), and dendritic cells.

Host cells can contain more than one vector. Thus, different nucleotide sequences can be introduced on different vectors of the same cell. Similarly, nucleic acid molecules of interest can be introduced either alone or with other unrelated nucleic acid molecules such as those providing trans-acting factors for expression vectors. When more than one vector is introduced into a cell, the vectors can be introduced independently, co-introduced, or joined to the nucleic acid molecule vector.

Bacteriophage and viral vectors can be introduced into cells as packaged or encapsulated virus by standard procedures for infection and transduction. Viral vectors can be replication-competent or replication-defective. If viral replication is defective, replication can occur in host cells that provide functions that complement the defects.

Vectors can include selectable markers that enable the selection of a subpopulation of cells that contain the recombinant vector constructs. Markers can be contained in the same vector that contains the nucleic acid molecules of interest or can be on a separate vector. Exemplary markers include tetracycline or ampicillin-resistance genes for prokaryotic host cells, and dihydrofolate reductase or neomycin resistance for eukaryotic host cells. However, any marker that provides selection for a phenotypic trait can be used.

While mature proteins can be produced in bacteria, yeast, mammalian cells, and other cells under the control of appropriate regulatory sequences, cell-free transcription and translation systems can also be used to produce these proteins using RNA derived from the DNA constructs described herein.

If secretion of a protein is desired, appropriate secretion signals can be incorporated into a vector. The signal sequence can be endogenous or heterologous to the protein.

If a protein is not secreted into a medium, the protein can be isolated from a host cell by standard disruption procedures, including freeze/thaw, sonication, mechanical disruption, use of lysing agents, and the like. A protein can then be recovered and purified by well-known purification methods including, for example, ammonium sulfate precipitation, acid extraction, anion or cationic exchange chromatography, phosphocellulose chromatography, hydrophobic-interaction chromatography, affinity chromatography, hydroxylapatite chromatography, lectin chromatography, or high performance liquid chromatography.

It is also understood that, depending upon the host cell used in recombinant production of a protein, proteins can have various glycosylation patterns or can be non-glycosylated, such as when produced in bacteria. In addition, proteins can include an initial modified methionine in some instances as a result of a host-mediated process.

Recombinant host cells that express a LCM protein have a variety of uses. For example, such host cells are useful for producing LCM proteins, which can be further purified to produce desired amounts of the protein or fragments thereof. Thus, host cells containing expression vectors are useful for protein production.

Host cells are also useful for conducting cell-based assays involving a LCM protein or fragments thereof. For example, a recombinant host cell expressing a LCM protein can be used to assay compounds that stimulate or inhibit the protein's function.

Host cells are also useful for identifying mutant LCM proteins in which the protein's function is affected. Host cells expressing mutant proteins are useful for assaying compounds that have a desired effect on the mutant proteins (e.g., stimulating or inhibiting function), particularly if the mutant proteins naturally occur and give rise to a pathology.

### 6. Diagnosis and Treatment in General

The following terms, as used in the present specification and claims, are intended to have the meaning as defined below, unless indicated otherwise.

As used herein, a "biological sample" (or just "sample") can comprise, for example, tissue, blood, sera, cells, cell lines, or biological fluids such as plasma, interstitial fluid, urine, cerebrospinal fluid, and the like. A biological sample is typically, although not necessarily, obtained from an individual by a medical practitioner.

As used herein, a "individual" can be a mammalian individual or non-mammalian individual, preferably a mammalian individual. A mammalian individual can be a human or non-human, preferably a human. The terms "individual", "individual", and "patient" are used herein interchangeably.

A "healthy" or "normal" individual or biological sample is a individual or biological sample in which the disease of interest (e.g., lung cancer) is not detectable, as ascertained by using conventional diagnostic methods (such a biological sample can interchangeably be referred to as a "control." sample).

As used herein, "disease(s)" include cancer, especially aerodigestive cancers, and particularly lung cancer, as well as associated diseases and pathologies, such as other lung diseases.

The term "diagnose" (or "diagnosing", etc.) refers to determining the current state or status (e.g., the presence/absence or characteristics) of a disease condition, such as initially detecting the presence of a disease, characterizing/classifying a disease, or detecting disease progression, remission, or recurrence.

The term "prognose" (or "prognosing", etc.) refers to predicting the future course of a disease in a patient who has the disease (e.g., predicting patient survival).

The term "assess" (or "assessing", etc.) can encompass "diagnose" and "prognose" but can also encompass making future determinations/predictions about the disease in an individual who does not have the disease or determining/predicting the likelihood that a disease will recur in an individual who apparently has been cured of the disease. The term "assess" can also encompass making assessments of an individual's response to a therapy, such as predicting whether an individual is likely to respond favorably to a therapeutic agent or is unlikely to respond to a therapeutic agent (or will experience toxic or other undesirable side effects, for example), selecting a therapeutic agent for administration to an individual, or monitoring or determining an individual's response to a therapy that has been administered to the individual.

Thus, "assessing" lung cancer can include, for example, prognosing the future course of lung cancer; predicting recurrence of lung cancer in an individual who apparently has been cured of lung cancer; and/or determining or predicting an individual's response to a lung cancer treatment or selecting a lung cancer treatment to administer to an individual based on the individual's LCM profile (i.e., the differential abundance level of one or more LCM in the individual).

The following examples may be referred to as either "diagnosing" or "assessing" lung cancer: initially detecting the presence of lung cancer; determining a specific stage, type or sub-type, or other classification or characteristic of lung cancer; determining whether a lung lesion is a benign lesion or a malignant lung tumor; and/or detecting/monitoring lung cancer progression (e.g., monitoring lung tumor growth or metastatic spread), remission, or recurrence.

LCM are therefore useful as "prognostic markers" (e.g., predicting disease progression) and "predictive markers" (e.g., predicting drug response), among other uses.

"Treat", "treating", or "treatment" of a disease includes: (1) inhibiting the disease, i.e.. arresting or reducing the development of the disease or its clinical symptoms, or (2) relieving the disease, i.e., causing regression of the disease or its clinical symptom(s).

The term "prophylaxis" is used to distinguish from "treatment," and to encompass both "preventing" and "suppressing." It is not always possible to distinguish between "preventing" and "suppressing," as the ultimate inductive event or events may be unknown, latent, or the patient is not ascertained until well after the occurrence of the event or events. Therefore, the term "protection", as used herein, is meant to include "prophylaxis."

A "therapeutically effective amount" means the amount of an agent that, when administered to a individual for treating a disease, is sufficient to effect such treatment for the disease. The "therapeutically effective amount" can vary depending on such factors as the agent, the disease and its severity, and the age, weight, etc., of the individual to be treated.

Disclosed herein are methods for treating diseases, especially cancer, and particularly lung cancer, comprising administering to a patient a therapeutically effective amount of an antagonist, agonist, or a pharmaceutical composition thereof. Further, disclosed herein are agonists and antagonists to LCM proteins, as well as pharmaceutical compositions that comprise an agonist or antagonist with a suitable carrier such as a pharmaceutically acceptable excipient.

Exemplary agonists or antagonists include antibodies that specifically bind to a LCM protein. Antibodies can be used alone or in combination with one or more other therapeutic agents (e.g., as an antibody-drug conjugate or a combination therapy). Further examples of molecules that can be used as antagonists include, but are not limited to, small molecules that inhibit the function or abundance level of LCM, and inhibitory nucleic acid molecules such as RNAi or antisense nucleic acid molecules that specifically hybridize to LCM nucleic acid.

The present disclosure also describes novel agents identified by screening assays using LCM, such as the screening assays described herein, as well as methods of using these agents, such as for treatment or diagnostic purposes. For example, an agent identified as described herein (e.g., a LCM-modulating agent, a LCM-specific nucleic acid molecule such as an RNAi or antisense molecule, a LCM-specific antibody, a LCM-specific antibody-drug conjugate, or a LCM-binding partner) can be used in an animal or other model, such as to determine efficacy, toxicity, or side effects of treatment with the agent.

Modulators of LCM protein activity, such as modulators identified according to the drug screening assays described herein, can be used to treat a individual with a disorder mediated by a LCM, e.g., by treating cells or tissues that express LCM at a differential level. Methods of treatment can include the step of administering a modulator of LCM activity in a pharmaceutical composition to a individual in need of such treatment.

In certain aspects of the disclosure, if decreased expression or activity of a protein is desired, an antibody to the protein or an inhibitor/antagonist and the like, or a pharmaceutical agent containing one or more of these molecules, can be administered to an individual. In other aspects disclosed herein, if increased expression or activity of a protein is desired, the protein itself or an agonist/enhancer and the like, or a pharmaceutical agent containing one or more of these molecules, can be administered. Administration can be effected by methods well known in the art and may include delivery by an antibody specifically targeted to the protein. Neutralizing antibodies, which inhibit dimer formation, can be used when decreased expression or activity of a protein is desired.

Although modulating agents can be administered in a pure or substantially pure form, modulating agents can also be administed as pharmaceutical compositions, formulations, or preparations with a carrier. Exemplary formulations, such as for human or veterinary use, comprise a suitable active LCM-modulating agent, together with one or more pharmaceutically acceptable carriers and, optionally, other therapeutic ingredients. The carrier(s) are "acceptable" in the sense of being compatible with other ingredients of a formulation and not deleterious to the recipient thereof. The formulations can be presented in unit dosage form and can be prepared by any method known to the skilled artisan.

Examples of suitable pharmaceutical carriers include proteins such as albumins (e.g., U.S. Pat. No. 4,507,234), peptides and polysaccharides such as aminodextran (e.g., U.S. Pat. No. 4,699,784), and water. A carrier can also bear an agent by noncovalent bonding or by encapsulation, such as within a liposome vesicle (e.g., U.S. Pat. Nos. 4,429,008 and 4,873,088). Carriers specific for radionuclide agents include radiohalogenated small molecules and chelating compounds. For examples, U.S. Pat. No. 4,735,792 discloses representative radiohalogenated small molecules and their synthesis. A radionuclide chelate can be formed from chelating compounds that include those containing nitrogen and sulfur atoms as the donor atoms for binding the metal, metal oxide, radionuclide. For example, U.S. Pat. No. 4,673,562 discloses representative chelating compounds and their synthesis.

Methods of preparing pharmaceutical formulations typically include the step of bringing into association the active ingredient with the carrier, which constitutes one or more accessory ingredients. Formulations can be prepared by uniformly and intimately bringing into association the active ingredient with liquid carriers or finely divided solid carriers, or both, and then, if necessary, shaping the product into the desired formulation.

Formulations suitable for intravenous, intramuscular, subcutaneous, or intraperitoneal administration can comprise sterile aqueous solutions of the active ingredient with solutions, which can be isotonic with the blood of the recipient. Such formulations can be prepared by dissolving solid active ingredient in water containing physiologically compatible substances such as sodium chloride (e.g., 0.1-2.0 M), glycine, and the like, and having a buffered pH compatible with physiological conditions to produce an aqueous solution, and rendering the solution sterile. These may be present in unit or multi-dose containers, for example, sealed ampoules or vials.

Exemplary formulations can incorporate a stabilizer. Exemplary stabilizers include polyethylene glycol, proteins, saccharides, amino acids, inorganic acids, detergents, and organic acids, which can be used either alone or as admixtures. These stabilizers can be incorporated in an amount of, for example, 0.11-10,000 parts by weight per part by weight of an agent. If two or more stabilizers are to be used, their total amount can be within the range specified above. These stabilizers can be used in aqueous solutions at an appropriate concentration and pH. The specific osmotic pressure of such aqueous solutions can be in the range of 0.1-3.0 osmoles, preferably in the range of 0.8-1.2. The pH of the aqueous solution can be adjusted to be within the range of 5.0-9.0, preferably within the range of 6-8. In formulating an antibody or antibody-drug conjugate, an anti-adsorption agent can be used.

Additional pharmaceutical methods can be employed to control duration of action. Controlled release can be achieved through the use of polymer to complex or absorb the proteins or their derivatives. Controlled delivery can be achieved by selecting appropriate macromolecules (e.g., polyester, polyamino acids, polyvinyl, pyrrolidone, ethylenevinylacetate, methylcellulose, carboxymethylcellulose, or protamine sulfate) and the concentration of macromolecules as well as the methods of incorporation in order to control release. Another possible method to control the duration of action by controlled-release preparations is to incorporate an anti-LCM antibody into particles of a polymeric material such as polyesters, polyamino acids, hydrogels, poly(lactic acid) or ethylene vinylacetate copolymers. Alternatively, instead of incorporating these agents into polymeric particles, it is possible to entrap these materials in microcapsules prepared, for example, by coacervation techniques or by interfacial polymerization, for example, hydroxymethylcellulose or gelatin-microcapsules and poly(methylmethacylate) microcapsules, respectively, or in colloidal drug delivery systems, for example, liposomes, albumin microspheres, microemulsions, nanoparticles, and nanocapsules or in macroemulsions.

When oral preparations are desired, the compositions can be combined with typical carriers, such as lactose, sucrose, starch, talc magnesium stearate, crystalline cellulose, methyl cellulose, carboxymethyl cellulose, glycerin, sodium alginate or gum arabic, among others.

Any of the therapeutic agents provided herein may be administered in combination with other therapeutic agents. Selection of agents for use in combination therapy can be made by one of ordinary skill in the art according to conventional pharmaceutical principles. A combination of therapeutic agents may act synergistically to affect treatment of a particular disorder at a lower dosage of each agent.

### 7. Methods of Detection and Diagnosis Based on LCM Proteins

LCM proteins are useful for diagnosing a disease, particularly diseases in which the protein is over- or under-expressed, especially cancer, and particularly lung cancer. The diagnostic methods may be further suitable for monitoring disease progression in patients undergoing treatment, or for testing for reoccurrence of disease in patients who were previously treated for a disease, for example. Accordingly, disclosed herein are methods for detecting the presence of, or abundance levels of, a LCM protein in a biological sample.

*In vitro* techniques for detection of proteins include, but are not limited to, enzyme linked immunosorbent assays (ELISAs). Western blots, immunoprecipitations, and immunofluorescence using a detection reagent, such as an antibody or protein binding agent. Alternatively, a protein can be detected *in vivo* in a individual by introducing into the individual a labeled antibody (or other types of detection agent) specific for the protein marker. For example, an antibody can be labeled with a radioactive marker whose presence and location in a individual can be detected by standard imaging techniques. Also useful are methods that detect variants of a protein (e.g., allelic variants or mutations) and methods that detect fragments of a protein in a sample.

Examples of immunoassays that can be used in accordance with exemplary embodiments of the invention include, but are not limited to, competitive and non-competitive assays using techniques such as Western blots, radioimmunoassays, ELISA, "sandwich" immunoassays, immunoprecipitation assays, precipitation reactions, gel diffusion precipitin reactions, immunodiffusion assays, agglutination assays, complement-fixation assays, immunoradiometric assays, fluorescent immunoassays, and protein A immunoassays, as well as fluorescence polarization immunoassay (FPIA), fluorescence immunoassay (FIA), enzyme immunoassay (EIA), and nephelometric inhibition immunoassay (NIA). Immunoassays such as these are well known in the art and are described in, for example, Ausubel et al., Current Protocols in Molecular Biology, 1992-2006.

For example, ELISA can be used to detect or quantify one or more LCM. For example, ELISA (or other types of LCM assays) can be used to detect LCM in, for example, a high-risk individual or population, in an individual suspected of having lung cancer, or in an individual with no suspicion of having lung cancer (e.g., an individual undergoing routine screening for lung cancer).

In certain exemplary ELISA methods, an antibody that specifically binds to an LCM antigen may be coated to the well of a suitable container (e.g., a 96 well microtiter plate), a patient sample (e.g., a serum sample) can be added to the well and incubated for a period of time, and the presence of the LCM antigen in the patient sample can be detected upon binding of the LCM antigen in the patient sample to the antibody that is coated to the well. In this instance, a second antibody conjugated to a detectable moeity may optionally be added following the addition of the patient sample to the coated well. ELISA methods such as these may be modified or optimized as desired.

Further, instead of coating the well with an antibody to an LCM antigen, the LCM antigen itself may be coated to the well. Thus, in certain exemplary ELISA methods, an LCM antigen can be coated to the well of a suitable container (e.g., a 96 well microtiter plate), an antibody (which may optionally be conjugated to a detectable moiety such as an enzymatic substrate like horseradish peroxidase or alkaline phosphatase) to the LCM antigen can be added to the well and incubated for a period of time, and the presence of the LCM antigen can be detected. The antibody to the LCM antigen does not have to be conjugated to a detectable moiety; for example, a second antibody (which recognizes the antibody to the LCM antigen) conjugated to a detectable moeity may be added to the well. ELISA methods such as these may be modified or optimized as desired.

Proteins can be isolated from a biological sample (such as from a patient having a disease) and assayed for the presence of a mutation. A mutation can include, for example, one or more amino acid substitutions, deletions, insertions, rearrangements (such as from aberrant splicing events), or inappropriate post-translational modifications. Examples of analytic methods useful for detecting mutations in a protein include, but are not limited to, altered electrophoretic mobility, altered tryptic peptide digest, altered protein activity in cell-based or cell-free assays, alteration in substrate or antibody-binding patterns, altered isoelectric point, and direct amino acid sequencing.

Information obtained by detecting a protein can be used, for example, to determine prognosis and appropriate course of treatment for a disease. For example, individuals with a particular LCM expression level or stage of disease may respond differently to a given treatment that individuals lacking LCM expression, or individuals over- or under-expressing LCM. Information obtained from diagnostic methods of the invention can provide for the personalization of diagnosis and treatment.

The present disclosure describes methods for diagnosing disease (including, for example, monitoring treatment response or recurrence of disease following treatment) in a individual comprising: determining the abundance level of LCM (e.g., LCM protein or nucleic acid, or protein or nucleic acid fragments thereof) in a test sample from the individual; wherein a difference in the abundance level of LCM relative to the abundance level of LCM in a test sample from a healthy individual, or the level established for a healthy individual, is indicative of disease.

Disclosed herein are methods for diagnosing diseases having differential protein expression. For example, normal, control, or standard values (e.g., that represent typical expression levels of a protein in healthy individuals) can be established, such as by combining body fluids, tissues, or cell extracts taken from a normal healthy mammalian or human individual with specific antibodies to a protein under conditions for complex formation. Standard values for complex formation in normal and disease tissues can be established by various methods, such as photometric means. Complex formation, as it is expressed in a test sample, can be compared with the standard values. Deviation from a normal standard and toward a disease standard can provide parameters for disease diagnosis or prognosis while deviation away from a disease standard and toward a normal standard can be used to evaluate treatment efficacy, for example,

Immunological methods for detecting and measuring complex formation as a measure of protein expression using either specific polyclonal or monoclonal antibodies are known in the art. Examples of such techniques include ELISAs, radioimmunoassays (RIAs), flow cytometry (also referred to as fluorescence-activated cell sorting, or FACS), and antibody arrays. Such immunoassays typically involve the measurement of complex formation between a protein and its specific antibody. These assays and their quantitation against purified, labeled standards are well known in the art (Ausubel, supra, unit 10.1-10.6). For example, a two-site, monoclonal-based inumunoassay utilizing antibodies reactive to two non-interfering epitopes can be utilized, and competitive binding assay can also be utilized (Pound (1998) Immunochemical Protocols, Humana Press, Totowa N.J.).

For diagnostic applications, an antibody can be labeled with a detectable moiety (interchangeably referred to as a "label" or "detectable substance"), such as to facilitate detection by various imaging methods. Methods for detection of labels include, but are not limited to, fluorescence, light, confocal, and electron microscopy: magnetic resonance imaging and spectroscopy; fluoroscopy, computed tomography and positron emission tomography. Examples of suitable labels include, but are not limited to, fluorescein, rhodamine, eosin and other fluorophores, radioisotopes, gold, gadolinium and other lanthanides, paramagnetic iron, fluorine-18 and other positron-emitting radionuclides. Additionally, labels may be bi- or multi-functional and be detectable by more than one of the methods listed. Antibodies may be directly or indirectly labeled. Attachment of labels to antibodies includes covalent attachment of a label, incorporation of a label into an antibody, and covalent attachment of a chelating compound for binding of a label, among others well known in the art.

Numerous detectable moieties are available for labeling antibodies, including, but not limited to, those in the following categories:
(a) Radioisotopes, such as ³⁶S, ¹⁴C, ¹²⁵I, ³H, and ¹³¹I. An antibody can be labeled with a radioisotope using the techniques described in Current Protocols in Immunology, vol 1-2, Coligen et al., Ed., Wiley-Interscience, New York, Pubs. (1991-2006), for example, and radioactivity can be measured using scintillation counting.
(b) Fluorescent labels such as rare earth chelates (europium chelates) or fluorescein and its derivatives, rhodamine and its derivatives, dansyl, Lissamine, phycoerythrin and Texas Red are available. Fluorescent labels can be conjugated to an antibody using the techniques disclosed in Current Protocols in Immunology, supra, for example. Fluorescence can be quantified using a fluorometer.
(c) Various enzyme-substrate labels are available (e.g., U.S. Pat. Nos. 4,275,149 and 4,318,980). An enzyme generally catalyzes a chemical alteration of a chromogenic substrate which can be measured using various techniques. For example, an enzyme may catalyze a color change in a substrate, which can be measured spectrophotometrically. Alternatively, an enzyme may alter the fluorescence or chemiluminescence of a substrate. Techniques for quantifying a change in fluorescence are described herein and well known in the art A chemiluminescent substrate becomes electronically excited by a chemical reaction and may then emit light which can be measured (using a chemiluminometer, for example) or donates energy to a fluorescent acceptor. Examples of enzymatic labels include luciferases (e.g., firefly luciferase and bacterial luciferase; U.S. Pat. No. 4,737,456), luciferin, 2,3-dihydrophthalazinediones, malate dehydrogenase, urease, peroxidase such as horseradish peroxidase (HRPO), alkaline phosphatase, β-galactosidase, glucoamylase, lysozyme, saccharide oxidases (e.g.. glucose oxidase, galactose oxidase, and glucose-6-phosphate dehydrogenase), heterocyclic oxidases (such as uricase and xanthine oxidase), lactoperoxidase, microperoxidase, and the like. Techniques for conjugating enzymes to antibodies are described in O'Sullivan et al.,Methods for the Preparation of Enzyme-Antibody Conjugates for Use in Enzyme Immunoassay, in Methods in Enzyme. (Ed. J. Langone & H. Van Vunakis), Academic press, New York, 73: 147-166 (1981).

A label can be indirectly conjugated with an antibody. The skilled artisan will be aware of various techniques for achieving this. For example, an antibody can be conjugated with biotin and any of the three broad categories of labels mentioned above can be conjugated with avidin, or vice versa. Biotin binds selectively to avidin and thus, the label can be conjugated with the antibody in this indirect manner. Alternatively, to achieve indirect conjugation of a label with an antibody, an antibody can be conjugated with a small hapten (e.g., digoxin) and one of the different types of labels mentioned above can be conjugated with an anti-hapten antibody (e.g., anti-digoxin antibody). Thus, indirect conjugation of a label with an antibody can be achieved.

Antibodies can be used to isolate LCM proteins by standard techniques, such as affinity chromatography or immunoprecipitation, and antibodies can facilitate the purification of the natural protein from cells and recombinantly-produced protein expressed in host cells. Biological samples can be tested directly for the presence of a LCM protein by assays (e.g., ELISA or radioimmunoassay) and format (e.g., microwells, dipstick, etc., as described in International Patent Publication WO 93/03367). Alternatively, proteins in a sample can be size separated (e.g., by polyacrylamide gel electrophoresis (PAGE)), in the presence or absence of sodium dodecyl sulfate (SDS), and the presence of a LCM detected by immunoblotting (e.g., Western blotting).

Antibody binding can also be detected by "sandwich" immunoassays, immunoradiometric assays, gel diffusion precipitation reactions, immunodiffusion assays, *in situ* immunoassays (e.g., using colloidal gold, enzyme or radioisotope labels, for example, precipitation reactions, agglutination assays (e.g., gel agglutination assays, hemagglutination assays, etc.). complement fixation assays, immunofluorescence assays, protein A assays, and immunoelectrophoresis assays, etc.

In certain exemplary embodiments, antibody binding can be detected by detecting a label on the primary antibody. In other exemplary embodiments, a primary antibody can be detected by detecting binding of a secondary antibody or reagent to the primary antibody. In further exemplary embodiments, the secondary antibody is labeled. Numerous means are known in the art for detecting binding in an immunoassay. In some embodiments, an automated detection assay is utilized. Methods for the automation of immunoassays are well known in the art (e.g., U.S. Pat. Nos. 5,885,530: 4,981,785: 6,159,750: and 5,358,691 ). In some embodiments, the analysis and presentation of results are also automated. For example, in some embodiments, software that generates a prognosis based on the presence or absence of one or more antigens can be implemented.

Competitive binding assays typically rely on the ability of a labeled standard to compete with a test sample for binding with a limited amount of antibody. The amount of antigen in the test sample is inversely proportional to the amount of standard that becomes bound to the antibodies. To facilitate determining the amount of standard that becomes bound, the antibodies generally are insolubilized before or after the competition. As a result, the standard and test sample that are bound to the antibodies can be separated from the standard and test sample that remain unbound.

Sandwich assays typically involve the use of two antibodies, each capable of binding to a different immunogenic portion, or epitope, of the protein to be detected. In typical sandwich assays, the test sample to be analyzed is bound by a first antibody, which is immobilized on a solid support, and thereafter a second antibody binds to the test sample, thus forming an insoluble three-part complex (e.g., U.S. Pat. No. 4,376,110). The second antibody can itself be labeled with a detectable moiety (direct sandwich assays) or can be measured using an anti-immunoglobulin antibody that is labeled with a detectable moiety (indirect sandwich assay). For example, one type of sandwich assay is an ELISA assay, in which case the detectable moiety is an enzyme.

Antibodies can also be used for *in vivo* diagnostic assays. Generally, an antibody can be labeled with a radionuclide (such as ¹¹¹In, ⁹⁹Tc, ¹⁴C, ¹³¹I, ³H, ³²P or ³⁵S) so that disease cells or tissues can be localized using immunoscintiography, for example. In certain aspects of the disclosure, antibodies or fragments thereof bind to the extracellular domains of two or more LCM proteins and the affinity value (Kd) is less than 1 x 10⁸ M.

For immunohistochemistry, a disease tissue sample may be, for example, fresh or frozen or may be embedded in paraffin and fixed with a preservative such as formalin. A fixed or embedded section can be contacted with a labeled primary antibody and secondary antibody, wherein the antibody is used to detect LCM protein expression *in situ.*

Antibodies can be used to detect a marker protein *in situ, in vitro,* or in a cell lysate or supernatant in order to evaluate the abundance and pattern of expression. Also, antibodies can be used to assess abnormal tissue distribution or abnormal expression during development or progression of a biological condition. Antibodies against LCM proteins are useful for detecting the presence of the proteins in cells or tissues to determine the pattern of expression of the proteins among various tissues in an organism and over the course of the organism's development.

Further, antibodies can be used to assess expression in disease states such as in active stages of a disease or in an individual with a predisposition toward disease related to the protein's function. When a disorder is caused by inappropriate tissue distribution, developmental expression, or level of expression of a protein, or expressed/processed form, for example, an antibody can be prepared against the normal protein. If a disorder is characterized by a specific mutation in a protein, antibodies specific for the mutant protein can be used to assay for the presence of the specific mutant protein and to target the mutant protein for therapeutic purposes. Antibodies are also useful as diagnostic tools, as immunological markers for aberrant protein analyzed by electrophoretic mobility, isoelectric point, tryptic peptide digest, and other physical assays known in the art.

Certain exemplary diagnostic methods disclosed herein can also include monitoring a treatment modality. Accordingly, where treatment is ultimately aimed at correcting, for example, the function, activity, expression level, tissue distribution, or developmental expression of a protein, antibodies directed against the protein can be used to monitor therapeutic efficacy and to modify a treatment regimen as necessary.

Additionally, antibodies to a marker protein are useful in pharmacogenomic analysis. For example, antibodies prepared against polymorphic proteins can be used to identify individuals that require modified treatment modalities. Moreover, the marker proteins and antibodies thereto can be used for clinical trials, such as to identify individuals that should be included (e.g., individuals more likely to respond to a therapy) or excluded (e.g., individuals less likely to respond to a therapy, or individuals more likely to experience harmful side effects from a therapy) from a clinical trial.

The present disclosure also describes kits for using antibodies to detect the presence of a marker protein in a biological samples. An exemplary kit can comprise antibodies such as a labeled or labelable antibody and a compound or agent for detecting protein in a biological sample; means for determining the amount of protein in the sample; means for comparing the amount of protein in the sample with a standard; and instructions for use. Such a kit can be configured to detect a single marker protein or epitope or can be configured to detect one of a multitude of epitopes, such as in an antibody detection array.

### LC/MS and ICAT

The present disclosure describes detection or diagnostic methods of a LCM by using LC/MS. Proteins can be prepared from cells by methods known in the art (e.g., Zhang et al., Nature Biotechnology 21(6):660-666 (2003)). The differential expression of proteins in disease and healthy (or drug-resistant and drug-sensitive, for example) samples can be quantitated using mass spectrometry and ICAT (Isotope Coded Affinity Tag) labeling, which is known in the art. ICAT is an isotope label technique that allows for discrimination between two populations of proteins, such as a healthy and a disease sample. Over-expression or under-expression of a LCM protein, as measured by ICAT, can indicate, for example, the likelihood of having or developing a disease or an associated pathology.

LC/MS spectra can be collected for labeled samples and processed as follows. The raw scans from the LC/MS instrument can be individualed to peak detection and noise reduction software. Filtered peak lists can then be used to detect 'features' corresponding to specific peptides from the original sample(s). Features are characterized by their mass/charge ratio, charge, retention time, isotope pattern, and/or intensity, for example.

The intensity of a peptide present in both healthy and disease samples can be used to calculate the differential expression, or relative abundance, of the peptide. The intensity of a peptide found exclusively in one sample can be used to calculate a theoretical expression ratio for that peptide (singleton). Expression ratios can be calculated for each peptide in an assay or experiment.

Statistical tests can be performed to assess the robustness of the data and select statistically significant differentials. To ensure the accuracy of data, the following steps can be taken: a) ensure that similar features are detected in all replicates of an experiment; b) assess the distribution of the log ratios of all peptides (a Gaussian is expected); c) calculate the overall pair wise correlations between ICAT LC/MS maps to ensure that the expression ratios for peptides are reproducible across multiple replicates; and d) aggregate multiple experiments in order to compare the expression ratio of a peptide in multiple diseases or disease samples.

### 8. Methods of Treatment Based on LCM Proteins

### a. Antibody Therapy

Antibodies disclosed herein can be used for therapeutic purposes. It is contemplated that antibodies disclosed herein may be used to treat a mammal, preferably a human, with a disease, especially cancer, and particularly lung cancer. The antibodies can be delivered alone, in a pharmaceutical composition (such as with a carrier), or conjugated to one or more therapeutic agents, for example.

Antibodies can be useful for modulating (e.g., agonizing or antagonizing) protein function, such as for therapeutic purposes. Antibodies can also be useful for inhibiting protein function by, for example, blocking the binding of a LCM protein to a binding partner such as a substrate, which can be useful therapeutically. Antibodies can be prepared against, for example, specific portions of a protein that contain domains required for protein function, or against intact protein that is associated with a cell membrane.

Antibodies can also be used for enhancing the immune response. The antibodies can be administered in amounts similar to those used for other therapeutic administrations of antibodies. For example, pooled gamma globulin can be administered at a range of about 1 mg to about 100 mg per patient.

Antibodies reactive with LCM proteins can be administered alone or in conjunction with other therapies, such as anti-cancer therapies, to a mammal afflicted with cancer or other disease. Examples of anti-cancer therapies include, but are not limited to, chemotherapy, radiation therapy, and adoptive immunotherapy therapy with TIL (tumor infiltrating lymphocytes).

The selection of an antibody subclass for therapy may depend upon the nature of the antigen to be acted upon. For example, an IgM may be preferred in situations where the antigen is highly specific for the disease marker and rarely occurs on normal cells. However, where the disease-associated antigen is also expressed in normal tissues, although at lower levels, the IgG subclass may be preferred. The IgG subclass may be preferred in these instances because the binding of at least two IgG molecules in close proximity is typically required to activate complement, and therefore less complement-mediated damage may occur in normal tissues that express smaller amounts of the antigen and thus bind fewer IgG antibody molecules. Furthermore, IgG molecules, by being smaller, may be more able than IgM molecules to localize to a diseased tissue.

A mechanism for antibody therapy can be that a therapeutic antibody recognizes a soluble or cell surface marker protein that is expressed (preferably, over-expressed) in a disease cell. By NK cell or complement activation, or conjugation of the antibody with an immunotoxin or radiolabel, the interaction of the antibody with the marker protein can abrogate ligand/receptor interaction or activation of apoptosis, for example.

Potential mechanisms of antibody-mediated cytotoxicity of diseased cells include phagocyte (antibody-dependent cellular cytotoxicity (ADCC)), complement (complement-dependent cytotoxicity (CDC)), naked antibody (receptor cross-linking apoptosis and growth factor inhibition), or targeted payload labeled with a therapeutic agent, such as a radionuclide, immunotoxin, or immunochemotherapeutic or other therapeutic agent.

In certain aspects of the disclosure, an antibody is administered to a nonhuman mammal for the purposes of obtaining preclinical data, for example. Exemplary nonhuman mammals to be treated include nonhuman primates, dogs, cats, rodents, and other mammals in which preclinical studies are performed. Such mammals may be established animal models for a disease or may be used to study toxicity of an antibody of interest, for example. Dose escalation studies may be performed in the mammal, for example.

An antibody can be administered to an individual by any suitable means, including parenteral, subcutaneous, intraperitoneal, intrapulmonary, and intranasal, and, if desired for local immunomodulatory treatment, intralesional administration. Parenteral infusions include intramuscular, intravenous, intraarterial, intraperitoneal, or subcutaneous administration. In addition, an antibody can be administered by pulse infusion, particularly with declining doses of the antibody. The dosing can be given by injections, such as intravenous or subcutaneous injections, which may depend in part on whether the administration is brief or chronic.

For the prevention or treatment of a disease, the appropriate dosage of an antibody may depend on the type of disease to be treated, the severity and the course of the disease, whether the antibody is administered for preventive or therapeutic purposes, previous therapy, the patient's clinical history and response to the antibody, and the discretion of the attending physician.

Depending on the type and severity of disease, about 1 µg/kg to 150 mg/kg (e.g., 0.1-20 mg/kg) of antibody can be an initial candidate dosage for administration to a patient, whether, for example, by one or more separate administrations, or by continuous infusion. A typical daily dosage may range from about 1 µg/kg to 100 mg/kg or more, depending on such factors as those mentioned above. An antibody-drug conjugate can be administered from about 1 µg/kg to 50 mg/kg, typically from about 0.1-30 mg/kg, whether, for example, by one or more separate administrations, or by continuous infusion. A typical daily dosage may range from about 0.1 mg/kg to 10 mg/kg, or from about 0.3 mg/kg to about 7.5 mg/kg, depending on such factors as those mentioned above. For repeated administrations over several days or longer, depending on the condition, the treatment can be sustained until a desired suppression of disease symptoms occurs. However, other dosage regimens may be useful. Therapy progress can be monitored by conventional techniques and assays.

Antibody composition can be formulated, dosed, and administered in a manner consistent with good medical practice. Factors for consideration in this context include the particular disorder being treated, the particular mammal being treated, the clinical condition of the individual patient, the cause of the disorder, the site of delivery of the agent, the method of administration, the scheduling of administration, and other factors known to medical practitioners.

An antibody may optionally be formulated with, or administered with, one or more therapeutic agents used to prevent or treat the disorder in question. For example, an antibody can be administered as a co-therapy with a standard of care therapeutic for the specific disease being treated.

### b. Other Immunotherapy

An "immunogenic peptide" is a peptide that comprises an allele-specifc motif such that the peptide typically will bind an MHC allele (HLA in human) and be capable of inducing a CTL (cytotoxic T-lymphocytes) response. Thus, immunogenic peptides typically are capable of binding to an appropriate class I or II MHC molecule and inducing a cytotoxic T cell or T helper cell response against the antigen from which the immunogenic peptide is derived.

Peptides derived from a LCM protein can be modified to increase their immunogenicity, such as by enhancing the binding of the peptide to the MHC molecules in which the peptide is presented. The peptide or modified peptide can be conjugated to a carrier molecule to enhance the antigenicity of the peptide. Examples of carrier molecules, include, but are not limited to, human albumin, bovine albumin, lipoprotein and keyhole limpet hemo-cyanin ("Basic and Clinical Immunology" (1991) Stites and Terr (eds) Appleton and Lange, Norwalk Conn., San Mateo, Calif.).

Further, amino acid sequence variants of a peptide can be prepared, such as by altering the nucleic acid sequence of the DNA which encodes the peptide, or by peptide synthesis. At the genetic level, these variants can be prepared by, for example, site-directed mutagenesis of nucleotides in the DNA encoding the peptide, thereby producing DNA encoding the variant, and thereafter expressing the DNA in recombinant cell culture. The variants can exhibit the same qualitative biological activity as the nonvariant peptide.

The present disclosure describes peptides or modified peptides derived from a LCM protein that are differentially expressed in disease. Examples of peptide modifications include, but are not limited to, substitutions, deletions, or additions of one or more amino acids in a given immunogenic peptide sequence, or mutation of existing amino acids within a given immunogenic peptide sequence, or derivatization of existing amino acids within a given immunogenic peptide sequence. Any amino acid in an immunogenic peptide sequence may be modified. In some aspects disclosed herein, at least one amino acid can be substituted or replaced within the given immunogenic peptide sequence. Any amino acid may be used to substitute or replace a given amino acid within the immunogenic peptide sequence. Modified peptides can include any immunogenic peptide obtained from differentially expressed proteins, which has been modified and exhibits enhanced binding to the MHC molecule with which it associates when presented to a T-cell. These modified peptides can be synthetically or recombinantly produced by conventional methods, for example.

In certain aspects disclosed herein, the peptides comprise, or consist of, sequences of about 5-30 amino acids in length which are immunogenic (i.e., capable of inducing an immune response when injected into a individual).

In certain aspects of the disclosure, the peptides may be used, for example, to treat T cell-mediated pathologies. The term "T cell-mediated pathologies" refers to any condition in which an inappropriate T cell response is a component of the pathology. The term is intended to encompass both T cell mediated diseases and diseases resulting from unregulated clonal T cell replication.

Modified (e.g., recombinant) or natural LCM proteins, or fragments thereof, can be used as a vaccine either prophylactically or therapeutically. When provided prophylactically, a vaccine can be provided in advance of any evidence of disease. The prophylactic administration of a disease vaccine may serve to prevent or attenuate a disease in a mammal such as a human.

An exemplary vaccine formulation can comprise an immunogen that induces an immune response directed against a disease-associated antigen such as a LCM protein. For example, a substantially or partially purified LCM protein or fragments thereof can be administered as a vaccine in a pharmaceutically acceptable carrier. An immunogen can be administered in a pure or substantially pure form, or can be administered as a pharmaceutical composition, formulation, or preparation. Exemplary doses of protein that can be administered are about 0.001 to about 100 mg per patient, or about 0.01 to about 100 mg per patient. Immunization can be repeated as necessary until a sufficient titer of anti-immunogen antibody or immune cells has been obtained.

Vaccine can be prepared using, for example, recombinant protein or expression vectors comprising a nucleic acid sequence encoding all or part of a LCM protein. Examples of vectors that can be used in vaccines include, but are not limited to, defective retroviral vectors, adenoviral vectors vaccinia viral vectors, fowl pox viral vectors, or other viral vectors (Mulligan, R. C., (1993) Science 260:926-932). The vectors can be introduced into a mammal (e.g., a human) either prior to any evidence of a disease or to mediate regression of a disease in a mammal afflicted with the disease. Examples of methods for administering a viral vector into mammals include, but are not limited to, exposure of cells to the virus *ex vivo,* or injection of the retrovirus or a producer cell line of the virus into the affected tissue, or intravenous administration of the virus. Alternatively, the vector can be administered locally by direct injection into a disease lesion or topical application in a pharmaceutically acceptable carrier. The quantity of viral vector to be administered can be based on the titer of virus particles. An exemplary range can be about 10⁶ to about 10¹¹ virus particles per mammal.

After immunization, the efficacy of the vaccine can be assessed by, for example, the production of antibodies or immune cells that recognize the antigen, as assessed by specific lytic activity, specific cytokine production, or disease regression, which can be measured using conventional methods. If the mammal to be immunized is already afflicted with a disease, the vaccine can be administered in conjunction with other therapeutic treatments. Examples of other therapeutic treatments include, but are not limited to, adoptive T cell immunotherapy and coadministration of cytokines or other therapeutic drugs.

In certain aspects of the disclosure, mammals, preferably humans at high risk for disease, especially cancer, are prophylactically treated with vaccines disclosed herein. Examples include, but are not limited to, individuals with a family history of a disease, individuals who themselves have a history of disease (e.g., cancer that has been previously resected and at risk for reoccurrence), or individuals already afflicted with a disease. When provided therapeutically, a vaccine can be provided to enhance the patient's own immune response to a disease antigen. An exemplary vaccine, which acts as an immunogen, can be a cell, cell lysate from cells transfected with a recombinant expression vector, or a culture supernatant containing the expressed protein, for example. Alternatively, an immunogen can be, for example, a partially or substantially purified recombinant protein, peptide, or analog thereof, or a modified protein, peptide, or analog thereof. The proteins or peptides can be, for example, conjugated with lipoprotein or administered in liposomal form or with adjuvant.

Vaccination can be carried out using conventional methods. For example, an immunogen can be used in a suitable diluent such as saline or water, or complete or incomplete adjuvants. Further, an immunogen may or may not be bound to a carrier, including carriers to increase the immunogenicity of the immunogen. Examples of carrier molecules include, but are not limited to, bovine serum albumin (BSA), keyhole limpet hemocyanin (KLH), tetanus toxoid, and the like. An immunogen also may be coupled with lipoproteins or administered in liposomal form or with adjuvants. An immunogen can be administered by any route appropriate for antibody production such as intravenous, intraperitoneal, intramuscular, subcutaneous, and the like. An immunogen can be administered once or at periodic intervals until a significant titer of anti-LCM immune cells or anti-LCM antibody is produced. The presence of anti-LCM immune cells can be assessed by measuring the frequency of precursor CTL (cytotoxic T-lymphocytes) against LCM antigen prior to and after immunization by a CTL precursor analysis assay (Coulie et al., 1992, International Journal Of Cancer 50:289-297). An immunoassay can be used to detect antibody in serum.

The safety of a vaccine can be determined by examining the effect of immunization on the general health of an immunized animal (e.g., weight change, fever, change in appetite or behavior, etc.) and looking for pathological changes during autopsies. After initial testing in animals, a vaccine can be tested in patients having a disease of interest. Conventional methods can be used to evaluate the immune response of a patient to determine the efficiency of the vaccine.

An disclosed herein, a LCM protein or fragments thereof, or a modified LCM protein, can be exposed to dendritic cells cultured *in vitro.* The cultured dendritic cells provide a means of producing T-cell dependent antigens comprised of dendritic cell-modified antigen or dendritic cells pulsed with antigen, in which the antigen is processed and expressed on the antigen-activated dendritic cell. The antigen-activated dendritic cells or processed dendritic cell antigens can be used as immunogens for vaccines or for the treatment of diseases. The dendritic cells can be exposed to the antigen for sufficient time to allow the antigens to be internalized and presented on the surface of dendritic cells. The resulting dendritic cells or the dendritic cell-processed antigens can then be administered to an individual in need of therapy. Such methods are described in Steinman et al. (WO93/208185) and in Banchereau et al. (EPO Application 0563485A1).

As disclosed herein, T-cells isolated from individuals can be exposed to a LCM protein or fragment thereof, or a modified LCM protein, *in vitro* and then administered in a therapeutically effective amount to a patient in need of such treatment. Examples of where T-lymphocytes can be isolated include, but are not limited to, peripheral blood cells lymphocytes (PBL), lymph nodes, or tumor infiltrating lymphocytes (TIL). Such lymphocytes can be isolated from the individual to be treated or from a donor by methods known in the art and cultured *in vitro* (Kawakami et al., 1989, J. Immunol. 142: 2453-3461). Lymphocytes can be cultured in media such as RPMI or RPMI 1640 or AIM V for 1-10 weeks. Viability can be assessed by trypan blue dye exclusion assay. Examples of how these sensitized T-cells can be administered to a mammal include, but are not limited to, intravenously, intraperitoneally, or intralesionally. Parameters that can be assessed to determine the efficacy of these sensitized T-lymphocytes include, but are not limited to, production of immune cells in the mammal being treated or tumor regression. Conventional methods can be used to assess these parameters. Such treatment can be given in conjunction with cytokines or gene-modified cells, for example (Rosenberg et al., 1992, Human Gene Therapy, 3: 75-90; Rosenberg et al., 1992, Human Gene Therapy, 3: 57-73).

### 9. Screening Methods Using LCM Proteins

The present disclosure describes methods of screening for agents (interchangeably referred to by such terms as candidate agents, compounds, or candidate compounds) that modulate LCM protein activity (interchangeably referred to as protein function). Examples of candidate agents include, but are not limited to, proteins, peptides, antibodies, nucleic acids (such as antisense and RNAi nucleic acid molecules), and small molecules. Further aspects disclosed herein are agents identified by these screening methods, and methods of using these agents, such as for treating diseases, especially cancer, and particularly lung cancer.

Exemplary screening methods can typically comprise the steps of (i) contacting a LCM protein with a candidate agent, and (ii) assaying for LCM protein activity, wherein a change in protein activity in the presence of the agent relative to protein activity in the absence of the agent indicates that the agent modulates LCM protein activity.

Other exemplary screening methods can determine a candidate agent's ability to modulate LCM expression. Exemplary methods can typically comprise the steps of (i) contacting a candidate agent with a system that is capable of expressing LCM protein or LCM mRNA, and (ii) assaying for the level of LCM protein or LCM mRNA, wherein a change in the level in the presence of the agent relative to the level in the absence of the agent indicates that the agent modulates LCM expression levels.

Disclosed herein are methods to screen for agents that bind to LCM proteins. Exemplary methods can typically comprise the steps of contacting a LCM protein with a test agent and measuring the extent of binding of the agent to the LCM protein.

LCM proteins can be used to identify agents that modulate activity of a protein in its natural state or an altered form that causes a specific disease or pathology. LCM proteins and appropriate variants and fragments can be used in high-throughput screens to assay candidate compounds for their ability to bind to LCM. These compounds can be further screened against functional LCM proteins to determine the effect of the compound on the protein's activity. Further, these compounds can be tested in animal or invertebrate systems to determine activity/effectiveness. Compounds can be identified that activate (agonist) or inactivate (antagonist) LCM proteins to a desired degree.

LCM proteins can be used to screen agents for their ability to stimulate or inhibit interaction between a LCM protein and a target molecule that normally interacts with the LCM protein (e.g., a substrate, an extracellular binding ligand, or a component of a signal pathway that a LCM protein normally interacts with such as a cytosolic signal protein). Exemplary assays can include the steps of combining a LCM protein or fragment thereof with a candidate compound under conditions that allow the LCM protein (or fragment thereof) to interact with a target molecule, and detecting the formation of a complex between the LCM protein and the target molecule or detecting the biochemical consequence of the interaction between the LCM protein and the target molecule, such as any of the associated effects of signal transduction (e.g., protein phosphorylation, cAMP turnover, adenylate cyclase activation, etc.). Any of the biological or biochemical functions mediated by a LCM protein can be used as an endpoint assay to identify an agent that modulates LCM activity.

Candidate compounds or agents include, but are not limited to, 1) peptides such as soluble peptides, including Ig-tailed fusion peptides and members of random peptide libraries (see, e.g., Lam et al., Nature 354:82-84 (1991); Houghten et al., Nature 354:84-86 (1991)) and combinatorial chemistry-derived molecular libraries made of D- and/or L-configuration amino acids; 2) phosphopeptides (e.g., members of random and partially degenerate, directed phosphopeptide libraries, see, e.g., Songyang et al., Cell 72:767-778 (1993)): 3) antibodies (e.g., polyclonal, monoclonal, humanized, anti-idiotypic, chimeric, and single chain antibodies as well as Fab, F(ab')₂, Fab expression library fragments, and epitope-binding fragments of antibodies); and 4) small organic and inorganic molecule (e.g.. molecules obtained from combinatorial and natural product libraries).

An exemplary candidate compound or agent is a soluble fragment of a LCM that competes for substrate binding. Other exemplary candidate compounds include mutant LCM proteins or appropriate fragments containing mutations that affect LCM function and thus compete for substrate. Accordingly, a fragment that competes for substrate, for example with a higher affinity, or a fragment that binds substrate but does not allow release, is also disclosed herein.

Compounds can also be screened by using chimeric proteins in which any portion of a protein such as an amino terminal extracellular domain, a transmembrane domain (e.g., transmembrane segments or intracellular or extracellular loops), or a carboxy terminal intracellular domain can be replaced in whole or part by heterologous domains or subregions. For example, a substrate-binding region can be used that interacts with a different substrate than the substrate that is recognized by a native marker protein. Accordingly, a different set of signal transduction components can be available as an endpoint assay for activation, thereby allowing assays to be performed in other than the specific host cell from which a marker is derived.

Competition binding assays can also be used to screen for compounds that interact with a marker protein (e.g., binding partners and/or ligands). For example, a test compound can be exposed to a marker protein under conditions that allow the test compound to bind or otherwise interact with the marker protein. Soluble marker protein can also be added to the mixture. If the test compound interacts with the soluble marker protein, it can decrease the amount of complex formed or activity of the marker protein. This type of assay is particularly useful in instances in which compounds are sought that interact with specific regions of a marker protei n. Thus, the soluble marker protein that competes with the marker protein can contain peptide sequences corresponding to the marker region of interest.

To perform cell-free drug screening assays, it may be desirable to immobilize either a LCM protein (or fragment thereof) or a molecule that binds the LCM protein (referred to herein as a "binding partner") to facilitate separation of complexes from uncompleted forms, as well as to facilitate automation of the assays.

Techniques for immobilizing proteins on matrices can be utilized in exemplary drug screening assays. For example, a fusion protein can be provided which adds a domain that allows a protein to be bound to a matrix. For example, glutathione-S-transferase fusion proteins can be adsorbed onto glutathione SEPHAROSE beads (Sigma Chemical, St. Louis, MO) or glutathione derivatized microtitre plates, which are then combined with cell lysates (e.g., ³⁵S-labeled) and a candidate compound, and the mixture incubated under conditions conducive to complex formation (e.g.. at physiological conditions for salt and pH). Following incubation, the beads can be washed to remove any unbound label, and the matrix immobilized and radiolabel determined directly, or in the supernatant after the complexes are dissociated. Alternatively, the complexes can be dissociated from the matrix, separated by SDS-PAGE, and the level of a binding partner found in the bead fraction quantitated from the gel using standard electrophoretic techniques. For example, either a marker protein or a binding partner can be immobilized by conjugation of biotin and streptavidin using techniques well known in the art. Alternatively, antibodies that are reactive with a marker protein but do not interfere with binding of the marker protein to its binding partner can be derivatized to the wells of a plate, and the marker protein trapped in the wells by antibody conjugation. Preparations of a binding partner and a candidate compound can be incubated in marker protein-presenting wells and the amount of complex trapped in the well can be quantitated. Methods for detecting such complexes, in addition to those described for GST-immobilized complexes, include immunodetection of complexes using antibodies reactive with a binding partner, or which are reactive with a marker protein and compete with the binding partner, as well as marker protein-linked assays which rely on detecting an enzymatic activity associated with a binding partner.

In a further aspect of the present disclosure, a LCM protein can be used as a "bait protein" in a two-hybrid assay or three-hybrid assay (see, e.g., U.S. Patent No. 5,283,317; Zervos et al. (1993) Cell 72:223-232; Madura et al. (1993) J. Biol. Chem. 268:12046-12054: Bartel et al. (1993) Biotechniques 14:920-924; Iwabuchi et al. (1993) Oncogene 8:1693-1696; and Brent WO94/10300), to identify other proteins, which bind to or interact with a LCM protein and are involved in the protein's activity. The two-hybrid system is based on the modular nature of most transcription factors, which typically consist of separable DNA-binding and activation domains. For example, the two-hybrid assay can utilize two different DNA constructs. In one construct, a gene that encodes a LCM protein can be fused to a gene encoding the DNA binding domain of a known transcription factor (e.g., GAL-4). In the other construct, a DNA sequence from a library of DNA sequences that encode an unidentified protein ("prey" or "sample") can be fused to a gene that encodes the activation domain of the known transcription factor. If the "bait" and the "prey" proteins are able to interact *in vivo,* forming a LCM-dependent complex, the DNA-binding and activation domains of the transcription factor are brought into close proximity. This proximity allows transcription of a reporter gene (e.g., LacZ), which can be operably linked to a transcriptional regulatory site responsive to the transcription factor. Expression of the reporter gene can be detected and cell colonies containing the functional transcription factor can be isolated and used to obtain the cloned gene that encodes the protein that interacts with the LCM protein.

Agents that modulate a LCM protein can be identified using one or more of the above assays, alone or in combination. For example, a cell-based or cell free system can be used for initial identification of agents, and then activity of the agents can be confirmed in an animal or other model system. Such model systems are well known in the art and can readily be employed in this context.

### 10. Diagnosis, Treatment, and Screening Methods Using LCM Nucleic Acid Molecules

The nucleic acid molecules disclosed herein are useful, for example, as probes, primers, chemical intermediates, and in biological assays. The nucleic acid molecules are useful as hybridization probes for messenger RNA, transcript/cDNA, and genomic DNA to detect or isolate full-length cDNA and genomic clones encoding a LCM protein, or variants thereof. The nucleic acid molecules are also useful as primers for PCR to amplify any given region of a nucleic acid molecule and are useful to synthesize antisense molecules of desired length and sequence. The nucleic acid molecules are also useful for producing ribozymes corresponding to all, or a part, of the mRNA produced from the nucleic acid molecules described herein.

The nucleic acid molecule are also useful for constructing recombinant vectors. Exemplary vectors include expression vectors that express a portion of, or all of, a LCM protein. The nucleic acid molecules are also useful for expressing antigenic portions of the proteins. The nucleic acid molecules are also useful for constructing host cells expressing a part, or all, of the proteins. The nucleic acid molecule are also useful for constructing transgenic animals expressing all, or a part, of the proteins.

A primer or probe can correspond to any sequence along the entire length of a LCM-encoding nucleic acid molecule. Accordingly, a primer or probe can be derived from 5' noncoding regions, coding regions, or 3' noncoding regions, for example.

Exemplary *in vitro* techniques for detection of mRNA include Northern hybridizations and *in situ* hybridizations. Exemplary *in vitro* techniques for detecting DNA include Southern hybridizations and *in situ* hybridization. Reverse transcriptase PCR amplification (RT-PCR) and the like can also be used for detecting RNA expression. A specific exemplary method of detection comprises using TaqMan technology (Applied Biosystems, Foster City, CA).

### a. Methods of Diagnosis Using Nucleic Acids

Nucleic acid molecules disclosed herein are useful, for example, as hybridization probes for determining the presence, level, form, and/or distribution of nucleic acid expression. Exemplary probes can be used to detect the presence of, or to determine levels of, a specific nucleic acid molecule in cells, tissues, and in organisms. Accordingly, probes corresponding to a LCM described herein can be used to assess expression and/or gene copy number in a given cell, tissue, or organism, which can be applied to, for example, diagnosis of disorders involving an increase or decrease in LCM protein expression relative to normal LCM protein expression levels.

Probes can be used as part of a diagnostic test kit for identifying cells or tissues that express LCM protein differentially, such as by measuring a level of a LCM-encoding nucleic acid (e.g., mRNA or genomic DNA) in a sample of cells from a individual, or determining if a LCM-encoding nucleic acid is mutated.

Further, disclosed herein are kits for detecting the presence of LCM-encoding nucleic acid (e.g., mRNA or genomic DNA) in a biological sample. For example, an exemplary kit can comprise reagents such as a labeled or labelable nucleic acid or agent capable of detecting LCM nucleic acid in a biological sample; means for determining the amount of LCM nucleic acid in the sample; and means for comparing the amount of LCM nucleic acid in the sample with a standard. The compound or agent can be packaged in a suitable container. The kit can further comprise instructions for using the kit to detect LCM nucleic acid.

The nucleic acid molecules are useful in diagnostic assays for qualitative changes in LCM nucleic acid expression, and particularly in qualitative changes that lead to pathology. The nucleic acid molecules can be used to detect mutations in LCM genes and gene expression products such as mRNA. The nucleic acid molecules can be used as hybridization probes to detect naturally occurring genetic mutations in a LCM gene and to determine whether a individual with the mutation is at risk for a disorder caused by the mutation. Examples of mutations include deletions, additions, or substitutions of one or more nucleotides in a gene, chromosomal rearrangements (such as inversions or transpositions), and modification of genomic DNA such as aberrant methylation patterns or changes in gene copy number (such as amplification). Detection of a mutated form of a LCM gene associated with a dysfunction can provide a diagnostic tool for an active disease or susceptibility to disease in instances in which the disease results from overexpression, underexpression, or altered expression of a LCM protein, for example.

Mutations in a LCM gene can be detected at the nucleic acid level by a variety of techniques. For example, genomic DNA, RNA, or cDNA can be analyzed directly or can be amplified (e.g., using PCR) prior to analysis. For example, detection of a mutation involves the use of a probe/primer in a PCR reaction (see, e.g. U.S. Patent Nos. 4,683,195 and 4,683,202), such as anchor PCR or RACE PCR, or, alternatively, in a ligation chain reaction (LCR) (see, e.g., Landegran et al., Science 241:1077-1080 (1988) and Nakazawa et al., PNAS 91:360-364 (1994)), the latter of which can be particularly useful for detecting point mutations in a gene (see Abravaya et al., Nucleic Acids Res. 23:675-682 (1995)). Exemplary methods such as these can include the steps of collecting a sample of cells from a patient, isolating nucleic acid (e.g., genomic, mRNA, or both) from the cells of the sample, contacting the nucleic acid with one or more primers which specifically hybridize to a marker nucleic acid under conditions such that hybridization and amplification of the marker nucleic acid (if present) occurs, and detecting the presence or absence of an amplification product, or detecting the size of the amplification product and comparing the length to a control sample. Deletions and insertions can be detected by a change in size of the amplified product compared to a normal genotype. Point mutations can be identified by hybridizing amplified DNA to normal RNA or antisense DNA sequences, for example.

Alternatively, mutations in a LCM gene can be identified, for example, by alterations in restriction enzyme digestion patterns as determined by gel electrophoresis. Further, sequence-specific ribozymes (U.S. Patent No. 5.498,531) can be used to identify the presence of specific mutations by development or loss of a ribozyme cleavage site. Perfectly matched sequences can be distinguished from mismatched sequences by nuclease cleavage digestion assays or by differences in melting temperature.

Sequence changes at specific locations can be assessed by nuclease protection assays such as RNase and SI protection, or chemical cleavage methods. Furthermore, sequence differences between a mutant LCM gene and a corresponding wild-type gene can be determined by direct DNA sequencing. A variety of automated sequencing procedures can be utilized when performing diagnostic assays (Naeve, C.W., (1995) Biotechniques 19:448), including sequencing by mass spectrometry (e.g., PCT International Publication No. WO 94/16101; Cohen et al., Adv. Chromatogr. 36:127-162 (1996); and Griffin et al., Appl. Biochem. Biotechnol. 38:147-159 (1993)).

Other methods for detecting mutations in a nucleic acid include methods in which protection from cleavage agents is used to detect mismatched bases in RNA/RNA or RNA/DNA duplexes (Myers et al., Science 230:1242 (1985)); Cotton et al., PNAS 85:4397 (1988); Saleeba et al., Meth. Enzymol. 217:286-295 (1992)), electrophoretic mobility of mutant and wild type nucleic acid is compared (Orita et al., PNAS 86:2766 (1989); Cotton et al., Mutat. Res. 285:125-144 (1993); and Hayashi et al., Genet. Anal. Tech. Appl. 9:73-79 (1992)), and movement of mutant or wild-type fragments in polyacrylamide gels containing a gradient of denaturant is assayed using denaturing gradient gel electrophoresis (DGGE) (Myers et al., Nature 313:495 (1985)). Examples of other techniques for detecting point mutations include selective oligonucleotide hybridization, selective amplification, and selective primer extension.

### b. Methods of Monitoring Treatment and Pharmacogenomic Methods Using Nucleic Acids

Nucleic acid molecules disclosed herein are also useful for monitoring the effectiveness of modulating agents on the expression or activity of a LCM gene, such as in clinical trials or in a treatment regimen. For example, the gene expression pattern of a LCM gene can serve as a barometer for the continuing effectiveness of treatment with a compound, particularly with compounds to which a patient can develop resistance. The gene expression pattern can also serve as a marker indicative of a physiological response of the affected cells to the compound. For example, based on monitoring nucleic acid expression, the administration of a compound can be increased or alternative compounds to which the patient has not become resistant can be administered instead. Similarly, if the level of nucleic acid expression falls below a desirable level, administration of the compound can be commensurately decreased.

The nucleic acid molecules are also useful for testing an individual for a genotype that, while not necessarily causing a disease, nevertheless affects the treatment modality. Thus, the nucleic acid molecules can be used to study the relationship between an individual's genotype and the individual's response to a compound used for treatment (pharmacogenomic relationship). Accordingly, the nucleic acid molecules provided herein can be used to assess the mutation content of a marker gene in an individual in order to select an appropriate compound or dosage regimen for treatment. For example, marker nucleic acid molecules having genetic variations that affect treatment can provide diagnostic markers that can be used to tailor treatment to an individual. Accordingly, the production of recombinant cells and animals having these genetic variations allows effective clinical design of treatment compounds and dosage regimens, for example.

### c. Methods of Treatment Using Nucleic Acids

Nucleic acid molecules disclosed herein are useful to design antisense constructs to control LCM gene expression in cells, tissues, and organisms. An antisense nucleic acid molecule typically blocks translation of mRNA into LCM protein by hybridizing to marker mRNA in a sequence-specific manner. Nucleic acid molecules can also be used to specifically suppress gene expression by methods such as RNA interference (RNAi). RNAi and antisense-based gene suppression are well known in the art (e.g., Science 288:1370-1372, 2000). RNAi typically operates on a post-transcriptional level and is sequence specific. RNAi and antisense nucleic acid molecule are useful for treating diseases, especially cancer. RNAi fragments, particularly double-stranded (ds) RNAi, as well as antisense nucleic acid molecule can also be used to generate loss-of-function phenotypes by suppressing gene expression. Accordingly, disclosed herein are RNAi and antisense nucleic acid molecules, and methods of using these RNAi and antisense nucleic acid molecules, such as for therapy or for modulating cell function. Nucleic acid molecule may also be produced that are complementary to a region of a gene involved in transcription, such as to hybridize to the gene to prevent transcription.

Certain aspects of the disclosure relate to isolated RNA molecule (double-stranded; single-stranded) that are about 17 to about 29 nucleotides (nt) in length, and more particularly about 21 to about 25 nt in length, which mediate RNAi (e.g., degradation of mRNA, and such mRNA may be referred to herein as mRNA to be degraded). With respect to RNAi, the terms RNA, RNA molecule(s), RNA segment(s), and RNA fragment(s) are used interchangeably to refer to RNA that mediates RNAi. These terms include double-stranded RNA, single-stranded RNA, isolated RNA (e.g., partially purified RNA, essentially pure RNA, synthetic RNA, recombinantly produced RNA), as well as altered RNA that differs from naturally occurring RNA by the addition, deletion, substitution, and/or alteration of one or more nucleotides. Such alternations can include, for example, addition of non-nucleotide material, such as to the end(s) of a 21-25 nt RNA or internally (at one or more nucleotides of the RNA). Nucleotides in exemplary RNA molecules disclosed herein can also comprise non-standard nucleotides, including non-naturally occurring nucleotides or deoxyribonucleotides. Collectively, all such altered RNAs are referred to as analogs or analogs of naturally-occurring RNA. RNA of 21-25 nt typically need only be sufficiently similar to natural RNA that it has the ability to mediate RNAi. As used herein, the phrase "mediates RNAi" refers to the ability to distinguish which RNAs are to be degraded by RNAi processes. RNA that mediates RNAi directs degradation of particular mRNAs by RNAi processes. Such RNA may include RNAs of various structures, including short hairpin RNA.

In certain aspects, the present disclosure relates to RNA molecules of about 21 to about 25 nt that direct cleavage of specific mRNA to which their sequence corresponds. It is not necessary that there be a perfect correspondence (i.e., match) of the sequences, but the correspondence must be sufficient to enable the RNA to direct RNAi cleavage of the marker mRNA (Holen et al., Nucleic Acids Res. 33:4704-4710 (2005)). In an aspects of the disclosure, the 21-25 nt RNA molecules disclosed herein comprise a 3' hydroxyl group.

Certain aspects of the present disclosure relate to 21-25 nt RNAs of specific genes, produced by chemical synthesis or recombinant DNA techniques, that mediate RNAi. As used herein, the term "isolated RNA" includes RNA obtained by any means, including processing or cleavage of dsRNA, production by chemical synthetic methods, and production by recombinant DNA techniques, for example. Exemplary aspects of the disclosure relate to uses of the 21-25 nt RNAs, such as for therapeutic or prophylactic treatment and compositions comprising 21-25 nt RNAs that mediate RNAi, such as pharmaceutical compositions comprising 21-25 nt RNAs and an appropriate carrier.

Further aspects of the present disclosure relate to methods of mediating RNAi of genes of a patient. For example, RNA of about 21 to about 25 nt which targets a specific mRNA to be degraded can be introduced into a patient's cells. The cells can be maintained under conditions allowing degradation of the mRNA, resulting in RNA-mediated interference of the mRNA of the gene in the cells of the patient. Treatment of cancer patients, for example, with RNAi may inhibit the growth and spread of the cancer and reduce tumor size. Treatment of patients using RNAi can also be in combination with other therapies. For example. RNAi can be used in combination with other treatment modalities, such as chemotherapy, radiation therapy, and other treatments. In an aspects of the disclosure, a chemotherapy agent is used in combination with RNAi. In a further aspects of the disclosure, GEMZAR (gemcitabine HCl) chemotherapy is used with RNAi.

Treatment of certain diseases by RNAi may require introduction of the RNA into the disease cells. RNA can be directly introduced into a cell, or introduced extracellularly into a cavity, interstitial space, into the circulation of a patient, or introduced orally, for example. Physical methods of introducing nucleic acids, such as injection directly into a cell or extracellular injection into a patient, may also be used. RNA may be introduced into vascular or extravascular circulation, the blood or lymph system, or the cerebrospinal fluid, for example. RNA may be introduced into an embryonic stem cell or another multipotent cell, which may be derived from a patient. Physical methods of introducing nucleic acids include injection of a solution containing the RNA, bombardment by particles covered by the RNA, soaking cells or tissue in a solution of the RNA, or electroporation of cell membranes in the presence of the RNA. A viral construct packaged into a viral particle may be used to introduce an expression construct into a cell, with the construct expressing the RNA. Other methods known in the art for introducing nucleic acids to cells may be used, such as lipid-mediated carrier transport, chemical-mediated transport, and the like. The RNA may be introduced along with components that perform one or more of the following activities: enhance RNA uptake by the cell, promote annealing of the duplex strands, stabilize the annealed strands, or otherwise increase inhibition of the marker gene.

Exemplary RNA disclosed herein can be used alone or as a component of a kit having at least one reagent for carrying out *in vitro* or *in vivo* introduction of the RNA to a cell, tissue/fluid, or patient. Exemplary components of a kit include dsRNA and a vehicle that promotes introduction of the dsRNA. A kit may also include instructions for using the kit.

Certain exemplary aspects of the present disclosure describe compositions and methods for cleavage of mRNA by ribozymes having nucleotide sequences complementary to one or more regions in the mRNA, thereby attenuating the translation of the mRNA. Examples of regions in mRNA that can be targeted by ribozymes include coding regions, particularly coding regions corresponding to catalytic or other functional activities of a marker protein, such as substrate binding. These compositions and methods may be used to treat a disorder characterized by abnormal or undesired marker nucleic acid expression. the

In certain exemplary aspects of the disclosure, nucleic acid molecules disclosed herein may be used for gene therapy in individuals having cells that are aberrant in gene expression of a marker. For example, recombinant cells that have been engineered *ex vivo* (which can include an individual's own cells) can be introduced into an individual where the cells produce the desired marker protein to thereby treat the individual.

### d. Methods of Screening Using Nucleic Acids

Nucleic acid expression assays are useful for drug screening to identify compounds that modulate LCM nucleic acid expression.

The present disclosure thus describes methods for identifying a compound that can be used to treat a disease associated with differential expression of a LCM gene, especially cancer. Exemplary methods can typically include assaying the ability of a compound to modulate the expression of a marker nucleic acid to thereby identify a compound that can be used to treat a disorder characterized by undesired marker nucleic acid expression. The assays can be performed in cell-based or cell-free systems. Examples of cell-based assays include cells naturally expressing marker nucleic acid or recombinant cells genetically engineered to express specific marker nucleic acid sequences.

Assays for marker nucleic acid expression can involve direct assay of marker nucleic acid levels, such as mRNA levels, or on collateral compounds involved in a signal pathway. Further, the expression of genes that are up- or down-regulated in response to a signal pathway can also be assayed. The regulatory regions of these genes can be operably linked to a reporter gene such as luciferase.

Thus, modulators of gene expression of a marker can be identified in methods wherein a cell is contacted with a candidate agent and the expression of marker mRNA determined. The level of expression of marker mRNA in the presence of the candidate agent is compared to the level of expression of marker mRNA in the absence of the candidate agent. The candidate agent can then be identified as a modulator of marker nucleic acid expression based on this comparison and may be used, for example, to treat a disorder characterized by aberrant marker nucleic acid expression. When expression of marker mRNA is statistically significantly greater in the presence of the candidate agent than in its absence, the candidate agent is identified as a stimulator (agonist) of nucleic acid expression. When nucleic acid expression is statistically significantly less in the presence of the candidate agent than in its absence, the candidate compound is identified as an inhibitor (antagonist) of nucleic acid expression.

### 11. Arrays and Expression Analysis

"Array" (interchangeably referred to as "microarray") typically refers to an arrangement of at least one, but more typically at least two, nucleic acid molecules, proteins, or antibodies on a substrate. In certain exemplary arrangements, at least one of the nucleic acid molecules, proteins, or antibodies typically represents a control or standard, and other nucleic acid molecules, proteins, or antibodies are of diagnostic or therapeutic interest. In exemplary aspects disclosed herein, the arrangement of nucleic acid molecules, proteins, or antibodies on the substrate is such that the size and signal intensity of each labeled complex (e.g., formed between each nucleic acid molecule and a complementary nucleic acid, or between each protein and a ligand or antibody, or between each antibody and a protein to which the antibody specifically binds) is individually distinguishable.

An "expression profile" is a representation of marker expression in a samples. A nucleic acid expression profile can be produced using, for example, arrays, sequencing, hybridization, or amplification technologies for nucleic acids from a sample. A protein expression profile can be produced using, for example, arrays, gel electrophoresis, mass spectrometry, or antibodies (and, optionally, labeling moieties) which specifically bind proteins. Nucleic acids, proteins, or antibodies can be attached to a substrate or provided in solution, and their detection can be based on methods well known in the art.

A substrate includes, but is not limited to, glass, paper, nylon or other type of membrane, filter, chip, metal, or any other suitable solid or semi-solid (e.g., gel) support.

Exemplary arrays can be prepared and used according to the methods described in U.S. Patent No. 5,837,832; PCT application WO95/11995; Lockhart et al., 1996, Nat. Biotech. 14: 1675-1680; Schena et al., 1996; Proc. Natl. Acad. Sci. 93: 10614-10619; and U.S. Patent No. 5,807,522. Described herein are antibody arrays (see, e.g., de Wildt et al. (2000) Nat. Biotechnol. 18:989-94).

Certain aspects of the present disclosure describe a nucleic acid array for assaying marker expression, which can be composed of single-stranded nucleic acid molecules, usually either synthetic antisense oligonucleotides or fragments of cDNAs, fixed to a solid support. The oligonucleotides can be, for example, about 6-60 nucleotides in length, about 15-30 nucleotides in length, or about 20-25 nucleotides in length.

To produce oligonucleotides to a marker nucleic acid molecule for an array, the marker nucleic acid molecule of interest is typically examined using a computer algorithm to identify oligonucleotides of defined length that are unique to the nucleic acid molecule, have a GC content within a range suitable for hybridization, and lack predicted secondary structure that may interfere with hybridization. In certain instances, it may be desirable to use pairs of oligonucleotides on an array. In another aspects disclosed herein, the "pairs" can be identical, except for one nucleotide (which can be located in the center of the sequence, for example). The second oligonucleotide in the pair (mismatched by one) serves as a control. Any number of oligonucleotide pairs may be utilized.

Oligonucleotides can be synthesized on the surface of a substrate, such as by using a light-directed chemical process or by using a chemical coupling procedure and an ink jet application apparatus (e.g. PCT application WO95/251116).

In some aspects disclosed herein, an array can be used to diagnose or monitor the progression of disease, for example, by assaying marker expression.

For example, an oligonucleotide probe specific for a marker can be labeled by standard methods and added to a biological sample from a patient under conditions that allow for the formation of hybridization complexes. After an incubation period, the sample can be washed and the amount of label (or signal) associated with hybridization complexes can be quantified and compared with a standard value. If complex formation in the patient sample is significantly altered (higher or lower) in comparison to a normal (e.g., healthy) standard, or is similar to a disease standard, this differential expression can be diagnostic of a disorder.

By analyzing changes in patterns of marker expression, disease may be diagnosed at earlier stages before a patient is symptomatic. In exemplary aspects of the present disclosure, arrays or marker expression analysis methods can be used to formulate a diagnosis or prognosis, to design a treatment regimen, and/or to monitor the efficacy of treatment. For example, a treatment dosage can be established that causes a change in marker expression patterns indicative of successful treatment, and marker expression patterns associated with the onset of undesirable side effects can be avoided. In further aspects of the disclosure, assays of marker expression can be repeated on a regular basis to determine if the level of marker expression in a patient begins to approximate that which is observed in a normal individual. The results obtained from successive assays may be used to show the efficacy of treatment over a period ranging from several days to years, for example.

The arrays disclosed herein can also be used to screen candidate agents, such as to identify agents that produce a marker expression profile similar to that caused by known therapeutic agents, with the expectation that agents that cause a similar expression profile of a marker may have similar therapeutic effects and/or modes of action on the marker.

### EXAMPLES

Exemplary embodiments of the invention are further described in the following examples, which serve the purpose of reference only.

### 1. Tissue Samples and Cell Lines

### Tissue Processing and Preparation of Single Cell Suspensions from Tissue

Tissue samples (e.g., normal tissues or disease tissues such as surgically resected neoplastic or metastatic lesions) can be procured from clinical sites and transported in transport buffer. Tissues can be collected as remnant tissues following surgical resection of cancer (or other disease) tissues. Remnant tissues are supplied following processing for pathological diagnosis according to proper standards of patient care. Normal tissue specimens can be normal tissue adjacent to tumors (or other disease tissue) that is collected during tumor resection. Normal tissue from healthy patients not having cancer (or other disease of interest) can also be included, such as to reduce the contribution from pre-neoplastic changes that may exist in normal adjacent tissue. Procurement of tissue samples is carried out in an anonymous manner in compliance with federally mandated ethical and legal guidelines (HIPAA) and in accordance with clinical institution ethical review board and internal institutional review board guidelines.

Tissue can be crudely minced and incubated for 20-30 minutes with periodic agitation at 37°C in Enzyme Combination #1 (200 units collagenase, cat# C5894 Sigma; 126 µg DNAse I, cat#D4513 Sigma (in 10 mM Tris/HCl pH7.5); 50 mM NaCl; 10 mM MgCl2; 0.05% elastase, cat# E7885 Sigma) (additionally, hyaluronidase enzyme may also be utilized). D-PBS is added at 3x the volume of the enzyme combination, the tissue finely minced, and disassociated cells passed through a 200 µm filter. The cells are washed twice with D-PBS. Red blood cells are lysed with PharMLyse (BD Biosciences) when necessary. Cell number and viability are determined by PI exclusion (GUAVA). Cells at a total cell number greater than 20 x 10⁶ are sorted using a high-speed sorter (MoFlo Cytomation) for epithelial cells (EpCAM positive).

The remaining undigested tissue is incubated for 20-30 minutes with periodic agitation at 37°C in Enzyme Combination #2 (1X Liberase Blendzyme 1, cat# 988-417 Roche; 1X Liberase Blendzyme 3, cat#814-184 Roche; 0.05% elastase, cat# E7885 Sigma). D-PBS is added at 3x the volume of the enzyme combination, and the tissue finely minced until tissue is completely disassociated. The cells are passed through a 200 µm filter, washed twice with D-PBS, and pooled with cells from the Enzyme Combination #1 digestion.

Cells are passed through a 70 µm filter for single cell suspension, and cell number and viability are determined by PI exclusion (GUAVA). When needed, red blood cells are lysed with PharMLyse (BD Biosciences). Cells are incubated in 20 ml of 1X PharMLyse in D-PBS for 30 seconds with gentle agitation and cells pelleted at 300xg for 5 minutes at 4°C. Cells are washed once in D-PBS and cell number and viability are recalculated by PI exclusion using the GUAVA. Cells at a total cell number greater than 20 x 10⁶ are sorted using a high-speed sorter (MoFlo Cytomation) for epithelial cells (EpCAM positive).

Single cell suspensions can also be prepared from tissue samples as follows: specimens are washed in DTT for 15 min, digested with Dispase (30-60 min), then filtered twice (380 µm/74 µm) before red blood cells are removed through addition of ACK lysis buffer. Epithelial (EpCAM) and leukocyte (CD45) content and cellular viability (PI exclusion) can be determined through flow cytometry analysis (LSR I, BD Biosciences, San Jose, CA).

The epithelial content of both disease and normal specimens can be enriched through depletion of immune CD45-positive cells by flow cytometry or purification of Epithelial Cell Surface Antigen (ECSA/EpCam)-positive cells by bead capture.

Bead capture of epithelial cells can be performed using a Dynal CELLection Epithelial Enrich kit (Invitrogen, Carlsbad, CA) as follows. Dynal CELLection beads at a concentration of 2x10⁸ beads are incubated with 1x10⁸ cells in HBSS with 10% fetal calf serum for 30 minutes at 4°C. Cells and beads are placed in a magnet system Dynal MPC for 2 minutes. Bead/cell complexes are washed in RPMI 1640 media with 1% fetal calf serum. Cells are released from the bead complex with 15 minute incubation with DNase with agitation in RPMI with 1% fetal calf serum.

DynalBead cell depletion of CD45 cells can be carried out as follows. DynalBead M-450 CD45 beads and cells are incubated at a concentration of 250 µl beads per 2x10⁷ cells for 30 minutes at 4°C. Bead/cell complexes are washed in DPBS buffer with 2% fetal bovine serum. Cells and beads are placed in a magnet system Dynal MPC for 2 minutes. The supernatant contains EpCAM enriched cells.

### Cell Line Culture

Cell lines can be obtained from the American Type Culture Collection (ATCC, Manassas, VA). For example, lung cancer cell lines and normal control lung cell lines (e.g., Beas2B cells can be used as a normal control lung cell line) can be used, such as to determine the expression levels of markers (e.g., proteins or encoding mRNA transcripts) in lung cancer cells compared with normal lung cells. Cell lines can be grown in a culturing medium that is supplemented as necessary with growth factors and serum, in accordance with the ATCC guidelines for each particular cell line. Cultures are established from frozen stocks in which the cells are suspended in a freezing medium (cell culture medium with 10% DMSO [v/v]) and flash frozen in liquid nitrogen. Frozen stocks prepared in this way are stored in liquid nitrogen vapor. Cell cultures are established by rapidly thawing frozen stocks at 37°C. Thawed stock cultures are slowly transferred to a culture vessel containing a large volume of supplemented culture medium. For maintenance of culture, cells are seeded at 1x10⁵ cells/per ml in medium and incubated at 37°C until confluence of cells in the culture vessel exceeds 50% by area. At this time, cells are harvested from the culture vessel using enzymes or EDTA where necessary. The density of harvested, viable cells is estimated by hemocytometry and the culture reseeded as above. A passage of this nature is repeated no more than 25 times, at which point the culture is destroyed and reestablished from frozen stocks as described above.

Alternatively, for secreted protein analysis, cells can be grown under routine tissue culture conditions in 490 cm² roller bottles at an initial seeding density of approximately 15 million cells per roller bottle. When the cells reach ∼70-80% confluence, the culturing media is removed, the cells are washed 3 times with D-PBS and once with CD293 protein-free media (Invitrogen cat# 11913-019), and the culturing media is replaced with CD293 for generating conditioned media. Cells are incubated for 72 hours in CD293 and the media is collected for analysis, such as mass spectrometry analysis of secreted proteins (30-300 ml). Cell debris is removed from the conditioned media by centrifugation at 300g for 5 minutes and filtering through a 0.2 micron filter prior to analysis.

### 2. Cloning and Expression of Marker Proteins

### cDNA Retrieval

Peptide sequences can be searched using the BLAST algorithm against relevant protein sequence databases to identify the corresponding full-length protein (reference sequence). Each full-length protein sequence can then be searched using the BLAST algorithm against a human cDNA clone collection. For each sequence of interest, clones can be pulled and streaked onto LB/Ampicillin (100 µg/ml) plates. Plasmid DNA is isolated using Qiagen spin mini-prep kit and verified by restriction digest. Subsequently, the isolated plasmid DNA is sequence verified against the reference full-length protein sequence. Sequencing reactions are carried out using Applied Biosystems BigDye Terminator kit followed by ethanol precipitation. Sequence data is collected using the Applied Biosystems 3700 Genetic Analyzer and analyzed by alignment to the reference full-length protein sequence using the Clone Manager alignment tool.

### PCR

PCR primers are designed to amplify the region encoding the full-length protein and/or any regions of the protein that are of interest for expression (e.g., antigenic or hydrophilic regions as determined by the Clone Manager sequence analysis tool). Primers also contain 5' and 3' overhangs to facilitate cloning (see below). PCR reactions contain 2.5 units Platinum Taq DNA Polymerase High Fidelity (Invitrogen), 50 ng cDNA plasmid template, 1 µM forward and reverse primers, 800 µM dNTP cocktail (Applied Biosystems), and 2 mM MgSO₄. After 20-30 cycles (94°C for 30 seconds, 55°C for I minute, and 73°C for 2 minutes), the resulting product is verified by sequence analysis and quantitated by agarose gel electrophoresis.

### Construction of Entry Clones

PCR products are cloned into an entry vector for use with the Gateway recombination based cloning system (Invitrogen). These vectors include pDonr221, pDonr201, pEntr/D-TOPO, or pEntr/SD/D-TOPO and are used as described in the cloning methods below.

### TOPO Cloning into pEntr/D-TOPO or pEntr/SD/D-TOPO

For cloning using this method, the forward PCR primer contains a 5' overhang containing the sequence "CACC". PCR products are generated as described above and cloned into the entry vector using the Invitrogen TOPO^{®} cloning kit. Reactions are typically carried out at room temperature for 10 minutes and subsequently transformed into TOP 10 chemically competent cells (Invitrogen, CA). Candidate clones are picked, and plasmid DNA is prepared using a Qiagen spin mini-prep kit and screened by restriction enzyme digestion. Inserts are subsequently sequence-verified as described above.

### Gateway Cloning into pDonr201 or pDonr221

For cloning using this method, PCR primers contain forward and reverse 5' overhangs. PCR products are generated as described above. Protein-encoding nucleic acid molecules are recombined into the entry vector using the Invitrogen Gateway BP Clonase enzyme mix. Reactions are typically carried out at 25°C for 1 hour, treated with Proteinase K at 37°C for 10 minutes, and transformed into Library Efficiency DH5α chemically competent cells (Invitrogen, CA). Candidate clones are picked, plasmid DNA is prepared using a Qiagen spin mini-prep kit, and screened by restriction enzyme digestion. Inserts are subsequently sequence-verified as described above.

### Construction of Expression Clones

Protein-encoding nucleic acid molecules are transferred from the entry construct into a series of expression vectors using the Gateway LR Clonase enzyme mix. Reactions are typically carried out for 1 hour at 25°C, treated with Proteinase K at 37°C for 10 minutes, and subsequently transformed into Library Efficiency DH5a chemically competent cells (Invitrogen). Candidate clones are picked, plasmid DNA is prepared using a Qiagen spin mini-prep kit, and screened by restriction enzyme digestion. Expression vectors include, but are not limited to, pDest14, pDest15, pDest17, pDest8, pDest10 and pDest20. These vectors allow expression in systems such as *E*. *coli* and recombinant baculovirus. Other vectors not listed here allow expression in yeast, mammalian cells, or *in vitro.*

### Expression of Recombinant Proteins in E. coli

Constructs are transformed into one or more of the following host strains: BL21 SI, BL21 AI, (Invitrogen), Origami B (DE3), Origami B (DE3) pLysS, Rosetta (DE3), Rosetta (DE3) pLysS, Rosetta-Gami (DE3), Rosetta-Gami (DE3) pLysS, or Rosetta-Gami B (DE3) pLysS (Novagen). The transformants are grown in LB with or without NaCl and with appropriate antibiotics, at temperatures in the range of 20-37°C, with aeration. Expression is induced with the addition of IPTG (0.03-0.30 mM) or NaCl (75-300 mM) when the cells are in mid-log growth. Growth is continued for one to 24 hours post-induction. Cells are harvested by centrifugation in a Sorvall RC-3C centrifuge in a H6000A rotor for 10 minutes at 3000 rpm at 4°C. Cell pellets are stored at -80°C.

### Expression of Recombinant Proteins using Baculovirus

Recombinant proteins are expressed using baculovirus in Sf21 fall army worm ovarian cells. Recombinant baculoviruses are prepared using the Bac-to-Bac system (Invitrogen) per the manufacturer's instructions. Proteins are expressed on the large scale in Sf900II serum-free medium (Invitrogen) in a 10 L bioreactor tank (27°C, 130 rpm, 50% dissolved oxygen for 48 hours).

### 3. Recombinant Protein Purification

Recombinant proteins can be purified from *E. coli* and/or insect cells using a variety of standard chromatography methods. Briefly, cells are lysed using sonication or detergents, The insoluble material is pelleted by centrifugation at 10,000Xg for 15 minutes. The supernatant is applied to an appropriate affinity column. For example, His-tagged proteins are separated using a pre-packed chelating sepharose column (Pharmacia) or GST-tagged proteins are separated using a glutathione sepharose column (Pharmacia). After using the affinity column, proteins are further separated using various techniques, such as ion exchange chromatography (columns from Pharmacia) to separate on the basis of electrical charge or size exclusion chromatography (columns from Tosohaas) to separate on the basis of molecular weight, size, and shape.

Expression and purification of the protein can also be achieved using either a mammalian cell expression system or an insect cell expression system. The pUB6/V5-His vector system (Invitrogen, CA) can be used to express cDNA in CHO cells. The vector contains the selectable bsd gene, multiple cloning sites, the promoter/enhancer sequence from the human ubiquitin C gene, a C-terminal V5 epitope for antibody detection with anti-V5 antibodies, and a C-terminal polyhistidine (6x His) sequence for rapid purification on PROBOND resin (Invitrogen, CA). Transformed cells are selected on media containing blasticidin.

*Spodoptera frugiperda* (Sf9) insect cells are infected with recombinant *Autographica californica* nuclear polyhedrosis virus (baculovirus). The polyhedrin gene is replaced with the cDNA by homologous recombination and the polyhedrin promoter drives cDNA transcription. The protein is synthesized as a fusion protein with 6x His which enables purification as described above. Purified proteins can be used to produce antibodies.

### 4. Chemical Synthesis of Proteins

Proteins or portions thereof can be produced not only by recombinant methods (such as described above), but also by using chemical methods well known in the art. Solid phase peptide synthesis can be carried out in a batchwise or continuous flow process which sequentially adds α-amino- and side chain-protected amino acid residues to an insoluble polymeric support via a linker group. A linker group such as methylamine-derivatized polyethylene glycol is attached to poly(styrene-co-divinylbenzene) to form the support resin. The amino acid residues are N-a-protected by acid labile Boc (t-butyloxycarbonyl) or base-labile Fmoc (9-fluorenylmethoxycarbonyl) groups. The carboxyl group of the protected amino acid is coupled to the amine of the linker group to anchor the residue to the solid phase support resin. Trifluoroacetic acid or piperidine are used to remove the protecting group in the case of Boc or Fmoc, respectively. Each additional amino acid is added to the anchored residue using a coupling agent or pre-activated amino acid derivative, and the resin is washed. The full-length peptide is synthesized by sequential deprotection, coupling of derivitized amino acids, and washing with dichloromethane and/or N,N-dimethylformamide. The peptide is cleaved between the peptide carboxy terminus and the linker group to yield a peptide acid or amide. (Novabiochem 1997/98 Catalog and Peptide Synthesis Handbook, San Diego Calif. pp. S1-S20).

Automated synthesis can also be carried out on machines such as the 431A peptide synthesizer (Applied Biosystems, Foster City, CA). A protein or portion thereof can be purified by preparative high performance liquid chromatography and its composition confirmed by amino acid analysis or by sequencing (Creighton, 1984, Proteins, Structures and Molecular Properties, W H Freeman, New York N.Y.).

### 5. Antibody Production

### Polyclonal Antibodies

Polyclonal antibodies against recombinant proteins can be raised in rabbits (Green Mountain Antibodies, Burlington, VT). Briefly, two New Zealand rabbits are immunized with 0.1 mg of antigen in complete Freund's adjuvant. Subsequent immunizations are carried out using 0.05 mg of antigen in incomplete Freund's adjuvant at days 14, 21, and 49. Bleeds are collected and screened for recognition of the antigen by solid phase ELISA and Western blot analysis. The IgG fraction is separated by centrifugation at 20.000Xg for 20 minutes followed by a 50% ammonium sulfate cut. The pelleted protein is resuspended in 5mM Tris and separated by ion exchange chromatography. Fractions are pooled based on IgG content. Antigen-specific antibody is affinity purified using Pierce AminoLink resin coupled to the appropriate antigen.

### Isolation of Antibody Fragments Directed Against a Marker Protein from a Library of scFvs

Naturally occurring V-genes isolated from human PBLs can be constructed into a library of antibody fragments which contain reactivities against a marker protein to which the donor may or may not have been exposed (see, for example, U.S. Pat. No. 5,885,793).

Rescue of the library: A library of scFvs is constructed from the RNA of human PBLs, as described in PCT publication WO 92/01047. To rescue phage displaying antibody fragments, approximately 10⁹ *E*. *coli* harboring the phagemid are used to inoculate 50 ml of 2x TY containing 1% glucose and 100 µg/ml of ampicillin (2x TY-AMP-GLU) and grown to an O.D. of 0.8 with shaking. Five ml of this culture is used to innoculate 50 ml of 2x TY-AMP-GLU, 2x 10⁸ TU of delta gene 3 helper (M13 delta gene III, see PCT publication WO 92/01047) are added and the culture incubated at 37°C for 45 minutes without shaking and then at 37°C for 45 minutes with shaking. The culture is centrifuged at 4000 rpm. for 10 min. and the pellet resuspended in 2 liters of 2 x TY containing 100 µg/ml ampicillin and 50 µg/ml kanamycin and grown overnight. Phage are prepared as described in PCT publication WO 92/01047.

Preparation of M 13 delta gene III: M 13 delta gene III helper phage does not encode gene III protein, hence the phage(mid) displaying antibody fragments have a greater avidity of binding to antigen. Infectious M13 delta gene III particles are made by growing the helper phage in cells harboring a pUC19 derivative supplying the wild type gene III protein during phage morphogenesis. The culture is incubated for 1 hour at 37°C without shaking and then for a further hour at 37°C with shaking. CelLs are spun down (IEC-Centra 8,400 rpm for 10 min), resuspended in 300 ml 2 x TY broth containing 100 µg ampicillin/ml and 25 µg kanamycin/ml (2 x TY-AMP-KAN) and grown overnight, shaking at 37°C. Phage particles are purified and concentrated from the culture medium by two PEG-precipitations (Sambrook et al., 2001. Molecular Cloning: A Laboratory Manual. 3rd. ed., Cold Spring Harbor Laboratory Press, Cold Spring Harbor, NY). resuspended in ml PBS and passed through a 0.45 µm filter (Minisart NML: Sartorius) to give a final concentration of approximately 10¹³ transducing units/ml (ampicillin-resistant clones).

Panning of the library: Immunotubes (Nunc) are coated overnight in PBS with 4 ml of either 100 µg/ml or 10 µg/ml of a marker protein of interest. Tubes are blocked with 2% Marvel-PBS for 2 hours at 37°C and then washed 3 times in PBS. Approximately 10¹³ TU of phage is applied to the tube and incubated for 30 minutes at room temperature tumbling on an over-and-under turntable and then left to stand for another 1.5 hours. Tubes are washed 10 times with PBS 0.1% Tween-20 and 10 times with PBS. Phage are eluted by adding 1 ml of 100 mM triethylamine and rotating 15 minutes on an under-and-over turntable after which the solution is immediately neutralized with 0.5 ml of 1.0 M Tris-HCl, pH 7,4. Phages are then used to infect 10 ml of mid-log *E*. *coli* TG1 by incubating eluted phage with bacteria for 30 minutes at 37°C. The *E. coli* are then plated on TYE plates containing 1% glucose and 100 µg/ml ampicillin. The resulting bacterial library is then rescued with delta gene 3 helper phage as described above to prepare phage for a subsequent round of selection. This process is then repeated for a total of 4 rounds of affinity purification with tube-washing increased to 20 times with PBS, 0.1% Tween-20 and 20 times with PBS for rounds 3 and 4.

Characterization of binders: Eluted phage from the 3rd and 4th rounds of selection are used to infect *E*. *coli* HB 2151 and soluble scFv is produced (Marks et al., 1991, J. Mol. Biol. 222: 581-597) from single colonies for assay. ELISAs are performed with microtitre plates coated with either 10 µg/ml of the marker protein of interest in 50 mM bicarbonate pH 9.6. Clones positive in ELISA are further characterized by PCR fingerprinting (see, e.g., PCT publication WO 92/01047) and then by sequence analysis.

### Monoclonal Antibodies

### a) Materials:

1. Complete Media No Sera (CMNS) for washing of the myeloma and spleen cells; Hybridoma medium CM-HAT (Cell Mab (BD), 10% FBS (or HS); 5% Origen HCF (hybridoma cloning factor) containing 4 mM L-glutamine and antibiotics) to be used for plating hybridomas after the fusion.
2. Hybridoma medium CM-HT (no aminopterin) (Cell Mab (BD), 10% FBS 5% Origen HCF containing 4 mM L-glutamine and antibiotics) to be used for fusion maintenance is stored in the refrigerator at 4-6°C. The fusions are fed on days 4, 8. and 12, and subsequent passages. Inactivated and pre-filtered commercial fetal bovine serum (FBS) or horse serum (HS) are thawed and stored in the refrigerator at 4°C and is pretested for myeloma growth from single cells prior to use.
3. The L-glutamine (200 mM, 100X solution), which is stored at -20°C, is thawed and warmed until completely in solution. The L-glutamine is dispensed into media to supplement growth. L-glutamine is added to 2 mM for myelomas and 4 mM for hybridoma media. Further, the penicillin, streptomycin, amphotericin (antibacterial-antifungal stored at -20°C) is thawed and added to Cell Mab Media to 1%.
4. Myeloma growth media is Cell Mab Media (Cell Mab Media, Quantum Yield, from BD, which is stored in the refrigerator at 4°C in the dark), to which is added L-glutamine to 2 mM and antibiotic/antimycotic solution to 1% and is called CMNS.
5. One bottle of PEG 1500 in Hepes (Roche, NJ) is prepared.
6. 8-Azaguanine is stored as the dried powder supplied by SIGMA at -700°C until needed. One vial/500 ml of media is reconstituted and the entire contents are added to 500 ml media (e.g., 2 vials/liter).
7. Myeloma Media is CM which has 10% FBS (or HS) and 8-Aza (1X) stored in the refrigerator at 4°C.
8. Clonal cell medium D (Stemcell, Vancouver) contains HAT and methyl cellulose for semi-solid direct cloning from the fusion. This comes in 90 ml bottles with a CoA and is melted at 37°C in a waterbath in the morning of the day of the fusion. The cap is loosened and the bottle is left in a CO₂ incubator to sufficiently gas the medium D and bring the pH down.
9. Hybridoma supplements HT [hypoxanthine, thymidine] to be used in medium for die section of hybridomas and maintenance of hybridomas through the cloning stages, respectively.
10. Origen HCF can be obtained directly from Igen and is a cell supernatant produced from a macrophage-like cell-line. It can be thawed and aliqouted to 15 ml tubes at 5 ml per tube and stored frozen at -20°C. Positive hybridomas are fed HCF through the first subcloning and are gradually weaned (individual hybridomas can continue to be supplemented, as needed). This and other additives are typically more effective in promoting new hybridoma growth than conventional feeder layers.

### b) Procedure:

To generate monoclonal antibodies, mice are immunized with 5-50 µg of antigen, either intra-peritoneally (i.p.) or by intravenous injection in the tail vein (i.v.). The antigen used can be a recombinant marker protein of interest, for example. The primary immunization takes place two months prior to the harvesting of splenocytes from the mouse, and the immunization is typically boosted by i.v. injection of 5-50 µg of antigen every two weeks. At least one week prior to the expected fusion date, a fresh vial of myeloma cells is thawed and cultured. Several flasks of different densities can be maintained so that a culture at the optimum density is ensured at the time of fusion. An optimum density can be 3-6 x10⁵cells/ml, for example. 2-5 days before the scheduled fusion, a final immunization of approximately 5 µg of antigen in PBS is administered (either i.p. or i.v).

Myeloma cells are washed with 30 ml serum free media by centrifugation at 500g at 4°C for 5 minutes. Viable cell density is determined in resuspended cells using hemocytometry and vital stains. Cells resuspended in complete growth medium are stored at 37°C during the preparation of splenocytes. Meanwhile, to test aminopterin sensitivity, 1 x 10⁶ myeloma cells are transferred to a 15 ml conical tube and centrifuged at 500g at 4°C for 5 minutes. The resulting pellet is resuspended in 15 ml of HAT media and cells plated at 2 drops/well on a 96-well plate.

To prepare splenocytes from immunized nice, the animals are euthanised and submerged in 70% ethanol. Under sterile conditions, the spleen is surgically removed and placed in 10 ml of RPM1 medium supplemented with 20% fetal calf serum in a petri dish. Cells are extricated from the spleen by infusing the organ with medium >50 times using a 21g syringe.

Cells are harvested and washed by centrifugation (at 500g at 4°C for 5 minutes) with 30 ml of medium. Cells are resuspended in 10 ml of medium and the density of viable cells determined by hemocytometry using vital stains. The splenocytes are mixed with myeloma cells at a ratio of 5:1 (spleen cells: myeloma cells). Both the myeloma and spleen cells are washed twice more with 30 ml of RPMI-CMNS, and the cells are spun at 800 rpm for 12 minutes.

Supernatant is removed and cells are resuspended in 5 ml of RPMI-CMNS and are pooled to fill volume to 30 ml and spun down as before. Then, the pellet is broken up by gently tapping on the flow hood surface and resuspending in 1 ml of BMB REG 1500 (prewarmed to 37°C) dropwise with a 1cc needle over 1 minute.

RPMI-CMNS to the PEG cells and RPMI-CMNS are added to slowly dilute out the PEG. Cells are centrifuged and diluted in 5 ml of Complete media and 95 ml of Clonacell Medium D (HAT) media (with 5 ml of HCF). The cells are plated out 10 ml per small petri plate.

Myeloma/HAT control is prepared as follows: dilute about 1000 P3X63 Ag8.653 myeloma cells into 1 ml of medium D and transfer into a single well of a 24-well plate. Plates are placed in an incubator, with two plates inside of a large petri plate, with an additional petri plate full of distilled water, for 10-18 days under 5% CO₂ overlay at 37°C. Clones are picked from semisolid agarose into 96-well plates containing 150-200 µl of CM-HT. Supernatants are screened 4 days later in ELISA. and positive clones are moved up to 24-well plates. Heavy growth requires changing of the media at day 8 (+/-150 ml). The HCF can be further decreased to 0.5% (gradually *-* 2%, then 1%, then 0.5%) in the cloning plates.

### 6. Liquid Chromatography and Mass Spectrometry (LC/MS)

For LC/MS analysis, proteins are reduced in 2.5 mM DTT for 1 hour at 37°C. and alkylated with ICAT™ reagent according to the procedures recommended by the manufacturer (Applied Biosystems, Framingham, MA). The reaction is quenched by adding excess DTT. Proteins are digested using sequencing grade modified trypsin overnight at 37°C followed by desalting using 3 cc Oasis HLB solid phase extraction columns (Waters, Milford, MA) and vacuum drying. Cysteine-containing peptides are purified by avidin column (Applied Biosystems, Framingham, MA). The peptides are reconstituted in buffer A (0.1% formic acid in water) and separated over a C18 monomeric column (150 mm, 150 µm i.d., Grace Vydac 238EV5, 5 µm) at a flow rate of 1.5 µl/min with a trap column. Peptides are eluted from the column using a gradient, 3%-30% buffer B (0.1 % formic acid in 90% acetonitrile) in 215 min. 30%-90% buffer B in 30 min. Eluted peptides are analyzed using an online QSTAR XL system (MDS/Sciex, Toronto, ON). Peptide ion peaks from the map are automatically detected with RESPECT™ (PPL Inc., UK).

The sequence-composition of peptides detected, for example, at higher levels in disease samples (or drug-resistant samples) relative to adjacent normal tissue (or drug-sensitive samples) can be resolved through tandem mass spectrometry and database analysis. For data analysis, peptide ion peaks of LC/MS maps from normal and disease samples can be aligned based on mass to charge ratio (m/z), retention time (Rt), and charge state (z). The list of aligned peptide ions is loaded into Spotfire™ (Spotfire Inc. Somerville, MA). Intensities can be normalized before further differential analysis between disease and normal samples. Differentially expressed ions are manually verified before LC-MS/MS-based peptide sequencing and database searching for protein/protein identification.

For intensity normalization and expression analysis, a heat map can be constructed by sorting the rows by the ratio of the mean intensity in the disease samples to the mean intensity of the normal samples. Rows are included if there is at least one MS/MS identification of an ion in the row. The display colors are determined for each row separately by assigning black to the median intensity in the row, green to the lowest intensity in the row, and red to the highest intensity.

Using a mass spectrometry procedure such as this, a comprehensive analysis of proteins differentially expressed by disease cells (or drug resistant cells, for example) compared with normal cells (or cells responsive/sensitive to a drug, for example) can be carried out.

### 7. mRNA Expression Analysis

Expression of marker mRNA can be quantitated by RT-PCR using TaqMan^{®} technology. The Taqman^{®} system couples a 5' fluorogenic nuclease assay with PCR for real-time quantitation. A probe is used to monitor the formation of the amplification product.

Total RNA can be isolated from disease model cell lines using an RNEasy kit® (Qiagen, Valencia, CA) with DNase treatment (per the manufacturer's instructions). Normal human tissue RNAs can be acquired from commercial vendors (e.g., Ambion, Austin, TX; Stratagene, La Jolla, CA; BioChain Institute, Newington, NH), as well as RNAs from matched disease/normal tissues.

Marker transcript sequences can be identified for differentially expressed peptides by database searching using a search algorithm such as BLAST. TaqMan^{®} assays (PCR primer/probe sets) specific for those transcripts can be obtained from Applied Biosystems (AB) as part of the Assays on Demand™ product line or by custom design through the AB Assays by Design^{SM} service. If desired, the assays can be designed to span exon-exon borders so as not to amplify genomic DNA.

RT-PCR can be accomplished using AmpliTaq Gold^{®} and MultiScribe™ reverse transcriptase in the One Step RT-PCR Master Mix reagent kit (AB) (according to the manufacturer's instructions). Probe and primer concentrations are 250 nM and 900 nM, respectively, in a 15 µl reaction. For each experiment, a master mix of the above components is made and aliquoted into each optical reaction well. Eight nanograms of total RNA is used as template. Quantitative RT-PCR can be performed using the ABI Prism® 7900HT Sequence Detection System (SDS). The following cycling parameters are used: 48°C for 30 min. for one cycle; 95 °C for 10 min for one cycle; and 95 °C for 15 sec, 60 °C for I min. for 40 cycles.

SDS software can be utilized to calculate the threshold cycle (C_{T}) for each reaction, and C_{T} values are used to quantitate the relative amount of starting template in the reaction. The C_{T} values for each set of reactions can be averaged for all subsequent calculations

Data can be analyzed to determine estimated copy number per cell. Gene expression can be quantitated relative to 18S rRNA expression and copy number estimated assuming 5 x 10⁶ copies of 18S rRNA percell. Alternatively, data can be analyzed for fold difference in expression using an endogenous control for normalization and expressed relative to a normal tissue or normal cell line reference. The choice of endogenous control can be determined empirically by testing various candidates against the cell line and tissue RNA panels and selecting the one with the least variation in expression. Relative changes in expression can be quantitated using the 2^{-ΔΔCT} method (Livak et al., 2001, Methods 25: 402-408; User bulletin #2: ABI Prism 7700 Sequence Detection System). Alternatively, total RNA can be quantitated using a RiboGreen RNA Quantitation Kit according to manufacturer's instructions and the percentage mRNA expression calculated using total RNA for normalization. Percentage knockdown can then be calculated relative to a no addition control.

### 8. Flow Cytometry (FACS) Analysis

Flow cytometry is interchangeably referred to as fluorescence-activated cell sorting (FACS). Quantitative flow cytometry can be used to compare the level of expression of a protein on disease cells to the level found on normal cells, for example.

Expression levels of a marker protein on primary tissue samples can be quantified using the Quantum Simply Cellular System (Bangs Laboratories, Fishers, IN) and a marker-specific antibody. Normal adjacent and disease tissues can be processed into single cell suspensions, as described above, which can be stained for various markers (e.g., the epithelial marker EpCam) and the marker-specific antibody. At least 0.5 x 10⁶ cells are typically used for each analysis. Cells are washed once with Flow Staining Buffer (0.5% BSA, 0.05% NaN3 in D-PBS). To the cells, 20 µl of each marker-specific antibody are added. An additional 5 µl of anti-EpCam antibody conjugated to APC can be added when unsorted cells are used. Cells are incubated with antibodies for 30 minutes at 4°C. Cells are washed once with Flow Staining Buffer and either analyzed immediately on an LSR flow cytometry apparatus or fixed in 1% formaldehyde and stored at 4°C until LSR analysis. Antibodies used to detect a marker can be PE-conjugated. PE-conjugated mouse 1gG I k can used as an isotype control antibody. Cells are analyzed by flow cytometry and epitope copy number and the percentage of viable epithelial cells positive for marker expression can be measured. Cell numbers and viability can be determined by PI exclusion (GUAVA) for cells isolated from both normal and disease tissue. Standard curve and samples can be analyzed on a LSR I (BDBiosciences, San Jose CA) flow cytometer. Antibody binding capacity for each lineage population can be calculated using geometric means and linear regression.

Expression levels of a marker protein can be quantified in cell lines with QIFIKIT flow cytometric indirect immunofluorescence assay (Dako A/S) using a primary antibody to the marker. Briefly, cells are detached with versene or trypsin and washed once with complete media and then PBS. 5 x 10⁵ cells/sample are incubated with saturating concentration (10 µg/ml) of primary antibody for 60 minutes at 4°C. After washes, a FITC-conjugated secondary antibody (1:50 dilution) is added for 45 minutes at 4°C. QIFIKIT standard beads are simultaneously labeled with the secondary antibody. Binding of antibodies is analyzed by flow cytometry and specific antigen density is calculated by subtracting background antibody equivalent from antibody-binding capacity based on a standard curve of log mean fluorescence intensity versus log antigen binding capacity.

Cells can also be prepared for flow cytometry analysis (as well as other types of analysis) as follows: cells are incubated with 1:100 dilution of BrdU in culturing media for 2-4 hours (BrdU Flow Kit, cat# 559619 BD Biosciences). Cells are washed 3 times with D-PBS and disassociated from the flask with versene. Cell numbers and viability can be determined by PI exclusion (GUAVA). Cells are washed once with Flow Staining Buffer (0.5% BSA, 0.05% NaN₃ in D-PBS). Cells are incubated with 400 µl of Cytofix/Cytoperm Buffer (BrdU Flow Kit, BD Biosciences) for 15-30 minutes at 4°C. Cells are washed once with Flow Staining Buffer and resuspended in 400 µl Cytoperm Plus Buffer (BrdU Flow Kit BD Biosciences). Cells are incubated for 10 minutes at 4°C and washed once with 1X Perm/Wash Buffer (BrdU Flow Kit, BD Biosciences). Cells are incubated for 1 hour at 37°C protected from light in DNAse solution (BrdU Flow Kit, BD Biosciences). Cells are washed once with 1X Perm/Wash Buffer and incubated for 20 min at room temperature with anti-BrdU FITC-conjugated antibody (BrdU Flow Kit, BD Biosciences), PE-conjugated active caspase 3 (BD Biosciences cat# 550821), and PE mouse IgG2B isotype control. Cells are washed once with 1X Perm/Wash Buffer and resuspended in DAPI for LSR flow cytometry analysis.

### 9. Immunohistochemistry (IHC)

### IHC of Tissue Sections

Paraffin embedded, fixed tissue sections (e.g., from disease tissue samples such as solid tumors or other cancer tissues) can be obtained from a panel of normal tissues as well as tumor (or other disease) samples with matched normal adjacent tissues, along with replicate sections (if desired). For example, for an initial survery of marker expression, a panel of common cancer formalin-fixed paraffin-embedded (FFPE) tissue microarrays (TMAs) can be used for analysis, and such TMAs can be obtained from commercial sources (TriStar, Rockville, MD; USBiomax, Rockville, MD; Imgenex, San Diego, CA; Petagen/Abxis, Seoul, Korea). Sections can be stained with hemotoxylin and eosin and histologically examined to ensure adequate representation of cell types in each tissue section.

An identical set of tissues can be obtained from frozen sections for use in those instances where it is not possible to generate antibodies that are suitable for fixed sections. Frozen tissues do not require an antigen retrieval step.

### Paraffin Fixed Tissue Sections

An exemplary protocol for hemotoxylin and eosin staining of paraffin embedded, fixed tissue sections is as follows. Sections are deparaffinized in three changes of xylene or xylene substitute for 2-5 minutes each. Sections are rinsed in two changes of absolute alcohol for 1-2 minutes each, in 95% alcohol for 1 minute, followed by 80% alcohol for 1 minute. Slides are washed in running water and stained in Gill solution 3 hemotoxylin for 3-5 minutes. Following a vigorous wash in running water for 1 minute, sections are stained in Scott's solution for 2 minutes. Sections are washed for 1 minute In running water and then counterstained in eosin solution for 2-3 minutes, depending upon the desired staining intensity. Following a brief wash in 95% alcohol, sections are dehydrated in three changes of absolute alcohol for 1 minute each and three changes of xylene or xylene substitute for 1-2 minutes each. Slides are coverslipped and stored for analysis.

### Optimization of Antibody Staining

For each antibody, a positive and negative control sample can be generated using data from ICAT analysis of disease cell lines or tissues. Cells can be selected that are known to express low levels of a particular marker as determined from the ICAT data, and this cell line can be used as a reference normal control. Similarly, a disease cell line that is determined to over-express the marker can also be selected.

### Antigen Retrieval

Sections are deparaffinized and rehydrated by washing 3 times for 5 minutes in xylene, two times for 5 minutes in 100% ethanol, two times for 5 minutes in 95% ethanol, and once for 5 minutes in 80% ethanol. Sections are then placed in endogenous blocking solution (methanol + 2% hydrogen peroxide) and incubated for 20 minutes at room temperature. Sections are rinsed twice for 5 minutes each in deionized water and twice for 5 minutes in phosphate buffered saline (PBS), pH 7,4.

Alternatively, where necessary, sections are de-parrafinized by High Energy Antigen Retrieval as follows: sections are washed three times for 5 minutes in xylene, two times for 5 minutes in 100% ethanol, two times for 5 minutes in 95% ethanol, and once for 5 minutes in 80% ethanol. Sections are placed in a Coplin jar with dilute antigen retrieval solution (10 mM citrate acid, pH 6). The Coplin jar containing slides is placed in a vessel filled with water and microwaved on high for 2-3 minutes (700 watt oven). Following cooling for 2-3 minutes, steps 3 and 4 are repeated four times (depending on the tissue), followed by cooling for 20 minutes at room temperature. Sections are then rinsed in deionized water (two times for 5 minutes), placed in modified endogenous oxidation blocking solution (PBS + 2% hydrogen peroxide), and rinsed for 5 minutes in PBS.

Alternatively, formalin fixed paraffin embedded tissues can be deparaffinized and processed for antigen retrieval using the EZ-retriever system (BioGenex, San Ramon, CA). EZ-antigen Retrieval common solution is used for deparaffinization and EZ-retrieval citrate-based buffer used for antigen retrieval. Samples are pre-blocked with non-serum protein block (Dako A/S, Glostrup, Denmark) for 15 min. Primary antibodies (at 2.5-5.0 µg/ml, for example) are incubated overnight at room temperature. Envision Plus system HRP (Dako A/S) is used for detection with diaminobenzidine (DAB) as substrate for horseradish peroxidase.

### Blacking and Staining

Sections are blocked with PBS/1% bovine serum albumin (PBA) for 1 hour at room temperature followed by incubation in normal serum diluted in PBA (2%) for 30 minutes at room temperature to reduce non-specific binding of antibody. Incubations are performed in a sealed humidity chamber to prevent air-drying of the tissue sections. The choice of blocking serum is typically the same as the species of the biotinylated secondary antibody. Excess antibody is gently removed by shaking and sections covered with primary antibody diluted in PBA and incubated either at room temperature for 1 hour or overnight at 4°C (care is taken that the sections do not touch during incubation). Sections are rinsed twice for 5 minutes in PBS, shaking gently. Excess PBS is removed by gently shaking. The sections are covered with diluted biotinylated secondary antibody in PBA and incubated for 30 minutes to 1 hour at room temperature in the humidity chamber. If using a monoclonal primary antibody, addition of 2% rat serum can be used to decrease the background on rat tissue sections. Following incubation, sections are rinsed twice for 5 minutes in PBS, shaking gently. Excess PBS is removed and sections incubated for 1 hour at room temperature in Vectastain ABC reagent (as per kit instructions). The lid of the humidity chamber is secured during all incubations to ensure a moist environment. Sections are rinsed twice for 5 minutes in PBS, shaking gently.

### Developing and Counterstaining

Sections are incubated for 2 minutes in peroxidase substrate solution that is made up immediately prior to use as follows: 10 mg diaminobenzidine (DAB) dissolved in 10 ml of 50 mM sodium phosphate buffer, pH 7.4; 12.5 microliters 3% CoCl₂/NiCl₂ in deionized water; and 1.25 microliters hydrogen peroxide.

Slides are rinsed well three times for 10 minutes in deionized water and counterstained with 0.01% Light Green acidified with 0.01% acetic acid for 1-2 minutes, depending on the desired intensity of counterstain.

Slides are rinsed three times for 5 minutes with deionized water and dehydrated two times for 2 minutes in 95% ethanol; two times for 2 minutes in 100% ethanol; and two times for 2 minutes in xylene, Stained slides are mounted for visualization by microscopy.

Slides are scored manually using a microscope such as the Zeiss Axiovert 200M microscope (Carl Zeiss Microimaging, Thornwood, NY). Representative images are acquired using 40x objective (400x magnification).

### IHC Staining of Frozen Tissue Sections

For IHC staining of frozen tissue sections, fresh tissues are embedded in OCT in plastic mold, without trapping air bubbles surrounding the tissue. Tissues are frozen by setting the mould on top of liquid nitrogen until 70-80% of the block turns white at which point the mold is placed on dry ice. The frozen blocks are stored at - 80°C. Blocks are sectioned with a cryostat with care taken to avoid warming to greater than -10°C. Initially, the block is equilibrated in the cryostat for about 5 minutes and 6-10mm sections are cut sequentially. Sections are allowed to dry for at least 30 minutes at room temperature. Following drying, tissues are stored at 4°C for short term and -80°C for long term storage.

Sections are fixed by immersing in an acetone jar for 1-2 minutes at room temperature, followed by drying at room temperature. Primary antibody is added (diluted in 0.05 M Tris-saline [0.05 M Tris, 0.15 M NaCl, pH 7.4], 2.5% serum) directly to the sections by covering the section dropwise to cover the tissue entirely. Binding is carried out by incubation in a chamber for 1 hour at room temperature. Without letting the sections dry out, the secondary antibody (diluted in Tris-saline/2.5% serum) is added in a similar manner to the primary antibody and incubated as before (at least 45 minutes).

Following incubation, the sections are washed gently in Tris-saline for 3-5 minutes and then in Tris-saline/2.5% serum for another 3-5 minutes. If a biotinylated primary antibody is used, in place of the secondary antibody incubation, slides are covered with 100 µl of diluted alkaline phosphatase conjugated streptavidin, incubated for 30 minutes at room temperature and washed as above. Sections are incubated with alkaline phosphatase substrate (1mg/ml Fast Violet; 0.2 mg/ml Napthol AS-MX phosphate in Tris-Saline pH 8.5) for 10-20 minutes until the desired positive staining is achieved at which point the reaction is stopped by washing twice with Tris-saline. Slides are counter-stained with Mayer's hematoxylin for 30 seconds and washed with tap water for 2-5 minutes. Sections are mounted with Mount coverslips and mounting media.

### 10. RNAi Assays in Cell Lines

### RNAi Transfections

Expression of a marker can be knocked down by transfection with small interfering RNA (siRNA) to that marker. Synthetic siRNA oligonucleotides can be obtained from Dharmacon (Lafayette, CO) or Qiagen (Valencia, CA). For siRNA transfection, cells (e.g., disease cells) can be seeded into 96 well tissue culture plates at a density of 2,500 cells per well 24 hours before transfection. Culture medium is removed and 50µl of reaction mix containing siRNA (final concentration 1 to 100 nM) and 0.4 µl of DharmaFECT4 (Dharmacon, Lafayette, CO) diluted in Opti-MEM is added to each well. An equal volume of complete medium follows and the cells are then incubated at 5% CO₂ at 37°C for 1 to 4 days.

Alternatively, in the initial screening phase, RNAi can be performed using 100nM (final) of Smartpools (Dharmacon, Lafayette, CO), pool of 4- for Silencing siRNA duplexes (Qiagen. Valencia, CA), or non-targeting negative control siRNA (Dharmacon or Qiagen). In the breakout phase, each individual duplex is used at 100nM (final). In the titration phase, individual duplex is used at 0.1-100nM (final). Transient transfections are carried out using either Lipofectamine 2000 from Invitrogen (Carlsbad, CA) or GeneSilencer from Gene Therapy Systems (San Diego, CA) (see below). One day after transfections, total RNA is isolated using the RNeasy 96 Kit (Qiagen) according to manufacturer's instructions and expression of mRNA is quantitated using TaqMan technology. Apoptosis and cell proliferation assays can be performed daily using Apopone homogeneous caspase-3/7 kit and Alamar Blue or CellTiter 96 AQueous One Solution Cell Proliferation Assays (see below).

### RNAi Trarrsfections - Lipofectamine 2000 and GeneSilencer

Transient RNAi transfections can be carried out using Lipofectamine 2000 (Invitrogen, Carlsbad, CA) or GeneSilencer (Gene Therapy Systems, San Diego, CA), such as on sub-confluent disease cell lines, as described elsewhere (Elbashir et al.. 2001, Nature 411: 494-498; Caplen et al., 2001, Proc Natl Acad Sci USA 98: 9742-9747; Sharp, 2001, Genes and Development 15: 485-490). Synthetic RNA to a gene of interest or non-targeting negative control siRNA are transfected using Lipofectamine 2000 or GeneSilencer according to manufacturer's instructions. Cells are plated in 96-well plates in antibiotic-free medium. The next day, the transfection reagent and siRNA are prepared for transfections as follows.

0.1-100 nM siRNA is resuspended in 20-25 µl serum-free media in each well (with Plus for Lipofectamine 2000) and incubated at room temperature for 15 minutes. 0.1-1 µl of Lipofectamine 2000 or 1-1.5 µl of GeneSilencer is also resuspended in serum-free medium to a final volume of 20-25 µl per well. After incubation, the diluted siRNA and either the Lipofectamine 2000 or the GeneSilencer are combined and incubated for 15 minutes (Lipofectamine 2000) or 5-20 minutes (GeneSilencer) at room temperature. Media is then removed from the cells and the combined siRNA-Lipofectamine 2000 reagent or siRNA-GeneSilencer reagent is added to a final volume of 50 µl per well. After further incubation at 37°C for 4 hours, 50 µl serum-containing medium is added back to the cells. 1-4 days after transfection, expression of mRNA can be quantitated by RT-PCR using TaqMan technology, and protein expression levels can be measured by flow cytometry. Apoptosis and proliferation assays can be performed daily using Apop-one homogeneous caspase-3/7 kit and Alamar Blue or CellTiter 96 AQueous One Solution Cell Proliferation Assays (see below).

### mRNA and Protein Knockdowns

Knockdown of marker mRNA levels can be monitored by Q-PCR one day after siRNA transfection by using a TaqMan® assay (Applied Biosystems, Foster City, CA). RT-PCR is accomplished in a one-step reaction by using M-MLV reverse transcriptase (Promega, Madison, WI) and AmpliTaq Gold® (ABI) and analyzed on the ABI Prism® 7900HT Sequence Detection System (ABI). Relative gene expression can be quantitated by the ΔΔCT method (User Bulletin #2, ABI) with 18S rRNA serving as the endogenous control.

Protein knockdown can be monitored by FACS four days after transfection by using an antibody to the marker. The samples can be run on a LSR flow cytometer (BD Biosciences, San Jose, CA) and live cells monitored by using PI exclusion (50 µg/ml PI, 2.5 units/ml RNase A, 0.1% Triton X-100 in D-PBS). The data can be analyzed using CellQuest software.

### Cell Proliferation - Alamar Blue

Cell growth can be assessed four days after transfection by adding a 1:10 dilution of Alamar blue reagent (Invitrogen, Carlsbad, CA or Biosource, Camarillo, CA) and incubated for 2 hours at 37°C. Analysis can be performed on a Spectrafluor Plus (Tecan, Durham, NC) set at excitation wavelength of 530 nm and emission wavelength of 595 nm.

### Cell Proliferation - MTS

Alternatively, cell proliferation assays can be performed using a CellTiter 96 AQueous One Solution Cell Proliferation Assay kit (Promega, Madison, WI). 20µl of CellTiter 96 AQueous One Solution is added to 100µl of culture medium. The plates are then incubated for 1-4 hours at 37°C in a humidified 5% CO₂ incubator. After incubation, the change in absorbance is read at 490nm.

### Apoptosis

Apoptosis assays can be performed using the Apop-one homogeneous caspase-3/7 kit (Promega, Madison, WI). Briefly, the caspase-3/7 substrate is thawed to room temperature and diluted 1:100 with buffer. The diluted substrate is then added 1:1 to cells, control, or blank. The plates are then placed on a plate shaker for 30 minutes to 18 hours at 300-500rpm. The fluorescence of each well is then measured using an excitation wavelength of 485 +/- 20nm and an emission wavelength of 530 +/- 2nm.

### 11. Antibody Assays in Cell Lines

### Cytotoxicity Assays

Cytotoxicity can be measured using a Resazurin (Sigma, MO) dye reduction assay (McMillian et al., 2002, Cell Biol. Toxicol. 18:157-173). Briefly, cells are plated at 1,000-5,500 cells/well in 96 well plates, allowed to attach to the plates for 18 hours before addition of fresh media with or without antibody. After 96-144 hours of exposure to antibody, resazurin is added to cells to a final concentration of 50 µM. Cells are incubated for 2-6 hours depending on dye conversion of cell lines, and dye reduction is measured on a Fusion HT fluorescent plate reader (Packard Instruments, Meridien, CT) with excitation and emission wavelengths of 530 nm and 590 nm, respectively. The IC₅₀ value is defined here as the drug concentration that results in 50% reduction in growth or viability as compared with untreated control cultures.

### Assays for Antibody-Dependent Cellular Cytotoxicity

Antibody-dependent cellular cytotoxicity (ADCC) assays can be carried out as follows. Cultured disease cells (e.g., tumor cells) are labeled with 100 µCi ⁵¹Cr for 1 hour (Livingston et al., 1997, Cancer immunol. Immunother. 43, 324-330). After being washed three times with culture medium, cells are resuspended at 10⁵/ml, and 100 µl/well are plated onto 96-well round-bottom plates. A range of antibody concentrations are applied to the wells, including an isotype control together with donor peripheral blood mononuclear cells that are plated at a 100:1 and 50:1 ratio. After an 18 hour incubation at 37°C, supernatant (30 µl/well) is harvested and transferred onto Lumaplate 96 (Packard), dried, and read in a Packard Top-Count NXT γ counter. Spontaneous release is determined by cpm of disease cells incubated with medium and maximum release by cpm of disease cells plus 1% Triton X-100 (Sigma). Specific lysis is defined as: % specific lysis = [(experimental release-spontaneous release)/(maximum release-spontaneous release)] x 100. The percent ADCC is expressed as peak specific lysis postimmune subtracted by preimmune percent specific lysis. A doubling of the ADCC to >20% can typically be considered significant.

### Assays for Complement Dependent Cytotoxicity

Chromium release assays to assess complement dependent cytotoxicity (CDC) can be carried out as follows (Dickler et al., 1999, Clin. Cancer Res. 5, 2773-2779). Cultured disease cells (e.g., tumor cells) are washed in FCS-free media two times, resuspended in 500 µl of media, and incubated with 100 µCi ⁵¹Cr per 10 million cells for 2 hours at 37°C. The cells are then shaken every 15 min for 2 hours, washed 3 times in media to achieve a concentration of approximately 20,000 cells/well, and then plated in round-bottom plates. The plates contain either 50 µl cells plus 50 µl monoclonal antibody, 50 µl cells plus serum (pre- and post-therapy), or 50 µl cells plus mouse serum as a control. The plates are incubated in a cold room on a shaker for 45 min. Human complement of a 1:5 dilution (resuspended in 1 ml of ice-cold water and diluted with 3% human serum albumin) is added to each well at a volume of 100 µl. Control wells include those for maximum release of isotope in 10% Triton X-100 (Sigma) and for spontaneous release in the absence of complement with medium alone. The plates are incubated for 2 hours at 37°C, centrifuged for 3 min, and then 100 µl of supernatant is removed for radioactivity counting. The percentage of specific lysis is calculated as follows: % cytotoxicity = [(experimental release-spontaneous release)/(maximum release-spontaneous release)] x 100. A doubling of the CDC to >20% can typically be considered significant.

### Cell Proliferation Assays

To measure cell proliferation, cells can be plated, grown and treated as for the cytotoxicity assay (above) in 96 well plates. After 96-144 hours of treatment, 0.5 µCi/well ³H-Thymidine (PerkinElmer, 6.7 Ci/mmol) is added to cells and incubated for 4-6 hours at 37°C, 5% CO₂ in an incubator. To lyse cells. plates are frozen overnight at - 20°C and then cell lysates are harvested using FilterMate (Packard Instrument, Meridien, CT) into 96 well filter plates. Radioactivity associated with cells is measured on a TopCount (Packard) scintillation counter.

Other cell assays (e.g., proliferation assays such as Alamar blue and MTS, and apoptosis assays) can be carried out using antibodies, as described above for RNAi.

### Testing of Function-Blocking Antibodies

For testing of function-blocking antibodies, sub-confluent disease cell lines are serum-starved overnight. The next day, serum-containing media is added back to the cells in the presence of 5-50 ng/ml of function-blocking antibodies. After 2 or 5 days incubation at 37°C 5% CO₂, antibody binding is examined by flow cytometry, and apoptosis and proliferation are measured.

### Cell Invasion

Cell invasion assays can be performed using a 96-well cell invasion assay kit (Chemicon). After the cell invasion chamber plates are adjusted to room temperature, 100 µl serum-free media is added to the interior of the inserts. 1-2 hours later, cell suspensions of 1 x 10⁶ cells/ml are prepared. Media is then carefully removed from the inserts and 100 µl of prepared cells are added into the insert +/- 0 to 50 ng function blocking antibodies. The cells are pre-incubated for 15 minutes at 37°C before 150 µl of media containing 10% FBS is added to the lower chamber. The cells are then incubated for 48 hours at 37°C. After incubation, the cells from the top side of the insert are discarded and the invasion chamber plates are then placed on a new 96-well feeder tray containing 150 µl of pre-warmed cell detachment solution in the wells. The plates are incubated for 30 minutes at 37°C and are periodically shaken. Lysis buffer/dye solution (4 µl CyQuant Dye/300 µl 4X lysis buffer) is prepared and added to each well of dissociation buffer/cells on feeder tray. The plates are incubated for 15 minutes at room temperature before 150 µl is transferred to a new 96-well plate. Fluorescence of invading cells is then read at 480 nm excitation and 520 nm emission.

### Receptor Internalization

For quantification of receptor internalization, ELISA assays can be performed essentially as described by Daunt et al. (Daunt et al., 1997, Mol. Pharmacol. 51, 711-720). Cell lines are plated at 6×10⁵ cells per in a 24-well tissue culture dishes that have previously been coated with 0.1mg/ml poly-L-lysine. The next day, the cells are washed once with PBS and incubated in DMEM at 37°C for several minutes. Agonist to the cell surface marker of interest is then added to the wells at a pre-determined concentration in prewarmed DMEM. The cells are then incubated for various times at 37°C and reactions are stopped by removing the media and fixing the cells in 3.7% forntaldehyde/TBS for 5 min at room temperature. The cells are then washed three times with TBS and nonspecific binding blocked with TBS containing 1% BSA for 45 min at room temperature. The first antibody is added at a pre-determined dilution in TBS/BSA for 1 hr at room temperature. Three washes with TBS follow, and cells are briefly reblocked for 15 min at room temperature. Incubation with goat anti-mouse conjugated alkaline phosphatase (Bio-Rad) diluted 1:1000 in TBS/BSA is carried out for 1 hr at room temperature. The cells are washed three times with TBS and a colorimetric alkaline phosphatase substrate is added. When the adequate color change is reached, 100 µl samples are taken for colorimetric readings.

### 12. Treatment with Antibodies

### Treatment of Disease Cells with Monoclonal Antibodies.

Disease cells (e.g., cancer cells), or cells such as NIH 3T3 cells that express a marker of interest, are seeded at a density of 4 x 10⁴ cells per well in 96-well microtiter plates and allowed to adhere for 2 hours. The cells are then treated with different concentrations of monoclonal antibody (Mab) specific for the marker protein of interest, or irrelevant isotype matched (e.g., anti-rHuIFN-gamma) Mab, at 0.05, 0.5 or 5.0 µg/ml. After a 72 hour incubation, the cell monolayers are stained with crystal violet dye for determination of relative percent viability (RPV) compared to control (untreated) cells. Each treatment group can have replicates. Cell growth inhibition is monitored.

### In vivo Treatment with Monoclonal Antibodies.

NIH 3T3 cells transfected with either an expression plasmid that expresses the marker of interest or a neo-DHFR vector are injected into nu/nu (athymic) mice subcutaneously at a dose of 10⁶ cells in 0.1 ml of phosphate-buffered saline. On days 0.1, 5, and every 4 days thereafter, 100 µg (0.1 ml in PBS) of a Mab specific for the marker protein of interest, or an irrelevant Mab, of the IgA2 subclass is injected intraperitoneally. Disease progression (e.g., tumor occurrence and size) can be monitored for a one month period of treatment, for example.

### 13. Identification of LCM

A mass spectrometry (MS)-based proteomics platform was used for the identification of secreted and shed proteins (secreted and shed proteins are collectively referred to herein as soluble proteins) and cell surface antigens that combines the discovery of candidate biomarkers from human lung tumor specimens resected from surgery and in a panel of lung cancer cell lines, followed by validation of expression levels in patient serum (such as by using ELISA). For example, proteomic analysis techniques such as MALDI-TOF/TOF LC/MS-based protein expression analysis was used to determine the expression levels of certain proteins in lung tumor tissues and/or lung cancer cell lines (tissues and cell lines may be collectively referred to herein as "samples") and in normal tissues and/or normal cell lines, such that proteins that are differentially expressed (e.g., over- or under-expressed) in lung cancer samples compared with normal samples were identified.

Certain candidate markers were identified by mass spectrometry-based methods that were differentially expressed on the cell surface of lung tumors, lung cancer cell lines, or secreted into the conditioned medium of cell lines. Certain of these candidate markers that were identified as differentially expressed by mass spectrometry, as well as certain other candidate markers, were assayed by ELISA and scored in panels of lung cancer patient sera and sera from individuals without lung cancer (individuals without lung cancer are referred to herein as "normal", "control", or "healthy" individuals). Individual markers were scored "positive" for a given cancer sample if the value exceeded a defined threshold (e.g., greater than or equal to two standard deviations above the mean value for a group of "normal" samples tested). From these candidate markers, lung cancer markers ("LCM") were identified that, particularly when used in combination, distinguished lung cancer samples from healthy control samples with various degrees of sensitivity and specificity.

Several methods and algorithms were applied to select optimum panels/combinations of LCM including sum of the logs of the ratios of the tumor concentration to the mean of the normal concentration, defining a concentration cutoff manually for each marker to optimize sensitivity and specificity, and use of Naive Bayes to assign a probability that a sample is a tumor based on the expression level of each marker. Additionally, ROC curves may be constructed for each panel and their effectiveness may be evaluated in several ways, such as maximizing the AUC of the ROC curve as well as maximizing the sensitivity at a desired specificity or maximizing the specificity at a desired sensitivity.

To further validate the specificity of certain panels, co-morbidity studies were carried out to challenge certain panels with other lung disease samples besides lung cancer, particularly chronic obstructive pulmonary disease (COPD), asthma, bronchitis, and other benign lung diseases (Figure 20). Prevalence of COPD/asthma is 10-25% in smokers. An initial panel of 30 bronchitis/asthma/benign lung disease samples was tested. Results indicated that these co-morbidities may reduce specificity only marginally if considered independent of false positives in 54 control samples (the specificity of the 9-member panel of Cyfra, SLPI, TIMPI, SCC, TFPI, CEACAM5, MMP2, OPN, and MDK in a 54 normal/53 lung tumor sample set was 98% on samples from smoking controls).

### 14. ELISA Immunoassays

Immunoassay kits, such as for performing ELISA assays, for various LCM disclosed herein are commercially available. For example, immunoassay kits can be obtained from a variety of commercial sources, as follows: SLPI. MMP2, MIF, and OPN immunoassay kits can be obtained from R&D Systems (Minneapolis, MN); CYFRA 21-1 and SCC immunoassay kits can be obtained from DRG-International (Mountainside, NJ); DEFA1 immunoassay kits can be obtained from *Cell* Sciences (Canton, MA); TIMP1 immunoassay kits can be obtained from Siemens Healthcare Diagnostics (Cambridge, MA); CEA and GRP immunoassay kits can be obtained from IBL International (Toronto, Ontario); TFPI immunoassay kits can be obtained from American Diagnostica (Stamford, CT); and MDK immunoassay kits can be obtained from BioVendor (Candler, NC.) or R&D Systems (Minneapolis, MN.). Assays can be performed following manufacturers instructions. Plates can be read on a Spectra Max M2 Microplate Reader (Molecular Devices, Sunnyvale, CA) with the appropriate baseline correction for each assay.

HNP1-3 (defensin, DEFA1) is employed as a representative marker in the following exemplary ELISA protocol, which can be used for the analysis of LCM. An HNP1-3 ELISA test kit can be used that is a solid-phase enzyme-linked immunosorbent assay based on the sandwich principle. Samples and standards are incubated in microtiter wells coated with antibodies recognizing human HNP1-3. During this incubation, human HNP1-3 is captured by solid bound antibody. Unbound material present in the sample is removed by washing. Biotinylated second antibody (tracer) to human HNP 1-3 is then added to the wells. If HNP1-3 is present in the sample, the tracer antibodies will bind to the captured HNP1-3. The excess tracer is removed by washing. A streptavidin-peroxidase conjugate is then applied to the wells, which reacts specifically with the biotinylated tracer antibody bound onto the detected HNP1-3. The excess streptavidin-peroxidase conjugate is removed by washing and substrate tetramethylbenzidine (TMB) is added to the wells. Color develops proportionally to the amount of human HNP1-3 present in the sample. The enzyme reaction is stopped by the addition of citric acid and the absorption at 450 nm is measured with a spectrophotometer. A standard curve is obtained by plotting the absorptions versus the corresponding concentrations of the known standards. The concentration of human HNP1-3 in test samples, which are run concurrently with the standards, can be determined from the standard curve.

### 15. Scoring of LCM Levels

This example describes an exemplary method of scoring LCM levels using split-point analysis.

The term "split-point analysis" refers to a method adapted from Mor et al., PNAS, (2005) 102, 7677-7682. In this exemplary method, measurements for each marker are taken on all samples. A cutoff value is determined for each marker. This cutoff value may be set to, for example, maximize the accuracy of correct classitications between the groups of interest (e.g., tumor and control sample groups) or may be set to maximize the sensitivity or specificity of one group. For each marker, a score is assigned to that sample whenever the value of that marker is found to be on the diseased side of the cutoff value (e.g., the side of the cutoff corresponding to lung tumor samples). After all the measurements have been taken on one sample, the scores are summed to produce a total score for the panel of markers. All markers can be weighted equally such that a panel of 9 markers may have a maximum score of 9 (each marker having a score of either 1 or 0) and a minimum score of 0, for example. Alternatively, markers can be weighted unequally, with a higher individual score for more significant measures.

Other more sophisticated statistical modelling methods can also be applied such as logistic regression (see, e.g., Planque et al., Clin Cancer Res (2008), 14, 1355-1362) and decision tree modeling (see, e.g., Patz et al., J Clin Oncol (2007), 25, 5578-5583).

An exemplary method of applying split-point analysis to an LCM panel is described for illustrative purposes.

A patients sample can be tested to determine the patient's likelihood of having lung cancer using a panel comprising the 9 biomarkers Cyfra, CEA, SLPI, OPN, MDK, TFPI, TIMP1, MMP2, and SCC and the split and score method. The predetermined total score (or threshold) for the panel can be set at I (or other value).

After obtaining a test sample from the patient, the amount of each of the 9 biomarkers (Cyfra, CEA, SLPI, OPN, MDK, TFPI, TIMP1, MMP2, SCC) in the patient's test sample is quantified. For the purpose of this example, the amount of each of the 9 biomarkers in the test sample is determined to be as follows (values are expressed in ng/ml): Cyfra = 0.891, CEA = 4.087, SLPI = 62.94, OPN = 21.514, MDK = 0.174, TFPI = 104.503, TIMP1 = 398.7, MMP2 = 194.41, and SCC = 1.35. The amount of each of these biomarkers is then compared to the corresponding predetermined cutoff (or split point). For the purpose of this example, the predetermined cutoffs for each of the biomarkers are as follows: Cyfra = 1.20, CEA = 5.00, SLPI = 52, OPN = 32, MDK = 0.15, TFPI = 150, TIMP1 = 385, MMP2 = 210, and SCC = 2.2. For each biomarker having an amount that is higher than its corresponding predetermined cutoff (split point), a score of 1 can be assigned. For each biomarker having an amount that is less than or equal to its corresponding predetermined cutoff, a score of 0 can be assigned. Thereupon, based on said comparison, each biomarker would be assigned a score as follows: Cyfra = 0, CEA = 0, SLPI = 1, OPN = 0, MDK = 1, TFP1 = 0, TIMP1 = 1, MMP2 = 0, and SCC = 0.

The score for each of the 9 biomarkers can then be combined mathematically (e.g., by adding each of the scores of the biomarkers together) to arrive at the total score for the patient. In this particular example, the total score for the patient is 3 (the total score is calculated as follows: 0+0+1+0+1+0+1+0+0=3). The total score for the patient is compared to the predetermined total score, which is 1 in this particular example. A total score greater than the predetermined total score of 1 would indicate a positive result for the patient (i.e., in this particular example, a total score of 2 or greater would indicate that the patient has lung cancer). A total score equal to or less than 1 would indicate a negative result for the patient. In this example, because the patient's total score is greater than I, the patient would be considered to have a positive result (and thus may be referred for further testing for an indication or suspicion of lung cancer). In contrast, had the patient's total score been 1 or 0, the patient would have been considered to have a negative result (and thus would not be referred for any further testing).

**TABLE 1: Lung Cancer Markers**

| **Description** | **Symbol** | **Genbank Accession Number for Protein** | **SEQ ID NO for Exemplary Protein Sequence** | **Reference Citations (see below)** |
|---|---|---|---|---|
| secretory leukocyte protease inhibitor | SLPI | P03973 | 1 | |
| macrophage migration inhibitory factor | MIF | P14174 | 2 | |
| tissue inhibitor of metalloproteinase 1 | TIMP1 | P01033 | 3 | |
| tissue factor pathway inhibitor | TFPI (TFP1, TFPI1) | P10646 | 4 | |
| Gamma enolase | ENO2 (NSE) | P09104 | 5 | |
| CEA (carcinoembryonic antigen-related cell adhesion molecule 5) | CEA (CEACAM5) | NP_004354 | 6 | 1, 2, 3, 4, 5 |
| matrix metalloproteinase 2 | MMP2 | P08253 | 7 | |
| alpha-1-microglobulin/bikunin precursor | AMBP | P02760 | 8 | |
| Cytra 21-1 | Cyfra 21-1 (Cyfra, KRT19) | P08727 | 9 | 1 |
| Squamous Cell Carcinoma Antigen (serpinB3) | SCC (SERPINB3) | NP_008850 | 10 | 1 |
| Osteopontin | OPN | P10451 | 11 | |
| Neutrophil defensin 1 precursor | defensin (DEFA1, HNP-1, HNP1-3) | P11479 | 12 | |
| Cancer associated antigen CA 242 (carbohydrate antigen) | CA 242 | N/A (carbohydrate antigen) | N/A (carbohydrate antigen) | 4 |
| Cancer associated antigen CA 19-9 (carbohydrate antigen) | CA 19-9 | N/A (carbohydrate antigen) | N/A (carbohydrate antigen) | 1, 2, 4, 5 |
| Cancer associated antigen CA 72-4 (carbohydrate antigen) | CA 72-4 | N/A (carbohydrate antigen) | N/A (carbohydrate antigen) | 1, 2, 3, 4, 5 |
| MN/CAIX | MN/CAIX (CA9, CAIX) | Q16790 | 13 | |
| Pro-gastrin-releasing peptide | ProGRP (GRP) | P07492 | 14 | |
| Cytokeratin-18 (tissue polypeptide-specific antigen) | KRT18 (TPS) | P05783 | 15 | |
| Epithelial-cadherin precursor (ECAD) | ECAD (CDH1) | P12830 | 16 | |
| tissue inhibitor of metalloproteinase 2 | TIMP2 | P16035 | 17 | |
| CD44 antigen precursor | CD44 | 016208 (P16070) | 18 | |
| lectin, galactoside-binding, soluble, 3 binding protein (S90K Mac-2B) | LGALS3BP | Q08380 | 19 | |
| Receptor protein-tyrosine kinase erbB-2 precursor | ERBB2 (HER-2) | P04626 | 20 | |
| urokinase plasminogen activator | UPA (PLAU) | P00749 | 21 | |
| dickkopf homolog 1 (Xonopus laevis) | DKK (DKK1) | 094907 | 22 | |
| Chromogranin A (parathyroid secretory protein 1) | CHGA | P10645 | 23 | |
| Vascular endothelial growth factor A precursor | VEGF | P15692 | 24 | |
| stem cell factor/c-kit ligand | KITLG | P21583 | 25 | |
| Vistatin (PRE-B CELL ENHANCING FACTOR) | PBEF | P43490 | 26 | |
| Sortilin precursor | SORT1 | Q99523 | 27 | |
| midkine (neurite growth-promoting factor 2) | MDK | P21741 | 28 | |
| insulin-like growth factor binding protein 3 | IGFBP3 | P17936 | 29 | |
| insulin-like growth tractor binding protein 4 | IGFBP4 | P22692 | 30 | |
| Dipeptidyl-peptidase I precursor | CTSC | P53834 | 31 | |
| intercellular adhesion molecule 3 | ICAM3 | P32942 | 32 | |
| connective tissue growth factor | CTGF | P29279 | 33 | |
| lipocalin 2 (oncogene 24p3) | LCN2 | P80188 | 34 | |
| epidermal growth factor receptor | EGFR | P00533 | 35 | |
| biglycan | BGN | P21810 | 38 | |
| tissue inhibitor of metalloproteinase 3 | TIMP3 | P35625 | 37 | |
| Hepatocyte growth factor | HGF | P14210 | 38 | |
| mucin 16 (CA125) | MUC16 (CA125) | Q8WXI7 | 56 | 6, 7 |
| neural cell adhesion molecule | NCAM | P13591 | 57 | |
| C-reactive protein | CRP | P02741 | 58 | |
| serpin peptidase inhibitor, (alpha-1-antitrypsin) | SERPINA1 (ATT) | P01009 | 59 | |
| pyruvate kinase M2 | PKM2 | P14618 | 60 | |
| retinol binding protein 1 | RBP | P29374 | 61 | |
| kallikrein-related peptidase 11 | KLK11 | Q9UBX7 | 62 | |
| kallikrein-related peptidase 13 | KLK13 | Q9UKR3 | 63 | |
| serum amyloid A | SAA | P02735 | 64 | |
| apolipoprotein C-III | APOC3 | P02556 | 65 | |
| | | | | |
| Reference Citations for Carbohydrate Antigens ("CA") and certain other markers: | | | | |
| (numbers below correspond with numbers indicated in "Reference Citations" column above) | | | | |
| | | | | |
| 1) Stieber et al., "CYFRA 21.1. A new marker in lung cancer", Cancer. 1993 Aug 1;72(3):707-13. | | | | |
| 2) Ferronl et al., "CA 72-4 radioimmunoassay in the diagnosis of malignant effusions Comparison of various tumor markers", Int J Cancer. 1990 Sep 15:46(6):445-51 | | | | |
| 3) Gero et al., CA 72-4 radioImmunoassay for the detection of the TAG-72 carcinoma-association antigen in serum of patients", J Clin Lab Anal. 1989;3(6):360-9 | | | | |
| 4) Carpelan-Holms from et al., "CEA, CA 242, CA 19-9, CA 72-4 and hCGbeta in the diagnosis of recurrent colorecial cancer", Tumour Biol. 2004 Sep-Dec:25(5-6):228-34 | | | | |
| 5) Fernandez-Fernandez et al., "Significance of CA 72-4 in colorectal carcinoma. Comparison w n CEA and CA 19-9", Eur J Surg Oncol. 1995 Aug:21(4) 388-90. | | | | |
| 6) Yin et al., "Molecular cloning of the CA125 ovarian cancer antingen: identification as a new mucin MUC16", J. Biol. Chem. 276: 27371-27375, 2001. | | | | |
| 7) Argueso et al., "MUC16 mucin is expressed by the human ocular surface epithelia and carries the H185 carbohydrate epitope", Invest. Ophthal. Vis. Sci. 44:2487-2495. | | | | |

**TABLE 2: Autoantibody Lung Cancer Markers**

| **Description** | **Symbol** | **Genbank Accession Number for Protein** | **SEQ ID NO for Exemplary Protein Sequence** |
|---|---|---|---|
| tumor protein p53 | TP53 (p53) | NP_000537 | 39 |
| kallikrein B, plasma (Fletcher factor) 1 | KLKB1 | P03952 | 40 |
| cofilin 1 (non-muscle) | CFL1 (CFLN) | P23528 | 41 |
| eukaryotic translation elongation factor 1 gamma | EEF1G | P26641 | 42 |
| heat shock protein 90kDa alpha | HSP90α (HSP90AA1) | NP_001017963 | 43 |
| Reticulon 4 | RTN4 | Q9Y5U6 | 44 |
| aldolase A, fructose-bisphosphate | ALDOA | NP_000025 | 45 |
| golgi apparatus protein 1 | GLG1 | NP_036333 | 46 |
| PTK7 protein tyrosine kinase 7 | PTK7 | NP_002812 | 47 |
| EGF-containing fibulin-like extracellular matrix protein 1 | EFEMP1 | Q12805 | 48 |
| solute carrier family 3 member 2 | SLC3A2 (CD98) | NP_001012679 | 49 |
| chromogranin B (secretogranin 1) | CHGB | P05060 | 50 |
| carcinoembryonic antigen-related cell adhesion molecule 1 | CEACAM1 | P13688 | 51 |
| activated leukocyte cell adhesion molecule | ALCAM | NP_001618 | 52 |
| Heat-shock protein beta-1 | HSPB1 (HSP27) | P04792 | 53 |
| lectin, galactoside-binding, soluble, 1 (galectin 1) | LGALS1 | P09382 | 54 |
| B7 homolog 3 - CD276 molecule | B7H3 | NP_001019907 | 55 |

**TABLE 3**

| | | | | | | | | | | |
|---|---|---|---|---|---|---|---|---|---|---|
| Cyfra | MIF | SLPI | TIMP1 | SCC | NSE | TFPI | CEA | OPN | CA242 | GRP |
| Cyfra | MIF | SLPI | TIMP1 | SCC | NSE | TFPI | CEA | OPN | CA242 | HNP-1 |
| Cyfra | MIF | SLPI | TIMP1 | SCC | TFPI | CEA | MMP2 | OPN | CA242 | GRP |
| Cyfra | MIF | SLPI | TIMP1 | SCC | TFPI | CEA | OPN | Ca19-9 | CA242 | GRP |
| Cyfra | MIF | SLPI | TIMP1 | SCC | TFPI | CEA | OPN | Ca72-4 | CA242 | GRP |
| Cyfra | MIF | SLPI | TIMP1 | SCC | TFPI | CEA | OPN | CA242 | CAIX | GRP |
| Cyfra | MIF | SLPI | TIMP1 | SCC | TFPI | CEA | OPN | CA242 | GRP | HNP-1 |
| Cyfra | SLPI | TIMP1 | SCC | NSE | TFPI | CEA | OPN | CA242 | GRP | HNP-1 |
| MIF | SLPI | TIMP1 | SCC | NSE | TFPI | CEA | MMP2 | OPN | CA242 | GRP |
| MIF | SLPI | TIMP1 | SCC | NSE | TFPI | CEA | OPN | AMBP | CA242 | GRP |
| MIF | SLPI | TIMP1 | SCC | NSE | TFPI | CEA | OPN | Ca19-9 | CA242 | GRP |
| MIF | SLPI | TIMP1 | SCC | NSE | TFPI | CEA | OPN | Ca72-4 | CA242 | GRP |
| MIF | SLPI | TIMP1 | SCC | NSE | TFPI | CEA | OPN | CA242 | CAIX | GRP |
| MIF | SLPI | TIMP1 | SCC | NSE | TFPI | CEA | OPN | CA242 | CAIX | HNP-1 |
| MIF | SLPI | TIMP1 | SCC | NSE | TFPI | CEA | OPN | CA242 | GRP | HNP-1 |
| MIF | SLPI | TIMP1 | SCC | TFPI | CEA | MMP2 | OPN | AMBP | CA242 | GRP |
| MIF | SLPI | TIMP1 | SCC | TFPI | CEA | MMP2 | OPN | Ca19-9 | CA242 | GRP |
| MIF | SLPI | TIMP1 | SCC | TFPI | CEA | MMP2 | OPN | Ca72-4 | CA242 | GRP |
| MIF | SLPI | TIMP1 | SCC | TFPI | CEA | MMP2 | OPN | CA242 | CAIX | GRP |
| MIF | SLPI | TIMP1 | SCC | TFPI | CEA | MMP2 | OPN | CA242 | GRP | HNP-1 |
| MIF | SLPI | TIMP1 | SCC | TFPI | CEA | OPN | AMBP | Ca19-9 | CA242 | GRP |
| MIF | SLPI | TIMP1 | SCC | TFPI | CEA | OPN | AMBP | Ca72-4 | CA242 | GRP |
| MIF | SLPI | TIMP1 | SCC | TFPI | CEA | OPN | AMBP | CA242 | CAIX | GRP |
| MIF | SLPI | TIMP1 | SCC | TFPI | CEA | OPN | AMBP | CA242 | GRP | HNP-1 |
| MIF | SLPI | TIMP1 | SCC | TFPI | CEA | OPN | Ca19-9 | Ca72-4 | CA242 | GRP |
| MIF | SLPI | TIMP1 | SCC | TFPI | CEA | OPN | Ca19-9 | CA242 | CAIX | GRP |
| MIF | SLPI | TIMP1 | SCC | TFPI | CEA | OPN | Ca19-9 | CA242 | GRP | HNP-1 |
| MIF | SLPI | TIMP1 | SCC | TFPI | CEA | OPN | Ca72-4 | CA242 | CAIX | GRP |
| MIF | SLPI | TIMP1 | SCC | TFPI | CEA | OPN | Ca72-4 | CA242 | GRP | HNP-1 |
| MIF | SLPI | TIMP1 | SCC | TFPI | CEA | OPN | CA242 | CAIX | GRP | HNP-1 |
| SLPI | TIMP1 | SCC | NSE | TFPI | CEA | MMP2 | OPN | CA242 | GRP | HNP-1 |
| SLPI | TIMP1 | SCC | NSE | TFPI | CEA | OPN | AMBP | CA242 | GRP | HNP-1 |
| SLPI | TIMP1 | SCC | NSE | TFPI | CEA | OPN | Ca19-9 | CA242 | GRP | HNP-1 |
| SLPI | TIMP1 | SCC | NSE | TFPI | CEA | OPN | Ca72-4 | CA242 | GRP | HNP-1 |
| SLPI | TIMP1 | SCC | NSE | TFPI | CEA | OPN | CA242 | CAIX | GRP | HNP-1 |
| | | | | | | | | | | |

| | | Count | Marker | | | | | | | |
|---|---|---|---|---|---|---|---|---|---|---|
| | | 7 | AMBP | | | | | | | |
| | | 8 | Cyfra | | | | | | | |
| | | 8 | MMP2 | | | | | | | |
| | | 8 | Ca72-4 | | | | | | | |
| | | 8 | Ca19-9 | | | | | | | |
| | | 9 | CAIX | | | | | | | |
| | | 15 | NSE | | | | | | | |
| | | 15 | HNP-1 | | | | | | | |
| | | 29 | MIF | | | | | | | |
| | | 33 | GRP | | | | | | | |
| | | 35 | SLPI | | | | | | | |
| | | 35 | TIMP1 | | | | | | | |
| | | 35 | TFPI | | | | | | | |
| | | 35 | SCC | | | | | | | |
| | | 35 | OPN | | | | | | | |
| | | 35 | CEA | | | | | | | |
| | | 35 | CA242 | | | | | | | |

**TABLE 4: Lung Cancer Markers**

| **Description** | **Symbol** | **Genbank Accession Number for Protein** |
|---|---|---|
| gamma enolase | ENO2 (NSE) | P09104 |
| CA125 | CA125 (MUC16) | Q8WXI7 |
| cytokeratin-18 (tissue polypeptide specific antigen) | KRT18 | P05783 |
| insulin-like growth factor binding protein 4 | IGFBP4 | P22692 |
| neural cell adhesion molecule | NCAM | P13591 |
| C-reactive protein | CRP | P02741 |
| serpin peptidase inhibitor, (alpha-1-antitrypsin) | SERPINA1 (ATT) | P01009 |
| pyruvate kinase M2 | PKM2 | P14618 |
| retinol binding protein 1 | RBP | P29374 |
| kallikrein-related peptidase 11 | KLK11 | Q9UBX7 |
| kallikrein-related peptidase 13 | KLK13 | Q9UKR3 |
| serum amyloid A | SAA | P02735 |
| apolipoprotein C-III | APOC3 | P02656 |

**TABLE 5**

| Tumor vs. Normal | | Logistic Regression | | | | | | Split Point | | | | | |
|---|---|---|---|---|---|---|---|---|---|---|---|---|---|
| | | Trained on 54x53, tested on 50x50 | | | Trained on 54x53, tested on 54x53 | | | Trained on 54x53, tested on 50x50 | | | Trained on 54x53, tested on 54x53 | | |
| Panel | Size | AUC | Sens at 95% Spec | Spec at 95% Sens | AUC | Sens at 95% Spec | Spec at 95% Sens | AUC | Sens at 95% Spec | Spec at 95% Sens | AUC | Sens at 95% Spec | Spec at 95% Sens |
| Cyfra SLPI CEACAM5 MMP2 OPN MDK | 6 | 0.98 | 94 | 94 | 0.99 | 89 | 94 | 0.93 | 72 | 84 | 0.95 | 77 | 69 |
| Cyfra SLPI TIMP1 CEACAM5 MDK | 5 | 0.98 | 94 | 94 | 0.99 | 89 | 94 | 0.90 | 32 | 82 | 0.96 | 72 | 85 |
| Cyfra SLPI CEACAM5 MMP2 MDK | 5 | 0.98 | 94 | 94 | 0.99 | 89 | 94 | 0.93 | 60 | 88 | 0.96 | 91 | 81 |
| Cyfra SLPI TIMP1 CEACAM5 MMP2 MDK | 6 | 0.98 | 92 | 94 | 0.99 | 89 | 94 | 0.91 | 46 | 80 | 0.96 | 81 | 81 |
| Cyfra SLPI TIMP1 CEACAM5 OPN MDK | 6 | 0.98 | 94 | 94 | 0.99 | 89 | 94 | 0.91 | 50 | 80 | 0.96 | 85 | 93 |
| Cyfra TFPI CEACAM5 MDK | 4 | 0.98 | 94 | 94 | 0.99 | 91 | 91 | 0.82 | 62 | 18 | 0.95 | 75 | 91 |
| Cyfra SLPI TIMP1 CEACAM5 MMP2 OPN MDK | 7 | 0.98 | 92 | 94 | 0.99 | 89 | 94 | 0.92 | 58 | 78 | 0.96 | 89 | 91 |
| Cyfra SLPI CEACAM5 MDK | 4 | 0.98 | 94 | 94 | 0.99 | 89 | 94 | 0.91 | 46 | 88 | 0.95 | 87 | 85 |
| Cyfra SLPI CEACAM5 OPN MDK | 5 | 0.98 | 94 | 94 | 0.99 | 89 | 94 | 0.92 | 66 | 84 | 0.94 | 74 | 72 |
| Cyfra CEACAM5 MMP2 OPN MDK | 5 | 0.98 | 94 | 94 | 0.99 | 89 | 94 | 0.88 | 54 | 28 | 0.93 | 47 | 74 |
| Cyfra TFPI CEACAM5 MMP2 MDK | 5 | 0.98 | 92 | 92 | 0.99 | 92 | 91 | 0.85 | 72 | 18 | 0.95 | 79 | 89 |
| Cyfra CEACAM5 MMP2 MDK | 4 | 0.98 | 94 | 94 | 0.99 | 89 | 91 | 0.86 | 34 | 30 | 0.93 | 64 | 54 |
| Cyfra CEACAM5 OPN MDK | 4 | 0.98 | 92 | 94 | 0.99 | 89 | 94 | 0.85 | 44 | 30 | 0.93 | 87 | 76 |
| Cyfra SLPI TFPI CEACAM5 MMP2 MDK | 6 | 0.98 | 90 | 90 | 0.99 | 92 | 93 | 0.90 | 84 | 54 | 0.97 | 92 | 81 |
| Cyfra SLPI TFPI CEACAM5 MDK | 5 | 0.98 | 92 | 92 | 0.99 | 92 | 94 | 0.89 | 82 | 56 | 0.96 | 91 | 85 |
| Cyfra CEACAMS MDK | 3 | 0.98 | 92 | 94 | 0.99 | 89 | 91 | 0.80 | 20 | 32 | 0.93 | 51 | 57 |
| Cyfra TIMP1 CEACAM5 MMP2 OPN MDK | 6 | 0.98 | 90 | 92 | 0.99 | 87 | 94 | 0.88 | 74 | 26 | 0.96 | 77 | 93 |
| Cyfra TIMP1 CEACAM5 MMP2 MDK | 5 | 0.98 | 90 | 92 | 0.99 | 87 | 94 | 0.87 | 64 | 28 | 0.96 | 85 | 85 |
| Cyfra SLPI TIMP1 SCC TFPI CEACAM5 MDK | 7 | 0.98 | 80 | 92 | 1.00 | 98 | 98 | 0.89 | 36 | 46 | 0.98 | 79 | 93 |
| Cyfra SLPI SCC TFPI CEACAM5 MDK | 6 | 0.98 | 78 | 92 | 1.00 | 98 | 98 | 0.88 | 50 | 52 | 0.97 | 89 | 93 |
| Cyfra SLPI TIMP1 SCC TFPI CEACAM5 MMP2 MDK | 8 | 0.98 | 84 | 90 | 1.00 | 98 | 98 | 0.90 | 48 | 46 | 0.98 | 83 | 93 |
| Cyfra SLPI TIMP1 TFPI CEACAM5 MMP2 MDK | 7 | 0.98 | 86 | 90 | 0.99 | 92 | 94 | 0.90 | 68 | 48 | 0.97 | 87 | 81 |
| Cyfra TIMP1 CEACAM5 OPN MDK | 5 | 0.98 | 92 | 90 | 0.99 | 87 | 94 | 0.86 | 62 | 26 | 0.95 | 70 | 94 |
| Cyfra MDK | 2 | 0.98 | 90 | 88 | 0.98 | 91 | 87 | 0.74 | 64 | 36 | 0.91 | 77 | 72 |
| Cyfra SLPI SCC CEACAM5 MMP2 MDK | 6 | 0.98 | 82 | 92 | 0.99 | 98 | 98 | 0.90 | 66 | 82 | 0.97 | 83 | 94 |
| Cyfra SLPI SCC TFPI CEACAM5 MMP2 MDK | 7 | 0.98 | 82 | 92 | 0.99 | 98 | 98 | 0.89 | 58 | 50 | 0.98 | 91 | 93 |
| Cyfra TIMP1 CEACAM5 MDK | 4 | 0.98 | 92 | 90 | 0.99 | 87 | 94 | 0.84 | 52 | 28 | 0.95 | 81 | 87 |
| Cyfra SLPI TIMP1 SCC CEACAM5 MMP2 MDK | 7 | 0.98 | 84 | 92 | 0.99 | 98 | 98 | 0.90 | 52 | 76 | 0.97 | 72 | 94 |
| Cyfra SLPI SCC CEACAM5 MMP2 OPN MDK | 7 | 0.98 | 82 | 90 | 0.99 | 98 | 98 | 0.91 | 74 | 78 | 0.96 | 92 | 85 |
| Cyfra SLPI TIMP1 SCC CEACAM5 MMP2 OPN MDK | 8 | 0.98 | 82 | 90 | 0.99 | 98 | 98 | 0.90 | 64 | 74 | 0.97 | 87 | 94 |
| Cyfra SLPI SCC CEACAM5 MDK | 5 | 0.98 | 80 | 92 | 1.00 | 98 | 98 | 0.89 | 56 | 82 | 0.97 | 77 | 94 |
| Cyfra SLPI TIMP1 SCC CEACAM5 MDK | 6 | 0.98 | 80 | 92 | 0.99 | 98 | 98 | 0.89 | 38 | 78 | 0.96 | 64 | 94 |
| Cyfra SLPI TIMP1 TFPI CEACAM5 MDK | 6 | 0.98 | 84 | 90 | 0.99 | 91 | 94 | 0.90 | 62 | 48 | 0.97 | 85 | 85 |
| Cyfra SLPI SCC CEACAM5 OPN MDK | 6 | 0.98 | 78 | 92 | 0.99 | 98 | 98 | 0.90 | 72 | 78 | 0.96 | 91 | 87 |
| Cyfra SLPI TIMP1 SCC CEACAM5 OPN MDX | 7 | 0.98 | 78 | 92 | 0.99 | 98 | 98 | 0.90 | 56 | 76 | 0.96 | 81 | 94 |
| Cyfra SCC TFPI CEACAM5 MMP2 MDK | 6 | 0.97 | 78 | 88 | 0.99 | 96 | 96 | 0.85 | 38 | 16 | 0.97 | 94 | 74 |
| Cyfra OPN MDK | 3 | 0.97 | 92 | 92 | 0.98 | 89 | 94 | 0.79 | 36 | 34 | 0.89 | 57 | 43 |
| Cyfra MMP2 MDK | 3 | 0.97 | 92 | 94 | 0.98 | 91 | 83 | 0.82 | 74 | 34 | 0.92 | 32 | 69 |
| Cyfra SCC TFPI CEACAM5 MDK | 5 | 0.97 | 78 | 88 | 0.99 | 96 | 98 | 0.81 | 26 | 16 | 0.97 | 94 | 76 |
| Cyfra MMP2 OPN MDK | 4 | 0.97 | 94 | 94 | 0.98 | 91 | 93 | 0.83 | 46 | 32 | 0.90 | 64 | 41 |
| Cyfra SCC CEACAM5 MMP2 OPN MDK | 6 | 0.97 | 80 | 90 | 0.99 | 98 | 98 | 0.86 | 62 | 26 | 0.95 | 81 | 87 |
| Cyfra TIMP1 SCC TFPI CEACAM5 MMP2 MDK | 7 | 0.97 | 80 | 86 | 1.00 | 98 | 98 | 0.87 | 60 | 16 | 0.98 | 85 | 93 |
| Cyfra SCC CEACAM5 MMP2 MDK | 5 | 0.97 | 80 | 90 | 0.99 | 96 | 98 | 0.84 | 48 | 28 | 0.95 | 87 | 74 |
| Cyfra TIMP1 SCC CEACAM5 MMP2 OPN MDK | 7 | 0.97 | 84 | 86 | 0.99 | 98 | 98 | 0.86 | 48 | 24 | 0.97 | 94 | 83 |
| Cyfra TIMP1 SCC CEACAM5 MMP2 MDK | 6 | 0.97 | 82 | 88 | 0.99 | 98 | 98 | 0.85 | 32 | 26 | 0.96 | 7S | 94 |
| Cyfra SCC CEACAM5 MDK | 4 | 0.97 | 80 | 90 | 0.99 | 96 | 98 | 0.79 | 34 | 30 | 0.95 | 85 | 76 |
| Cyfra SCC CEACAM5 OPN MDK | 5 | 0.97 | 80 | 90 | 0.99 | 98 | 98 | 0.84 | 52 | 28 | 0.95 | 77 | 89 |
| Cyfra TIMP1 SCC TFPI CEACAM5 MDK | 6 | 0.97 | 74 | 86 | 1.00 | 98 | 98 | 0.85 | 50 | 16 | 0.98 | 83 | 93 |
| Cyfra TIMP1 TFPI CEACAM5 MMP2 MDK | 6 | 0.97 | 80 | 88 | 0.99 | 94 | 94 | 0.88 | 52 | 18 | 0.97 | 89 | 85 |
| Cyfra SLPI SCC TFPI CEACAM5 MMP2 OPN MDK | 8 | 0.97 | 84 | 84 | 0.99 | 98 | 98 | 0.90 | 68 | 48 | 0.98 | 94 | 83 |
| Cyfra TIMP1 TFPI CEACAM5 MDK | 5 | 0.97 | 80 | 88 | 0.99 | 91 | 94 | 0.86 | 38 | 18 | 0.97 | 89 | 87 |
| SLPI CEACAM5 OPN MDK | 4 | 0.97 | 90 | 88 | 0.96 | 85 | 76 | 0.89 | 66 | 76 | 0.90 | 28 | 41 |
| Cyfra TIMP1 SCC CEACAM5 MDK | 5 | 0.97 | 82 | 90 | 0.99 | 98 | 98 | 0.83 | 20 | 26 | 0.96 | 74 | 72 |
| Cyfra TIMP1 SCC CEACAM5 OPN MDK | 6 | 0.97 | 78 | 88 | 0.99 | 98 | 98 | 0.85 | 38 | 24 | 0.96 | 92 | 85 |
| SLPI CEACAM5 MDK | 3 | 0.97 | 86 | 88 | 0.96 | 83 | 70 | 0.87 | 48 | 82 | 0.90 | 40 | 70 |
| SLPI CEACAM5 MMP2 MDK | 4 | 0.97 | 88 | 86 | 0.96 | 83 | 70 | 0.89 | 62 | 80 | 0.91 | 53 | 67 |
| Cyfra SLPI TFPI CEACAM5 MMP2 OPN MDK | 7 | 0.97 | 86 | 84 | 0.99 | 94 | 94 | 0.91 | 68 | 52 | 0.96 | 85 | 69 |
| SLPI CEACAM5 MMP2 OPN MDK | 5 | 0.97 | 90 | 88 | 0.96 | 85 | 70 | 0.90 | 72 | 74 | 0.91 | 42 | 80 |
| Cyfra SCC MDK | 3 | 0.96 | 86 | 80 | 0.98 | 91 | 91 | 0.73 | 20 | 34 | 0.94 | 53 | 91 |
| Cyfra SLPI TFPI CEACAM5 OPN MDK | 6 | 0.96 | 82 | 86 | 0.99 | 92 | 94 | 0.90 | 60 | 54 | 0.96 | 81 | 72 |
| SLPI TIMP1 CEACAM5 OPN MDK | S | 0.96 | 82 | 88 | 0.96 | 91 | 85 | 0.88 | 50 | 68 | 0.93 | 66 | 81 |
| Cyfra TIMP1 MMP2 OPN MDK | 5 | 0.96 | 86 | 74 | 0.99 | 89 | 93 | 0.83 | 66 | 30 | 0.94 | 85 | 80 |
| SLPI TIMP1 CEACAM5 MDK | 4 | 0.96 | 80 | 86 | 0.96 | 91 | 70 | 0.87 | 34 | 72 | 0.93 | 75 | 89 |
| CEACAM5 MMP2 MDK | 3 | 0.96 | 88 | 84 | 0.94 | 81 | 43 | 0.80 | 34 | 0 | 0.85 | 26 | 70 |
| Cyfra SCC MMP2 MDK | 4 | 0.96 | 84 | 80 | 0.99 | 92 | 85 | 0.80 | 32 | 32 | 0.96 | 70 | 89 |
| CEACAM5 MMP2 OPN MDK | 4 | 0.96 | 88 | 82 | 0.95 | 83 | 56 | 0.83 | 56 | 0 | 0.86 | 49 | 44 |
| CEACAM5 OPN MDK | 3 | 0.96 | 88 | 76 | 0.95 | 83 | 61 | 0.79 | 46 | 0 | 0.84 | 36 | 46 |
| Cyfra SLPI MMP2 OPN MDK | 5 | 0.96 | 94 | 68 | 0.99 | 89 | 93 | 0.90 | 64 | 30 | 0.93 | 79 | 78 |
| Cyfra TFPI CEACAM5 MMP2 OPN MDK | 6 | 0.96 | 86 | 82 | 0.99 | 91 | 91 | 0.88 | 80 | 16 | 0.95 | 66 | 74 |
| Cyfra SLPI MMP2 MDK | 4 | 0.96 | 94 | 68 | 0.99 | 91 | 93 | 0.91 | 50 | 32 | 0.94 | 66 | 67 |
| CEACAM5 MDK | 2 | 0.96 | 90 | 74 | 0.94 | 81 | 43 | 0.73 | 20 | 0 | 0.83 | 0 | 76 |
| Cyfra TFPI CEACAM5 OPN MDK | 5 | 0.96 | 86 | 82 | 0.99 | 91 | 91 | 0.86 | 76 | 16 | 0.95 | 60 | 76 |
| Cyfra TIMP1 MMP2 MDK | 4 | 0.96 | 86 | 70 | 0.98 | 89 | 93 | 0.82 | 54 | 32 | 0.95 | 74 | 61 |
| Cyfra SLPI TIMP1 MMP2 OPN MDK | 6 | 0.96 | 92 | 58 | 0.99 | 91 | 93 | 0.89 | 52 | 28 | 0.95 | 74 | 76 |
| Cyfra SLPI SCC TFPI CEACAM5 OPN MDK | 7 | 0.96 | 76 | 78 | 0.99 | 98 | 98 | 0.90 | 64 | 50 | 0.97 | 94 | 85 |
| Cyfra SLPI TIMP1 TFPI CEACAM5 MMP2 OPN MDK | 8 | 0.96 | 80 | 78 | 0.99 | 94 | 94 | 0.91 | 52 | 46 | 0.97 | 91 | 89 |
| Cyfra SLPI OPN MDK | 4 | 0.96 | 94 | 66 | 0.99 | 89 | 93 | 0.90 | 56 | 32 | 0.92 | 75 | 41 |
| SLPI TIMP1 CEACAM5 MMP2 OPN MDK | 6 | 0.96 | 88 | 78 | 0.97 | 92 | 85 | 0.89 | 58 | 68 | 0.94 | 70 | 76 |
| Cyfra SLPI TIMP1 SCC TFPI CEACAM5 MMP2 OPN MDK | 9 | 0.96 | 76 | 84 | 1.00 | 98 | 98 | 0.90 | 58 | 44 | 0.98 | 91 | 94 |
| Cyfra SLPI MDK | 3 | 0.96 | 94 | 66 | 0.99 | 89 | 93 | 0.90 | 32 | 34 | 0.93 | 62 | 69 |
| Cyfra SCC OPN MDK | 4 | 0.96 | 90 | 82 | 0.98 | 94 | 91 | 0.78 | 42 | 32 | 0.94 | 32 | 78 |
| Cyfra SLPI TIMP1 MMP2 MDK | 5 | 0.96 | 92 | 56 | 0.99 | 91 | 93 | 0.89 | 64 | 30 | 0.96 | 87 | 87 |
| Cyfra TIMP1 OPN MDK | 4 | 0.96 | 86 | 70 | 0.98 | 89 | 93 | 0.81 | 58 | 30 | 0.94 | 79 | 81 |
| Cyfra TIMP1 TFPI CEACAM5 OPN MDK | 6 | 0.96 | 78 | 78 | 0.99 | 92 | 94 | 0.88 | 58 | 16 | 0.97 | 87 | 93 |
| SLPI TIMP1 CEACAM5 MMP2 MDK | 5 | 0.96 | 88 | 78 | 0.96 | 89 | 70 | 0.88 | 48 | 72 | 0.94 | 83 | 87 |
| Cyfra TIMP1 TFPI CEACAM5 MMP2 OPN MDK | 7 | 0.95 | 78 | 78 | 0.99 | 92 | 94 | 0.88 | 68 | 16 | 0.97 | 87 | 91 |
| Cyfra SLPI TIMP1 TFPI CEACAM5 OPN MDK | 7 | 0.95 | 78 | 82 | 0.99 | 94 | 94 | 0.90 | 42 | 46 | 0.97 | 91 | 91 |
| Cyfra TFPI MMP2 MOK | 4 | 0.95 | 80 | 78 | 0.99 | 91 | 87 | 0.81 | 58 | 20 | 0.95 | 58 | 69 |
| Cyfra SLPI TIMP1 OPN MDK | 5 | 0.95 | 92 | 58 | 0.99 | 89 | 93 | 0.89 | 46 | 28 | 0.95 | 70 | 80 |
| Cyfra SLPI TIMP1 SCC TFPI CEACAM5 OPN MDK | 8 | 0.95 | 74 | 78 | 0.99 | 98 | 98 | 0.90 | 48 | 44 | 0.97 | 91 | 94 |
| Cyfra SLPI TIMP1 MDK | 4 | 0.95 | 92 | 56 | 0.99 | 89 | 93 | 0.88 | 58 | 30 | 0.95 | 79 | 91 |
| Cyfra SCC MMP2 OPN MDK | 5 | 0.95 | 86 | 70 | 0.99 | 94 | 89 | 0.82 | 52 | 30 | 0.94 | 51 | 76 |
| TIMP1 CEACAM5 OPN MDK | 4 | 0.95 | 80 | 66 | 0.96 | 91 | 76 | 0.81 | 64 | 0 | 0.91 | 32 | 83 |
| Cyfra TFPI MDK | 3 | 0.95 | 78 | 74 | 0.99 | 91 | 87 | 0.77 | 44 | 20 | 0.95 | 89 | 72 |
| Cyfra SCC TFPI CEACAM5 MMP2 OPN MOK | 7 | 0.95 | 78 | 78 | 0.99 | 98 | 98 | 0.87 | 58 | 14 | 0.97 | 92 | 85 |
| TIMP1 CEACAM5 MMP2 OPN MDK | 5 | 0.95 | 80 | 66 | 0.96 | 91 | 78 | 0.83 | 42 | 0 | 0.92 | 42 | 80 |
| Cyfra SLPI TFPI MMP2 MDK | 5 | 0.95 | 84 | 80 | 0.99 | 92 | 93 | 0.88 | 78 | 20 | 0.96 | 75 | 89 |
| Cyfra SLPI TFPI MDK | 4 | 0.95 | 84 | 76 | 0.99 | 94 | 93 | 0.87 | 76 | 20 | 0.96 | 74 | 69 |
| MMP2 MDK | 2 | 0.95 | 86 | 76 | 0.91 | 85 | 22 | 0.73 | 76 | 0 | 0.82 | 34 | 0 |
| Cyfra SLPI SCC MMP2 MOK | S | 0.95 | 92 | 46 | 0.99 | 96 | 96 | 0.88 | 58 | 30 | 0.97 | 92 | 83 |
| Cyfra SCC TFPI CEACAM5 OPN MDK | 6 | 0.95 | 76 | 74 | 0.99 | 98 | 98 | 0.85 | 46 | 14 | 0.97 | 92 | 87 |
| TIMP1 CEACAM5 MDK | 3 | 0.95 | 76 | 68 | 0.96 | 91 | 54 | 0.78 | 54 | 0 | 0.89 | 51 | 69 |
| Cyfra TIMP1 MDK | 3 | 0.95 | 86 | 70 | 0.98 | 89 | 93 | 0.79 | 42 | 32 | 0.94 | 64 | 65 |
| TIMP1 CEACAM5 MMP2 MDK | 4 | 0.95 | 80 | 68 | 0.96 | 91 | 54 | 0.82 | 66 | 0 | 0.91 | 62 | 63 |
| Cyfra SLPI SCC MMP2 OPN MDK | 6 | 0.95 | 90 | 46 | 0.99 | 98 | 96 | 0.88 | 68 | 28 | 0.96 | 79 | 89 |
| Cyfra SLPI TIMP1 SCC MMP2 MDK | 6 | 0.94 | 88 | 56 | 0.99 | 96 | 96 | 0.87 | 46 | 28 | 0.97 | 81 | 94 |
| Cyfra SLPI SCC MDK | 4 | 0.94 | 94 | 48 | 0.99 | 94 | 94 | 0.86 | 44 | 32 | 0.96 | 87 | 87 |
| Cyfra TIMP1 SCC TFPI CEACAM5 MMP2 OPN MOK | 8 | 0.94 | 72 | 72 | 1.00 | 98 | 98 | 0.88 | 44 | 14 | 0.98 | 96 | 96 |
| Cyfra SLPI TIMP1 SCC MMP2 OPN MDK | 7 | 0.94 | 88 | 58 | 0.99 | 98 | 98 | 0.88 | 56 | 26 | 0.96 | 68 | 87 |
| Cyfra TIMP1 SCC MMP2 MDK | 5 | 0.94 | 82 | 66 | 0.99 | 94 | 94 | 0.81 | 26 | 30 | 0.96 | 87 | 83 |
| Cyfra SLPI TIMP1 TFPI MMP2 MDK | 6 | 0.94 | 80 | 62 | 0.99 | 92 | 93 | 0.88 | 58 | 20 | 0.97 | 72 | 87 |
| MMP2 OPN MDK | 3 | 0.94 | 90 | 66 | 0.92 | 81 | 22 | 0.75 | 48 | 0 | 0.80 | 30 | 52 |
| Cyfra TIMP1 SCC MMP2 OPN MDK | 6 | 0.94 | 84 | 76 | 0.99 | 98 | 96 | 0.82 | 44 | 28 | 0.96 | 79 | 87 |
| Cyfra SLPI SCC OPN MDK | 5 | 0.94 | 94 | 44 | 0.99 | 96 | 96 | 0.88 | 64 | 30 | 0.95 | 74 | 76 |
| SLPI SCC CEACAMS MDK | 4 | 0.94 | 70 | 70 | 0.98 | 92 | 87 | 0.85 | 58 | 74 | 0.93 | 81 | 81 |
| SLPI TIMP1 SCC CEACAM5 MDK | 5 | 0.94 | 68 | 70 | 0.98 | 94 | 85 | 0.86 | 40 | 66 | 0.94 | 68 | 78 |
| Cyfra SLPI TIMP1 SCC OPN MDK | 6 | 0.94 | 92 | 58 | 0.99 | 96 | 96 | 0.87 | 50 | 26 | 0.96 | 66 | 89 |
| Cyfra TIMP1 SCC TFPI CEACAM5 OPN MDK | 7 | 0.94 | 72 | 66 | 0.99 | 98 | 98 | 0.87 | 32 | 14 | 0.97 | 96 | 96 |
| OPN MDK | 2 | 0.94 | 84 | 74 | 0.92 | 81 | 41 | 0.69 | 38 | 0 | 0.77 | 62 | 0 |
| Cytra SLPI TIMP1 SCC MDK | 5 | 0.94 | 90 | 58 | 0.99 | 98 | 96 | 0.86 | 32 | 28 | 0.96 | 75 | 83 |
| Cyfra SLPI TIMP1 TFPI MOK | 5 | 0.94 | 76 | 62 | 0.99 | 94 | 93 | 0.88 | 50 | 20 | 0.97 | 66 | 91 |
| Cyfra TIMP1 TFPI MMP2 MDK | 5 | 0.94 | 68 | 64 | 0.99 | 94 | 94 | 0.84 | 42 | 20 | 0.96 | 83 | 93 |
| Cyfra SLPI SCC TFPI MMP2 MDK | 6 | 0.94 | 76 | 74 | 0.99 | 94 | 94 | 0.87 | 50 | 18 | 0.98 | 74 | 94 |
| SLPI SCC CEACAM5 OPN MDK | 5 | 0.94 | 70 | 72 | 0.98 | 94 | 91 | 0.87 | 72 | 68 | 0.93 | 68 | 70 |
| MDK | 1 | 0.94 | 84 | 80 | 0.90 | 77 | 0 | 0.63 | 64 | 0 | 0.79 | 0 | 0 |
| Cyfra TIMP1 SCC OPN MDK | 5 | 0.94 | 88 | 52 | 0.99 | 96 | 96 | 0.80 | 32 | 28 | 0.95 | 75 | 89 |
| Cyfra SLPI TIMP1 SCC TFPI MMP2 MDK | 7 | 0.94 | 74 | 68 | 0.99 | 94 | 94 | 0.87 | 62 | 18 | 0.98 | 91 | 93 |
| SLPI TIMP1 SCC CEACAM5 MMP2 MDK | 6 | 0.94 | 74 | 50 | 0.98 | 92 | 81 | 0.87 | 54 | 66 | 0.95 | 74 | 76 |
| SLPI SCC CEACAM5 MMP2 MOK | 5 | 0.94 | 76 | so | 0.98 | 92 | 81 | 0.87 | 69 | 72 | 0.94 | 85 | 80 |
| Cyfra SLPI TIMP1 CEACAM5 MMP2 | 5 | 0.94 | 68 | 66 | 0.98 | 89 | 87 | 0.83 | 26 | 26 | 0.94 | 43 | 89 |
| Cyfra TIMP1 TFPI MDK | 4 | 0.94 | 68 | 62 | 0.99 | 94 | 94 | 0.82 | 28 | 20 | 0.96 | 79 | 94 |
| Cyfra SLPI TFPI MMP2 OPN MDK | 6 | 0.94 | 76 | 72 | 0.99 | 96 | 96 | 0.89 | 58 | 18 | 0.95 | 68 | 78 |
| SLPI TIMP1 SCC CEACAM5 OPN MDK | 6 | 0.94 | 70 | 72 | 0.98 | 96 | 98 | 0.87 | 56 | 62 | 0.94 | 85 | 89 |
| Cyfra SLPI SCC TFPI MDK | 5 | 0.93 | 76 | 70 | 0.99 | 96 | 96 | 0.85 | 38 | 18 | 0.97 | 70 | 87 |
| Cyfra SLPI TFPI OPN MDK | 5 | 0.93 | 70 | 72 | 0.99 | 94 | 94 | 0.88 | 48 | 18 | 0.95 | 81 | 81 |
| Cyfra SLPI TIMP1 CEACAM5 MMP2 OPN | 6 | 0.93 | 68 | 66 | 0.98 | 89 | 87 | 0.86 | 44 | 24 | 0.94 | 74 | 72 |
| SLPI TIMP1 SCC CEACAM5 MMP2 OPN MDK | 7 | 0.93 | 76 | 54 | 0.99 | 96 | 96 | 0.88 | 64 | 62 | 0.95 | 91 | 89 |
| Cyfra SLPI TIMP1 SCC TFPI MDK | 6 | 0.93 | 74 | 70 | 0.99 | 94 | 94 | 0.87 | 56 | 18 | 0.97 | 87 | 93 |
| SLPI SCC CEACAM5 MMP2 OPN MDK | 6 | 0.93 | 78 | 52 | 0.98 | 94 | 93 | 0.88 | 74 | 66 | 0.94 | 72 | 93 |
| Cyfra TIMP1 SCC MDK | 4 | 0.93 | 84 | 44 | 0.99 | 92 | 91 | 0.78 | 14 | 30 | 0.95 | 87 | 85 |
| Cyfra SCCTFPI MMP2 MDK | 5 | 0.93 | 76 | 72 | 0.99 | 98 | 96 | 0.81 | 66 | 18 | 0.97 | 89 | 89 |
| Cyfra TIMP1 CEACAM5 MMP2 OPN | 5 | 0.93 | 68 | 66 | 0.98 | 89 | 89 | 0.80 | 52 | 26 | 0.92 | 43 | 76 |
| SCC CEACAM5 MMP2 OPN MDK | 5 | 0.93 | 72 | 66 | 0.98 | 92 | 87 | 0.82 | 64 | 0 | 0.91 | 43 | 69 |
| SCC CEACAM5 MDK | 3 | 0.93 | 70 | 62 | 0.97 | 89 | 91 | 0.72 | 34 | 0 | 0.86 | 42 | 57 |
| Cyfra TIMP1 CEACAM5 MMP2 | 4 | 0.93 | 68 | 66 | 0.98 | 89 | 87 | 0.75 | 32 | 28 | 0.93 | 58 | 48 |
| SCC CEACAM5 OPN MDK | 4 | 0.93 | 70 | 62 | 0.98 | 92 | 91 | 0.78 | 54 | 0 | 0.89 | 19 | 72 |
| SCC CEACAM5 MMP2 MDK | 4 | 0.93 | 70 | 68 | 0.98 | 92 | 83 | 0.79 | 48 | 0 | 0.90 | 64 | 52 |
| Cyfra TFPI MMP2 OPN MDK | 5 | 0.93 | 72 | 74 | 0.99 | 91 | 94 | 0.84 | 70 | 18 | 0.94 | 47 | 85 |
| TIMP1 SCC CEACAM5 OPN MDK | 5 | 0.93 | 70 | 48 | 0.98 | 94 | 93 | 0.81 | 40 | 0 | 0.92 | 68 | 69 |
| TIMP1 SCC CEACAM5 MDK | 4 | 0.93 | 70 | 40 | 0.98 | 92 | 83 | 0.78 | 20 | 0 | 0.91 | 75 | 56 |
| Cyfra SLPI TIMP1 TFPI OPN MOK | 6 | 0.93 | 70 | 70 | 0.99 | 96 | 96 | 0.89 | 40 | 18 | 0.97 | 77 | 80 |
| TIMP1 SCC CEACAM5 MMP2 MDK | 5 | 0.93 | 70 | 54 | 0.98 | 92 | 83 | 0.81 | 32 | 0 | 0.92 | 77 | 78 |
| Cyfra TFPI OPN MDK | 4 | 0.93 | 68 | 74 | 0.99 | 91 | 94 | 0.83 | 64 | 18 | 0.94 | 74 | 87 |
| Cyfra SLPI TIMP1 SCC CEACAM5 MMP2 OPN | 7 | 0.93 | 64 | 70 | 0.99 | 92 | 91 | 0.85 | 46 | 22 | 0.95 | 66 | 85 |
| Cyfra SLPI TIMP1 TFPI MMP2 OPN MDK | 7 | 0.93 | 68 | 66 | 0.99 | 96 | 96 | 0.89 | 48 | 18 | 0.97 | 83 | 76 |
| Cyfra TIMP1 SCC TFPI MMP2 MDK | 6 | 0.93 | 72 | 58 | 0.99 | 96 | 96 | 0.83 | 48 | 18 | 0.98 | 75 | 94 |
| TIMP1 SCC CEACAM5 MMP2 OPN MDK | 6 | 0.93 | 72 | 52 | 0.98 | 94 | 93 | 0.82 | 50 | 0 | 0.94 | 74 | 65 |
| Cyfra SLPI TIMP1 SCC TFPI MMP2 OPN MDK | 8 | 0.93 | 72 | 68 | 0.99 | 98 | 98 | 0.88 | 52 | 16 | 0.98 | 83 | 85 |
| SLPI TIMP1 OPN MDK | 4 | 0.93 | 86 | 70 | 0.95 | 89 | 50 | 0.85 | 46 | 0 | 0.91 | 75 | 44 |
| Cyfra TIMP1 SCC CEACAM5 MMD2 | 5 | 0.93 | 62 | 66 | 0.99 | 92 | 93 | 0.76 | 14 | 26 | 0.93 | 55 | 78 |
| Cyfra SLPI TIMP1 SCC CEACAM5 MMP2 | 6 | 0.93 | 62 | 68 | 0.99 | 92 | 89 | 0.83 | 36 | 24 | 0.95 | 40 | 74 |
| Cyfra TIMP1 TFPI MMP2 OPN MDK | 6 | 0.93 | 66 | 66 | 0.99 | 92 | 94 | 0.85 | 58 | 18 | 0.97 | 74 | 80 |
| Cyfra SLPI SCC TFPI MMP2 OPN MDK | 7 | 0.93 | 76 | 72 | 0.99 | 98 | 98 | 0.88 | 60 | 16 | 0.97 | 85 | 87 |
| SLPI TIMP1 MDK | 3 | 0.93 | 84 | 70 | 0.95 | 89 | 54 | 0.84 | 58 | 0 | 0.91 | 57 | 78 |
| Cyfra SCC TFPI MDK | 4 | 0.92 | 76 | 68 | 0.99 | 98 | 96 | 0.77 | 54 | 18 | 0.97 | 85 | 91 |
| Cyfra TIMP1 SCC CEACAM5 MMP2 OPN | 6 | 0.92 | 62 | 66 | 0.99 | 92 | 93 | 0.80 | 30 | 24 | 0.94 | 75 | 85 |
| SLPI OPN MDK | 3 | 0.92 | 90 | 14 | 0.94 | 85 | 33 | 0.85 | 56 | 0 | 0.86 | 53 | 48 |
| SLPI MOK | 2 | 0.92 | 92 | 8 | 0.94 | 85 | 54 | 0.86 | 34 | 0 | 0.88 | 66 | 0 |
| Cyfra SLPI SCC TFPI OPN MDK | 6 | 0.92 | 72 | 70 | 0.99 | 98 | 98 | 0.88 | 54 | 16 | 0.97 | 81 | 89 |
| Cyfra TIMP1 TFPI OPN MOK | 5 | 0.92 | 66 | 72 | 0.99 | 92 | 94 | 0.84 | 50 | 18 | 0.97 | 91 | 81 |
| Cyfra TIMP1 SCC TFPI MOK | 5 | 0.92 | 68 | 64 | 0.99 | 98 | 96 | 0.81 | 38 | 18 | 0.97 | 74 | 85 |
| Cyfra SLPI TIMP1 CEACAM5 | 4 | 0.92 | 68 | 44 | 0.98 | 89 | 91 | 0.81 | 16 | 26 | 0.94 | 32 | 93 |
| SLPI TIMP1 MMP2 OPN MDK | 5 | 0.92 | 86 | 36 | 0.96 | 87 | 85 | 0.85 | 52 | 0 | 0.92 | 77 | 44 |
| SLPI TIMP1 MMP2 MDK | 4 | 0.92 | 86 | 36 | 0.96 | 85 | 65 | 0.85 | 44 | 0 | 0.92 | 62 | 74 |
| TFPI CEACAM5 MMP2 MDK | 4 | 0.92 | 74 | 72 | 0.97 | 81 | 81 | 0.81 | 74 | 0 | 0.90 | 53 | 70 |
| Cyfra SLPI TIMP1 SCC TFPI OPN MDK | 7 | 0.92 | 70 | 64 | 0.99 | 98 | 98 | 0.88 | 44 | 16 | 0.97 | 75 | 87 |
| SLPI MMP2 OPN MDK | 4 | 0.92 | 90 | 16 | 0.95 | 83 | 63 | 0.86 | 64 | 0 | 0.87 | 58 | 48 |
| TFPI CEACAM5 MDK | 3 | 0.92 | 74 | 72 | 0.97 | 83 | 81 | 0.76 | 64 | 0 | 0.90 | 42 | 76 |
| Cyfra SLPI TIMP1 CEACAM5 OPN | 5 | 0.92 | 68 | 44 | 0.98 | 89 | 89 | 0.84 | 28 | 24 | 0.94 | 68 | 76 |
| SLPI TIMP1 SCC OPN MDK | 5 | 0.92 | 86 | 46 | 0.97 | 94 | 65 | 0.83 | 50 | 0 | 0.93 | 72 | 81 |
| SLPI MMP2 MDK | 3 | 0.92 | 90 | 6 | 0.95 | 83 | S9 | 0.86 | 52 | 0 | 0.90 | 72 | 80 |
| SLPI SCC MDK | 3 | 0.92 | 80 | 26 | 0.96 | 92 | 74 | 0.81 | 46 | 0 | 0.93 | 42 | 59 |
| Cyfra SCC TFPI MMP2 OPN MDK | 6 | 0.92 | 70 | 64 | 0.99 | 98 | 98 | 0.84 | 44 | 16 | 0.97 | 75 | 91 |
| Cyfra TIMP1 CEACAM5 | 3 | 0.92 | 70 | 44 | 0.98 | 89 | 91 | 0.71 | 18 | 28 | 0.92 | 45 | 52 |
| SLPI TIMP1 SCC MDK | 4 | 0.91 | 84 | 40 | 0.97 | 91 | 69 | 0.82 | 34 | 0 | 0.93 | 36 | S7 |
| SLPI SCC OPN MDK | 4 | 0.91 | 80 | 28 | 0.97 | 94 | 65 | 0.83 | 64 | 0 | 0.92 | 79 | 83 |
| Cyfra SLPI TIMP1 SCC CEACAM5 | 5 | 0.91 | 56 | 56 | 0.99 | 94 | 94 | 0.82 | 28 | 24 | 0.94 | 17 | 78 |
| TFPI CEACAM5 MMP2 OPN MDK | 5 | 0.91 | 68 | 58 | 0.97 | 85 | 87 | 0.84 | S2 | 0 | 0.91 | 72 | 80 |
| Cyfra SLPI TIMP1 SCC CEACAM5 OPN | 6 | 0.91 | 54 | 58 | 0.99 | 94 | 94 | 0.84 | 32 | 22 | 0.94 | 58 | 87 |
| Cyfra TlMP1 SCC TFPI MMP2 OPN MDK | 7 | 0.91 | 68 | 62 | 0.99 | 98 | 98 | 0.84 | 58 | 16 | 0.98 | 91 | 85 |
| Cyfra TIMP1 CEACAM5 OPN | 4 | 0.91 | 68 | 44 | 0.98 | 89 | 91 | 0.78 | 40 | 26 | 0.92 | 79 | 78 |
| TFPI CEACAMS OPN MDK | 4 | 0.91 | 68 | 56 | 0.97 | 87 | 83 | 0.81 | 76 | 0 | 0.91 | 68 | 83 |
| SCC MOK | 2 | 0.91 | 80 | 54 | 0.94 | 83 | 24 | 0.63 | 20 | 0 | 0.84 | 62 | 63 |
| SLPI TFPI CEACAM5 MMP2 OPN MDK | 6 | 0.91 | 70 | 62 | 0.98 | 81 | 87 | 0.88 | 68 | 38 | 0.94 | 62 | 78 |
| SLPI TFPI CEACAM5 MDK | 4 | 0.91 | 70 | 58 | 0.97 | 83 | 85 | 0.85 | 42 | 42 | 0.94 | 66 | 70 |
| SLPI TFPI CEACAM5 MMP2 MDK | 5 | 0.91 | 70 | 60 | 0.98 | 83 | 85 | 0.86 | 54 | 42 | 0.94 | 72 | 67 |
| SLPI TFPI CEACAM5 OPN MDK | 5 | 0.91 | 70 | 54 | 0.98 | 83 | 89 | 0.87 | 60 | 38 | 0.94 | 57 | 83 |
| Cyfra TIMP1 SCC CEACAM5 OPN | 5 | 0.91 | 54 | 58 | 0.99 | 96 | 96 | 0.78 | 22 | 24 | 0.93 | 70 | 87 |
| TIMP1 MMP2 OPN MDK | 4 | 0.91 | 82 | 52 | 0.95 | 89 | 72 | 0.76 | 66 | 0 | 0.89 | 58 | 46 |
| Cyfra TIMP1 SCC TFPI OPN MDK | 6 | 0.91 | 68 | 56 | 0.99 | 98 | 98 | 0.83 | 54 | 16 | 0.97 | 89 | 87 |
| SCC MMP2 MDK | 3 | 0.91 | 80 | 30 | 0.95 | 92 | 44 | 0.72 | 32 | 0 | 0.89 | 77 | 57 |
| Cyfra TIMP1 SCC CEACAM5 | 4 | 0.91 | 56 | 54 | 0.99 | 96 | 96 | 0.73 | 8 | 26 | 0.92 | 30 | 80 |
| Cyfra SCC TFPI OPN MDK | 5 | 0.91 | 66 | 58 | 0.99 | 98 | 98 | 0.82 | 32 | 16 | 0.97 | 70 | 93 |
| SCC TFPI CEACAM5 MMP2 MDK | 5 | 0.91 | 74 | 42 | 0.98 | 94 | 91 | 0.81 | 38 | 0 | 0.94 | 75 | 81 |
| Cyfra SLPI TIMP1 TFPI CEACAM5 MMP2 | 6 | 0.91 | 58 | 58 | 0.99 | 85 | 91 | 0.82 | 52 | 18 | 0.96 | 64 | 87 |
| Cyfra SLPI CEACAM5 MMP2 OPN | 5 | 0.91 | 68 | 58 | 0.96 | 85 | 72 | 0.84 | 54 | 26 | 0.90 | 42 | 33 |
| TIMP1 MMP2 MOK | 3 | 0.91 | 80 | 48 | 0.94 | 89 | 26 | 0.75 | 56 | 0 | 0.89 | 81 | 0 |
| SCC TFPI CEACAM5 MDK | 4 | 0.91 | 74 | 36 | 0.98 | 94 | 91 | 0.76 | 26 | 0 | 0.93 | 72 | 81 |
| Cyfra SLPI CEACAM5 MMP2 | 4 | 0.91 | 66 | 58 | 0.96 | 85 | 72 | 0.78 | 38 | 28 | 0.91 | 53 | 54 |
| SCC OPN MDK | 3 | 0.91 | 82 | 46 | 0.95 | 89 | 83 | 0.70 | 44 | 0 | 0.86 | 38 | 37 |
| Cyfra TIMP1 TFPI CEACAM5 MMP2 | 5 | 0.91 | 56 | 56 | 0.99 | 85 | 91 | 0.76 | 28 | 18 | 0.96 | 75 | 91 |
| SLPI TIMP1 SCC MMP2 MDK | 5 | 0.90 | 82 | 20 | 0.98 | 89 | 81 | 0.84 | 48 | 0 | 0.95 | 55 | 89 |
| Cyfra SLPI TIMP1 TFPI CEACAM5 MMP2 OPN | 7 | 0.90 | 62 | 50 | 0.99 | 85 | 93 | 0.85 | 40 | 16 | 0.96 | 83 | 70 |
| Cyfra SLPI TIMP1 MMP2 OPN | 5 | 0.90 | 70 | 52 | 0.97 | 81 | 83 | 0.83 | 40 | 28 | 0.91 | 49 | 41 |
| Cyfra TIMP1 TFPI CEACAM5 MMP2 OPN | 6 | 0.90 | 62 | 50 | 0.99 | 85 | 93 | 0.82 | 46 | 16 | 0.96 | 64 | 74 |
| SLPI SCC MMP2 MDK | 4 | 0.90 | 84 | 22 | 0.98 | 91 | 76 | 0.83 | 60 | 0 | 0.95 | 58 | 93 |
| Cyfra SLPI TIMP1 MMP2 | 4 | 0.90 | 68 | 52 | 0.97 | 81 | 83 | 0.79 | 48 | 30 | 0.92 | 62 | 67 |
| SLPI TFPI OPN MDK | 4 | 0.90 | 66 | 58 | 0.98 | 87 | 87 | 0.85 | 48 | 0 | 0.91 | 70 | 48 |
| SLPI SCC MMP2 OPN MDK | 5 | 0.90 | 88 | 20 | 0.98 | 92 | 93 | 0.84 | 68 | 0 | 0.93 | 83 | 78 |
| TIMP1 OPN MDK | 3 | 0.90 | 78 | 18 | 0.95 | 87 | 61 | 0.73 | 58 | 0 | 0.87 | 53 | 48 |
| SCC MMP2 OPN MDK | 4 | 0.90 | 80 | 22 | 0.97 | 92 | 72 | 0.74 | 54 | 0 | 0.89 | 55 | 81 |
| TIMP1 SCC TFPI CEACAM5 MMP2 MDK | 6 | 0.90 | 70 | 38 | 0.99 | 96 | 98 | 0.83 | 26 | 0 | 0.96 | 87 | 76 |
| TIMP1 TFPI CEACAM5 MDK | 4 | 0.90 | 60 | 52 | 0.98 | 92 | 85 | 0.82 | 40 | 0 | 0.94 | 70 | 69 |
| SLPI TIMP1 SCC MMP2 OPN MDK | 6 | 0.90 | 80 | 14 | 0.98 | 89 | 87 | 0.84 | 56 | 0 | 0.94 | 72 | 76 |
| TIMP1 TFPI CEACAM5 MMP2 MOK | 5 | 0.90 | 60 | 52 | 0.98 | 92 | 85 | 0.84 | 54 | 0 | 0.94 | 75 | 63 |
| Cyfra MMP2 OPN | 3 | 0.90 | 72 | 46 | 0.91 | 68 | 54 | 0.67 | 54 | 32 | 0.77 | 30 | 44 |
| TIMP1 SCC MMP2 MDK | 4 | 0.90 | 78 | 14 | 0.96 | 89 | 44 | 0.74 | 26 | 0 | 0.92 | 74 | 56 |
| Cyfra SLPI CEACAM5 OPN | 4 | 0.90 | 68 | 46 | 0.96 | 89 | 65 | 0.82 | 44 | 28 | 0.90 | 75 | 33 |
| SLPI TFPI MMP2 OPN MOK | 5 | 0.90 | 66 | 64 | 0.98 | 87 | 87 | 0.85 | 58 | 0 | 0.92 | 74 | 48 |
| TIMP1 TFPI CEACAM5 MMP2 OPN MDK | 6 | 0.90 | 62 | 48 | 0.99 | 92 | 87 | 0.85 | 68 | 0 | 0.94 | 68 | 78 |
| Cyfra OPN | 2 | 0.90 | 56 | 70 | 0.91 | 66 | 65 | 0.61 | 44 | 34 | 0.73 | 0 | 46 |
| TFPI OPN MOK | 3 | 0.90 | 64 | 62 | 0.97 | 89 | 80 | 0.76 | 64 | 0 | 0.89 | 40 | 54 |
| TIMP1 SCC OPN MDK | 4 | 0.90 | 78 | 16 | 0.96 | 89 | 89 | 0.73 | 34 | 0 | 0.91 | 32 | 81 |
| TIMP1 TFPI CEACAM5 OPN MOK | 5 | 0.90 | 64 | 48 | 0.99 | 92 | 89 | 0.84 | 58 | 0 | 0.94 | 58 | 81 |
| SLPI TIMP1 SCC TFPI CEACAM5 MDK | 6 | 0.90 | 72 | 40 | 0.99 | 96 | 98 | 0.86 | 38 | 34 | 0.96 | 81 | 94 |
| TIMP1 SCC TFPI CEACAM5 MDK | 5 | 0.90 | 68 | 38 | 0.99 | 96 | 98 | 0.81 | 52 | 0 | 0.95 | 85 | 76 |
| TIMP1 SCC MMP2 OPN MDK | 5 | 0.90 | 78 | 16 | 0.97 | 91 | 83 | 0.76 | 46 | 0 | 0.93 | 51 | 78 |
| SLPI TFPI MDK | 3 | 0.90 | 64 | 60 | 0.98 | 85 | 87 | 0.82 | 28 | 0 | 0.94 | 77 | 83 |
| SLPI TFPI MMP2 MDK | 4 | 0.90 | 64 | 60 | 0.98 | 85 | 87 | 0.83 | 44 | 0 | 0.93 | 55 | 80 |
| SLPI TIMP1 TFPI CEACAM5 MDK | 5 | 0.90 | 68 | 52 | 0.98 | 92 | 89 | 0.87 | 62 | 38 | 0.96 | 87 | 87 |
| SLPI TIMP1 TFPI CEACAM5 MMP2 MDK | 6 | 0.90 | 68 | 52 | 0.98 | 92 | 87 | 0.87 | 42 | 38 | 0.96 | 87 | 85 |
| SLPI SCC TFPI CEACAM5 MMP2 MDK | 6 | 0.90 | 72 | 38 | 0.99 | 96 | 98 | 0.85 | 60 | 36 | 0.96 | 91 | 78 |
| Cyfra CEACAM5 MMP2 OPN | 4 | 0.90 | 68 | 58 | 0.94 | 79 | 70 | 0.75 | 28 | 28 | 0.84 | 55 | 33 |
| SLPI TIMP1 CEACAM5 MMP2 OPN | 5 | 0.90 | 62 | 42 | 0.94 | 81 | 76 | 0.81 | 46 | 0 | 0.91 | 75 | 80 |
| SLPI TIMP1 SCC TFPI CEACAM5 MMP2 MDK | 7 | 0.90 | 72 | 42 | 0.99 | 96 | 98 | 0.87 | 50 | 34 | 0.97 | 83 | 94 |
| Cyfra SLPI SCC CEACAM5 MMP2 OPN | 6 | 0.90 | 56 | 50 | 0.97 | 85 | 89 | 0.83 | 58 | 24 | 0.93 | 74 | 65 |
| SLPI SCC TFPI CEACAM5 MDK | 5 | 0.90 | 72 | 40 | 0.99 | 96 | 98 | 0.84 | 52 | 36 | 0.96 | 91 | 80 |
| TFPI MMP2 OPN MDK | 4 | 0.89 | 60 | 56 | 0.97 | 89 | 85 | 0.79 | 70 | 0 | 0.88 | 49 | 52 |
| Cyfra SLPI TIMP1 SCC TFPI CEACAM5 MMP2 | 7 | 0.89 | 60 | 58 | 0.99 | 96 | 96 | 0.82 | 32 | 48 | 0.97 | 58 | 94 |
| TIMP1 MDK | 2 | 0.89 | 80 | 22 | 0.93 | 89 | 26 | 0.70 | 44 | 0 | 0.86 | 74 | 0 |
| TFPI MDK | 2 | 0.89 | 62 | 58 | 0.96 | 83 | 83 | 0.69 | 46 | 0 | 0.90 | 57 | 0 |
| TIMP1 SCC MDK | 3 | 0.89 | 74 | 8 | 0.94 | 89 | 44 | 0.70 | 14 | 0 | 0.90 | 53 | 61 |
| Cyfra TIMP1 TFPI CEACAM5 | 4 | 0.89 | 56 | 60 | 0.98 | 85 | 93 | 0.73 | 16 | 18 | 0.96 | 70 | 93 |
| Cyfra SLPI TIMP1 TFPI CEACAM5 | 5 | 0.89 | 56 | 60 | 0.98 | 85 | 93 | 0.80 | 46 | 18 | 0.96 | 58 | 91 |
| Cyfra SLPI CEACAM5 | 3 | 0.89 | 64 | 46 | 0.96 | 89 | 67 | 0.73 | 26 | 30 | 0.91 | 42 | 56 |
| TFPI MMP2 MDK | 3 | 0.89 | 62 | 58 | 0.96 | 83 | 83 | 0.75 | 60 | 0 | 0.89 | 64 | 0 |
| Cyfra SLPI TIMP1 SCC MMP2 OPN | 6 | 0.89 | 70 | 24 | 0.98 | 89 | 87 | 0.82 | 40 | 26 | 0.94 | 40 | 74 |
| SLPI SCC TFPI CEACAM5 OPN MDK | 6 | 0.89 | 70 | 40 | 0.99 | 98 | 98 | 0.87 | 64 | 32 | 0.96 | 83 | 93 |
| SLPI SCC TFPI CEACAM5 MMP2 OPN MDK | 7 | 0.89 | 70 | 38 | 0.99 | 98 | 98 | 0.87 | 68 | 32 | 0.96 | 85 | 93 |
| Cyfra TIMP1 MMP2 OPN | 4 | 0.89 | 72 | 48 | 0.97 | 81 | 83 | 0.74 | 44 | 30 | 0.89 | 58 | 41 |
| Cyfra TIMP1 MMP2 | 3 | 0.89 | 72 | 48 | 0.97 | 81 | 85 | 0.68 | 16 | 32 | 0.90 | 28 | 67 |
| Cyfra TIMP1 SCC TFPI CEACAM5 MMP2 | 6 | 0.89 | 60 | 56 | 0.99 | 98 | 96 | 0.77 | 38 | 16 | 0.96 | 70 | 76 |
| SLPI TIMP1 TFPI CEACAM5 MMP2 OPN MDK | 7 | 0.89 | 64 | 50 | 0.99 | 92 | 94 | 0.88 | 52 | 34 | 0.95 | 83 | 74 |
| Cyfra CEACAM5 MMP2 | 3 | 0.89 | 68 | 54 | 0.94 | 79 | 72 | 0.64 | 6 | 30 | 0.83 | 70 | 54 |
| SLPI TIMP1 CEACAM5 MMP2 | 4 | 0.89 | 58 | 42 | 0.94 | 79 | 81 | 0.77 | 26 | 0 | 0.91 | 49 | 63 |
| SCC TFPI CEACAM5 OPN MDK | 5 | 0.89 | 70 | 30 | 0.99 | 96 | 98 | 0.81 | 48 | 0 | 0.94 | 60 | 70 |
| Cyfra SLPI TFPI CEACAM5 MMP2 | 5 | 0.89 | 56 | 54 | 0.96 | 83 | 69 | 0.78 | 62 | 18 | 0.94 | 68 | 87 |
| SCC TFPI CEACAM5 MMP2 OPN MDK | 6 | 0.89 | 70 | 30 | 0.99 | 96 | 98 | 0.83 | 60 | 0 | 0.95 | 66 | 67 |
| Cyfra SLPI TIMP1 SCC MMP2 | 5 | 0.89 | 66 | 24 | 0.98 | 87 | 85 | 0.79 | 28 | 28 | 0.94 | 53 | 81 |
| Cyfra SLPI SCC CEACAM5 MMP2 | 5 | 0.89 | 54 | 48 | 0.97 | 87 | 87 | 0.78 | 48 | 26 | 0.94 | 49 | 78 |
| Cyfra SLPI TIMP1 TFPI CEACAM5 OPN | 6 | 0.89 | 58 | 60 | 0.98 | 87 | 93 | 0.84 | 26 | 16 | 0.96 | 79 | 74 |
| SLPI TIMP1 TFPI MDK | 4 | 0.89 | 66 | 52 | 0.98 | 92 | 89 | 0.84 | 50 | 0 | 0.95 | 70 | 78 |
| Cyfra TIMP1 TFPI CEACAM5 OPN | 5 | 0.89 | 58 | 60 | 0.98 | 87 | 93 | 0.81 | 32 | 16 | 0.96 | 91 | 76 |
| SLPI TIMP1 TFPI MMP2 OPN MDK | 6 | 0.89 | 64 | 50 | 0.99 | 92 | 94 | 0.86 | 48 | 0 | 0.94 | 87 | 44 |
| SLPI TIMP1 TFPI MMP2 MDK | S | 0.89 | 66 | 52 | 0.98 | 92 | 89 | 0.85 | 36 | 0 | 0.95 | 77 | 74 |
| SLPI TIMP1 TFPI OPN MDK | 5 | 0.89 | 64 | 48 | 0.99 | 92 | 94 | 0.86 | 40 | 0 | 0.94 | 83 | 44 |
| Cyfra MMP2 | 2 | 0.89 | 66 | 36 | 0.91 | 64 | 46 | 0.49 | 24 | 34 | 0.77 | 36 | 0 |
| TIMP1 TFPI MMP2 OPN MDK | 5 | 0.89 | 58 | 44 | 0.98 | 92 | 89 | 0.81 | 58 | 0 | 0.93 | 81 | 46 |
| Cyfra CEACAM5 OPN | 3 | 0.89 | 69 | 48 | 0.95 | 79 | 70 | 0.70 | 16 | 30 | 0.83 | 40 | 35 |
| TIMP1 CEACAM5 MMP2 OPN | 4 | 0.89 | 54 | 42 | 0.94 | 77 | 52 | 0.74 | 20 | 0 | 0.84 | 45 | 41 |
| Cyfra SLPI TFPI CEACAM5 MMP2 OPN | 6 | 0.89 | 60 | 54 | 0.96 | 81 | 76 | 0.83 | 48 | 16 | 0.94 | 60 | 72 |
| TIMP1 TFPI OPN MDK | 4 | 0.89 | 58 | 44 | 0.98 | 92 | 89 | 0.79 | 50 | 0 | 0.93 | 75 | 48 |
| TIMP1 TFPI MMP2 MDK | 4 | 0.89 | 56 | 42 | 0.98 | 92 | 87 | 0.79 | 44 | 0 | 0.93 | 87 | 76 |
| TIMP1 TFPI MDK | 3 | 0.88 | 54 | 42 | 0.98 | 92 | 87 | 0.77 | 30 | 0 | 0.93 | 85 | 81 |
| Cyfra SLPI SCC CEACAM5 OPN | 5 | 0.88 | 54 | 52 | 0.97 | 87 | 89 | 0.81 | 48 | 26 | 0.93 | 68 | 69 |
| SLPI TIMP1 SCC TFPI MMP2 MDK | 6 | 0.88 | 70 | 42 | 0.99 | 96 | 98 | 0.84 | 42 | 0 | 0.97 | 70 | 87 |
| Cyfra TIMP1 SCC MMP2 OPN | 5 | 0.88 | 68 | 44 | 0.98 | 85 | 87 | 0.74 | 28 | 28 | 0.92 | 49 | 78 |
| Cyfra SLPI TIMP1 TFPI MMP2 | 5 | 0.88 | 58 | 48 | 0.98 | 87 | 89 | 0.80 | 40 | 20 | 0.95 | 75 | 67 |
| Cyfra SCC CEACAM5 MMP2 OPN | 5 | 0.88 | 52 | 54 | 0.97 | 81 | 87 | 0.75 | 44 | 26 | 0.90 | 51 | 69 |
| Cyfra TIMP1 TFPI MMP2 | 4 | 0.88 | 58 | 50 | 0.98 | 87 | 89 | 0.72 | 14 | 20 | 0.94 | 55 | 67 |
| SLPI SCC TFPI OPN MDK | 5 | 0.88 | 68 | 38 | 0.99 | 98 | 98 | 0.84 | 54 | 0 | 0.96 | 87 | 81 |
| Cyfra TIMP1 SCC TFPI CEACAM5 MMP2 OPN | 7 | 0.88 | 54 | 52 | 0.99 | 98 | 96 | 0.81 | 24 | 14 | 0.96 | 87 | 85 |
| Cyfra SLPI TIMP1 SCC TFPI CEACAM5 MMP2 OPN | 8 | 0.88 | 54 | 52 | 0.99 | 98 | 96 | 0.84 | 44 | 46 | 0.97 | 79 | 85 |
| Cyfra TIMP1 SCC MMP2 | 4 | 0.88 | 66 | 48 | 0.98 | 85 | 85 | 0.70 | 2 | 30 | 0.92 | 68 | 85 |
| SLPI SCC TFPI MDK | 4 | 0.88 | 70 | 42 | 0.99 | 96 | 98 | 0.81 | 40 | 0 | 0.97 | 74 | 93 |
| SLPI SCC TFPI MMP2 OPN MDK | 6 | 0.88 | 68 | 38 | 0.99 | 98 | 98 | 0.85 | 60 | 0 | 0.96 | 89 | 76 |
| Cyfra SLPI TIMP1 OPN | 4 | 0.88 | 70 | 44 | 0.97 | 81 | 83 | 0.81 | 26 | 28 | 0.90 | 42 | 41 |
| Cyfra SLPI MMP2 OPN | 4 | 0.88 | 68 | 24 | 0.95 | 83 | 65 | 0.80 | 42 | 30 | 0.85 | 58 | 44 |
| SLPI TIMP1 SCC TFPI MMP2 OPN MDK | 7 | 0.88 | 68 | 38 | 0.99 | 100 | 98 | 0.86 | 52 | 0 | 0.96 | 87 | 74 |
| SCC TFPI MMP2 MDK | 4 | 0.88 | 66 | 36 | 0.98 | 91 | 89 | 0.76 | 26 | 0 | 0.95 | 92 | 57 |
| TIMP1 CEACAM5 MMP2 | 3 | 0.88 | 54 | 38 | 0.94 | 83 | 54 | 0.67 | 32 | 0 | 0.82 | 64 | 0 |
| SLPI SCC TFPI MMP2 MDK | 5 | 0.88 | 70 | 42 | 0.99 | 96 | 98 | 0.82 | 52 | 0 | 0.97 | 79 | 91 |
| SLPI TIMP1 SCC TFPI OPN MDK | 6 | 0.88 | 68 | 38 | 0.99 | 100 | 98 | 0.85 | 44 | 0 | 0.96 | 81 | 80 |
| TIMP1 SCC TFPI CEACAM5 MMP2 OPN MDK | 7 | 0.88 | 70 | 36 | 0.99 | 98 | 98 | 0.85 | 46 | 0 | 0.96 | 85 | 91 |
| Cyfra SLPI SCC CEACAM5 | 4 | 0.88 | 52 | 50 | 0.97 | 87 | 89 | 0.74 | 36 | 28 | 0.93 | 23 | 81 |
| SLPI TIMP1 SCC CEACAM5 MMP2 OPN | 6 | 0.88 | 58 | 36 | 0.97 | 87 | 85 | 0.81 | 48 | 0 | 0.93 | 68 | 67 |
| TIMP1 SCC TFPI CEACAM5 OPN MDK | 6 | 0.88 | 70 | 36 | 0.99 | 98 | 98 | 0.84 | 34 | 0 | 0.96 | 83 | 91 |
| SLPI TIMP1 SCC TFPI MDK | 5 | 0.88 | 68 | 42 | 0.99 | 96 | 98 | 0.83 | 56 | 0 | 0.96 | 64 | 89 |
| Cyfra CEACAM5 | 2 | 0.88 | 60 | 48 | 0.94 | 77 | 67 | 0.54 | 0 | 32 | 0.81 | 60 | 57 |
| SLPI TIMP1 CEACAM5 OPN | 4 | 0.88 | 54 | 38 | 0.94 | 74 | 78 | 0.79 | 30 | 0 | 0.90 | 72 | 39 |
| Cyfra SCC CEACAM5 MMP2 | 4 | 0.88 | 52 | 52 | 0.97 | 81 | 78 | 0.68 | 20 | 28 | 0.89 | 66 | 41 |
| Cyfra SLPI TIMP1 TFPI MMP2 OPN | 6 | 0.88 | 56 | 48 | 0.98 | 83 | 89 | 0.83 | 34 | 18 | 0.94 | 66 | 78 |
| Cyfra SLPI TIMP1 | 3 | 0.88 | 70 | 32 | 0.97 | 79 | 83 | 0.77 | 38 | 30 | 0.91 | 55 | 69 |
| Cyfra SLPI MMP2 | 3 | 0.88 | 66 | 24 | 0.95 | 83 | 65 | 0.74 | 64 | 32 | 0.86 | 32 | 72 |
| Cyfra SCC CEACAM5 OPN | 4 | 0.87 | 52 | 56 | 0.97 | 77 | 87 | 0.70 | 28 | 28 | 0.88 | 21 | 70 |
| Cyfra SLPI TIMP1 SCC TFPI CEACAM5 | 6 | 0.87 | 54 | 50 | 0.99 | 98 | 96 | 0.80 | 24 | 48 | 0.96 | 51 | 94 |
| Cyfra TIMP1 SCC TFPI CEACAM5 | 5 | 0.87 | 54 | 50 | 0.99 | 98 | 96 | 0.74 | 26 | 16 | 0.96 | 62 | 78 |
| Cyfra TIMP1 TFPI MMP2 OPN | 5 | 0.87 | 58 | 48 | 0.98 | 83 | 89 | 0.77 | 38 | 18 | 0.94 | 75 | 81 |
| Cyfra | 1 | 0.87 | 46 | 46 | 0.88 | 58 | 0 | 0.35 | 0 | 36 | 0.70 | 0 | 0 |
| TIMP1 SCC CEACAM5 MMP2 OPN | 5 | 0.87 | 48 | 36 | 0.96 | 83 | 80 | 0.74 | 30 | 0 | 0.89 | 42 | 69 |
| Cyfra SLPI TFPI CEACAM5 | 4 | 0.87 | 54 | 44 | 0.96 | 83 | 70 | 0.75 | 58 | 18 | 0.94 | 66 | 91 |
| SCC TFPI MDK | 3 | 0.87 | 66 | 30 | 0.98 | 87 | 89 | 0.70 | 56 | 0 | 0.94 | 91 | 63 |
| SLPI TIMP1 SCC CEACAM5 MMP2 | 5 | 0.87 | 48 | 44 | 0.97 | 85 | 85 | 0.78 | 36 | 0 | 0.92 | 45 | 81 |
| SLPI TIMP1 TFPI CEACAM5 OPN MDK | 6 | 0.87 | 64 | 48 | 0.99 | 92 | 94 | 0.88 | 42 | 34 | 0.95 | 79 | 80 |
| Cyfra SCC MMP2 OPN | 4 | 0.87 | 52 | 32 | 0.94 | 64 | 76 | 0.68 | 36 | 30 | 0.87 | 15 | 80 |
| Cyfra SLPI TFPI CEACAM5 OPN | 5 | 0.87 | 52 | 48 | 0.96 | 81 | 76 | 0.82 | 40 | 16 | 0.94 | 85 | 76 |
| SLP1 TIMP1 SCC TFPI CEACAM5 OPN MDK | 7 | 0.87 | 68 | 38 | 0.99 | 100 | 98 | 0.87 | 48 | 30 | 0.96 | 92 | 89 |
| SCC TFPI MMP2 OPN MDK | 5 | 0.87 | 64 | 30 | 0.99 | 96 | 98 | 0.79 | 46 | 0 | 0.95 | 83 | 80 |
| Cyfra SLPI TIMP1 SCC OPN | 5 | 0.87 | 68 | 18 | 0.98 | 79 | 85 | 0.81 | 28 | 26 | 0.93 | 21 | 78 |
| SCC TFPI OPN MDK | 4 | 0.87 | 64 | 30 | 0.99 | 96 | 98 | 0.76 | 34 | 0 | 0.95 | 79 | 83 |
| TIMP1 SCC TFPI MMP2 MDK | 5 | 0.87 | 68 | 32 | 0.99 | 96 | 96 | 0.79 | 50 | 0 | 0.96 | 79 | 89 |
| Cyfra SLPI TIMP1 SCC | 4 | 0.87 | 66 | 18 | 0.98 | 87 | 85 | 0.78 | 18 | 28 | 0.93 | 34 | 85 |
| Cyfra TFPI CEACAM5 MMP2 | 4 | 0.87 | 56 | 52 | 0.95 | 77 | 76 | 0.69 | 46 | 18 | 0.90 | 49 | 54 |
| TIMP1 SCC TFPI MMP2 OPN MDK | 6 | 0.87 | 68 | 36 | 0.99 | 98 | 98 | 0.81 | 42 | 0 | 0.96 | 74 | 76 |
| SLPI TIMP1 CEACAM5 | 3 | 0.87 | 52 | 38 | 0.94 | 74 | 70 | 0.74 | 16 | 0 | 0.90 | 36 | 67 |
| TIMP1 SCC CEACAM5 MMP2 | 4 | 0.87 | 40 | 34 | 0.96 | 79 | 78 | 0.69 | 14 | 0 | 0.86 | 60 | 52 |
| TIMP1 SCC TFPI OPN MDK | 5 | 0.87 | 68 | 36 | 0.99 | 98 | 98 | 0.79 | 54 | 0 | 0.96 | 70 | 80 |
| Cyfra TFPI CEACAM5 MMP2 OPN | 5 | 0.87 | 54 | 52 | 0.95 | 77 | 78 | 0.79 | 66 | 16 | 0.91 | 70 | 78 |
| SLPI TIMP1 SCC CEACAM5 OPN | 5 | 0.87 | 44 | 42 | 0.96 | 81 | 83 | 0.79 | 34 | 0 | 0.92 | 62 | 72 |
| TMP1 SCC TFPI MDK | 4 | 0.86 | 68 | 40 | 0.99 | 96 | 96 | 0.76 | 40 | 0 | 0.95 | 79 | 61 |
| SLPI TIMP1 SCC TFPI CEACAM5 MMP2 OPN MDK | 8 | 0.86 | 68 | 32 | 0.99 | 100 | 98 | 0.88 | 58 | 30 | 0.97 | 92 | 89 |
| Cyfra SLPI TIMP1 SCC TFPI MMP2 | 6 | 0.86 | 50 | 46 | 0.99 | 96 | 98 | 0.79 | 46 | 18 | 0.96 | 70 | 80 |
| Cyfra SCC CEACAM5 | 3 | 0.86 | 52 | 54 | 0.97 | 83 | 76 | 0.60 | 10 | 30 | 0.86 | 42 | 44 |
| Cyfra SCC MMP2 | 3 | 0.86 | 50 | 42 | 0.94 | 64 | 63 | 0.58 | 4 | 32 | 0.85 | 21 | 52 |
| Cyfra SLPI TIMP1 SCC TFPI CEACAM5 OPN | 7 | 0.86 | 50 | 44 | 0.99 | 98 | 96 | 0.84 | 32 | 46 | 0.96 | 75 | 87 |
| Cyfra SLPI TIMP1 TFPI | 4 | 0.86 | 52 | 50 | 0.98 | 81 | 91 | 0.78 | 32 | 20 | 0.95 | 74 | 69 |
| Cyfra TIMP1 TFPI | 3 | 0.86 | 52 | 50 | 0.98 | 81 | 91 | 0.68 | 0 | 20 | 0.94 | 45 | 70 |
| Cyfra TIMP1 SCC TFPI MMP2 | 5 | 0.86 | 44 | 46 | 0.99 | 96 | 96 | 0.73 | 30 | 18 | 0.96 | 83 | 83 |
| Cyfra TIMP1 SCC TFPI CEACAM5 OPN | 6 | 0.86 | 46 | 44 | 0.99 | 98 | 96 | 0.80 | 18 | 14 | 0.96 | 87 | 87 |
| SLPI TIMP1 SCC CEACAM5 | 4 | 0.86 | 42 | 46 | 0.96 | 77 | 81 | 0.75 | 28 | 0 | 0.91 | 21 | 83 |
| Cyfra SLPI SCC MMP2 OPN | 5 | 0.86 | 64 | 26 | 0.96 | 81 | 83 | 0.79 | 48 | 28 | 0.91 | 47 | 76 |
| Cyfra SLPI OPN | 3 | 0.86 | 66 | 18 | 0.94 | 83 | 57 | 0.79 | 30 | 32 | 0.83 | 57 | 44 |
| Cyfra TIMP1 SCC OPN | 4 | 0.86 | 72 | 18 | 0.98 | 79 | 93 | 0.72 | 16 | 28 | 0.90 | 26 | 80 |
| Cyfra SLPI SCC TFPI CEACAM5 MMP2 | 6 | 0.86 | 56 | 40 | 0.98 | 91 | 83 | 0.78 | 44 | 16 | 0.96 | 62 | 94 |
| Cyfra SLPI TIMP1 TFPI OPN | 5 | 0.86 | 52 | 52 | 0.98 | 83 | 93 | 0.82 | 22 | 18 | 0.94 | 64 | 81 |
| SLPI CEACAM5 MMP2 OPN | 4 | 0.86 | 48 | 46 | 0.86 | 45 | 43 | 0.77 | 56 | 0 | 0.82 | 45 | 43 |
| Cyfra SLPI TIMP1 SCC TFPI MMP2 OPN | 7 | 0.86 | 50 | 44 | 0.99 | 98 | 96 | 0.82 | 36 | 16 | 0.96 | 60 | 87 |
| TIMP1 CEACAM5 OPN | 3 | 0.86 | 52 | 34 | 0.93 | 68 | 67 | 0.70 | 42 | 0 | 0.82 | 32 | 43 |
| Cyfra TIMP1 TFPI OPN | 4 | 0.85 | 52 | 52 | 0.99 | 83 | 93 | 0.75 | 26 | 18 | 0.94 | 72 | 83 |
| SLPI TIMP1 MMP2 OPN | 4 | 0.85 | 58 | 6 | 0.92 | 75 | 41 | 0.76 | 42 | 0 | 0.85 | 53 | 50 |
| Cyfra TFPI CEACAM5 | 3 | 0.85 | 56 | 48 | 0.95 | 74 | 74 | 0.63 | 30 | 18 | 0.89 | 81 | 57 |
| SLPI TIMP1 MMP2 | 3 | 0.85 | 58 | 6 | 0.92 | 74 | 37 | 0.71 | 12 | 0 | 0.86 | 26 | 0 |
| SLPI TIMP1 SCC MMP2 OPN | 5 | 0.85 | 66 | 6 | 0.96 | 79 | 78 | 0.76 | 42 | 0 | 0.90 | 43 | 78 |
| Cyfra TIMP1 SCC | 3 | 0.85 | 68 | 18 | 0.97 | 77 | 89 | 0.65 | 0 | 30 | 0.90 | 47 | 54 |
| Cyfra SLPI SCC MMP2 | 4 | 0.85 | 62 | 26 | 0.96 | 79 | 83 | 0.73 | 38 | 30 | 0.91 | 62 | 85 |
| Cyfra TFPI CEACAM5 OPN | 4 | 0.85 | 52 | 46 | 0.95 | 79 | 78 | 0.75 | S6 | 16 | 0.91 | 66 | 80 |
| TIMP1 SCC CEACAM5 OPN | 4 | 0.85 | 42 | 32 | 0.96 | 74 | 78 | 0.71 | 22 | 0 | 0.87 | 17 | 72 |
| Cyfra TIMP1 SCC TFPI MMP2 OPN | 6 | 0.85 | 50 | 44 | 0.99 | 98 | 96 | 0.77 | 44 | 16 | 0.95 | 70 | 87 |
| Cyfra SLPI | 2 | 0.85 | 64 | 18 | 0.93 | 83 | 46 | 0.70 | 58 | 34 | 0.84 | 0 | 0 |
| Cyfra TIMP1 OPN | 3 | 0.85 | 70 | 22 | 0.97 | 85 | 87 | 0.71 | 34 | 30 | 0.87 | 47 | 43 |
| SLPI TIMP1 SCC MMP2 | 4 | 0.85 | 62 | 6 | 0.96 | 79 | 76 | 0.72 | 28 | 0 | 0.90 | 13 | 61 |
| SLPI CEACAM5 OPN | 3 | 0.85 | 48 | 34 | 0.86 | 45 | 44 | 0.74 | 46 | 0 | 0.81 | 34 | 43 |
| Cyfra SLPI SCC TFPI CEACAM5 | 5 | 0.85 | 54 | 40 | 0.98 | 91 | 83 | 0.76 | 32 | 16 | 0.95 | 60 | 80 |
| Cyfra SLPI SCC TFPI CEACAM5 MMP2 OPN | 7 | 0.85 | 52 | 40 | 0.98 | 92 | 87 | 0.82 | 54 | 14 | 0.96 | 85 | 87 |
| Cyfra SCC OPN | 3 | 0.84 | 42 | 38 | 0.94 | 66 | 74 | 0.63 | 18 | 32 | 0.84 | 0 | 81 |
| TIMP1 CEACAM5 | 2 | 0.84 | 50 | 30 | 0.92 | 72 | 67 | 0.60 | 18 | 0 | 0.79 | 53 | 0 |
| Cyfra SLPI TIMP1 SCC TFPI | 5 | 0.84 | 46 | 44 | 0.99 | 98 | 98 | 0.78 | 36 | 18 | 0.96 | 66 | 83 |
| SLPI SCC CEACAM5 MMP2 OPN | 5 | 0.84 | 58 | 36 | 0.92 | 77 | 41 | 0.77 | 60 | 0 | 0.88 | 42 | 69 |
| Cyfra TIMP1 | 2 | 0.84 | 70 | 18 | 0.97 | 81 | 87 | 0.62 | 0 | 32 | 0.88 | 68 | 70 |
| Cyfra TIMP1 SCC TFPI | 4 | 0.84 | 44 | 42 | 0.99 | 98 | 96 | 0.69 | 16 | 18 | 0.95 | 79 | 85 |
| SLPI TIMP1 TFPI CEACAM5 MMP2 OPN | 6 | 0.84 | 44 | 40 | 0.98 | 85 | 89 | 0.81 | 42 | 0 | 0.93 | 85 | 80 |
| SLPI MMP2 OPN | 3 | 0.84 | 56 | 6 | 0.82 | 53 | 0 | 0.72 | 44 | 0 | 0.73 | 30 | 0 |
| Cyfra SLPI SCC TFPI CEACAM5 OPN | 6 | 0.84 | 52 | 40 | 0.98 | 92 | 87 | 0.81 | 46 | 14 | 0.96 | 83 | 89 |
| Cyfra TIMP1 SCC TFPI OPN | 5 | 0.84 | 46 | 42 | 0.99 | 98 | 96 | 0.75 | 32 | 16 | 0.95 | 64 | 89 |
| Cyfra SLPI TIMP1 SCC TFPI OPN | 6 | 0.84 | 46 | 42 | 0.99 | 98 | 96 | 0.82 | 26 | 16 | 0.96 | 57 | 89 |
| SLPI CEACAM5 MMP2 | 3 | 0.84 | 46 | 46 | 0.85 | 47 | 44 | 0.69 | 38 | 0 | 0.82 | 25 | 69 |
| SLP1 TIMP1 TFPI CEACAM5 OPN | 5 | 0.84 | 42 | 40 | 0.98 | 89 | 89 | 0.80 | 28 | 0 | 0.93 | 83 | 85 |
| TIMP1 TFPI CEACAM5 MMP2 OPN | 5 | 0.84 | 46 | 36 | 0.97 | 81 | 89 | 0.77 | 48 | 0 | 0.90 | 70 | 39 |
| Cyfra SLPI TFPI MMP2 | 4 | 0.84 | 50 | 50 | 0.95 | 83 | 70 | 0.75 | 54 | 20 | 0.91 | 57 | 72 |
| Cyfra SCC TFPI CEACAM5 MMP2 | 5 | 0.84 | 54 | 38 | 0.97 | 91 | 76 | 0.71 | 20 | 16 | 0.94 | 79 | 80 |
| Cyfra SLPI TFPI MMP2 OPN | 5 | 0.84 | 48 | 50 | 0.95 | 81 | 74 | 0.81 | 36 | 18 | 0.90 | 72 | 44 |
| SLPI SCC CEACAM5 OPN | 4 | 0.84 | 56 | 36 | 0.92 | 75 | 46 | 0.74 | 50 | 0 | 0.88 | 15 | 74 |
| SLPI TIMP1 TFPI CEACAM5 MMP2 | 5 | 0.84 | 40 | 42 | 0.97 | 79 | 85 | 0.77 | 24 | 0 | 0.94 | 70 | 61 |
| Cyfra SCC | 2 | 0.83 | 46 | 46 | 0.94 | 62 | 69 | 0.48 | 0 | 34 | 0.79 | 0 | 56 |
| TIMP1 TFPI CEACAM5 OPN | 4 | 0.83 | 42 | 36 | 0.98 | 83 | 89 | 0.74 | 34 | 0 | 0.90 | 64 | 41 |
| TIMP1 SCC CEACAM5 | 3 | 0.83 | 38 | 32 | 0.94 | 74 | 72 | 0.64 | 8 | 0 | 0.83 | 38 | 57 |
| Cyfra SLPI SCC OPN | 4 | 0.83 | 58 | 26 | 0.96 | 70 | 83 | 0.77 | 36 | 30 | 0.90 | 30 | 80 |
| SLPI TIMP1 SCC OPN | 4 | 0.83 | 58 | 26 | 0.94 | 75 | 76 | 0.75 | 30 | 0 | 0.89 | 23 | 37 |
| TIMP1 SCC MMP2 OPN | 4 | 0.83 | 56 | 16 | 0.94 | 77 | 70 | 0.66 | 28 | 0 | 0.85 | 13 | 39 |
| Cyfra SCC TFPI CEACAM5 OPN | 4 | 0.83 | 52 | 36 | 0.98 | 87 | 87 | 0.75 | 22 | 14 | 0.94 | 60 | 69 |
| Cyfra SCC TFPI CEACAM5 | 4 | 0.83 | 54 | 34 | 0.97 | 91 | 78 | 0.65 | 10 | 16 | 0.93 | 75 | 81 |
| SLPI SCC CEACAM5 MMP2 | 4 | 0.83 | 52 | 34 | 0.92 | 68 | 43 | 0.70 | 48 | 0 | 0.88 | 55 | 52 |
| TIMP1 TFPI CEACAM5 MMP2 | 4 | 0.83 | 38 | 40 | 0.97 | 77 | 81 | 0.71 | 28 | 0 | 0.89 | 77 | 63 |
| SLPI TIMP1 TFPI CEACAM5 | 4 | 0.83 | 42 | 40 | 0.97 | 81 | 87 | 0.75 | 48 | 0 | 0.94 | 62 | 65 |
| TIMP1 SCC MMP2 | 3 | 0.83 | 50 | 20 | 0.93 | 75 | 59 | 0.60 | 2 | 0 | 0.83 | 17 | 63 |
| SLPI MMP2 | 2 | 0.83 | 48 | 8 | 0.82 | 55 | 0 | 0.62 | 18 | 0 | 0.73 | 32 | 0 |
| Cyfra SCC TFPI CEACAM5 MMP2 OPN | 6 | 0.83 | 52 | 36 | 0.98 | 87 | 87 | 0.78 | 36 | 14 | 0.94 | 64 | 67 |
| SLPI TIMP1 OPN | 3 | 0.83 | 52 | 14 | 0.90 | 64 | 31 | 0.74 | 28 | 0 | 0.84 | 43 | 0 |
| TIMP1 SCC TFPI CEACAM5 MMP2 | 5 | 0.83 | 44 | 44 | 0.98 | 89 | 89 | 0.71 | 14 | 0 | 0.92 | 72 | 50 |
| TIMP1 TFPI CEACAM5 | 3 | 0.83 | 40 | 38 | 0.97 | 77 | 80 | 0.66 | 16 | 0 | 0.89 | 74 | 69 |
| TIMP1 MMP2 OPN | 3 | 0.83 | 60 | 18 | 0.91 | 72 | 41 | 0.64 | 46 | 0 | 0.77 | 26 | 52 |
| SLPI TIMP1 SCC TFPI CEACAM5 MMP2 | 6 | 0.83 | 46 | 38 | 0.98 | 91 | 89 | 0.77 | 32 | 36 | 0.95 | 64 | 81 |
| SLPI TIMP1 SCC TFPI CEACAM5 MMP2 OPN | 7 | 0.83 | 46 | 42 | 0.99 | 96 | 96 | 0.81 | 46 | 32 | 0.95 | 81 | 91 |
| TIMP1 SCC TFPI CEACAM5 MMP2 OPN | 6 | 0.83 | 46 | 42 | 0.99 | 96 | 96 | 0.76 | 24 | 0 | 0.93 | 64 | 69 |
| MMP2 OPN | 2 | 0.82 | 54 | 6 | 0.63 | 40 | 4 | 0.53 | 14 | 0 | 0.56 | 32 | 0 |
| TIMP1 MMP2 | 2 | 0.82 | 60 | 18 | 0.90 | 72 | 41 | 0.56 | 16 | 0 | 0.76 | 30 | 0 |
| Cyfra SLPI SCC | 3 | 0.82 | 58 | 24 | 0.96 | 74 | 81 | 0.70 | 20 | 32 | 0.90 | 43 | 54 |
| SCC CEACAM5 MMP2 OPN | 4 | 0.82 | 42 | 12 | 0.90 | 53 | 20 | 0.66 | 44 | 0 | 0.80 | 9 | 35 |
| SLPI TIMP1 SCC | 3 | 0.82 | 52 | 22 | 0.94 | 74 | 72 | 0.70 | 18 | 0 | 0.89 | 0 | 65 |
| SLPI TIMP1 | 2 | 0.82 | 52 | 14 | 0.91 | 64 | 39 | 0.68 | 40 | 0 | 0.84 | 0 | 0 |
| SLPI TIMP1 TFPI MMP2 OPN | 5 | 0.82 | 48 | 36 | 0.97 | 83 | 91 | 0.78 | 36 | 0 | 0.91 | 72 | 48 |
| SLPI CEACAM5 | 2 | 0.82 | 46 | 26 | 0.85 | 45 | 54 | 0.63 | 26 | 0 | 0.80 | 45 | 72 |
| CEACAM5 MMP2 OPN | 3 | 0.82 | 42 | 24 | 0.81 | 32 | 22 | 0.66 | 28 | 0 | 0.69 | 25 | 0 |
| Cyfra SLPI TFPI OPN | 4 | 0.82 | 44 | 46 | 0.96 | 81 | 70 | 0.79 | 26 | 18 | 0.90 | 68 | 44 |
| TIMP1 TFPI MMP2 OPN | 4 | 0.82 | 46 | 34 | 0.97 | 81 | 91 | 0.70 | 40 | 0 | 0.87 | 45 | 50 |
| SLPI TIMP1 TFPI OPN | 4 | 0.82 | 44 | 44 | 0.97 | 83 | 87 | 0.77 | 24 | 0 | 0.90 | 68 | 48 |
| Cyfra SLPI TFPI | 3 | 0.81 | 46 | 44 | 0.95 | 81 | 61 | 0.73 | 50 | 20 | 0.90 | 51 | 74 |
| SLPI SCC CEACAM5 | 3 | 0.81 | 46 | 34 | 0.92 | 60 | 50 | 0.65 | 36 | 0 | 0.86 | 26 | 56 |
| SLPI TIMP1 TFPI MMP2 | 4 | 0.81 | 44 | 38 | 0.97 | 77 | 85 | 0.74 | 12 | 0 | 0.91 | 51 | 80 |
| SLPI TIMP1 SCC TFPI CEACAM5 OPN | 6 | 0.81 | 44 | 36 | 0.99 | 96 | 96 | 0.80 | 34 | 32 | 0.95 | 79 | 91 |
| SLPI SCC MMP2 OPN | 4 | 0.81 | 60 | 8 | 0.92 | 68 | 56 | 0.71 | 50 | 0 | 0.84 | 51 | 39 |
| TIMP1 TFPI MMP2 | 3 | 0.81 | 40 | 40 | 0.96 | 81 | 69 | 0.64 | 14 | 0 | 0.87 | 60 | 0 |
| TIMP1 TFPI OPN | 3 | 0.81 | 46 | 38 | 0.97 | 81 | 91 | 0.68 | 28 | 0 | 0.87 | 32 | 52 |
| TIMP1 SCC TFPI CEACAM5 OPN | 5 | 0.81 | 44 | 36 | 0.99 | 96 | 96 | 0.74 | 18 | 0 | 0.93 | 57 | 72 |
| SLPI OPN | 2 | 0.81 | 62 | 2 | 0.80 | 64 | 0 | 0.69 | 32 | 0 | 0.71 | 58 | 0 |
| SLPI TIMP1 TFPI | 3 | 0.81 | 40 | 44 | 0.97 | 79 | 87 | 0.72 | 34 | 0 | 0.91 | 42 | 83 |
| Cyfra TFPI MMP2 OPN | 4 | 0.81 | 46 | 44 | 0.95 | 72 | 61 | 0.73 | 46 | 18 | 0.86 | 51 | 44 |
| TIMP1 SCC TFPI CEACAM5 | 4 | 0.81 | 42 | 40 | 0.98 | 91 | 87 | 0.67 | 26 | 0 | 0.91 | 66 | 56 |
| SLPI TIMP1 SCC TFPI CEACAM5 | 5 | 0.81 | 42 | 36 | 0.98 | 92 | 91 | 0.75 | 24 | 36 | 0.95 | 55 | 83 |
| TIMP1 TFPI | 2 | 0.81 | 42 | 42 | 0.96 | 77 | 76 | 0.59 | 0 | 0 | 0.87 | 49 | 0 |
| SLPI TFPI CEACAM5 MMP2 OPN | 5 | 0.81 | 40 | 36 | 0.93 | 58 | 69 | 0.78 | 50 | 0 | 0.88 | 66 | 41 |
| TIMP1 SCC TFPI MMP2 OPN | 5 | 0.81 | 50 | 26 | 0.98 | 94 | 94 | 0.70 | 24 | 0 | 0.92 | 38 | 80 |
| Cyfra SLPI SCC TFPI MMP2 OPN | 6 | 0.81 | 48 | 36 | 0.98 | 91 | 89 | 0.80 | 44 | 16 | 0.95 | 70 | 74 |
| SLPI TIMP1 SCC TFPI MMP2 OPN | 6 | 0.80 | 46 | 28 | 0.98 | 94 | 94 | 0.77 | 38 | 0 | 0.94 | 66 | 78 |
| Cyfra TFPI MMP2 | 3 | 0.80 | 46 | 44 | 0.94 | 72 | 65 | 0.62 | 22 | 20 | 0.86 | 62 | 74 |
| Cyfra SLPI SCC TFPI MMP2 | 5 | 0.80 | 48 | 32 | 0.97 | 89 | 80 | 0.75 | 36 | 18 | 0.95 | 79 | 83 |
| Cyfra SLPI SCC TFPI OPN | 5 | 0.80 | 48 | 36 | 0.98 | 91 | 89 | 0.79 | 34 | 16 | 0.94 | 66 | 78 |
| CEACAM5 OPN | 2 | 0.80 | 42 | 32 | 0.81 | 32 | 30 | 0.58 | 16 | 0 | 0.63 | 0 | 0 |
| SLPI TFPI CEACAM5 OPN | 4 | 0.80 | 40 | 36 | 0.93 | 58 | 72 | 0.76 | 42 | 0 | 0.89 | 58 | 41 |
| SLPI TIMP1 SCC TFPI MMP2 | 5 | 0.80 | 44 | 32 | 0.98 | 91 | 89 | 0.74 | 26 | 0 | 0.94 | 43 | 89 |
| Cyfra TFPI OPN | 3 | 0.80 | 44 | 40 | 0.95 | 72 | 63 | 0.69 | 34 | 18 | 0.86 | 43 | 46 |
| TIMP1 SCC TFPI MMP2 | 4 | 0.80 | 44 | 30 | 0.98 | 87 | 91 | 0.66 | 30 | 0 | 0.92 | 53 | 61 |
| SLPI SCC MMP2 | 3 | 0.80 | 56 | 8 | 0.92 | 70 | 65 | 0.64 | 38 | 0 | 0.84 | 17 | 63 |
| SCC CEACAM5 MMP2 | 3 | 0.80 | 42 | 12 | 0.87 | 57 | 17 | 0.55 | 20 | 0 | 0.77 | 15 | 0 |
| Cyfra SLPI SCC TFPI | 4 | 0.80 | 48 | 30 | 0.97 | 89 | 81 | 0.73 | 18 | 18 | 0.94 | 79 | 87 |
| OPN | 1 | 0.80 | 46 | 28 | 0.64 | 6 | 0 | 0.43 | 0 | 0 | 0.41 | 0 | 0 |
| SCC CEACAM5 OPN | 3 | 0.80 | 40 | 16 | 0.88 | 49 | 31 | 0.60 | 28 | 0 | 0.75 | 0 | 37 |
| SLPI TFPI CEACAM5 MMP2 | 4 | 0.80 | 40 | 36 | 0.92 | 64 | 63 | 0.72 | 36 | 0 | 0.88 | 47 | 67 |
| SLPI TIMP1 SCC TFPI OPN | 5 | 0.80 | 42 | 34 | 0.98 | 94 | 94 | 0.77 | 28 | 0 | 0.93 | 60 | 83 |
| Cyfra TFPI | 2 | 0.79 | 44 | 38 | 0.94 | 70 | 65 | 0.55 | 0 | 20 | 0.86 | 60 | 0 |
| TIMP1 SCC TFPI OPN | 4 | 0.79 | 44 | 32 | 0.98 | 94 | 94 | 0.68 | 34 | 0 | 0.91 | 13 | 83 |
| Cyfra SCC TFPI OPN | 4 | 0.79 | 46 | 26 | 0.98 | 89 | 85 | 0.69 | 16 | 16 | 0.92 | 19 | 80 |
| SLPI TFPI CEACAMS | 3 | 0.79 | 38 | 36 | 0.92 | 58 | 67 | 0.69 | 24 | 0 | 0.89 | 70 | 70 |
| Cyfra SCC TFPI MMP2 OPN | 5 | 0.79 | 46 | 26 | 0.98 | 89 | 87 | 0.72 | 32 | 16 | 0.93 | 42 | 78 |
| SLPI TIMP1 SCC TFPI | 4 | 0.79 | 40 | 36 | 0.98 | 92 | 87 | 0.72 | 38 | 0 | 0.94 | 23 | 91 |
| Cyfra SCC TFPI MMP2 | 4 | 0.79 | 48 | 32 | 0.97 | 85 | 72 | 0.65 | 40 | 18 | 0.93 | 53 | 52 |
| SLPI SCC OPN | 3 | 0.79 | 56 | 12 | 0.91 | 74 | 50 | 0.69 | 38 | 0 | 0.82 | 32 | 39 |
| SLPI SCC TFPI CEACAMS MMP2 OPN | 6 | 0.79 | 48 | 24 | 0.97 | 83 | 83 | 0.78 | 56 | 0 | 0.93 | 60 | 69 |
| Cyfra SCC TFPI | 3 | 0.79 | 48 | 32 | 0.97 | 85 | 72 | 0.59 | 22 | 18 | 0.91 | 36 | 56 |
| TIMP1 SCC TFPI | 3 | 0.79 | 40 | 38 | 0.98 | 87 | 85 | 0.61 | 16 | 0 | 0.90 | 30 | 67 |
| SLPI SCC TFPI CEACAM5 OPN | 5 | 0.78 | 48 | 24 | 0.97 | 83 | 81 | 0.76 | 48 | 0 | 0.93 | 53 | 74 |
| TIMP1 SCC OPN | 3 | 0.78 | 54 | 18 | 0.91 | 62 | 50 | 0.62 | 16 | 0 | 0.82 | 0 | 41 |
| TFPI CEACAM5 MMP2 OPN | 4 | 0.78 | 38 | 34 | 0.90 | 47 | 54 | 0.72 | 22 | 0 | 0.81 | 43 | 44 |
| CEACAM5 MMP2 | 2 | 0.78 | 28 | 24 | 0.79 | 38 | 7 | 0.48 | 6 | 0 | 0.63 | 30 | 0 |
| TFPI CEACAMS OPN | 3 | 0.78 | 38 | 34 | 0.90 | 51 | 54 | 0.67 | 58 | 0 | 0.80 | 30 | 46 |
| SLPI SCC TFPI CEACAM5 MMP2 | 5 | 0.78 | 42 | 28 | 0.96 | 81 | 74 | 0.72 | 44 | 0 | 0.93 | 68 | 85 |
| SLPI | 1 | 0.78 | 58 | 2 | 0.79 | 55 | 0 | 0.56 | 0 | 0 | 0.67 | 0 | 0 |
| SCC MMP2 OPN | 3 | 0.78 | 40 | 2 | 0.83 | 47 | 15 | 0.55 | 36 | 0 | 0.71 | 17 | 41 |
| SLPI SCC TFPI CEACAM5 | 4 | 0.77 | 42 | 24 | 0.96 | 79 | 72 | 0.70 | 32 | 0 | 0.92 | 64 | 87 |
| SCC TFPI CEACAM5 MMP2 OPN | S | 0.77 | 48 | 26 | 0.96 | 75 | 83 | 0.72 | 36 | 0 | 0.88 | 40 | 72 |
| SCC TFPI CEACAM5 OPN | 4 | 0.77 | 48 | 26 | 0.96 | 74 | 81 | 0.68 | 22 | 0 | 0.88 | 11 | 76 |
| TIMP1 SCC | 2 | 0.77 | 48 | 10 | 0.89 | 58 | 41 | 0.53 | 0 | 0 | 0.77 | 0 | 0 |
| TIMP1 OPN | 2 | 0.77 | 52 | 10 | 0.89 | 58 | 35 | 0.59 | 36 | 0 | 0.72 | 0 | 0 |
| SLPI SCC TFPI MMP2 OPN | 5 | 0.77 | 46 | 26 | 0.97 | 83 | 81 | 0.74 | 46 | 0 | 0.91 | 75 | 37 |
| SLPI SCC | 2 | 0.77 | 46 | 8 | 0.90 | 75 | 52 | 0.58 | 20 | 0 | 0.80 | 0 | 67 |
| SLPI SCC TFPI OPN | 4 | 0.77 | 46 | 26 | 0.97 | 83 | 80 | 0.72 | 36 | 0 | 0.91 | 70 | 37 |
| SCC MMP2 | 2 | 0.76 | 42 | 2 | 0.79 | 43 | 9 | 0.40 | 4 | 0 | 0.65 | 23 | 0 |
| SLPI SCC TFPI MMP2 | 4 | 0.76 | 42 | 24 | 0.96 | 81 | 74 | 0.68 | 36 | 0 | 0.91 | 49 | 61 |
| TFPI CEACAM5 MMP2 | 3 | 0.76 | 34 | 28 | 0.89 | 58 | 35 | 0.60 | 46 | 0 | 0.77 | 55 | 0 |
| SLPI SCC TFPI | 3 | 0.76 | 40 | 22 | 0.96 | 81 | 72 | 0.66 | 18 | 0 | 0.91 | 28 | 65 |
| SLPI TFPI MMP2 OPN | 4 | 0.76 | 34 | 34 | 0.90 | 68 | 52 | 0.75 | 38 | 0 | 0.83 | 45 | 0 |
| SCC TFPI CEACAM5 MMP2 | 4 | 0.76 | 46 | 22 | 0.94 | 79 | 56 | 0.63 | 20 | 0 | 0.87 | 51 | 52 |
| SCC TFPI MMP2 OPN | 4 | 0.76 | 46 | 22 | 0.96 | 77 | 83 | 0.64 | 32 | 0 | 0.86 | 43 | 39 |
| SLPI TFPI OPN | 3 | 0.76 | 34 | 32 | 0.90 | 70 | 52 | 0.73 | 28 | 0 | 0.82 | 72 | 0 |
| SCC TFPI OPN | 3 | 0.75 | 46 | 22 | 0.96 | 77 | 83 | 0.59 | 16 | 0 | 0.84 | 19 | 41 |
| TIMP1 | 1 | 0.75 | 46 | 8 | 0.88 | 57 | 33 | 0.47 | 0 | 0 | 0.69 | 0 | 0 |
| SLPI TFPI MMP2 | 3 | 0.75 | 30 | 32 | 0.89 | 68 | 44 | 0.68 | 18 | 0 | 0.82 | 58 | 0 |
| TFPI CEACAM5 | 2 | 0.75 | 34 | 30 | 0.89 | 55 | 35 | 0.51 | 30 | 0 | 0.76 | 47 | 0 |
| SLPI TFPI | 2 | 0.75 | 30 | 30 | 0.90 | 66 | 41 | 0.64 | 0 | 0 | 0.81 | 53 | 0 |
| SCC CEACAM5 | 2 | 0.75 | 42 | 16 | 0.85 | 49 | 35 | 0.43 | 10 | 0 | 0.69 | 0 | 0 |
| SCC TFPI CEACAM5 | 3 | 0.75 | 44 | 22 | 0.94 | 81 | 59 | 0.55 | 10 | 0 | 0.84 | 28 | 57 |
| SCC TFPI MMP2 | 3 | 0.74 | 44 | 20 | 0.94 | 81 | 57 | 0.55 | 4 | 0 | 0.83 | 58 | 63 |
| TFPI MMP2 OPN | 3 | 0.74 | 32 | 32 | 0.88 | 68 | 46 | 0.64 | 48 | 0 | 0.73 | 53 | 0 |
| TFPI OPN | 2 | 0.74 | 32 | 32 | 0.89 | 68 | 46 | 0.58 | 36 | 0 | 0.70 | 43 | 0 |
| SCC TFPI | 2 | 0.73 | 42 | 22 | 0.93 | 81 | 56 | 0.46 | 22 | 0 | 0.80 | 40 | 69 |
| CEACAM5 | 1 | 0.73 | 30 | 0 | 0.79 | 32 | 20 | 0.32 | 0 | 0 | 0.53 | 0 | 0 |
| MMP2 | 1 | 0.73 | 44 | 2 | 0.59 | 34 | 2 | 0.24 | 24 | 0 | 0.36 | 0 | 0 |
| TFPI MMP2 | 2 | 0.72 | 30 | 32 | 0.87 | 64 | 17 | 0.50 | 22 | 0 | 0.65 | 34 | 0 |
| TFPI | 1 | 0.71 | 28 | 28 | 0.87 | 64 | 28 | 0.38 | 0 | 0 | 0.63 | 64 | 0 |
| SCC OPN | 2 | 0.71 | 34 | 6 | 0.77 | 36 | 30 | 0.48 | 18 | 0 | 0.62 | 0 | 43 |
| SCC | 1 | 0.58 | 24 | 0 | 0.70 | 26 | 0 | 0.23 | 0 | 0 | 0.52 | 0 | 0 |

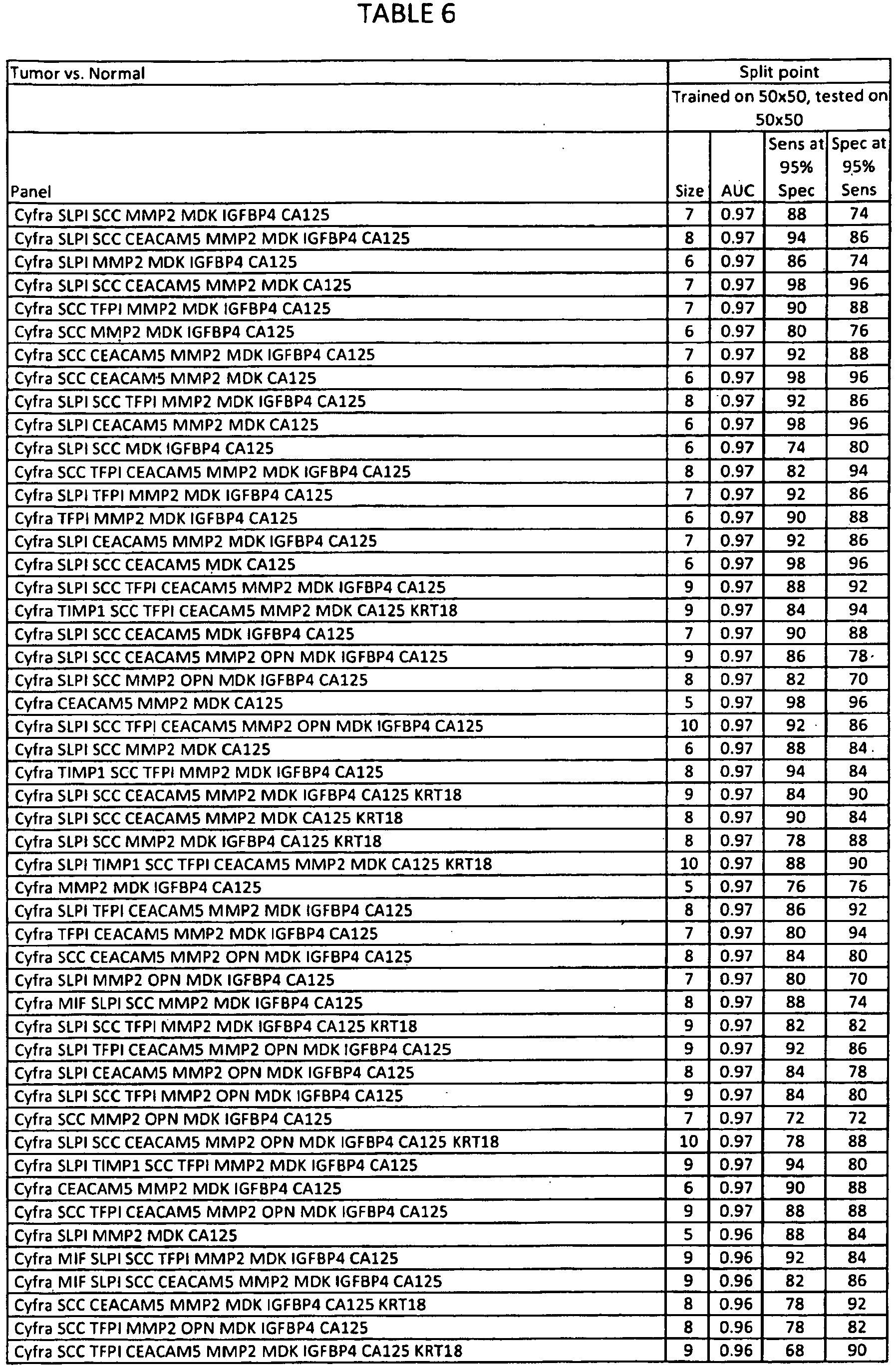

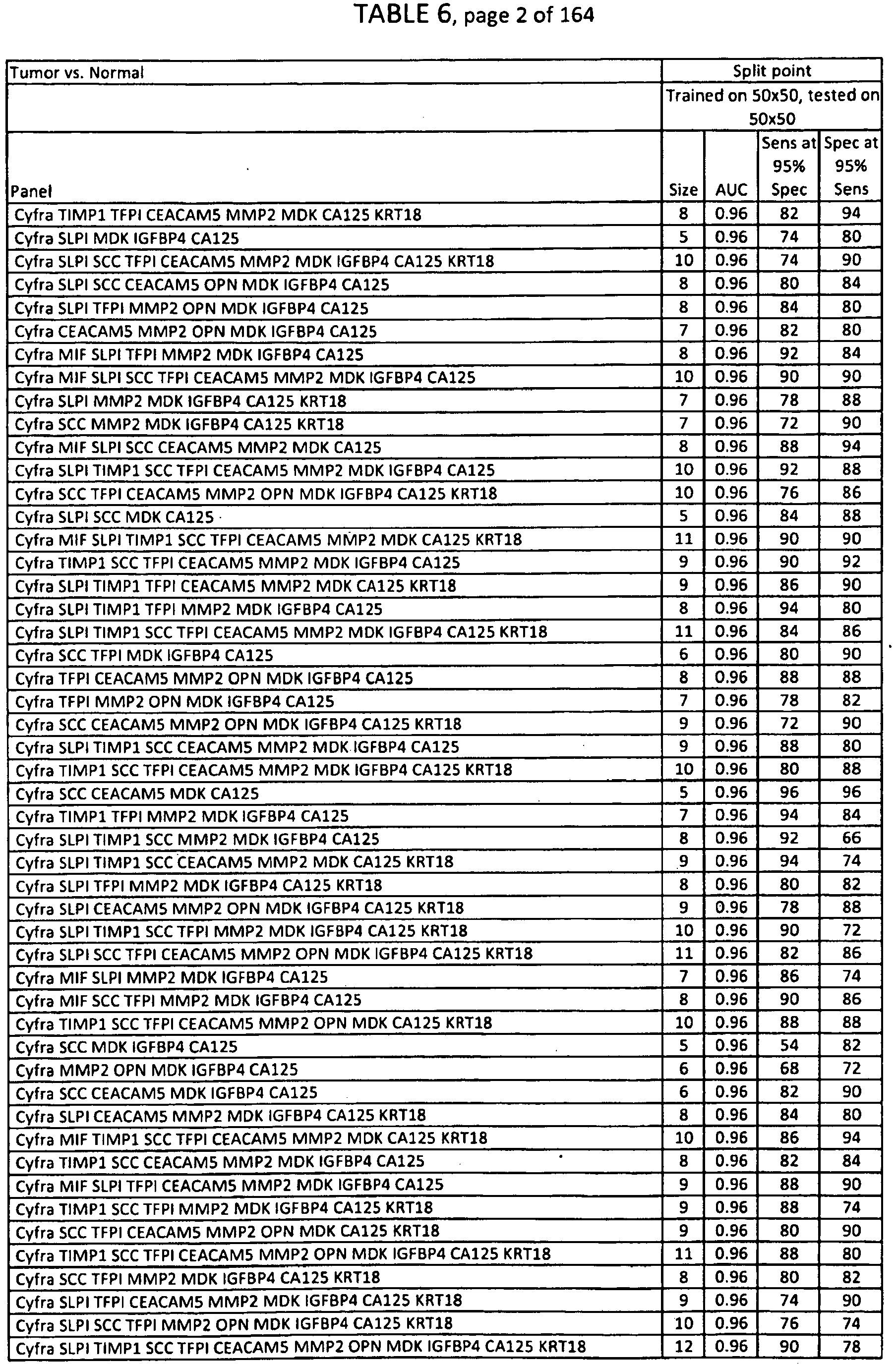

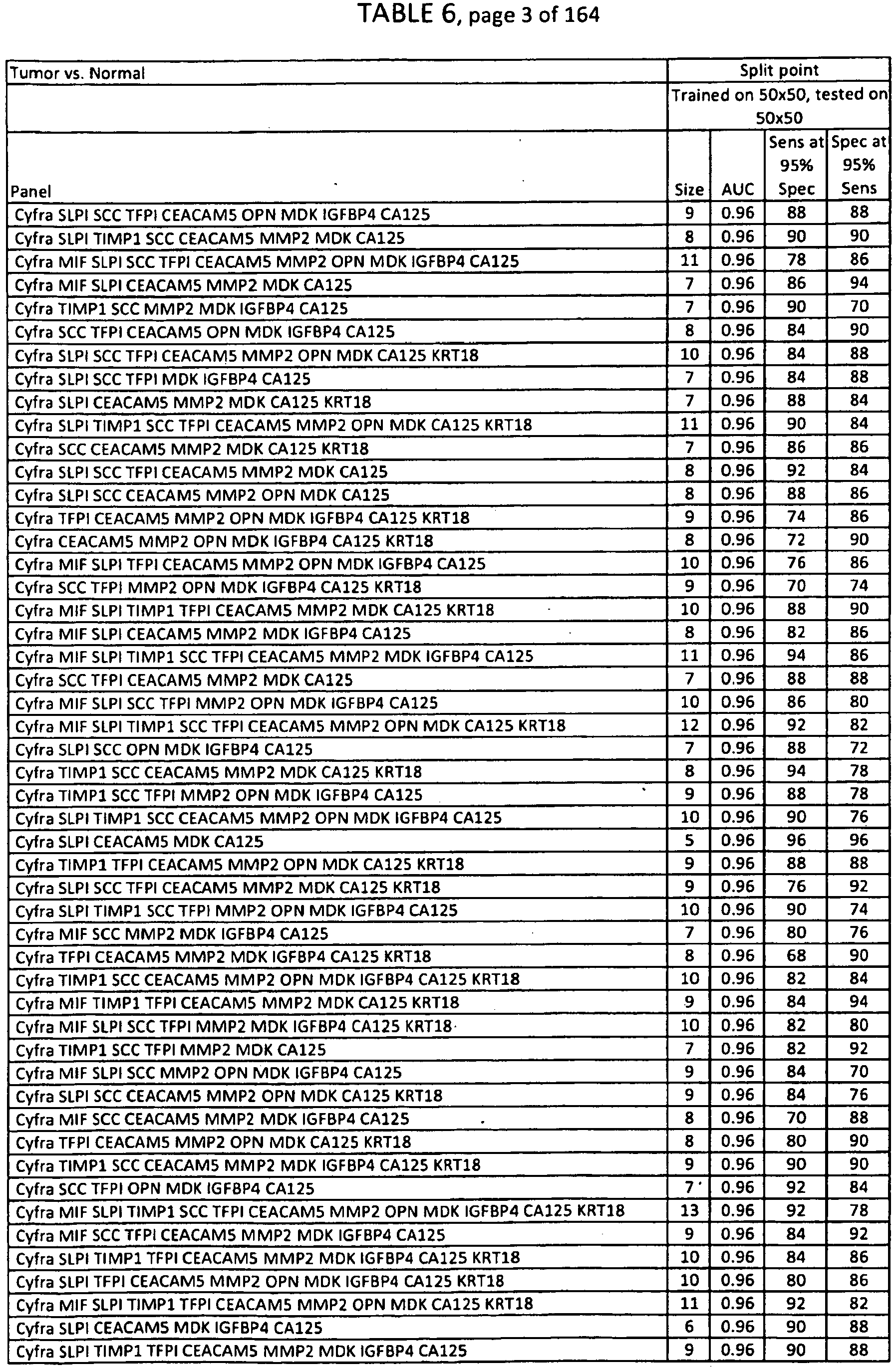

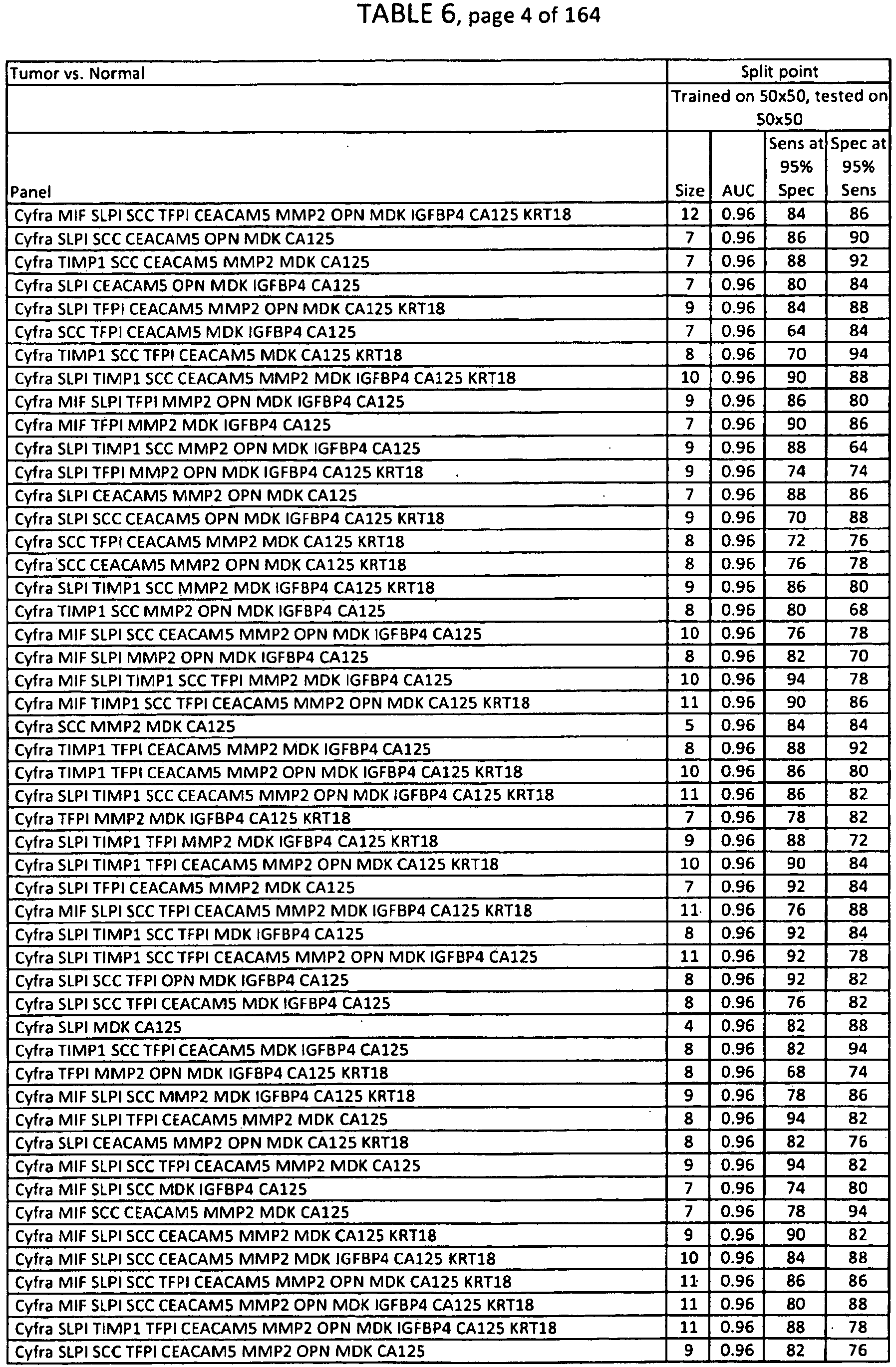

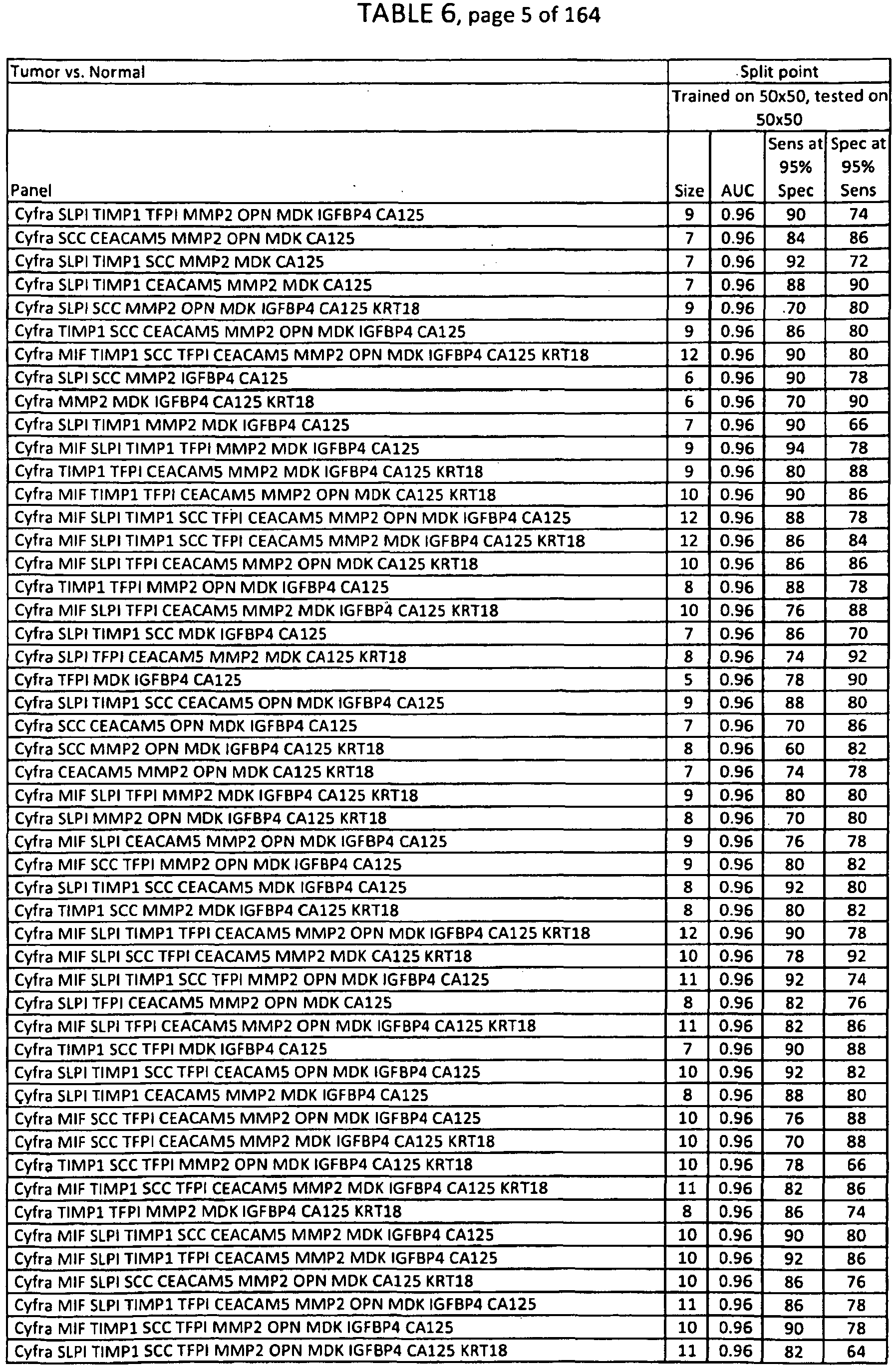

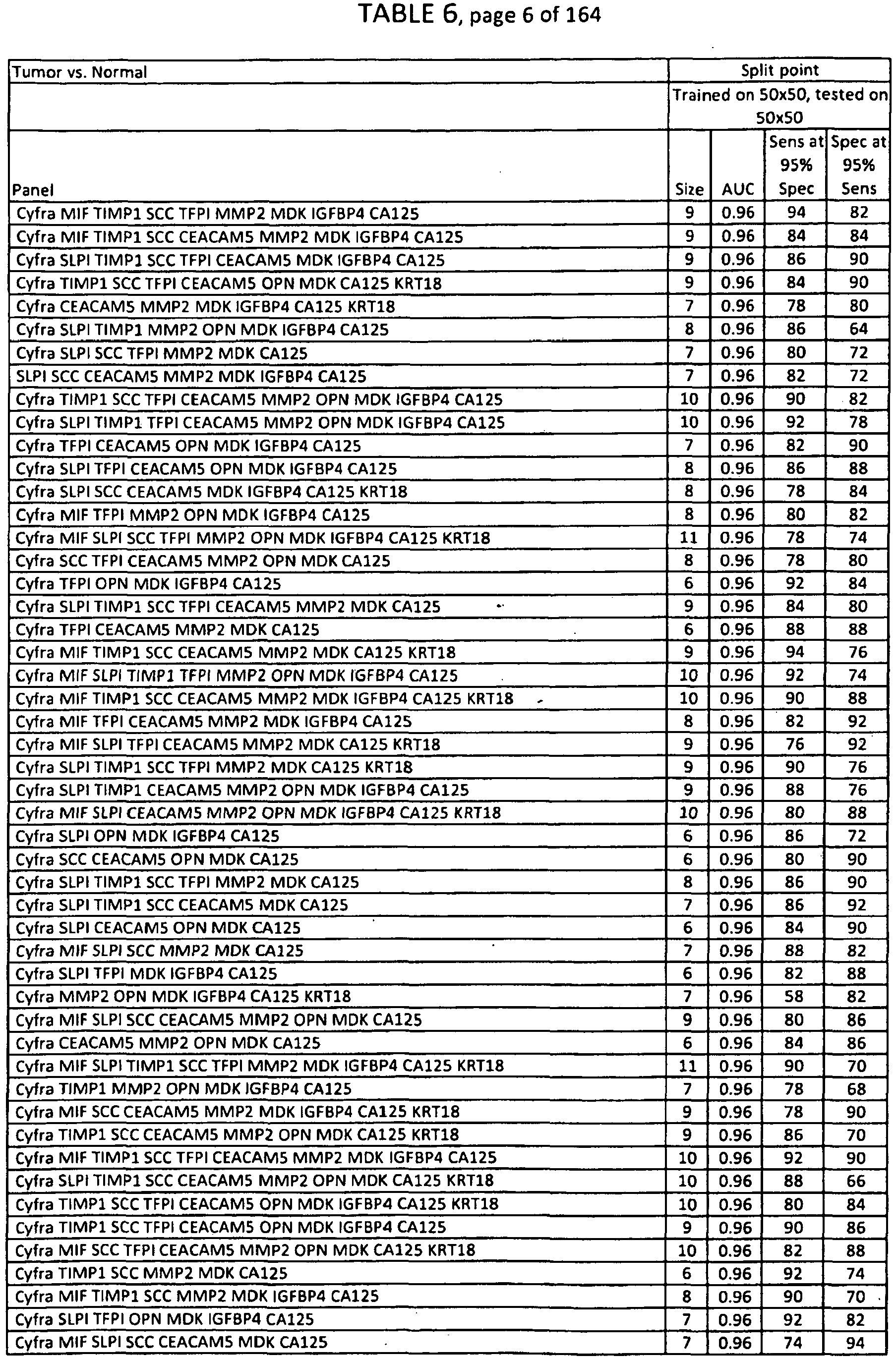

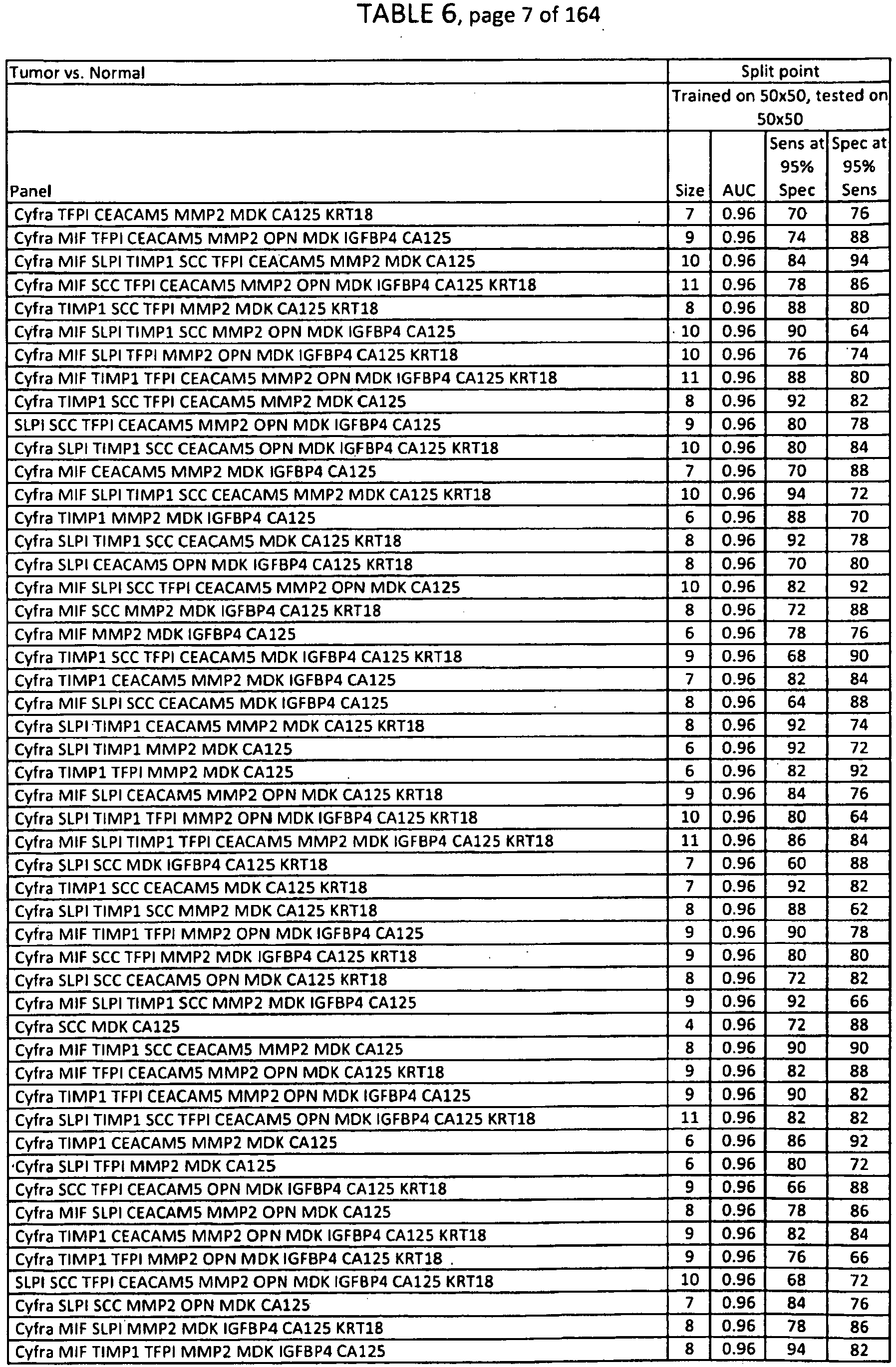

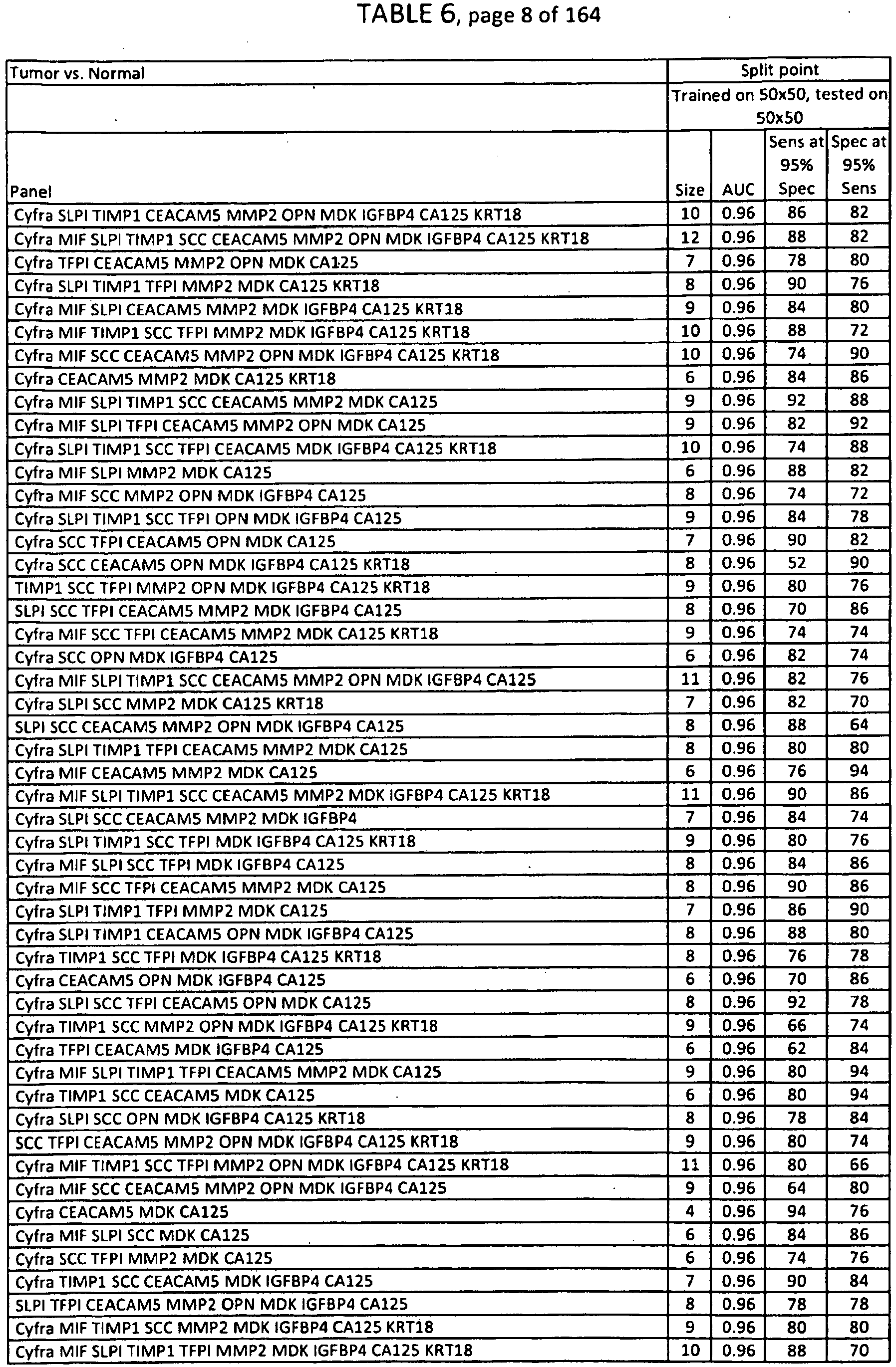

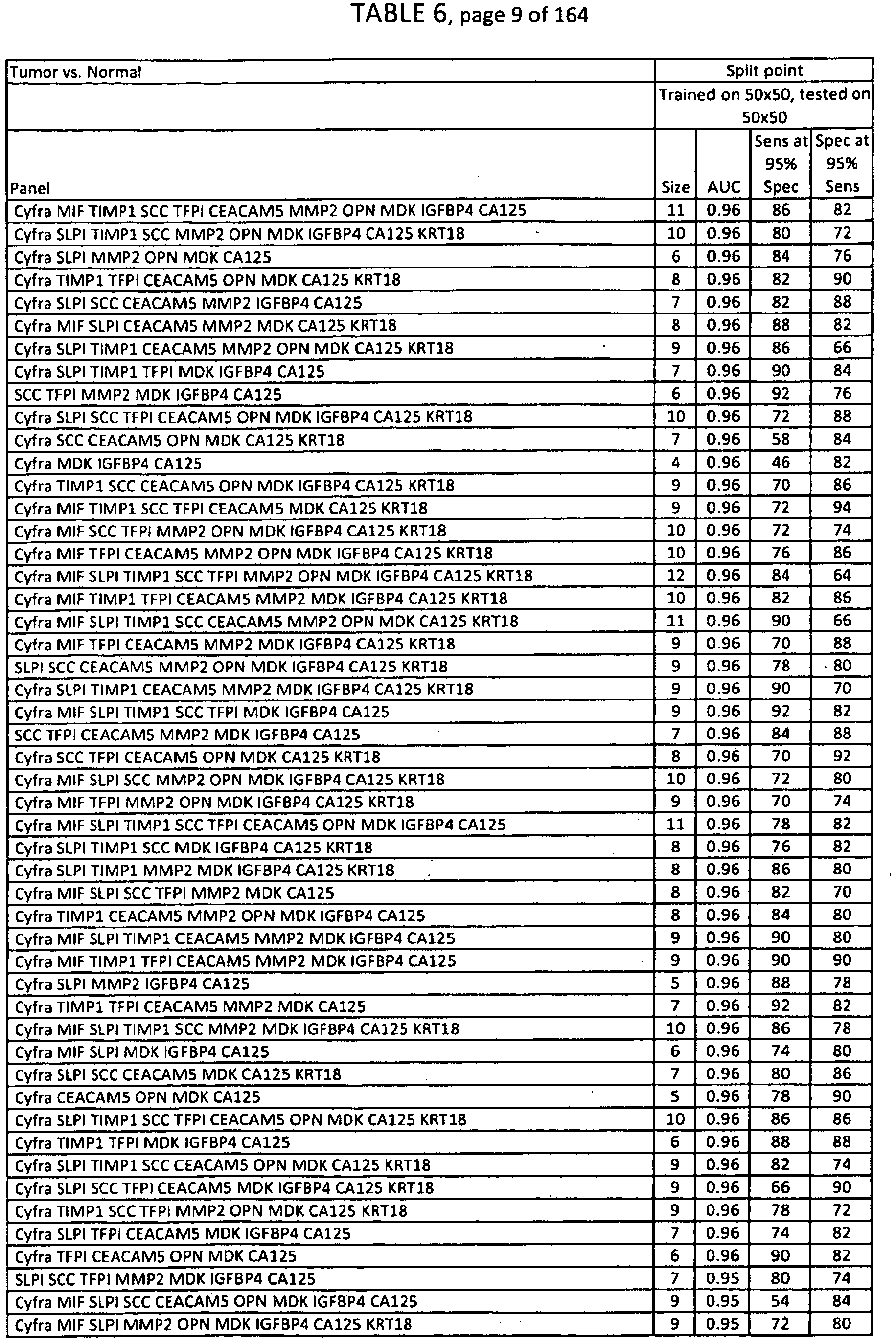

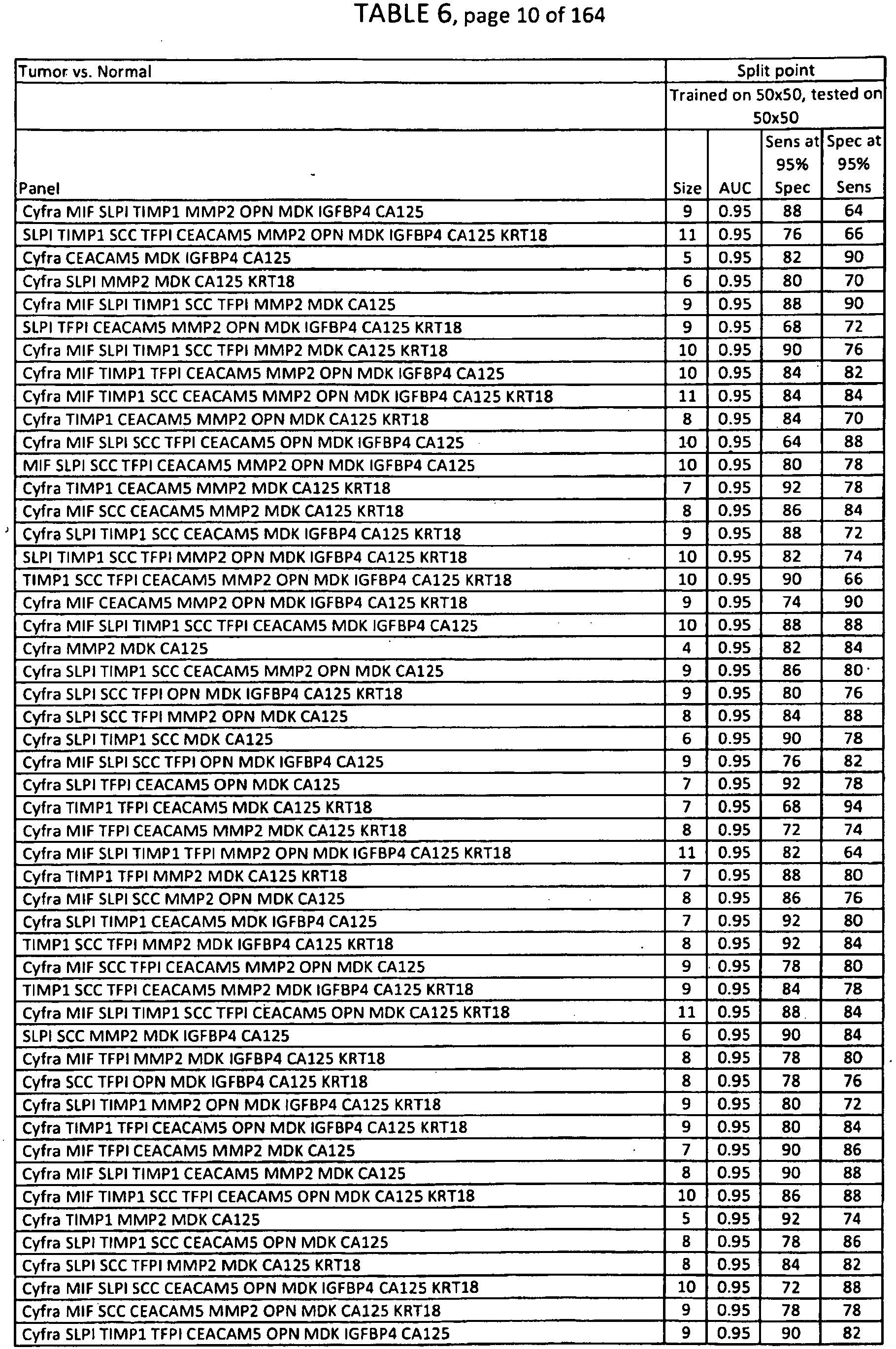

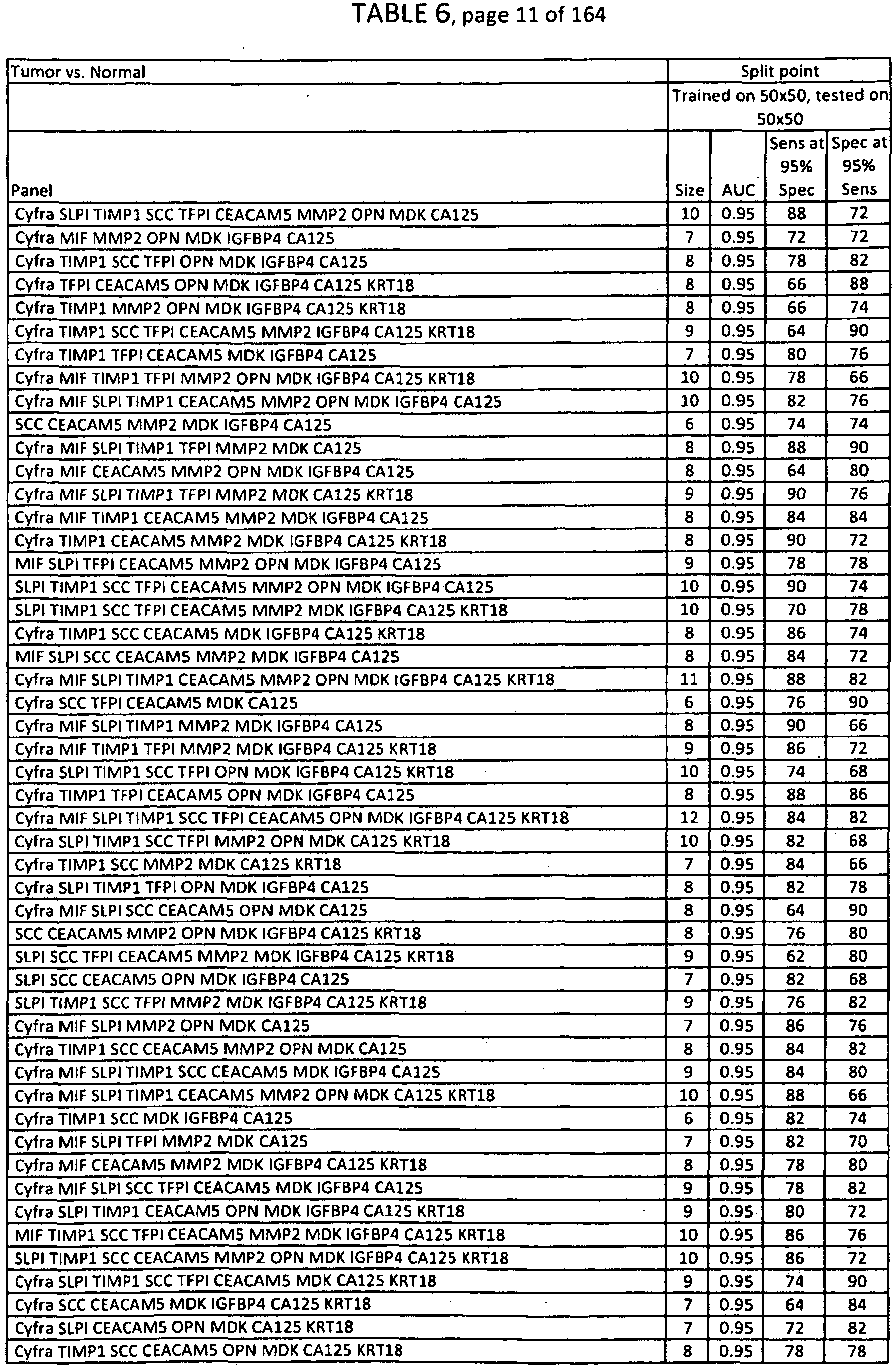

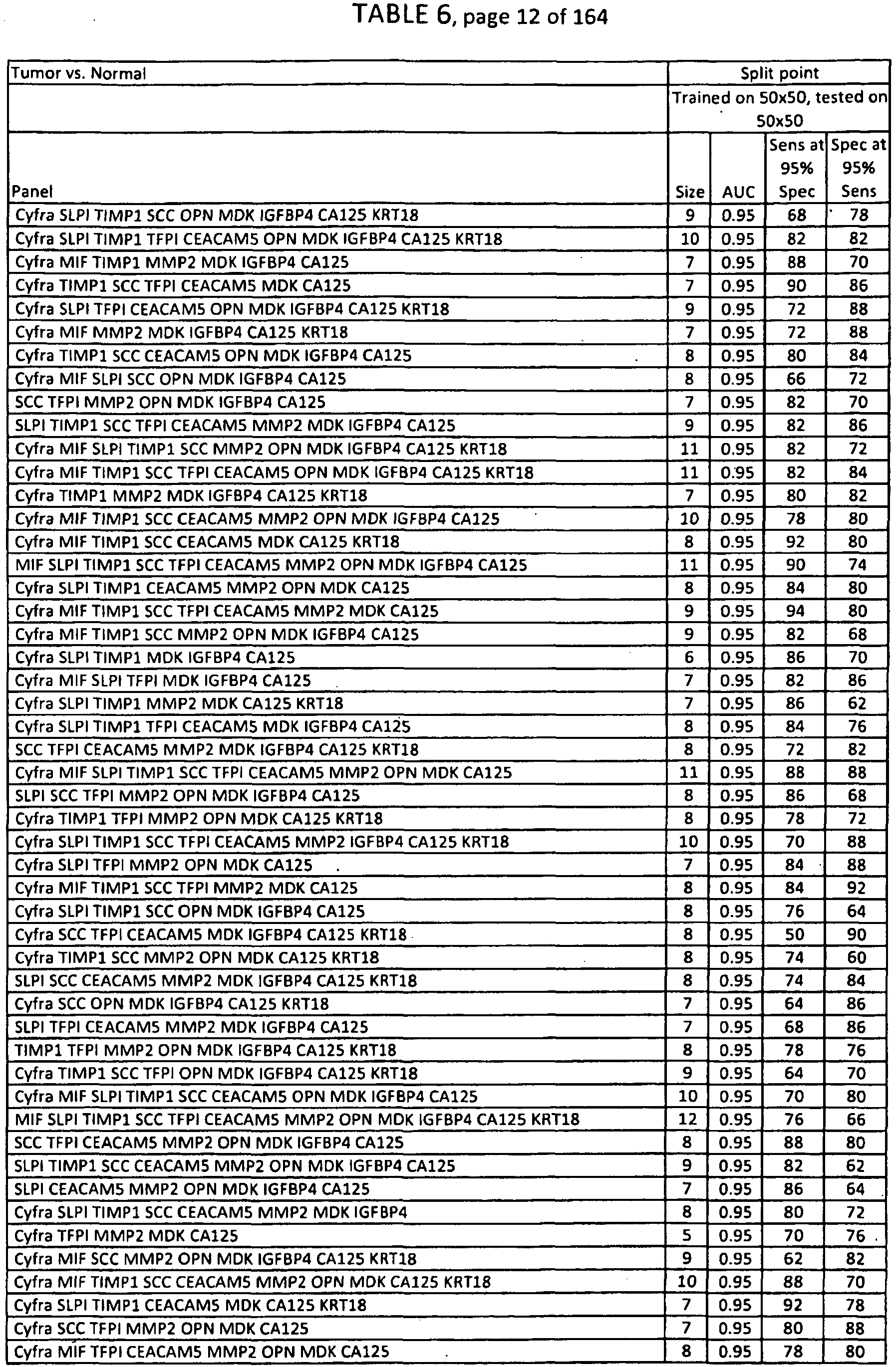

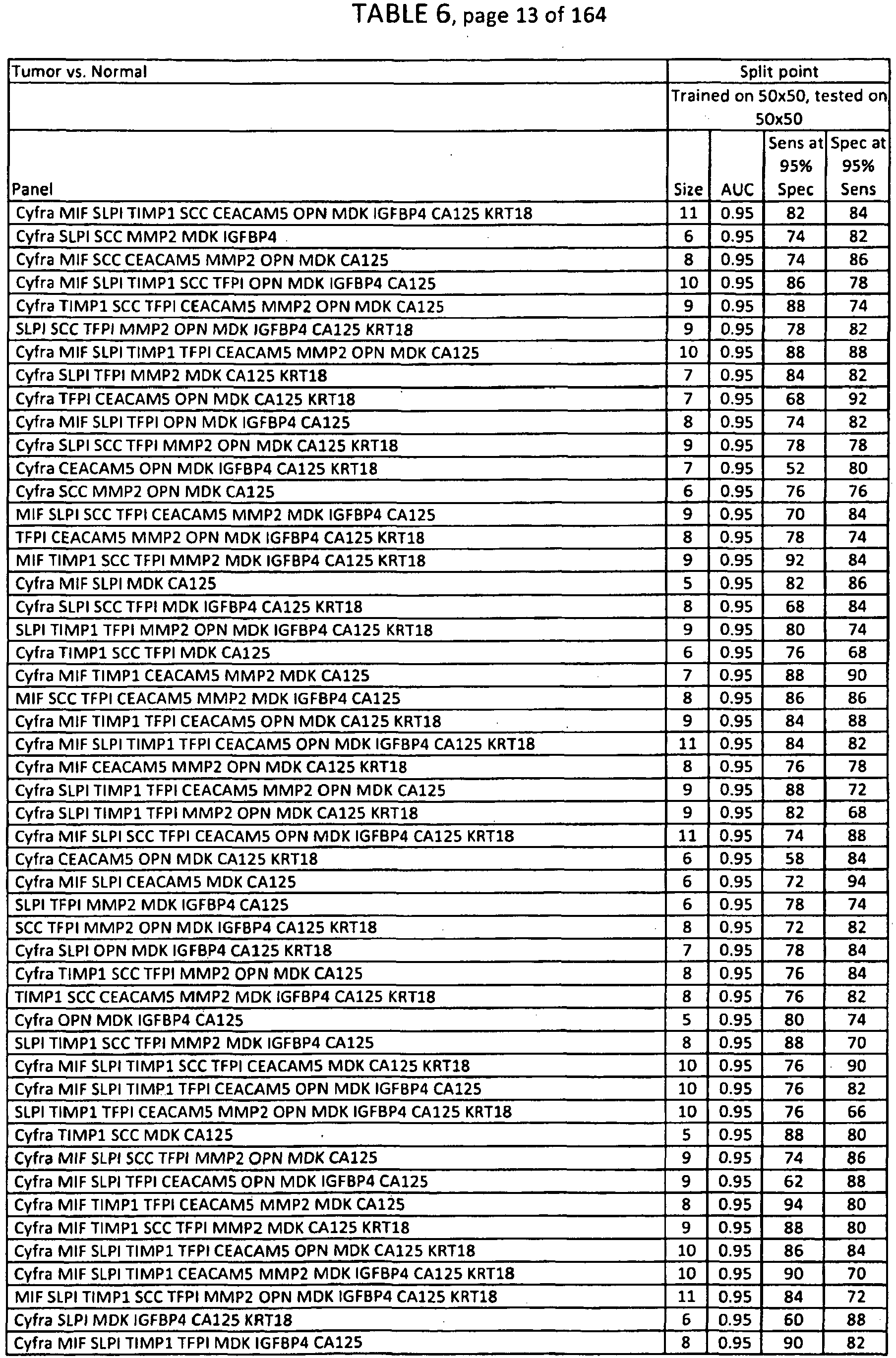

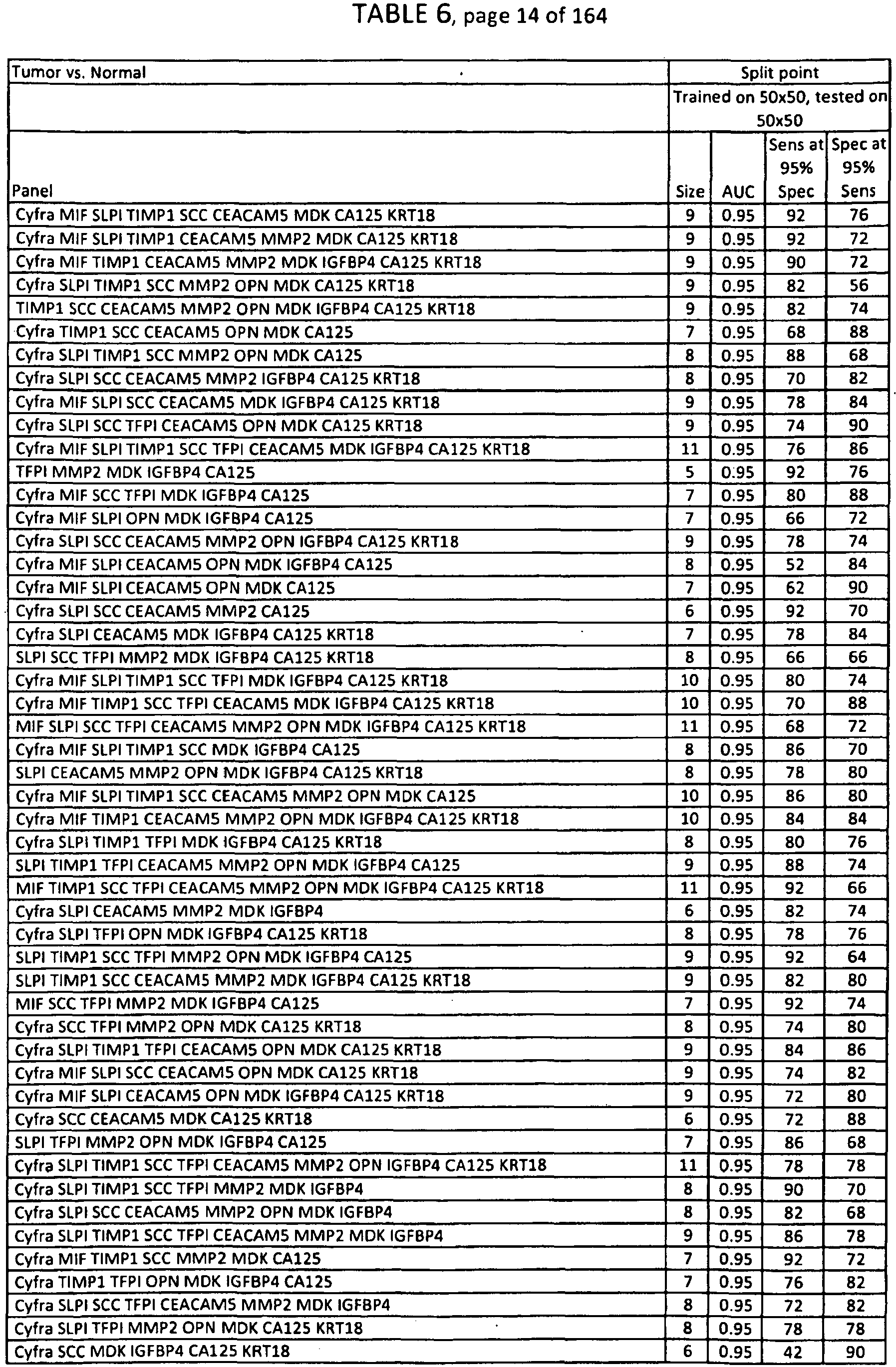

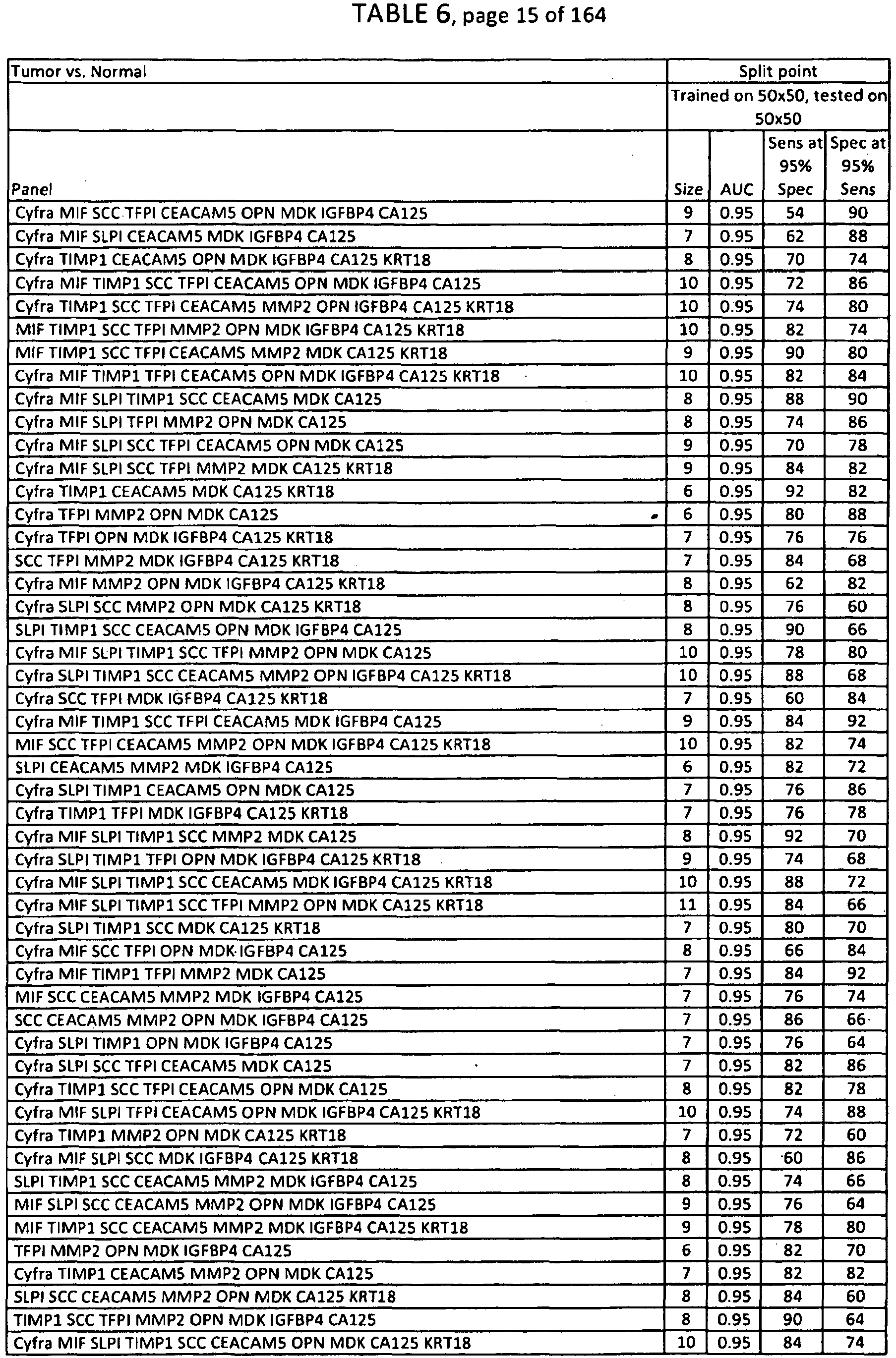

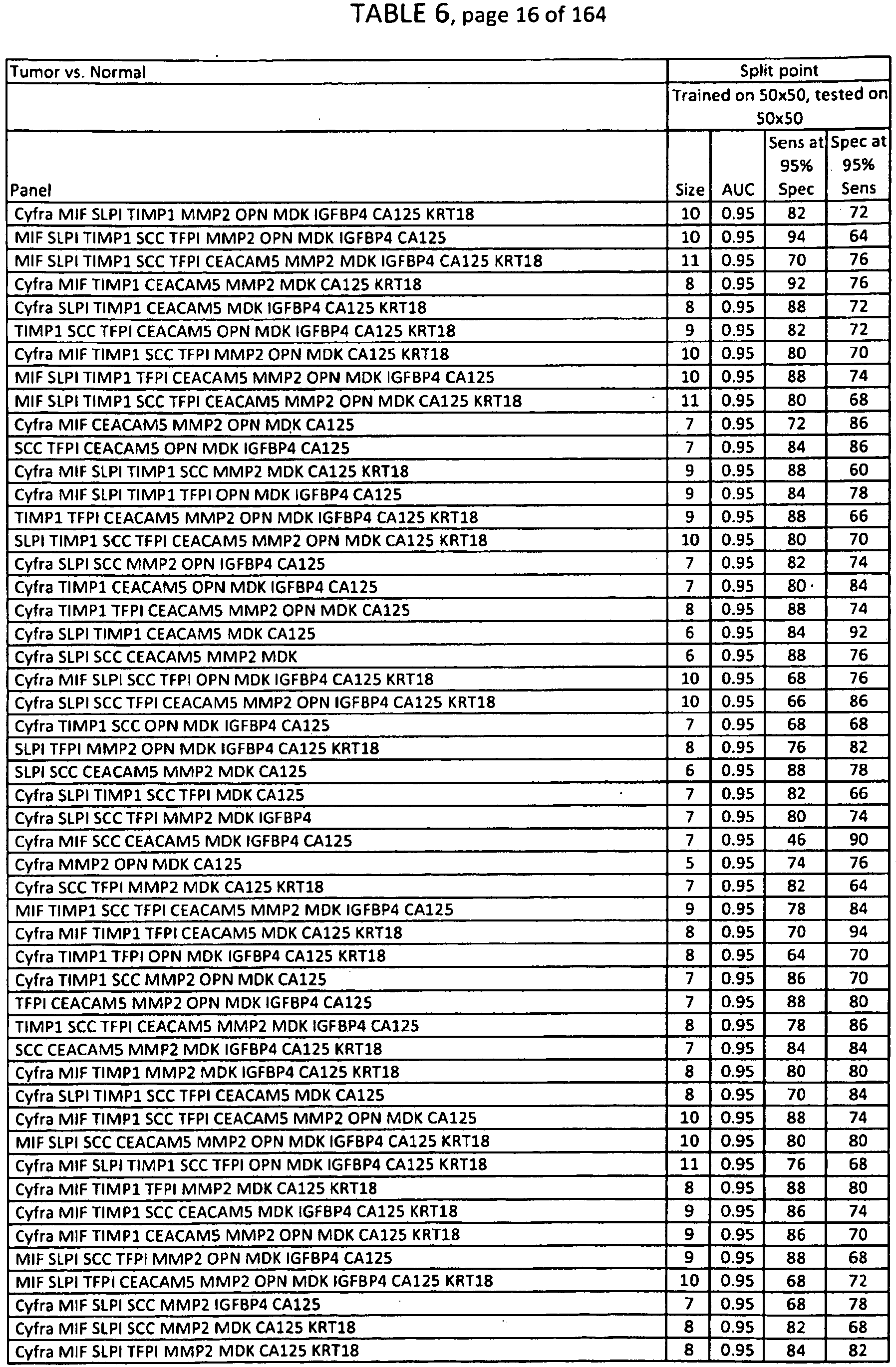

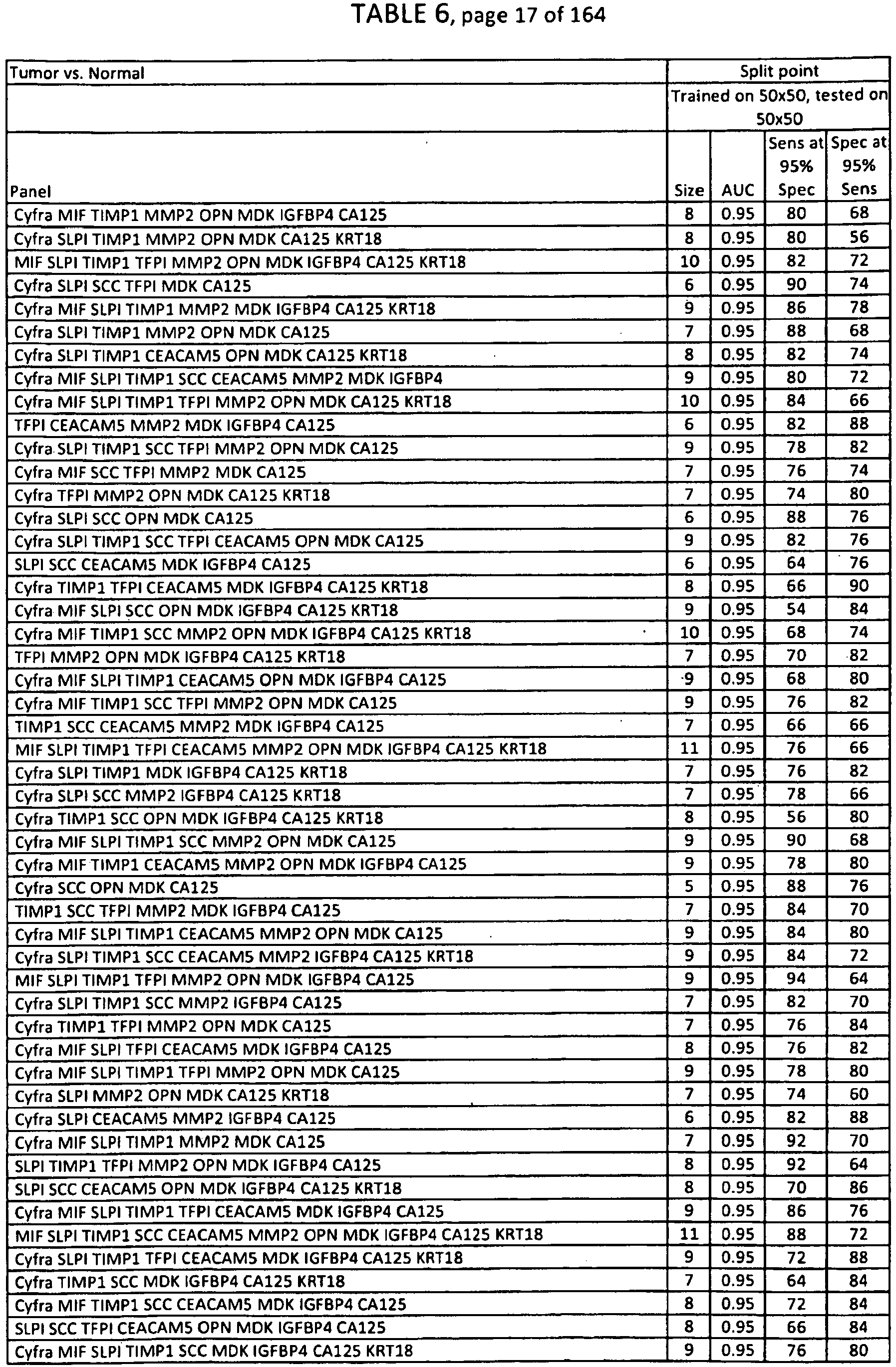

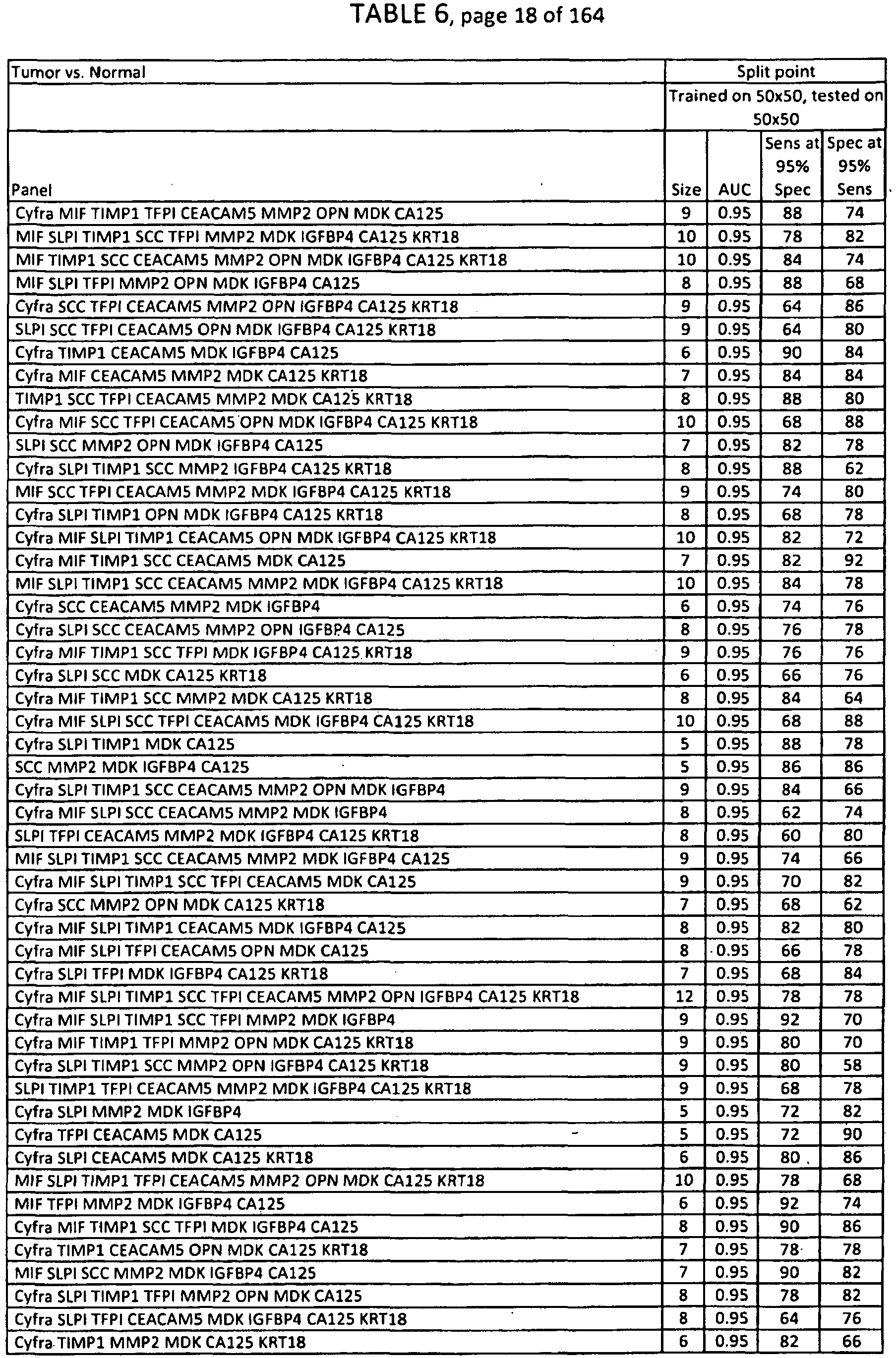

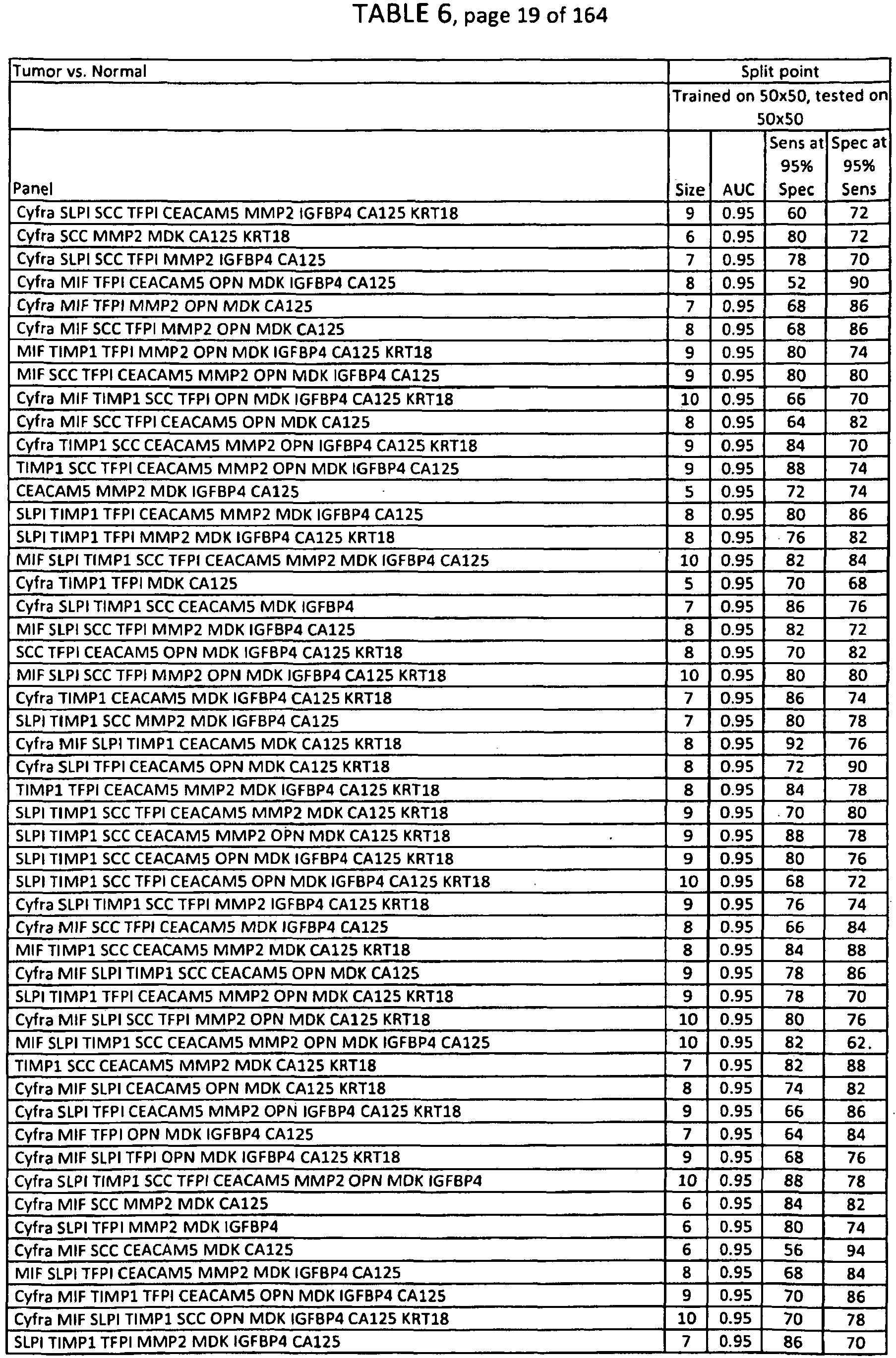

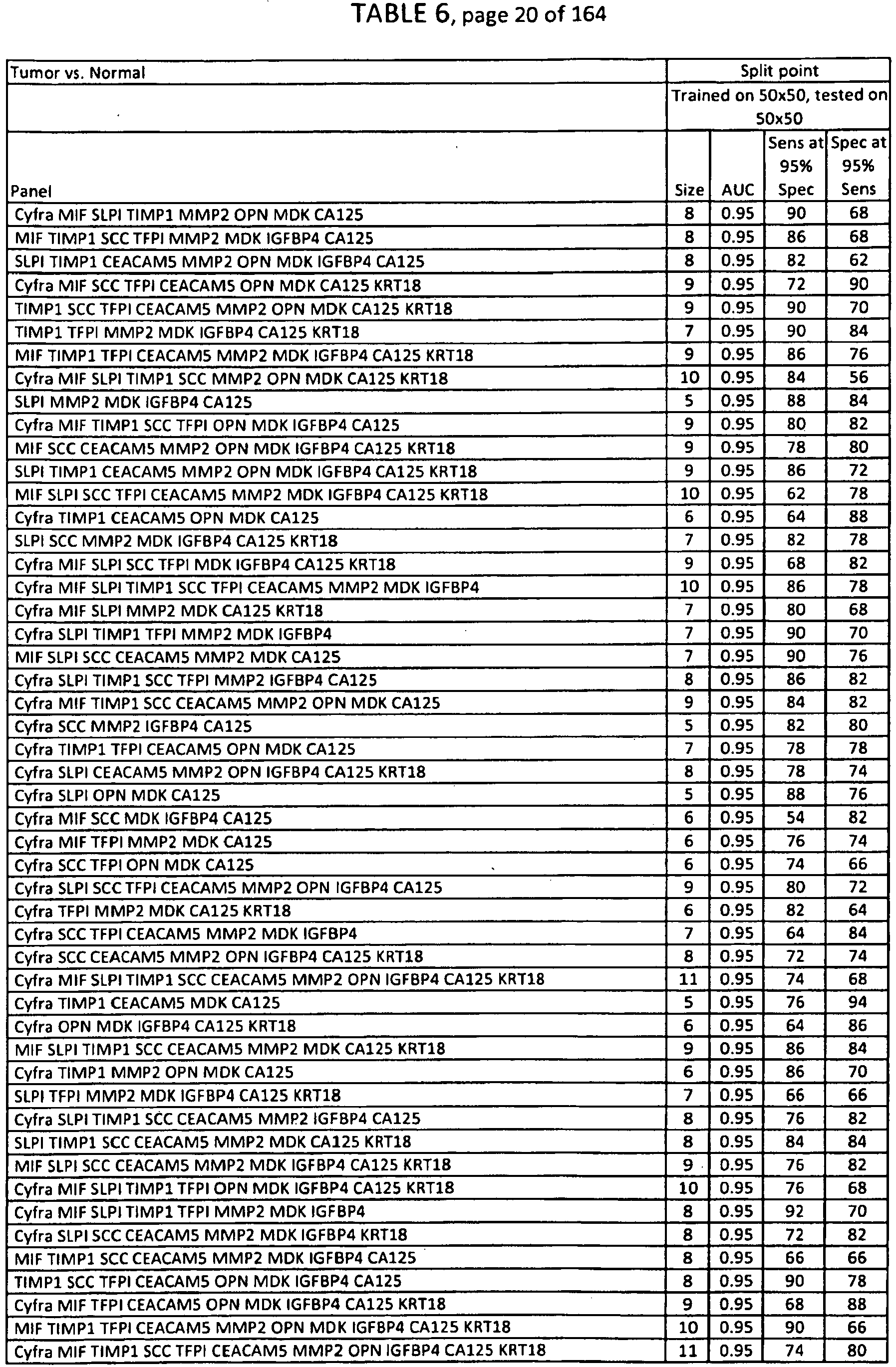

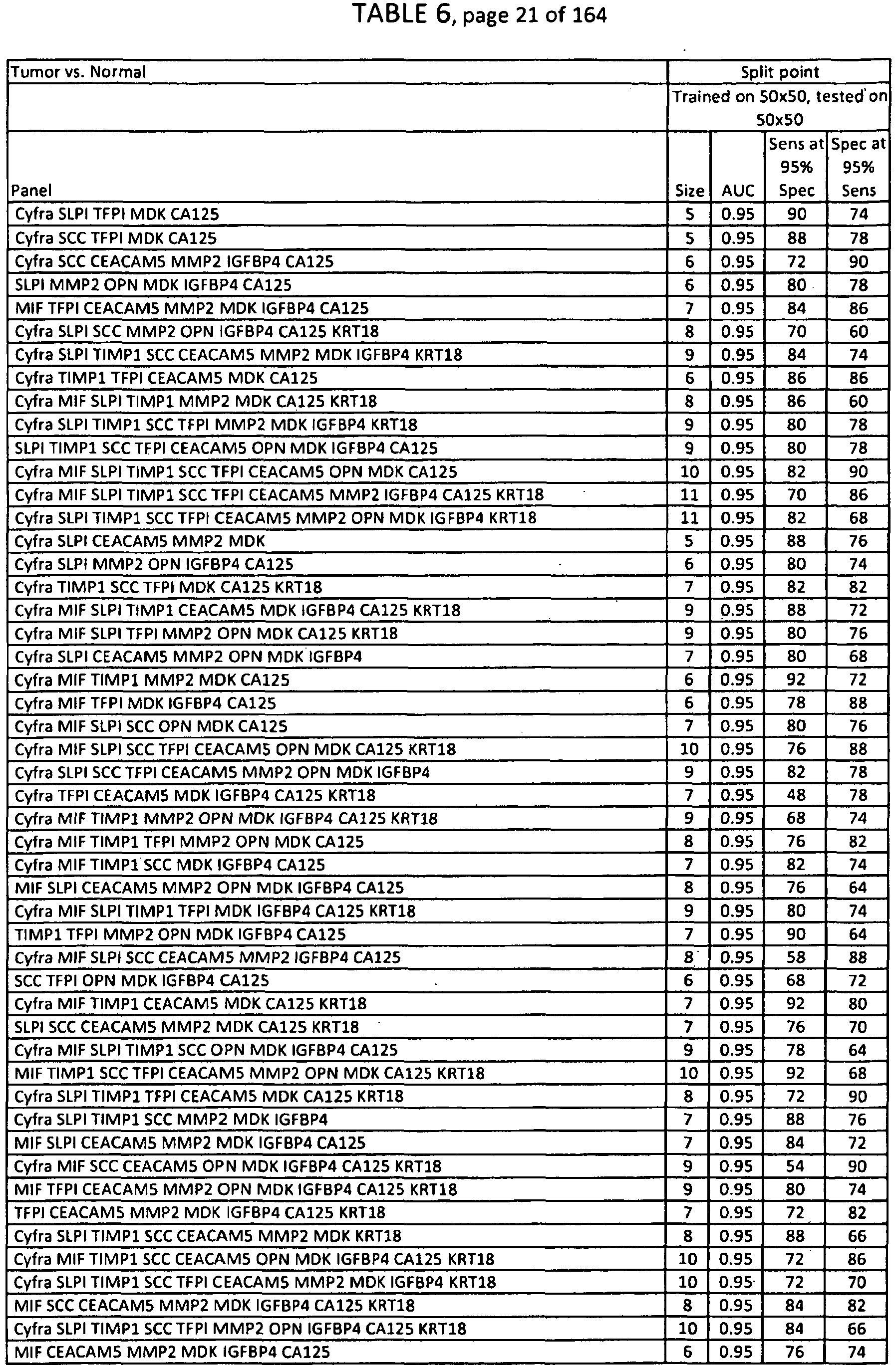

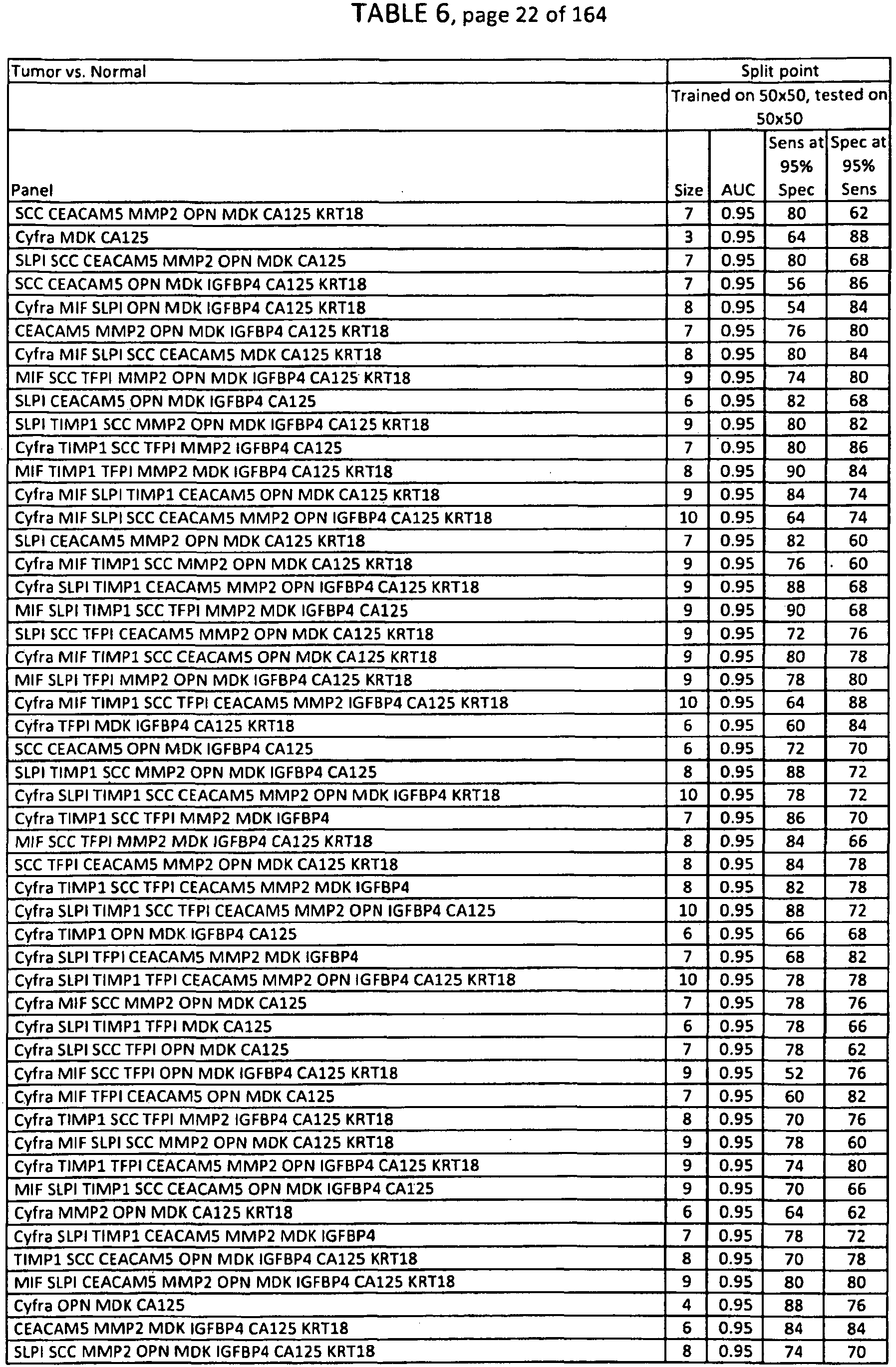

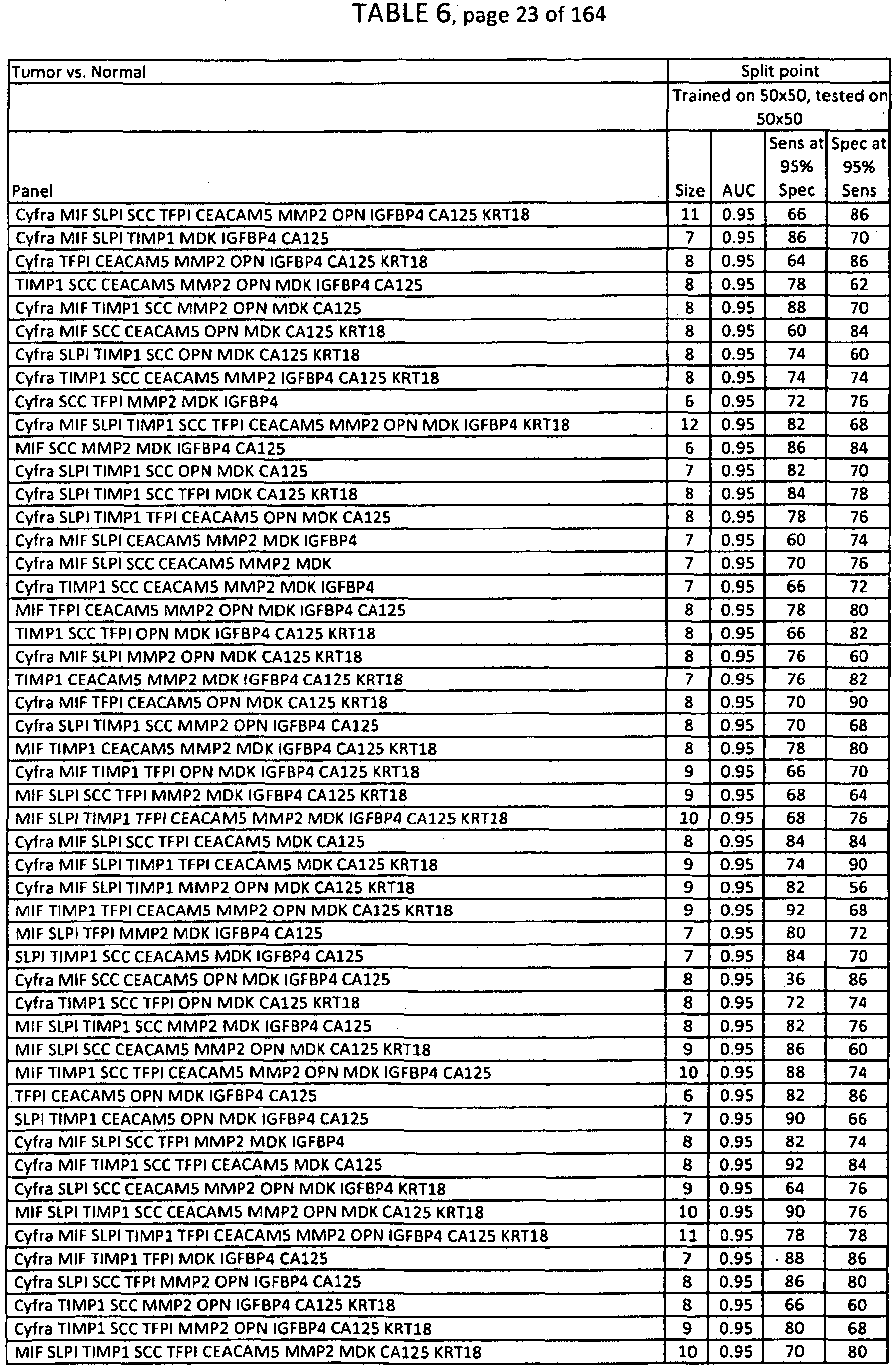

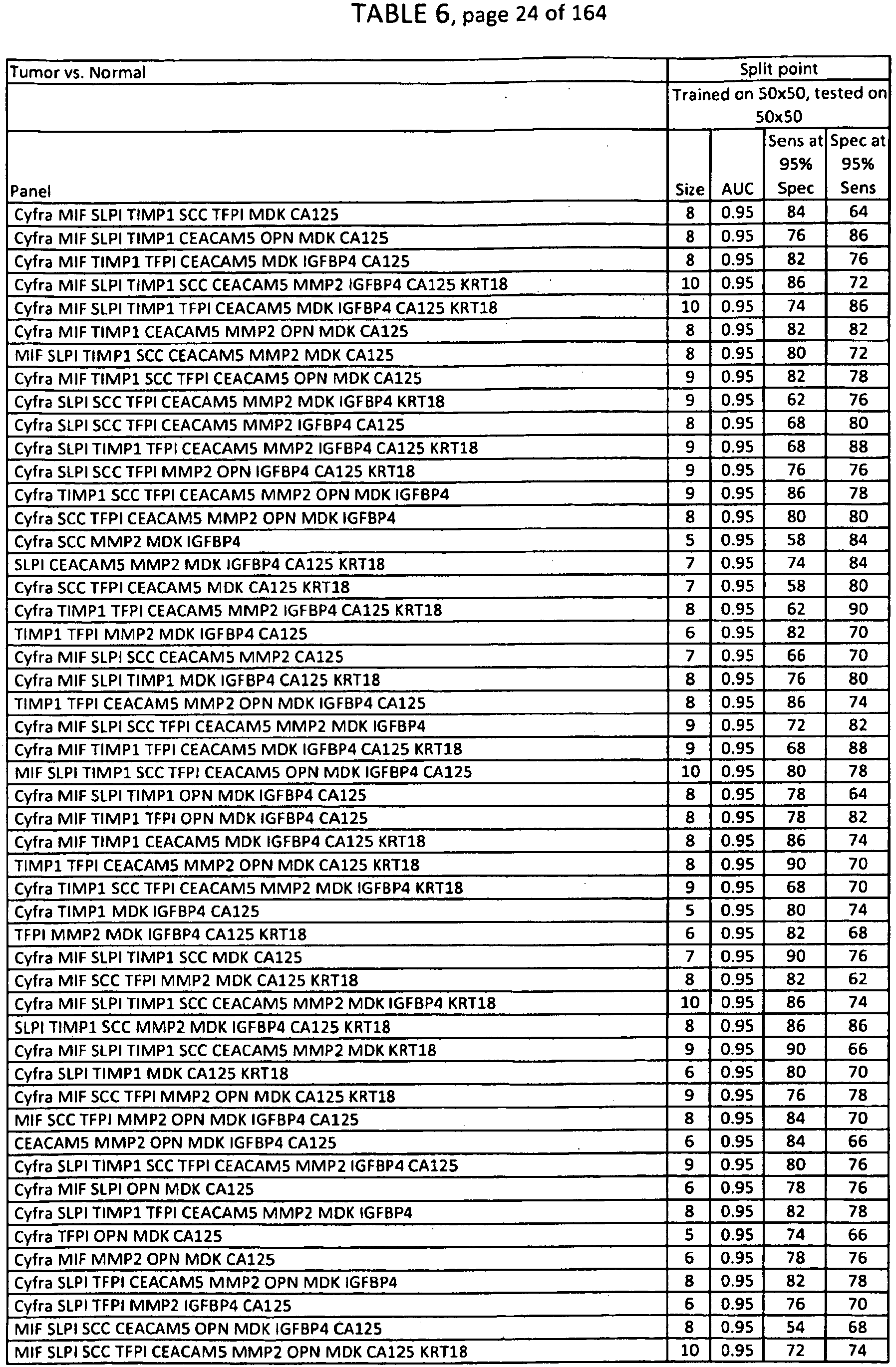

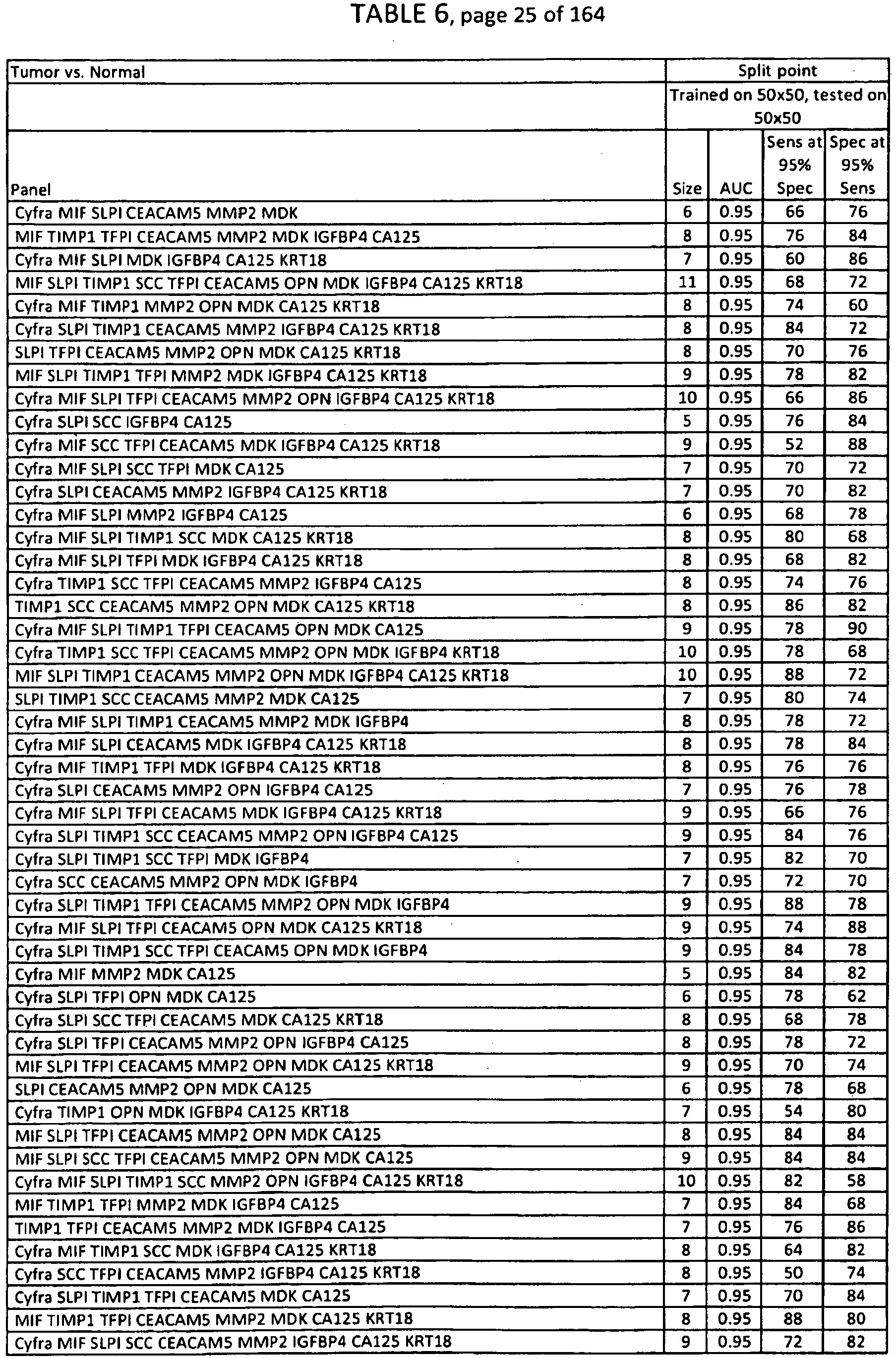

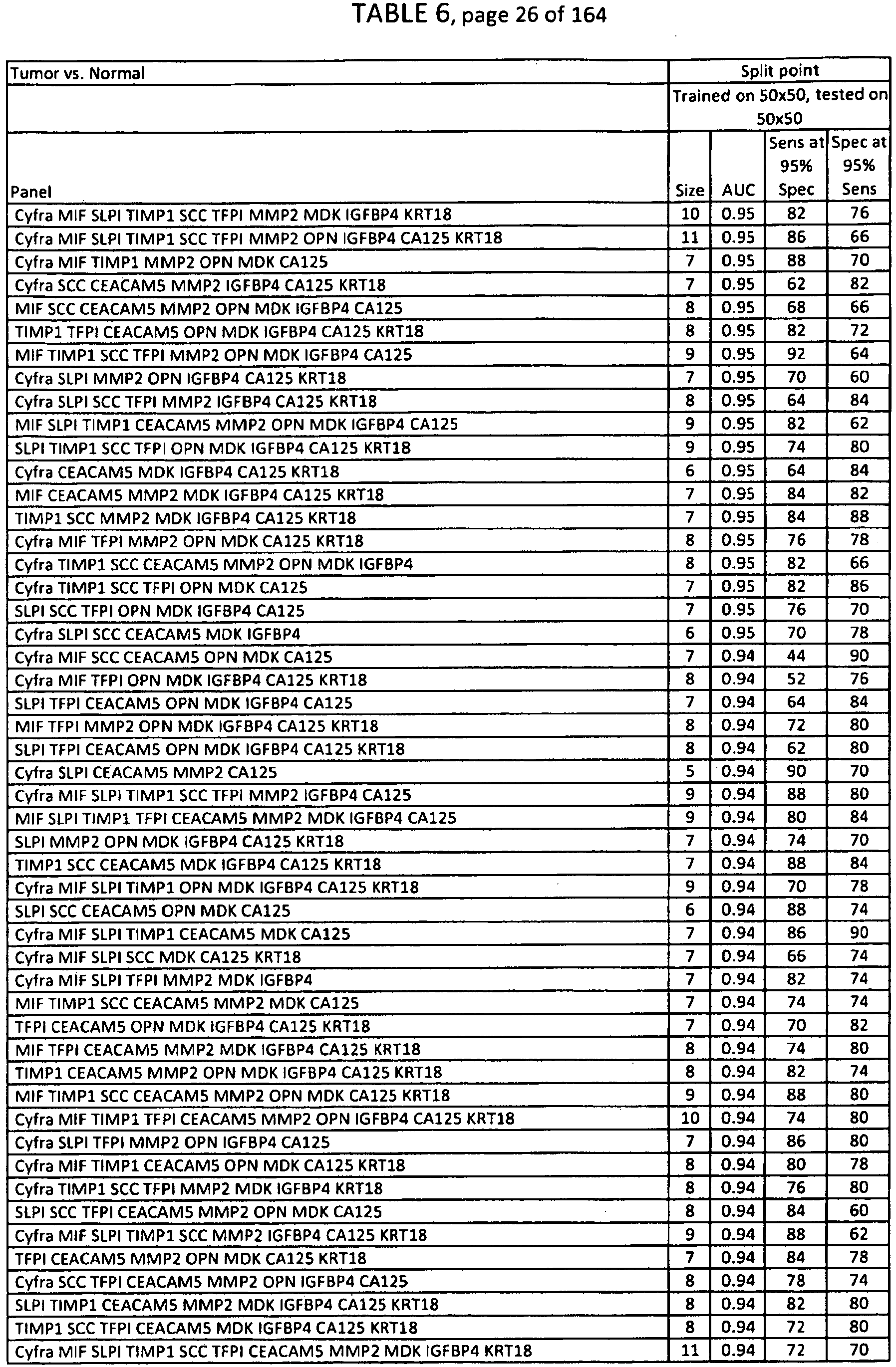

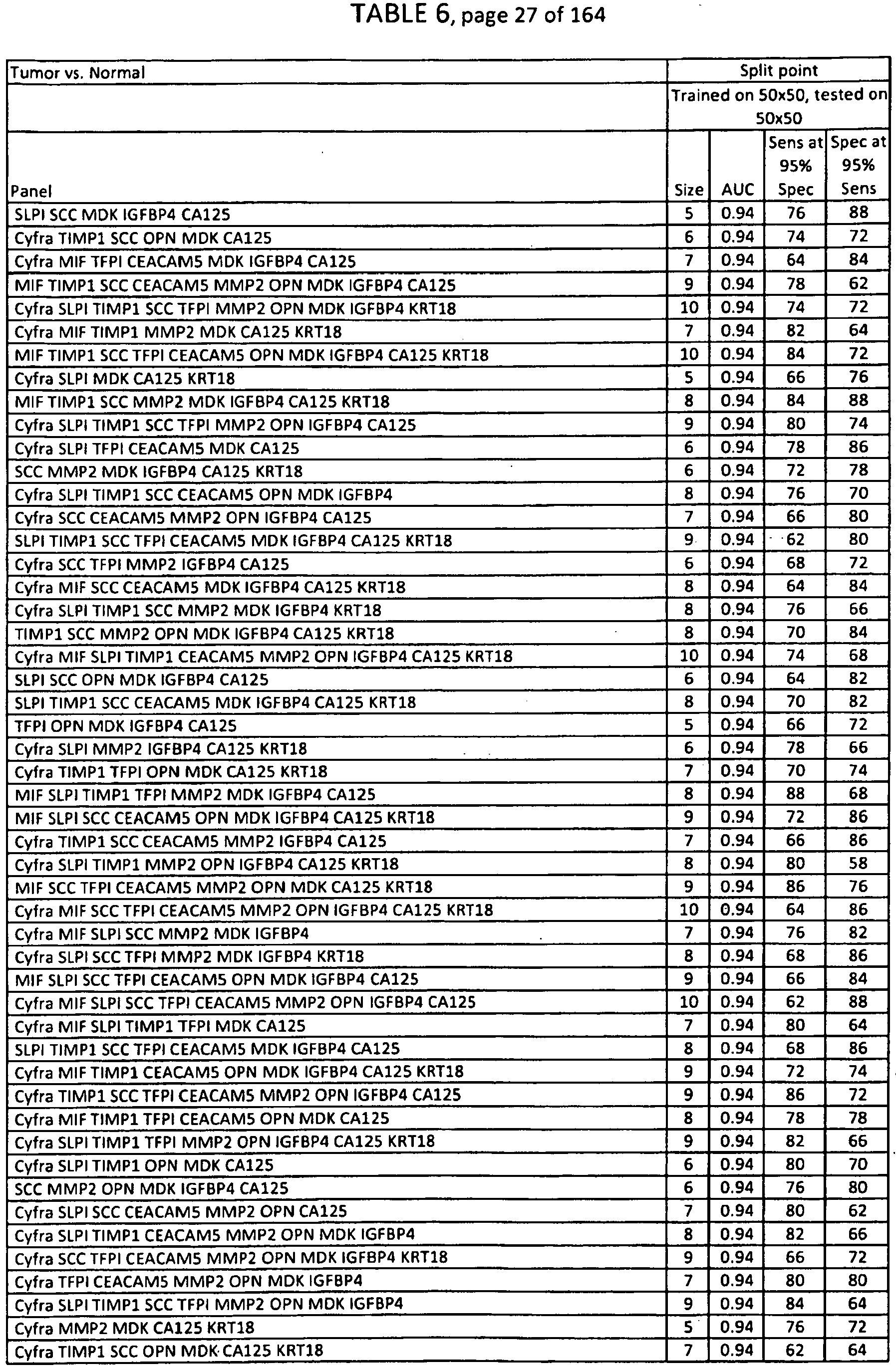

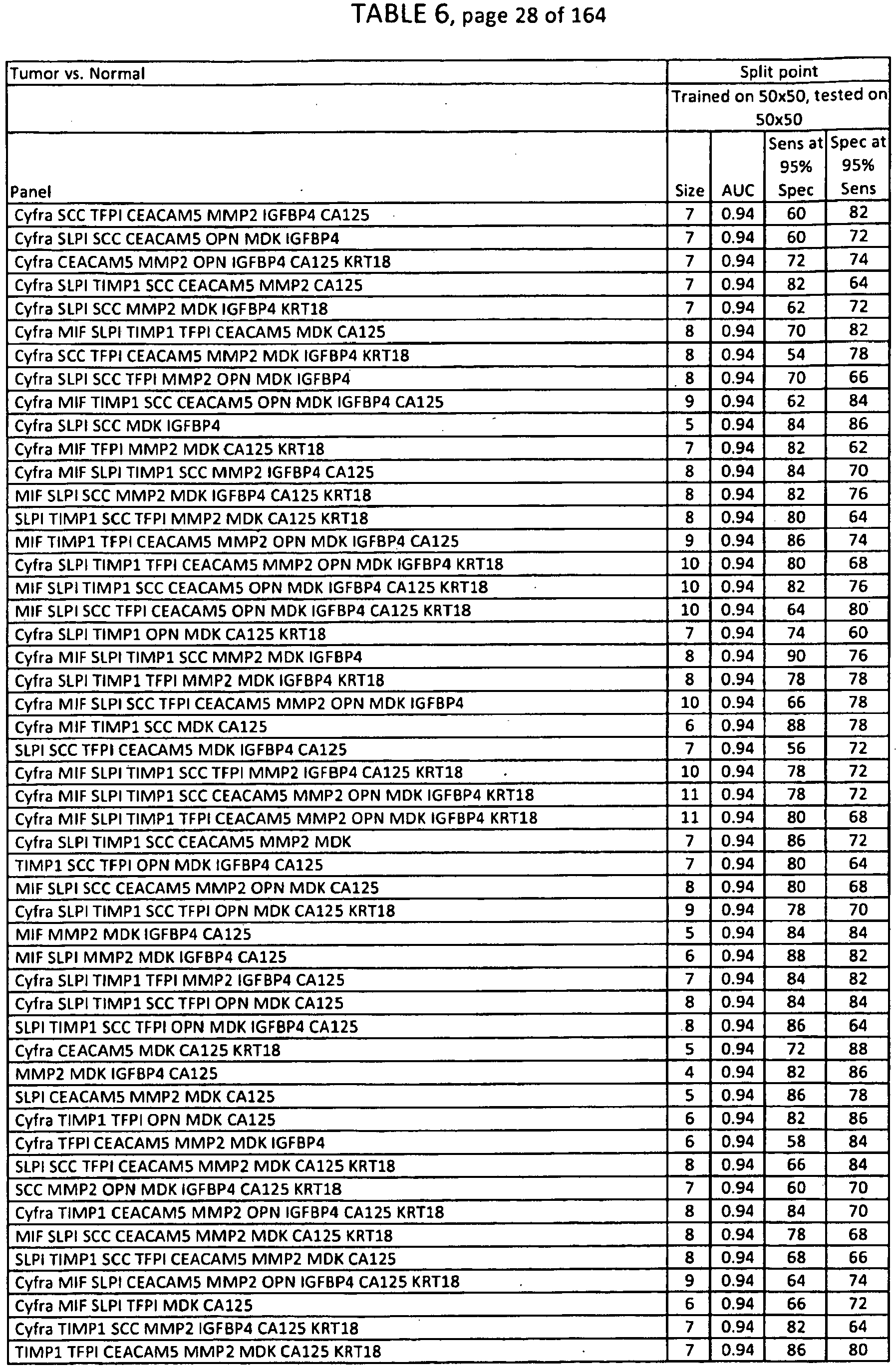

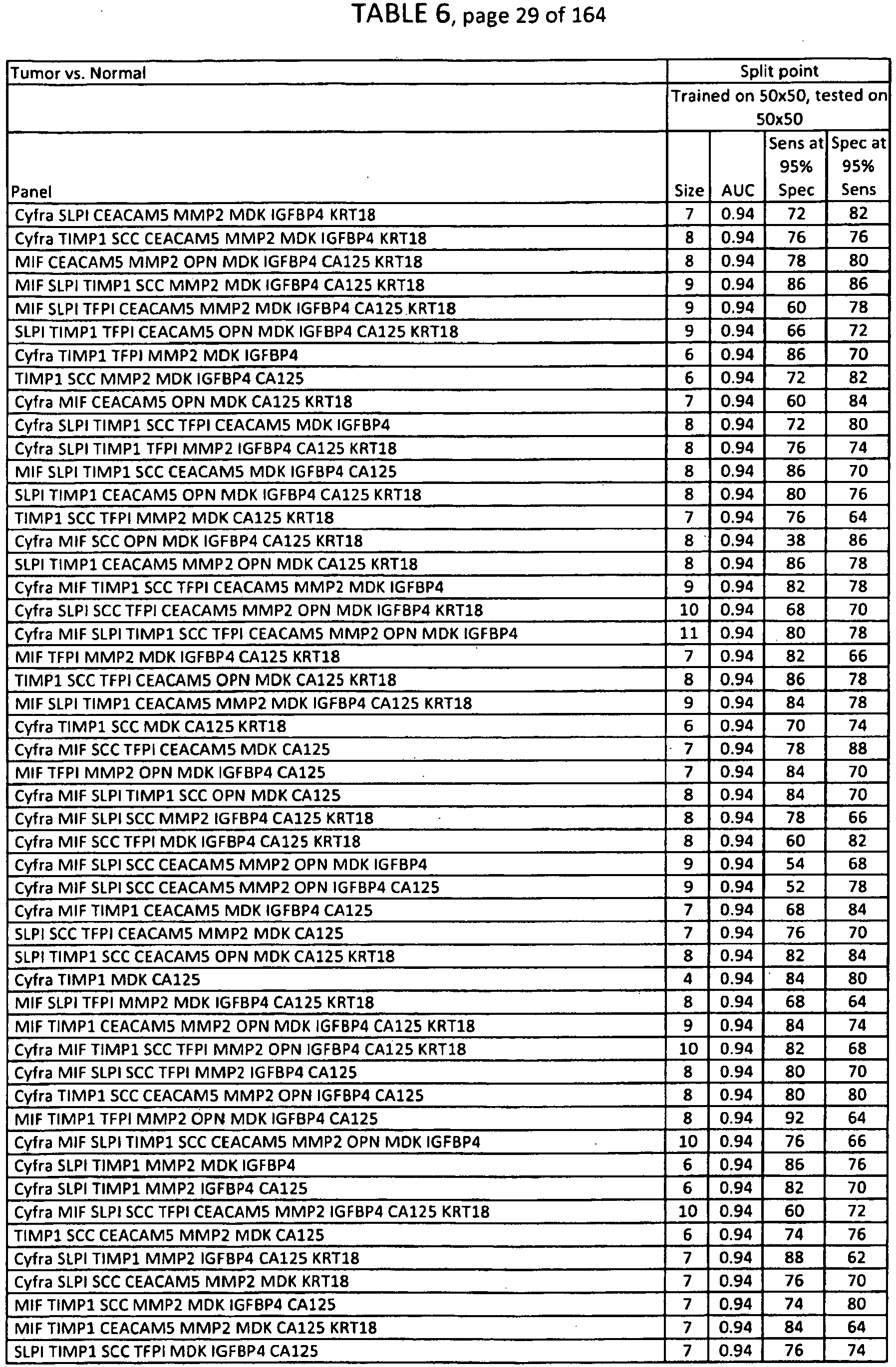

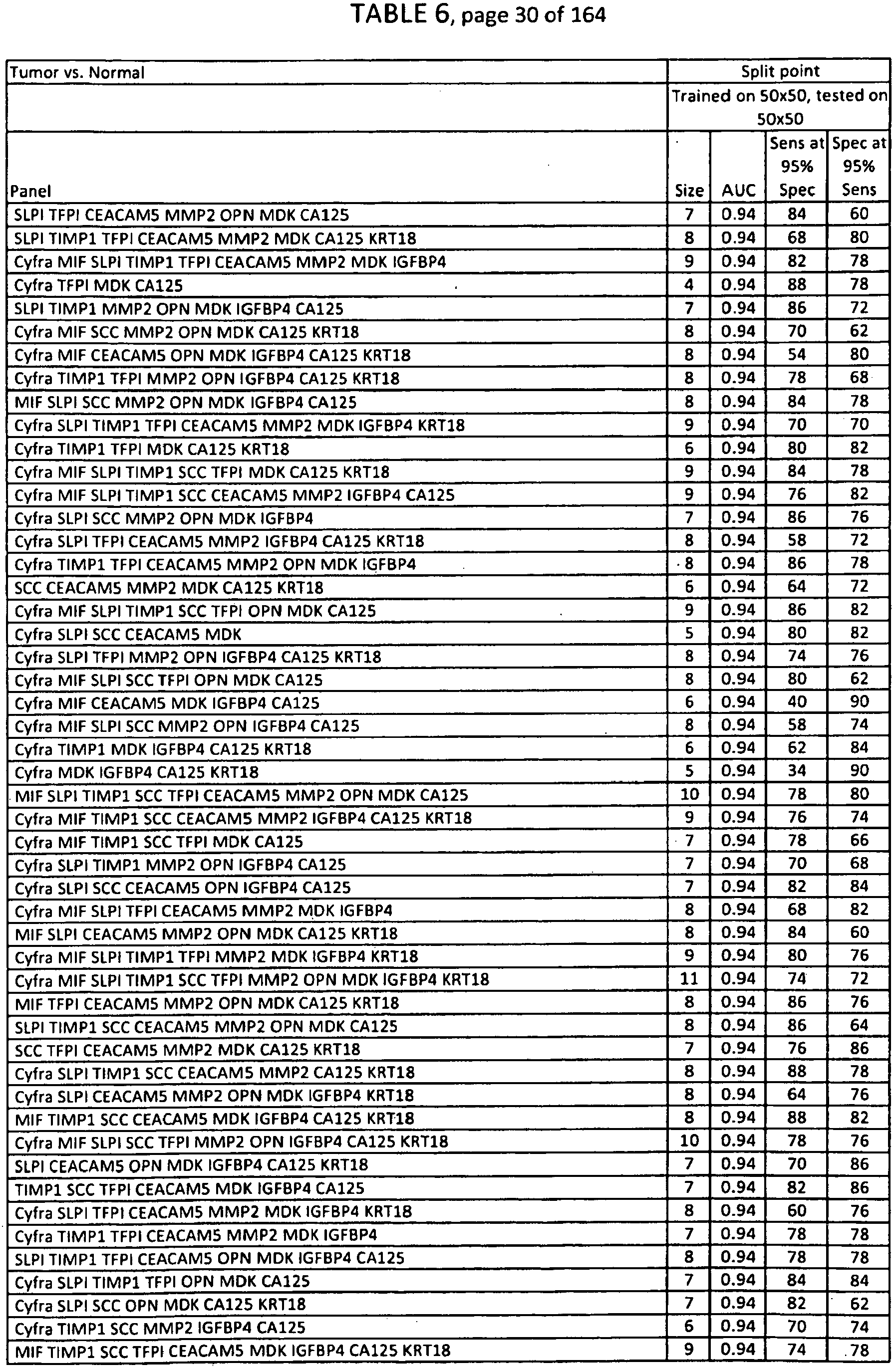

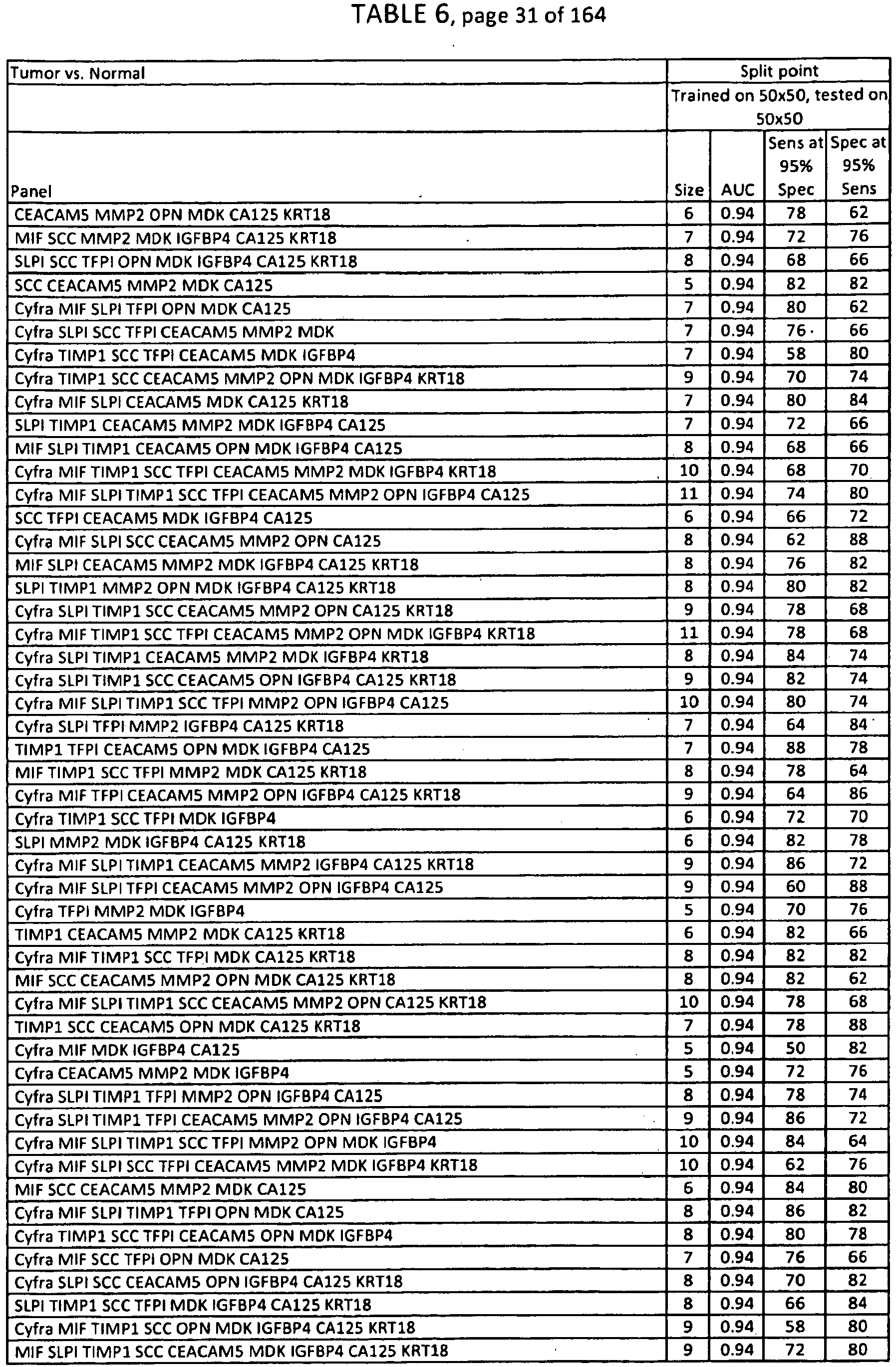

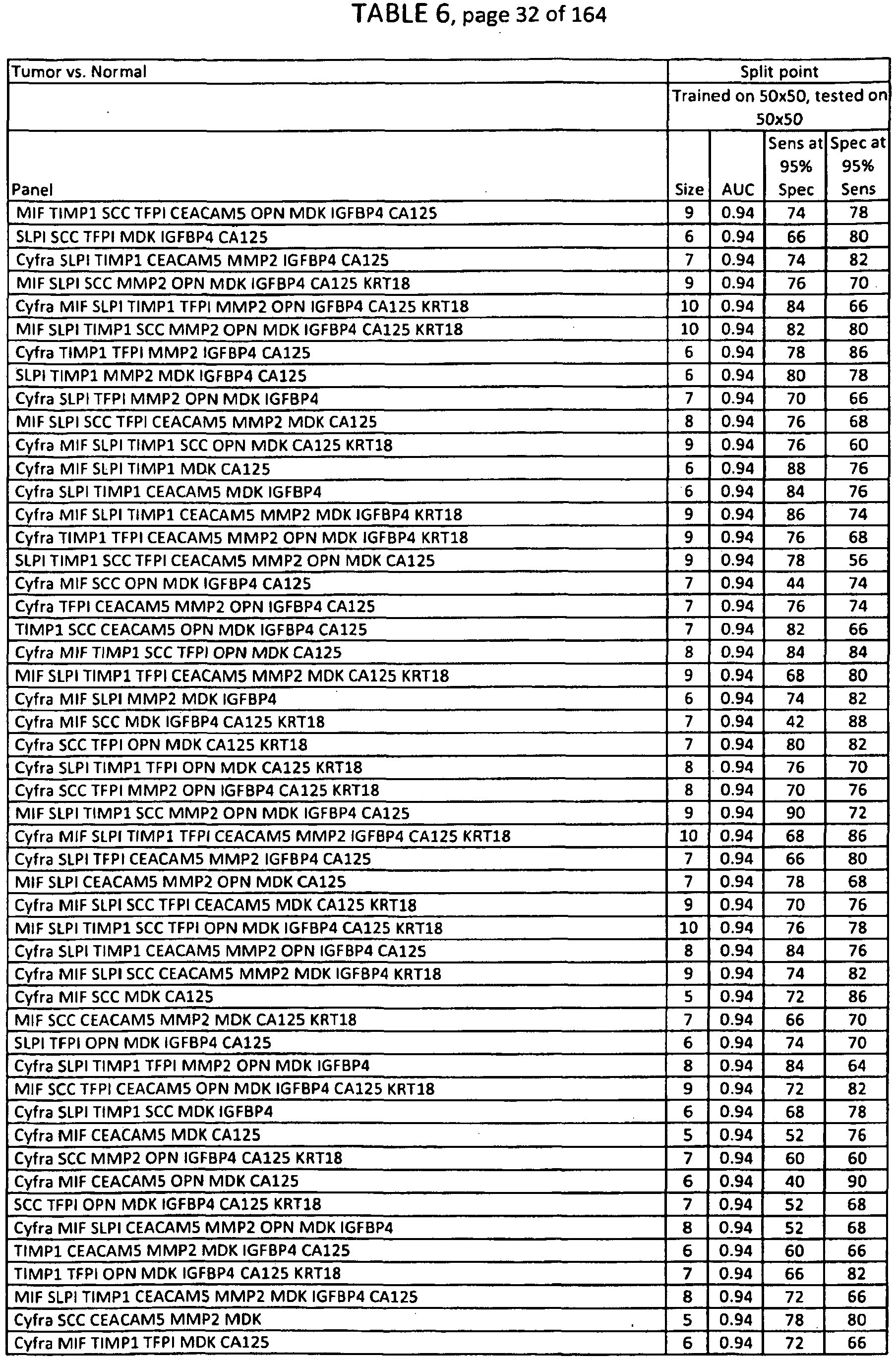

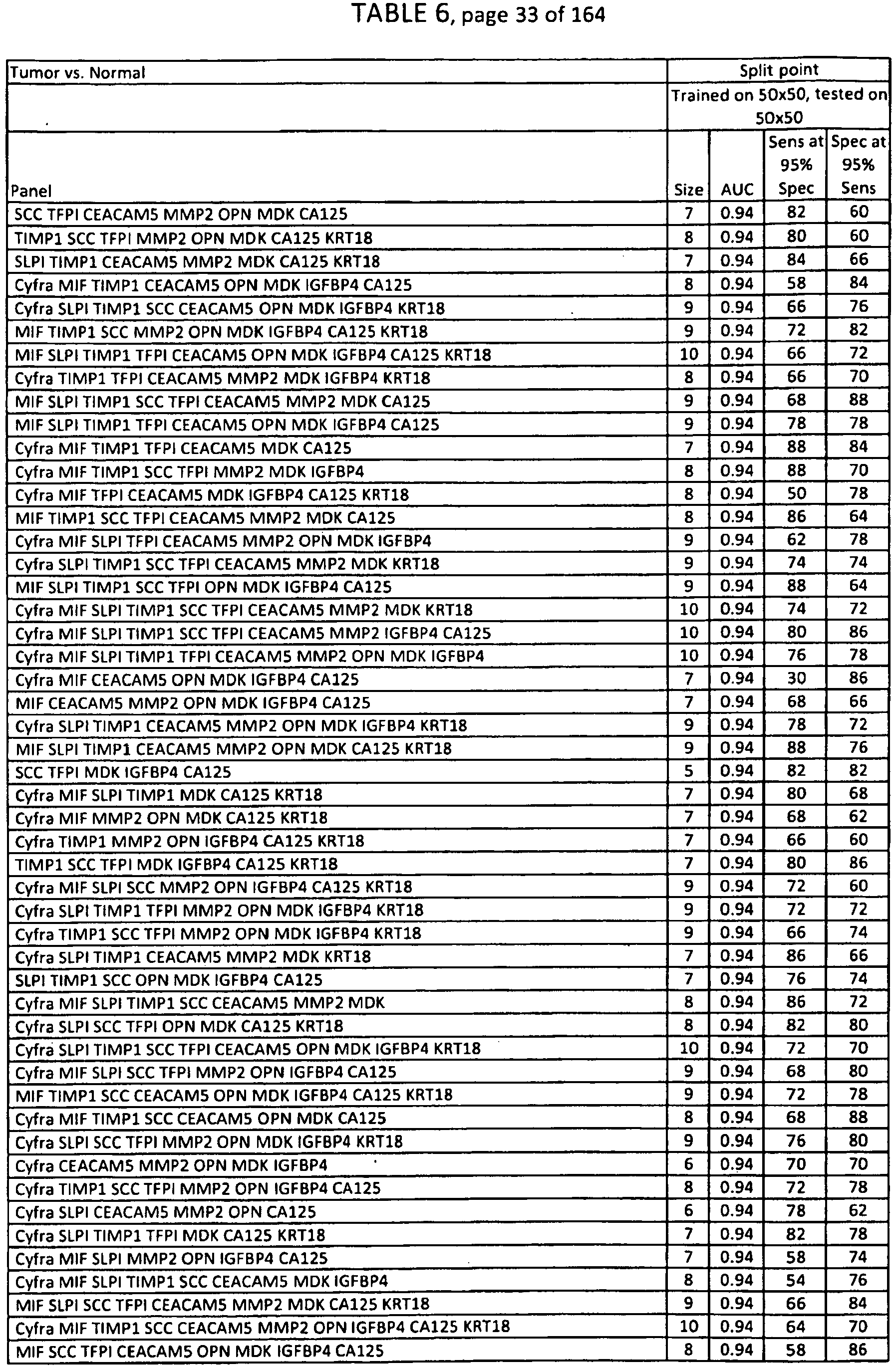

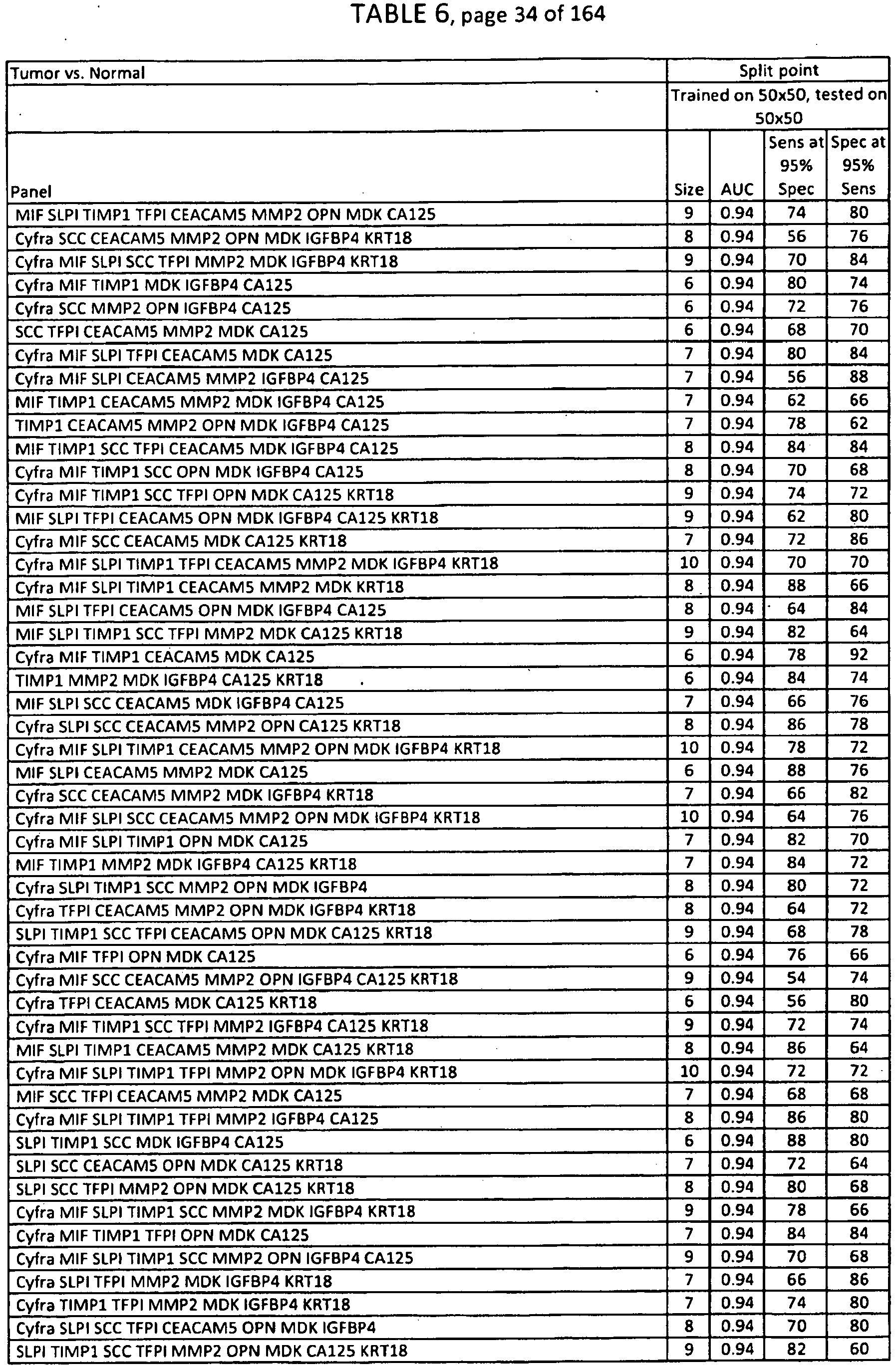

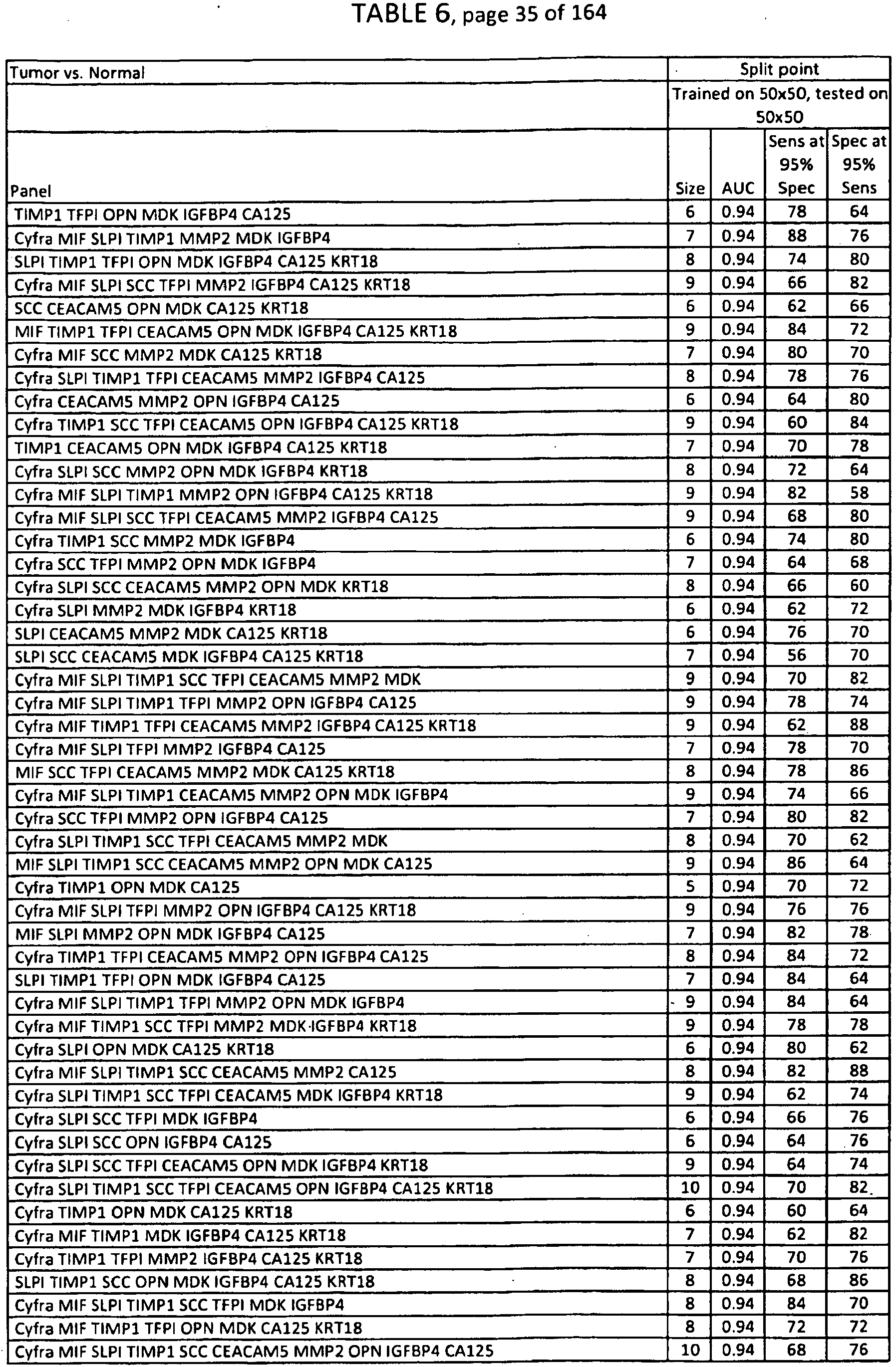

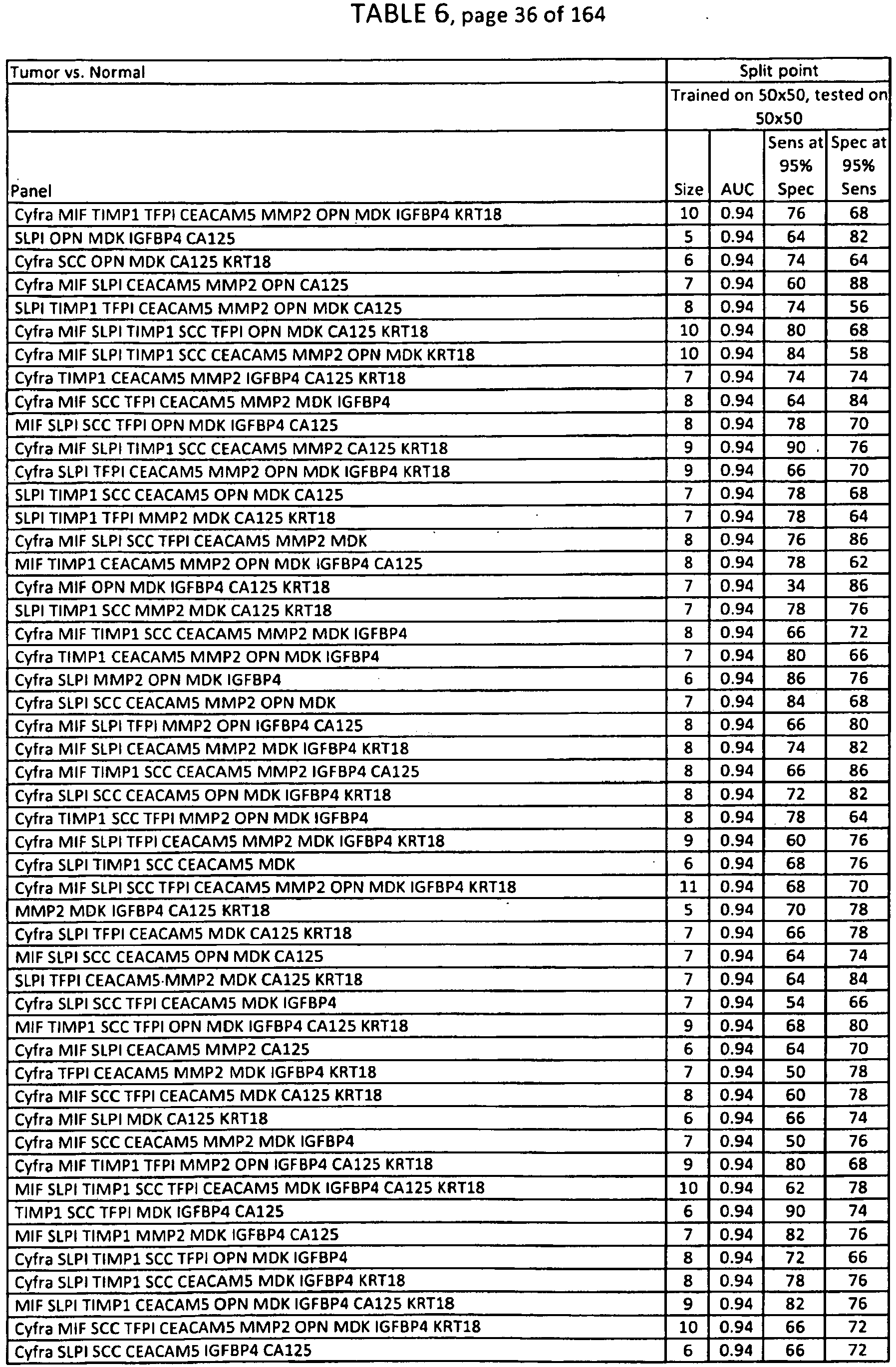

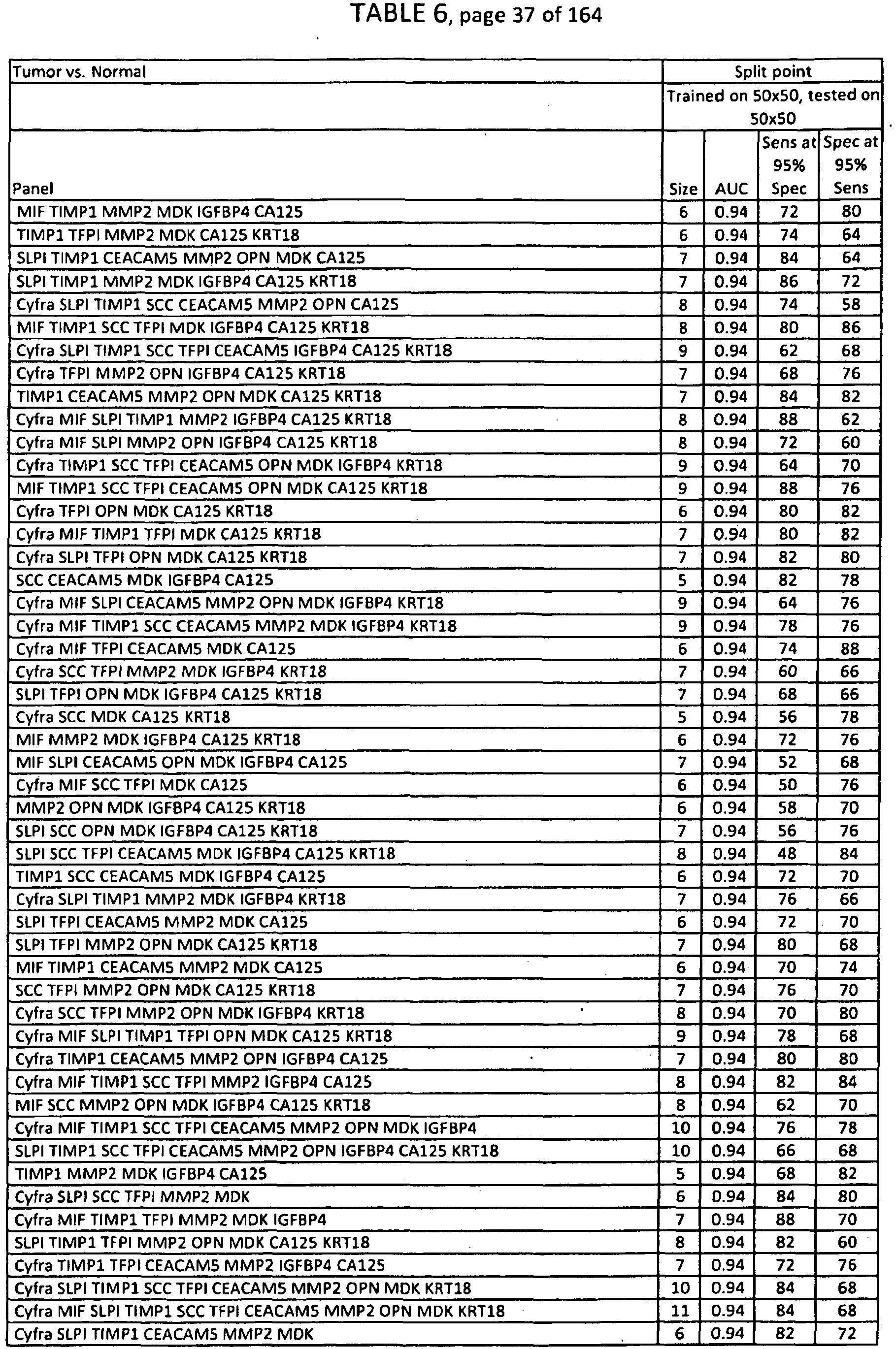

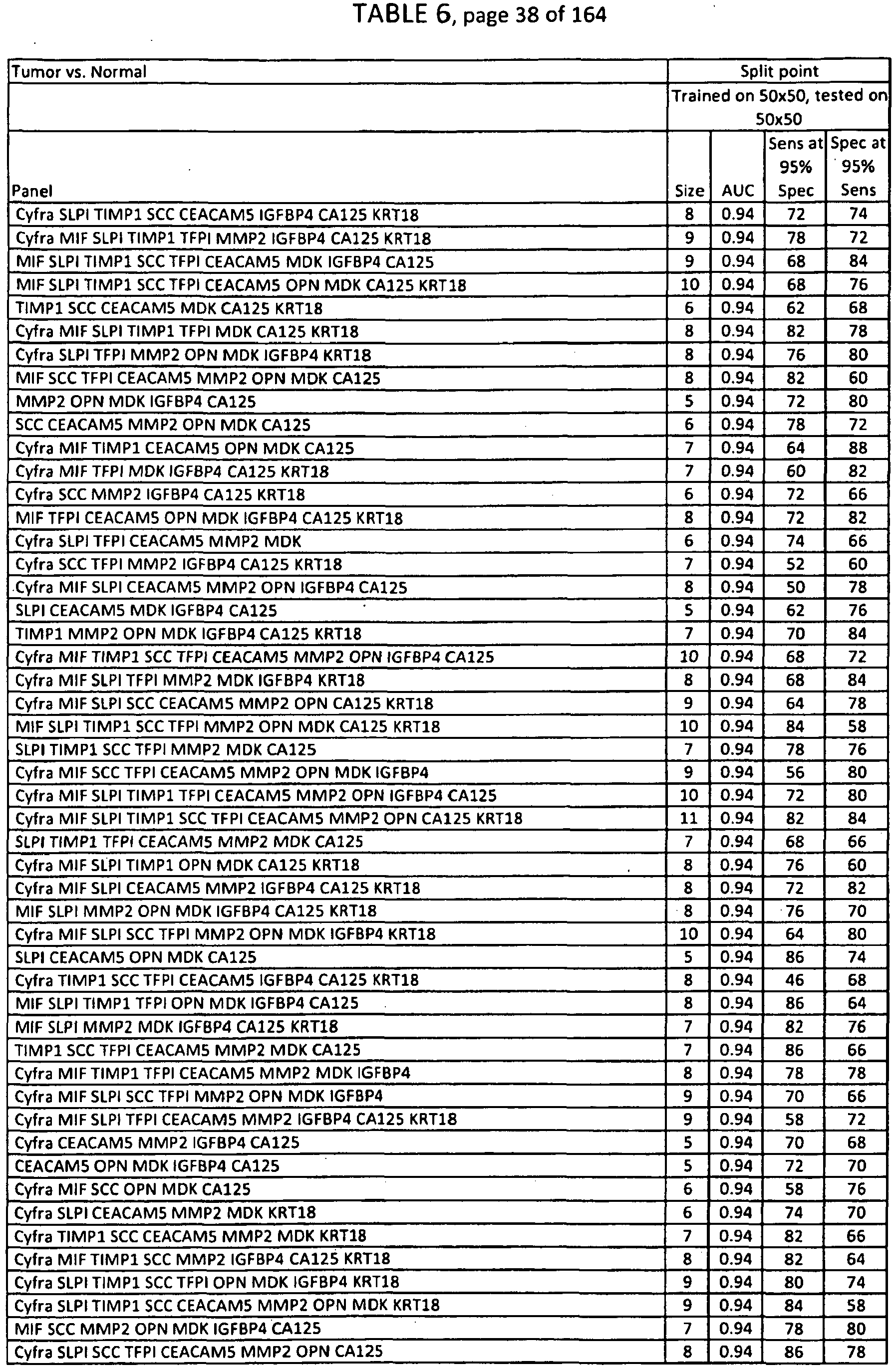

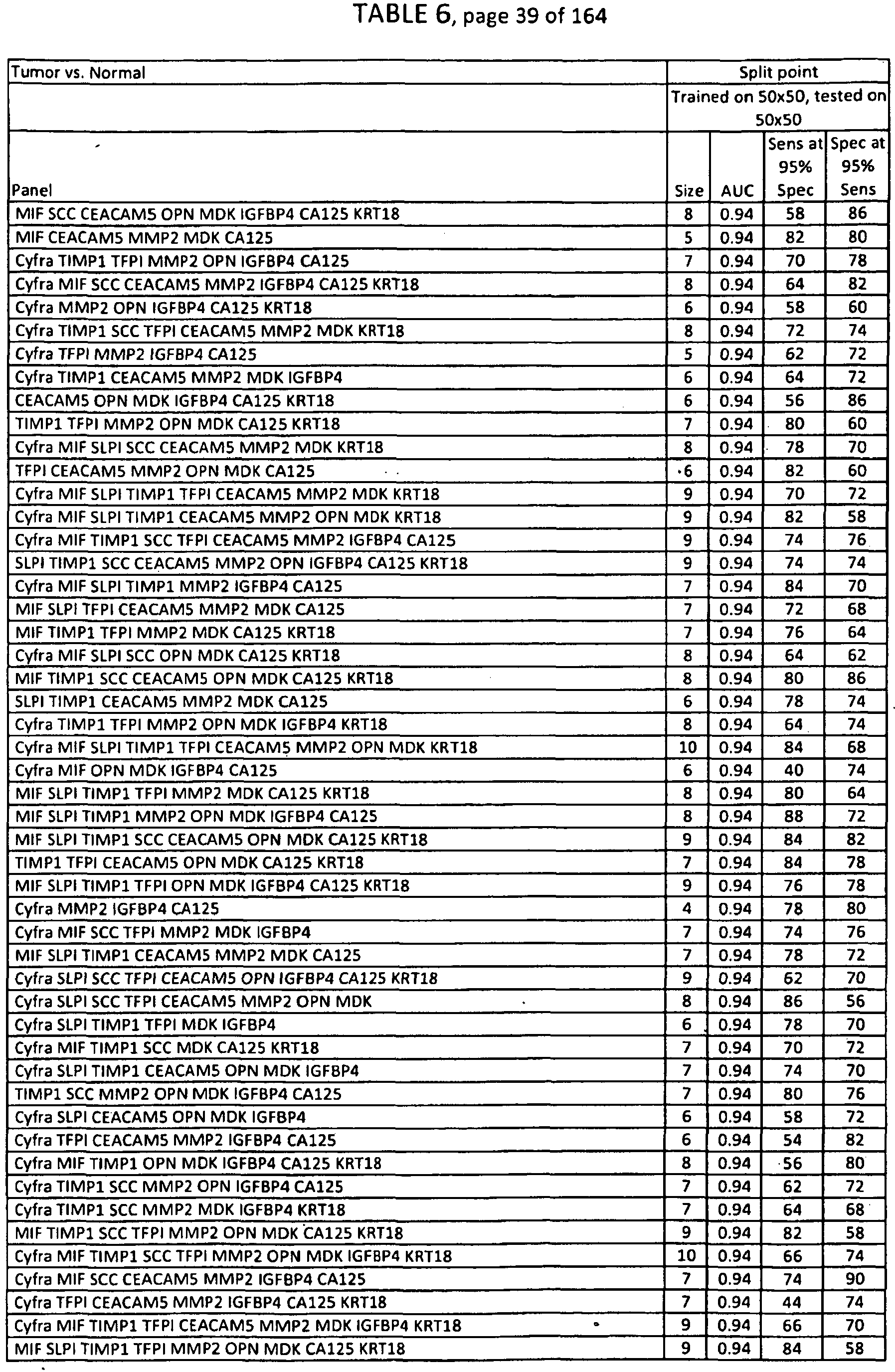

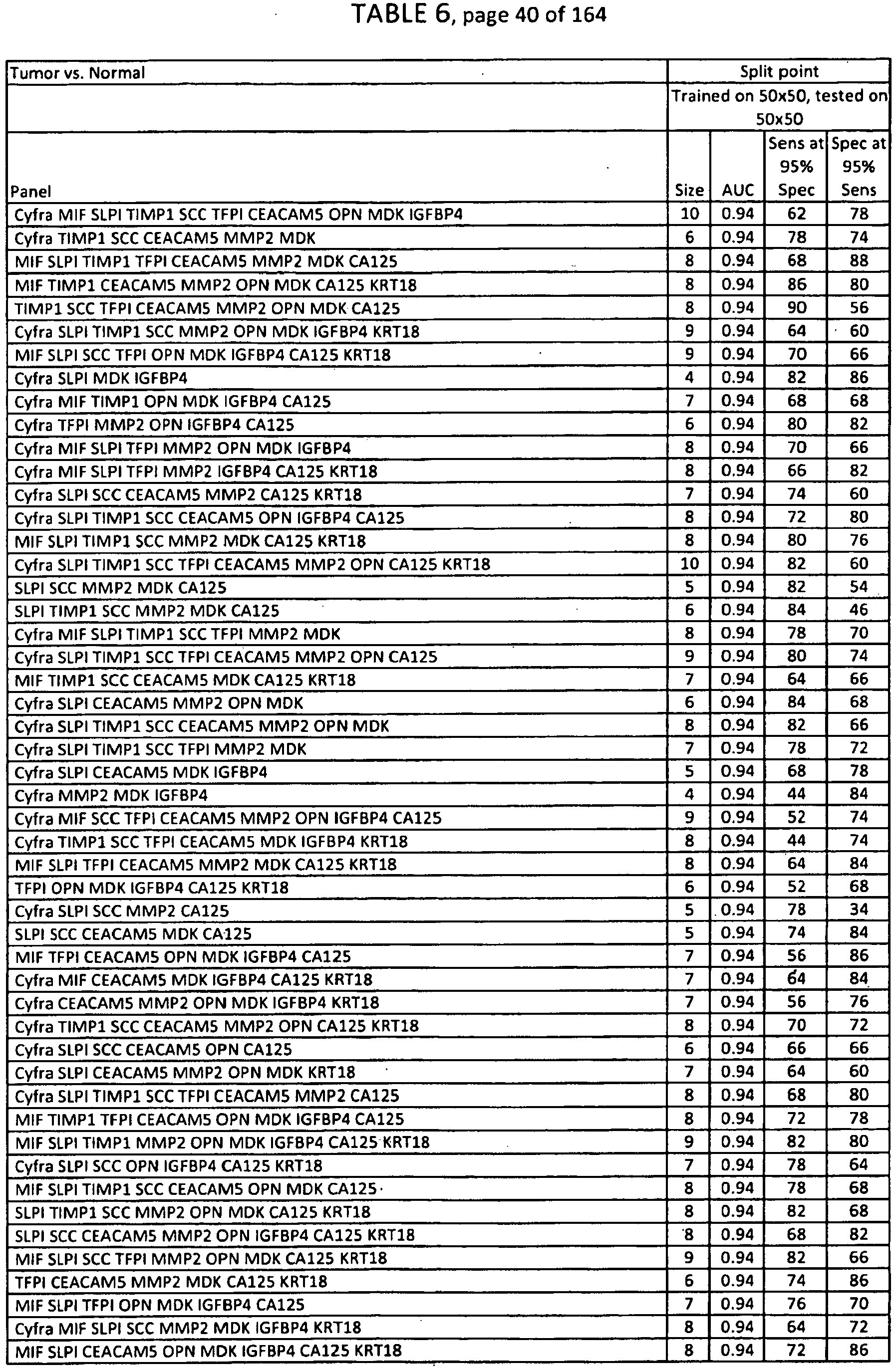

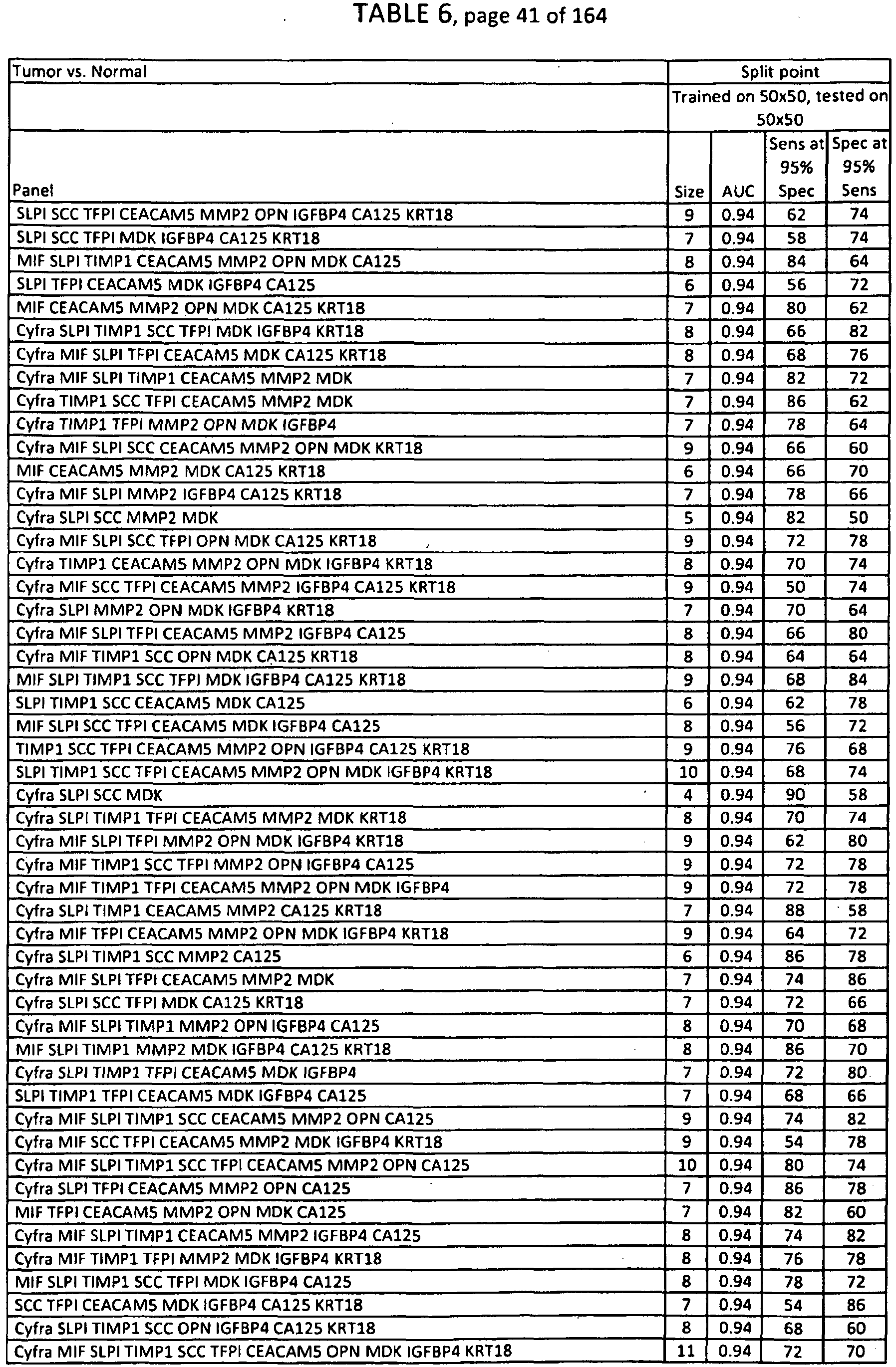

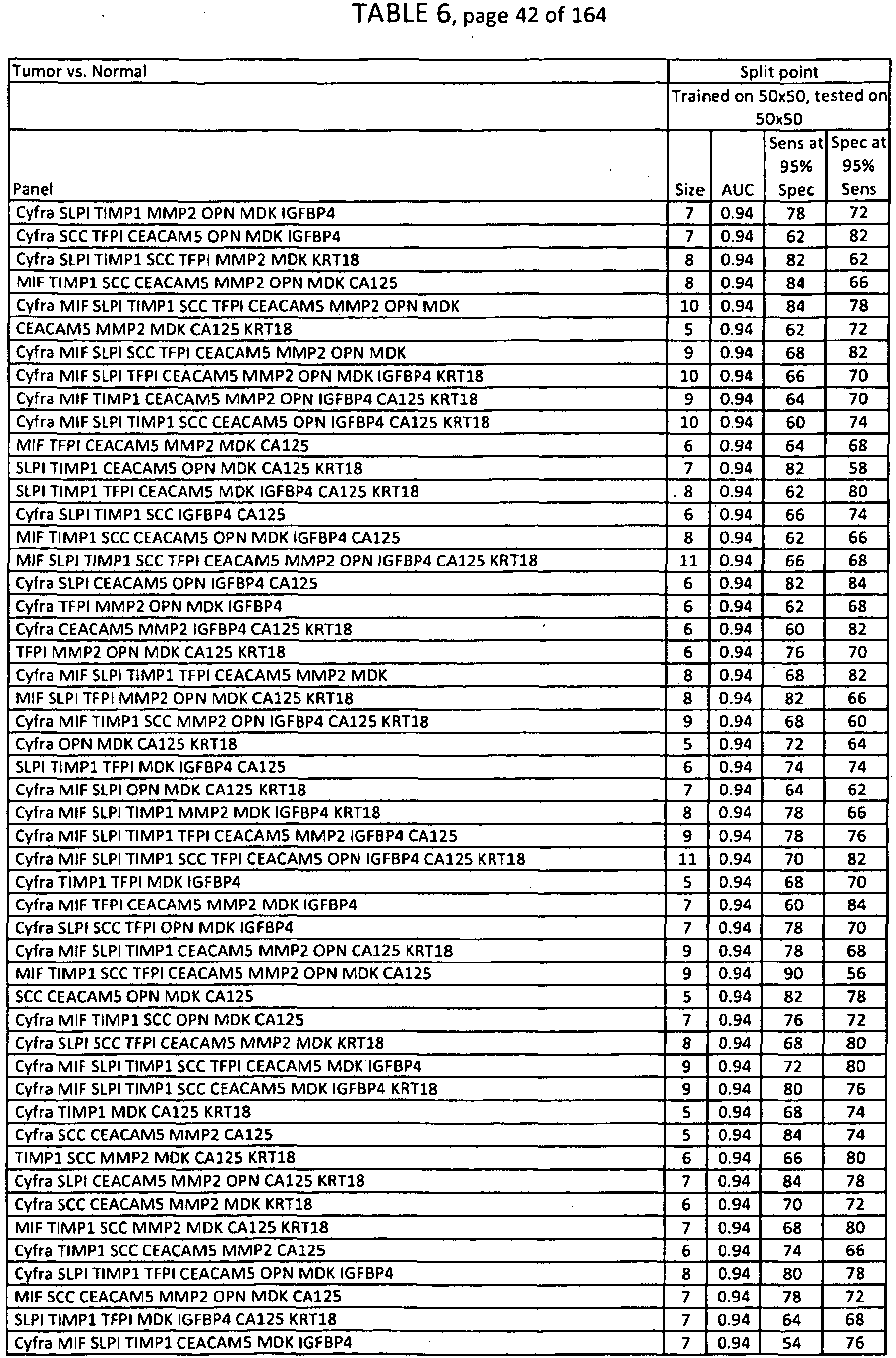

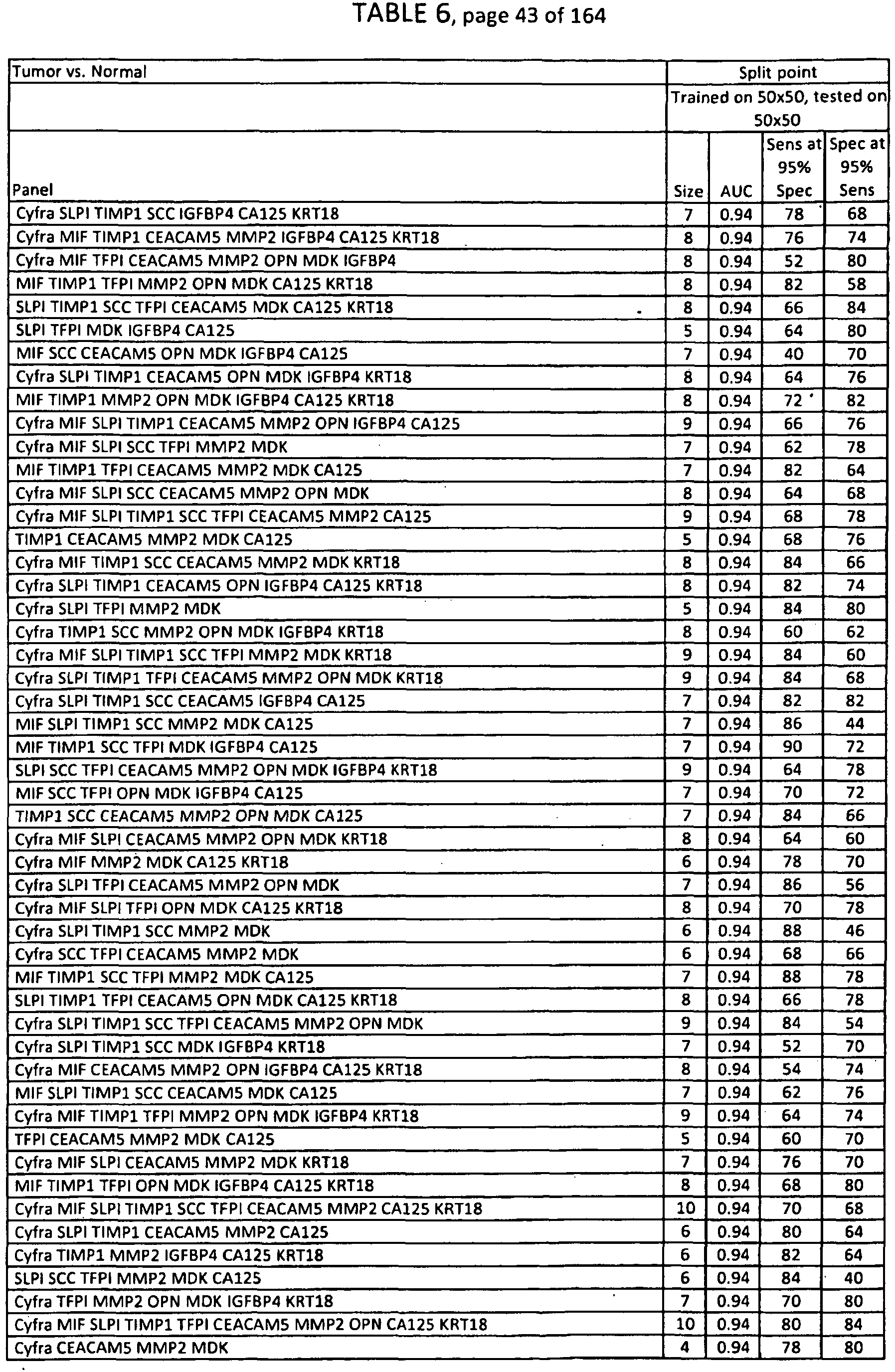

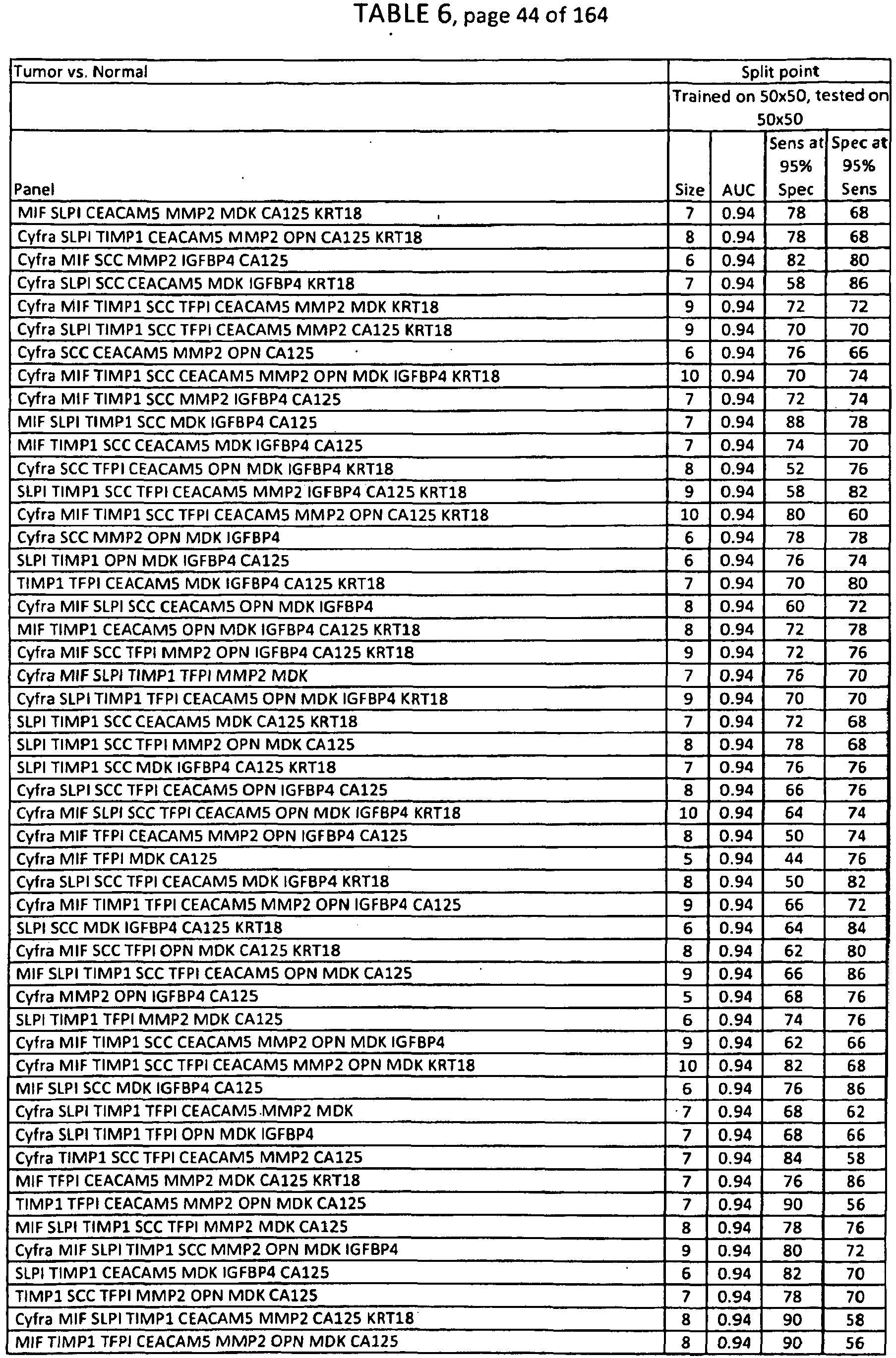

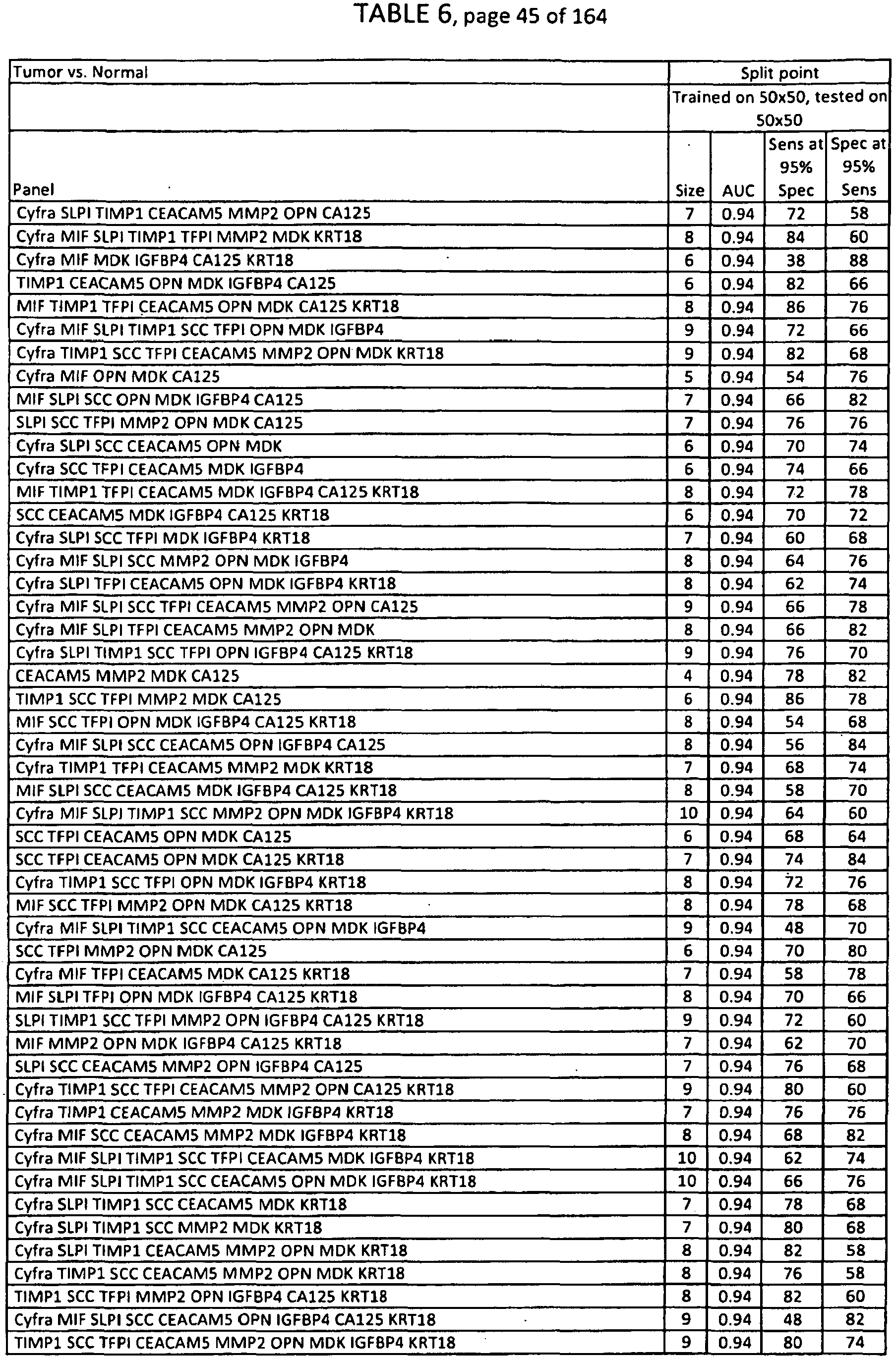

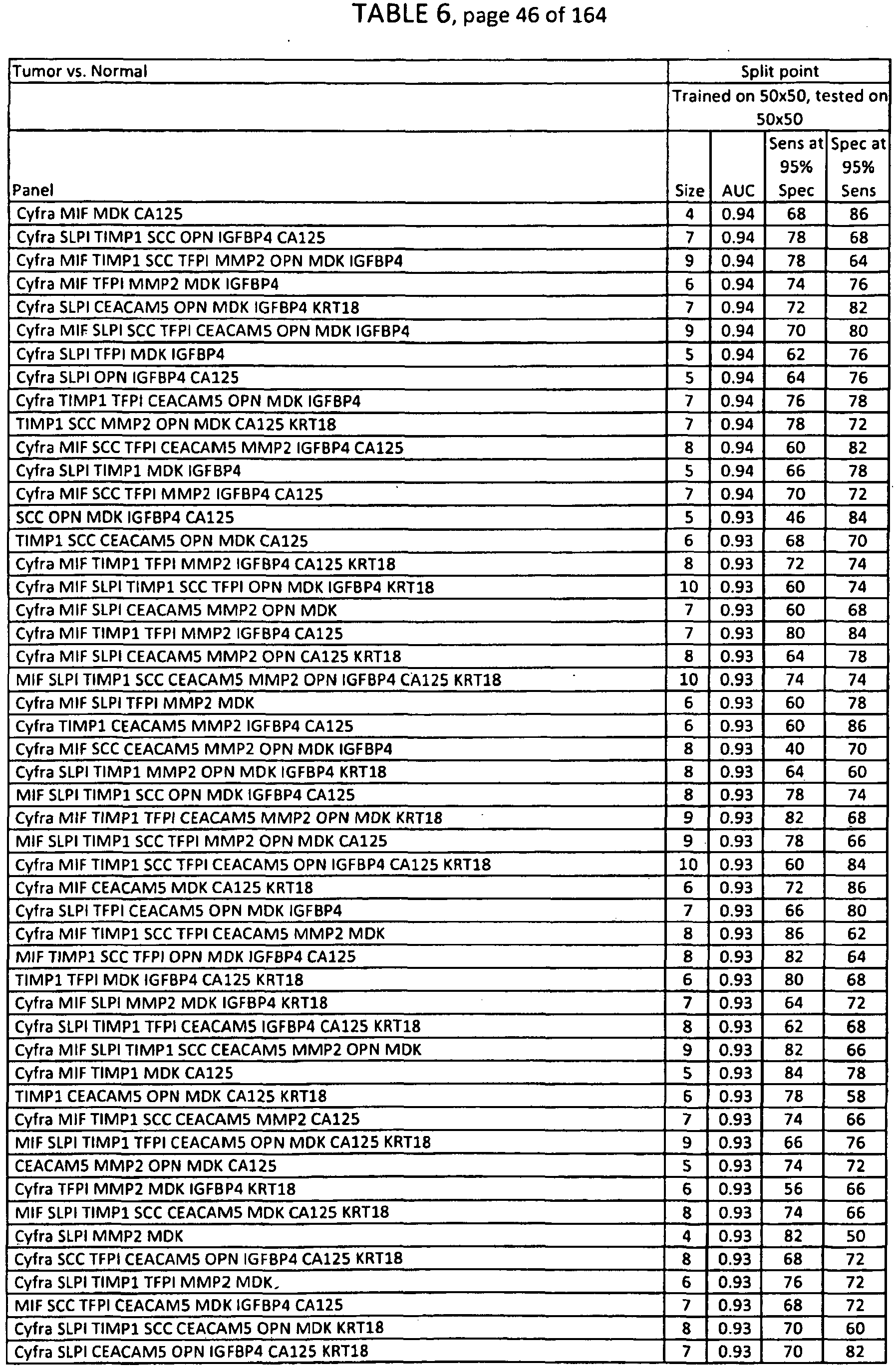

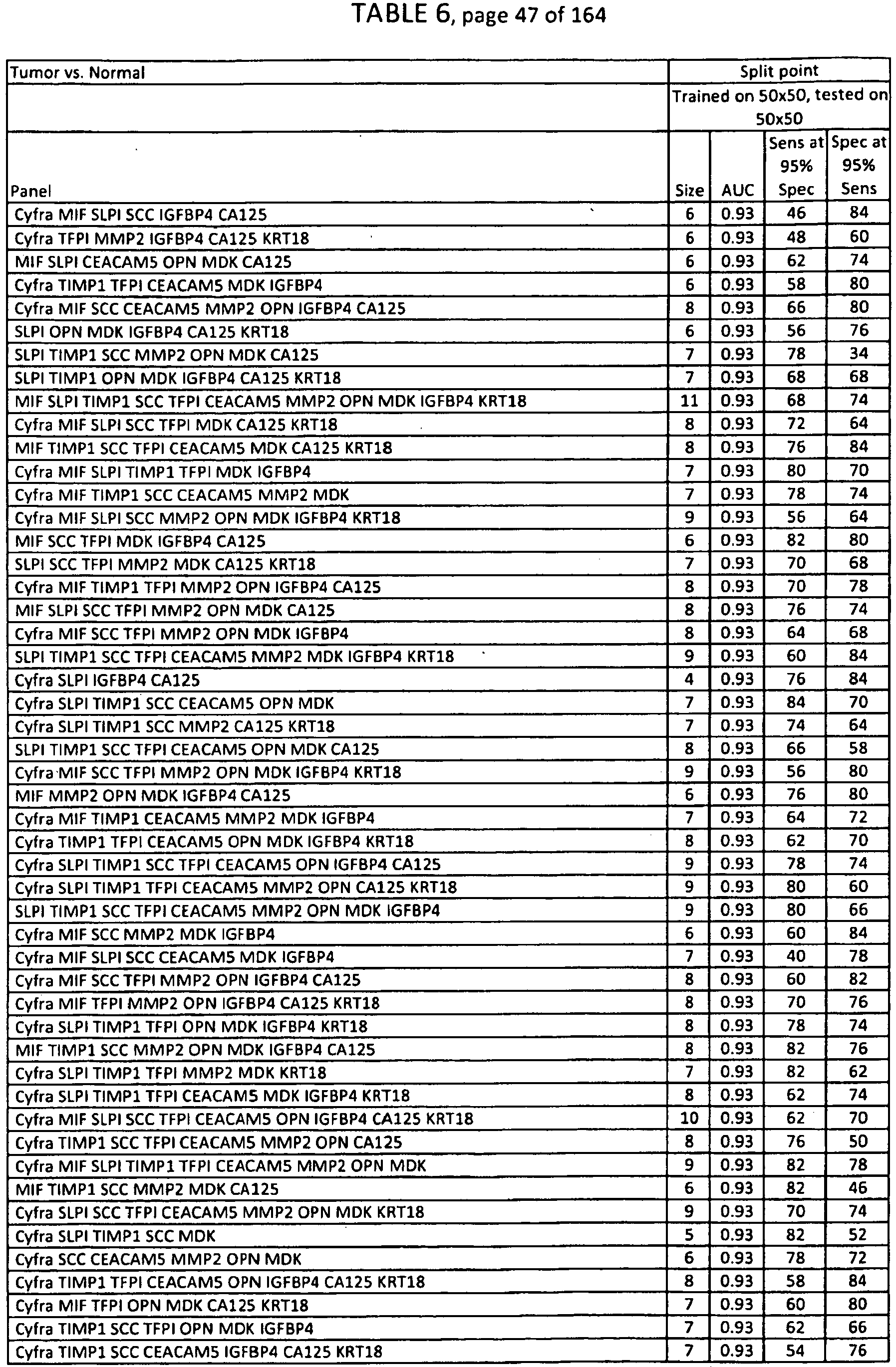

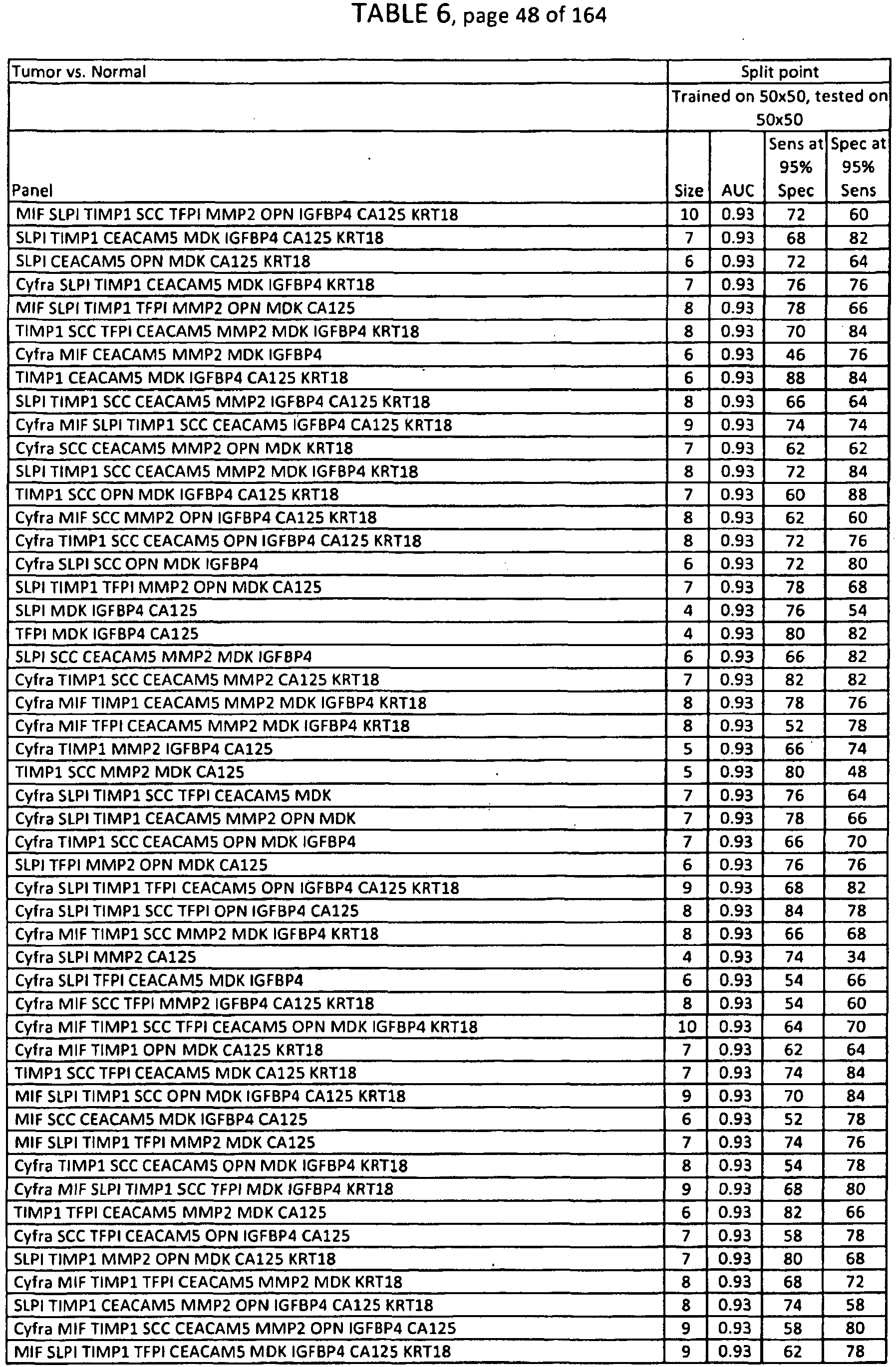

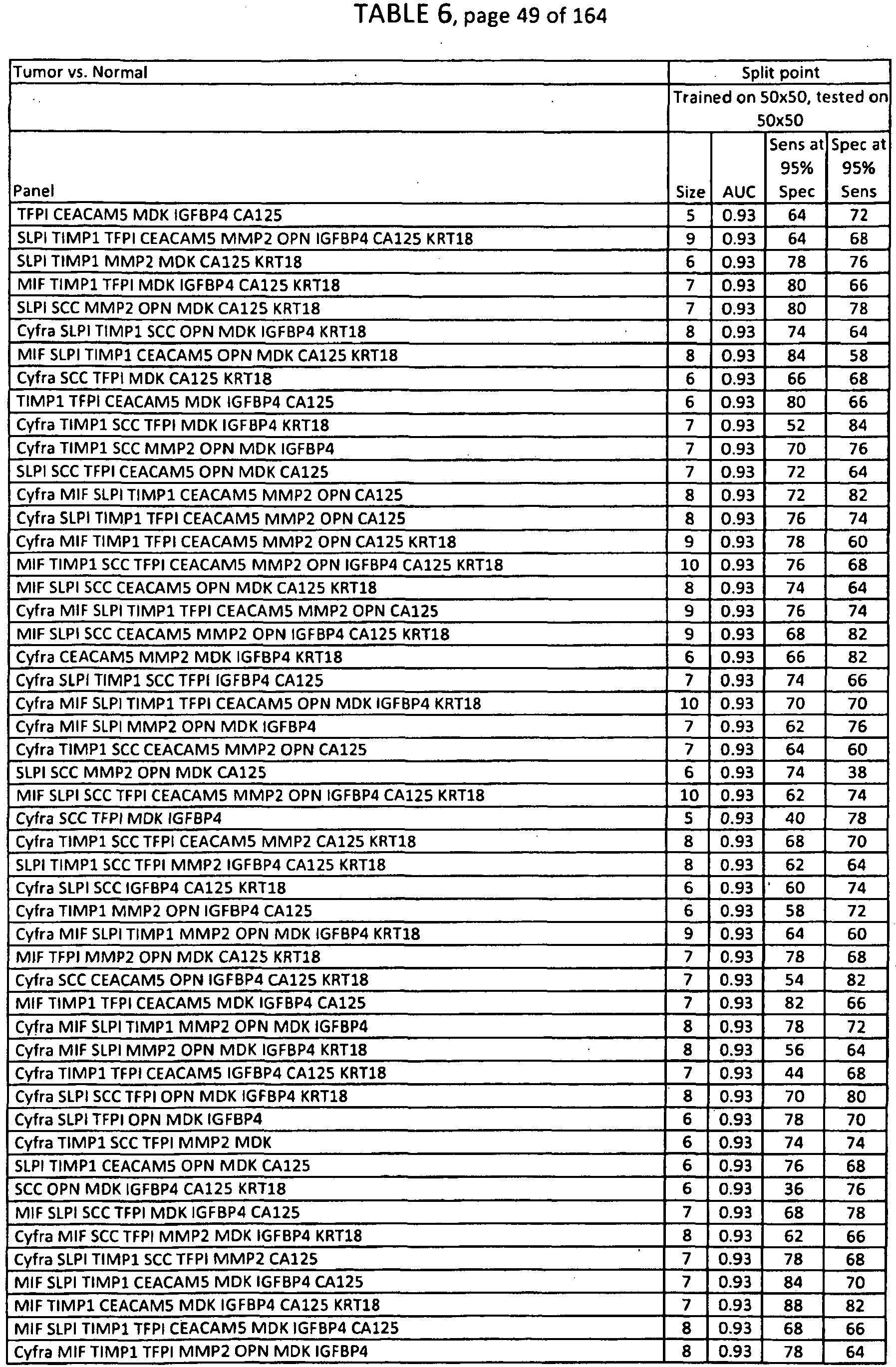

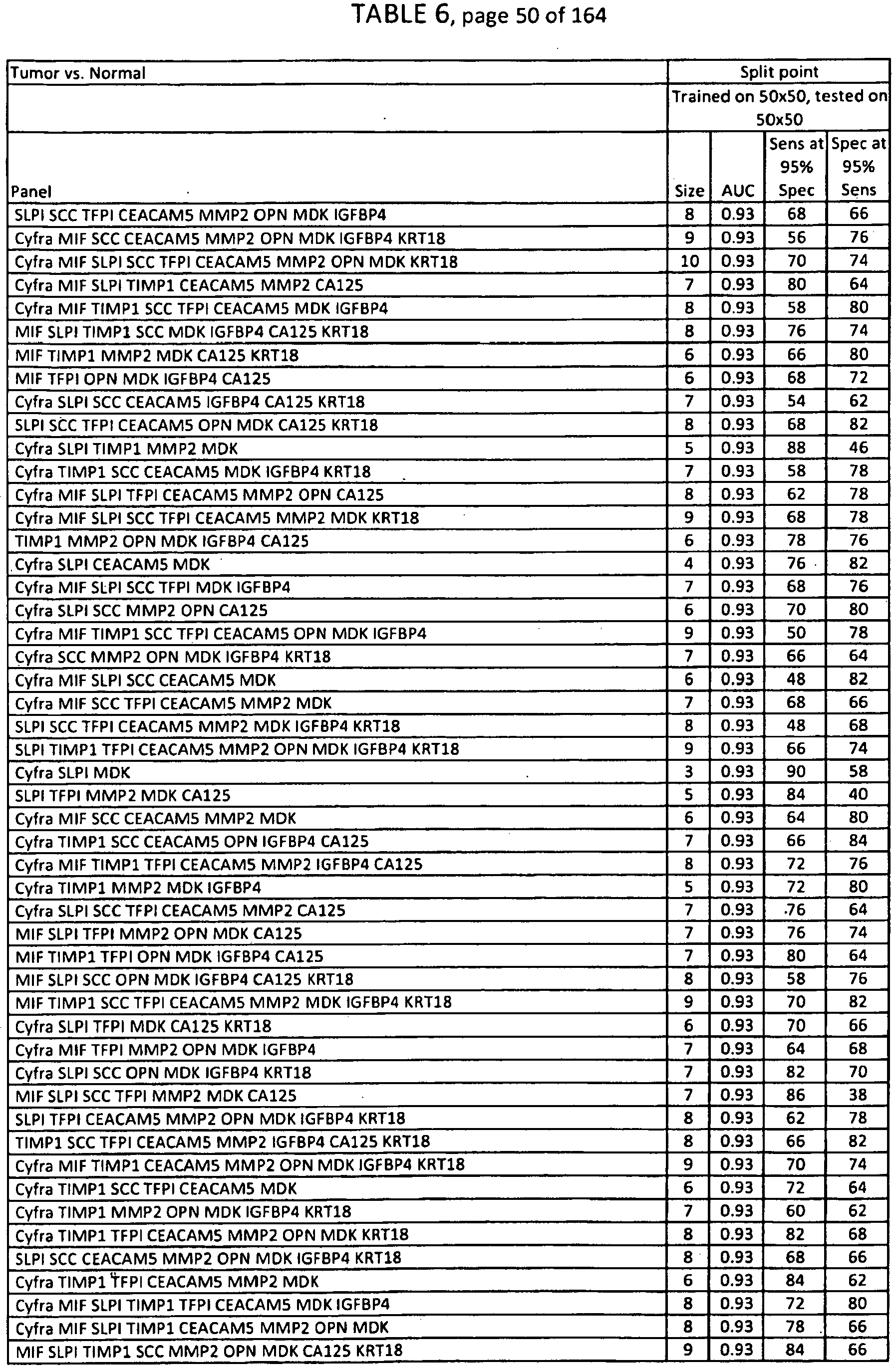

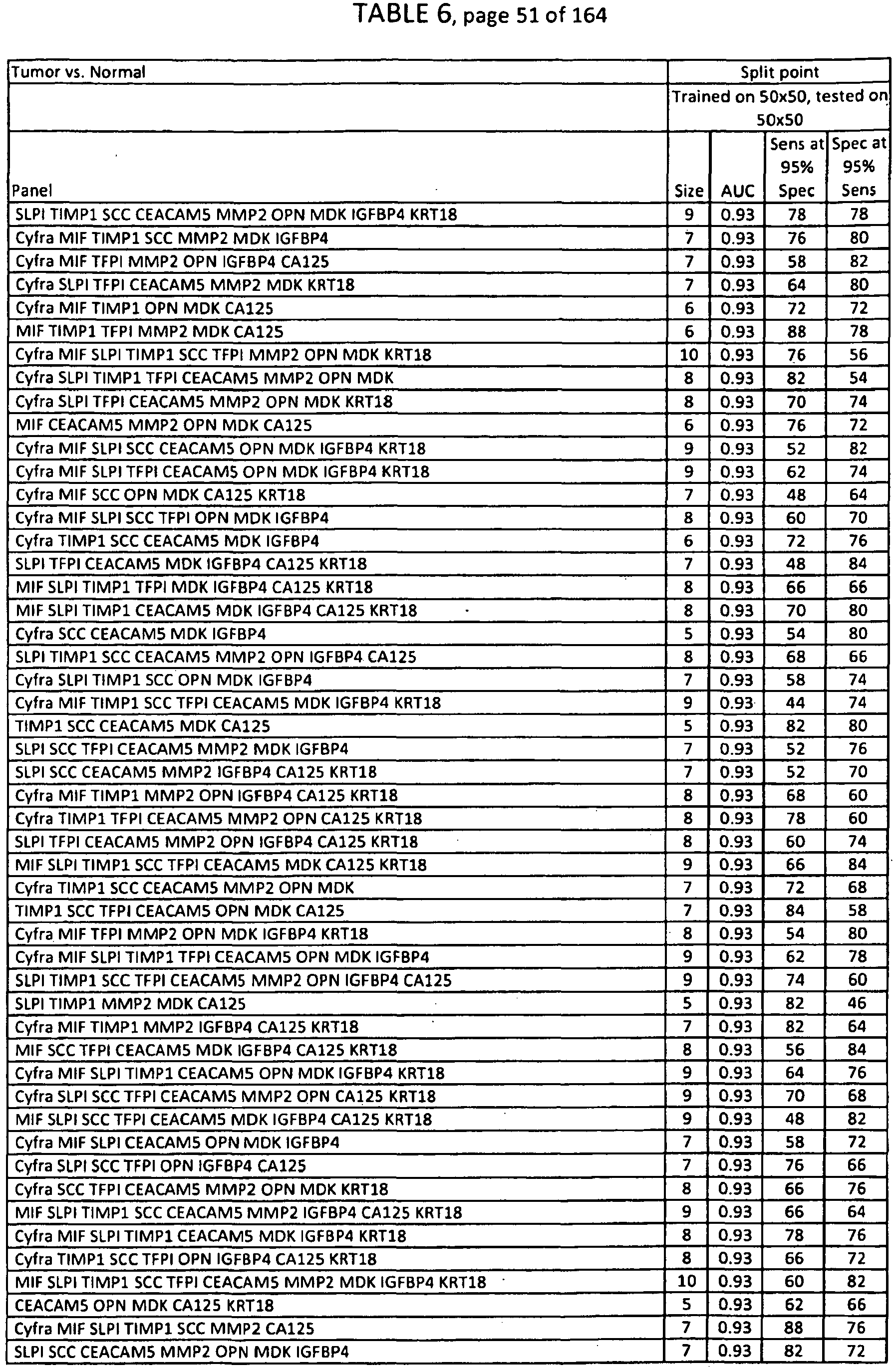

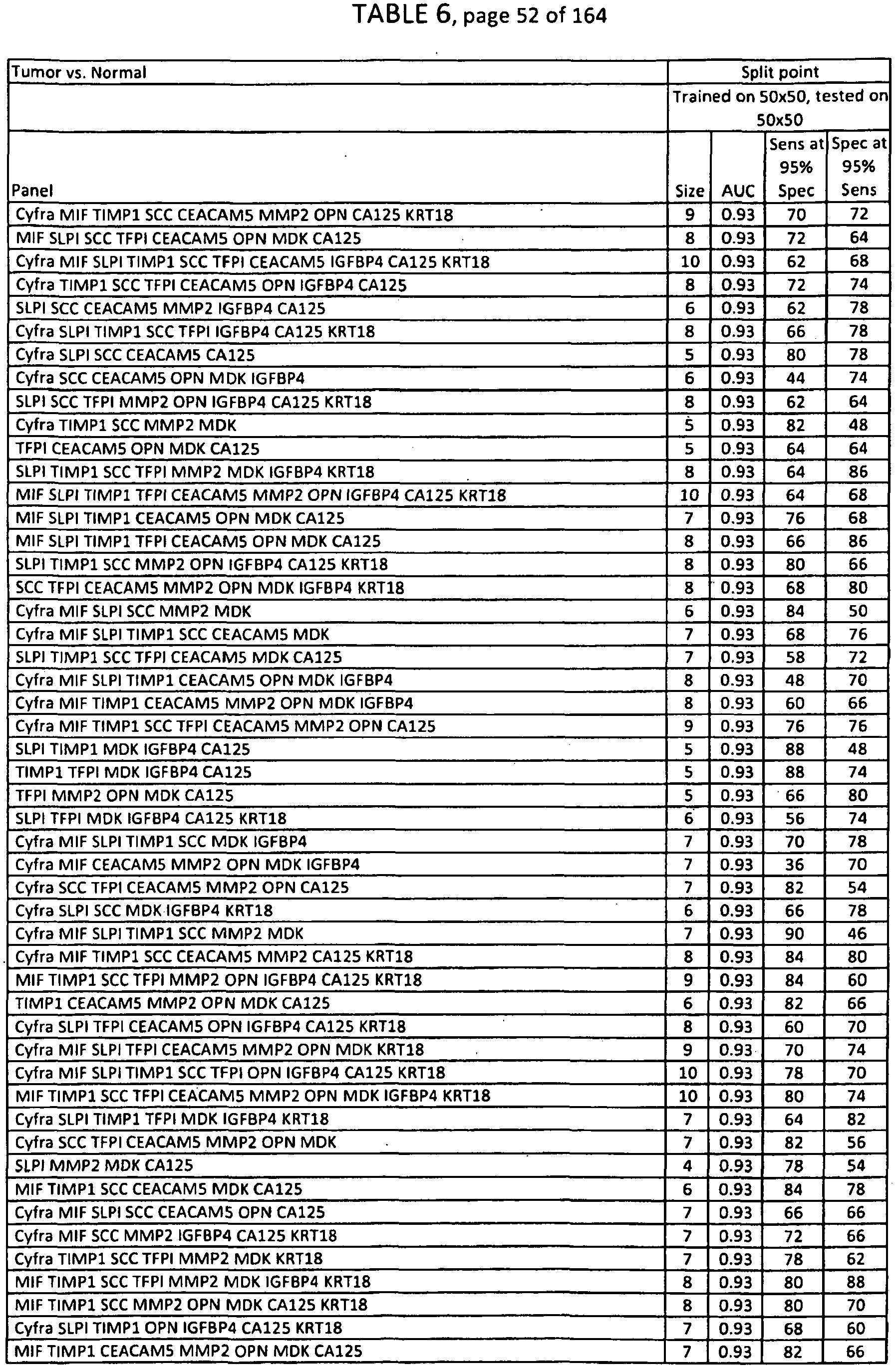

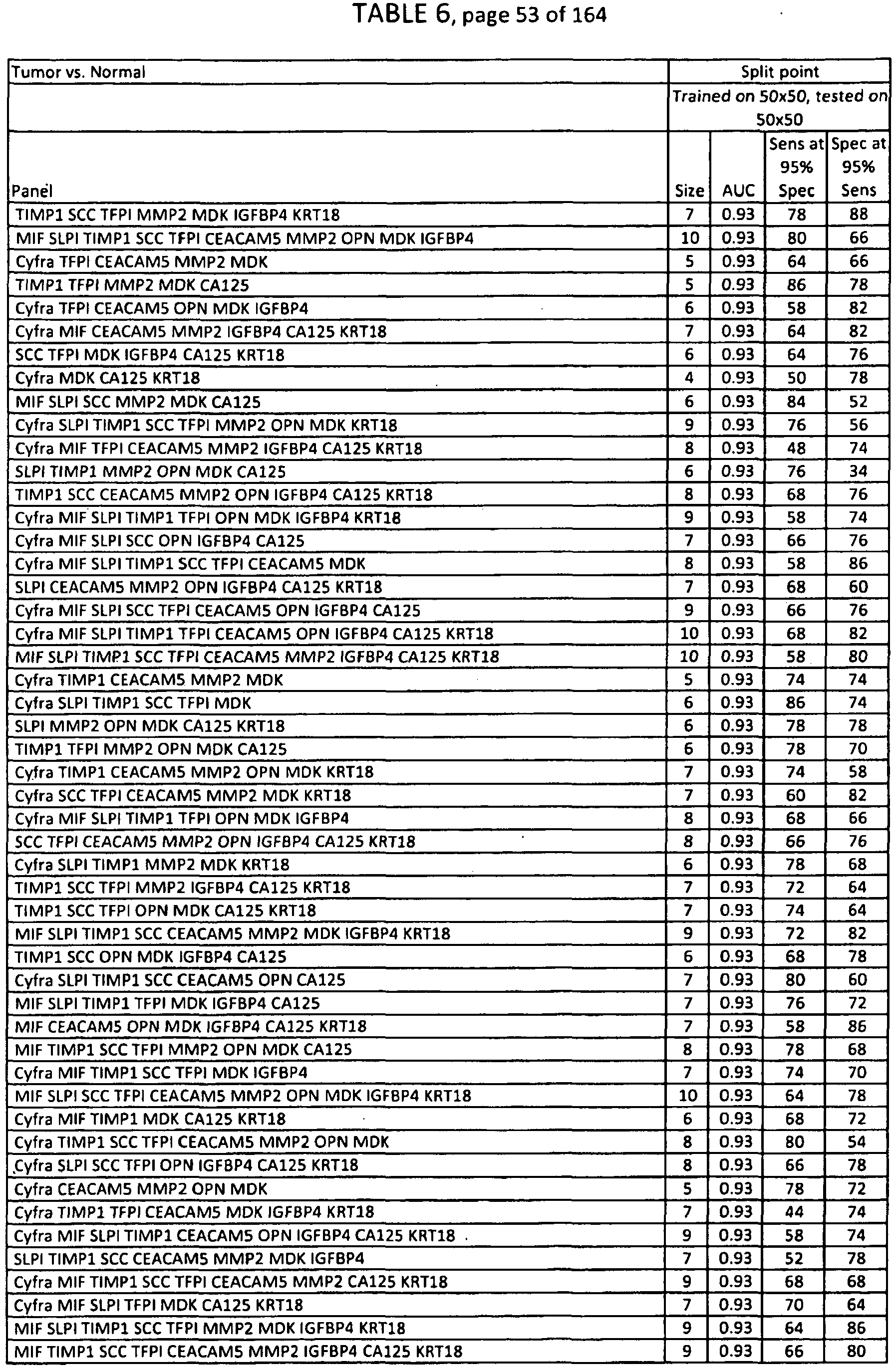

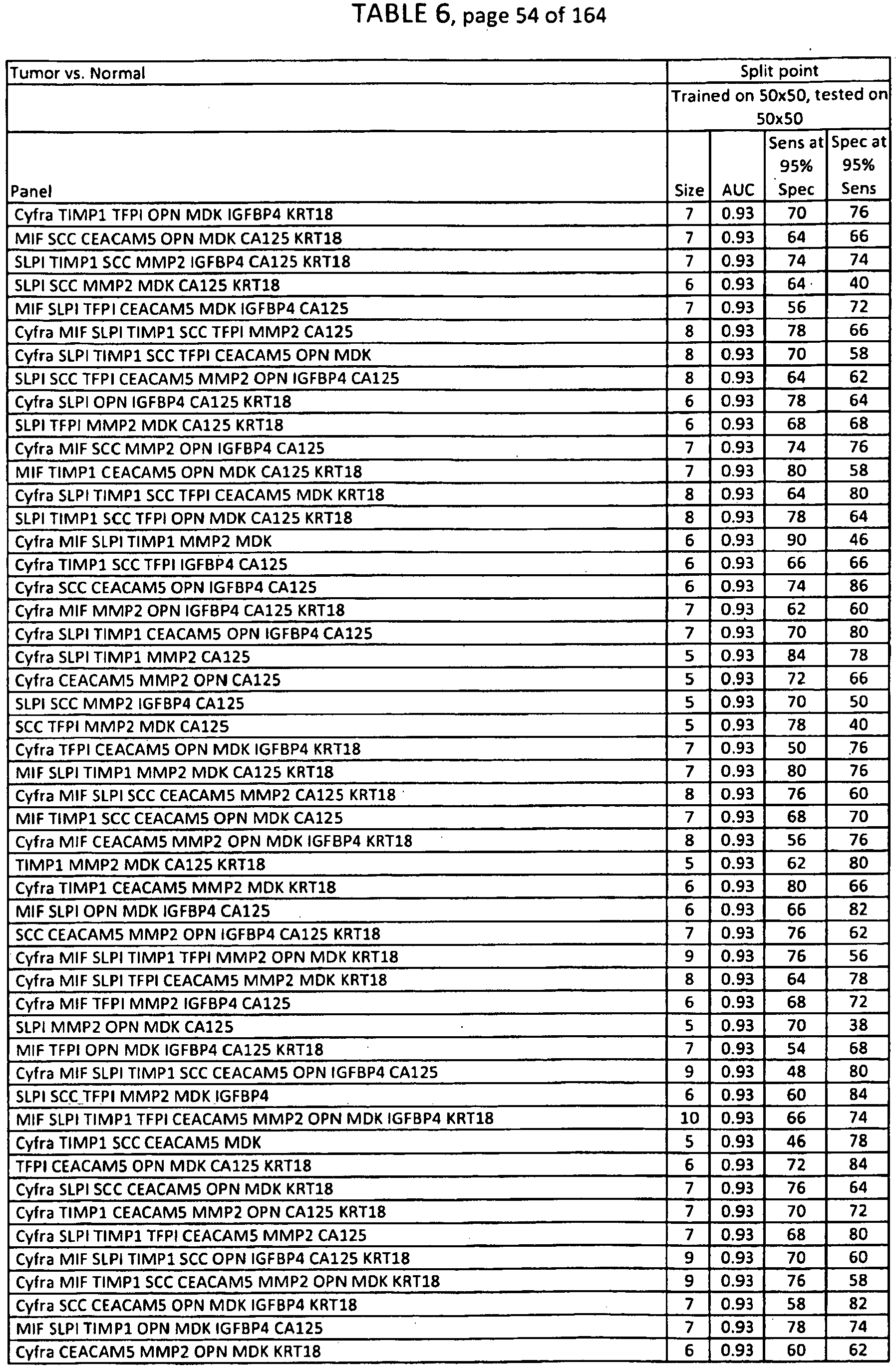

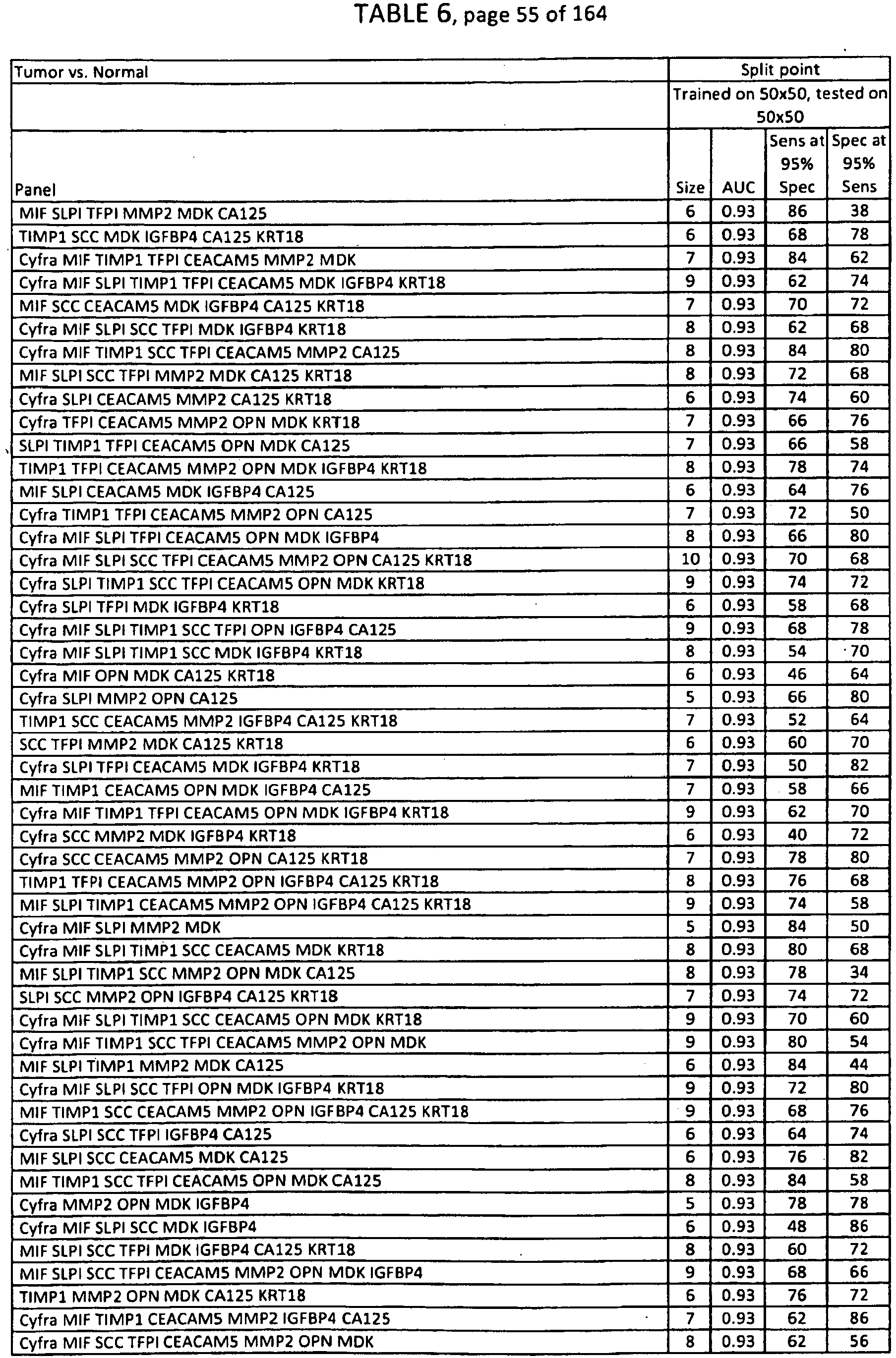

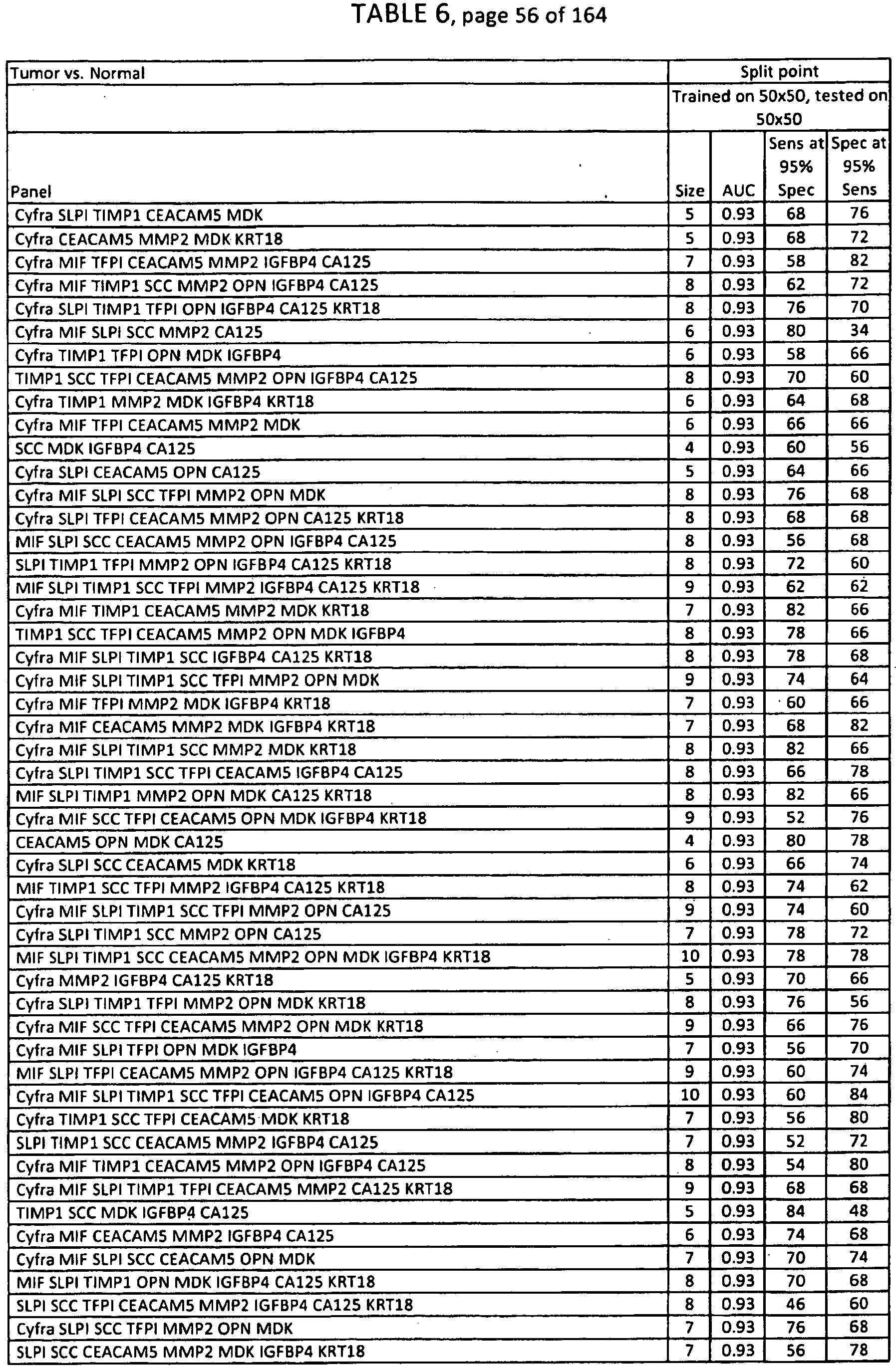

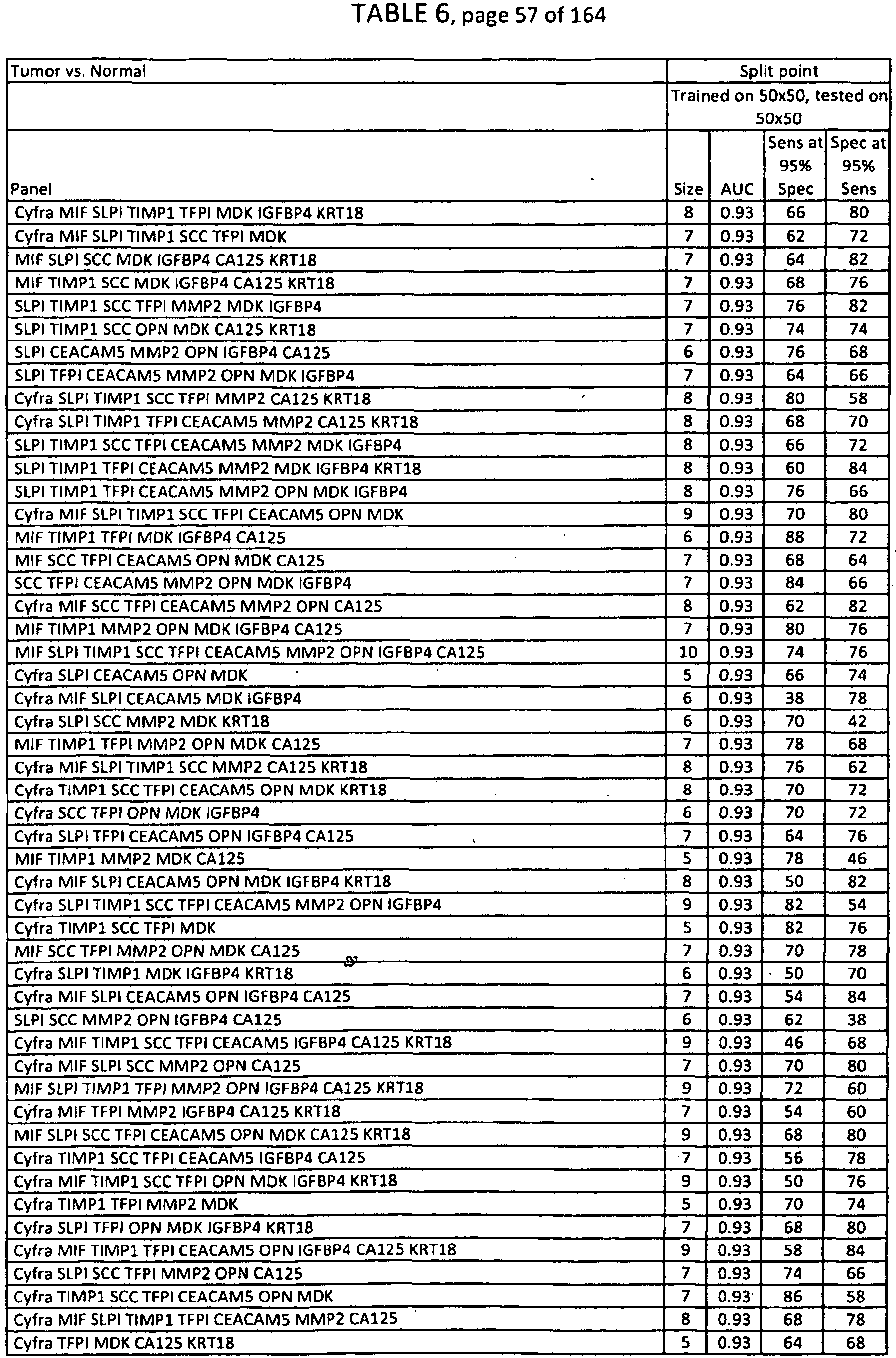

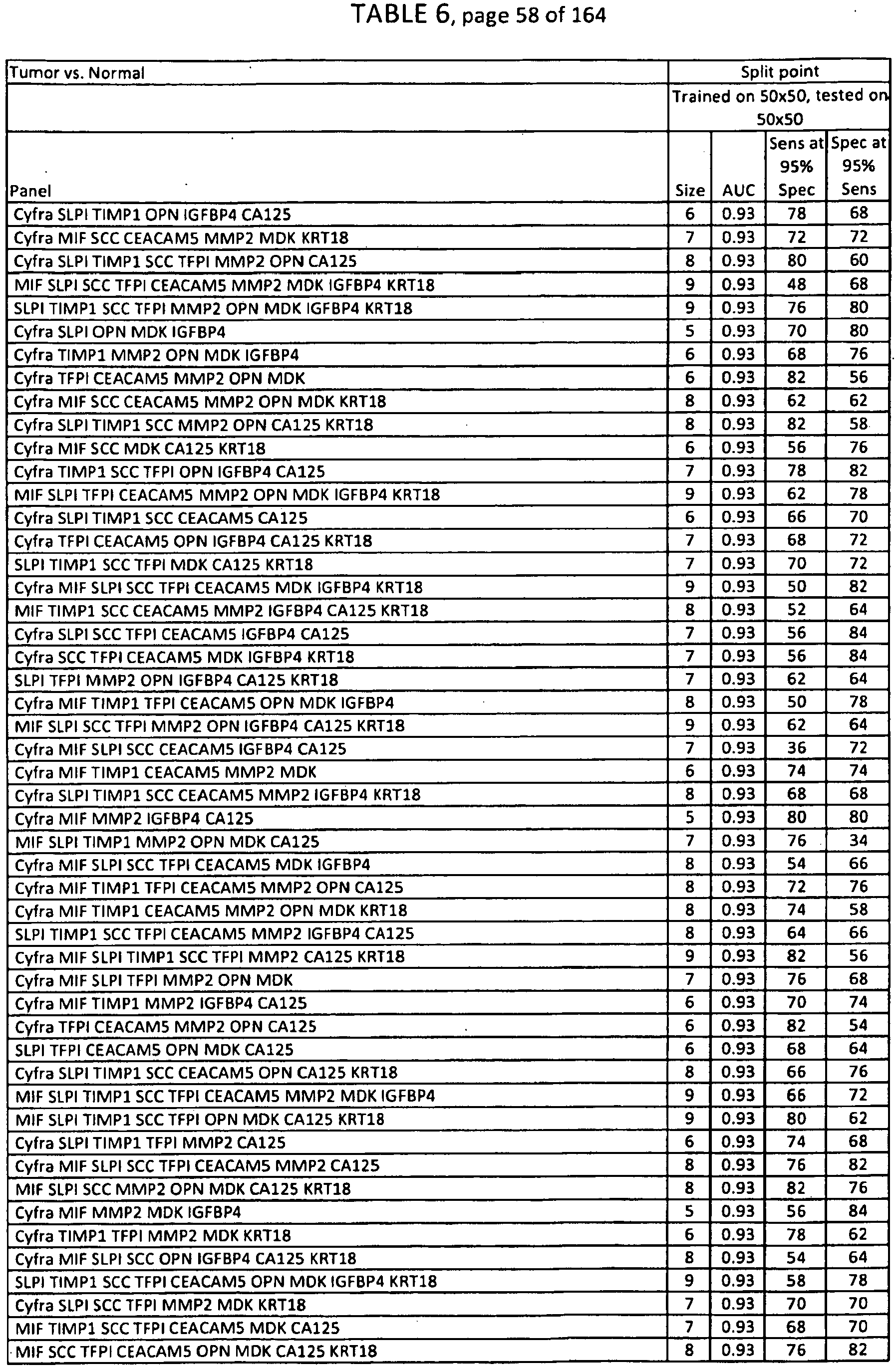

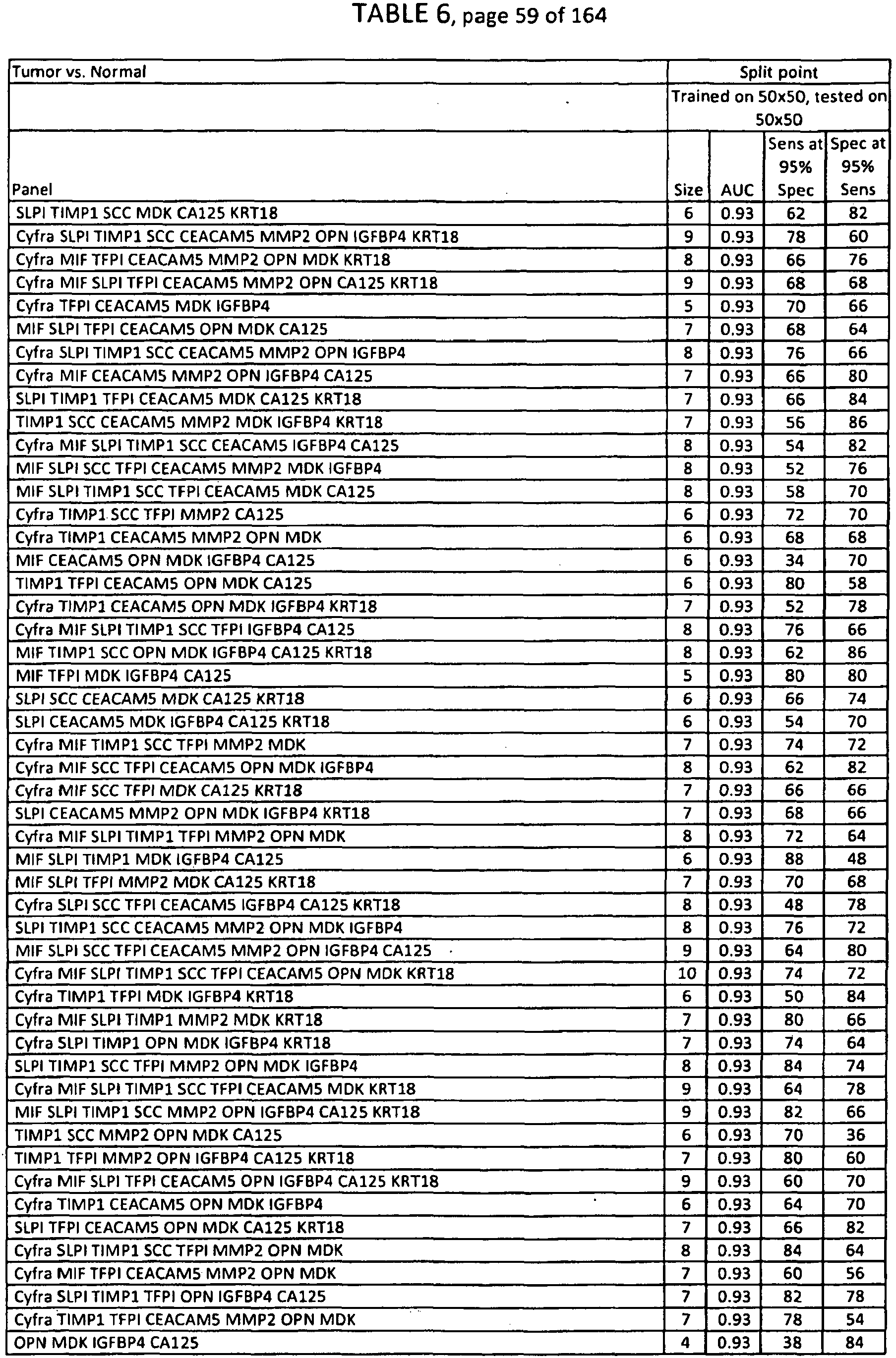

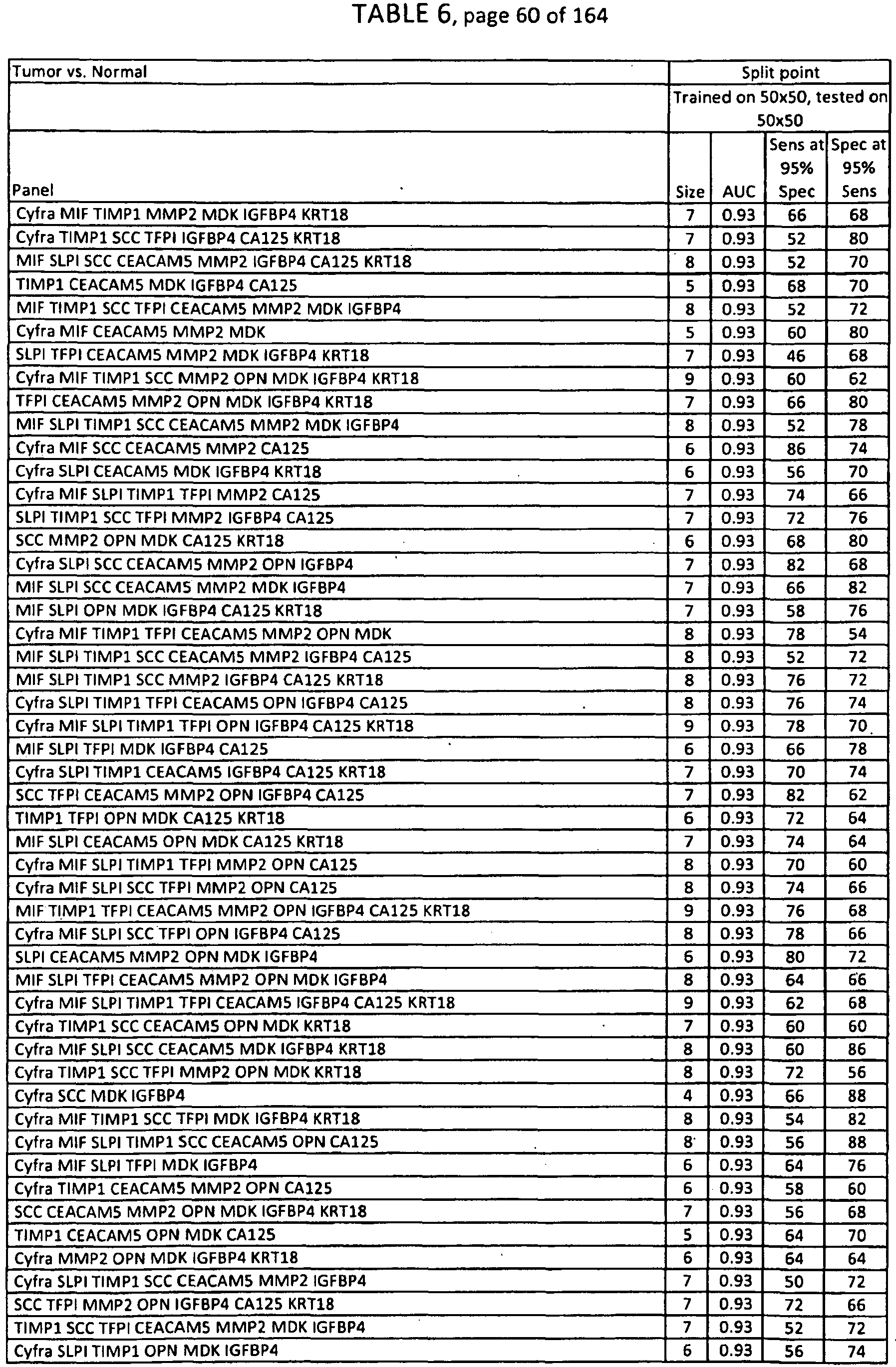

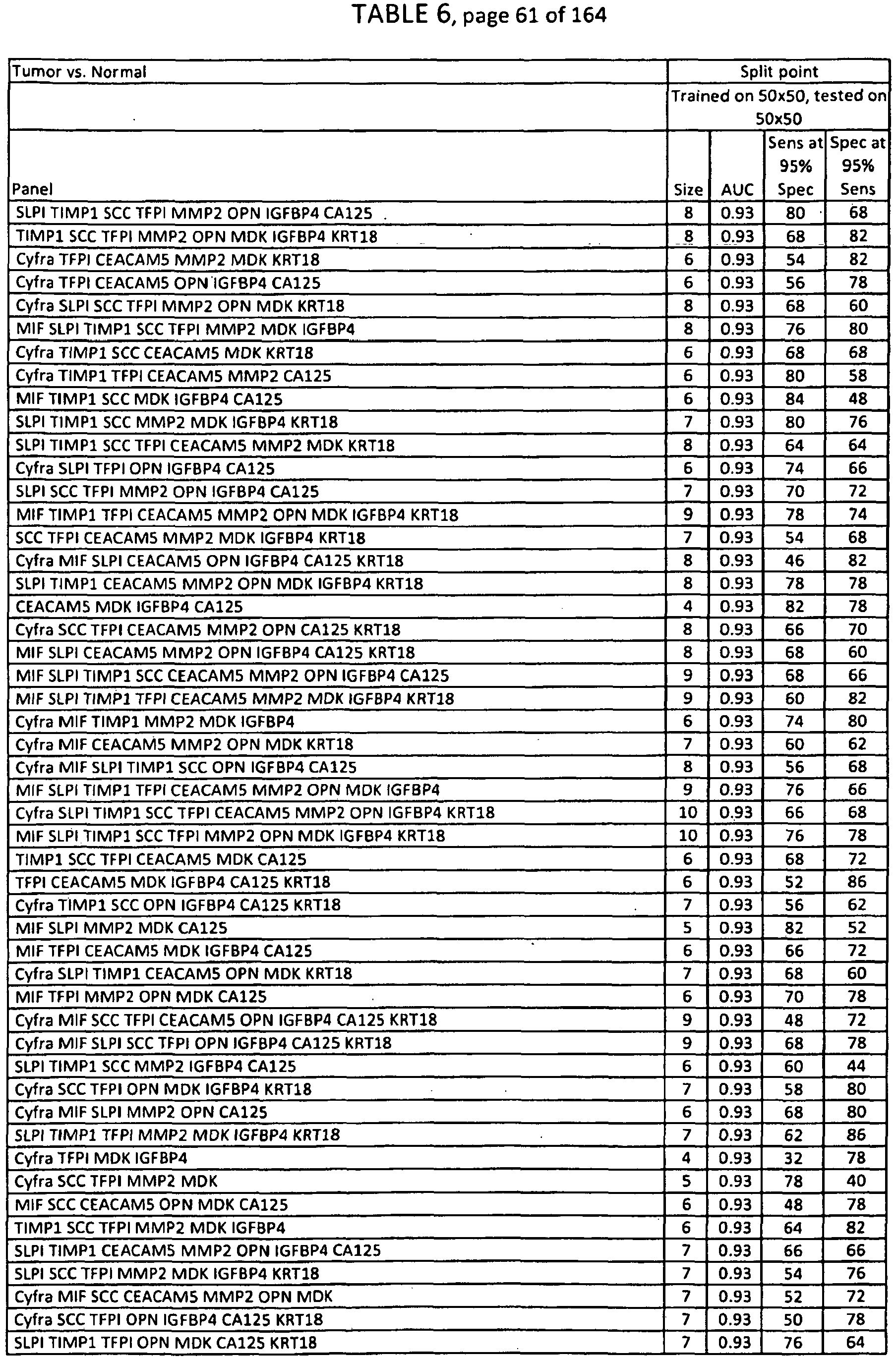

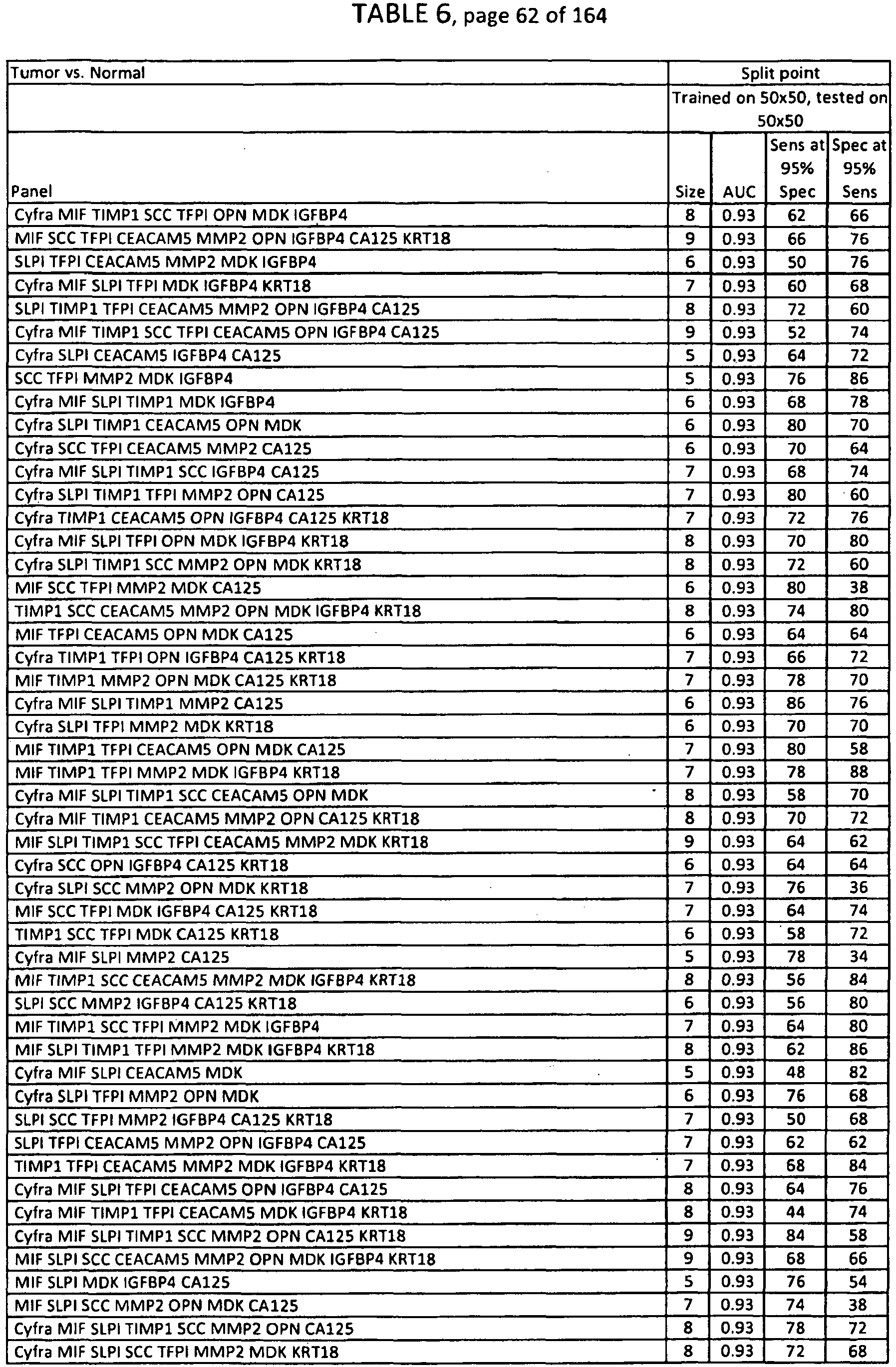

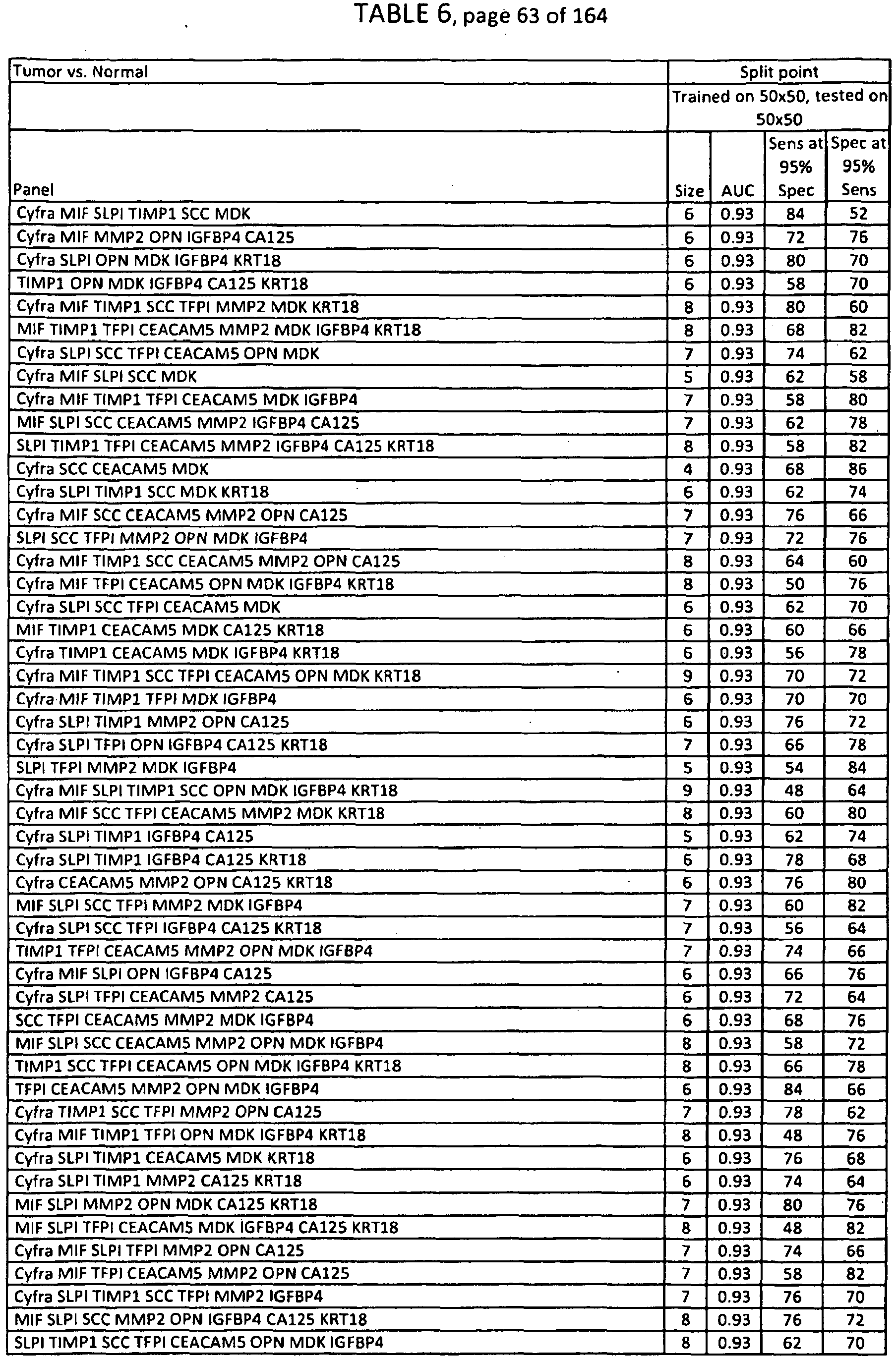

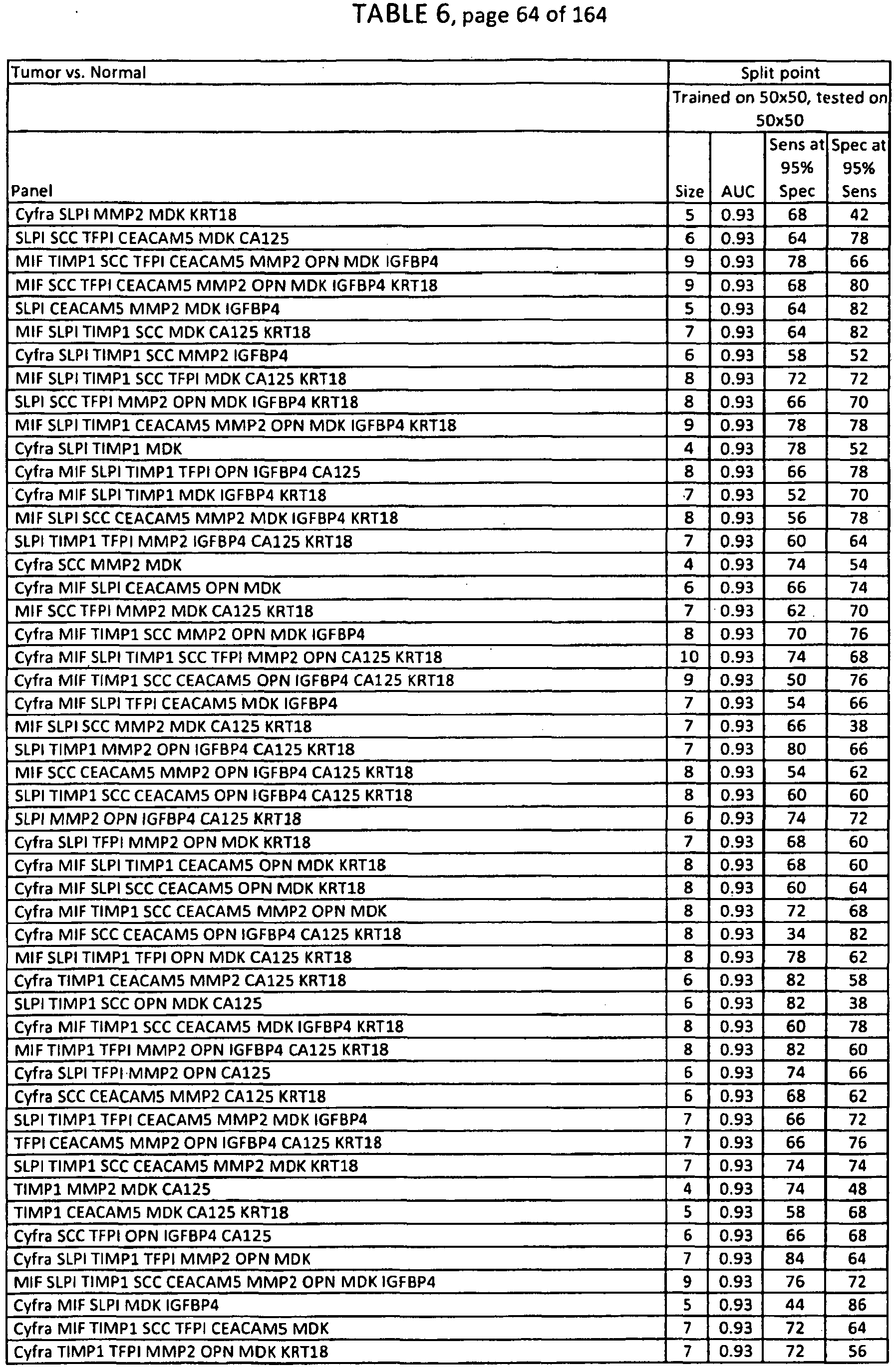

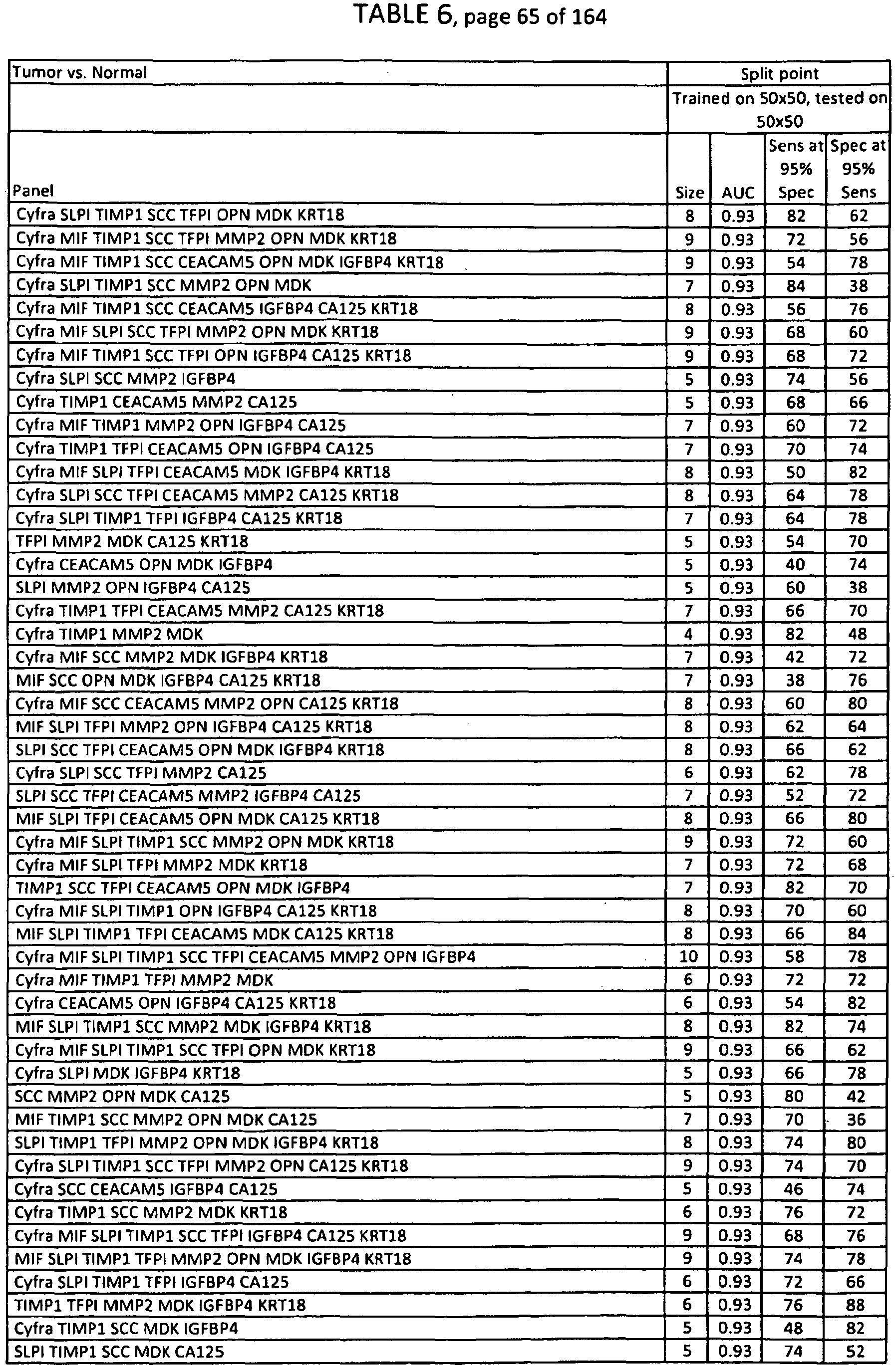

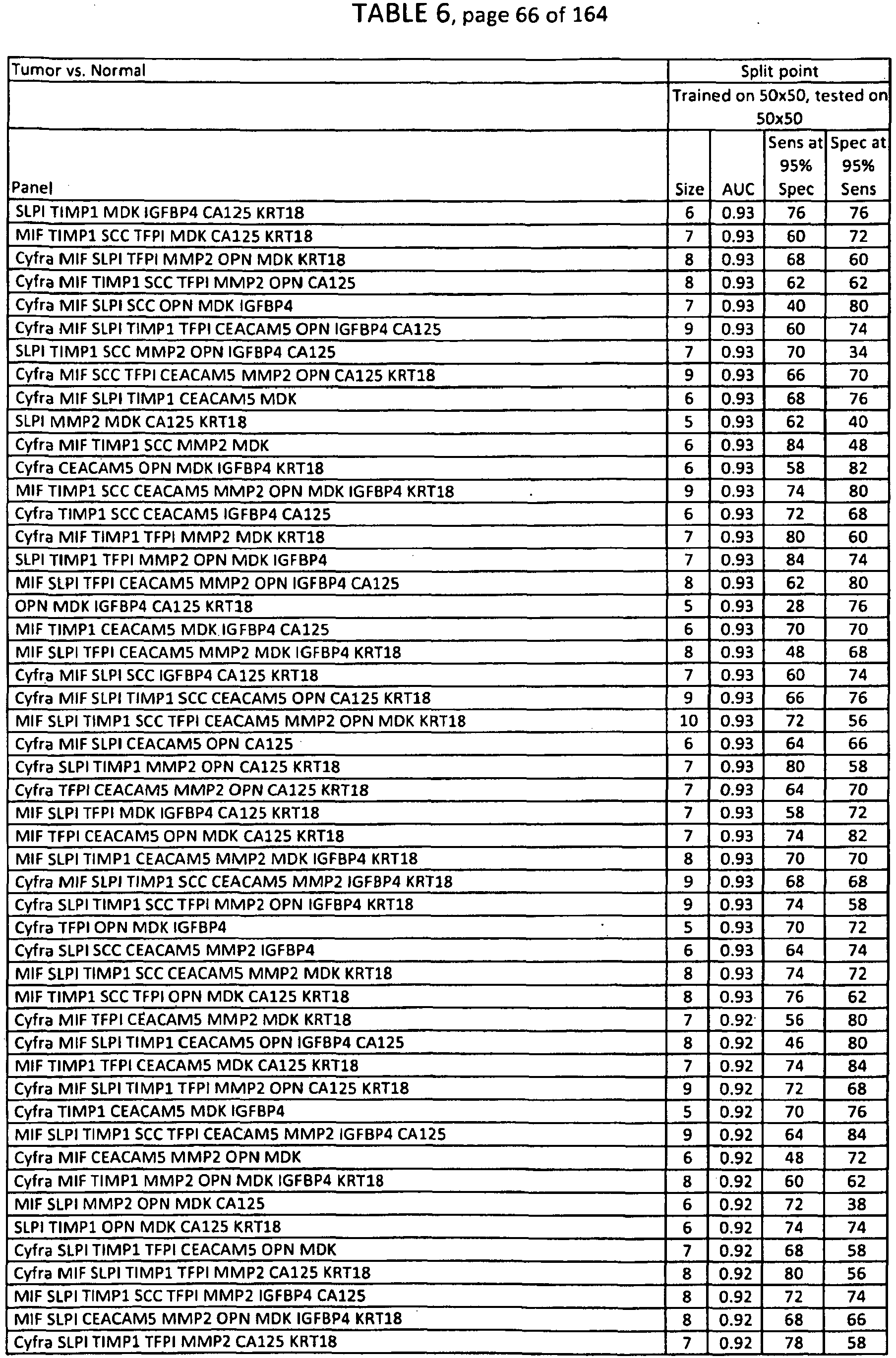

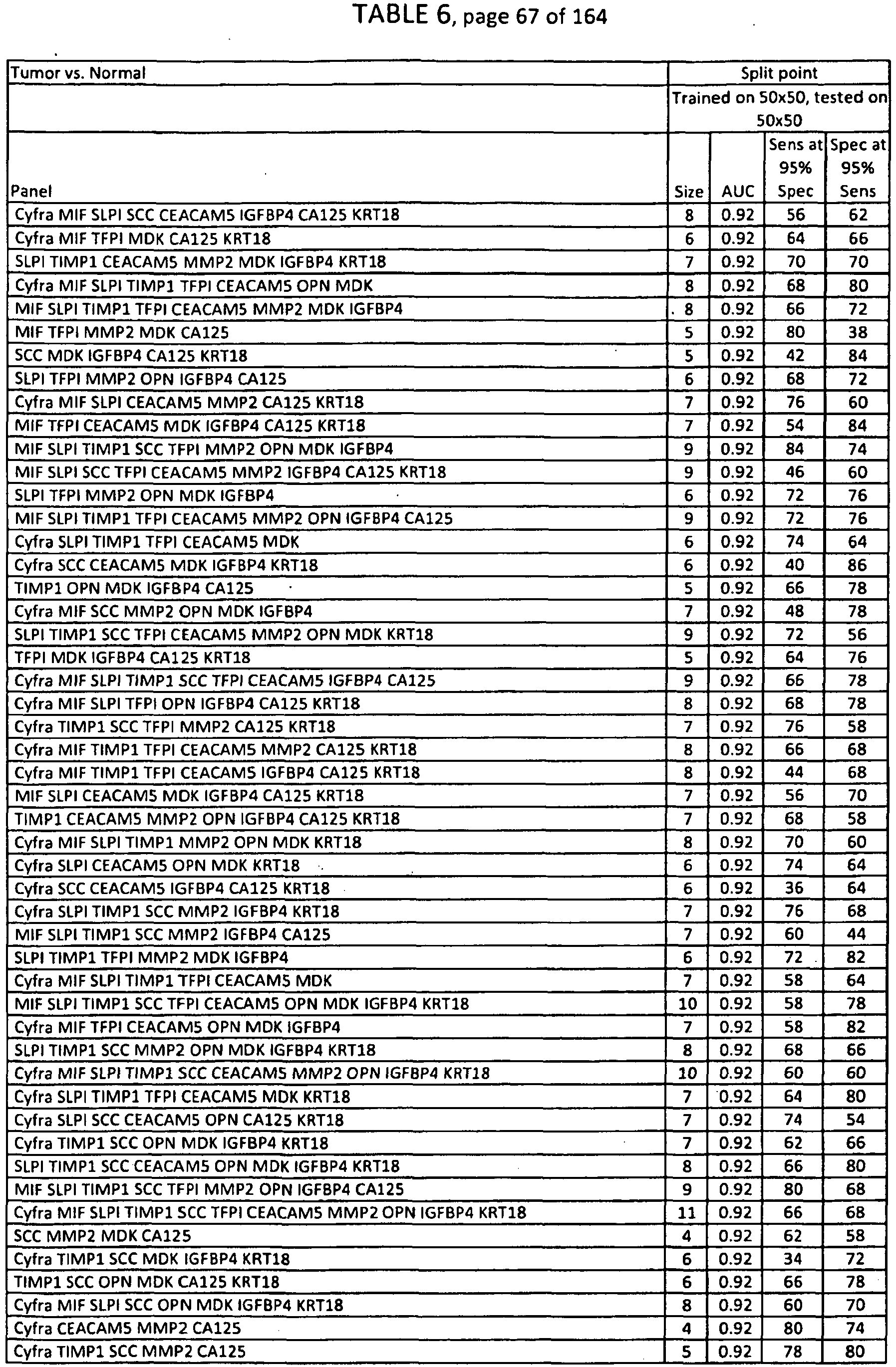

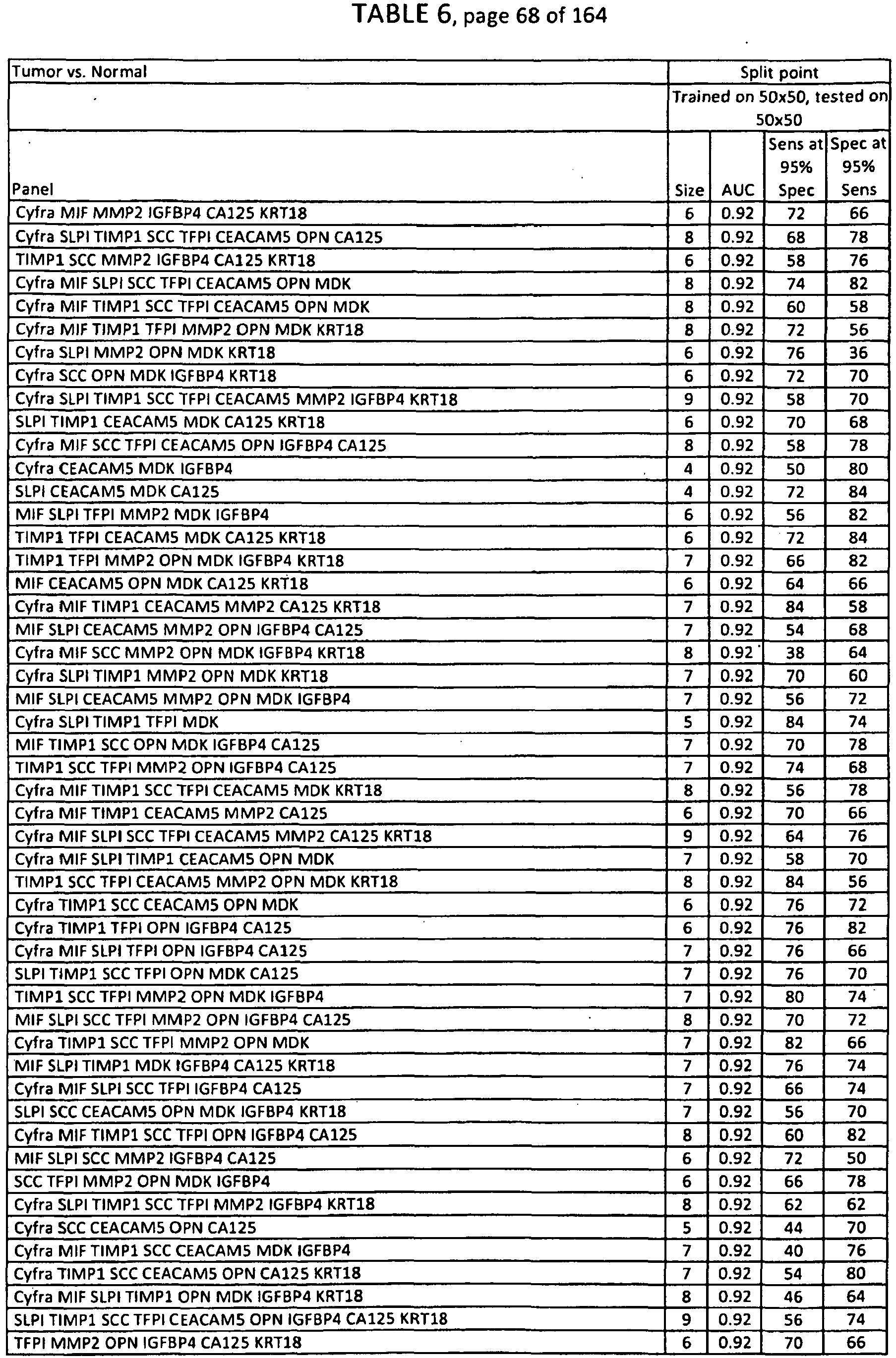

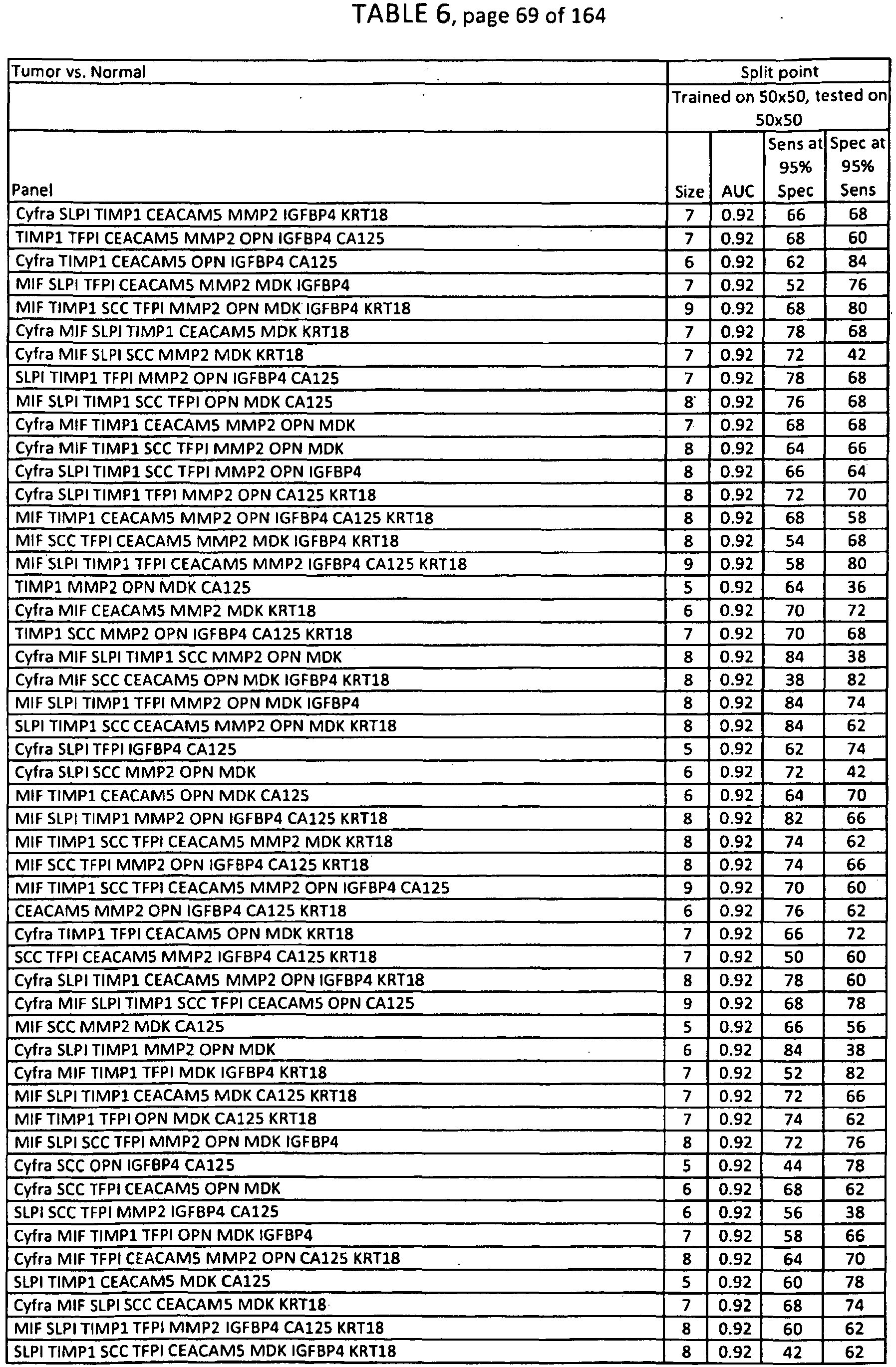

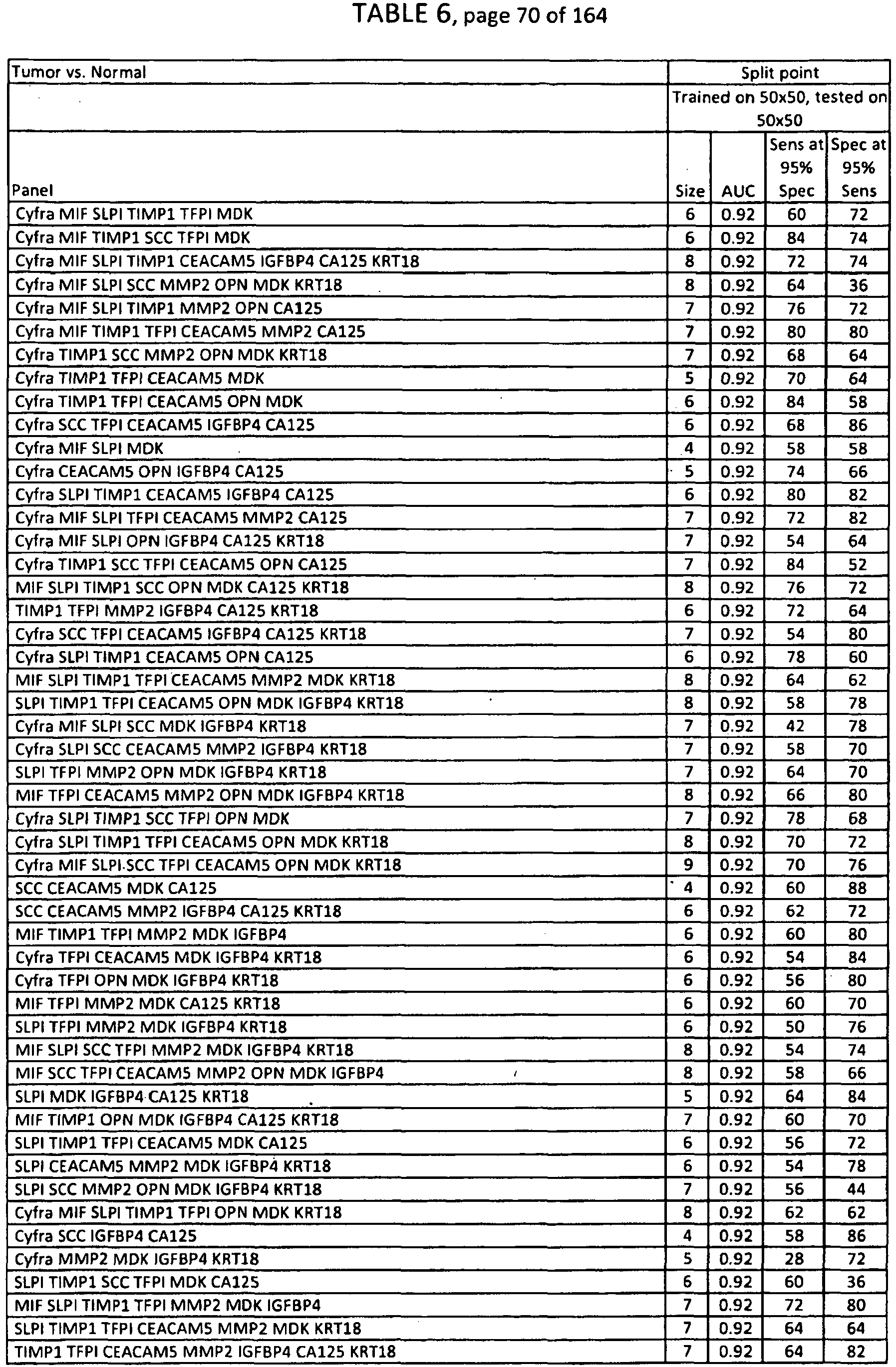

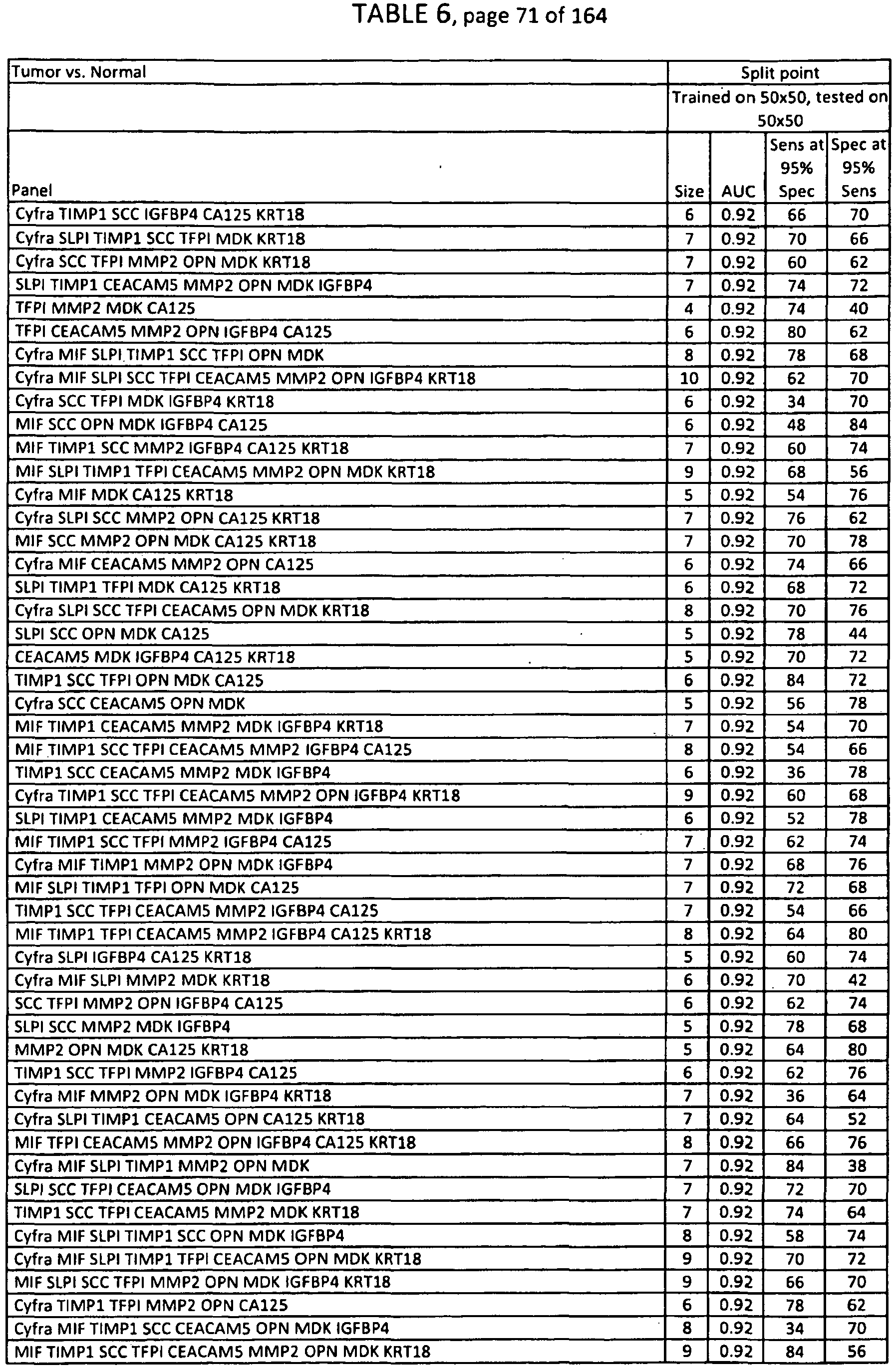

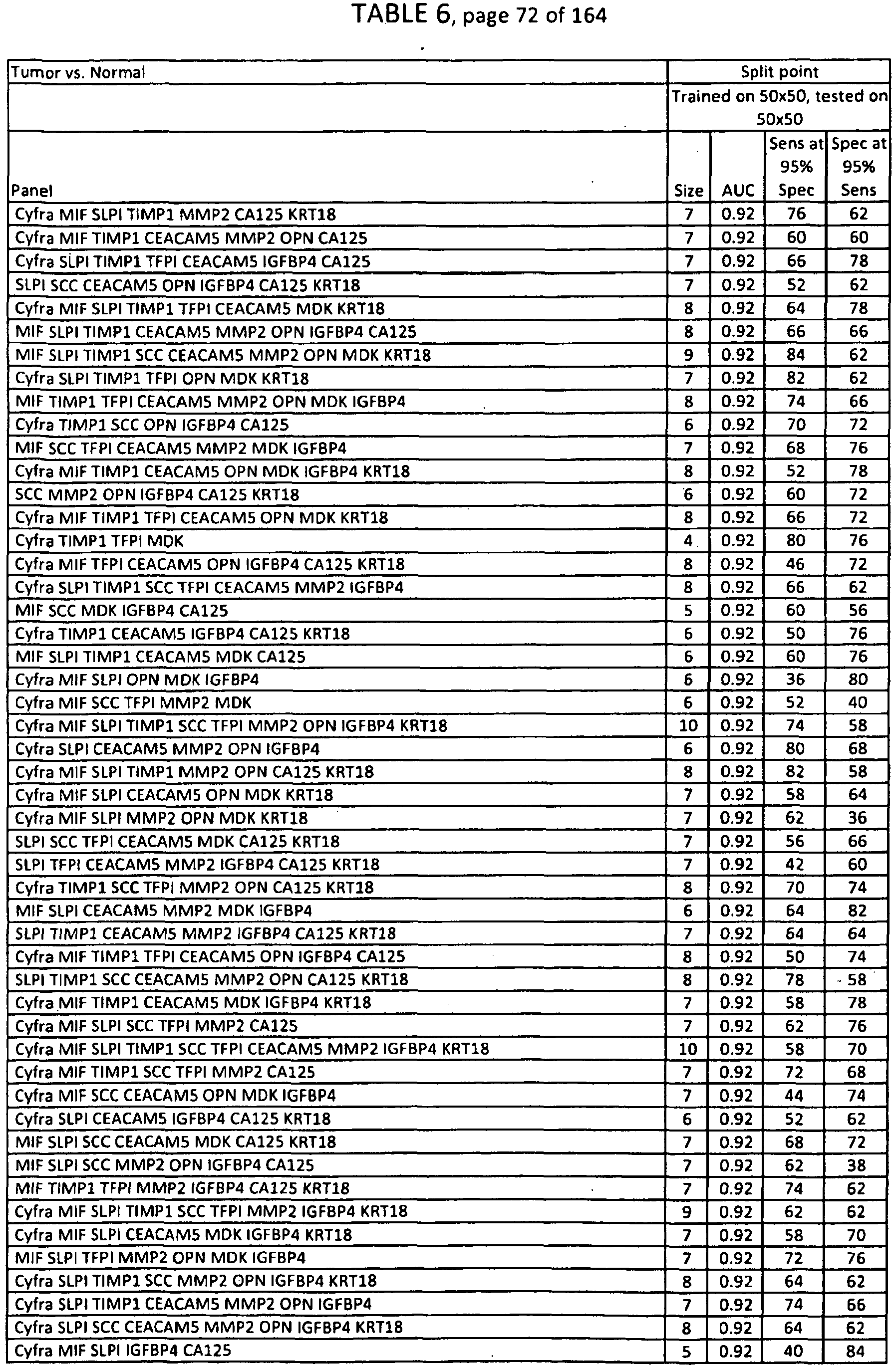

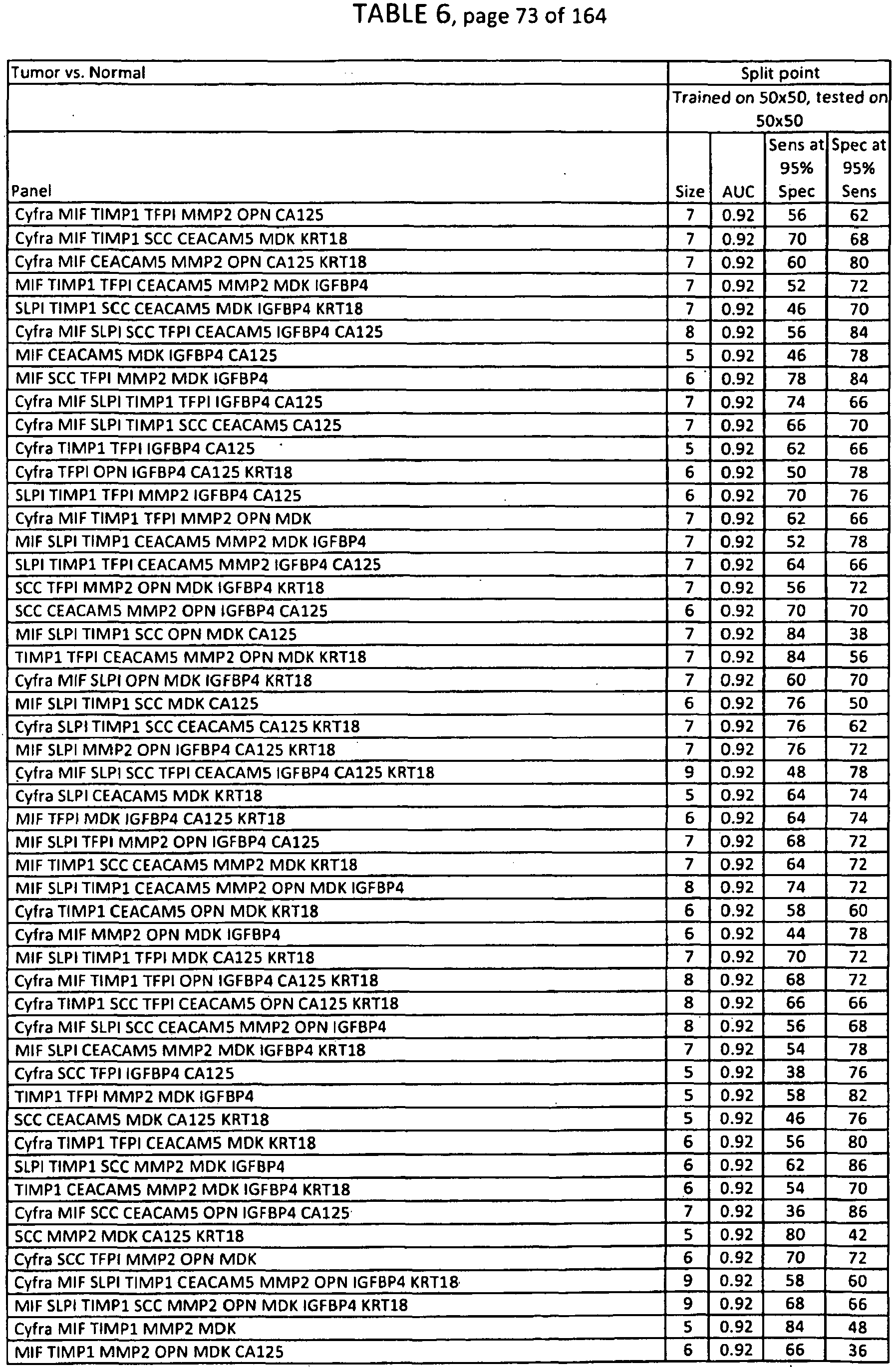

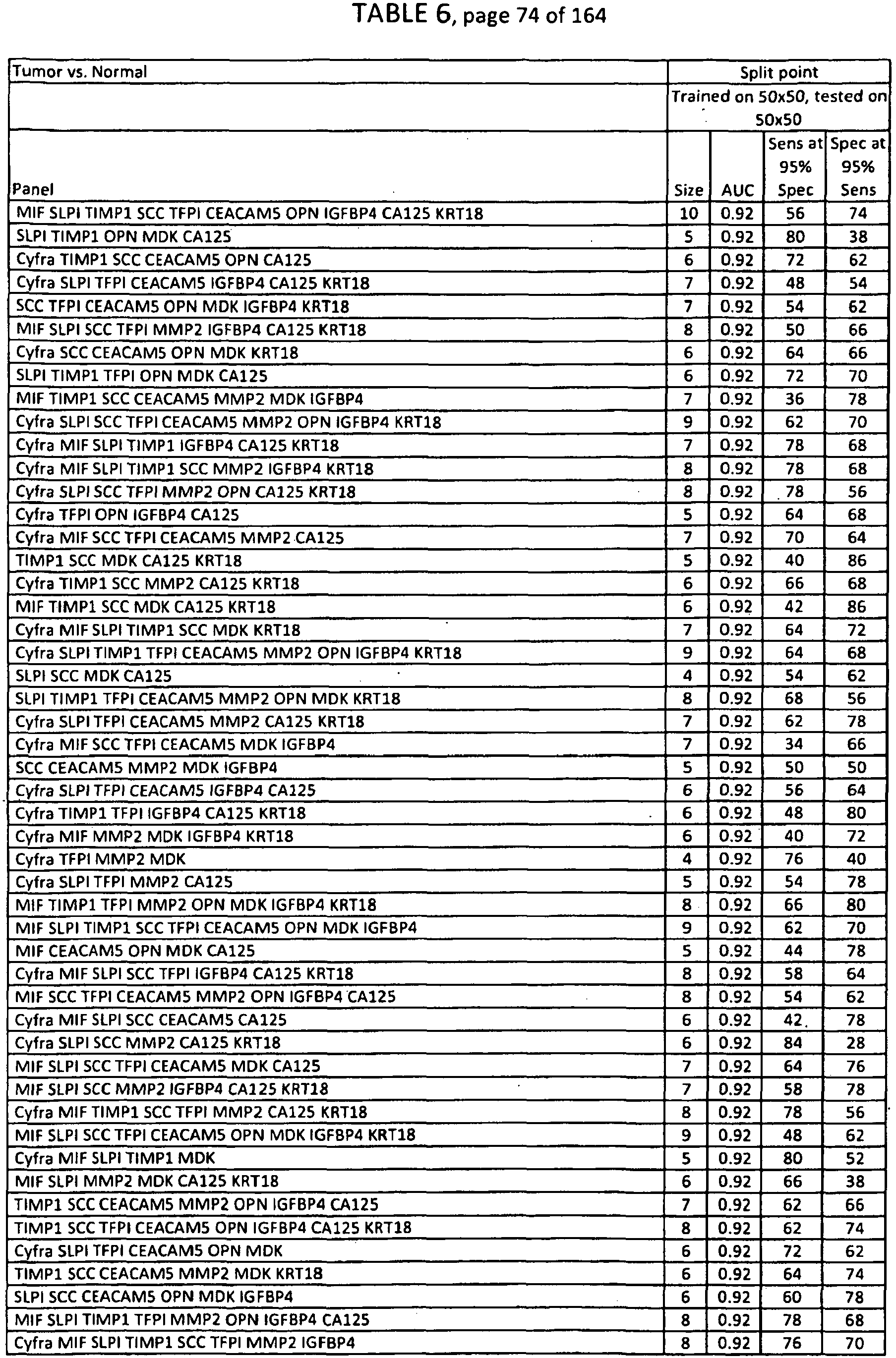

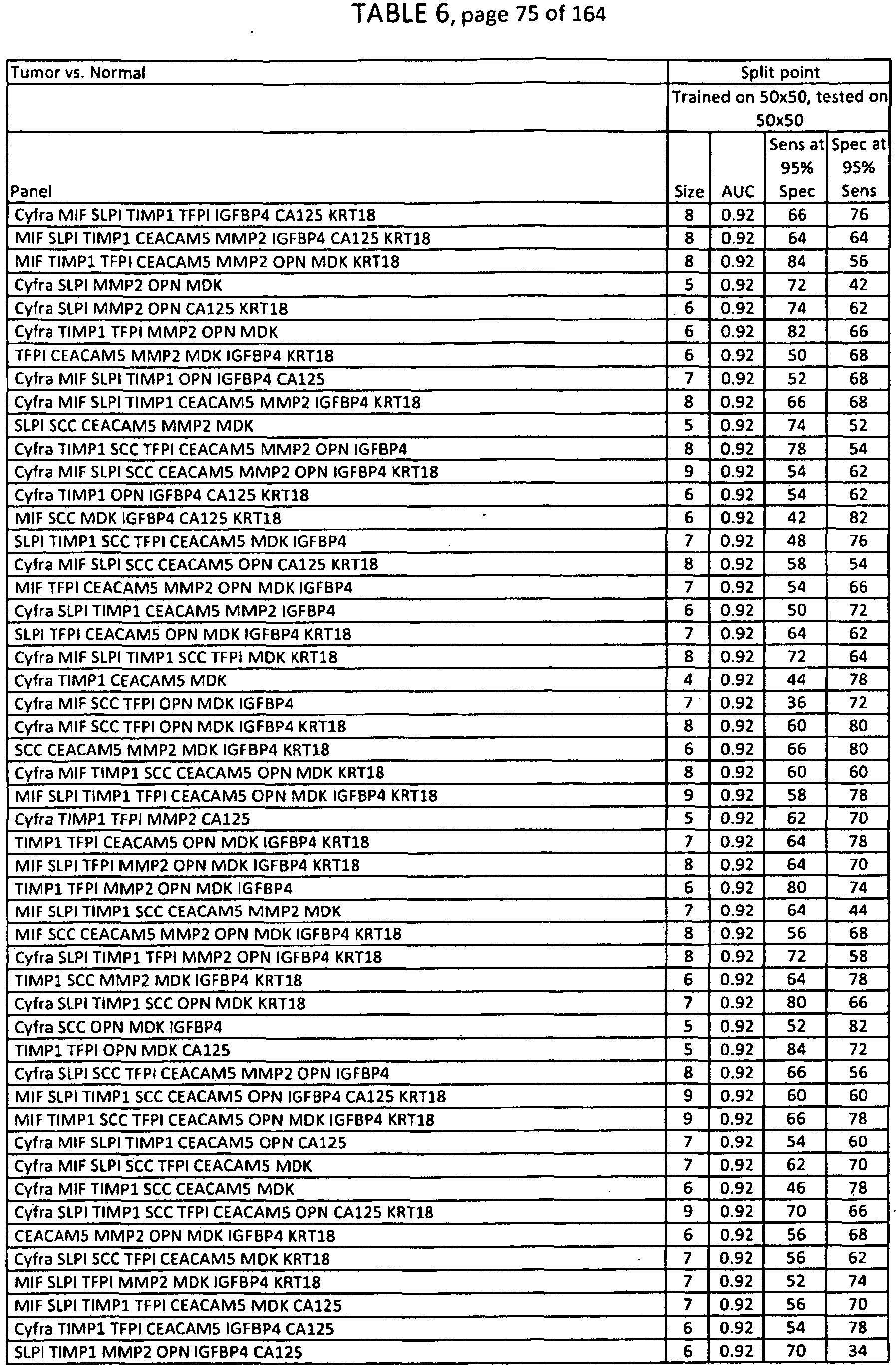

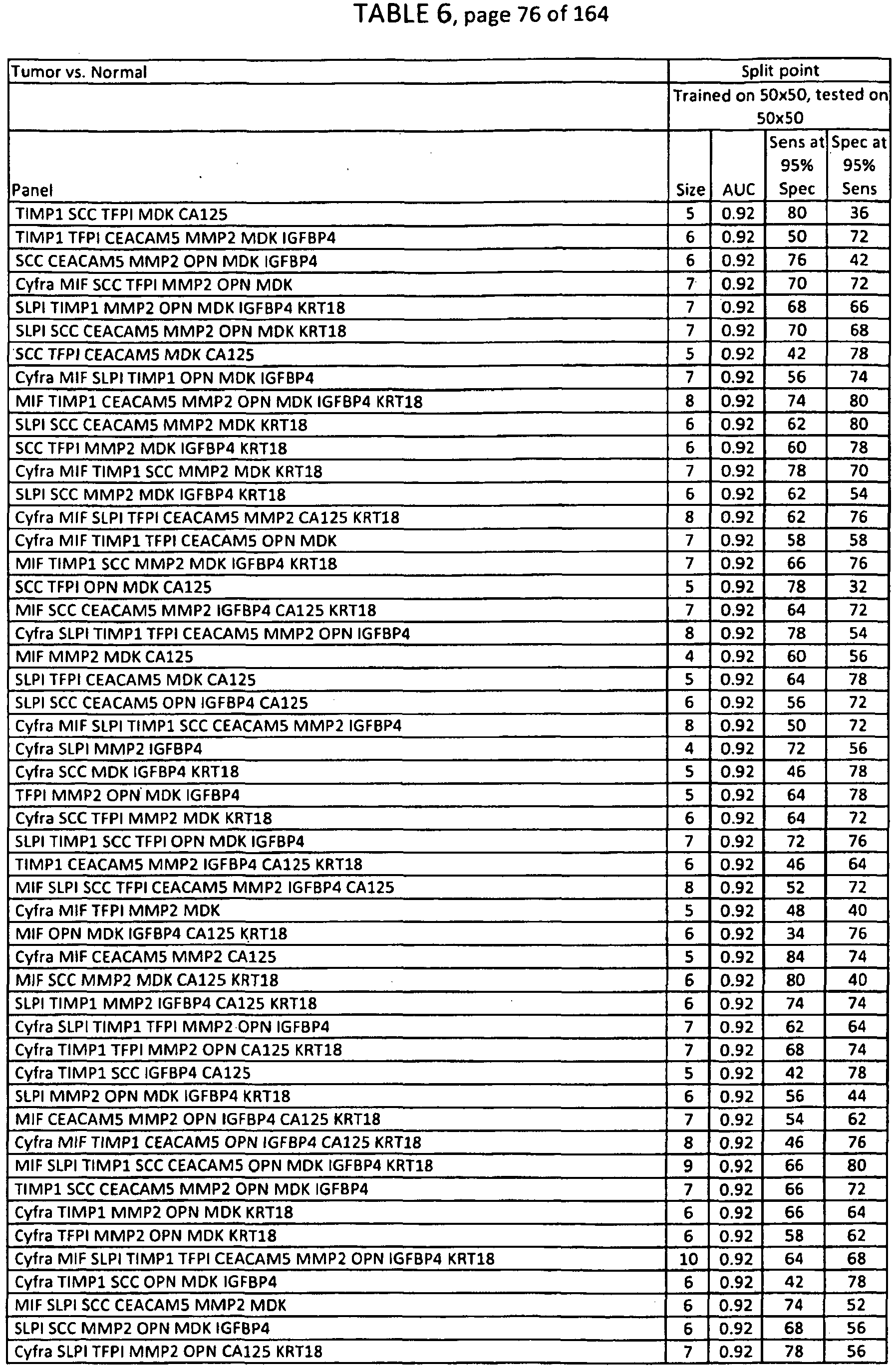

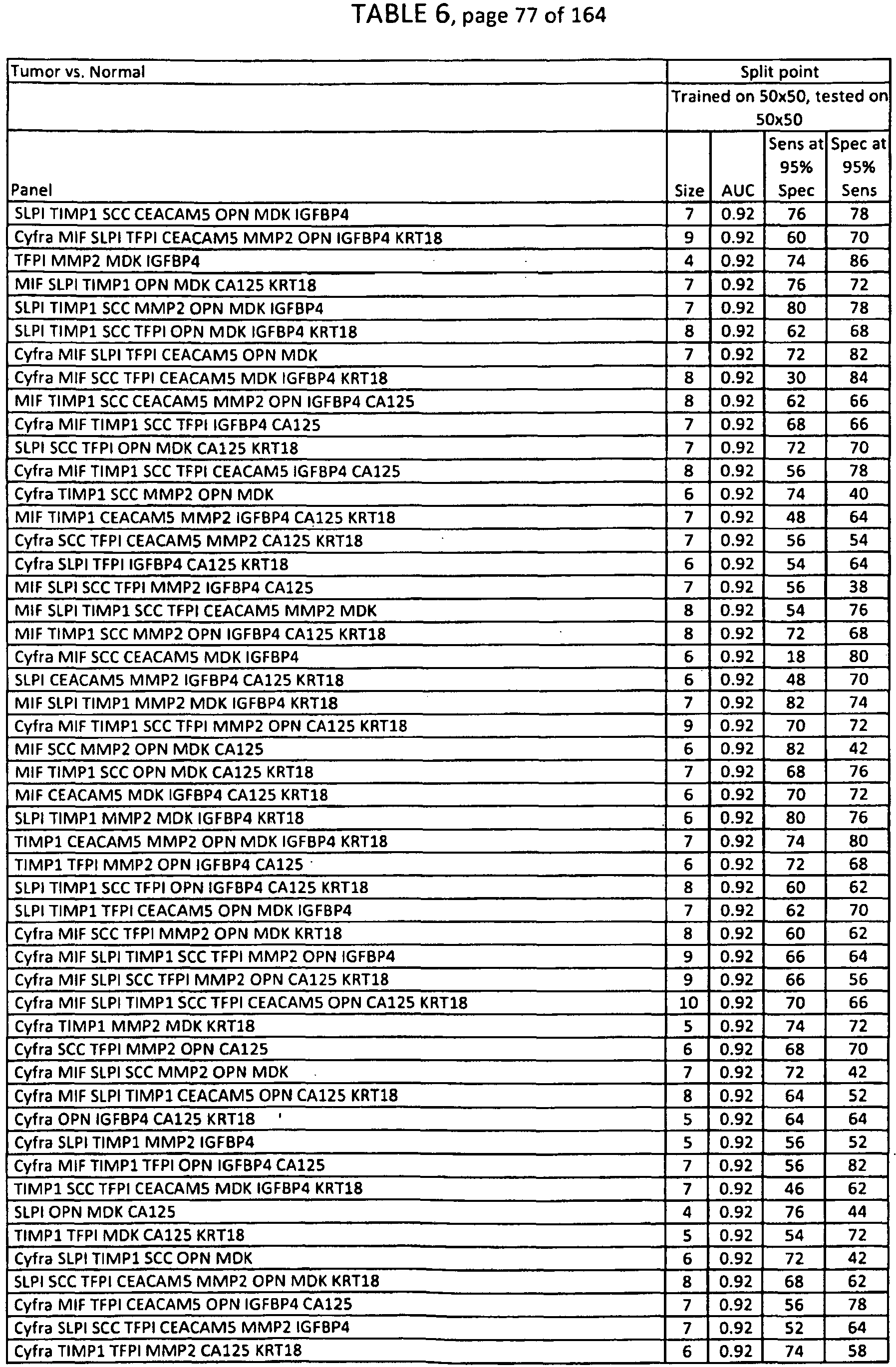

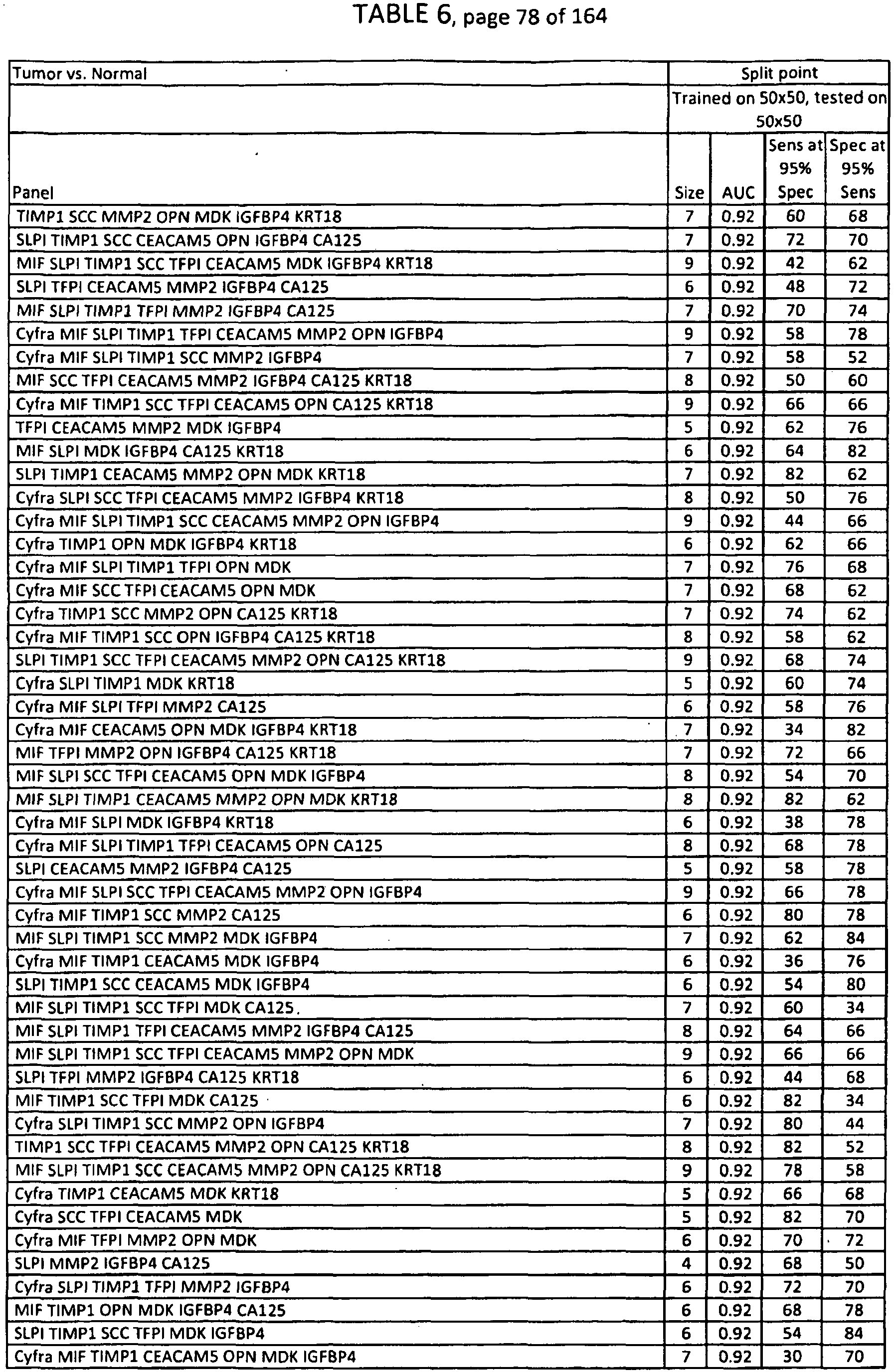

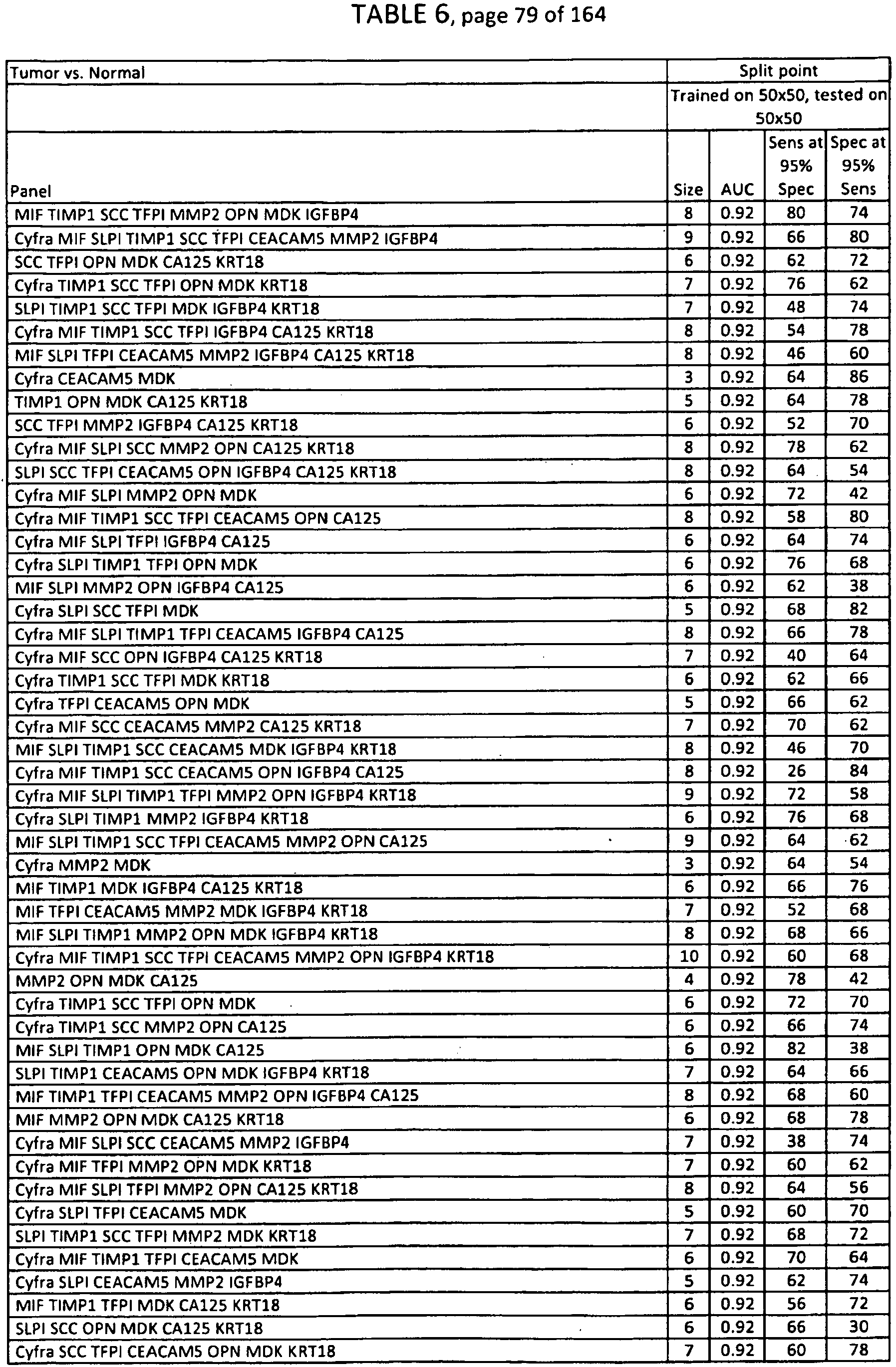

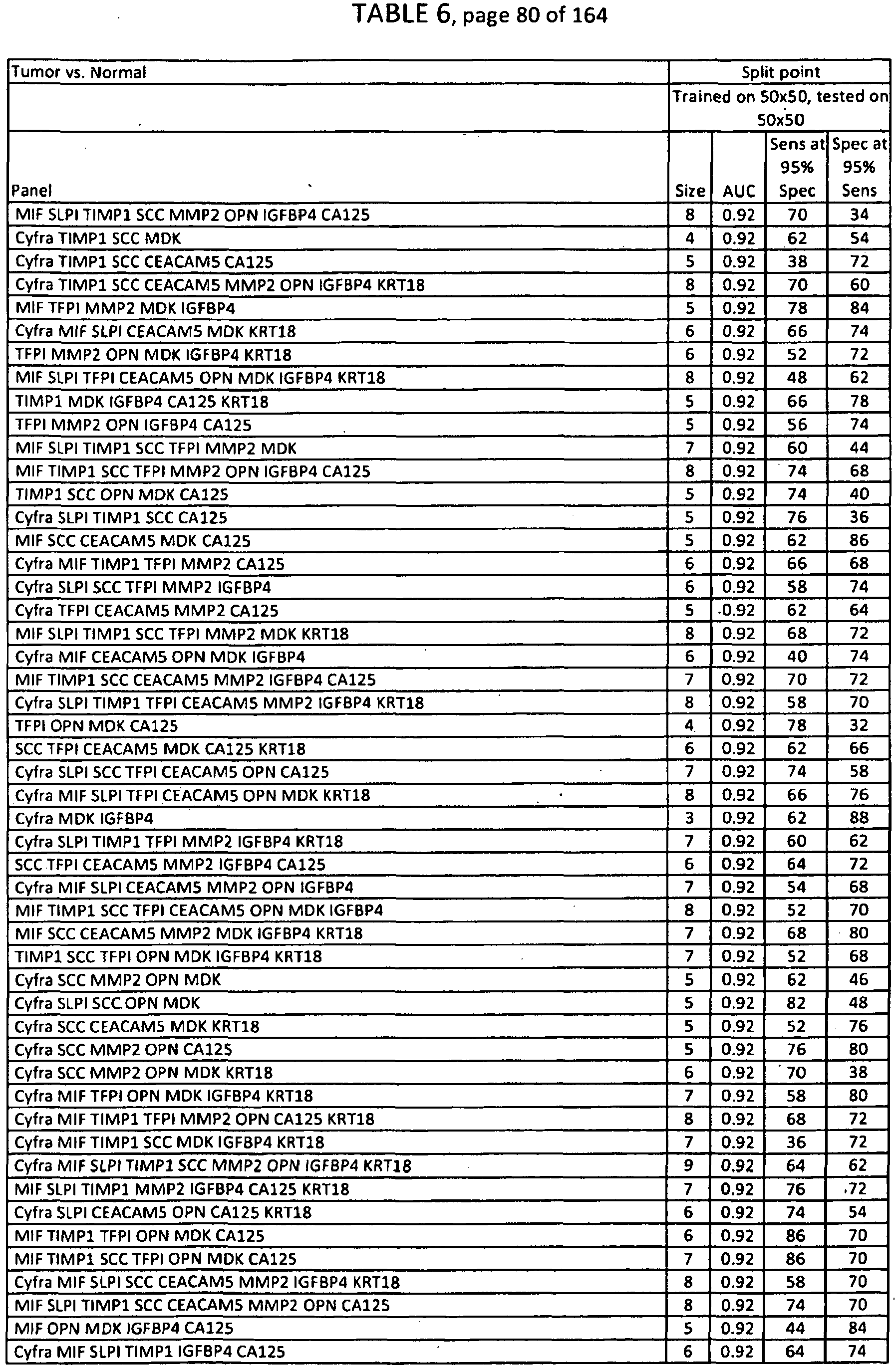

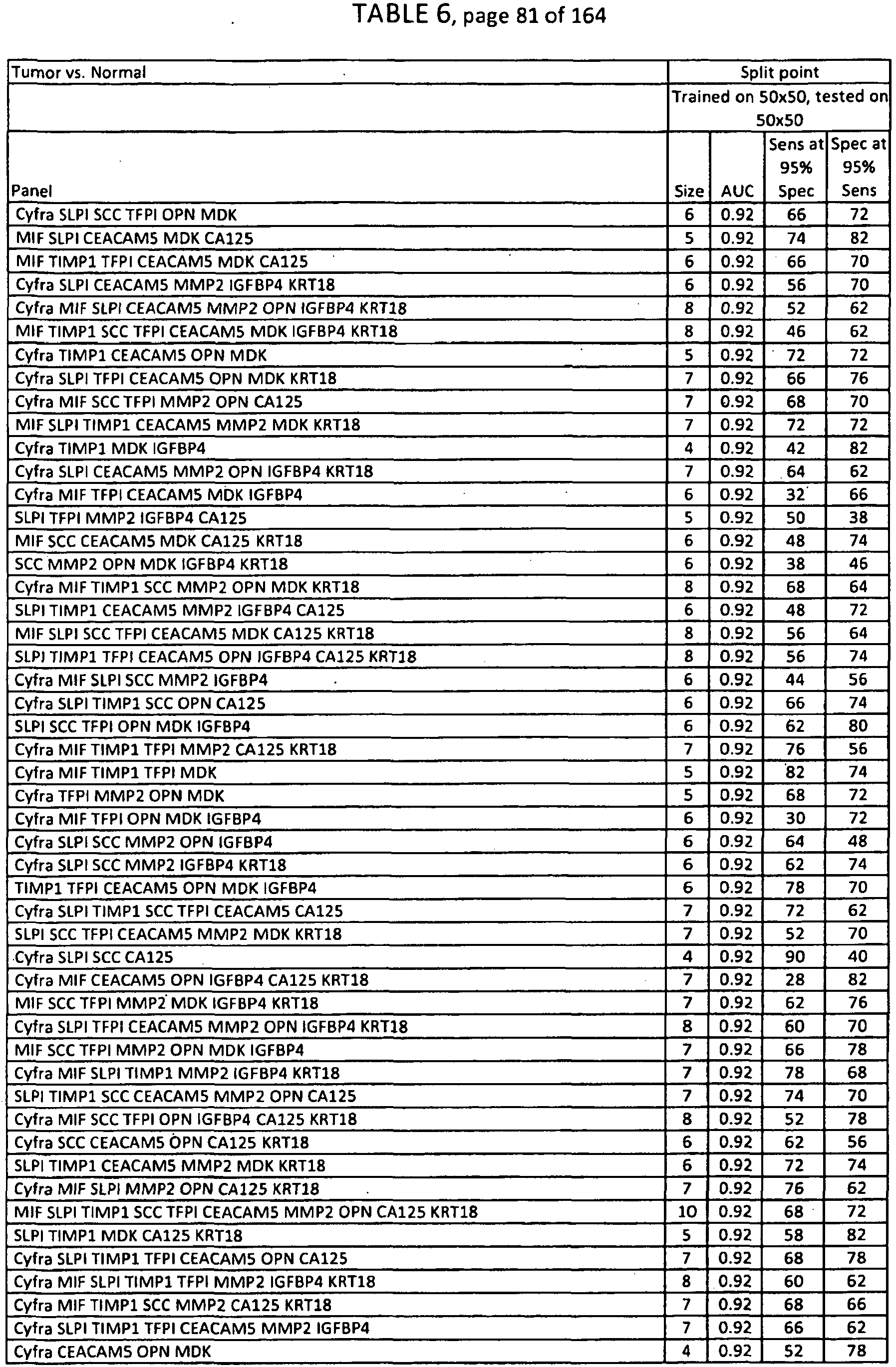

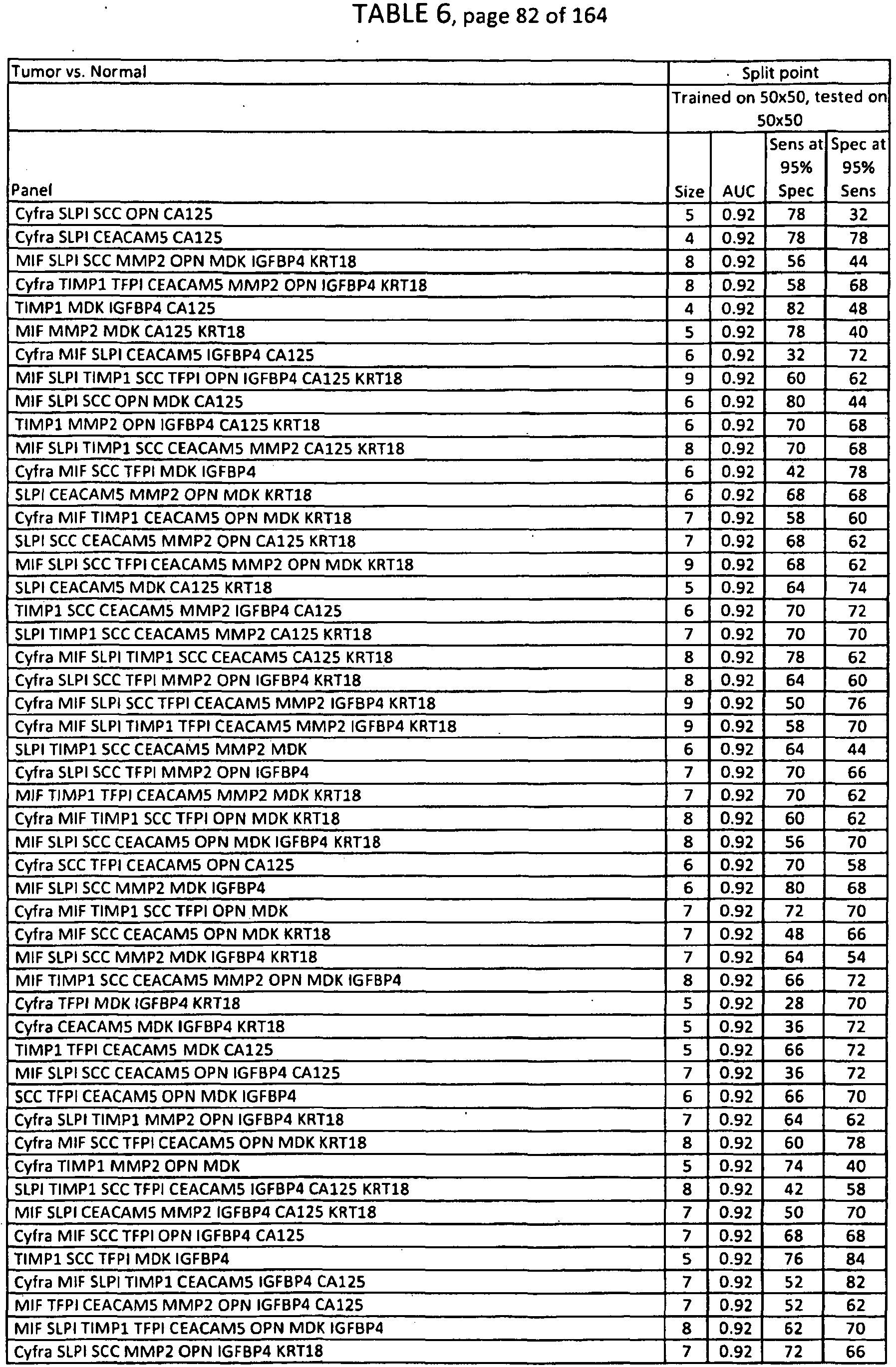

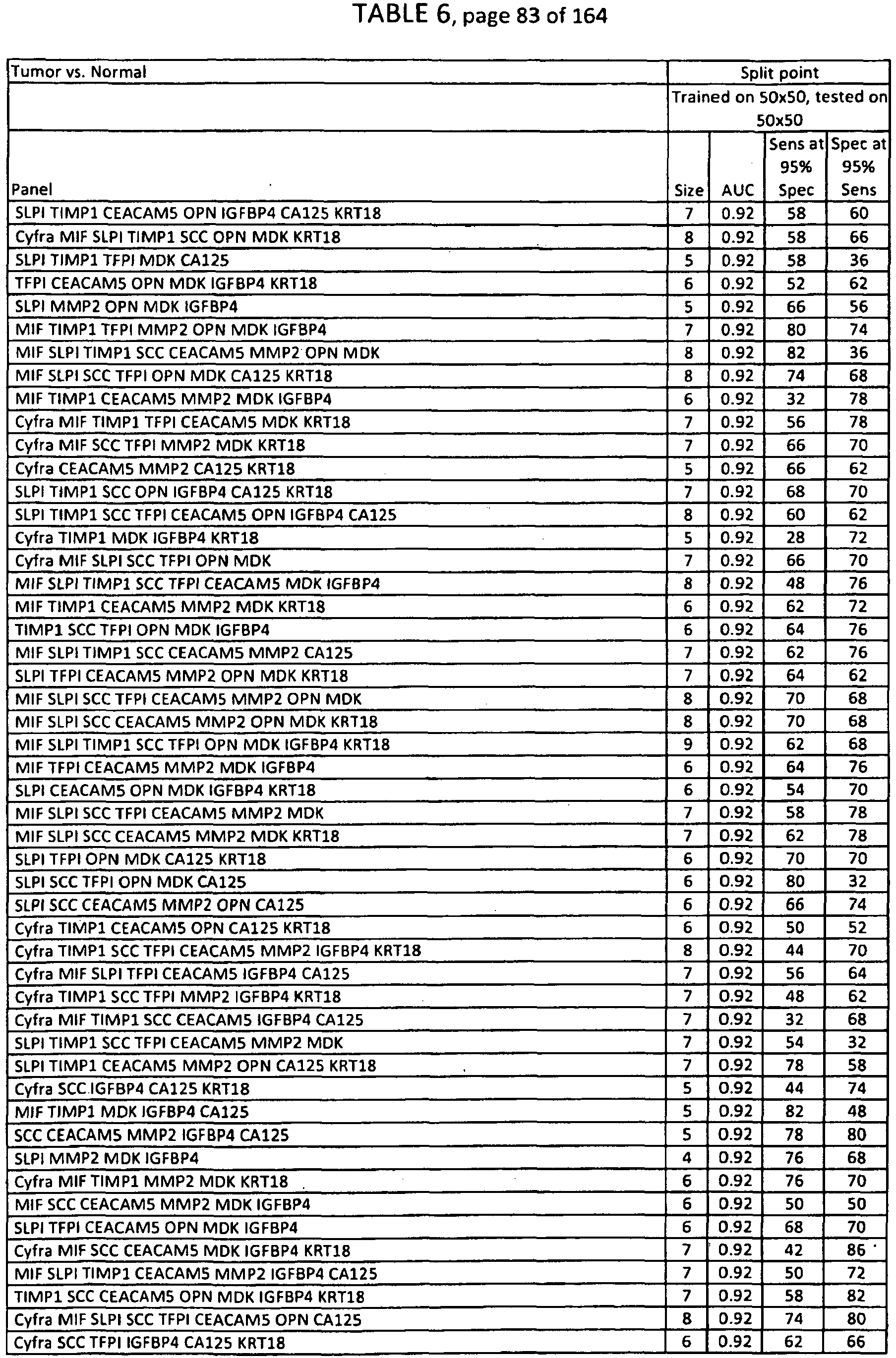

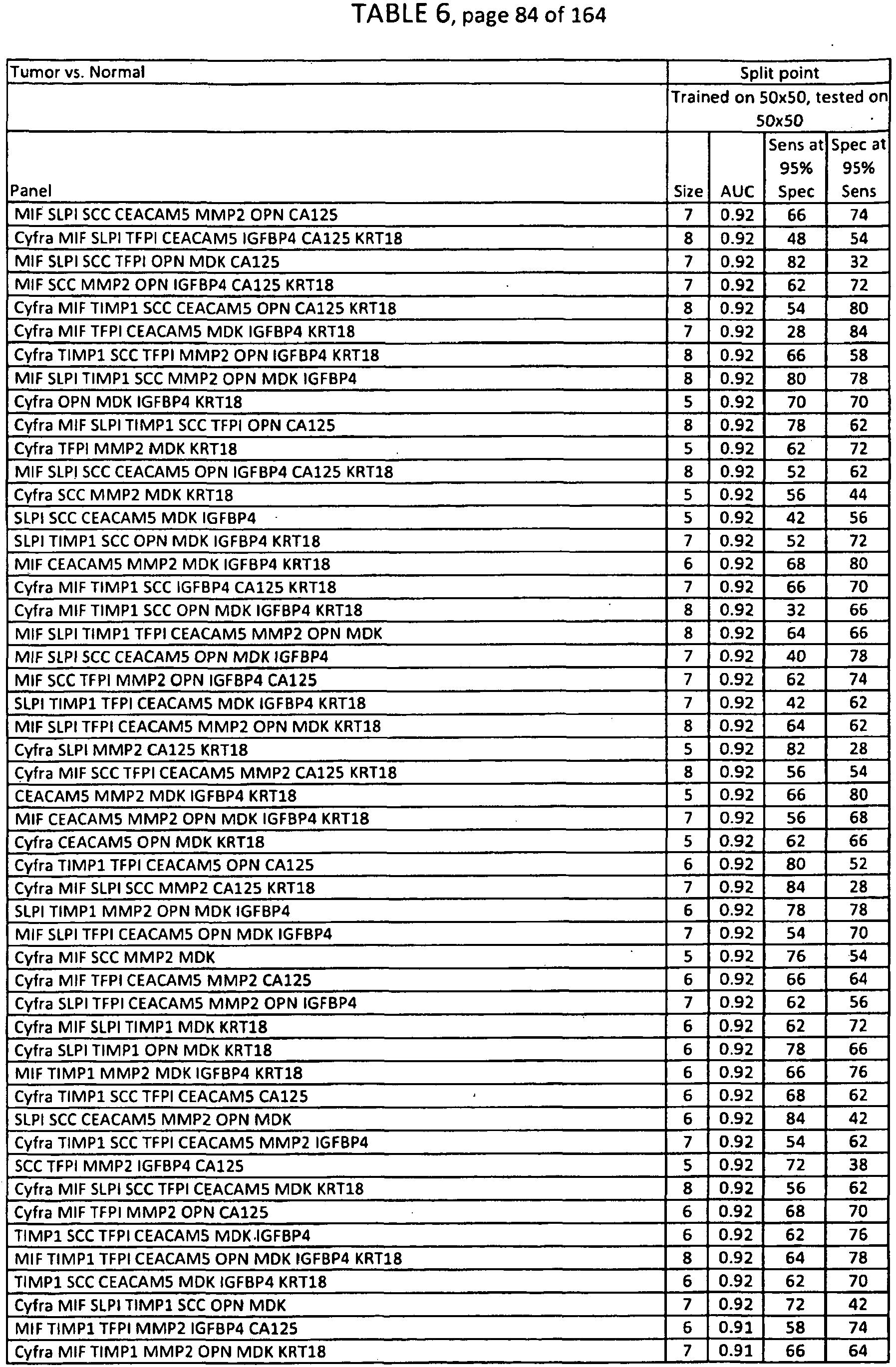

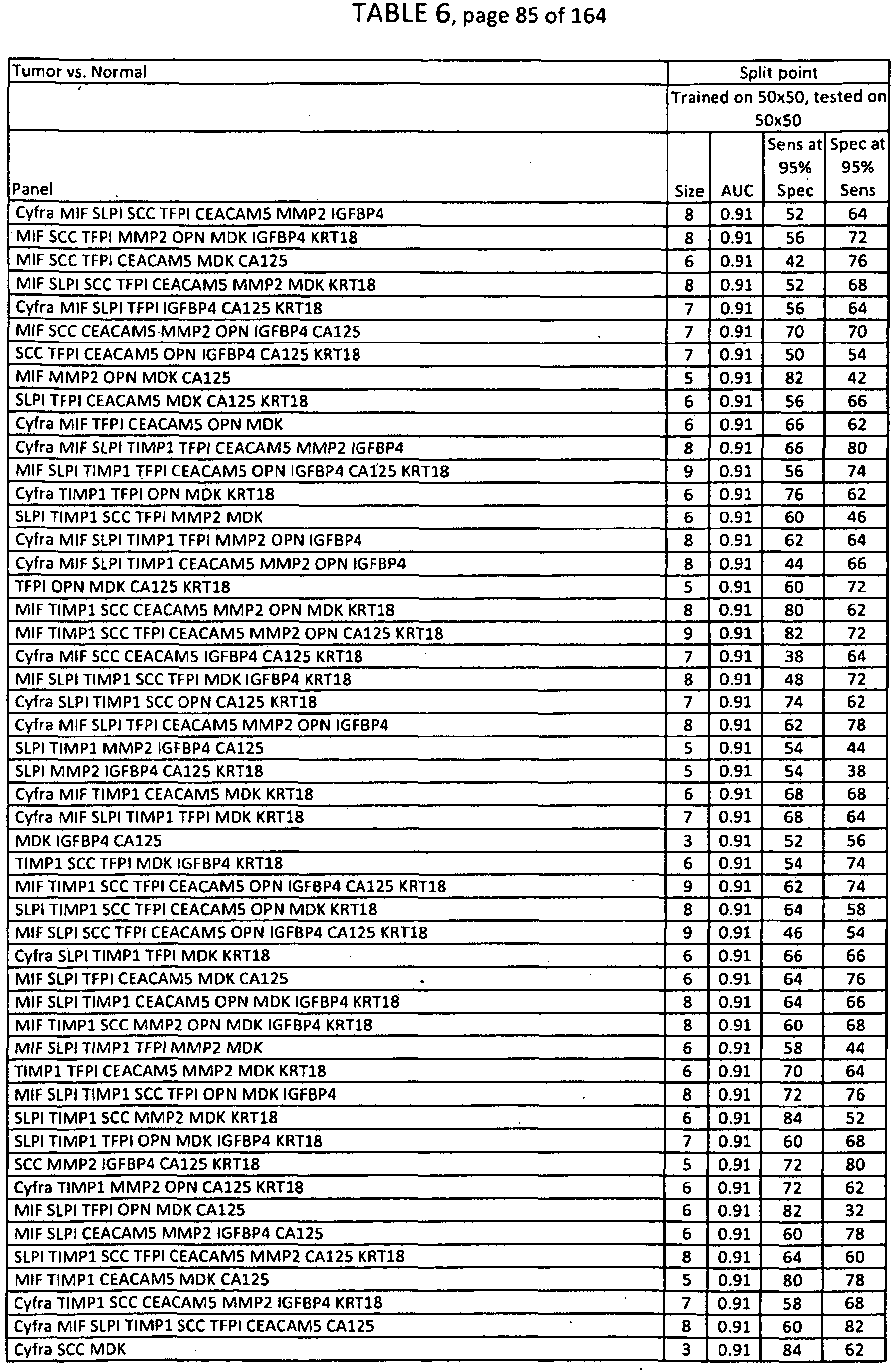

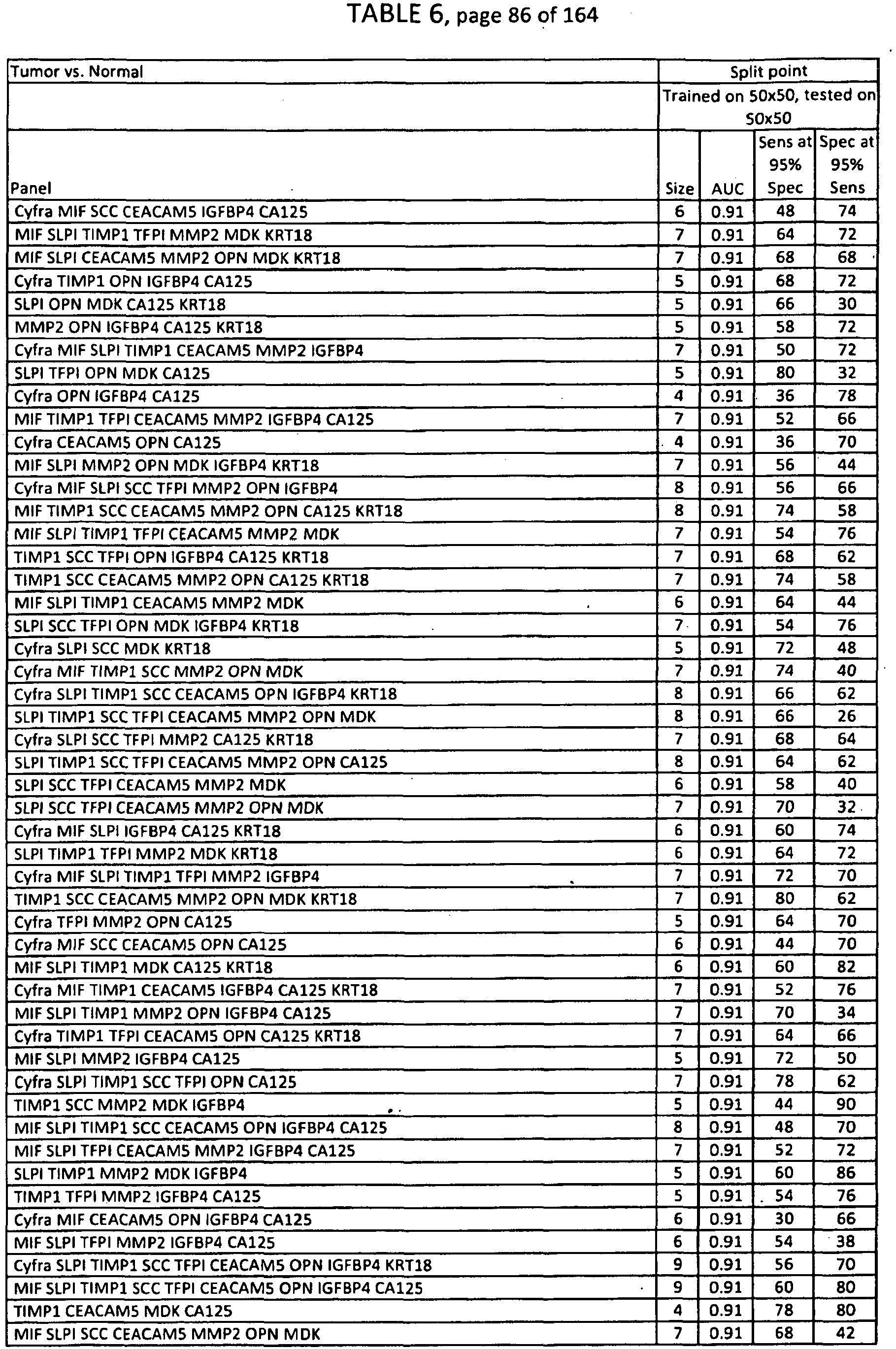

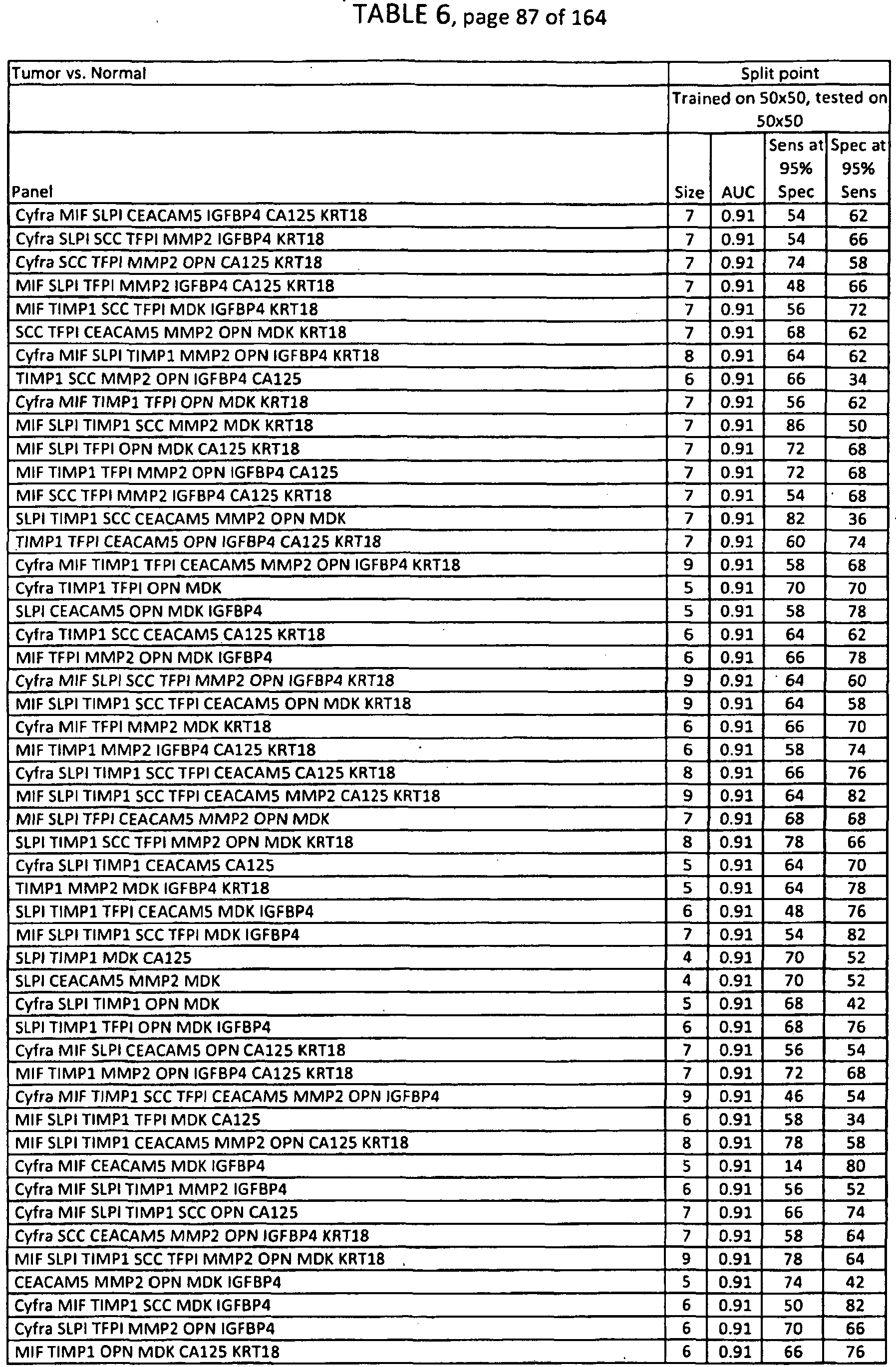

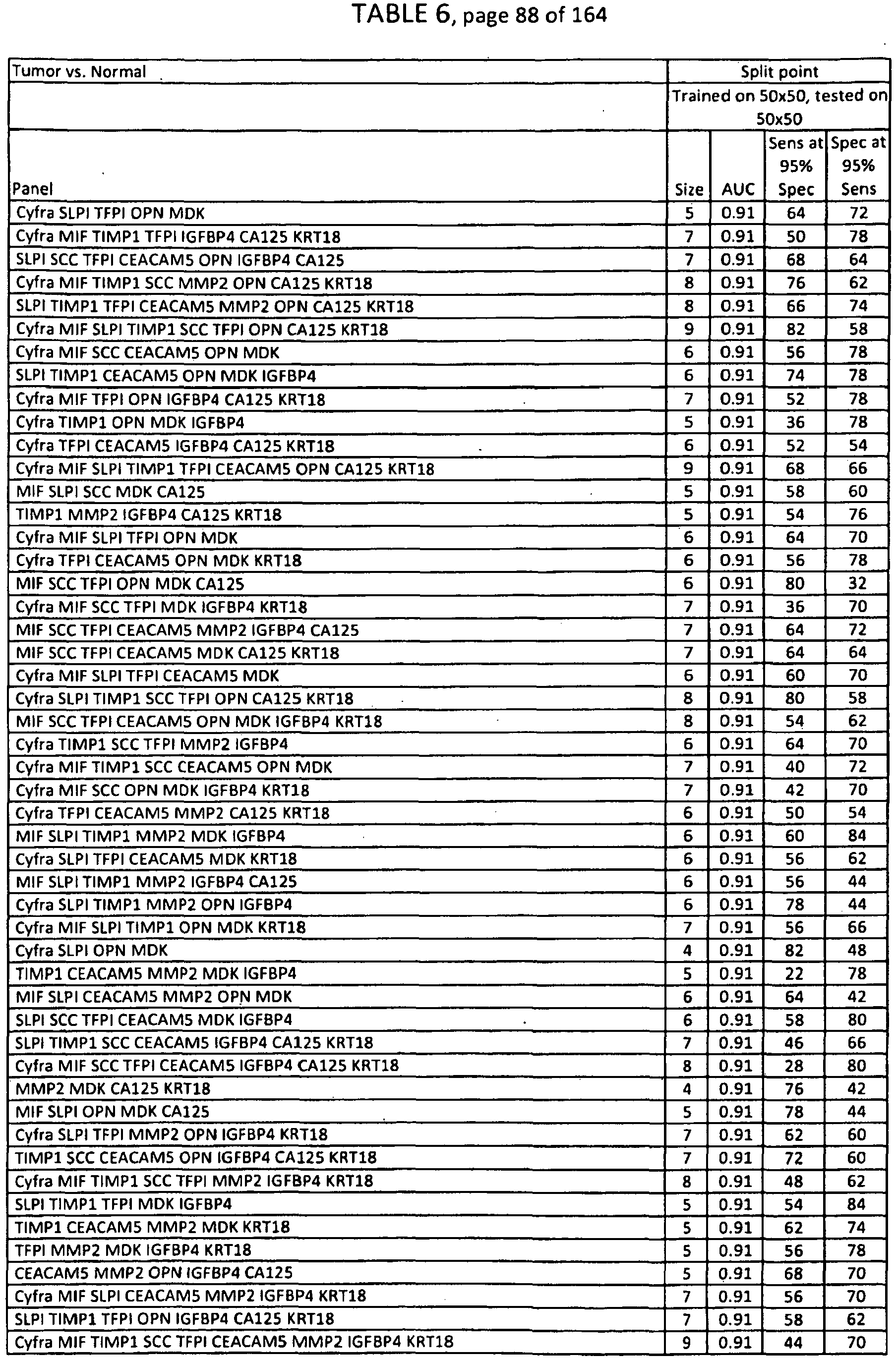

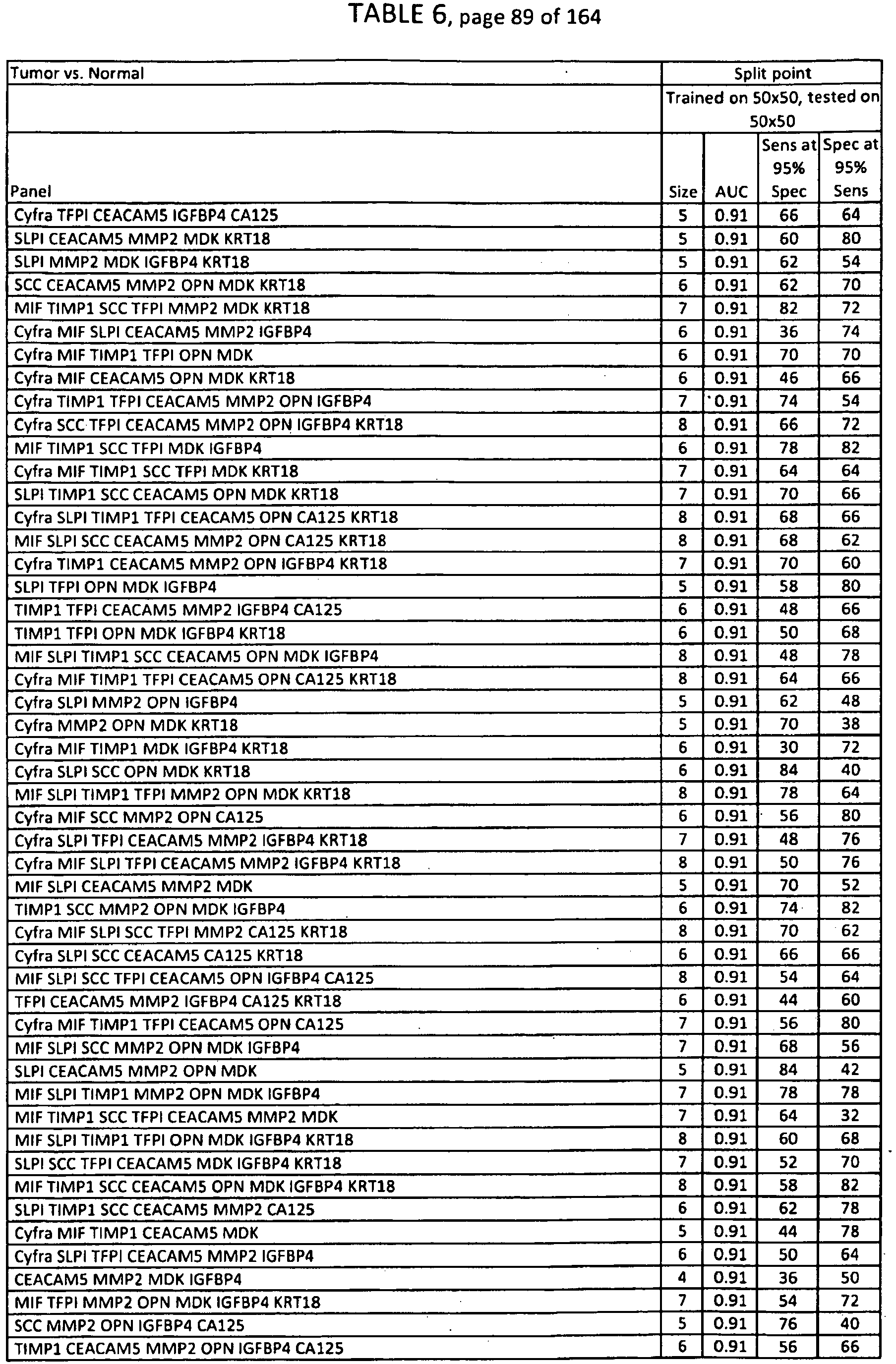

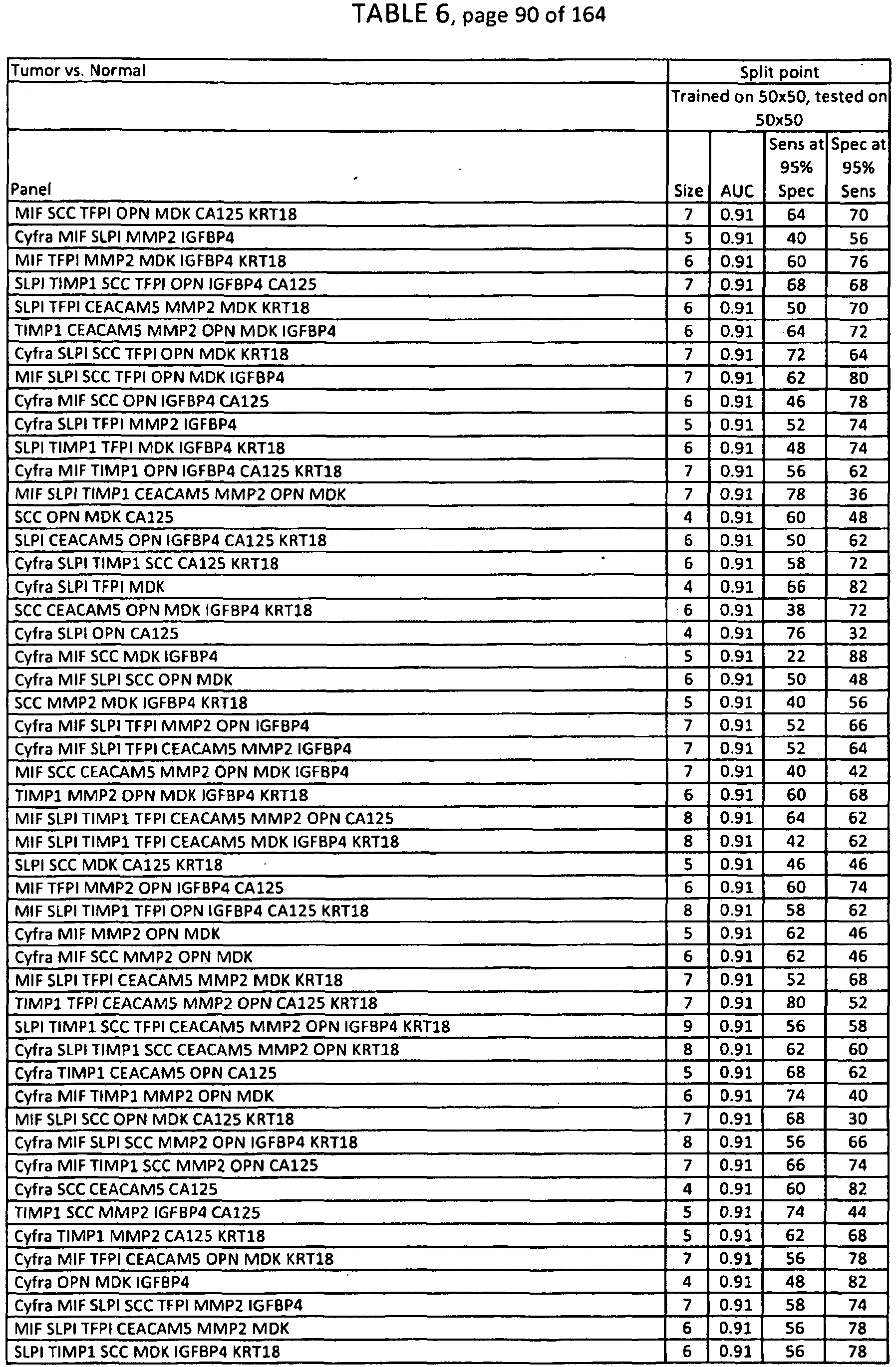

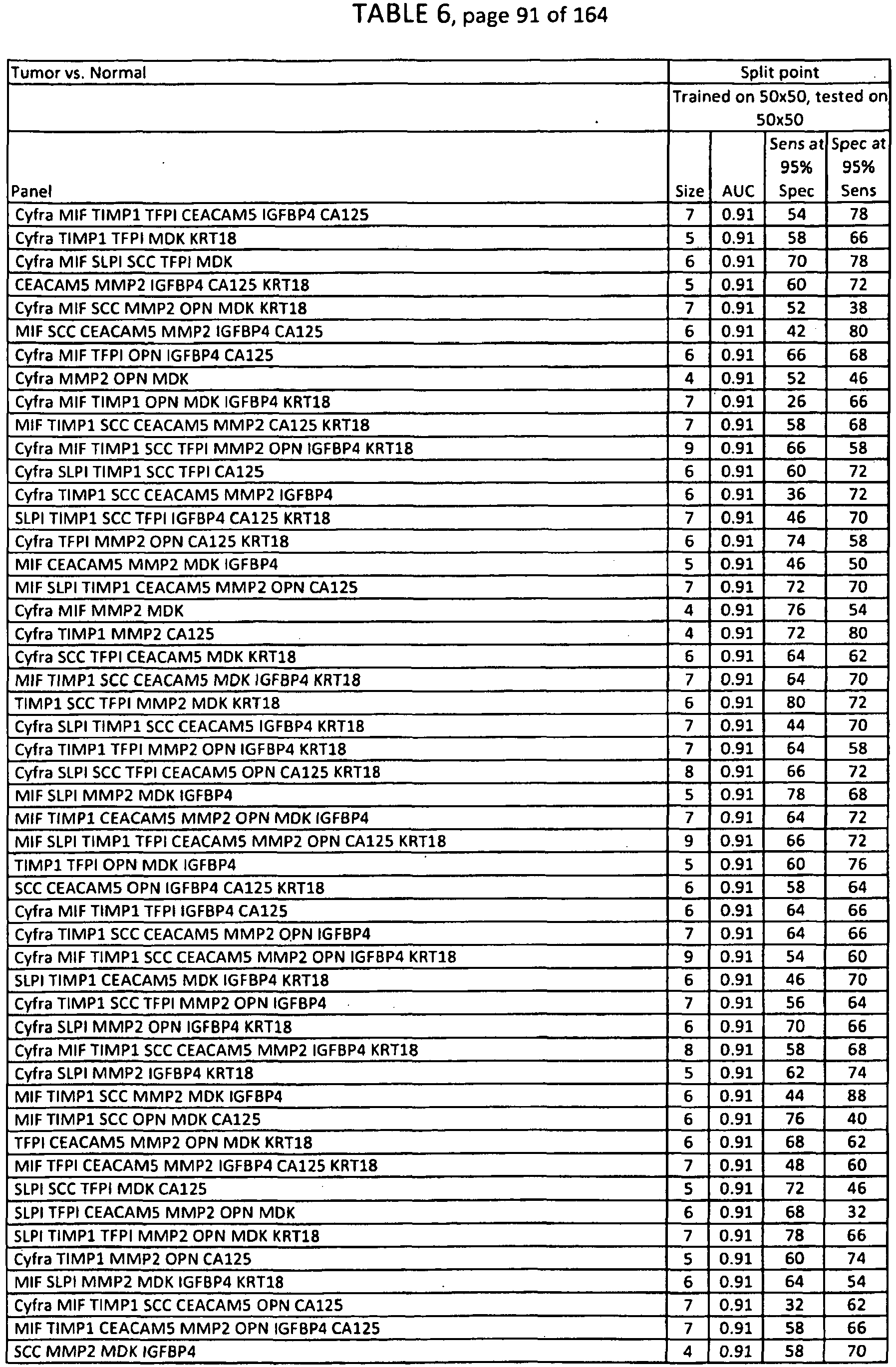

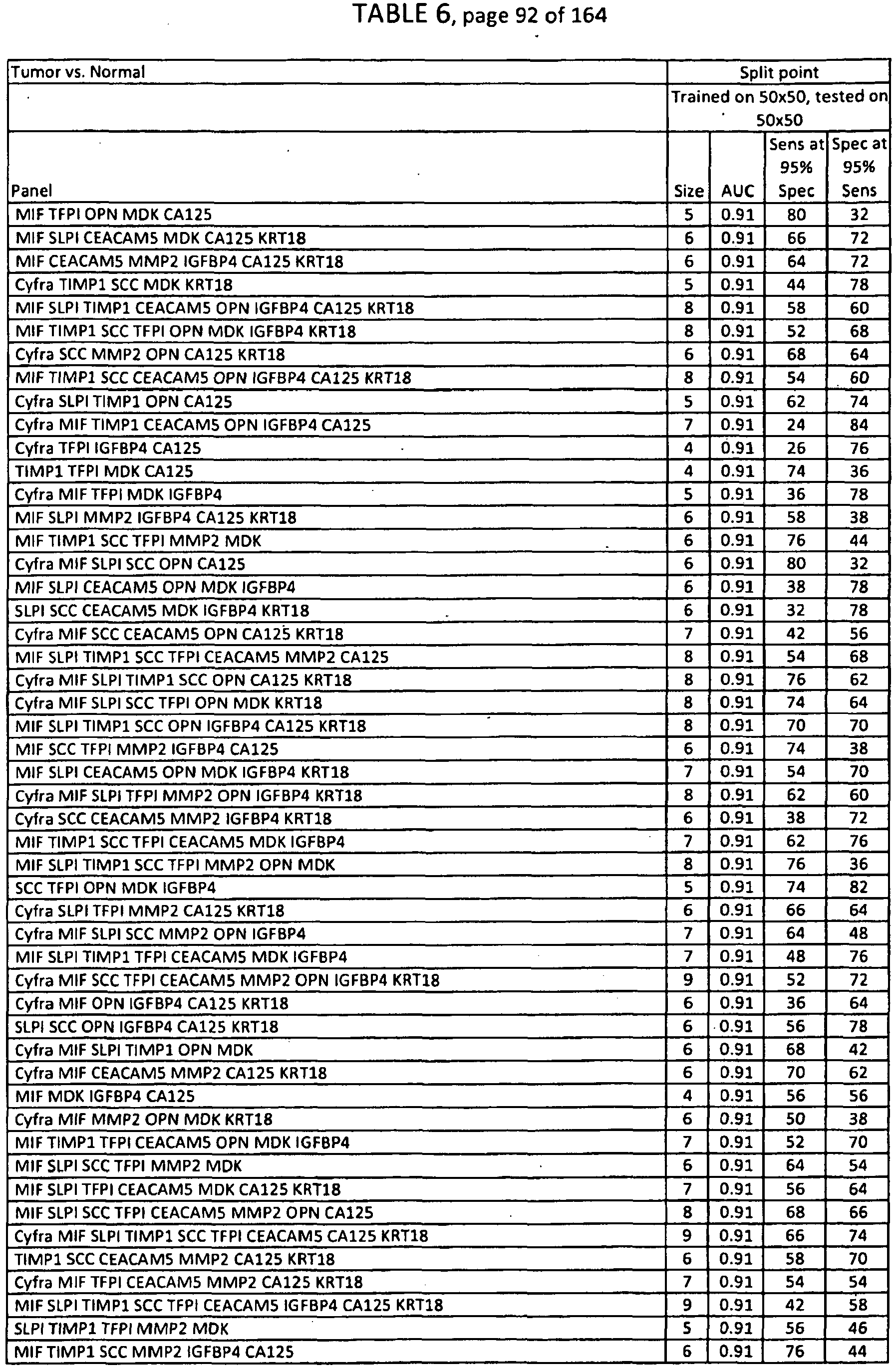

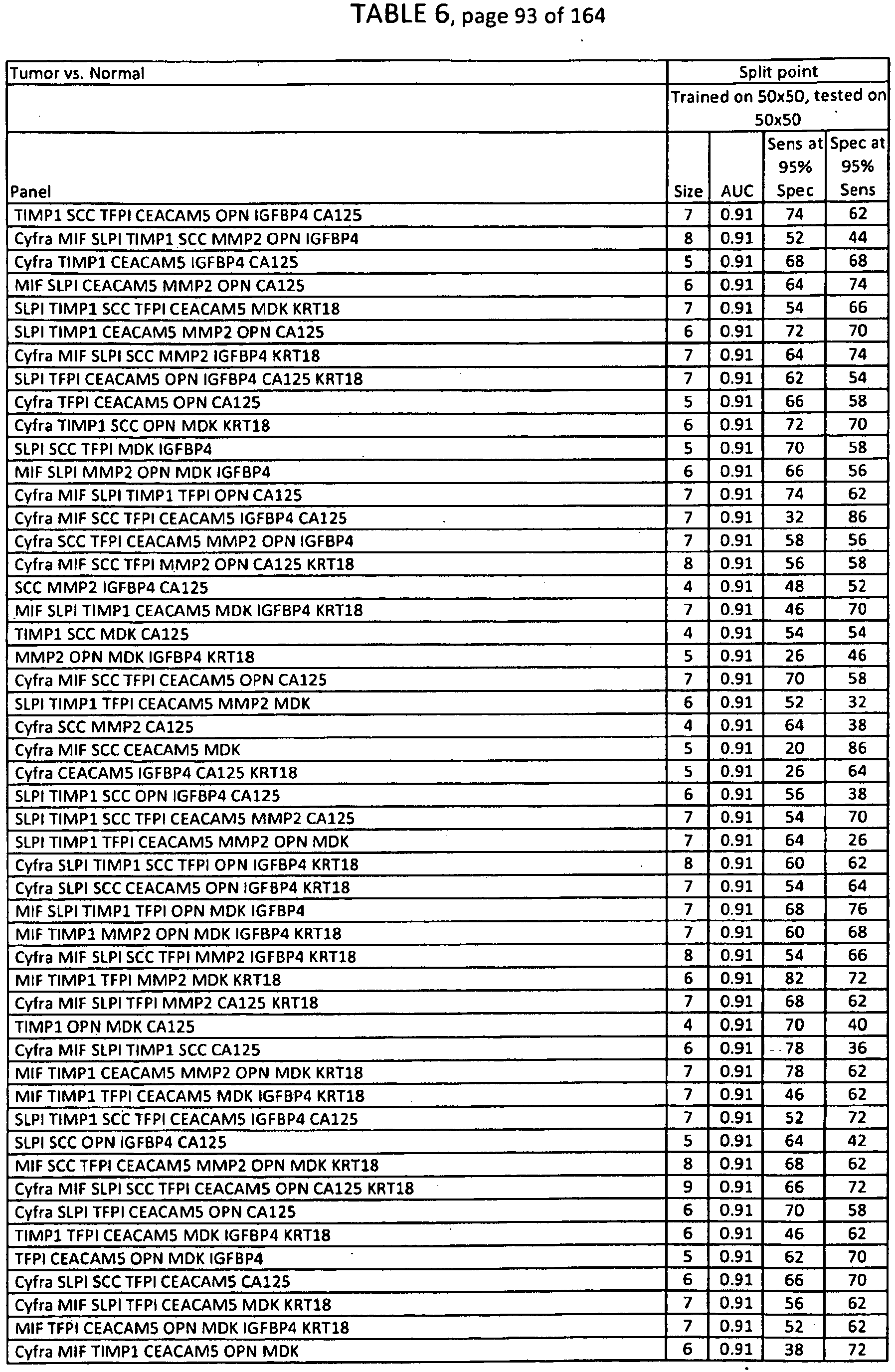

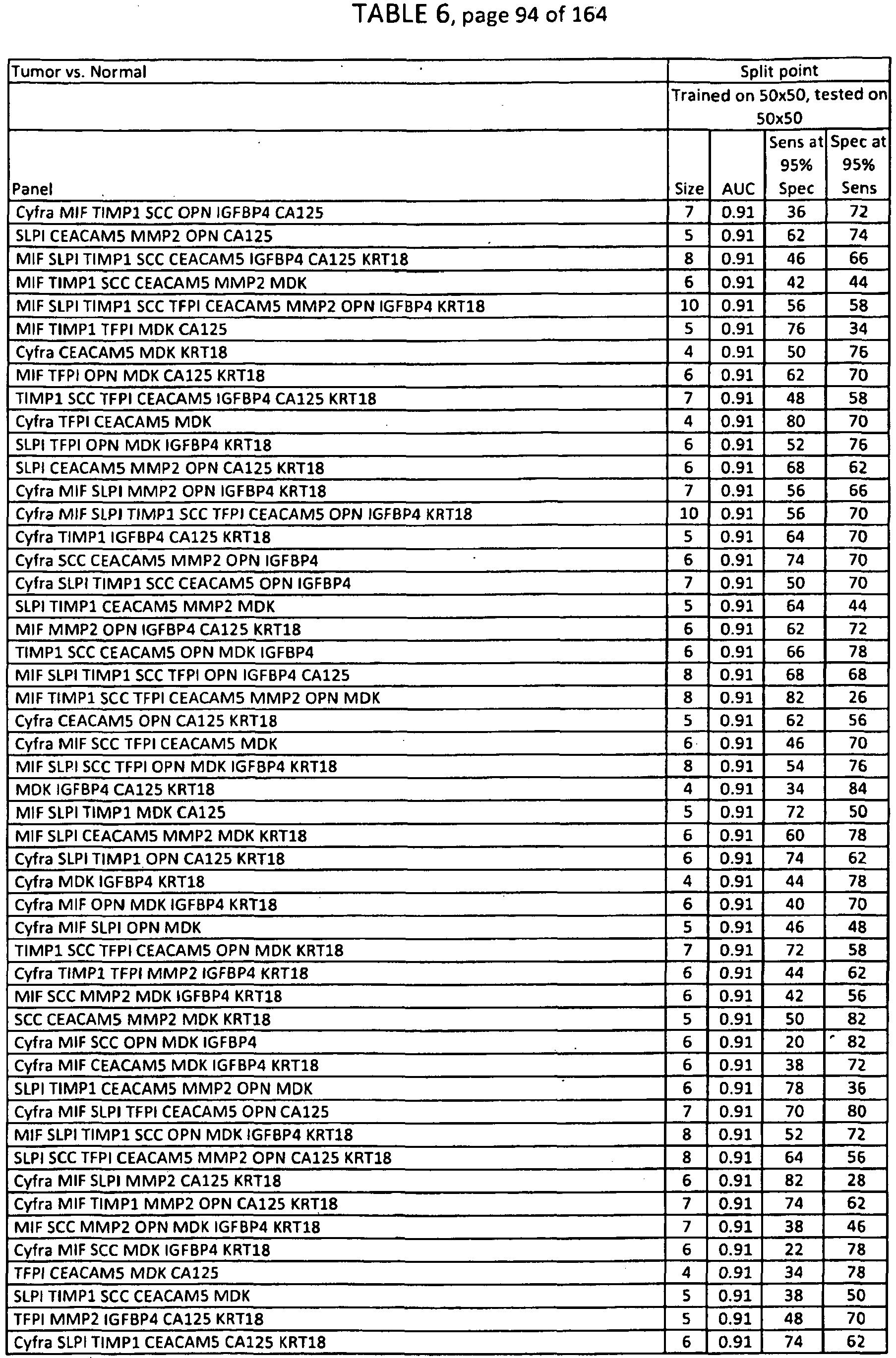

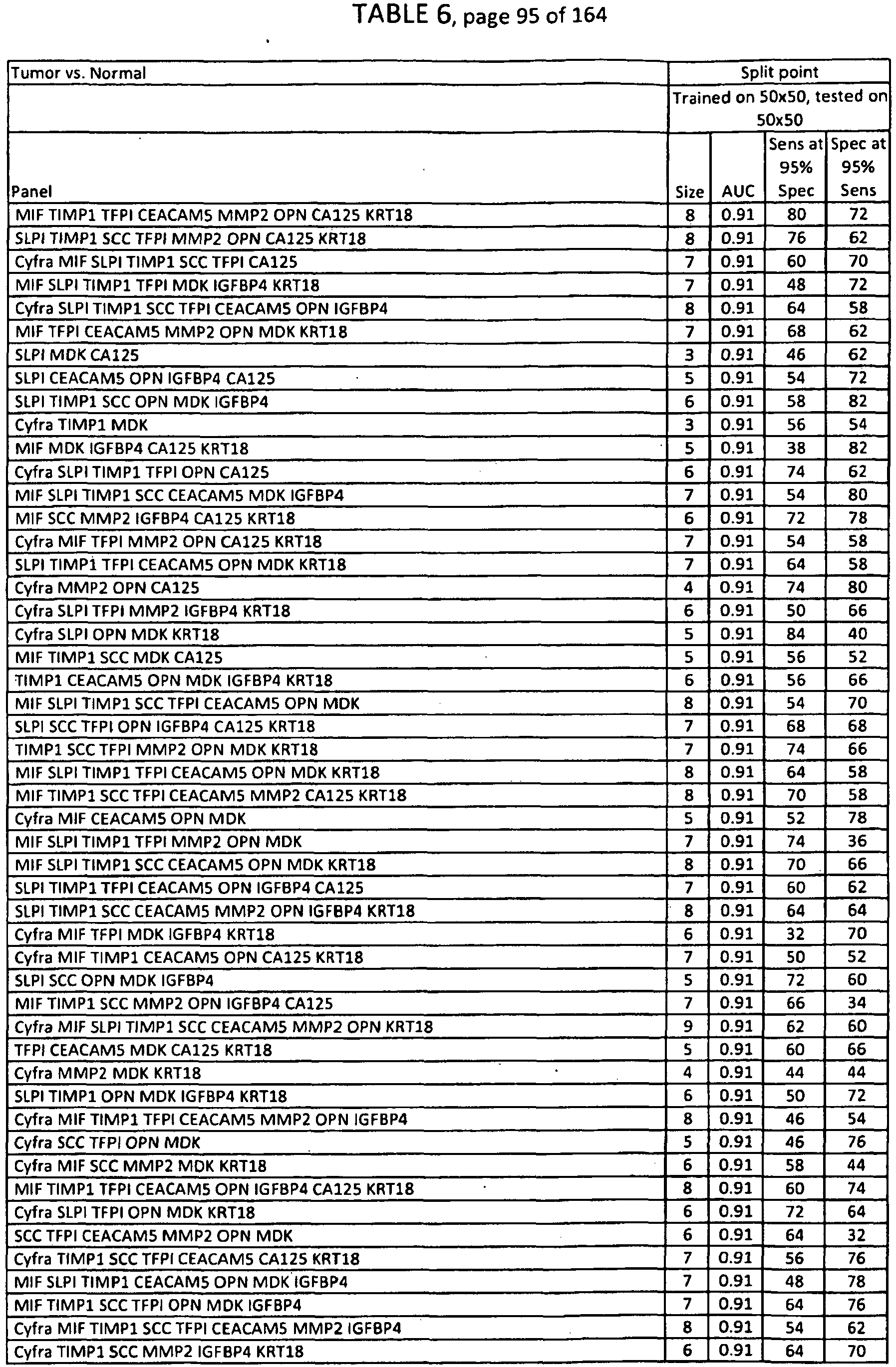

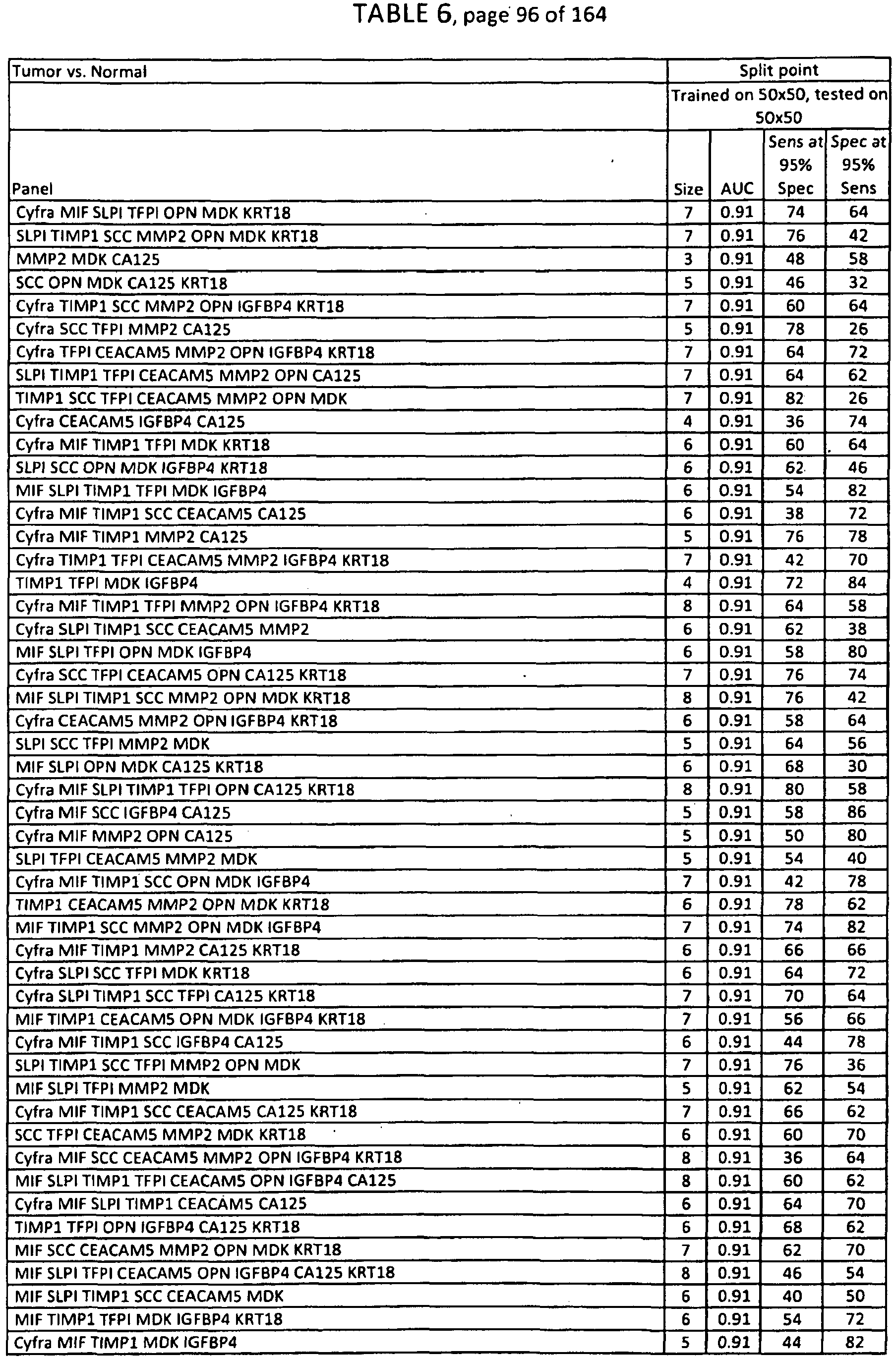

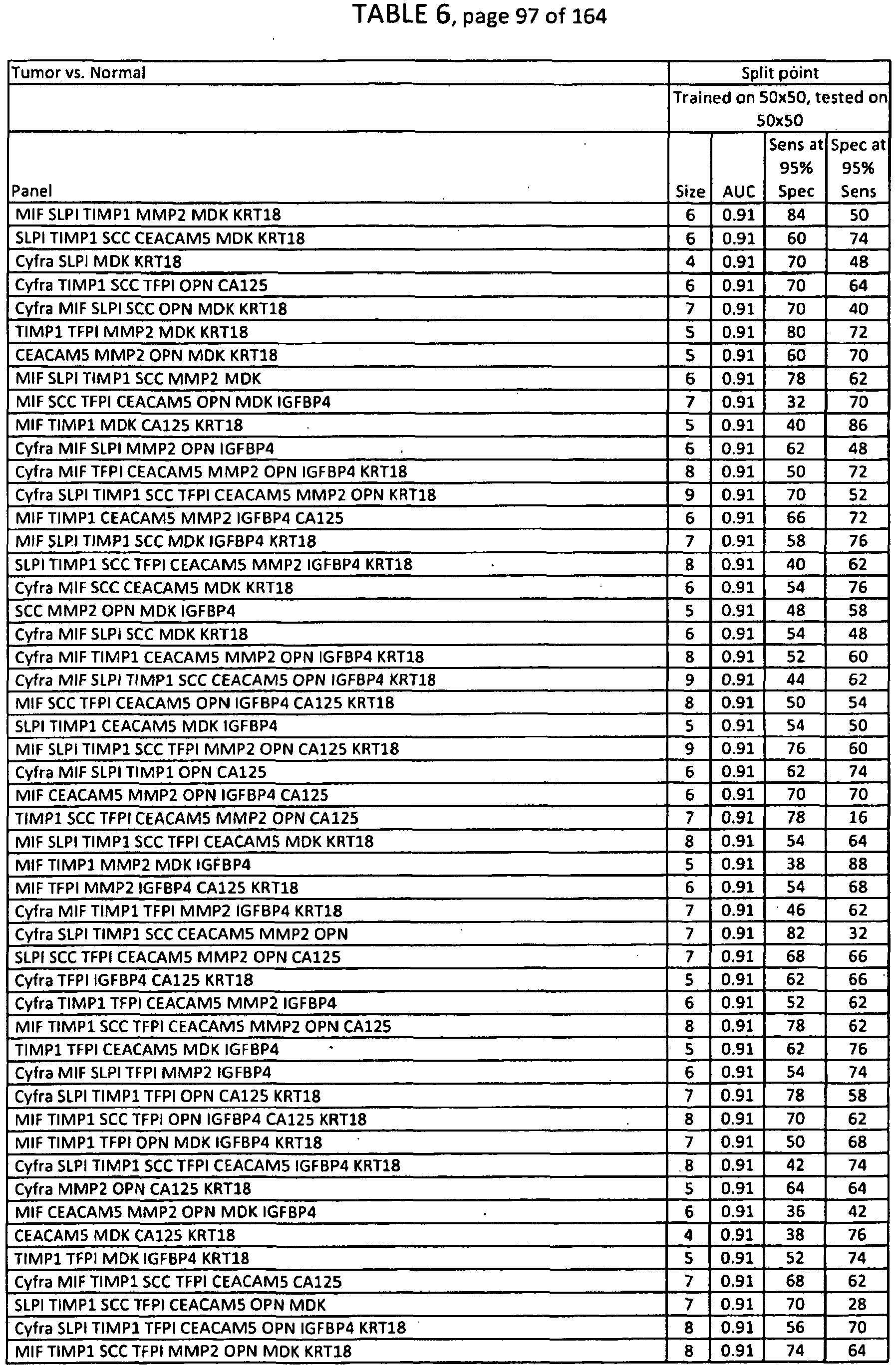

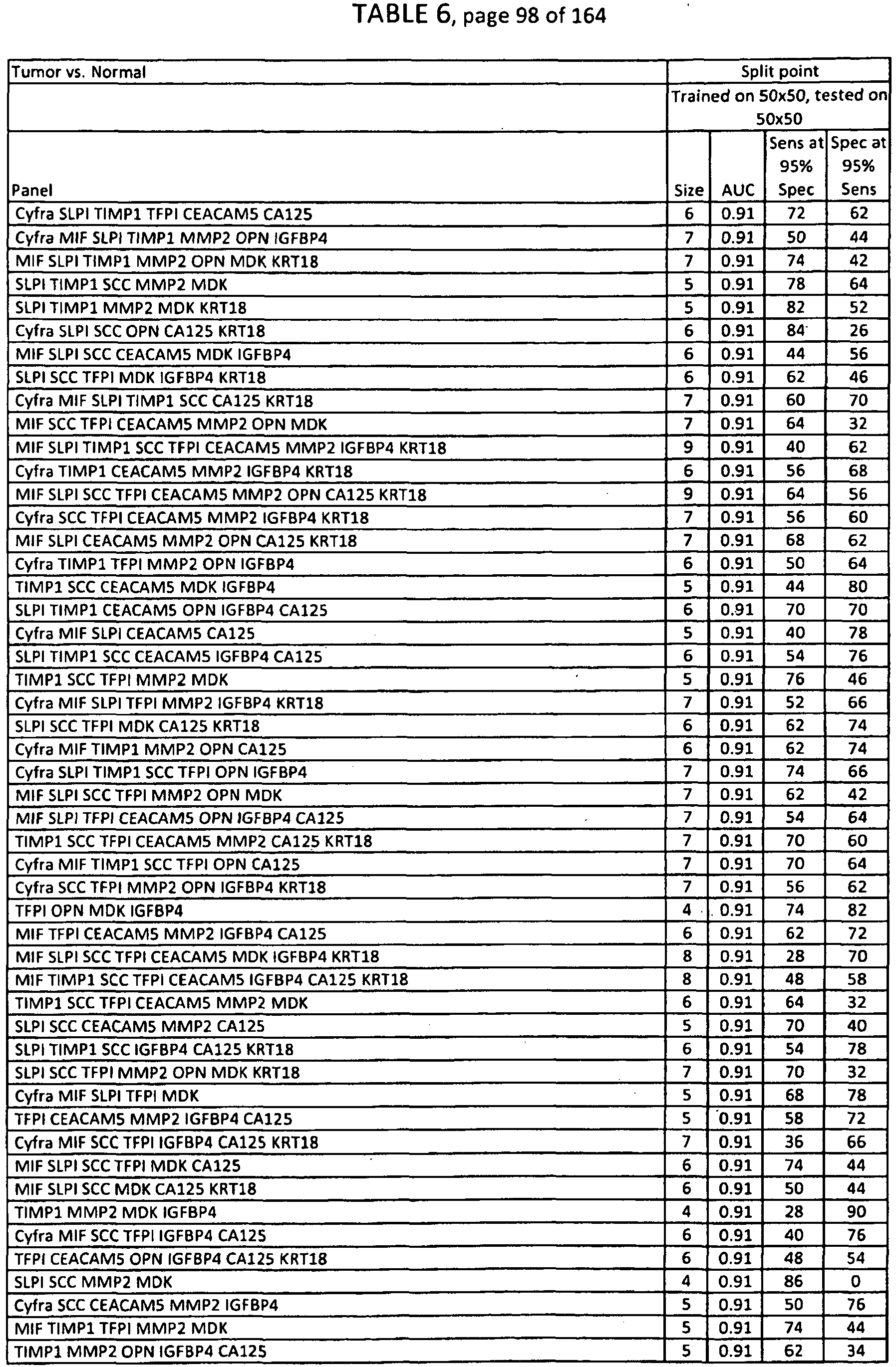

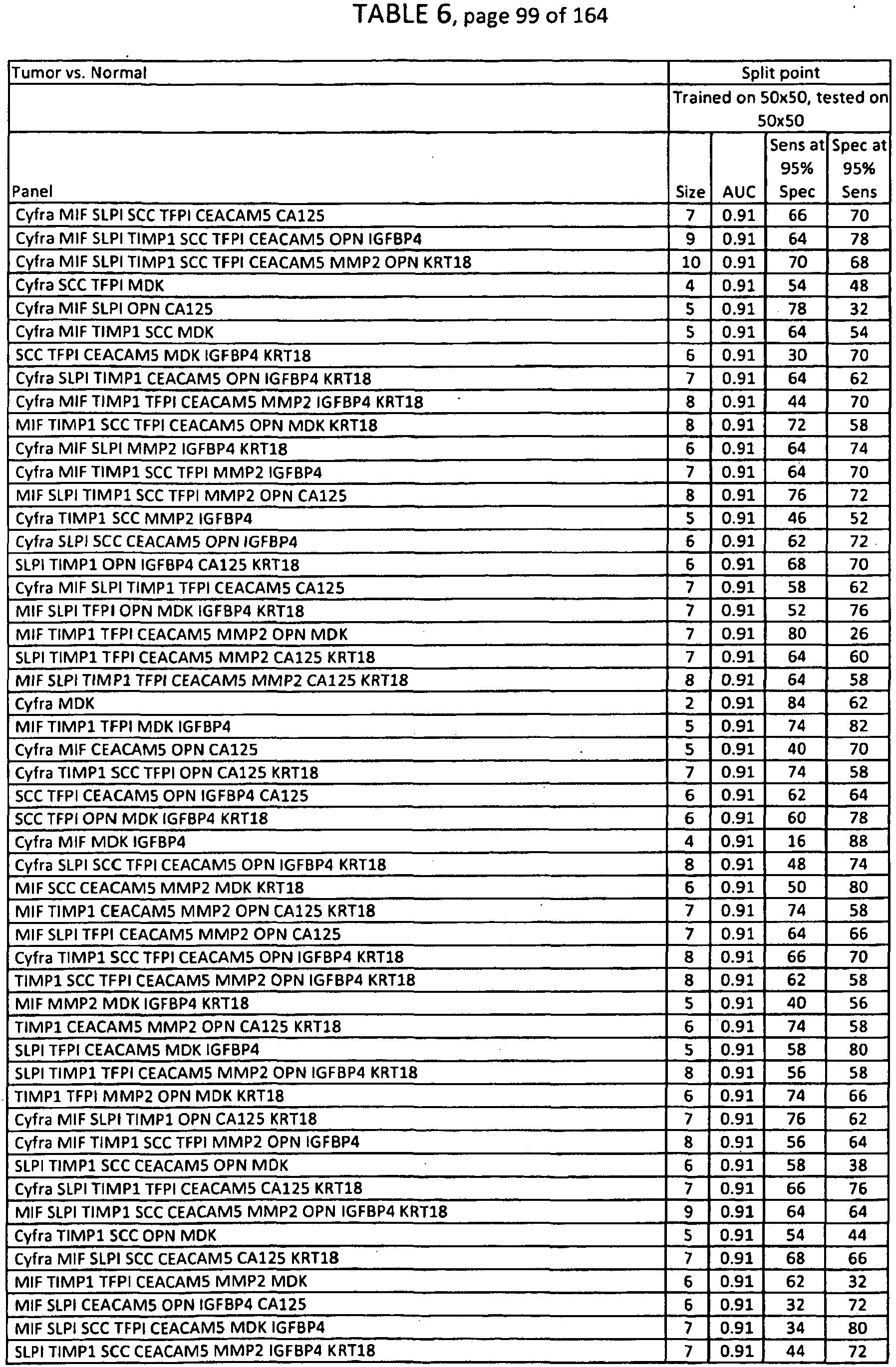

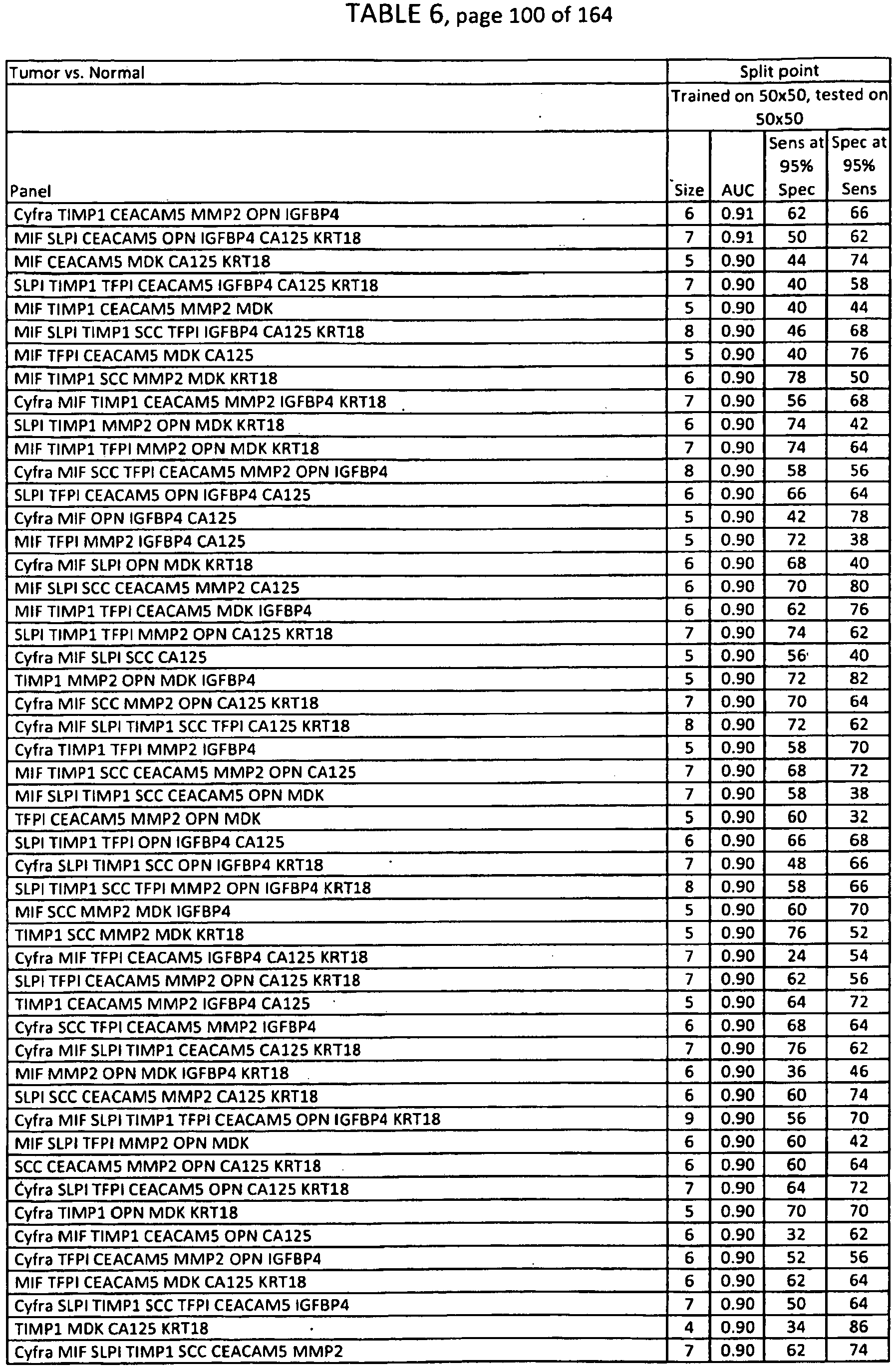

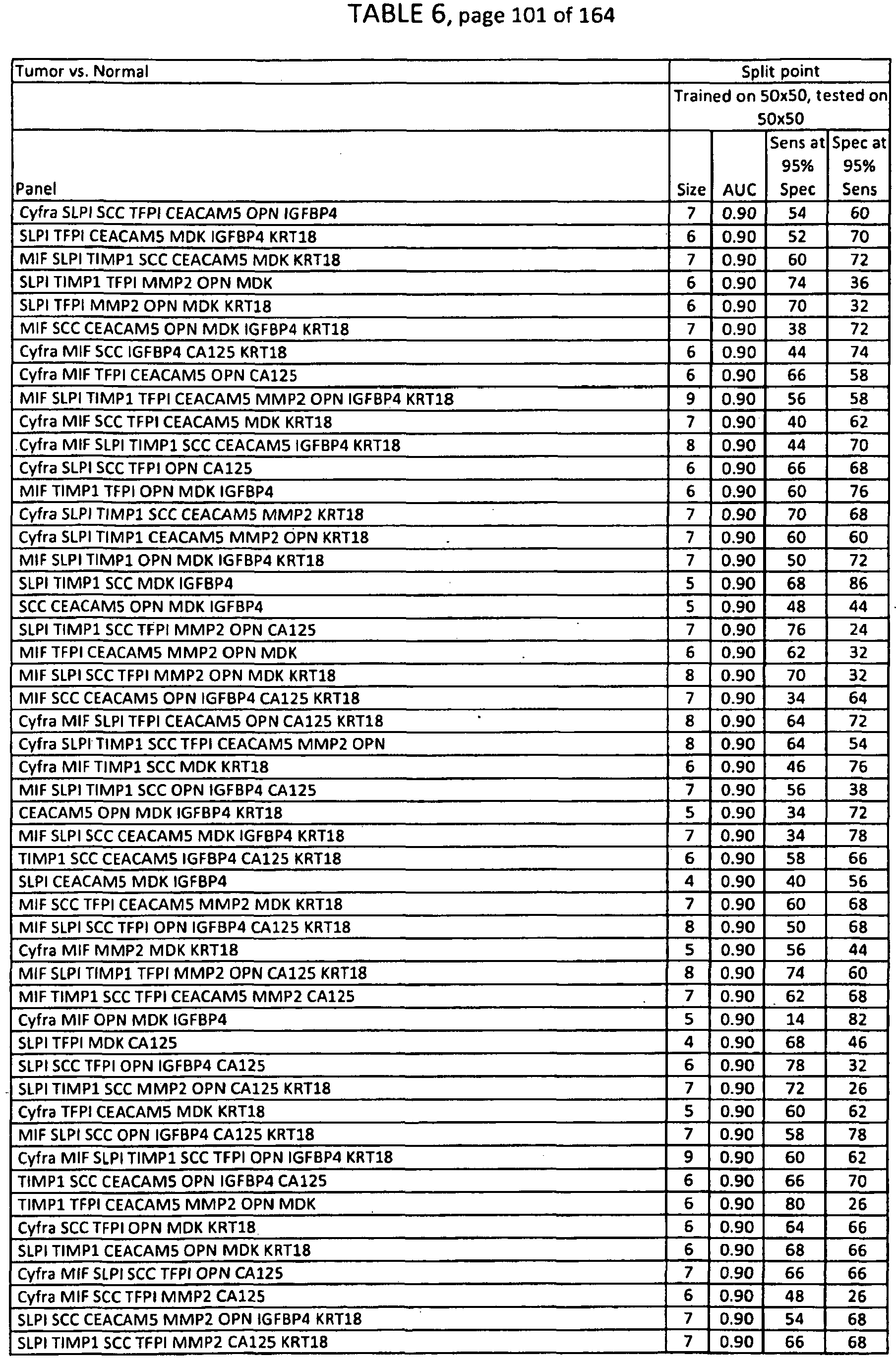

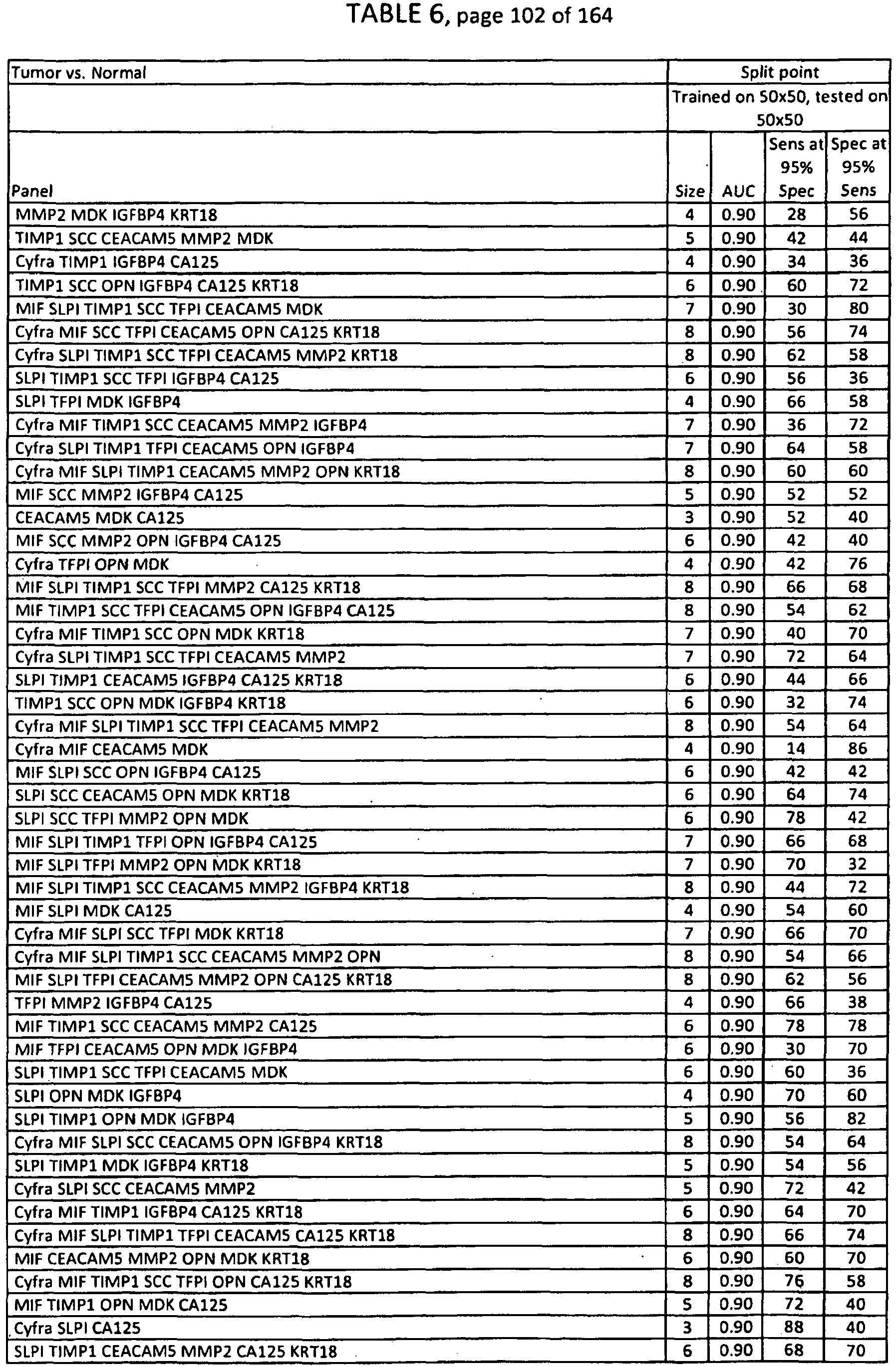

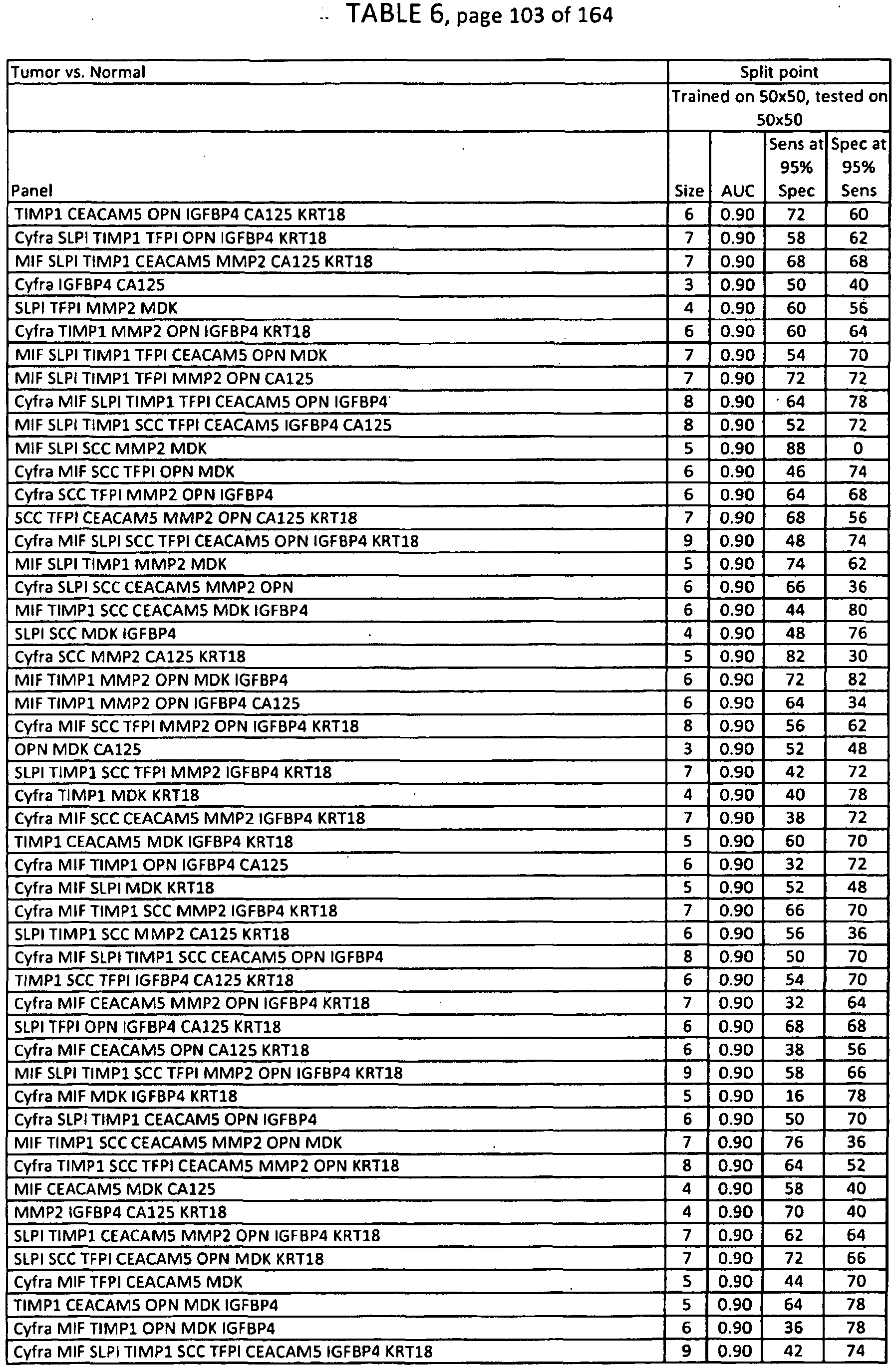

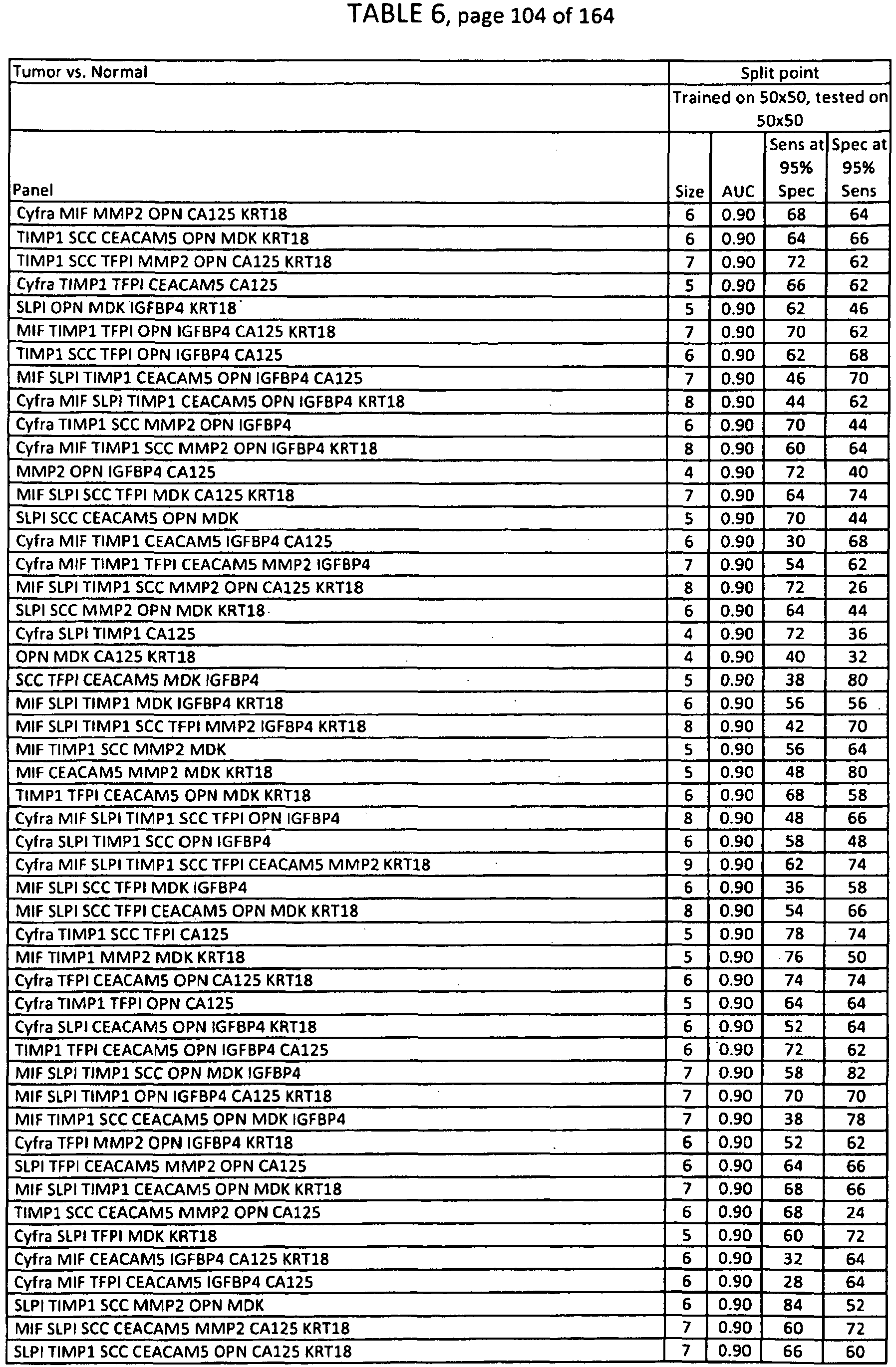

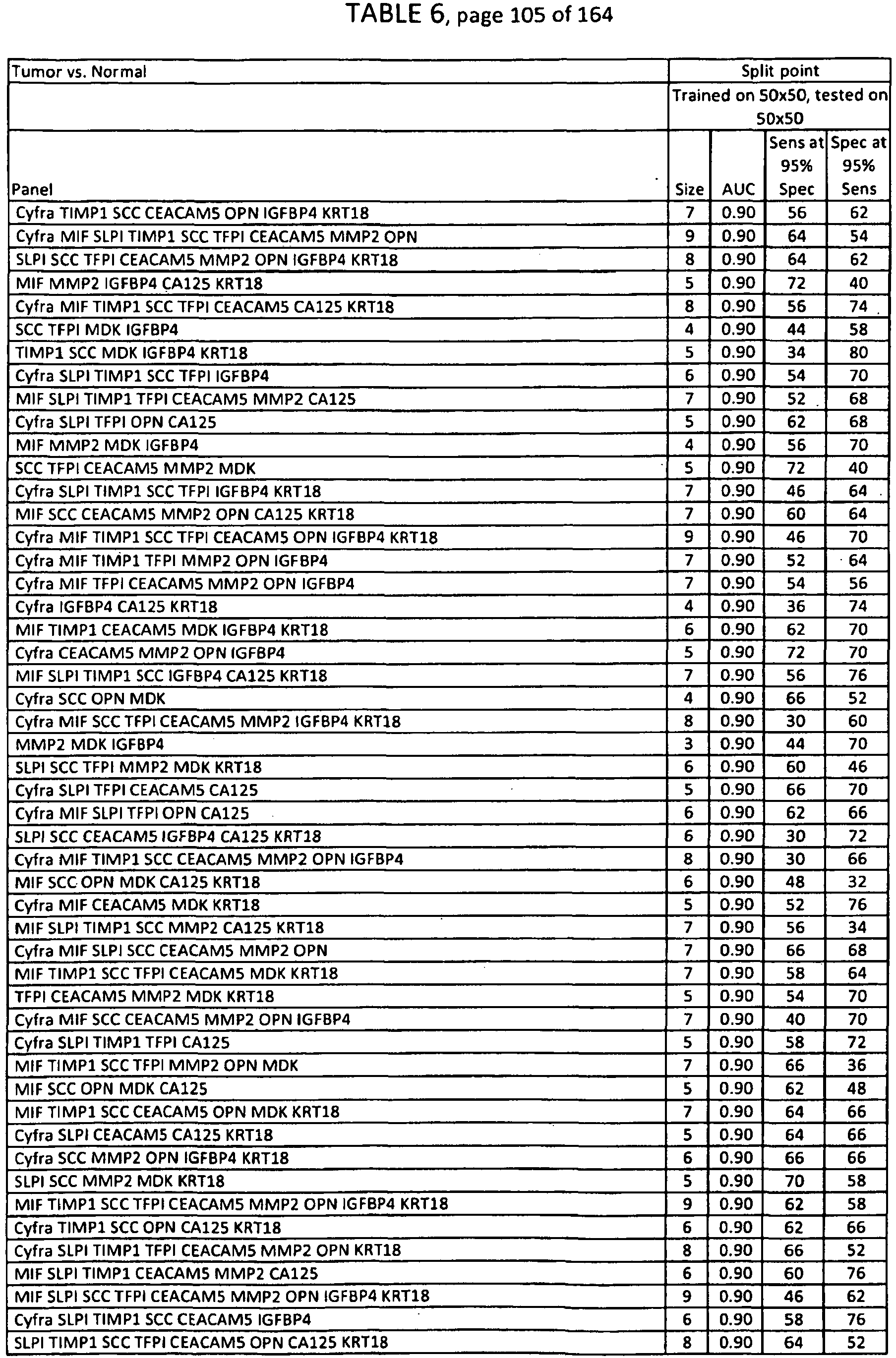

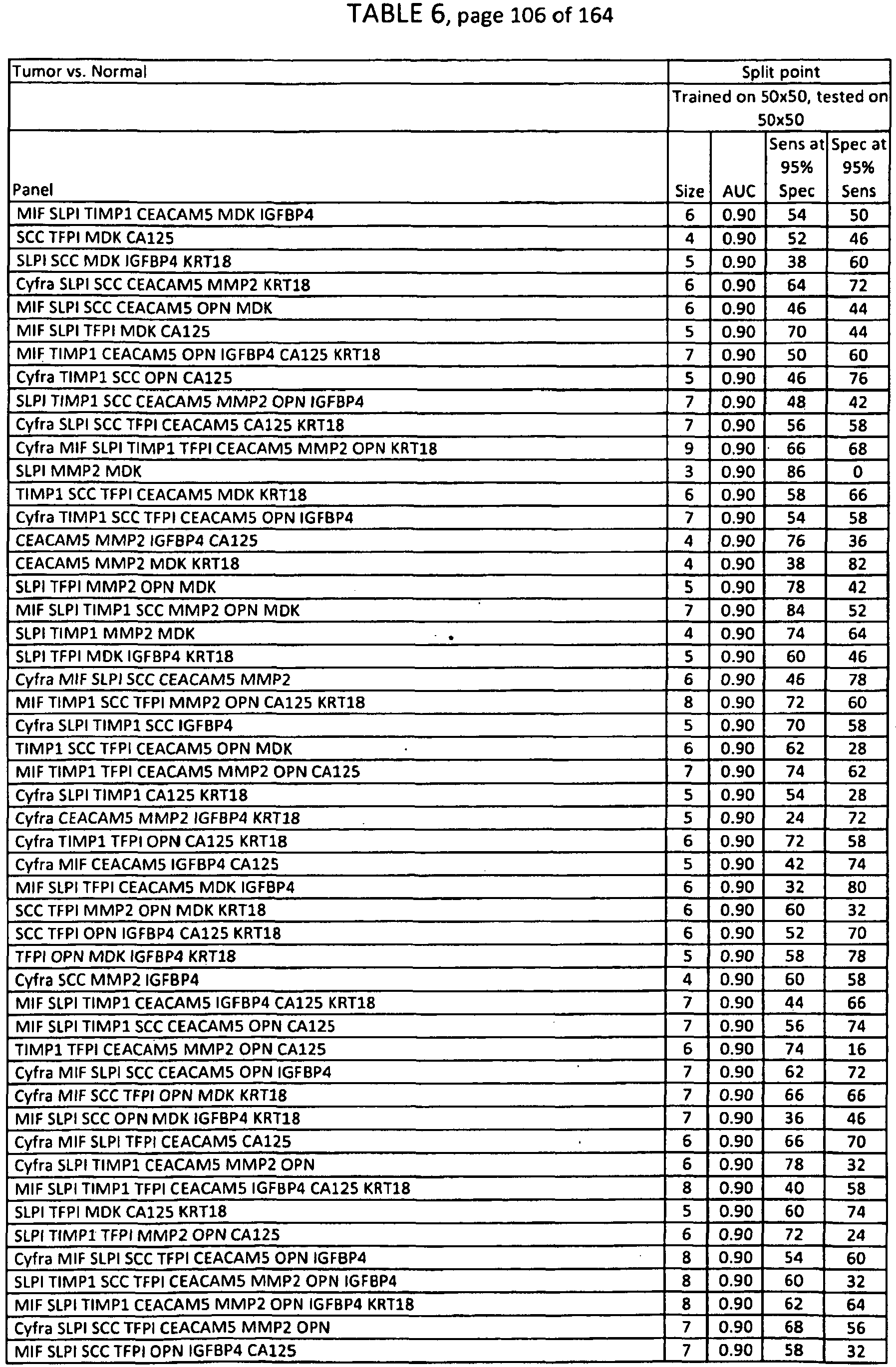

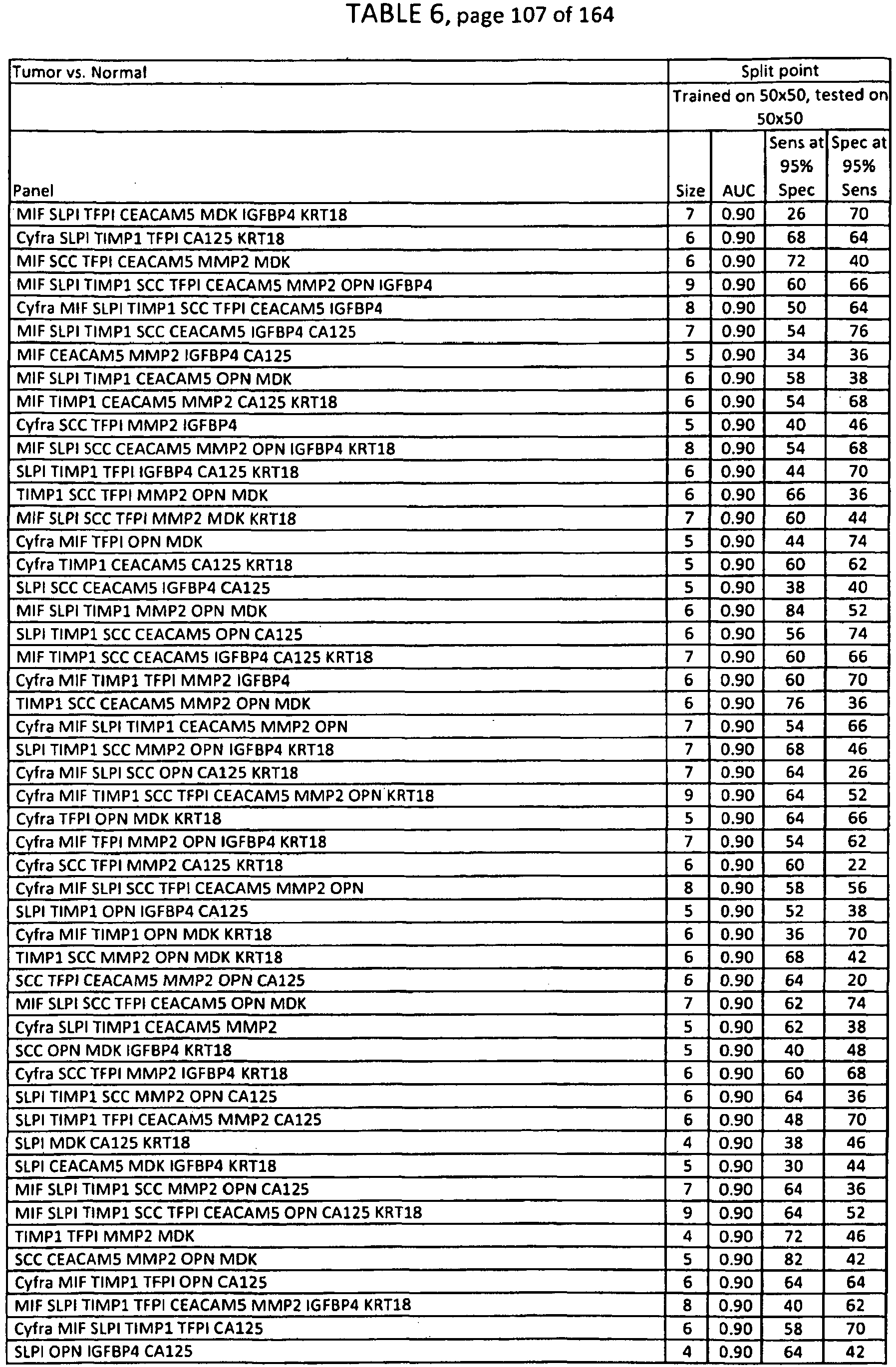

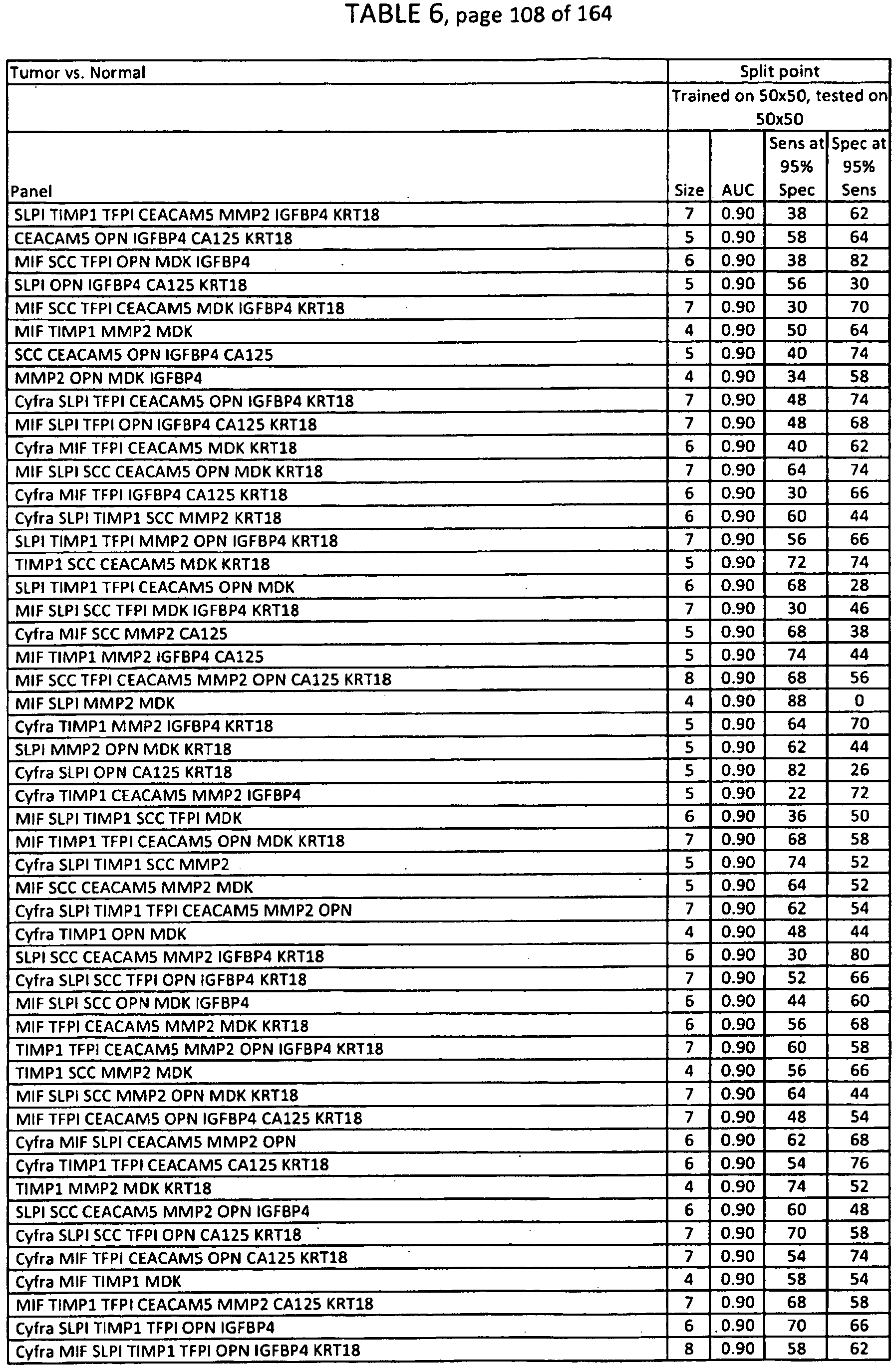

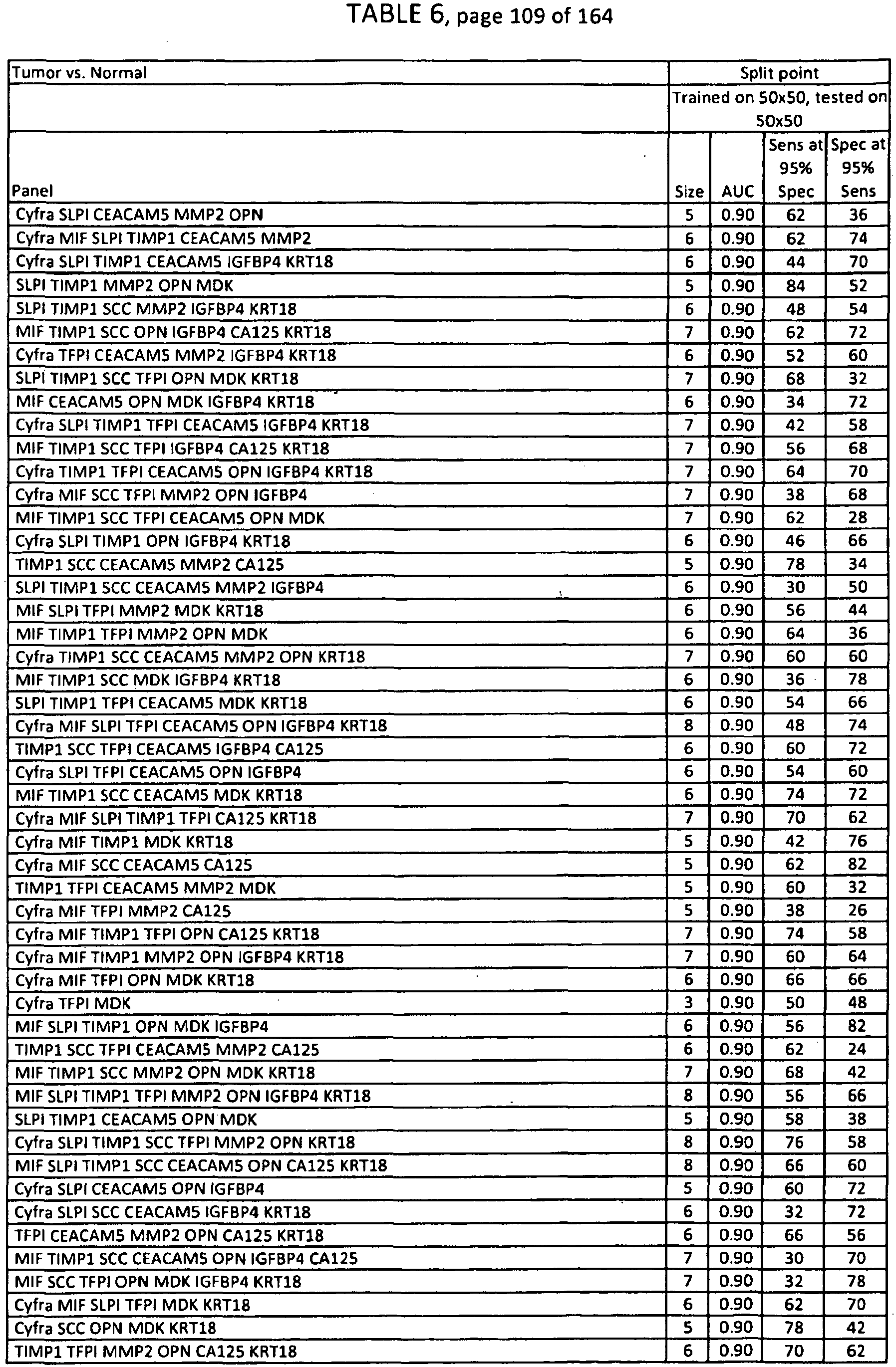

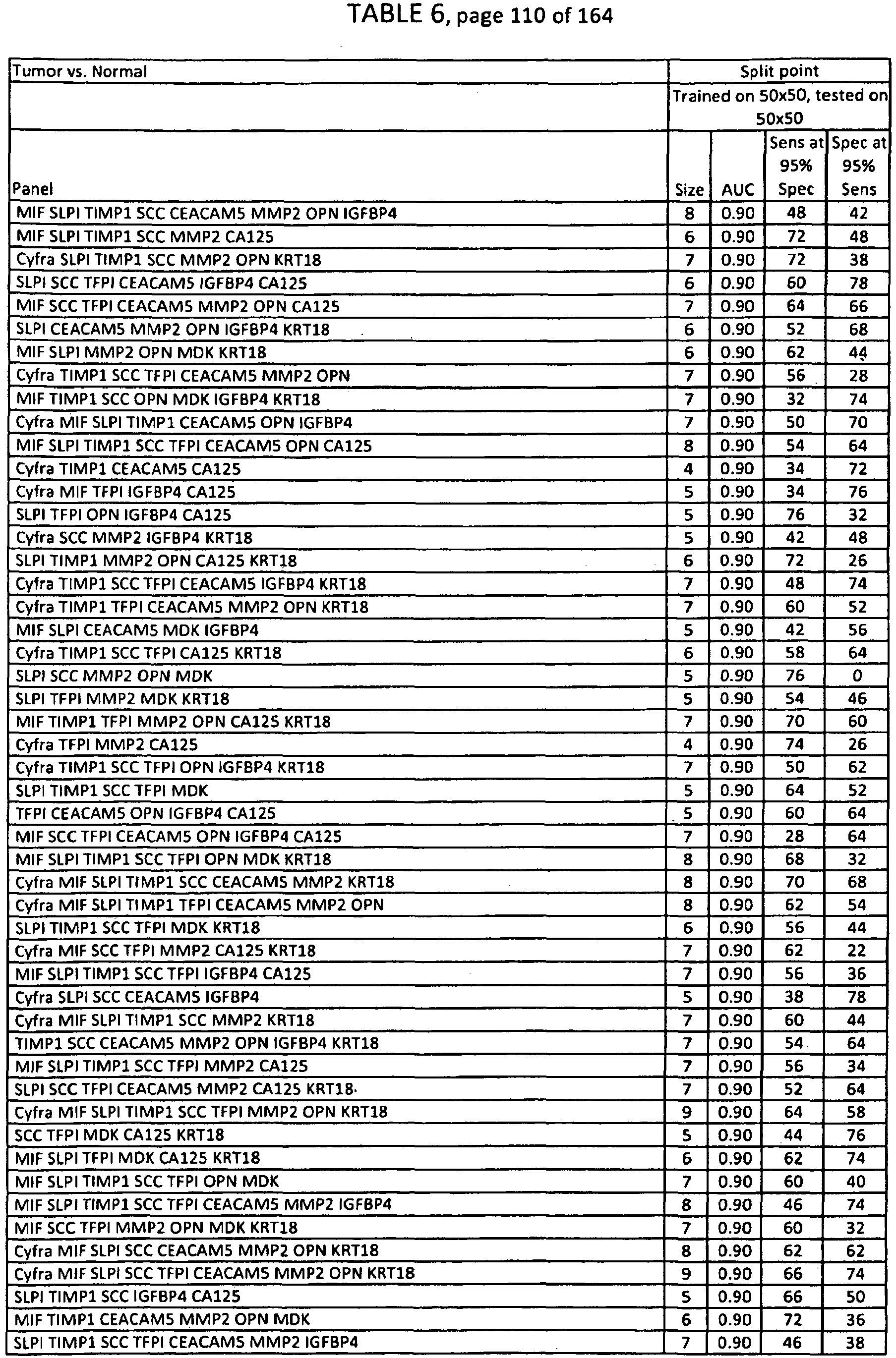

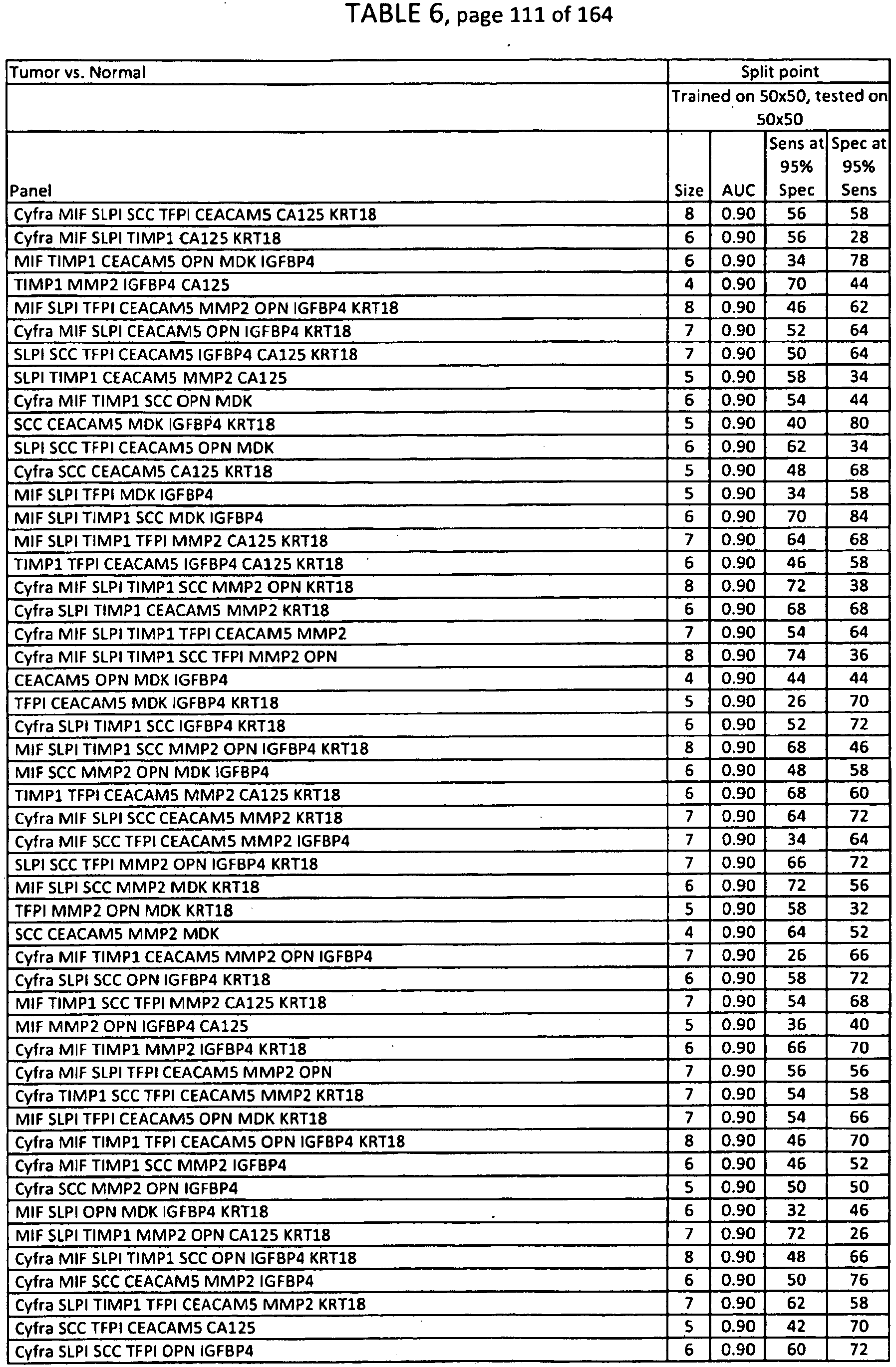

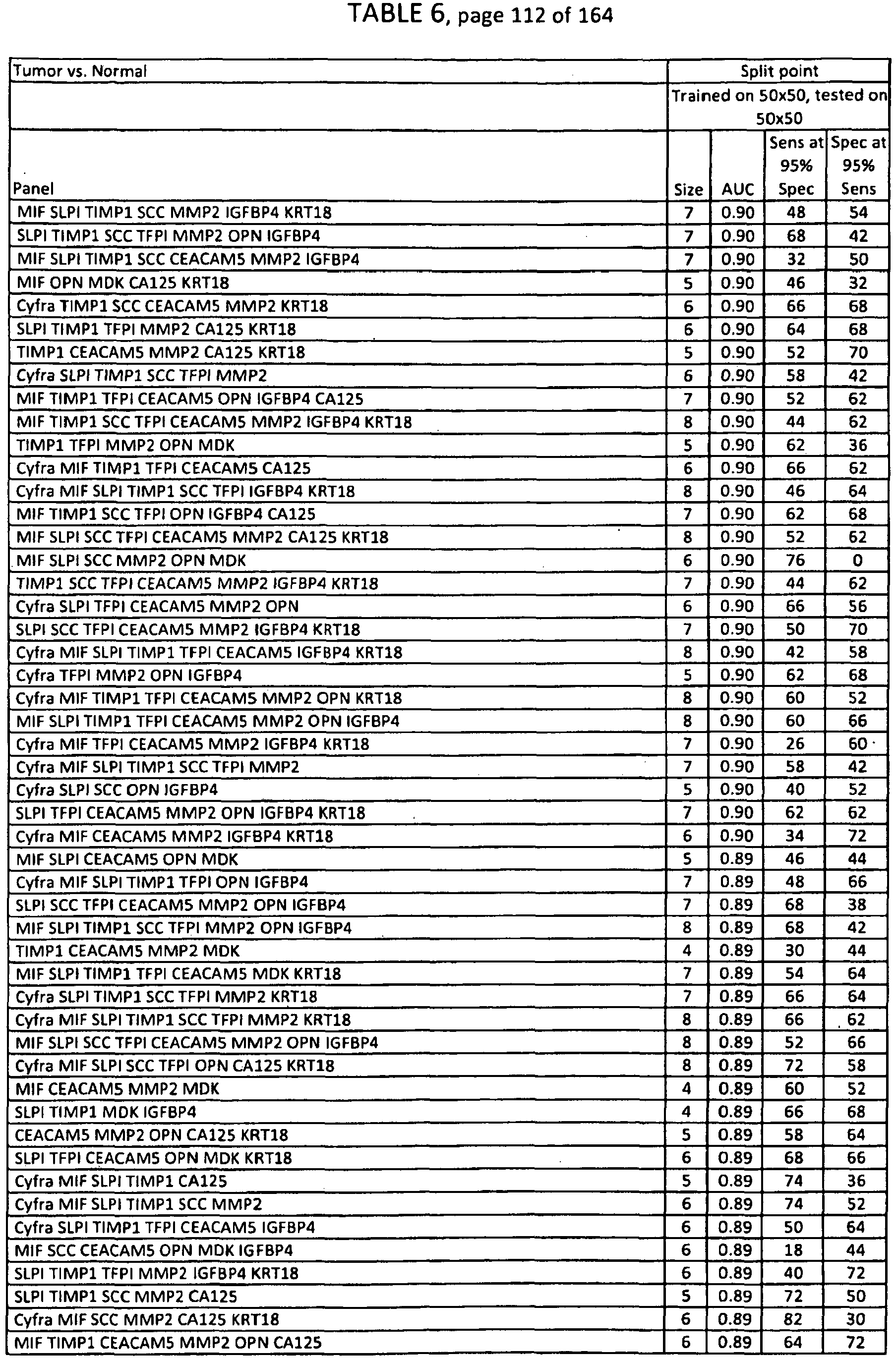

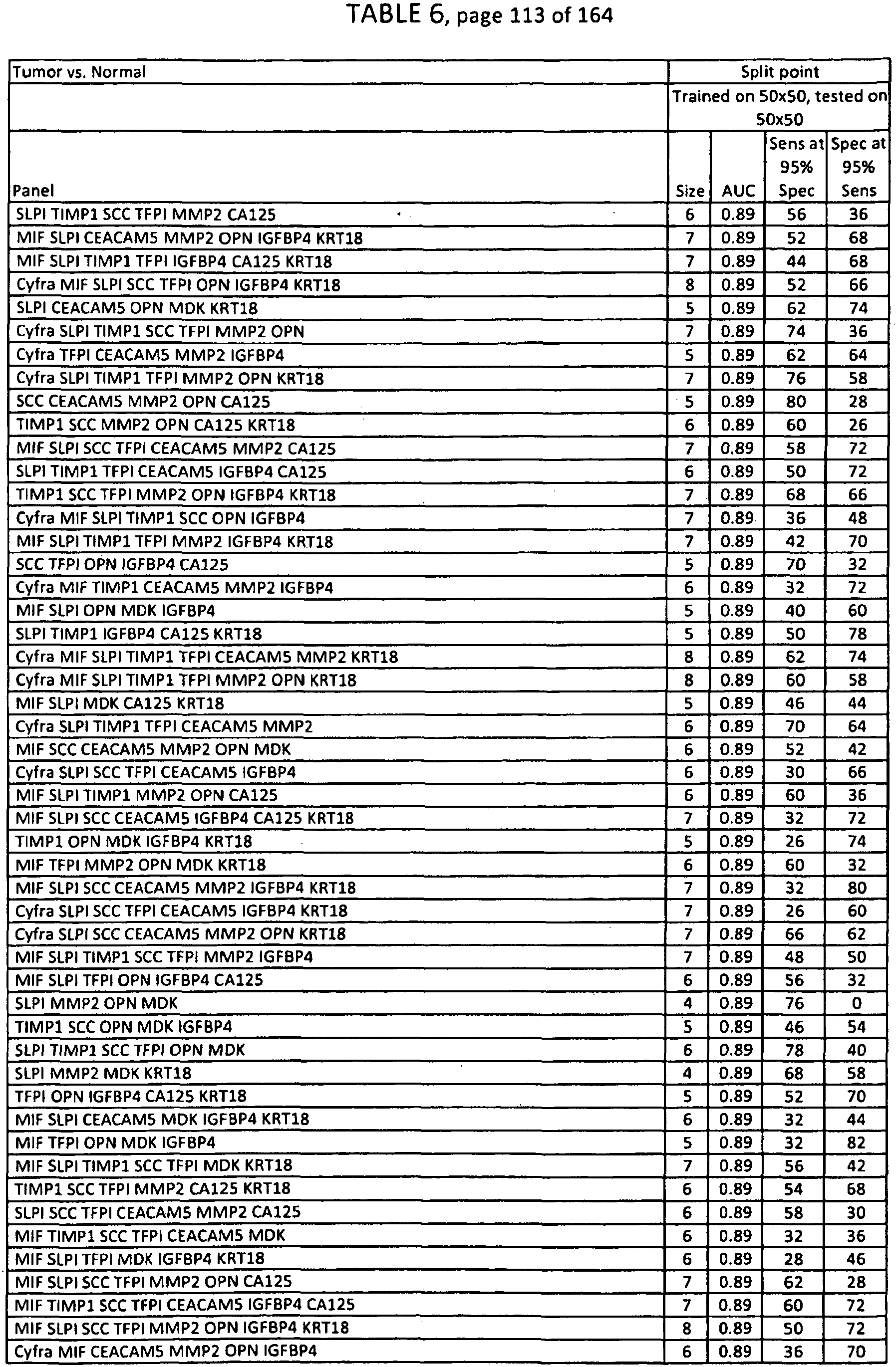

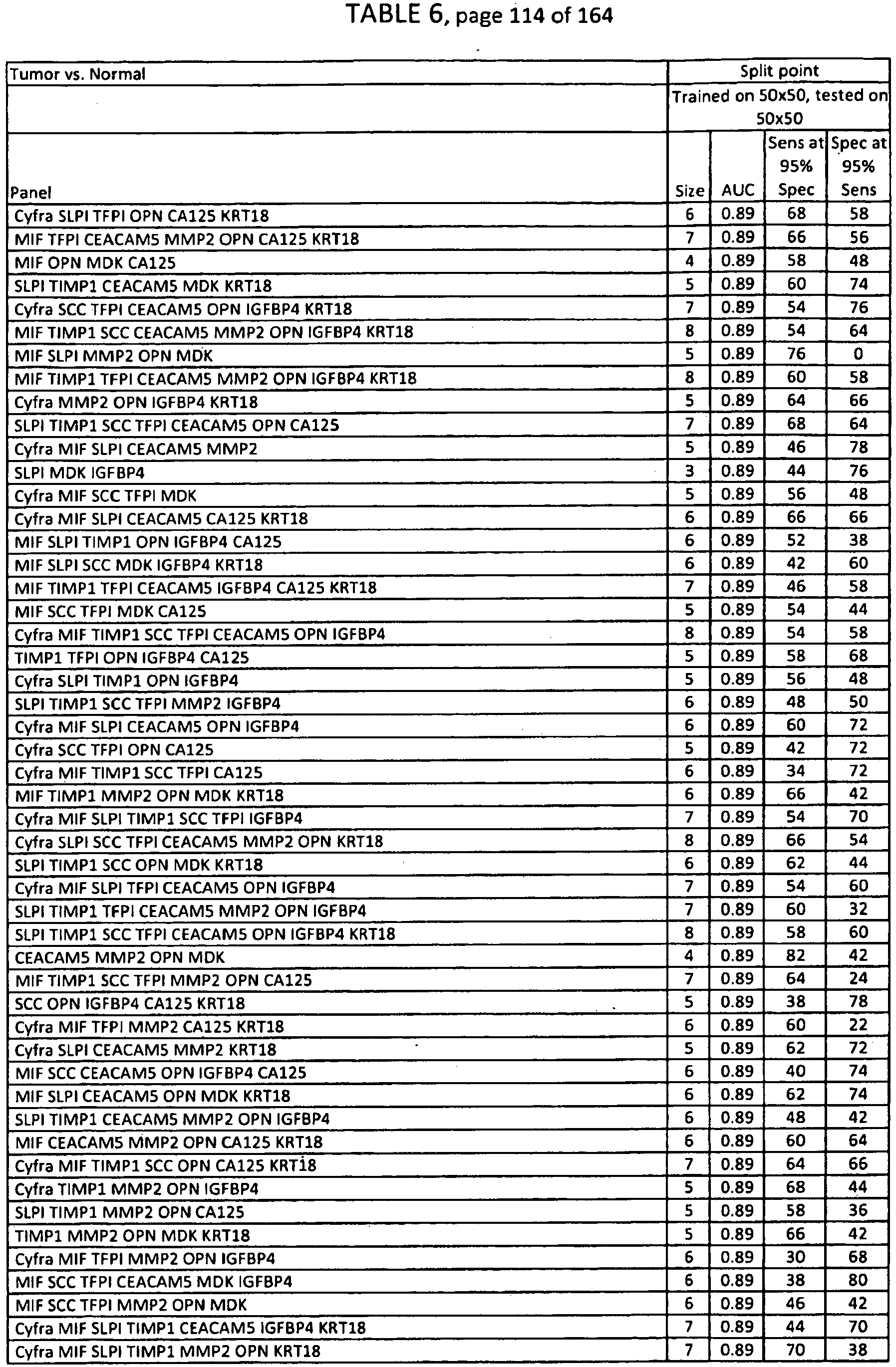

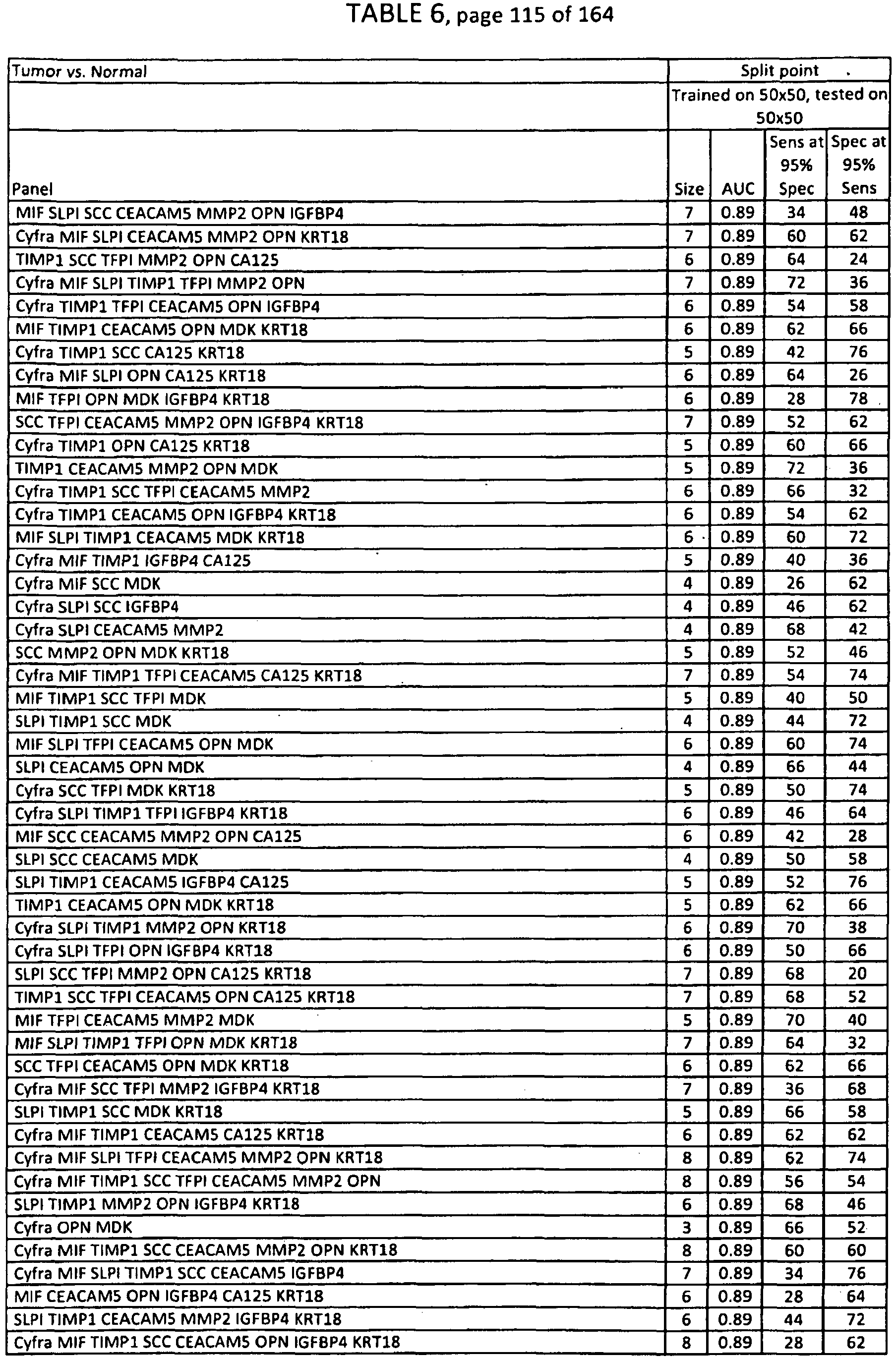

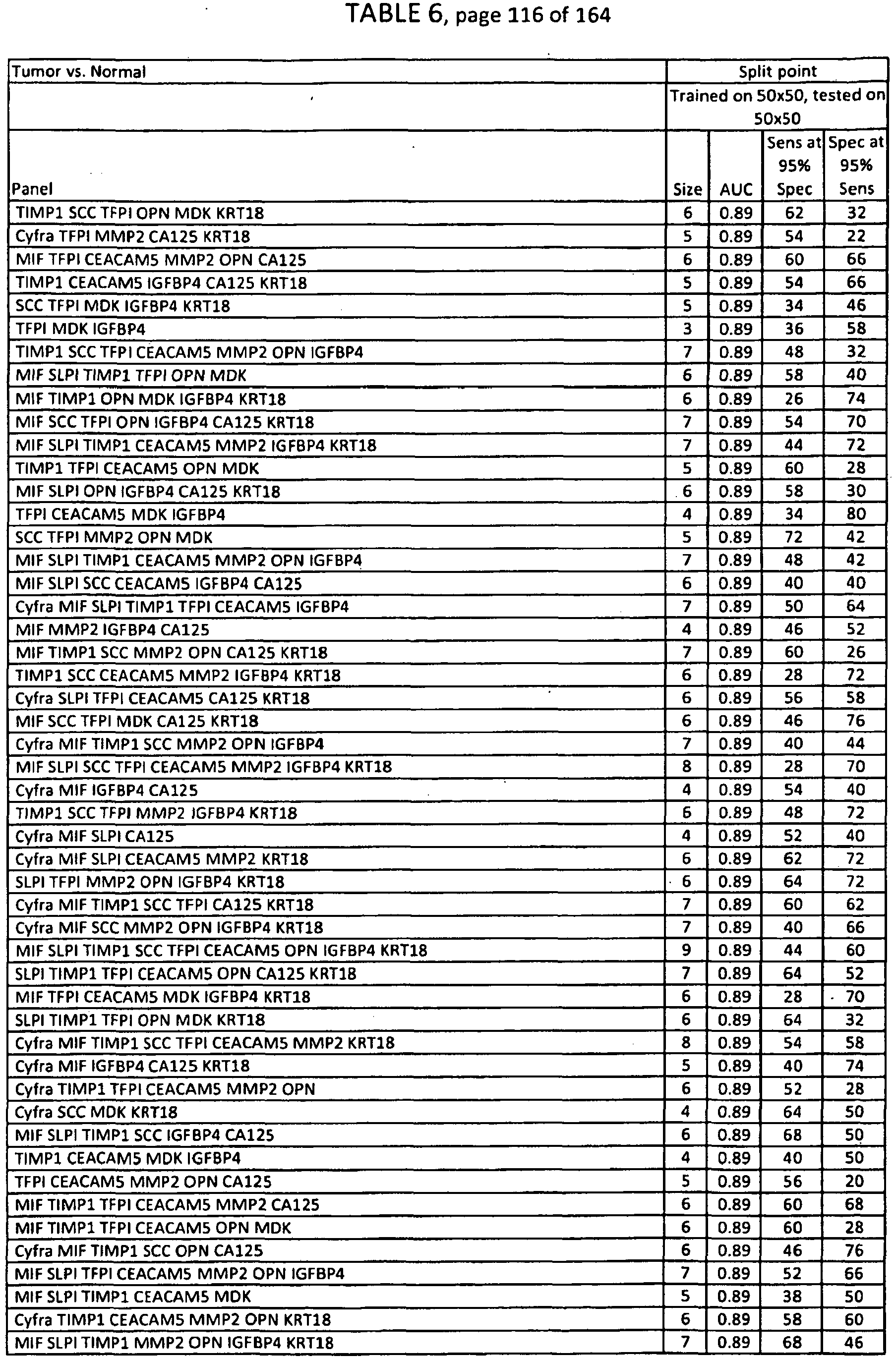

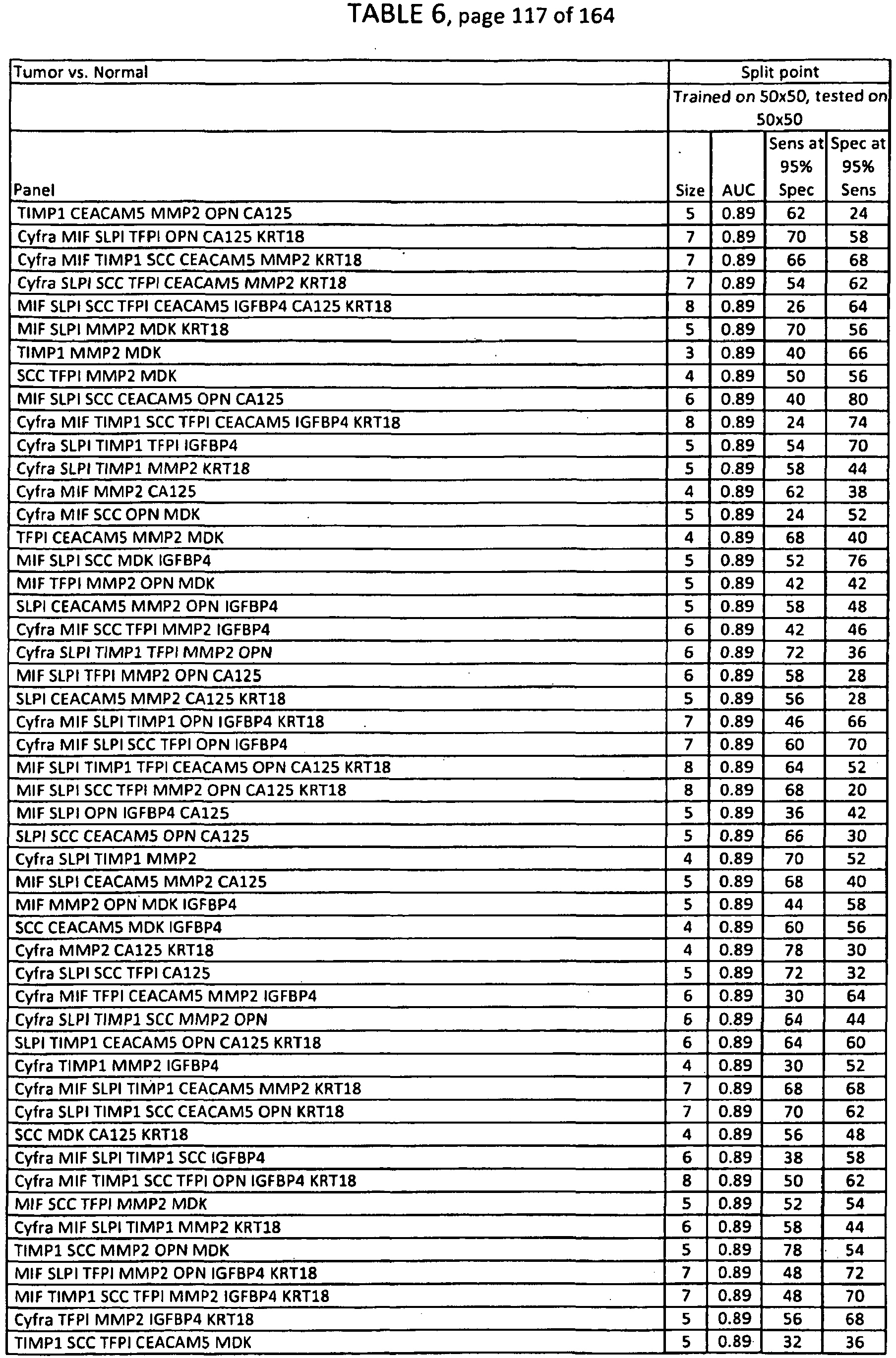

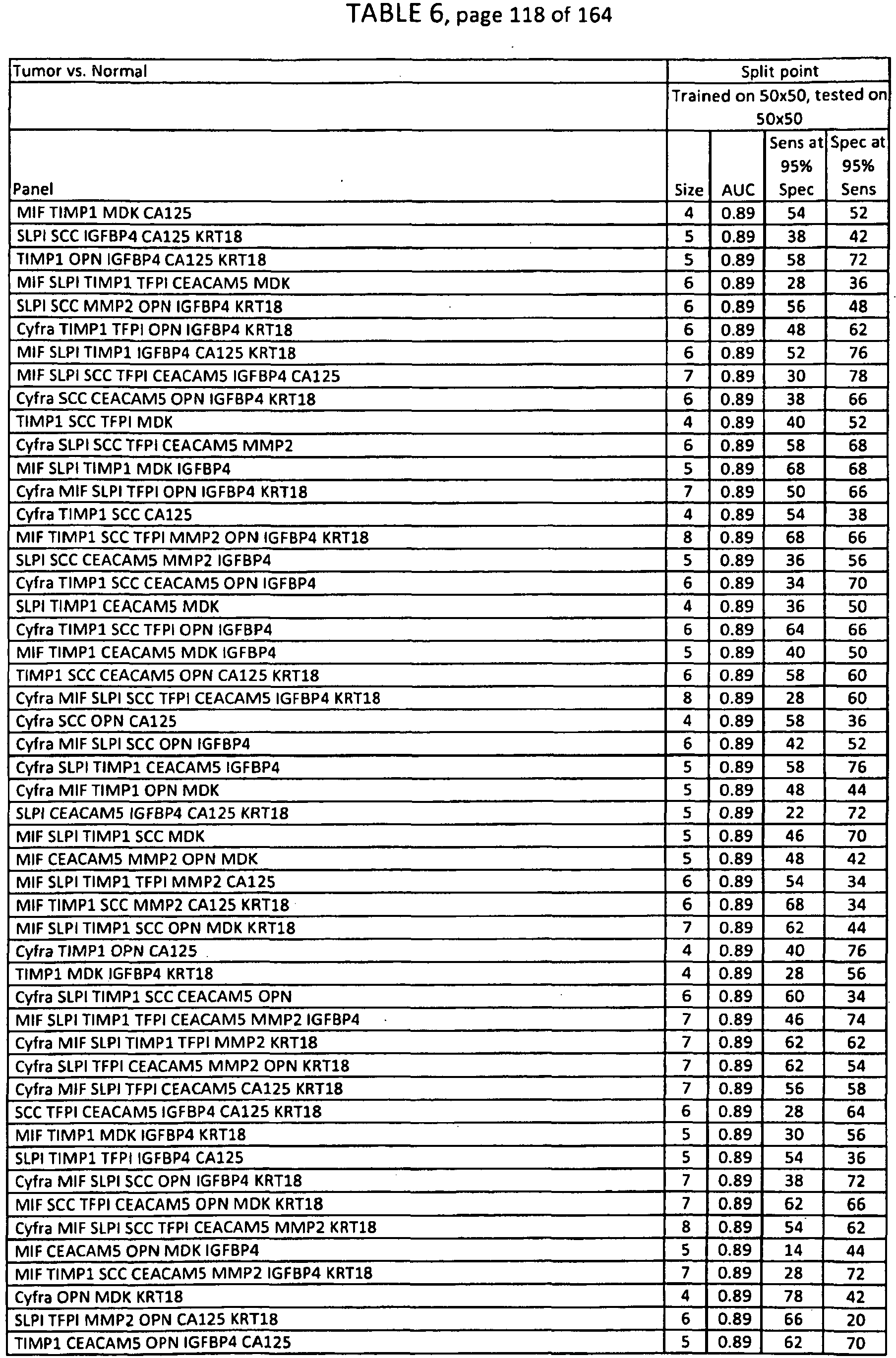

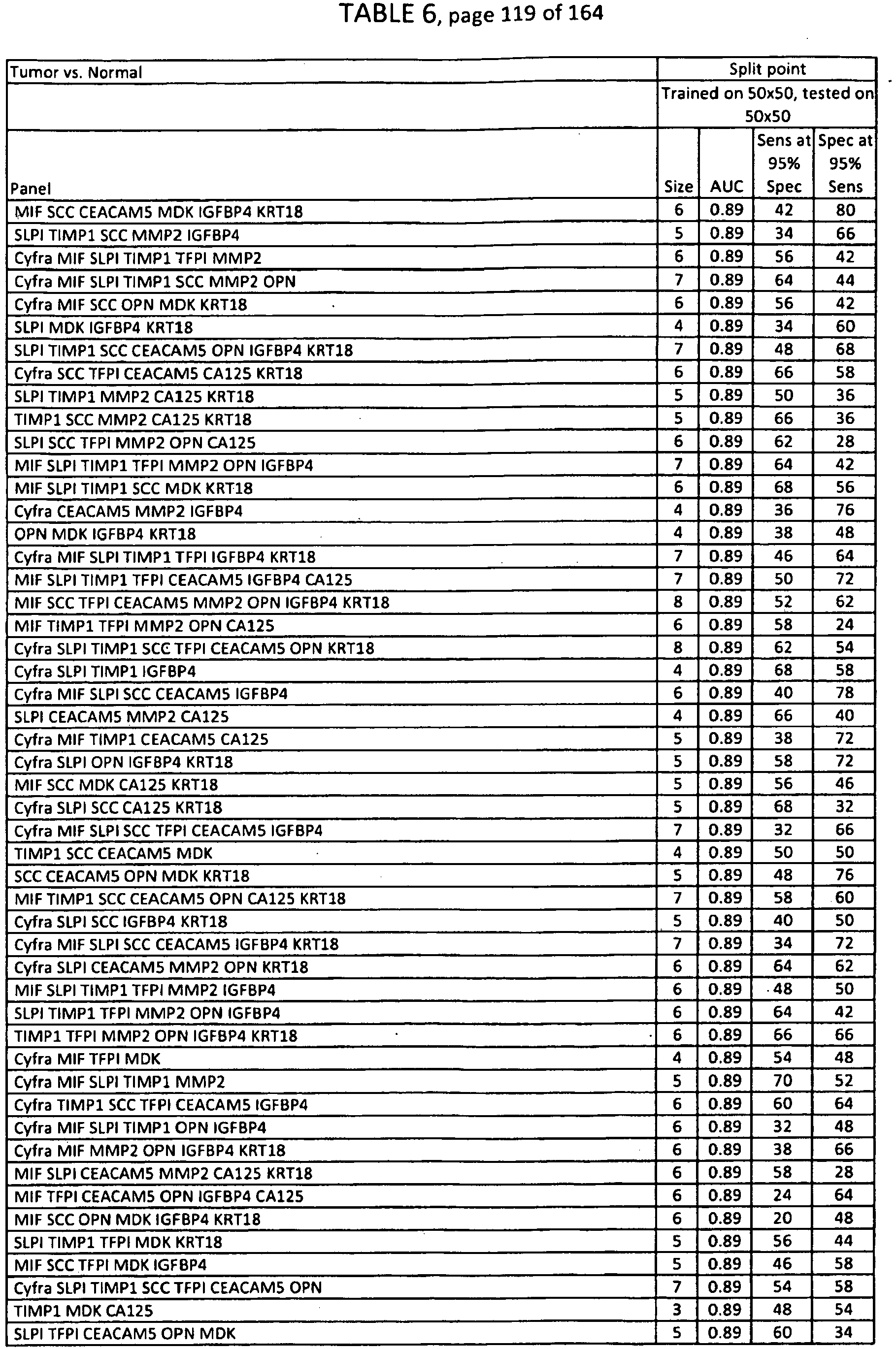

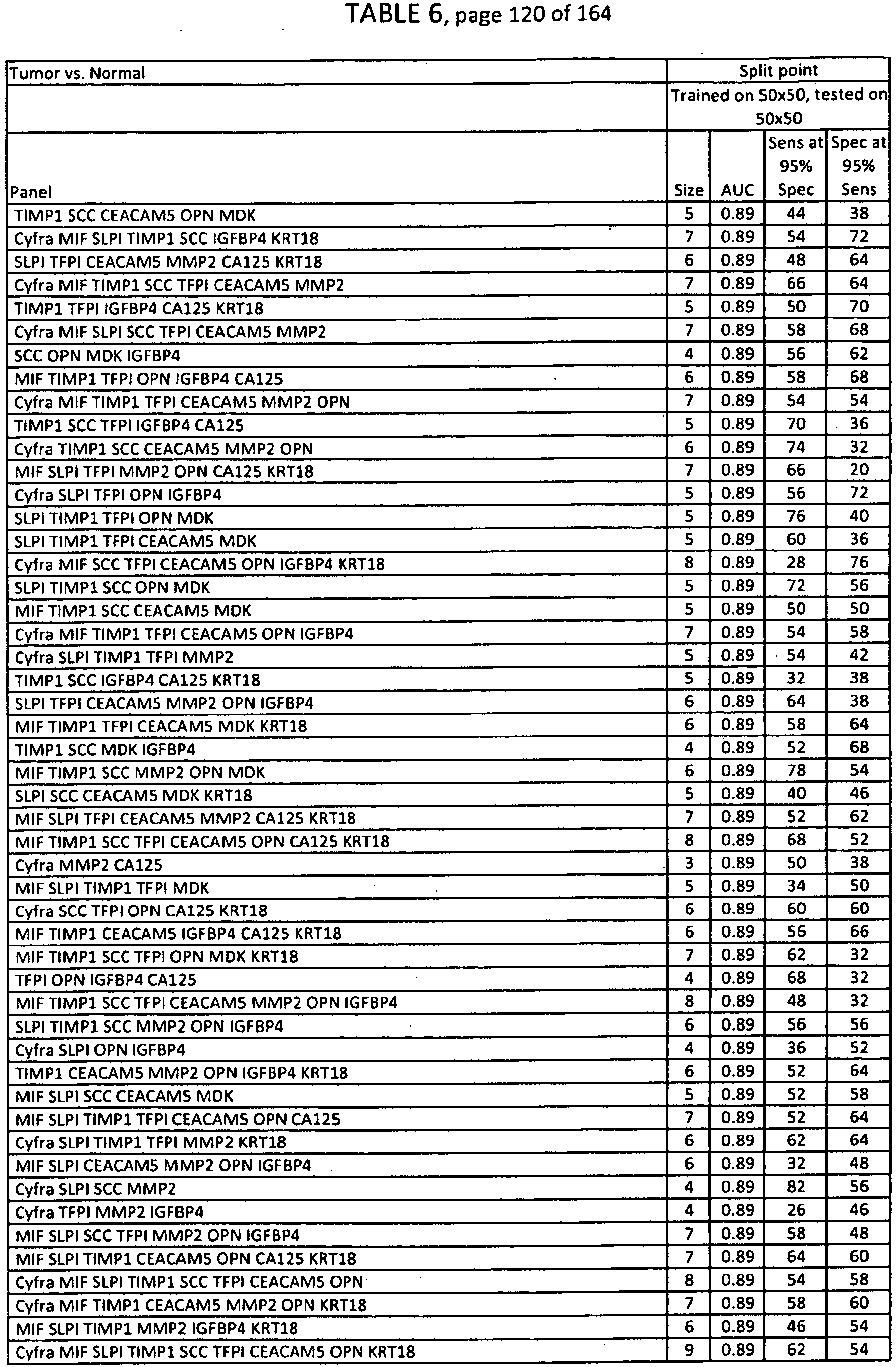

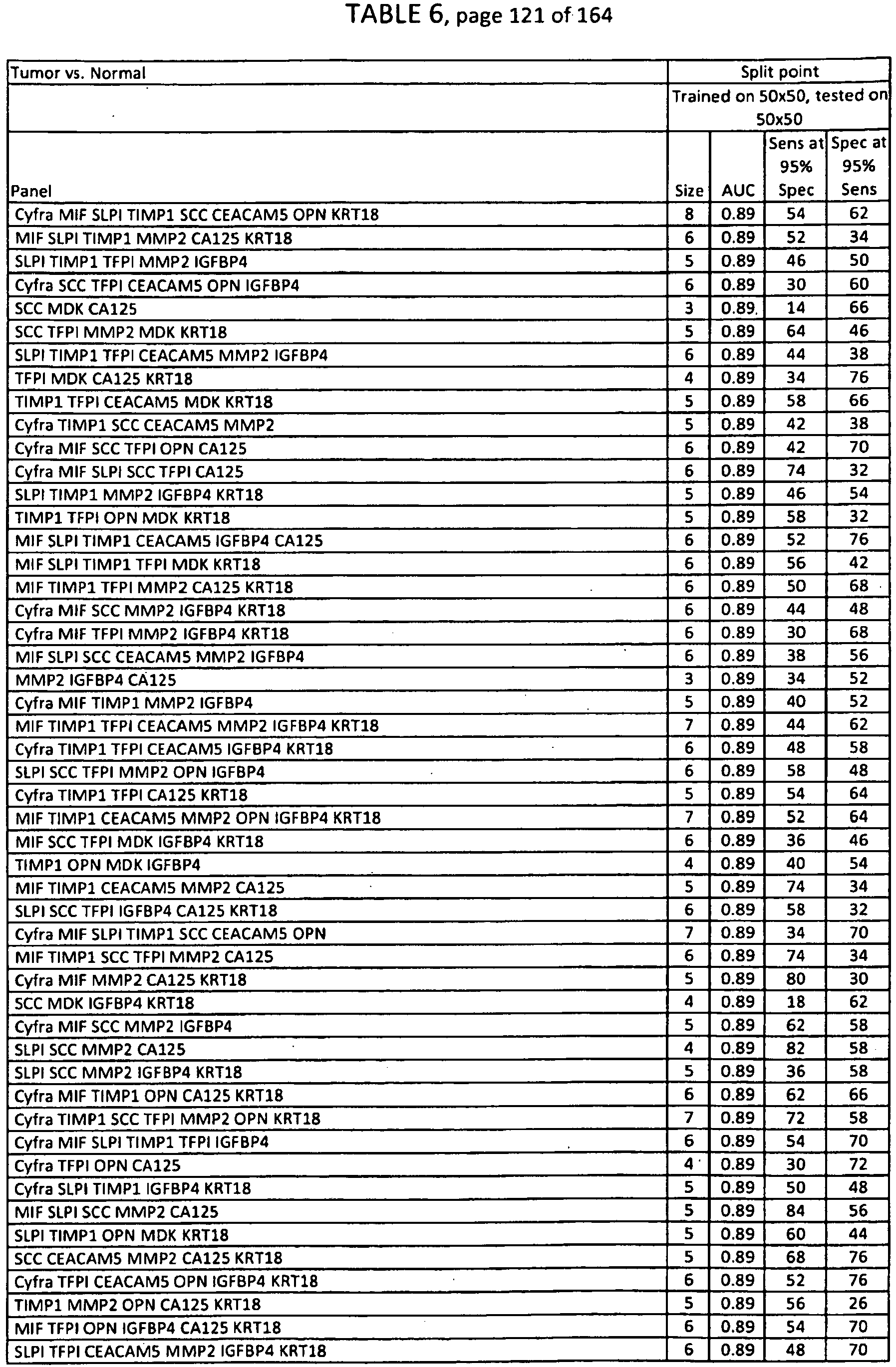

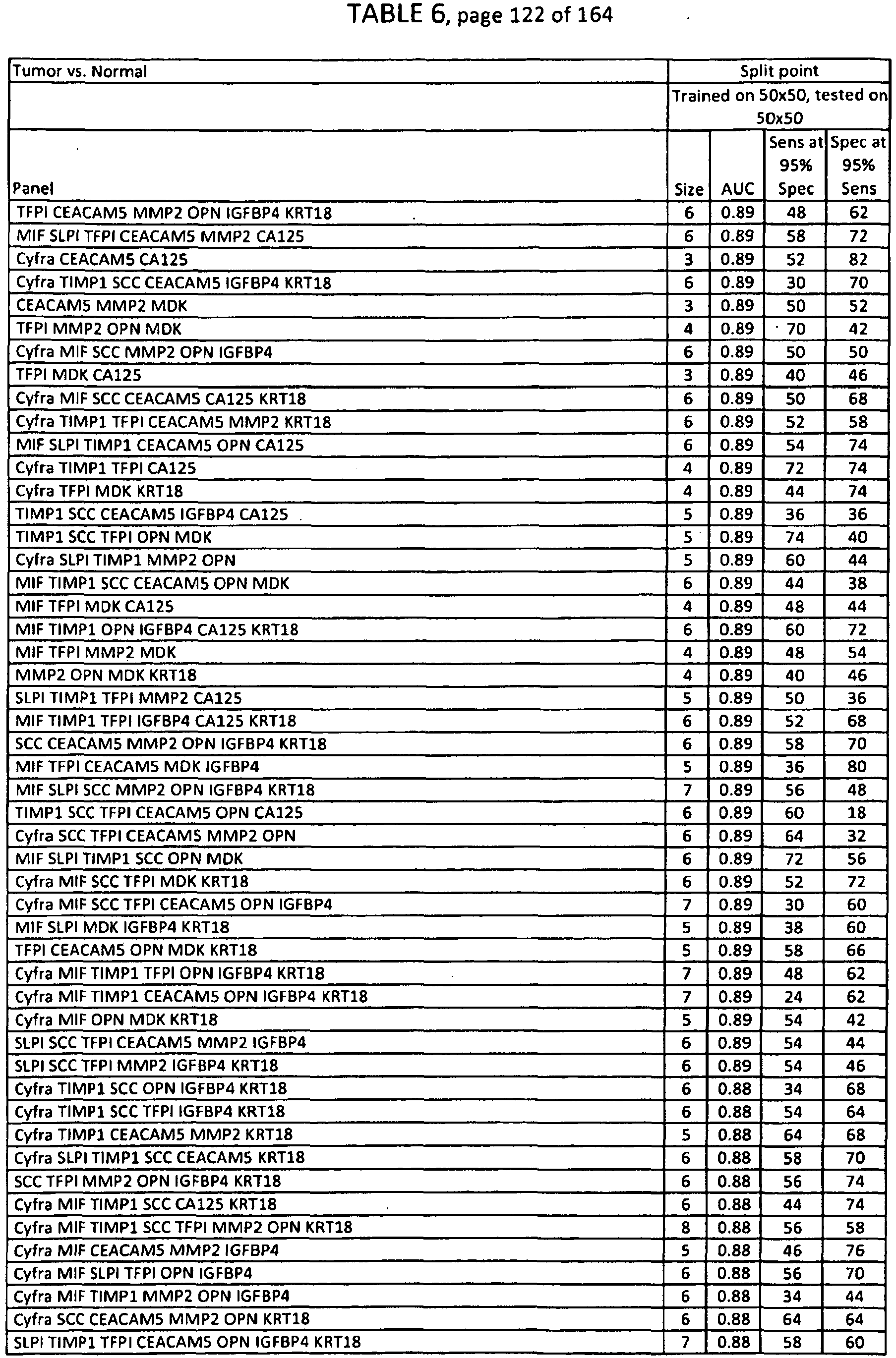

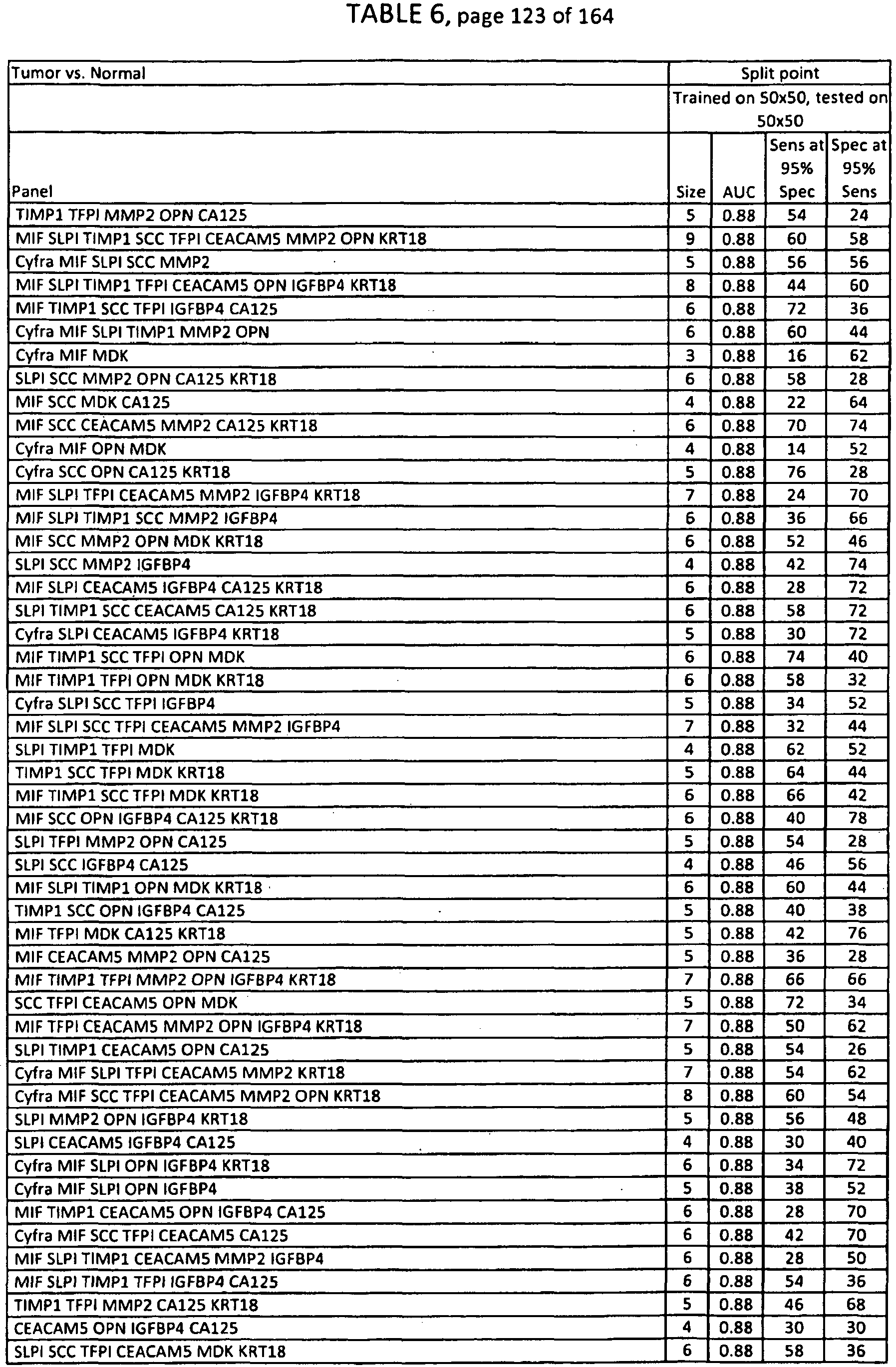

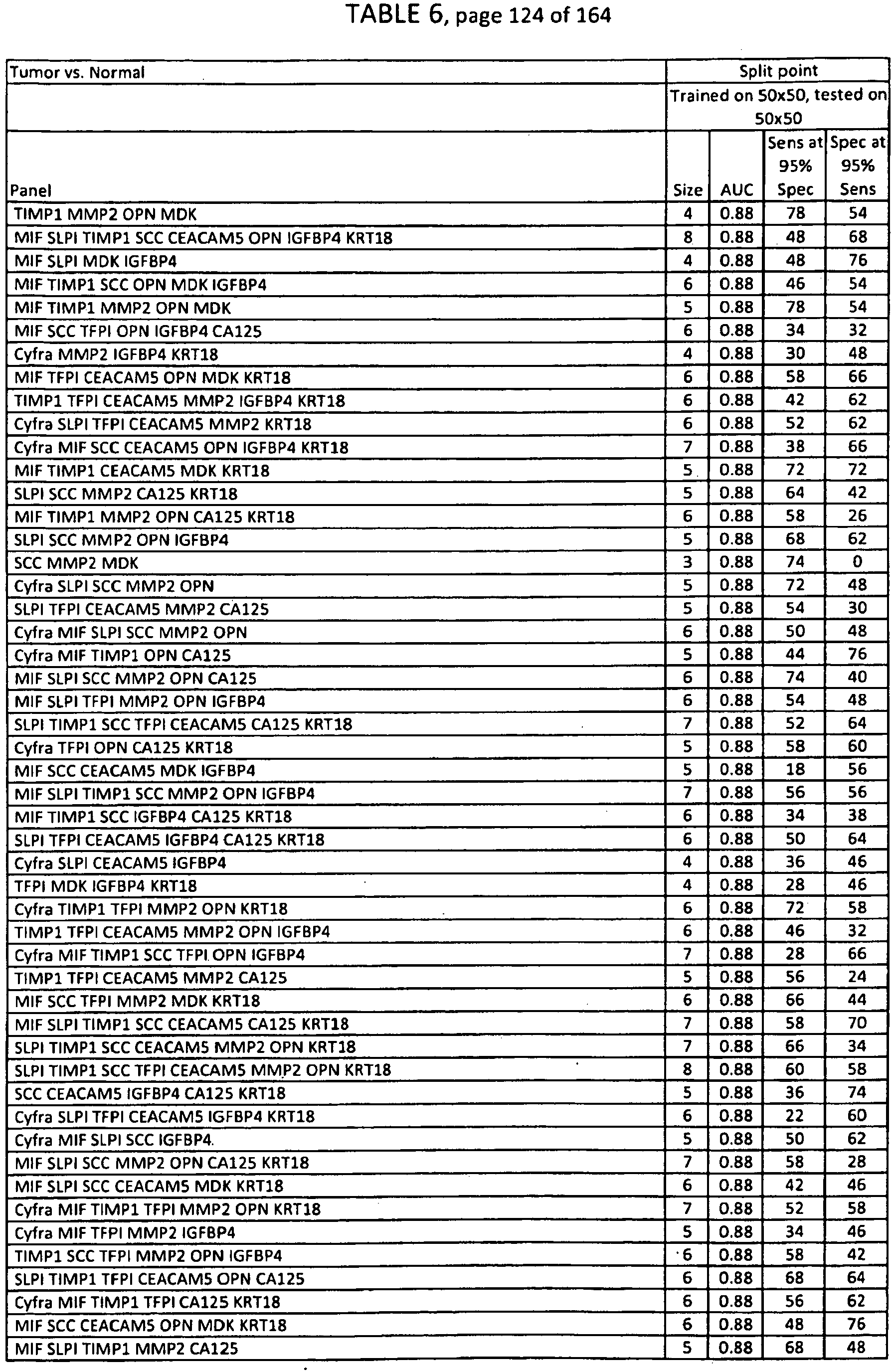

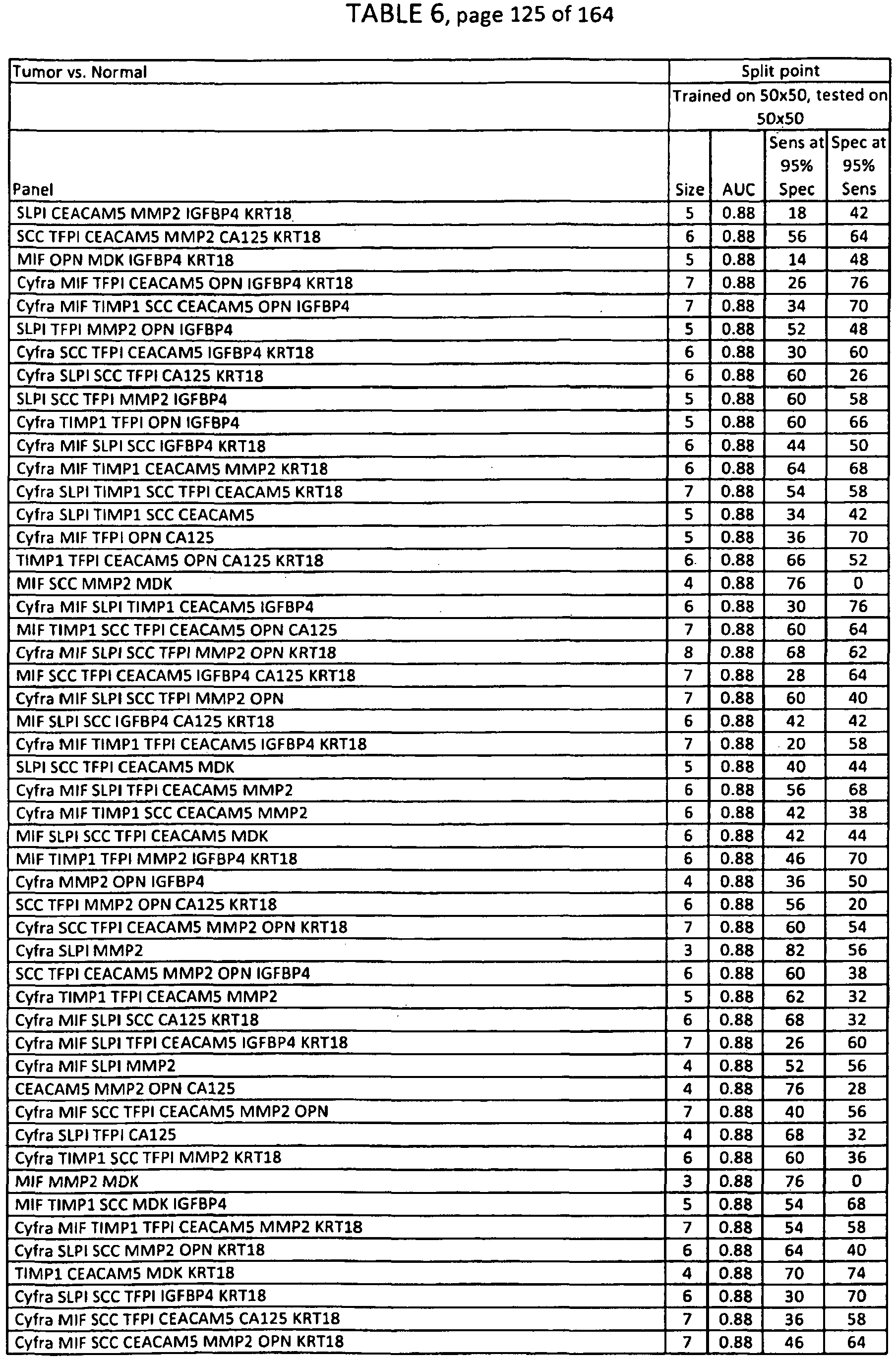

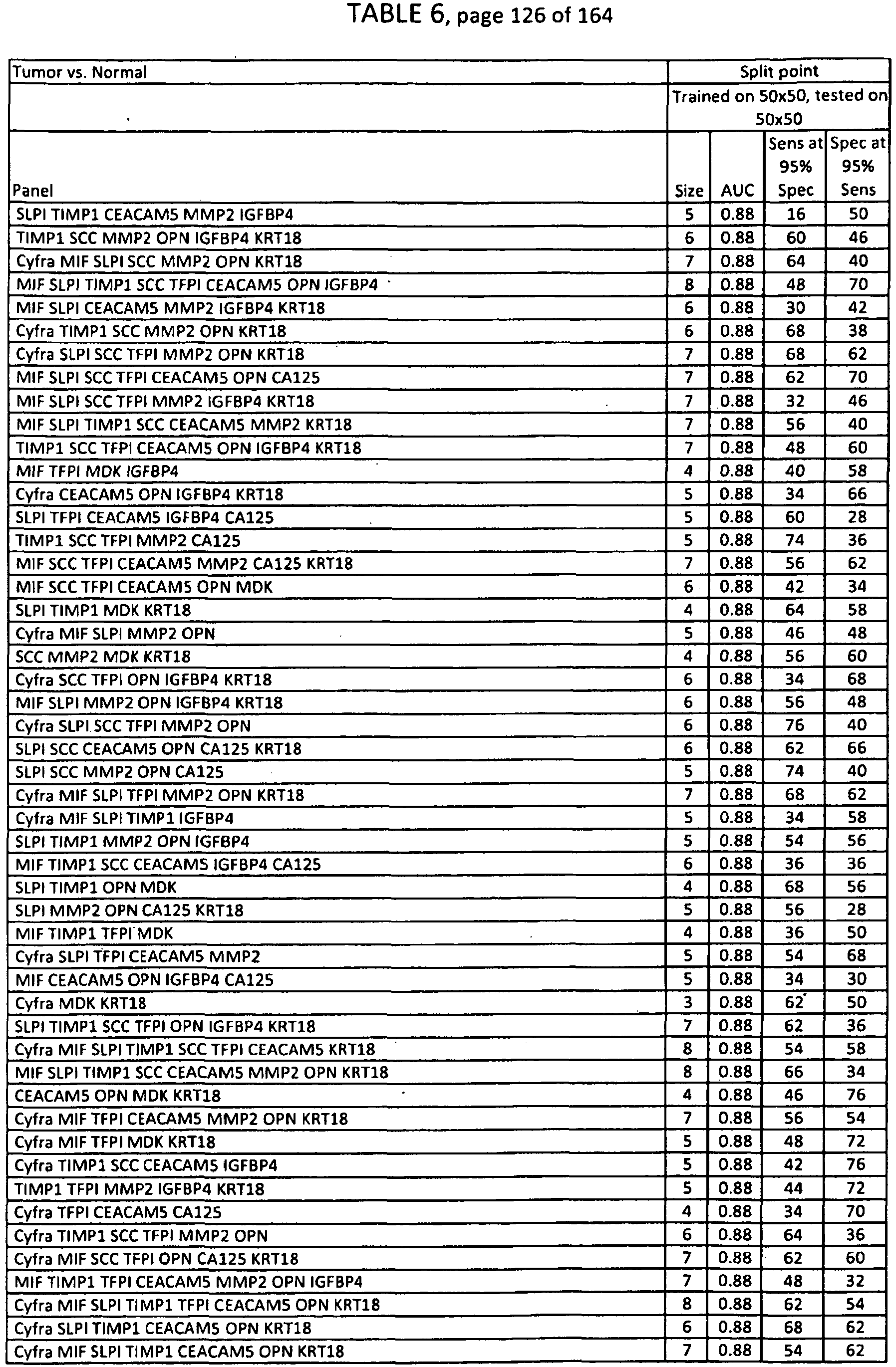

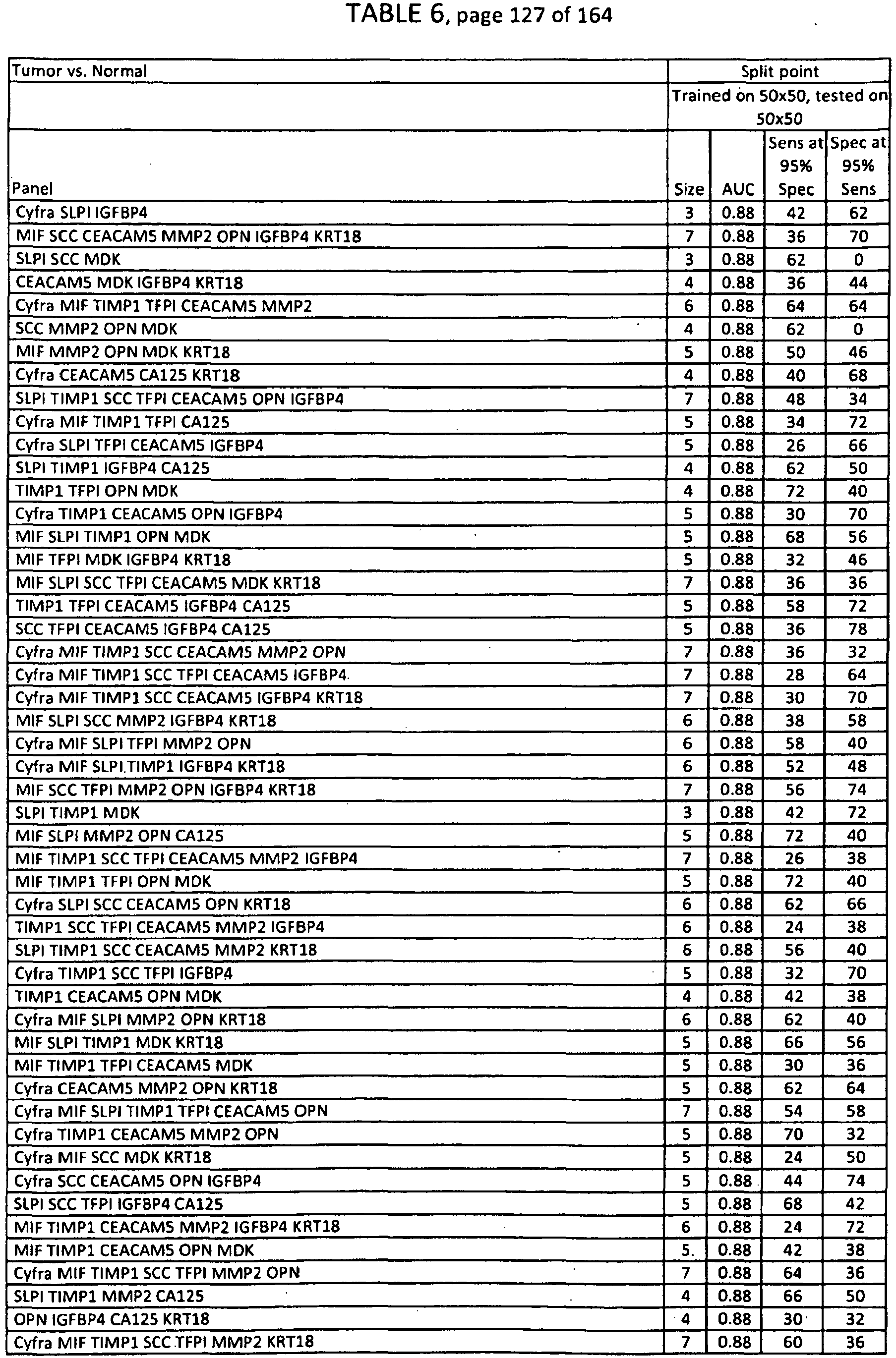

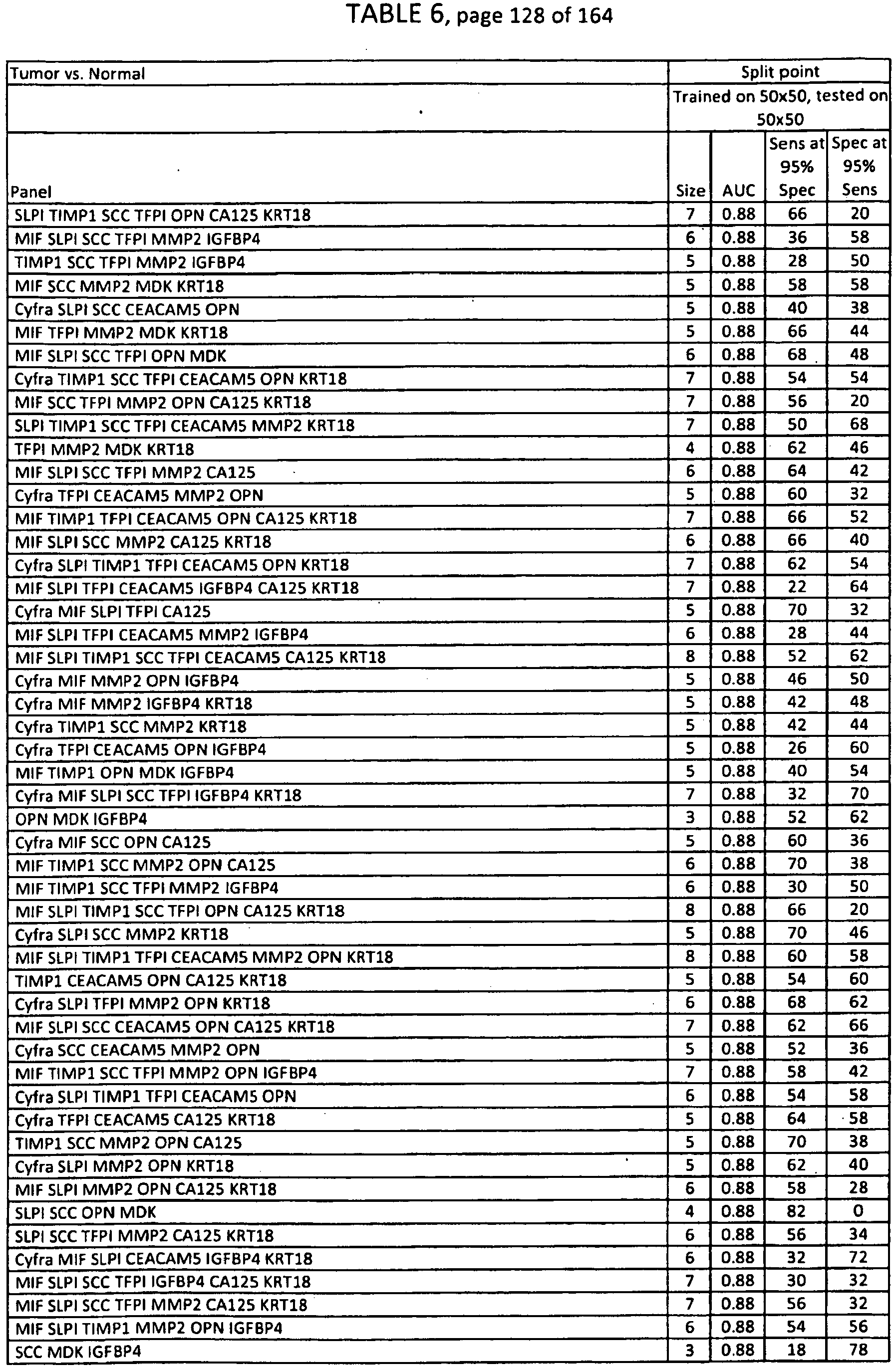

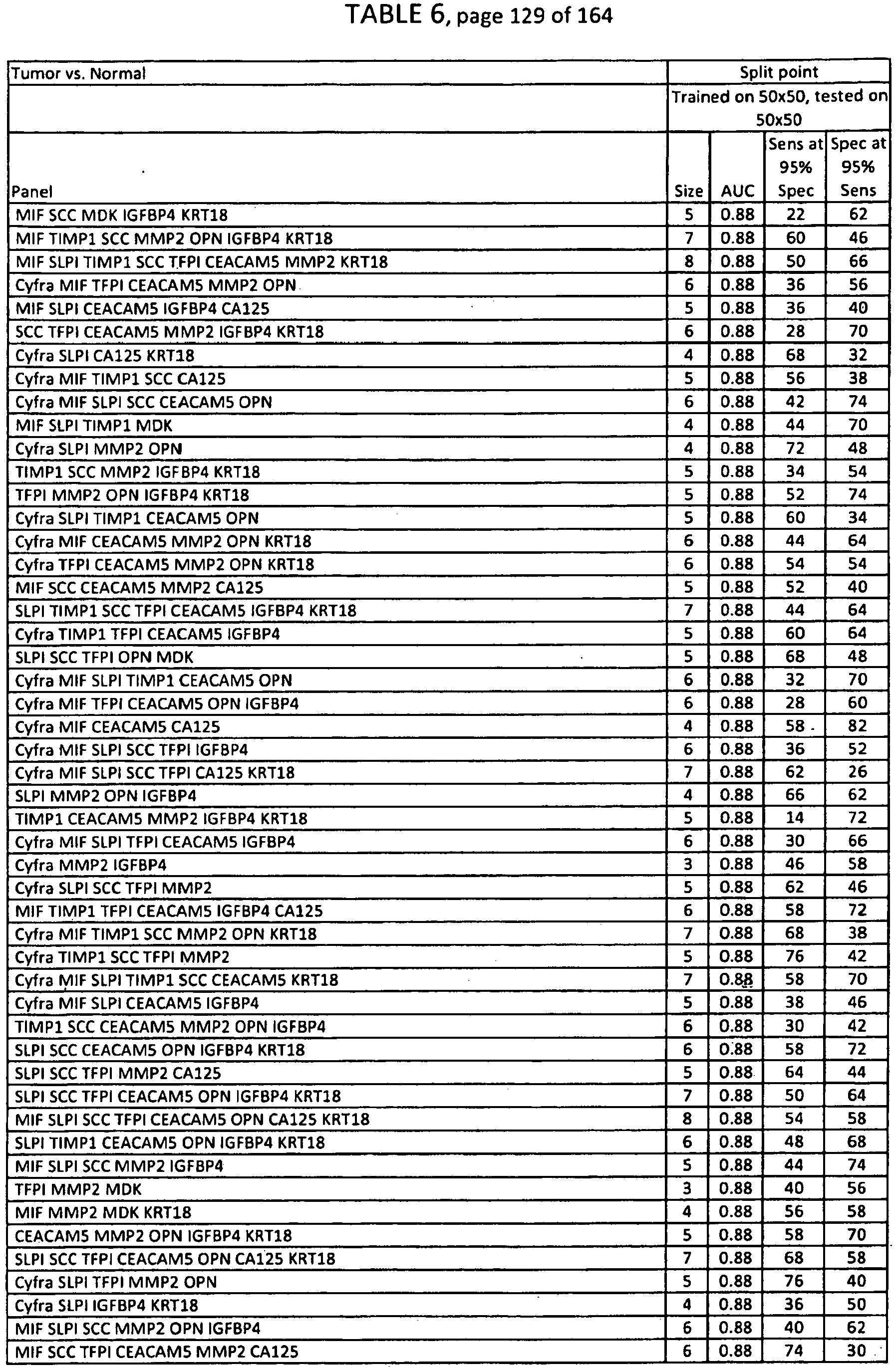

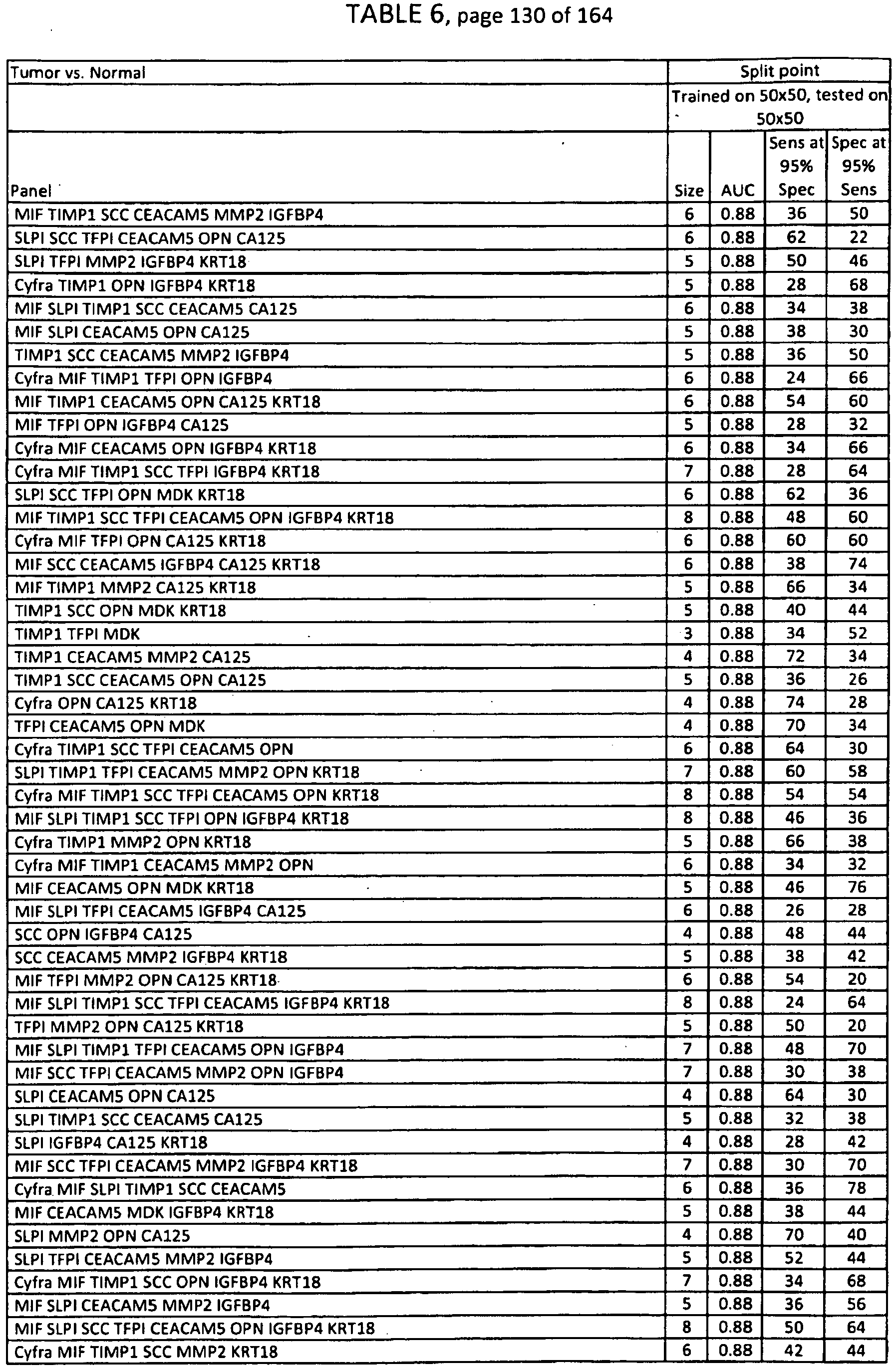

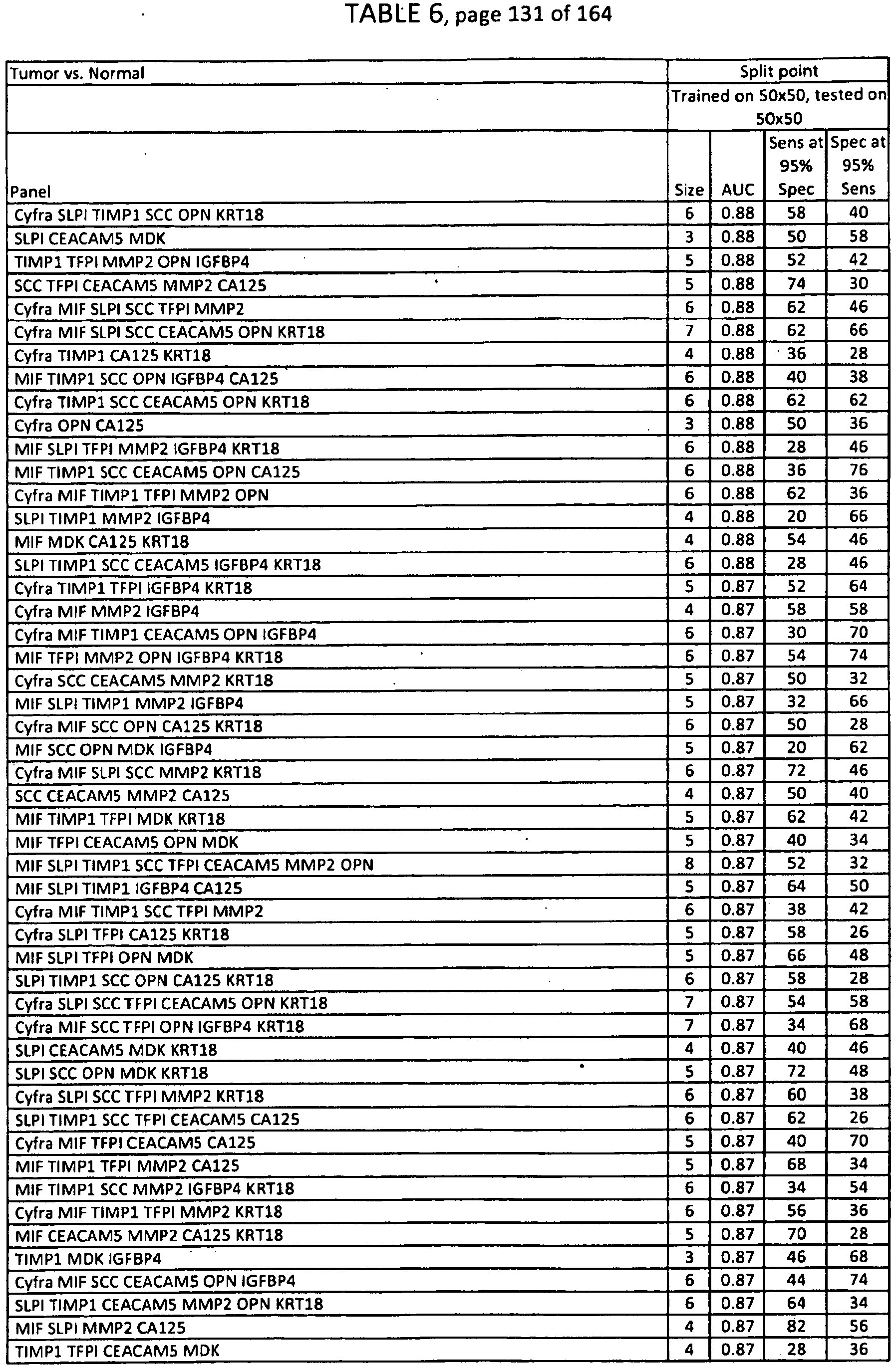

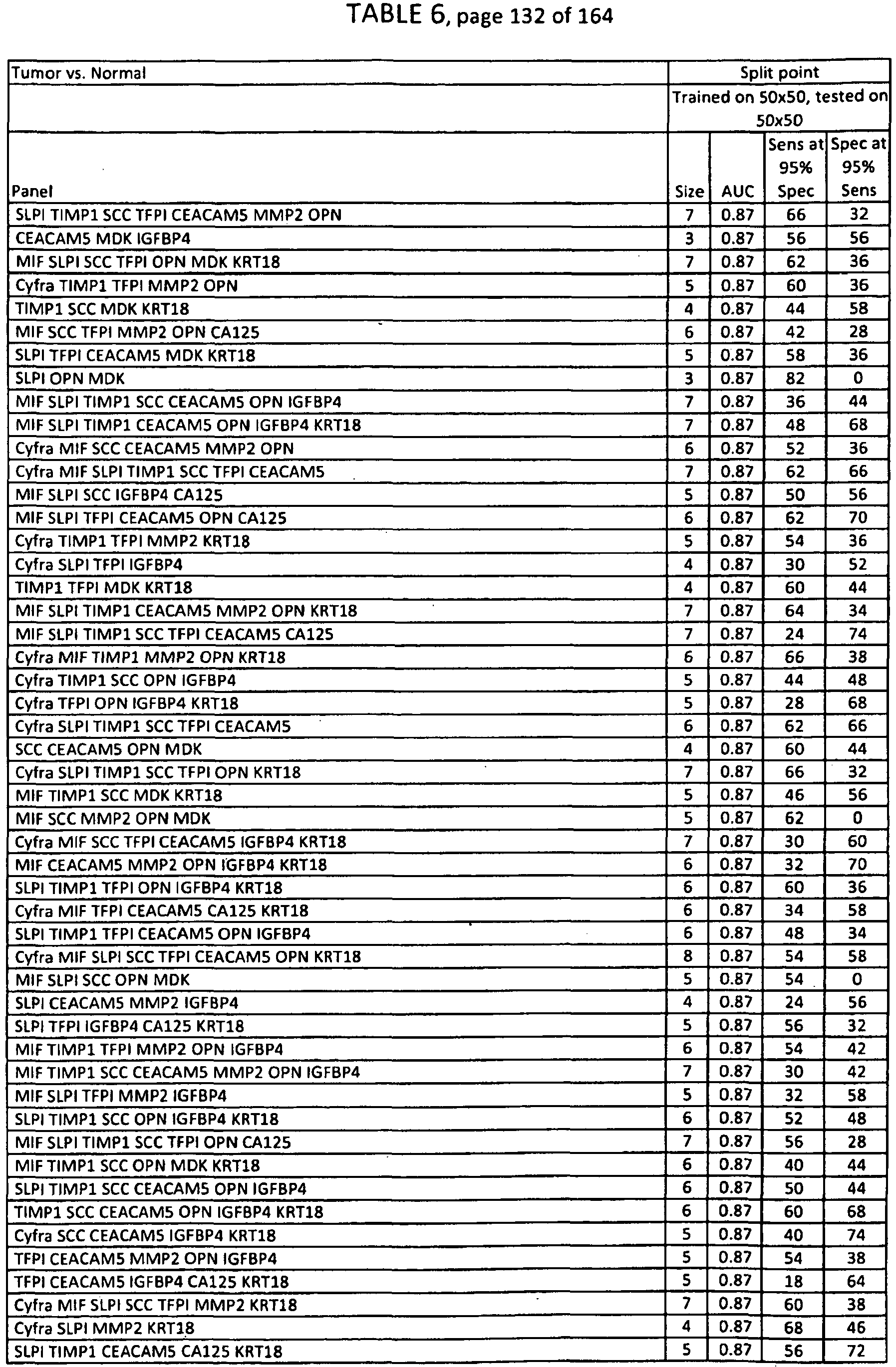

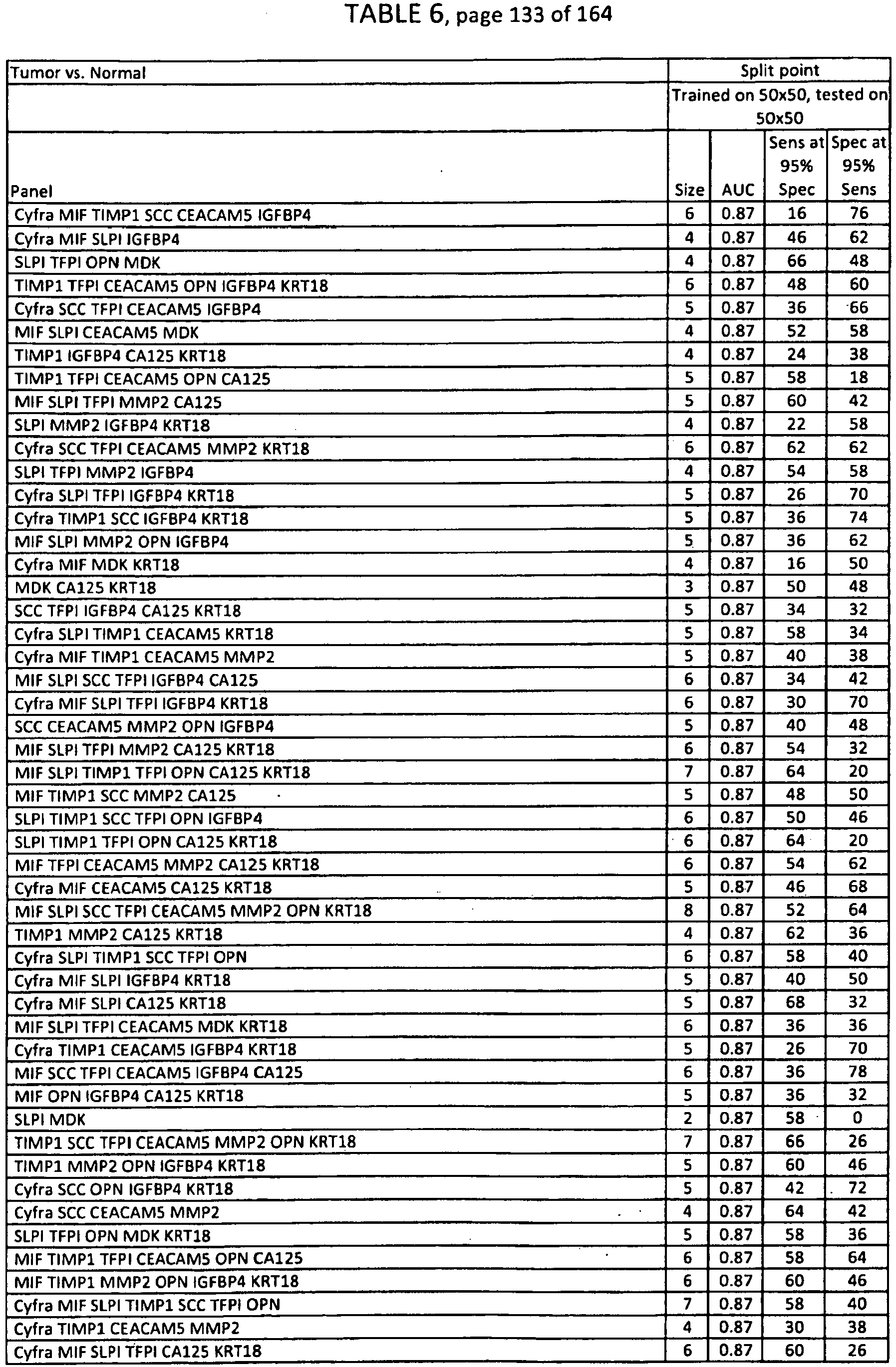

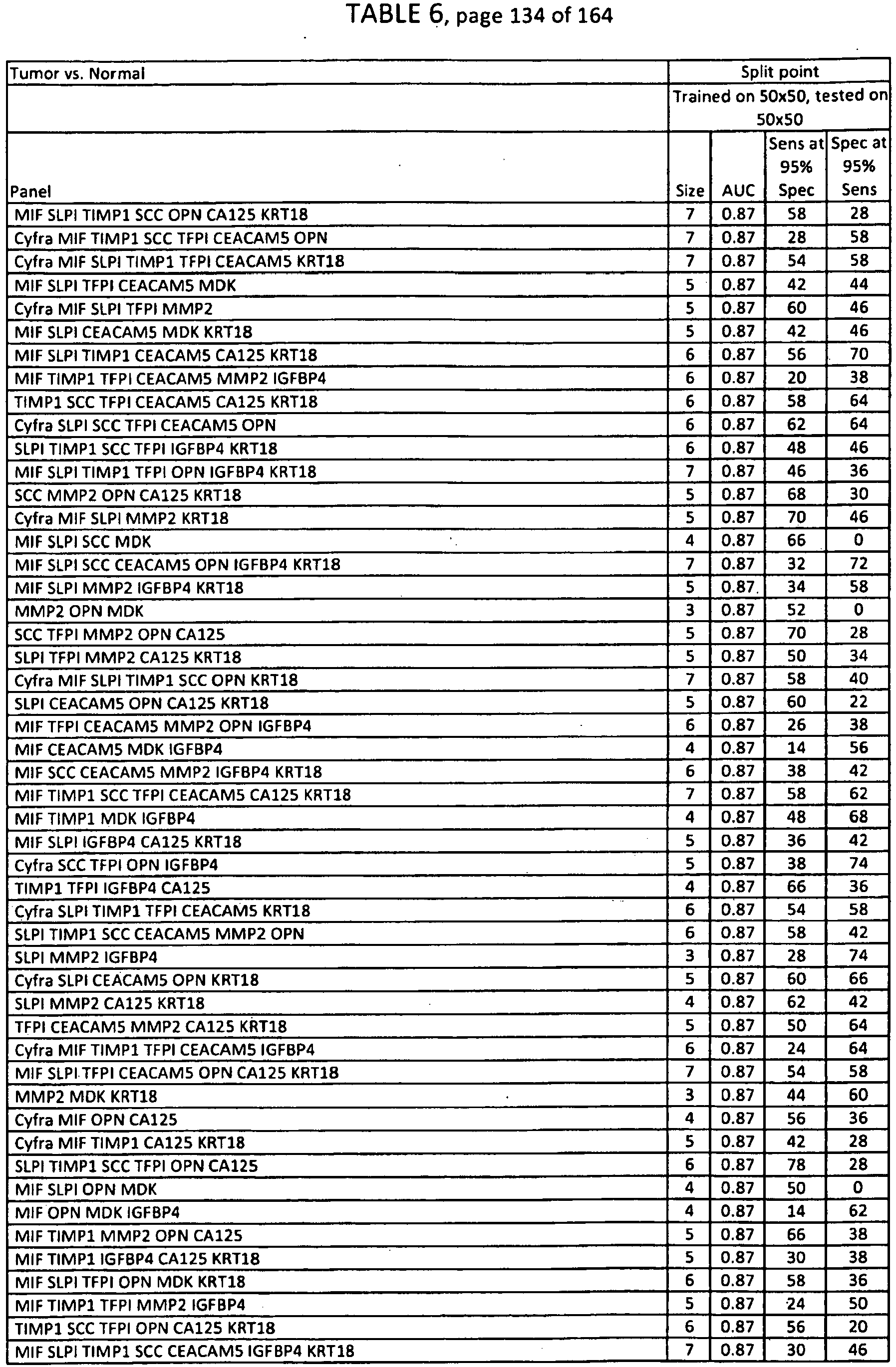

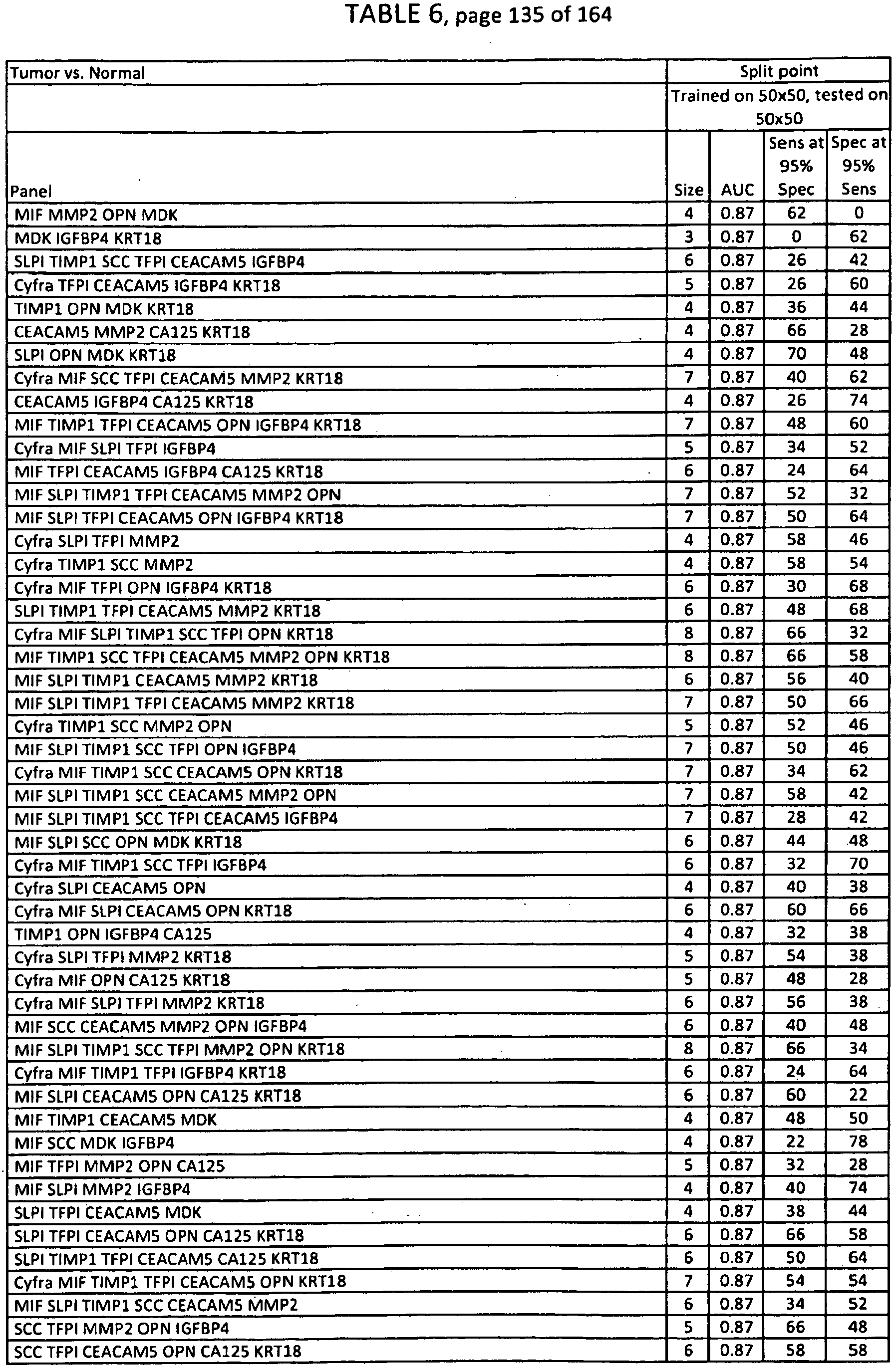

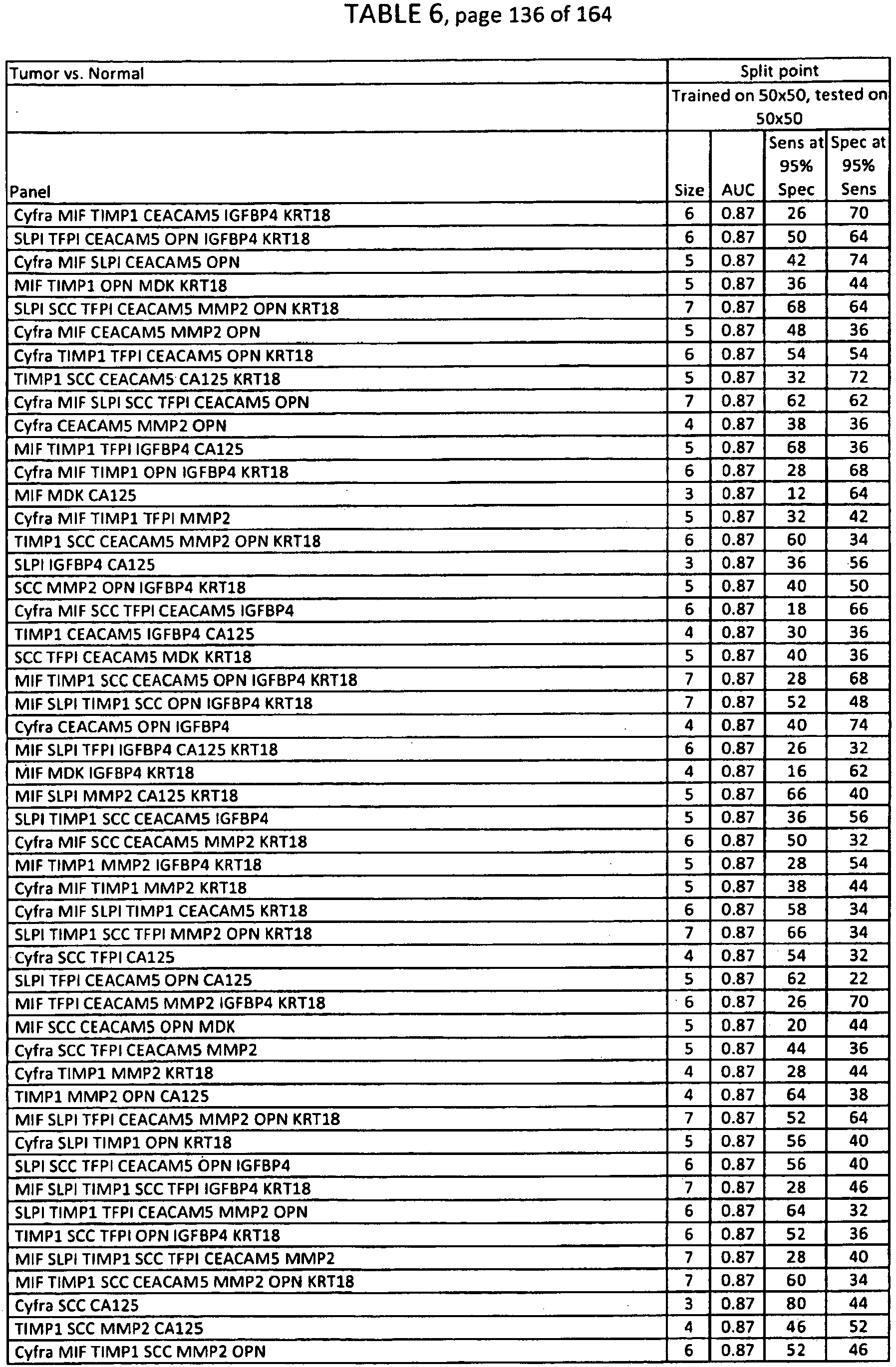

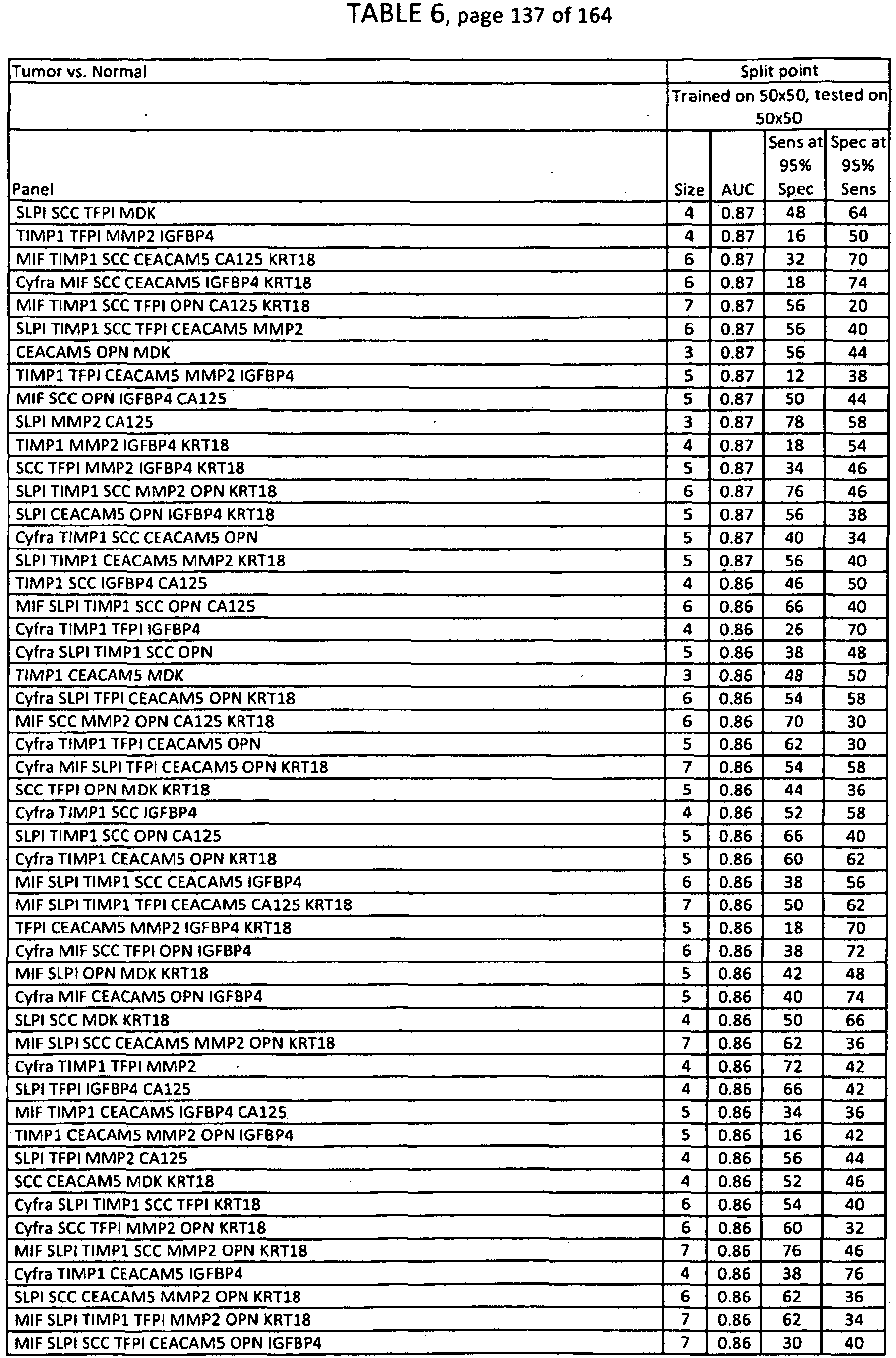

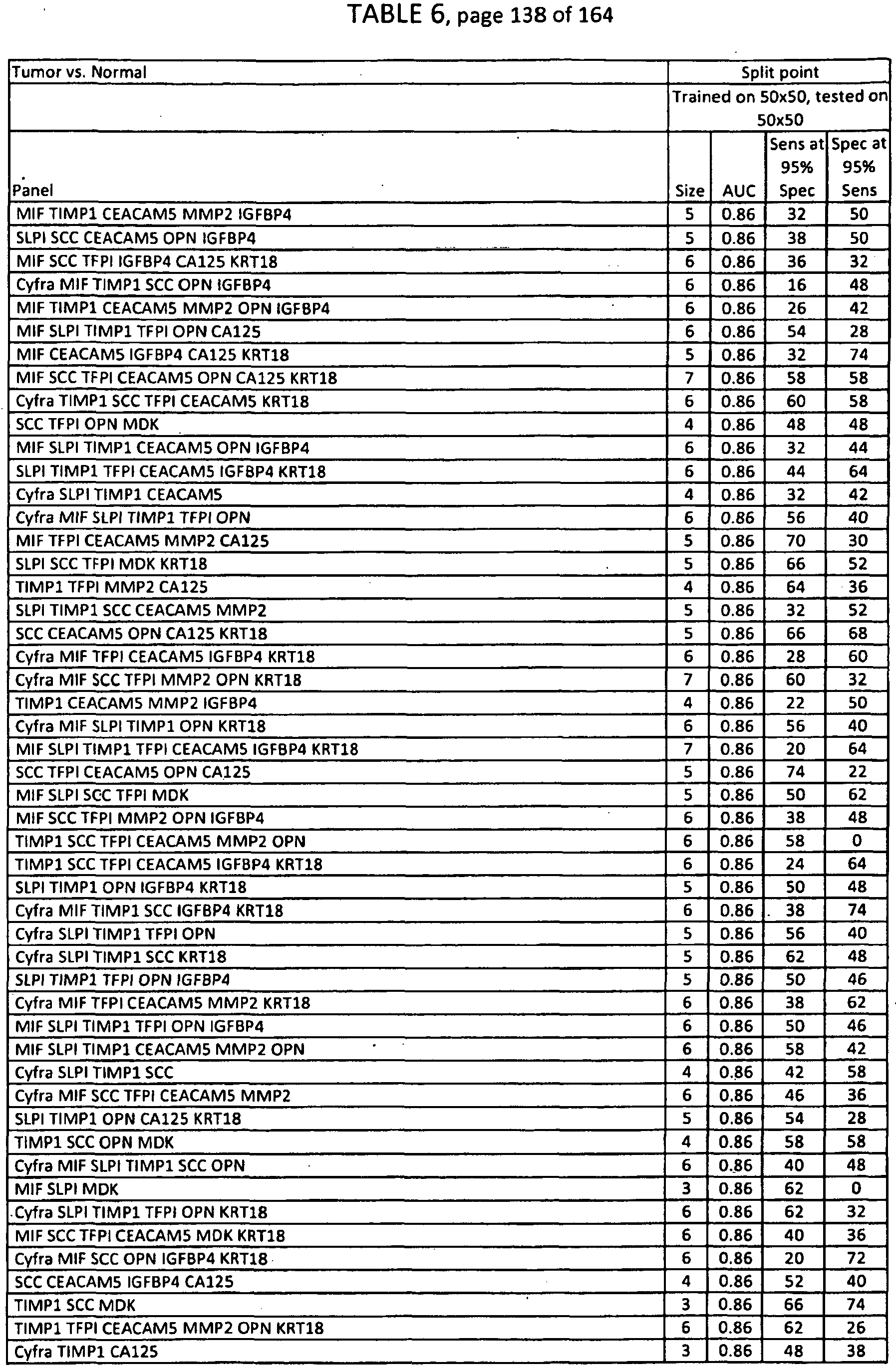

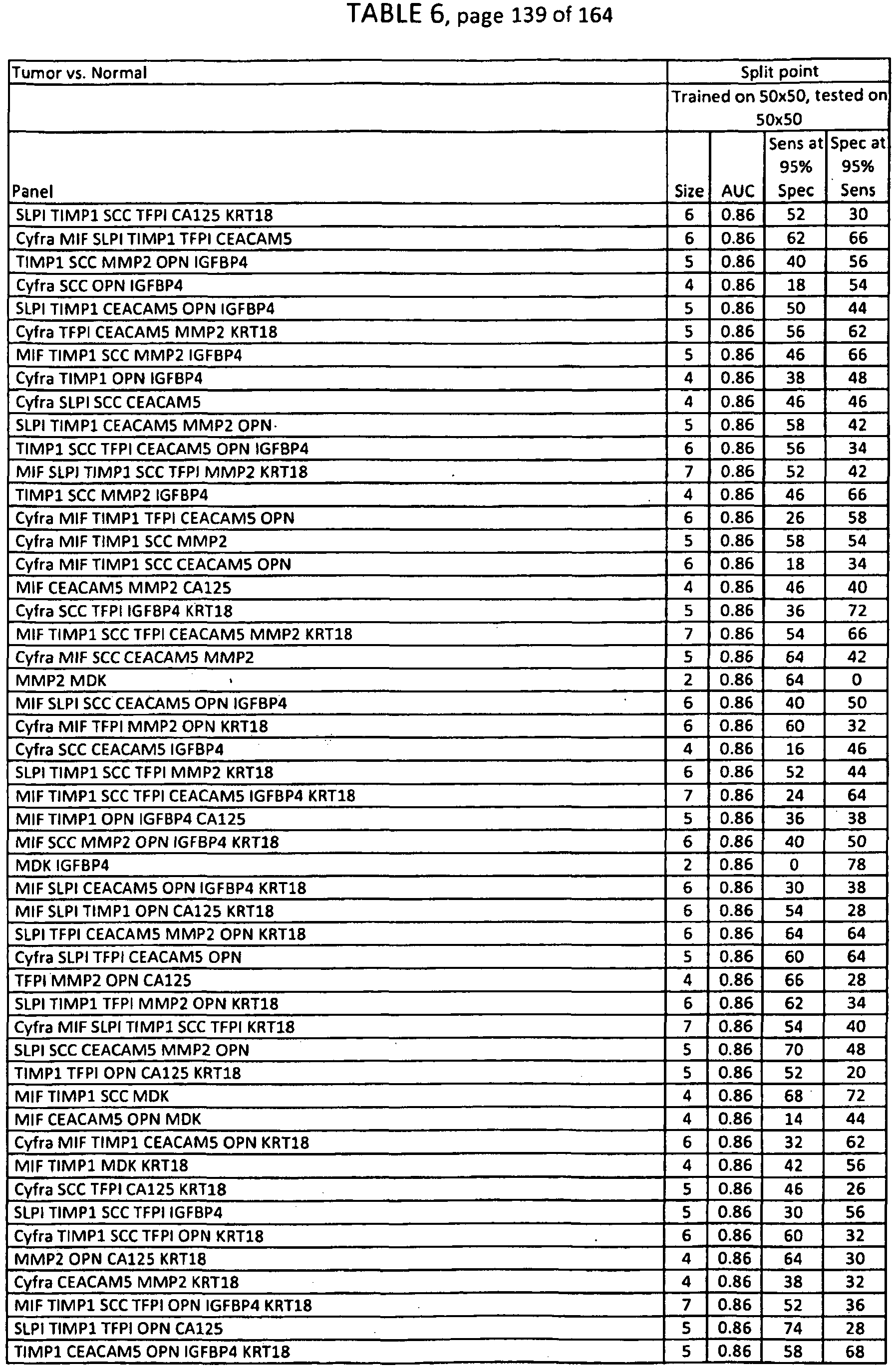

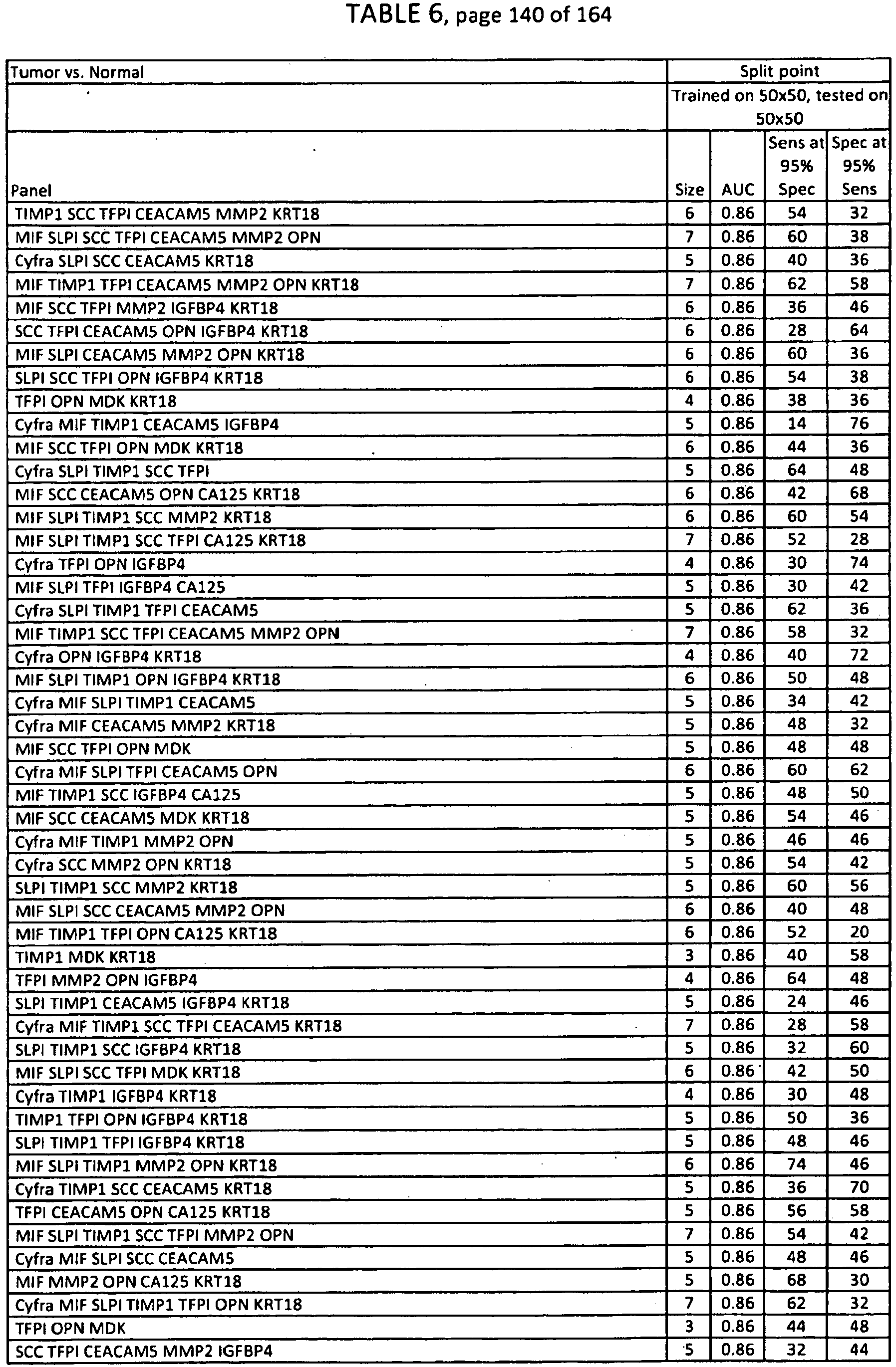

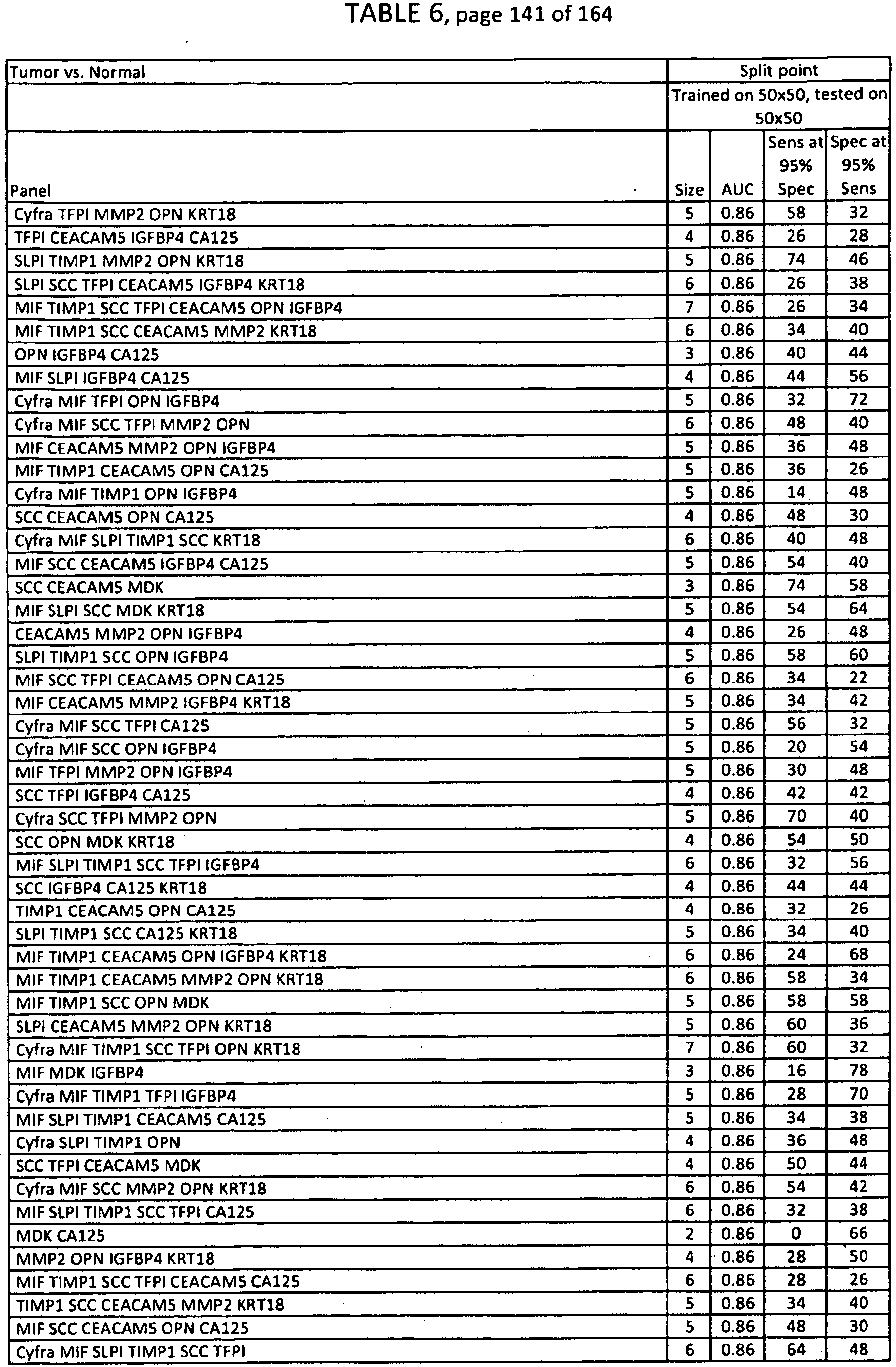

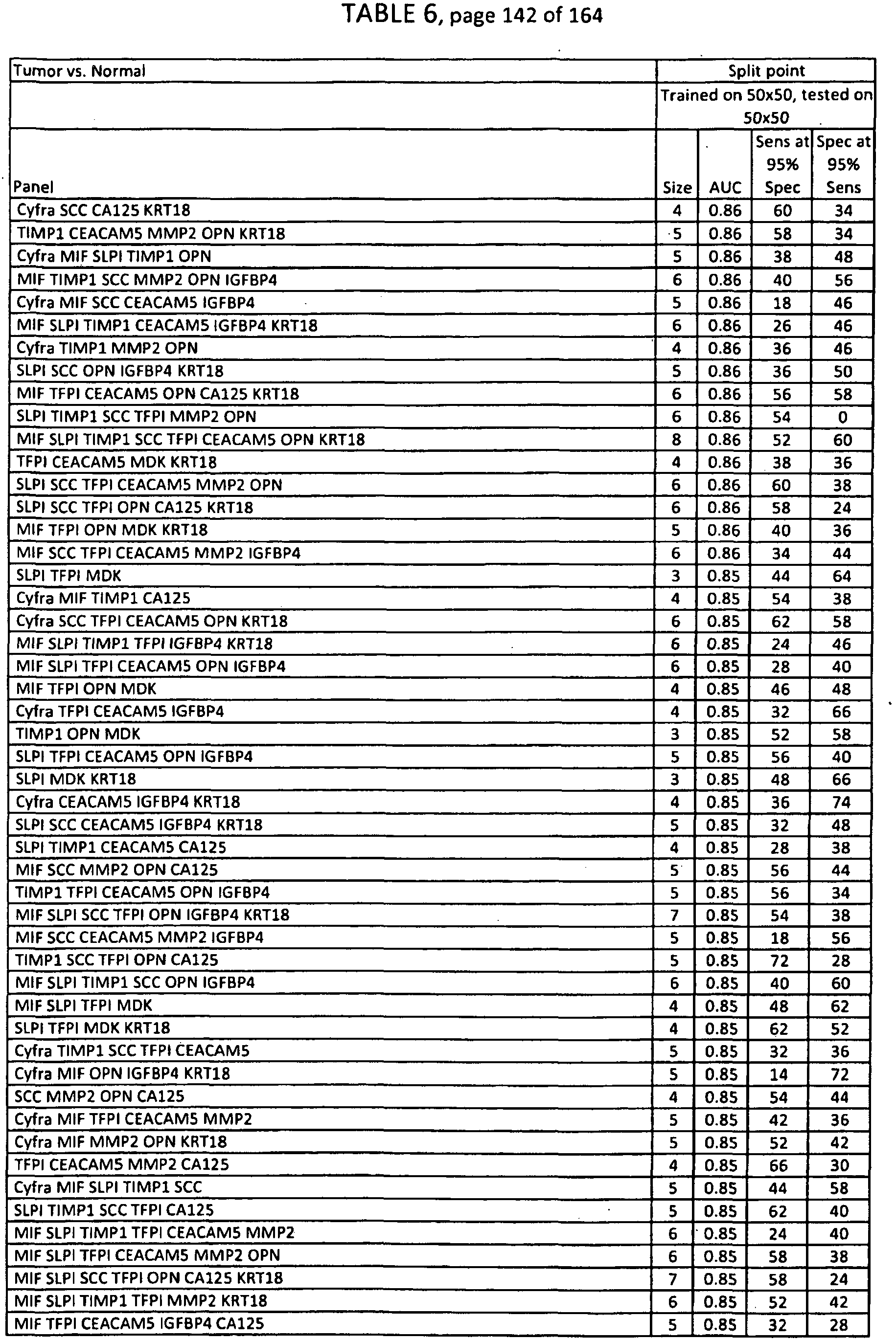

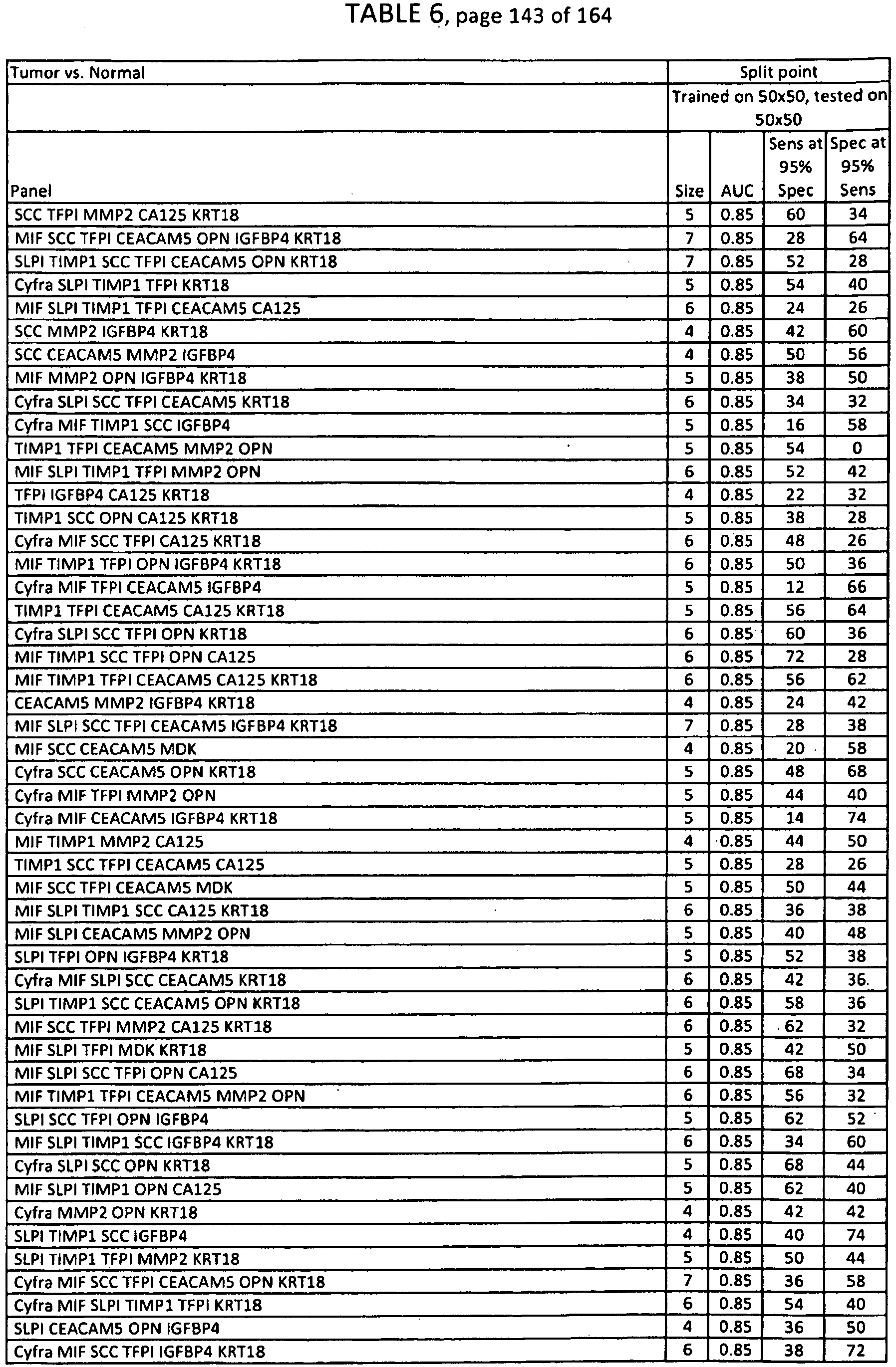

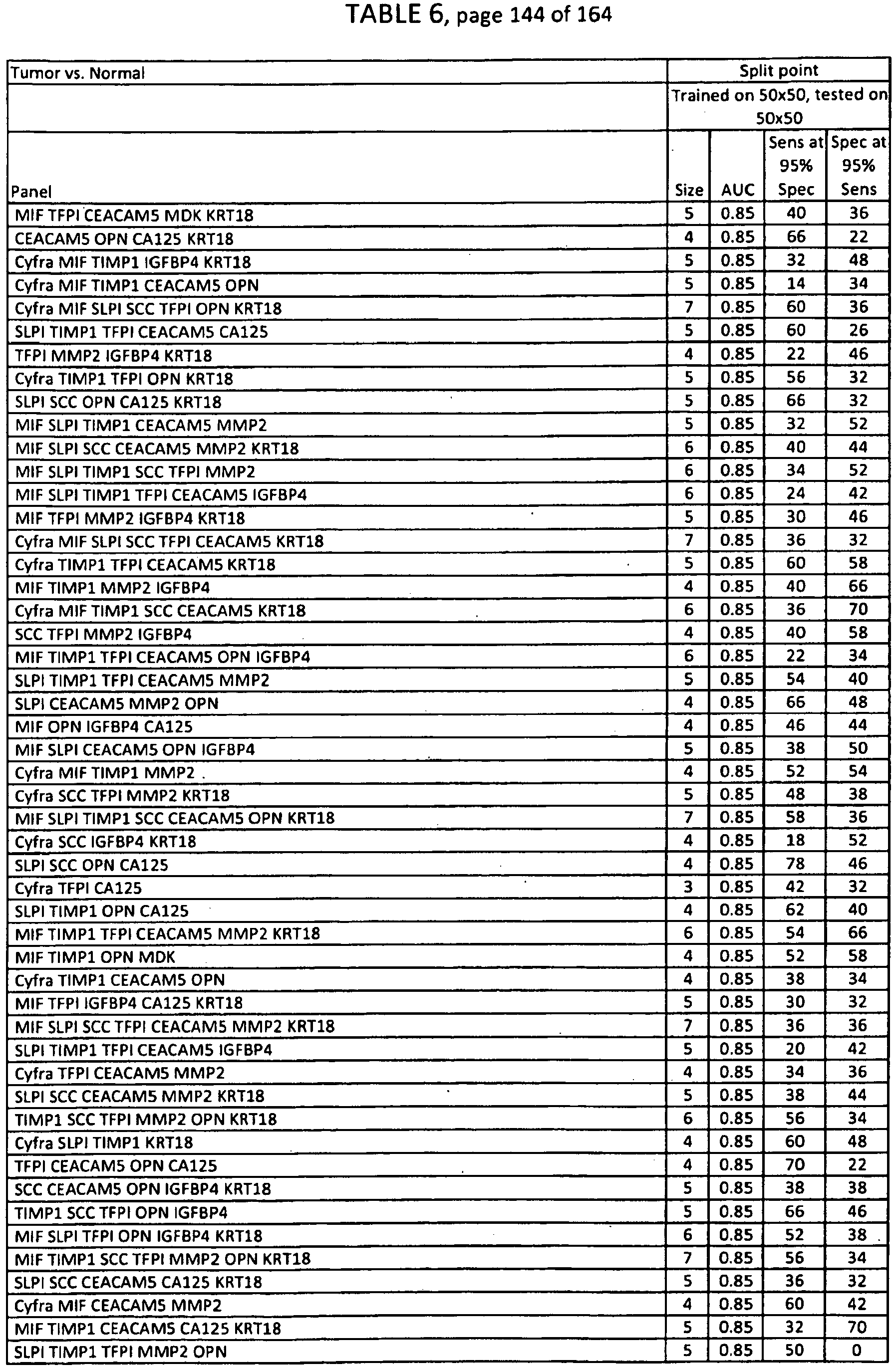

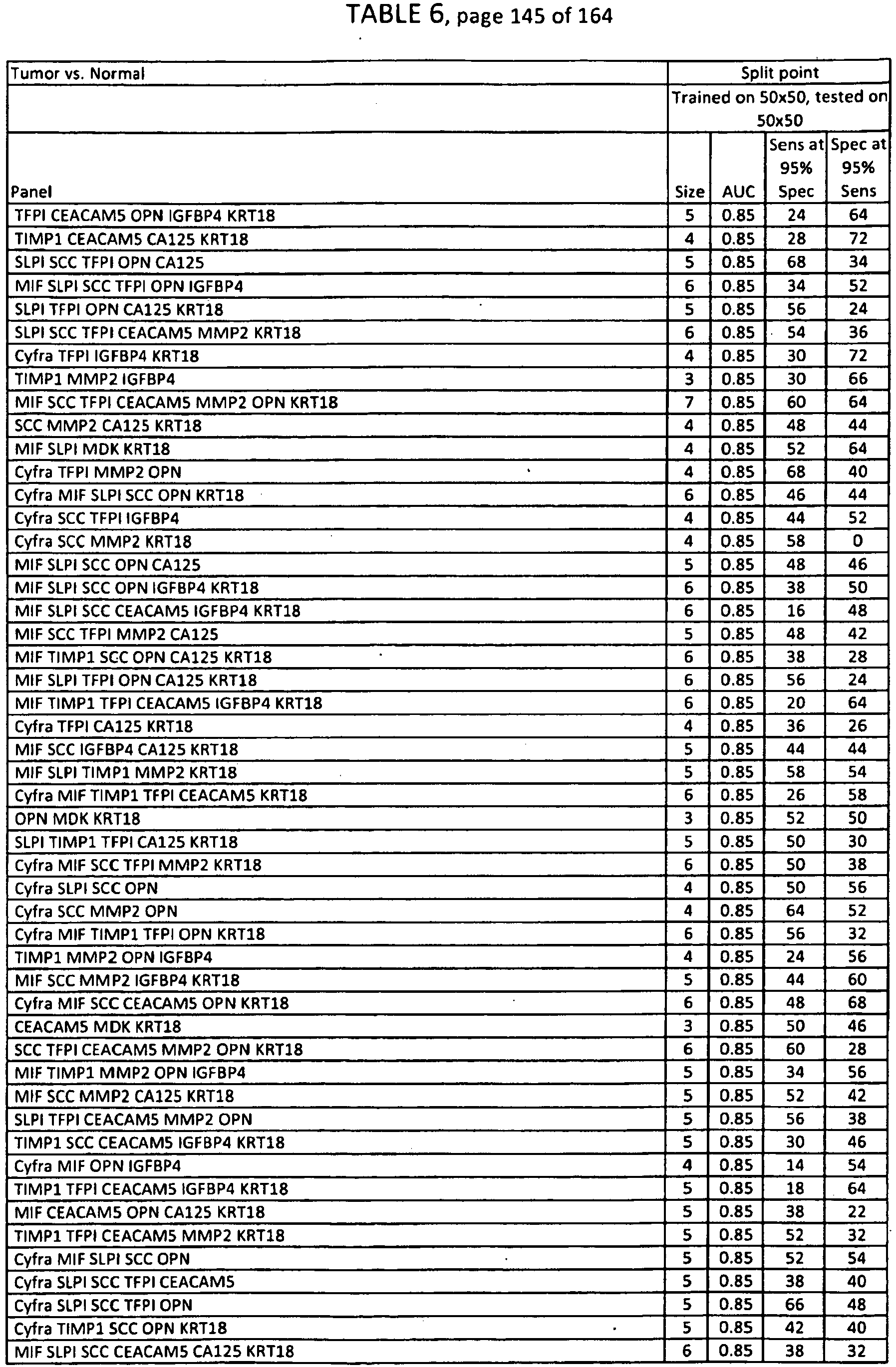

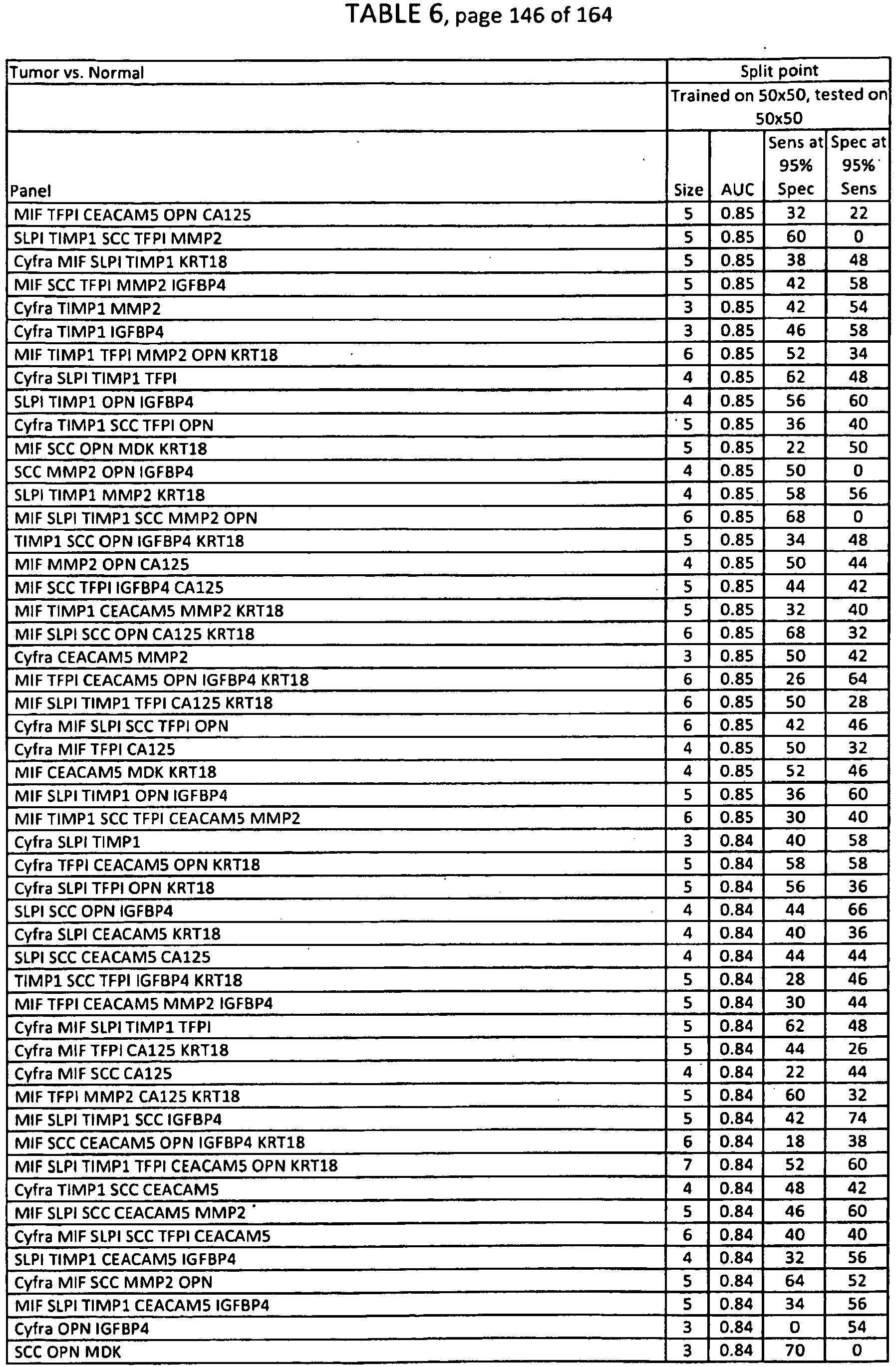

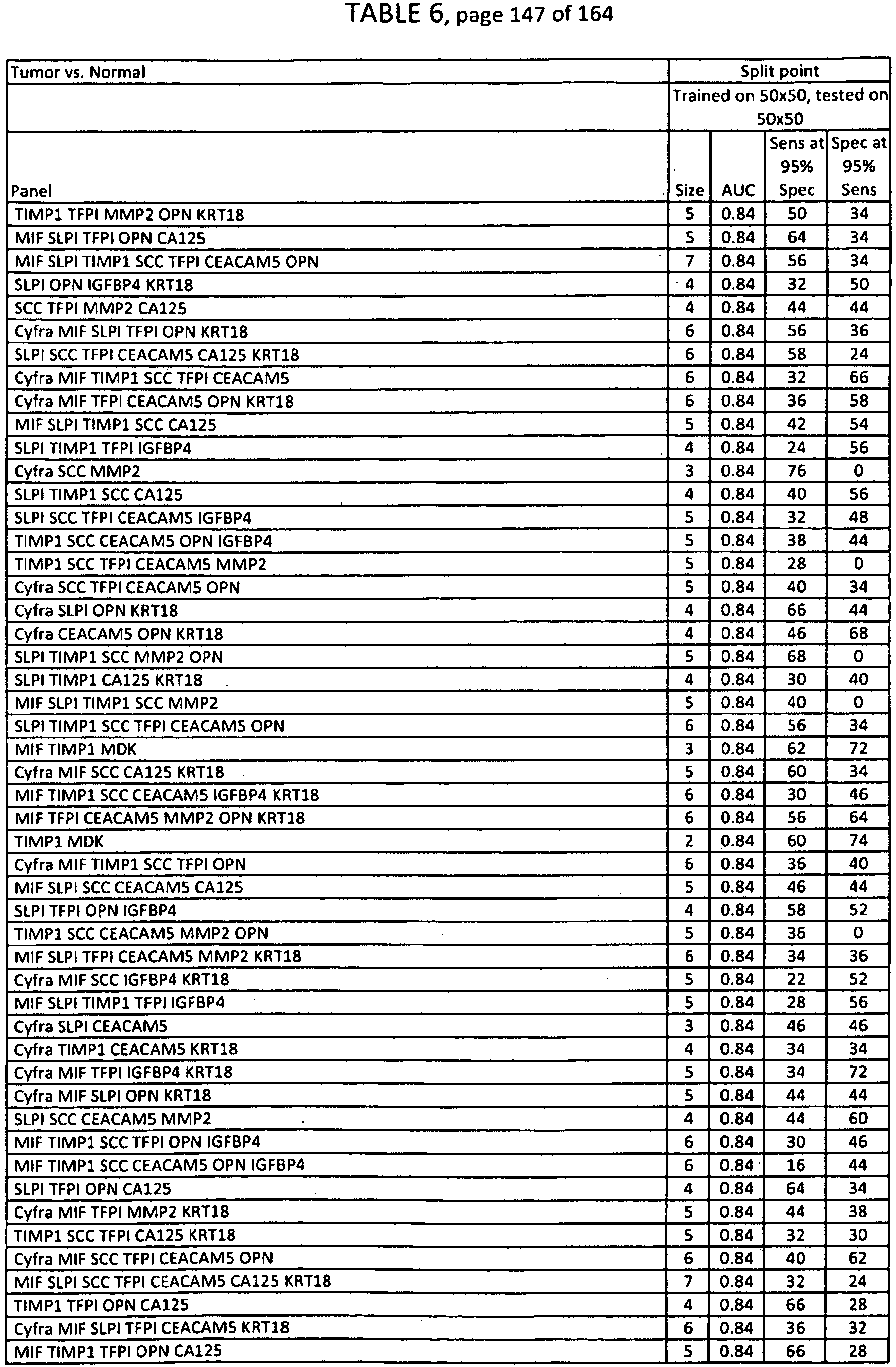

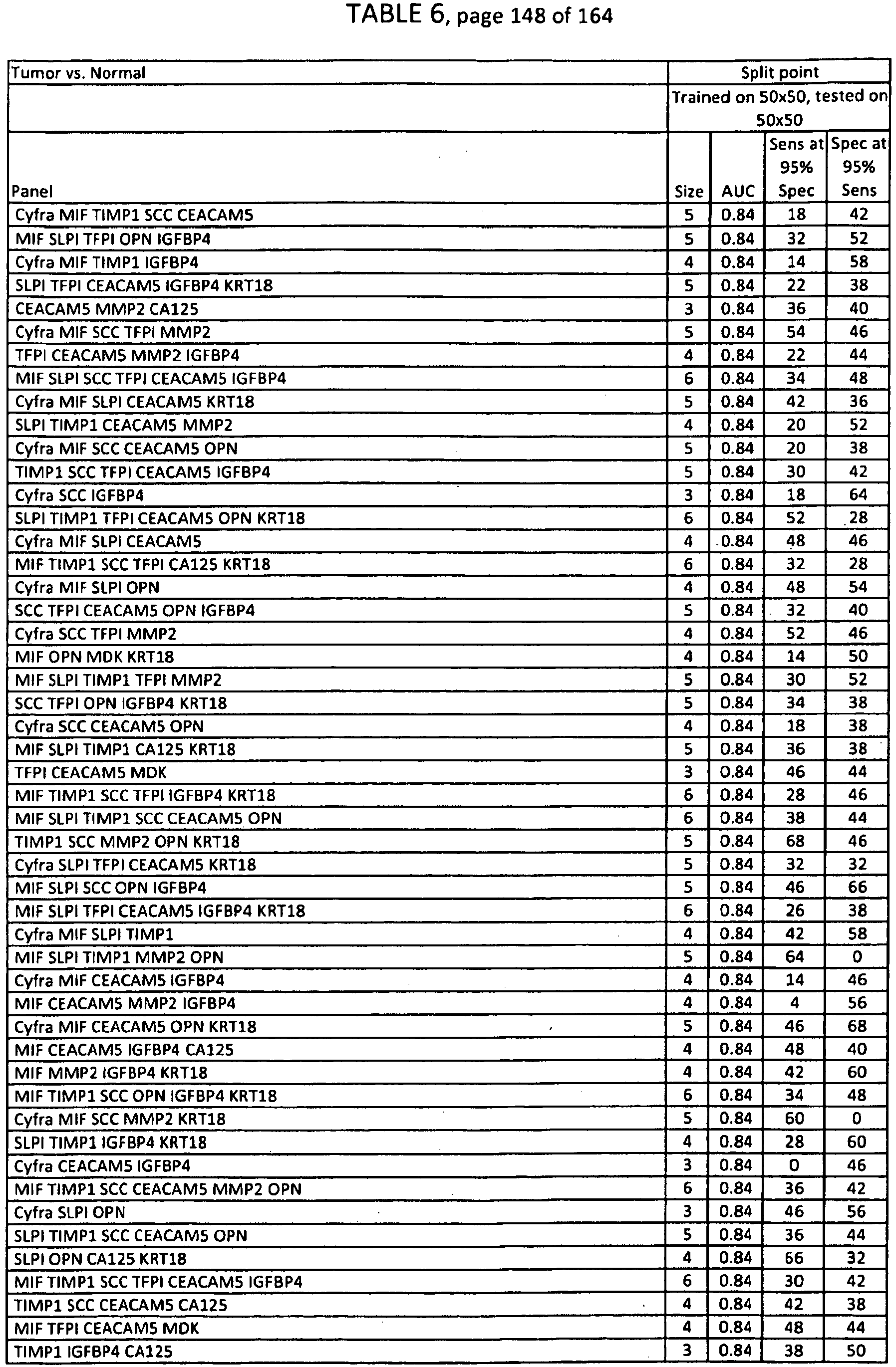

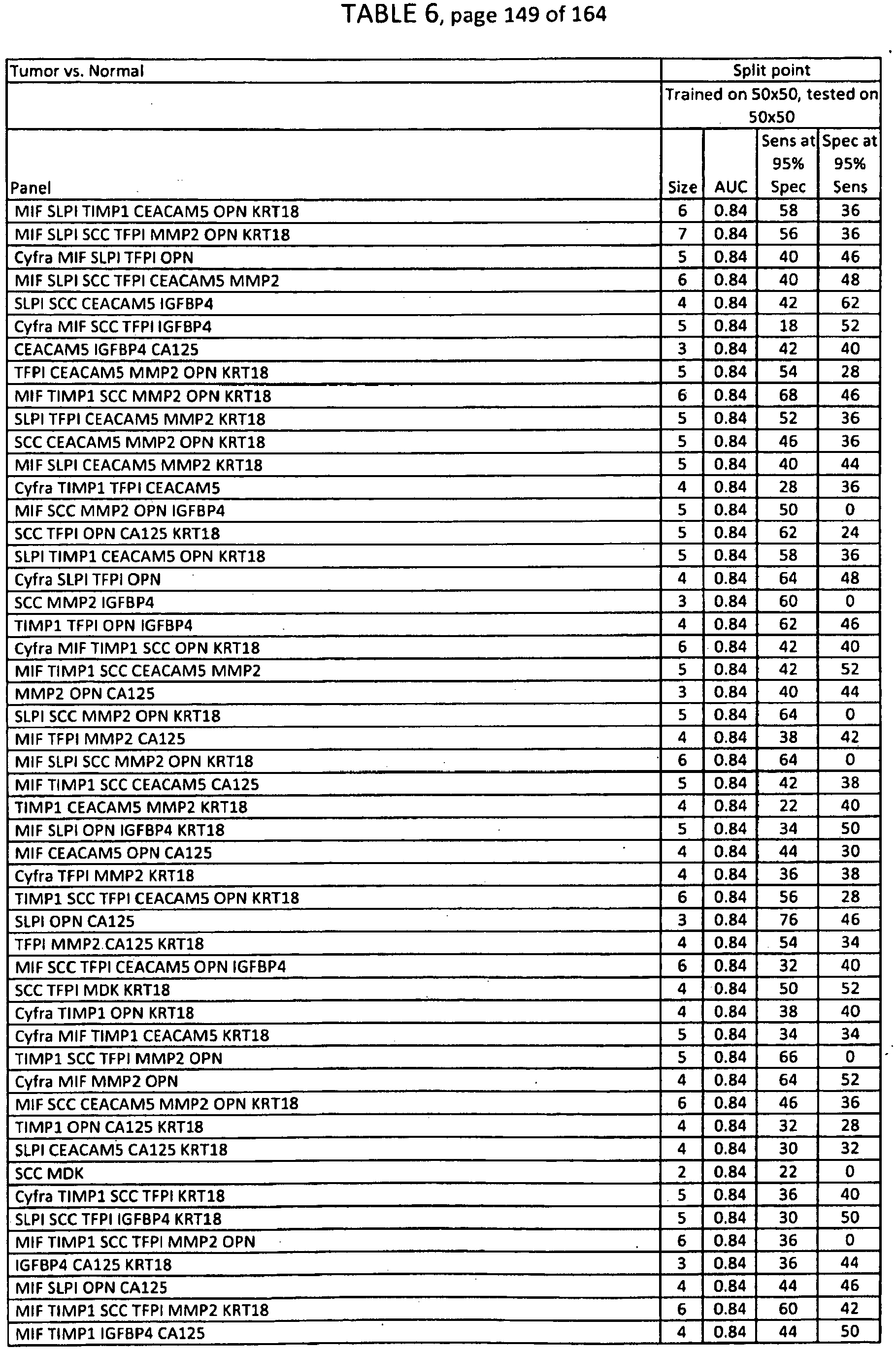

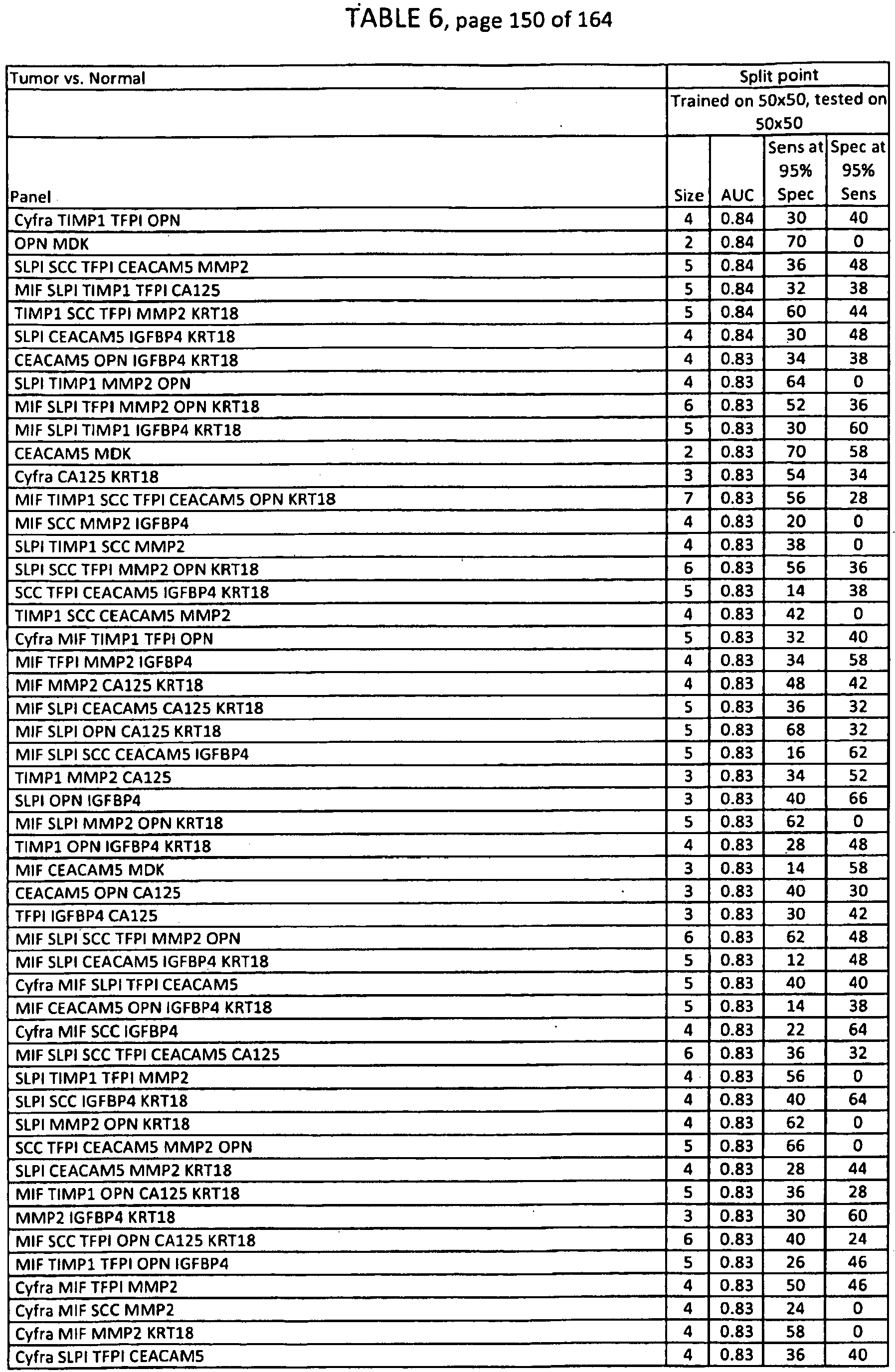

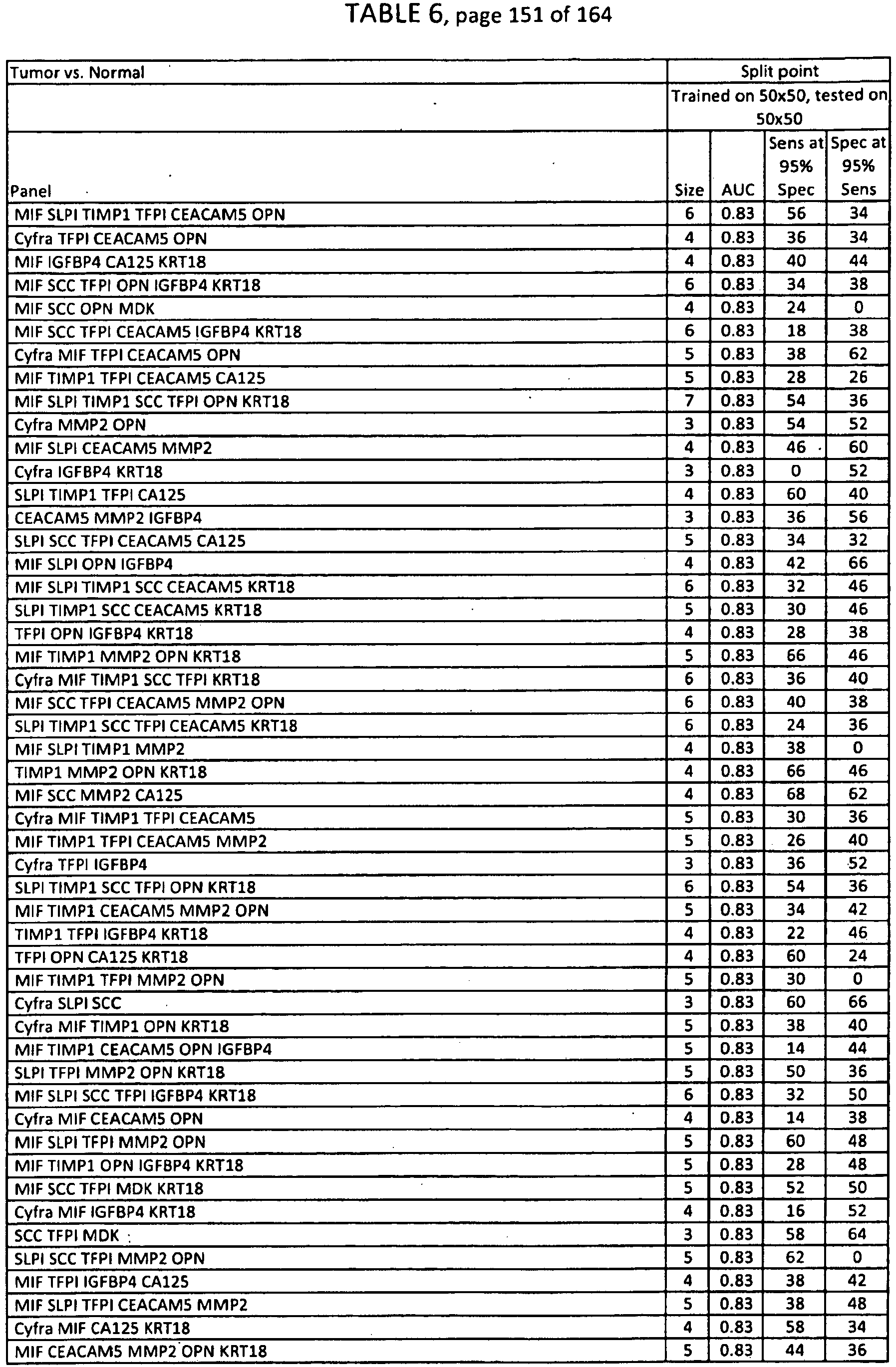

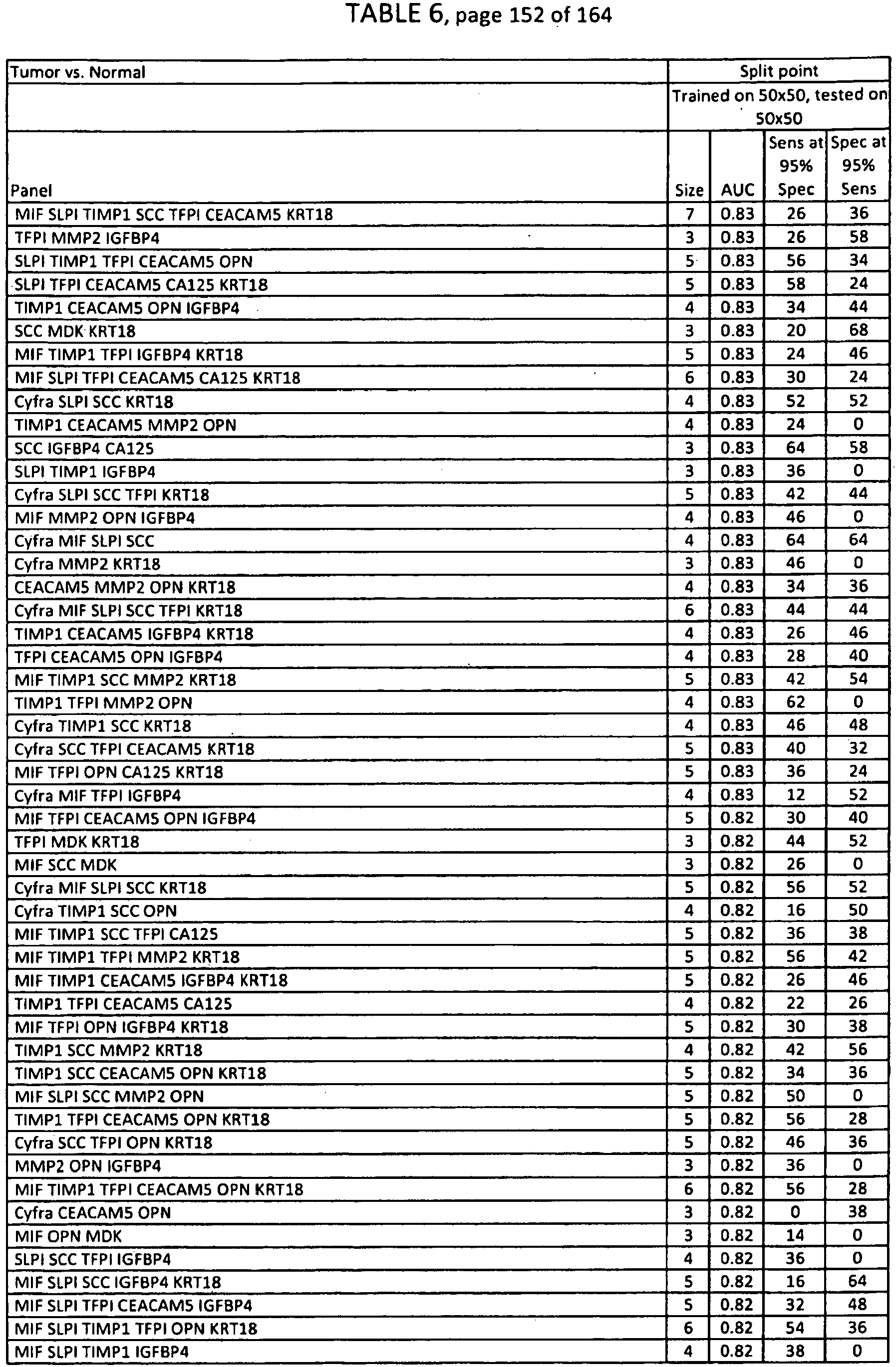

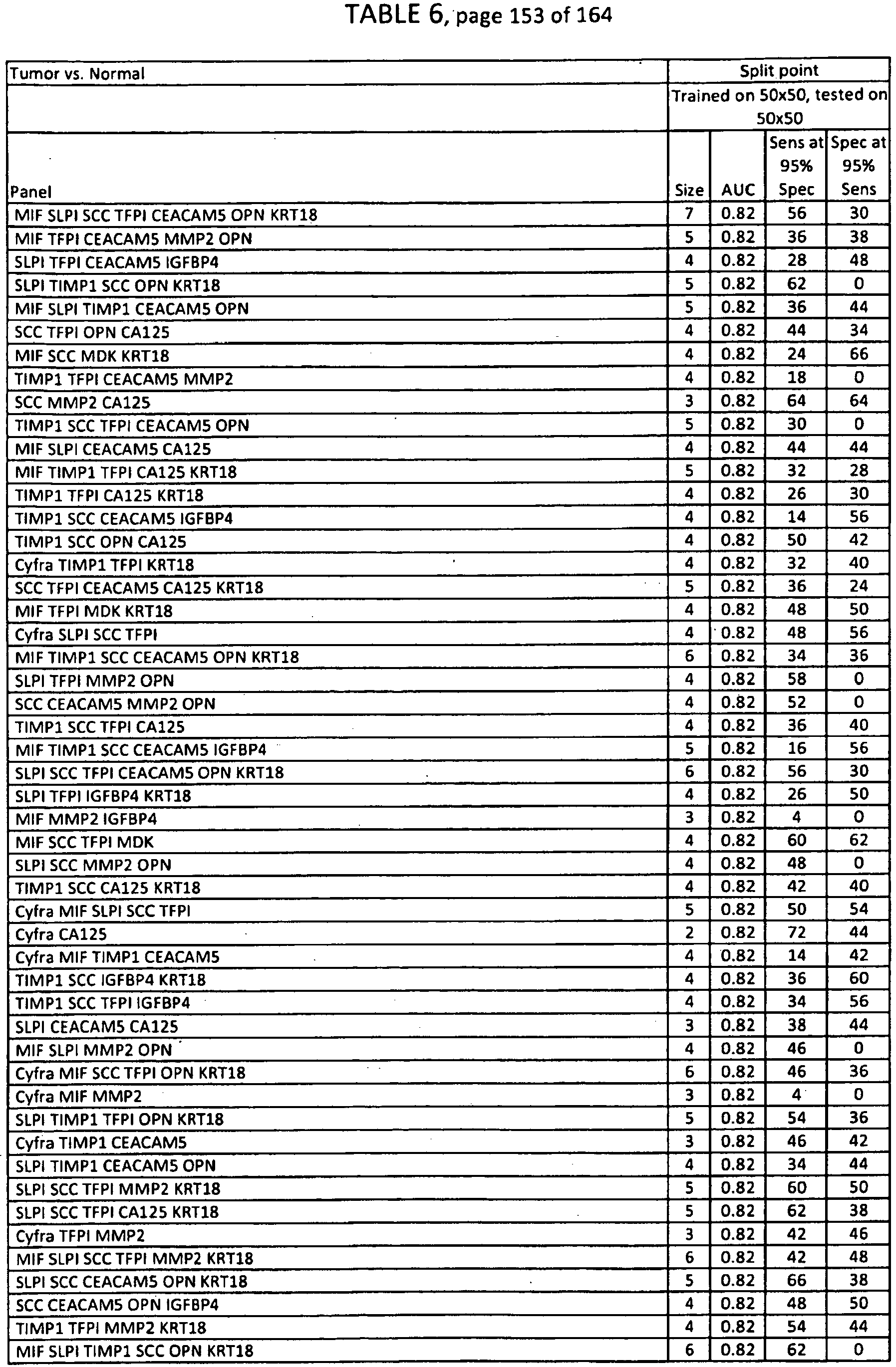

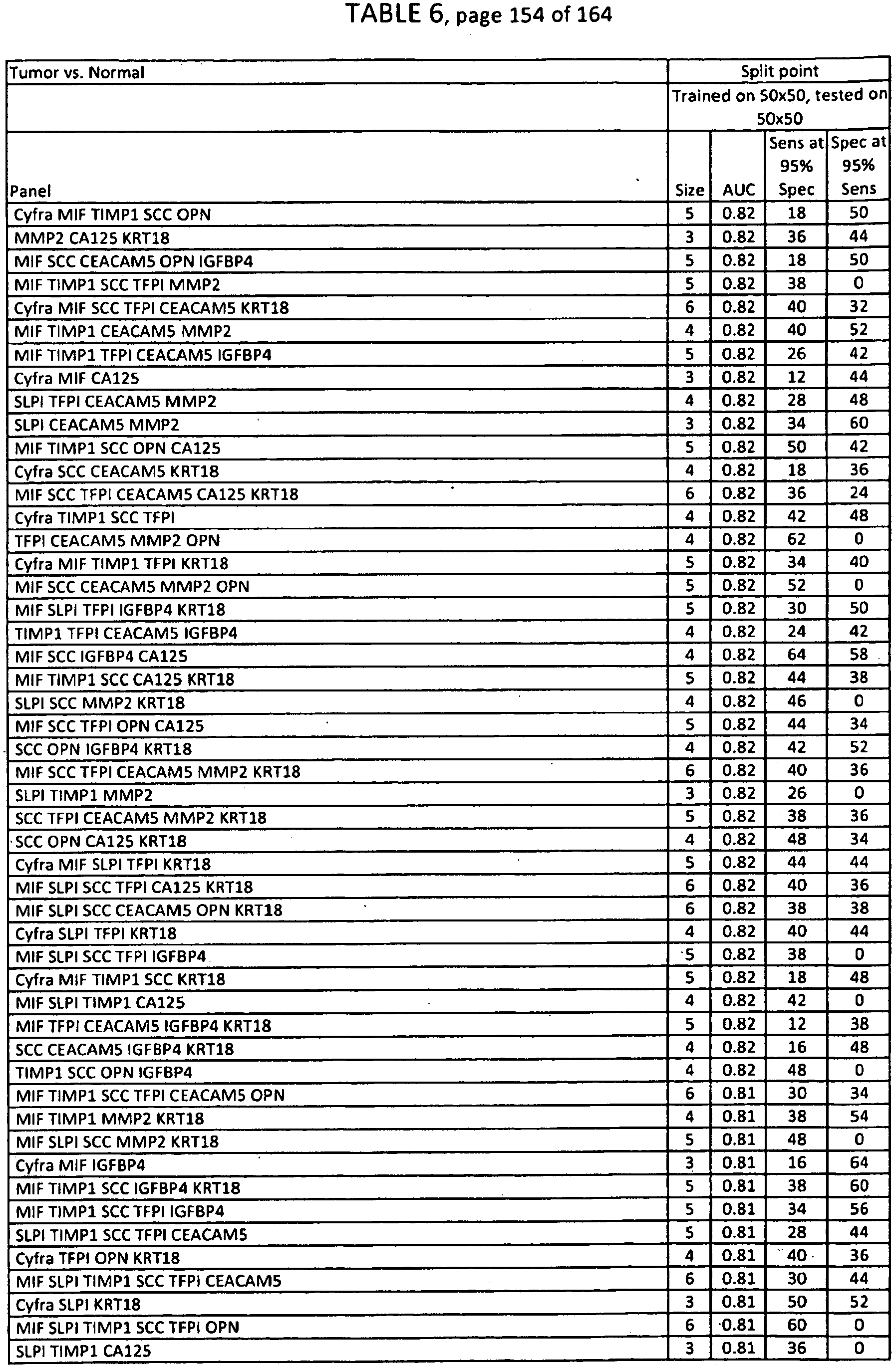

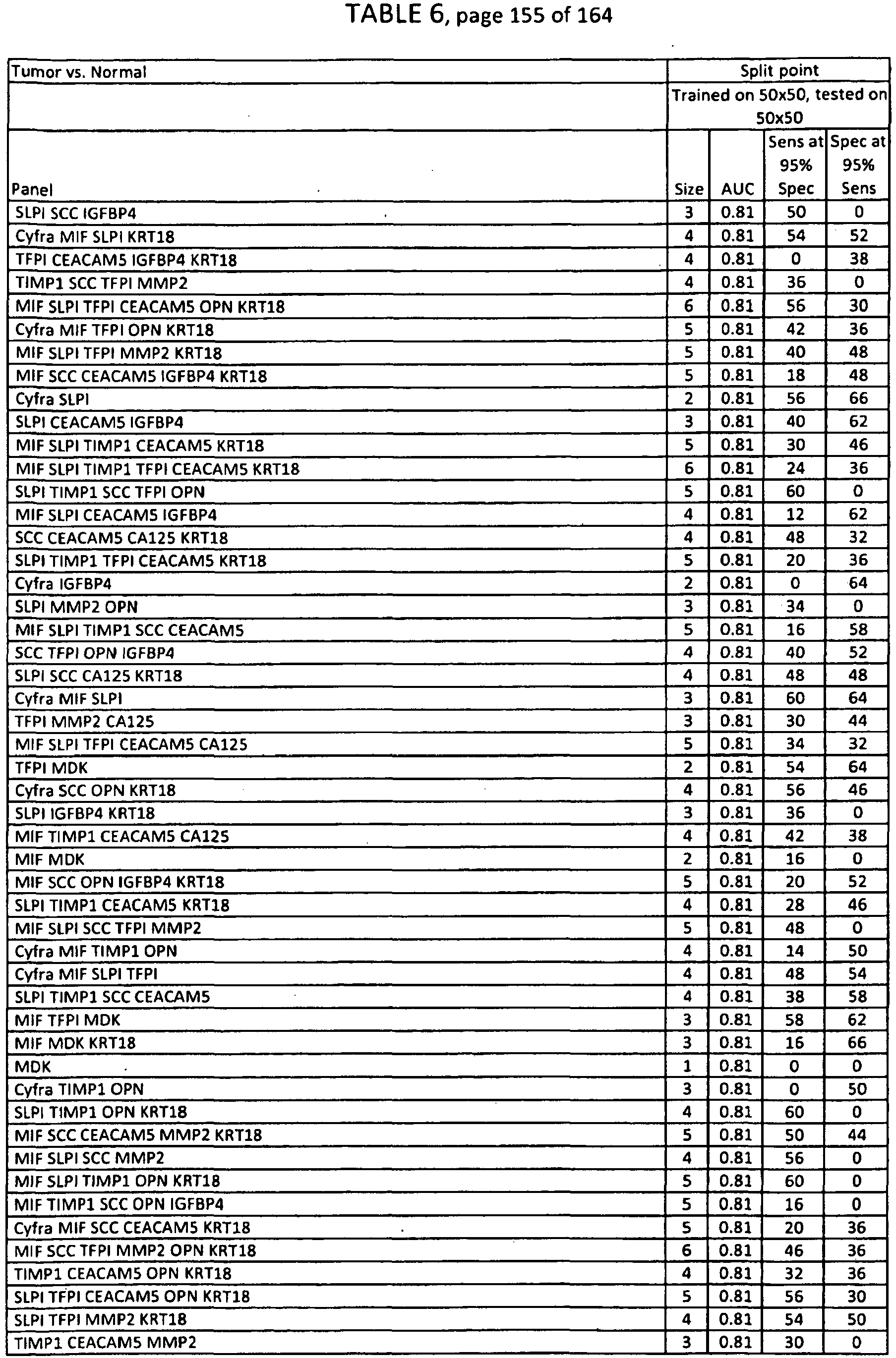

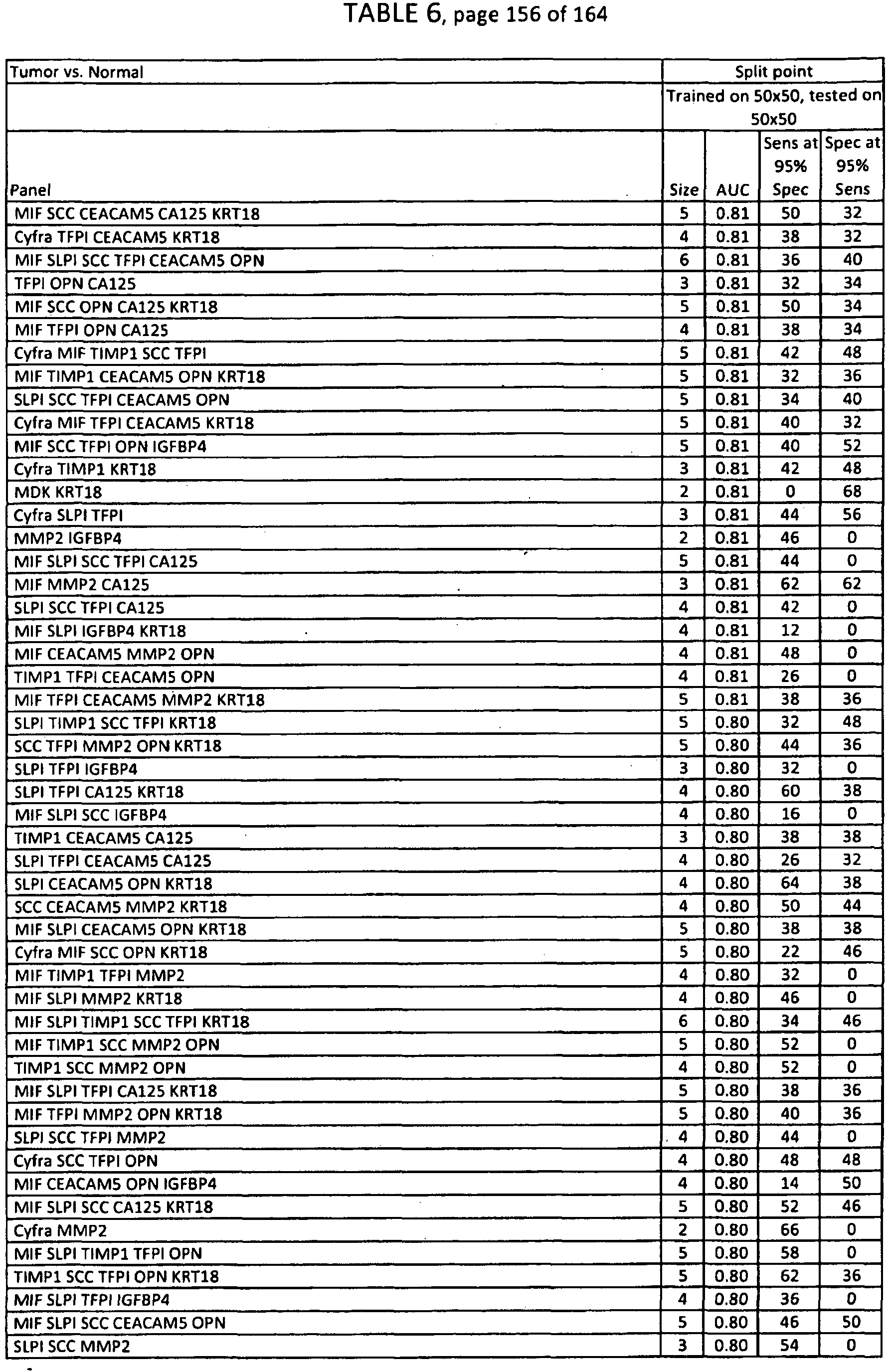

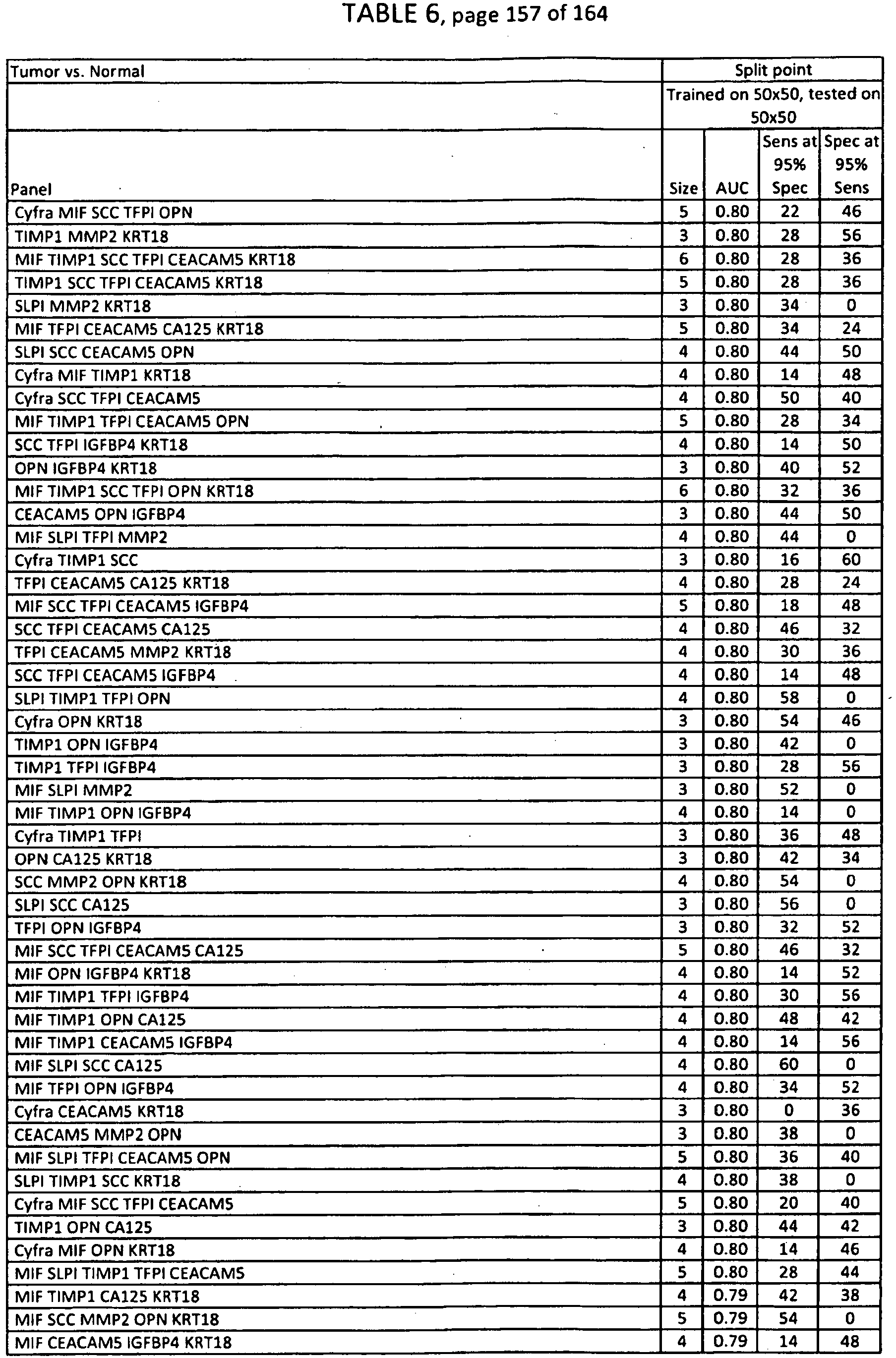

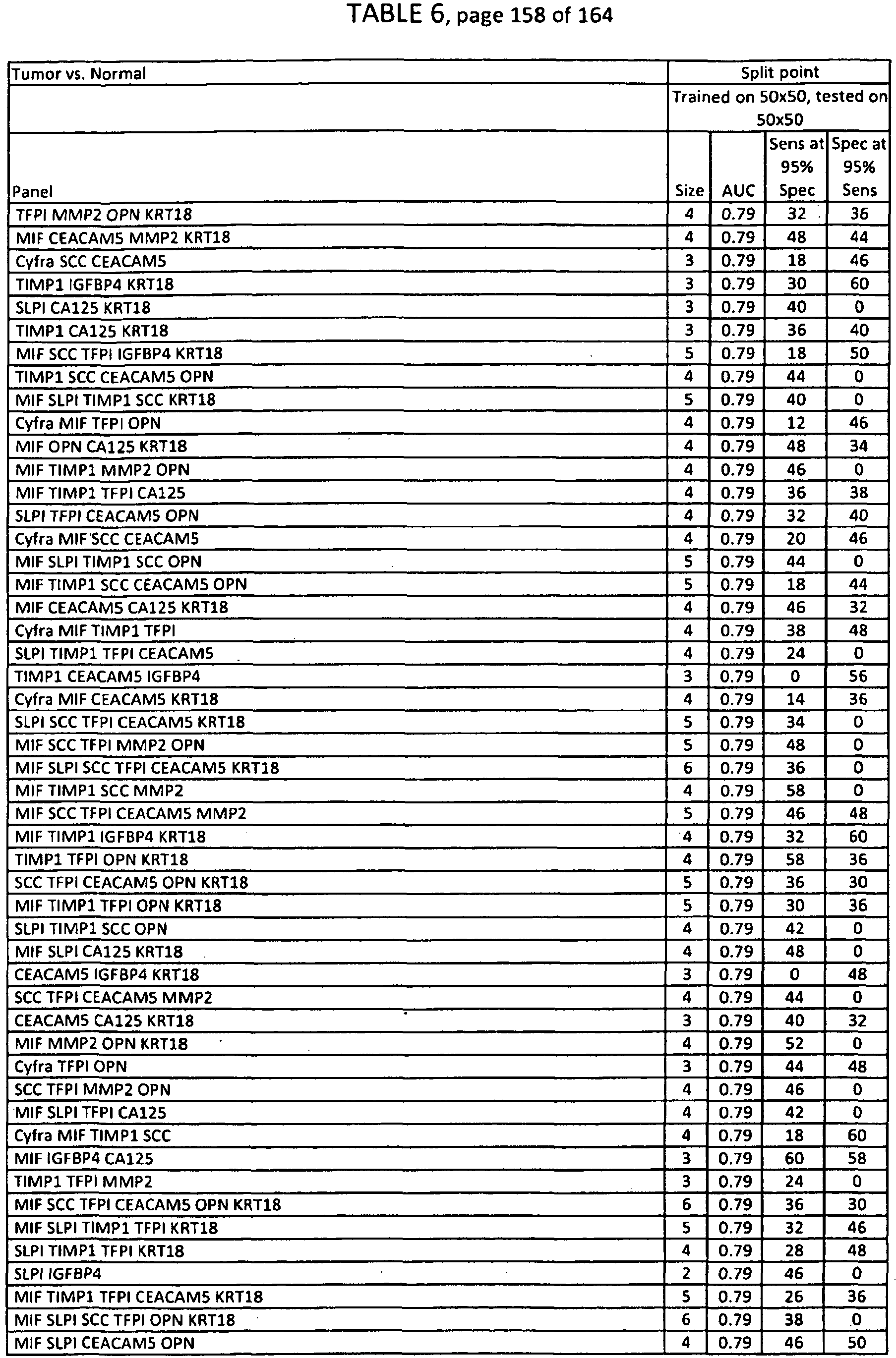

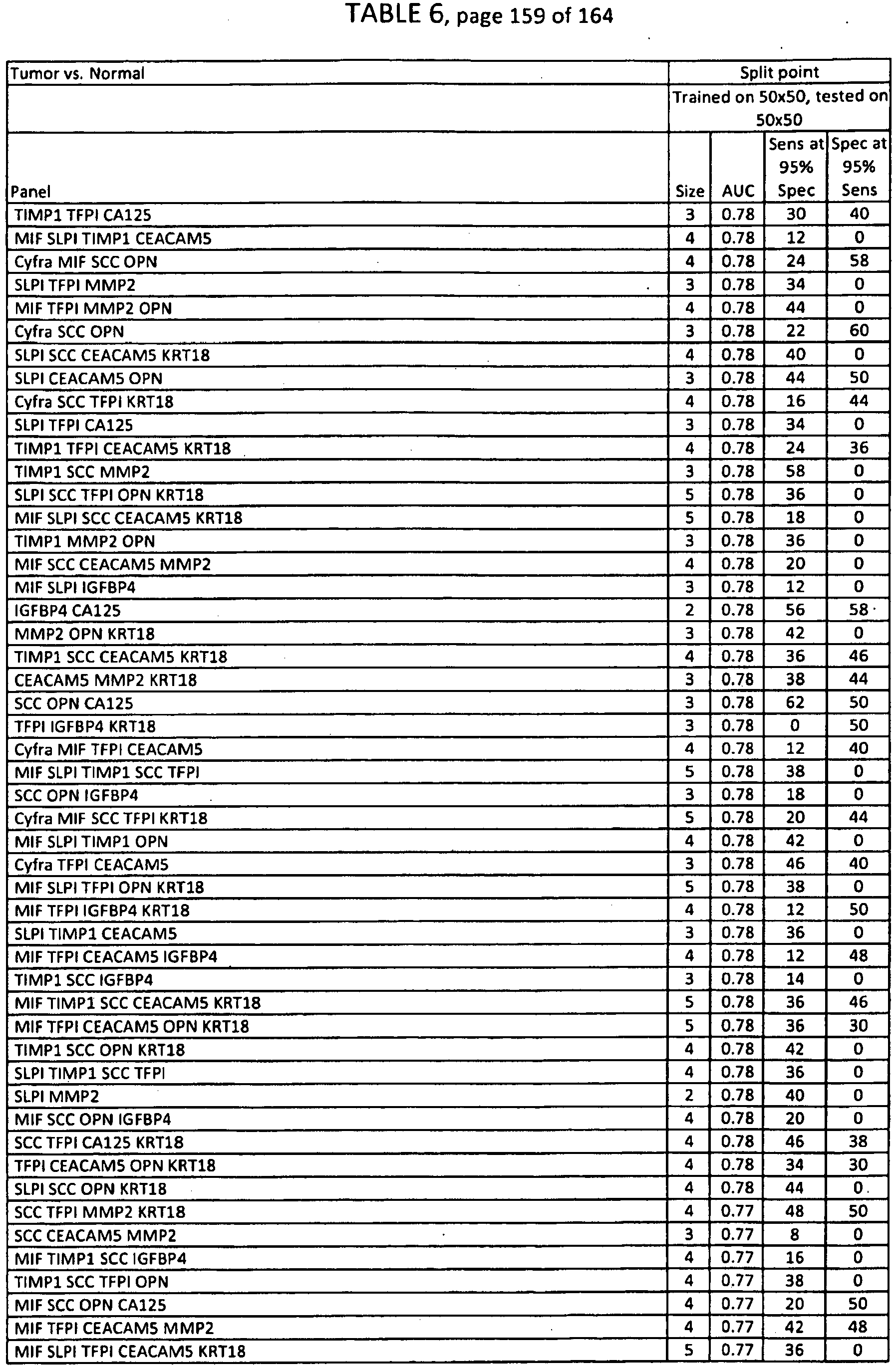

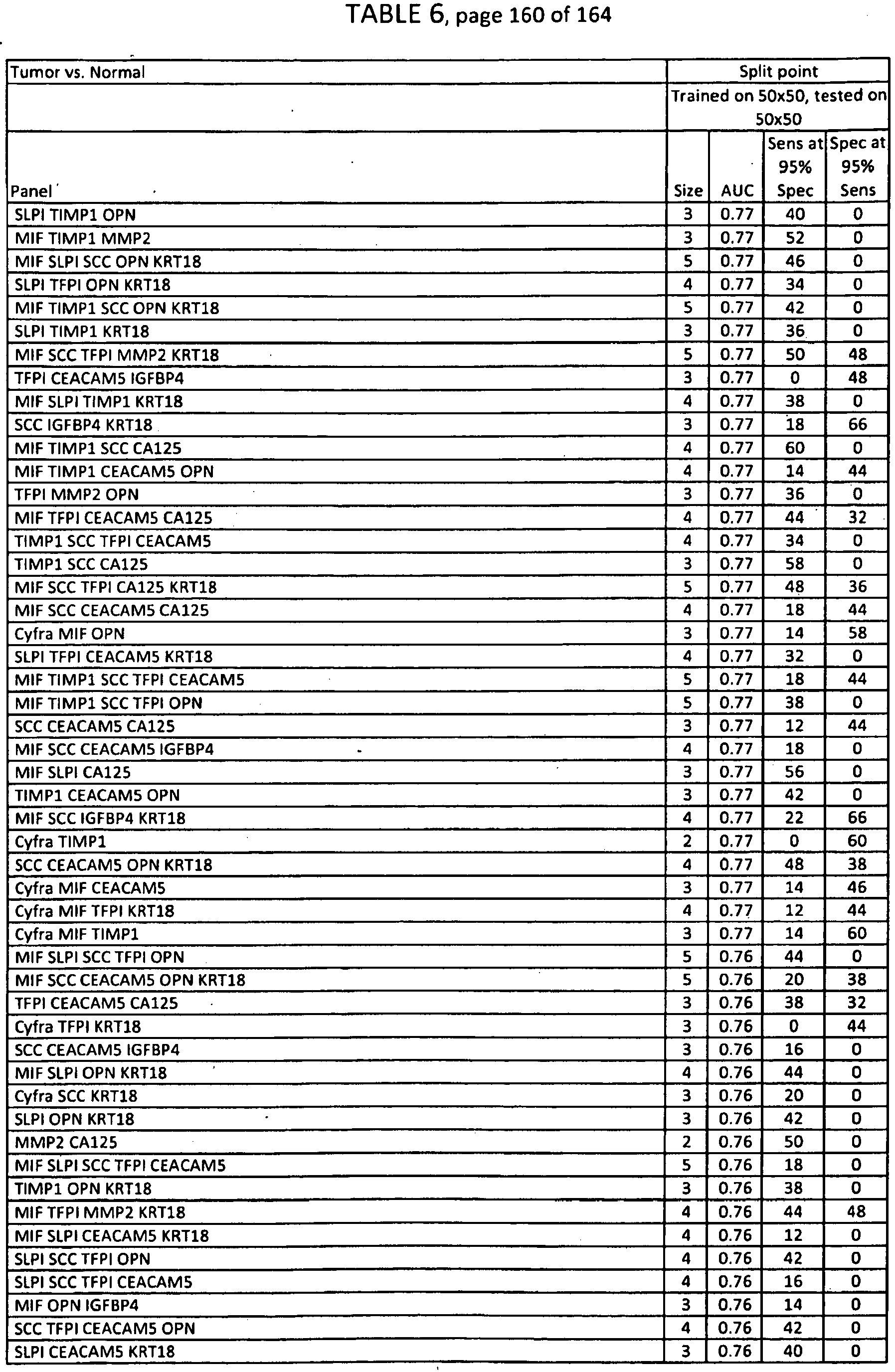

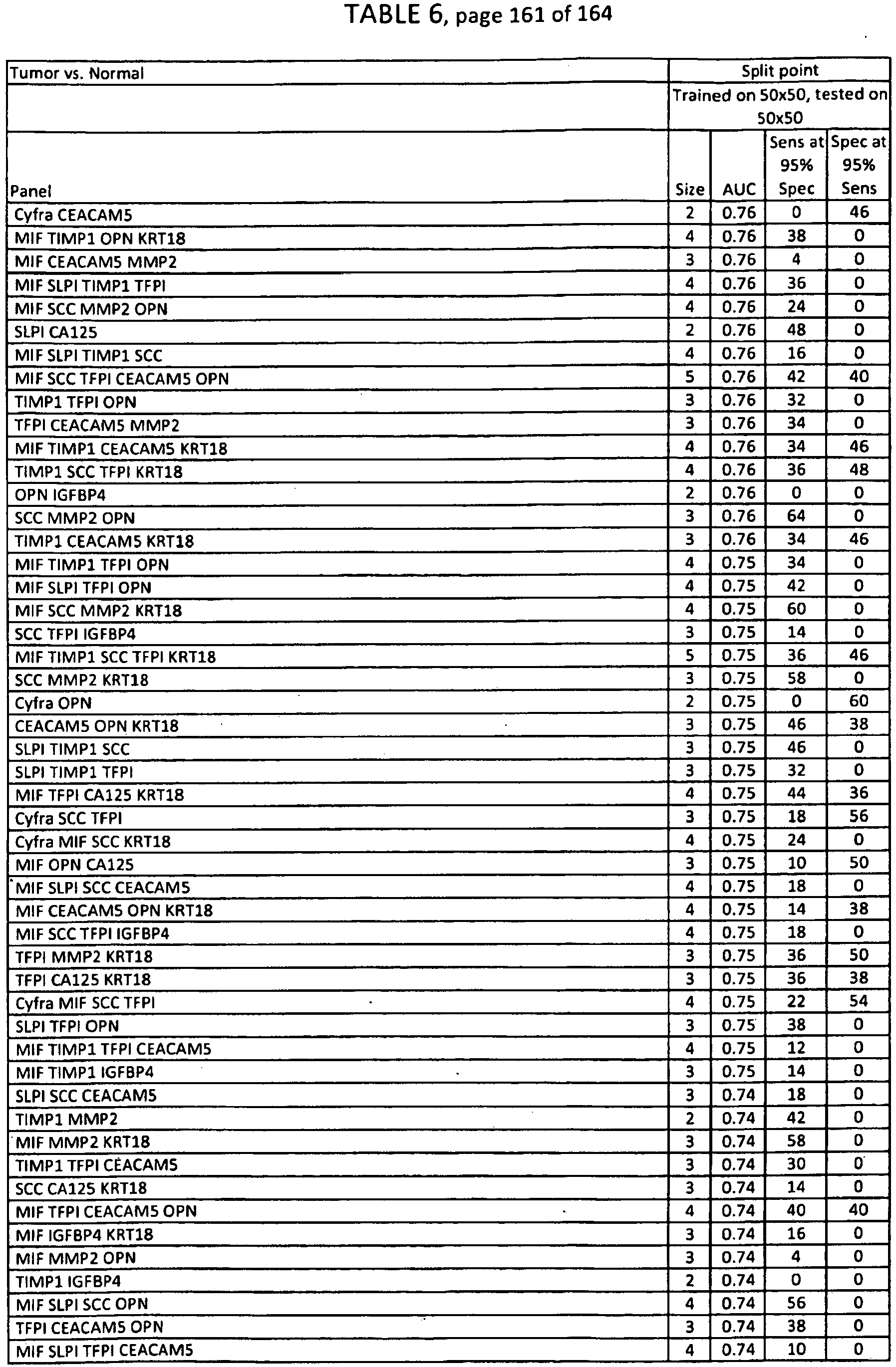

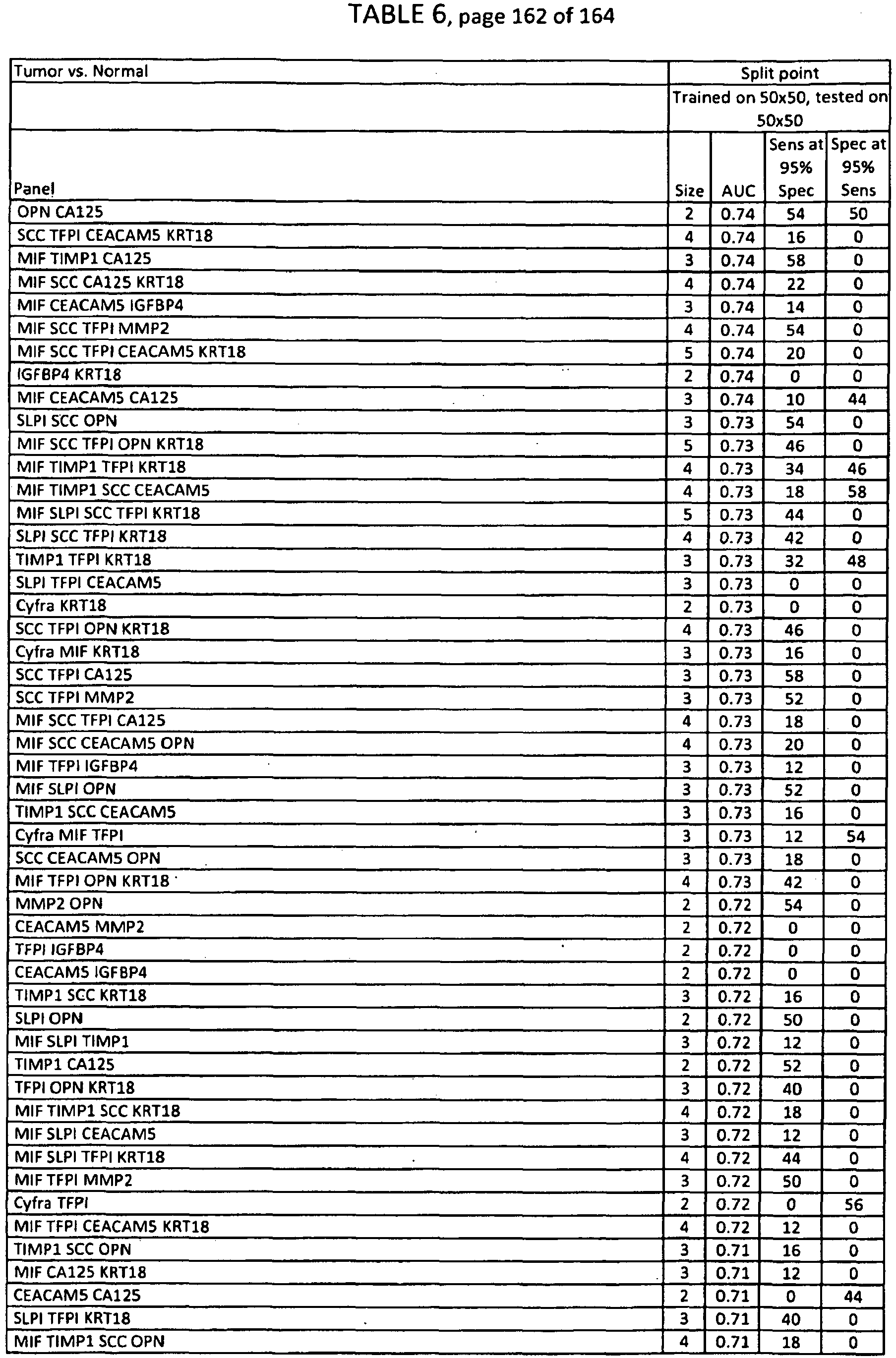

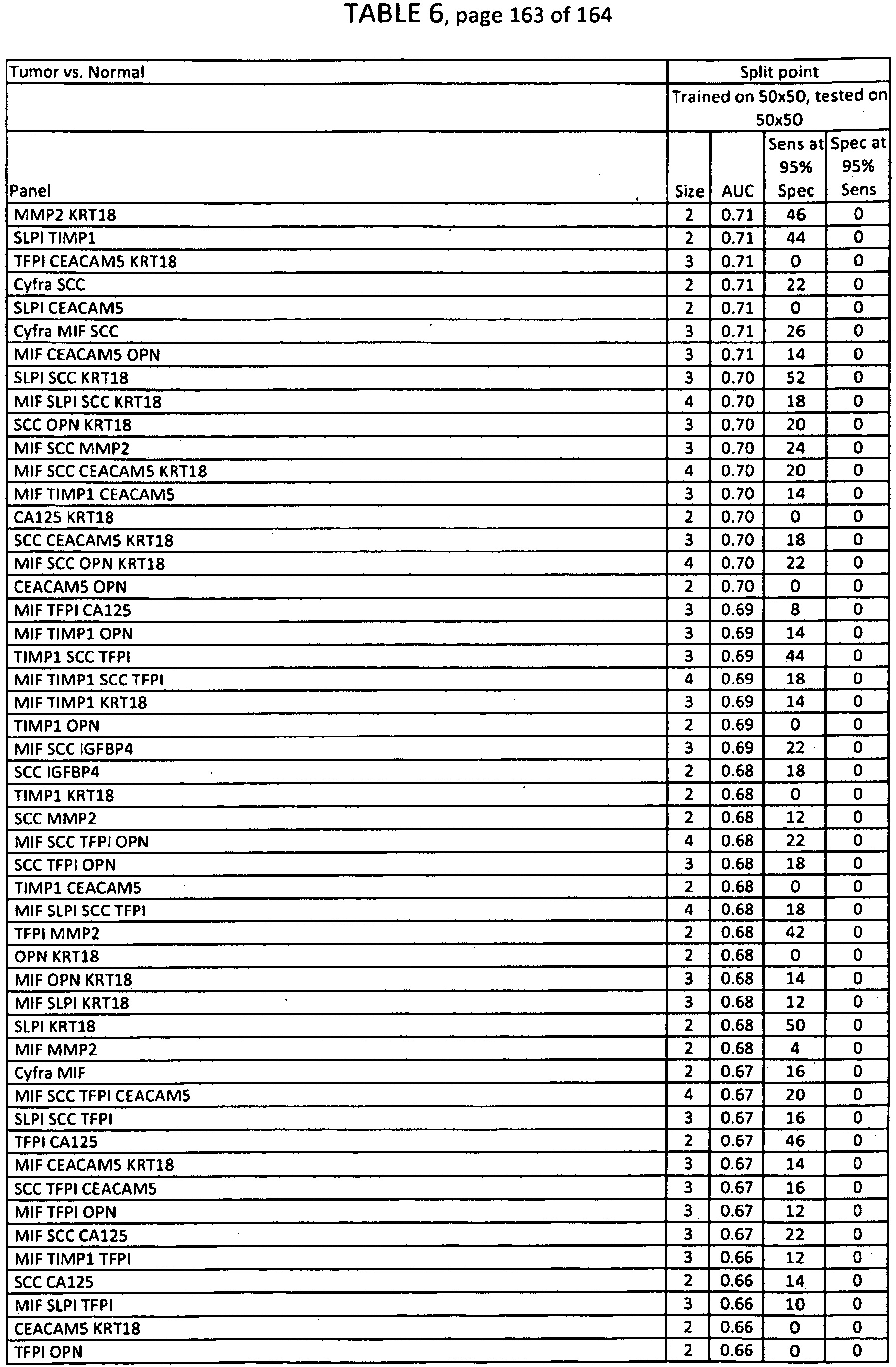

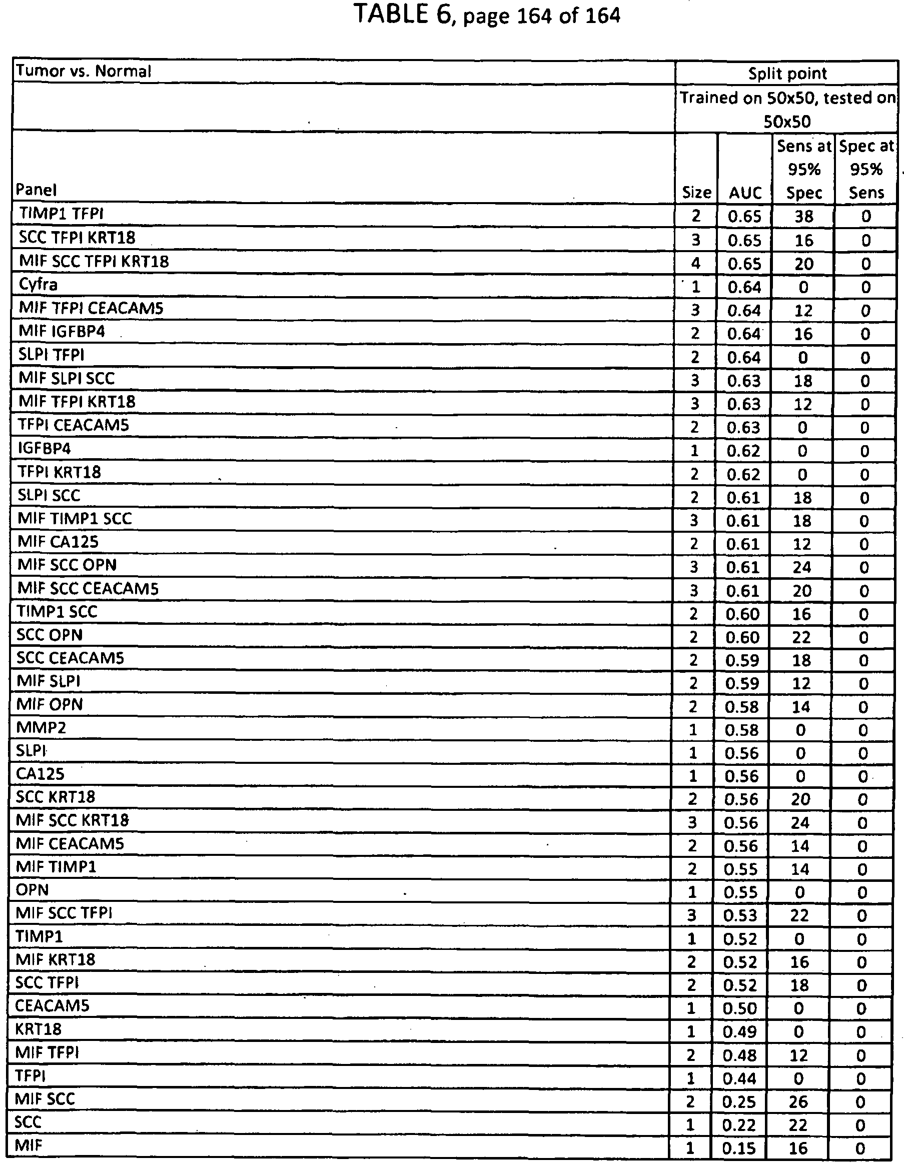

**TABLE 7**

| Adeno vs. Squamous | | Logistic Regression | | | | | |
|---|---|---|---|---|---|---|---|
| | | Trained on 54x53, tested on 50x50 | | | Trained on 54x53, tested on 54x53 | | |
| Panel | Size | AUC | Sens at 95% Spec | Spec at 95% Sens | AUC | Sens at 95% Spec | Spec at 95% Sens |
| SCC TFPI CEACAM5 OPN | 4 | 0.90 | 67 | 64 | 0.95 | 73 | 69 |
| SLPI SCC TFPI CEACAM5 OPN | 5 | 0.89 | 67 | 55 | 0.95 | 73 | 69 |
| SLPI TIMP1 SCC TFPI CEACAM5 OPN | 6 | 0.89 | 67 | 64 | 0.96 | 67 | 69 |
| TIMP1 SCC TFPI CEACAM5 OPN | 5 | 0.87 | 57 | 64 | 0.96 | 67 | 69 |
| Cyfra SCC TFPI CEACAM5 MMP2 OPN MDK | 7 | 0.87 | 62 | 45 | 1.00 | 100 | 100 |
| Cyfra TIMP1 SCC TFPI CEACAM5 OPN MDK | 7 | 0.87 | 0 | 73 | 1.00 | 100 | 100 |
| SCC TFPI CEACAM5 | 3 | 0.87 | 48 | 55 | 0.97 | 82 | 69 |
| TIMP1 SCCTFPI CEACAM5 | 4 | 0.87 | 48 | 55 | 0.96 | 82 | 69 |
| SLPI TIMP1 SCC TFPI CEACAM5 | 5 | 0.87 | 48 | 55 | 0.96 | 82 | 69 |
| Cyfra SLPI TIMP1 SCC TFPI CEACAM5 OPN | 7 | 0.87 | 0 | 82 | 1.00 | 100 | 100 |
| Cyfra SLPI TIMP1 SCC TFPI CEACAM5 OPN MDK | 8 | 0.87 | 0 | 64 | 1.00 | 100 | 100 |
| SLPI SCC TFPI CEACAM5 | 4 | 0.86 | 48 | 55 | 0.97 | 82 | 69 |
| Cyfra SCC TFPI CEACAM5 MDK | 5 | 0.86 | 0 | 73 | 1.00 | 100 | 100 |
| Cyfra SLPI TIMP1 SCC TFPI OPN | 6 | 0.86 | 29 | 64 | 0.99 | 88 | 92 |
| Cyfra TIMP1 SCC TFPI CEACAM5 OPN | 6 | 0.85 | 0 | 82 | 1.00 | 100 | 100 |
| Cyfra TIMP1 CEACAM5 | 3 | 0.84 | 14 | 55 | 0.98 | 88 | 92 |
| Cyfra SLPI TIMP1 CEACAM5 | 4 | 0.84 | 14 | 55 | 0.99 | 94 | 92 |
| TIMP1 SCC TFPI CEACAM5 OPN MDK | 6 | 0.84 | 0 | 55 | 1.00 | 100 | 100 |
| Cyfra SLPI SCC TFPI CEACAM5 OPN MDK | 7 | 0.84 | 0 | 64 | 1.00 | 100 | 100 |
| SLPI TIMP1 SCC TFPI CEACAM5 OPN MDK | 7 | 0.84 | 0 | 55 | 1.00 | 100 | 100 |
| Cyfra SLPI TIMP1 SCC CEACAM5 OPN | 6 | 0.84 | 0 | 64 | 1.00 | 100 | 100 |
| SLPI TIMP1 SCC TFPI MDK | 5 | 0.84 | 24 | 55 | 0.95 | 45 | . 85 |
| SLPI TIMP1 SCC TFPI OPN MDK | 6 | 0.84 | 24 | 55 | 0.94 | 42 | 85 |
| Cyfra SLPI SCC TFPI CEACAM5 MMP2 MDK | 7 | 0.84 | 0 | 73 | 1.00 | 100 | 100 |
| SLPI TIMP1 SCC TFPI CEACAM5 MMP2 MDK | 7 | 0.84 | 0 | 73 | 1.00 | 100 | 100 |
| SCC TFPI MDK | 3 | 0.84 | 24 | 55 | 0.93 | 36 | 77 |
| SLPI SCC TFPI MDK | 4 | 0.84 | 24 | 55 | 0.93 | 39 | 69 |
| TIMP1 SCC TFPI MDK | 4 | 0.84 | 24 | 55 | 0.93 | 33 | 85 |
| SLPI SCC TFPI OPN MDK | 5 | 0.84 | 24 | 55 | 0.93 | 45 | 69 |
| TIMP1 SCC TFPI OPN MDK | 5 | 0.84 | 24 | 55 | 0.93 | 36 | 85 |
| SCC TFPI CEACAM5 OPN MDK | 5 | 0.84 | 0 | 55 | 1.00 | 100 | 100 |
| Cyfra TIMP1 SCC CEACAM5 OPN MDK | 6 | 0.84 | 0 | 64 | 1.00 | 100 | 100 |
| Cyfra SCC TFPI CEACAM5 OPN MDK | 6 | 0.84 | 0 | 45 | 1.00 | 100 | 100 |
| SCC TFPI OPN MDK | 4 | 0.83 | 24 | 55 | 0.93 | 45 | 85 |
| SLPI TIMP1 SCC TFPI OPN | 5 | 0.83 | 52 | 55 | 0.91 | 39 | 77 |
| SCC TFPI CEACAM5 MMP2 OPN MDK | 6 | 0.83 | 0 | 45 | 1.00 | 100 | 100 |
| SLPI SCC TFPI CEACAM5 MMP2 OPN MDK | 7 | 0.83 | 0 | 45 | 1.00 | 100 | 100 |
| Cyfra SLPI SCC TFPI CEACAM5 MMP2 OPN MDK | 8 | 0.83 | 0 | 64 | 1.00 | 100. | 100 |
| Cyfra TIMP1 SCC TFPI OPN | 5 | 0.83 | 19 | 55 | 0.99 | 88 | 92 |
| SLPI SCC TFPI OPN | 4 | 0.83 | 38 | 55 | 0.90 | 36 | 77 |
| SLPI SCC TFPI CEACAM5 MMP2 MDK | 6 | 0.83 | 0 | 45 | 1.00 | 100 | 100 |
| Cyfra SLPI TIMP1 SCC CEACAM5 OPN MDK | 7 | 0.83 | 0 | 55 | 1.00 | 100 | 100 |
| Cyfra TIMP1 SCC CEACAM5 | 4 | 0.83 | 0 | 64 | 1.00 | 100 | 100 |
| Cyfra MDK | 2 | 0.82 | 24 | 55 | 0.98 | 70 | 92 |
| SCC TFPI OPN | 3 | 0.82 | 38 | 55 | 0.90 | 36 | 77 |
| TIMP1 SCC TFPI OPN | 4 | 0.82 | 38 | 55 | 0.90 | 36 | 77 |
| SCC TFPI CEACAM5 MMP2 MDK | 5 | 0.82 | 0 | 45 | 1.00 | 100 | 100 |
| TIMP1 SCC TFPI CEACAM5 MMP2 MDK | 6 | 0.82 | 0 | 55 | 1.00 | 100 | 100 |
| SCC TFPI | 2 | 0.82 | 43 | 55 | 0.91 | 27 | 69 |
| SLPI SCC TFPI | 3 | 0.82 | 43 | 55 | 0.91 | 27 | 69 |
| TIMP1 SCC TFPI | 3 | 0.82 | 43 | 55 | 0.91 | 27 | 77 |
| TIMP1 SCC TFPI CEACAM5 MMP2 | 5 | 0.82 | 24 | 55 | 0.99 | 88 | 92 |
| SLPI TIMP1 SCC TFPI | 4 | 0.81 | 43 | 55 | 0.91 | 24 | 77 |
| Cyfra TIMP1 MDK | 3 | 0.81 | 24 | 55 | 0.98 | 76 | 92 |
| Cyfra SLPI TIMP1 SCC TFPI MDK | 6 | 0.81 | 48 | 55 | 0.99 | 91 | 92 |
| Cyfra TIMP1 SCC TFPI | 4 | 0.81 | 52 | 55 | 0.98 | 76 | 92 |
| Cyfra SLPI TIMP1 SCC TFPI | 5 | 0.81 | 52 | 55 | 0.98 | 79 | 92 |
| Cyfra SCC TFPI CEACAM5 | 4 | 0.81 | 29 | 55 | 0.99 | 82 | 92 |
| TIMP1 SCC TFPI MMP2 MDK | 5 | 0.81 | 33 | 45 | 0.99 | 91 | 92 |
| SLPI TIMP1 SCC CEACAM5 MDK | 5 | 0.81 | 29 | 64 | 0.97 | 73 | 77 |
| SLPI TIMP1 SCC TFPI CEACAM5 MMP2 | 6 | 0.81 | 24 | 55 | 0.99 | 88 | 92 |
| Cyfra SLPI MDK | 3 | 0.81 | 24 | 55 | 0.98 | 76 | 85 |
| Cyfra SLPI TIMP1 SCC MDK | 5 | 0.81 | 43 | 55 | 1.00 | 94 | 92 |
| TIMP1 SCC CEACAM5 MDK | 4 | 0.81 | 29 | 64 | 0.97 | 73 | 77 |
| Cyfra SLPI SCC TFPI CEACAM5 MDK | 6 | 0.80 | 0 | 73 | 1.00 | 100 | 100 |
| Cyfra TIMP1 OPN MDK | 4 | 0.80 | 38 | 55 | 0.99 | 91 | 92 |
| Cyfra SLPI SCC TFPI MDK | 5 | 0.80 | 24 | 55 | 0.98 | 73 | 92 |
| Cyfra TIMP1 SCC OPN MDK | 5 | 0.80 | 38 | 55 | 0.99 | 91 | 92 |
| Cyfra TFPI MDK | 3 | 0.80 | 24 | 55 | 0.98 | 76 | 92 |
| Cyfra SLPI TFPI MDK | 4 | 0.80 | 24 | 55 | 0.98 | 76 | 92 |
| Cyfra TIMP1 TFPI CEACAM5 | 4 | 0.80 | 5 | 55 | 0.99 | 94 | 85 |
| SCC TFPI MMP2 MDK | 4 | 0.80 | 29 | 45 | 0.99 | 91 | 92 |
| SCC CEACAM5 OPN MDK | 4 | 0.80 | 33 | 55 | 0.97 | 85 | 77 |
| Cyfra SLPI TIMP1 TFPI CEACAM5 | 5 | 0.80 | 10 | 55 | 0.99 | 94 | 85 |
| SLPI SCC CEACAM5 OPN MDK | 5 | 0.80 | 33 | 55 | 0.97 | 82 | 77 |
| Cyfra TIMP1 SCC MDK | 4 | 0.80 | 38 | 55 | 0.99 | 82 | 92 |
| Cyfra TIMP1 SCC TFPI MDK | 5 | 0.80 | 38 | 55 | 0.99 | 82 | 92 |
| SLPI TIMP1 SCC CEACAM5 OPN MDK | 6 | 0.80 | 29 | 55 | 0.97 | 79 | 77 |
| Cyfra SCC MDK | 3 | 0.80 | 24 | 55 | 0.98 | 70 | 92 |
| Cyfra TIMP1 SCC CEACAM5 OPN | 5 | 0.80 | 0 | 64 | 1.00 | 100 | 100 |
| Cyfra SLPI TIMP1 SCC | 4 | 0.80 | 38 | 55 | 0.98 | 70 | 92 |
| SCC CEACAM5 MDK | 3 | 0.80 | 29 | 55 | 0.96 | 76 | 77 |
| SLPI SCC CEACAM5 MDK | 4 | 0.80 | 29 | 55 | 0.97 | 76 | 77 |
| TIMP1 SCC CEACAM5 OPN MDK | 5 | 0.80 | 29 | 55 | 0.97 | 76 | 77 |
| SLPI SCC TFPI CEACAM5 MDK | 5 | 0.79 | 0 | 64 | 1.00 | 100 | 100 |
| Cyfra TIMP1 SCC | 3 | 0.79 | 38 | 55 | 0.98 | 70 | 92 |
| Cyfra SLPI TIMP1 MDK | 4 | 0.79 | 24 | 55 | 0.98 | 79 | 92 |
| Cyfra TIMP1 | 2 | 0.79 | 33 | 55 | 0.97 | 79 | 85 |
| Cyfra SLPI SCC TFPI MMP2 MDK | 6 | 0.79 | 0 | 55 | 1.00 | 100 | 100 |
| Cyfra SLPI SCC TFPI | 4 | 0.79 | 38 | 55 | 0.95 | 39 | 92 |
| Cyfra SCC TFPI CEACAM5 MMP2 OPN | 6 | 0.79 | 0 | 73 | 1.00 | 100 | 100 |
| Cyfra SLPI TIMP1 TFPI OPN | 5 | 0.79 | 5 | 55 | 0.99 | 88 | 92 |
| Cyfra SCC TFPI CEACAM5 OPN | 5 | 0.79 | 0 | 64 | 1.00 | 100 | 100 |
| TIMP1 SCC TFPI MMP2 OPN | 5 | 0.79 | 43 | 55 | 0.99 | 91 | 92 |
| SLPI TIMP1 SCC TFPI MMP2 OPN | 6 | 0.79 | 43 | 55 | 0.99 | 91 | 92 |
| SLPI TIMP1 SCC TFPI MMP2 MDK | 6 | 0.79 | 0 | 45 | 0.99 | 88 | 92 |
| Cyfra OPN MDK | 3 | 0.79 | 29 | 55 | 0.99 | 91 | 92 |
| Cyfra SLPI TIMP1 TFPI OPN MDK | 6 | 0.79 | 0 | 55 | 1.00 | 97 | 100 |
| Cyfra TIMP1 SCC CEACAM5 MDK | 5 | 0.78 | 0 | 55 | 1.00 | 100 | 100 |
| SCC TFPI CEACAM5 MDK | 4 | 0.78 | 0 | 64 | 1.00 | 97 | 100 |
| TIMP1 SCC TFPI CEACAM5 MDK | 5 | 0.78 | 0 | 64 | 1.00 | 100 | 100 |
| Cyfra SLPI TIMP1 SCC TFPI CEACAM5 MDK | 7 | 0.78 | 0 | 55 | 1.00 | 100 | 100 |
| TIMP1 SCC TFPI MMP2 | 4 | 0.78 | 33 | 55 | 0.98 | 76 | 92 |
| SLPI TIMP1 SCC TFPI MMP2 | 5 | 0.78 | 33 | 55 | 0.98 | 76 | 92 |
| Cyfra SLPI TIMP1 SCC TFPI CEACAM5 MMP2 OPN | 8 | 0.78 | 0 | 55 | 1.00 | 100 | 100 |
| SLPI SCC TFPI MMP2 MDK | 5 | 0.78 | 0 | 45 | 0.99 | 91 | 92 |
| Cyfra TIMP1 OPN | 3 | 0.78 | 5 | 55 | 0.99 | 91 | 92 |
| Cyfra SLPI TIMP1 OPN | 4 | 0.78 | 5 | 55 | 0.99 | 91 | 92 |
| Cyfra TIMP1 TFPI OPN | 4 | 0.78 | 5 | 55 | 0.99 | 88 | 92 |
| SLPI TIMP1 SCC TFPI CEACAM5 MDK | 6 | 0.78 | 0 | 64 | 1.00 | 100 | 100 |
| SLPI SCC TFPI CEACAM5 OPN MDK | 6 | 0.78 | 0 | 55 | 1.00 | 100 | 100 |
| Cyfra SLPI OPN MDK | 4 | 0.78 | 29 | 55 | 0.99 | 91 | 92 |
| SLPI TIMP1 SCC MDK | 4 | 0.78 | 24 | 55 | 0.93 | 42. | 85 |
| SCC TFPI MMP2 OPN MDK | 5 | 0.78 | 0 | 45 | 1.00 | 94 | 92 |
| Cyfra SLPI SCC MDK | 4 | 0.77 | 24 | 55 | 0.98 | 73 | 92 |
| Cyfra SLPI TIMP1 SCC TFPI CEACAM5 MMP2 MDK | 8 | 0.77 | 0 | 55 | 1.00 | 100 | 100 |
| Cyfra OPN | 2 | 0.77 | 5 | 55 | 0.98 | 76 | 92 |
| TIMP1 TFPI | 2 | 0.77 | 5 | 9 | 0.61 | 3 | 0 |
| Cyfra SCC TFPI | 3 | 0.77 | 24 | 55 | 0.95 | 33 | 92 |
| Cyfra SLPI TIMP1 SCC TFPI CEACAM5 MMP2 OPN MDK | 9 | 0.77 | 0 | 55 | 1.00 | 100 | 100 |
| SLPI SCC MDK | 3 | 0.77 | 24 | 55 | 0.93 | 42 | 69 |
| TIMP1 SCC MDK | 3 | 0.77 | 24 | 55 | 0.93 | 33 | 85 |
| Cyfra TIMP1 SCC TFPI CEACAM5 MMP2 OPN | 7 | 0.77 | 0 | 27 | 1.00 | 100 | 100 |
| Cyfra SCC CEACAM5 OPN | 4 | 0.77 | 0 | 55 | 1.00 | 100 | 100 |
| Cyfra SCC CEACAM5 | 3 | 0.77 | 19 | 55 | 0.99 | 82 | 92 |
| Cyfra SLPI OPN | 3 | 0.77 | 5 | 55 | 0.98 | 76 | 92 |
| Cyfra SLPI SCC CEACAM5 | 4 | 0.77 | 19 | 55 | 0.99 | 82 | 92 |
| SCC TFPI CEACAM5 MMP2 | 4 | 0.77 | 24 | 55 | 0.98 | 79 | 85 |
| Cyfra SLPI SCC TFPI CEACAM5 | 5 | 0.77 | 19 | 55 | 0.99 | 82 | 92 |
| SLPI SCC TFPI CEACAM5 MMP2 | 5 | 0.77 | 19 | 55 | 0.98 | 82 | 85 |
| SCC MDK | 2 | 0.77 | 24 | 55 | 0.93 | 39 | 69 |
| SLPI TIMP1 SCC OPN MDK | 5 | 0.77 | 24 | 55 | 0.94 | 45 | 85 |
| Cyfra TIMP1 SCC TFPI CEACAM5 MMP2 OPN MDK | 8 | 0.77 | 0 | 27 | 1.00 | 100 | 100 |
| TIMP1 SCC OPN MDK | 4 | 0.77 | 24 | 55 | 0.93 | 42 | 85 |
| Cyfra MMP2 OPN | 3 | 0.77 | 10 | 55 | 0.99 | 88 | 92 |
| Cyfra SLPI MMP2 OPN | 4 | 0.77 | 10 | 55 | 0.99 | 88 | 92 |
| Cyfra TFPI OPN | 3 | 0.77 | 5 | 55 | 0.98 | 76 | 92 |
| Cyfra SLPI TFPI OPN | 4 | 0.77 | 5 | 55 | 0.98 | 76 | 92 |
| Cyfra SCC TFPI OPN | 4 | 0.77 | 5 | 55 | 0.98 | 70 | 92 |
| Cyfra SLPI TIMP1 SCC CEACAM5 | 5 | 0.77 | 0 | 55 | 1.00 | 100 | 100 |
| Cyfra SLPI SCC TFPI OPN | 5 | 0.77 | 5 | 55 | 0.98 | 70 | 92 |
| Cyfra TIMP1 SCC TFPI CEACAM5 | 5 | 0.77 | 0 | 64 | 1.00 | 100 | 100 |
| Cyfra SCC TFPI MDK | 4 | 0.77 | 24 | 55 | 0.98 | 70 | 92 |
| Cyfra SLPI TIMP1 | 3 | 0.77 | 29 | 55 | 0.98 | 88 | 85 |
| Cyfra SLPI TIMP1 OPN MDK | 5 | 0.77 | 0 | 55 | 1.00 | 97 | 100 |
| Cyfra SLPI TIMP1 TFPI MMP2 OPN | 6 | 0.76 | 0 | 45 | 1.00 | 100 | 100 |
| Cyfra TIMP1 SCC OPN | 4 | 0.76 | 5 | 55 | 0.99 | 91 | 92 |
| Cyfra SLPI TIMP1 SCC OPN | 5 | 0.76 | 5 | 55 | 0.99 | 91 | 92 |
| Cyfra SLPI TIMP1 SCC TFPI CEACAM5 MMP2 | 7 | 0.76 | 0 | 55 | 1.00 | 100 | 100 |
| Cyfra SLPI SCC TFPI MMP2 OPN MDK | 7 | 0.76 | 0 | 45 | 1.00 | 100 | 100 |
| SCC OPN MDK | 3 | 0.76 | 24 | 55 | 0.93 | 42 | 77 |
| SLPI SCC CEACAM5 OPN | 4 | 0.76 | 24 | 55 | 0.94 | 70 | 69 |
| SLPI SCC OPN MDK | 4 | 0.76 | 24 | 55 | 0.93 | 48 | 77 |
| Cyfra TIMP1 MMP2 | 3 | 0.76 | 10 | 55 | 0.99 | 91 | 92 |
| Cyfra TIMP1 MMP2 OPN | 4 | 0.76 | 0 | 55 | 1.00 | 100 | 100 |
| Cyfra TIMP1 TFPI MMP2 OPN | 5 | 0.76 | 0 | 55 | 1.00 | 100 | 100 |
| Cyfra TIMP1 SCC TFPI CEACAM5 MMP2 MDK | 7 | 0.76 | 0 | 55 | 1.00 | 100 | 100 |
| SLPI SCC CEACAM5 | 3 | 0.76 | 19 | 55 | 0.94 | 64 | 62 |
| SLPI TIMP1 SCC CEACAM5 | 4 | 0.76 | 19 | 55 | 0.94 | 64 | 69 |
| Cyfra TIMP1 SCC TFPI MMP2 OPN | 6 | 0.76 | 0 | 55 | 1.00 | 100 | 100 |
| Cyfra SLPI TIMP1 TFPI MMP2 OPN MDK | 7 | 0.76 | 0 | 55 | 1.00 | 100 | 100 |
| Cyfra TIMP1 SCC TFPI MMP2 OPN MDK | 7 | 0.76 | 0 | 55 | 1.00 | 100 | 100 |
| Cyfra SLPI TFPI | 3 | 0.76 | 14 | 55 | 0.97 | 73 | 77 |
| Cyfra TFPI OPN MDK | 4 | 0.76 | 29 | 55 | 0.99 | 88 | 92 |
| Cyfra SLPI TFPI OPN MDK | 5 | 0.76 | 29 | 55 | 0.99 | 91 | 92 |
| Cyfra TIMP1 CEACAM5 MMP2 OPN MDK | 6 | 0.75 | 0 | 55 | 1.00 | 100 | 100 |
| Cyfra SLPI TIMP1 CEACAM5 MMP2 OPN MDK | 7 | 0.75 | 0 | 55 | 1.00 | 100 | 100 |
| TIMP1 SCC CEACAM5 | 3 | 0.75 | 19 | 55 | 0.94 | 67 | 62 |
| Cyfra SLPI TFPI CEACAM5 MDK | 5 | 0.75 | 0 | 64 | 0.99 | 91 | 92 |
| Cyfra MMP2 MDK | 3 | 0.75 | 29 | 55 | 1.00 | 97 | 100 |
| Cyfra TIMP1 TFPI MDK | 4 | 0.75 | 24 | 55 | 0.98 | 79 | 92 |
| Cyfra CEACAM5 MMP2 MDK | 4 | 0.75 | 0 | 45 | 1.00 | 97 | 100 |
| SCC CEACAM5 OPN | 3 | 0.75 | 24 | 55 | 0.94 | 67 | 69 |
| TIMP1 SCC CEACAM5 OPN | 4 | 0.75 | 19 | 55 | 0.94 | 70 | 69 |
| SLPI TIMP1 SCC CEACAM5 OPN | 5 | 0.75 | 19 | 55 | 0.94 | 67 | 69 |
| Cyfra SCC OPN | 3 | 0.75 | 5 | 55 | 0.98 | 70 | 92 |
| Cyfra SLPI SCC OPN | 4 | 0.75 | 5 | 55 | 0.98 | 70 | 92 |
| Cyfra SLPI TIMP1 MMP2 OPN | 5 | 0.75 | 0 | 55 | 1.00 | 100 | 100 |
| Cyfra TIMP1 CEACAM5 MMP2 OPN | 5 | 0.75 | 0 | 55 | 1.00 | 100 | 100 |
| Cyfra SLPI TIMP1 TFPI CEACAM5 MMP2 OPN MDK | 8 | 0.75 | 0 | 55 | 1.00 | 100 | 100 |
| Cyfra SLPI TIMP1 MMP2 | 4 | 0.75 | 10 | 55 | 0.99 | 94 | 92 |
| Cyfra TFPI CEACAM5 MDK | 4 | 0.75 | 0 | 64 | 0.99 | 91 | 92 |
| Cyfra SCC TFPI CEACAM5 MMP2 MDK | 6 | 0.75 | 0 | 45 | 1.00 | 100 | 100 |
| SLPI SCC CEACAM5 MMP2 | 4 | 0.75 | 10 | 55 | 0.98 | 79 | 92 |
| SCC TFPI MMP2 OPN | 4 | 0.75 | 33 | 55 | 0.98 | 85 | 85 |
| SLPI TIMP1 SCC CEACAM5 MMP2 | 5 | 0.75 | 10 | 55 | 0.98 | 79 | 92 |
| SLPI SCC TFPI MMP2 OPN | 5 | 0.75 | 33 | 55 | 0.98 | 85 | 85 |
| Cyfra SLPI TIMP1 TFPI | 4 | 0.75 | 19 | 55 | 0.98 | 88 | 77 |
| Cyfra SLPI TIMP1 TFPI MDK | 5 | 0.75 | 24 | 55 | 0.98 | 79 | 92 |
| SLPI SCC CEACAM5 MMP2 MDK | 5 | 0.75 | 0 | 55 | 1.00 | 94 | 92 |
| Cyfra SLPI TIMP1 CEACAM5 MMP2 OPN | 6 | 0.74 | 0 | 55 | 1.00 | 100 | 100 |
| Cyfra SLPI TIMP1 TFPI CEACAM5 MMP2 OPN | 7 | 0.74 | 0 | 55 | 1.00 | 100 | 100 |
| SCC CEACAM5 | 2 | 0.74 | 19 | 55 | 0.94 | 70 | 54 |
| SCC TFPI CEACAM5 MMP2 OPN | 5 | 0.74 | 0 | 55 | 0.99 | 85 | 92 |
| Cyfra TIMP1 SCC MMP2 OPN MDK | 6 | 0.74 | 0 | 55 | 1.00 | 100 | 100 |
| TFPI | 1 | 0.74 | 33 | 36 | 0.52 | 6 | 8 |
| SCC CEACAM5 MMP2 | 3 | 0.74 | 5 | 55 | 0.98 | 73 | 92 |
| TIMP1 SCC CEACAM5 MMP2 | 4 | 0.74 | 14 | 55 | 0.98 | 76 | 92 |
| Cyfra | 1 | 0.74 | 10 | 55 | 0.96 | 76 | 77 |
| SLPI SCC TFPI MMP2 | 4 | 0.74 | 29 | 55 | 0.98 | 82 | 85 |
| Cyfra TIMP1 TFPI MMP2 OPN MDK | 6 | 0.74 | 0 | 55 | 1.00 | 100 | 100 |
| SLPI TIMP1 SCC MMP2 MDK | 5 | 0.74 | 24 | 55 | 0.98 | 76 | 92 |
| SCC CEACAM5 MMP2 MDK | 4 | 0.74 | 0 | 55 | 1.00 | 94 | 92 |
| Cyfra SLPI TIMP1 SCCTFPI MMP2 OPN | 7 | 0.74 | 0 | 45 | 1.00 | 100 | 100 |
| Cyfra SLPI TIMP1 SCC TFPI MMP2 OPN MDK | 8 | 0.74 | 0 | 45 | 1.00 | 100 | 100 |
| SLPI SCC TFPI MMP2 OPN MDK | 6 | 0.74 | 0 | 45 | 1.00 | 100 | 100 |
| SLPI TIMP1 SCC TFPI MMP2 OPN MDK | 7 | 0.74 | 0 | 55 | 1.00 | 100 | 100 |
| Cyfra TIMP1 TFPI MMP2 | 4 | 0.74 | 10 | 55 | 0.99 | 94 | 85 |
| Cyfra TFPI MMP2 OPN | 4 | 0.74 | 10 | 55 | 1.00 | 94 | 92 |
| Cyfra SLPI TIMP1 TFPI MMP2 | 5 | 0.74 | 10 | 55 | 0.99 | 94 | 85 |
| Cyfra SLPI TFPI MMP2 OPN | 5 | 0.74 | 10 | 55 | 1.00 | 94 | 92 |
| SLPI SCC TFPI CEACAM5 MMP2 OPN | 6 | 0.74 | 0 | 55 | 0.99 | 85 | 92 |
| SCC TFPI MMP2 | 3 | 0.74 | 29 | 55 | 0.97 | 79 | 85 |
| Cyfra SLPI TIMP1 CEACAM5 MDK | 5 | 0.74 | 0 | 55 | 1.00 | 94 | 92 |
| Cyfra SLPI TIMP1 MMP2 OPN MDK | 6 | 0.74 | 0 | 55 | 1.00 | 100 | 100 |
| Cyfra TIMP1 TFPI OPN MDK | 5 | 0.74 | 0 | 55 | 0.99 | 91 | 92 |
| Cyfra TFPI CEACAM5 | 3 | 0.74 | 0 | 45 | 0.98 | 94 | 69 |
| Cyfra SCC TFPI MMP2 OPN | 5 | 0.74 | 10 | 55 | 0.99 | 88 | 92 |
| SLPI SCC CEACAM5 MMP2 OPN | 5 | 0.74 | 0 | 55 | 0.99 | 91 | 85 |
| Cyfra TIMP1 TFPI | 3 | 0.74 | 5 | 55 | 0.97 | 82 | 77 |
| TIMP1 SCC MMP2 OPN MDK | 5 | 0.74 | 14 | 55 | 0.98 | 79 | 85 |
| SLPI TIMP1 SCC MMP2 OPN MDK | 6 | 0.74 | 14 | 55 | 0.98 | 79 | 85 |
| Cyfra SLPI CEACAM5 MDK | 4 | 0.74 | 0 | 64 | 1.00 | 94 | 92 |
| TIMP1 SCC MMP2 MDK | 4 | 0.74 | 19 | 55 | 0.98 | 76 | 92 |
| SCC MMP2 MDK | 3 | 0.74 | 33 | 55 | 0.98 | 82 | 85 |
| SLPI SCC MMP2 MDK | 4 | 0.74 | 33 | 55 | 0.98 | 82 | 85 |
| SCC MMP2 OPN MDK | 4 | 0.74 | 33 | 55 | 0.98 | 85 | 85 |
| SLPI SCC MMP2 OPN MDK | 5 | 0.74 | 33 | 55 | 0.98 | 82 | 85 |
| Cyfra SCC MMP2 OPN | 4 | 0.73 | 10 | 55 | 0.99 | 91 | 92 |
| Cyfra SLPI SCC MMP2 OPN | 5 | 0.73 | 10 | 55 | 0.99 | 91 | 92 |
| Cyfra TIMP1 MMP2 OPN MDK | 5 | 0.73 | 0 | 55 | 1.00 | 100 | 100 |
| Cyfra SLPI SCC TFPI MMP2 OPN | 6 | 0.73 | 10 | 55 | 0.99 | 88 | 92 |
| Cyfra TIMP1 CEACAM5 MMP2 | 4 | 0.73 | 10 | 55 | 0.99 | 94 | 77 |
| SCC CEACAM5 MMP2 OPN | 4 | 0.73 | 0 | 55 | 0.99 | 91 | 85 |
| Cyfra SLPI TIMP1 CEACAM5 MMP2 | 5 | 0.73 | 10 | 55 | 0.99 | 94 | 77 |
| Cyfra TIMP1 CEACAM5 MDK | 4 | 0.73 | 0 | 55 | 0.99 | 94 | 92 |
| Cyfra MMP2 | 2 | 0.73 | 10 | 55 | 0.98 | 94 | 69 |
| Cyfra SLPI MMP2 | 3 | 0.73 | 10 | 55 | 0.98 | 91 | 69 |
| Cyfra SLPI TFPI MMP2 | 4 | 0.73 | 10 | 55 | 0.98 | 88 | 69 |
| Cyfra TFPI MMP2 MDK | 4 | 0.73 | 0 | 55 | 1.00 | 100 | 100 |
| Cyfra TIMP1 TFPI MMP2 MDK | 5 | 0.73 | 0 | 55 | 1.00 | 100 | 100 |
| Cyfra SLPI TFPI MMP2 OPN MDK | 6 | 0.73 | 0 | 55 | 1.00 | 100 | 100 |
| Cyfra CEACAM5 MDK | 3 | 0.73 | 0 | 55 | 0.99 | 94 | 92 |
| Cyfra TFPI MMP2 | 3 | 0.72 | 10 | 55 | 0.98 | 94 | 69 |
| Cyfra TIMP1 TFPI CEACAM5 MMP2 | 5 | 0.72 | 10 | 55 | 0.99 | 91 | 92 |
| Cyfra TFPI CEACAM5 MMP2 MDK | 5 | 0.72 | 0 | 45 | 1.00 | 100 | 100 |
| SCC OPN | 2 | 0.72 | 5 | 55 | 0.90 | 36 | 69 |
| Cyfra CEACAM5 OPN | 3 | 0.72 | 0 | 45 | 0.99 | 91 | 92 |
| SLPI SCC OPN | 3 | 0.72 | 5 | 55 | 0.90 | 36 | 69 |
| Cyfra SLPI CEACAM5 OPN | 4 | 0.72 | 0 | 45 | 0.99 | 91 | 92 |
| SLPI SCC | 2 | 0.72 | 10 | 55 | 0.91 | 24 | 69 |
| TIMP1 SCC MMP2 | 3 | 0.72 | 10 | 55 | 0.98 | 76 | 92 |
| SLPI TIMP1 SCC MMP2 | 4 | 0.72 | 10 | 55 | 0.98 | 76 | 92 |
| TIMP1 SCC OPN | 3 | 0.72 | 5 | 55 | 0.90 | 36 | 69 |
| SLPI TIMP1 SCC OPN | 4 | 0.72 | 5 | 55 | 0.90 | 36 | 69 |
| SCC | 1 | 0.71 | 0 | 55 | 0.91 | 24 | 62 |
| Cyfra SLPI SCC CEACAM5 OPN MDK | 6 | 0.71 | 0 | 64 | 1.00 | 100 | 100 |
| SLPI TIMP1 TFPI CEACAM5 MMP2 MDK | 6 | 0.71 | 38 | 27 | 0.93 | 70 | 62 |
| Cyfra SLPI CEACAM5 MMP2 | 4 | 0.71 | 10 | 55 | 0.98 | 85 | 77 |
| Cyfra SCC TFPI MMP2 | 4 | 0.71 | 10 | 55 | 0.99 | 91 | 92 |
| Cyfra TFPI CEACAM5 MMP2 | 4 | 0.71 | 10 | 55 | 0.98 | 88 | 69 |
| TFPI CEACAM5 MMP2 MDK | 4 | 0.71 | 38 | 27 | 0.92 | 58 | 62 |
| TIMP1 TFPI CEACAM5 MMP2 MDK | 5 | 0.71 | 38 | 27 | 0.92 | 61 | 62 |
| Cyfra SLPI TFPI CEACAM5 | 4 | 0.71 | 0 | 45 | 0.98 | 91 | 69 |
| Cyfra TIMP1 SCC MMP2 | 4 | 0.71 | 0 | 55 | 1.00 | 100 | 100 |
| Cyfra TIMP1 SCC TFPI MMP2 | 5 | 0.71 | 0 | 55 | 1.00 | 100 | 100 |
| Cyfra TIMP1 SCC MMP2 OPN | 5 | 0.71 | 0 | 55 | 1.00 | 100 | 100 |
| Cyfra TIMP1 SCC MMP2 MDK | 5 | 0.71 | 0 | 55 | 1.00 | 100 | 100 |
| Cyfra SLPI TIMP1 SCC MMP2 OPN | 6 | 0.71 | 0 | 55 | 1.00 | 100 | 100 |
| Cyfra SLPI TIMP1 SCC MMP2 MDK | 6 | 0.71 | 0 | 55 | 1.00 | 100 | 100 |
| Cyfra SLPI TIMP1 SCC CEACAM5 MMP2 OPN | 7 | 0.71 | 0 | 55 | 1.00 | 100 | 100 |
| Cyfra SLPI TIMP1 SCC MMP2 OPN MDK | 7 | 0.71 | 0 | 55 | 1.00 | 100 | 100 |
| Cyfra SLPI TIMP1 SCC CEACAM5 MMP2 OPN MDK | 8 | 0.71 | 0 | 55 | 1.00 | 100 | 100 |
| TIMP1 SCC | 2 | 0.71 | 0 | 55 | 0.91 | 24 | 69 |
| Cyfra CEACAM5 MMP2 | 3 | 0.71 | 10 | 55 | 0.98 | 88 | 69 |
| SLPI TIMP1 SCC | 3 | 0.71 | 0 | 55 | 0.91 | 24 | 69 |
| Cyfra CEACAM5 MMP2 OPN | 4 | 0.71 | 0 | 45 | 0.99 | 91 | 92 |
| TIMP1 SCC MMP2 OPN | 4 | 0.71 | 10 | 55 | 0.98 | 79 | 85 |
| Cyfra SLPI SCC CEACAM5 OPN | 5 | 0.71 | 0 | 64 | 1.00 | 100 | 100 |
| Cyfra SLPI TFPI CEACAM5 MMP2 | 5 | 0.71 | 10 | 55 | 0.98 | 85 | 77 |
| Cyfra SLPI CEACAM5 MMP2 OPN | 5 | 0.71 | 0 | 45 | 0.99 | 91 | 92 |
| Cyfra TIMP1 TFPI CEACAM5 MDK | 5 | 0.71 | 0 | 64 | 1.00 | 97 | 100 |
| Cyfra SCC CEACAM5 OPN MDK | 5 | 0.71 | 0 | 64 | 1.00 | 100 | 100 |
| SLPI TIMP1 SCC MMP2 OPN | 5 | 0.71 | 10 | 55 | 0.98 | 79 | 85 |
| Cyfra SLPI TIMP1 TFPI CEACAM5 MDK | 6 | 0.71 | 0 | 64 | 1.00 | 94 | 92 |
| Cyfra SCC CEACAM5 MMP2 OPN | 5 | 0.71 | 0 | 55 | 1.00 | 100 | 100 |
| Cyfra SLPI SCC CEACAM5 MMP2 OPN | 6 | 0.71 | 0 | 55 | 1.00 | 100 | 100 |
| Cyfra SCC | 2 | 0.71 | 0 | 55 | 0.95 | 36 | 92 |
| Cyfra SLPI SCC | 3 | 0.71 | 0 | 55 | 0.95 | 39 | 92 |
| Cyfra SLPI SCC TFPI MMP2 | 5 | 0.71 | 10 | 55 | 0.99 | 88 | 92 |
| Cyfra TIMP1 SCC TFPI CEACAM5 MDK | 6 | 0.71 | 0 | 55 | 1.00 | 100 | 100 |
| Cyfra TIMP1 SCC TFPI MMP2 MDK | 6 | 0.71 | 0 | 55 | 1.00 | 100 | 100 |
| Cyfra SCC MMP2 | 3 | 0.71 | 10 | 55 | 0.99 | 88 | 92 |
| Cyfra SLPI SCC MMP2 | 4 | 0.71 | 10 | 55 | 0.99 | 88 | 92 |
| Cyfra SCC CEACAM5 MDK | 4 | 0.71 | 0 | 64 | 1.00 | 100 | 100 |
| SLPI TFPI CEACAM5 MMP2 MDK | 5 | 0.71 | 38 | 27 | 0.93 | 70 | 62 |
| Cyfra SLPI TIMP1 SCC MMP2 | 5 | 0.70 | 0 | 55 | 1.00 | 100 | 100 |
| Cyfra TIMP1 SCC CEACAM5 MMP2 | 5 | 0.70 | 0 | 55 | 1.00 | 100 | 100 |
| Cyfra SLPI TIMP1 SCC TFPI MMP2 | 6 | 0.70 | 0 | 55 | 1.00 | 100 | 100 |
| Cyfra SLPI TIMP1 SCC CEACAM5 MMP2 | 6 | 0.70 | 0 | 55 | 1.00 | 100 | 100 |
| Cyfra SLPI TIMP1 SCC CEACAM5 MDK | 6 | 0.70 | 0 | 55 | 1.00 | 100 | 100 |
| Cyfra TIMP1 SCC TFPI CEACAM5 MMP2 | 6 | 0.70 | 0 | 55 | 1.00 | 100 | 100 |
| Cyfra TIMP1 SCC CEACAM5 MMP2 MDK | 6 | 0.70 | 0 | 55 | 1.00 | 100 | 100 |
| Cyfra TIMP1 TFPI CEACAM5 MMP2 OPN | 6 | 0.70 | 0 | 55 | 1.00 | 100 | 100 |
| Cyfra SLPI TIMP1 SCCTFPI MMP2 MDK | 7 | 0.70 | 0 | 55 | 1.00 | 100 | 100 |
| Cyfra SLPI TIMP1 SCC CEACAM5 MMP2 MDK | 7 | 0.70 | 0 | 55 | 1.00 | 100 | 100 |
| Cyfra SLPI SCC TFPI CEACAM5 MMP2 OPN | 7 | 0.70 | 0 | 64 | 1.00 | 100 | 100 |
| SCC MMP2 OPN | 3 | 0.70 | 10 | 55 | 0.97 | 82 | 85 |
| SLPI SCC MMP2 OPN | 4 | 0.70 | 10 | 55 | 0.97 | 82 | 85 |
| Cyfra SLPI TIMP1 SCC TFPI CEACAM5 | 6 | 0.70 | 0 | 55 | 1.00 | 100 | 100 |
| Cyfra SLPI SCC TFPI CEACAM5 OPN | 6 | 0.70 | 0 | 64 | 1.00 | 100 | 100 |
| Cyfra SLPI SCC CEACAM5 MDK | 5 | 0.70 | 0 | 64 | 1.00 | 100 | 100 |
| Cyfra SCC OPN MDK | 4 | 0.70 | 0 | 45 | 0.99 | 88 | 92 |
| TIMP1 TFPI CEACAM5 MDK | 4 | 0.70 | 0 | 45 | 0.74 | 24 | 15 |
| SCC MMP2 | 2 | 0.70 | 10 | 55 | 0.97 | 70 | 85 |
| SLPI SCC MMP2 | 3 | 0.70 | 10 | 55 | 0.97 | 70 | 85 |
| Cyfra TIMP1 SCC CEACAM5 MMP2 OPN | 6 | 0.70 | 0 | 55 | 1.00 | 100 | 100 |
| Cyfra TIMP1 TFPI CEACAM5 MMP2 OPN MDK | 7 | 0.70 | 0 | 55 | 1.00 | 100 | 100 |
| Cyfra SLPI | 2 | 0.70 | 0 | 55 | 0.97 | 88 | 77 |
| TFPI CEACAM5 MDK | 3 | 0.69 | 0 | 36 | 0.71 | 27 | 38 |
| SLPI TFPI CEACAM5 MDK | 4 | 0.69 | 0 | 36 | 0.72 | 33 | 38 |
| Cyfra SLPI TIMP1 TFPI CEACAM5 MMP2 | 6 | 0.69 | 0 | 55 | 0.99 | 97 | 100 |
| Cyfra TIMP1 SCC CEACAM5 MMP2 OPN MDK | 7 | 0.69 | 0 | 55 | 1.00 | 100 | 100 |
| Cyfra CEACAM5 | 2 | 0.69 | 0 | 45 | 0.98 | 94 | 69 |
| SLPI TIMP1 TFPI CEACAM5 MDK | 5 | 0.69 | 5 | 45 | 0.78 | 21 | 38 |
| Cyfra SLPI CEACAM5 | 3- | 0.69 | 5 | 45 | 0.98 | 91 | 69 |
| Cyfra TIMP1 CEACAM5 OPN | 4 | 0.69 | 0 | 45 | 1.00 | 100 | 100 |
| Cyfra SLPI TIMP1 CEACAM5 OPN | 5 | 0.69 | 0 | 45 | 1.00 | 100 | 100 |
| TIMP1 SCC CEACAM5 MMP2 OPN | 5 | 0.69 | 0 | 55 | 0.99 | 94 | 92 |
| Cyfra SCC TFPI MMP2 OPN MDK | 6 | 0.69 | 0 | 36 | 1.00 | 100 | 100 |
| TFPI MMP2 MDK | 3 | 0.68 | 38 | 18 | 0.92 | 55 | 62 |
| TIMP1 TFPI MMP2 MDK | 4 | 0.68 | 38 | 18 | 0.92 | 55 | 62 |
| Cyfra SLPI TIMP1 CEACAM5 OPN MDK | 6 | 0.68 | 0 | 45 | 1.00 | 100 | 100 |
| SLPI TIMP1 SCC CEACAM5 MMP2 OPN | 6 | 0.68 | 0 | 55 | 1.00 | 97 | 100 |
| Cyfra SLPI SCC OPN MDK | 5 | 0.68 | 0 | 45 | 0.99 | 91 | 92 |
| SLPI TFPI | 2 | 0.68 | 33 | 18 | 0.54 | 0 | 23 |
| TIMP1 TFPI CEACAM5 | 3 | 0.68 | 0 | 36 | 0.66 | 27 | 8 |
| SLPI TIMP1 TFPI MMP2 MDK | 5 | 0.68 | 29 | 18 | 0.93 | 64 | 69 |
| Cyfra TIMP1 CEACAM5 OPN MDK | 5 | 0.68 | 0 | 45 | 1.00 | 100 | 100 |
| TIMP1 SCC CEACAM5 MMP2 MDK | 5 | 0.68 | 0 | 55 | 1.00 | 97 | 100 |
| SLPI TFPI MMP2 MDK | 4 | 0.67 | 33 | 18 | 0.93 | 61 | 62 |
| TIMP1 SCC TFPI MMP2 OPN MDK | 6 | 0.67 | 0 | 55 | 1.00 | 100 | 100 |
| TIMP1 TFPI MDK | 3 | 0.67 | 24 | 36 | 0.67 | 9 | 8 |
| Cyfra SCC TFPI OPN MDK | 5 | 0.67 | 0 | 45 | 0.99 | 91 | 92 |
| Cyfra SLPI TIMP1 SCC OPN MDK | 6 | 0.67 | 0 | 45 | 1.00 | 97 | 100 |
| Cyfra TFPI MMP2 OPN MDK | 5 | 0.67 | 0 | 55 | 1.00 | 100 | 100 |
| SLPI TIMP1 TFPI CEACAM5 MMP2 | 5 | 0.67 | 14 | 27 | 0.90 | 58 | 54 |
| SLPI TIMP1 TFPI | 3 | 0.67 | 0 | 9 | 0.63 | 3 | 8 |
| SLPI TIMP1 TFPI CEACAM5 | 4 | 0.66 | 0 | 27 | 0.65 | 30 | 8 |
| Cyfra TIMP1 MMP2 MDK | 4 | 0.66 | 0 | 55 | 1.00 | 97 | 100 |
| SLPI TIMP1 TFPI MDK | 4 | 0.66 | 24 | 27 | 0.73 | 15 | 31 |
| TIMP1 TFPI CEACAM5 MMP2 | 4 | 0.66 | 14 | 27 | 0.90 | 61 | 54 |
| Cyfra TIMP1 TFPI CEACAM5 MMP2 MDK | 6 | 0.66 | 0 | 55 | 1.00 | 100 | 100 |
| Cyfra SLPI SCC TFPI OPN MDK | 6 | 0.66 | 0 | 45 | 0.99 | 91 | 92 |
| TFPI CEACAM5 MMP2 | 3 | 0.66 | 14 | 27 | 0.89 | 61 | 23 |
| TFPI CEACAM5 | 2 | 0.66 | 0 | 36 | 0.64 | 27 | 8 |
| TFPI MMP2 OPN MDK | 4 | 0.66 | 38 | 9 | 0.93 | 55 | 69 |
| TFPI MDK | 2 | 0.65 | 24 | 27 | 0.60 | 15 | 8 |
| TIMP1 TFPI MMP2 OPN MDK | 5 | 0.65 | 38 | 9 | 0.94 | 55 | 69 |
| TFPI CEACAM5 MMP2 OPN MDK | 5 | 0.65 | 33 | 9 | 0.93 | 55 | 69 |
| TIMP1 TFPI CEACAM5 MMP2 OPN MDK | 6 | 0.65 | 33 | 9 | 0.94 | 55 | 69 |
| SLPI TFPI CEACAMS | 3 | 0.65 | 0 | 36 | 0.64 | 27 | 8 |
| CEACAM5 MMP2 MDK | 3 | 0.65 | 38 | 18 | 0.90 | 64 | 54 |
| SLPI TFPI CEACAM5 MMP2 | 4 | 0.65 | 14 | 27 | 0.89 | 61 | 23 |
| TIMP1 CEACAM5 MMP2 MDK | 4 | 0.65 | 38 | 18 | 0.90 | 61 | 62 |
| Cyfra SLPI MMP2 OPN MDK | 5 | 0.65 | 0 | 55 | 1.00 | 100 | 100 |
| TFPI MMP2 | 2 | 0.65 | 14 | 9 | 0.90 | 67 | 15 |
| SLPI TFPI MMP2 OPN MDK | 5 | 0.65 | 33 | 9 | 0.95 | 64 | 77 |
| SLPI TIMP1 TFPI MMP2 OPN MDK | 6 | 0.65 | 33 | 9 | 0.95 | 64 | 77 |
| TFPI CEACAM5 MMP2 OPN | 4 | 0.65 | 14 | 18 | 0.90 | 48 | 38 |
| TIMP1 TFPI CEACAM5 MMP2 OPN | 5 | 0.65 | 14 | 18 | 0.91 | 42 | 69 |
| SLPI TFPI CEACAM5 MMP2 OPN MDK | 6 | 0.65 | 33 | 9 | 0.95 | 64 | 77 |
| SLPI TIMP1 TFPI CEACAM5 MMP2 OPN MDK | 7 | 0.65 | 33 | 9 | 0.96 | 70 | 77 |
| TIMP1 | 1 | 0.65 | 5 | 18 | 0.61 | 0 | 8 |
| Cyfra TFPI | 2 | 0.65 | 0 | 55 | 0.96 | 76 | 77 |
| SLPI TIMP1 TFPI CEACAM5 MMP2 OPN | 6 | 0.64 | 14 | 18 | 0.91 | 42 | 69 |
| SLPI TIMP1 TFPI MMP2 | 4 | 0.64 | 14 | 9 | 0.89 | 67 | 23 |
| SLPI CEACAM5 MMP2 MDK | 4 | 0.64 | 38 | 18 | 0.90 | 73 | 38 |
| SLPI TIMP1 CEACAM5 MMP2 MDK | 5 | 0.64 | 38 | 18 | 0.91 | 73 | 46 |
| SLPI TFPI MMP2 | 3 | 0.64 | 14 | 9 | 0.90 | 70 | 15 |
| Cyfra SLPI TIMP1 SCC TFP1 OPN MDK | 7 | 0.64 | 0 | 45 | 1.00 | 97 | 100 |
| SLPI TFPI CEACAM5 MMP2 OPN | 5 | 0.64 | 14 | 18 | 0.91 | 48 | 46 |
| TIMP1 MDK | 2 | 0.64 | 24 | 27 | 0.67 | 9 | 15 |
| SLPI TFPI MDK | 3 | 0.63 | 24 | 9 | 0.65 | 18 | 8 |
| TIMP1 TFPI MMP2 | 3 | 0.63 | 14 | 9 | 0.89 | 70 | 15 |
| CEACAM5 MMP2 | 2 | 0.63 | 10 | 18 | 0.89 | 61 | 38 |
| Cyfra SCC CEACAM5 MMP2 OPN MDK | 6 | 0.63 | 0 | 55 | 1.00 | 100 | 100 |
| Cyfra SLPI SCC CEACAM5 MMP2 OPN MDK | 7 | 0.63 | 0 | 55 | 1.00 | 100 | 100 |
| Cyfra TFPI CEACAM5 OPN | 4 | 0.62 | 0 | 45 | 1.00 | 97 | 100 |
| Cyfra SLPI TFPI CEACAM5 OPN | 5 | 0.62 | 0 | 45 | 1.00 | 94 | 92 |
| Cyfra TFPI CEACAM5 MMP2 OPN | 5 | 0.62 | 0 | 45 | 1.00 | 94 | 92 |
| Cyfra SLPI TFPI CEACAM5 MMP2 OPN | 6 | 0.62 | 0 | 45 | 1.00 | 94 | 92 |
| Cyfra SCC CEACAM5 MMP2 | 4 | 0.62 | 0 | 55 | 1.00 | 100 | 100 |
| Cyfra SLPI SCC CEACAM5 MMP2 | 5 | 0.62 | 0 | 55 | 1.00 | 100 | 100 |
| Cyfra SLPI SCC TFPI CEACAM5 MMP2 | 6 | 0.62 | 0 | 55 | 1.00 | 100 | 100 |
| Cyfra SLPI SCC CEACAM5 MMP2 MDK | 6 | 0.62 | 0 | 55 | 1.00 | 100 | 100 |
| CEACAM5 MMP2 OPN MDK | 4 | 0.62 | 38 | 18 | 0.90 | 45 | 69 |
| TIMP1 CEACAM5 MMP2 | 3 | 0.62 | 10 | 18 | 0.89 | 58 | 38 |
| SLPI TIMP1 CEACAM5 MMP2 | 4 | 0.62 | 10 | 18 | 0.89 | 58 | 38 |
| Cyfra SCC TFPI CEACAM5 MMP2 | 5 | 0.62 | 0 | 55 | 1.00 | 100 | 100 |
| SCC CEACAM5 MMP2 OPN MDK | 5 | 0.62 | 0 | 55 | 1.00 | 97 | 100 |
| TIMP1 CEACAM5 MMP2 OPN MDK | 5 | 0.61 | 38 | 18 | 0.90 | 45 | 77 |
| Cyfra SLPI SCC MMP2 OPN MDK | 6 | 0.61 | 0 | 45 | 1.00 | 100 | 100 |
| SLPI CEACAM5 MMP2 | 3 | 0.61 | 10 | 18 | 0.89 | 61 | 38 |
| Cyfra SCC CEACAM5 MMP2 MDK | 5 | 0.61 | 0 | 55 | 1.00 | 100 | 100 |
| SLPI TIMP1 SCC TFPI CEACAM5 MMP2 OPN | 7 | 0.61 | 0 | 55 | 1.00 | 100 | 100 |
| Cyfra MMP2 OPN MDK | 4 | 0.61 | 0 | 55 | 1.00 | 100 | 100 |
| Cyfra TIMP1 TFPI CEACAM55 OPN | 5 | 0.61 | 0 | 45 | 1.00 | 100 | 100 |
| Cyfra SLPI TFPI CEACAM5 MMP2 OPN MDK | 7 | 0.61 | 0 | 55 | 1.00 | 100 | 100 |
| SLPI TIMP1 SCC TFPI CEACAM5 MMP2 OPN MDK | 8 | 0.61 | 0 | 55 | 1.00 | 100 | 100 |
| CEACAM5 MMP2 OPN | 3 | 0.61 | 10 | 18 | 0.90 | 48 | 62 |
| Cyfra TIMP1 CEACAM5 MMP2 MDK | 5 | 0.61 | 0 | 55 | 1.00 | 100 | 100 |
| Cyfra TFPI CEACAM5 MMP2 OPN MDK | 6 | 0.61 | 0 | 45 | 1.00 | 100 | 100 |
| TIMP1 SCC TFPI CEACAM5 MMP2 OPN MDK | 7 | 0.61 | 0 | 55 | 1.00 | 100 | 100 |
| SLPI CEACAM5 MMP2 OPN MDK | 5 | 0.60 | 38 | 18 | 0.90 | 48 | 54 |
| SLPI TIMP1 CEACAM5 MMP2 OPN MDK | 6 | 0.60 | 38 | 18 | 0.90 | 52 | 46 |
| Cyfra CEACAM5 OPN MDK | 4 | 0.60 | 0 | 45 | 1.00 | 100 | 100 |
| Cyfra SLPI CEACAM5 OPN MDK | 5 | 0.60 | 0 | 45 | 1.00 | 100 | 100 |
| Cyfra SLPI TIMP1 TFPI CEACAM5 OPN | 6 | 0.60 | 0 | 45 | 1.00 | 100 | 100 |
| Cyfra SLPI CEACAM5 MMP2 OPN MDK | 6 | 0.60 | 0 | 45 | 1.00 | 100 | 100 |
| TIMP1 CEACAM5 MDK | 3 | 0.60 | 5 | 9 | 0.72 | 27 | 23 |
| MMP2 MDK | 2 | 0.60 | 38 | 9 | 0.88 | 52 | 54 |
| TIMP1 MMP2 MDK | 3 | 0.60 | 38 | 9 | 0.88 | 52 | 54 |
| Cyfra CEACAM5 MMP2 OPN MDK | 5 | 0.60 | 0 | 45 | 1.00 | 100 | 100 |
| Cyfra TIMP1 SCC TFPI OPN MDK | 6 | 0.59 | 0 | 45 | 1.00 | 94 | 92 |
| TIMP1 CEACAM5 MMP2 OPN | 4 | 0.59 | 10 | 18 | 0.90 | 45 | 54 |
| SLPI TIMP1 CEACAM5 MMP2 OPN | 5 | 0.59 | 10 | 18 | 0.90 | 45 | 54 |
| TIMP1 TFPI CEACAM5 OPN MDK | 5 | 0.59 | 0 | 18 | 0.83 | 48 | 46 |
| SLPI MMP2 MDK | 3 | 0.59 | 38 | 9 | 0.88 | 55 | 46 |
| CEACAM5 MDK | 2 | 0.59 | 0 | 18 | 0.68 | 33 | 15 |
| SLPI TIMP1 MMP2 MDK | 4 | 0.59 | 33 | 9 | 0.89 | 61 | 46 |
| TFPI CEACAM5 OPN MDK | 4 | 0.59 | 0 | 18 | 0.82 | 45 | 38 |
| SLPI TIMP1 TFPI CEACAM5 OPN MDK | 6 | 0.59 | 0 | 18 | 0.87 | 58 | 15 |
| Cyfra SLPI TIMP1 TFPI CEACAM5 OPN MDK | 7 | 0.59 | 0 | 45 | 1.00 | 100 | 100 |
| SLPI CEACAM5 MMP2 OPN | 4 | 0.58 | 10 | 18 | 0.89 | 52 | 46 |
| SLPI TIMP1 TFPI MMP2 OPN | 5 | 0.58 | 14 | 9 | 0.91 | 45 | 62 |
| Cyfra SLPI TFPI CEACAM5 OPN MDK | 6 | 0.58 | 0 | 45 | 1.00 | 100 | 100 |
| MDK | 1 | 0.58 | 24 | 0 | 0.62 | 15 | 0 |
| TIMP1 TFPI MMP2 OPN | 4 | 0.58 | 14 | 9 | 0.90 | 45 | 54 |
| SLPI CEACAM5 MDK | 3 | 0.58 | 0 | 18 | 0.67 | 36 | 15 |
| Cyfra TFPI CEACAM5 OPN MDK | 5 | 0.58 | 0 | 45 | 1.00 | 100 | 100 |
| Cyfra TIMP1 TFPI CEACAM5 OPN MDK | 6 | 0.58 | 0 | 45 | 1.00 | 100 | 100 |
| TIMP1 MMP2 | 2 | 0.58 | 10 | 9 | 0.86 | 58 | 31 |
| SLPI TIMP1 MMP2 | 3 | 0.58 | 10 | 9 | 0.86 | 58 | 31 |
| TFPI MMP2 OPN | 3 | 0.58 | 14 | 9 | 0.89 | 52 | 15 |
| SLPI TFPI CEACAM5 OPN MDK | 5 | 0.58 | 0 | 18 | 0.85 | 48 | 31 |
| MMP2 | 1 | 0.58 | 10 | 9 | 0.86 | 61 | 23 |
| SLPLTIMP1 CEACAM5 MDK | 4 | 0.56 | 5 | 9 | 0.75 | 24 | 23 |
| SLPI MMP2 | 2 | 0.56 | 10 | 9 | 0.87 | 64 | 23 |
| SLPI TFPI MMP2 OPN | 4 | 0.56 | 14 | 9 | 0.89 | 48 | 31 |
| SLPI TIMP1 MDK | 3 | 0.56 | 24 | 18 | 0.72 | 18 | 15 |
| Cyfra SCC MMP2 OPN MDK | 5 | 0.56 | 0 | 45 | 1.00 | 100 | 100 |
| SLPI MDK | 2 | 0.55 | 24 | 0 | 0.63 | 18 | 8 |
| Cyfra SLPI MMP2 MDK | 4 | 0.55 | 0 | 55 | 1.00 | 100 | 100 |
| Cyfra SLPI SCC MMP2 MDK | 5 | 0.55 | 0 | 55 | 1.00 | 100 | 100 |
| Cyfra SLPI TFPI MMP2 MDK | 5 | 0.55 | 0 | 55 | 1.00 | 100 | 100 |
| Cyfra SLPI CEACAM5 MMP2 MDK | 5 | 0.55 | 0 | 55 | 1.00 | 100 | 100 |
| Cyfra SLPI TIMP1 TFPI MMP2 MDK | 6 | 0.55 | 0 | 55 | 1.00 | 100 | 100 |
| Cyfra SLPI TIMP1 CEACAM5 MMP2 MDK | 6 | 0.55 | 0 | 55 | 1.00 | 100 | 100 |
| TIMP1 SCC TFPI CEACAM5 MMP2 OPN | 6 | 0.55 | 0 | 55 | 1.00 | 100 | 100 |
| Cyfra SLPI TIMP1 TFPI CEACAM5 MMP2 MDK | 7 | 0.55 | 0 | 55 | 1.00 | 100 | 100 |
| SLPI TIMP1 SCC CEACAM5 MMP2 OPN MDK | 7 | 0.55 | 0 | 55 | 1.00 | 97 | 100 |
| TIMP1 MMP2 OPN MDK | 4 | 0.55 | 38 | 0 | 0.91 | 39 | 85 |
| MMP2 OPN MDK | 3 | 0.55 | 38 | 0 | 0.91 | 42 | 69 |
| Cyfra SCC MMP2 MDK | 4 | 0.54 | 0 | 55 | 1.00 | 100 | 100 |
| Cyfra SLPI TIMP1 MMP2 MDK | 5 | 0.54 | 0 | 55 | 1.00 | 100 | 100 |
| Cyfra SLPI TFPI CEACAM5 MMP2 MDK | 6 | 0.54 | 0 | 55 | 1.00 | 100 | 100 |
| SLPI TIMP1 SCC CEACAM5 MMP2 MDK | 6 | 0.54 | 0 | 55 | 1.00 | 100 | 100 |
| SLPI SCC CEACAM5 MMP2 OPN MDK | 6 | 0.54 | 0 | 55 | 1.00 | 97 | 100 |
| TIMP1 SCC CEACAM5 MMP2 OPN MDK | 6 | 0.54 | 0 | 55 | 1.00 | 100 | 100 |
| SLPI TIMP1 MMP2 OPN MDK | 5 | 0.54 | 38 | 0 | 0.91 | 48 | 54 |
| SLPI MMP2 OPN MDK | 4 | 0.53 | 38 | 0 | 0.91 | 42 | 54 |
| Cyfra SCC TFPI MMP2 MDK | 5 | 0.52 | 0 | 45 | 1.00 | 100 | 100 |
| TFPI OPN MDK | 3 | 0.51 | 33 | 0 | 0.80 | 21 | 31 |
| TIMP1 TFPI OPN MDK | 4 | 0.51 | 33 | 9 | 0.81 | 21 | 38 |
| SLPI TIMP1 TFPI OPN MDK | 5 | 0.51 | 29 | 0 | 0.83 | 30 | 31 |
| SLPI TFPI OPN MDK | 4 | 0.50 | 33 | 0 | 0.81 | 21 | 23 |
| MMP2 OPN | 2 | 0.48 | 10 | 0 | 0.89 | 42 | 54 |
| TIMP1 TFPI CEACAM5 OPN | 4 | 0.46 | 0 | 18 | 0.79 | 24 | 38 |
| SLPI TFPI CEACAM5 OPN | 4 | 0.46 | 0 | 9 | 0.79 | 24 | 23 |
| SLPI TIMP1 TFPI CEACAM5 OPN | 5 | 0.46 | 0 | 9 | 0.80 | 24 | 23 |
| TFPI CEACAM5 OPN | 3 | 0.46 | 0 | 18 | 0.79 | 24 | 31 |
| SLPI TIMP1 MMP2 OPN | 4 | 0.45 | 10 | 0 | 0.89 | 33 | 69 |
| TIMP1 MMP2 OPN | 3 | 0.45 | 10 | 0 | 0.89 | 33 | 69 |
| OPN MDK | 2 | 0.45 | 24 | 0 | 0.81 | 9 | 38 |
| SLPI MMP2 OPN | 3 | 0.44 | 10 | 0 | 0.88 | 42 | 46 |
| TIMP1 OPN MDK | 3 | 0.44 | 29 | 0 | 0.80 | 9 | 46 |
| SLPI OPN MDK | 3 | 0.44 | 24 | 0 | 0.80 | 15 | 23 |
| SLPI TIMP1 CEACAM5 OPN MDK | 5 | 0.43 | 5 | 0 | 0.82 | 33 | 31 |
| CEACAM5 OPN MDK | 3 | 0.43 | 0 | 0 | 0.83 | 12 | 38 |
| TIMP1 CEACAM5 OPN MDK | 4 | 0.43 | 0 | 0 | 0.81 | 15 | 31 |
| SLPI TIMP1 OPN MDK | 4 | 0.43 | 29 | 0 | 0.80 | 33 | 38 |
| SLPI CEACAM5 OPN MDK | 4 | 0.43 | 0 | 0 | 0.82 | 15 | 31 |
| SLPI TIMP1 | 2 | 0.42 | 0 | 9 | 0.60 | 6 | 0 |
| CEACAM5 | 1 | 0.39 | 0 | 0 | 0.56 | 24 | 8 |
| SLPI CEACAM5 | 2 | 0.39 | 0 | 0 | 0.57 | 24 | 8 |
| TIMP1 CEACAM5 | 2 | 0.39 | 0 | 0 | 0.60 | 30 | 0 |
| TFPI OPN | 2 | 0.38 | 0 | 0 | 0.77 | 12 | 31 |
| TIMP1 TFPI OPN | 3 | 0.38 | 0 | 0 | 0.77 | 12 | 31 |
| SLPI TFPI OPN | 3 | 0.38 | 0 | 0 | 0.79 | 12 | 23 |
| SLPI TIMP1 TFPI OPN | 4 | 0.38 | 0 | 0 | 0.79 | 12 | 23 |
| SLPI TIMP1 CEACAM5 | 3 | 0.38 | 0 | 0 | 0.61 | 33 | 0 |
| CEACAM5 OPN | 2 | 0.30 | 0 | 0 | 0.82 | 18 | 46 |
| TIMP1 CEACAM5 OPN | 3 | 0.30 | 0 | 0 | 0.81 | 18 | 46 |
| TIMP1 OPN | 2 | 0.29 | 0 | 0 | 0.78 | 12 | 38 |
| OPN | 1 | 0.29 | 0 | 0 | 0.78 | 12 | 46 |
| SLPI CEACAM5 OPN | 3 | 0.29 | 0 | 0 | 0.83 | 18 | 46 |
| SLPI OPN | 2 | 0.28 | 0 | 0 | 0.79 | 9 | 54 |
| SLPI TIMP1 CEACAM5 OPN | 4 | 0.28 | 0 | 0 | 0.82 | 21 | 62 |
| SLPI TIMP1 OPN | 3 | 0.28 | 0 | 0 | 0.79 | 12 | 46 |
| SLPI | 1 | 0.26 | 0 | 9 | 0.58 | 0 | 38 |

**TABLE 8**

| Stage I vs. Stage III | | Split-point | | |
|---|---|---|---|---|
| | | Trained on 104x103, tested on 104x103 | | |
| Panel | Size | AUC | Sens at 95% Spec | Spec at 95% Sens |
| Cyfra SLPI SCC TFPI MMP2 MDK | 6 | 0.77 | 27 | 35 |
| Cyfra SCC TFPI MMP2 MDK | 5 | 0.77 | 31 | 42 |
| Cyfra SLPI SCC TFPI CEACAM5 MMP2 MDK | 7 | 0.77 | 27 | 32 |
| Cyfra TIMP1 SCC TFPI MMP2 MDK | 6 | 0.77 | 23 | 39 |
| SLPI SCC TFPI MMP2 MDK | 5 | 0.76 | 12 | 35 |
| Cyfra SCC TFPI MDK | 4 | 0.76 | 23 | 61 |
| Cyfra SCC TFPI CEACAM5 MMP2 MDK | 6 | 0.76 | 31 | 39 |
| Cyfra SLPI SCC MMP2 MDK | 5 | 0.76 | 31 | 35 |
| SLPI SCC TFPI CEACAM5 MMP2 MDK | 6 | 0.76 | 15 | 32 |
| Cyfra TIMP1 SCC TFPI CEACAM5 MMP2 MDK | 7 | 0.76 | 23 | 35 |
| Cyfra SCC MMP2 MDK | 4 | 0.76 | 62 | 45 |
| TIMP1 SCC TFPI MMP2 MDK | 5 | 0.76 | 8 | 39 |
| Cyfra SLPI TIMP1 SCC TFPI MMP2 MDK | 7 | 0.76 | 38 | 32 |
| Cyfra SLPI TIMP1 SCC MMP2 MDK | 6 | 0.76 | 54 | 32 |
| Cyfra SLPI SCC CEACAM5 MMP2 MDK | 6 | 0.75 | 31 | 32 |
| SLPI SCC MMP2 MDK | 4 | 0.75 | 27 | 35 |
| SLPI TIMP1 SCC MMP2 MDK | 5 | 0.75 | 54 | 32 |
| SCC TFPI MDK | 3 | 0.75 | 8 | 61 |
| TIMP1 SCC TFPI CEACAM5 MMP2 MDK | 6 | 0.75 | 12 | 35 |
| Cyfra SLPI SCC TFPI MDK | 5 | 0.75 | 35 | 48 |
| Cyfra SLPI TIMP1 SCC TFPI CEACAM5 MMP2 MDK | 8 | 0.75 | 38 | 29 |
| SCC TFPI MMP2 MDK | 4 | 0.75 | 15 | 42 |
| Cyfra TIMP1 SCC TFPI MDK | 5 | 0.75 | 31 | 52 |
| SLPI TIMP1 SCC TFPI MMP2 MDK | 6 | 0.75 | 38 | 32 |
| Cyfra SLPI TIMP1 SCC CEACAM5 MMP2 MDK | 7 | 0.75 | 54 | 29 |
| SLPI TIMP1 SCC TFPI CEACAM5 MMP2 MDK | 7 | 0.75 | 38 | 29 |
| SLPI TIMP1 SCC CEACAM5 MMP2 MDK | 6 | 0.75 | 54 | 29 |
| SLPI SCC TFPI MDK | 4 | 0.75 | 35 | 48 |
| SLPI SCC CEACAM5 MMP2 MDK | 5 | 0.75 | 27 | 32 |
| SCC TFPI CEACAM5 MMP2 MDK | 5 | 0.75 | 19 | 39 |
| TIMP1 SCC TFPI MDK | 4 | 0.75 | 31 | 52 |
| Cyfra SLPI TIMP1 SCC TFPI MDK | 6 | 0.75 | 50 | 39 |
| Cyfra SCC CEACAM5 MMP2 MDK | 5 | 0.75 | 62 | 42 |
| Cyfra SCC TFPI CEACAM5 MDK | 5 | 0.75 | 23 | 58 |
| SLPI TIMP1 SCC TFPI MDK | 5 | 0.74 | 50 | 39 |
| Cyfra SLPI SCC TFPI CEACAM5 MDK | 6 | 0.74 | 35 | 45 |
| Cyfra TIMP1 SCC MMP2 MDK | 5 | 0.74 | 35 | 42 |
| Cyfra TIMP1 SCC TFPI CEACAM5 MDK | 6 | 0.74 | 31 | 48 |
| Cyfra SLPI SCCTFPI OPN MDK | 6 | 0.74 | 27 | 35 |
| Cyfra SLPI SCC TFPI MMP2 OPN MDK | 7 | 0.74 | 15 | 32 |
| SCC TFPI CEACAM5 MDK | 4 | 0.74 | 12 | 58 |
| SLPI SCC TFPI CEACAM5 MDK | 5 | 0.74 | 35 | 45 |
| TIMP1 SCC TFPI CEACAM5 MDK | 5 | 0.74 | 31 | 48 |
| Cyfra SLPI TIMP1 SCC TFPI CEACAM5 MDK | 7 | 0.74 | 50 | 35 |
| Cyfra SLPI TIMP1 SCC TFPI MMP2 OPN MDK | 8 | 0.74 | 27 | 29 |
| Cyfra TIMP1 SCC CEACAM5 MMP2 MDK | 6 | 0.73 | 35 | 39 |
| Cyfra SLPI SCC TFPI CEACAM5 MMP2 OPN MDK | 8 | 0.73 | 15 | 29 |
| Cyfra SLPI SCC MMP2 OPN MDK | 6 | 0.73 | 23 | 32 |
| SCC MMP2 MDK | 3 | 0.73 | 58 | 45 |
| SLPI TIMP1 SCC TFPI CEACAM5 MDK | 6 | 0.73 | 50 | 35 |
| SLPI SCC TFPI OPN MDK | 5 | 0.73 | 19 | 35 |
| Cyfra SLPI TIMP1 SCC TFPI CEACAM5 MMP2 OPN MDK | 9 | 0.73 | 27 | 26 |
| Cyfra SLPI SCC TFPI CEACAM5 OPN MDK | 7 | 0.73 | 27 | 32 |
| SLPI TIMP1 SCC TFPI MMP2 OPN MDK | 7 | 0.73 | 19 | 29 |
| TIMP1 SCC MMP2 MDK | 4 | 0.73 | 31 | 42 |
| SLPI SCC TFPI MMP2 OPN MDK | 6 | 0.73 | 8 | 32 |
| Cyfra TIMP1 SCC TFPI MMP2 OPN MDK | 7 | 0.73 | 31 | 35 |
| SLPI TIMP1 SCC TFPI CEACAM5 MMP2 OPN MDK | 8 | 0.73 | 19 | 26 |
| Cyfra SLPI TIMP1 SCC MMP2 OPN MDK | 7 | 0.73 | 35 | 29 |
| Cyfra SLPI SCC CEACAM5 MMP2 OPN MDK | 7 | 0.73 | 23 | 29 |
| Cyfra SLPI SCC MDK | 4 | 0.73 | 23 | 52 |
| Cyfra SLPI TIMP1 SCC TFPI OPN MDK | 7 | 0.73 | 31 | 29 |
| SLPI SCC TFPI CEACAM5 MMP2 OPN MDK | 7 | 0.73 | 12 | 29 |
| SLPI SCC TFPI CEACAM5 OPN MDK | 6 | 0.72 | 19 | 32 |
| TIMP1 SCC CEACAM5 MMP2 MDK | 5 | 0.72 | 31 | 39 |
| SLPI TIMP1 SCC TFPI OPN MDK | 6 | 0.72 | 31 | 29 |
| SLPI TIMP1 SCC MMP2 OPN MDK | 6 | 0.72 | 35 | 29 |
| SLPI SCC MDK | 3 | 0.72 | 19 | 52 |
| Cyfra SCC MDK | 3 | 0.72 | 35 | 0 |
| Cyfra SLPI TIMP1 SCC CEACAM5 MMP2 OPN MDK | 8 | 0.72 | 35 | 26 |
| Cyfra TIMP1 SCC TFPI CEACAM5 MMP2 OPN MDK | 8 | 0.72 | 31 | 32 |
| SCC CEACAM5 MMP2 MDK | 4 | 0.72 | 58 | 42 |
| Cyfra SCC TFPI MMP2 OPN MDK | 6 | 0.72 | 19 | 39 |
| SLPI SCC MMP2 OPN MDK | 5 | 0.72 | 12 | 32 |
| SLPI TIMP1 SCC CEACAM5 MMP2 OPN MDK | 7 | 0.72 | 35 | 26 |
| Cyfra SLPI TIMP1 SCC TFPI CEACAM5 OPN MDK | 8 | 0.72 | 31 | 26 |
| TIMP1 SCC TFPI MMP2 OPN MDK | 6 | 0.72 | 23 | 35 |
| SLPI TIMP1 SCC TFPI CEACAM5 OPN MDK | 7 | 0.72 | 31 | 26 |
| Cyfra SLPI TIMP1 SCC MDK | 5 | 0.72 | 38 | 39 |
| SLPI TIMP1 SCC MDK | 4 | 0.71 | 38 | 39 |
| Cyfra TIMP1 SCC TFPI OPN MDK | 6 | 0.71 | 38 | 39 |
| Cyfra TIMP1 SCC MDK | 4 | 0.71 | 31 | 0 |
| Cyfra SLPI SCC CEACAM5 MDK | 5 | 0.71 | 23 | 48 |
| TIMP1 SCC TFPI CEACAM5 MMP2 OPN MDK | 7 | 0.71 | 23 | 32 |
| TIMP1 SCC TFPI OPN MDK | 5 | 0.71 | 38 | 39 |
| SLPI SCC CEACAM5 MMP2 OPN MDK | 6 | 0.71 | 12 | 29 |
| SLPI SCC CEACAM5 MDK | 4 | 0.71 | 19 | 48 |
| Cyfra SCC TFPI OPN MDK | 5 | 0.71 | 27 | 45 |
| TIMP1 SCC MDK | 3 | 0.71 | 27 | 0 |
| Cyfra SCC TFPI CEACAM5 MMP2 OPN MDK | 7 | 0.71 | 19 | 35 |
| SCC MDK | 2 | 0.71 | 31 | 0 |
| Cyfra SLPI SCC OPN MDK | 5 | 0.71 | 19 | 39 |
| Cyfra SLPI TIMP1 SCC CEACAM5 MDK | 6 | 0.71 | 38 | 35 |
| Cyfra SCC CEACAM5 MDK | 4 | 0.70 | 35 | 0 |
| SLPI TIMP1 SCC CEACAM5 MDK | 5 | 0.70 | 38 | 35 |
| Cyfra TIMP1 SCC TFPI CEACAM5 OPN MDK | 7 | 0.70 | 38 | 35 |
| Cyfra SCC MMP2 OPN MDK | 5 | 0.70 | 35 | 42 |
| TIMP1 SCC TFPI CEACAM5 OPN MDK | 6 | 0.70 | 38 | 35 |
| Cyfra TIMP1 SCC CEACAM5 MDK | 5 | 0.70 | 31 | 0 |
| Cyfra SLPI TIMP1 SCC OPN MDK | 6 | 0.70 | 27 | 29 |
| SLPI SCC OPN MDK | 4 | 0.70 | 8 | 39 |
| SCC TFPI OPN MDK | 4 | 0.70 | 19 | 45 |
| Cyfra TIMP1 SCC TFPI MMP2 | 5 | 0.70 | 31 | 0 |
| SLPI TIMP1 SCC OPN MDK | 5 | 0.70 | 27 | 29 |
| SCC TFPI MMP2 OPN MDK | 5 | 0.70 | 12 | 39 |
| Cyfra TIMP1 SCC MMP2 OPN MDK | 6 | 0.70 | 27 | 39 |
| TIMP1 SCC CEACAM5 MDK | 4 | 0.69 | 27 | 0 |
| Cyfra SLPI TIMP1 SCC TFPI MMP2 | 6 | 0.69 | 27 | 0 |
| Cyfra SLPI SCC CEACAM5 OPN MDK | 6 | 0.69 | 19 | 35 |
| Cyfra SCC CEACAM5 MMP2 OPN MDK | 6 | 0.69 | 35 | 39 |
| Cyfra SCC TFPI CEACAM5 OPN MDK | 6 | 0.69 | 27 | 42 |
| Cyfra TIMP1 SCC CEACAM5 MMP2 OPN MDK | 7 | 0.69 | 27 | 35 |
| Cyfra SLPI TIMP1 SCC CEACAM5 OPN MDK | 7 | 0.69 | 27 | 26 |
| SCC CEACAM5 MDK | 3 | 0.69 | 31 | 0 |
| Cyfra TIMP1 SCC TFPI CEACAM5 MMP2 | 6 | 0.69 | 31 | 0 |
| TIMP1 SCC MMP2 OPN MDK | 5 | 0.69 | 15 | 39 |
| Cyfra SLPI TIMP1 SCC TFPI CEACAM5 MMP2 | 7 | 0.69 | 27 | 0 |
| SCC TFPI CEACAM5 MMP2 OPN MDK | 6 | 0.69 | 15 | 35 |
| Cyfra SLPI TIMP1 TFPI MMP2 MDK | 6 | 0.69 | 27 | 32 |
| SLPI SCC CEACAM5 OPN MDK | 5 | 0.69 | 8 | 35 |
| SLPI TIMP1 SCC CEACAM5 OPN MDK | 6 | 0.69 | 27 | 26 |
| SCC TFPI CEACAM5 OPN MDK | 5 | 0.68 | 19 | 42 |
| SLPI TIMP1 SCC TFPI MMP2 | 5 | 0.68 | 15 | 0 |
| TIMP1 SCC CEACAM5 MMP2 OPN MDK | 6 | 0.68 | 15 | 35 |
| Cyfra TIMP1 SCC OPN MDK | 5 | 0.68 | 35 | 42 |
| SLPI TIMP1 SCC TFPI CEACAM5 MMP2 | 6 | 0.68 | 19 | 0 |
| Cyfra SLPI TIMP1 TFPI CEACAM5 MMP2 MDK | 7 | 0.68 | 27 | 29 |
| TIMP1 SCCTFPI MMP2 | 4 | 0.68 | 19 | 0 |
| TIMP1 SCC OPN MDK | 4 | 0.68 | 35 | 42 |
| Cyfra SCC OPN MDK | 4 | 0.68 | 23 | 0 |
| Cyfra SLPI SCC TFPI MMP2 | 5 | 0.68 | 31 | 0 |
| Cyfra SCC TFPI MMP2 | 4 | 0.68 | 12 | 0 |
| Cyfra SLPI TIMP1 SCC TFPI MMP2 OPN | 7 | 0.68 | 12 | 0 |
| SLPI TIMP1 SCC MMP2 | 4 | 0.68 | 38 | 0 |
| Cyfra SLPI TIMP1 SCC MMP2 | 5 | 0.68 | 38 | 0 |
| SLPI TIMP1 TFPI MMP2 MDK | 5 | 0.68 | 12 | 32 |
| TIMP1 SCC TFPI CEACAM5 MMP2 | 5 | 0.68 | 23 | 0 |
| Cyfra TIMP1 SCC MMP2 | 4 | 0.67 | 19 | 0 |
| Cyfra TIMP1 TFPI MMP2 MDK | 5 | 0.67 | 4 | 39 |
| SCC MMP2 OPN MDK | 4 | 0.67 | 23 | 42 |
| SLPI TIMP1 TFPI CEACAM5 MMP2 MDK | 6 | 0.67 | 15 | 29 |
| Cyfra SLPI TIMP1 SCC TFPI CEACAM5 MMP2 OPN | 8 | 0.67 | 12 | 0 |
| Cyfra TIMP1 SCC CEACAM5 OPN MDK | 6 | 0.67 | 35 | 39 |
| Cyfra SLPI TIMP1 TFPI MDK | 5 | 0.67 | 35 | 39 |
| Cyfra SLPI SCC TFPI CEACAM5 MMP2 | 6 | 0.67 | 31 | 0 |
| Cyfra SLPI TIMP1 TFPI MMP2 OPN MDK | 7 | 0.67 | 15 | 29 |
| SLPI TIMP1 TFPI MDK | 4 | 0.67 | 35 | 39 |
| SLPI TIMP1 SCC CEACAM5 MMP2 | 5 | 0.67 | 38 | 0 |
| Cyfra SLPI TIMP1 SCC CEACAM5 MMP2 | 6 | 0.67 | 38 | 0 |
| Cyfra SLPI TFPI MMP2 MDK | 5 | 0.67 | 8 | 35 |
| TIMP1 SCC CEACAM5 OPN MDK | 5 | 0.67 | 35 | 39 |
| SLPI TIMP1 SCC TFPI MMP2 OPN | 6 | 0.67 | 8 | 0 |
| SCC OPN MDK | 3 | 0.67 | 12 | 0 |
| Cyfra TIMP1 TFPI CEACAM5 MMP2 MDK | 6 | 0.67 | 8 | 35 |
| Cyfra SLPI TIMP1 SCC MMP2 OPN | 6 | 0.67 | 19 | 0 |
| Cyfra SCC TFPI CEACAM5 MMP2 | 5 | 0.67 | 15 | 0 |
| Cyfra SLPI SCC MMP2 | 4 | 0.66 | 23 | 0 |
| SCC CEACAM5 MMP2 OPN MDK | 5 | 0.66 | 23 | 39 |
| Cyfra TIMP1 SCC TFPI MMP2 OPN | 6 | 0.66 | 15 | 0 |
| Cyfra TIMP1 SCC CEACAM5 MMP2 | 5 | 0.66 | 19 | 0 |
| SLPI TIMP1 SCC TFPI CEACAM5 MMP2 OPN | 7 | 0.66 | 12 | 0 |
| Cyfra SLPI TIMP1 MMP2 MDK | 5 | 0.66 | 35 | 32 |
| Cyfra SCC CEACAM5 OPN MDK | 5 | 0.66 | 23 | 0 |
| Cyfra SLPI TIMP1 TFPI CEACAM5 MMP2 OPN MDK | 8 | 0.66 | 15 | 26 |
| Cyfra SLPI TIMP1 TFPI CEACAM5 MDK | 6 | 0.66 | 35 | 35 |
| Cyfra SLPI TIMP1 SCC CEACAM5 MMP2 OPN | 7 | 0.66 | 19 | 0 |
| Cyfra SLPI TFPI CEACAM5 MMP2 MDK | 6 | 0.66 | 12 | 32 |
| SLPI TIMP1 TFPI CEACAM5 MDK | 5 | 0.66 | 35 | 35 |
| Cyfra TIMP1 SCC TFPI CEACAM5 MMP2 OPN | 7 | 0.66 | 15 | 0 |
| Cyfra TIMP1 TFPI MDK | 4 | 0.66 | 23 | 0 |
| SLPI TIMP1 TFPI MMP2 OPN MDK | 6 | 0.66 | 8 | 29 |
| SLPI SCC TFPI MMP2 | 4 | 0.66 | 19 | 0 |
| TIMP1 SCC MMP2 | 3 | 0.66 | 0 | 0 |
| SLPI TIMP1 SCC MMP2 OPN | 5 | 0.66 | 12 | 0 |
| SLPI SCC TFPI CEACAM5 MMP2 | 5 | 0.65 | 23 | 0 |
| Cyfra SLPI TIMP1 CEACAM5 MMP2 MDK | 6 | 0.65 | 35 | 29 |
| Cyfra SLPI TIMP1 TFPI OPN MDK | 6 | 0.65 | 27 | 29 |
| Cyfra SLPI TFPI MDK | 4 | 0.65 | 23 | 48 |
| Cyfra SLPI SCC CEACAM5 MMP2 | 5 | 0.65 | 23 | 0 |
| Cyfra SCC MMP2 | 3 | 0.65 | 27 | 0 |
| SCC CEACAM5 OPN MDK | 4 | 0.65 | 12 | 0 |
| Cyfra SLPI TIMP1 SCC TFPI OPN | 6 | 0.65 | 23 | 0 |
| Cyfra SLPI SCC TFPI MMP2 OPN | 6 | 0.65 | 19 | 0 |
| TIMP1 SCC TFPI MMP2 OPN | 5 | 0.65 | 12 | 0 |
| Cyfra SLPI TIMP1 MMP2 OPN MDK | 6 | 0.65 | 27 | 29 |
| SLPI TIMP1 TFPI CEACAM5 MMP2 OPN MDK | 7 | 0.65 | 12 | 26 |
| TIMP1 SCC CEACAM5 MMP2 | 4 | 0.65 | 4 | 0 |
| SLPI TIMP1 SCC CEACAM5 MMP2 OPN | 6 | 0.65 | 12 | 0 |
| SLPI TIMP1 SCC TFPI OPN | 5 | 0.65 | 15 | 0 |
| SLPI TIMP1 TFPI OPN MDK | 5 | 0.65 | 19 | 29 |
| Cyfra SLPI SCC TFPI CEACAM5 MMP2 OPN | 7 | 0.65 | 19 | 0 |
| TIMP1 SCC TFPI CEACAM5 MMP2 OPN | 6 | 0.65 | 15 | 0 |
| Cyfra SLPI TIMP1 SCC TFPI CEACAM5 OPN | 7 | 0.65 | 23 | 0 |
| SLPI SCC MMP2 | 3 | 0.65 | 4 | 0 |
| Cyfra TIMP1 SCC TFPI | 4 | 0.65 | 23 | 0 |
| SLPI TIMP1 MMP2 MDK | 4 | 0.65 | 31 | 32 |
| Cyfra SLPI TFPI MMP2 OPN MDK | 6 | 0.65 | 8 | 32 |
| Cyfra SLPI TIMP1 TFPI CEACAM5 OPN MDK | 7 | 0.65 | 27 | 26 |
| Cyfra SLPI SCC MMP2 OPN | 5 | 0.64 | 12 | 0 |
| SLPI TIMP1 SCC TFPI CEACAM5 OPN | 6 | 0.64 | 15 | 0 |
| Cyfra SLPI TIMP1 SCC TFPI | 5 | 0.64 | 42 | 0 |
| Cyfra TIMP1 SCC MMP2 OPN | 5 | 0.64 | 27 | 0 |
| Cyfra TIMP1 TFPI CEACAM5 MDK | 5 | 0.64 | 23 | 0 |
| Cyfra SCC TFPI MMP2 OPN | 5 | 0.64 | 27 | 0 |
| Cyfra TIMP1 TFPI MMP2 OPN MDK | 6 | 0.64 | 23 | 35 |
| TIMP1 TFPI MMP2 MDK | 4 | 0.64 | 0 | 39 |
| Cyfra TIMP1 SCC TFPI OPN | 5 | 0.64 | 23 | 0 |
| Cyfra SLPI TIMP1 CEACAM5 MMP2 OPN MDK | 7 | 0.64 | 27 | 26 |
| SLPI TFPI MMP2 MDK | 4 | 0.64 | 4 | 35 |
| SLPI TIMP1 SCCTFPI | 4 | 0.64 | 42 | 0 |
| Cyfra TFPI MMP2 MDK | 4 | 0.64 | 8 | 0 |
| TIMP1 TFPI CEACAM5 MMP2 MDK | 5 | 0.64 | 0 | 35 |
| TIMP1 SCC TFPI | 3 | 0.64 | 12 | 0 |
| SCC TFPI MMP2 | 3 | 0.64 | 4 | 0 |
| Cyfra SLPI SCC CEACAM5 MMP2 OPN | 6 | 0.64 | 12 | 0 |
| SLPI TIMP1 TFPI CEACAM5 OPN MDK | 6 | 0.64 | 19 | 26 |
| SLPI SCC CEACAM5 MMP2 | 4 | 0.64 | 8 | 0 |
| SLPI TIMP1 CEACAM5 MMP2 MDK | 5 | 0.64 | 31 | 29 |
| Cyfra SLPI TIMP1 SCC TFPI CEACAM5 | 6 | 0.64 | 42 | 0 |
| Cyfra SLPI TFPI CEACAM5 MDK | 5 | 0.64 | 23 | 45 |
| Cyfra TIMP1 SCC CEACAM5 MMP2 OPN | 6 | 0.64 | 27 | 0 |
| SLPI TFPI MDK | 3 | 0.64 | 8 | 48 |
| Cyfra SLPI TFPI CEACAM5 MMP2 OPN MDK | 7 | 0.64 | 12 | 29 |
| Cyfra SLPI TIMP1 MDK | 4 | 0.64 | 23 | 0 |
| Cyfra TIMP1 MMP2 MDK | 4 | 0.64 | 19 | 0 |
| TIMP1 TFPI MDK | 3 | 0.64 | 8 | 0 |
| Cyfra TIMP1 SCC TFPI CEACAM5 | 5 | 0.64 | 23 | 0 |
| SLPI TIMP1 SCC TFPI CEACAM5 | 5 | 0.63 | 42 | 0 |
| Cyfra TIMP1 SCC TFPI CEACAM5 OPN | 6 | 0.63 | 23 | 0 |
| Cyfra SCC CEACAM5 MMP2 | 4 | 0.63 | 27 | 0 |
| SLPI TFPI CEACAM5 MMP2 MDK | 5 | 0.63 | 4 | 32 |
| Cyfra TIMP1 TFPI CEACAM5 MMP2 OPN MDK | 7 | 0.63 | 23 | 32 |
| Cyfra SCC TFPI CEACAM5 MMP2 OPN | 6 | 0.63 | 27 | |
| TIMP1 SCC TFPI OPN | 4 | 0.63 | 15 | 0 |
| SCC TFPI CEACAM5 MMP2 | 4 | 0.63 | 4 | 0 |
| Cyfra TFPI CEACAM5 MMP2 MDK | 5 | 0.63 | 12 | 0 |
| SLPI TIMP1 MMP2 OPN MDK | 5 | 0.63 | 15 | 29 |
| TIMP1 SCC TFPI CEACAM5 | 4 | 0.63 | 15 | 0 |
| Cyfra SLPI TFPI OPN MDK | 5 | 0.63 | 15 | 35 |
| SLPI SCC TFPI MMP2 OPN | 5 | 0.63 | 15 | 0 |
| Cyfra SLPI MMP2 MDK | 4 | 0.63 | 19 | 35 |
| TIMP1 SCC TFPI CEACAM5 OPN | 5 | 0.63 | 15 | 0 |
| Cyfra SLPI SCC TFPI OPN | 5 | 0.63 | 12 | 0 |
| SLPI SCC TFPI CEACAM5 MMP2 OPN | 6 | 0.63 | 19 | 0 |
| SLPI TIMP1 MDK | 3 | 0.63 | 19 | 0 |
| TIMP1 TFPI CEACAM5 MDK | 4 | 0.63 | 12 | 0 |
| Cyfra TIMP1 CEACAM5 MMP2 MDK | 5 | 0.62 | 19 | 0 |
| Cyfra SLPI TIMP1 SCC OPN | 5 | 0.62 | 35 | 0 |
| Cyfra SCC TFPI | 3 | 0.62 | 27 | 0 |
| SLPI TIMP1 CEACAM5 MMP2 OPN MDK | 6 | 0.62 | 15 | 26 |
| SLPI TFPI CEACAM5 MDK | 4 | 0.62 | 12 | 45 |
| SLPI TFPI MMP2 OPN MDK | 5 | 0.62 | 4 | 32 |
| Cyfra SLPI TIMP1 CEACAM5 MDK | 5 | 0.62 | 23 | 0 |
| SLPI TIMP1 SCC OPN | 4 | 0.62 | 35 | 0 |
| TIMP1 SCC MMP2 OPN | 4 | 0.62 | 19 | 0 |
| SLPI SCC MMP2 OPN | 4 | 0.62 | 0 | 0 |
| Cyfra SLPI SCC TFPI CEACAM5 OPN | 6 | 0.62 | 12 | 0 |
| Cyfra TIMP1 TFPI OPN MDK | 5 | 0.62 | 31 | 0 |
| Cyfra SLPI MMP2 OPN MDK | 5 | 0.62 | 12 | 32 |
| SLPI TFPI OPN MDK | 4 | 0.62 | 8 | 35 |
| SLPI SCC CEACAM5 MMP2 OPN | 5 | 0.62 | 4 | 0 |
| SCC TFPI MMP2 OPN | 4 | 0.62 | 23 | 0 |
| Cyfra SLPI TFPI CEACAM5 OPN MDK | 6 | 0.62 | 15 | 32 |
| Cyfra SCC MMP2 OPN | 4 | 0.62 | 12 | 0 |
| TIMP1 SCC CEACAM5 MMP2 OPN | 5 | 0.62 | 19 | 0 |
| SLPI SCC TFPI OPN | 4 | 0.62 | 8 | 0 |
| Cyfra SLPI TIMP1 SCC CEACAM5 OPN | 6 | 0.62 | 35 | 0 |
| Cyfra SLPI TIMP1 OPN MDK | 5 | 0.62 | 19 | 29 |
| Cyfra TIMP1 MMP2 OPN MDK | 5 | 0.62 | 12 | 0 |
| Cyfra TFPI MDK | 3 | 0.62 | 27 | 0 |
| SLPI TIMP1 SCC CEACAM5 OPN | 5 | 0.61 | 35 | 0 |
| TIMP1 TFPI MMP2 OPN MDK | 5 | 0.61 | 15 | 35 |
| SLPI TIMP1 CEACAM5 MDK | 4 | 0.61 | 19 | 0 |
| SLPI TFPI CEACAM5 MMP2 OPN MDK | 6 | 0.61 | 4 | 29 |
| Cyfra SLPI SCC TFPI | 4 | 0.61 | 23 | 0 |
| SLPI SCC TFPI CEACAM5 OPN | 5 | 0.61 | 12 | 0 |
| SCC TFPI CEACAM5 MMP2 OPN | 5 | 0.61 | 27 | 0 |
| Cyfra SLPI CEACAM5 MMP2 MDK | 5 | 0.61 | 19 | 32 |
| Cyfra SLPI TIMP1 TFPI MMP2 | 5 | 0.61 | 12 | 0 |
| Cyfra SCC TFPI CEACAM5 | 4 | 0.61 | 27 | 0 |
| SLPI TIMP1 OPN MDK | 4 | 0.61 | 8 | 29 |
| Cyfra SLPI SCC TFPI CEACAM5 | 5 | 0.61 | 23 | 0 |
| TIMP1 TFPI CEACAM5 MMP2 OPN MDK | 6 | 0.61 | 19 | 32 |
| SLPI TFPI CEACAM5 OPN MDK | 5 | 0.61 | 12 | 32 |
| Cyfra TIMP1 TFPI CEACAM5 OPN MDK | 6 | 0.61 | 31 | 0 |
| Cyfra SCC TFPI OPN | 4 | 0.61 | 15 | 0 |
| Cyfra SCC CEACAM5 MMP2 OPN | 5 | 0.61 | 12 | 0 |
| Cyfra SLPI CEACAM5 MMP2 OPN MDK | 6 | 0.61 | 12 | 29 |
| SLPI TIMP1 SCC | 3 | 0.61 | 50 | 0 |
| Cyfra SLPI TIMP1 SCC | 4 | 0.61 | 50 | 0 |
| Cyfra SLPI SCC OPN | 4 | 0.61 | 19 | 0 |
| Cyfra SLPI TIMP1 TFPI CEACAM5 MMP2 | 6 | 0.61 | 15 | 0 |
| SCC TFPI | 2 | 0.61 | 15 | 0 |
| Cyfra TIMP1 CEACAM5 MMP2 OPN MDK | 6 | 0.61 | 12 | 0 |
| Cyfra SLPI TIMP1 CEACAM5 OPN MDK | 6 | 0.60 | 19 | 26 |
| SLPI SCC TFPI | 3 | 0.60 | 12 | 0 |
| SLPI SCC TFPI CEACAM5 | 4 | 0.60 | 15 | 0 |
| TIMP1 TFPI OPN MDK | 4 | 0.60 | 23 | 0 |
| Cyfra TIMP1 MDK | 3 | 0.60 | 15 | 0 |
| Cyfra SLPI MDK | 3 | 0.60 | 15 | 0 |
| Cyfra SLPI TIMP1 TFPI MMP2 OPN | 6 | 0.60 | 8 | 0 |
| SLPI TIMP1 SCC CEACAM5 | 4 | 0.60 | 50 | 0 |
| Cyfra SLPI TIMP1 SCC CEACAM5 | 5 | 0.60 | 50 | 0 |
| Cyfra TFPI MMP2 OPN MDK | 5 | 0.60 | 8 | 0 |
| Cyfra TFPI CEACAM5 MDK | 4 | 0.60 | 27 | 0 |
| SLPI TIMP1 CEACAM5 OPN MDK | 5 | 0.60 | 8 | 26 |
| SCC TFPI CEACAM5 | 3 | 0.60 | 19 | 0 |
| SCC MMP2 | 2 | 0.60 | 8 | 0 |
| Cyfra SLPI SCC CEACAM5 OPN | 5 | 0.60 | 19 | 0 |
| SLPI SCC OPN | 3 | 0.60 | 12 | 0 |
| Cyfra SLPI TIMP1 TFPI CEACAM5 MMP2 OPN | 7 | 0.59 | 12 | 0 |
| SLPI MMP2 OPN MDK | 4 | 0.59 | 4 | 32 |
| Cyfra SCC TFPI CEACAM5 OPN | 5 | 0.59 | 15 | 0 |
| Cyfra TIMP1 SCC OPN | 4 | 0.59 | 23 | 0 |
| Cyfra TFPI CEACAM5 MMP2 OPN MDK | 6 | 0.59 | 12 | 0 |
| TIMP1 TFPI CEACAM5 OPN MDK | 5 | 0.59 | 23 | 0 |
| TIMP1 MMP2 MDK | 3 | 0.59 | 4 | 0 |
| Cyfra TIMP1 CEACAM5 MDK | 4 | 0.59 | 15 | 0 |
| SLPI CEACAM5 MMP2 OPN MDK | 5 | 0.59 | 8 | 29 |
| SLPI SCC CEACAM5 OPN | 4 | 0.59 | 12 | 0 |
| SCC CEACAM5 MMP2 | 3 | 0.58 | 12 | 0 |
| SLPI TIMP1 TFPI MMP2 | 4 | 0.58 | 4 | 0 |
| TIMP1 SCC OPN | 3 | 0.58 | 15 | 0 |
| SLPI MMP2 MDK | 3 | 0.58 | 4 | 35 |
| Cyfra SLPI TIMP1 MMP2 | 4 | 0.58 | 23 | 0 |
| TIMP1 CEACAM5 MMP2 MDK | 4 | 0.58 | 8 | 0 |
| SCC TFPI OPN | 3 | 0.58 | 12 | 0 |
| SLPI TIMP1 TFPI CEACAM5 MMP2 | 5 | 0.58 | 4 | 0 |
| SCC MMP2 OPN | 3 | 0.58 | 0 | 0 |
| Cyfra TIMP1 TFPI MMP2 | 4 | 0.58 | 12 | 0 |
| Cyfra TIMP1 OPN MDK | 4 | 0.58 | 27 | 0 |
| Cyfra SLPI CEACAM5 MDK | 4 | 0.58 | 15 | 0 |
| Cyfra TIMP1 SCC CEACAM5 OPN | 5 | 0.58 | 23 | 0 |
| Cyfra SLPI TIMP1 MMP2 OPN | 5 | 0.58 | 12 | 0 |
| Cyfra SLPI OPN MDK | 4 | 0.58 | 8 | 39 |
| SLPI SCC | 2 | 0.58 | 38 | 0 |
| TIMP1 SCC | 2 | 0.58 | 38 | 0 |
| Cyfra SLPI SCC | 3 | 0.58 | 38 | 0 |
| Cyfra TIMP1 SCC | 3 | 0.58 | 38 | 0 |
| SLPI TIMP1 TFPI MMP2 OPN | 5 | 0.58 | 0 | 0 |
| SCC CEACAM5 MMP2 OPN | 4 | 0.57 | 4 | 0 |
| SLPI MDK | 2 | 0.57 | 0 | 0 |
| TIMP1 SCC CEACAM5 OPN | 4 | 0.57 | 15 | 0 |
| TIMP1 MMP2 OPN MDK | 4 | 0.57 | 4 | 0 |
| SLPI CEACAM5 MMP2 MDK | 4 | 0.57 | 8 | 32 |
| Cyfra TIMP1 TFPI CEACAM5 MMP2 | 5 | 0.57 | 15 | 0 |
| Cyfra MMP2 MDK | 3 | 0.57 | 35 | 0 |
| SLPI TIMP1 TFPI CEACAM5 MMP2 OPN | 6 | 0.57 | 0 | 0 |
| Cyfra SLPI TIMP1 CEACAM5 MMP2 | 5 | 0.57 | 23 | 0 |
| Cyfra SLPI TIMP1 CEACAM5 MMP2 OPN | 6 | 0.57 | 12 | 0 |
| Cyfra TFPI OPN MDK | 4 | 0.57 | 19 | 0 |
| TIMP1 MDK | 2 | 0.57 | 0 | 0 |
| SCC TFPI CEACAM5 OPN | 4 | 0.57 | 15 | 0 |
| Cyfra TIMP1 CEACAM5 OPN MDK | 5 | 0.57 | 27 | 0 |
| SLPI SCC CEACAM5 | 3 | 0.57 | 38 | 0 |
| TIMP1 SCC CEACAM5 | 3 | 0.57 | 38 | 0 |
| Cyfra SLPI SCC CEACAM5 | 4 | 0.57 | 38 | 0 |
| Cyfra TIMP1 SCC CEACAM5 | 4 | 0.57 | 38 | 0 |
| Cyfra SLPI TIMP1 TFPI OPN | 5 | 0.57 | 12 | 0 |
| TIMP1 CEACAM5 MMP2 OPN MDK | 5 | 0.57 | 8 | 0 |
| SLPI OPN MDK | 3 | 0.57 | 0 | 39 |
| Cyfra SLPI TFPI MMP2 | 4 | 0.56 | 19 | 0 |
| TFPI CEACAM5 MMP2 MDK | 4 | 0.56 | 4 | 0 |
| Cyfra SLPI TIMP1 TFPI CEACAM5 OPN | 6 | 0.56 | 12 | 0 |
| Cyfra MMP2 OPN MDK | 4 | 0.56 | 19 | 0 |
| TFPI MMP2 MDK | 3 | 0.56 | 4 | 0 |
| Cyfra SLPI CEACAM5 OPN MDK | 5 | 0.56 | 8 | 35 |
| Cyfra TIMP1 TFPI MMP2 OPN | 5 | 0.56 | 8 | 0 |
| TFPI MDK | 2 | 0.56 | 12 | 0 |
| TIMP1 CEACAM5 MDK | 3 | 0.56 | 4 | 0 |
| Cyfra SLPI TIMP1 TFPI | 4 | 0.56 | 23 | 0 |
| Cyfra SCC OPN | 3 | 0.56 | 23 | 0 |
| TIMP1 OPN MDK | 3 | 0.56 | 15 | 0 |
| Cyfra SLPI TFPI CEACAM5 MMP2 | 5 | 0.56 | 23 | 0 |
| Cyfra CEACAM5 MMP2 MDK | 4 | 0.56 | 35 | 0 |
| Cyfra TFPI CEACAM5 OPN MDK | 5 | 0.56 | 19 | 0 |
| Cyfra SLPI TFPI MMP2 OPN | 5 | 0.55 | 12 | 0 |
| Cyfra SLPI TIMP1 TFPI CEACAM5 | 5 | 0.55 | 23 | 0 |
| SLPI CEACAM5 MDK | 3 | 0.55 | 4 | 0 |
| Cyfra TIMP1 TFPI CEACAM5 MMP2 OPN | 6 | 0.55 | 12 | 0 |
| TFPI MMP2 OPN MDK | 4 | 0.55 | 4 | 0 |
| SLPI TIMP1 TFPI OPN | 4 | 0.55 | 8 | 0 |
| TFPI CEACAM5 MDK | 3 | 0.55 | 15 | 0 |
| SLPI CEACAM5 OPN MDK | 4 | 0.55 | 4 | 35 |
| Cyfra CEACAM5 MMP2 OPN MDK | 5 | 0.55 | 19 | 0 |
| Cyfra SLPI TFPI CEACAM5 MMP2 OPN | 6 | 0.55 | 15 | 0 |
| SLPI TIMP1 MMP2 OPN | 4 | 0.55 | 0 | 0 |
| SLPI TIMP1 TFPI CEACAM5 OPN | 5 | 0.55 | 12 | 0 |
| TIMP1 CEACAM5 OPN MDK | 4 | 0.55 | 15 | 0 |
| TFPI CEACAM5 MMP2 OPN MDK | 5 | 0.55 | 4 | 0 |
| SLPI TIMP1 TFPI | 3 | 0.55 | 12 | 0 |
| SLPI TIMP1 CEACAM5 MMP2 OPN | 5 | 0.54 | 4 | 0 |
| Cyfra SCC CEACAM5 OPN | 4 | 0.54 | 23 | 0 |
| SLPI TIMP1 TFPI CEACAM5 | 4 | 0.54 | 15 | 0 |
| SLPI TIMP1 MMP2 | 3 | 0.54 | 4 | 0 |
| TIMP1 TFPI MMP2 | 3 | 0.54 | 4 | 0 |
| Cyfra MDK | 2 | 0.54 | 15 | 0 |
| SCC OPN | 2 | 0.54 | 15 | 0 |
| SLPI TIMP1 CEACAM5 MMP2 | 4 | 0.54 | 8 | 0 |
| TIMP1 TFPI CEACAM5 MMP2 | 4 | 0.53 | 4 | 0 |
| Cyfra TIMP1 MMP2 | 3 | 0.53 | 0 | 0 |
| Cyfra SLPI MMP2 OPN | 4 | 0.53 | 0 | 0 |
| SLPI TFPI MMP2 OPN | 4 | 0.53 | 4 | 0 |
| SLPI TFPI CEACAM5 MMP2 OPN | 5 | 0.53 | 4 | 0 |
| TFPI OPN MDK | 3 | 0.53 | 12 | 0 |
| Cyfra SLPI TIMP1 OPN | 4 | 0.53 | 19 | 0 |
| Cyfra SCC | 2 | 0.53 | 54 | 0 |
| Cyfra OPN MDK | 3 | 0.53 | 8 | 0 |
| Cyfra TIMP1 MMP2 OPN | 4 | 0.53 | 15 | 0 |
| SLPI TFPI MMP2 | 3 | 0.53 | 12 | 0 |
| Cyfra SLPI CEACAM5 MMP2 OPN | 5 | 0.52 | 4 | 0 |
| TIMP1 TFPI MMP2 OPN | 4 | 0.52 | 0 | 0 |
| Cyfra SLPI MMP2 | 3 | 0.52 | 0 | 0 |
| SCC | 1 | 0.52 | 54 | 0 |
| Cyfra TIMP1 CEACAM5 MMP2 | 4 | 0.52 | 4 | 0 |
| SLPI TFPI CEACAM5 MMP2 | 4 | 0.52 | 12 | 0 |
| Cyfra SLPI TIMP1 CEACAM5 OPN | 5 | 0.52 | 19 | 0 |
| Cyfra CEACAM5 MDK | 3 | 0.52 | 15 | 0 |
| SCC CEACAM5 OPN | 3 | 0.52 | 15 | 0 |
| Cyfra TIMP1 TFPI | 3 | 0.52 | 8 | 0 |
| Cyfra TIMP1 CEACAM5 MMP2 OPN | 5 | 0.52 | 15 | 0 |
| Cyfra SCC CEACAM5 | 3 | 0.52 | 19 | 0 |
| TIMP1 TFPI CEACAM5 MMP2 OPN | 5 | 0.52 | 0 | 0 |
| Cyfra CEACAM5 OPN MDK | 4 | 0.51 | 8 | 0 |
| SLPI TIMP1 OPN | 3 | 0.51 | 12 | 0 |
| TFPI CEACAM5 OPN MDK | 4 | 0.51 | 15 | 0 |
| Cyfra SLPI CEACAM5 MMP2 | 4 | 0.51 | 4 | 0 |
| Cyfra TIMP1 TFPI OPN | 4 | 0.51 | 15 | 0 |
| Cyfra SLPI TFPI OPN | 4 | 0.51 | 8 | 0 |
| Cyfra TIMP1 TFPI CEACAM5 | 4 | 0.51 | 12 | 0 |
| SLPI TIMP1 CEACAM5 OPN | 4 | 0.51 | 12 | 0 |
| SCC CEACAM5 | 2 | 0.51 | 4 | 0 |
| MMP2 OPN MDK | 3 | 0.50 | 12 | 0 |
| Cyfra SLPI TFPI CEACAM5 OPN | 5 | 0.50 | 12 | 0 |
| Cyfra TIMP1 TFPI CEACAM5 OPN | 5 | 0.50 | 15 | 0 |
| Cyfra SLPI TIMP1 | 3 | 0.50 | 35 | 0 |
| SLPI MMP2 OPN | 3 | 0.50 | 0 | 0 |
| CEACAM5 MMP2 OPN MDK | 4 | 0.50 | 15 | 0 |
| SLPI TIMP1 | 2 | 0.49 | 35 | 0 |
| SLPI CEACAM5 MMP2 OPN | 4 | 0.49 | 0 | 0 |
| Cyfra SLPI TIMP1 CEACAM5 | 4 | 0.49 | 35 | 0 |
| SLPI TIMP1 CEACAM5 | 3 | 0.49 | 35 | 0 |
| TIMP1 MMP2 OPN | 3 | 0.48 | 4 | 0 |
| Cyfra SLPI TFPI | 3 | 0.48 | 8 | 0 |
| TIMP1 TFPI | 2 | 0.48 | 0 | 0 |
| Cyfra SLPI TFPI CEACAM5 | 4 | 0.48 | 12 | 0 |
| SLPI TFPI OPN | 3 | 0.48 | 0 | 0 |
| TIMP1 CEACAM5 MMP2 OPN | 4 | 0.48 | 8 | 0 |
| TIMP1 TFPI CEACAM5 | 3 | 0.48 | 0 | 0 |
| TIMP1 TFPI OPN | 3 | 0.48 | 12 | 0 |
| Cyfra TFPI MMP2 | 3 | 0.48 | 0 | 0 |
| Cyfra TFPI CEACAM5 MMP2 | 4 | 0.48 | 0 | 0 |
| Cyfra SLPI OPN | 3 | 0.48 | 12 | 0 |
| OPN MDK | 2 | 0.47 | 0 | 0 |
| SLPI TFPI CEACAM5 OPN | 4 | 0.47 | 0 | 0 |
| CEACAM5 MMP2 MDK | 3 | 0.47 | 23 | 0 |
| TIMP1 MMP2 | 2 | 0.47 | 0 | 0 |
| MMP2 MDK | 2 | 0.47 | 19 | 0 |
| TIMP1 TFPI CEACAM5 OPN | 4 | 0.47 | 15 | 0 |
| Cyfra TFPI MMP2 OPN | 4 | 0.47 | 15 | 0 |
| Cyfra TFPI CEACAM5 MMP2 OPN | 5 | 0.47 | 19 | 0 |
| MDK | 1 | 0.46 | 0 | 0 |
| TIMP1 CEACAM5 MMP2 | 3 | 0.46 | 0 | 0 |
| SLPI MMP2 | 2 | 0.46 | 0 | 0 |
| Cyfra SLPI CEACAM5 OPN | 4 | 0.46 | 12 | 0 |
| CEACAM5 OPN MDK | 3 | 0.46 | 4 | 0 |
| CEACAM5 MDK | 2 | 0.46 | 4 | 0 |
| SLPI CEACAM5 MMP2 | 3 | 0.46 | 0 | 0 |
| SLPI TFPI | 2 | 0.45 | 0 | 0 |
| SLPI TFPI CEACAM5 | 3 | 0.45 | 0 | 0 |
| Cyfra TIMP1 OPN | 3 | 0.45 | 12 | 0 |
| SLPI OPN | 2 | 0.44 | 0 | 0 |
| Cyfra TIMP1 CEACAM5 OPN | 4 | 0.44 | 12 | 0 |
| SLPI CEACAM5 OPN | 3 | 0.43 | 4 | 0 |
| Cyfra MMP2 OPN | 3 | 0.42 | 0 | 0 |
| Cyfra SLPI | 2 | 0.42 | 19 | 0 |
| Cyfra TIMP1 | 2 | 0.42 | 19 | 0 |
| Cyfra CEACAM5 MMP2 OPN | 4 | 0.42 | 4 | 0 |
| TIMP1 OPN | 2 | 0.41 | 0 | 0 |
| Cyfra SLPI CEACAM5 | 3 | 0.41 | 19 | 0 |
| Cyfra TIMP1 CEACAM5 | 3 | 0.41 | 19 | 0 |
| Cyfra TFPI OPN | 3 | 0.40 | 8 | 0 |
| TFPI CEACAM5 MMP2 OPN | 4 | 0.40 | 8 | 0 |
| TIMP1 CEACAM5 OPN | 3 | 0.40 | 4 | 0 |
| TFPI MMP2 OPN | 3 | 0.40 | 8 | 0 |
| Cyfra MMP2 | 2 | 0.40 | 0 | 0 |
| Cyfra TFPI CEACAM5 OPN | 4 | 0.40 | 12 | 0 |
| Cyfra CEACAM5 MMP2 | 3 | 0.39 | 4 | 0 |
| TFPI CEACAM5 MMP2 | 3 | 0.39 | 0 | 0 |
| TFPI MMP2 | 2 | 0.38 | 0 | 0 |
| Cyfra TFPI | 2 | 0.38 | 8 | 0 |
| Cyfra TFPI CEACAM5 | 3 | 0.37 | 12 | 0 |
| SLPI | 1 | 0.37 | 0 | 0 |
| TIMP1 | 1 | 0.37 | 0 | 0 |
| SLPI CEACAM5 | 2 | 0.37 | 4 | 0 |
| TIMP1 CEACAM5 | 2 | 0.37 | 4 | 0 |
| MMP2 OPN | 2 | 0.35 | 0 | 0 |
| CEACAM5 MMP2 OPN | 3 | 0.35 | 0 | 0 |
| TFPI CEACAM5 OPN | 3 | 0.34 | 0 | 0 |
| TFPI OPN | 2 | 0.34 | 0 | 0 |
| Cyfra OPN | 2 | 0.32 | 12 | 0 |
| Cyfra CEACAM5 OPN | 3 | 0.31 | 12 | 0 |
| TFPI CEACAM5 | 2 | 0.29 | 0 | 0 |
| CEACAM5 MMP2 | 2 | 0.27 | 0 | 0 |
| TFPI | 1 | 0.27 | 0 | 0 |
| MMP2 | 1 | 0.26 | 0 | 0 |
| CEACAM5 OPN | 2 | 0.23 | 4 | 0 |
| OPN | 1 | 0.23 | 0 | 0 |
| Cyfra | 1 | 0.19 | 19 | 0 |
| Cyfra CEACAM5 | 2 | 0.19 | 19 | 0 |
| CEACAM5 | 1 | 0.04 | 4 | 0 |

**TABLE 9**

| Small Cell vs. Other Tumors | Split-point | | | | | | Split-point | | | |
|---|---|---|---|---|---|---|---|---|---|---|
| | Trained/tested on 44x44, higher in NSC | | | | | | Trained/tested on 44x44, higher in small cell | | | |
| Panel | Size | AUC | Sens at 95% Spec | Spec at 95% Sens | | Panel | Size | AUC | Sens at 95% Spec | Spec at 95% Sens |
| MIF SLPI SCC OPN GRP | 5 | 0.95 | 95 | 100 | | ENO2 MMP2 Ca19-9 CAIX GRP | 5 | 0.92 | 40 | 87 |
| SLPI SCC OPN DEFA1 | 4 | 0.93 | 87 | 80 | | ENO2 MMP2 CA242 CAIX GRP | 5 | 0.92 | 40 | 87 |
| SLPI TIMP1 SCC CEACAM5 OPN Ca72-4 DEFA1 | 7 | 0.93 | 92 | 20 | | SLPI EN02 MMP2 Ca19-9 CAIX GRP | 6 | 0.92 | 40 | 87 |
| SLPI SCC OPN | 3 | 0.93 | 92 | 20 | | SLPI ENO2 MMP2 CA242 CAIX GRP | 6 | 0.92 | 40 | 87 |
| MIF SLPI SCC OPN | 4 | 0.93 | 90 | 60 | | ENO2 MMP2 Ca19-9 CA242 CAIX GRP | 6 | 0.92 | 40 | 87 |
| SLPI SCC OPN Ca72-4 DEFA1 | 5 | 0.93 | 90 | 40 | | SLPI ENO2 MMP2 Ca19-9 CA242 CAIX GRP | 7 | 0.92 | 40 | 87 |
| MIF SLPI SCC OPN DEFA1 | 5 | 0.92 | 72 | 80 | | ENO2 MMP2 Ca19-9 CA242 CAIX | 5 | 0.92 | 20 | 90 |
| SCC OPN DEFA1 | 3 | 0.92 | 90 | 20 | | SLPI EN02 MMP2 Ca19-9 CA242 CAIX | 6 | 0.92 | 20 | 90 |
| SLPI SCC CEACAM5 OPN DEFA1 | 5 | 0.91 | 72 | 80 | | MIF ENO2 MMP2 Ca19-9 CA242 CAIX GRP | 7 | 0.92 | 40 | 87 |
| SLPI TIMP1 SCC OPN DEFA1 | 5 | 0.91 | 90 | 20 | | TIMP1 ENO2 MMP2 Ca19-9 CA242 CAIX GRP | 7 | 0.92 | 40 | 87 |
| SLPI SCC OPN CA242 DEFA1 | 5 | 0.91 | 90 | 20 | | ENO2 MMP2 AMBP Ca19-9 CA242 CAIX GRP | 7 | 0.92 | 40 | 87 |
| SLPI SCC OPN GRP DEFA1 | 5 | 0.91 | 90 | 20 | | MIF SLPI ENO2 MMP2 Ca19-9 CA242 CAIX GRP | 8 | 0.92 | 40 | 87 |
| MIF SLPI SCC OPN Ca72-4 GRP DEFA1 | 7 | 0.91 | 90 | 20 | | SLPI TIMP1 ENO2 MMP2 Ca19-9 CA242 CAIX GRP | 8 | 0.92 | 40 | 87 |
| Mlf SIPI SCC OPN GRP DEFA1 | 6 | 0.91 | 82 | 60 | | SLDI ENO2 MMP2 AMBP Ca19-9 CA242 CAIX GRP | 8 | 0.92 | 40 | 87 |
| SLPI SCC CEACAM5 OPN Ca72-4 DEFA1 | 6 | 0.91 | 82 | 60 | | MIF ENO2 MMP2 Ca19-9 CA242 CAIX | 6 | 0.91 | 20 | 90 |
| SLPI TIMP1 SCC OPN Ca72-4 DEFA1 | 6 | 0.91 | 74 | 80 | | MIF ENO2 MMP2 Cal9-9 CAIX GRP | 6 | 0.91 | 40 | 87 |
| SLPI SCC OPN Ca72-4 GRP DEFA1 | 6 | 0.91 | 74 | 80 | | MIF ENO2 MMP2 CA242 CAIX GRP | 6 | 0.91 | 40 | 87 |
| MIF SLPI SCC CEACAM5 OPN GRP DEFA1 | 7 | 0.91 | 72 | 80 | | TIMP1 ENO2 MMP2 Ca19-9 CA242 CAIX | 6 | 0.91 | 20 | 90 |
| SLPI SCC CEACAM5 OPN | 4 | 0.91 | 87 | 40 | | TIMP1 ENO2 MMP2 Ca19-9 CAIX GRP | 6 | 0.91 | 40 | 87 |
| MIF SLPI SCC OPN Ca72-4 DEFA1 | 6 | 0.91 | 79 | 60 | | TIMP1 ENO2 MMP2 CA242 CAIX GRP | 6 | 0.91 | 40 | 87 |
| MIF SLPI SCC CEACAM5 OPN Ca72-4 DEFA1 | 7 | 0.90 | 67 | 80 | | ENO2 MMP2 AMBP Ca19-9 CAIX GRP | 6 | 0.9128205 | 40 | 87.1794872 |
| SLPI SCC OPN Ca19-9 DEFA1 | 5 | 0.903 | 71.795 | 40 | | ENO2 MMP2 AMBP CA242 CAIX GRP | 6 | 0.9128205 | 40 | 87.1794872 |
| SCC CEACAM5 OPN DEFA1 | 4 | 0.90 | 79 | 60 | | MIF SLPI ENO2 MMP2 Ca19-9 CA242 CAIX | 7 | 0.9128205 | 20 | 89.7435897 |
| SLPI TIMP1 SCC CEACAM5 OPN DEFA1 | 6 | 0.90 | 79 | 60 | | MIF SLPI ENO2 MMP2 Ca19.9 CAIX GRP | 7 | 0.912820S | 40 | 87.1794872 |
| SLPI SCC CEACAM5 OPN CA242 OEFA1 | 6 | 0.90 | 79 | 60 | | MIF SLPI EN02 MMP2 CA242 CAIX GRP | 7 | 0.9128205 | 40 | 87.1794872 |
| MIF SLPI SCC CEACAM5 OPN Ca72-4 GRP DEFA1 | 8 | 0.90 | 79 | 60 | | SLPI TIMP1 ENO2 MMP2 Ca19-9 CA242 CAIX | 7 | 0.9128205 | 20 | 89.7435897 |
| MIF SLPI SCC CEACAM5 OPN | 5 | 0.90 | 67 | 80 | | SLPI TIMP1 ENO2 MMP2 Ca19-9 CAIX GRP | 7 | 0.9128205 | 40 | 87.1794872 |
| Cyfra MIF SLPI SCC OPN | 5 | 0.90 | 90 | 0 | | SLPI TIMP1 ENO2 MMP2 CA242 CAIX GRP | 7 | 0.9128205 | 40 | 87.1794872 |
| MIF SLPI SCC CEACAM5 OPN GRP | 6 | 0.90 | 85 | 40 | | SLPI ENO2 MMP2 AMBP Ca19-9 CAIX GRP | 7 | 0.9128205 | 40 | 87.1794872 |
| MIF SLPI SCC OPN Ca19-9 GRP | 6 | 0.90 | 85 | 40 | | SLPI ENO2 MMP2 AMBP CA242 CAIX GRP | 7 | 0.9128205 | 40 | 87.1794872 |
| SLPI SCC CEACAM5 OPN GRP DEFA1 | 6 | 0.90 | 85 | 40 | | MIF TIMP1 ENO2 MMP2 Ca19-9 CA242 CAIX GRP | 8 | 0.9128205 | 40 | 87.1794872 |
| SLPI TIMP1 SCC OPN | 4 | 0.90 | 82 | 60 | | MIF ENO2 MMP2 AMBP Ca19-9 CA242 CAIX GRP | 8 | 0.9128205 | 40 | 87.1794872 |
| Cyfra SLPI SCC OPN Ca19-9 DEFA1 | 6 | 0.90 | 82 | 60 | | TIMP1 ENO2 MMP2 AMBP Ca19-9 CA242 CAIX GRP | 8 | 0.9128205 | 40 | 87.1794872 |
| SLPI SCC OPN Ca19-9 GRP DEFA1 | 6 | 0.90 | 82 | 60 | | MIF SLPI TIMP1 EN02 MMP2 Ca19-9 CA242 CAIX GRP | 9 | 0.9128205 | 40 | 87.1794872 |
| Cyfra MIF SLPI SCC CEACAM5 OPN DEFA1 | 7 | 0.89 | 64 | 80 | | MIF SLPI ENO2 MMP2 AMBP Ca19-9 CA242 CAIX GRP | 9 | 0.9128205 | 40 | 87.1794872 |
| MIF SLPI SCC CEACAM5 OPN CA242 OEFA1 | 7 | 0.89 | 72 | 20 | | SLPI TIMP1 EN02 MMP2 AMBP Ca19-9 CA242 CAIX GRP | 9 | 0.9128205 | 40 | 87.1794872 |
| SLPI SCC OPN AMBP DEFA1 | 5 | 0.89 | 72 | 40 | | Cyfra ENO2 MMP2 CAIX GRP | 5 | 0.9076923 | 40 | 84.6153846 |
| Cyfra SLPI SCC OPN OEFA1 | 5 | 0.89 | 87 | 20 | | Cyfra SLPI ENO2 MMP2 CAIX GRP | 6 | 0.9076923 | 40 | 84.6153846 |
| SLPI SCC OPN Ca19-9 | 4 | 0.89 | 85 | 40 | | Cyfra ENO2 MMP2 Ca19-9 CAIX GRP | 6 | 0.9076923 | 40 | 84.6153846 |
| Cyfra MIF SLPI SCC OPN OEFA1 | 6 | 0.89 | 77 | 60 | | Cyfra ENO2 MMP2 CA242 CAIX GRP | 6 | 0.9076923 | 40 | 84.6153846 |
| SLPI SCC CEACAM5 OPN Ca72-4 GRP DEFA1 | 7 | 0.89 | 64 | 40 | | SCC ENO2 MMP2 Ca19-9 CAIX GRP | 6 | 0.9076923 | 40 | 84.6153846 |
| MIF SLPI SCC OPN Ca19-9 GRP DEFA1 | 7 | 0.89 | 77 | 20 | | SCC ENO2 MMP2 CA242 CAIX GRP | 6 | 0.9076923 | 40 | 84.6153846 |
| SLPI SCC OPN AM8P | 4 | 0.89 | 85 | 20 | | ENO2 TFPI MMP2 Ca19-9 CAIX GRP | 6 | 0.9076923 | 40 | 84.6153846 |
| Cyfra MIF SLPI SCC CEACAM5 OPN Ca72-4 DEFA1 | 8 | 0.89 | 79 | 60 | | ENO2 TFPI MMP2 CA242 CAIX GRP | 6 | 0.9076923 | 40 | 94.6153846 |
| MIF SLPI SCC OPN AMBP DEFA1 | 6 | 0**.**88 | 51 | 80 | | EN02 CEACAM5 MMP2 Ca19-9 CAIX GRP | 6 | 0.9076923 | 40 | 84.6153946 |
| SLPI SCC CEACAM5 OPN Ca72-4 CA242 GRP DEFA1 | 8 | 0**.**88 | 51 | 80 | | ENO2 CEACAM5 MMP2 CA242 CAIX GRP | 6 | 0.9076923 | 40 | 84.6153846 |
| SLPI OPN DEFA1 | 3 | 0**.**88 | 56 | 80 | | Cyfra SLPI ENO2 MMP2 Ca19-9 CAIX GRP | 7 | 0.9076923 | 40 | 84.6153846 |
| SLPI SCC TFPI OPN DEFA1 | 5 | 0**.**88 | 67 | 40 | | Cyfra SLPI ENO2 MMP2 CA242 CAIX GRP | 7 | 0.9076923 | 40 | 84.6153846 |
| Cyfra SLPI SCC OPN Ca72-4 DEFA1 | 6 | 0**.**88 | 72 | 0 | | Cyfra ENO2 MMP2 Ca19-9 CA242 CAIX GRP | 7 | 0.9076923 | 40 | 94.6153846 |
| SLPI SCC OPN Ca72-4 | 4 | 0**.**88 | 79 | 20 | | MIF TIMP1 ENO2 MMP2 Ca19-9 CA242 CAIX | 7 | 0.9076923 | 20 | 89.7435897 |
| SLPI SCC OPN CA242 | 4 | 0**.**88 | 82 | 0 | | SLPI SCC ENO2 MMP2 Ca19-9 CAIX GRP | 7 | 0.9076923 | 40 | 84.6153846 |
| Cyfra SLPI SCC CEACAM5 OPN DEFA1 | 6 | 0**.**88 | 82 | 40 | | SLPI SCC ENO2 MMP2 CA242 CAIX GRP | 7 | 0.9076923 | 40 | 84.6153846 |
| MIF SLPI SCC CEACAM5 OPN CA242 | 6 | 0**.**88 | 82 | 40 | | SLPI ENO2 TFPI MMP2 Ca19-9 CAIX GRP | 7 | 0.9076923 | 40 | 84.6153846 |
| MIF SLPI SCC CEACAM5 OPN DEFA1 | 6 | 0**.**88 | 49 | 80 | | SLPI ENO2 TFPI MMP2 CA242 CAIX GRP | 7 | 0.9076923 | 40 | 84.6153846 |
| MIF SLPI SCC TFPI OPN DEFA1 | 6 | 0.88 | 59 | 80 | | SLPI ENO2 CEACAM5 MMP2 Ca19-9 CAIX GRP | 7 | 0.9076923 | 40 | 84.6153846 |
| MIF SLPI SCC OPN CA242 | 5 | 0.88 | 64 | 20 | | SLPI ENO2 CEACAM5 MMP2 CA242 CAIX GRP | 7 | 0.9076923 | 40 | 84.6153846 |
| Cyfra MIF SLP1 SCC OPN Ca19-9 DEFA1 | 7 | 0.88 | 64 | 20 | | SCC ENO2 MMP2 Ca19-9 CA242 CAIX GRP | 7 | 0.9076923 | 40 | 84.6153846 |
| MIF SLPI SCC TFPI OPN GRP DEFA1 | 7 | 0.88 | 64 | 20 | | ENO2 TFPI MMP2 Ca19-9 CA242 CAIX GRP | 7 | 0.9076923 | 40 | 84.6153846 |
| SLPI SCC CEACAM5 OPN CA242 GRP DEFA1 | 7 | 0**.**88 | 64 | 20 | | ENO2 CEACAM5 MMP2 Ca19-9 CA242 CAIX GRP | 7 | 0.9076923 | 40 | 84.6153846 |
| SLPI SCC CEACAM5 OPN Ca72-4 CA242 DEFA1 | 7 | 0.88 | 64 | 40 | | Cyfra SLPI ENO2 MMP2 Ca19-9 CA242 CAIX GRP | 8 | 0.9076923 | 40 | 94.6153846 |
| MIF SLPI SCC CEACAM5 OPN Ca72-4 CA242 DEFA1 | 8 | 0**.**88 | 82 | 40 | | MIF SLPI TIMP1 ENO2 MMP2 Ca19-9 CA242 CAIX | 8 | 0.9076923 | 20 | 89.7435897 |
| SCC OPN | 2 | 0**.**88 | 77 | 60 | | SLPI SCC EN02 MMP2 Ca19-9 CA242 CAIX GRP | 8 | 0.9076923 | 40 | 84.6153846 |
| SLPI SCC OPN GRP | 4 | 0**.**88 | 77 | 60 | | SLPI ENO2 TFPI MMP2 Ca19.9 CA242 CAIX GRP | 8 | 0.9076923 | 40 | 84.6153846 |
| SCC OPN Ca72-4 OEFA1 | 4 | 0.88 | 74 | 60 | | SLPI ENO2 CEACAM5 MMP2 Ca19-9 CA242 CAIX GRP | 8 | 0.9076923 | 40 | 84.6153846 |
| SLPI TIMP1 SCC OPN Ca19-9 DEFA1 | 6 | 0**.**88 | 77 | 60 | | MIF TIMP1 ENO2 MMP2 AMBP Ca19.9 CA242 CAIX GRP | 9 | 0.9076923 | 20 | 87.1794872 |
| SLPI SCC OPN CA242 GRP DEFA1 | 6 | 0**.**88 | 77 | 60 | | MIF SLPI TIMP1 ENO2 MMP2 AMBP Ca19-9 CA242 CAIX GRP | 10 | 0.9076923 | 20 | 87.1794872 |
| MIF SLPI TIMP1 SCC CEACAM5 OPN Ca72-4 DEFA1 | 8 | 0**.**88 | 77 | 60 | | Cyfra MIF ENO2 MMP2 Ca19.9 CAIX GRP | 7 | 0.9025641 | 40 | 84.6153846 |
| MIF SLPI TIMP1 SCC CEACAM5 OPN DEFA1 | 7 | 0.87 | 62 | 20 | | Cyfra MIF ENO2 MMP2 CA242 CAIX GRP | 7 | 0.9025641 | 40 | 84.6153846 |
| MIF SLPI SCC CEACAM5 OPN CA242 GRP | 7 | 0.87 | 67 | 0 | | Cyfra TIMP1 ENO2 MMP2 Ca19.9 CAIX GRP | 7 | 0.9025641 | 40 | 84.6153846 |
| MIF SLPI SCC TFPI OPN | S | 0.87 | 72 | 20 | | Cyfra TIMP1 ENO2 MMP2 CA242 CAIX GRP | 7 | 0.9025641 | 40 | 84.6153846 |
| MIF SLPI $CC OPN Ca19-9 DEFA1 | 6 | 0.87 | 56 | 60 | | Cyfra ENO2 MMP2 AMBP Ca19-9 CAIX GRP | 7 | 0.9025641 | 40 | 84.6153846 |
| Cyfra SLPI TIMP1 SCC OPN Ca19-9 DEFA1 | 7 | 0.87 | 87 | 0 | | Cyfra ENO2 MMP2 AMBP CA242 CAIX GRP | 7 | 0.9025641 | 40 | 84.6153846 |
| Cyfra SLPI SCC CEACAM5 OPN Ca72-4 DEFA1 | 7 | 0.87 | 67 | 40 | | MIF TIMP1 ENO2 MMP2 Ca19-9 CAIX GRP | 7 | 0.9025641 | 40 | 87.1794872 |
| SLPI TIMP1 SCC CEACAM5 OPN CA242 DEFA1 | 7 | 0.87 | 87 | 0 | | MIF TIMP1 ENO2 MMP2 CA242 CAIX GRP | 7 | 0.9025641 | 40 | 87.1794872 |
| Cyfra MIF SLPI SCC OPN Ca72-4 DEFA1 | 7 | 0.87 | 85 | 20 | | MIF ENO2 MMP2 AMBP Ca19-9 CAIX GRP | 7 | 0.9025641 | 40 | 87.1794872 |
| Cyfra SLPI SCC OPN CA242 DEFA1 | 6 | 0.87 | 79 | 0 | | MIF ENO2 MMP2 AMBP CA242 CAIX GRP | 7 | 0.9025641 | 40 | 87.1794872 |
| SLPI TIMP1 SCC OPN GRP DEFA1 | 6 | 0.87 | 79 | 0 | | TIMP1 ENO2 MMP2 AMBP Ca19-9 CAIX GRP | 7 | 0.9025641 | 40 | 87.1794872 |
| SLPI SCC TFPI OPN CA242 DEFA1 | 6 | 0.87 | 79 | 0 | | TIMP1 ENO2 MMP2 AMBP CA242 CAIX GRP | 7 | 0.9025641 | 40 | 87.1794872 |
| SLFI SCC OPN AMBP CA242 DEFA1 | 6 | 0.87 | 79 | 0 | | Cyfra MIF SLPI ENO2 MMP2 Ca19-9 CAIX GRP | 8 | 0.9025641 | 40 | 84.6153846 |
| Cyfra MIF SLPI SCC CEACAM5 OPN CA242 DEFA1 | 8 | 0.87 | 82 | 40 | | Cyfra MIF SLPI ENO2 MMP2 CA242 CAIX GRP | 8 | 0.9025641 | 40 | 84.6153846 |
| MIF SLPI SCC CEACAM5 OPN CA242 GRP DEFA1 | 8 | 0.87 | 82 | 40 | | Cyfra MIF ENO2 MMP2 Ca19-9 CA242 CAIX GRP | 8 | 0.9025641 | 40 | 84.6153846 |
| MIF SLPI SCC OPN CA242 DEFA1 | 6 | 0.87 | 79 | 40 | | Cyfra SLPI TIMP1 ENO2 MMP2 Ca19-9 CAIX GRP | 8 | 0.9025641 | 40 | 84.6153846 |
| SLPI SCC OPN AMBP Ca72-4 DEFA1 | 6 | 0.87 | 74 | 60 | | Cyfra SLPI TIMP1 ENO2 MMP2 CA242 CAIX GRP | 8 | 0.9025641 | 40 | 84.6153846 |
| SLPI SCC OPN Ca72-4 CA242 DEFA1 | 6 | 0.87 | 74 | 60 | | Cyfra SLPI ENO2 MMP2 AMBP Ca19.9 CAIX GRP | 8 | 0.9025641 | 40 | 84.6153846 |
| SLPI OPN Ca72-4 DEFA1 | 4 | 0.87 | 72 | 60 | | Cyfra SLPI ENO2 MMP2 AMBP CA242 CAIX GRP | 8 | 0.9025641 | 40 | 84.6153846 |
| SLPI T1MP1 SCC CEACAM5 OPN | 5 | 0.87 | 51 | 80 | | Cyfra TIMP1 ENO2 MMP2 Ca19-9 CA242 CAIX GRP | 8 | 0.9025641 | 40 | 84.6153846 |
| MIF SLPI TIMP1 SCC OPN | 5 | 0.87 | 59 | 20 | | Cyfra EN02 MMP2 AM8P Ca19-9 CA242 CAIX GRP | 8 | 0.9025641 | 40 | 84.6153846 |
| MIF SLPI SCC OPN Ca72-4 | 5 | 0.87 | 54 | 40 | | MIF SLPI TIMP1 ENO2 MMP2 Ca19-9 CAIX GRP | 8 | 0.9025641 | 40 | 87.1794872 |
| MIF SCC CEACAM5 OPN DEFA1 | 5 | 0.87 | 56 | 20 | | MIF SLPI TIMP1 ENO2 MMP2 CA242 CAIX GRP | 8 | 0.9025641 | 40 | 87.1794872 |
| SLPI SCC CEACAM5 OPN CA242 | 5 | 0.87 | 67 | 20 | | MIF SLPI ENO2 MMP2 AMBP Ca19-9 CAIX GRP | 8 | 0.9025641 | 40 | 87.1794872 |
| Cyfra SLPI SCC CEACAM5 OPN CA242 DEFA1 | 7 | 0.87 | 67 | 20 | | MIF SLPI ENO2 MMP2 AMBP CA242 CAIX GRP | 8 | 0.9025641 | 40 | 87.1794872 |
| SLPI SCC OPN Ca19-9 Ca72-4 GRP DEFA1 | 7 | 0.87 | 64 | 40 | | MIF SCC ENO2 MMP2 Ca19-9 CA242 CAIX GRP | 8 | 0.9025641 | 40 | 84.6153846 |
| SLPI TIMP1 SCC OPN Ca72-4 GRP DEFA1 | 7 | 0.87 | 85 | 20 | | MIF ENO2 TFPI MMP2 Ca19-9 CA242 CAIX GRP | 8 | 0.9025641 | 40 | 84.6153846 |
| SLPI SCC TFPI OPN | 4 | 0.87 | 82 | 20 | | MIF EN02 CEACAM5 MMP2 Ca19-9 CA242 CAIX GRP | 8 | 0.9025641 | 40 | 84.6153846 |
| MIF SLPI TIMP1 SCC CEACAM5 OPN | 6 | 0.87 | 79 | 40 | | SLPI TIMP1 ENO2 MMP2 AMBP Ca19-9 CAIX GRP | 8 | 0.9025641 | 40 | 87.1794872 |
| MIF SLPI SCC CEACAM5 OPN Ca72-4 | 6 | 0.87 | 79 | 40 | | SLPI TIMP1 ENO2 MMP2 AMBP CA242 CAIX GRP | 8 | 0.9025641 | 40 | 87.1794872 |
| SCC TFPI OPN DEFA1 | 4 | 0.87 | 74 | 60 | | TIMP1 SCC ENO2 MMP2 Ca19-9 CA242 CAIX GRP | 8 | 0.9025641 | 40 | 84.6153846 |
| SCC OPN AMBP DEFA1 | 4 | 0.87 | 74 | 60 | | TIMP1 ENO2 TFPI MMP2 Ca19-9 CA242 CAIX GRP | 8 | 0.9025641 | 40 | 84.6153846 |
| SLPI TIMP1 SCC TFPI OPN DEFA1 | 6 | 0.87 | 74 | 60 | | TIMP1 ENO2 CEACAM5 MMP2 Ca19-9 CA242 CAIX GRP | 8 | 0.9025641 | 40 | 84.6153846 |
| SLPI TIMP1 SCC OPN AMBP DEFA1 | 6 | 0.87 | 74 | 60 | | SCC ENO2 MMP2 AMBP Ca19-9 CA242 CAIX GRP | 8 | 0.9025641 | 40 | 84.6153846 |
| SLPI TIMP1 SCC CEACAM5 OPN Ca72-4 GRP DEFA1 | 8 | 0.87 | 74 | 60 | | ENO2 TFPI MMP2 AMBP Ca19-9 CA242 CAIX GRP | 8 | 0.9025641 | 40 | 84.6153846 |
| MIF SLPI SCC OPN AMBP | 5 | 0.86 | 56 | 20 | | EN02 CEACAM5 MMP2 AMBP Ca19-9 CA242 CAIX GRP | 8 | 0.9025641 | 40 | 84.6153846 |
| SLPI SCC OPN CAIX DEFA1 | 5 | 0.86 | 59 | 20 | | Cyfra MIF SLPI ENO2 MMP2 Ca19-9 CA242 CAIX GRP | 9 | 0.9025641 | 40 | 84.6153846 |
| MIF SLPI SCC CEACAM5 OPN Ca72-4 GRP | 7 | 0.86 | 56 | 20 | | Cyfra SLPI TIMP1 ENO2 MMP2 Ca19-9 CA242 CAIX GRP | 9 | 0.9025641 | 40 | 84.6153846 |
| SLPI SCC OPN Ca19-9 GRP | 5 | 0.86 | 64 | 20 | | Cyfra SLPI ENO2 MMP2 AMBP Ca19-9 CA242 CAIX GRP | 9 | 0.9025641 | 40 | 84.6153846 |
| MIF SLPI SCC OPN AMBP Ca72-4 DEFA1 | 7 | 0.86 | 62 | 40 | | MIF SLPI SCC ENO2 MMP2 Ca19-9 CA242 CAIX GRP | 9 | 0.9025641 | 40 | 84.6153846 |
| MIF SLPI SCC OPN CA242 GRP DEFA1 | 7 | 0.86 | 72 | 20 | | MIF SLPI EN02 TFPI MMP2 Ca19-9 CA242 CAIX GRP | 9 | 0.9025641 | 40 | 84.6153846 |
| MIF SLPI SCC TFPI OPN Ca72-4 DEFA1 | 7 | 0.86 | 67 | 40 | | MIF SLPI ENO2 CEACAM5 MMP2 Ca19-9 CA242 CAIX GRP | 9 | 0.9025641 | 40 | 84.615384b |
| MIF SLPI TIMP1 SCC TFPI OPN | 6 | 0.86 | 82 | 0 | | SLPI TIMP1 SCC ENO2 MMP2 Ca19-9 CA242 CAIX GRP | 9 | 0.9025641 | 40 | 84.6153846 |
| MIF SLPI SCC TFPI OPN CA242 | 6 | 0.86 | 82 | 0 | | SLPI TIMP1 ENO2 TFPI MMP2 Ca19-9 CA242 CAIX GRP | 9 | 0.9025641 | 40 | 84.6153846 |
| SLPI SCC CEACAM5 OPN Ca19-9 DEFA1 | 6 | 0.86 | 56 | 60 | | SLPI TIMP1 ENO2 CEACAM5 MMP2 Ca19-9 CA242 CAIX GRP | 9 | 0.9025641 | 40 | 84.6153846 |
| SLPI SCC TFPI OPN GRP DEFA1 | 6 | 0.86 | 79 | 40 | | SLPI SCC ENO2 MMP2 AMBP Ca19-9 CA242 CAIX GRP | 9 | 0.9025641 | 40 | 84.6153846 |
| MIF SLPI SCC OPN Ca19-9 Ca72-4 GRP DEFA1 | 8 | 0.86 | 79 | 40 | | SLPI ENO2 TFPI MMP2 AMBP Ca19-9 CA242 CAIX GRP | 9 | 0.9025641 | 40 | 84.6153846 |
| Cyfra SLPI SCC OPN | 4 | 0.86 | 77 | 0 | | SLPI ENO2 CEACAM5 MMP2 AMBP Ca19-9 CA242 CAIX GRP | 9 | 0.9025641 | 40 | 84.6153846 |
| SCC OPN GRP DEFA1 | 4 | 0.86 | 77 | 0 | | ENO2 MMP2 CAIX GRP | 4 | 0.8974359 | 20 | 87.1794872 |
| SLPI SCC TFPI OPN Ca72-4 DEFA1 | 6 | 0.86 | 74 | 20 | | Cyfra ENO2 MMP2 Ca19-9 CAIX | 5 | 0.8974359 | 20 | 87.1794872 |
| MIF SLPI SCC OPN Ca72-4 GRP | 6 | 0.86 | 69 | 60 | | Cyfra ENO2 MMP2 CA242 CAIX | S | 0.8974359 | 20 | 87.1794872 |
| SCC CEACAM5 OPN Ca72-4 DEFA1 | 5 | 0.86 | 49 | 40 | | SLPI ENO2 MMP2 CAIX GRP | 5 | 0.8974359 | 20 | 87.1794872 |
| Cyfra SLPI SCC OPN Ca19-9 GRP DEFA1 | 7 | 0.86 | 64 | 20 | | Cyfra MIF ENO2 MMP2 CAIX GRP | 6 | 0.8974359 | 40 | 84.6153846 |
| SLPI TIMP1 SCC CEACAM5 OPN GRP DEFA1 | 7 | 0.86 | 62 | 20 | | Cyfra SLPI ENO2 MMP2 Ca19-9 CAIX | 6 | 0.8974359 | 20 | 87.1794872 |
| MIF SLPI SCC TFPI OPN CA242 DEFA1 | 7 | 0.86 | 69 | 20 | | Cyfra SLPI ENO2 MMP2 CA242 CAIX | 6 | 0.8974359 | 20 | 87.1794872 |
| MIF SLPI SCC OPN Ca72-4 CA242 DEFA1 | 7 | 0.86 | 67 | 20 | | Cyfra TIMP1 ENO2 MMP2 CAIX GRP | 6 | 0.8974359 | 40 | 84.6153846 |
| MIF SLPI SCC CEACAM5 OPN Ca19-9 GRP DEFA1 | 8 | 0.86 | 56 | 60 | | Cyfra ENO2 MMP2 AMBP CAIX GRP | 6 | 0.8974359 | 40 | 84.6153846 |
| MIF SLPI TIMP1 SCC OPN Ca72-4 DEFA1 | 7 | 0.86 | 64 | 40 | | Cyfra ENO2 MMP2 Ca19-9 CA242 CAIX | 6 | 0.8974359 | 20 | 87.1794872 |
| SLPI OPN | 2 | 0.86 | 79 | 40 | | SCC ENO2 MMP2 Ca19-9 CA242 CAIX | 6 | 0.8974359 | 20 | 87.1794872 |
| SLPI SCC OPN CAIX | 4 | 0.86 | 79 | 40 | | ENO2 TFPI MMP2 Ca19-9 CA242 CAIX | 6 | 0.8974359 | 20 | 87.1794872 |
| MIF SLPI SCC TFPI OPN GRP | 6 | 0.86 | 79 | 40 | | ENO2 CEACAM5 MMP2 Ca19-9 CA242 CAIX | 6 | 0.8974359 | 20 | 87.1794872 |
| Cyfra MIF SLPI SCC OPN Ca19-9 GRP DEFA1 | 8 | 0.86 | 79 | 40 | | ENO2 MMP2 AMBP Ca19-9 CA242 CAIX | 6 | 0.8974359 | 20 | 87.1794872 |
| MIF SLPI SCC TFPI OPN CA242 GRP DEFA1 | 8 | 0.86 | 79 | 40 | | Cyfra MIF SLPI ENO2 MMP2 CAIX GRP | 7 | 0.8974359 | 40 | 84.6153846 |
| SLPI TIMP1 SCC OPN CA242 DEFA1 | 6 | 0.86 | 72 | 60 | | Cyfra MIF ENO2 MMP2 Ca19-9 CA242 CAIX | 7 | 0.8974359 | 20 | 87.1794872 |
| MIF SLPI SCC TFPI OPN Ca72-4 GRP DEFA1 | 8 | 0.86 | 72 | 60 | | Cyfra SLPI TIMP1 ENO2 MMP2 CAIX GRP | 7 | 0.8974359 | 40 | 84.6153846 |
| MIF SLPI SCC CEACAM5 OPN Ca19-9 Ca72-4 GRP DEFA1 | 9 | 0.86 | 72 | 40 | | Cyfra SLPI ENO2 MMP2 AMBP CAIX GRP | 7 | 0.8974359 | 40 | 84.6153846 |
| SLPI TIMP1 SCC OPN Ca19-9 | 5 | 0.85 | 59 | 20 | | Cyfra SLPI ENO2 MMP2 Ca19-9 CA242 CAIX | 7 | 0.8974359 | 20 | 87.1794872 |
| SCC CEACAM5 OPN GRP DEFA1 | 5 | 0.85 | 59 | 20 | | Cyfra TIMP1 ENO2 MMP2 Ca19-9 CA242 CAIX | 7 | 0.8974359 | 20 | 87.1794872 |
| MIF SLPI TIMP1 SCC OPN Ca72-4 | 6 | 0.85 | 36 | 80 | | MIF SCC ENO2 MMP2 Ca19-9 CAIX GRP | 7 | 0.8974359 | 40 | 84.6153846 |
| Cyfra MIF SLPI SCC OPN GRP DEFA1 | 7 | 0.85 | 59 | 20 | | MIF SCC ENO2 MMP2 CA242 CAIX GRP | 7 | 0.8974359 | 40 | 84.6153846 |
| MIF SLPI SCC OPN AMBP GRP DEFA1 | 7 | 0.85 | 59 | 20 | | MIF ENO2 TFPI MMP2 Ca19-9 CAIX GRP | 7 | 0.8974359 | 40 | 84.6153846 |
| Cyfra MIF SLPI SCC CEACAM5 OPN Ca72-4 CA242 DEFA1 | 9 | 0.85 | 62 | 20 | | MIF ENO2 TFPI MMP2 CA242 CAIX GRP | 7 | 0.8974359 | 40 | 84.6153846 |
| SLPI TIMP1 SCC CEACAM5 OPN Ca19-9 DEFA1 | 7 | 0.85 | 67 | 20 | | MIF ENO2 CEACAM5 MMP2 Ca19-9 CAIX GRP | 7 | 0.8974359 | 40 | 84.6153846 |
| MIF SLPI SCC CEACAM5 OPN Ca72-4 CA242 GRP DEFA1 | 9 | 0.85 | 67 | 20 | | MIF ENO2 CEACAM5 MMP2 CA242 CAIX GRP | 7 | 0.8974359 | 40 | 84.6153846 |
| SLPI TIMP1 SCC OPN Ca19-9 Ca72-4 DEFA1 | 7 | 0.85 | 62 | 40 | | SLPI SCC ENO2 MMP2 Ca19-9 CA242 CAIX | 7 | 0.8974359 | 20 | 87.1794872 |
| SLPI TIMP1 SCC TFPI OPN Ca72-4 DEFA1 | 7 | 0.85 | 82 | 20 | | SLPI ENO2 TFPI MMP2 Ca19-9 CA242 CAIX | 7 | 0.8974359 | 20 | 87.1794872 |
| MIF SLPI SCC TFPI CEACAM5 OPN CA242 DEFA1 | 8 | 0.85 | 62 | 60 | | SLPI ENO2 CEACAM5 MMP2 Ca19-9 CA242 CAIX | 7 | 0.8974359 | 20 | 87.1794872 |
| MIF SLPI SCC TFPI OPN Ca19-9 GRP DEFA1 | 8 | 0.85 | 51 | 60 | | SLPI ENO2 MMP2 AMBP Ca19-9 CA242 CAIX | 7 | 0.8974359 | 20 | 87.1794872 |
| MIF SLPI SCC OPN Ca19-9 CA242 GRP DEFA1 | 8 | 0.85 | 82 | 20 | | TIMP1 SCC ENO2 MMP2 Ca19-9 CAIX GRP | 7 | 0.8974359 | 40 | 84.6153846 |
| MIF SLPI SCC TFPI OPN Ca72-4 CA242 DEFA1 | 8 | 0.85 | 79 | 20 | | TIMP1 SCC ENO2 MMP2 CA242 CAIX GRP | 7 | 0.8974359 | 40 | 84.6153846 |
| Cyfra SCC OPN DEFA1 | 4 | 0.85 | 74 | 0 | | TIMP1 ENO2 TFPI MMP2 Ca19-9 CAIX GRP | 7 | 0.8974359 | 40 | 84.6153846 |
| SCC OPN Ca19-9 DEFA1 | 4 | 0.85 | 77 | 40 | | TIMP1 ENO2 TFPI MMP2 CA242 CAIX GRP | 7 | 0.8974359 | 40 | 84.6153846 |
| Cyfra MIF SLPI SCC CEACAM5 OPN | 6 | 0.85 | 77 | 40 | | TIMP1 ENO2 CEACAM5 MMP2 Ca19-9 CAIX GRP | 7 | 0.8974359 | 40 | 84.6153846 |
| Cyfra MIF SLPI SCC OPN Ca19-9 | 6 | 0.85 | 77 | 40 | | TIMP1 ENO2 CEACAM5 MMP2 CA242 CAIX GRP | 7 | 0.8974359 | 40 | 84.6153846 |
| Cyfra SLPI TIMP1 SCC OPN DEFA1 | 6 | 0.85 | 74 | 0 | | SCC ENO2 MMP2 AMBP Ca19-9 CAIX GRP | 7 | 0.8974359 | 40 | 84.6153846 |
| MIF SLPI SCC OPN CA242 GRP | 6 | 0.85 | 77 | 40 | | SCC ENO2 MMP2 AMBP CA242 CAIX GRP | 7 | 0.8974359 | 40 | 84.6153846 |
| MIF SCC CEACAM5 OPN Ca72-4 DEFA1 | 6 | 0.85 | 77 | 40 | | ENO2 TFPI MMP2 AMBP Ca19-9 CAIX GRP | 7 | 0.8974359 | 40 | 84.6153846 |
| SLPI SCC OPN Ca19-9 Ca72-4 DEFA1 | 6 | 0.85 | 77 | 40 | | ENO2 TFPI MMP2 AMBP CA242 CAIX GRP | 7 | 0.8974359 | 40 | 84.6153846 |
| Cyfra SLPI TIMP1 SCC CEACAM5 OPN Ca72-4 DEFA1 | 8 | 0.85 | 74 | 0 | | ENO2 CEACAM5 MMP2 AMBP Ca19-9 CAIX GRP | 7 | 0.8974359 | 40 | 84.6153846 |
| SLPI SCC CEACAM5 OPN Ca19-9 GRP DEFA1 | 7 | 0.85 | 69 | 40 | | ENO2 CEACAM5 MMP2 AMBP CA242 CAIX GRP | 7 | 0.8974359 | 40 | 84.6153846 |
| Cyfra SLPI TIMP1 SCC CEACAM5 OPN DEFA1 | 7 | 0.85 | 56 | 20 | | Cyfra MIF SLPI ENO2 MMP2 Ca19-9 CA242 CAIX | 8 | 0.8974359 | 20 | 87.1794872 |
| MIF SLPI TIMP1 SCC TFPI CEACAM5 OPN Ca72-4 DEFA1 | 9 | 0.85 | 59 | 20 | | Cyfra MIF TIMP1 ENO2 MMP2 Ca19-9 CA242 CAIX | 8 | 0.8974359 | 20 | 87.1794872 |
| SLPI SCC CEACAM5 OPN Ca72-4 | 5 | 0.85 | 64 | 20 | | Cyfra MIF TIMP1 ENO2 MMP2 Ca19-9 CAIX GRP | 8 | 0.8974359 | 40 | 84.6153846 |
| Cyfra MIF SLPI SCC OPN CA242 DEFA1 | 7 | 0.85 | 64 | 20 | | Cyfra MIF TIMP1 ENO2 MMP2 CA242 CAIX GRP | 8 | 0.8974359 | 40 | 84.6153846 |
| SLPI SCC OPN Ca19-9 CA242 GRP DEFA1 | 7 | 0.85 | 67 | 20 | | Cyfra MIF ENO2 MMP2 AMBP Ca19-9 CAIX GRP | 8 | 0.8974359 | 40 | 84.6153846 |
| SLPI OPN Ca72-4 GRP DEFA1 | 5 | 0.85 | 85 | 0 | | Cyfra MIF ENO2 MMP2 AMBP CA242 CAIX GRP | 8 | 0.8974359 | 40 | 84.6153846 |
| SLPI TIMP1 SCC OPN Ca19-9 GRP DEFA1 | 7 | 0.85 | 85 | 0 | | Cyfra SLPI TIMP1 ENO2 MMP2 Ca19-9 CA242 CAIX | 8 | 0.8974359 | 20 | 87.1794872 |
| Cyfra MIF SLPI TIMP1 SCC CEACAM5 OPN Ca72-4 DEFA1 | 9 | 0.85 | 85 | 0 | | Cyfra TIMP1 ENO2 MMP2 AMBP Ca19-9 CAIX GRP | 8 | 0.8974359 | 40 | 84.6153846 |
| MIF SLPI TIMP1 SCC TFPI OPN Ca72-4 GRP DEFA1 | 9 | 0.85 | 85 | 0 | | Cyfra TIMP1 ENO2 MMP2 AMBP CA242 CAIX GRP | 8 | 0.8974359 | 40 | 84.6153846 |
| MIF SCC TFPI OPN | 4 | 0.85 | 79 | 0 | | MIF SLPI SCC EN02 MMP2 Ca19-9 CAIX GRP | 8 | 0.8974359 | 40 | 84.6153846 |
| Cyfra SLPI TIMP1 SCC CEACAM5 OPN Ca19-9 DEFA1 | 8 | 0.85 | 79 | 0 | | MIF SLPI SCC ENO2 MMP2 CA242 CAIX GRP | 8 | 0.8974359 | 40 | 84.6153846 |
| MIF SLPI TIMP1 SCC CEACAM5 OPN CA242 DEFA1 | 8 | 0.85 | 79 | 0 | | MIF SLPI ENO2 TFPI MMP2 Ca19-9 CAIX GRP | 8 | 0.8974359 | 40 | 84.6153846 |
| SLPI SCC OPN AMBP GRP DEFA1 | 6 | 0.85 | 77 | 40 | | MIF SLPI EN02 TFPI MMP2 CA242 CAIX GRP | 8 | 0.8974359 | 40 | 84.6153846 |
| MIF SLPI TIMP1 SCC TFPI OPN Ca72-4 DEFA1 | 8 | 0.85 | 77 | 0 | | MIF SLPI ENO2 CEACAM5 MMP2 Ca19-9 CAIX GRP | 8 | 0.8974359 | 40 | 84.6153846 |
| SLPI SCC CEACAM5 OPN Ca19-9 Ca72-4 GRP DEFA1 | 8 | 0.85 | 54 | 60 | | MIF SLPI ENO2 CEACAM5 MMP2 CA242 CAIX GRP | 8 | 0.8974359 | 40 | 84.6153846 |
| MIF SCC OPN DEFA1 | 4 | 0.85 | 74 | 40 | | SLPI TIMP1 SCC EN02 MMP2 Ca19-9 CAIX GRP | 8 | 0.8974359 | 40 | 84.6153846 |
| SLPI SCC TFPI CEACAM5 OPN CA242 DEFA1 | 7 | 0.85 | 72 | 40 | | SLPI TIMP1 SCC EN02 MMP2 CA242 CAIX GRP | 8 | 0.8974359 | 40 | 84.6153846 |
| SLPI SCC CEACAM5 OPN Ca19-9 CA242 DEFA1 | 7 | 0.85 | 72 | 40 | | SLPI TIMP1 ENO2 TFPI MMP2 Ca19-9 CAIX GRP | 8 | 0.8974359 | 40 | 84.6153846 |
| SLPI TIMP1 SCC CEACAM5 OPN Ca72-4 CA242 DEFA1 | 8 | 0.85 | 69 | 60 | | SLPI TIMP1 ENO2 TFPI MMP2 CA242 CAIX GRP | 8 | 0.8974359 | 40 | 84.6153846 |
| MIF SLPI SCC OPN Ca19-9 | 5 | 0.85 | 67 | 60 | | SLPI T1MP1 ENO2 CEACAM5 MMP2 Ca19-9 CAIX GRP | 8 | 0.8974359 | 40 | 84.6153846 |
| MIF SLPI SCC OPN Ca19-9 Ca72-4 DEFA1 | 7 | 0.85 | 67 | 40 | | SLPI TIMP1 ENO2 CEACAMS MMP2 CA242 CAIX GRP | 8 | 0.8974359 | 40 | 84.6153846 |
| Cyfra MIF SLPI SCC OPN Ca19-9 GRP | 7 | 0.84 | 51 | 0 | | SLPI SCC EN02 MMP2 AMBP Ca19-9 CAIX GRP | 8 | 0.8974359 | 40 | 84.6153846 |
| Cyfra SCC CEACAM5 OPN DEFA1 | 5 | 0.84 | 54 | 20 | | SLPI SCC ENO2 MMP2 AMBP CA242 CAIX GRP | 8 | 0.8974359 | 40 | 84.6153846 |
| SLPI SCC TFPI OPN CA242 | 5 | 0.84 | 59 | 0 | | SLPI ENO2 TFPI MMP2 AMBP Ca19-9 CAIX GRP | 8 | 0.8974359 | 40 | 84.6153846 |
| Cyfra MIF SLPI SCC CEACAM5 OPN CA242 | 7 | 0.84 | 62 | 0 | | SLPI ENO2 TFPI MMP2 AMBP CA242 CAIX GRP | 8 | 0.8974359 | 40 | 84.6153846 |
| MIF SLPI TIMP1 SCC CEACAM5 OPN Ca72-4 | 7 | 0.84 | 54 | 20 | | SLPI ENO2 CEACAM5 MMP2 AMBP Ca19-9 CAIX GRP | 8 | 0.8974359 | 40 | 84.6153846 |
| Cyfra MIF SLPI TIMP1 SCC CEACAM5 OPN Ca19-9 DEFA1 | 9 | 0.84 | 62 | 0 | | SLPI ENO2 CEACAM5 MMP2 AMBP CA242 CAIX GRP | 8 | 0.8974359 | 40 | 84.6153846 |
| SCC CEACAM5 OPN | 3 | 0.84 | 38 | 80 | | Cyfra MIF SLPI TIMP1 ENO2 MMP2 Ca19-9 CA242 CAIX | 9 | 0.8974359 | 20 | 87.1794872 |
| SLPI TIMP1 SCC OPN Ca72-4 | 5 | 0.84 | 51 | 40 | | Cyfra MIF SLPI TIMP1 ENO2 MMP2 Ca19-9 CAIX GRP | 9 | 0.8974359 | 40 | 84.6153846 |
| SLPI TIMP1 SCC OPN AMBP Ca19-9 DEFA1 | 7 | 0.84 | 64 | 20 | | Cyfra MIF SLPI TIMP1 ENO2 MMP2 CA242 CAIX GRP | 9 | 0.8974359 | 40 | 84.6153846 |
| SLPI SCC TFPI OPN CA242 GRP DEFA1 | 7 | 0.84 | 62 | 20 | | Cyfra MIF SLPI ENO2 MMP2 AMBP Ca19-9 CAIX GRP | 9 | 0.8974359 | 40 | 84.6153846 |
| MIF SCC TFPI OPN DEFA1 | S | 0.84 | 67 | 20 | | Cyfra MIF SLPI EN02 MMP2 AMBP CA242 CAIX GRP | 9 | 0.8974359 | 40 | 84.6153846 |
| Cyfra SLPI SCC OPN Ca19-9 Ca72-4 DEFA1 | 7 | 0.84 | 56 | 40 | | Cyfra MIF TIMP1 ENO2 MMP2 Ca19-9 CA242 CAIX GRP | 9 | 0.8974359 | 40 | 84.6153846 |
| MIF SLPI TIMP1 SCC TFPI OPN DEFA1 | 7 | 0.84 | 69 | 20 | | Cyfra MIF EN02 MMP2 AMBP Ca19-9 CA242 CAIX GRP | 9 | 0.8974359 | 40 | 84.6153846 |
| MIF SLPI SCC OPN AMBP CA242 DEFA1 | 7 | 0.84 | 69 | 20 | | Cyfra SLPI TIMP1 ENO2 MMP2 AMBP Ca19-9 CAIX GRP | 9 | 0.8974359 | 40 | 84.6153846 |
| SLPI SCC OPN AMBP Ca19-9 DEFA1 | 6 | 0.84 | 51 | 60 | | Cyfra SLPI TIMP1 ENO2 MMP2 AMBP CA242 CAIX GRP | 9 | 0.8974359 | 40 | 84.6153846 |
| SLPI SCC TFPI OPN Ca72-4 GRP DEFA1 | 7 | 0.84 | 62 | 40 | | Cyfra TIMP1 ENO2 MMP2 AMBP Ca19-9 CA242 CAIX GRP | 9 | 0.8974359 | 40 | 84.6153846 |
| SLPI SCC OPN Ca72-4 CA242 GRP DEFA1 | 7 | 0.84 | 62 | 40 | | MIF TIMP1 SCC ENO2 MMP2 Ca19-9 CA242 CAIX GRP | 9 | 0.8974359 | 40 | 84.6153846 |
| MIF SLPI SCC TFPI CEACAM5 OPN Ca72-4 DEFA1 | 8 | 0.84 | 54 | 60 | | MIF TIMP1 ENO2 TFPI MMP2 Ca19-9 CA242 CAIX GRP | 9 | 0.8974359 | 40 | 84.6153846 |
| Cyfra SLPI SCC OPN GRP DEFA1 | 6 | 0.84 | 77 | 20 | | MIF TIMP1 ENO2 CEACAM5 MMP2 Ca19-9 CA242 CAIX GRP | 9 | 0.8974359 | 40 | 84.6153846 |
| MIF SCC CEACAM5 OPN GRP DEFA1 | 6 | 0.84 | 77 | 40 | | MIF SCC ENO2 MMP2 AMBP Ca19-9 CA242 CAIX GRP | 9 | 0.8974359 | 40 | 84.6153846 |
| MIF SLPI TIMP1 SCC CEACAM5 OPN GRP DEFA1 | 8 | 0.84 | 77 | 40 | | MIF ENO2 TFPI MMP2 AMBP Ca19-9 CA242 CAIX GRP | 9 | 0.8974359 | 40 | 84.6153846 |
| MIF SLPI SCC OPN Ca72-4 CA242 GRP DEFA1 | 8 | 0.84 | 77 | 20 | | MIF ENO2 CEACAM5 MMP2 AMBP Ca19-9 CA242 CAIX GRP | 9 | 0.8974359 | 40 | 84.6153846 |
| MIF SLPI TIMP1 SCC OPN DEFA1 | 6 | 0.84 | 74 | 40 | | nMP1 SCC EN02 MMP2 AMBP Ca19-9 CA242 CAIX GRP | 9 | 0.8974359 | 40 | 84.6153846 |
| SLPI SCC OPN Ca72-4 CAIX DEFA1 | 6 | 0.84 | 72 | 0 | | TIMP1 ENO2 TFPI MMP2 AMBP Ca19-9 CA242 CAIX GRP | 9 | 0.8974359 | 40 | 84.6153846 |
| MIF SLPI TIMP1 SCC OPN Ca19-9 DEFA1 | 7 | 0.84 | 69 | 40 | | TIMP1 ENO2 CEACAM5 MMP2 AMBP Ca19-9 CA242 CAIX GRP | 9 | 0.8974359 | 40 | 84.6153846 |
| Cyfra SLPI SCC CEACAM5 OPN Ca19-9 DEFA1 | 7 | 0.84 | 67 | 40 | | Cyfra MIF SLPI TIMP1 ENO2 MMP2 Ca19-9 CA242 CAIX GRP | 10 | 0.8974359 | 40 | 84.6153846 |
| SLPI SCC CEACAM5 OPN Ca19-9 Ca72-4 DEFA1 | 7 | 0.84 | 67 | 40 | | Cyfra MIF SLPI ENO2 MMP2 AMBP Ca19-9 CA242 CAIX GRP | 10 | 0.8974359 | 40 | 84.6153846 |
| SLPI SCC TFPI OPN Ca72-4 CA242 GRP DEFA1 | 8 | 0.84 | 49 | 0 | | Cyfra SLPI TIMP1 ENO2 MMP2 AMBP Ca19-9 CA242 CAIX GRP | 10 | 0.8974359 | 40 | 84.6153846 |
| SLPI TIMP1 SCC TFPI OPN | 5 | 0.84 | 54 | 20 | | MIF SLPI TMP1 SCC ENO2 MMP2 Ca19-9 CA242 CAIX GRP | 10 | 0.8974359 | 40 | 84.6153846 |
| SLPI CEACAM5 OPN Ca72-4 DEFA1 | 5 | 0.84 | 51 | 20 | | MIF SLPI TIMP1 ENO2 TFPI MMP2 Ca19-9 CA242 CAIX GRP | 10 | 0.8974359 | 40 | 84.6153846 |
| SLPI OPN Ca19-9 GRP DEFA1 | 5 | 0.84 | 59 | 0 | | MIF SLPI TIMP1 EN02 CEACAM5 MMP2 Ca19-9 CA242 CAIX GRP | 10 | 0.8974359 | 40 | 84.6153846 |
| MIF SLPI TIMP1 SCC CEACAM5 OPN CA242 | 7 | 0.84 | 59 | 0 | | MIF SLPI SCC ENO2 MMP2 AMBP Ca19-9 CA242 CAIX GRP | 10 | 0.8974359 | 40 | 84.6153846 |
| MIF SLPI TIMP1 SCC CEACAM5 OPN GRP | 7 | 0.84 | 59 | 0 | | MIF SLPI ENO2 TFPI MMP2 AMBP Ca19-9 CA242 CAIX GRP | 10 | 0.8974359 | 40 | 84.6153846 |
| MIF SLPI SCC TFPI OPN CA242 GRP | 7 | 0.84 | 59 | 0 | | MIF SLPI EN02 CEACAM5 MMP2 AMBP Ca19-9 CA242 CAIX GRP | 10 | 0.8974359 | 40 | 84.6153846 |
| SLPI SCC DEFA1 | 3 | 0.84 | 56 | 20 | | SLPI TIMP1 SCC ENO2 MMP2 AMBP Ca19-9 CA242 CAIX GRP | 10 | 0.8974359 | 40 | 84.6153846 |
| SLPI SCC OPN AMBP CA242 | 5 | 0.84 | 62 | 20 | | SLPI TIMP1 ENO2 TFPI MMP2 AMBP Ca19-9 CA242 CAIX GRP | 10 | 0.8974359 | 40 | 84.6153846 |
| SCC CEACAM5 OPN CA242 DEFA1 | 5 | 0.84 | 59 | 20 | | SLPI TIMP1 ENO2 CEACAM5 MMP2 AMBP Ca19-9 CA242 CAIX GRP | 10 | 0.8974359 | 40 | 84.6153846 |
| Cyfra SLPI SCC OPN Ca19-9 CA242 DEFA1 | 7 | 0.84 | 62 | 20 | | ENO2 MMP2 Ca19-9 CAIX | 4 | 0.8974359 | 0 | 89.7435897 |
| Cyfra MIF SLPI SCC CEACAM5 OPN Ca19-9 DEFA1 | 8 | 0.84 | 46 | 60 | | ENO2 MMP2 CA242 CAIX | 4 | 0.8974359 | 0 | 89.7435897 |
| MIF SLPI SCC CEACAM5 OPN Ca19-9 Ca72-4 DEFA1 | 8 | 0.84 | 49 | 40 | | MIF ENO2 MMP2 Ca19-9 CAIX | 5 | 0.8974359 | 0 | 89.7435897 |
| Cyfra MIF SLPI SCC CEACAM5 OPN Ca72-4 GRP DEFA1 | 9 | 0.84 | 59 | 20 | | MIF ENO2 MMP2 CA242 CAIX | 5 | 0.8974359 | 0 | 89.7435897 |
| MIF SLPI TIMP1 SCC CEACAM5 OPN Ca19-9 GRP DEFA1 | 9 | 0.84 | 69 | 0 | | SLPI ENO2 MMP2 Ca19-9 CAIX | S | 0.8974359 | 0 | 89.7435897 |
| MIF SLPI SCC TFPI CEACAM5 OPN DEFA1 | 7 | 0.84 | 38 | 40 | | SLPI ENO2 MMP2 CA242 CAIX | 5 | 0.8974359 | 0 | 89.7435897 |
| MIF SLPI SCC CEACAM5 OPN Ca19-9 DEFA1 | 7 | 0.84 | 38 | 40 | | TIMP1 ENO2 MMP2 Ca19-9 CAIX | 5 | 0.8974359 | 0 | 89.7435897 |
| SLPI SCC OPN AMBP Ca72-4 CA242 DEFA1 | 7 | 0.84 | 59 | 40 | | TIMP1 ENO2 MMP2 CA242 CAIX | 5 | 0.8974359 | 0 | 89.7435897 |
| SLPI SCC OPN AMBP Ca72-4 GRP DEFA1 | 7 | 0.84 | 59 | 40 | | MIF SLPI ENO2 MMP2 Ca19-9 CAIX | 6 | 0.8974359 | 0 | 89.7435897 |
| MIF SCC CEACAM5 OPN CA242 DEFA1 | 6 | 0.84 | 79 | 20 | | MIF SLPI ENO2 MMP2 CA242 CAIX | 6 | 0.8974359 | 0 | 89.7435897 |
| MIF SCC OPN Ca19-9 GRP DEFA1 | 6 | 0.84 | 79 | 20 | | MIF TIMP1 ENO2 MMP2 Ca19-9 CAIX | 6 | 0.8974359 | 0 | 89.7435897 |
| Cyfra MIF SLPI SCC TFPI OPN CA242 DEFA1 | 8 | 0.84 | 79 | 0 | | MIF TIMP1 ENO2 MMP2 CA242 CAIX | 6 | 0.8974359 | 0 | 89.7435897 |
| MIF SLPI TIMP1 SCC OPN Ca19-9 GRP DEFA1 | 8 | 0.84 | 79 | 0 | | SIPI TIMP1 ENO2 MMP2 Ca19-9 CAIX | 6 | 0.8974359 | 0 | 89.7435897 |
| MIF SLPI TIMP1 SCC OPN AMBP Ca72-4 DEFA1 | 8 | 0.84 | 74 | 0 | | SLPI TIMP1 ENO2 MMP2 CA242 CAIX | 6 | 0.8974359 | 0 | 89.7435897 |
| MIF SLPI TIMP1 SCC OPN Ca72-4 GRP DEFA1 | 8 | 0.84 | 74 | 0 | | MIF SLPI TIMP1 ENO2 MMP2 Ca19-9 CAIX | 7 | 0.8974359 | 0 | 89.7435897 |
| MIF SLPI SCC CEACAM5 OPN Ca19-9 CA242 GRP DEFA1 | 9 | 0.84 | 74 | 20 | | MIF SLPI TIMP1 ENO2 MMP2 CA242 CAIX | 7 | 0.8974359 | 0 | 89.7435897 |
| MIF SCC CEACAM5 OPN | 4 | 0.84 | 74 | 40 | | MIF ENO2 MMP2 CAIX GRP | S | 0.8923077 | 20 | 87.1794872 |
| SLPI OPN CA242 DEFA1 | 4 | 0.84 | 74 | 40 | | TIMP1 ENO2 MMP2 CAIX GRP | 5 | 0.8923077 | 20 | 87.1794877 |
| SLPI OPN GRP DEFA1 | 4 | 0.84 | 74 | 40 | | ENO2 MMP2 AMBP CAIX GRP | 5 | 0.8923077 | 20 | 87.1794872 |
| SLPI SCC TFPI CEACAM5 OPN Ca72-4 DEFA1 | 7 | 0.84 | 69 | 40 | | Cyfra MIF ENO2 MMP2 Ca19-9 CAIX | 6 | 0.8923077 | 20 | 87.1794872 |
| SLPI SCC OPN AMBP Ca19-9 GRP DEFA1 | 7 | 0.84 | 69 | 40 | | Cyfra MIF ENO2 MMP2 CA242 CAIX | 6 | 0.8923077 | 20 | 87.1794872 |
| MIF SLPI SCC OPN AMBP Ca72-4 GRP DEFA1 | 8 | 0.84 | 67 | 60 | | Cyfra TIMP1 ENO2 MMP2 Ca19-9 CAIX | 6 | 0.8923077 | 20 | 87.1794872 |
| SLPI TIMP1 SCC OPN AMBP | 5 | 0.83 | 56 | 20 | | Cyfra TIMP1 ENO2 MMP2 CA242 CAIX | 6 | 0.8923077 | 20 | 87.1794872 |
| SLPI TIMP1 SCC OPN CA242 | 5 | 0.83 | 56 | 20 | | MIF SLPI ENO2 MMP2 CAIX GRP | 6 | 0.8923077 | 20 | 87.1794872 |
| SLPI SCC OPN CA242 GRP | 5 | 0.83 | 56 | 20 | | SLPI TIMP1 ENO2 MMP2 CAIX GRP | 6 | 0.8923077 | 20 | 87.1794872 |
| SLPI TIMP1 SCC TFPI OPN GRP DEFA1 | 7 | 0.83 | 56 | 20 | | SLPI ENO2 MMP2 AMBP CAIX GRP | 6 | 0.8923077 | 20 | 87.1794872 |
| MIF SLPI TIMP1 SCC CEACAMS OPN AMBP Ca72-4 DEFA1 | 9 | 0.83 | 59 | 20 | | Cyfra MIF SLPI ENO2 MMP2 Ca19-9 CAIX | 7 | 0.8923077 | 20 | 87.1794872 |
| MIF SLPI TIMP1 SCC CEACAM5 OPN Ca72-4 CA242 DEFA1 | 9 | 0.83 | 59 | 20 | | Cyfra MIF SLPI ENO2 MMP2 CA242 CAIX | 7 | 0.8923077 | 20 | 87.1794872 |
| MIF SLPI SCC TFPI OPN Ca72-4 CA242 GRP DEFA1 | 9 | 0.83 | 59 | 20 | | Cyfra SLPI TIMP1 ENO2 MMP2 Ca19-9 CAIX | 7 | 0.8923077 | 20 | 87.1794872 |
| MIF SCC OPN | 3 | 0.83 | 38 | 20 | | Cyfra SLPI TIMP1 ENO2 MMP2 CA242 CAIX | 7 | 0.8923077 | 20 | 87.1794872 |
| SCC OPN Ca19.9 GRP DEFA1 | 5 | 0.83 | 67 | 20 | | MIF SCC ENO2 MMP2 Ca19-9 CA242 CAIX | 7 | 0.8923077 | 20 | 87.1794872 |
| MIF SLPI TIMP1 SCC OPN AMBP DEFA1 | 7 | 0.83 | 64 | 20 | | MIF ENO2 TFPI MMP2 Ca19-9 CA242 CAIX | 7 | 0.8923077 | 20 | 87.1794872 |
| MIF SLPI TIMP1 SCC OPN GRP DEFA1 | 7 | 0.83 | 64 | 20 | | MIF ENO2 CEACAM5 MMP2 Ca19-9 CA242 CAIX | 7 | 0.8923077 | 20 | 87.1794872 |
| SLPI SCC TFPI OPN Ca72-4 CA242 DEFA1 | 7 | 0.83 | 56 | 40 | | MIF ENO2 MMP2 AMBP Ca19-9 CA242 CAIX | 7 | 0.8923077 | 20 | 87.1794872 |
| SLPI SCC OPN AMBP CA242 GRP DEFA1 | 7 | 0.83 | 67 | 20 | | TIMP1 SCC ENO2 MMP2 Ca19-9 CA242 CAIX | 7 | 0.8923077 | 20 | 87.1794872 |
| MIF SLPI SCC TFPI CEACAM5 OPN GRP DEFA1 | 8 | 0.83 | 51 | 40 | | TIMP1 ENO2 TFPI MMP2 Ca19-9 CA242 CAIX | 7 | 0.8923077 | 20 | 87.1794872 |
| SLPI SCC OPN Ca72-4 GRP | 5 | 0.83 | 56 | 40 | | TIMP1 ENO2 CEACAMS MMP2 Ca19-9 CA242 CAIX | 7 | 0.8923077 | 20 | 87.1794872 |
| SLPI SCC TFPI CEACAM5 OPN DEFA1 | 6 | 0.83 | 56 | 40 | | TIMP1 ENO2 MMP2 AMBP Ca19-9 CA242 CAIX | 7 | 0.8923077 | 20 | 87.1794872 |
| MIF SLPI SCC CEACAM5 OPN AMBP CA242 DEFA1 | 8 | 0.83 | 56 | 0 | | MIF SLPI SCC ENO2 MMP2 Ca19-9 CA242 CAIX | 8 | 0.8923077 | 20 | 87.1794872 |
| SLPI TIMP1 SCC OPN AMBP Ca72-4 DEFA1 | 7 | 0.83 | 79 | 20 | | MIF SLPI ENO2 TFPI MMP2 Ca19-9 CA242 CAIX | 8 | 0.8923077 | 20 | 87.1794872 |
| SLPI TIMP1 SCC OPN Ca72-4 CA242 DEFA1 | 7 | 0.83 | 62 | 40 | | MIF SLPI ENO2 CEACAMS MMP2 Ca19-9 CA242 CAIX | 8 | 0.8923077 | 20 | 87.1794872 |
| Cyfra MIF SLPI SCC CEACAM5 OPN GRP DEFA1 | 8 | 0.83 | 79 | 20 | | MIF SLPI ENO2 MMP2 AMBP Ca19-9 CA242 CAIX | 8 | 0.8923077 | 20 | 87.1794872 |
| SLPI TIMP1 SCC OPN Ca19.9 GRP | 6 | 0.83 | 77 | 0 | | MIF TIMP1 ENO2 MMP2 AMBP Ca19-9 CAIX GRP | 8 | 0.8923077 | 0 | 87.1794872 |
| SLPI TIMP1 SCC CEACAMS OPN Ca19-9 GRP DEFA1 | 8 | 0.83 | 77 | 0 | | MIF TIMP1 ENO2 MMP2 AMBP CA242 CAIX GRP | 8 | 0.8923077 | 0 | 87.1794872 |
| SLPI TIMP1 SCC OPN Ca19-9 Ca72-4 GRP DEFA1 | 8 | 0.83 | 77 | 0 | | SLDI TIMP1 SCC ENO2 MMP2 Ca19-9 CA242 CAIX | 8 | 0.8923077 | 20 | 87.1794872 |
| MIF SLPI TIMP1 SCC TFPI CEACAM5 OPN Ca72-4 GRP DEFA1 | 10 | 0.83 | 77 | 0 | | SLPI TIMD1 ENO2 TFPI MMP2 Ca19-9 CA242 CAIX | 8 | 0.8923077 | 20 | 87.1794872 |
| SLPI SCC OPN Ca19-9 CA242 DEFA1 | 6 | 0.83 | 74 | 40 | | SLPI TIMP1 ENO2 CEACAM5 MMP2 Ca19-9 CA242 CAIX | 8 | 0.8923077 | 20 | 87.1794872 |
| Cyfra MIF SLPI SCC OPN Ca19-9 Ca72-4 DEFA1 | 8 | 0.83 | 74 | 40 | | SLPI TIMP1 ENO2 MMP2 AMBP Ca19-9 CA242 CAIX | 8 | 0.8923077 | 20 | 87.1794872 |
| SLPI TIMP1 SCC CEACAM5 OPN Ca19-9 Ca72-4 DEFA1 | 8 | 0.83 | 74 | 40 | | Cyfra MIF TIMP1 ENO2 MMP2 AMBP Ca19-9 CAIX GRP | 9 | 0.8923077 | 20 | 84.6153846 |
| Cyfra MIF SLPI SCC OPN Ca72-4 GRP DEFA1 | 8 | 0.83 | 69 | 0 | | Cyfra MIF TIMP1 ENO2 MMP2 AMBP CA242 CAIX GRP | 9 | 0.8923077 | 20 | 84.6153MC |
| Cyfra MIF SLPI SCC CEACAM5 OPN Ca19-9 GRP DEFA1 | 9 | 0.83 | 69 | 20 | | MIF SLPI TIMP1 ENO2 MMP2 AM8P Ca19-9 CAIX GRP | 9 | 0.8923077 | 0 | 87.1794872 |
| MIF SLPI SCC TFPI CEACAM5 OPN Ca72-4 CA242 DEFA1 | 9 | 0.83 | 69 | 40 | | MIF SLPI TIMP1 ENO2 MMP2 AMBP CA242 CAIX GRP | 9 | 0.8923077 | 0 | 87.1794872 |
| MIF SLPI SCC TFPI CEACAM5 OPN CA242 GRP DEFA1 | 9 | 0.83 | 69 | 20 | | Cyfra MIF SLPI TIMP1 ENO2 MMP2 AMBP Ca19-9 CAIX GRP | 10 | 0.8923077 | 20 | 84.6153846 |
| SLPI SCC CEACAM5 OPN GRP | 5 | 0.83 | 64 | 40 | | Cyfra MIF SLPI TIMP1 ENO2 MMP2 AMBP CA242 CAIX GRP | 10 | 0.8923077 | 20 | 84.6153846 |
| MIF SLPI SCC CEACAM5 OPN Ca19-9 GRP | 7 | 0.83 | 64 | 60 | | Cyfra MIF TIMP1 ENO2 MMP2 AMBP Ca19-9 CA242 CAIX GRP | 10 | 0.8923077 | 20 | 84.6153846 |
| SLPI SCC CEACAM5 OPN AMBP CA242 DEFA1 | 7 | 0.83 | 67 | 40 | | MIF TIMP1 SCC ENO2 MMP2 AMBP Ca19-9 CA242 CAIX GRP | 10 | 0.8923077 | 20 | 84.6153846 |
| Cyfra SLPI SCC OPN Ca19-9 | 5 | 0.83 | 54 | 20 | | MIF TIMP1 ENO2 TFPI MMP2 AMBP Ca19-9 CA242 CAIX GRP | 10 | 0.8923077 | 20 | 84.6153846 |
| MIF SLPI SCC OPN CAIX | 5 | 0.83 | 44 | 0 | | MIF TIMP1 EN02 CEACAM5 MMP2 AMBP Ca19-9 CA242 CAIX GRP | 10 | 0.8923077 | 20 | 84.6153846 |
| SLPI TIMP1 SCC OPN GRP | 5 | 0.83 | 54 | 20 | | Cyfra MIF SLPI TIMP1 ENO2 MMP2 AMBP Ca19-9 CA242 CAIX GRP | 11 | 0.8923077 | 20 | 84.6153846 |
| SLPI CEACAM5 OPN CA242 DEFA1 | 5 | 0.83 | 64 | 0 | | MIF SLPI TIMP1 SCC ENO2 MMP2 AMBP Ca19-9 CA242 CAIX GRP | 11 | 0.8923077 | 20 | 84.6153846 |
| Cyfra MIF SLPI SCC CEACAM5 OPN Ca72-4 | 7 | 0.83 | 54 | 20 | | MIF SLPI TIMP1 ENO2 TFPI MMP2 AMBP Ca19-9 CA242 CAIX GRP | 11 | 0.8923077 | 20 | 84.6153846 |
| SLPI TIMP1 SCC TFPI OPN Ca19-90 DEFA1 | 7 | 0.83 | 54 | 20 | | MIF SLPI TIMP1 ENO2 CEACAM5 MMP2 AMBP Ca19-9 CA242 CAIX GRP | 6 | 0.8923077 | 20 | 84.6153846 |
| Cyfra SLPI SCC CEACAM5 OPN Ca72-4 CA242 DEFA1 | 8 | 0.83 | 44 | 0 | | Cyfra SCC ENO2 MMP2 CAIX GRP | 6 | 0.8923077 | 40 | 82.0512821 |
| Cyfra SLPI TIMP1 SCC CEACAM5 OPN Ca19-9 Ca72-4 DEFA1 | 9 | 0.83 | 56 | 20 | | Cyfra ENO2 TFPI MMP2 CAIX GRP | 6 | 0.8923077 | 40 | 82.0512821 |
| MIF SLPI TIMP1 SCC TFPI CEACAM5 OPN CA242 DEFA1 | 9 | 0.83 | 64 | 0 | | Cyfra ENO2 CEACAM5 MMP2 CAIX GRP | 6 | 0.8923077 | 40 | 82.0512821 |
| MIF SLPI TIMP1 SCC TFPI OPN AMBP Ca72-4 DEFA1 | 9 | 0.83 | 62 | 0 | | Cyfra SLPI SCC ENO2 MMP2 CAIX GRP | 7 | 0.8923077 | 40 | 82.0512821 |
| Cyfra MIF SLPI SCC TFPI OPN DEFA1 | 7 | 0.83 | 69 | 20 | | Cyfra SLPI ENO2 TFPI MMP2 CAIX GRP | 7 | 0.8923077 | 40 | 82.0512821 |
| MIF SLPI SCC TFPI OPN Ca19-9 DEFA1 | 7 | 0.83 | 33 | 40 | | Cyfra SLPI ENO2 CEACAM5 MMP2 CAIX GRP | 7 | 0.8923077 | 40 | 82.0512821 |
| MIF SLPI TIMP1 SCC TFPI CEACAM5 OPN DEFA1 | 8 | 0.83 | 49 | 60 | | Cyfra SCC ENO2 MMP2 Ca19-9 CAIX GRP | 7 | 0.8923077 | 40 | 82.0512821 |
| MIF SLPI SCC CEACAM5 OPN Ca19-9 CA242 DEFA1 | 8 | 0.83 | 59 | 20 | | Cyfra SCC ENO2 MMP2 CA242 CAIX GRP | 7 | 0.8923077 | 40 | 82.0512821 |
| SLPI SCC CEACAM5 OPN Ca19-9 CA242 GRP DEFA1 | 8 | 0.83 | 56 | 0 | | Cyfra ENO2 TFPI MMP2 Ca19-9 CAIX GRP | 7 | 0.8923077 | 40 | 82.0512821 |
| Cyfra SLPI TIMP1 SCC OPN Ca72-4 DEFA1 | 7 | 0.83 | 79 | 20 | | Cyfra ENO2 TFPI MMP2 CA242 CAIX GRP | 7 | 0.8923077 | 40 | 82.0512821 |
| SLPI SCC TFPI OPN Ca19-9 DEFA1 | 6 | 0.83 | 44 | 60 | | Cyfra ENO2 CEACAM5 MMP2 Ca19-9 CAIX GRP | 7 | 0.8923077 | 40 | 82.0512821 |
| MIF SLPI TIMP1 SCC TFPI OPN Ca19-9 DEFA1 | 8 | 0.83 | 54 | 60 | | Cyfra ENO2 CEACAM5 MMP2 CA242 CAIX GRP | 7 | 0.8923077 | 40 | 82.0512821 |
| MIF SLPI SCC CEACAM5 OPN AMBP Ca72-4 DEFA1 | 8 | 0.83 | 44 | 60 | | SCC ENO2 TFPI MMP2 Ca19-9 CAIX GRP | 7 | 0.8923077 | 40 | 82.0512821 |
| MIF SLPI SCC CEACAM5 OPN AMBP CA242 GRP DEFA1 | 9 | 0.83 | 74 | 20 | | SCC ENO2 TFPI MMP2 CA242 CAIX GRP | 7 | 0.8923077 | 40 | 82.0512821 |
| Cyfra MIF SLPI SCC OPN CA242 | 6 | 0.83 | 74 | 40 | | SCC ENO2 CEACAM5 MMP2 Ca19-9 CAIX GRP | 7 | 0.8923077 | 40 | 82.0512821 |
| Cyfra SLPI SCC OPN AMBP DEFA1 | 6 | 0.83 | 74 | 20 | | SCC ENO2 CEACAM5 MMP2 CA242 CAIX GRP | 7 | 0.8923077 | 40 | 82.0512821 |
| MIF SLPI SCC OPN AMBP CA242 | 6 | 0.83 | 74 | 40 | | ENO2 TFPI CEACAM5 MMP2 Ca19-9 CAIX GRP | 7 | 0.8923077 | 40 | 82.0512821 |
| MIF SCC TFPI OPN Ca72-4 DEFA1 | 6 | 0.83 | 74 | 20 | | ENO2 TFPI CEACAM5 MMP2 CA242 CAIX GRP | 7 | 0.8923077 | 40 | 82.0512821 |
| SLPI CEACAM5 OPN Ca72-4 GRP DEFA1 | 6 | 0.83 | 74 | 40 | | Cyfra SLPI SCC ENO2 MMP2 Ca19-9 CAIX GRP | 8 | 0.8923077 | 40 | 82.0512821 |
| Cyfra MIF SLPI TIMP1 SCC CEACAM5 OPN DEFA1 | 8 | 0.83 | 74 | 40 | | Cyfra SLPI SCC ENO2 MMP2 CA242 CAIX GRP | 8 | 0.8923077 | 40 | 82.0512821 |
| MIF SLPI TIMP1 SCC TFPI OPN GRP DEFA1 | 8 | 0.83 | 74 | 40 | | Cyfra SLPI ENO2 TFPI MMP2 Ca19-9 CAIX GRP | 8 | 0.8923077 | 40 | 82.0512821 |
| Cyfra MIF SLPI TIMP1 SCC CEACAM5 OPN Ca19-9 Ca72-4 DEFA1 | 10 | 0.83 | 74 | 40 | | Cyfra SLPI ENO2 TFPI MMP2 CA242 CAIX GRP | 8 | 0.8923077 | 40 | 82.0512821 |
| SLPI SCC CEACAM5 OPN CA242 GRP | 6 | 0.83 | 44 | 60 | | Cyfra SLPI ENO2 CEACAM5 MMP2 Ca19-9 CAIX GRP | 8 | 0.8923077 | 40 | 82.0512821 |
| MIF SLPI SCC OPN Ca19-9 CA242 DEFA1 | 7 | 0.83 | 69 | 20 | | Cyfra SLPI ENO2 CEACAM5 MMP2 CA242 CAIX GRP | 8 | 0.8923077 | 40 | 82.0512821 |
| Cyfra MIF SLPI SCC OPN Ca72-4 | 6 | 0.83 | 64 | 60 | | Cyfra SCC ENO2 MMP2 Ca19-9 CA242 CAIX GRP | 8 | 0.8923077 | 40 | 82.0512821 |
| SLPI SCC CEACAM5 OPN AMBP Ca72-4 DEFA1 | 7 | 0.83 | 64 | 40 | | Cyfra ENO2 TFPI MMP2 Ca19-9 CA242 CAIX GRP | 8 | 0.8923077 | 40 | 82.0512821 |
| MIF SLPI SCC TFPI CEACAM5 OPN Ca72-4 GRP DEFA1 | 9 | 0.83 | 64 | 40 | | Cyfra ENO2 CEACAM5 MMP2 Ca19-9 CA242 CAIX GRP | 8 | 0.8923077 | 40 | 82.0512821 |
| Cyfra SLPI SCC OPN CA242 | 5 | 0.82 | 54 | 20 | | SLPI SCC ENO2 TFPI MMP2 Ca19-9 CAIX GRP | 8 | 0.8923077 | 40 | 82.0512821 |
| Cyfra SLPI OPN Ca19-9 DEFA1 | 5 | 0.82 | 41 | 0 | | SLPI SCC ENO2 TFPI MMP2 CA242 CAIX GRP | 8 | 0.8923077 | 40 | 82.0512821 |
| SCC TFPI OPN GRP DEFA1 | 5 | 0.82 | 51 | 20 | | SLPI SCC ENO2 CEACAM5 MMP2 Ca19-9 CAIX GRP | 8 | 0.8923077 | 40 | 82.0512821 |
| MIF SLPI TIMP1 SCC OPN Ca19-9 GRP | 7 | 0.82 | 62 | 0 | | SLPI SCC ENO2 CEACAM5 MMP2 CA242 CAIX GRP | 8 | 0.8923077 | 40 | 82.0512821 |
| MIF SCC OPN Ca72-4 DEFA1 | 5 | 0.82 | 56 | 20 | | SLPI ENO2 TFPI CEACAM5 MMP2 Ca19-9 CAIX GRP | 8 | 0.8923077 | 40 | 82.0512821 |
| MIF SCC OPN GRP DEFA1 | 5 | 0.82 | 56 | 20 | | SLPI ENO2 TFPI CEACAM5 MMP2 CA242 CAIX GRP | 8 | 0.8923077 | 40 | 82.0512821 |
| SCC OPN Ca72-4 GRP DEFA1 | S | 0.82 | 51 | 40 | | SCC ENO2 TFPI MMP2 Ca19-9 CA242 CAIX GRP | 8 | 0.8923077 | 40 | 82.0512821 |
| Cyfra MIF SLPI SCC OPN AMBP DEFA1 | 7 | 0.82 | 59 | 20 | | SCC ENO2 CEACAM5 MMP2 Ca19-9 CA242 CAIX GRP | 8 | 0.8923077 | 40 | 82.0512821 |
| Cyfra SLPI SCC OPN AMBP CA242 DEFA1 | 7 | 0.82 | 64 | 0 | | ENO2 TFPI CEACAM5 MMP2 Ca19-9 CA242 CAIX GRP | 8 | 0.8923077 | 40 | 82.0512821 |
| SLPI TIMP1 SCC OPN AMBP GRP DEFA1 | 7 | 0.82 | 62 | 20 | | Cyfra SLPI SCC ENO2 MMP2 Ca19-9 CA242 CAIX GRP | 9 | 0.8923077 | 40 | 82.0512821 |
| TIMP1 SCC CEACAM5 OPN DEFA1 | 5 | 0.82 | 82 | 0 | | Cyfra SLPI ENO2 TFPI MMP2 Ca19-9 CA242 CAIX GRP | 9 | 0.8923077 | 40 | 82.0512821 |
| SLPI SCC CEACAM5 OPN AMBP DEFA1 | 6 | 0.82 | 51 | 40 | | Cyfra SLPI ENO2 CEACAM5 MMP2 Ca19-9 CA242 CAIX GRP | 9 | 0.8923077 | 40 | 82.0512821 |
| Cyfra SLPI SCC OPN Ca72-4 CA242 DEFA1 | 7 | 0.82 | 56 | 40 | | SLPI SCC ENO2 TFPI MMP2 Ca19-9 CA242 CAIX GRP | 9 | 0.8923077 | 40 | 82.0512821 |
| MIF SLPI SCC OPN AMBP Ca19-9 DEFA1 | 7 | 0.82 | 36 | 40 | | SLPI SCC ENO2 CEACAM5 MMP2 Ca19-9 CA242 CAIX GRP | 9 | 0.8923077 | 40 | 82.0512821 |
| SLPI TIMP1 SCC TFPI OPN CA242 DEFA1 | 7 | 0.82 | 82 | 0 | | SLPI ENO2 TFPI CEACAM5 MMP2 Ca19-9 CA242 CAIX GRP | 9 | 0.8923077 | 40 | 82.0512821 |
| SLPI TIMP1 SCC OPN AMBP CA242 DEFA1 | 7 | 0.82 | 82 | 0 | | Cyfra MIF TIMP1 ENO2 MMP2 CAIX GRP | 7 | 0.8871795 | 40 | 84.6153846 |
| MIF SLPI SCC OPN AMBP Ca19-9 GRP DEFA1 | 8 | 0.82 | 51 | 0 | | Cyfra MIF ENO2 MMP2 AMBP CAIX GRP | 7 | 0.8871795 | 40 | 84.6153846 |
| Cyfra MIF SLPI TIMP1 SCC TFPI OPN Ca72-4 DEFA1 | 9 | 0.82 | 82 | 0 | | Cyfra TIMP1 ENO2 MMP2 AMBP CAIX GRP | 7 | 0.8871795 | 40 | 84.6153846 |
| MIF SLPI TIMP1 SCC CEACAM5 OPN Ca72-4 GRP DEFA1 | 9 | 0.82 | 82 | 0 | | Cyfra MIF SLPI TIMP1 ENO2 MMP2 CAIX GRP | 8 | 0.8871795 | 40 | 84.6153846 |
| Cyfra SLPI SCC OPN Ca72-4 GRP DEFA1 | 7 | 0.82 | 62 | 40 | | Cyfra MIF SLPI ENO2 MMP2 AMBP CAIX GRP | 8 | 0.8871795 | 40 | 84.6153846 |
| Cyfra SLPI TIMP1 SCC OPN Ca19-9 Ca72-4 DEFA1 | 8 | 0.82 | 74 | 0 | | Cyfra SLPI TIMP1 ENO2 MMP2 AMBP CAIX GRP | 8 | 0.8871795 | 40 | 84.6153846 |
| MIF SLPI TIMP1 SCC OPN Ca19-9 Ca72-4 DEFA1 | 8 | 0.82 | 51 | 20 | | MIF TIMP1 SCC ENO2 MMP2 Ca19-9 CAIX GRP | 8 | 0.8871795 | 40 | 84.6153846 |
| MIF SLPI SCC OPN AMBP CA242 GRP DEFA1 | 8 | 0.8Z | 77 | 0 | | MIF TIMP1 SCC ENO2 MMP2 CA242 CAIX GRP | 8 | 0.8871795 | 40 | 84.6153846 |
| SLPI TIMP1 SCC OPN AMBP Ca19-9 GRP DEFA1 | 8 | 0.82 | 77 | 0 | | MIF TIMD1 ENO2 TFPI MMP2 Ca19-9 CAIX GRP | 8 | 0.8871795 | 40 | 84.6153846 |
| Cyfra MIF SLPI TIMP1 SCC TFPI CEACAM5 OPN Ca72-4 DEFA1 | 10 | 0.82 | 77 | 0 | | MIF TIMP1 ENO2 TFPI MMP2 CA242 CAIX GRP | 8 | 0.8871795 | 40 | 84.6153846 |
| MIF SLPI TIMP1 SCC CEACAM5 OPN Ca19-9 Ca72-4 GRP DEFA1 | 10 | 0.82 | 77 | 0 | | MIF TIMP1 ENO2 CEACAM5 MMP2 Ca19-9 CAIX GRP | 8 | 0.8871795 | 40 | 84.6153846 |
| Cyfra MIF SLPI SCC OPN Ca72-4 CA242 DEFA1 | 8 | 0.82 | 74 | 20 | | MIF TIMP1 ENO2 CEACAM5 MMP2 CA242 CAIX GRP | 8 | 0.8871795 | 40 | 84.6153846 |
| MIF SLPI SCC OPN AMBP Ca72-4 CA242 DEFA1 | 8 | 0.82 | 74 | 20 | | MIF SCC EN02 MMP2 AMBP Ca19-9 CAIX GRP | 8 | 0.8871795 | 40 | 84.6153846 |
| Cyfra SLPI OPN DEFA1 | 4 | 0.82 | 72 | 40 | | MIF SCC ENO2 MMP2 AMBP CA242 CAIX GRP | 8 | 0.8871795 | 40 | 84.6153846 |
| SLPI TIMP1 SCC CEACAM5 OPN CA242 | 6 | 0.82 | 72 | 40 | | MIF ENO2 TFPI MMP2 AMBP Ca19-9 CAIX GRP | 8 | 0.8871795 | 40 | 84.6153846 |
| MIF SLPI SCC TFPI CEACAM5 OPN CA242 | 7 | 0.82 | 69 | 20 | | MIF ENO2 TFPI MMP2 AMBP CA242 CAIX GRP | 8 | 0.8871795 | 40 | 84.6153846 |
| Cyfra MIF SLPI SCC CEACAM5 OPN CA242 GRP DEFA1 | 9 | 0.82 | 72 | 20 | | MIF ENO2 CEACAM5 MMP2 AMBP Ca19-9 CAIX GRP | 8 | 0.8871795 | 40 | 84.6153846 |
| SLPI SCC Ca72-4 DEFA1 | 4 | 0.82 | 67 | 0 | | MIF ENO2 CEACAM5 MMP2 AMBP CA242 CAIX GRP | 8 | 0.8871795 | 40 | 84.6153846 |
| TIMP1 SCC OPN DEFA1 | 4 | 0.82 | 67 | 0 | | TIMP1 SCC EN02 MMP2 AMBP Ca19-9 CAIX GRP | 8 | 0.8871795 | 40 | 84.6153846 |
| SLPI SCC CEACAM5 OPN Ca19-9 | 5 | 0.82 | 64 | 20 | | TIMP1 SCC ENO2 MMP2 AMBP CA242 CAIX GRP | 8 | 0.8871795 | 40 | 84.6153846 |
| Cyfra SLPI SCC CEACAM5 OPN GRP DEFA1 | 7 | 0.82 | 67 | 20 | | TIMP1 ENO2 TFPI MMP2 AMBP Ca19-9 CAIX GRP | 8 | 0.8871795 | 40 | 84.6153846 |
| SLPI TIMP1 SCC TFPI OPN Ca72-4 GRP DEFA1 | 8 | 0.82 | 67 | 0 | | TIMP1 ENO2 TFPI MMP2 AMBP CA242 CAIX GRP | 8 | 0.8871795 | 40 | 84.6153846 |
| MIF SLPI TIMP1 SCC TFPI OPN GRP | 7 | 0.82 | 49 | 0 | | TIMP1 ENO2 CEACAM5 MMP2 AMBP Ca19-9 CAIX GRP | 8 | 0.8871795 | 40 | 84.6153846 |
| SLPI SCC OPN AMBP Ca72-4 | S | 0.82 | 49 | 20 | | TIMP1 ENO2 CEACAM5 MMP2 AMBP CA242 CAIX GRP | 8 | 0.8871795 | 40 | 84.6153846 |
| MIF SCC OPN AMBP DEFA1 | 5 | 0.82 | 54 | 20 | | MIF SLPI TIMP1 SCC EN02 MMP2 Ca19-9 CAIX GRP | 9 | 0.8871795 | 40 | 84.6153846 |
| Cyfra SLPI SCC TFPI OPN CA242 DEFA1 | 7 | 0.82 | 59 | 20 | | MIF SLPI TIMP1 SCC ENO2 MMP2 CA242 CAIX GRP | 9 | 0.8871795 | 40 | 84.6153846 |
| MIF SLPI TIMP1 SCC CEACAM5 OPN AMBP CA242 DEFA1 | 9 | 0.82 | 69 | 0 | | MIF SLPI TIMP1 ENO2 TFPI MMP2 Ca19-9 CAIX GRP | 9 | 0.8871795 | 40 | 84.6153846 |
| MIF SLPI SCC CEACAM5 OPN Ca19-9 | 6 | 0.82 | 49 | 40 | | MIF SLPI TIMP1 ENO2 TFPI MMP2 CA242 CAIX GRP | 9 | 0.8871795 | 40 | 84.6153846 |
| SCC CEACAM5 OPN Ca72-4 GRP DEFA1 | 6 | 0.82 | 28 | 80 | | MIF SLPI TIMP1 ENO2 CEACAM5 MMP2 Ca19-9 CAIX GRP | 9 | 0.8871795 | 40 | 84.6153846 |
| Cyfra MIF SIPI TIMP1 SCC OPN DEFA1 | 7 | 0.82 | 64 | 20 | | MIF SLPI TIMP1 ENO2 CEACAM5 MMP2 CA242 CAIX GRP | 9 | 0.8871795 | 40 | 84.6153846 |
| SLPI TIMP1 SCC OPH CA242 GRP DEFA1 | 7 | 0.82 | 67 | 20 | | MIF SLPI SCC ENO2 MMP2 AMBP Ca19-9 CAIX GRP | 9 | 0.8871795 | 40 | 84.6153846 |
| MIF SLPI TIMPI SCC CEACAM5 OPN Ca19-9 DEFA1 | 8 | 0.82 | 46 | 0 | | MIF SLPI SCC ENO2 MMP2 AMBP CA242 CAIX GRP | 9 | 0.8871795 | 40 | 84.6153846 |
| MIF SLPI SCC CEACAM5 OPN AMBP GRP DEFA1 | 8 | 0.82 | 51 | 40 | | MIF SLPI ENO2 TFPI MMP2 AMBP Ca19-9 CAIX GRP | 9 | 0.8871795 | 40 | 84.6153846 |
| MIF SLDI SCC CEACAM5 OPN AMBP DEFA1 | 7 | 0.82 | 28 | 40 | | MIF SLPI ENO2 TFPI MMP2 AMBP CA242 CAIX GRP | 9 | 0.8871795 | 40 | 84.6153846 |
| MIF SLPI TIMP1 SCC TFPI CEACAM5 OPN CA242 GRP DEFA1 | 10 | 0.82 | 79 | 0 | | MIF SLPI ENO2 CEACAM5 MMP2 AMBP Ca19-9 CAIX GRP | 9 | 0.8871795 | 40 | 84.6153846 |
| Cyfra SLPI SCC CEACAM5 OPN Ca19-9 Ca72-4 DEFA1 | 8 | 0.82 | 41 | 60 | | MIF SLPI ENO2 CEACAMS MMP2 AMBP CA242 CAIX GRP | 9 | 0.8871795 | 40 | 84.6153846 |
| SLPI SCC TFPI CEACAM5 OPN Ca72-4 CA242 DEFA1 | 8 | 0.82 | 54 | 20 | | SLPI TIMP1 SCC ENO2 MMP2 AMBP Ca19-9 CAIX GRP | 9 | 0.8871795 | 40 | 84.6153846 |
| SIPI SCC CEACAM5 OPN AMBP Ca72-4 CA242 DEFA1 | 8 | 0.82 | 51 | 20 | | SLPI TIMP1 SCC ENO2 MMP2 AMBP CA242 CAIX GRP | 9 | 0.8871795 | 40 | 84.6153846 |
| Cyfra MIF SLPI SCC CEACAMS OPN Ca19-9 Ca72-4 GRP DEFA1 | 10 | 0.82 | 46 | 60 | | SLPI TIMP1 ENO2 TFPI MMP2 AMBP Ca19-9 CAIX GRP | 9 | 0.8871795 | 40 | 84.6153846 |
| SLPI SCC | 2 | 0.82 | 74 | 0 | | SLPI TIMP1 ENO2 TFPI MMP2 AMBP CA242 CAIX GRP | 9 | 0.8871795 | 40 | 84.6153846 |
| Cyfra SLPI CEACAM5 OPN Ca72-4 DEFA1 | 6 | 0.82 | 74 | 0 | | SLPI TIMP1 ENO2 CEACAM5 MMP2 AMBP Ca19-9 CAIX GRP | 9 | 0.8871795 | 40 | 84.6153846 |
| SLPI CEACAM5 OPN Ca72-4 CA242 DEFA1 | 6 | 0.82 | 74 | 0 | | SLPI TIMP1 ENO2 CEACAM5 MMP2 AMBP CA242 CAIX GRP | 9 | 0.8871795 | 40 | 84.6153846 |
| Cyfra MIF SLIPI TIMP1 SCC OPN Ca19-9 DEFA1 | 8 | 0.82 | 74 | 0 | | MIF TIMP1 ENO2 MMP2 CAIX GRP | 6 | 0.8871795 | 20 | 87.1794872 |
| Cyfra SLPI TIMP1 SCC CEACAM5 OPN CA242 DEFA1 | 8 | 0.82 | 74 | 0 | | MIF ENO2 MMP2 AMBP CAIX GRP | 6 | 0.8871795 | 20 | 87.1794872 |
| SLPI TIMP1 SCC TFPI CEACAM5 OPN CA242 DEFA1 | 8 | 0.82 | 74 | 0 | | TIMP1 ENO2 MMP2 AMBP CAIX GRP | 6 | 0.8871795 | 20 | 87.1794872 |
| SLPI TIMP1 SCC CEACAM5 OPN CA242 GRP DEFA1 | 8 | 0.82 | 74 | 0 | | Cyfra MIF TIMP1 ENO2 MMP2 Ca19-9 CAIX | 7 | 0.8871795 | 20 | 87.1794872 |
| MIF SLPI TIMP1 SCC TFPI CEACAM5 OPN Ca72-4 CA242 DEFA1 | 10 | 0.82 | 74 | 0 | | Cyfra MIF TIMP1 ENO2 MMP2 CA242 CAIX | 7 | 0.8871795 | 20 | 87.1794872 |
| SLPI SCC TFPI CEACAM5 OPN | 5 | 0.82 | 72 | 40 | | MIF SLPI TIMP1 ENO2 MMP2 CAIX GRP | 7 | 0.8871795 | 20 | 87.1794872 |
| MIF SLPI TIMP1 SCC OPN Ca19-9 | 6 | 0.82 | 36 | 60 | | MIF SLPI ENO2 MMP2 AMBP CAIX GRP | 7 | 0.8871795 | 20 | 87.1794872 |
| MIF SLPI SCC OPN AMBP Ca72-4 | 6 | 0.82 | 72 | 40 | | SLPI TIMP1 ENO2 MMP2 AMBP CAIX GRP | 7 | 0.8871795 | 20 | 87.1794872 |
| SLPI TIMP1 SCC TFPI CEACAM5 OPN Ca72-4 DEFA1 | 8 | 0.82 | 72 | 40 | | Cyfra MIF SLPI TIMP1 ENO2 MMP2 Ca19-9 CAIX | 8 | 0.8871795 | 20 | 87.1794872 |
| SLPI SCC TFPI OPN Ca19-9 CA242 GRP DEFA1 | 8 | 0.82 | 54 | 60 | | Cyfra MIF SLPI TIMP1 ENO2 MMP2 CA242 CAIX | 8 | 0.8871795 | 20 | 87.1794872 |
| MIF SLPI TIMP1 SCC OPN AMBP Ca19-9 Ca72-4 DEFA1 | 9 | 0.82 | 69 | 40 | | Cyfra MIF SCC ENO2 MMP2 Ca19-9 CAIX GRP | 8 | 0.8871795 | 40 | 82.0512821 |
| MIF SLPI TIMP1 SCC OPN CA242 | 6 | 0.82 | 44 | 60 | | Cyfra MIF SCC ENO2 MMP2 CA242 CAIX GRP | 8 | 0.8871795 | 40 | 82.0512821 |
| MIF SLPI TIMP1 SCC OPN CA242 DEFA1 | 7 | 0.82 | 64 | 40 | | Cyfra MIF ENO2 TFPI MMP2 Ca19-9 CAIX GRP | 8 | 0.8871795 | 40 | 82.0512821 |
| SLPI SCC TFPI OPN AMBP CA242 DEFA1 | 7 | 0.82 | 67 | 40 | | Cyfra MIF ENO2 TFPI MMP2 CA242 CAIX GRP | 8 | 0.8871795 | 40 | 82.0512821 |
| SLPI SCC OPN AMBP Ca19-9 CA242 DEFA1 | 7 | 0.82 | 67 | 40 | | Cyfra MIF ENO2 CEACAM5 MMP2 Ca19-9 CAIX GRP | 8 | 0.8871795 | 40 | 82.0512821 |
| MIF SLPI SCC CEACAM5 OPN Ca72-4 CA242 GRP | 8 | 0.82 | 44 | 60 | | Cyfra MIF ENO2 CEACAM5 MMP2 CA242 CAIX GRP | 8 | 0.8871795 | 40 | 82.0512821 |
| Cyfra MIF SLPI SCC TFPI CEACAM5 OPN Ca72-4 DEFA1 | 9 | 0.82 | 67 | 40 | | Cyfra TIMP1 SCC ENO2 MMP2 Ca19-9 CAIX GRP | 8 | 0.8871795 | 40 | 82.0512821 |
| Cyfra MIF SLPI SCC TFPI CEACAM5 OPN CA242 DEFA1 | 9 | 0.82 | 67 | 20 | | Cyfra TIMP1 SCC ENO2 MMP2 CA242 CAIX GRP | 8 | 0.8871795 | 40 | 82.0512821 |
| Cyfra MIF SLPI SCC CEACAM5 OPN Ca19-9 CA242 DEFA1 | 9 | 0.82 | 67 | 20 | | Cyfra TIMP1 ENO2 TFPI MMP2 Ca19-9 CAIX GRP | 8 | 0.8871795 | 40 | 82.0512821 |
| MIF SLPI TIMP1 SCC CEACAM5 OPN Ca19-9 Ca72-4 DEFA1 | 9 | 0.82 | 64 | 40 | | Cyfra TIMP1 ENO2 TFPI MMP2 CA242 CAIX GRP | 8 | 0.8871795 | 40 | 82.0512821 |
| Cyfra SLPI SCC CEACAM5 OPN | S | 0.82 | 62 | 40 | | Cyfra TIMP1 ENO2 CEACAM5 MMP2 Ca19-9 CAIX GRP | 8 | 0.8871795 | 40 | 82.0512821 |
| MIF SLPI SCC CEACAM5 OPN Ca72-4 CA242 | 7 | 0.82 | 62 | 40 | | Cyfra TIMP1 ENO2 CEACAM5 MMP2 CA242 CAIX GRP | 8 | 0.8871795 | 40 | 82.0512821 |
| Cyfra MIF SLPI SCC CEACAM5 OPN Ca19-9 Ca72-4 DEFA1 | 9 | 0.82 | 62 | 60 | | Cyfra SCC ENO2 MMP2 AMBP Ca19-9 CAIX GRP | 8 | 0.8871795 | 40 | 82.0512821 |
| Cyfra SCC OPN Ca19-9 DEFA1 | 5 | 0.81 | 49 | 20 | | Cyfra SCC ENO2 MMP2 AMBP CA242 CAIX GRP | 8 | 0.8871795 | 40 | 82.0512821 |
| MIF SCC CEACAM5 OPN GRP | 5 | 0.81 | 46 | 0 | | Cyfra ENO2 TFPI MMP2 AMBP Ca19-9 CAIX GRP | 8 | 0.8871795 | 40 | 82.0512821 |
| Cyfra MIF SLPI TIMP1 SCC CEACAM5 OPN | 7 | 0.81 | S6 | 0 | | Cyfra ENO2 TFPI MMP2 AMBP CA242 CAIX GRP | 8 | 0.8871795 | 40 | 82.0512821 |
| Cyfra SLPI SCC OPN Ca19-9 Ca72-4 GRP DEFA1 | 8 | 0.81 | 46 | 0 | | Cyfra ENO2 CEACAM5 MMP2 AMBP Ca19-9 CAIX GRP | 8 | 0.8871795 | 40 | 82.0512821 |
| Cyfra MIF SLPI SCC TFPI OPN Ca72-4 CA242 DEFA1 | 9 | 0.81 | 54 | 0 | | Cyfra ENO2 CEACAMS MMP2 AMBP CA242 CAIX GRP | 8 | 0.8871795 | 40 | 82.0512821 |
| MIF SLPI TIMP1 SCC TFPI CEACAM5 OPN Ca72-4 CA242 GRP DEFA1 | 11 | 0.81 | 56 | 0 | | MIF TIMP1 SCC ENO2 MMP2 Ca19-9 CA242 CAIX | 8 | 0.8871795 | 20 | 87.1794872 |
| SLPI SCC TFPI OPN Ca72-4 | 5 | 0.81 | 54 | 20 | | MIF TIMP1 ENO2 TFPI MMP2 Ca19-9 CA242 CAIX | 8 | 0.8871795 | 20 | 87.1794872 |
| MIF SLPI TIMP1 SCC TFPI OPN Ca72-4 CA242 DEFA1 | 9 | 0.81 | 59 | 0 | | MIF TIMP1 ENO2 CEACAM5 MMP2 Ca19-9 CA242 CAIX | 8 | 0.8871795 | 20 | 87.1794872 |
| MIF SLPI SCC TFPI CEACAM5 OPN | 6 | 0.81 | 49 | 40 | | MIF TIMP1 ENO2 MMP2 AMBP Ca19-9 CA242 CAIX | 8 | 0.8871795 | 20 | 87.1794872 |
| MIF SCC CEACAM5 OPN Ca72-4 GRP DEFA1 | 7 | 0.81 | 62 | 20 | | Cyfra MIF SLPI SCC ENO2 MMP2 Ca19-9 CAIX GRP | 9 | 0.8871795 | 40 | 82.0512821 |
| SLPI TIMP1 SCC OPN Ca19-9 CA242 DEFA1 | 7 | 0.81 | 64 | 20 | | Cyfra MIF SLPI SCC ENO2 MMP2 CA242 CAIX GRP | 9 | 0.8871795 | 40 | 82.0512821 |
| Cyfra SLPI SCC CEACAM5 OPN Ca19-9 CA242 DEFA1 | 8 | 0.81 | 49 | 0 | | Cyfra MIF SLPI ENO2 TFPI MMP2 Ca19-9 CAIX GRP | 9 | 0.8871795 | 40 | 82.0512821 |
| Cyfra MIF SLPI SCC OPN Ca19-9 Ca72-4 GRP DEFA1 | 9 | 0.81 | 62 | 20 | | Cyfra MIF SLPI EN02 TFPI MMP2 CA242 CAIX GRP | 9 | 0.8871795 | 40 | 82.0512821 |
| SLPI TIMP1 SCC CEACAM5 OPN Ca19-9 Ca72-4 GRP DEFA1 | 9 | 0.81 | 62 | 20 | | Cyfra MIF SLPI ENO2 CEACAM5 MMP2 Ca19-9 CAIX GRP | 9 | 0.8871795 | 40 | 82.0512821 |
| MIF SLPI SCC TFPI CEACAM5 OPN Ca72-4 CA242 GRP DEFA1 | 10 | 0.81 | 49 | 0 | | Cyfra MIF SLPI ENO2 CEACAM5 MMP2 CA242 CAIX GRP | 9 | 0.8871795 | 40 | 82.0512821 |
| SCC TFPI OPN Ca72-4 DEFA1 | 5 | 0.81 | 46 | 40 | | Cyfra MIF SCC ENO2 MMP2 Ca19-9 CA242 CAIX GRP | 9 | 0.8871795 | 40 | 82.0512821 |
| Cyfra SLPI SCC CEACAM5 OPN Ca19-9 GRP DEFA1 | 8 | 0.81 | 54 | 40 | | Cyfra MIF ENO2 TFPI MMP2 Ca19-9 CA242 CAIX GRP | 9 | 0.8871795 | 40 | 82.0512821 |
| MIF SLPI TIMP1 SCC OPN Ca19-9 Ca72-4 GRP DEFA1 | 9 | 0.81 | 72 | 20 | | Cyfra MIF ENO2 CEACAM5 MMP2 Ca19-9 CA242 CAIX GRP | 9 | 0.8871795 | 40 | 82.0512821 |
| SLPI SCC TFPI CEACAM5 OPN Ca72-4 CA242 GRP DEFA1 | 9 | 0.81 | 41 | 40 | | Cyfra SLPI TIMP1 SCC ENO2 MMP2 Ca19-9 CAIX GRP | 9 | 0.8871795 | 40 | 82.0512821 |
| MIF SCC TFPI CEACAM5 OPN DEFA1 | 6 | 0.81 | 41 | 60 | | Cyfra SLPI TIMP1 SCC EN02 MMP2 CA242 CAIX GRP | 9 | 0.8871795 | 40 | 82.0512821 |
| SLPI TIMP1 SCC OPN AMBP Ca19-9 | 6 | 0.81 | 77 | 20 | | Cyfra SLPI TIMP1 ENO2 TFPI MMP2 Ca19-9 CAIX GRP | 9 | 0.8871795 | 40 | 82.0512821 |
| SLPI SCC OPN CAIX GRP DEFA1 | 6 | 0.81 | 77 | 20 | | Cyfra SLPI TIMP1 ENO2 TFPI MMP2 CA242 CAIX GRP | 9 | 0.8871795 | 40 | 82.0512821 |
| Cyfra SLPI SCC OPN AMBP Ca72-4 DEFA1 | 7 | 0.81 | 62 | 40 | | Cyfra SLPI TIMP1 ENO2 CEACAM5 MMP2 Ca19-9 CAIX GRP | 9 | 0.8871795 | 40 | 82.0512821 |
| Cyfra MIF SLPI TIMP1 SCC TFP1 OPN DEFA1 | 8 | 0.81 | 77 | 0 | | Cyfra SLPI TIMP1 ENO2 CEACAM5 MMP2 CA242 CAIX GRP | 9 | 0.8871795 | 40 | 82.0512821 |
| Cyfra MIF SLPI SCC OPN Ca19-9 CA242 DEFA1 | 8 | 0.81 | 77 | 20 | | Cyfra SLPI SCC ENO2 MMP2 AMBP Ca19-9 CAIX GRP | 9 | 0.8871795 | 40 | 82.0512821 |
| Cyfra SLPI SCC CEACAM5 OPN Ca72-4 GRP DEFA1 | 8 | 0.81 | 46 | 20 | | Cyfra SLPI SCC ENO2 MMP2 AMBP CA242 CAIX GRP | 9 | 0.8871795 | 40 | 82.0512821 |
| SLPI TIMP1 SCC CEACAM5 OPN Ca19-9 CA242 DEFA1 | 8 | 0.81 | 77 | 0 | | Cyfra SLPI ENO2 TFPI MMP2 AMBP Ca19-9 CAIX GRP | 9 | 0.8871795 | 40 | 82.0512821 |
| MIF SLPI SCC OPN AMBP Ca19-9 CA242 GRP DEFA1 | 9 | 0.81 | 74 | 0 | | Cyfra SLPI ENO2 TFPI MMP2 AMBP CA242 CAIX GRP | 9 | 0.8871795 | 40 | 82.0512821 |
| SLPI TIMP1 SCC OPN Ca72-4 CA242 GRP DEFA1 | 8 | 0.81 | 74 | 20 | | Cyfra SLPI ENO2 CEACAM5 MMP2 AMBP Ca19-9 CAIX GRP | 9 | 0.8871795 | 40 | 82.0512821 |
| SLPI CEACAM5 OPN DEFA1 | 4 | 0.81 | 33 | 60 | | Cyfra SLPI ENO2 CEACAM5 MMP2 AMBP CA242 CAIX GRP | 9 | 0.8871795 | 40 | 82.0512821 |
| Cyfra MIF SCC CEACAM5 OPN DEFA1 | 6 | 0.81 | 72 | 40 | | Cyfra TIMP1 SCC ENO2 MMP2 Ca19-9 CA242 CAIX GRP | 9 | 0.8871795 | 40 | 82.0512821 |
| Cyfra SLPI SCC TFPI OPN DEFA1 | 6 | 0.81 | 72 | 20 | | Cyfra TIMD1 ENO2 TFPI MMP2 Ca19-9 CA242 CAIX GRP | 9 | 0.8871795 | 40 | 82.0512821 |
| MIF SLPI SCC TFPI OPN Ca72-4 | 6 | 0.81 | 72 | 20 | | Cyfra TIMP1 ENO2 CEACAM5 MMP2 Ca19-9 CA242 CAIX GRP | 9 | 0.8871795 | 40 | 82.0512821 |
| MIF SCC TFPI OPN GRP DEFA1 | 6 | 0.81 | 72 | 40 | | Cyfra SCC ENO2 MMP2 AMBP Ca19-9 CA242 CAIX GRP | 9 | 0.8871795 | 40 | 82.0512821 |
| Cyfra MIF SLPI TIMP1 SCC OPN Ca72-4 DEFA1 | 8 | 0.81 | 72 | 0 | | Cyfra EN02 TFPI MMP2 AMBP Ca19-9 CA242 CAIX GRP | 9 | 0.8871795 | 40 | 82.0512821 |
| Cyfra MIF SLPI SCC TFPI OPN Ca72-4 DEFA1 | 8 | 0.81 | 72 | 0 | | Cyfra ENO2 CEACAM5 MMP2 AMBP Ca19-9 CA242 CAIX GRP | 9 | 0.8871795 | 40 | 82.0512821 |
| Cyfra MIF SLPI SCC OPN AMBP Ca72-4 DEFA1 | 8 | 0.81 | 72 | 0 | | MIF SLPI TIMP1 SCC ENO2 MMP2 Ca19-9 CA242 CAIX | 9 | 0.8871795 | 20 | 87.1794872 |
| MIF SLPI TIMP1 SCC CEACAM5 OPN Ca72-4 GRP | 8 | 0.81 | 72 | 40 | | MIF SLPI TIMP1 ENO2 TFPI MMP2 Ca19-9 CA242 CAIX | 9 | 0.8871795 | 20 | 87.1794872 |
| SLPI TIMP1 SCC OPN AMBP Ca72-4 GRP DEFA1 | 8 | 0.81 | 72 | 0 | | MIF SLPI TIMP1 ENO2 CEACAM5 MMP2 Ca19-9 CA242 CAIX | 9 | 0.8871795 | 20 | 87.1794872 |
| MIF SLPI TIMP1 SCC CEACAM5 OPN Ca19-9 CA242 DEFA1 | 9 | 0.81 | 72 | 0 | | MIF SLPI TIMP1 ENO2 MMP2 AMBP Ca19-9 CA242 CAIX | 9 | 0.8871795 | 20 | 87.1794872 |
| MIF SLPI SCC TFPI OPN Ca19-9 CA242 GRP DEFA1 | 9 | 0.81 | 69 | 20 | | MIF SCC ENO2 TFPI MMP2 Ca19-9 CA242 CAIX GRP | 9 | 0.8871795 | 40 | 82.0512821 |
| MIF SLPI SCC OPN Ca19-9 CA242 GRP | 7 | 0.81 | 67 | 20 | | MIF SCC ENO2 CEACAM5 MMP2 Ca19-9 CA242 CAIX GRP | 9 | 0.8871795 | 40 | 82.0512821 |
| SLPI SCC TFPI OPN Ca19-9 CA242 DEFAI | 7 | 0.81 | 64 | 40 | | MIF ENO2 TFPI CEACAM5 MMP2 Ca19-9 CA242 CAIX GRP | 9 | 0.8871795 | 40 | 82.0512821 |
| SLPI SCCTFPI OPN Ca19-9 Ca27-4 GRP DEFA1 | 8 | 0.81 | 46 | 60 | | TIMP1 SCC ENO2 TFPI MMP2 Ca19-9 CA242 CAIX GRP | 9 | 0.8871795 | 40 | 82.0512821 |
| MIF SLPI SCC CEACAM5 OPN AMBP Ca72-4 CA242 DEFA1 | 9 | 0.81 | 64 | 40 | | TIMP1 SCC ENO2 CEACAM5 MMP2 Ca19-9 CA242 CAIX GRP | 9 | 0.8871795 | 40 | 82.0512821 |
| MIF SLPI SCC OPN AMBP Ca19-9 Ca72-4 GRP DEFA1 | 9 | 0.81 | 64 | 40 | | TIMP1 ENO2 TFPI CEACAM5 MMP2 Ca19-9 CA242 CAIX GRP | 9 | 0.8871795 | 40 | 82.0512821 |
| SCC CEACAM5 OPN Ca19-9 DEFA1 | S | 0.81 | 62 | 40 | | SCC ENO2 TFPI MMP2 AMBP Ca19-9 CA242 CAIX GRP | 9 | 0.8871795 | 40 | 82.0512821 |
| MIF SLPI SCC TFPI OPN Ca19-9 GRP | 7 | 0.81 | 59 | 60 | | SCC ENO2 CEACAM5 MMP2 AMBP Ca19-9 CA242 CAIX GRP | 9 | 0.8871795 | 40 | 82.0512821 |
| SLPI SCC TFPI OPN Ca19-9 GRP DEFA1 | 7 | 0.81 | 59 | 40 | | ENO2 TFPI CEACAM5 MMP2 AMBP Ca19-9 CA242 CAIX GRP | 9 | 0.8871795 | 40 | 82.0512821 |
| MIF SLPI SCC CEACAM5 OPN AMBP Ca72-4 GRP DEFA1 | 9 | 0.81 | 62 | 40 | | Cyfra MIF SLPI SCC ENO2 MMP2 Ca19-9 CA242 CAIX GRP | 10 | 0.8871795 | 40 | 82.0512821 |
| SLPI OPN CA242 GRP DEFA1 | 5 | 0.81 | 54 | 0 | | Cyfra MIF SLPI ENO2 TFPI MMP2 Ca19-9 CA242 CAIX GRP | 10 | 0.8871795 | 40 | 82.0512821 |
| Cyfra MIF SLPI TIMP1 SCC TFPI CEACAM5 OPN Ca72-4 CA242 DEFA1 | 11 | 0.81 | 54 | 0 | | Cyfra MIF SLPI ENO2 CEACAM5 MMP2 Ca19-9 CA242 CAIX GRP | 10 | 0.8871795 | 40 | 82.0512821 |
| SLPI OPN CA242 | 3 | 0.81 | 44 | 0 | | Cyfra SLPI TIMP1 SCC ENO2 MMP2 Ca19-9 CA242 CAIX GRP | 10 | 0.8871795 | 40 | 82.0512821 |
| Cyfra MIF SCC CEACAM5 OPN Ca72-4 DEFA1 | 7 | 0.81 | 54 | 20 | | Cyfra SLPI TIMP1 ENO2 TFPI MMP2 Ca19-9 CA242 CAIX GRP | 10 | 0.8871795 | 40 | 82.0512821 |
| Cyfra SLPI SCC TFPI OPN Ca72-4 CA242 DEFA1 | 8 | 0.81 | 44 | 0 | | Cyfra SLPI TIMP1 ENO2 CEACAM5 MMP2 Ca19-9 CA242 CAIX GRP | 10 | 0.8871795 | 40 | 82.0512821 |
| Cyfra MIF SLPI TIMP1 SCC CEACAM5 OPN CA242 DEFA1 | 9 | 0.81 | 64 | 0 | | Cyfra SLPI SCC ENO2 MMP2 AMBP Ca19-9 CA242 CAIX GRP | 10 | 0.8871795 | 40 | 82.0512821 |
| MIF SLPI TIMP1 SCCTFPI OPN Ca19-9 GRP DEFA1 | 9 | 0.81 | 64 | 0 | | Cyfra SLPI ENO2 TFPI MMP2 AMBP Ca19-9 CA242 CAIX GRP | 10 | 0.8871795 | 40 | 82.0512821 |
| MIF SLPI SCC | 3 | 0.81 | 33 | 0 | | Cyfra SLPI EN02 CEACAM5 MMP2 AMBP Ca19-9 CA242 CAIX GRP | 10 | 0.8871795 | 40 | 82.0512821 |
| SCC TFPI OPN | 3 | 0.81 | 38 | 20 | | MIF SLPI SCC ENO2 TFPI MMP2 Ca19-9 CA242 CAIX GRP | 10 | 0.8871795 | 40 | 82.0512821 |
| Cyfra MIF SCC OPN DEFA1 | 5 | 0.81 | 56 | 20 | | MIF SLPI SCC ENO2 CEACAM5 MMP2 Ca19-9 CA242 CAIX GRP | 10 | 0.8871795 | 40 | 82.0512821 |
| Cyfra SLPI SCC OPN Ca72-4 | 5 | 0.81 | 49 | 40 | | MIF SLPI ENO2 TFPI CEACAM5 MMP2 Ca19-9 CA242 CAIX GRP | 10 | 0.8871795 | 40 | 82.0512821 |
| Cyfra SLPI TIMP1 SCC OPN CA242 DEFA1 | 7 | 0.81 | 62 | 20 | | SLPI TIMP1 SCC ENO2 TFPI MMP2 Ca19-9 CA242 CAIX GRP | 10 | 0.8871795 | 40 | 82.0512821 |
| MIF SLPI SCC TFPI OPN AMBP CA242 DEFA1 | 8 | 0.81 | 54 | 0 | | SLPI TIMP1 SCC ENO2 CEACAM5 MMP2 Ca19-9 CA242 CAIX GRP | 10 | 0.8871795 | 40 | 82.0512821 |
| SCC OPN AMBP Ca72-4 DEFA1 | 5 | 0.81 | 49 | 40 | | SLPI TIMP1 ENO2 TFPI CEACAM5 MMP2 Ca19-9 CA242 CAIX GRP | 10 | 0.8871795 | 40 | 82.0512821 |
| SLPI SCC TFPI OPN AMBP DEFA1 | 6 | 0.81 | 49 | 40 | | SLPI SCC ENO2 TFPI MMP2 AMBP Ca19-9 CA242 CAIX GRP | 10 | 0.8871795 | 40 | 82.0512821 |
| Cyfra MIF SLPI SCC TFPI CEACAM5 OPN DEFA1 | 8 | 0.81 | 51 | 40 | | SLPI SCC ENO2 CEACAM5 MMP2 AMBP Ca19-9 CA242 CAIX GRP | 10 | 0.8871795 | 40 | 82.0512821 |
| MIF SLPI TIMP1 SCC CEACAM5 OPN AMBP CA242 GRP DEFA1 | 10 | 0.81 | 79 | 0 | | SLPI ENO2 TFPI CEACAM5 MMP2 AMBP Ca19-9 CA242 CAIX GRP | 10 | 0.8971795 | 40 | 82.0512821 |
| MIF SLPI SCC CEACAM5 OPN Ca19-9 Ca72-4 CA242 GRP DEFA1 | 10 | 0.81 | 56 | 20 | | Cyfra MIF SCC ENO2 MMP2 CAIX GRP | 7 | 0.8820513 | 40 | 82.0512821 |
| Cyfra MIF SLPI SCC OPN AMBP Ca19-9 DEFA1 | 8 | 0.81 | 51 | 0 | | Cyfra MIF ENO2 TFPI MMP2 CAIX GRP | 7 | 0.8820513 | 40 | 82.0512821 |
| Cyfra MIF SLPI SCC OPN CA242 GRP DEFA1 | 8 | 0.81 | 51 | 0 | | Cyfra MIF ENO2 CEACAM5 MMP2 CAIX GRP | 7 | 0.8820513 | 40 | 82.0512821 |
| MIF SLPI TIMP1 SCC CEACAM5 OPN Ca19-9 GRP | 8 | 0.81 | 77 | 20 | | Cyfra TIMP1 SCC ENO2 MMP2 CAIX GRP | 7 | 0.8820513 | 40 | 82.0512821 |
| SLPI SCC CEACAM5 OPN Ca19-9 Ca72-4 CA242 GRP DEFA1 | 9 | 0.81 | 44 | 40 | | Cyfra TIMP1 ENO2 TFPI MMP2 CAIX GRP | 7 | 0.8820513 | 40 | 82.0512821 |
| Cyfra MIF SLPI SCC TFPI OPN | 6 | 0.81 | 74 | 0 | | Cyfra TIMP1 ENO2 CEACAM5 MMP2 CAIX GRP | 7 | 0.8820513 | 40 | 82.0512821 |
| SLPI TIMP1 SCC OPN AMBP CA242 | 6 | 0.81 | 74 | 0 | | Cyfra SCC ENO2 MMP2 AMBP CAIX GRP | 7 | 0.8820513 | 40 | 82.0512821 |
| Cyfra SLPI TIMP1 SCC OPN AMBP Ca19-9 DEFA1 | 8 | 0.81 | 74 | 0 | | Cyfra ENO2 TFPI MMP2 AMBP CAIX GRP | 7 | 0.8820513 | 40 | 82.0512821 |
| Cyfra SLPI TIMP1 SCC OPN Ca19-9 GRP DEFA1 | 8 | 0.81 | 74 | 0 | | Cyfra ENO2 CEACAM5 MMP2 AMBP CAIX GRP | 7 | 0.8820513 | 40 | 82.0512821 |
| Cyfra SLPI TIMP1 SCC OPN Ca72-4 CA242 DEFA1 | 8 | 0.81 | 74 | 20 | | MIF TIMP1 ENO2 MMP2 AMBP CAIX GRP | 7 | 0.8820513 | 0 | 87.1794872 |
| SLPI TIMP1 SCC CEACAM5 OPN AMBP CA242 DEFA1 | 8 | 0.81 | 74 | 0 | | Cyfra MIF SLPI SCC ENO2 MMP2 CAIX GRP | 8 | 0.8820513 | 40 | 82.0512821 |
| MIF SLPI TIMP1 SCC TFPI OPN Ca19-9 Ca72-4 GRP DEFA1 | 10 | 0.81 | 74 | 0 | | Cyfra MIF SLPI ENO2 TFPI MMP2 CAIX GRP | 8 | 0.8820513 | 40 | 82.0512821 |
| SLPI SCC OPN Ca19-9 CAIX DEFA1 | 6 | 0.81 | 44 | 60 | | Cyfra MIF SLPI ENO2 CEACAM5 MMP2 CAIX GRP | 8 | 0.8820513 | 40 | 82.0512821 |
| MIF SCC CEACAMS OPN CA242 GRP DEFA1 | 7 | 0.81 | 72 | 20 | | Cyfra SLPI TIMP1 SCC ENO2 MMP2 CAIX GRP | 8 | 0.8820513 | 40 | 82.0512821 |
| Cyfra MIF SLPI SCC TFPI OPN Ca19-9 DEFA1 | 8 | 0.81 | 38 | 60 | | Cyfra SLPI TIMP1 ENO2 TFPI MMP2 CAIX GRP | 8 | 0.8820513 | 40 | 82.0512821 |
| SLPI TIMP1 SCC OPN AMBP Ca19-9 Ca72-4 DEFA1 | 8 | 0.81 | 72 | 20 | | Cyfra SLPI TIMP1 ENO2 CEACAM5 MMP2 CAIX GRP | 8 | 0.8820513 | 40 | 82.0512821 |
| SCC OPN CA242 DEFA1 | 4 | 0.81 | 69 | 40 | | Cyfra SLPI SCC ENO2 MMP2 AMBP CAIX GRP | 8 | 0.8820513 | 40 | 82.0512821 |
| Cyfra MIF SLPI SCC OPN GRP | 6 | 0.81 | 69 | 40 | | Cyfra SLPI ENO2 TFPI MMP2 AMBP CAIX GRP | 8 | 0.8820513 | 40 | 82.0512821 |
| MIF SLPI TIMP1 SCC OPN AMBP | 6 | 0.81 | 69 | 40 | | Cyfra SLPI ENO2 CEACAM5 MMP2 AMBP CAIX GRP | 8 | 0.8820513 | 40 | 82.0512821 |
| MIF SLPI TIMP1 SCC OPN GRP | 6 | 0.81 | 69 | 40 | | MIF SLPI TIMP1 ENO2 MMP2 AMBP CAIX GRP | 8 | 0.8820513 | 0 | 87.1794872 |
| MIF SLPI SCC OPN AMBP GRP | 6 | 0.81 | 69 | 40 | | MIF SCC ENO2 TFPI MMP2 Ca19-9 CAIX GRP | 8 | 0.8820513 | 40 | 82.0512821 |
| SLPI TIMP1 SCC CEACAM5 OPN Ca72-4 | 6 | 0.81 | 69 | 40 | | MIF SCC ENO2 TFPI MMP2 CA242 CAIX GRP | 8 | 0.8820513 | 40 | 82.0512821 |
| SLPI SCC CEACAM5 OPN AMBP GRP DEFA1 | 7 | 0.81 | 69 | 40 | | MIF SCC ENO2 CEACAM5 MMP2 Ca19-9 CAIX GRP | 8 | 0.8820513 | 40 | 82.0512821 |
| SLPI OPN Ca19-9 DEFA1 | 4 | 0.81 | 31 | 60 | | MIF SCC ENO2 CEACAM5 MMP2 CA242 CAIX GRP | 8 | 0.8820513 | 40 | 82.0512821 |
| SLPI TIMP1 SCC CEACAM5 OPN GRP | 6 | 0.81 | 36 | 60 | | MIF ENO2 TFPI CEACAM5 MMP2 Ca19-9 CAIX GRP | 8 | 0.8820513 | 40 | 82.0512821 |
| MIF SLPI SCC DEFA1 | 4 | 0.81 | 28 | 60 | | MIF ENO2 TFPI CEACAM5 MMP2 CA242 CAIX GRP | 8 | 0.8820513 | 40 | 82.0512821 |
| SLPI SCC OPN Ca19-9 CA242 | 5 | 0.81 | 62 | 40 | | TIMP1 SCC ENO2 TFPI MMP2 Ca19-9 CAIX GRP | 8 | 0.8820513 | 40 | 82.0512821 |
| SCC TFPI CEACAM5 OPN DEFA1 | 5 | 0.81 | 59 | 40 | | TIMP1 SCC ENO2 TFPI MMP2 CA242 CAIX GRP | 8 | 0.8820513 | 40 | 82.0512821 |
| SLPI TIMP1 SCC TFPI CEACAM5 OPN DEFA1 | 7 | 0.81 | 59 | 40 | | TIMP1 SCC ENO2 CEACAM5 MMP2 Ca19-9 CAIX GRP | 8 | 0.8820513 | 40 | 82.0512821 |
| MIF SLPI SCC TFPI OPN Ca19-9 Ca72-4 GRP DEFA1 | 9 | 0.81 | 59 | 40 | | TIMP1 SCC ENO2 CEACAM5 MMP2 CA242 CAIX GRP | 8 | 0.8820513 | 40 | 82.0512821 |
| Cyfra MIF SLPI TIMP1 SCC OPN Ca19-9 | 7 | 0.80 | 51 | 0 | | TIMP1 ENO2 TFPI CEACAM5 MMP2 Ca19-9 CAIX GRP | 8 | 0.8820513 | 40 | 82.0512821 |
| SLPI OPN Ca72-4 CA242 DEFA1 | 5 | 0.80 | 49 | 20 | | TIMP1 ENO2 TFPI CEACAM5 MMP2 CA242 CAIX GRP | 8 | 0.8820513 | 40 | 82.0512821 |
| SCC OPN AMBP GRP DEFA1 | 5 | 0.80 | 51 | 20 | | SCC ENO2 TFPI MMP2 AMBP Ca19-9 CAIX GRP | 8 | 0.8820513 | 40 | 82.0512821 |
| Cyfra SLPI TIMP1 SCC CEACAM5 OPN Ca19-9 CA242 DEFA1 | 9 | 0.80 | 62 | 0 | | SCC ENO2 TFPI MMP2 AMBP CA242 CAIX GRP | 8 | 0.8820513 | 40 | 82.0512821 |
| MIF SLPI TIMP1 SCC TFPI OPN CA242 GRP DEFA1 | 9 | 0.80 | 62 | 0 | | SCC ENO2 CEACAM5 MMP2 AMBP Ca19-9 CAIX GRP | 8 | 0.8820513 | 40 | 82.0512821 |
| Cyfra SLPI TIMP1 SCC OPN | 5 | 0.80 | 56 | 20 | | SCC ENO2 CEACAM5 MMP2 AMBP CA242 CAIX GRP | 8 | 0.8820513 | 40 | 82.0512821 |
| MIF SLPI SCC TFPI OPN Ca72-4 GRP | 7 | 0.80 | 56 | 20 | | EN02 TFPI CEACAM5 MMP2 AMBP Ca19-9 CAIX GRP | 8 | 0.8820513 | 40 | 82.0512821 |
| MIF SLPI TIMP1 SCC OPN AMBP Ca19-9 DEFA1 | 8 | 0.80 | 51 | 0 | | ENO2 TFPI CEACAM5 MMP2 AMBP CA242 CAIX GRP | 8 | 0.8820513 | 40 | 82.0512821 |
| SLPI SCC OPN AMBP Ca19-9 C242 GRP DEFA1 | 8 | 0.80 | 51 | 0 | | Cyfra MIF TIMD1 SCC ENO2 MMP2 Ca19-9 CAIX GRP | 9 | 0.8820513 | 40 | 82.0512821 |
| Cyfra SLPI SCC CEACAM5 OPN Ca19-9 CA242 GRP DEFA1 | 9 | 0.80 | 44 | 20 | | Cyfra MIF TIMP1 SCC ENO2 MMP2 CA242 CAIX GRP | 9 | 0.8820513 | 40 | 82.0512821 |
| MIF SLPI TIMP1 SCC TFPI CEACAM5 OPN Ca19-9 DEFA1 | 9 | 0.80 | 36 | 20 | | Cyfra MIF TIMP1 ENO2 TFPI MMP2 Ca19-9 CAIX GRP | 9 | 0.8820513 | 40 | 82.0512821 |
| MIF SLPI TIMP1 SCC CEACAM5 OPN CA242 GRP DEFA1 | 9 | 0.80 | 72 | 0 | | Cyfra MIF TIMD1 ENO2 TFPI MMP2 CA242 CAIX GRP | 9 | 0.8820513 | 40 | 82.0512821 |
| SLPI TIMP1 SCC TFPI CEACAM5 OPN Ca72-4 CA242 DEFA1 | 9 | 0.80 | 59 | 20 | | Cyfra MIF TIMP1 ENO2 CEACAM5 MMP2 Ca19-9 CAIX GRP | 9 | 0.8820513 | 40 | 82.0512821 |
| TIMP1 SCC OPN Ca72-4 DEFA1 | 5 | 0.80 | 51 | 40 | | Cyfra MIF TIMP1 ENO2 CEACAM5 MMP2 CA242 CAIX GRP | 9 | 0.8820513 | 40 | 82.0512821 |
| SCC TFPI OPN Ca72-4 GRP DEFA1 | 6 | 0.80 | 31 | 0 | | Cyfra MIF SCC ENO2 MMP2 AMBP Ca19-9 CAIX GRP | 9 | 0.8820513 | 40 | 82.0512821 |
| MIF SLPI TIMP1 SCC OPN AMBP Ca19-9 CA242 GRP DEFA1 | 10 | 0.80 | 79 | 0 | | Cyfra MIF SCC ENO2 MMP2 AMBP CA242 CAIX GRP | 9 | 0.8820513 | 40 | 82.0512821 |
| SLPI SCC CEACAM5 OPN Ca19-9 GRP | 6 | 0.80 | 46 | 40 | | Cyfra MIF ENO2 TFPI MMP2 AMBP Ca19-9 CAIX GRP | 9 | 0.8820513 | 40 | 82.0512821 |
| SCC CEACAM5 OPN Ca19-9 GRP DEFA1 | 6 | 0.80 | 46 | 0 | | Cyfra MIF ENO2 TFPI MMP2 AMBP CA242 CAIX GRP | 9 | 0.8820513 | 40 | 82.0512821 |
| Cyfra SLPI SCC TFPI OPN Ca72-4 DEFA1 | 7 | 0.80 | 56 | 40 | | Cyfra MIF ENO2 CEACAM5 MMP2 AMBP Ca19-9 CAIX GRP | 9 | 0.8820513 | 40 | 82.0512821 |
| Cyfra SLPI SCC OPN Ca19-9 CA242 GRP DEFA1 | 8 | 0.80 | 49 | 0 | | Cyfra MIF ENO2 CEACAM5 MMP2 AMBP CA242 CAIX GRP | 9 | 0.8820513 | 40 | 82.0512821 |
| MIF SLPI TIMP1 SCC CEACAM5 OPN AMBP DEFA1 | | 0.80 | 41 | 0 | | Cyfra TIMP1 SCC ENO2 MMP2 AMBP Ca19-9 CAIX GRP | 9 | 0.8820513 | 40 | 82.0512821 |
| MIF SLPI TIMP1 SCC CEACAM5 OPN CA242 GRP | 8 | 0.80 | 77 | 0 | | Cyfra TIMP1 SCC ENO2 MMP2 AMBP CA242 CAIX GRP | 9 | 0.8820513 | 40 | 82.0512821 |
| MIF SLPI TIMP1 SCC OPN Ca72-4 CA242 DEFA1 | 8 | 0.80 | 49 | 20 | | Cyfra TIMP1 ENO2 TFPI MMP2 AMBP Ca19-9 CAIX GRP | 9 | 0.8820513 | 40 | 82.0512821 |
| MIF SLPI SCC OPN AMBP Ca19-9 CA242 DEFA1 | 8 | 0.80 | 59 | 20 | | Cyfra TIMP1 ENO2 TFPI MMP2 AMBP CA242 CAIX GRP | 9 | 0.8820513 | 40 | 82.0512821 |
| SLPI SCC CEACAM5 OPN AMBP CA242 GRP DEFA1 | 8 | 0.80 | 56 | 20 | | Cyfra TIMP1 ENO2 CEACAM5 MMP2 AMBP Ca19-9 CAIX GRP | 9 | 0.8820513 | 40 | 82.0512821 |
| SLPI SCC CEACAM5 OPN Ca19-9 Ca72-4 CA242 DEFA1 | 8 | 0.80 | 56 | 40 | | Cyfra TIMP1 ENO2 CEACAM5 MMP2 AMBP CA242 CAIX GRP | 9 | 0.8820513 | 40 | 82.0512821 |
| Cyfra SLPI TIMP1 SCC OPN Ca19-9 | 6 | 0.80 | 72 | 0 | | MIF SLPI SCC ENO2 TFDI MMP2 Ca19-9 CAIX GRP | 9 | 0.8820513 | 40 | 82.0512821 |
| Cyfra SLPI SCC CEACAM5 OPN CA242 | 6 | 0.80 | 38 | 60 | | MIF SLPI SCC ENO2 TFPI MMP2 CA241 CAIX GRP | 9 | 0.8820513 | 40 | 82.0512821 |
| SLPI SCC CEACAM5 OPN Ca72-4 GRP | 6 | 0.80 | 36 | 60 | | MIF SLPI SCC ENO2 CEACAM5 MMP2 Ca19-9 CAIX GRP | 9 | 0.8820513 | 40 | 82.0512821 |
| MIF SLPI TIMP1 SCC TFPI OPN CA242 DEFA1 | 8 | 0.80 | 72 | 0 | | MIF SLPI SCC ENO2 CEACAM5 MMP2 CA242 CAIX GRP | 9 | 0.8820513 | 40 | 82.0512821 |
| SLPI TIMP1 SCC TFPI OPN AMBP Ca72-4 DEFA1 | 8 | 0.80 | 72 | 20 | | MIF SLPI ENO2 TFPI CEACAM5 MMP2 Ca19-9 CAIX GRP | 9 | 0.8820513 | 40 | 82.0512821 |
| SLPI SCC OPN AMBP Ca19-9 Ca72-4 GRP DEFA1 | 8 | 0.80 | 44 | 20 | | MIF SLPI ENO2 TFPI CEACAM5 MMP2 CA242 CAIX GRP | 9 | 0.8820513 | 40 | 82.0512821 |
| SLPI SCC OPN Ca19-9 Ca72-4 CA242 GRP DEFA1 | 8 | 0.80 | 51 | 20 | | SLPI TIMP1 SCC ENO2 TFPI MMP2 Ca19-9 CAIX GRP | 9 | 0.8820513 | 40 | 82.0512821 |
| MIF SLPI SCC OPN Ca19-9 Ca72-4 CA242 GRP DEFA1 | 9 | 0.80 | 69 | 20 | | SLPI TIMP1 SCC EN02 TFPI MMP2 CA242 CAIX GRP | 9 | 0.8820513 | 40 | 82.0512821 |
| SLPI TIMP1 SCC CEACAM5 OPN AMBP Ca72-4 DEFA1 | 8 | 0.80 | 69 | 40 | | SLPI TIMP1 SCC ENO2 CEACAM5 MMP2 Ca19-9 CAIX GRP | 9 | 0.8820513 | 40 | 82.0512821 |
| MIF SLPI SCC CEACAM5 OPN Ca19-9 Ca72-4 CA242 DEFA1 | 9 | 0.80 | 69 | 40 | | SLPI TIMP1 SCC ENO2 CEACAM5 MMP2 CA242 CAIX GRP | 9 | 0.8820513 | 40 | 82.0512821 |
| MIF SLPI SCC OPN AMBP Ca19-9 Ca72-4 CA242 DEFA1 | 9 | 0.80 | 69 | 40 | | SLPI TIMP1 ENO2 TFPI CEACAM5 MMP2 Ca19-9 CAIX GRP | 9 | 0.8820513 | 40 | 82.0512821 |
| OPN DEFA1 | 2 | 0.80 | 67 | 40 | | SLPI TIMP1 ENO2 TFPI CEACAM5 MMP2 CA242 CAIX GRP | 9 | 0.8820513 | 40 | 82.0512821 |
| SLPI SCC OPN AMBP Ca19-9 | 5 | 0.80 | 64 | 20 | | SLPI SCC ENO2 TFPI MMP2 AMBP Ca19-9 CAIX GRP | 9 | 0.8820513 | 40 | 82.0512821 |
| MIF SLPI SCC CEACAM5 OPN AMBP CA242 | 7 | 0.80 | 64 | 20 | | SLPI SCC ENO2 TFPI MMP2 AMBP CA242 CAIX GRP | 9 | 0.8820513 | 40 | 82.0512821 |
| SLPI TIMP1 SCC CEACAM5 OPN Ca19-9 Ca72-4 CA242 DEFA1 | 9 | 0.80 | 67 | 40 | | SLPI SCC ENO2 CEACAM5 MMP2 AMBP Ca19-9 CAIX GRP | 9 | 0.8820513 | 40 | 82.0512821 |
| SLPI SCC TFPI OPN GRP | 5 | 0.80 | 62 | 40 | | SLPI SCC ENO2 CEACAMS MMP2 AMBP CA242 CAIX GRP | 9 | 0.8820513 | 40 | 82.0512821 |
| SLPI SCC OPN Ca19-9 Ca72-4 | S | 0.80 | 59 | 40 | | SLPI ENO2 TFPI CEACAM5 MMP2 AMBP Ca19-9 CAIX GRP | 9 | 0.8820513 | 40 | 82.0512821 |
| TIMP1 SCC CEACAM5 OPN Ca72-4 DEFA1 | 6 | 0.80 | 62 | 0 | | SLPI ENO2 TFPI CEACAM5 MMP2 AMBP CA242 CAIX GRP | 9 | 0.8820513 | 40 | 82.0512821 |
| Cyfra MIF SLPI SCC CEACAM5 OPN Ca19-9 | 7 | 0.80 | 59 | 20 | | Cyfra MIF SLPI TIMP1 SCC ENO2 MMP2 Ca19-9 CAIX GRP | 10 | 0.8820513 | 40 | 82.0512821 |
| Cyfra SLPI SCC OPN AMBP Ca19-9 DEFA1 | 7 | 0.80 | 62 | 40 | | Cyfra MIF SLPI TIMP1 SCC ENO2 MMP2 CA242 CAIX GRP | 10 | 0.8820513 | 40 | 82.0512811 |
| SLPI TIMP1 SCC TFPI OPN AMBP DEFA1 | 7 | 0.80 | 62 | 40 | | Cyfra MIF SLPI TIMP1 ENO2 TFPI MMP2 Ca19-9 CAIX GRP | 10 | 0.8820513 | 40 | 82.0512821 |
| SLPI SCC OPN AMBP Ca19-9 Ca72-4 DEFA1 | 7 | 0.80 | 59 | 40 | | Cyfra MIF SLPI TIMP1 ENO2 TFPI MMP2 CA242 CAIX GRP | 10 | 0.8820513 | 40 | 82.0512821 |
| MIF SLPI TIMP1 SCC TFPI CEACAM5 OPN GRP DEFA1 | 9 | 0.80 | 62 | 20 | | Cyfra MIF SLPI TIMP1 ENO2 CEACAM5 MMP2 Ca19-9 CAIX GRP | 10 | 0.8820513 | 40 | 82.05128Z1 |
| SLPI SCC OPN CA242 CAIX | 5 | 0.79 | 49 | 0 | | Cyfra MIF SLPI TIMP1 ENO2 CEACAM5 MMP2 CA242 CAIX GRP | 10 | 0.8820513 | 40 | 82.0512821 |
| MIF SLPI TIMP1 SCC TFPI OPN CA242 | 7 | 0.79 | 59 | 0 | | Cyfra MIF SLPI SCC ENO2 MMP2 AMBP Ca19-9 CAIX GRP | 10 | 0.8820513 | 40 | 82.0512821 |
| Cyfra MIF SLPI TIMP1 SCC TFPI OPN Ca19-9 DEFA1 | 9 | 0.79 | 59 | 0 | | Cyfra MIF SLPI SCC ENO2 MMP2 AMBP CA242 CAIX GRP | 10 | 0.8820513 | 40 | 82.0512821 |
| MIF SLPI SCC Ca72-4 DEFA1 | 5 | 0.79 | 51 | 20 | | Cyfra MIF SLPI ENO2 TFPI MMP2 AMBP Ca19-9 CAIX GRP | 10 | 0.8820513 | 40 | 82.0512821 |
| SCC TFPI OPN CA242 DEFA1 | 5 | 0.79 | 54 | 20 | | Cyfra MIF SLPI ENO2 TFPI MMP2 AMBP CA242 CAIX GRP | 10 | 0.8820513 | 40 | 82.0512821 |
| SCC OPN AMBP CA242 DEFA1 | 5 | 0.79 | 56 | 20 | | Cyfra MIF SLPI ENO2 CEACAM5 MMP2 AMBP Ca19-9 CAIX GRP | 10 | 0.8820513 | 40 | 82.0512821 |
| MIF SLPI TIMD1 SCC TFPI OPN Ca72-4 | 7 | 0.79 | 54 | 20 | | Cyfra MIF SLPI ENO2 CEACAM5 MMP2 AMBP CA242 CAIX GRP | 10 | 0.8820513 | 40 | 82.0512821 |
| MIF SLPI TIMP1 SCC OPN Ca72-4 GRP | 7 | 0.79 | 46 | 20 | | Cyfra MIF TIMP1 SCC ENO2 MMP2 Ca19-9 CA242 CAIX GRP | 10 | 0.8820513 | 40 | 82.0512821 |
| MIF SLPI SCC OPN Ca72-4 CAIX DEFA1 | 7 | 0.79 | 54 | 20 | | Cyfra MIF TIMP1 ENO2 TFPI MMP2 Ca19-9 CA242 CAIX GRP | 10 | 0.8820513 | 40 | 82.0512821 |
| Cyfra SLPI TIMP1 SCC CEACAM5 OPN Ca72-4 CA242 DEFA1 | 9 | 0.79 | 56 | 20 | | Cyfra MIF TIMP1 ENO2 CEACAM5 MMP2 Ca19-9 CA242 CAIX GRP | 10 | 0.8820513 | 40 | 82.0512821 |
| MIF SLPI TIMP1 SCC OPN AMBP Ca19-9 GRP DEFA1 | 9 | 0.79 | 69 | 0 | | Cyfra MIF SCC ENO2 MMP2 AMBP Ca19-9 CA242 CAIX GRP | 10 | 0.8820513 | 40 | 82.0512821 |
| Cyfra SLPI OPN Ca72-4 DEFA1 | 5 | 0.79 | 79 | 0 | | Cyfra MIF ENO2 TFPI MMP2 AMBP Ca19-9 CA242 CAIX GRP | 10 | 0.8820513 | 40 | 82.0512821 |
| Cyfra SCC OPN Ca72-4 DEFA1 | 5 | 0.79 | 49 | 40 | | Cyfra MIF ENO2 CEACAM5 MMP2 AMBP Ca19-9 CA242 CAIX GRP | 10 | 0.8820513 | 40 | 82.0512821 |
| TIMP1 SCC OPN AMBP DEFA1 | 5 | 0.79 | 79 | 0 | | Cyfra SLPI TIMP1 SCC ENO2 MMP2 AMBP Ca19-9 CAIX GRP | 10 | 0.8820513 | 40 | 82.0512821 |
| SLPI SCC TFPI CEACAM5 OPN CA242 | 6 | 0.79 | 51 | 20 | | Cyfra SLPI TIMP1 SCC ENO2 MMP2 AMBP CA242 CAIX GRP | 10 | 0.8820513 | 40 | 82.0512821 |
| Cyfra SLPI TIMP1 SCC OPN AMBP DEFA1 | 7 | 0.79 | 64 | 20 | | Cyfra SLPI TIMP1 ENO2 TFPI MMP2 AMBP Ca19-9 CAIX GRP | 10 | 0.8820513 | 40 | 82.0512821 |
| SLPI TIMP1 SCC OPN Ca72-4 CAIX DEFA1 | 7 | 0.79 | 79 | 0 | | Cyfra SLPI TIMP1 ENO2 TFPI MMP2 AMBP CA242 CAIX GRP | 10 | 0.8820513 | 40 | 82.0512821 |
| MIF SLPI TIMP1 SCC OPN Ca19-9 CA242 DEFA1 | 8 | 0.79 | 54 | 20 | | Cyfra SLPI TIMP1 ENO2 CEACAM5 MMP2 AMBP Ca19-9 CAIX GRP | 10 | 0.8820513 | 40 | 82.0512821 |
| MIF SLPI SCC CEACAM5 OPN Ca19-9 CA242 GRP | 8 | 0.79 | 49 | 40 | | Cyfra SLPI TIMP1 ENO2 CEACAM5 MMP2 AMBP CA242 CAIX GRP | 10 | 0.8820513 | 40 | 82.0512821 |
| Cyfra MIF SLPI TIMP1 SCC OPN AMBP Ca72-4 DEFA1 | 9 | 0.79 | 79 | 0 | | Cyfra TIMP1 SCC ENO2 MMP2 AMBP Ca19-9 CA242 CAIX GRP | 10 | 0.8820513 | 40 | 82.0512821 |
| Cyfra MIF SLPI TIMP1 SCC OPN Ca19-9 Ca72-4 DEFA1 | 9 | 0.79 | 64 | 20 | | Cyfra TIMP1 ENO2 TFPI MMP2 AMBP Ca19-9 CA242 CAIX GRP | 10 | 0.8820513 | 40 | 82.0512821 |
| MIF SLPI CEACAM5 OPN DEFA1 | 5 | 0.79 | 21 | 20 | | Cyfra TIMP1 ENO2 CEACAM5 MMP2 AMBP Ca19-9 CA242 CAIX GRP | 10 | 0.8820513 | 40 | 82.0512821 |
| MIF SLPI SCC TFPI OPN Ca19-9 | 6 | 0.79 | 36 | 40 | | MIF TIMP1 SCC ENO2 TFPI MMP2 Ca19-9 CA242 CAIX GRP | 10 | 0.8820513 | 40 | 82.0512821 |
| MIF SLPI SCC CEACAM5 OPN CAIX DEFA1 | 7 | 0.79 | 31 | 20 | | MIF TIMP1 SCC ENO2 CEACAM5 MMP2 Ca19-9 CA242 CAIX GRP | 10 | 0.8820513 | 40 | 82.0512821 |
| MIF SLPI TIMP1 SCC TFPI OPN AMBP DEFA1 | 8 | 0.79 | 51 | 0 | | MIF TIMP1 ENO2 TFPI CEACAM5 MMP2 Ca19-9 CA242 CAIX GRP | 10 | 0.8820513 | 40 | 82.0512821 |
| MIF SLPI TIMP1 SCC CEACAM5 OPN AMBP CA242 | 8 | 0.79 | 77 | 0 | | MIF SCC ENO2 TFPI MMP2 AMBP Ca19-9 CA242 CAIX GRP | 10 | 0.8820513 | 40 | 82.0512821 |
| MIF SLPI SCC TFPI CEACAM5 OPN CA242 GRP | 8 | 0.79 | 49 | 40 | | MIF SCC ENO2 CEACAM5 MMP2 AMBP Ca19-9 CA242 CAIX GRP | 10 | 0.8820513 | 40 | 82.0512821 |
| MIF SLPI SCC TFPI OPN AMBP Ca72-4 DEFA1 | 8 | 0.79 | 46 | 20 | | MIF EN02 TFPI CEACAM5 MMP2 AMBP Ca19-9 CA242 CAIX GRP | 10 | 0.8820513 | 40 . | 82.0512821 |
| SLPI SCC TFPI CEACAM5 OPN Ca72-4 GRP DEFA1 | 8 | 0.79 | 49 | 40 | | TIMP1 SCC ENO2 TFPI MMP2 AMBP Ca19-9 CA242 CAIX GRP | 10 | 0.8820513 | 40 | 82.0512821 |
| Cyfra MIF SLPI TIMP1 SCC CEACAM5 OPN Ca19-9 CA242 DEFA1 | 10 | 0.79 | 77 | 0 | | TIMP1 SCC ENO2 CEACAM5 MMP2 AMBP Ca19-9 CA242 CAIX GRP | 10 | 0.8820513 | 40 | 82.0512821 |
| Cyfra MIF SCC TFPI OPN DEFA1 | 6 | 0.79 | 74 | 0 | | TIMP1 ENO2 TFPI CEACAM5 MMP2 AMBP Ca19-9 CA242 CAIX GRP | 10 | 0.8820513 | 40 | 82.0512821 |
| MIF SLPI SCC TFPI OPN AMBP DEFA1 | 7 | 0.79 | 33 | 40 | | Cyfra MIF SLPI TIMP1 SCC ENO2 MMP2 Ca19-9 CA242 CAIX GRP | 11 | 0.8820513 | 40 | 82.0512821 |
| Cyfra MIF SLPI SCC OPN AMBP CA242 DEFA1 | 8 | 0.79 | 74 | 0 | | Cyfra MIF SLPI TIMP1 ENO2 TFPI MMP2 Ca19-9 CA242 CAIX GRP | 11 | 0.8820513 | 40 | 82.0512821 |
| MIF SLPI TIMP1 SCC OPN AMBP CA242 DEFA1 | 8 | 0.79 | 74 | 0 | | Cyfra MIF SLPI TIMP1 ENO2 CEACAM5 MMP2 Ca19-9 CA242 CAIX GRP | 11 | 0.8820513 | 40 | 82.0512821 |
| MIF SLPI SCC TFPI OPN Ca19-9 Ca72-4 DEFA1 | 8 | 0.79 | 44 | 40 | | Cyfra MIF SLPI SCC ENO2 MMP2 AMBP Ca19-9 CA242 CAIX GRP | 11 | 0.8820513 | 40 | 82.0512821 |
| MIF SLPI SCC TFPI OPN Ca19-9 CA242 DEFA1 | 8 | 0.79 | 56 | 40 | | Cyfra MIF SLPI ENO2 TFPI MMP2 AMBP Ca19-9 CA242 CAIX GRP | 11 | 0.8820513 | 40 | 82.0512821 |
| MIF SLPI SCC OPN Ca19-9 Ca72-4 CA242 DEFA1 | 8 | 0.79 | 56 | 20 | | Cyfra MIF SLPI ENO2 CEACAM5 MMP2 AMBP Ca19-9 CA242 CAIX GRP | 11 | 0.8820513 | 40 | 82.0512821 |
| SLPI TIMP1 SCC OPN AMBP Ca19-9 CA242 DEFA1 | 8 | 0.79 | 74 | 0 | | Cyfra SLPI TIMP1 SCC ENO2 MMP2 AMBP Ca19-9 CA242 CAIX GRP | 11 | 0.8820513 | 40 | 82.0512821 |
| SLPI TIMP1 SCC OPN AMBP CA242 GRP DEFA1 | 8 | 0.79 | 74 | 0 | | Cyfra SLPI TIMP1 ENO2 TFPI MMP2 AMBP Ca19-9 CA242 CAIX GRP | 11 | 0.8820513 | 40 | 82.0512821 |
| SLPI TIMP1 SCC OPN Ca19-9 CA242 GRP DEFA1 | 8 | 0.79 | 74 | 0 | | Cyfra SLPI TIMP1 ENO2 CEACAM5 MMP2 AMBP Ca19-9 CA242 CAIX GRP | 11 | 0.8820513 | 40 | 82.0512821 |
| SLPI SCC TFPI CEACAM5 OPN CA242 GRP DEFA1 | 8 | 0.79 | 54 | 40 | | MIF SLPI TIMP1 SCC ENO2 TFPI MMP2 Ca19-9 CA242 CAIX GRP | 11 | 0.8820513 | 40 | 82.0512821 |
| MIF SLPI TIMP1 SCC TFPI OPN AMBP Ca72-4 CA242 DEFA1 | 10 | 0.79 | 74 | 0 | | MIF SLPI TIMP1 SCC ENO2 CEACAM5 MMP2 Ca19-9 CA242 CAIX GRP | 11 | 0.8820513 | 40 | 82.0512821 |
| MIF SLPI TIMP1 SCC CEACAM5 OPN Ca72-4 CA242 GRP DEFA1 | 10 | 0.79 | 74 | 0 | | MIF SLPI TIMP1 ENO2 TFPI CEACAM5 MMP2 Ca19-9 CA242 CAIX GRP | 11 | 0.8820513 | 40 | 82.0512821 |
| MIF SLPI SCC OPN CAIX DEFA1 | 6 | 0.79 | 36 | 60 | | MIF SLPI SCC ENO2 TFPI MMP2 AMBP Ca19-9 CA242 CAIX GRP | 11 | 0.8820513 | 40 | 82.0512821 |
| SLPI SCC CEACAM5 OPN Ca72-4 CA242 | 6 | 0.79 | 36 | 20 | | MIF SLPI SCC ENO2 CEACAM5 MMP2 AMBP Ca19-9 CA242 CAIX GRP | 11 | 0.8820513 | 40 | 82.0512821 |
| MIF SLPI CEACAM5 OPN CA242 GRP DEFA1 | 7 | 0.79 | 72 | 0 | | MIF SLPI EN02 TFPI CEACAM5 MMP2 AMBP Ca19-9 CA242 CAIX GRP | 11 | 0.8820513 | 40 | 82.0512821 |
| SLPI TIMP1 SCC OPN AMBP Ca72-4 CA242 DEFA1 | 8 | 0.79 | 72 | 0 | | SLPI TIMP1 SCC ENO2 TFPI MMP2 AMBP Ca19-9 CA242 CAIX GRP | 11 | 0.8820513 | 40 | 82.0512821 |
| Cyfra MIF SLPI SCC CEACAM5 OPN AMBP CA242 DEFA1 | 9 | 0.79 | 69 | 20 | | SLPI TIMP1 SCC ENO2 CEACAM5 MMP2 AMBP Ca19-9 CA242 CAIX GRP | 11 | 0.8820513 | 40 | 82.0512821 |
| Cyfra MIF SLPI SCC OPN Ca19-9 CA242 GRP DEFA1 | 9 | 0.79 | 69 | 20 | | SLPI TIMP1 ENO2 TFPI CEACAM5 MMP2 AMBP Ca19-9 CA242 CAIX GRP | 11 | 0.8820513 | 40 | 82.0512821 |
| MIF SLPI TIMP1 SCC OPN Ca19-9 CA242 GRP DEFA1 | 9 | 0.79 | 72 | 0 | | SCC ENO2 MMP2 CAIX GRP | 5 | 0.8769231 | 20 | 84.6153846 |
| Cyfra MIF SLPI SCC CEACAM5 OPN Ca72-4 CA242 GRP DEFA1 | 10 | 0.79 | 49 | 0 | | ENO2 TFPI MMP2 CAIX GRP | 5 | 0.8769231 | 20 | 84.6153846 |
| Cyfra MIF SLPI TIMP1 SCC OPN | 6 | 0.79 | 69 | 40 | | ENO2 CEACAM5 MMP2 CAIX GRP | 5 | 0.8769231 | 20 | 84.6153846 |
| MIF SCC OPN Ca72-4 GRP DEFA1 | 6 | 0.79 | 69 | 20 | | Cyfra SCC EN02 MMP2 Ca19-9 CAIX | 6 | 0.8769231 | 20 | 84.6153846 |
| SLPI TIMP1 SCC OPN AMBP Ca72-4 | 6 | 0.79 | 69 | 20 | | Cyfra SCC ENO2 MMP2 CA242 CAIX | 6 | 0.8769231 | 20 | 84.6153846 |
| SLPI TIMP1 SCC OPN Ca72-4 GRP | 6 | 0.79 | 69 | 20 | | Cyfra ENO2 TFPI MMP2 Ca19-9 CAIX | 6 | 0.8769231 | 20 | 84.6153846 |
| SLPI SCC OPN CA242 CAIX DEFA1 | 6 | 0.79 | 69 | 40 | | Cyfra ENO2 TFPI MMP2 CA242 CAIX | 6 | 0.8769231 | 20 | 84.6153846 |
| SLPI OPN Ca19-9 Ca72-4 GRP DEFA1 | 6 | 0.79 | 69 | 40 | | Cyfra ENO2 CEACAM5 MMP2 Ca19-9 CAIX | 6 | 0.8769231 | 20 | 84.6153846 |
| SLPI TIMP1 SCC TFPI OPN Ca19-9 GRP DEFA1 | 8 | 0.79 | 69 | 40 | | Cyfra ENO2 CEACAM5 MMP2 CA242 CAIX | 6 | 0.8769231 | 20 | 84.6153846 |
| MIF SLPI SCC OPN Ca72-4 CA242 | 6 | 0.79 | 33 | 60 | | Cyfra ENO2 MMP2 AMBP Ca19-9 CAIX | 6 | 0.8769231 | 20 | 84.6153846 |
| SLPI SCC OPN Ca19-9 Ca72-4 GRP | 6 | 0.79 | 38 | 60 | | Cyfra ENO2 MMP2 AMBP CA242 CAIX | 6 | 0.8769231 | 20 | 84.6153846 |
| MIF SCC TFPI CEACAM5 OPN CA242 DEFA1 | 7 | 0.79 | 64 | 20 | | SLPI SCC ENO2 MMP2 CAIX GRP | 6 | 0.8769231 | 20 | 84.6153846 |
| MIF SCC CEACAM5 OPN Ca19-9 GRP DEFA1 | 7 | 0.79 | 64 | 20 | | SLPI ENO2 TFPI MMP2 CAIX GRP | | 0.8769231 | 20 | 84.6153846 |
| MIF SLPI TIMP1 SCC CEACAM5 OPN Ca72-4 CA242 | 8 | 0.79 | 44 | 60 | | SLPI ENO2 CEACAM5 MMP2 CAIX GRP | 6 | 0.8769231 | 20 | 84.6153846 |
| SLPI TIMP1 SCC TFPI OPN Ca72-4 CA242 DEFA1 | 8 | 0.79 | 64 | 0 | | Cyfra SLPI SCC ENO2 MMP2 Ca19-9 CAIX | 7 | 0.8769231 | 20 | 84.6153846 |
| MIF SLPI TIMP1 SCC OPN Ca72-4 CA242 GRP DEFA1 | 9 | 0.79 | 67 | 0 | | Cyfra SLPI SCC ENO2 MMP2 CA242 CAIX | 7 | 0.8769231 | 20 | 84.6153846 |
| MIF SLPI SCC TFPI CEACAM5 OPN Ca19-9 GRP DEFA1 | 9 | 0.79 | 38 | 40 | | Cyfra SLPI ENO2 TFPI MMP2 Ca19-9 CAIX | 7 | 0.8769231 | 20 | 84.6153846 |
| MIF SLPI SCC TFPI OPN AMBP CA242 GRP DEFA1 | 9 | 0.79 | 67 | 20 | | Cyfra SLPI ENO2 TFPI MMP2 CA242 CAIX | 7 | 0.8769231 | 20 | 84.6153846 |
| MIF SCC OPN Ca19-9 DEFA1 | 5 | 0.79 | 62 | 20 | | Cyfra SLPI ENO2 CEACAM5 MMP2 Ca19-9 CAIX | 7 | 0.8769231 | 20 | 84.6153846 |
| Cyfra SLPI TIMP1 SCC CEACAM5 OPN | 6 | 0.79 | 36 | 60 | | Cyfra SLPI ENO2 CEACAM5 MMP2 CA242 CAIX | 7 | 0.8769231 | 20 | 84.6153846 |
| Cyfra SLPI SCC OPN CA242 GRP DEFA1 | 7 | 0.79 | 62 | 40 | | Cyfra SLPI ENO2 MMP2 AMBP Ca19-9 CAIX | 7 | 0.8769231 | 20 | 84.6153846 |
| Cyfra MIF SLPI SCC CEACAM5 OPN AMBP Ca72-4 DEFA1 | 9 | 0.79 | 62 | 40 | | Cyfra SLPI ENO2 MMP2 AMBP CA242 CAIX | 7 | 0.8769231 | 20 | 84.6153846 |
| SLPI TIMP1 SCC CEACAM5 OPN AMBP DEFA1 | 7 | 0.79 | 56 | 40 | | Cyfra SCC ENO2 MMP2 Ca19-9 CA242 CAIX | 7 | 0.8769231 | 20 | 84.6153846 |
| Cyfra SLPI OPN CA242 DEFA1 | 5 | 0.79 | 46 | 0 | | Cyfra ENO2 TFPI MMP2 Ca19-9 CA242 CAIX | 7 | 0.8769231 | 20 | 84.6153846 |
| SLPI OPN AMBP CA242 DEFA1 | 5 | 0.79 | 56 | 0 | | Cyfra ENO2 CEACAM5 MMP2 Ca19-9 CA242 CAIX | 7 | 0.8769231 | 20 | 84.6153846 |
| Cyfra MIF SLPI SCC TFPI OPN CA242 | 7 | 0.79 | 56 | 0 | | Cyfra ENO2 MMP2 AMBP Ca19-9 CA242 CAIX | 7 | 0.8769231 | 20 | 84.6153846 |
| SLPI SCC OPN AMBP Ca72-4 CA242 GRP DEFA1. | 8 | 0.79 | 46 | 0 | | SCC ENO2 TFPI MMP2 Ca19-9 CA242 CAIX | 7 | 0.8769231 | 20 | 84.6153846 |
| MIF SCC TFPI OPN Ca72-4 GRP DEFA1 | 7 | 0.79 | 54 | 20 | | SCC ENO2 CEACAM5 MMP2 Ca19-9 CA242 CAIX | 7 | 0.8769231 | 20 | 84.6153846 |
| SCC OPN GRP | 3 | 0.79 | 44 | 20 | | SCC ENO2 MMP2 AMBP Ca19-9 CA242 CAIX | 7 | 0.8769231 | 20 | 84.6153846 |
| SLPI TIMP1 OPN Ca72-4 DEFA1 | 5 | 0.79 | 44 | 20 | | ENO2 TFPI CEACAM5 MMP2 Ca19-9 CA242 CAIX | 7 | 0.8769231 | 20 | 84.6153846 |
| SLPI OPN AMBP Ca72-4 DEFA1 | 5 | 0.79 | 44 | 20 | | ENO2 TFPI MMP2 AMBP Ca19-9 CA242 CAIX | 7 | 0.8769231 | 20 | 84.6153846 |
| MIF SLPI TIMP1 SCC OPN AMBP Ca72-4 | 7 | 0.79 | 44 | 20 | | ENO2 CEACAM5 MMP2 AMBP Ca19-9 CA242 CAIX | 7 | 0.8769231 | 20 | 84.6153846 |
| SLPI TIMP1 SCC CEACAM5 OPN AMBP Ca19-9 DEFA1 | 8 | 0.79 | 49 | 20 | | Cyfra MIF TIMP1 ENO2 MMP2 AMBP CAIX GRP | 8 | 0.8769231 | 0 | 84.6153846 |
| MIF SLPI TIMP1 SCC CEACAM5 OPN AMBP Ca19-9 DEFA1 | 9 | 0.79 | 33 | 0 | | Cyfra MIF SCC ENO2 MMP2 Ca19-9 CA242 CAIX | 8 | 0.8769231 | 20 | 84.6153846 |
| SLPI TIMP1 SCC CEACAM5 OPN Ca72-4 CA242 GRP DEFA1 | 9 | 0.79 | 59 | 20 | | Cyfra MIF ENO2 TFPI MMP2 Ca19-9 CA242 CAIX | 8 | 0.8769231 | 20 | 84.6153846 |
| SLPI TIMP1 SCC OPN AMBP Ca19-9 Ca72-4 GRP DEFA1 | 9 | 0.79 | 64 | 0 | | Cyfra MIF ENO2 CEACAM5 MMP2 Ca19-9 CA242 CAIX | 8 | 0.8769231 | 20 | 84.6153846 |
| Cyfra MIF SLPI SCC CEACAM5 OPN Ca19-9 CA242 GRP DEFA1 | 10 | 0.79 | 56 | 0 | | Cyfra MIF ENO2 MMP2 AMBP Ca19-9 CA242 CAIX | 8 | 0.8769231 | 20 | 84.6153846 |
| MIF SLPI TIMP1 SCC TFPI CEACAM5 OPN Ca19-9 GRP DEFA1 | 10 | 0.79 | 51 | 0 | | Cyfra SLPI SCC ENO2 MMP2 Ca19-9 CA242 CAIX | 8 | 0.8769231 | 20 | 84.6153846 |
| MIF SLPI SCC CEACAM5 DEFA1 | 5 | 0.79 | 18 | 20 | | Cyfra SLPI ENO2 TFPI MMP2 Ca19-9 CA242 CAIX | 8 | 0.8769231 | 20 | 84.6153846 |
| MIF SLPI TIMP1 SCC OPN CA242 GRP DEFA1 | 8 | 0.79 | 56 | 0 | | Cyfra SLPI ENO2 CEACAM5 MMP2 Ca19-9 CA242 CAIX | 8 | 0.8769231 | 20 | 84.6153846 |
| MIF SLPI CEACAM5 OPN Ca72-4 CA242 GRP DEFA1 | 8 | 0.79 | 77 | 0 | | Cyfra SLPI ENO2 MMP2 AMBP Ca19-9 CA242 CAIX | 8 | 0.8769231 | 20 | 84.6153846 |
| Cyfra SLPI SCC CEACAM5 OPN Ca19-9 Ca72-4 CA242 DEFA1 | 9 | 0.79 | 36 | 40 | | Cyfra TIMP1 SCC ENO2 MMP2 Ca19-9 CA242 CAIX | 8 | 0.8769231 | 20 | 84.6153846 |
| MIF SLPI TIMP1 SCC CEACAM5 OPN Ca19-9 CA242 GRP DEFA1 | 10 | 0.79 | 77 | 0 | | Cyfra TIMP1 ENO2 TFPI MMP2 Ca19-9 CA242 CAIX | 8 | 0.8769231 | 20 | 84.6153846 |
| MIF SLPI TIMP1 SCC OPN AMBP Ca19-9 Ca72-4 GRP DEFA1 | 10 | 0.79 | 77 | 0 | | Cyfra TIMP1 ENO2 CEACAM5 MMP2 Ca19-9 CA242 CAIX | 8 | 0.8769231 | 20 | 84.6153846 |
| MIF SLPI SCC CEACAM5 OPN AMBP Ca72-4 CA242 GRP DEFA1 | 10 | 0.79 | 51 | 0 | | Cyfra TIMP1 ENO2 MMP2 AMBP Ca19-9 CA242 CAIX | 8 | 0.8769231 | 20 | 84.6153846 |
| MIF SCC CEACAM5 OPN Ca19-9 DEFA1 | 6 | 0.79 | 44 | 20 | | SLPI SCC ENO2 TFPI MMP2 Ca19-9 CA242 CAIX | 8 | 0.8769231 | 20 | 84.6153846 |
| SLPI TIMP1 SCC CEACAM5 OPN Ca19-9 | 6 | 0.79 | 74 | 20 | | SLPI SCC ENO2 CEACAM5 MMP2 Ca19-9 CA242 CAIX | 8 | 0.8769231 | 20 | 84.6153846 |
| Cyfra MIF SLPI SCC CEACAM5 OPN AMBP DEFA1 | 8 | 0.79 | 54 | 40 | | SLPI SCC ENO2 MMP2 AMBP Ca19-9 CA242 CAIX | 8 | 0.8769231 | 20 | 84.6153846 |
| Cyfra SLPI SCC CEACAM5 OPN CA242 GRP DEFA1 | 8 | 0.79 | 54 | 40 | | SLPI ENO2 TFPI CEACAM5 MMP2 Ca19-9 CA242 CAIX | 8 | 0.8769231 | 20 | 84.6153846 |
| MIF SLPI SCC OPN AMBP Ca19-9 Ca72-4 DEFA1 | 8 | 0.79 | 51 | 40 | | SLPI ENO2 TFPI MMP2 AMBP Ca19-9 CA242 CAIX | 8 | 0.8769231 | 20 | 84.6153846 |
| SLPI SCC OPN Ca19-9 CA242 GRP | 6 | 0.79 | 41 | 0 | | SLPI ENO2 CEACAM5 MMP2 AMBP Ca19-9 CA242 CAIX | 8 | 0.8769231 | 20 | 84.6153846 |
| TIMP1 SCC OPN Ca72-4 GRP DEFA1 | 6 | 0.79 | 72 | 20 | | Cyfra MIF SLPI TIMP1 ENO2 MMP2 AMBP CAIX GRP | 9 | 0.8769231 | 0 | 84.6153846 |
| SCC TFPI CEACAM5 OPN CA242 DEFA1 | 6 | 0.79 | 44 | 0 | | Cyfra MIF SLPI SCC EN02 MMP2 Ca19-9 CA242 CAIX | 9 | 0.8769231 | 20 | 84.6153846 |
| SCC CEACAM5 OPN CA242 GRP DEFA1 | 6 | 0.79 | 44 | 0 | | Cyfra MIF SLPI ENO2 TFPI MMP2 Ca19-9 CA242 CAIX | 9 | 0.8769231 | 20 | 84.6153846 |
| SLPI SCC CEACAM5 OPN Ca72-4 CA242 GRP | 7 | 0.79 | 26 | 40 | | Cyfra MIF SLPI ENO2 CEACAM5 MMP2 Ca19-9 CA242 CAIX | 9 | 0.8769231 | 20 | 84.6153846 |
| Cyfra MIF SLPI TIMP1 SCC OPN CA242 DEFA1 | 8 | 0.79 | 56 | 0 | | Cyfra MIF SLPI ENO2 MMP2 AMBP Ca19-9 CA242 CAIX | 9 | 0.8769231 | 20 | 84.6153846 |
| Cyfra SLP1 TIMP1 SCC OPN Ca19-9 CA242 DEFA1 | 8 | 0.79 | 72 | 0 | | Cyfra MIF TIMP1 SCC ENO2 MMP2 Ca19-9 CA242 CAIX | 9 | 0.8769231 | 20 | 84.6153846 |
| MIF SLPI TIMP1 SCC OPN AMBP GRP DEFA1 | 8 | 0.79 | 72 | 0 | | Cyfra MIF TIMP1 ENO2 TFPI MMP2 Ca19-9 CA242 CAIX | 9 | 0.8769231 | 20 | 84.6153846 |
| MIF SLPI SCC CEACAM5 OPN AMBP Ca19-9 DEFA1 | 8 | 0.79 | 26 | 20 | | Cyfra MIF TIMP1 ENO2 CEACAM5 MMP2 Ca19-9 CA242 CAIX | 9 | 0.8769231 | 20 | 84.6153846 |
| Cyfra SLPI SCC CEACAM5 OPN Ca19-9 Ca72-4 GRP DEFA1 | 9 | 0.79 | 31 | 40 | | Cyfra MIF TIMP1 ENO2 MMP2 AMBP Ca19-9 CA242 CAIX | 9 | 0.8769231 | 20 | 84.6153846 |
| Cyfra MIF SLPI TIMP1 SCC TFPI OPN Ca19-9 Ca72-4 DEFA1 | 10 | 0.79 | 72 | 0 | | Cyfra SLPI TIMP1 SCC ENO2 MMP2 Ca19-9 CA242 CAIX | 9 | 0.8769231 | 20 | 84.6153846 |
| MIF SLPI TIMP1 SCC TFPI OPN Ca72-4 CA242 GRP DEFA1 | 10 | 0.79 | 72 | 0 | | Cyfra SLPI TIMP1 ENO2 TFPI MMP2 Ca 19-9 CA242 CAIX | 9 | 0.8769231 | 20 | 84.6153846 |
| MIF SLPI TIMP1 SCC CEACAM5 OPN AMBP Ca72-4 CA242 DEFA1 | 10 | 0.79 | 72 | 0 | | Cyfra SLPI TIMD1 ENO2 CEACAM5 MMP2 Ca19-9 CA242 CAIX | 9 | 0.8769231 | 20 | 84.6153846 |
| MIF SLPI SCC TFPI CEACAM5 OPN Ca72-4 | 7 | 0.79 | 64 | 20 | | Cyfra SLPI TIMP1 ENO2 MMP2 AMBP Ca19-9 CA242 CAIX | 9 | 0.8769231 | 20 | 84.6153846 |
| SLPI SCC TFPI CEACAM5 OPN GRP DEFA1 | 7 | 0.79 | 67 | 40 | | MIF TIMP1 SCC ENO2 MMP2 AMBP Ca19-9 CAIX GRP | 9 | 0.8769231 | 0 | 84.6153846 |
| SLPI SCC OPN Ca19-9 CAIX GRP DEFA1 | 7 | 0.79 | 67 | 20 | | MIF TIMP1 SCC ENO2 MMP2 AMBP CA242 CAIX GRP | 9 | 0.8769231 | 0 | 84.6153846 |
| MIF SLPI SCC TFPI CEACAM5 OPN Ca19-9 DEFA1 | 8 | 0.79 | 23 | 60 | | MIF TIMP1 ENO2 TFPI MMP2 AM8P Ca19-9 CAIX GRP | 9 | 0.8769231 | 0 | 84.6153846 |
| SLDI TIMP1 SCC OPN Ca19-9 Ca72-4 CA242 DEFA1 | 8 | 0.79 | 56 | 20 | | MIF TIMP1 ENO2 TFPI MMP2 AMBP CA242 CAIX GRP | 9 | 0.8769231 | 0 | 84.6153846 |
| MIF SLPI TIMP1 SCC TFPI CEACAM5 OPN | 7 | 0.79 | 59 | 20 | | MIF TIMP1 ENO2 CEACAM5 MMP2 AMBP Ca19-9 CAIX GRP | 9 | 0.8769231 | 0 | 84.6153846 |
| MIF SCC TFPI CEACAM5 OPN GRP DEFA1 | 7 | 0.79 | 59 | 20 | | MIF TIMP1 ENO2 CEACAM5 MMP2 AMBP CA242 CAIX GRP | 9 | 0.8769231 | 0 | 84.6153846 |
| SLPI SCC OPN Ca19-9 Ca72-4 CA242 DEFA1 | 7 | 0.79 | 59 | 40 | | Cyfra MIF SLPI TIMP1 SCC ENO2 MMP2 Ca19-9 CA242 CAIX | 10 | 0.8769231 | 20 | 84.6153846 |
| SLPI SCC TFPI CEACAM5 OPN Ca19-9 CA242 DEFA1 | 8 | 0.79 | 59 | 20 | | Cyfra MIF SLPI TIMP1 ENO2 TFPI MMP2 Ca19-9 CA242 CAIX | 10 | 0.8769231 | 20 | 84.6153846 |
| MIF SLPI TIMP1 SCC TFPI OPN Ca19-9 Ca72-4 DEFA1 | 9 | 0.79 | 59 | 40 | | Cyfra MIF SLPI TIMP1 ENO2 CEACAM5 MMP2 Ca19-9 CA242 CAIX | 10 | 0.8769231 | 20 | 84.6153846 |
| MIF SLPI SCCTFPI OPN AMBP Ca72-4 GRP DEFA1 | 9 | 0.79 | 62 | 40 | | Cyfra MIF SLPI TIMP1 ENO2 MMP2 AMBP Ca19-9 CA242 CAIX | 10 | 0.8769231 | 20 | 84.6153846 |
| MIF SLPI SCC TFPI CEACAM5 OPN Ca19-9CA242 GRP DEFA1 | 10 | 0.79 | 59 | 0 | | MIF SLPI TIMP1 SCC ENO2 MMP2 AMBP Ca19-9 CAIX GRP | 10 | 0.8769231 | 0 | 84.6153846 |
| MIF SLPI OPN Ca72-4 DEFA1 | 5 | 0.79 | 54 | 40 | | MIF SLPI TIMP1 SCC ENO2 MMP2 AM8P CA242 CAIX GRP | 10 | 0.8769231 | 0 | 84.6153846 |
| SLPI CEACAM5 OPN GRP DEFA1 | S | 0.79 | 56 | 20 | | MIF SLPI TIMP1 ENO2 TFPI MMP2 AMBP Ca19-9 CAIX GRP | 10 | 0.8769231 | 0 | 84.6153846 |
| MIF SLPI TIMP1 SCC CEACAM5 OPN Ca 19-9 | 7 | 0.79 | 56 | 20 | | MIF SLPI TIMP1 ENO2 TFPI MMP2 AMBP CA242 CAIX GRP | 10 | 0.8769231 | 0 | 84.6153846 |
| MIF SLPI SCC OPN Ca19-9 C72-4 GRP | 7 | 0.79 | 54 | 40 | | MIF SLPI TIMP1 ENO2 CEACAM5 MMP2 AMBP Ca19-9 CAIX GRP | 10 | 0.8769231 | 0 | 84.6153846 |
| Cyfra MIF SLPI CEACAM5 OPN Ca72-4 DEFA1 | 7 | 0.78 | 54 | 0 | | MIF SLPI TIMP1 ENO2 CEACAM5 MMP2 AMBP CA242 CAIX GRP | 10 | 0.8769231 | 0 | 84.6153846 |
| SLPI OPN GRP | 3 | 0.78 | 44 | 0 | | Cyfra SCC ENO2 TFPI MMP2 CAIX GRP | 7 | 0.8769231 | 40 | 79.4871795 |
| MIF SCC TFPI OPN GRP | 5 | 0.78 | 44 | 0 | | Cyfra SCC ENO2 CEACAM5 MMP2 CAIX GRP | 7 | 0.8769231 | 40 | 79.4871795 |
| MIF SLPI SCC OPN AMBP Ca72-4 GRP | 7 | 0.78 | 49 | 20 | | Cyfra ENO2 TFPI CEACAM5 MMP2 CAIX GRP | 7 | 0.8769231 | 40 | 79.4871795 |
| SLPI SCC OPN Ca72-4 CAIX GRP DEFA1 | 7 | 0.78 | 51 | 20 | | Cyfra SLPI SCC ENO2 TFPI MMP2 CAIX GRP | 8 | 0.8769231 | 40 | 79.4871795 |
| MIF SLPI TIMP1 SCC TFPI OPN AMBP CA242 DEFA1 | 9 | 0.78 | 64 | 0 | | Cyfra SLPI SCC ENO2 CEACAM5 MMP2 CAIX GRP | 8 | 0.8769231 | 40 | 79.4871795 |
| SLPI TIMP1 SCC CEACAM5 OPN Ca19-9CA242 GRP DEFA1 | 9 | 0.78 | 64 | 0 | | Cyfra SLPI ENO2 TFPI CEACAM5 MMP2 CAIX GRP | 8 | 0.8769231 | 40 | 79.4871795 |
| SLPI TIMP1 SCC OPN AMBP Ca19-9 CA242 GRP DEFA1 | 9 | 0.78 | 64 | 0 | | Cyfra SCC ENO2 TFPI MMP2 Ca19-9 CAIX GRP | 8 | 0.8769231 | 40 | 79.4871795 |
| Cyfra SLPI TIMP1 SCC OPN Ca19-9 Ca72-4 GRP DEFA1 | 9 | 0.78 | 64 | 0 | | Cyfra SCC ENO2 TFPI MMP2 CA242 CAIX GRP | 8 | 0.8769231 | 40 | 79.4871795 |
| MIF SLPI SCC OPN AMBP Ca72-4 CA242 GRP DEFA1 | 9 | 0.78 | 59 | 20 | | Cyfra SCC ENO2 CEACAM5 MMP2 Ca19-9 CAIX GRP | 8 | 0.8769231 | 40 | 79.4871795 |
| SLPI TIMP1 SCC CEACAM5 OPN AMBP Ca72-4 CA242 DEFA1 | 9 | 0.78 | 59 | 20 | | Cyfra SCC ENO2 CEACAM5 MMP2 CA242 CAIX GRP | 8 | 0.8769231 | 40 | 79.4871795 |
| SCC OPN AMBP | 3 | 0.78 | 33 | 20 | | Cyfra ENO2 TFPI CEACAM5 MMP2 Ca19-9 CAIX GRP | 8 | 0.8769231 | 40 | 79.4871795 |
| SLPI SCC CEACAM5 OPN Ca19-9 CA242 GRP | 7 | 0.78 | 33 | 20 | | Cyfra ENO2 TFPI CEACAM5 MMP2 CA242 CAIX GRP | 8 | 0.8769231 | 40 | 79.4871795 |
| Cyfra SLPI TIMP1 SCC OPN AMBP Ca72-4 DEFA1 | 8 | 0.78 | 77 | 0 | | SCC ENO2 TFPI CEACAM5 MMP2 Ca19-9 CAIX GRP | 8 | 0.8769231 | 40 | 79.4871795 |
| Cyfra SLPI SCC TFPI CEACAM5 OPN CA242 DEFA1 | 8 | 0.78 | 49 | 20 | | SCC ENO2 TFPI CEACAM5 MMP2 CA242 CAIX GRP | 8 | 0.8769231 | 40 | 79.4871795 |
| Cyfra SLPI SCC CEACAM5 OPN AMBP CA242 DEFA1 | 8 | 0.78 | S1 | 20 | | Cyfra SLPI SCC ENO2 TFPI MMP2 Ca19-9 CAIX GRP | 9 | 0.8769231 | 40 | 79.4871795 |
| SLPI TIMP1 SCC TFPI CEACAM5 OPN Ca19-9 DEFA1 | 8 | 0.78 | 49 | 20 | | Cyfra SLPI SCC ENO2 TFPI MMP2 CA242 CAIX GRP | 9 | 0.8769231 | 40 | 79.4871795 |
| Cyfra MIF SLPI TIMP1 SCC CEACAM5 OPN AMBP CA242 DEFA1 | 10 | 0.78 | 77 | 0 | | Cyfra SLPI SCC ENO2 CEACAM5 MMP2 Ca19-9 CAIX GRP | 9 | 0.8769231 | 40 | 79.4871795 |
| Cyfra MIF SLPI SCC TFPI CEACAM5 OPN Ca72-4 CA242 DEFA1 | 10 | 0.78 | 41 | 0 | | Cyfra SLPI SCC ENO2 CEACAM5 MMP2 CA242 CAIX GRP | 9 | 0.8769231 | 40 | 79.4871795 |
| MIF SLPI TIMP1 SCC TFPI CEACAM5 OPN Ca19-9 Ca72-4 DEFA1 | 10 | 0.78 | 49 | 40 | | Cyfra SLPI ENO2 TFPI CEACAM5 MMP2 Ca19-9 CAIX GRP | 9 | 0.8769231 | 40 | 79.4871795 |
| MIF SLPI TIMP1 SCC TFPI OPN Ca19-9 CA242 GRP DEFA1 | 10 | 0.78 | 77 | 0 | | Cyfra SLPI ENO2 TFPI CEACAM5 MMP2 CA242 CAIX GRP | 9 | 0.8769231 | 40 | 79.4871795 |
| SLPI CEACAM5 OPN CA242 | 4 | 0.78 | 74 | 20 | | Cyfra SCC ENO2 TFPI MMP2 Ca19-9 CA242 CAIX GRP | 9 | 0.8769231 | 40 | 79.4871795 |
| Cyfra SLPI CEACAM5 OPN CA242 DEFA1 | 6 | 0.78 | 74 | 10 | | Cyfra SCC ENO2 CEACAM5 MMP2 Ca19-9 CA242 CAIX GRP | 9 | 0.8769231 | 40 | 79.4871795 |
| MIF SLPI SCC OPN Ca19-9 CA242 | 6 | 0.78 | 49 | 40 | | Cyfra ENO2 TFPI CEACAM5 MMP2 Ca19-9 CA242 CAIX GRP | 9 | 0.8769231 | 40 | 79.4871795 |
| SLPI CEACAM5 OPN CA242 GRP DEFA1 | 6 | 0.78 | 74 | 20 | | SLPI SCC ENO2 TFPI CEACAM5 MMP2 Ca19-9 CAIX GRP | 9 | 0.8769231 | 40 | 79.4871795 |
| MIF SLPI TIMP1 SCC OPN Ca19-9 Ca72-4 | 7 | 0.78 | 18 | 40 | | SLPI SCC ENO2 TFPI CEACAM5 MMP2 CA242 CAIX GRP | 9 | 0.8769231 | 40 | 79.4871795 |
| Cyfra MIF SLPI TIMP1 SCC CEACAM5 OPN Ca19-9 | 8 | 0.78 | 74 | 20 | | SCC ENO2 TFPI CEACAM5 MMP2 Ca19-9 CA242 CAIX GRP | 9 | 0.8769231 | 40 | 79.4871795 |
| Cyfra SLPI TIMP1 SCC OPN AMBP CA242 DEFA1 | 8 | 0.78 | 74 | 0 | | Cyfra MIF TIMP1 SCC ENO2 MMP2 AMBP Ca19-9 CAIX GRP | 10 | 0.8769231 | 20 | 82.0512821 |
| MIF SLPI SCC TFPI OPN AMBP GRP DEFA1 | 8 | 0.78 | 51 | 40 | | Cyfra MIF TIMP1 SCC ENO2 MMP2 AMBP CA242 CAIX GRP | 10 | 0.8769231 | 20 | 82.0512821 |
| Cyfra MIF SLPI SCC CEACAM5 OPN Ca19-9 Ca72-4 GRP | 9 | 0.78 | 26 | 20 | | Cyfra MIF TIMP1 ENO2 TFPI MMP2 AMBP Ca19-9 CAIX GRP | 10 | 0.8769231 | 20 | 82.0512821 |
| MIF SLPI SCC CEACAM5 OPN AMBP | 6 | 0.78 | 38 | 40 | | Cyfra MIF TIMP1 ENO2 TFPI MMP2 AMBP CA242 CAIX GRP | 10 | 0.8769231 | 20 | 82.0512821 |
| MIF SLPI SCC CEACAM5 OPN Ca19-9 Ca72-4 GRP | 8 | 0.78 | 38 | 40 | | Cyfra MIF TIMP1 ENO2 CEACAM5 MMP2 AMBP Ca19-9 CAIX GRP | 10 | 0.8769231 | 20 | 82.0512821 |
| SLPI SCC CEACAM5 OPN AMBP Ca72-4 GRP DEFA1 | 8 | 0.78 | 44 | 40 | | Cyfra MIF TIMP1 ENO2 CEACAMS MMP2 AMBP CA242 CAIX GRP | 10 | 0.8769231 | 20 | 82.0512821 |
| Cyfra MIF SLPI SCC CEACAM5 OPN AMBP Ca72-4 CA242 DEFA1 | 10 | 0.78 | 51 | 0 | | Cyfra SLPI SCC ENO2 TFPI MMP2 Ca19-9 CA242 CAIX GRP | 10 | 0.8769231 | 40 | 79.4871795 |
| MIF SLPI OPN Ca72-4 GRP DEFA1 | 6 | 0.78 | 69 | 0 | | Cyfra SLPI SCC ENO2 CEACAM5 MMP2 Ca19-9 CA242 CAIX GRP | 10 | 0.8769231 | 40 | 79.4871795 |
| SLPI TIMP1 SCC TFPI OPN CA242 | 6 | 0.78 | 69 | 0 | | Cyfra SLPI ENO2 TFPI CEACAM5 MMP2 Ca19-9 CA242 CAIX GRP | 10 | 0.8769231 | 40 | 79.4871795 |
| Cyfra MIF SLPI TIMP1 SCC CEACAM5 OPN Ca72-4 | 8 | 0.78 | 69 | 0 | | SLPI SCC ENO2 TFPI CEACAM5 MMP2 Ca19-9 CA242 CAIX GRP | 10 | 0.8769231 | 40 | 79.4871795 |
| SLPI TIMP1 SCC TFPI OPN CA242 GRP DEFA1 | 8 | 0.78 | 69 | 0 | | Cyfra MIF SLPI TIMP1 SCC ENO2 MMP2 AMBP Ca19-9 CAIX GRP | 11 | 0.8769231 | 20 | 82.0512821 |
| Cyfra MIF SLPI TIMP1 SCC CEACAM5 OPN Ca72-4 CA242 DEFA1 | 10 | 0.78 | 69 | 0 | | Cyfra MIF SLPI TIMP1 SCC ENO2 MMP2 AMBP CA242 CAIX GRP | 11 | 0.8769231 | 20 | 82.0512821 |
| SLPI TFPI OPN DEFA1 | 4 | 0.78 | 36 | 60 | | Cyfra MIF SLPI TIMP1 ENO2 TFPI MMP2 AMBP Ca19-9 CAIX GRP | 11 | 0.8769231 | 20 | 82.0512821 |
| MIF SLPI CEACAM5 OPN Ca72-4 DEFA1 | 6 | 0.78 | 33 | 60 | | Cyfra MIF SLPI TIMP1 ENO2 TFPI MMP2 AMBP CA242 CAIX GRP | 11 | 0.8769231 | 20 | 82.0512821 |
| SLPI TIMP1 SCC OPN Ca19-9 CA242 | 6 | 0.78 | 46 | 0 | | Cyfra MIF SLPI TIMP1 ENO2 CEACAM5 MMP2 AMBP Ca19-9 CAIX GRP | 11 | 0.8769231 | 20 | 82.0571821 |
| Cyfra SLPI TIMP1 SCC CEACAM5 OPN Ca19-9 GRP DEFA1 | 9 | 0.78 | 67 | 20 | | Cyfra MIF SLPI TIMP1 ENO2 CEACAM5 MMP2 AM8P CA242 CAIX GRP | 11 | 0.8769231 | 20 | 82.0512821 |
| MIF SLPI TIMP1 SCC CEACAM5 OPN AMBP GRP DEFA1 | 9 | 0.78 | 64 | 20 | | Cyfra MIF TIMP1 SCC ENO2 MMP2 AMBP Ca19-9 CA242 CAIX GRP | 11 | 0.8769231 | 20 | 82.0512821 |
| MIF SLPI SCC TFPI CEACAM5 OPN AM8P CA242 DEFA1 | 9 | 0.78 | 49 | 40 | | Cyfra MIF TIMP1 ENO2 TFPI MMP2 AM8P Ca19-9 CA242 CAIX GRP | 11 | 0.8769231 | 20 | 82.0512821 |
| MIF SLPI SCC TFPI CEACAM5 OPN Ca19-9 CA242 DEFA1 | 9 | 0.78 | 44 | 40 | | Cyfra MIF TIMP1 ENO2 CEACAM5 MMP2 AMBP Ca19-9 CA242 CAIX GRP | 11 | 0.8769231 | 20 | 82.0512821 |
| MIF SLPI OPN CA242 DEFA1 | S | 0.78 | 64 | 40 | | MIF TIMP1 SCC ENO2 TFPI MMP2 AMBP Ca19-9 CA242 CAIX GRP | 11 | 0.8769231 | 20 | 82.0512821 |
| SLPI OPN AMBP DEFA1 | 4 | 0.78 | 23 | 60 | | MIF TIMP1 SCC ENO2 CEACAM5 MMP2 AMBP Ca19-9 CA242 CAIX GRP | 11 | 0.8769231 | 20 | 82.0512821 |
| Cyfra SLPI SCC OPN GRP | 5 | 0.78 | 59 | 40 | | MIF TIMP1 ENO2 TFPI CEACAM5 MMP2 AMBP Ca19-9 CA242 CAIX GRP | 11 | 0.8769231 | 20 | 82.0512821 |
| SLPI TIMP1 SCC OPN Ca19-9 Ca72-4 | 6 | 0.78 | 33 | 60 | | Cyfra MIF SLPI TIMP1 SCC ENO2 MMP2 AMBP Ca19-9 CA242 CAIX GRP | 12 | 0.8769231 | 20 | 82.0512821 |
| SCC TFPI OPN Ca19-9 GRP DEFA1 | 6 | 0.78 | 38 | 60 | | Cyfra MIF SLPI TIMP1 ENO2 TFPI MMP2 AMBP Ca19-9 CA242 CAIX GRP | 12 | 0.8769231 | 20 | 82.0512821 |
| SLPI SCC TFPI OPN AMBP Ca72-4 DEFA1 | 7 | 0.78 | 59 | 40 | | Cyfra MIF SLPI TIMP1 ENO2 CEACAM5 MMP2 AMBP Ca19-9 CA242 CAIX GRP | 12 | 0.8769231 | 20 | 82.0512821 |
| SLPI SCC CEACAM5 OPN AMBP Ca19-9 DEFA1 | 7 | 0.78 | 41 | 40 | | MIF SLPI TIMP1 SCC ENO2 TFPI MMP2 AMBP Ca19-9CA242 CAIX GRP | 12 | 0.8769231 | 20 | 82.0512821 |
| Cyfra MIF SLPI TIMP1 SCC TFPI CEACAM5 OPN DEFA1 | 9 | 0.78 | 62 | 20 | | MIF SLPI TIMP1 SCC ENO2 CEACAM5 MMP2 AMBP Ca19-9 CA242 CAIX GRP | 12 | 0.8769231 | 20 | 82.0512821 |
| Cyfra MIF SLPI SCC OPN AMBP Ca19-9 Ca72-4 DEFA1 | 9 | 0.78 | 62 | 40 | | MIF SLPI TIMP1 ENO2 TFPI CEACAM5 MMP2 AMBP Ca19-9 CA242 CAIX GRP | 12 | 0.8769231 | 20 | 82.0512821 |
| SLPI TIMP1 SCC CEACAM5 OPN AMBP Ca72-4 GRP DEFA1 | 9 | 0.78 | S9 | 40 | | MIF SCC ENO2 MMP2 CAIX GRP | 6 | 0.8717949 | 20 | 84.6153846 |
| SLPI SCC TFPI CEACAM5 OPN Ca19-9 CA242 GRP DEFA1 | 9 | 0.78 | 51 | 40 | | MIF ENO2 TFPI MMP2 CAIX GRP | 6 | 0.8717949 | 20 | 84.6153846 |
| SLPI SCC OPN AMBP GRP | 5 | 0.78 | 56 | 40 | | MIF ENO2 CEACAM5 MMP2 CAIX GRP | 6 | 0.8717949 | 20 | 84.6153846 |
| SLPI SCC OPN Ca72-4 CA242 | 5 | 0.78 | 54 | 60 | | TIMP1 SCC ENO2 MMP2 CAIX GRP | 6 | 0.8717949 | 20 | 84.6153846 |
| SCC CEACAM5 OPN AMBP DEFA1 | 5 | 0.78 | 54 | 40 | | TIMP1 ENO2 TFPI MMP2 CAIX GRP | 6 | 0.8717949 | 20 | 84.6153846 |
| MIF SCC TFPI CEACAM5 OPN Ca72-4 DEFA1 | 7 | 0.78 | 56 | 40 | | TIMP1 ENO2 CEACAM5 MMP2 CAIX GRP | 6 | 0.8717949 | 20 | 84.6153846 |
| SLPI TIMP1 SCC OPN CAIX | 5 | 0.78 | 41 | 0 | | SCC ENO2 MMP2 AMBP CAIX GRP | 6 | 0.8717949 | 20 | 84.6153846 |
| TIMP1 SCC OPN GRP DEFA1 | 5 | 0.78 | 51 | 0 | | ENO2 TFPI MMP2 AMBP CAIX GRP | 6 | 0.8717949 | 20 | 84.6153846 |
| Mif SLPI CEACAM5 OPN Ca72-4 GRP DEFA1 | 7 | 0.78 | 62 | 0 | | ENO2 CEACAM5 MMP2 AMBP CAIX GRP | 6 | 0.8717949 | 20 | 84.6153846 |
| MIF TIMP1 SCC TFPI OPN Ca72-4 DEFA1 | 7 | 0.78 | 54 | 0 | | Cyfra MIF SCC ENO2 MMP2 Ca19-9 CAIX | 7 | 0.8717949 | 20 | 84.6153846 |
| Cyfra MIF SLPI TIMP1 SCC TFPI OPN CA242 DEFA1 | 9 | 0.78 | 62 | 0 | | Cyfra MIF SCC ENO2 MMP2 CA242 CAlX | 7 | 0.8717949 | 20 | 84.6153846 |
| Cyfra MIF SLPI TIMP1 SCC OPN AMBP Ca19-9 DEFA1 | 9 | 0.78 | 62 | 0 | | Cyfra MIF ENO2 TFPI MMP2 Ca19-9 CAIX | 7 | 0.8717949 | 20 | 84.6153846 |
| TIMP1 SCC OPN Ca19-9 DEFA1 | 5 | 0.78 | 56 | 20 | | Cyfra MIF ENO2 TFPI MMP2 CA242 CAIX | 7 | 0.8717949 | 20 | 84.6153846 |
| Cyfra MIF SLPI SCC OPN AMBP Ca72-4 CA242 DEFA1 | 9 | 0.78 | 62 | 0 | | Cyfra MIF ENO2 CEACAM5 MMP2 Ca19-9 CAIX | 7 | 0.8717949 | 20 | 84.6153946 |
| MIF SLPI TIMP1 SCC OPN Ca19-9 Ca72-4 CA242 DEFA1 | 9 | 0.78 | 46 | 20 | | Cyfra MIF ENO2 CEACAM5 MMP2 CA242 CAIX | 7 | 0.8717949 | 20 | 84.6153846 |
| SLPI TIMP1 SCC TFPI OPN Ca19-9 Ca72-4 GRP DEFA1 | 9 | 0.78 | 54 | 20 | | Cyfra MIF ENO2 MMP2 AMBP Ca19-9 CAIX | 7 | 0.8717949 | 20 | 84.6153846 |
| Cyfra SCC CEACAM5 OPN Ca72-4 DEFA1 | 6 | 0.78 | 21 | 0 | | Cyfra MIF ENO2 MMP2 AMBP CA242 CAIX | 7 | 0.8717949 | 20 | 84.6153846 |
| MIF SLPI CEACAM5 OPN CA242 DEFA1 | 6 | 0.78 | 46 | 0 | | Cyfra TIMP1 SCC ENO2 MMP2 Ca19-9 CAIX | 7 | 0.8717949 | 20 | 84.6153846 |
| MIF SLPI TIMP1 SCC OPN AMBP Ca72-4 CA242 DEFA1 | 9 | 0.78 | 67 | 0 | | Cyfra TIMP1 SCC ENO2 MMP2 CA242 CAIX | 7 | 0.8717949 | 20 | 84.6153846 |
| Cyfra SLPI SCC CEACAM5 OPN Ca19-9 Ca72-4 CA242 GRP DEFA1 | 10 | 0.78 | 31 | 0 | | Cyfra TIMP1 ENO2 TFPI MMP2 Ca19-9 CAIX | 7 | 0.8717949 | 20 | 84.6153846 |
| MIF SlPI TIMP1 SCC TFPI CEACAM5 OPN Ca19-9 CA242 DEFA1 | 10 | 0.78 | 56 | 0 | | Cyfra TMP1 ENO2 TFPI MMP2 CA242 CAIX | 7 | 0.8717949 | 20 | 84.6153846 |
| MIF SlPI TIMP1 SCC CEACAM5 OPN AMBP Ca19-9 GRP DEFA1 | 10 | 0.78 | 56 | 0 | | Cyfra TIMP1 ENO2 CEACAM5 MMP2 Ca19-9 CAIX | 7 | 0.8717949 | 20 | 84.6153846 |
| SLPI SCC CEACAM5 OPN Ca19-9 CA242 | 6 | 0.78 | 49 | 40 | | Cyfra TIMP1 ENO2 CEACAM5 MMP2 CA242 CAIX | 7 | 0.8717949 | 20 | 84.6153846 |
| Cyfra MIF SLPI TIMP1 SCC CEACAM5 OPN CA242 | 8 | 0.78 | 74 | 0 | | Cyfra TIMP1 ENO2 MMP2 AMBP Ca19-9 CAIX | 7 | 0.8717949 | 20 | 84.6153846 |
| MIF SLPI SCC TFPI CEACAM5 OPN Ca72-4 CA242 | 8 | 0.78 | 44 | 20 | | Cyfra TIMP1 ENO2 MMP2 AMBP CA242 CAIX | 7 | 0.8717949 | 20 | 84.6153846 |
| Cyfra MIF SLPI TIMP1 SCC TFPI CEACAM5 OPN CA242 DEFA1 | 10 | 0.78 | 74 | 0 | | MIF SLPI SCC ENO2 MMP2 CAIX GRP | 7 | 0.8717949 | 20 | 84.6153846 |
| MIF SLPI SCC OPN AMBP Ca19-9 | 6 | 0.78 | 41 | 40 | | MIF SLPI ENO2 TFPI MMP2 CAIX GRP | 7 | 0.8717949 | 20 | 84.6153846 |
| MIF TIMP1 SCC TFPI OPN DEFA1 | 6 | 0.78 | 72 | 0 | | MIF SLPI ENO2 CEACAM5 MMP2 CAIX GRP | 7 | 0.8717949 | 20 | 84.6153846 |
| TIMP1 SCC CEACAM5 OPN Ca19-9 DEFA1 | 6 | 0.78 | 72 | 0 | | SLPI TIMP1 SCC ENO2 MMP2 CAIX GRP | 7 | 0.8717949 | 20 | 84.6153846 |
| Cyfra MIF SLPI TIMP1 SCC OPN AMBP DEFA1 | 8 | 0.78 | 72 | 0 | | SLPI TIMP1 ENO2 TFPI MMP2 CAIX GRP | 7 | 0.8717949 | 20 | 84.6153846 |
| Cyfra MIF SLPI SCC CEACAM5 OPN Ca19-9 GRP | 8 | 0.78 | 41 | 40 | | SLPI TIMPI ENO2 CEACAM5 MMP2 CAIX GRP | 7 | 0.8717949 | 20 | 84.6153846 |
| Cyfra SLPI SCC TFP CEACAM5 OPN Ca72-4 DEFA1 | 8 | 0.78 | 46 | 40 | | SLPI SCC ENO2 MMP2 AMBP CAIX GRP | 7 | 0.8717949 | 20 | 84.6153846 |
| Cyfra SLPI SCC CEACAM5 OPN AMBP Ca72-4 DEFA1 | 8 | 0.78 | 41 | 40 | | SIDI ENO2 TFPI MMP2 AMBP CAIX GRP | 7 | 0.8717949 | 20 | 84.6153846 |
| SLPI TIMP1 SCC TFPI OPN AMBD CA242 DEFA1 | 8 | 0.78 | 72 | 0 | | SLPI ENO2 CEACAM5 MMP2 AMBP CAIX GRP | 7 | 0.8717949 | 20 | 84.6153846 |
| Cyfra MIF SLPI SCC OPN AMBP Ca19-9 CA242 DEFA1 | 9 | 0.78 | 69 | 0 | | Cyfra MIF SLPI SCC ENO2 MMP2 Ca19-9 CAIX | 8 | 0.8717949 | 20 | 84.6153846 |
| Cyfra SLPI SCC CEACAM5 OPN Ca72-4 CA242 GRP DEFA1 | 9 | 0.78 | 36 | 40 | | Cyfra MIF SLPI SCC ENO2 MMP2 CA242 CAIX | 8 | 0.8717949 | 20 | 84.6153846 |
| SLPI SCC TFPI OPN Ca19-9Ca72-4 CA242 GRP DEFA1 | 9 | 0.78 | 41 | 40 | | Cyfra MIF SLPI ENO2 TFPI MMP2 Ca19-9 CAIX | 8 | 0.8717949 | 20 | 84.6153846 |
| SLPI SCC CEACAM5 OPN AMBP Ca72-4 CA242 GRP DEFA1 | 9 | 0.78 | 36 | 40 | | Cyfra MIF SLPI ENO2 TFPI MMP2 CA242 CAIX | 8 | 0.8717949 | 20 | 84.6153846 |
| Cyfra MIF SLPI TIMP1 SCC TFPI OPN AMBP Ca72-4 DEFA1 | 10 | 0.78 | 72 | 0 | | Cyfra MIF SLPI ENO2 CEACAM5 MMP2 Ca19-9 CAIX | 8 | 0.8717949 | 20 | 84.6153846 |
| Cyfra MIF SLPI TIMP1 SCC TFPI OPN Ca72-4 CA242 DEFA1 | 10 | 0.78 | 72 | 0 | | Cyfra MIF SLPI ENO2 CEACAM5 MMP2 CA242 CAIX | 8 | 0.8717949 | 20 | 84.6153846 |
| MIF SLPI TIMP1 SCC OPN Ca72-4 CA242 | 7 | 0.78 | 26 | 40 | | Cyfra MIF SLPI ENO2 MMP2 AMBP Ca19-9 CAIX | 8 | . 0.8717949 | 20 | 84.6153846 |
| MIF SCC OPN Ca19-9 CA242 GRP DEFA1 | 7 | 0.78 | 69 | 20 | | Cyfra MIF SLPI ENO2 MMP2 AMBP CA242 CAIX | 8 | 0.8717949 | 20 | 84.6153846 |
| Cyfra SLPI TIMP1 SCC OPN Ca72-4 GRP DEFA1 | 8 | 0.78 | 69 | 20 | | Cyfra SLPI TIMP1 SCC ENO2 MMP2 Ca19-9 CAIX | 8 | 0.8717949 | 20 | 84.6153846 |
| MIF SLPI OPN DEFA1 | 4 | 0.78 | 31 | 40 | | Cyfra SLPI TIMP1 SCC ENO2 MMP2 CA242 CAIX | 8 | 0.8717949 | 20 | 84.6153846 |
| MIF SCC OPN AMBP Ca72-4 DEFA1 | 6 | 0.78 | 67 | 20 | | Cyfra SLPI TIMP1 ENO2 TFPI MMP2 Ca19-9 CAIX | 8 | 0.8717949 | 20 | 84.6153846 |
| SLPI SCC TFPI OPN CA242 GRP | 6 | 0.78 | 38 | 0 | | Cyfra SLPI TIMP1 ENO2 TFPI MMP2 CA242 CAIX | 8 | 0.8717949 | 20 | 84.6153846 |
| TIMP1 SCC TFPI OPN Ca72-4 DEFA1 | 6 | 0.78 | 64 | 0 | | Cyfra SLPI TIMP1 ENO2 CEACAM5 MMP2 Ca19-9 CAIX | 8 | 0.8717949 | 20 | 84.6153846 |
| MIF SCC CEACAM5 OPN AMBP CA242 DEFA1 | 7 | 0.78 | 64 | 0 | | Cyfra SLPI TIMP1 ENO2 CEACAM5 MMP2 CA242 CAIX | 8 | 0.8717949 | 20 | 84.6153846 |
| SLPI SCC TFPI CEACAM5 OPN Ca19-9 DEFA1 | 7 | 0.78 | 36 | 40 | | Cyfra SLPI TIMP1 ENO2 MMP2 AMBP Ca19-9 CAIX | 8 | 0.8717949 | 20 | 84.6153846 |
| MIF SCC TFPI CEACAM5 OPN Ca72-4 GRP DEFA1 | 8 | 0.78 | 38 | 0 | | Cyfra SLPI TIMP1 ENO2 MMP2 AMBP CA242 CAIX | 8 | 0.8717949 | 20 | 84.6153846 |
| MIF SLPI SCC CEACAM5 OPN AMBP Ca19-9 CA242 DEFA1 | 9 | 0.78 | 51 | 0 | | MIF SCC ENO2 TFPI MMP2 Ca19-9 CA242 CAIX | 8 | 0.8717949 | 20 | 84.6153846 |
| MIF SLPI SCC CEACAM5 OPN AMBP Ca19-9 GRP DEFA1 | 9 | 0.78 | 38 | 40 | | MIF SCC ENO2 CEACAM5 MMP2 Ca19-9 CA242 CAIX | 8 | 0.8717949 | 20 | 84.6153846 |
| SLPI TIMP1 SCC OPN Ca19-9 Ca72-4 CA242 GRP DEFA1 | 9 | 0.78 | 67 | 0 | | MIF SCC ENO2 MMP2 AMBP Ca19-9 CA242 CAIX | 8 | 0.8717949 | 20 | 84.6153846 |
| Cyfra MIF SLPI SCC TFPI OPN Ca19-9 Ca72-4 GRP DEFA1 | 10 | 0.78 | 46 | 0 | | MIF ENO2 TFPI CEACAM5 MMP2 Ca19-9 CA242 CAIX | 8 | 0.8717949 | 20 | 84.6153846 |
| SLPI TIMP1 SCC TFPI CEACAM5 OPN Ca72-4 CA242 GRP DEFA1 | 10 | 0.78 | 49 | 0 | | MIF ENO2 TFPI MMP2 AMBP Ca19-9 CA242 CAIX | 8 | 0.8717949 | 20 | 84.6153846 |
| MIF SLPI TIMP1 SCC TFPI CEACAM5 OPN Ca19-9 Ca72-4 CA242 DEFA1 | 11 | 0.78 | 67 | 40 | | MIF ENO2 CEACAM5 MMP2 AMBP Ca19-9 CA242 CAIX | 8 | 0.8717949 | 20 | 84.6153846 |
| SLPI SCC Ca19-9 DEFA1 | 4 | 0.78 | 33 | 60 | | TIMP1 SCC ENO2 TFPI MMP2 Ca19-9 CA242 CAIX | 8 | 0.8717949 | 20 | 84.6153846 |
| SCC OPN CA242 GRP DEFA1 | 5 | 0.78 | 59 | 40 | | TIMP1 SCC ENO2 CEACAM5 MMP2 Ca19-9 CA242 CAIX | 8 | 0.8717949 | 20 | 84.6153846 |
| SLPI TIMP1 SCC TFPI OPN GRP | 6 | 0.78 | 36 | 60 | | TIMP1 SCC ENO2 MMP2 AMBP Ca19-9 CA242 CAIX | 8 | 0.8717949 | 20 | 84.6153846 |
| SCC CEACAM5 OPN Ca72-4 CA242 DEFA1 | 6 | 0.78 | 33 | 60 | | TIMP1 ENO2 TFPI CEACAM5 MMP2 Ca19-9 CA242 CAIX | 8 | 0.8717949 | 20 | 84.6153846 |
| MIF SCC TFPI OPN Ca19-9 GRP DEFA1 | 7 | 0.78 | 62 | 20 | | TIMP1 ENO2 TFPI MMP2 AMBP Ca19-9 CA242 CAIX | 8 | 0.8717949 | 20 | 84.6153846 |
| SLPI TIMP1 SCC CEACAM5 OPN Ca19-9 GRP | 7 | 0.78 | 59 | 20 | | TIMP1 ENO2 CEACAM5 MMP2 AMBP Ca19-9 CA242 CAIX | 8 | 0.8717949 | 20 | 84.6153846 |
| SLPI SCC CEACAM5 OPN CA242 CAIX DEFA1 | 7 | 0.78 | 62 | 20 | | MIF SLPI SCC ENO2 TFPI MMP2 Ca19-9 CA242 CAIX | 9 | 0.8717949 | 20 | 84.6153846 |
| Cyfra MIF SLPI SCC CEACAM5 OPN Ca72-4 GRP | 8 | 0.78 | 36 | 60 | | MIF SLPI SCC ENO2 CEACAM5 MMP2 Ca19-9 CA242 CAIX | 9 | 0.8717949 | 20 | 84.6153846 |
| MIF SLPI SCC TFPI OPN AMBP Ca19-9 DEFA1 | 8 | 0.78 | 23 | 60 | | MIF SLPI SCC ENO2 MMP2 AMBP Ca19-9 CA242 CAIX | 9 | 0.8717949 | 20 | 84.6153846 |
| MIF SLPI SCC CEACAM5 OPN Ca72-4 CAIX DEFA1 | 8 | 0.78 | 36 | 60 | | MIF SLPI ENO2 TFPI CEACAM5 MMP2 Ca19-9 CA242 CAIX | 9 | 0.8717949 | 20 | 84.6153846 |
| SLPI SCC TFPI CEACAM5 OPN AMBP CA242 DEFA1 | 8 | 0.78 | 62 | 40 | | MIF SLPI ENO2 TFPI MMP2 AMBP Ca19-9 CA242 CAIX | 9 | 0.8717949 | 20 | 84.6153846 |
| Cyfra SLPI TIMP1 SCC CEACAM5 OPN AMBP Ca72-4 DEFA1 | 9 | 0.78 | 62 | 40 | | MIF SLPI ENO2 CEACAM5 MMP2 AMBP Ca19-9 CA242 CAIX | 9 | 0.8717949 | 20 | 84.6153846 |
| MIF SLPI SCC TFPI OPN AMBP Ca72-4 CA242 DEFA1 | 9 | 0.78 | 62 | 0 | | SLPI TIMP1 SCC ENO2 TFPI MMP2 Ca19-9 CA242 CAIX | 9 | 0.8717949 | 20 | 84.6153846 |
| MIF TIMP1 SCC OPN DEFA1 | 5 | 0.78 | 56 | 40 | | SLPI TIMP1 SCC ENO2 CEACAM5 MMP2 Ca19-9 CA242 CAIX | 9 | 0.8717949 | 20 | 84.6153846 |
| SLPI SCC TFPI OPN Ca19-9 | 5 | 0.78 | 56 | 20 | | SLPI TIMP1 SCC ENO2 MMP2 AMBP Ca19-9 CA242 CAIX | 9 | 0.8717949 | 20 | 84.6153846 |
| SCC OPN AMBP Ca19-9 DEFA1 | 5 | 0.78 | 56 | 40 | | SLPI TIMP1 ENO2 TFPI CEACAM5 MMP2 Ca19-9 CA242 CAIX | 9 | 0.8717949 | 20 | 84.6153846 |
| SCC OPN Ca19-9 Ca72-4 DEFA1 | 5 | 0.78 | 54 | 40 | | SIPI TIMP1 ENO2 TFPI MMP2 AMBP Ca19-9 CA242 CAIX | 9 | 0.8711949 | 20 | 84.6153846 |
| Cyfra MIF SLPI SCC CEACAM5 OPN GRP | 7 | 0.78 | 54 | 20 | | SLPI TIMP1 ENO2 CEACAM5 MMP2 AMBP Ca19-9 CA242 CAIX | 9 | 0.8717949 | 20 | 84.6153846 |
| MIF SLPI TIMP1 SCC TFPI OPN Ca19-9 | 7 | 0.78 | 56 | 20 | | Cyfra ENO2 MMP2 CAIX | 4 | 0.8717949 | 0 | 87.1794872 |
| MIF SLPI SCC OPN AMBP Ca19-9 GRP | 7 | 0.78 | 56 | 20 | | Cyfra MIF ENO2 MMP2 CAIX | 5 | 0.8717949 | 0 | 87.1794872 |
| SLPI SCC TFPI OPN Ca19-9 Ca72-4 DEFA1 | 7 | 0.78 | 54 | 40 | | Cyfra SLPI ENO2 MMP2 CAIX | 5 | 0.8717949 | 0 | 87.1794872 |
| SLPI SCC OPN Ca19-9 Ca72-4 CAIX DEFA1 | 7 | 0.78 | 56 | 40 | | Cyfra TIMP1 ENO2 MMP2 CAIX | 5 | 0.8717949 | 0 | 87.1794872 |
| SLPI SCC CEACAM5 OPN AMBP Ca19-9 CA242 DEFA1 | 8 | 0.78 | 56 | 20 | | SCC ENO2 MMP2 Ca19-9 CAIX | 5 | 0.8717949 | 0 | 87.1794872 |
| SLPI SCC CEACAM5 OPN AMBP Ca19-9 GRP DEFA1 | 8 | 0.78 | 56 | 40 | | SCC ENO2 MMP2 CA242 CAIX | 5 | 0.8717949 | 0 | 87.1794872 |
| Cyfra MIF SLPI SCC TFPI OPN Ca19-9 GRP DEFA1 | 9 | 0.78 | 56 | 20 | | ENO2 TFPI MMP2 Ca19-9 CAIX | S | 0.8717949 | 0 | 87.1794872 |
| SLPI TIMP1 SCC TFPI CEACAM5 OPN CA242 GRP | 8 | 0.78 | 49 | 60 | | ENO2 TFPI MMP2 CA242 CAIX | 5 | 0.8717949 | 0 | 87.1794872 |
| Cyfra MIF SCC CEACAM5 OPN | 5 | 0.77 | 49 | 0 | | ENO2 CEACAM5 MMP2 Ca19-9 CAIX | 5 | 0.8717949 | 0 | 87.1794872 |
| MIF SLPI SCC OPN AMBP CA242 GRP | 7 | 0.77 | 59 | 0 | | ENO2 CEACAM5 MMP2 CA242 CAIX | 5 | 0.8717949 | 0 | 87.1794872 |
| Cyfra SLPI TIMP1 SCC OPN AMBP Ca19-9 CA242 DEFA1 | 9 | 0.77 | 59 | 0 | | ENO2 MMP2 AMBP Ca19-9 CAIX | 5 | 0.8717949 | 0 | 87.1794872 |
| CEACAM5 OPN DEFA1 | 3 | 0.77 | 38 | 0 | | ENO2 MMP2 AMBP CA242 CAIX | S | 0.8717949 | 0 | 87.1794872 |
| Cyfra SLPI TIMP1 SCC OPN AMBP Ca19-9 Ca72-4 DEFA1 | 9 | 0.77 | 59 | 0 | | Cyfra MIF SLPI ENO2 MMP2 CAIX | 6 | 0.8717949 | 0 | 87.1794872 |
| MIF SLPI TIMP1 SCC OPN AMBP CA242 GRP DEFA1 | 9 | 0.77 | 69 | 0 | | Cyfra MIF TIMP1 ENO2 MMP2 CAIX | 6 | 0.8717949 | 0 | 87.1794872 |
| SLPI TIMP1 SCC TFPI OPN AMBP Ca72-4 CA242 DEFA1 | 9 | 0.77 | 59 | 0 | | Cyfra SLPI TIMP1 ENO2 MMP2 CAIX | 6 | 0.8717949 | 0 | 87.1794872 |
| TIMP1 SCC OPN | 3 | 0.77 | 36 | 20 | | MIF SCC ENO2 MMP2 Ca19-9 CAIX | 6 | 0.8717949 | 0 | 87.1794872 |
| Cyfra SLPI TIMP1 SCC TFPI OPN DEFA1 | 7 | 0.77 | 59 | 20 | | MIF SCC ENO2 MMP2 CA242 CAIX | 6 | 0.8717949 | 0 | 87.1794872 |
| Cyfra MIF SLPI SCC TFPI OPN Ca72-4 GRP DEFA1 | 9 | 0.77 | 62 | 20 | | MIF ENO2 TFPI MMP2 Ca19-9 CAIX | 6 | 0.8717949 | 0 | 87.1794872 |
| MIF SLPI TIMP1 SCC OPN Ca19-9 Ca72-4 CA242 GRP DEFA1 | 10 | 0.77 | 56 | 20 | | MIF ENO2 TFPI MMP2 CA242 CAIX | 6 | 0.8717949 | 0 | 87.1794872 |
| Cyfra MIF SLPI SCC TFPI CEACAM5 OPN Ca72-4CA242 GRP DEFA1 | 11 | 0.77 | 33 | 0 | | MIF ENO2 CEACAM5 MMP2 Ca19-9 CAIX | 6 | 0.8717949 | 0 | 87.1794872 |
| MIF SLPI OPN Ca19-9 GRP DEFA1 | 6 | 0.77 | 33 | 0 | | MIF ENO2 CEACAM5 MMP2 CA242 CAIX | 6 | 0.8717949 | 0 | 87.1794872 |
| SLPI OPN Ca19-9 CA242 GRP DEFA1 | 6 | 0.77 | 74 | 0 | | MIF ENO2 MMP2 AMBP Ca19-9 CAIX | 6 | 0.8717949 | 0 | 87.1794872 |
| MIF SLPI SCC OPN Ca19-9 CAIX DEFA1 | 7 | 0.77 | 28 | 20 | | MIF ENO2 MMP2 AMBP CA242 CAIX | 6 | 0.8717949 | 0 | 87.1794872 |
| SLPI SCC CEACAM5 OPN Ca19-9 Ca72-4 GRP | 7 | 0.77 | 21 | 20 | | SLPI SCC ENO2 MMP2 Ca19-9 CAIX | 6 | 0.8717949 | 0 | 87.1794872 |
| MIF SLPI TIMP1 SCC OPN AMBP Ca19-9 GRP | 8 | 0.77 | 74 | 0 | | SLPI SCC ENO2 MMP2 CA242 CAIX | 6 | 0.8717949 | 0 | 87.1794872 |
| SIDI TIMP1 SCC CEACAM5 OPN AMBP GRP DEFA1 | 8 | 0.77 | 49 | 20 | | SLPI ENO2 TFPI MMP2 Ca19-9 CAIX | 6 | 0.8717949 | 0 | 87.1794872 |
| SLPI SCC TFPI OPN AMBP CA242 GRP DEFA1 | 8 | 0.77 | 46 | 20 | | SLPI ENO2 TFPI MMP2 CA242 CAIX | 6 | 0.8717949 | 0 | 87.1794872 |
| Cyfra SLPI SCC TFPI CEACAM5 OPN Ca72-4 CA242 DEFA1 | 9 | 0.77 | 33 | 0 | | SLPI ENO2 CEACAM5 MMP2 Ca19-9 CAIX | 6 | 0.8717949 | 0 | 87.1794872 |
| Cyfra MIF SLPI TIMP1 SCC CEACAM5 OPN Ca19-9 GRP DEFA1 | 10 | 0.77 | 74 | 0 | | SLPI ENO2 CEACAM5 MMP2 CA242 CAIX | 6 | 0.8717949 | 0 | 87.1794872 |
| Cyfra MIF SLPI TIMP1 SCC OPN Ca19-9 Ca72-4 GRP DEFA1 | 10 | 0.77 | 74 | 0 | | SLPI ENO2 MMP2 AMBP Ca19-9 CAIX | 6 | 0.8717949 | 0 | 87.1794872 |
| Cyfra MIF SLPI SCC TFPI CEACAM5 OPN Ca72-4 GRP DEFA1 | 10 | 0.77 | 46 | 20 | | SLPI ENO2 MMP2 AMBP CA242 CAIX | 6 | 0.8717949 | 0 | 87.1794872 |
| Cyfra MIF SLPI SCC CEACAM5 OPN Ca19-9 Ca72-4 CA242 DEFA1 | 10 | 0.77 | 49 | 40 | | TIMP1 SCC ENO2 MMP2 Ca19-9 CAIX | 6 | 0.8717949 | 0 | 87.1794872 |
| MIF SLPI TIMP1 SCC TFPI CEACAM5 OPN AMBP Ca72-4 DEFA1 | 10 | 0.77 | 44 | 20 | | TIMP1 SCC ENO2 MMP2 CA242 CAIX | 6 | 0.8717949 | 0 | 87.1794872 |
| MIF SLPI TIMP1 SCC CEACAM5 OPN AMBP Ca19-9 Ca72-4 DEFA1 | 10 | 0.77 | 46 | 20 | | TIMP1 ENO2 TFPI MMP2 Ca19-9 CAIX | 6 | 0.8717949 | 0 | 87.1794872 |
| MIF SLPI TIMP1 SCC CEACAM5 OPN AMBP Ca19-9 CA242 DEFA1 | 10 | 0.77 | 59 | 0 | | TIMP1 ENO2 TFPI MMP2 CA242 CAIX | 6 | 0.8717949 | 0 | 87.1794872 |
| MIF SLPI TIMP1 SCC CEACAM5 OPN Ca19-9 Ca72-4 CA242 DEFA1 | 10 | 0.77 | 54 | 20 | | TIMP1 ENO2 CEACAM5 MMP2 Ca19-9 CAIX | 6 | -0.8717949 | 0 | 87.1794872 |
| Cyfra SLPI SCC DEFA1 | 4 | 0.77 | 72 | 20 | | TIMP1 ENO2 CEACAM5 MMP2 CA242 CAIX | 6 | 0.8717949 | 0 | 87.1794872 |
| SLPI SCC GRP DEFA1 | 4 | 0.77 | 72 | 20 | | TIMP1 ENO2 MMP2 AMBP Ca19-9 CAIX | 6 | 0.8717949 | 0 | 87.1794872 |
| MIF SCC TFPI OPN CA242 DEFA1 | 6 | 0.77 | 72 | 20 | | TIMP1 ENO2 MMP2 AMBP CA242 CAIX | 6 | 0.8717949 | 0 | 87.1794872 |
| MIF SCC OPN AMBP CA242 DEFA1 | 6 | 0.77 | 72 | 20 | | Cyfra MIF SLPI TIMP1 ENO2 MMP2 CAIX | 7 | 0.8717949 | 0 | 87.1794872 |
| SLPI SCC CEACAMS OPN AMBP CA242 | 6 | 0.77 | 51 | 40 | | MIF SLPI SCC ENO2 MMP2 Ca19-9 CAIX | 7 | 0.8717949 | 0 | 87.1794872 |
| SLPI SCC CEACAM5 OPN Ca19-9 Ca72-4 | 6 | 0.77 | 36 | 40 | | MIF SLPI SCC ENO2 MMP2 CA242 CAIX | 7 | 0.8717949 | 0 | 87.1794872 |
| SLPI SCC CEACAM5 OPN CAIX DEFA1 | 6 | 0.77 | 41 | 40 | | MIF SLDI ENO2 TFPI MMD2 Ca19-9 CAIX | 7 | 0.8717949 | 0 | 87.1794872 |
| SLPI CEACAMS OPN Ca19-9 GRP DEFA1 | 6 | 0.77 | 44 | 40 | | MIF SLPI ENO2 TFPI MMP2 CA242 CAIX | 7 | 0.8717949 | 0 | 87.1794872 |
| SCC TFPI CEACAM5 OPN Ca72-4 GRP DEFA1 | 7 | 0.77 | 18 | 40 | | MIF SLPI ENO2 CEACAM5 MMP2 Ca19-9 CAIX | 7 | 0.8717949 | 0 | 87.1794872 |
| Cyfra MIF SLPI TIMP1 SCC OPN GRP DEFA1 | 8 | 0.77 | 46 | 0 | | MIF SLPI ENO2 CEACAM5 MMP2 CA242 CAIX | 7 | 0.8717949 | 0 | 87.1794872 |
| SLPI SCC OPN AMBP Ca19-9 Ca72-4 CA242 DEFA1 | 8 | 0.77 | 49 | 40 | | MIF SLPI ENO2 MMP2 AMBP Ca19-9 CAIX | 7 | 0.8717949 | 0 | 87.1794872 |
| Cyfra MIF SLPI TIMP1 SCCTFPI CEACAMS OPN Ca19-9 DEFA1 | 10 | 0.77 | 44 | 40 | | MIF SLPI ENO2 MMP2 AMBP CA242 CAIX | 7 | 0.8717949 | 0 | 87.1794872 |
| MIF SLPI SCC TFPI OPN AMBP Ca72-4 CA242 GRP DEFA1 | 10 | 0.77 | 46 | 0 | | MIF TIMP1 SCC ENO2 MMP2 Ca19-9 CAIX | 7 | 0.8717949 | 0 | 87.1794872 |
| MIF SLPI SCC OPN Ca19-9 Ca72-4 | 6 | 0.77 | 33 | 40 | | MIF TIMP1 SCC ENO2 MMP2 CA242 CAIX | 7 | 0.8717949 | 0 | 87.1794872 |
| MIF TIMP1 SCC TFPI CEACAM5 OPN Ca72-4 DEFA1 | 8 | 0.77 | 69 | 0 | | MIF TIMP1 ENO2 TFPI MMP2 Ca19-9 CAIX | 7 | 0.8717949 | 0 | 87.1794872 |
| SLPI TIMP1 SCC TFPI CEACAM5 OPN GRP DEFA1 | 8 | 0.77 | 44 | 40 | | MIF TIMP1 ENO2 TFPI MMP2 CA242 CAIX | 7 | 0.8717949 | 0 | 87.1794872 |
| Cyfra MIF SLPI TIMP1 SCC CEACAM5 OPN AMBP Ca72-4 DEFA1 | 10 | 0.77 | 69 | 0 | | MIF TIMP1 ENO2 CEACAM5 MMP2 Ca19-9 CAIX | 7 | 0.8717949 | 0 | 87.1794872 |
| MIF SLPI TIMP1 SCC TFPI OPN AMBP Ca72-4 GRP DEFA1 | 10 | 0.77 | 69 | 0 | | MIF TIMP1 ENO2 CEACAM5 MMP2 CA242 CAIX | 7 | 0.8717949 | 0 | 87.1794872 |
| MIF SLPI TIMP1 SCC CEACAM5 OPN AMBP Ca72-4 GRP DEFA1 | 10 | 0.77 | 69 | 0 | | MIF TIMP1 ENO2 MMP2 AMBP Ca19-9 CAIX | 7 | 0.8717949 | 0 | 87.1794872 |
| Cyfra MIF SCC OPN Ca72-4 DEFA1 | 6 | 0.77 | 67 | 20 | | MIF TIMP1 ENO2 MMP2 AMBP CA242 CAIX | 7 | 0.8717949 | 0 | 87.1794872 |
| Cyfra SLPI SCC OPN Ca19-9 GRP | 6 | 0.77 | 36 | 0 | | SLPI TIMP1 SCC ENO2 MMP2 Ca19-9 CAIX | 7 | 0.8717949 | 0 | 87.1794872 |
| MIF SCC CEACAMS OPN AMBP DEFA1 | 6 | 0.77 | 31 | 0 | | SLPI TIMP1 SCC ENO2 MMP2 CA242 CAIX | 7 | 0.8717949 | 0 | 87.1794872 |
| MIF SCC OPN Ca19-9 Ca72-4 GRP DEFA1 | 7 | 0.77 | 64 | 20 | | SLPI TIMP1 ENO2 TFPI MMP2 Ca19-9 CAIX | 7 | 0.8717949 | 0 | 87.1794872 |
| Cyfra MIF SLPI SCC CEACAM5 OPN Ca72-4 CA242 | 8 | 0.77 | 36 | 0 | | SLPI TIMP1 ENO2 TFPI MMP2 CA242 CAIX | 7 | 0.8717949 | 0 | 87.1794872 |
| Cyfra SLPI TIMP1 SCC TFPI OPN Ca72-4 DEFA1 | 8 | 0.77 | 67 | 0 | | SLPI TIMP1 ENO2 CEACAM5 MMP2 Ca19-9 CAIX | 7 | 0.8717949 | 0 | 87.1794872 |
| Cyfra SLPI SCC OPN Ca19-9 Ca72-4 CA242 DEFAI | 8 | 0.77 | 46 | 20 | | SLPI TIMP1 ENO2 CEACAM5 MMP2 CA242 CAIX | 7 | 0.8717949 | 0 | 87.1794872 |
| MIF SLPI TIMP1 SCC OPN Ca19-9 Ca72-4 GRP | 8 | 0.77 | 36 | 0 | | SLPI TIMP1 ENO2 MMP2 AMBP Ca19-9 CAIX | 7 | 0.8717949 | 0 | 87.1794872 |
| SLFI SCC TFPI OPN AMBP Ca72-4 CA242 DEFA1 | 8 | 0.77 | 41 | 20 | | SLPI TIMP1 ENO2 MMP2 AMBP CA242 CAIX | 7 | 0.8717949 | 0 | 87.1794872 |
| Cyfra MIF SLPI SCC TFPI OPN AMBP CA242 DEFA1 | 9 | 0.77 | 64 | 0 | | MIF SLPI TIMP1 SCC ENO2 MMP2 Ca19-9 CAIX | 8 | 0.8717949 | 0 | 87.1794872 |
| MIF SLPI TIMP1 SCC OPN AMBP Ca19-9 CA242 DEFA1 | 9 | 0.77 | 67 | 0 | | MIF SLPI TIMP1 SCC ENO2 MMP2 CA242 CAIX | 8 | 0.8717949 | 0 | 87.1794872 |
| SLPI TIMP1 SCC CEACAMS OPN AMBP Ca19-9 CA242 DEFA1 | 9 | 0.77 | 67 | 0 | | MIF SLPI TIMP1 ENO2 TFPI MMP2 Ca19-9 CAIX | 8 | 0.8717949 | 0 | 87.1794872 |
| SLPI SCC CEACAM5 OPN AMBP | S | 0.77 | 64 | 40 | | MIF SLPI TIMP1 ENO2 TFPI MMP2 CA242 CAIX | 8 | 0.8717949 | 0 | 87.1794872 |
| Cyfra SLPI SCC TFPI CEACAM5 OPN DEFA1 | 7 | 0.77 | 64 | 40 | | MIF SLPI TIMP1 ENO2 CEACAM5 MMP2 Ca19-9 CAIX | 8 | 0.8717949 | 0 | 87.1794872 |
| Cyfra SLPI SCC CEACAM5 OPN AMBP DEFA1 | 7 | 0.77 | 64 | 40 | | MIF SLPI TIMP1 ENO2 CEACAM5 MMP2 CA242 CAIX | 8 | 0.8717949 | 0 | 87.1794872 |
| MIF SLPI SCC TFPI CEACAM5 OPN GRP | 7 | 0.77 | 64 | 40 | | MIF SLPI TIMP1 ENO2 MMP2 AMBP Ca19-9 CAIX | 8 | 0.8717949 | 0 | 87.1794872 |
| MIF SLPI SCC OPN AMBP Ca19-9 CA242 | 7 | 0.77 | 64 | 40 | | MIF SLPI TIMP1 ENO2 MMP2 AMBP CA242 CAIX | 8 | 0.8717949 | 0 | 87.1794872 |
| SLPI SCC TFPI OPN AMBP GRP DEFA1 | 7 | 0.77 | 64 | 40 | | Cyfra MIF SCC ENO2 TFPI MMP2 Ca19-9 CAIX GRP | 9 | 0.8717949 | 40 | 79.4871795 |
| MIF SCC OPN CA242 DEFA1 | 5 | 0.77 | 59 | 20 | | Cyfra MIF SCC ENO2 TFPI MMP2 CA242 CAIX GRP | 9 | 0.8717949 | 40 | 79.4871795 |
| Cyfra SLPI SCC CEACAM5 OPN Ca72-4 | 6 | 0.77 | 31 | 20 | | Cyfra MIF SCC ENO2 CEACAM5 MMP2 Ca19-9 CAIX GRP | 9 | 0.8717949 | 40 | 79.4871795 |
| SLPI SCC OPN Ca72-4 CA242 GRP | 6 | 0.77 | 38 | 20 | | Cyfra MIF SCC ENO2 CEACAM5 MMP2 CA242 CAIX GRP | 9 | 0.8717949 | 40 | 79.4871795 |
| SCC TFPI CEACAM5 OPN Ca72-4 DEFA1 | 6 | 0.77 | 28 | 60 | | Cyfra MIF ENO2 TFPI CEACAM5 MMP2 Ca19-9 CAIX GRP | 9 | 0.8717949 | 40 | 79.4871795 |
| MIF SLPI SCC TFPI OPN AMBP CA242 | 7 | 0.77 | 59 | 20 | | Cyfra MIF ENO2 TFPI CEACAM5 MMP2 CA242 CAIX GRP | 9 | 0.8717949 | 40 | 79.4871795 |
| MIF SLPI CEACAM5 OPN Ca72-4 CA242 DEFA1 | 7 | 0.77 | 62 | 20 | | Cyfra TIMP1 SCC ENO2 TFPI MMP2 Ca19-9 CAIX GRP | 9 | 0.8717949 | 40 | 79.4871795 |
| Cyfra MIF SLPI SCC TFPI OPN Ca19-9 CA242 DEFA1 | 9 | 0.77 | 59 | 20 | | Cyfra TIMP1 SCC ENO2 TFPI MMP2 CA242 CAIX GRP | 9 | 0.8717949 | 40 | 79.4871795 |
| MIF SLDI SCC CEACAM5 OPN AMBP Ca19-9 CA262 GRP DEFA1 | 10 | 0.77 | 59 | 0 | | Cyfra TIMP1 SCC ENO2 CEACAM5 MMP2 Ca19-9 CAIX GRP | 9 | 0.8717949 | 40 | 79.4871795 |
| MIF SLPI TIMP1 SCC TFPI CEACAM5 OPN Ca19-9 Ca72-4 GRP DEFA1 | 11 | 0.77 | 59 | 20 | | Cyfra TIMP1 SCC ENO2 CEACAM5 MMP2 CA242 CAIX GRP | 9 | 0.8717949 | 40 | 79.4871795 |
| MIF SLPI OPN GRP DEFA1 | 5 | 0.77 | 54 | 20 | | Cyfra TIMP1 ENO2 TFPI CEACAM5 MMP2 Ca19-9 CAIX GRP | 9 | 0.8717949 | 40 | 79.4871795 |
| MIF SLPI SCC TFPI OPN Ca72-4 CA242 | 7 | 0.77 | 56 | 40 | | Cyfra TIMP1 ENO2 TFPI CEACAM5 MMP2 CA242 CAIX GRP | 9 | 0.8717949 | 40 | 0.8717949 |
| MIF SCC CEACAM5 OPN Ca72-4 CA242 DEFA1 | 7 | 0.77 | 56 | 20 | | Cyfra SCC ENO2 TFPI MMP2 AMBP Ca19-9 CAIX GRP | 9 | 0.8717949 | 40 | 79.4871795 |
| SLPI TIMP1 SCC OPN Ca19-9 Ca72-4 GRP | 7 | 0.77 | 56 | 40 | | Cyfra SCC ENO2 TFPI MMP2 AMBP CA242 CAIX GRP | 9 | 0.8717949 | 40 | 79.4871795 |
| SLPI SCC CEACAM5 OPN Ca72-4 CAIX DEFA1 | 7 | 0.77 | 54 | 40 | | Cyfra SCC ENO2 CEACAM5 MMP2 AMBP Ca19-9 CAIX GRP | 9 | 0.8717949 | 40 | 79.4871795 |
| MIF SLPI SCC TFPI CEACAM5 OPN AMBP Ca72-4 CA242 DEFA1 | 10 | 0.77 | 56 | 20 | | Cyfra SCC ENO2 CEACAM5 MMP2 AMBP CA242 CAIX GRP | 9 | 0.8717949 | 40 | 79.4871795 |
| SLPI TFPI OPN CA242 DEFA1 | 5 | 0.77 | 56 | 0 | | Cyfra ENO2 TFPI CEACAM5 MMP2 AMBP Ca19-9 CAIX GRP | 9 | 0.8717949 | 40 | 79.4871795 |
| Cyfra SLPI CEACAM5 OPN Ca72-4 CA242 DEFA1 | 7 | 0.77 | 46 | 0 | | Cyfra EN02 TFPI CEACAM5 MMP2 AMBP CA242 CAIX GRP | 9 | 0.8717949 | 40 | 79.4871795 |
| MIF TIMP1 SCC TFPI CEACAM5 OPN DEFA1 | 7 | 0.77 | 56 | 0 | | Cyfra MIF SLPI SCC ENO2 TFPI MMP2 Ca19-9 CAIX GRP | 10 | 0.8717949 | 40 | 79.4871795 |
| Cyfra MIF SLPI TIMP1 SCC TFPI OPN AMBP Ca72-4 CA242 DEFA1 | 11 | 0.77 | 56 | 0 | | Cyfra MIF SLPI SCC ENO2 TFPI MMP2 CA242 CAIX GRP | 10 | 0.8717949 | 40 | 79.4871795 |
| Cyfra SCC OPN AMBP DEFA1 | S | 0.77 | 51 | 20 | | Cyfra MIF SLPI SCC ENO2 CEACAM5 MMP2 Ca19-9 CAIX GRP | 10 | 0.8717949 | 40 | 79.4871795 |
| Cyfra MIF SLPI TIMP1 SCC OPN Ca19-9 GRP DEFA1 | 9 | 0.77 | 67 | 0 | | Cyfra MIF SLPI SCC ENO2 CEACAM5 MMP2 CA242 CAIX GRP | 10 | 0.8717949 | 40 | 79.4871795 |
| Cyfra MIF SLPI TIMP1 SCC OPN Ca72-4 GRP DEFA1 | 9 | 0.77 | 54 | 0 | | Cyfra MIF SLPI ENO2 TFPI CEACAM5 MMP2 Ca19-9 CAIX GRP | 10 | 0.8717949 | 40 | 79.4871795 |
| Cyfra MIF SLPI SCC TFPI CEACAM5 OPN CA242 GRP DEFA1 | 10 | 0.77 | 49 | 20 | | Cyfra MIF SLPI ENO2 TFPI CEACAM5 MMP2 CA242 CAIX GRP | 10 | 0.8717949 | 40 | 79.4871795 |
| SLPI TIMP1 SCC Ca72-4 DEFA1 | 5 | 0.77 | 77 | 0 | | Cyfra MIF SCC ENO2 TFPI MMP2 Ca19-9 CA242 CAIX GRP | 10 | 0.8717949 | 40 | 79.4871795 |
| TIMP1 SCC TFPI OPN DEFA1 | 5 | 0.77 | 77 | 0 | | Cyfra MIF SCC ENO2 CEACAM5 MMP2 Ca19-9 CA242 CAIX GRP | 10 | 0.8717949 | 40 | 79.4871795 |
| MIF SLPI CEACAM5 OPN CA242 GRP | 6 | 0.77 | 77 | 0 | | Cyfra MIF ENO2 TFPI CEACAM5 MMP2 Ca19-9 CA242 CAIX GRP | 10 | 0.8717949 | 40 | 0.8717949 |
| TIMP1 SCC TFPI CEACAM5 OPN Ca72-4 DEFA1 | 7 | 0.77 | 44 | 20 | | Cyfra SLPI TIMP1 SCC ENO2 TFPI MMP2 Ca19-9 CAIX GRP | 10 | 0.8717949 | 40 | 79.4871795 |
| MIF SCC TFPI CEACAM5 OPN CA242 GRP DEFA1 | 8 | 0.77 | 46 | 0 | | Cyfra SLPI TIMP1 SCC ENO2 TFPI MMP2 CA242 CAIX GRP | 10 | 0.8717949 | 40 | 79.4871795 |
| MIF SLPI TIMP1 SCC TFPI OPN AMBP Ca19-9 DEFA1 | 9 | 0.77 | 36 | 20 | | Cyfra SLPI TIMP1 SCC ENO2 CEACAM5 MMP2 Ca19-9 CAIX GRP | 10 | 0.8717949 | 40 | 79.4871795 |
| MIF SLPI TIMP1 SCC OPN AMBP Ca72-4 GRP DEFA1 | 9 | 0.77 | 77 | 0 | | Cyfra SLPI TIMP1 SCC ENO2 CEACAM5 MMP2 CA242 CAIX GRP | 10 | 0.8717949 | 40 | 79.4871795 |
| Cyfra MIF SLPI SCC CEACAM5 OPN AMBP CA242 GRP DEFA1 | 10 | 0.77 | 56 | 20 | | Cyfra SLPI TIMD1 ENO2 TFPI CEACAM5 MMP2 Ca19-9 CAIX GRP | 10 | 0.8717949 | 40 | 79.4871795 |
| MIF SLPI SCC OPN AMBP Ca19-9 Ca72-4 CA242 GRP DEFA1 | 10 | 0.77 | 51 | 20 | | Cyfra SLPI TIMP1 ENO2 TFPI CEACAM5 MMP2 CA242 CAIX GRP | 10 | 0.8717949 | 40 | 79.4871795 |
| MIF SLPI CEACAM5 OPN Ca19-9 GRP DEFA1 | 7 | 0.77 | 26 | 0 | | Cyfra SLPI SCC ENO2 TFPI MMP2 AMBP Ca19-9 CAIX GRP | 10 | 0.8717949 | 40 | 79.4871795 |
| Cyfra MIF SLPI SCC TFPI OPN AMBP DEFA1 | 8 | 0.77 | 51 | 20 | | Cyfra SLPI SCC ENO2 TFPI MMP2 AMBP CA242 CAIX GRP | 10 | 0.8717949 | 40 | 79.4871795 |
| MIF SLPI SCC CEACAM5 OPN AMBP CA242 GRP | 8 | 0.77 | 46 | 0 | | Cyfra SLPI SCC ENO2 CEACAM5 MMP2 AMBP Ca19-9 CAIX GRP | 10 | 0.8717949 | 40 | 79.4871795 |
| MIF SCC TFPI CEACAM5 OPN Ca72-4 CA242 GRP DEFA1 | 9 | 0.77 | 31 | 0 | | Cyfra SLPI SCC ENO2 CEACAM5 MMP2 AMBP CA242 CAIX GRP | 10 | 0.8717949 | 40 | 79.4871795 |
| MIF SLDI TIMP1 SCC TFPI CEACAM5 OPN AMBP CA242 DEFA1 | 10 | 0.77 | 56 | 0 | | Cyfra SLPI ENO2 TFPI CEACAM5 MMP2 AMBP Ca19-9 CAIX GRP | 10 | 0.8717949 | 40 | 79.4871795 |
| MIF SLPI TIMP1 SCC TFPI OPN AMBP CA242 GRP DEFA1 | 10 | 0.77 | 74 | 0 | | Cyfra SLPI ENO2 TFPI CEACAM5 MMP2 AMBP CA242 CAIX GRP | 10 | 0.8717949 | 40 | 79.4871795 |
| MIF SLPI SCC TFPI OPN Ca19-9 Ca72-4 CA242 GRP DEFA1 | 10 | 0.77 | 49 | 20 | | Cyfra TIMP1 SCC ENO2 TFPI MMP2 Ca19-9 CA242 CAIX GRP | 10 | 0.8717949 | 40 | 79.4871795 |
| SLPI TFPI OPN Ca19-9 DEFA1 | 5 | 0.77 | 26 | 20 | | Cyfra TIMP1 SCC ENO2 CEACAM5 MMP2 Ca19-9 CA242 CAIX GRP | 10 | 0.8717949 | 40 | 79.4871795 |
| Cyfra SLPI TIMP1 SCC OPN CA242 | 6 | 0.77 | 72 | 20 | | Cyfra TIMP1 ENO2 TFPI CEACAM5 MMP2 Ca19-9 CA242 CAIX GRP | 10 | 0.8717949 | 40 | 79.4871795 |
| Cyfra SLPI SCC OPN CAIX DEFA1 | 6 | 0.77 | 72 | 20 | | Cyfra SCC ENO2 TFPI MMP2 AMBP Ca19-9 CA242 CAIX GRP | 10 | 0.8717949 | 40 | 79.4871795 |
| MIF SLPI SCC TFPI OPN AMBP | 6 | 0.77 | 41 | 20 | | Cyfra SCC ENO2 CEACAM5 MMP2 AMBP Ca19-9 CA242 CAIX GRP | 10 | 0.8717949 | 40 | 79.4871795 |
| MIF SLPI CEACAM5 OPN GRP DEFA1 | 6 | 0.77 | 36 | 40 | | Cyfra ENO2 TFPI CEACAM5 MMP2 AMBP Ca19-9 CA242 CAIX GRP | 10 | 0.8717949 | 40 | 79.4871795 |
| SLPI TIMP1 SCC OPN CA242 GRP | 6 | 0.77 | 72 | 20 | | MIF SCC ENO2 TFPI CEACAM5 MMP2 Ca19-9 CA242 CAIX GRP | 10 | 0.8717949 | 40 | 79.4871795 |
| SLPI SCC OPN AMBP Ca19-9 GRP | 6 | 0.77 | 36 | 20 | | TIMP1 SCC ENO2 TFPI CEACAM5 MMP2 Ca19-9 CA242 CAIX GRP | 10 | 0.8717949 | 40 | 79.4871795 |
| TIMP1 SCC OPN AMBP Ca72-4 DEFA1 | 6 | 0.77 | 69 | 0 | | SCC ENO2 TFPI CEACAM5 MMP2 AMBP Ca19-9 CA242 CAIX GRP | 10 | 0.8717949 | 40 | 79.4871795 |
| TIMP1 SCC OPN Ca19-9 GRP DEFA1 | 6 | 0.77 | 72 | 0 | | Cyfra MIF SLPI SCC ENO2 TFPI MMP2 Ca19-9 CA242 CAIX GRP | 11 | 0.8717949 | 40 | 79.4871795 |
| SCC CEACAM5 OPN AMBP CA242 DEFA1 | 6 | 0.77 | 41 | 0 | | Cyfra MIF SLPI SCC ENO2 CEACAM5 MMP2 Ca19-9 CA242 CAIX GRP | 11 | 0.8717949 | 40 | 79.4871795 |
| Cyfra MIF SLPI SCC CEACAM5 OPN CA242 GRP | 8 | 0.77 | 41 | 40 | | Cyfra MIF SLPI ENO2 TFPI CEACAM5 MMP2 Ca19-9 CA242 CAIX GRP | 11 | 0.8717949 | 40 | 79.4871795 |
| Cyfra SLPI TIMP1 SCC CEACAM5 OPN GRP DEFA1 | 8 | 0.77 | 46 | 20 | | Cyfra SLPI TIMP1 SCC ENO2 TFPI MMP2 Ca19-9 CA242 CAIX GRP | 11 | 0.8717949 | 40 | 79.4871795 |
| Cyfra SLPI SCC OPN Ca72-4 CA242 GRP DEFA1 | 8 | 0.77 | 49 | 20 | | Cyfra SLPI TIMP1 SCC ENO2 CEACAM5 MMP2 Ca19-9 CA242 CAIX GRP | 11 | 0.8717949 | 40 | 79.4871795 |
| MIF SLPI TIMP1 SCC TFPI OPN AMBP Ca72-4 | 8 | 0.77 | 72 | 20 | | Cyfra SLPI TIMP1 EN02 TFPI CEACAM5 MMP2 Ca19-9 CA242 CAIX GRP | 11 | 0.8717949 | 40 | 79.4871795 |
| MIF SLPI TIMP1 SCC TFPI OPN Ca19-9 GRP | 8 | 0.77 | 72 | 20 | | Cyfra SLPI SCC ENO2 TFPI MMP2 AMBP Ca19-9 CA242 CAIX GRP | 11 | 0.8717949 | 40 | 79.4871795 |
| Cyfra MIF SLPI TIMP1 SCC OPN AMBP Ca19-9 Ca72-4 DEFA1 | 10 | 0.77 | 72 | 0 | | Cyfra SLPI SCC ENO2 CEACAM5 MMP2 AMBP Ca19-9 CA242 CAIX GRP | 11 | 0.8717949 | 40 | 79.4871795 |
| Cyfra TIMP1 SCC OPN Ca72-4 DEFA1 | 6 | 0.77 | 69 | 20 | | Cyfra SLPI ENO2 TFPI CEACAM5 MMP2 AMBP Ca19-9 CA242 CAIX GRP | 11 | 0.8717949 | 40 | 79.4871795 |
| MIF SLPI OPN Ca19-9 CA242 GRP DEFA1 | 7 | 0.77 | 69 | 0 | | MIF SLPI SCC ENO2 TFPI CEACAM5 MMP2 Ca19-9 CA242 CAIX GRP | 11 | 0.8717949 | 40 | 79.4871795 |
| MIF SLPI SCC TFPI CEACAM5 OPN Ca72-4 GRP | 8 | 0.77 | 44 | 40 | | SLPI TIMP1 SCC ENO2 TFPI CEACAM5 MMP2 Ca19-9 CA242 CAIX GRP | 11 | 0.8717949 | 40 | 79.4871795 |
| MIF SLPI SCC TFPI OPN Ca19-9 CA242 GRP | 8 | 0.77 | 46 | 40 | | SLPI SCC ENO2 TFPI CEACAM5 MMP2 AMBP Ca19-9 CA242 CAIX GRP | 11 | 0.8717949 | 40 | 79.4871795 |
| SLPI TIMP1 SCC DEFA1 | 4 | 0.77 | 67 | 0 | | Cyfra MIF TIMP1 SCC ENO2 MMP2 CAIX GRP | 8 | 0.8717949 | 40 | 82.0512821 |
| SLPI SCC CA242 DEFA1 | 4 | 0.77 | 67 | 0 | | Cyfra MIF TIMP1 ENO2 TFPI MMP2 CAIX GRP | 8 | 0.8717949 | 40 | 82.0512821 |
| SCC OPN CAIX DEFA1 | 4 | 0.77 | 67 | 0 | | Cyfra MIF TIMP1 ENO2 CEACAM5 MMP2 CAIX GRP | 8 | 0.8717949 | 40 | 82.0512821 |
| Cyfra SCC CEACAM5 OPN Ca19-9 DEFA1 | 6 | 0.77 | 31 | 0 | | Cyfra MIF SCC ENO2 MMP2 AMBP CAIX GRP | 8 | 0.8717949 | 40 | 82.0512821 |
| Cyfra SCC CEACAM5 OPN CA242 DEFA1 | 6 | 0.77 | 33 | 0 | | Cyfra MIF ENO2 TFPI MMP2 AMBP CAIX GRP | 8 | 0.8717949 | 40 | 82.0512821 |
| SLPI TIMP1 SCC OPN CAIX DEFA1 | 6 | 0.77 | 67 | 0 | | Cyfra MIF ENO2 CEACAM5 MMP2 AMBP CAIX GRP | 8 | 0.8717949 | 40 | 82.0512821 |
| TIMP1 SCC TFPI CEACAM5 OPN DEFA1 | 6 | 0.77 | 67 | 0 | | Cyfra TIMP1 SCC ENO2 MMP2 AMBP CAIX GRP | 8 | 0.8717949 | 40 | 82.0512821 |
| SLPI TIMP1 SCC CEACAM5 OPN Ca19-9 CA242 | 7 | 0.77 | 64 | 20 | | Cyfra TIMP1 ENO2 TFPI MMP2 AMBP CAIX GRP | 8 | 0.8717949 | 40 | 82.0512821 |
| Cyfra SLPI SCC OPN AMBP Ca19-9 Ca72-4 DEFA1 | 8 | 0.77 | 41 | 20 | | Cyfra TIMP1 ENO2 CEACAM5 MMP2 AMBP CAIX GRP | 8 | 0.8717949 | 40 | 82.0512821 |
| Cyfra MIF SLPI SCC OPN Ca19-9 Ca72-4 CA242 DEFA1 | 9 | 0.77 | 64 | 20 | | Cyfra MIF SLPI TIMP1 SCC ENO2 MMP2 CAIX GRP | 9 | 0.8717949 | 40 | 82.0512821 |
| SLPI TIMP1 SCC TFPI CEACAM5 OPN Ca19-9 CA242 DEFA1 | 9 | 0.77 | 64 | 0 | | Cyfra MIF SLPI TIMP1 ENO2 TFPI MMP2 CAIX GRP | 9 | 0.8717949 | 40 | 82.0512821 |
| SLPI TIMP1 SCC CEACAM5 OPN AMBP Ca19-9 GRP DEFA1 | 9 | 0.77 | 67 | 0 | | Cyfra MIF SLPI TIMP1 ENO2 CEACAM5 MMP2 CAIX GRP | 9 | 0.8717949 | 40 | 82.0512821 |
| SLPI TIMP1 SCC CEACAM5 OPN AMBP CA242 GRP DEFA1 | 9 | 0.77 | 64 | 0 | | Cyfra MIF SLPI SCC ENO2 MMP2 AMBP CAIX GRP | 9 | 0.8717949 | 40 | 82.0512821 |
| SLPI SCC CEACAM5 OPN AMBP Ca19-9 CA242 GRP DEFA1 | 9 | 0.77 | 49 | 0 | | Cyfra MIF SLPI ENO2 TFPI MMP2 AMBP CAIX GRP | 9 | 0.8717949 | 40 | 82.0512821 |
| Cyfra SLPI TIMP1 SCC CEACAM5 OPN Ca19-9 Ca72-4 CA242 DEFA1 | 10 | 0.77 | 49 | 0 | | Cyfra MIF SLPI ENO2 CEACAM5 MMP2 AMBP CAIX GRP | 9 | 0.8717949 | 40 | 82.0512821 |
| MIF SLPI TIMP1 SCC TFPI CEACAM5 OPN AMBP Ca72-4 CA242 DEFA1 | 11 | 0.77 | 64 | 0 | | Cyfra SLPI TIMP1 SCC ENO2 MMP2 AMBP CAIX GRP | 9 | 0.87179491 | 40 | 82.0512821 |
| MIF SLPI TIMP1 SCC CEACAM5 OPN AMBP Ca19-9 Ca72-4 GRP DEFA1 | 11 | 0.77 | 64 | 0 | | Cyfra SLPI TIMP1 ENO2 TFPI MMP2 AMBP CAIX GRP | 9 | 0.8717949 | 40 | 82.0512821 |
| SLPI TIMP1 SCC TFPI OPN Ca19-9 Ca72-4 DEFA1 | 8 | 0.77 | 64 | 40 | | Cyfra SLPI TIMP1 ENO2 CEACAM5 MMP2 AMBP CAIX GRP | 9 | 0.8717949 | 40 | 82.0512821 |
| SLPI OPN Ca19-9 CA242 DEFA1 | 5 | 0.77 | 62 | 20 | | MIF TIMP1 SCC ENO2 TFPI MMP2 Ca19-9 CAIX GRP | 9 | 0.8717949 | 40 | 82.0512821 |
| SLPI TIMP1 SCC OPN Ca72-4 CA242 | 6 | 0.77 | 33 | 20 | | MIF TIMP1 SCC ENO2 TFPI MMP2 CA242 CAIX GRP | 9 | 0.8717949 | 40 | 82.0512821 |
| Cyfra MIF SLPI SCC OPN Ca19-9 CA242 | 7 | 0.77 | 59 | 20 | | MIF TIMP1 SCC ENO2 CEACAM5 MMP2 Ca19-9 CAIX GRP | 9 | 0.8717949 | 40 | 82.0512821 |
| MIF SLPI TIMP1 SCC OPN CA242 GRP | 7 | 0.77 | 62 | 20 | | MIF TIMP1 SCC ENO2 CEACAM5 MMP2 CA242 CAIX GRP | 9 | 0.8717949 | 40 | 82.0512821 |
| MIF SLPI SCC OPN CA242 CAIX DEFA1 | 7 | 0.77 | 59 | 20 | | MIF TIMP1 ENO2 TFPI CEACAM5 MMP2 Ca19-9 CAIX GRP | 9 | 0.8717949 | 40 | 82.0512821 |
| SLPI TIMP1 SCC OPN AMBP Ca19-9 GRP | 7 | 0.77 | 59 | 0 | | MIF TIMP1 ENO2 TFPI CEACAM5 MMP2 CA242 CAIX GRP | 9 | 0.8717949 | 40 | 82.0512821 |
| SLPI CEACAM5 OPN Ca19-9 Ca72-4 GRP DEFA1 | 7 | 0.77 | 59 | 20 | | MIF SCC ENO2 TFPI MMP2 AMBP Ca19-9 CAIX GRP | 9 | 0.8717949 | 40 | 82.0512821 |
| MIF SLPI SCC TFPI OPN Ca72-4 CA242 GRP | 8 | 0.77 | 38 | 0 | | MIF SCC ENO2 TFPI MMP2 AMBP CA242 CAIX GRP | 9 | 0.8717949 | 40 | 82.0512821 |
| Cyfra MIF SLPI TIMP1 SCC CEACAM5 OPN GRP DEFA1 | 9 | 0.77 | 62 | 20 | | MIF SCC ENO2 CEACAM5 MMP2 AMBP Ca19-9 CAIX GRP | 9 | 0.8717949 | 40 | 82.0512821 |
| Cyfra MIF SLPI TIMP1 SCC OPN Ca72-4 CA242 DEFA1 | 9 | 0.77 | 62 | 0 | | MIF SCC ENO2 CEACAM5 MMP2 AMBP CA242 CAIX GRP | 9 | 0.8717949 | 40 | 82.0512821 |
| MIF SLPI TIMP1 SCC CEACAM5 OPN Ca19-9 Ca72-4 GRP | 9 | 0.77 | 59 | 20 | | MIF ENO2 TFPI CEACAM5 MMP2 AMBP Ca19-9 CAIX GRP | 9 | 0.8717949 | 40 | 82.0512821 |
| SLPI TIMP1 SCC CEACAM5 OPN AMBP Ca19-9 Ca72-4 DEFA1 | 9 | 0.77 | 59 | 20 | | MIF ENO2 TFPI CEACAM5 MMP2 AMBP CA242 CAIX GRP | 9 | 0.8717949 | 40 | 82.0512821 |
| SLPI SCC TFPI CEACAM5 OPN Ca19-9 Ca72-4 CA242 GRP DEFA1 | 10 | 0.77 | 36 | 20 | | TIMP1 SCC ENO2 TFPI MMP2 AMBP Ca19-9 CAIX GRP | 9 | 0.8717949 | 40 | 82.0512821 |
| SCC TFPI OPN AMBP DEFA1 | 5 | 0.77 | 54 | 40 | | TIMP1 SCC ENO2 TFPI MMP2 AMBP CA242 CAIX GRP | 9 | 0.8717949 | 40 | 82.0512821 |
| MIF SLPI SCC CEACAM5 Ca72-4 DEFA1 | 6 | 0.77 | 31 | 60 | | TIMP1 SCC ENO2 CEACAM5 MP2 AMBP Ca19-9 CAIX GRP | 9 | 0.8717949 | 40 | 82.0512821 |
| Cyfra SLPI TIMP1 SCC OPN GRP DEFA1 | 7 | 0.77 | 56 | 0 | | TIMP1 SCC ENO2 CEACAM5 MMP2 AMBP CA242 CAIX GRP | 9 | 0.8717949 | 40 | 82.0512821 |
| MIF SLPI TIMP1 SCC OPN AMBP Ca19-9 | 7 | 0.77 | 56 | 20 | | TIMP1 ENO2 TFPI CEACAM5 MMP2 AMBP Ca19-9 CAIX GRP | 9 | 0.8717949 | 40 | 82.0512821 |
| Cyfra SLPI SCC TFPI OPN Ca19-9 Ca72-4 DEFA1 | 8 | 0.77 | 41 | 20 | | TIMP1 ENO2 TFPI CEACAM5 MMP2 AMBP CA242 CAIX GRP | 9 | 0.8717949 | 40 | 82.0512821 |
| SLPI SCC TFPI CEACAM5 OPN Ca19-9 GRP DEFA1 | 8 | 0.77 | 56 | 40 | | MIF SLPI TIMP1 SCC ENO2 TFPI MMP2 Ca19-9 CAIX GRP | 10 | 0.8717949 | 40 | 82.0512821 |
| Cyfra SLPI TIMP1 SCC CEACAM5 OPN Ca72-4 GRP DEFA1 | 9 | 0.77 | 56 | 40 | | MIF SLPI TIMP1 SCC ENO2 TFPI MMP2 CA242 CAIX GRP | 10 | 0.8717949 | 40 | 82.0512821 |
| MIF SLPI SCC TFPI CEACAM5 OPN Ca19-9 Ca72-4 DEFA1 | 9 | 0.77 | 31 | 40 | | MIF SLPI TIMP1 SCC ENO2 CEACAM5 MMP2 Ca19-9 CAIX GRP | 10 | 0.8717949 | 40 | 82.0512821 |
| SLPI TIMP1 SCC TFPI CEACAM5 OPN Ca19-9 Ca72-4 DEFA1 | 9 | 0.77 | 56 | 20 | | MIF SLPI TIMP1 SCC ENO2 CEACAM5 MMP2 CA242 CAIX GRP | 10 | 0.8717949 | 40 | 82.0512821 |
| SCC CEACAM5 OPN CA242 | 4 | 0.77 | 23 | 60 | | MIF SLPI TIMP1 ENO2 TFPI CEACAM5 MMP2 Ca19-9 CAIX GRP | 10 | 0.8717949 | 40 | 82.0512821 |
| Cyfra SLPI CEACAM5 OPN DEFA1 | 5 | 0.77 | 51 | 20 | | MIF SLPI TIMP1 ENO2 TFPI CEACAM5 MMP2 CA242 CAIX GRP | 10 | 0.8717949 | 40 | 82.0512821 |
| Cyfra SLPI SCC TFPI OPN Ca19-9 DEFA1 | 7 | 0.77 | 51 | 40 | | MIF SLPI SCC ENO2 TFPI MMP2 AMBP Ca19-9 CAIX GRP | 10 | 0.8717949 | 40 | 82.0512821 |
| SLPI SCC CEACAM5 OPN AMBP Ca19-9 Ca72-4 DEFA1 | 8 | 0.77 | 49 | 60 | | MIF SLPI SCC ENO2 TFPI MMP2 AMBP CA242 CAIX GRP | 10 | 0.8717949 | 40 | 82.0512821 |
| SLPI TIMP1 SCC TFPI CEACAM5 OPN Ca72-4 GRP DEFA1 | 9 | 0.77 | 51 | 40 | | MIF SLPI SCC ENO2 CEACAM5 MMP2 AMBP Ca19-9 CAIX GRP | 10 | 0.8717949 | 40 | 82.0512821 |
| SLPI SCC TFPI CEACAM5 OPN Ca19-9 Ca72-4 GRP DEFA1 | 9 | 0.77 | 44 | 40 | | MIF SLPI SCC ENO2 CEACAM5 MMP2 AMBP CA242 CAIX GRP | 10 | 0.8717949 | 40 | 82.0512821 |
| MIF SLPI SCC TFPI CEACAM5 OPN Ca19-9 Ca72-4 GRP DEFA1 | 10 | 0.77 | 49 | 60 | | MIF SLPI ENO2 TFPI CEACAM5 MMP2 AMBP Ca19-9 CAIX GRP | 10 | 0.8717949 | 40 | 82.0512821 |
| Cyfra MIF SLPI TIMP1 SCC TFPI OPN | 7 | 0.76 | 54 | 0 | | MIF SLPI ENO2 TFPI CEACAM5 MMP2 AMBP CA242 CAIX GRP | 10 | 0.8717949 | 40 | 82.0512821 |
| MIF TIMP1 SCC TFPI OPN AMBP DEFA1 | 7 | 0.76 | 54 | 0 | | SLPI TIMP1 SCC ENO2 TFPI MMP2 AMBP Ca19-9 CAIX GRP | 10 | 0.8717949 | 40 | 82.0512821 |
| SLPI CEACAM5 OPN Ca72-4 CA242 GRP DEFA1 | 7 | 0.76 | 54 | 0 | | SLPI TIMP1 SCC ENO2 TFPI MMP2 AMBP CA242 CAIX GRP | 10 | 0.8717949 | 40 | 82.0512821 |
| Cyfra TIMP1 SCC OPN DEFA1 | 5 | 0.76 | 51 | 0 | | SLPI TIMP1 SCC ENO2 CEACAM5 MMP2 AMBP Ca19-9 CAIX GRP | 10 | 0.8717949 | 40 | 82.0512821 |
| MIF TIMP1 SCCTFPI OPN | 5 | 0.76 | 44 | 0 | | SLPI TIMP1 SCC ENO2 CEACAM5 MMP2 AMBP CA242 CAIX GRP | 10 | 0.8717949 | 40 | 82.0512821 |
| Cyfra SLPI SCC OPN AMBP Ca72-4 CA242 DEFA1 | 8 | 0.76 | 44 | 0 | | SLPI TIMP1 ENO2 TFPI CEACAM5 MMP2 AMBP Ca19-9 CAIX GRP | 10 | 0.8717949 | 40 | 82.0512821 |
| Cyfra SLPI TIMP1 SCC OPN AMBP Ca72-4 CA242 DEFA1 | 9 | 0.76 | 59 | 0 | | SLPI TIMP1 ENO2 TFPI CEACAM5 MMP2 AMBP CA242 CAIX GRP | 10 | 0.8717949 | 40 | 82.0512821 |
| MIF SLPI TIMP1 SCC CEACAM5 OPN AMBP Ca72-4 CA242 GRP DEFA1 | 11 | 0.76 | 64 | 0 | | MIF TIMP1 SCC ENO2 MMP2 CAIX GRP | 7 | 0.8666667 | 20 | 84.6153846 |
| SLPI SCC OPN Ca72-4 CAIX | 5 | 0.76 | 41 | 20 | | MIF TIMP1 ENO2 TFPI MMP2 CAIX GRP | 7 | 0.8666667 | 20 | 84.6153846 |
| Cyfra MIF SLPI SCC OPN Ca72-4 GRP | 7 | 0.76 | 46 | 20 | | MIF TIMP1 ENO2 CEACAM5 MMP2 CAIX GRP | 7 | 0.8666667 | 20 | 84.6153846 |
| MIF TIMP1 SCC CEACAM5 OPN Ca72-4 DEFA1 | 7 | 0.76 | 56 | 20 | | MIF SCC ENO2 MMP2 AMBP CAIX GRP | 7 | 0.8666667 | 20 | 84.6153846 |
| SLPI SCC OPN Ca72-4 CA242 CAIX DEFA1 | 7 | 0.76 | 54 | 20 | | MIF ENO2 TFPI MMP2 AMBP CAIX GRP | 7 | 0.8666667 | 20 | 84.6153846 |
| Cyfra SLPI SCC OPN AMBP Ca19-9 CA242 DEFA1 | 8 | 0.76 | 41 | 0 | | MIF ENO2 CEACAM5 MMP2 AMBP CAIX GRP | 7 | 0.8666667 | 20 | 84.6153846 |
| SLPI TIMP1 SCC TFPI OPN Ca72-4 CA242 GRP DEFA1 | 9 | 0.76 | 56 | 20 | | TIMP1 SCC ENO2 MMP2 AMBP CAIX GRP | 7 | 0.8666667 | 20 | 84.6153846 |
| SLPI TIMP1 SCC OPN AMBP Ca72-4 CA242 GRP DEFA1 | 9 | 0.76 | 62 | 0 | | TIMP1 ENO2 TFPI MMP2 AMBP CAIX GRP | 7 | 0.8666667 | 20 | 84.6153846 |
| Cyfra SLPI SCC OPN Ca19-9 CA242 GRP | 7 | 0.76 | 28 | 0 | | TIMP1 ENO2 CEACAM5 MMP2 AMBP CAIX GRP | 7 | 0.8666667 | 20 | 84.6153846 |
| SLPI SCC OPN AMBP Ca19-9 CA242 GRP | 7 | 0.76 | 28 | 0 | | Cyfra MIF TIMP1 SCC ENO2 MMP2 Ca19-9 CAIX | 8 | 0.8666667 | 20 | 84.6153846 |
| MIF SLPI TIMP1 SCC OPN AMBP CA242 GRP | 8 | 0.76 | 74 | 0 | | Cyfra MIF TIMP1 SCC ENO2 MMP2 CA242 CAIX | 8 | 0.8666667 | 20 | 84.6153846 |
| SCC TFPI CEACAM5 OPN Ca72-4 CA242 GRP DEFA1 | 8 | 0.76 | 13 | 0 | | Cyfra MIF TIMP1 ENO2 TFPI MMP2 Ca19-9 CAIX | 8 | 0.8666667 | 20 | 84.6153846 |
| Cyfra MIF SCC TFPI CEACAM5 OPN Ca72-4 CA242 DEFA1 | 9 | 0.76 | 28 | 0 | | Cyfra MIF TIMP1 ENO2 TFPI MMP2 CA242 CAIX | 8 | 0.8666667 | 20 | 84.6153846 |
| Cyfra MIF SLPI TMP1 SCC TFPI OPN AMBP CA242 DEFA1 | 10 | 0.76 | 74 | 0 | | Cyfra MIF TIMP1 ENO2 CEACAM5 MMP2 Ca19-9 CAIX | 8 | 0.8666667 | 20 | 84.6153846 |
| SLPI OPN Ca72-4 | 3 | 0.76 | 31 | 20 | | Cyfra MIF TIMP1 ENO2 CEACAM5 MMP2 CA242 CAIX | 8 | 0.8666667 | 20 | 84.6153846 |
| MIF SCC OPN CA242 GRP DEFA1 | 6 | 0.76 | 41 | 20 | | Cyfra MIF TIMP1 ENO2 MMP2 AMBP Ca19-9 CAIX | 8 | 0.8666667 | 20 | 84.6153846 |
| SLPI SCC TFPI CEACAM5 OPN Ca72-4 | 6 | 0.76 | 38 | 20 | | Cyfra MIF TIMP1 ENO2 MMP2 AMBP CA242 CAIX | 8 | 0.8666667 | 20 | 84.6153846 |
| SCC CEACAM5 OPN Ca19-9 CA242 DEFA1 | 6 | 0.76 | 44 | 20 | | Cyfra MIF SCC ENO2 TFPI MMP2 CAIX GRP | 8 | 0.8666667 | 40 | 79.4871795 |
| SCC OPN AMBP Ca19-9 GRP DEFA1 | 6 | 0.76 | 36 | 0 | | Cyfra MIF SCC ENO2 CEACAM5 MMP2 CAIX GRP | 8 | 0.8666667 | 40 | 79.4871795 |
| Cyfra MIF SLPI CEACAM5 OPN Ca19-9 DEFA1 | 7 | 0.76 | 23 | 0 | | Cyfra MIF ENO2 TFPI CEACAM5 MMP2 CAIX GRP | 8 | 0.8666667 | 40 | 79.4871795 |
| MIF SLPI TIMP1 SCC OPN Ca19-9 CA242 | 7 | 0.76 | 31 | 20 | | Cyfra TIMP1 SCC ENO2 TFPI MMP2 CAIX GRP | 8 | 0.8666667 | 40 | 79.4871795 |
| MIF SLPI SCC OPN AMBP CAIX DEFA1 | 7 | 0.76 | 31 | 20 | | Cyfra TIMP1 SCC ENO2 CEACAM5 MMP2 CAIX GRP | 8 | 0.8666667 | 40 | 79.4871795 |
| SCC TFPI CEACAM5 OPN CA242 GRP DEFA1 | 7 | 0.76 | 31 | 20 | | Cyfra TIMP1 ENO2 TFPI CEACAM5 MMP2 CAIX GRP | 8 | 0.8666667 | 40 | 79.4871795 |
| Cyfra SLPI SCC OPN AMBP Ca19-9 GRP DEFA1 | 8 | 0.76 | 46 | 20 | | Cyfra SCC ENO2 TFPI MMP2 AMBP CAIX GRP | 8 | 0.8666667 | 40 | 79.4871795 |
| MIF SLPI TIMP1 SCC TFPI CEACAM5 OPN CA242 | 8 | 0.76 | 72 | 20 | | Cyfra SCC EN02 CEACAM5 MMP2 AMBP CAIX GRP | 8 | 0.8666667 | 40 | 79.4871795 |

**TABLE 10,**

| Tumor vs. Benign | Split point | | | |
|---|---|---|---|---|
| | Trained on benigns/54x53, tested on benigns/54x53 | | | |
| Panel | Size | AUC | Sens at 95% Spec | Spec at 95% Sens |
| Cyfra TIMP1 SCC TFPI CEACAM5 MDK | 6 | 0.99 | 96 | 100 |
| TIMP1 SCC TFPI CEACAM5 MDK | 5 | 0.99 | 98 | 100 |
| TIMP1 SCC TFPI MDK | 4 | 0.99 | 92 | 89 |
| SLPI TIMP1 SCC TFPI CEACAM5 MDK | 6 | 0.99 | 98 | 100 |
| TIMP1 SCC TFPI CEACAM5 OPN MDK | 6 | 0.99 | 98 | 100 |
| Cyfra SLPI TIMP1 SCC TFPI CEACAM5 MDK | 7 | 0.99 | 96 | 100 |
| Cyfra TIMP1 SCC TFPI CEACAM5 OPN MDK | 7 | 0.99 | 96 | 100 |
| SLPI TIMP1 SCC TFPI CEACAM5 OPN MDK | 7 | 0.99 | 98 | 100 |
| Cyfra TIMP1 SCC TFPI MDK | 5 | 0.99 | 91 | 89 |
| SCC TFPI MDK | 3 | 0.99 | 98 | 100 |
| Cyfra SCC TFPI MDK | 4 | 0.99 | 96 | 100 |
| SLPI SCC TFPI MDK | 4 | 0.99 | 96 | 100 |
| SCC TFPI OPN MDK | 4 | 0.99 | 96 | 100 |
| Cyfra TIMP1 SCC CEACAM5 MDK | 5 | 0.99 | 98 | 100 |
| Cyfra SLPI TIMP1 SCC CEACAM5 MDK | 6 | 0.99 | 98 | 100 |
| Cyfra TIMP1 SCC CEACAM5 OPN MDK | 6 | 0.99 | 98 | 100 |
| SLPI TIMP1 SCC TFPI MDK | 5 | 0.99 | 92 | 89 |
| TIMP1 SCC TFPI OPN MDK | 5 | 0.99 | 92 | 89 |
| Cyfra SLPI TIMP1 SCC TFPI CEACAM5 OPN MDK | 8 | 0.99 | 94 | 78 |
| SLPI TIMP1 SCC CEACAM5 MDK | 5 | 0.98 | 98 | 100 |
| TIMP1 SCC CEACAM5 OPN MDK | 5 | 0.98 | 98 | 100 |
| SLPI TIMP1 SCC CEACAM5 OPN MDK | 6 | 0.98 | 98 | 100 |
| Cyfra SLPI TIMP1 SCC TFPI MDK | 6 | 0.98 | 89 | 89 |
| Cyfra TIMP1 SCC TFPI OPN MDK | 6 | 0.98 | 89 | 89 |
| Cyfra SLPI TIMP1 SCC CEACAM5 OPN MDK | 7 | 0.98 | 96 | 100 |
| SLPI TIMP1 SCC TFPI OPN MDK | 6 | 0.98 | 87 | 89 |
| SLPI SCC TFPI CEACAM5 MDK | 5 | 0.98 | 85 | 89 |
| SCC TFPI CEACAM5 OPN MDK | 5 | 0.98 | 85 | 89 |
| Cyfra SLPI TIMP1 SCC MDK | 5 | 0.97 | 91 | 78 |
| Cyfra TIMP1 SCC OPN MDK | 5 | 0.97 | 91 | 78 |
| Cyfra TIMP1 SCC MDK | 4 | 0.97 | 92 | 67 |
| Cyfra SLPI SCC TFPI MDK | 5 | 0.97 | 92 | 67 |
| Cyfra SCC TFPI OPN MDK | 5 | 0.97 | 92 | 67 |
| Cyfra SLPI TIMP1 SCC TFPI OPN MDK | 7 | 0.97 | 85 | 89 |
| Cyfra SCC TFPI CEACAM5 MDK | 5 | 0.97 | 83 | 89 |
| SLPI TIMP1 SCC MDK | 4 | 0.97 | 92 | 78 |
| TIMP1 SCC OPN MDK | 4 | 0.97 | 92 | 78 |
| Cyfra SLPI SCC TFPI CEACAM5 MDK | 6 | 0.97 | 81 | 89 |
| Cyfra SCC TFPI CEACAM5 OPN MDK | 6 | 0.97 | 81 | 89 |
| SLPI SCC TFPI CEACAM5 OPN MDK | 6 | 0.97 | 79 | 89 |
| SLPI TIMP1 CEACAM5 MDK | 4 | 0.97 | 94 | 67 |
| TIMP1 CEACAM5 OPN MDK | 4 | 0.97 | 94 | 67 |
| SLPI SCC TFPI OPN MDK | 5 | 0.97 | 91 | 67 |
| SCC TFPI CEACAM5 MDK | 4 | 0.97 | 85 | 78 |
| SLPI TIMP1 SCC TFPI CEACAM5 | 5 | 0.96 | 87 | 78 |
| TIMP1 SCC TFPI CEACAM5 OPN | 5 | 0.96 | 87 | 78 |
| Cyfra SLPI TIMP1 CEACAM5 MDK | 5 | 0.96 | 89 | 78 |
| Cyfra TIMP1 CEACAM5 OPN MDK | 5 | 0.96 | 89 | 78 |
| Cyfra SLPI TIMP1 SCC OPN MDK | 6 | 0.96 | 87 | 78 |
| Cyfra TIMP1 SCC TFPI MMP2 MDK | 6 | 0.96 | 74 | 78 |
| TIMP1 SCC TFPI | 3 | 0.96 | 68 | 89 |
| Cyfra SLPI SCC CEACAM5 MDK | 5 | 0.96 | 85 | 78 |
| Cyfra SCC CEACAM5 OPN MDK | 5 | 0.96 | 85 | 78 |
| SLPI TIMP1 TFPI CEACAM5 MDK | 5 | 0.96 | 89 | 78 |
| TIMP1 TFPI CEACAM5 OPN MDK | 5 | 0.96 | 89 | 78 |
| Cyfra TIMP1 SCCTFPI CEACAM5 MMP2 MDK | 7 | 0.96 | 85 | 78 |
| Cyfra TIMP1 TFPI CEACAM5 MDK | 5 | 0.96 | 87 | 78 |
| Cyfra SLPI SCC TFPI OPN MDK | 6 | 0.96 | 89 | 67 |
| Cyfra SLPI SCC TFPI CEACAM5 OPN MDK | 7 | 0.96 | 77 | 67 |
| Cyfra SLPI TIMP1 TFPI CEACAM5 MDK | 6 | 0.96 | 85 | 78 |
| Cyfra TIMP1 TFPI CEACAM5 OPN MDK | 6 | 0.96 | 85 | 78 |
| SLPI TIMP1 SCC OPN MDK | 5 | 0.96 | 87 | 78 |
| SLPI TIMP1 CEACAM5 OPN MDK | 5 | 0.96 | 89 | 78 |
| Cyfra SLPI TIMP1 SCC CEACAM5 MMP2 MDK | 7 | 0.96 | 87 | 78 |
| Cyfra TIMP1 SCC CEACAM5 MMP2 OPN MDK | 7 | 0.96 | 87 | 78 |
| SLPI SCC CEACAM5 MDK | 4 | 0.96 | 91 | 67 |
| SCC CEACAM5 OPN MDK | 4 | 0.96 | 91 | 67 |
| TIMP1 SCC TFPI MMP2 MDK | 5 | 0.96 | 79 | 89 |
| TIMP1 SCC CEACAM5 MDK | 4 | 0.96 | 87 | 78 |
| Cyfra TIMP1 SCC TFPI CEACAM5 | 5 | 0.96 | 85 | 78 |
| Cyfra SLPI TIMP1 SCC TFPI CEACAM5 | 6 | 0.96 | 83 | 78 |
| Cyfra TIMP1 SCC TFPI CEACAM5 OPN | 6 | 0.96 | 83 | 78 |
| Cyfra SLPI TIMP1 SCC TFPI CEACAM5 MMP2 MDK | 8 | 0.96 | 83 | 78 |
| Cyfra TIMP1 SCC TFPI CEACAM5 MMP2 OPN MDK | 8 | 0.96 | 83 | 78 |
| TIMP1 TFPI OPN MDK | 4 | 0.96 | 75 | 78 |
| SLPI TIMP1 SCC TFPI MMP2 MDK | 6 | 0.96 | 74 | 89 |
| TIMP1 SCC TFPI MMP2 OPN MDK | 6 | 0.96 | 74 | 89 |
| SLPI SCC CEACAM5 OPN MDK | 5 | 0.96 | 85 | 78 |
| SLPI TIMP1 SCC TFPI CEACAM5 MMP2 MDK | 7 | 0.96 | 85 | 78 |
| SLPI TIMP1 SCC CEACAM5 MMP2 OPN MDK | 7 | 0.96 | 87 | 78 |
| TIMP1 SCC TFPI CEACAM5 MMP2 OPN MDK | 7 | 0.96 | 85 | 78 |
| TFPI MDK | 2 | 0.96 | 87 | 67 |
| Cyfra SCC CEACAM5 MDK | 4 | 0.96 | 87 | 67 |
| SLPI TIMP1 SCC CEACAM5 MMP2 MDK | 6 | 0.96 | 91 | 67 |
| TIMP1 SCC CEACAM5 MMP2 OPN MDK | 6 | 0.96 | 91 | 67 |
| TIMP1 TFPI MDK | 3 | 0.96 | 83 | 44 |
| Cyfra SLPI TIMP1 SCCTFPI MMP2 MDK | 7 | 0.96 | 70 | 78 |
| Cyfra TIMP1 SCC TFPI MMP2 OPN MDK | 7 | 0.96 | 70 | 78 |
| Cyfra SLPI TIMP1 SCC TFPI CEACAM5 MMP2 OPN MDK | 9 | 0.95 | 81 | 78 |
| Cyfra SLPI TIMP1 CEACAM5 OPN MDK | 6 | 0.95 | 85 | 78 |
| Cyfra SLPI TIMP1 SCC CEACAM5 MMP2 OPN MDK | 8 | 0.95 | 85 | 78 |
| TIMP1 TFPI CEACAM5 MDK | 4 | 0.95 | 89 | 67 |
| Cyfra TIMP1 SCC CEACAM5 MMP2 MDK | 6 | 0.95 | 89 | 67 |
| SLPI TIMP1 SCC MMP2 MDK | 5 | 0.95 | 79 | 89 |
| TIMP1 SCC MMP2 OPN MDK | 5 | 0.95 | 79 | 89 |
| SLPI TIMP1 TFPI MDK | 4 | 0.95 | 72 | 78 |
| Cyfra TIMP1 SCC MMP2 MDK | 5 | 0.95 | 77 | 89 |
| SLPI TIMP1 SCC TFPI CEACAM5 OPN | 6 | 0.95 | 81 | 78 |
| Cyfra SLPI TIMP1 TFPI CEACAM5 OPN MDK | 7 | 0.95 | 81 | 78 |
| Cyfra SLPI SCC CEACAM5 OPN MDK | 6 | 0.95 | 81 | 78 |
| SLPI TIMP1 SCC CEACAM5 | 4 | 0.95 | 92 | 44 |
| TIMP1 SCC CEACAM5 OPN | 4 | 0.95 | 92 | 44 |
| Cyfra TIMP1 CEACAM5 MDK | 4 | 0.95 | 91 | 56 |
| Cyfra SLPI TIMP1 SCC MMP2 MDK | 6 | 0.95 | 74 | 67 |
| Cyfra TIMP1 SCC MMP2 OPN MDK | 6 | 0.95 | 74 | 67 |
| TIMP1 SCC TFPI OPN | 4 | 0.95 | 60 | 89 |
| TFPI OPN MDK | 3 | 0.95 | 79 | 78 |
| SLPI TIMP1 TFPI CEACAM5 OPN MDK | 6 | 0.95 | 83 | 78 |
| TIMP1 SCC MDK | 3 | 0.95 | 92 | 33 |
| Cyfra SLPI TIMP1 SCC CEACAM5 | 5 | 0.95 | 87 | 67 |
| Cyfra TIMP1 SCC CEACAM5 OPN | 5 | 0.95 | 87 | 67 |
| SLPI TIMP1 SCC TFPI MMP2 OPN MDK | 7 | 0.95 | 70 | 78 |
| SCC OPN MDK | 3 | 0.95 | 70 | 89 |
| Cyfra SLPI TIMP1 SCC TFPI CEACAM5 OPN | 7 | 0.95 | 79 | 67 |
| SLPI TIMP1 SCC TFPI CEACAM5 MMP2 OPN MDK | 8 | 0.95 | 81 | 78 |
| Cyfra SLPI TIMP1 SCC TFPI MMP2 OPN MDK | 8 | 0.95 | 66 | 78 |
| Cyfra TIMP1 TFPI MDK | 4 | 0.95 | 68 | 67 |
| SLPI TIMP1 SCC TFPI | 4 | 0.95 | 57 | 89 |
| SLPI TIMP1 SCC CEACAM5 OPN | 5 | 0.95 | 87 | 67 |
| TIMP1 OPN MDK | 3 | 0.95 | 81 | 44 |
| SLPI TIMP1 TFPI OPN MDK | 5 | 0.95 | 70 | 78 |
| SLPI TIMP1 SCC MMP2 OPN MDK | 6 | 0.95 | 75 | 67 |
| SLPI SCC MDK | 3 | 0.95 | 66 | 89 |
| Cyfra SCC MDK | 3 | 0.95 | 60 | 89 |
| TIMP1 SCC TFPI CEACAM5 MMP2 MDK | 6 | 0.94 | 85 | 67 |
| Cyfra TIMP1 TFPI OPN MDK | 5 | 0.94 | 62 | 78 |
| Cyfra SLPI TIMP1 SCC MMP2 OPN MDK | 7 | 0.94 | 70 | 67 |
| SLPI TFPI MDK | 3 | 0.94 | 75 | 78 |
| SLPI SCC TFPI CEACAM5 MMP2 MDK | 6 | 0.94 | 70 | 78 |
| SCC TFPI CEACAM5 MMP2 OPN MDK | 6 | 0.94 | 70 | 78 |
| SLPI TIMP1 MDK | 3 | 0.94 | 77 | 44 |
| TIMP1 SCC TFPI CEACAM5 | 4 | 0.94 | 87 | 56 |
| Cyfra TIMP1 SCC TFPI | 4 | 0.94 | 53 | 89 |
| Cyfra SCC TFPI MMP2 MDK | 5 | 0.94 | 75 | 78 |
| SLPI SCC TFPI MMP2 MDK | 5 | 0.94 | 75 | 78 |
| SCC TFPI MMP2 OPN MDK | 5 | 0.94 | 75 | 78 |
| Cyfra SCC TFPI CEACAM5 MMP2 MDK | 6 | 0.94 | 68 | 78 |
| Cyfra SLPI SCC TFPI CEACAM5 MMP2 MDK | 7 | 0.94 | 64 | 78 |
| Cyfra SCC TFPI CEACAM5 MMP2 OPN MDK | 7 | 0.94 | 64 | 78 |
| Cyfra SLPI TIMP1 TFPI MDK | 5 | 0.94 | 58 | 78 |
| OPN MDK | 2 | 0.94 | 85 | 67 |
| Cyfra SLPI TIMP1 SCC CEACAM5 OPN | 6 | 0.94 | 83 | 67 |
| Cyfra SCC OPN MDK | 4 | 0.94 | 55 | 67 |
| SLPI TIMP1 OPN MDK | 4 | 0.94 | 75 | 67 |
| SLPI SCC OPN MDK | 4 | 0.94 | 64 | 67 |
| Cyfra TIMP1 OPN MDK | 4 | 0.94 | 66 | 56 |
| SLPI SCC TFPI CEACAM5 MMP2 OPN MDK | 7 | 0.93 | 66 | 78 |
| SLPI TIMP1 SCC TFPI OPN | 5 | 0.93 | 55 | 67 |
| CEACAM5 OPN MDK | 3 | 0.93 | 55 | 89 |
| Cyfra SLPI SCC MDK | 4 | 0.93 | 51 | 67 |
| Cyfra TFPI MDK | 3 | 0.93 | 72 | 78 |
| Cyfra SLPI SCC TFPI MMP2 MDK | 6 | 0.93 | 72 | 67 |
| Cyfra SCC TFPI MMP2 OPN MDK | 6 | 0.93 | 72 | 67 |
| SLPI TFPI OPN MDK | 4 | 0.93 | 74 | 67 |
| Cyfra SLPI SCC TFPI CEACAM5 MMP2 OPN MDK | 8 | 0.93 | 60 | 67 |
| Cyfra SLPI TIMP1 TFPI OPN MDK | 6 | 0.93 | 57 | 78 |
| Cyfra SLPI TIMP1 MDK | 4 | 0.93 | 62 | 56 |
| TFPI CEACAM5 OPN MDK | 4 | 0.93 | 55 | 78 |
| TIMP1 SCC MMP2 MDK | 4 | 0.93 | 85 | 67 |
| Cyfra SLPI SCC CEACAM5 MMP2 MDK | 6 | 0.93 | 64 | 67 |
| Cyfra SCC CEACAM5 MMP2 OPN MDK | 6 | 0.93 | 64 | 67 |
| SLPI CEACAM5 MDK | 3 | 0.93 | 51 | 89 |
| Cyfra TIMP1 SCC TFPI OPN | 5 | 0.93 | 47 | 67 |
| SLPI SCC TFPI MMP2 OPN MDK | 6 | 0.93 | 72 | 67 |
| SLPI SCC CEACAM5 MMP2 OPN MDK | 6 | 0.93 | 66 | 67 |
| SLPI TFPI CEACAM5 MDK | 4 | 0.93 | 51 | 78 |
| MDK | 1 | 0.92 | 92 | 0 |
| SLPI MDK | 2 | 0.92 | 81 | 67 |
| TIMP1 MDK | 2 | 0.92 | 89 | 0 |
| Cyfra TIMP1 SCC CEACAM5 | 4 | 0.92 | 89 | 33 |
| Cyfra SLPI TIMP1 OPN MDK | 5 | 0.92 | 60 | 67 |
| Cyfra SLPI TIMP1 SCC TFPI | 5 | 0.92 | 43 | 67 |
| Cyfra SLPI SCC OPN MDK | 5 | 0.92 | 49 | 67 |
| Cyfra SLPI SCC TFPI MMP2 OPN MDK | 7 | 0.92 | 68 | 67 |
| TIMP1 SCC CEACAM5 MMP2 MDK | 5 | 0.92 | 74 | 44 |
| SLPI SCC CEACAM5 MMP2 MDK | 5 | 0.92 | 70 | 33 |
| SCC CEACAM5 MMP2 OPN MDK | 5 | 0.92 | 70 | 33 |
| Cyfra SLPI SCC CEACAM5 MMP2 OPN MDK | 7 | 0.92 | 60 | 67 |
| Cyfra TFPI CEACAM5 MDK | 4 | 0.92 | 49 | 78 |
| Cyfra TFPI CEACAM5 OPN MDK | 5 | 0.92 | 43 | 78 |
| Cyfra TIMP1 MDK | 3 | 0.92 | 72 | 44 |
| Cyfra TFPI OPN MDK | 4 | 0.92 | 66 | 67 |
| SLPI TIMP1 SCC TFPI CEACAM5 MMP2 OPN | 7 | 0.92 | 68 | 78 |
| SLPI OPN MDK | 3 | 0.92 | 79 | 67 |
| SLPI TFPI CEACAM5 OPN MDK | 5 | 0.92 | 49 | 78 |
| Cyfra SLPI TIMP1 SCC TFPI CEACAM5 MMP2 | 7 | 0.92 | 66 | 78 |
| Cyfra TIMP1 SCC TFPI CEACAM5 MMP2 OPN | 7 | 0.92 | 66 | 78 |
| SCC TFPI MMP2 MDK | 4 | 0.92 | 81 | 56 |
| Cyfra SLPI TFPI CEACAM5 MDK | 5 | 0.92 | 40 | 78 |
| TFPI CEACAM5 MDK | 3 | 0.92 | 62 | 33 |
| TIMP1 CEACAM5 MDK | 3 | 0.92 | 58 | 33 |
| TIMP1 SCC OPN | 3 | 0.91 | 66 | 78 |
| Cyfra SLPI TIMP1 TFPI CEACAM5 MMP2 MDK | 7 | 0.91 | 66 | 78 |
| Cyfra TIMP1 TFPI CEACAM5 MMP2 OPN MDK | 7 | 0.91 | 66 | 78 |
| SLPI TIMP1 TFPI CEACAM5 MMP2 OPN MDK | 7 | 0.91 | 68 | 78 |
| Cyfra TIMP1 TFPI CEACAM5 MMP2 MDK | 6 | 0.91 | 70 | 67 |
| SLPI TIMP1 TFPI CEACAM5 MMP2 MDK | 6 | 0.91 | 72 | 67 |
| TIMP1 TFPI CEACAM5 MMP2 OPN MDK | 6 | 0.91 | 72 | 67 |
| SLPI TIMP1 SCC TFPI CEACAM5 MMP2 | 6 | 0.91 | 72 | 67 |
| TIMP1 SCC TFPI CEACAM5 MMP2 OPN | 6 | 0.91 | 72 | 67 |
| Cyfra SLPI SCC MMP2 MDK | 5 | 0.91 | 75 | 67 |
| Cyfra SCC MMP2 OPN MDK | 5 | 0.91 | 75 | 67 |
| TIMP1 TFPI MMP2 OPN MDK | 5 | 0.91 | 53 | 78 |
| Cyfra SLPI TIMP1 SCC TFPI OPN | 6 | 0.91 | 42 | 67 |
| SLPI CEACAM5 OPN MDK | 4 | 0.91 | 49 | 67 |
| SCC MDK | 2 | 0.91 | 77 | 67 |
| SLPI SCC MMP2 OPN MDK | 5 | 0.91 | 77 | 56 |
| Cyfra SCC CEACAM5 MMP2 MDK | 5 | 0.91 | 68 | 33 |
| Cyfra CEACAMS OPN MDK | 4 | 0.91 | 43 | 67 |
| Cyfra SLPI TFPI MDK | 4 | 0.91 | 62 | 67 |
| SLPI TIMP1 TFPI MMP2 OPN MDK | 6 | 0.91 | 49 | 78 |
| SLPI SCC MMP2 MDK | 4 | 0.91 | 81 | 56 |
| SCC MMP2 OPN MDK | 4 | 0.91 | 81 | 56 |
| SLPI TIMP1 TFPI MMP2 MDK | 5 | 0.91 | 49 | 78 |
| Cyfra SLPI TIMP1 TFPI CEACAM5 MMP2 OPN MDK | 8 | 0.91 | 62 | 78 |
| Cyfra SLPI TIMP1 SCC TFPI CEACAM5 MMP2 OPN | 8 | 0.91 | 62 | 67 |
| SLPI TIMP1 CEACAM5 MMP2 OPN MDK | 6 | 0.91 | 68 | 67 |
| Cyfra TIMP1 SCC TFPI CEACAM5 MMP2 | 6 | 0.91 | 70 | 67 |
| Cyfra SCC MMP2 MDK | 4 | 0.91 | 79 | 56 |
| Cyfra SLPI TFPI CEACAM5 OPN MDK | 6 | 0.91 | 38 | 67 |
| Cyfra SLPI CEACAM5 MDK | 4 | 0.91 | 40 | 67 |
| SLPI TIMP1 SCC | 3 | 0.91 | 62 | 78 |
| TIMP1 SCC TFPI MMP2 OPN | 5 | 0.91 | 45 | 78 |
| TIMP1 TFPI CEACAM5 MMP2 MDK | 5 | 0.91 | 77 | 56 |
| Cyfra TIMP1 TFPI MMP2 OPN MDK | 6 | 0.91 | 43 | 78 |
| Cyfra CEACAM5 MDK | 3 | 0.91 | 49 | 22 |
| Cyfra SLPI TIMP1 CEACAM5 MMP2 MDK | 6 | 0.90 | 66 | 67 |
| Cyfra TIMP1 CEACAM5 MMP2 OPN MDK | 6 | 0.90 | 66 | 67 |
| TIMP1 TFPI MMP2 MDK | 4 | 0.90 | 55 | 56 |
| Cyfra SLPI TFPI OPN MDK | 5 | 0.90 | 60 | 67 |
| SLPI TIMP1 SCC TFPI MMP2 | 5 | 0.90 | 42 | 78 |
| Cyfra SLPI TIMP1 TFPI MMP2 MDK | 6 | 0.90 | 40 | 78 |
| Cyfra SLPI SCC MMP2 OPN MDK | 6 | 0.90 | 72 | 67 |
| Cyfra TIMP1 TFPI MMP2 MDK | 5 | 0.90 | 45 | 44 |
| TIMP1 MMP2 OPN MDK | 4 | 0.90 | 53 | 67 |
| SCC TFPI CEACAM5 MMP2 MDK | 5 | 0.90 | 75 | 33 |
| SLPI TIMP1 MMP2 OPN MDK | 5 | 0.90 | 49 | 56 |
| SCC TFPI CEACAM5 OPN | 4 | 0.90 | 49 | 56 |
| Cyfra SLPI TIMP1 CEACAM5 MMP2 OPN MDK | 7 | 0.90 | 62 | 78 |
| Cyfra SLPI CEACAM5 OPN MDK | 5 | 0.90 | 38 | 67 |
| SLPI TIMP1 MMP2 MDK | 4 | 0.90 | 49 | 67 |
| SLPI TIMP1 SCC OPN | 4 | 0.90 | 60 | 67 |
| Cyfra SLPI TIMP1 TFPI MMP2 OPN MDK | 7 | 0.90 | 40 | 78 |
| Cyfra OPN MDK | 3 | 0.90 | 70 | 67 |
| Cyfra TIMP1 SCC TFPI MMP2 | 5 | 0.90 | 38 | 78 . |
| Cyfra TIMP1 MMP2 OPN MDK | 5 | 0.90 | 43 | 44 |
| SCC CEACAM5 MDK | 3 | 0.90 | 53 | 33 |
| SLPI SCC TFPI CEACAM5 | 4 | 0.90 | 45 | 56 |
| Cyfra SLPI TIMP1 SCC CEACAM5 MMP2 | 6 | 0.89 | 66 | 56 |
| Cyfra TIMP1 SCC CEACAM5 MMP2 OPN | 6 | 0.89 | 66 | 56 |
| SLPI TIMP1 SCC CEACAM5 MMP2 OPN | 6 | 0.89 | 68 | 56 |
| Cyfra TIMP1 SCC TFPI MMP2 OPN | 6 | 0.89 | 36 | 67 |
| SCC TFPI OPN | 3 | 0.89 | 68 | 67 |
| SLPI TIMP1 CEACAM5 MMP2 MDK | 5 | 0.89 | 72 | 67 |
| TIMP1 CEACAM5 MMP2 OPN MDK | 5 | 0.89 | 72 | 67 |
| SLPI TIMP1 SCC TFPI MMP2 OPN | 6 | 0.89 | 42 | 67 |
| SLPI SCC TFPI CEACAM5 OPN | 5 | 0.89 | 43 | 67 |
| Cyfra SLPI TIMP1 SCC CEACAM5 MMP2 OPN | 7 | 0.89 | 62 | 67 |
| Cyfra SLPI TIMP1 MMP2 MDK | 5 | 0.89 | 40 | 44 |
| Cyfra MDK | 2 | 0.89 | 75 | 56 |
| Cyfra SCC TFPI CEACAM5 OPN | 5 | 0.89 | 38 | 67 |
| Cyfra SLPI TIMP1 SCC TFPI MMP2 | 6 | 0.89 | 32 | 67 |
| Cyfra SLPI TIMP1 MMP2 OPN MDK | 6 | 0.89 | 40 | 67 |
| SLPI TFPI CEACAM5 MMP2 OPN MDK | 6 | 0.89 | 40 | 78 |
| Cyfra SLPI SCC TFPI CEACAM5 | 5 | 0.89 | 34 | 67 |
| Cyfra TIMP1 SCC OPN | 4 | 0.88 | 51 | 78 |
| Cyfra SLPI TIMP1 SCC TFPI MMP2 OPN | 7 | 0.88 | 32 | 67 |
| Cyfra SLPI MDK | 3 | 0.88 | 66 | 67 |
| Cyfra TIMP1 CEACAM5 MMP2 MDK | 5 | 0.88 | 70 | 56 |
| TFPI CEACAM5 MMP2 OPN MDK | 5 | 0.88 | 43 | 56 |
| Cyfra TIMP1 MMP2 MDK | 4 | 0.88 | 45 | 56 |
| Cyfra SCC TFPI CEACAM5 | 4 | 0.88 | 43 | 56 |
| TFPI MMP2 OPN MDK | 4 | 0.88 | 57 | 67 |
| Cyfra TFPI CEACAM5 MMP2 OPN MDK | 6 | 0.88 | 36 | 78 |
| SLPI SCC TFPI | 3 | 0.88 | 64 | 67 |
| TIMP1 SCC TFPI CEACAM5 MMP2 | 5 | 0.88 | 77 | 33 |
| SLPI TFPI MMP2 OPN MDK | 5 | 0.88 | 53 | 56 |
| SLPI TFPI CEACAM5 MMP2 MDK | 5 | 0.88 | 40 | 56 |
| Cyfra SLPI SCC TFPI CEACAM5 OPN | 6 | 0.88 | 32 | 67 |
| TIMP1 TFPI CEACAM5 OPN | 4 | 0.88 | 53 | 67 |
| Cyfra SLPI TFPI CEACAM5 MMP2 MDK | 6 | 0.88 | 32 | 78 |
| SLPI CEACAM5 MMP2 OPN MDK | 5 | 0.88 | 40 | 56 |
| Cyfra SLPI OPN MDK | 4 | 0.88 | 64 | 67 |
| Cyfra SLPI TIMP1 SCC | 4 | 0.87 | 47 | 78 |
| Cyfra SLPI TFPI CEACAM5 MMP2 OPN MDK | 7 | 0.87 | 32 | 67 |
| SLPI SCC TFPI OPN | 4 | 0.87 | 62 | 67 |
| SLPI TIMP1 SCC CEACAM5 MMP2 | 5 | 0.87 | 72 | 33 |
| TIMP1 SCC CEACAM5 MMP2 OPN | 5 | 0.87 | 72 | 33 |
| SLPI TFPI MMP2 MDK | 4 | 0.87 | 53 | 67 |
| Cyfra TFPI CEACAM5 MMP2 MDK | 5 | 0.87 | 38 | 56 |
| SLPI TIMP1 TFPI CEACAM5 OPN | 5 | 0.87 | 47 | 78 |
| Cyfra TFPI MMP2 OPN MDK | 5 | 0.87 | 47 | 56 |
| Cyfra CEACAM5 MMP2 OPN MDK | 5 | 0.87 | 36 | 56 |
| SLPI TIMP1 SCC MMP2 OPN | 5 | 0.87 | 42 | 44 |
| SLPI TIMP1 TFPI CEACAM5 | 4 | 0.87 | 49 | 67 |
| Cyfra SLPI TIMP1 SCC OPN | 5 | 0.87 | 45 | 67 |
| Cyfra SLPI CEACAM5 MMP2 MDK | 5 | 0.87 | 32 | 56 |
| Cyfra SCC TFPI | 3 | 0.87 | 62 | 67 |
| SLPI SCC TFPI CEACAM5 MMP2 OPN | 6 | 0.87 | 34 | 67 |
| TIMP1 SCC TFPI MMP2 | 4 | 0.87 | 47 | 44 |
| Cyfra TIMP1 SCC MMP2 OPN | 5 | 0.87 | 36 | 67 |
| CEACAM5 MMP2 OPN MDK | 4 | 0.87 | 43 | 67 |
| TIMP1 MMP2 MDK | 3 | 0.86 | 55 | 33 |
| Cyfra TFPI MMP2 MDK | 4 | 0.86 | 49 | 67 |
| Cyfra SLPI TFPI MMP2 MDK | 5 | 0.86 | 43 | 56 |
| Cyfra SLPI TFPI MMP2 OPN MDK | 6 | 0.86 | 43 | 67 |
| Cyfra SLPI CEACAM5 MMP2 OPN MDK | 6 | 0.86 | 32 | 67 |
| Cyfra SCC TFPI CEACAM5 MMP2 OPN | 6 | 0.86 | 30 | 67 |
| SLPI CEACAM5 MMP2 MDK | 4 | 0.86 | 40 | 67 |
| Cyfra SLPI TIMP1 SCC MMP2 | 5 | 0.86 | 32 | 67 |
| TIMP1 CEACAM5 MMP2 MDK | 4 | 0.86 | 42 | 67 |
| Cyfra SLPI SCC TFPI CEACAM5 MMP2 OPN | 7 | 0.86 | 26 | 67 |
| Cyfra TIMP1 TFPI CEACAM5 OPN | 5 | 0.86 | 42 | 78 |
| Cyfra SLPI SCC TFPI CEACAM5 MMP2 | 6 | 0.86 | 26 | 67 |
| SCC CEACAM5 MMP2 MDK | 4 | 0.86 | 75 | 11 |
| Cyfra TIMP1 SCC CEACAM5 MMP2 | 5 | 0.86 | 70 | 22 |
| TIMP1 SCC MMP2 OPN | 4 | 0.86 | 45 | 44 |
| SCC MMP2 MDK | 3 | 0.86 | 51 | 22 |
| MMP2 OPN MDK | 3 | 0.86 | 57 | 33 |
| SLPI TIMP1 CEACAM5 OPN | 4 | 0.86 | 47 | 56 |
| SLPI MMP2 OPN MDK | 4 | 0.86 | 53 | 33 |
| TIMP1 TFPI OPN | 3 | 0.86 | 0 | 78 |
| Cyfra TIMP1 SCC | 3 | 0.86 | 57 | 67 |
| Cyfra TIMP1 TFPI CEACAM5 | 4 | 0.85 | 47 | 56 |
| SLPI TIMP1 SCC MMP2 | 4 | 0.85 | 42 | 44 |
| Cyfra SLPI TIMP1 SCC MMP2 OPN | 6 | 0.85 | 32 | 67 |
| SLPI TIMP1 TFPI | 3 | 0.85 | 0 | 78 |
| Cyfra SLPI TIMP1 TFPI CEACAM5 | 5 | 0.85 | 38 | 78 |
| Cyfra MMP2 OPN MDK | 4 | 0.85 | 47 | 67 |
| SCC TFPI | 2 | 0.85 | 77 | 33 |
| Cyfra SCC TFPI OPN | 4 | 0.85 | 55 | 67 |
| SLPI MMP2 MDK | 3 | 0.85 | 53 | 33 |
| Cyfra SLPI TIMP1 TFPI CEACAM5 OPN | 6 | 0.85 | 36 | 67 |
| TFPI CEACAM5 MMP2 MDK | 4 | 0.84 | 45 | 22 |
| SLPI SCC TFPI MMP2 OPN | 5 | 0.84 | 47 | 44 |
| SLPI TIMP1 TFPI OPN | 4 | 0.84 | 0 | 67 |
| Cyfra CEACAM5 MMP2 MDK | 4 | 0.84 | 38 | 67 |
| Cyfra TIMP1 CEACAM5 OPN | 4 | 0.84 | 42 | 44 |
| Cyfra SLPI MMP2 MDK | 4 | 0.84 | 43 | 67 |
| TFPI MMP2 MDK | 3 | 0.84 | 58 | 44 |
| SLPI SCC CEACAMS OPN | 4 | 0.84 | 43 | 56 |
| TIMP1 SCC CEACAM5 | 3 | 0.84 | 49 | 11 |
| Cyfra TIMP1 TFPI OPN | 4 | 0.84 | 0 | 56 |
| SLPI TIMP1 TFPI CEACAM5 MMP2 OPN | 6 | 0.84 | 38 | 67 |
| Cyfra SLPI MMP2 OPN MDK | 5 | 0.84 | 43 | 67 |
| Cyfra SLPI SCC TFPI | 4 | 0.84 | 51 | 67 |
| Cyfra SCC TFPI MMP2 OPN | 5 | 0.83 | 42 | 67 |
| TIMP1 TFPI CEACAM5 | 3 | 0.83 | 60 | 22 |
| Cyfra SLPI TIMP1 TFPI | 4 | 0.83 | 0 | 56 |
| Cyfra SLPI TIMP1 CEACAM5 | 4 | 0.83 | 38 | 44 |
| Cyfra SLPI TIMP1 CEACAM5 OPN | 5 | 0.83 | 36 | 67 |
| Cyfra SCC CEACAM5 OPN | 4 | 0.83 | 38 | 56 |
| Cyfra TIMP1 TFPI CEACAM5 MMP2 OPN | 6 | 0.83 | 34 | 67 |
| TIMP1 CEACAM5 OPN | 3 | 0.83 | 53 | 67 |
| Cyfra SLPI SCC TFPI OPN | 5 | 0.83 | 49 | 67 |
| SCC TFPI CEACAM5 | 3 | 0.83 | 57 | 11 |
| Cyfra SLPI TIMP1 TFPI OPN | 5 | 0.83 | 0 | 67 |
| Cyfra SLPI TIMP1 TFPI CEACAM5 MMP2 OPN | 7 | 0.83 | 30 | 56 |
| SCC TFPI CEACAM5 MMP2 OPN | 5 | 0.83 | 38 | 22 |
| Cyfra SLPI SCC TFPI MMP2 | 5 | 0.83 | 38 | 67 |
| Cyfra SLPI SCC CEACAM5 OPN | 5 | 0.83 | 32 | 67 |
| Cyfra SLPI SCC TFPI MMP2 OPN | 6 | 0.83 | 38 | 67 |
| Cyfra SLPI SCC CEACAM5 MMP2 OPN | 6 | 0.83 | 26 | 67 |
| Cyfra SCC TFPI CEACAM5 MMP2 | 5 | 0.83 | 32 | 22 |
| Cyfra TIMP1 SCC MMP2 | 4 | 0.83 | 38 | 33 |
| Cyfra MMP2 MDK | 3 | 0.82 | 49 | 22 |
| Cyfra SLPI SCC CEACAM5 | 4 | 0.82 | 34 | 56 |
| TIMP1 SCC | 2 | 0.82 | 74 | 33 |
| SLPI SCC TFPI CEACAM5 MMP2 | 5 | 0.82 | 34 | 22 |
| SCC TFPI MMP2 OPN | 4 | 0.82 | 51 | 44 |
| Cyfra SLPI TIMP1 TFPI CEACAM5 MMP2 | 6 | 0.82 | 30 | 67 |
| TIMP1 TFPI CEACAM5 MMP2 OPN | 5 | 0.82 | 42 | 56 |
| TIMP1 TFPI | 2 | 0.82 | 0 | 44 |
| CEACAM5 MDK | 2 | 0.82 | 62 | 56 |
| SLPI TIMP1 CEACAM5 | 3 | 0.82 | 49 | 67 |
| SLPI SCC CEACAM5 MMP2 OPN | 5 | 0.82 | 34 | 33 |
| SLPI TFPI CEACAM5 OPN | 4 | 0.82 | 0 | 67 |
| Cyfra SLPI TIMP1 CEACAM5 MMP2 OPN | 6 | 0.82 | 30 | 56 |
| Cyfra TIMP1 TFPI | 3 | 0.82 | 0 | 67 |
| SLPI SCC TFPI MMP2 | 4 | 0.81 | 47 | 44 |
| SLPI TIMP1 OPN | 3 | 0.81 | 0 | 44 |
| SLPI TIMP1 TFPI CEACAM5 MMP2 | 5 | 0.81 | 38 | 56 |
| Cyfra SCC CEACAM5 MMP2 OPN | 5 | 0.81 | 30 | 33 |
| SLPI TIMP1 TFPI MMP2 OPN | 5 | 0.81 | 0 | 44 |
| SLPI TIMP1 CEACAM5 MMP2 OPN | 5 | 0.81 | 38 | 56 |
| Cyfra TFPI CEACAM5 OPN | 4 | 0.81 | 0 | 67 |
| Cyfra SLPI TFPI CEACAM5 OPN | 5 | 0.81 | 0 | 67 |
| Cyfra SLPI SCC CEACAM5 MMP2 | 5 | 0.81 | 26 | 33 |
| Cyfra SLPI TFPI CEACAM5 | 4 | 0.81 | 0 | 67 |
| Cyfra SCC TFPI MMP2 | 4 | 0.81 | 43 | 44 |
| TIMP1 OPN | 2 | 0.81 | 0 | 44 |
| Cyfra TIMP1 TFPI MMP2 OPN | 5 | 0.80 | 0 | 33 |
| TFPI CEACAM5 OPN | 3 | 0.80 | 0 | 22 |
| Cyfra SLPI TIMP1 TFPI MMP2 OPN | 6 | 0.80 | 0 | 67 |
| SLPI SCC OPN | 3 | 0.80 | 0 | 44 |
| Cyfra TIMP1 TFPI CEACAM5 MMP2 | 5 | 0.80 | 36 | 44 |
| SLPI TFPI CEACAM5 | 3 | 0.80 | 0 | 22 |
| Cyfra SLPI TIMP1 TFPI MMP2 | 5 | 0.80 | 0 | 33 |
| TIMP1 TFPI MMP2 OPN | 4 | 0.80 | 0 | 56 |
| Cyfra TIMP1 CEACAM5 MMP2 OPN | 5 | 0.80 | 34 | 44 |
| SCC CEACAM5 OPN | 3 | 0.80 | 49 | 22 |
| SLPI TIMP1 TFPI MMP2 | 4 | 0.79 | 0 | 56 |
| Cyfra SLPI TFPI CEACAM5 MMP2 OPN | 6 | 0.79 | 0 | 44 |
| Cyfra SLPI TIMP1 OPN | 4 | 0.79 | 0 | 56 |
| TIMP1 CEACAM5 MMP2 OPN | 4 | 0.79 | 42 | 67 |
| TIMP1 SCC CEACAM5 MMP2 | 4 | 0.79 | 34 | 0 |
| SCC TFPI CEACAM5 MMP2 | 4 | 0.79 | 40 | 11 |
| Cyfra SCC OPN | 3 | 0.79 | 0 | 67 |
| SLPI TIMP1 | 2 | 0.79 | 0 | 44 |
| Cyfra SLPI TIMP1 CEACAM5 MMP2 | 5 | 0.79 | 30 | 44 |
| Cyfra TFPI CEACAM5 | 3 | 0.79 | 0 | 11 |
| SLPI SCC CEACAM5 | 3 | 0.78 | 45 | 22 |
| SLPI SCC MMP2 OPN | 4 | 0.78 | 47 | 22 |
| SLPI TFPI CEACAM5 MMP2 OPN | 5 | 0.78 | 0 | 44 |
| Cyfra TFPI CEACAM5 MMP2 OPN | 5 | 0.78 | 0 | 44 |
| SLPI TIMP1 MMP2 OPN | 4 | 0.78 | 0 | 22 |
| Cyfra SLPI TIMP1 MMP2 OPN | 5 | 0.78 | 0 | 44 |
| Cyfra SLPI SCC | 3 | 0.78 | 0 | 67 |
| SLPI TIMP1 CEACAM5 MMP2 | 4 | 0.78 | 38 | 67 |
| Cyfra SLPI TFPI CEACAM5 MMP2 | 5 | 0.78 | 0 | 44 |
| Cyfra SLPI SCC OPN | 4 | 0.78 | 0 | 67 |
| Cyfra SLPI SCC MMP2 OPN | 5 | 0.78 | 38 | 56 |
| Cyfra TIMP1 OPN | 3 | 0.78 | 0 | 56 |
| Cyfra TIMP1 TFPI MMP2 | 4 | 0.78 | 0 | 44 |
| Cyfra SCC MMP2 OPN | 4 | 0.77 | 42 | 56 |
| SCC OPN | 2 | 0.77 | 0 | 44 |
| Cyfra TIMP1 MMP2 OPN | 4 | 0.77 | 0 | 44 |
| SCC CEACAM5 MMP2 OPN | 4 | 0.77 | 38 | 11 |
| Cyfra SLPI TIMP1 MMP2 | 4 | 0.77 | 0 | 44 |
| Cyfra SLPI TIMP1 | 3 | 0.77 | 0 | 56 |
| SLPI SCC CEACAM5 MMP2 | 4 | 0.76 | 34 | 11 |
| TIMP1 SCC MMP2 | 3 | 0.76 | 47 | 11 |
| Cyfra SLPI SCC MMP2 | 4 | 0.76 | 38 | 56 |
| Cyfra SCC CEACAM5 | 3 | 0.76 | 43 | 11 |
| CEACAM5 MMP2 MDK | 3 | 0.76 | 45 | 22 |
| SLPI CEACAM5 OPN | 3 | 0.76 | 0 | 33 |
| SLPI SCC | 2 | 0.75 | 0 | 44 |
| TIMP1 TFPI CEACAM5 MMP2 | 4 | 0.75 | 43 | 11 |
| MMP2 MDK | 2 | 0.75 | 58 | 44 |
| Cyfra SLPI CEACAM5 MMP2 OPN | 5 | 0.75 | 0 | 56 |
| Cyfra SLPI CEACAM5 OPN | 4 | 0.75 | 0 | 67 |
| SLPI TFPI OPN | 3 | 0.75 | 0 | 56 |
| Cyfra TIMP1 CEACAM5 | 3 | 0.75 | 47 | 22 |
| SCC TFPI MMP2 | 3 | 0.75 | 53 | 22 |
| TFPI OPN | 2 | 0.74 | 0 | 56 |
| TFPI CEACAM5 MMP2 OPN | 4 | 0.74 | 0 | 11 |
| TIMP1 MMP2 OPN | 3 | 0.74 | 0 | 22 |
| Cyfra CEACAM5 OPN | 3 | 0.74 | 0 | 22 |
| Cyfra SCC CEACAM5 MMP2 | 4 | 0.74 | 32 | 0 |
| Cyfra SLPI TFPI MMP2 OPN | 5 | 0.74 | 0 | 56 |
| SLPI TFPI CEACAM5 MMP2 | 4 | 0.74 | 0 | 11 |
| Cyfra TFPI OPN | 3 | 0.74 | 0 | 67 |
| SLPI CEACAM5 MMP2 OPN | 4 | 0.74 | 0 | 11 |
| Cyfra SLPI TFPI OPN | 4 | 0.74 | 0 | 67 |
| SLPI TFPI | 2 | 0.74 | 0 | 56 |
| SLPI TIMP1 MMP2 | 3 | 0.73 | 0 | 22 |
| Cyfra SLPI CEACAM5 | 3 | 0.73 | 0 | 22 |
| Cyfra TFPI CEACAM5 MMP2 | 4 | 0.73 | 0 | 0 |
| Cyfra SLPI TFPI | 3 | 0.73 | 0 | 67 |
| Cyfra TIMP1 CEACAM5 MMP2 | 4 | 0.73 | 36 | 56 |
| SCC MMP2 OPN | 3 | 0.73 | 51 | 22 |
| SLPI TFPI MMP2 OPN | 4 | 0.73 | 0 | 33 |
| Cyfra SLPI TFPI MMP2 | 4 | 0.73 | 0 | 56 |
| Cyfra TFPI MMP2 OPN | 4 | 0.73 | 0 | 56 |
| TIMP1 TFPI MMP2 | 3 | 0.72 | 0 | 33 |
| Cyfra CEACAM5 MMP2 OPN | 4 | 0.72 | 0 | 0 |
| Cyfra SLPI CEACAM5 MMP2 | 4 | 0.72 | 0 | 0 |
| SLPI SCC MMP2 | 3 | 0.71 | 47 | 22 |
| Cyfra TFPI | 2 | 0.70 | 0 | 44 |
| Cyfra SCC | 2 | 0.70 | 0 | 33 |
| TFPI CEACAM5 | 2 | 0.69 | 0 | 0 |
| Cyfra TIMP1 | 2 | 0.69 | 0 | 44 |
| Cyfra SLPI MMP2 OPN | 4 | 0.69 | 0 | 22 |
| TFPI MMP2 OPN | 3 | 0.69 | 0 | 33 |
| SLPI TFPI MMP2 | 3 | 0.69 | 0 | 33 |
| Cyfra SCC MMP2 | 3 | 0.68 | 43 | 11 |
| Cyfra TIMP1 MMP2 | 3 | 0.68 | 0 | 22 |
| SLPI MMP2 OPN | 3 | 0.67 | 0 | 0 |
| Cyfra TFPI MMP2 | 3 | 0.67 | 0 | 22 |
| TIMP1 CEACAM5 | 2 | 0.66 | 0 | 33 |
| CEACAM5 OPN | 2 | 0.66 | 0 | 33 |
| Cyfra MMP2 OPN | 3 | 0.66 | 0 | 22 |
| Cyfra SLPI MMP2 | 3 | 0.66 | 0 | 22 |
| Cyfra SLPI OPN | 3 | 0.65 | 0 | 56 |
| TIMP1 CEACAM5 MMP2 | 3 | 0.65 | 0 | 11 |
| TFPI CEACAM5 MMP2 | 3 | 0.64 | 0 | 0 |
| SLPI CEACAM5 | 2 | 0.64 | 0 | 33 |
| CEACAM5 MMP2 OPN | 3 | 0.63 | 0 | 11 |
| SCC CEACAM5 MMP2 | 3 | 0.63 | 40 | 0 |
| SCC CEACAM5 | 2 | 0.63 | 0 | 0 |
| TFPI | 1 | 0.63 | 0 | 0 |
| Cyfra OPN | 2 | 0.63 | 0 | 56 |
| SLPI CEACAM5 MMP2 | 3 | 0.62 | 0 | 11 |
| Cyfra SLPI | 2 | 0.62 | 0 | 56 |
| SLPI OPN | 2 | 0.62 | 0 | 0 |
| TIMP1 | 1 | 0.60 | 0 | 0 |
| OPN | 1 | 0.60 | 0 | 0 |
| Cyfra CEACAM5 MMP2 | 3 | 0.58 | 0 | 0 |
| SLPI | 1 | 0.58 | 0 | 0 |
| Cyfra CEACAM5 | 2 | 0.58 | 0 | 22 |
| MMP2 OPN | 2 | 0.58 | 0 | 0 |
| TIMP1 MMP2 | 2 | 0.57 | 0 | 33 |
| SLPI MMP2 | 2 | 0.56 | 0 | 0 |
| SCC MMP2 | 2 | 0.56 | 0 | 0 |
| TFPI MMP2 | 2 | 0.56 | 0 | 0 |
| SCC | 1 | 0.55 | 0 | 0 |
| Cyfra MMP2 | 2 | 0.50 | 0 | 0 |
| Cyfra | 1 | 0.46 | 0 | 0 |
| CEACAM5 MMP2 | 2 | 0.45 | 0 | 0 |
| CEACAM5 | 1 | 0.38 | 0 | 0 |
| MMP2 | 1 | 0.28 | 0 | 0 |

**TABLE 11**

| Small Cell vs. Normal | Split-point | | | |
|---|---|---|---|---|
| | Trained/tested on 44x44 | | | |
| Panel | Size | AUC | Sens at 95% Spec | Spec at 95% Sens |
| TIMP1 TFPI CEACAM5 MMP2 CAIX GRP | 6 | 0.98 | 80 | 91 |
| TIMP1 CEACAM5 MMP2 Ca19-9 CAIX GRP | 6 | 0.98 | 80 | 91 |
| TIMP1 CEACAM5 MMP2 CA242 CAIX GRP | 6 | 0.98 | 80 | 91 |
| SLPI TIMP1 TFPI CEACAM5 MMP2 CAIX GRP | 7 | 0.98 | 80 | 91 |
| SLPI TIMP1 CEACAM5 MMP2 Ca19-9 CAIX GRP | 7 | 0.98 | 80 | 91 |
| SLPI TIMP1 CEACAM5 MMP2 CA242 CAIX GRP | 7 | 0.98 | 80 | 91 |
| TIMP1 TFPI CEACAM5 MMP2 Ca19-9 CAIX GRP | 7 | 0.98 | 80 | 91 |
| TIMP1 TFPI CEACAM5 MMP2 CA242 CAIX GRP | 7 | 0.98 | 80 | 91 |
| TIMP1 CEACAM5 MMP2 Ca19-9 CA242 CAIX GRP | 7 | 0.98 | 80 | 91 |
| SLPI TIMP1 TFPI CEACAM5 MMP2 Ca19-9 CAIX GRP | 8 | 0.98 | 80 | 91 |
| SLPI TIMP1 TFPI CEACAM5 MMP2 CA242 CAIX GRP | 8 | 0.98 | 80 | 91 |
| SLPI TIMP1 CEACAM5 MMP2 Ca19-9 CA242 CAIX GRP | 8 | 0.98 | 80 | 91 |
| TIMP1 TFPI CEACAM5 MMP2 Ca19-9 CA242 CAIX GRP | 8 | 0.98 | 80 | 91 |
| SLPI TIMP1 TFPI CEACAM5 MMP2 Ca19-9 CA242 CAIX GRP | 9 | 0.98 | 80 | 91 |
| TIMP1 TFPI CEACAM5 CAIX | 4 | 0.97 | 60 | 93 |
| TIMP1 CEACAM5 Ca19-9 CAIX | 4 | 0.97 | 60 | 93 |
| TIMP1 CEACAM5 CA242 CAIX | 4 | 0.97 | 60 | 93 |
| Cyfra TIMP1 CEACAM5 MMP2 GRP | 5 | 0.97 | 80 | 89 |
| SLPI TIMP1 TFPI CEACAM5 CAIX | 5 | 0.97 | 60 | 93 |
| SLPI TIMP1 CEACAM5 Ca19-9 CAIX | 5 | 0.97 | 60 | 93 |
| SLPI TIMP1 CEACAM5 CA242 CAIX | 5 | 0.97 | 60 | 93 |
| TIMP1 TFPI CEACAM5 MMP2 GRP | 5 | 0.97 | 80 | 91 |
| TIMP1 TFPI CEACAM5 Ca19-9 CAIX | 5 | 0.97 | 60 | 93 |
| TIMP1 TFPI CEACAM5 CA242 CAIX | 5 | 0.97 | 60 | 93 |
| TIMP1 CEACAM5 MMP2 Ca19-9 GRP | 5 | 0.97 | 80 | 91 |
| TIMP1 CEACAM5 MMP2 CA242 GRP | 5 | 0.97 | 80 | 91 |
| TIMP1 CEACAM5 MMP2 CAIX GRP | 5 | 0.97 | 80 | 91 |
| TIMP1 CEACAM5 Ca19-9 CA242 CAIX | 5 | 0.97 | 60 | 93 |
| Cyfra SLPI TIMP1 CEACAM5 MMP2 GRP | 6 | 0.97 | 80 | 89 |
| Cyfra TIMP1 TFPI CEACAM5 MMP2 CAIX | 6 | 0.97 | 80 | 91 |
| Cyfra TIMP1 TFPI CEACAM5 MMP2 GRP | 6 | 0.97 | 80 | 89 |
| Cyfra TIMP1 CEACAM5 MMP2 Ca19-9 CAIX | 6 | 0.97 | 80 | 91 |
| Cyfra TIMP1 CEACAM5 MMP2 Ca19-9 GRP | 6 | 0.97 | 80 | 89 |
| Cyfra TIMP1 CEACAM5 MMP2 CA242 CAIX | 6 | 0.97 | 80 | 91 |
| Cyfra TIMP1 CEACAM5 MMP2 CA242 GRP | 6 | 0.97 | 80 | 89 |
| Cyfra TIMP1 CEACAM5 MMP2 CAIX GRP | 6 | 0.97 | 80 | 89 |
| SLPI TIMP1 TFPI CEACAM5 MMP2 GRP | 6 | 0.97 | 80 | 91 |
| SLPI TIMP1 TFPI CEACAM5 Ca19-9 CAIX | 6 | 0.97 | 60 | 93 |
| SLPI TIMP1 TFPI CEACAM5 CA242 CAIX | 6 | 0.97 | 60 | 93 |
| SLPI TIMP1 CEACAM5 MMP2 Ca19-9 GRP | 6 | 0.97 | 80 | 91 |
| SLPI TIMP1 CEACAM5 MMP2 CA242 GRP | 6 | 0.97 | 80 | 91 |
| SLPI TIMP1 CEACAM5 MMP2 CAIX GRP | 6 | 0.97 | 80 | 91 |
| SLPI TIMP1 CEACAM5 Ca19-9 CA242 CAIX | 6 | 0.97 | 60 | 93 |
| TIMP1 TFPI CEACAM5 MMP2 Ca19-9 CAIX | 6 | 0.97 | 80 | 93 |
| TIMP1 TFPI CEACAM5 MMP2 Ca19-9 GRP | 6 | 0.97 | 80 | 91 |
| TIMP1 TFPI CEACAM5 MMP2 CA242 CAIX | 6 | 0.97 | 80 | 93 |
| TIMP1 TFPI CEACAM5 MMP2 CA242 GRP | 6 | 0.97 | 80 | 91 |
| TIMP1 TFPI CEACAM5 Ca19-9 CA242 CAIX | 6 | 0.97 | 60 | 93 |
| TIMP1 CEACAM5 MMP2 Ca19-9 CA242 CAIX | 6 | 0.97 | 80 | 93 |
| TIMP1 CEACAM5 MMP2 Ca19-9 CA242 GRP | 6 | 0.97 | 80 | 91 |
| Cyfra SLPI TIMP1 TFPI CEACAM5 MMP2 CAIX | 7 | 0.97 | 80 | 91 |
| Cyfra SLPI TIMP1 TFPI CEACAM5 MMP2 GRP | 7 | 0.97 | 80 | 89 |
| Cyfra SLPI TIMP1 CEACAM5 MMP2 Ca19-9 CAIX | 7 | 0.97 | 80 | 91 |
| Cyfra SLPI TIMP1 CEACAM5 MMP2 Ca19-9 GRP | 7 | 0.97 | 80 | 89 |
| Cyfra SLPI TIMP1 CEACAM5 MMP2 CA242 CAIX | 7 | 0.97 | 80 | 91 |
| Cyfra SLPI TIMP1 CEACAM5 MMP2 CA242 GRP | 7 | 0.97 | 80 | 89 |
| Cyfra SLPI TIMP1 CEACAM5 MMP2 CAIX GRP | 7 | 0.97 | 80 | 89 |
| Cyfra TIMP1 TFPI CEACAM5 MMP2 Ca19-9 CAIX | 7 | 0.97 | 80 | 91 |
| Cyfra TIMP1 TFPI CEACAM5 MMP2 Ca19-9 GRP | 7 | 0.97 | 80 | 89 |
| Cyfra TIMP1 TFPI CEACAM5 MMP2 CA242 CAIX | 7 | 0.97 | 80 | 91 |
| Cyfra TIMP1 TFPI CEACAM5 MMP2 CA242 GRP | 7 | 0.97 | 80 | 89 |
| Cyfra TIMP1 TFPI CEACAM5 MMP2 CAIX GRP | 7 | 0.97 | 80 | 89 |
| Cyfra TIMP1 CEACAM5 MMP2 Ca19-9 CA242 CAIX | 7 | 0.97 | 80 | 91 |
| Cyfra TIMP1 CEACAM5 MMP2 Ca19-9 CA242 GRP | 7 | 0.97 | 80 | 89 |
| Cyfra TIMP1 CEACAM5 MMP2 Ca19-9 CAIX GRP | 7 | 0.97 | 80 | 89 |
| Cyfra TIMP1 CEACAM5 MMP2 CA242 CAIX GRP | 7 | 0.97 | 80 | 89 |
| MIF TIMP1 TFPI CEACAM5 MMP2 CAIX GRP | 7 | 0.97 | 80 | 89 |
| MIF TIMP1 CEACAM5 MMP2 Ca19-9 CAIX GRP | 7 | 0.97 | 80 | 89 |
| MIF TIMP1 CEACAM5 MMP2 CA242 CAIX GRP | 7 | 0.97 | 80 | 89 |
| SLPI TIMP1 TFPI CEACAM5 MMP2 Ca19-9 CAIX | 7 | 0.97 | 80 | 93 |
| SLPI TIMP1 TFPI CEACAM5 MMP2 Ca19-9 GRP | 7 | 0.97 | 80 | 91 |
| SLPI TIMP1 TFPI CEACAM5 MMP2 CA242 CAIX | 7 | 0.97 | 80 | 93 |
| SLPI TIMP1 TFPI CEACAM5 MMP2 CA242 GRP | 7 | 0.97 | 80 | 91 |
| SLPI TIMP1 TFPI CEACAM5 Ca19-9 CA242 CAIX | 7 | 0.97 | 60 | 93 |
| SLPI TIMP1 CEACAM5 MMP2 Ca19-9 CA242 CAIX | 7 | 0.97 | 80 | 93 |
| SLPI TIMP1 CEACAM5 MMP2 Ca19-9 CA242 GRP | 7 | 0.97 | 80 | 91 |
| TIMP1 ENO2 TFPI CEACAM5 MMP2 CAIX GRP | 7 | 0.97 | 80 | 89 |
| TIMP1 ENO2 CEACAM5 MMP2 Ca19-9 CAIX GRP | 7 | 0.97 | 80 | 89 |
| TIMP1 ENO2 CEACAM5 MMP2 CA242 CAIX GRP | 7 | 0.97 | 80 | 89 |
| TIMP1 TFPI CEACAM5 MMP2 AMBP CAIX GRP | 7 | 0.97 | 80 | 89 |
| TIMP1 TFPI CEACAM5 MMP2 Ca19-9 CA242 CAIX | 7 | 0.97 | 80 | 93 |
| TIMP1 TFPI CEACAM5 MMP2 Ca19-9 CA242 GRP | 7 | 0.97 | 80 | 91 |
| TIMP1 TFPI CEACAM5 MMP2 CAIX GRP DEFA1 | 7 | 0.97 | 80 | 89 |
| TIMP1 CEACAM5 MMP2 AMBP Ca19-9 CAIX GRP | 7 | 0.97 | 80 | 89 |
| TIMP1 CEACAM5 MMP2 AMBP CA242 CAIX GRP | 7 | 0.97 | 80 | 89 |
| TIMP1 CEACAM5 MMP2 Ca19-9 CAIX GRP DEFA1 | 7 | 0.97 | 80 | 89 |
| TIMP1 CEACAM5 MMP2 CA242 CAIX GRP DEFA1 | 7 | 0.97 | 80 | 89 |
| Cyfra SLPI TIMP1 TFPI CEACAM5 MMP2 Ca19-9 CAIX | 8 | 0.97 | 80 | 91 |
| Cyfra SLPI TIMP1 TFPI CEACAM5 MMP2 Ca19-9 GRP | 8 | 0.97 | 80 | 89 |
| Cyfra SLPI TIMP1 TFPI CEACAM5 MMP2 CA242 CAIX | 8 | 0.97 | 80 | 91 |
| Cyfra SLPI TIMP1 TFPI CEACAM5 MMP2 CA242 GRP | 8 | 0.97 | 80 | 89 |
| Cyfra SLPI TIMP1 TFPI CEACAM5 MMP2 CAIX GRP | 8 | 0.97 | 80 | 89 |
| Cyfra SLPI TIMP1 CEACAM5 MMP2 Ca19-9 CA242 CAIX | 8 | 0.97 | 80 | 91 |
| Cyfra SLPI TIMP1 CEACAM5 MMP2 Ca19-9 CA242 GRP | 8 | 0.97 | 80 | 89 |
| Cyfra SLPI TIMP1 CEACAM5 MMP2 Ca19-9 CAIX GRP | 8 | 0.97 | 80 | 89 |
| Cyfra SLPI TIMP1 CEACAM5 MMP2 CA242 CAIX GRP | 8 | 0.97 | 80 | 89 |
| Cyfra TIMP1 TFPI CEACAM5 MMP2 Ca19-9 CA242 CAIX | 8 | 0.97 | 80 | 91 |
| Cyfra TIMP1 TFPI CEACAM5 MMP2 Ca19-9 CA242 GRP | 8 | 0.97 | 80 | 89 |
| Cyfra TIMP1 TFPI CEACAM5 MMP2 Ca19-9 CAIX GRP | 8 | 0.97 | 80 | 89 |
| Cyfra TIMP1 TFPI CEACAM5 MMP2 CA242 CAIX GRP | 8 | 0.97 | 80 | 89 |
| Cyfra TIMP1 CEACAM5 MMP2 Ca19-9 CA242 CAIX GRP | 8 | 0.97 | 80 | 89 |
| MIF SLPI TIMP1 TFPI CEACAM5 MMP2 CAIX GRP | 8 | 0.97 | 80 | 89 |
| MIF SLPI TIMP1 CEACAM5 MMP2 Ca19-9 CAIX GRP | 8 | 0.97 | 80 | 89 |
| MIF SLPI TIMP1 CEACAM5 MMP2 CA242 CAIX GRP | 8 | 0.97 | 80 | 89 |
| MIF TIMP1 TFPI CEACAM5 MMP2 Ca19-9 CAIX GRP | 8 | 0.97 | 80 | 89 |
| MIF TIMP1 TFPI CEACAM5 MMP2 CA242 CAIX GRP | 8 | 0.97 | 80 | 89 |
| MIF TIMP1 CEACAM5 MMP2 Ca19-9 CA242 CAIX GRP | 8 | 0.97 | 80 | 89 |
| SLPI TIMP1 ENO2 TFPI CEACAM5 MMP2 CAIX GRP | 8 | 0.97 | 80 | 89 |
| SLPI TIMP1 ENO2 CEACAM5 MMP2 Ca19-9 CAIX GRP | 8 | 0.97 | 80 | 89 |
| SLPI TIMP1 ENO2 CEACAM5 MMP2 CA242 CAIX GRP | 8 | 0.97 | 80 | 89 |
| SLPI TIMP1 TFPI CEACAM5 MMP2 AMBP CAIX GRP | 8 | 0.97 | 80 | 89 |
| SLPI TIMP1 TFPI CEACAM5 MMP2 Ca19-9 CA242 CAIX | 8 | 0.97 | 80 | 93 |
| SLPI TIMP1 TFPI CEACAM5 MMP2 Ca19-9 CA242 GRP | 8 | 0.97 | 80 | 91 |
| SLPI TIMP1 TFPI CEACAM5 MMP2 CAIX GRP DEFA1 | 8 | 0.97 | 80 | 89 |
| SLPI TIMP1 CEACAM5 MMP2 AMBP Ca19-9 CAIX GRP | 8 | 0.97 | 80 | 89 |
| SLPI TIMP1 CEACAM5 MMP2 AMBP CA242 CAIX GRP | 8 | 0.97 | 80 | 89 |
| SLPI TIMP1 CEACAM5 MMP2 Ca19-9 CAIX GRP DEFA1 | 8 | 0.97 | 80 | 89 |
| SLPI TIMP1 CEACAM5 MMP2 CA242 CAIX GRP DEFA1 | 8 | 0.97 | 80 | 89 |
| TIMP1 ENO2 TFPI CEACAM5 MMP2 Ca19-9 CAIX GRP | 8 | 0.97 | 80 | 89 |
| TIMP1 ENO2 TFPI CEACAM5 MMP2 CA242 CAIX GRP | 8 | 0.97 | 80 | 89 |
| TIMP1 ENO2 CEACAM5 MMP2 Ca19-9 CA242 CAIX GRP | 8 | 0.97 | 80 | 89 |
| TIMP1 TFPI CEACAM5 MMP2 AMBP Ca19-9 CAIX GRP | 8 | 0.97 | 80 | 89 |
| TIMP1 TFPI CEACAM5 MMP2 AMBP CA242 CAIX GRP | 8 | 0.97 | 80 | 89 |
| TIMP1 TFPI CEACAM5 MMP2 Ca19-9 CAIX GRP DEFA1 | 8 | 0.97 | 80 | 89 |
| TIMP1 TFPI CEACAM5 MMP2 CA242 CAIX GRP DEFA1 | 8 | 0.97 | 80 | 89 |
| TIMP1 CEACAM5 MMP2 AMBP Ca19-9 CA242 CAIX GRP | 8 | 0.97 | 80 | 89 |
| TIMP1 CEACAM5 MMP2 Ca19-9 CA242 CAIX GRP DEFA1 | 8 | 0.97 | 80 | 89 |
| Cyfra SLPI TIMP1 TFPI CEACAM5 MMP2 Ca19-9 CA242 CAIX | 9 | 0.97 | 80 | 91 |
| Cyfra SLPI TIMP1 TFPI CEACAM5 MMP2 Ca19-9 CA242 GRP | 9 | 0.97 | 80 | 89 |
| Cyfra SLPI TIMP1 TFPI CEACAM5 MMP2 Ca19-9 CAIX GRP | 9 | 0.97 | 80 | 89 |
| Cyfra SLPI TIMP1 TFPI CEACAM5 MMP2 CA242 CAIX GRP | 9 | 0.97 | 80 | 89 |
| Cyfra SLPI TIMP1 CEACAM5 MMP2 Ca19-9 CA242 CAIX GRP | 9 | 0.97 | 80 | 89 |
| Cyfra TIMP1 TFPI CEACAM5 MMP2 Ca19-9 CA242 CAIX GRP | 9 | 0.97 | 80 | 89 |
| MIF SLPI TIMP1 TFPI CEACAM5 MMP2 Ca19-9 CAIX GRP | 9 | 0.97 | 80 | 89 |
| MIF SLPI TIMP1 TFPI CEACAM5 MMP2 CA242 CAIX GRP | 9 | 0.97 | 80 | 89 |
| MIF SLPI TIMP1 CEACAM5 MMP2 Ca19-9 CA242 CAIX GRP | 9 | 0.97 | 80 | 89 |
| MIF TIMP1 TFPI CEACAM5 MMP2 Ca19-9 CA242 CAIX GRP | 9 | 0.97 | 80 | 89 |
| SLPI TIMP1 ENO2 TFPI CEACAM5 MMP2 Ca19-9 CAIX GRP | 9 | 0.97 | 80 | 89 |
| SLPI TIMP1 ENO2 TFPI CEACAM5 MMP2 CA242 CAIX GRP | 9 | 0.97 | 80 | 89 |
| SLPI TIMP1 ENO2 CEACAM5 MMP2 Ca19-9 CA242 CAIX GRP | 9 | 0.97 | 80 | 89 |
| SLPI TIMP1 TFPI CEACAM5 MMP2 AMBP Ca19-9 CAIX GRP | 9 | 0.97 | 80 | 89 |
| SLPI TIMP1 TFPI CEACAM5 MMP2 AMBP CA242 CAIX GRP | 9 | 0.97 | 80 | 89 |
| SLPI TIMP1 TFPI CEACAM5 MMP2 Ca19-9 CAIX GRP DEFA1 | 9 | 0.97 | 80 | 89 |
| SLPI TIMP1 TFPI CEACAMS MMP2 CA242 CAIX GRP DEFA1 | 9 | 0.97 | 80 | 89 |
| SLPI TIMP1 CEACAM5 MMP2 AMBP Ca19-9 CA242 CAIX GRP | 9 | 0.97 | 80 | 89 |
| SLPI TIMP1 CEACAM5 MMP2 Ca19-9 CA242 CAIX GRP DEFA1 | 9 | 0.97 | 80 | 89 |
| TIMP1 ENO2 TFPI CEACAM5 MMP2 Ca19-9 CA242 CAIX GRP | 9 | 0.97 | 80 | 89 |
| TIMP1 TFPI CEACAM5 MMP2 AMBP Ca19-9 CA242 CAIX GRP | 9 | 0.97 | 80 | 89 |
| TIMP1 TFPI CEACAM5 MMP2 Ca19-9 CA242 CAIX GRP DEFA1 | 9 | 0.97 | 80 | 89 |
| Cyfra SLPI TIMP1 TFPI CEACAM5 MMP2 Ca19-9 CA242 CAIX GRP | 10 | 0.97 | 80 | 89 |
| MIF SLPI TIMP1 TFPI CEACAM5 MMP2 Ca19-9 CA242 CAIX GRP | 10 | 0.97 | 80 | 89 |
| SLPI TIMP1 ENO2 TFPI CEACAM5 MMP2 Ca19-9 CA242 CAIX GRP | 10 | 0.97 | 80 | 89 |
| SLPI TIMP1 TFPI CEACAM5 MMP2 AMBP Ca19-9 CA242 CAIX GRP | 10 | 0.97 | 80 | 89 |
| SLPI TIMP1 TFPI CEACAM5 MMP2 Ca19-9 CA242 CAIX GRP DEFA1 | 10 | 0.97 | 80 | 89 |
| Cyfra MIF TIMP1 CEACAM5 MMP2 GRP | 6 | 0.97 | 80 | 86 |
| Cyfra TIMP1 ENO2 CEACAM5 MMP2 GRP | 6 | 0.97 | 80 | 86 |
| Cyfra TIMP1 CEACAM5 MMP2 AMBP GRP | 6 | 0.97 | 80 | 89 |
| Cyfra TIMP1 CEACAM5 MMP2 GRP DEFA1 | 6 | 0.97 | 80 | 86 |
| MIF TIMP1 TFPI CEACAM5 MMP2 GRP | 6 | 0.97 | 80 | 89 |
| MIF TIMP1 CEACAM5 MMP2 Ca19-9 GRP | 6 | 0.97 | 80 | 89 |
| MIF TIMP1 CEACAM5 MMP2 CA242 GRP | 6 | 0.97 | 80 | 89 |
| MIF TIMP1 CEACAM5 MMP2 CAIX GRP | 6 | 0.97 | 80 | 89 |
| TIMP1 ENO2 TFPI CEACAM5 MMP2 GRP | 6 | 0.97 | 80 | 89 |
| TIMP1 ENO2 CEACAM5 MMP2 Ca19-9 GRP | 6 | 0.97 | 80 | 89 |
| TIMP1 ENO2 CEACAM5 MMP2 CA242 GRP | 6 | 0.97 | 80 | 89 |
| TIMP1 ENO2 CEACAM5 MMP2 CAIX GRP | 6 | 0.97 | 80 | 89 |
| TIMP1 TFPI CEACAM5 MMP2 AMBP GRP | 6 | 0.97 | 80 | 89 |
| TIMP1 TFPI CEACAM5 MMP2 GRP DEFA1 | 6 | 0.97 | 80 | 89 |
| TIMP1 CEACAM5 MMP2 AMBP Ca19-9 GRP | 6 | 0.97 | 80 | 89 |
| TIMP1 CEACAM5 MMP2 AMBP CA242 GRP | 6 | 0.97 | 80 | 89 |
| TIMP1 CEACAM5 MMP2 AMBP CAIX GRP | 6 | 0.97 | 80 | 89 |
| TIMP1 CEACAM5 MMP2 Ca19-9 GRP DEFA1 | 6 | 0.97 | 80 | 89 |
| TIMP1 CEACAM5 MMP2 CA242 GRP DEFA1 | 6 | 0.97 | 80 | 89 |
| TIMP1 CEACAM5 MMP2 CAIX GRP DEFA1 | 6 | 0.97 | 80 | 89 |
| Cyfra MIF SLPI TIMP1 CEACAM5 MMP2 GRP | 7 | 0.97 | 80 | 86 |
| Cyfra MIF TIMP1 TFPI CEACAM5 MMP2 CAIX | 7 | 0.97 | 80 | 89 |
| Cyfra MIF TIMP1 TFPI CEACAM5 MMP2 GRP | 7 | 0.97 | 80 | 86 |
| Cyfra MIF TIMP1 CEACAM5 MMP2 Ca19-9 CAIX | 7 | 0.97 | 80 | 89 |
| Cyfra MIF TIMP1 CEACAM5 MMP2 Ca19-9 GRP | 7 | 0.97 | 80 | 86 |
| Cyfra MIF TIMP1 CEACAM5 MMP2 CA242 CAIX | 7 | 0.97 | 80 | 89 |
| Cyfra MIF TIMP1 CEACAM5 MMP2 CA242 GRP | 7 | 0.97 | 80 | 86 |
| Cyfra MIF TIMP1 CEACAM5 MMP2 CAIX GRP | 7 | 0.97 | 80 | 86 |
| Cyfra SLPI TIMP1 ENO2 CEACAM5 MMP2 GRP | 7 | 0.97 | 80 | 86 |
| Cyfra SLPI TIMP1 CEACAM5 MMP2 AMBP GRP | 7 | 0.97 | 80 | 89 |
| Cyfra SLPI TIMP1 CEACAM5 MMP2 GRP DEFA1 | 7 | 0.97 | 80 | 86 |
| Cyfra TIMP1 ENO2 TFPI CEACAM5 MMP2 CAIX | 7 | 0.97 | 80 | 89 |
| Cyfra TIMP1 ENO2 TFPI CEACAM5 MMP2 GRP | 7 | 0.97 | 80 | 86 |
| Cyfra TIMP1 ENO2 CEACAM5 MMP2 Ca19-9 CAIX | 7 | 0.97 | 80 | 89 |
| Cyfra TIMP1 ENO2 CEACAM5 MMP2 Ca19-9 GRP | 7 | 0.97 | 80 | 86 |
| Cyfra TIMP1 EN02 CEACAM5 MMP2 CA242 CAIX | 7 | 0.97 | 80 | 89 |
| Cyfra TIMP1 ENO2 CEACAM5 MMP2 CA242 GRP | 7 | 0.97 | 80 | 86 |
| Cyfra TIMP1 ENO2 CEACAM5 MMP2 CAIX GRP | 7 | 0.97 | 80 | 86 |
| Cyfra TIMP1 TFPI CEACAM5 MMP2 AMBP GRP | 7 | 0.97 | 80 | 89 |
| Cyfra TIMP1 TFPI CEACAM5 MMP2 CAIX DEFA1 | 7 | 0.97 | 80 | 89 |
| Cyfra TIMP1 TFPI CEACAM5 MMP2 GRP DEFA1 | 7 | 0.97 | 80 | 86 |
| Cyfra TIMP1 CEACAM5 MMP2 AMBP Ca19-9 GRP | 7 | 0.97 | 80 | 89 |
| Cyfra TIMP1 CEACAM5 MMP2 AMBP CA242 GRP | 7 | 0.97 | 80 | 89 |
| Cyfra TIMP1 CEACAM5 MMP2 AMBP CAIX GRP | 7 | 0.97 | 80 | 89 |
| Cyfra TIMP1 CEACAM5 MMP2 Ca19-9 CAIX DEFA1 | 7 | 0.97 | 80 | 89 |
| Cyfra TIMP1 CEACAM5 MMP2 Ca19-9 GRP DEFA1 | 7 | 0.97 | 80 | 86 |
| Cyfra TIMP1 CEACAM5 MMP2 CA242 CAIX DEFA1 | 7 | 0.97 | 80 | 89 |
| Cyfra TIMP1 CEACAM5 MMP2 CA242 GRP DEFA1 | 7 | 0.97 | 80 | 86 |
| Cyfra TIMP1 CEACAM5 MMP2 CAIX GRP DEFA1 . | 7 | 0.97 | 80 | 86 |
| MIF SLPI TIMP1 TFPI CEACAM5 MMP2 GRP | 7 | 0.97 | 80 | 89 |
| MIF SLPI TIMP1 CEACAM5 MMP2 Ca19-9 GRP | 7 | 0.97 | 80 | 89 |
| MIF SLPI TIMP1 CEACAM5 MMP2 CA242 GRP | 7 | 0.97 | 80 | 89 |
| MIF SLPI TIMP1 CEACAM5 MMP2 CAIX GRP | 7 | 0.97 | 80 | 89 |
| MIF TIMP1 TFPI CEACAM5 MMP2 Ca19-9 GRP | 7 | 0.97 | 80 | 89 |
| MIF TIMP1 TFPI CEACAM5 MMP2 CA242 GRP | 7 | 0.97 | 80 | 89 |
| MIF TIMP1 CEACAM5 MMP2 Ca19-9 CA242 GRP | 7 | 0.97 | 80 | 89 |
| SLPI TIMP1 ENO2 TFPI CEACAM5 MMP2 GRP | 7 | 0.97 | 80 | 89 |
| SLPI TIMP1 ENO2 CEACAM5 MMP2 Ca19-9 GRP | 7 | 0.97 | 80 | 89 |
| SLPI TIMP1 ENO2 CEACAM5 MMP2 CA242 GRP | 7 | 0.97 | 80 | 89 |
| SLPI TIMP1 ENO2 CEACAM5 MMP2 CAIX GRP | 7 | 0.97 | 80 | 89 |
| SLPI TIMP1 TFPI CEACAM5 MMP2 AMBP GRP | 7 | 0.97 | 80 | 89 |
| SLPI TIMP1 TFPI CEACAM5 MMP2 GRP DEFA1 | 7 | 0.97 | 80 | 89 |
| SLPI TIMP1 CEACAM5 MMP2 AMBP Ca19-9 GRP | 7 | 0.97 | 80 | 89 |
| SLPI TIMP1 CEACAM5 MMP2 AMBP CA242 GRP | 7 | 0.97 | 80 | 89 |
| SLPI TIMP1 CEACAM5 MMP2 AMBP CAIX GRP | 7 | 0.97 | 80 | 89 |
| SLPI TIMP1 CEACAM5 MMP2 Ca19-9 GRP DEFA1 | 7 | 0.97 | 80 | 89 |
| SLPI TIMP1 CEACAM5 MMP2 CA242 GRP DEFA1 | 7 | 0.97 | 80 | 89 |
| SLPI TIMP1 CEACAM5 MMP2 CAIX GRP DEFA1 | 7 | 0.97 | 80 | 89 |
| TIMP1 ENO2 TFPI CEACAM5 MMP2 Ca19-9 GRP | 7 | 0.97 | 80 | 89 |
| TIMP1 ENO2 TFPI CEACAM5 MMP2 CA242 GRP | 7 | 0.97 | 80 | 89 |
| TIMP1 ENO2 CEACAM5 MMP2 Ca19-9 CA242 GRP | 7 | 0.97 | 80 | 89 |
| TIMP1 TFPI CEACAM5 MMP2 AMBP Ca19-9 GRP | 7 | 0.97 | 80 | 89 |
| TIMP1 TFPI CEACAM5 MMP2 AMBP CA242 GRP | 7 | 0.97 | 80 | 89 |
| TIMP1 TFPI CEACAM5 MMP2 Ca19-9 GRP DEFA1 | 7 | 0.97 | 80 | 89 |
| TIMP1 TFPI CEACAM5 MMP2 CA242 GRP DEFA1 | 7 | 0.97 | 80 | 89 |
| TIMP1 CEACAM5 MMP2 AMBP Ca19-9 CA242 GRP | 7 | 0.97 | 80 | 89 |
| TIMP1 CEACAM5 MMP2 Ca19-9 CA242 GRP DEFA1 | 7 | 0.97 | 80 | 89 |
| Cyfra MIF SLPI TIMP1 TFPI CEACAM5 MMP2 CAIX | 8 | 0.97 | 80 | 89 |
| Cyfra MIF SLPI TIMP1 TFPI CEACAM5 MMP2 GRP | 8 | 0.97 | 80 | 86 |
| Cyfra MIF SLPI TIMP1 CEACAM5 MMP2 Ca19-9 CAIX | 8 | 0.97 | 80 | 89 |
| Cyfra MIF SLPI TIMP1 CEACAM5 MMP2 Ca19-9 GRP | 8 | 0.97 | 80 | 86 |
| Cyfra MIF SLPI TIMP1 CEACAM5 MMP2 CA242 CAIX | 8 | 0.97 | 80 | 89 |
| Cyfra MIF SLPI TIMPI CEACAM5 MMP2 CA242 GRP | 8 | 0.97 | 80 | 86 |
| Cyfra MIF SLPI TIMP1 CEACAM5 MMP2 CAIX GRP | 8 | 0.97 | 80 | 86 |
| Cyfra MIF TIMP1 TFPI CEACAM5 MMP2 Ca19-9 CAIX | 8 | 0.97 | 80 | 89 |
| Cyfra MIF TIMP1 TFPI CEACAM5 MMP2 Ca19-9 GRP | 8 | 0.97 | 80 | 86 |
| Cyfra MIF TIMP1 TFPI CEACAM5 MMP2 CA242 CAIX | 8 | 0.97 | 80 | 89 |
| Cyfra MIF TIMP1 TFPI CEACAM5 MMP2 CA242 GRP | 8 | 0.97 | 80 | 86 |
| Cyfra MIF TIMP1 TFPI CEACAM5 MMP2 CAIX GRP | 8 | 0.97 | 80 | 86 |
| Cyfra MIF TIMP1 CEACAM5 MMP2 Ca19-9 CA242 CAIX | 8 | 0.97 | 80 | 89 |
| Cyfra MIF TIMP1 CEACAM5 MMP2 Ca19-9 CA242 GRP | 8 | 0.97 | 80 | 86 |
| Cyfra MIF TIMP1 CEACAM5 MMP2 Ca19-9 CAIX GRP | 8 | 0.97 | 80 | 86 |
| Cyfra MIF TIMP1 CEACAM5 MMP2 CA242 CAIX GRP | 8 | 0.97 | 80 | 86 |
| Cyfra SLPI TIMP1 ENO2 TFPI CEACAM5 MMP2 CAIX | 8 | 0.97 | 80 | 89 |
| Cyfra SLPI TIMP1 ENO2 TFPI CEACAM5 MMP2 GRP | 8 | 0.97 | 80 | 86 |
| Cyfra SLPI TIMP1 ENO2 CEACAM5 MMP2 Ca19-9 CAIX | 8 | 0.97 | 80 | 89 |
| Cyfra SLPI TIMP1 ENO2 CEACAM5 MMP2 Ca19-9 GRP | 8 | 0.97 | 80 | 86 |
| Cyfra SLPI TIMP1 ENO2 CEACAM5 MMP2 CA242 CAIX | 8 | 0.97 | 80 | 89 |
| Cyfra SLPI TIMP1 ENO2 CEACAM5 MMP2 CA242 GRP | 8 | 0.97 | 80 | 86 |
| Cyfra SLPI TIMP1 ENO2 CEACAM5 MMP2 CAIX GRP | 8 | 0.97 | 80 | 86 |
| Cyfra SLPI TIMP1 TFPI CEACAM5 MMP2 AMBP GRP | 8 | 0.97 | 80 | 89 |
| Cyfra SLPI TIMP1 TFPI CEACAM5 MMP2 CAIX DEFA1 | 8 | 0.97 | 80 | 89 |
| Cyfra SLPI TIMP1 TFPI CEACAM5 MMP2 GRP DEFA1 | 8 | 0.97 | 80 | 86 |
| Cyfra SLPI TIMP1 CEACAM5 MMP2 AMBP Ca19-9 GRP | 8 | 0.97 | 80 | 89 |
| Cyfra SLPI TIMP1 CEACAM5 MMP2 AMBP CA242 GRP | 8 | 0.97 | 80 | 89 |
| Cyfra SLPI TIMP1 CEACAM5 MMP2 AMBP CAIX GRP | 8 | 0.97 | 80 | 89 |
| Cyfra SLPI TIMP1 CEACAM5 MMP2 Ca19-9 CAIX DEFA1 | 8 | 0.97 | 80 | 89 |
| Cyfra SLPI TIMP1 CEACAM5 MMP2 Ca19-9 GRP DEFA1 | 8 | 0.97 | 80 | 86 |
| Cyfra SLPI TIMP1 CEACAM5 MMP2 CA242 CAIX DEFA1 | 8 | 0.97 | 80 | 89 |
| Cyfra SLPI TIMP1 CEACAM5 MMP2 CA242 GRP DEFA1 | 8 | 0.97 | 80 | 86 |
| Cyfra SLPI TIMP1 CEACAM5 MMP2 CAIX GRP DEFA1 | 8 | 0.97 | 80 | 86 |
| Cyfra TIMP1 ENO2 TFPI CEACAM5 MMP2 Ca19-9 CAIX | 8 | 0.97 | 80 | 89 |
| Cyfra TIMP1 ENO2 TFPI CEACAM5 MMP2 Ca19-9 GRP | 8 | 0.97 | 80 | 86 |
| Cyfra TIMP1 ENO2 TFPI CEACAM5 MMP2 CA242 CAIX | 8 | 0.97 | 80 | 89 |
| Cyfra TIMP1 ENO2 TFPI CEACAM5 MMP2 CA242 GRP | 8 | 0.97 | 80 | 86 |
| Cyfra TIMP1 ENO2 TFPI CEACAM5 MMP2 CAIX GRP | 8 | 0.97 | 80 | 86 |
| Cyfra TIMP1 ENO2 CEACAM5 MMP2 Ca19-9 CA242 CAIX | 8 | 0.97 | 80 | 89 |
| Cyfra TIMP1 ENO2 CEACAM5 MMP2 Ca19-9 CA242 GRP | 8 | 0.97 | 80 | 86 |
| Cyfra TIMP1 ENO2 CEACAM5 MMP2 Ca19-9 CAIX GRP | 8 | 0.97 | 80 | 86 |
| Cyfra TIMP1 ENO2 CEACAM5 MMP2 CA242 CAIX GRP | 8 | 0.97 | 80 | 86 |
| Cyfra TIMP1 TFPI CEACAM5 MMP2 AMBP Ca19-9 GRP | 8 | 0.97 | 80 | 89 |
| Cyfra TIMP1 TFPI CEACAM5 MMP2 AMBP CA242 GRP | 8 | 0.97 | 80 | 89 |
| Cyfra TIMP1 TFPI CEACAM5 MMP2 AMBP CAIX GRP | 8 | 0.97 | 80 | 89 |
| Cyfra TIMP1 TFPI CEACAM5 MMP2 Ca19-9 CAIX DEFA1 | 8 | 0.97 | 80 | 89 |
| Cyfra TIMP1 TFPI CEACAM5 MMP2 Ca19-9 GRP DEFA1 | 8 | 0.97 | 80 | 86 |
| Cyfra TIMP1 TFPI CEACAM5 MMP2 CA242 CAIX DEFA1 | 8 | 0.97 | 80 | 89 |
| Cyfra TIMP1 TFPI CEACAM5 MMP2 CA242 GRP DEFA1 | 8 | 0.97 | 80 | 86 |
| Cyfra TIMP1 TFPI CEACAM5 MMP2 CAIX GRP DEFA1 | 8 | 0.97 | 80 | 86 |
| Cyfra TIMP1 CEACAM5 MMP2 AMBP Ca19-9 CA242 GRP | 8 | 0.97 | 80 | 89 |
| Cyfra TIMP1 CEACAM5 MMP2 AMBP Ca19-9 CAIX GRP | 8 | 0.97 | 80 | 89 |
| Cyfra TIMP1 CEACAM5 MMP2 AMBP CA242 CAIX GRP | 8 | 0.97 | 80 | 89 |
| Cyfra TIMP1 CEACAM5 MMP2 Ca19-9 CA242 CAIX DEFA1 | 8 | 0.97 | 80 | 89 |
| Cyfra TIMP1 CEACAM5 MMP2 Ca19-9 CA242 GRP DEFA1 | 8 | 0.97 | 80 | 86 |
| Cyfra TIMP1 CEACAM5 MMP2 Ca19-9 CAIX GRP DEFA1 | 8 | 0.97 | 80 | 86 |
| Cyfra TIMP1 CEACAM5 MMP2 CA242 CAIX GRP DEFA1 | 8 | 0.97 | 80 | 86 |
| MIF SLPI TIMP1 TFPI CEACAM5 MMP2 Ca19-9 GRP | 8 | 0.97 | 80 | 89 |
| MIF SLPI TIMP1 TFPI CEACAM5 MMP2 CA242 GRP | 8 | 0.97 | 80 | 89 |
| MIF SLPI TIMP1 CEACAM5 MMP2 Ca19-9 CA242 GRP | 8 | 0.97 | 80 | 89 |
| MIF TIMP1 ENO2 TFPI CEACAM5 MMP2 CAIX GRP | 8 | 0.97 | 80 | 86 |
| MIF TIMP1 ENO2 CEACAM5 MMP2 Ca19-9 CAIX GRP | 8 | 0.97 | 80 | 86 |
| MIF TIMP1 ENO2 CEACAM5 MMP2 CA242 CAIX GRP | 8 | 0.97 | 80 | 86 |
| MIF TIMP1 TFPI CEACAM5 MMP2 AMBP CAIX GRP | 8 | 0.97 | 80 | 86 |
| MIF TIMP1 TFPI CEACAM5 MMP2 Ca19-9 CA242 GRP | 8 | 0.97 | 80 | 89 |
| MIF TIMP1 TFPI CEACAM5 MMP2 CAIX GRP DEFA1 | 8 | 0.97 | 80 | 86 |
| MIF TIMP1 CEACAM5 MMP2 AMBP Ca19-9 CAIX GRP | 8 | 0.97 | 80 | 86 |
| MIF TIMP1 CEACAM5 MMP2 AMBP CA242 CAIX GRP | 8 | 0.97 | 80 | 86 |
| MIF TIMP1 CEACAM5 MMP2 Ca19-9 CAIX GRP DEFA1 | 8 | 0.97 | 80 | 86 |
| MIF TIMP1 CEACAM5 MMP2 CA242 CAIX GRP DEFAL1 | 8 | 0.97 | 80 | 86 |
| SLPI TIMP1 ENO2 TFPI CEACAM5 MMP2 Ca19-9 GRP | 8 | 0.97 | 80 | 89 |
| SLPI TIMP1 ENO2 TFPI CEACAM5 MMP2 CA242 GRP | 8 | 0.97 | 80 | 89 |
| SLPI TIMP1 ENO2 CEACAM5 MMP2 Ca19-9 CA242 GRP | 8 | 0.97 | 80 | 89 |
| SLPI TIMP1 TFPI CEACAM5 MMP2 AMBP Ca19-9 GRP | 8 | 0.97 | 80 | 89 |
| SLPI TIMP1 TFPI CEACAM5 MMP2 AMBP CA242 GRP | 8 | 0.97 | 80 | 89 |
| SLPI TIMP1 TFPI CEACAM5 MMP2 Ca19-9 GRP DEFAL1 | 8 | 0.97 | 80 | 89 |
| SLPI TIMP1 TFPI CEACAM5 MMP2 CA242 GRP DEFA1 | 8 | 0.97 | 80 | 89 |
| SLPI TIMP1 CEACAM5 MMP2 AMBP Ca19-9 CA242 GRP | 8 | 0.97 | 80 | 89 |
| SLPI TIMP1 CEACAM5 MMP2 Ca19-9 CA242 GRP DEFAL1 | 8 | 0.97 | 80 | 89 |
| TIMP1 ENO2 TFPI CEACAM5 MMP2 AMBP CAIX GRP | 8 | 0.97 | 80 | 86 |
| TIMP1 ENO2 TFPI CEACAM5 MMP2 Ca19-9 CA242 GRP | 8 | 0.97 | 80 | 89 |
| TIMP1 ENO2 TFPI CEACAM5 MMP2 CAIX GRP DEFA1 | 8 | 0.97 | 80 | 86 |
| TIMP1 ENO2 CEACAM5 MMP2 AMBP Ca19-9 CAIX GRP | 8 | 0.97 | 80 | 86 |
| TIMP1 ENO2 CEACAM5 MMP2 AMBP CA242 CAIX GRP | 8 | 0.97 | 80 | 86 |
| TIMP1 ENO2 CEACAM5 MMP2 Ca19-9 CAIX GRP DEFA1 | 8 | 0.97 | 80 | 86 |
| TIMP1 ENO2 CEACAM5 MMP2 CA242 CAIX GRP DEFA1 | 8 | 0.97 | 80 | 86 |
| TIMP1 TFPI CEACAM5 MMP2 AMBP Ca19-9 CA242 GRP | 8 | 0.97 | 80 | 89 |
| TIMP1 TFPI CEACAM5 MMP2 AMBP CAIX GRP DEFA1 | 8 | 0.97 | 80 | 86 |
| TIMP1 TFPI CEACAM5 MMP2 Ca19-9 CA242 GRP DEFA1 | 8 | 0.97 | 80 | 89 |
| TIMP1 CEACAM5 MMP2 AMBP Ca19-9 CAIX GRP DEFA1 | 8 | 0.97 | 80 | 86 |
| TIMP1 CEACAM5 MMP2 AMBP CA242 CAIX GRP DEFA1 | 8 | 0.97 | 80 | 86 |
| Cyfra MIF SLPI TIMP1 TFPI CEACAM5 MMP2 Ca19-9 CAIX | 9 | 0.97 | 80 | 89 |
| Cyfra MIF SLPI TIMP1 TFPI CEACAM5 MMP2 Ca19-9 GRP | 9 | 0.97 | 80 | 86 |
| Cyfra MIF SLPI TIMP1 TFPI CEACAM5 MMP2 CA242 CAIX | 9 | 0.97 | 80 | 89 |
| Cyfra MIF SLPI TIMP1 TFPI CEACAM5 MMP2 CA242 GRP | 9 | 0.97 | 80 | 86 |
| Cyfra MIF SLPI TIMP1 TFPI CEACAM5 MMP2 CAIX GRP | 9 | 0.97 | 80 | 86 |
| Cyfra MIF SLPI TIMP1 CEACAM5 MMP2 Ca19-9 CA242 CAIX | 9 | 0.97 | 80 | 89 |
| Cyfra MIF SLPI TIMP1 CEACAM5 MMP2 Ca19-9 CA242 GRP | 9 | 0.97 | 80 | 86 |
| Cyfra MIF SLPI TIMP1 CEACAM5 MMP2 Ca19-9 CAIX GRP | 9 | 0.97 | 80 | 86 |
| Cyfra MIF SLPI TIMP1 CEACAM5 MMP2 CA242 CAIX GRP | 9 | 0.97 | 80 | 86 |
| Cyfra MIF TIMP1 TFPI CEACAM5 MMP2 Ca19-9 CA242 CAIX | 9 | 0.97 | 80 | 89 |
| Cyfra MIF TIMP1 TFPI CEACAM5 MMP2 Ca19-9 CA242 GRP | 9 | 0.97 | 80 | 86 |
| Cyfra MIF TIMP1 TFPI CEACAM5 MMP2 Ca19-9 CAIX GRP | 9 | 0.97 | 80 | 86 |
| Cyfra MIF TIMP1 TFPI CEACAM5 MMP2 CA242 CAIX GRP | 9 | 0.97 | 80 | 86 |
| Cyfra MIF TIMP1 CEACAM5 MMP2 Ca19-9 CA242 CAIX GRP | 9 | 0.97 | 80 | 86 |
| Cyfra SLPI TIMP1 ENO2 TFPI CEACAM5 MMP2 Ca19-9 CAIX | 9 | 0.97 | 80 | 89 |
| Cyfra SLPI TIMP1 ENO2 TFPI CEACAM5 MMP2 Ca19-9 GRP | 9 | 0.97 | 80 | 86 |
| Cyfra SLPI TIMP1 ENO2 TFPI CEACAM5 MMP2 CA242 CAIX | 9 | 0.97 | 80 | 89 |
| Cyfra SLPI TIMP1 ENO2 TFPI CEACAM5 MMP2 CA242 GRP | 9 | 0.97 | 80 | 86 |
| Cyfra SLPI TIMP1 ENO2 TFPI CEACAM5 MMP2 CAIX GRP | 9 | 0.97 | 80 | 86 |
| Cyfra SLPI TIMP1 ENO2 CEACAM5 MMP2 Ca19-9 CA242 CAIX | 9 | 0.97 | 80 | 89 |
| Cyfra SLPI TIMP1 ENO2 CEACAM5 MMP2 Ca19-9 CA242 GRP | 9 | 0.97 | 80 | 86 |
| Cyfra SLPI TIMP1 ENO2 CEACAM5 MMP2 Ca19-9 CAIX GRP | 9 | 0.97 | 80 | 86 |
| Cyfra SLPI TIMP1 ENO2 CEACAM5 MMP2 CA242 CAIX GRP | 9 | 0.97 | 80 | 86 |
| Cyfra SLPI TIMP1 TFPI CEACAM5 MMP2 AMBP Ca19-9 GRP | 9 | 0.97 | 80 | 89 |
| Cyfra SLPI TIMP1 TFPI CEACAM5 MMP2 AMBP CA242 GRP | 9 | 0.97 | 80 | 89 |
| Cyfra SLPI TIMP1 TFPI CEACAM5 MMP2 AMBP CAIX GRP | 9 | 0.97 | 80 | 89 |
| Cyfra SLPI TIMP1 TFPI CEACAM5 MMP2 Ca19-9 CAIX DEFA1 | 9 | 0.97 | 80 | 89 |
| Cyfra SLPI TIMP1 TFPI CEACAM5 MMP2 Ca19-9 GRP DEFA1 | 9 | 0.97 | 80 | 86 |
| Cyfra SLPI TIMP1 TFPI CEACAM5 MMP2 CA242 CAIX DEFA1 | 9 | 0.97 | 80 | 89 |
| Cyfra SLPI TIMP1 TFPI CEACAM5 MMP2 CA242 GRP DEFA1 | 9 | 0.97 | 80 | 86 |
| Cyfra SLPI TIMP1 TFPI CEACAM5 MMP2 CAIX GRP DEFA1 | 9 | 0.97 | 80 | 86 |
| Cyfra SLPI TIMP1 CEACAM5 MMP2 AMBP Ca19-9 CA242 GRP | 9 | 0.97 | 80 | 89 |
| Cyfra SLPI TIMP1 CEACAM5 MMP2 AMBP Ca19-9 CAIX GRP | 9 | 0.97 | 80 | 89 |
| Cyfra SLPI TIMP1 CEACAM5 MMP2 AMBP CA242 CAIX GRP | 9 | 0.97 | 80 | 89 |
| Cyfra SLPI TIMP1 CEACAM5 MMP2 Ca19-9 CA242 CAIX DEFA1 | 9 | 0.97 | 80 | 89 |
| Cyfra SLPI TIMP1 CEACAM5 MMP2 Ca19-9 CA242 GRP DEFA1 | 9 | 0.97 | 80 | 86 |
| Cyfra SLPI TIMP1 CEACAM5 MMP2 Ca19-9 CAIX GRP DEFA1 | 9 | 0.97 | 80 | 86 |
| Cyfra SLPI TIMP1 CEACAM5 MMP2 CA242 CAIX GRP DEFA1 | 9 | 0.97 | 80 | 86 |
| Cyfra TIMP1 ENO2 TFPI CEACAM5 MMP2 Ca19-9 CA242 CAIX | 9 | 0.97 | 80 | 89 |
| Cyfra TIMP1 ENO2 TFPI CEACAM5 MMP2 Ca19-9 CA242 GRP | 9 | 0.97 | 80 | 86 |
| Cyfra TIMP1 ENO2 TFPI CEACAM5 MMP2 Ca19-9 CAIX GRP | 9 | 0.97 | 80 | 86 |
| Cyfra TIMP1 ENO2 TFPI CEACAM5 MMP2 CA242 CAIX GRP | 9 | 0.97 | 80 | 86 |
| Cyfra TIMP1 ENO2 CEACAM5 MMP2 Ca19-9 CA242 CAIX GRP | 9 | 0.97 | 80 | 86 |
| Cyfra TIMP1 TFPI CEACAM5 MMP2 AMBP Ca19-9 CA242 GRP | 9 | 0.97 | 80 | 89 |
| Cyfra TIMP1 TFPI CEACAM5 MMP2 AMBP Ca19-9 CAIX GRP | 9 | 0.97 | 80 | 89 |
| Cyfra TIMP1 TFPI CEACAM5 MMP2 AMBP CA242 CAIX GRP | 9 | 0.97 | 80 | 89 |
| Cyfra TIMP1 TFPI CEACAM5 MMP2 Ca19-9 CA242 CAIX DEFA1 | 9 | 0.97 | 80 | 89 |
| Cyfra TIMP1 TFPI CEACAM5 MMP2 Ca19-9 CA242 GRP DEFA1 | 9 | 0.97 | 80 | 86 |
| Cyfra TIMP1 TFPI CEACAM5 MMP2 Ca19-9 CAIX GRP DEFA1 | 9 | 0.97 | 80 | 86 |
| Cyfra TIMP1 TFPI CEACAM5 MMP2 CA242 CAIX GRP DEFA1 | 9 | 0.97 | 80 | 86 |
| Cyfra TIMP1 CEACAM5 MMP2 AMBP Ca19-9 CA242 CAIX GRP | 9 | 0.97 | 80 | 89 |
| Cyfra TIMP1 CEACAM5 MMP2 Ca19-9 CA242 CAIX GRP DEFA1 | 9 | 0.97 | 80 | 86 |
| MIF SLPI TIMP1 ENO2 TFPI CEACAM5 MMP2 CAIX GRP | 9 | 0.97 | 80 | 86 |
| MIF SLPI TIMP1 ENO2 CEACAM5 MMP2 Ca19-9 CAIX GRP | 9 | 0.97 | 80 | 86 |
| MIF SLPI TIMP1 ENO2 CEACAM5 MMP2 CA242 CAIX GRP | 9 | 0.97 | 80 | 86 |
| MIF SLPI TIMP1 TFPI CEACAM5 MMP2 AMBP CAIX GRP | 9 | 0.97 | 80 | 86 |
| MIF SLPI TIMP1 TFPI CEACAM5 MMP2 Ca19-9 CA242 GRP | 9 | 0.97 | 80 | 89 |
| MIF SLPI TIMP1 TFPI CEACAM5 MMP2 CAIX GRP DEFA1 | 9 | 0.97 | 80 | 86 |
| MIF SLPI TIMP1 CEACAM5 MMP2 AMBP Ca19-9 CAIX GRP | 9 | 0.97 | 80 | 86 |
| MIF SLPI TIMP1 CEACAM5 MMP2 AMBP CA242 CAIX GRP | 9 | 0.97 | 80 | 86 |
| MIF SLPI TIMP1 CEACAM5 MMP2 Ca19-9 CAIX GRP DEFA1 | 9 | 0.97 | 80 | 86 |
| MIF SLPI TIMP1 CEACAM5 MMP2 CA242 CAIX GRP DEFA1 | 9 | 0.97 | 80 | 86 |
| MIF TIMP1 ENO2 TFPI CEACAM5 MMP2 Ca19-9 CAIX GRP | 9 | 0.97 | 80 | 86 |
| MIF TIMP1 ENO2 TFPI CEACAM5 MMP2 CA242 CAIX GRP | 9 | 0.97 | 80 | 86 |
| MIF TIMP1 ENO2 CEACAM5 MMP2 Ca19-9 CA242 CAIX GRP | 9 | 0.97 | 80 | 86 |
| MIF TIMP1 TFPI CEACAM5 MMP2 AMBP Ca19-9 CAIX GRP | 9 | 0.97 | 80 | 86 |
| MIF TIMP1 TFPI CEACAM5 MMP2 AMBP CA242 CAIX GRP | 9 | 0.97 | 80 | 86 |
| MIF TIMP1 TFPI CEACAM5 MMP2 Ca19-9 CAIX GRP DEFA1 | 9 | 0.97 | 80 | 86 |
| MIF TIMP1 TFPI CEACAM5 MMP2 CA242 CAIX GRP DEFA1 | 9 | 0.97 | 80 | 86 |
| MIF TIMP1 CEACAM5 MMP2 AMBP Ca19-9 CA242 CAIX GRP | 9 | 0.97 | 80 | 86 |
| MIF TIMP1 CEACAM5 MMP2 Ca19-9 CA242 CAIX GRP DEFA1 | 9 | 0.97 | 80 | 86 |
| SLPI TIMP1 ENO2 TFPI CEACAM5 MMP2 AMBP CAIX GRP | 9 | 0.97 | 80 | 86 |
| SLPI TIMP1 ENO2 TFPI CEACAM5 MMP2 Ca19-9 CA242 GRP | 9 | 0.97 | 80 | 89 |
| SLPI TIMP1 ENO2 TFPI CEACAM5 MMP2 CAIX GRP DEFA1 | 9 | 0.97 | 80 | 86 |
| SLPI TIMP1 ENO2 CEACAM5 MMP2 AMBP Ca19-9 CAIX GRP | 9 | 0.97 | 80 | 86 |
| SLPI TIMP1 ENO2 CEACAM5 MMP2 AMBP CA242 CAIX GRP | 9 | 0.97 | 80 | 86 |
| SLPI TIMP1 ENO2 CEACAM5 MMP2 Ca19-9 CAIX GRP DEFA1 | 9 | 0.97 | 80 | 86 |
| SLPI TIMP1 ENO2 CEACAM5 MMP2 CA242 CAIX GRP DEFA1 | 9 | 0.97 | 80 | 86 |
| SLPI TIMP1 TFPI CEACAM5 MMP2 AMBP Ca19-9 CA242 GRP | 9 | 0.97 | 80 | 89 |
| SLPI TIMP1 TFPI CEACAM5 MMP2 AMBP CAIX GRP DEFA1 | 9 | 0.97 | 80 | 86 |
| SLPI TIMP1 TFPI CEACAM5 MMP2 Ca19-9 CA242 GRP DEFA1 | 9 | 0.97 | 80 | 89 |
| SLPI TIMP1 CEACAM5 MMP2 AMBP Ca19-9 CAIX GRP DEFA1 | 9 | 0.97 | 80 | 86 |
| SLPI TIMP1 CEACAM5 MMP2 AMBP CA242 CAIX GRP DEFA1 | 9 | 0.97 | 80 | 86 |
| TIMP1 ENO2 TFPI CEACAM5 MMP2 AMBP Ca19-9 CAIX GRP | 9 | 0.97 | 80 | 86 |
| TIMP1 ENO2 TFPI CEACAM5 MMP2 AMBP CA242 CAIX GRP | 9 | 0.97 | 80 | 86 |
| TIMP1 ENO2 TFPI CEACAM5 MMP2 Ca19-9 CAIX GRP DEFA1 | 9 | 0.97 | 80 | 86 |
| TIMP1 ENO2 TFPI CEACAM5 MMP2 CA242 CAIX GRP DEFA1 | 9 | 0.97 | 80 | 86 |
| TIMP1 ENO2 CEACAM5 MMP2 AMBP Ca19-9 CA242 CAIX GRP | 9 | 0.97 | 80 | 86 |
| TIMP1 ENO2 CEACAM5 MMP2 Ca19-9 CA242 CAIX GRP DEFA1 | 9 | 0.97 | 80 | 86 |
| TIMP1 TFPI CEACAM5 MMP2 AMBP Ca19-9 CAIX GRP DEFA1 | 9 | 0.97 | 80 | 86 |
| TIMP1 TFPI CEACAM5 MMP2 AMBP CA242 CAIX GRP DEFA1 | 9 | 0.97 | 80 | 86 |
| TIMP1 CEACAM5 MMP2 AMBP Ca19-9 CA242 CAIX GRP DEFA1 | 9 | 0.97 | 80 | 86 |
| Cyfra MIF SLPI TIMP1 TFPI CEACAM5 MMP2 Ca19-9 CA242 CAIX | 10 | 0.97 | 80 | 89 |
| Cyfra MIF SLPI TIMP1 TFPI CEACAM5 MMP2 Ca19-9 CA242 GRP | 10 | 0.97 | 80 | 86 |
| Cyfra MIF SLPI TIMP1 TFPI CEACAM5 MMP2 Ca19-9 CAIX GRP | 10 | 0.97 | 80 | 86 |
| Cyfra MIF SLPI TIMP1 TFPI CEACAM5 MMP2 CA242 CAIX GRP | 10 | 0.97 | 80 | 86 |
| Cyfra MIF SLPI TIMP1 CEACAM5 MMP2 Ca19-9 CA242 CAIX GRP | 10 | 0.97 | 80 | 86 |
| Cyfra MIF TIMP1 TFPI CEACAM5 MMP2 Ca19-9 CA242 CAIX GRP | 10 | 0.97 | 80 | 86 |
| Cyfra SLPI TIMP1 ENO2 TFPI CEACAM5 MMP2 Ca19-9 CA242 CAIX | 10 | 0.97 | 80 | 89 |
| Cyfra SLPI TIMP1 ENO2 TFPI CEACAM5 MMP2 Ca19-9 CA242 GRP | 10 | 0.97 | 80 | 86 |
| Cyfra SLPI TIMP1 ENO2 TFPI CEACAM5 MMP2 Ca19-9 CAIX GRP | 10 | 0.97 | 80 | 86 |
| Cyfra SLPI TIMP1 ENO2 TFPI CEACAM5 MMP2 CA242 CAIX GRP | 10 | 0.97 | 80 | 86 |
| Cyfra SLPI TIMP1 ENO2 CEACAM5 MMP2 Ca19-9 CA242 CAIX GRP | 10 | 0.97 | 80 | 86 |
| Cyfra SLPI TIMP1 TFPI CEACAM5 MMP2 AMBP Ca19-9 CA242 GRP | 10 | 0.97 | 80 | 89 |
| Cyfra SLPI TIMP1 TFPI CEACAM5 MMP2 AMBP Ca19-9 CAIX GRP | 10 | 0.97 | 80 | 89 |
| Cyfra SLPI TIMP1 TFPI CEACAM5 MMP2 AMBP CA242 CAIX GRP | 10 | 0.97 | 80 | 89 |
| Cyfra SLPI TIMP1 TFPI CEACAM5 MMP2 Ca19-9 CA242 CAIX DEFA1 | 10 | 0.97 | 80 | 89 |
| Cyfra SLPI TIMP1 TFPI CEACAM5 MMP2 Ca19-9 CA242 GRP DEFA1 | 10 | 0.97 | 80 | 86 |
| Cyfra SLPI TIMP1 TFPI CEACAM5 MMP2 Ca19-9 CAIX GRP DEFA1 | 10 | 0.97 | 80 | 86 |
| Cyfra SLPI TIMP1 TFPI CEACAM5 MMP2 CA242 CAIX GRP DEFA1 | 10 | 0.97 | 80 | 86 |
| Cyfra SLPI TIMP1 CEACAM5 MMP2 AMBP Ca19-9 CA242 CAIX GRP | 10 | 0.97 | 80 | 89 |
| Cyfra SLPI TIMP1 CEACAM5 MMP2 Ca19-9 CA242 CAIX GRP DEFA1 | 10 | 0.97 | 80 | 86 |
| Cyfra TIMP1 ENO2 TFPI CEACAM5 MMP2 Ca19-9 CA242 CAIX GRP | 10 | 0.97 | 80 | 86 |
| Cyfra TIMP1 TFPI CEACAM5 MMP2 AMBP Ca19-9 CA242 CAIX GRP | 10 | 0.97 | 80 | 89 |
| Cyfra TIMP1 TFPI CEACAM5 MMP2 Ca19-9 CA242 CAIX GRP DEFA1 | 10 | 0.97 | 80 | 86 |
| MIF SLPI TIMP1 ENO2 TFPI CEACAM5 MMP2 Ca19-9 CAIX GRP | 10 | 0.97 | 80 | 86 |
| MIF SLPI TIMP1 ENO2 TFPI CEACAM5 MMP2 CA242 CAIX GRP | 10 | 0.97 | 80 | 86 |
| MIF SLPI TIMP1 ENO2 CEACAM5 MMP2 Ca19-9 CA242 CAIX GRP | 10 | 0.97 | 80 | 86 |
| MIF SLPI TIMP1 TFPI CEACAM5 MMP2 AMBP Ca19-9 CAIX GRP | 10 | 0.97 | 80 | 86 |
| MIF SLPI TIMP1 TFPI CEACAM5 MMP2 AMBP CA242 CAIX GRP | 10 | 0.97 | 80 | 86 |
| MIF SLPI TIMP1 TFPI CEACAM5 MMP2 Ca19-9 CAIX GRP DEFA1 | 10 | 0.97 | 80 | 86 |
| MIF SLPI TIMP1 TFPI CEACAM5 MMP2 CA242 CAIX GRP DEFA1 | 10 | 0.97 | 80 | 86 |
| MIF SLPI TIMP1 CEACAM5 MMP2 AMBP Ca19-9 CA242 CAIX GRP | 10 | 0.97 | 80 | 86 |
| MIF SLPI TIMP1 CEACAM5 MMP2 Ca19-9 CA242 CAIX GRP DEFA1 | 10 | 0.97 | 80 | 86 |
| MIF TIMP1 ENO2 TFPI CEACAM5 MMP2 Ca19-9 CA242 CAIX GRP | 10 | 0.97 | 80 | 86 |
| MIF TIMP1 TFPI CEACAM5 MMP2 AMBP Ca19-9 CA242 CAIX GRP | 10 | 0.97 | 80 | 86 |
| MIF TIMP1 TFPI CEACAM5 MMP2 Ca19-9 CA242 CAIX GRP DEFA1 | 10 | 0.97 | 80 | 86 |
| SLPI TIMP1 ENO2 TFPI CEACAM5 MMP2 AMBP Ca19-9 CAIX GRP | 10 | 0.97 | 80 | 86 |
| SLPI TIMP1 ENO2 TFPI CEACAM5 MMP2 AMBP CA242 CAIX GRP | 10 | 0.97 | 80 | 86 |
| SLPI TIMP1 ENO2 TFPI CEACAM5 MMP2 Ca19-9 CAIX GRP DEFA1 | 10 | 0.97 | 80 | 86 |
| SLPI TIMP1 ENO2 TFPI CEACAM5 MMP2 CA242 CAIX GRP DEFA1 | 10 | 0.97 | 80 | 86 |
| SLPI TIMP1 ENO2 CEACAM5 MMP2 AMBP Ca19-9 CA242 CAIX GRP | 10 | 0.97 | 80 | 86 |
| SLPI TIMP1 ENO2 CEACAM5 MMP2 Ca19-9 CA242 CAIX GRP DEFA1 | 10 | 0.97 | 80 | 86 |
| SLPI TIMP1 TFPI CEACAM5 MMP2 AMBP Ca19-9 CAIX GRP DEFA1 | 10 | 0.97 | 80 | 86 |
| SLPI TIMP1 TFPI CEACAM5 MMP2 AMBP CA242 CAIX GRP DEFA1 | 10 | 0.97 | 80 | 86 |
| SLPI TIMP1 CEACAM5 MMP2 AMBP Ca19-9 CA242 CAIX GRP DEFA1 | 10 | 0.97 | 80 | 86 |
| TIMP1 ENO2 TFPI CEACAM5 MMP2 AMBP Ca19-9 CA242 CAIX GRP | 10 | 0.97 | 80 | 86 |
| TIMP1 ENO2 TFPI CEACAM5 MMP2 Ca19-9 CA242 CAIX GRP DEFA1 | 10 | 0.97 | 80 | 86 |
| TIMP1 TFPI CEACAM5 MMP2 AMBP Ca19-9 CA242 CAIX GRP DEFA1 | 10 | 0.97 | 80 | 86 |
| Cyfra MIF SLPI TIMP1 TFPI CEACAM5 MMP2 Ca19-9 CA242 CAIX GRP | 11 | 0.97 | 80 | 86 |
| Cyfra SLPI TIMP1 ENO2 TFPI CEACAM5 MMP2 Ca19-9 CA242 CAIX GRP | 11 | 0.97 | 80 | 86 |
| Cyfra SLPI TIMP1 TFPI CEACAM5 MMP2 AMBP Ca19-9 CA242 CAIX GRP | 11 | 0.97 | 80 | 89 |
| Cyfra SLPI TIMP1 TFPI CEACAM5 MMP2 Ca19-9 CA242 CAIX GRP DEFA1 | 11 | 0.97 | 80 | 86 |
| MIF SLPI TIMP1 ENO2 TFPI CEACAM5 MMP2 Ca19-9 CA242 CAIX GRP | 11 | 0.97 | 80 | 86 |
| MIF SLPI TIMP1 TFPI CEACAM5 MMP2 AMBP Ca19-9 CA242 CAIX GRP | 11 | 0.97 | 80 | 86 |
| MIF SLPI TIMP1 TFPI CEACAM5 MMP2 Ca19-9 CA242 CAIX GRP DEFA1 | 11 | 0.97 | 80 | 86 |
| SLPI TIMP1 ENO2 TFPI CEACAM5 MMP2 AMBP Ca19-9 CA242 CAIX GRP | 11 | 0.97 | 80 | 86 |
| SLPI TIMP1 ENO2 TFPI CEACAM5 MMP2 Ca19-9 CA242 CAIX GRP DEFA1 | 11 | 0.97 | 80 | 86 |
| SLPI TIMP1 TFPI CEACAM5 MMP2 AMBP Ca19-9 CA242 CAIX GRP DEFA1 | 11 | 0.97 | 80 | 86 |
| TIMP1 CEACAM5 MMP2 GRP | 4 | 0.97 | 80 | 91 |
| Cyfra TIMP1 TFPI CEACAM5 MMP2 | 5 | 0.97 | 80 | 91 |
| Cyfra TIMP1 CEACAM5 MMP2 Ca19-9 | 5 | 0.97 | 80 | 91 |
| Cyfra TIMP1 CEACAM5 MMP2 CA242 | 5 | 0.97 | 80 | 91 |
| Cyfra TIMP1 CEACAM5 MMP2 CAIX | 5 | 0.97 | 80 | 91 |
| SLPI TIMP1 CEACAM5 MMP2 GRP | 5 | 0.97 | 80 | 91 |
| TIMP1 TFPI CEACAM5 MMP2 CAIX | 5 | 0.97 | 80 | 93 |
| TIMP1 CEACAM5 MMP2 Ca19-9 CAIX | 5 | 0.97 | 80 | 93 |
| TIMP1 CEACAM5 MMP2 CA242 CAIX | 5 | 0.97 | 80 | 93 |
| Cyfra SLPI TIMP1 TFPI CEACAM5 MMP2 | 6 | 0.97 | 80 | 91 |
| Cyfra SLPI TIMP1 CEACAM5 MMP2 Ca19-9 | 6 | 0.97 | 80 | 91 |
| Cyfra SLPI TIMP1 CEACAM5 MMP2 CA242 | 6 | 0.97 | 80 | 91 |
| Cyfra SLPI TIMP1 CEACAM5 MMP2 CAIX | 6 | 0.97 | 80 | 91 |
| Cyfra TIMP1 TFPI CEACAM5 MMP2 Ca19-9 | 6 | 0.97 | 80 | 91 |
| Cyfra TIMP1 TFPI CEACAM5 MMP2 CA242 | 6 | 0.97 | 80 | 91 |
| Cyfra TIMP1 CEACAM5 MMP2 Ca19-9 CA242 | 6 | 0.97 | 80 | 91 |
| SLPI TIMP1 TFPI CEACAM5 MMP2 CAIX | 6 | 0.97 | 80 | 93 |
| SLPI TIMP1 CEACAM5 MMP2 Ca19-9 CAIX | 6 | 0.97 | 80 | 93 |
| SLPI TIMP1 CEACAM5 MMP2 CA242 CAIX | 6 | 0.97 | 80 | 93 |
| Cyfra SLPI TIMP1 TFPI CEACAM5 MMP2 Ca19-9 | 7 | 0.97 | 80 | 91 |
| Cyfra SLPI TIMP1 TFPI CEACAM5 MMP2 CA242 | 7 | 0.97 | 80 | 91 |
| Cyfra SLPI TIMP1 CEACAM5 MMP2 Ca19-9 CA242 | 7 | 0.97 | 80 | 91 |
| Cyfra TIMP1 TFPI CEACAM5 MMP2 Ca19-9 CA242 | 7 | 0.97 | 80 | 91 |
| MIF TIMP1 TFPI CEACAM5 MMP2 Ca19-9 CAIX | 7 | 0.97 | 80 | 91 |
| MIF TIMP1 TFPI CEACAM5 MMP2 CA242 CAIX | 7 | 0.97 | 80 | 91 |
| MIF TIMP1 CEACAM5 MMP2 Ca19-9 CA242 CAIX | 7 | 0.97 | 80 | 91 |
| TIMP1 ENO2 TFPI CEACAM5 MMP2 Ca19-9 CAIX | 7 | 0.97 | 80 | 91 |
| TIMP1 ENO2 TFPI CEACAM5 MMP2 CA242 CAIX | 7 | 0.97 | 80 | 91 |
| TIMP1 ENO2 CEACAM5 MMP2 Ca19-9 CA242 CAIX | 7 | 0.97 | 80 | 91 |
| TIMP1 TFPI CEACAM5 MMP2 AMBP Ca19-9 CAIX | 7 | 0.97 | 80 | 91 |
| TIMP1 TFPI CEACAM5 MMP2 AMBP CA242 CAIX | 7 | 0.97 | 80 | 91 |
| TIMP1 TFPI CEACAM5 MMP2 Ca19-9 CAIX DEFA1 | 7 | 0.97 | 80 | 91 |
| TIMP1 TFPI CEACAM5 MMP2 CA242 CAIX DEFA1 | 7 | 0.97 | 80 | 91 |
| TIMP1 CEACAM5 MMP2 AMBP Ca19-9 CA242 CAIX | 7 | 0.97 | 80 | 91 |
| TIMP1 CEACAM5 MMP2 Ca19-9 CA242 CAIX DEFA1 | 7 | 0.97 | 80 | 91 |
| Cyfra SLPI TIMP1 TFPI CEACAM5 MMP2 Ca19-9 CA242 | 8 | 0.97 | 80 | 91 |
| MIF SLPI TIMP1 TFPI CEACAM5 MMP2 Ca19-9 CAIX | 8 | 0.97 | 80 | 91 |
| MIF SLPI TIMP1 TFPI CEACAM5 MMP2 CA242 CAIX | 8 | 0.97 | 80 | 91 |
| MIF SLPI TIMP1 CEACAM5 MMP2 Ca19-9 CA242 CAIX | 8 | 0.97 | 80 | 91 |
| MIF TIMP1 TFPI CEACAM5 MMP2 Ca19-9 CA242 CAIX | 8 | 0.97 | 80 | 91 |
| SLPI TIMP1 ENO2 TFPI CEACAM5 MMP2 Ca19-9 CAIX | 8 | 0.97 | 80 | 91 |
| SLPI TIMP1 ENO2 TFPI CEACAM5 MMP2 CA242 CAIX | 8 | 0.97 | 80 | 91 |
| SLPI TIMP1 ENO2 CEACAM5 MMP2 Ca19-9 CA242 CAIX | 8 | 0.97 | 80 | 91 |
| SLPI TIMP1 TFPI CEACAM5 MMP2 AMBP Ca19-9 CAIX | 8 | 0.97 | 80 | 91 |
| SLPI TIMP1 TFPI CEACAM5 MMP2 AMBP CA242 CAIX | 8 | 0.97 | 80 | 91 |
| SLPI TIMP1 TFPI CEACAM5 MMP2 Ca19-9 CAIX DEFA1 | 8 | 0.97 | 80 | 91 |
| SLPI TIMP1 TFPI CEACAM5 MMP2 CA242 CAIX DEFA1 | 8 | 0.97 | 80 | 91 |
| SLPI TIMP1 CEACAM5 MMP2 AMBP Ca19-9 CA242 CAIX | 8 | 0.97 | 80 | 91 |
| SLPI TIMP1 CEACAM5 MMP2 Ca19-9 CA242 CAIX DEFA1 | 8 | 0.97 | 80 | 91 |
| TIMP1 ENO2 TFPI CEACAM5 MMP2 Ca19-9 CA242 CAIX | 8 | 0.97 | 80 | 91 |
| TIMP1 TFPI CEACAM5 MMP2 AMBP Ca19-9 CA242 CAIX | 8 | 0.97 | 80 | 91 |
| TIMP1 TFPI CEACAM5 MMP2 Ca19-9 CA242 CAIX DEFA1 | 8 | 0.97 | 80 | 91 |
| MIF SLPI TIMP1 TFPI CEACAM5 MMP2 Ca19-9 CA242 CAIX | 9 | 0.97 | 80 | 91 |
| SLPI TIMP1 ENO2 TFPI CEACAM5 MMP2 Ca19-9 CA242 CAIX | 9 | 0.97 | 80 | 91 |
| SLPI TIMP1 TFPI CEACAM5 MMP2 AMBP Ca19-9 CA242 CAIX | 9 | 0.97 | 80 | 91 |
| SLPI TIMP1 TFPI CEACAM5 MMP2 Ca19-9 CA242 CAIX DEFA1 | 9 | 0.97 | 80 | 91 |
| Cyfra TIMP1 CEACAM5 | 3 | 0.96 | 60 | 91 |
| TIMP1 CEACAM5 GRP | 3 | 0.96 | 60 | 91 |
| Cyfra SLPI TIMP1 CEACAM5 | 4 | 0.96 | 60 | 91 |
| Cyfra TIMP1 TFPI CEACAM5 | 4 | 0.96 | 60 | 91 |
| Cyfra TIMP1 CEACAM5 MMP2 | 4 | 0.96 | 80 | 91 |
| Cyfra TIMP1 CEACAM5 Ca19-9 | 4 | 0.96 | 60 | 91 |
| Cyfra TIMP1 CEACAM5 CA242 | 4 | 0.96 | 60 | 91 |
| Cyfra TIMP1 CEACAM5 CAIX | 4 | 0.96 | 60 | 91 |
| SLPI TIMP1 CEACAM5 GRP | 4 | 0.96 | 60 | 91 |
| TIMP1 TFPI CEACAM5 GRP | 4 | 0.96 | 60 | 91 |
| TIMP1 CEACAM5 Ca19-9 GRP | 4 | 0.96 | 60 | 91 |
| TIMP1 CEACAM5 CA242 GRP | 4 | 0.96 | 60 | 91 |
| TIMP1 CEACAM5 CAIX GRP | 4 | 0.96 | 60 | 91 |
| Cyfra SLPI TIMP1 TFPI CEACAM5 | 5 | 0.96 | 60 | 91 |
| Cyfra SLPI TIMP1 CEACAM5 MMP2 | 5 | 0.96 | 80 | 91 |
| Cyfra SLPI TIMP1 CEACAM5 Ca19-9 | 5 | 0.96 | 60 | 91 |
| Cyfra SLPI TIMP1 CEACAM5 CA242 | 5 | 0.96 | 60 | 91 |
| Cyfra SLPI TIMP1 CEACAM5 CAIX | 5 | 0.96 | 60 | 91 |
| Cyfra TIMP1 TFPI CEACAM5 Ca19-9 | 5 | 0.96 | 60 | 91 |
| Cyfra TIMP1 TFPI CEACAM5 CA242 | 5 | 0.96 | 60 | 91 |
| Cyfra TIMP1 TFPI CEACAM5 CAIX | 5 | 0.96 | 60 | 91 |
| Cyfra TIMP1 CEACAM5 Ca19-9 CA242 | 5 | 0.96 | 60 | 91 |
| Cyfra TIMP1 CEACAM5 Ca19-9 CAIX | 5 | 0.96 | 60 | 91 |
| Cyfra TIMP1 CEACAM5 CA242 CAIX | 5 | 0.96 | 60 | 91 |
| MIF TIMP1 TFPI CEACAM5 CAIX | 5 | 0.96 | 60 | 91 |
| MIF TIMP1 CEACAM5 Ca19-9 CAIX | 5 | 0.96 | 60 | 91 |
| MIF TIMP1 CEACAM5 CA242 CAIX | 5 | 0.96 | 60 | 91 |
| SLPI TIMP1 TFPI CEACAM5 GRP | 5 | 0.96 | 60 | 91 |
| SLPI TIMP1 CEACAM5 Ca19-9 GRP | 5 | 0.96 | 60 | 91 |
| SLPI TIMP1 CEACAM5 CA242 GRP | 5 | 0.96 | 60 | 91 |
| SLPI TIMP1 CEACAM5 CAIX GRP | 5 | 0.96 | 60 | 91 |
| TIMP1 ENO2 TFPI CEACAM5 CAIX | 5 | 0.96 | 60 | 91 |
| TIMP1 ENO2 CEACAM5 Ca19-9 CAIX | 5 | 0.96 | 60 | 91 |
| TIMP1 ENO2 CEACAM5 CA242 CAIX | 5 | 0.96 | 60 | 91 |
| TIMP1 TFPI CEACAM5 AMBP CAIX | 5 | 0.96 | 60 | 91 |
| TIMP1 TFPI CEACAM5 Ca19-9 GRP | 5 | 0.96 | 60 | 91 |
| TIMP1 TFPI CEACAM5 CA242 GRP | 5 | 0.96 | 60 | 91 |
| TIMP1 TFPI CEACAM5 CAIX GRP | 5 | 0.96 | 60 | 91 |
| TIMP1 TFPI CEACAM5 CAIX DEFA1 | 5 | 0.96 | 60 | 91 |
| TIMP1 CEACAM5 AMBP Ca19-9 CAIX | 5 | 0.96 | 60 | 91 |
| TIMP1 CEACAM5 AMBP CA242 CAIX | 5 | 0.96 | 60 | 91 |
| TIMP1 CEACAM5 Ca19-9 CA242 GRP | 5 | 0.96 | 60 | 91 |
| TIMP1 CEACAM5 Ca19-9 CAIX GRP | 5 | 0.96 | 60 | 91 |
| TIMP1 CEACAM5 Ca19-9 CAIX DEFA1 | 5 | 0.96 | 60 | 91 |
| TIMP1 CEACAM5 CA242 CAIX GRP | 5 | 0.96 | 60 | 91 |
| TIMP1 CEACAM5 CA242 CAIX DEFA1 | 5 | 0.96 | 60 | 91 |
| Cyfra SLPI TIMP1 TFPI CEACAM5 Ca19-9 | 6 | 0.96 | 60 | 91 |
| Cyfra SLPI TIMP1 TFPI CEACAM5 CA242 | 6 | 0.96 | 60 | 91 |
| Cyfra SLPI TIMP1 TFPI CEACAM5 CAIX | 6 | 0.96 | 60 | 91 |
| Cyfra SLPI TIMP1 CEACAM5 Ca19-9 CA242 | 6 | 0.96 | 60 | 91 |
| Cyfra SLPI TIMP1 CEACAM5 Ca19-9 CAIX | 6 | 0.96 | 60 | 91 |
| Cyfra SLPI TIMP1 CEACAM5 CA242 CAIX | 6 | 0.96 | 60 | 91 |
| Cyfra TIMP1 TFPI CEACAM5 Ca19-9 CA242 | 6 | 0.96 | 60 | 91 |
| Cyfra TIMP1 TFPI CEACAM5 Ca19-9 CAIX | 6 | 0.96 | 60 | 91 |
| Cyfra TIMP1 TFPI CEACAM5 CA242 CAIX | 6 | 0.96 | 60 | 91 |
| Cyfra TIMP1 CEACAM5 Ca19-9 CA242 CAIX | 6 | 0.96 | 60 | 91 |
| MIF SLPI TIMP1 TFPI CEACAM5 CAIX | 6 | 0.96 | 60 | 91 |
| MIF SLPI TIMP1 CEACAM5 Ca19-9 CAIX | 6 | 0.96 | 60 | 91 |
| MIF SLPI TIMP1 CEACAM5 CA242 CAIX | 6 | 0.96 | 60 | 91 |
| MIF TIMP1 TFPI CEACAM5 MMP2 CAIX | 6 | 0.96 | 80 | 91 |
| MIF TIMP1 TFPI CEACAM5 Ca19-9 CAIX | 6 | 0.96 | 60 | 91 |
| MIF TIMP1 TFPI CEACAM5 CA242 CAIX | 6 | 0.96 | 60 | 91 |
| MIF TIMP1 CEACAM5 MMP2 Ca19-9 CAIX | 6 | 0.96 | 80 | 91 |
| MIF TIMP1 CEACAM5 MMP2 CA242 CAIX | 6 | 0.96 | 80 | 91 |
| MIF TIMP1 CEACAM5 Ca19-9 CA242 CAIX | 6 | 0.96 | 60 | 91 |
| SLPI TIMP1 ENO2 TFPI CEACAM5 CAIX | 6 | 0.96 | 60 | 91 |
| SLPI TIMP1 ENO2 CEACAM5 Ca19-9 CAIX | 6 | 0.96 | 60 | 91 |
| SLPI TIMP1 ENO2 CEACAM5 CA242 CAIX | 6 | 0.96 | 60 | 91 |
| SLPI TIMP1 TFPI CEACAM5 AMBP CAIX | 6 | 0.96 | 60 | 91 |
| SLPI TIMP1 TFPI CEACAM5 Ca19-9 GRP | 6 | 0.96 | 60 | 91 |
| SLPI TIMP1 TFPI CEACAM5 CA242 GRP | 6 | 0.96 | 60 | 91 |
| SLPI TIMP1 TFPI CEACAM5 CAIX GRP | 6 | 0.96 | 60 | 91 |
| SLPI TIMP1 TFPI CEACAM5 CAIX DEFA1 | 6 | 0.96 | 60 | 91 |
| SLPI TIMP1 CEACAM5 AMBP Ca19-9 CAIX | 6 | 0.96 | 60 | 91 |
| SLPI TIMP1 CEACAM5 AMBP CA242 CAIX | 6 | 0.96 | 60 | 91 |
| SLPI TIMP1 CEACAM5 Ca19-9 CA242 GRP | 6 | 0.96 | 60 | 91 |
| SLPI TIMP1 CEACAM5 Ca19-9 CAIX GRP | 6 | 0.96 | 60 | 91 |
| SLPI TIMP1 CEACAM5 Ca19-9 CAIX DEFA1 | 6 | 0.96 | 60 | 91 |
| SLPI TIMP1 CEACAM5 CA242 CAIX GRP | 6 | 0.96 | 60 | 91 |
| SLPI TIMP1 CEACAM5 CA242 CAIX DEFA1 | 6 | 0.96 | 60 | 91 |
| TIMP1 ENO2 TFPI CEACAM5 MMP2 CAIX | 6 | 0.96 | 80 | 91 |
| TIMP1 ENO2 TFPI CEACAM5 Ca19-9 CAIX | 6 | 0.96 | 60 | 91 |
| TIMP1 ENO2 TFPI CEACAM5 CA242 CAIX | 6 | 0.96 | 60 | 91 |
| TIMP1 ENO2 CEACAM5 MMP2 Ca19-9 CAIX | 6 | 0.96 | 80 | 91 |
| TIMP1 ENO2 CEACAM5 MMP2 CA242 CAIX | 6 | 0.96 | 80 | 91 |
| TIMP1 ENO2 CEACAM5 Ca19-9 CA242 CAIX | 6 | 0.96 | 60 | 91 |
| TIMP1 TFPI CEACAM5 MMP2 AMBP CAIX | 6 | 0.96 | 80 | 91 |
| TIMP1 TFPI CEACAM5 MMP2 CAIX DEFA1 | 6 | 0.96 | 80 | 91 |
| TIMP1 TFPI CEACAM5 AMBP Ca19-9 CAIX | 6 | 0.96 | 60 | 91 |
| TIMP1 TFPI CEACAM5 AMBP CA242 CAIX | 6 | 0.96 | 60 | 91 |
| TIMP1 TFPI CEACAM5 Ca19-9 CA242 GRP | 6 | 0.96 | 60 | 91 |
| TIMP1 TFPI CEACAM5 Ca19-9 CAIX GRP | 6 | 0.96 | 60 | 91 |
| TIMP1 TFPI CEACAM5 Ca19-9 CAIX DEFA1 | 6 | 0.96 | 60 | 91 |
| TIMP1 TFPI CEACAM5 CA242 CAIX GRP | 6 | 0.96 | 60 | 91 |
| TIMP1 TFPI CEACAM5 CA242 CAIX DEFA1 | 6 | 0.96 | 60 | 91 |
| TIMP1 CEACAM5 MMP2 AMBP Ca19-9 CAIX | 6 | 0.96 | 80 | 91 |
| TIMP1 CEACAM5 MMP2 AMBP CA242 CAIX | 6 | 0.96 | 80 | 91 |
| TIMP1 CEACAM5 MMP2 Ca19-9 CAIX DEFA1 | 6 | 0.96 | 80 | 91 |
| TIMP1 CEACAM5 MMP2 CA242 CAIX DEFA1 | 6 | 0.96 | 80 | 91 |
| TIMP1 CEACAM5 AMBP Ca19-9 CA242 CAIX | 6 | 0.96 | 60 | 91 |
| TIMP1 CEACAM5 Ca19-9 CA242 CAIX GRP | 6 | 0.96 | 60 | 91 |
| TIMP1 CEACAM5 Ca19-9 CA242 CAIX DEFA1 | 6 | 0.96 | 60 | 91 |
| Cyfra MIF TIMP1 ENO2 CEACAM5 MMP2 GRP | 7 | 0.96 | 80 | 84 |
| Cyfra MIF TIMP1 CEACAM5 MMP2 AMBP GRP | 7 | 0.96 | 80 | 86 |
| Cyfra MIF TIMP1 CEACAM5 MMP2 GRP DEFA1 | 7 | 0.96 | 80 | 84 |
| Cyfra SLPI TIMP1 TFPI CEACAM5 Ca19-9 CA242 | 7 | 0.96 | 60 | 91 |
| Cyfra SLPI TIMP1 TFPI CEACAM5 Ca19-9 CAIX | 7 | 0.96 | 60 | 91 |
| Cyfra SLPI TIMP1 TFPI CEACAM5 CA242 CAIX | 7 | 0.96 | 60 | 91 |
| Cyfra SLPI TIMP1 CEACAM5 Ca19-9 CA242 CAIX | 7 | 0.96 | 60 | 91 |
| Cyfra TIMP1 ENO2 CEACAM5 MMP2 AMBP GRP | 7 | 0.96 | 80 | 86 |
| Cyfra TIMP1 ENO2 CEACAM5 MMP2 GRP DEFA1 | 7 | 0.96 | 80 | 84 |
| Cyfra TIMP1 TFPI CEACAM5 MMP2 AMBP CAIX | 7 | 0.96 | 80 | 91 |
| Cyfra TIMP1 TFPI CEACAM5 Ca19-9 CA242 CAIX | 7 | 0.96 | 60 | 91 |
| Cyfra TIMP1 CEACAM5 MMP2 AMBP Ca19-9 CAIX | 7 | 0.96 | 80 | 91 |
| Cyfra TIMP1 CEACAM5 MMP2 AMBP CA242 CAIX | 7 | 0.96 | 80 | 91 |
| Cyfra TIMP1 CEACAM5 MMP2 AMBP GRP DEFA1 | 7 | 0.96 | 80 | 86 |
| MIF SLPI TIMP1 TFPI CEACAM5 MMP2 CAIX | 7 | 0.96 | 80 | 91 |
| MIF SLPI TIMP1 TFPI CEACAM5 Ca19-9 CAIX | 7 | 0.96 | 60 | 91 |
| MIF SLPI TIMP1 TFPI CEACAM5 CA242 CAIX | 7 | 0.96 | 60 | 91 |
| MIF SLPI TIMP1 CEACAM5 MMP2 Ca19-9 CAIX | 7 | 0.96 | 80 | 91 |
| MIF SLPI TIMP1 CEACAM5 MMP2 CA242 CAIX | 7 | 0.96 | 80 | 91 |
| MIF SLPI TIMP1 CEACAM5 Ca19-9 CA242 CAIX | 7 | 0.96 | 60 | 91 |
| MIF TIMP1 ENO2 TFPI CEACAM5 MMP2 GRP | 7 | 0.96 | 80 | 86 |
| MIF TIMP1 ENO2 CEACAM5 MMP2 Ca19-9 GRP | 7 | 0.96 | 80 | 86 |
| MIF TIMP1 ENO2 CEACAM5 MMP2 CA242 GRP | 7 | 0.96 | 80 | 86 |
| MIF TIMP1 ENO2 CEACAM5 MMP2 CAIX GRP | 7 | 0.96 | 80 | 86 |
| MIF TIMP1 TFPI CEACAM5 MMP2 AMBP GRP | 7 | 0.96 | 80 | 86 |
| MIF TIMP1 TFPI CEACAM5 MMP2 GRP DEFA1 | 7 | 0.96 | 80 | 86 |
| MIF TIMP1 TFPI CEACAM5 Ca19-9 CA242 CAIX | 7 | 0.96 | 60 | 91 |
| MIF TIMP1 CEACAM5 MMP2 AMBP Ca19-9 GRP | 7 | 0.96 | 80 | 86 |
| MIF TIMP1 CEACAM5 MMP2 AMBP CA242 GRP | 7 | 0.96 | 80 | 86 |
| MIF TIMP1 CEACAM5 MMP2 AMBP CAIX GRP | 7 | 0.96 | 80 | 86 |
| MIF TIMP1 CEACAM5 MMP2 Ca19-9 GRP DEFA1 | 7 | 0.96 | 80 | 86 |
| MIF TIMP1 CEACAM5 MMP2 CA242 GRP DEFA1 | 7 | 0.96 | 80 | 86 |
| MIF TIMP1 CEACAM5 MMP2 CAIX GRP DEFA1 | 7 | 0.96 | 80 | 86 |
| SLPI TIMP1 ENO2 TFPI CEACAM5 MMP2 CAIX | 7 | 0.96 | 80 | 91 |
| SLPI TIMP1 ENO2 TFPI CEACAM5 Ca19-9 CAIX | 7 | 0.96 | 60 | 91 |
| SLPI TIMP1 ENO2 TFPI CEACAM5 CA242 CAIX | 7 | 0.96 | 60 | 91 |
| SLPI TIMP1 ENO2 CEACAM5 MMP2 Ca19-9 CAIX | 7 | 0.96 | 80 | 91 |
| SLPI TIMP1 ENO2 CEACAM5 MMP2 CA242 CAIX | 7 | 0.96 | 80 | 91 |
| SLPI TIMP1 ENO2 CEACAM5 Ca19-9 CA242 CAIX | 7 | 0.96 | 60 | 91 |
| SLPI TIMP1 TFPI CEACAM5 MMP2 AMBP CAIX | 7 | 0.96 | 80 | 91 |
| SLPI TIMP1 TFPI CEACAM5 MMP2 CAIX DEFA1 | 7 | 0.96 | 80 | 91 |
| SLPI TIMP1 TFPI CEACAM5 AMBP Ca19-9 CAIX | 7 | 0.96 | 60 | 91 |
| SLPI TIMP1 TFPI CEACAM5 AMBP CA242 CAIX | 7 | 0.96 | 60 | 91 |
| SLPI TIMP1 TFPI CEACAM5 Ca19-9 CA242 GRP | 7 | 0.96 | 60 | 91 |
| SLPI TIMP1 TFPI CEACAM5 Ca19-9 CAIX GRP | 7 | 0.96 | 60 | 91 |
| SLPI TIMP1 TFPI CEACAM5 Ca19-9 CAIX DEFA1 | 7 | 0.96 | 60 | 91 |
| SLPI TIMP1 TFPI CEACAM5 CA242 CAIX GRP | 7 | 0.96 | 60 | 91 |
| SLPI TIMP1 TFPI CEACAM5 CA242 CAIX DEFA1 | 7 | 0.96 | 60 | 91 |
| SLPI TIMP1 CEACAM5 MMP2 AMBP Ca19-9 CAIX | 7 | 0.96 | 80 | 91 |
| SLPI TIMP1 CEACAM5 MMP2 AMBP CA242 CAIX | 7 | 0.96 | 80 | 91 |
| SLPI TIMP1 CEACAM5 MMP2 Ca19-9 CAIX DEFA1 | 7 | 0.96 | 80 | 91 |
| SLPI TIMP1 CEACAM5 MMP2 CA242 CAIX DEFA1 | 7 | 0.96 | 80 | 91 |
| SLPI TIMP1 CEACAM5 AMBP Ca19-9 CA242 CAIX | 7 | 0.96 | 60 | 91 |
| SLPI TIMP1 CEACAM5 Ca19-9 CA242 CAIX GRP | 7 | 0.96 | 60 | 91 |
| SLPI TIMP1 CEACAM5 Ca19-9 CA242 CAIX DEFA1 | 7 | 0.96 | 60 | 91 |
| TIMP1 ENO2 TFPI CEACAM5 MMP2 AMBP GRP | 7 | 0.96 | 80 | 86 |
| TIMP1 ENO2 TFPI CEACAM5 MMP2 GRP DEFA1 | 7 | 0.96 | 80 | 86 |
| TIMP1 ENO2 TFPI CEACAM5 Ca19-9 CA242 CAIX | 7 | 0.96 | 60 | 91 |
| TIMP1 ENO2 CEACAM5 MMP2 AMBP Ca19-9 GRP | 7 | 0.96 | 80 | 86 |
| TIMP1 ENO2 CEACAM5 MMP2 AMBP CA242 GRP | 7 | 0.96 | 80 | 86 |
| TIMP1 ENO2 CEACAM5 MMP2 AMBP CAIX GRP | 7 | 0.96 | 80 | 86 |
| TIMP1 ENO2 CEACAM5 MMP2 Ca19-9 GRP DEFA1 | 7 | 0.96 | 80 | 86 |
| TIMP1 ENO2 CEACAM5 MMP2 CA242 GRP DEFA1 | 7 | 0.96 | 80 | 86 |
| TIMP1 ENO2 CEACAM5 MMP2 CAIX GRP DEFA1 | 7 | 0.96 | 80 | 86 |
| TIMP1 TFPI CEACAM5 MMP2 AMBP GRP DEFA1 | 7 | 0.96 | 80 | 86 |
| TIMP1 TFPI CEACAM5 AMBP Ca19-9 CA242 CAIX | 7 | 0.96 | 60 | 91 |
| TIMP1 TFPI CEACAM5 Ca19-9 CA242 CAIX GRP | 7 | 0.96 | 60 | 91 |
| TIMP1 TFPI CEACAM5 Ca19-9 CA242 CAIX DEFA1 | 7 | 0.96 | 60 | 91 |
| TIMP1 CEACAM5 MMP2 AMBP Ca19-9 GRP DEFA1 | 7 | 0.96 | 80 | 86 |
| TIMP1 CEACAM5 MMP2 AMBP CA242 GRP DEFA1 | 7 | 0.96 | 80 | 86 |
| TIMP1 CEACAM5 MMP2 AMBP CAIX GRP DEFA1 | 7 | 0.96 | 80 | 86 |
| Cyfra MIF SLPI TIMP1 ENO2 CEACAM5 MMP2 GRP | 8 | 0.96 | 80 | 84 |
| Cyfra MIF SLPI TIMP1 CEACAM5 MMP2 AMBP GRP | 8 | 0.96 | 80 | 86 |
| Cyfra MIF SLPI TIMP1 CEACAM5 MMP2 GRP DEFA1 | 8 | 0.96 | 80 | 84 |
| Cyfra MIF TIMP1 ENO2 TFPI CEACAM5 MMP2 CAIX | 8 | 0.96 | 80 | 86 |
| Cyfra MIF TIMP1 ENO2 TFPI CEACAM5 MMP2 GRP | 8 | 0.96 | 80 | 84 |
| Cyfra MIF TIMP1 ENO2 CEACAM5 MMP2 Ca19-9 CAIX | 8 | 0.96 | 80 | 86 |
| Cyfra MIF TIMP1 ENO2 CEACAM5 MMP2 Ca19-9 GRP | 8 | 0.96 | 80 | 84 |
| Cyfra MIF TIMP1 ENO2 CEACAM5 MMP2 CA242 CAIX | 8 | 0.96 | 80 | 86 |
| Cyfra MIF TIMP1 ENO2 CEACAM5 MMP2 CA242 GRP | 8 | 0.96 | 80 | 84 |
| Cyfra MIF TIMP1 ENO2 CEACAM5 MMP2 CAIX GRP | 8 | 0.96 | 80 | 84 |
| Cyfra MIF TIMP1 TFPI CEACAM5 MMP2 AMBP GRP | 8 | 0.96 | 80 | 86 |
| Cyfra MIF TIMP1 TFPI CEACAM5 MMP2 CAIX DEFA1 | 8 | 0.96 | 80 | 86 |
| Cyfra MIF TIMP1 TFPI CEACAM5 MMP2 GRP DEFA1 | 8 | 0.96 | 80 | 84 |
| Cyfra MIF TIMP1 CEACAM5 MMP2 AMBP Ca19-9 GRP | 8 | 0.96 | 80 | 86 |
| Cyfra MIF TIMP1 CEACAM5 MMP2 AMBP CA242 GRP | 8 | 0.96 | 80 | 86 |
| Cyfra MIF TIMP1 CEACAM5 MMP2 AMBP CAIX GRP | 8 | 0.96 | 80 | 86 |
| Cyfra MIF TIMP1 CEACAM5 MMP2 Ca19-9 CAIX DEFA1 | 8 | 0.96 | 80 | 86 |
| Cyfra MIF TIMP1 CEACAM5 MMP2 Ca19-9 GRP DEFA1 | 8 | 0.96 | 80 | 84 |
| Cyfra MIF TIMP1 CEACAM5 MMP2 CA242 CAIX DEFA1 | 8 | 0.96 | 80 | 86 |
| Cyfra MIF TIMP1 CEACAM5 MMP2 CA242 GRP DEFA1 | 8 | 0.96 | 80 | 84 |
| Cyfra MIF TIMP1 CEACAM5 MMP2 CAIX GRP DEFA1 | 8 | 0.96 | 80 | 84 |
| Cyfra SLPI TIMP1 ENO2 CEACAM5 MMP2 AMBP GRP | 8 | 0.96 | 80 | 86 |
| Cyfra SLPI TIMP1 ENO2 CEACAM5 MMP2 GRP DEFA1 | 8 | 0.96 | 80 | 84 |
| Cyfra SLPI TIMP1 TFPI CEACAM5 MMP2 AMBP CAIX | 8 | 0.96 | 80 | 91 |
| Cyfra SLPI TIMP1 TFPI CEACAM5 Ca19-9 CA242 CAIX | 8 | 0.96 | 60 | 91 |
| Cyfra SLPI TIMP1 CEACAM5 MMP2 AMBP Ca19-9 CAIX | 8 | 0.96 | 80 | 91 |
| Cyfra SLPI TIMP1 CEACAM5 MMP2 AMBP CA242 CAIX | 8 | 0.96 | 80 | 91 |
| Cyfra SLPI TIMP1 CEACAM5 MMP2 AMBP GRP DEFA1 | 8 | 0.96 | 80 | 86 |
| Cyfra TIMP1 ENO2 TFPI CEACAM5 MMP2 AMBP GRP | 8 | 0.96 | 80 | 86 |
| Cyfra TIMP1 ENO2 TFPI CEACAM5 MMP2 CAIX DEFA1 | 8 | 0.96 | 80 | 86 |
| Cyfra TIMP1 ENO2 TFPI CEACAM5 MMP2 GRP DEFA1 | 8 | 0.96 | . 80 | 84 |
| Cyfra TIMP1 ENO2 CEACAM5 MMP2 AMBP Ca19-9 GRP | 8 | 0.96 | 80 | 86 |
| Cyfra TIMP1 ENO2 CEACAM5 MMP2 AMBP CA242 GRP | 8 | 0.96 | 80 | 86 |
| Cyfra TIMP1 ENO2 CEACAM5 MMP2 AMBP CAIX GRP | 8 | 0.96 | 80 | 86 |
| Cyfra TIMP1 ENO2 CEACAM5 MMP2 Ca19-9 CAIX DEFA1 | 8 | 0.96 | 80 | 86 |
| Cyfra TIMP1 ENO2 CEACAM5 MMP2 Ca19-9 GRP DEFA1 | 8 | 0.96 | 80 | 84 |
| Cyfra TIMP1 ENO2 CEACAM5 MMP2 CA242 CAIX DEFA1 | 8 | 0.96 | 80 | 86 |
| Cyfra TIMP1 ENO2 CEACAM5 MMP2 CA242 GRP DEFA1 | 8 | 0.96 | 80 | 84 |
| Cyfra TIMP1 ENO2 CEACAM5 MMP2 CAIX GRP DEFA1 | 8 | 0.96 | 80 | 84 |
| Cyfra TIMP1 TFPI CEACAM5 MMP2 AMBP Ca19-9 CAIX | 8 | 0.96 | 80 | 91 |
| Cyfra TIMP1 TFPI CEACAM5 MMP2 AMBP CA242 CAIX | 8 | 0.96 | 80 | 91 |
| Cyfra TIMP1 TFPI CEACAM5 MMP2 AMBP GRP DEFA1 | 8 | 0.96 | 80 | 86 |
| Cyfra TIMP1 CEACAM5 MMP2 AMBP Ca19-9 CA242 CAIX | 8 | 0.96 | 80 | 91 |
| Cyfra TIMP1 CEACAM5 MMP2 AMBP Ca19-9 GRP DEFA1 | 8 | 0.96 | 80 | 86 |
| Cyfra TIMP1 CEACAM5 MMP2 AMBP CA242 GRP DEFA1 | 8 | 0.96 | 80 | 86 |
| Cyfra TIMP1 CEACAM5 MMP2 AMBP CAIX GRP DEFA1 | 8 | 0.96 | 80 | 86 |
| MIF SLPI TIMP1 ENO2 TFPI CEACAM5 MMP2 GRP | 8 | 0.96 | 80 | 86 |
| MIF SLPI TIMP1 ENO2 CEACAM5 MMP2 Ca19-9 GRP | 8 | 0.96 | 80 | 86 |
| MIF SLPI TIMP1 ENO2 CEACAM5 MMP2 CA242 GRP | 8 | 0.96 | 80 | 86 |
| MIF SLPI TIMP1 ENO2 CEACAM5 MMP2 CAIX GRP | 8 | 0.96 | 80 | 86 |
| MIF SLPI TIMP1 TFPI CEACAM5 MMP2 AMBP GRP | 8 | 0.96 | 80 | 86 |
| MIF SLPI TIMP1 TFPI CEACAM5 MMP2 GRP DEFA1 | 8 | 0.96 | 80 | 86 |
| MIF SLPI TIMP1 TFPI CEACAM5 Ca19-9 CA242 CAIX | 8 | 0.96 | 60 | 91 |
| MIF SLPI TIMP1 CEACAM5 MMP2 AMBP Ca19-9 GRP | 8 | 0.96 | 80 | 86 |
| MIF SLPI TIMP1 CEACAM5 MMP2 AMBP CA242 GRP | 8 | 0.96 | 80 | 86 |
| MIF SLPI TIMP1 CEACAM5 MMP2 AMBP CAIX GRP | 8 | 0.96 | 80 | 86 |
| MIF SLPI TIMP1 CEACAM5 MMP2 Ca19-9 GRP DEFA1 | 8 | 0.96 | 80 | 86 |
| MIF SLPI TIMP1 CEACAM5 MMP2 CA242 GRP DEFA1 | 8 | 0.96 | 80 | 86 |
| MIF SLPI TIMP1 CEACAM5 MMP2 CAIX GRP DEFA1 | 8 | 0.96 | 80 | 86 |
| MIF TIMP1 ENO2 TFPI CEACAM5 MMP2 Ca19-9 GRP | 8 | 0.96 | 80 | 86 |
| MIF TIMP1 ENO2 TFPI CEACAM5 MMP2 CA242 GRP | 8 | 0.96 | 80 | 86 |
| MIF TIMP1 ENO2 CEACAM5 MMP2 Ca19-9 CA242 GRP | 8 | 0.96 | 80 | 86 |
| MIF TIMP1 TFPI CEACAM5 MMP2 AMBP Ca19-9 GRP | 8 | 0.96 | 80 | 86 |
| MIF TIMP1 TFPI CEACAM5 MMP2 AMBP CA242 GRP | 8 | 0.96 | 80 | 86 |
| MIF TIMP1 TFPI CEACAM5 MMP2 Ca19-9 GRP DEFA1 | 8 | 0.96 | 80 | 86 |
| MIF TIMP1 TFPI CEACAM5 MMP2 CA242 GRP DEFA1 | 8 | 0.96 | 80 | 86 |
| MIF TIMP1 CEACAM5 MMP2 AMBP Ca19-9 CA242 GRP | 8 | 0.96 | 80 | 86 |
| MIF TIMP1 CEACAM5 MMP2 Ca19-9 CA242 GRP DEFA1 | 8 | 0.96 | 80 | 86 |
| SLPI TIMP1 ENO2 TFPI CEACAM5 MMP2 AMBP GRP | 8 | 0.96 | 80 | 86 |
| SLPI TIMP1 ENO2 TFPI CEACAM5 MMP2 GRP DEFA1 | 8 | 0.96 | 80 | 86 |
| SLPI TIMP1 ENO2 TFPI CEACAM5 Ca19-9 CA242 CAIX | 8 | 0.96 | 60 | 91 |
| SLPI TIMP1 ENO2 CEACAM5 MMP2 AMBP Ca19-9 GRP | 8 | 0.96 | 80 | 86 |
| SLPI TIMP1 ENO2 CEACAM5 MMP2 AMBP CA242 GRP | 8 | 0.96 | 80 | 86 |
| SLPI TIMP1 ENO2 CEACAM5 MMP2 AMBP CAIX GRP | 8 | 0.96 | 80 | 86 |
| SLPI TIMP1 ENO2 CEACAM5 MMP2 Ca19-9 GRP DEFA1 | 8 | 0.96 | 80 | 86 |
| SLPI TIMP1 ENO2 CEACAM5 MMP2 CA242 GRP DEFA1 | 8 | 0.96 | 80 | 86 |
| SLPI TIMP1 ENO2 CEACAM5 MMP2 CAIX GRP DEFA1 | 8 | 0.96 | 80 | 86 |
| SLPI TIMP1 TFPI CEACAM5 MMP2 AMBP GRP DEFA1 | 8 | 0.96 | 80 | 86 |
| SLPI TIMP1 TFPI CEACAM5 AMBP Ca19-9 CA242 CAIX | 8 | 0.96 | 60 | 91 |
| SLPI TIMP1 TFPI CEACAM5 Ca19-9 CA242 CAIX GRP | 8 | 0.96 | 60 | 91 |
| SLPI TIMP1 TFPI CEACAM5 Ca19-9 CA242 CAIX DEFA1 | 8 | 0.96 | 60 | 91 |
| SLPI TIMP1 CEACAM5 MMP2 AMBP Ca19-9 GRP DEFA1 | 8 | 0.96 | 80 | 86 |
| SLPI TIMP1 CEACAM5 MMP2 AMBP CA242 GRP DEFA1 | 8 | 0.96 | 80 | 86 |
| SLPI TIMP1 CEACAM5 MMP2 AMBP CAIX GRP DEFA1 | 8 | 0.96 | 80 | 86 |
| TIMP1 ENO2 TFPI CEACAM5 MMP2 AMBP Ca19-9 GRP | 8 | 0.96 | 80 | 86 |
| TIMP1 ENO2 TFPI CEACAM5 MMP2 AMBP CA242 GRP | 8 | 0.96 | 80 | 86 |
| TIMP1 ENO2 TFPI CEACAM5 MMP2 Ca19-9 GRP DEFA1 | 8 | 0.96 | 80 | 86 |
| TIMP1 ENO2 TFPI CEACAM5 MMP2 CA242 GRP DEFA1 | 8 | 0.96 | 80 | 86 |
| TIMP1 ENO2 CEACAM5 MMP2 AMBP Ca19-9 CA242 GRP | 8 | 0.96 | 80 | 86 |
| TIMP1 ENO2 CEACAM5 MMP2 Ca19-9 CA242 GRP DEFA1 | 8 | 0.96 | 80 | 86 |
| TIMP1 TFPI CEACAM5 MMP2 AMBP Ca19-9 GRP DEFA1 | 8 | 0.96 | 80 | 86 |
| TIMP1 TFPI CEACAM5 MMP2 AMBP CA242 GRP DEFA1 | 8 | 0.96 | 80 | 86 |
| TIMP1 CEACAM5 MMP2 AMBP Ca19-9 CA242 GRP DEFA1 | 8 | 0.96 | 80 | 86 |
| Cyfra MIF SLPI TIMP1 ENO2 TFPI CEACAM5 MMP2 CAIX | 9 | 0.96 | 80 | 86 |
| Cyfra MIF SLPI TIMP1 ENO2 TFPI CEACAM5 MMP2 GRP | 9 | 0.96 | 80 | 84 |
| Cyfra MIF SLPI TIMP1 ENO2 CEACAM5 MMP2 Ca19-9 CAIX | 9 | 0.96 | 80 | 86 |
| Cyfra MIF SLPI TIMP1 ENO2 CEACAM5 MMP2 Ca19-9 GRP | 9 | 0.96 | 80 | 84 |
| Cyfra MIF SLPI TIMP1 ENO2 CEACAM5 MMP2 CA242 CAIX | 9 | 0.96 | 80 | 86 |
| Cyfra MIF SLPI TIMP1 ENO2 CEACAM5 MMP2 CA242 GRP | 9 | 0.96 | 80 | 84 |
| Cyfra MIF SLPI TIMP1 ENO2 CEACAM5 MMP2 CAIX GRP | 9 | 0.96 | 80 | 84 |
| Cyfra MIF SLPI TIMP1 TFPI CEACAM5 MMP2 AMBP GRP | 9 | 0.96 | 80 | 86 |
| Cyfra MIF SLPI TIMP1 TFPI CEACAM5 MMP2 CAIX DEFA1 | 9 | 0.96 | 80 | 86 |
| Cyfra MIF SLPI TIMP1 TFPI CEACAM5 MMP2 GRP DEFA1 | 9 | 0.96 | 80 | 84 |
| Cyfra MIF SLPI TIMP1 CEACAM5 MMP2 AMBP Ca19-9 GRP | 9 | 0.96 | 80 | 86 |
| Cyfra MIF SLPI TIMP1 CEACAM5 MMP2 AMBP CA242 GRP | 9 | 0.96 | 80 | 86 |
| Cyfra MIF SLPI TIMP1 CEACAM5 MMP2 AMBP CAIX GRP | 9 | 0.96 | 80 | 86 |
| Cyfra MIF SLPI TIMP1 CEACAM5 MMP2 Ca19-9 CAIX DEFA1 | 9 | 0.96 | 80 | 86 |
| Cyfra MIF SLPI TIMP1 CEACAM5 MMP2 Ca19-9 GRP DEFA1 | 9 | 0.96 | 80 | 84 |
| Cyfra MIF SLPI TIMP1 CEACAM5 MMP2 CA242 CAIX DEFA1 | 9 | 0.96 | 80 | 86 |
| Cyfra MIF SLPI TIMP1 CEACAM5 MMP2 CA242 GRP DEFA1 | 9 | 0.96 | 80 | 84 |
| Cyfra MIF SLPI TIMP1 CEACAM5 MMP2 CAIX GRP DEFA1 | 9 | 0.96 | 80 | 84 |
| Cyfra MIF TIMP1 ENO2 TFPI CEACAM5 MMP2 Ca19-9 CAIX | 9 | 0.96 | 80 | 86 |
| Cyfra MIF TIMP1 ENO2 TFPI CEACAM5 MMP2 Ca19-9 GRP | 9 | 0.96 | 80 | 84 |
| Cyfra MIF TIMP1 ENO2 TFPI CEACAM5 MMP2 CA242 CAIX | 9 | 0.96 | 80 | 86 |
| Cyfra MIF TIMP1 ENO2 TFPI CEACAM5 MMP2 CA242 GRP | 9 | 0.96 | 80 | 84 |
| Cyfra MIF TIMP1 ENO2 TFPI CEACAM5 MMP2 CAIX GRP | 9 | 0.96 | 80 | 84 |
| Cyfra MIF TIMP1 ENO2 CEACAM5 MMP2 Ca19-9 CA242 CAIX | 9 | 0.96 | 80 | 86 |
| Cyfra MIF TIMP1 ENO2 CEACAM5 MMP2 Ca19-9 CA242 GRP | 9 | 0.96 | 80 | 84 |
| Cyfra MIF TIMP1 ENO2 CEACAM5 MMP2 Ca19-9 CAIX GRP | 9 | 0.96 | 80 | 84 |
| Cyfra MIF TIMP1 ENO2 CEACAM5 MMP2 CA242 CAIX GRP | 9 | 0.96 | 80 | 84 |
| Cyfra MIF TIMP1 TFPI CEACAM5 MMP2 AMBP Ca19-9 GRP | 9 | 0.96 | 80 | 86 |
| Cyfra MIF TIMP1 TFPI CEACAM5 MMP2 AMBP CA242 GRP | 9 | 0.96 | 80 | 86 |
| Cyfra MIF TIMP1 TFPI CEACAM5 MMP2 AMBP CAIX GRP | 9 | 0.96 | 80 | 86 |
| Cyfra MIF TIMP1 TFPI CEACAM5 MMP2 Ca19-9 CAIX DEFA1 | 9 | 0.96 | 80 | 86 |
| Cyfra MIF TIMP1 TFPI CEACAM5 MMP2 Ca19-9 GRP DEFA1 | 9 | 0.96 | 80 | 84 |
| Cyfra MIF TIMP1 TFPI CEACAM5 MMP2 CA242 CAIX DEFA1 | 9 | 0.96 | 80 | 86 |
| Cyfra MIF TIMP1 TFPI CEACAM5 MMP2 CA242 GRP DEFA1 | 9 | 0.96 | 80 | 84 |
| Cyfra MIF TIMP1 TFPI CEACAM5 MMP2 CAIX GRP DEFA1 | 9 | 0.96 | 80 | 84 |
| Cyfra MIF TIMP1 CEACAM5 MMP2 AMBP Ca19-9 CA242 GRP | 9 | 0.96 | 80 | 86 |
| Cyfra MIF TIMP1 CEACAM5 MMP2 AMBP Ca19-9 CAIX GRP | 9 | 0.96 | 80 | 86 |
| Cyfra MIF TIMP1 CEACAM5 MMP2 AMBP CA242 CAIX GRP | 9 | 0.96 | 80 | 86 |
| Cyfra MIF TIMP1 CEACAM5 MMP2 Ca19-9 CA242 CAIX DEFA1 | 9 | 0.96 | 80 | 86 |
| Cyfra MIF TIMP1 CEACAM5 MMP2 Ca19-9 CA242 GRP DEFA1 | 9 | 0.96 | 80 | 84 |
| Cyfra MIF TIMP1 CEACAM5 MMP2 Ca19-9 CAIX GRP DEFA1 | 9 | 0.96 | 80 | 84 |
| Cyfra MIF TIMP1 CEACAM5 MMP2 CA242 CAIX GRP DEFA1 | 9 | 0.96 | 80 | 84 |
| Cyfra SLPI TIMP1 ENO2 TFPI CEACAM5 MMP2 AMBP GRP | 9 | 0.96 | 80 | 86 |
| Cyfra SLPI TIMP1 ENO2 TFPI CEACAM5 MMP2 CAIX DEFA1 | 9 | 0.96 | 80 | 86 |
| Cyfra SLPI TIMP1 ENO2 TFPI CEACAM5 MMP2 GRP DEFA1 | 9 | 0.96 | 80 | 84 |
| Cyfra SLPI TIMP1 ENO2 CEACAM5 MMP2 AMBP Ca19-9 GRP | 9 | 0.96 | 80 | 86 |
| Cyfra SLPI TIMP1 ENO2 CEACAM5 MMP2 AMBP CA242 GRP | 9 | 0.96 | 80 | 86 |
| Cyfra SLPI TIMP1 ENO2 CEACAM5 MMP2 AMBP CAIX GRP | 9 | 0.96 | 80 | 86 |
| Cyfra SLPI TIMP1 ENO2 CEACAM5 MMP2 Ca19-9 CAIX DEFA1 | 9 | 0.96 | 80 | 86 |
| Cyfra SLPI TIMP1 ENO2 CEACAM5 MMP2 Ca19-9 GRP DEFA1 | 9 | 0.96 | 80 | 84 |
| Cyfra SLPI TIMP1 ENO2 CEACAM5 MMP2 CA242 CAIX DEFA1 | 9 | 0.96 | 80 | 86 |
| Cyfra SLPI TIMP1 ENO2 CEACAM5 MMP2 CA242 GRP DEFA1 | 9 | 0.96 | 80 | 84 |
| Cyfra SLPI TIMP1 ENO2 CEACAM5 MMP2 CAIX GRP DEFA1 | 9 | 0.96 | 80 | 84 |
| Cyfra SLPI TIMP1 TFPI CEACAM5 MMP2 AMBP Ca19-9 CAIX | 9 | 0.96 | 80 | 91 |
| Cyfra SLPI TIMP1 TFPI CEACAM5 MMP2 AMBP CA242 CAIX | 9 | 0.96 | 80 | 91 |
| Cyfra SLPI TIMP1 TFPI CEACAM5 MMP2 AMBP GRP DEFA1 | 9 | 0.96 | 80 | 86 |
| Cyfra SLPI TIMP1 CEACAM5 MMP2 AMBP Ca19-9 CA242 CAIX | 9 | 0.96 | 80 | 91 |
| Cyfra SLPI TIMP1 CEACAM5 MMP2 AMBP Ca19-9 GRP DEFA1 | 9 | 0.96 | 80 | 86 |
| Cyfra SLPI TIMP1 CEACAM5 MMP2 AMBP CA242 GRP DEFA1 | 9 | 0.96 | 80 | 86 |
| Cyfra SLPI TIMP1 CEACAM5 MMP2 AMBP CAIX GRP DEFA1 | 9 | 0.96 | 80 | 86 |
| Cyfra TIMP1 ENO2 TFPI CEACAM5 MMP2 AMBP Ca19-9 GRP | 9 | 0.96 | 80 | 86 |
| Cyfra TIMP1 ENO2 TFPI CEACAM5 MMP2 AMBP CA242 GRP | 9 | 0.96 | 80 | 86 |
| Cyfra TIMP1 ENO2 TFPI CEACAM5 MMP2 AMBP CAIX GRP | 9 | 0.96 | 80 | 86 |
| Cyfra TIMP1 ENO2 TFPI CEACAM5 MMP2 Ca19-9 CAIX DEFA1 | 9 | 0.96 | 80 | 86 |
| Cyfra TIMP1 ENO2 TFPI CEACAM5 MMP2 Ca19-9 GRP DEFA1 | 9 | 0.96 | 80 | 84 |
| Cyfra TIMP1 ENO2 TFPI CEACAM5 MMP2 CA242 CAIX DEFA1 | 9 | 0.96 | 80 | 86 |
| Cyfra TIMP1 ENO2 TFPI CEACAM5 MMP2 CA242 GRP DEFA1 | 9 | 0.96 | 80 | 84 |
| Cyfra TIMP1 ENO2 TFPI CEACAM5 MMP2 CAIX GRP DEFA1 | 9 | 0.96 | 80 | 84 |
| Cyfra TIMP1 ENO2 CEACAM5 MMP2 AMBP Ca19-9 CA242 GRP | 9 | 0.96 | 80 | 86 |
| Cyfra TIMP1 ENO2 CEACAM5 MMP2 AMBP Ca19-9 CAIX GRP | 9 | 0.96 | 80 | 86 |
| Cyfra TIMP1 ENO2 CEACAM5 MMP2 AMBP CA242 CAIX GRP | 9 | 0.96 | 80 | 86 |
| Cyfra TIMP1 ENO2 CEACAM5 MMP2 Ca19-9 CA242 CAIX DEFA1 | 9 | 0.96 | 80 | 86 |
| Cyfra TIMP1 ENO2 CEACAM5 MMP2 Ca19-9 CA242 GRP DEFA1 | 9 | 0.96 | 80 | 84 |
| Cyfra TIMP1 ENO2 CEACAM5 MMP2 Ca19-9 CAIX GRP DEFA1 | 9 | 0.96 | 80 | 84 |
| Cyfra TIMP1 ENO2 CEACAM5 MMP2 CA242 CAIX GRP DEFA1 | 9 | 0.96 | 80 | 84 |
| Cyfra TIMP1 TFPI CEACAM5 MMP2 AMBP Ca19-9 CA242 CAIX | 9 | 0.96 | 80 | 91 |
| Cyfra TIMP1 TFPI CEACAM5 MMP2 AMBP Ca19-9 GRP DEFA1 | 9 | 0.96 | 80 | 86 |
| Cyfra TIMP1 TFPI CEACAM5 MMP2 AMBP CA242 GRP DEFA1 | 9 | 0.96 | 80 | 86 |
| Cyfra TIMP1 TFPI CEACAM5 MMP2 AMBP CAIX GRP DEFA1 | 9 | 0.96 | 80 | 86 |
| Cyfra TIMP1 CEACAM5 MMP2 AMBP Ca19-9 CA242 GRP DEFA1 | 9 | 0.96 | 80 | 86 |
| Cyfra TIMP1 CEACAM5 MMP2 AMBP Ca19-9 CAIX GRP DEFA1 | 9 | 0.96 | 80 | 86 |
| Cyfra TIMP1 CEACAM5 MMP2 AMBP CA242 CAIX GRP DEFA1 | 9 | 0.96 | 80 | 86 |
| MIF SLPI TIMP1 ENO2 TFPI CEACAM5 MMP2 Ca19-9 GRP | 9 | 0.96 | . 80 | 86 |
| MIF SLPI TIMP1 ENO2 TFPI CEACAM5 MMP2 CA242 GRP | 9 | 0.96 | 80 | 86 |
| MIF SLPI TIMP1 ENO2 CEACAM5 MMP2 Ca19-9 CA242 GRP | 9 | 0.96 | 80 | 86 |
| MIF SLPI TIMP1 TFPI CEACAM5 MMP2 AMBP Ca19-9 GRP | 9 | 0.96 | 80 | 86 |
| MIF SLPI TIMP1 TFPI CEACAM5 MMP2 AMBP CA242 GRP | 9 | 0.96 | 80 | 86 |
| MIF SLPI TIMP1 TFPI CEACAM5 MMP2 Ca19-9 GRP DEFA1 | 9 | 0.96 | 80 | 86 |
| MIF SLPI TIMP1 TFPI CEACAM5 MMP2 CA242 GRP DEFA1 | 9 | 0.96 | 80 | 86 |
| MIF SLPI TIMP1 CEACAM5 MMP2 AMBP Ca19-9 CA242 GRP | 9 | 0.96 | 80 | 86 |
| MIF SLPI TIMP1 CEACAM5 MMP2 Ca19-9 CA242 GRP DEFA1 | 9 | 0.96 | 80 | 86 |
| MIF TIMP1 ENO2 TFPI CEACAM5 MMP2 AMBP CAIX GRP | 9 | 0.96 | 80 | 84 |
| MIF TIMP1 ENO2 TFPI CEACAM5 MMP2 Ca19-9 CA242 GRP | 9 | 0.96 | 80 | 86 |
| MIF TIMP1 ENO2 TFPI CEACAM5 MMP2 CAIX GRP DEFA1 | 9 | 0.96 | 80 | 84 |
| MIF TIMP1 ENO2 CEACAM5 MMP2 AMBP Ca19-9 CAIX GRP | 9 | 0.96 | 80 | 84 |
| MIF TIMP1 ENO2 CEACAM5 MMP2 AMBP CA242 CAIX GRP | 9 | 0.96 | 80 | 84 |
| MIF TIMP1 ENO2 CEACAM5 MMP2 Ca19-9 CAIX GRP DEFA1 | 9 | 0.96 | 80 | 84 |
| MIF TIMP1 ENO2 CEACAM5 MMP2 CA242 CAIX GRP DEFA1 | 9 | 0.96 | 80 | 84 |
| MIF TIMP1 TFPI CEACAM5 MMP2 AMBP Ca19-9 CA242 GRP | 9 | 0.96 | 80 | 86 |
| MIF TIMP1 TFPI CEACAM5 MMP2 AMBP CAIX GRP DEFA1 | 9 | 0.96 | 80 | 84 |
| MIF TIMP1 TFPI CEACAM5 MMP2 Ca19-9 CA242 GRP DEFA1 | 9 | 0.96 | 80 | 86 |
| MIF TIMP1 CEACAM5 MMP2 AMBP Ca19-9 CAIX GRP DEFA1 | 9 | 0.96 | 80 | 84 |
| MIF TIMP1 CEACAM5 MMP2 AMBP CA242 CAIX GRP DEFA1 | 9 | 0.96 | 80 | 84 |
| SLPI TIMP1 ENO2 TFPI CEACAM5 MMP2 AMBP Ca19-9 GRP | 9 | 0.96 | 80 | 86 |
| SLPI TIMP1 ENO2 TFPI CEACAM5 MMP2 AMBP CA242 GRP | 9 | 0.96 | 80. | 86 |
| SLPI TIMP1 ENO2 TFPI CEACAM5 MMP2 Ca19-9 GRP DEFA1 | 9 | 0.96 | 80 | 86 |
| SLPI TIMP1 ENO2 TFPI CEACAM5 MMP2 CA242 GRP DEFA1 | 9 | 0.96 | 80 | 86 |
| SLPI TIMP1 ENO2 CEACAM5 MMP2 AMBP Ca19-9 CA242 GRP | 9 | 0.96 | 80 | 86 |
| SLPI TIMP1 ENO2 CEACAM5 MMP2 Ca19-9 CA242 GRP DEFA1 | 9 | 0.96 | 80 | 86 |
| SLPI TIMP1 TFPI CEACAM5 MMP2 AMBP Ca19-9 GRP DEFA1 | 9 | 0.96 | 80 | 86 |
| SLPI TIMP1 TFPI CEACAM5 MMP2 AMBP CA242 GRP DEFA1 | 9 | 0.96 | 80 | 86 |
| SLPI TIMP1 CEACAM5 MMP2 AMBP Ca19-9 CA242 GRP DEFA1 | 9 | 0.96 | 80 | 86 |
| TIMP1 ENO2 TFPI CEACAM5 MMP2 AMBP Ca19-9 CA242 GRP | 9 | 0.96 | 80 | 86 |
| TIMP1 ENO2 TFPI CEACAM5 MMP2 AMBP CAIX GRP DEFA1 | 9 | 0.96 | 80 | 84 |
| TIMP1 ENO2 TFPI CEACAM5 MMP2 Ca19-9 CA242 GRP DEFA1 | 9 | 0.96 | 80 | 86 |
| TIMP1 ENO2 CEACAM5 MMP2 AMBP Ca19-9 CAIX GRP DEFA1 | 9 | 0.96 | 80 | 84 |
| TIMP1 ENO2 CEACAM5 MMP2 AMBP CA242 CAIX GRP DEFA1 | 9 | 0.96 | 80 | 84 |
| TIMP1 TFPI CEACAM5 MMP2 AMBP Ca19-9 CA242 GRP DEFA1 | 9 | 0.96 | 80 | 86 |
| Cyfra MIF SLPI TIMP1 ENO2 TFPI CEACAM5 MMP2 Ca19-9 CAIX | 10 | 0.96 | 80 | 86 |
| Cyfra MIF SLPI TIMP1 ENO2 TFPI CEACAM5 MMP2 Ca19-9 GRP | 10 | 0.96 | 80 | 84 |
| Cyfra MIF SLPI TIMP1 ENO2 TFPI CEACAM5 MMP2 CA242 CAIX | 10 | 0.96 | 80 | 86 |
| Cyfra MIF SLPI TIMP1 ENO2 TFPI CEACAM5 MMP2 CA242 GRP | 10 | 0.96 | 80 | 84 |
| Cyfra MIF SLPI TIMP1 ENO2 TFPI CEACAM5 MMP2 CAIX GRP | 10 | 0.96 | 80 | 84 |
| Cyfra MIF SLPI TIMP1 ENO2 CEACAM5 MMP2 Ca19-9 CA242 CAIX | 10 | 0.96 | 80 | 86 |
| Cyfra MIF SLPI TIMP1 ENO2 CEACAM5 MMP2 Ca19-9 CA242 GRP | 10 | 0.96 | 80 | 84 |
| Cyfra MIF SLPI TIMP1 ENO2 CEACAM5 MMP2 Ca19-9 CAIX GRP | 10 | 0.96 | 80 | 84 |
| Cyfra MIF SLPI TIMP1 ENO2 CEACAM5 MMP2 CA242 CAIX GRP | 10 | 0.96 | 80 | 84 |
| Cyfra MIF SLPI TIMP1 TFPI CEACAM5 MMP2 AMBP Ca19-9 GRP | 10 | 0.96 | 80 | 86 |
| Cyfra MIF SLPI TIMP1 TFPI CEACAM5 MMP2 AMBP CA242 GRP | 10 | 0.96 | 80 | 86 |
| Cyfra MIF SLPI TIMP1 TFPI CEACAM5 MMP2 AMBP CAIX GRP | 10 | 0.96 | 80 | 86 |
| Cyfra MIF SLPI TIMP1 TFPI CEACAM5 MMP2 Ca19-9 CAIX DEFA1 | 10 | 0.96 | 80 | 86 |
| Cyfra MIF SLPI TIMP1 TFPI CEACAM5 MMP2 Ca19-9 GRP DEFA1 | 10 | 0.96 | 80 | 84 |
| Cyfra MIF SLPI TIMP1 TFPI CEACAM5 MMP2 CA242 CAIX DEFA1 | 10 | 0.96 | 80 | 86 |
| Cyfra MIF SLPI TIMP1 TFPI CEACAM5 MMP2 CA242 GRP DEFA1 | 10 | 0.96 | 80 | 84 |
| Cyfra MIF SLPI TIMP1 TFPI CEACAM5 MMP2 CAIX GRP DEFA1 | 10 | 0.96 | 80 | 84 |
| Cyfra MIF SLPI TIMP1 CEACAM5 MMP2 AMBP Ca19-9 CA242 GRP | 10 | 0.96 | 80 | 86 |
| Cyfra MIF SLPI TIMP1 CEACAM5 MMP2 AMBP Ca19-9 CAIX GRP | 10 | 0.96 | 80 | 86 |
| Cyfra MIF SLPI TIMP1 CEACAM5 MMP2 AMBP CA242 CAIX GRP | 10 | 0.96 | 80 | 86 |
| Cyfra MIF SLPI TIMP1 CEACAM5 MMP2 Ca19-9 CA242 CAIX DEFA1 | 10 | 0.96 | 80 | 86 |
| Cyfra MIF SLPI TIMP1 CEACAM5 MMP2 Ca19-9 CA242 GRP DEFA1 | 10 | 0.96 | 80 | 84 |
| Cyfra MIF SLPI TIMP1 CEACAM5 MMP2 Ca19-9 CAIX GRP DEFA1 | 10 | 0.96 | 80 | 84 |
| Cyfra MIF SLPI TIMP1 CEACAM5 MMP2 CA242 CAIX GRP DEFA1 | 10 | 0.96 | 80 | 84 |
| Cyfra MIF TIMP1 ENO2 TFPI CEACAM5 MMP2 Ca19-9 CA242 CAIX | 10 | 0.96 | 80 | 86 |
| Cyfra MIF TIMP1 ENO2 TFPI CEACAM5 MMP2 Ca19-9 CA242 GRP | 10 | 0.96 | 80 | 84 |
| Cyfra MIF TIMP1 ENO2 TFPI CEACAM5 MMP2 Ca19-9 CAIX GRP | 10 | 0.96 | 80 | 84 |
| Cyfra MIF TIMP1 ENO2 TFPI CEACAM5 MMP2 CA242 CAIX GRP | 10 | 0.96 | 80 | 84 |
| Cyfra MIF TIMP1 ENO2 CEACAM5 MMP2 Ca19-9 CA242 CAIX GRP | 10 | 0.96 | 80 | 84 |
| Cyfra MIF TIMP1 TFPI CEACAM5 MMP2 AMBP Ca19-9 CA242 GRP | 10 | 0.96 | 80 | 86 |
| Cyfra MIF TIMP1 TFPI CEACAM5 MMP2 AMBP Ca19-9 CAIX GRP | 10 | 0.96 | 80 | 86 |
| Cyfra MIF TIMP1 TFPI CEACAM5 MMP2 AMBP CA242 CAIX GRP | 10 | 0.96 | 80 | 86 |
| Cyfra MIF TIMP1 TFPI CEACAM5 MMP2 Ca19-9 CA242 CAIX DEFA1 | 10 | 0.96 | 80 | 86 |
| Cyfra MIF TIMP1 TFPI CEACAM5 MMP2 Ca19-9 CA242 GRP DEFA1 | 10 | 0.96 | 80 | 84 |
| Cyfra MIF TIMP1 TFPI CEACAM5 MMP2 Ca19-9 CAIX GRP DEFA1 | 10 | 0.96 | 80 | 84 |
| Cyfra MIF TIMP1 TFPI CEACAM5 MMP2 CA242 CAIX GRP DEFA1 | 10 | 0.96 | 80 | 84 |
| Cyfra MIF TIMP1 CEACAM5 MMP2 AMBP Ca19-9 CA242 CAIX GRP | 10 | 0.96 | 80 | 86 |
| Cyfra MIF TIMP1 CEACAM5 MMP2 Ca19-9 CA242 CAIX GRP DEFA1 | 10 | 0.96 | 80 | 84 |
| Cyfra SLPI TIMP1 ENO2 TFPI CEACAM5 MMP2 AMBP Ca19-9 GRP | 10 | 0.96 | 80 | 86 |
| Cyfra SLPI TIMP1 ENO2 TFPI CEACAM5 MMP2 AMBP CA242 GRP | 10 | 0.96 | 80 | 86 |
| Cyfra SLPI TIMP1 ENO2 TFPI CEACAM5 MMP2 AMBP CAIX GRP | 10 | 0.96 | 80 | 86 |
| Cyfra SLPI TIMP1 ENO2 TFPI CEACAM5 MMP2 Ca19-9 CAIX DEFA1 | 10 | 0.96 | 80 | 86 |
| Cyfra SLPI TIMP1 ENO2 TFPI CEACAM5 MMP2 Ca19-9 GRP DEFA1 | 10 | 0.96 | 80 | 84 |
| Cyfra SLPI TIMP1 ENO2 TFPI CEACAM5 MMP2 CA242 CAIX DEFA1 | 10 | 0.96 | 80 | 86 |
| Cyfra SLPI TIMP1 ENO2 TFPI CEACAM5 MMP2 CA242 GRP DEFA1 | 10 | 0.96 | 80 | 84 |
| Cyfra SLPI TIMP1 ENO2 TFPI CEACAM5 MMP2 CAIX GRP DEFA1 | 10 | 0.96 | 80 | 84 |
| Cyfra SLPI TIMP1 ENO2 CEACAM5 MMP2 AMBP Ca19-9 CA242 GRP | 10 | 0.96 | 80 | 86 |
| Cyfra SLPI TIMP1 ENO2 CEACAM5 MMP2 AMBP Ca19-9 CAIX GRP | 10 | 0.96 | 80 | 86 |
| Cyfra SLPI TIMP1 ENO2 CEACAM5 MMP2 AMBP CA242 CAIX GRP | 10 | 0.96 | 80 | 86 |
| Cyfra SLPI TIMP1 ENO2 CEACAM5 MMP2 Ca19-9 CA242 CAIX DEFA1 | 10 | 0.96 | 80 | 86 |
| Cyfra SLPI TIMP1 ENO2 CEACAM5 MMP2 Ca19-9 CA242 GRP DEFA1 | 10 | 0.96 | 80 | 84 |
| Cyfra SLPI TIMP1 ENO2 CEACAM5 MMP2 Ca19-9 CAIX GRP DEFA1 | 10 | 0.96 | 80 | 84 |
| Cyfra SLPI TIMP1 EN02 CEACAM5 MMP2 CA242 CAIX GRP DEFA1 | 10 | 0.96 | 80 | 84 |
| Cyfra SLPI TIMP1 TFPI CEACAM5 MMP2 AMBP Ca19-9 CA242 CAIX | 10 | 0.96 | 80 | 91 |
| Cyfra SLPI TIMP1 TFPI CEACAM5 MMP2 AMBP Ca19-9 GRP DEFA1 | 10 | 0.96 | 80 | 86 |
| Cyfra SLPI TIMP1 TFPI CEACAM5 MMP2 AMBP CA242 GRP DEFA1 | 10 | 0.96 | 80 | 86 |
| Cyfra SLPI TIMP1 TFPI CEACAM5 MMP2 AMBP CAIX GRP DEFA1 | 10 | 0.96 | 80 | 86 |
| Cyfra SLPI TIMP1 CEACAM5 MMP2 AMBP Ca19-9 CA242 GRP DEFA1 | 10 | 0.96 | 80 | 86 |
| Cyfra SLPI TIMP1 CEACAM5 MMP2 AMBP Ca19-9 CAIX GRP DEFA1 | 10 | 0.96 | 80 | 86 |
| Cyfra SLPI TIMP1 CEACAM5 MMP2 AMBP CA242 CAIX GRP DEFA1 | 10 | 0.96 | 80 | 86 |
| Cyfra TIMP1 ENO2 TFPI CEACAM5 MMP2 AMBP Ca19-9 CA242 GRP | 10 | 0.96 | 80 | 86 |
| Cyfra TIMP1 ENO2 TFPI CEACAM5 MMP2 AMBP Ca19-9 CAIX GRP | 10 | 0.96 | 80 | 86 |
| Cyfra TIMP1 ENO2 TFPI CEACAM5 MMP2 AMBP CA242 CAIX GRP | 10 | 0.96 | 80 | 86 |
| Cyfra TIMP1 ENO2 TFPI CEACAM5 MMP2 Ca19-9 CA242 CAIX DEFA1 | 10 | 0.96 | 80 | 86 |
| Cyfra TIMP1 ENO2 TFPI CEACAM5 MMP2 Ca19-9 CA242 GRP DEFA1 | 10 | 0.96 | 80 | 84 |
| Cyfra TIMP1 ENO2 TFPI CEACAM5 MMP2 Ca19-9 CAIX GRP DEFA1 | 10 | 0.96 | 80 | 84 |
| Cyfra TIMP1 ENO2 TFPI CEACAM5 MMP2 CA242 CAIX GRP DEFA1 | 10 | 0.96 | 80 | 84 |
| Cyfra TIMP1 ENO2 CEACAM5 MMP2 AMBP Ca19-9 CA242 CAIX GRP | 10 | 0.96 | 80 | 86 |
| Cyfra TIMP1 ENO2 CEACAM5 MMP2 Ca19-9 CA242 CAIX GRP DEFA1 | 10 | 0.96 | 80 | 84 |
| Cyfra TIMP1 TFPI CEACAM5 MMP2 AMBP Ca19-9 CA242 GRP DEFA1 | 10 | 0.96 | 80 | 86 |
| Cyfra TIMP1 TFPI CEACAM5 MMP2 AMBP Ca19-9 CAIX GRP DEFA1 | 10 | 0.96 | 80 | 86 |
| Cyfra TIMP1 TFPI CEACAM5 MMP2 AMBP CA242 CAIX GRP DEFA1 | 10 | 0.96 | 80 | 86 |
| Cyfra TIMP1 CEACAM5 MMP2 AMBP Ca19-9 CA242 CAIX GRP DEFA1 | 10 | 0.96 | 80 | 86 |
| MIF SLPI TIMP1 ENO2 TFPI CEACAM5 MMP2 AMBP CAIX GRP | 10 | 0.96 | 80 | 84 |
| MIF SLPI TIMP1 ENO2 TFPI CEACAM5 MMP2 Ca19-9 CA242 GRP | 10 | 0.96 | 80 | 86 |
| MIF SLPI TIMP1 ENO2 TFPI CEACAM5 MMP2 CAIX GRP DEFA1 | 10 | 0.96 | 80 | 84 |
| MIF SLPI TIMP1 ENO2 CEACAM5 MMP2 AMBP Ca19-9 CAIX GRP | 10 | 0.96 | 80 | 84 |
| MIF SLPI TIMP1 ENO2 CEACAM5 MMP2 AMBP CA242 CAIX GRP | 10 | 0.96 | 80 | 84 |
| MIF SLPI TIMP1 ENO2 CEACAM5 MMP2 Ca19-9 CAIX GRP DEFA1 | 10 | 0.96 | 80 | 84 |
| MIF SLPI TIMP1 ENO2 CEACAM5 MMP2 CA242 CAIX GRP DEFA1 | 10 | 0.96 | 80 | 84 |
| MIF SLPI TIMP1 TFPI CEACAM5 MMP2 AMBP Ca19-9 CA242 GRP | 10 | 0.96 | 80 | 86 |
| MIF SLPI TIMP1 TFPI CEACAM5 MMP2 AMBP CAIX GRP DEFA1 | 10 | 0.96 | 80 | 84 |
| MIF SLPI TIMP1 TFPI CEACAM5 MMP2 Ca19-9 CA242 GRP DEFA1 | 10 | 0.96 | 80 | 86 |
| MIF SLPI TIMP1 CEACAM5 MMP2 AMBP Ca19-9 CAIX GRP DEFA1 | 10 | 0.96 | 80 | 84 |
| MIF SLPI TIMP1 CEACAM5 MMP2 AMBP CA242 CAIX GRP DEFA1 | 10 | 0.96 | 80 | 84 |

**TABLE 12**

| Small Cell and NSC vs. Normal | Split-point | | | |
|---|---|---|---|---|
| | Trained/tested on 44x44 | | | |
| Panel | Size | AUC | Sens at 95% Spec | Spec at 95% Sens |
| Cyfra TIMP1 SCC EN02 TFPI CEACAM5 OPN Ca72-4 CAIX GRP DEFA1 | 11 | 0.91 | 61 | 82 |
| Cyfra TIMP1 SCC ENO2 TFPI CEACAM5 OPN Ca72-4 GRP DEFA1 | 10 | 0.91 | 61 | 82 |
| Cyfra TIMP1 SCC ENO2 TFPI CEACAM5 OPN Ca72-4 CA242 GRP DEFA1 | 11 | 0.90 | 64 | 77 |
| Cyfra TIMP1 SCC ENO2 TFPI CEACAM5 OPN Ca72-4 CA242 CAIX GRP DEFA1 | 12 | 0.90 | 64 | 77 |
| Cyfra TIMP1 ENO2 TFPI CEACAM5 OPN Ca72-4 CAIX GRP DEFA1 | 10 | 0.90 | 57 | 82 |
| Cyfra TIMP1 ENO2 TFPI CEACAM5 OPN Ca72-4 GRP DEFA1 | 9 | 0.90 | 57 | 82 |
| TIMP1 SCC ENO2 TFPI CEACAM5 OPN Ca72-4 CA242 CAIX GRP DEFA1 | 11 | 0.90 | 64 | 77 |
| Cyfra TIMP1 ENO2 TFPI CEACAM5 OPN Ca72-4 CA242 GRP DEFA1 | 10 | 0.90 | 61 | 77 |
| Cyfra TIMP1 ENO2 TFPI CEACAM5 OPN Ca72-4 CA242 CAIX GRP DEFA1 | 11 | 0.90 | 61 | 77 |
| Cyfra TIMP1 SCC ENO2 CEACAM5 OPN Ca19-9 Ca72-4 CAIX GRP DEFA1 | 11 | 0.90 | 39 | 80 |
| Cyfra TIMP1 SCC ENO2 TFPI CEACAM5 OPN Ca19-9 Ca72-4 CAIX GRP DEFA1 | 12 | 0.90 | 45 | 75 |
| TIMP1 SCC ENO2 TFPI CEACAM5 OPN Ca72-4 CAIX GRP DEFA1 | 10 | 0.90 | 61 | 82 |
| Cyfra TIMP1 SCC ENO2 CEACAMS OPN Ca19-9 Ca72-4 GRP DEFA1 | 10 | 0.90 | 39 | 80 |
| TIMP1 SCC ENO2 TFPI CEACAM5 OPN Ca72-4 CA242 GRP DEFA1 | 10 | 0.90 | 64 | 77 |
| Cyfra TIMP1 SCC ENO2 CEACAM5 OPN Ca19-9 Ca72-4 CA242 CAIX GRP DEFA1 | 12 | 0.90 | 45 | 59 |
| Cyfra TIMP1 SCC ENO2 TFPI CEACAM5 OPN Ca19-9 Ca72-4 GRP DEFA1 | 11 | 0.90 | 45 | 75 |
| TIMP1 SCC ENO2 TFPI CEACAM5 OPN Ca72-4 GRP DEFA1 | 9 | 0.90 | 61 | 82 |
| Cyfra TIMP1 SCC ENO2 CEACAM5 OPN Ca72-4 CA242 CAIX GRP DEFA1 | 11 | 0.90 | 50 | 82 |
| Cyfra TIMP1 SCC ENO2 CEACAM5 OPN Ca19-9 Ca72-4 CA242 GRP DEFA1 | 11 | 0.90 | 45 | 59 |
| TIMP1 SCC ENO2 TFPI CEACAM5 OPN Ca19-9 Ca72-4 CAIX GRP DEFA1 | 11 | 0.90 | 45 | 75 |
| Cyfra TIMP1 ENO2 TFPI CEACAM5 OPN Ca19-9 Ca72-4 CAIX GRP DEFA1 | 11 | 0.90 | 41 | 75 |
| TIMP1 ENO2 TFPI CEACAM5 OPN Ca72-4 CA242 CAIX GRP DEFA1 | 10 | 0.90 | 61 | 77 |
| Cyfra TIMP1 SCC ENO2 CEACAM5 OPN Ca72-4 CA242 GRP DEFA1 | 10 | 0.90 | 50 | 82 |
| TIMP1 ENO2 TFPI CEACAM5 OPN Ca72-4 CAIX GRP DEFA1 | 9 | 0.90 | 57 | 82 |
| Cyfra TIMP1 SCC ENO2 TFPI CEACAM5 OPN Ca72-4 CAIX DEFA1 | 10 | 0.90 | 57 | 39 |
| TIMP1 SCC ENO2 TFPI CEACAM5 OPN Ca19-9 Ca72-4 GRP DEFA1 | 10 | 0.90 | 43 | 75 |
| Cyfra TIMP1 ENO2 TFPI CEACAM5 OPN Ca19-9 Ca72-4 GRP DEFA1 | 10 | 0.90 | 41 | 75 |
| TIMP1 ENO2 TFPI CEACAM5 OPN Ca72-4 CA242 GRP DEFA1 | 9 | 0.90 | 61 | 77 |
| TIMP1 ENO2 TFPI CEACAM5 OPN Ca72-4 GRP DEFA1 | 8 | 0.90 | 57 | 82 |
| Cyfra MIF TIMP1 SCC TFPI CEACAM5 OPN Ca19-9 Ca72-4 CAIX GRP DEFA1 | 12 | 0.90 | 66 | 30 |
| Cyfra TIMP1 SCC ENO2 TFPI CEACAM5 OPN Ca72-4 DEFA1 | 9 | 0.90 | 57 | 39 |
| TIMP1 SCC ENO2 CEACAM5 OPN Ca19-9 Ca72-4 CAIX GRP DEFA1 | 10 | 0.90 | 36 | 80 |
| TIMP1 ENO2 TFPI CEACAM5 OPN Ca19-9 Ca72-4 CAIX GRP DEFA1 | 10 | 0.90 | 41 | 75 |
| Cyfra TIMP1 ENO2 CEACAM5 OPN Ca72-4 CA242 CAIX GRP DEFA1 | 10 | 0.90 | 50 | 82 |
| Cyfra MIF TIMP1 SCC TFPI CEACAM5 OPN Ca19-9 Ca72-4 GRP DEFA1 | 11 | 0.90 | 66 | 30 |
| MIF TIMP1 SCC TFPI CEACAM5 OPN Ca19-9 Ca72-4 CAIX GRP DEFA1 | 11 | 0.90 | 64 | 32 |
| Cyfra MIF TIMP1 SCC TFPI CEACAM5 OPN Ca72-4 CAIX GRP DEFA1 | 11 | 0.90 | 75 | 50 |
| MIF TIMP1 SCC TFPI CEACAM5 OPN Ca19-9 Ca72-4 GRP DEFA1 | 10 | 0.90 | 64 | 32 |
| Cyfra MIF TIMP1 SCC CEACAM5 OPN Ca19-9 Ca72-4 CAIX GRP DEFA1 | 11 | 0.90 | 45 | 36 |
| Cyfra TIMP1 ENO2 TFPI CEACAM5 OPN Ca72-4 CAIX DEFA1 | 9 | 0.90 | 50 | 39 |
| TIMP1 ENO2 TFPI CEACAM5 OPN Ca19-9 Ca72-4 GRP DEFA1 | 9 | 0.90 | 39 | 75 |
| Cyfra TIMP1 SCC TFPI CEACAM5 OPN Ca72-4 CAIX GRP DEFA1 | 10 | 0.90 | 68 | 61 |
| Cyfra SCC ENO2 TFPI CEACAM5 OPN Ca72-4 CAIX GRP DEFA1 | 10 | 0.90 | 32 | 84 |
| Cyfra TIMP1 ENO2 CEACAM5 OPN Ca72-4 CA242 GRP DEFA1 | 9 | 0.90 | 50 | 82 |
| Cyfra MIF TIMP1 SCC CEACAM5 OPN Ca19-9 Ca72-4 GRP DEFA1 | 10 | 0.90 | 45 | 36 |
| Cyfra MIF TIMP1 SCC TFPI CEACAM5 OPN Ca72-4 GRP DEFA1 | 10 | 0.90 | 75 | 50 |
| TIMP1 SCC ENO2 CEACAM5 OPN Ca19-9 Ca72-4 GRP DEFA1 | 9 | 0.90 | 34 | 80 |
| TIMP1 SCC TFPI CEACAM5 OPN Ca72-4 CAIX GRP DEFA1 | 9 | 0.90 | 66 | 64 |
| Cyfra MIF TIMP1 TFPI CEACAM5 OPN Ca19-9 Ca72-4 CAIX GRP DEFA1 | 11 | 0.90 | 61 | 30 |
| Cyfra MIF TIMP1 SCC ENO2 TFPI CEACAMS OPN Ca19-9 Ca72-4 CAIX GRP DEFA1 | 13 | 0.90 | 55 | 59 |
| Cyfra TIMP1 ENO2 TFPI CEACAMS OPN Ca72-4 DEFA1 | 8 | 0.90 | 50 | 39 |
| TIMP1 SCC ENO2 TFPI CEACAM5 OPN Ca72-4 CAIX DEFA1 | 9 | 0.90 | 57 | 43 |
| MIF TIMP1 SCC TFPI CEACAM5 OPN Ca72-4 GRP DEFA1 | 9 | 0.90 | 73 | 52 |
| MIF TIMP1 SCC TFPI CEACAM5 OPN Ca72-4 CAIX GRP DEFA1 | 10 | 0.90 | 73 | 52 |
| Cyfra TIMP1 ENO2 CEACAM5 OPN Ca19-9 Ca72-4 CAIX GRP DEFA1 | 10 | 0.90 | 32 | 80 |
| Cyfra TIMP1 SCC TFPI CEACAM5 OPN Ca72-4 GRP DEFA1 | 9 | 0.90 | 68 | 61 |
| Cyfra MIF TIMP1 TFPI CEACAM5 OPN Ca19-9 Ca72-4 GRP DEFA1 | 10 | 0.90 | 61 | 30 |
| Cyfra MIF TIMP1 SCC ENO2 TFPI CEACAM5 OPN Ca19-9 Ca72-4 GRP DEFA1 | 12 | 0.90 | 55 | 59 |
| MIF TIMP1 TFPI CEACAM5 OPN Ca19-9 Ca72-4 CAIX GRP DEFA1 | 10 | 0.90 | 59 | 32 |
| Cyfra MIF TIMP1 SCC ENO2 TFPI CEACAM5 OPN Ca72-4 CAIX GRP DEFA1 | 12 | 0.90 | 70 | 70 |
| Cyfra TIMP1 SCC TFPI CEACAM5 OPN Ca72-4 CA242 GRP DEFA1 | 10 | 0.90 | 73 | 52 |
| Cyfra TIMP1 SCC TFPI CEACAM5 OPN Ca72-4 CA242 CAIX GRP DEFA1 | 11 | 0.90 | 73 | 52 |
| Cyfra TIMP1 ENO2 CEACAM5 OPN Ca19-9 Ca72-4 GRP DEFA1 | 9 | 0.90 | 32 | 80 |
| Cyfra TIMP1 TFPI CEACAM5 OPN Ca72-4 CAIX GRP DEFA1 | 9 | 0.90 | 68 | 61 |
| TIMP1 SCC TFPI CEACAM5 OPN Ca72-4 GRP DEFA1 | 8 | 0.90 | 66 | 64 |
| MIF TIMP1 TFPI CEACAM5 OPN Ca19-9 Ca72-4 GRP DEFA1 | 9 | 0.90 | 59 | 32 |
| TIMP1 SCC ENO2 TFPI CEACAM5 OPN Ca72-4 DEFA1 | 8 | 0.90 | 55 | 43 |
| Cyfra TIMP1 SCC TFPI CEACAM5 OPN Ca19-9 Ca72-4 CAIX GRP DEFA1 | 11 | 0.90 | 59 | 45 |
| MIF TIMP1 SCC CEACAM5 OPN Ca19-9 Ca72-4 CAIX GRP DEFA1 | 10 | 0.90 | 45 | 39 |
| Cyfra MIF TIMP1 TFPI CEACAM5 OPN Ca72-4 CAIX GRP DEFA1 | 10 | 0.90 | 75 | 50 |
| TIMP1 TFPI CEACAM5 OPN Ca72-4 CAIX GRP DEFA1 | 8 | 0.90 | 66 | 64 |
| Cyfra SCC ENO2 TFPI CEACAM5 OPN Ca72-4 GRP DEFA1 | 9 | 0.90 | 32 | 84 |
| Cyfra MIF TIMP1 SCC ENO2 TFPI CEACAM5 OPN Ca72-4 GRP DEFA1 | 11 | 0.90 | 70 | 70 |
| MIF TIMP1 SCC ENO2 TFPI CEACAM5 OPN Ca19-9 Ca72-4 CAIX GRP DEFA1 | 12 | 0.90 | 55 | 59 |
| MIF TIMP1 SCC CEACAM5 OPN Ca19-9 Ca72-4 GRP DEFA1 | 9 | 0.90 | 45 | 39 |
| Cyfra TIMP1 TFPI CEACAM5 OPN Ca72-4 CA242 GRP DEFA1 | 9 | 0.90 | 73 | 52 |
| Cyfra TIMP1 TFPI CEACAM5 OPN Ca72-4 CA242 CAIX GRP DEFA1 | 10 | 0.90 | 73 | 52 |
| Cyfra TIMP1 TFPI CEACAM5 OPN Ca72-4 GRP DEFA1 | 8 | 0.89 | 68 | 61 |
| TIMP1 ENO2 TFPI CEACAM5 OPN Ca72-4 CAIX DEFA1 | 8 | 0.89 | 50 | 43 |
| Cyfra TIMP1 SCC TFPI CEACAM5 OPN Ca19-9 Ca72-4 GRP DEFA1 | 10 | 0.89 | 59 | 45 |
| MIF TIMP1 SCC ENO2 TFPI CEACAM5 OPN Ca19-9 Ca72-4 GRP DEFA1 | 11 | 0.89 | 55 | 59 |
| Cyfra TIMP1 SCC ENO2 CEACAM5 OPN Ca72-4 CA242 CAIX DEFA1 | 10 | 0.89 | 48 | 32 |
| Cyfra MIF TIMP1 TFPI CEACAM5 OPN Ca72-4 GRP DEFA1 | 9 | 0.89 | 75 | 50 |
| TIMP1 SCC TFPI CEACAM5 OPN Ca72-4 CA242 CAIX GRP DEFA1 | 10 | 0.89 | 70 | 55 |
| TIMP1 TFPI CEACAM5 OPN Ca72-4 GRP DEFA1 | 7 | 0.89 | 66 | 64 |
| TIMP1 SCC TFPI CEACAM5 OPN Ca19-9 Ca72-4 CAIX GRP DEFA1 | 10 | 0.89 | 57 | 48 |
| Cyfra MIF TIMP1 CEACAM5 OPN Ca19-9 Ca72-4 CAIX GRP DEFA1 | 10 | 0.89 | 43 | 36 |
| Cyfra MIF TIMP1 SCC ENO2 TFPI CEACAM5 OPN Ca72-4 CAIX DEFA1 | 11 | 0.89 | 68 | 73 |
| Cyfra TIMP1 SCC ENO2 CEACAM5 OPN Ca72-4 CA242 DEFA1 | 9 | 0.89 | 48 | 32 |
| MIF TIMP1 TFPI CEACAM5 OPN Ca72-4 GRP DEFA1 | 8 | 0.89 | 73 | 52 |
| MIF TIMP1 TFPI CEACAM5 OPN Ca72-4 CAIX GRP DEFA1 | 9 | 0.89 | 73 | 52 |
| Cyfra TIMP1 SCC ENO2 TFPI CEACAM5 OPN Ca19-9 Ca72-4 CAIX GRF | 11 | 0.89 | 57 | 43 |
| Cyfra MIF TIMP1 ENO2 TFPI CEACAM5 OPN Ca19-9 Ca72-4 CAIX GRP DEFA1 | 12 | 0.89 | 50 | 59 |
| Cyfra SLPI TIMP1 SCC ENO2 TFPI CEACAM5 OPN Ca19-9 Ca72-4 CAIX GRP DEFA1 | 13 | 0.89 | 64 | 57 |
| TIMP1 ENO2 TFPI CEACAM5 OPN Ca72-4 DEFA1 | 7 | 0.89 | 48 | 43 |
| Cyfra TEMP1 TFPI CEACAM5 OPN Ca19-9 Ca72-4 CAIX GRP DEFA1 | 10 | 0.89 | 55 | 45 |
| Cyfra MIF TIMP1 CEACAM5 OPN Ca19-9 Ca72-4 GRP DEFA1 | 9 | 0.89 | 43 | 36 |
| Cyfra MIF TIMP1 SCC ENO2 TFPI CEACAM5 OPN Ca72-4 DEFA1 | 10 | 0.89 | 68 | 73 |
| TIMP1 SCC TFPI CEACAM5 OPN Ca72-4 CA242 GRP DEFA1 | 9 | 0.89 | 70 | 55 |
| TIMP1 TFPI CEACAM5 OPN Ca72-4 CA242 CAIX GRP DEFA1 | 9 | 0.89 | 70 | 55 |
| Cyfra TIMP1 SCC ENO2 TFPI CEACAM5 OPN Ca19-9 Ca72-4 CA242 CAIX GRP DEFA1 | 13 | 0.89 | 50 | 57 |
| Cyfra TIMP1 SCC ENO2 TFPI CEACAM5 OPN Ca72-4 CA242 CAIX DEFA1 | 11 | 0.89 | 59 | 23 |
| Cyfra SLPI TIMP1 SCC ENO2 TFPI CEACAM5 OPN Ca19-9 Ca72-4 GRP DEFA1 | 12 | 0.89 | 64 | 57 |
| TIMP1 SCC TFPI CEACAM5 OPN Ca19-9 Ca72-4 GRP DEFA1 | 9 | 0.89 | 55 | 48 |
| Cyfra MIF SLPI TIMP1 SCC CEACAM5 OPN Ca19-9 Ca72-4 GRP DEFA1 | 11 | 0.89 | 66 | 34 |
| Cyfra MIF TIMP1 ENO2 TFPI CEACAM5 OPN Ca19-9 Ca72-4 GRP DEFA1 | 11 | 0.89 | 50 | 59 |
| Cyfra MIF TIMP1 ENO2 TFPI CEACAM5 OPN Ca72-4 CAIX GRP DEFA1 | 11 | 0.89 | 66 | 70 |
| Cyfra MIF SLPI TIMP1 SCC CEACAM5 OPN Ca19-9 Ca72-4 CAIX GRP DEFA1 | 12 | 0.89 | 66 | 34 |
| TIMP1 ENO2 CEACAM5 OPN Ca19-9 Ca72-4 CAIX GRP DEFA1 | 9 | 0.89 | 30 | 80 |
| TIMP1 SCC ENO2 CEACAM5 OPN Ca19-9 Ca72-4 CA242 CAIX GRP DEFA1 | 11 | 0.89 | 45 | 59 |
| Cyfra TIMP1 SCC ENO2 TFPI CEACAM5 OPN Ca19-9 Ca72-4 GRF | 10 | 0.89 | 57 | 43 |
| Cyfra TIMP1 SCC ENO2 TFPI CEACAM5 OPN Ca19-9 Ca72-4 CA242 GRP DEFA1 | 12 | 0.89 | 50 | 57 |
| Cyfra TIMP1 SCC ENO2 TFPI CEACAM5 OPN Ca72-4 CA242 DEFA1 | 10 | 0.89 | 59 | 23 |
| Cyfra SLPI TIMP1 ENO2 TFPI CEACAM5 OPN Ca19-9 Ca72-4 CAIX GRP DEFA1 | 12 | 0.89 | 59 | 57 |
| Cyfra TIMP1 TFPI CEACAM5 OPN Ca19-9 Ca72-4 GRP DEFA1 | 9 | 0.89 | 55 | 45 |
| Cyfra MIF TIMP1 SCC TFPI CEACAM5 OPN Ca72-4 CA242 GRP DEFA1 | 11 | 0.89 | 55 | 36 |
| Cyfra MIF TIMP1 SCC TFPI CEACAM5 OPN Ca72-4 CA242 CAIX GRP DEFA1 | 12 | 0.89 | 55 | 36 |
| Cyfra TIMP1 SCC ENO2 CEACAM5 OPN Ca72-4 GRP DEFA1 | 9 | 0.89 | 45 | 50 |
| Cyfra TIMP1 SCC ENO2 CEACAM5 OPN Ca72-4 CAIX GRP DEFA1 | 10 | 0.89 | 45 | 50 |
| Cyfra MIF TIMP1 SCC ENO2 TFPI CEACAM5 OPN Ca72-4 CA242 GRP DEFA1 | 12 | 0.89 | 75 | 64 |
| Cyfra MIF TIMP1 SCC ENO2 TFPI CEACAM5 OPN Ca72-4 CA242 CAIX GRP DEFA1 | 13 | 0.89 | 75 | 64 |
| TIMP1 TFPI CEACAM5 OPN Ca72-4 CA242 GRP DEFA1 | 8 | 0.89 | 70 | 55 |
| Cyfra SCC ENO2 TFPI CEACAM5 OPN Ca19-9 Ca72-4 CAIX GRP DEFA1 | 11 | 0.89 | 25 | 77 |
| Cyfra TIMP1 ENO2 CEACAM5 OPN Ca19-9 Ca72-4 CA242 CAIX GRP DEFA1 | 11 | 0.89 | 43 | 59 |
| Cyfra SLPI TIMP1 ENO2 TFPI CEACAM5 OPN Ca19-9 Ca72-4 GRP DEFA1 | 11 | 0.89 | 59 | 57 |
| Cyfra MIF TIMP1 ENO2 TFPI CEACAM5 OPN Ca72-4 GRP DEFA1 | 10 | 0.89 | 66 | 70 |
| Cyfra TIMP1 SCC TFPI CEACAM5 OPN Ca72-4 CAIX GRP | 9 | 0.89 | 70 | 32 |
| TIMP1 TFPI CEACAM5 OPN Ca19-9 Ca72-4 CAIX GRP DEFA1 | 9 | 0.89 | 52 | 48 |
| Cyfra MIF TIMP1 ENO2 TFPI CEACAM5 OPN Ca72-4 CAIX DEFA1 | 10 | 0.89 | 61 | 73 |
| SLPI TIMP1 SCC ENO2 TFPI CEACAM5 OPN Ca19-9 Ca72-4 CAIX GRP DEFA1 | 12 | 0.89 | 64 | 59 |
| MIF TIMP1 CEACAM5 OPN Ca19-9 Ca72-4 CAIX GRP DEFA1 | 9 | 0.89 | 43 | 39 |
| MIF TIMP1 ENO2 TFPI CEACAM5 OPN Ca19-9 Ca72-4 CAIX GRP DEFA1 | 11 | 0.89 | 50 | 59 |
| Cyfra SLPI TIMP1 SCC ENO2 CEACAM5 OPN Ca19-9 Ca72-4 CAIX GRP DEFA1 | 12 | 0.89 | 48 | 64 |
| Cyfra TIMP1 SCC CEACAM5 OPN Ca19-9 Ca72-4 CAIX GRP DEFA1 | 10 | 0.89 | 43 | 50 |
| Cyfra TIMP1 ENO2 CEACAM5 OPN Ca19-9 Ca72-4 CA242 GRP DEFA1 | 10 | 0.89 | 43 | 59 |
| Cyfra TIMP1 ENO2 TFPI CEACAM5 OPN Ca19-9 Ca72-4 CAIX GRF | 10 | 0.89 | 50 | 43 |
| TIMP1 SCC ENO2 TFPI CEACAM5 OPN Ca72-4 CA242 CAIX DEFA1 | 10 | 0.89 | 59 | 27 |
| TIMP1 SCC ENO2 TFPI CEACAM5 OPN Ca19-9 Ca72-4 CA242 CAIX GRP DEFA1 | 12 | 0.89 | 50 | 57 |
| Cyfra ENO2 TFPI CEACAMS OPN Ca72-4 CAIX GRP DEFA1 | 9 | 0.89 | 30 | 84 |
| MIF TIMP1 ENO2 TFPI CEACAM5 OPN Ca19-9 Ca72-4 GRP DEFA1 | 10 | 0.89 | 50 | 59 |
| Cyfra SLPI TIMP1 SCC ENO2 CEACAM5 OPN Ca19-9 Ca72-4 GRP DEFA1 | 11 | 0.89 | 48 | 64 |
| Cyfra MIF TIMP1 ENO2 TFPI CEACAM5 OPN Ca72-4 DEFA1 | 9 | 0.89 | 61 | 73 |
| Cyfra TIMP1 SCC ENO2 TFPI CEACAM5 OPN GRP DEFA1 | 9 | 0.89 | 70 | 48 |
| Cyfra TIMP1 SCC ENO2 TFPI CEACAM5 OPN CAIX GRP DEFA1 | 10 | 0.89 | 70 | 48 |
| MIF TIMP1 CEACAM5 OPN Ca19-9 Ca72-4 GRP DEFA1 | 8 | 0.89 | 43 | 39 |
| Cyfra TIMP1 SCC CEACAM5 OPN Ca19-9 Ca72-4 GRP DEFA1 | 9 | 0.89 | 43 | 50 |
| Cyfra TIMP1 ENO2 CEACAM5 OPN Ca72-4 CA242 CAIX DEFA1 | 9 | 0.89 | 45 | 32 |
| Cyfra MIF TIMP1 SCC CEACAM5 OPN Ca72-4 CA242 CAIX GRP DEFA1 | 11 | 0.89 | 66 | 43 |
| Cyfra MIF TIMP1 SCC ENO2 CEACAM5 OPN Ca19-9 Ca72-4 CAIX GRP DEFA1 | 12 | 0.89 | 43 | 66 |
| TIMP1 ENO2 CEACAM5 OPN Ca19-9 Ca72-4 GRP DEFA1 | 8 | 0.89 | 27 | 80 |
| TIMP1 SCC ENO2 CEACAM5 OPN Ca19-9 Ca72-4 CA242 GRP DEFA1 | 10 | 0.89 | 43 | 59 |
| Cyfra TIMP1 SCC ENO2 TFPI CEACAM5 OPN Ca19-9 CAIX GRP DEFA1 | 11 | 0.89 | 64 | 27 |
| Cyfra SCC ENO2 TFPI CEACAM5 OPN Ca72-4 CA242 CAIX GRP DEFA1 | 11 | 0.89 | 43 | 80 |
| Cyfra MIF TIMP1 SCC ENO2 TFPI CEACAM5 OPN Ca19-9 Ca72-4 CAIX DEFA1 | 12 | 0.89 | 50 | 61 |
| TIMP1 SCC ENO2 TFPI CEACAM5 OPN Ca19-9 Ca72-4 CAIX GRF | 10 | 0.89 | 57 | 45 |
| Cyfra TIMP1 ENO2 TFPI CEACAM5 OPN Ca19-9 Ca72-4 CA242 CAIX GRP DEFA1 | 12 | 0.89 | 48 | 57 |
| Cyfra MIF SIPI TIMP1 CEACAM5 OPN Ca19-9 Ca72-4 GRP DEFA1 | 10 | 0.89 | 61 | 34 |
| Cyfra MIF SLPI TIMP1 CEACAM5 OPN Ca19-9 Ca72-4 CAIX GRP DEFA1 | 11 | 0.89 | 61 | 34 |
| Cyfra SLPI TIMP1 SCC ENO2 CEACAM5 OPN Ca19-9 Ca72-4 CA242 CAIX GRP DEFA1 | 13 | 0.89 | 52 | 45 |
| Cyfra TIMP1 SCC TFPI CEACAM5 OPN Ca72-4 GRP | 8 | 0.89 | 70 | 32 |
| TIMP1 TFPI CEACAMS OPN Ca19-9 Ca72-4 GRP DEFA1 | 8 | 0.89 | 50 | 48 |
| Cyfra MIF TIMP1 SCC TFPI CEACAM5 OPN Ca72-4 CAIX DEFA1 | 10 | 0.89 | 73 | 52 |
| SLPI TIMP1 SCC ENO2 TFPI CEACAM5 OPN Ca19-9 Ca72-4 GRP DEFA1 | 11 | 0.89 | 61 | 59 |
| SLPI TIMP1 ENO2 TFPI CEACAM5 OPN Ca19-9 Ca72-4 CAIX GRP DEFA1 | 11 | 0.89 | 59 | 59 |
| Cyfra SCC ENO2 TFPI CEACAM5 OPN Ca72-4 CAIX DEFA1 | 9 | 0.89 | 30 | 41 |
| MIF TIMP1 SCC TFPI CEACAM5 OPN Ca72-4 CA242 GRP DEFA1 | 10 | 0.89 | 55 | 39 |
| MIF TIMP1 SCC TFPI CEACAM5 OPN Ca72-4 CA242 CAIX GRP DEFA1 | 11 | 0.89 | 55 | 39 |
| Cyfra TIMP1 ENO2 CEACAM5 OPN Ca72-4 CA242 DEFA1 | 8 | 0.89 | 45 | 32 |
| TIMP1 SCC CEACAM5 OPN Ca19-9 Ca72-4 CAIX GRP DEFA1 | 9 | 0.89 | 43 | 52 |
| Cyfra MIF TIMP1 SCC ENO2 CEACAM5 OPN Ca19-9 Ca72-4 GRP DEFA1 | 11 | 0.89 | 43 | 66 |
| Cyfra SCC ENO2 TFPI CEACAM5 OPN Ca19-9 Ca72-4 GRP DEFA1 | 10 | 0.89 | 23 | 77 |
| Cyfra TIMP1 SCC ENO2 TFPI CEACAM5 OPN Ca19-9 GRP DEFA1 | 10 | 0.89 | 64 | 27 |
| TIMP1 SCC ENO2 TFPI CEACAM5 OPN Ca19-9 CAIX GRP DEFA1 | 10 | 0.89 | 61 | 32 |
| Cyfra TIMP1 ENO2 TFPI CEACAM5 OPN Ca19-9 Ca72-4 GRF | 9 | 0.89 | 50 | 43 |
| Cyfra TIMP1 ENO2 TFPI CEACAM5 OPN Ca19-9 Ca72-4 CA242 GRP DEFA1 | 11 | 0.89 | 48 | 57 |
| Cyfra TIMP1 TFPI CEACAM5 OPN Ca72-4 CAIX GRP | 8 | 0.89 | 70 | 32 |
| Cyfra MIF TIMP1 SCC TFPI CEACAM5 OPN Ca72-4 DEFA1 | 9 | 0.89 | 73 | 52 |
| Cyfra TIMP1 SCC ENO2 TFPI CEACAM5 OPN Ca72-4 CAIX GRF | 10 | 0.89 | 64 | 57 |
| SLPI TIMP1 SCC ENO2 TFPI CEACAM5 OPN Ca19-9 Ca72-4 CA242 CAIX GRP DEFA1 | 13 | 0.89 | 70 | 41 |
| Cyfra TIMP1 SCC ENO2 TFPI CEACAM5 OPN Ca72-4 CA242 GRF | 10 | 0.89 | 66 | 48 |
| Cyfra TIMP1 SCC ENO2 CEACAM5 OPN Ca19-9 Ca72-4 CAIX GRF | 10 | 0.89 | 41 | 50 |
| Cyfra TIMP1 SCC ENO2 TFPI CEACAM5 OPN Ca72-4 CA242 CAIX GRF | 11 | 0.89 | 66 | 48 |
| Cyfra MIF TIMP1 SCC CEACAM5 OPN Ca72-4 CA242 GRP DEFA1 | 10 | 0.89 | 66 | 43 |
| Cyfra SLPI TIMP1 SCC ENO2 TFPI CEACAM5 OPN Ca19-9 Ca72-4 CA242 GRP DEFA1 | 13 | 0.89 | 70 | 39 |
| Cyfra SLPI TIMP1 SCC ENO2 TFPI CEACAM5 OPN Ca19-9 Ca72-4 CA242 CAIX GRP DEFA1 | 14 | 0.89 | 70 | 39 |
| Cyfra SCC ENO2 TFPI CEACAM5 OPN Ca72-4 CA242 GRP DEFA1 | 10 | 0.89 | 43 | 80 |
| Cyfra MIF TIMP1 SCC ENO2 TFPI CEACAM5 OPN Ca19-9 Ca72-4 DEFA1 | 11 | 0.89 | 50 | 61 |
| TIMP1 SCC ENO2 TFPI CEACAM5 OPN Ca72-4 CA242 DEFA1 | 9 | 0.89 | 57 | 27 |
| Cyfra MIF TIMP1 SCC ENO2 TFPI CEACAM5 OPN Ca19-9 Ca72-4 CA242 GRP DEFA1 | 13 | 0.89 | 61 | 48 |
| Cyfra MIF TIMP1 SCC ENO2 TFPI CEACAM5 OPN Ca19-9 Ca72-4 CA242 CAIX GRP DEFA1 | 14 | 0.89 | 61 | 48 |
| TIMP1 SCC ENO2 TFPI CEACAM5 OPN Ca19-9 Ca72-4 GRF | 9 | 0.89 | 55 | 45 |
| Cyfra TIMP1 ENO2 TFPI CEACAM5 OPN Ca72-4 CA242 CAIX DEFA1 | 10 | 0.89 | 55 | 23 |
| TIMP1 SCC ENO2 TFPI CEACAM5 OPN Ca19-9 Ca72-4 CA242 GRP DEFA1 | 11 | 0.89 | 48 | 57 |
| SCC ENO2 TFPI CEACAM5 OPN Ca72-4 CAIX GRP DEFA1 | 9 | 0.89 | 32 | 84 |
| Cyfra SLPI TIMP1 SCC ENO2 CEACAM5 OPN Ca19-9 Ca72-4 CA242 GRP DEFA1 | 12 | 0.89 | 52 | 45 |
| TIMP1 SCC ENO2 CEACAM5 OPN Ca72-4 CA242 GRP DEFA1 | 9 | 0.89 | 50 | 36 |
| SLPI TIMP1 ENO2 TFPI CEACAM5 OPN Ca19-9 Ca72-4 GRP DEFA1 | 10 | 0.89 | 57 | 59 |
| TIMP1 SCC ENO2 CEACAM5 OPN Ca72-4 CA242 CAIX GRP DEFA1 | 10 | 0.89 | 50 | 36 |
| Cyfra TIMP1 SCC ENO2 CEACAM5 OPN Ca19-9 Ca72-4 GRF | 9 | 0.89 | 41 | 50 |
| Cyfra MIF TIMP1 SCC TFPI CEACAM5 OPN Ca19-9 Ca72-4 CA242 GRP DEFA1 | 12 | 0.89 | 75 | 68 |
| Cyfra MIF TIMP1 SCC TFPI CEACAM5 OPN Ca19-9 Ca72-4 CA242 CAIX GRP DEFA1 | 13 | 0.89 | 75 | 68 |
| Cyfra SLPI TIMP1 SCC CEACAM5 OPN Ca19-9 Ca72-4 CAIX GRP DEFA1 | 11 | 0.89 | 59 | 43 |
| TIMP1 SCC ENO2 TFPI CEACAM5 OPN Ca19-9 GRP DEFA1 | 9 | 0.89 | 61 | 32 |
| Cyfra TIMP1 SCC ENO2 CEACAM5 OPN Ca19-9 Ca72-4 CAIX DEFA1 | 10 | 0.89 | 36 | 27 |
| Cyfra TIMP1 ENO2 TFPI CEACAM5 OPN Ca72-4 CA242 DEFA1 | 9 | 0.89 | 55 | 23 |
| Cyfra TIMP1 SCC CEACAM5 OPN Ca72-4 GRP DEFA1 | 8 | 0.89 | 57 | 25 |
| Cyfra TIMP1 SCC CEACAM5 OPN Ca72-4 CAIX GRP DEFA1 | 9 | 0.89 | . 57 | 25 |
| TIMP1 ENO2 TFPI CEACAM5 OPN Ca19-9 Ca72-4 CA242 CAIX GRP DEFA1 | 11 | 0.89 | 48 | 57 |
| Cyfra TIMP1 TFPI CEACAM5 OPN Ca72-4 GRP | 7 | 0.89 | 70 | 32 |
| Cyfra ENO2 TFPI CEACAM5 OPN Ca72-4 GRP DEFA1 | 8 | 0.89 | 30 | 84 |
| Cyfra SCC ENO2 TFPI CEACAM5 OPN Ca72-4 DEFA1 | 8 | 0.89 | 27 | 41 |
| TIMP1 SCC TFPI CEACAM5 OPN Ca72-4 CAIX GRP | 8 | 0.89 | 68 | 36 |
| MIF TIMP1 SCC TFPI CEACAM5 OPN Ca72-4 CAIX DEFA1 | 9 | 0.89 | 70 | 55 |
| Cyfra MIF TIMP1 TFPI CEACAM5 OPN Ca72-4 CA242 GRP DEFA1 | 10 | 0.89 | so | 36 |
| Cyfra MIF TIMP1 TFPI CEACAM5 OPN Ca72-4 CA242 CAIX GRP DEFA1 | 11 | 0.89 | 50 | 36 |
| Cyfra TIMP1 ENO2 TFPI CEACAM5 OPN GRP DEFA1 | 8 | 0.89 | 70 | 48 |
| Cyfra TIMP1 SCC ENO2 TFPI CEACAM5 OPN Ca72-4 GRP | 9 | 0.89 | 64 | 57 |
| Cyfra TIMP1 ENO2 TFPI CEACAM5 OPN CAIX GRP DEFA1 | 9 | 0.89 | 70 | 48 |
| MIF TIMP1 SCC ENO2 TFPI CEACAM5 OPN Ca72-4 GRP DEFA1 | 10 | 0.89 | 70 | 70 |
| Cyfra SLPI TIMP1 ENO2 CEACAM5 OPN Ca19-9 Ca72-4 CAIX GRP DEFA1 | 11 | 0.89 | 43 | 64 |
| MIF TIMP1 SCC ENO2 TFPI CEACAM5 OPN Ca72-4 CAIX GRP DEFA1 | 11 | 0.89 | 70 | 70 |
| TIMP1 SCC ENO2 TFPI CEACAM5 OPN CAIX GRP DEFA1 | 9 | 0.89 | 68 | 52 |
| TIMP1 SCC CEACAM5 OPN Ca19-9 Ca72-4 GRP DEFA1 | 8 | 0.89 | 41 | 52 |
| Cyfra SLPI TIMP1 SCC CEACAM5 OPN Ca19-9 Ca72-4 GRP DEFA1 | 10 | 0.89 | 59 | 43 |
| MIF TIMP1 SCC EN02 TFPI CEACAM5 OPN Ca19-9 Ca72-4 CAIX DEFA1 | 11 | 0.89 | 50 | 61 . |
| Cyfra TIMP1 SCC TFPI CEACAM5 OPN Ca72-4 CA242 GRP | 9 | 0.89 | 55 | 75 |
| Cyfra TIMP1 SCC TFPI CEACAM5 OPN Ca72-4 CA242 CAIX GRF | 10 | 0.89 | 55 | 75 |
| Cyfra SLPI TIMP1 SCC TFPI CEACAM5 OPN Ca19-9 Ca72-4 CAIX GRP DEFA1 | 12 | 0.89 | 68 | 32 |
| TIMP1 ENO2 TFPI CEACAM5 OPN Ca19-9 Ca72-4 CAIX GRF | 9 | 0.89 | 50 | 45 |
| SCC ENO2 TFPI CEACAM5 OPN Ca72-4 GRP DEFA1 | 8 | 0.89 | 27 | 84 |
| Cyfra TIMP1 SCC ENO2 CEACAM5 OPN Ca19-9 GRP DEFA1 | 9 | 0.89 | 52 | 36 |
| Cyfra TIMP1 SCC ENO2 CEACAM5 OPN Ca19-9 CAIX GRP DEFA1 | 10 | 0.89 | 52 | 36 |
| MIF TIMP1 SCC TFPI CEACAM5 OPN Ca72-4 DEFA1 | 8 | 0.89 | 70 | 55 |
| Cyfra SLPI TIMP1 ENO2 CEACAM5 OPN Ca19-9 Ca72-4 GRP DEFA1 | 10 | 0.89 | 43 | 64 |
| Cyfra MIF TIMP1 ENO2 TFPI CEACAM5 OPN Ca72-4 CA242 GRP DEFA1 | 11 | 0.89 | 73 | 64 |
| Cyfra MIF TIMP1 SCC ENO2 TFPI CEACAM5 OPN Ca19-9 CAIX GRP DEFA1 | 12 | 0.89 | 70 | 64 |
| Cyfra MIF TIMP1 ENO2 TFPI CEACAM5 OPN Ca72-4 CA242 CAIX GRP DEFA1 | 12 | 0.89 | 73 | 64 |
| Cyfra TIMP1 CEACAM5 OPN Ca19-9 Ca72-4 CAIX GRP DEFA1 | 9 | 0.89 | 41 | 50 |
| Cyfra TIMP1 ENO2 TFPI CEACAM5 OPN Ca19-9 CAIX GRP DEFA1 | 10 | 0.89 | 61 | 27 |
| Cyfra MIF TIMP1 ENO2 TFPI CEACAM5 OPN Ca19-9 Ca72-4 CAIX DEFA1 | 11 | 0.89 | 48 | 61 |
| Cyfra TIMP1 SCC ENO2 CEACAM5 OPN Ca19-9 Ca72-4 DEFA1 | 9 | 0.89 | 34 | 27 |
| MIF TIMP1 SCC ENO2 TFPI CEACAM5 OPN Ca19-9 Ca72-4 DEFA1 | 10 | 0.89 | 48 | 61 |
| TIMP1 ENO2 TFPI CEACAM5 OPN Ca72-4 CA242 CAIX DEFA1 | 9 | 0.89 | 55 | 27 |
| Cyfra SLPI TIMP1 SCC TFPI CEACAM5 OPN Ca19-9 Ca72-4 GRP DEFA1 | 11 | 0.89 | 68 | 32 |
| Cyfra MIF SLPI TIMP1 SCC TFPI CEACAM5 OPN Ca19-9 Ca72-4 CAIX GRP DEFA1 | 13 | 0.89 | 73 | 20 |
| Cyfra SLPI TIMP1 EN02 CEACAM5 OPN Ca19-9 Ca72-4 CA242 CAIX GRP DEFA1 | 12 | 0.89 | 50 | 45 |
| TIMP1 SCC TFPI CEACAM5 OPN Ca72-4 GRP | 7 | 0.89 | 68 | 36 |
| Cyfra TIMP1 ENO2 CEACAM5 OPN Ca72-4 GRP DEFA1 | 8 | 0.89 | 43 | 50 |
| Cyfra TIMP1 ENO2 CEACAM5 OPN Ca72-4 CAIX GRP DEFA1 | 9 | 0.89 | 43 | 50 |
| TIMP1 SCC ENO2 TFPI CEACAM5 OPN GRP DEFA1 | 8 | 0.89 | 68 | 52 |
| Cyfra TIMP1 SCC ENO2 CEACAM5 OPN Ca19-9 CAIX DEFA1 | 9 | 0.89 | 50 | 36 |
| Cyfra TIMP1 SCC ENO2 CEACAM5 OPN Ca72-4 CAIX DEFA1 | 9 | 0.89 | 43 | 50 |
| Cyfra TIMP1 ENO2 TFPI CEACAM5 OPN Ca72-4 CA242 GRP | 9 | 0.89 | 66 | 48 |
| Cyfra TIMP1 ENO2 TFPI CEACAM5 OPN Ca72-4 CA242 CAIX GRF | 10 | 0.89 | 66 | 48 |
| Cyfra MIF TIMP1 SCC ENO2 TFPI CEACAM5 OPN Ca19-9 GRP DEFA1 | 11 | 0.89 | 70 | 64 |
| SLPI TIMP1 SCC ENO2 TFPI CEACAM5 OPN Ca19-9 Ca72-4 CA242 GRP DEFA1 | 12 | 0.89 | 68 | 41 |
| Cyfra TIMP1 CEACAM5 OPN Ca19-9 Ca72-4 GRP DEFA1 | 8 | 0.89 | 41 | 50 |
| Cyfra TIMP1 SCC ENO2 TFPI CEACAM5 OPN CAIX DEFA1 | 9 | 0.89 | 70 | 48 |
| Cyfra TIMP1 SCC TFPI CEACAM5 OPN Ca19-9 Ca72-4 CAIX GRF | 10 | 0.89 | 66 | 73 |
| SCC ENO2 TFPI CEACAM5 OPN Ca19-9 Ca72-4 CAIX GRP DEFA1 | 10 | 0.89 | 23 | 77 |
| Cyfra TIMP1 SCC ENO2 TFPI CEACAM5 OPN Ca72-4 CAIX | 9 | 0.89 | 61 | 59 |
| Cyfra TIMP1 SCC CEACAM5 OPN Ca19-9 Ca72-4 CAIX GRF | 9 | 0.89 | 50 | 75 |
| Cyfra TIMP1 ENO2 TFPI CEACAM5 OPN Ca19-9 GRP DEFA1 | 9 | 0.89 | 61 | 27 |
| TIMP1 ENO2 TFPI CEACAM5 OPN Ca19-9 CAIX GRP DEFA1 | 9 | 0.89 | 59 | 32 |
| Cyfra TIMP1 SCC ENO2 TFPI CEACAM5 OPN Ca19-9 Ca72-4 CAIX DEFA1 | 11 | 0.89 | 43 | 18 |
| Cyfra TIMP1 SCC ENO2 CEACAM5 OPN Ca19-9 Ca72-4 CA242 CAIX DEFA1 | 11 | 0.89 | 43 | 64 |
| Cyfra TIMP1 TFPI CEACAM5 OPN Ca72-4 CA242 GRP | 8 | 0.89 | 52 | 75 |
| Cyfra TIMP1 TFPI CEACAM5 OPN Ca72-4 CA242 CAIX GRP | 9 | 0.89 | 52 | 75 |
| TIMP1 ENO2 TFPI CEACAM5 OPN Ca19-9 Ca72-4 GRP | 8 | 0.89 | 48 | 45 |
| SLPI TIMP1 SCC TFPI CEACAM5 OPN Ca19-9 Ca72-4 CAIX GRP DEFA1 | 11 | 0.89 | 66 | 32 |
| Cyfra MIF TIMP1 TFPI CEACAM5 OPN Ca72-4 CAIX DEFA1 | 9 | 0.89 | 70 | 52 |
| TIMP1 ENO2 TFPI CEACAM5 OPN Ca19-9 Ca72-4 CA242 GRP DEFA1 | 10 | 0.89 | 45 | 57 |
| TIMP1 TFPI CEACAM5 OPN Ca72-4 CAIX GRP | 7 | 0.89 | 68 | 36 |
| Cyfra TIMP1 SCC ENO2 CEACAM5 OPN Ca19-9 DEFA1 | 8 | 0.89 | 50 | 36 |
| MIF TIMP1 TFPI CEACAM5 OPN Ca72-4 CA242 GRP DEFA1 | 9 | 0.89 | 50 | 39 |
| MIF TIMP1 TFPI CEACAM5 OPN Ca72-4 CA242 CAIX GRP DEFA1 | 10 | 0.89 | 50 | 39 |
| Cyfra TIMP1 SCC ENO2 CEACAM5 OPN Ca72-4 DEFA1 | 8 | 0.89 | 43 | 50 |
| Cyfra MIF TIMP1 SCC ENO2 TFPI CEACAM5 OPN Ca72-4 CA242 DEFA1 | 11 | 0.89 | 73 | 66 |
| Cyfra MIF TIMP1 SCC ENO2 TFPI CEACAM5 OPN Ca72-4 CA242 CAIX DEFA1 | 12 | 0.89 | 73 | 66 |
| Cyfra TIMP1 SCC ENO2 TFPI CEACAM5 OPN DEFA1 | 8 | 0.89 | 70 | 48 |
| Cyfra MIF TIMP1 SCC CEACAM5 OPN Ca72-4 GRP DEFA1 | 9 | 0.89 | 64 | 59 |
| Cyfra MIF TIMP1 SCC CEACAM5 OPN Ca72-4 CAIX GRP DEFA1 | 10 | 0.89 | 64 | 59 |
| Cyfra MIF TIMP1 CEACAM5 OPN Ca72-4 CA242 CAIX GRP DEFA1 | 10 | 0.89 | 64 | 43 |
| TIMP1 CEACAM5 OPN Ca19-9 Ca72-4 CAIX GRP DEFA1 | 8 | 0.89 | 41 | 52 |
| Cyfra ENO2 TFPI CEACAM5 OPN Ca19-9 Ca72-4 CAIX GRP DEFA1 | 10 | 0.89 | 23 | 77 |
| Cyfra TIMP1 SCC CEACAM5 OPN Ca19-9 Ca72-4 GRF | 8 | 0.89 | 50 | 75 |
| Cyfra MIF TIMP1 ENO2 TFPI CEACAM5 OPN Ca19-9 Ca72-4 DEFA1 | 10 | 0.89 | 48 | 61 |
| Cyfra SLPI TIMP1 CEACAM5 OPN Ca19-9 Ca72-4 CAIX GRP DEFA1 | 10 | 0.89 | 55 | 43 |
| SCC ENO2 TFPI CEACAM5 OPN Ca72-4 CA242 CAIX GRP DEFA1 | 10 | 0.89 | 43 | 80 |
| Cyfra TIMP1 CEACAM5 OPN Ca72-4 GRP DEFA1 | 7 | 0.89 | 55 | 25 |
| Cyfra TIMP1 CEACAM5 OPN Ca72-4 CAIX GRP DEFA1 | 8 | 0.89 | 55 | 25 |
| Cyfra MIF SLPI TIMP1 SCC TFPI CEACAM5 OPN Ca19-9 Ca72-4 GRP DEFA1 | 12 | 0.89 | 73 | 20 |
| Cyfra SLPI TIMP1 SCC ENO2 TFPI CEACAM5 OPN Ca19-9 Ca72-4 CAIX DEFA1 | 12 | 0.89 | 57 | 61 |
| Cyfra MIF TIMP1 TFPI CEACAM5 OPN Ca72-4 DEFA1 | 8 | 0.89 | 70 | 52 |
| Cyfra SLPI TIMP1 ENO2 CEACAM5 OPN Ca19-9 Ca72-4 CA242 GRP DEFA1 | 11 | 0.89 | 50 | 45 |
| TIMP1 ENO2 CEACAM5 OPN Ca72-4 CA242 GRP DEFA1 | 8 | 0.89 | 50 | 36 |
| TIMP1 ENO2 CEACAM5 OPN Ca72-4 CA242 CAIX GRP DEFA1 | 9 | 0.89 | 50 | 36 |
| MIF SLPI TIMP1 SCC CEACAM5 OPN Ca19-9 Ca72-4 CAIX GRP DEFA1 | 11 | 0.89 | 66 | 34 |
| Cyfra SLPI SCC ENO2 TFPI CEACAM5 OPN Ca19-9 Ca72-4 CAIX GRP DEFA1 | 12 | 0.89 | 41 | 59 |
| MIF TIMP1 SCC ENO2 TFPI CEACAM5 OPN Ca72-4 CAIX DEFA1 | 10 | 0.89 | 68 | 73 |
| Cyfra MIF TIMP1 SCC TFPI CEACAM5 OPN Ca19-9 Ca72-4 CAIX GRF | 11 | 0.89 | 73 | 57 |
| TIMP1 ENO2 TFPI CEACAM5 OPN CAIX GRP DEFA1 | 8 | 0.89 | 68 | 52 |
| Cyfra TIMP1 ENO2 TFPI CEACAM5 OPN Ca72-4 CAIX GRP | 9 | 0.89 | 61 | 57 |
| MIF TIMP1 SCC ENO2 TFPI CEACAM5 OPN Ca72-4 CA242 GRP DEFA1 | 11 | 0.89 | 75 | 64 |
| MIF TIMP1 SCC ENO2 TFPI CEACAM5 OPN Ca72-4 CA242 CAIX GRP DEFA1 | 12 | 0.89 | 75 | 64 |
| MIF TIMP1 SCC TFPI CEACAM5 OPN Ca19-9 Ca72-4 CA242 CAIX GRP DEFA1 | 12 | 0.89 | 73 | 68 |
| Cyfra MIF SLPI TIMP1 SCC CEACAM5 OPN Ca19-9 Ca72-4 CA242 CAIX GRP DEFA1 | 13 | 0.89 | 73 | 52 |
| Cyfra TIMP1 SCC TFPI CEACAM5 OPN Ca19-9 Ca72-4 GRF | 9 | 0.89 | 66 | 73 |
| Cyfra SCC ENO2 CEACAM5 OPN Ca72-4 CA242 CAIX GRP DEFA1 | 10 | 0.89 | 36 | 84 |
| SCC ENO2 TFPI CEACAM5 OPN Ca19-9 Ca72-4 GRP DEFA1 | 9 | 0.89 | 20 | 77 |
| Cyfra TIMP1 SCC ENO2 TFPI CEACAM5 OPN Ca72-4 | 8 | 0.89 | 61 | 59 |
| TIMP1 ENO2 TFPI CEACAM5 OPN Ca19-9 GRP DEFA1 | 8 | 0.89 | 59 | 32 |
| Cyfra SLPI TIMP1 CEACAM5 OPN Ca19-9 Ca72-4 GRP DEFA1 | 9 | 0.89 | 55 | 43 |
| Cyfra TIMP1 SCC ENO2 TFPI CEACAM5 OPN Ca19-9 Ca72-4 DEFA1 | 10 | 0.89 | 41 | 18 |
| TIMP1 ENO2 TFPI CEACAM5 OPN Ca72-4 CA242 DEFA1 | 8 | 0.89 | 52 | 27 |
| SLPI TIMP1 SCC CEACAM5 OPN Ca19-9 Ca72-4 CAIX GRP DEFA1 | 10 | 0.89 | 59 | 43 |
| TIMP1 SCC ENO2 TFPI CEACAM5 OPN Ca19-9 Ca72-4 CAIX DEFA1 | 10 | 0.89 | 41 | 23 |
| Cyfra SLPI TIMP1 TFPI CEACAM5 OPN Ca19-9 Ca72-4 CAIX GRP DEFA1 | 11 | 0.89 | 66 | 32 |
| Cyfra TIMP1 SCC ENO2 TFPI CEACAM5 OPN Ca19-9 CAIX DEFA1 | 10 | 0.89 | 59 | 27 |
| TIMP1 TFPI CEACAM5 OPN Ca72-4 GRP | 6 | 0.89 | 68 | 36 |
| SLPI TIMP1 SCC ENO2 CEACAM5 OPN Ca19-9 Ca72-4 CAIX GRP DEFA1 | 11 | 0.89 | 45 | 66 |
| Cyfra SLPI TIMP1 SCC ENO2 CEACAM5 OPN Ca19-9 Ca72-4 CAIX DEFA1 | 11 | 0.89 | 43 | 68 |
| Cyfra EN02 TFPI CEACAM5 OPN Ca72-4 CAIX DEFA1 | 8 | 0.89 | 30 | 41 |
| MIF TIMP1 SCC ENO2 TFPI CEACAM5 OPN Ca72-4 DEFA1 | 9 | 0.89 | 68 | 73 |
| Cyfra MIF TIMP1 SCC TFPI CEACAM5 OPN Ca19-9 Ca72-4 GRF | 10 | 0.89 | 73 | 57 |
| Cyfra TIMP1 SCC CEACAM5 OPN Ca72-4 CA242 CAIX GRP DEFA1 | 10 | 0.89 | 59 | 57 |
| Cyfra MIF TIMP1 CEACAM5 OPN Ca72-4 CA242 GRP DEFA1 | 9 | 0.89 | 64 | 43 |
| Cyfra SLPI TIMP1 SCC ENO2 CEACAM5 OPN Ca19-9 CAIX GRP DEFA1 | 11 | 0.89 | 64 | 68 |
| MIF TIMP1 SCC ENO2 TFPI CEACAM5 OPN Ca19-9 CAIX GRP DEFA1 | 11 | 0.89 | 68 | 64 |
| MIF TIMP1 SCC TFPI CEACAM5 OPN Ca19-9 Ca72-4 CA242 GRP DEFA1 | 11 | 0.89 | 73 | 68 |
| Cyfra MIF SLPI TIMP1 SCC CEACAM5 OPN Ca19-9 Ca72-4 CA242 GRP DEFA1 | 12 | 0.89 | 73 | 52 |
| TIMP1 SCC TFPI CEACAM5 OPN Ca19-9 Ca72-4 CAIX GRF | 9 | 0.89 | 64 | 73 |
| MIF TIMP1 ENO2 TFPI CEACAM5 OPN Ca19-9 Ca72-4 CAIX DEFA1 | 10 | 0.89 | 48 | 61 |
| Cyfra SLPI TIMP1 TFPI CEACAM5 OPN Ca19-9 Ca72-4 GRP DEFA1 | 10 | 0.89 | 66 | 32 |
| Cyfra TIMP1 SCC ENO2 TFPI CEACAM5 OPN Ca19-9 DEFA1 | 9 | 0.89 | 59 | 27 |
| TIMP1 SCC ENO2 CEACAM5 OPN Ca19-9 Ca72-4 CAIX DEFA1 | 9 | 0.89 | 30 | 32 |
| TIMP1 SCC ENO2 CEACAM5 OPN Ca19-9 CAIX GRP DEFA1 | 9 | 0.89 | 50 | 41 |
| SLPI TIMP1 SCC TFPI CEACAM5 OPN Ca19-9 Ca72-4 GRP DEFA1 | 10 | 0.89 | 64 | 32 |
| Cyfra TIMP1 SCC TFPI CEACAM5 OPN Ca72-4 CAIX DEFA1 | 9 | 0.89 | 64 | 14 |
| Cyfra SLPI TIMP1 SCC ENO2 TFPI CEACAM5 OPN Ca19-9 Ca72-4 DEFA1 | 11 | 0.89 | 55 | 61 |
| Cyfra TIMP1 SCC ENO2 TFPI CEACAM5 OPN CA242 CAIX GRP DEFA1 | 11 | 0.89 | 57 | 30 |
| MIF TIMP1 SCC ENO2 TFPI CEACAM5 OPN Ca19-9 Ca72-4 CA242 CAIX GRP DEFA1 | 13 | 0.89 | 61 | 48 |
| MIF TIMP1 TFPI CEACAM5 OPN Ca72-4 CAIX DEFA1 | 8 | 0.89 | 68 | 55 |
| MIF SLPI TIMP1 SCC CEACAM5 OPN Ca19-9 Ca72-4 GRP DEFA1 | 10 | 0.89 | 66 | 34 |
| Cyfra MIF TIMP1 SCC TFPI CEACAM5 OPN Ca19-9 Ca72-4 CAIX DEFA1 | 11 | 0.89 | 61 | 32 |
| TIMP1 SCC CEACAM5 OPN Ca72-4 CAIX GRP DEFA1 | 8 | 0.89 | 57 | 27 |
| MIF TIMP1 ENO2 TFPI CEACAM5 OPN Ca72-4 GRP DEFA1 | 9 | 0.89 | 66 | 70 |
| Cyfra SLPI TIMP1 SCC ENO2 CEACAM5 OPN Ca19-9 Ca72-4 DEFA1 | 10 | 0.89 | 41 | 68 |
| MIF TIMP1 ENO2 TFPI CEACAM5 OPN Ca72-4 CAIX GRP DEFA1 | 10 | 0.89 | 66 | 70 |
| Cyfra SLPI TIMP1 SCC ENO2 TFPI CEACAM5 OPN Ca72-4 CA242 CAIX GRP DEFA1 | 13 | 0.89 | 75 | 59 |
| TIMP1 SCC ENO2 CEACAM5 OPN Ca72-4 CAIX GRP DEFA1 | 9 | 0.89 | 43 | 55 |
| TIMP1 ENO2 TFPI CEACAM5 OPN GRP DEFA1 | 7 | 0.89 | 68 | 52 |
| Cyfra TIMP1 ENO2 TFPI CEACAM5 OPN Ca72-4 GRP | 8 | 0.89 | 61 | 57 |
| Cyfra SLPI TIMP1 SCC ENO2 CEACAM5 OPN Ca72-4 CA242 CAIX GRP DEFA1 | 12 | 0.89 | 68 | 66 |
| SLPI TIMP1 ENO2 TFPI CEACAM5 OPN Ca19-9 Ca72-4 CA242 CAIX GRP DEFA1 | 12 | 0.89 | 66 | 41 |
| Cyfra TIMP1 ENO2 TFPI CEACAM5 OPN CAIX DEFA1 | 8 | 0.89 | 70 | 48 |
| TIMP1 CEACAM5 OPN Ca19-9 Ca72-4 GRP DEFA1 | 7 | 0.89 | 39 | 52 |
| Cyfra SCC ENO2 CEACAM5 OPN Ca72-4 CA242 GRP DEFA1 | 9 | 0.89 | 36 | 84 |
| Cyfra SLPI TIMP1 SCC ENO2 CEACAM5 OPN Ca19-9 GRP DEFA1 | 10 | 0.89 | 64 | 68 |
| MIF TIMP1 SCC ENO2 TFPI CEACAM5 OPN Ca19-9 GRP DEFA1 | 10 | 0.89 | 68 | 64 |
| Cyfra SLPI TIMP1 ENO2 TFPI CEACAM5 OPN Ca19-9 Ca72-4 CA242 GRP DEFA1 | 12 | 0.89 | 66 | 39 |
| Cyfra SLPI TIMP1 ENO2 TFPI CEACAM5 OPN Ca19-9 Ca72-4 CA242 CAIX GRP DEFA1 | 13 | 0.89 | 66 | 39 |
| Cyfra TIMP1 SCC ENO2 TFPI CEACAM5 OPN Ca72-4 CA242 CAIX | 10 | 0.89 | 64 | 50 |
| Cyfra ENO2 TFPI CEACAM5 OPN Ca19-9 Ca72-4 GRP DEFA1 | 9 | 0.89 | 20 | 77 |
| Cyfra SLPI SCC ENO2 CEACAM5 OPN Ca19-9 Ca72-4 CAIX GRP DEFA1 | 11 | 0.89 | 25 | 66 |
| MIF TIMP1 ENO2 TFPI CEACAM5 OPN Ca19-9 Ca72-4 DEFA1 | 9 | 0.89 | 45 | 61 |
| Cyfra TIMP1 ENO2 TFPI CEACAM5 OPN Ca19-9 Ca72-4 CAIX DEFA1 | 10 | 0.89 | 41 | 18 |
| SLPI TIMP1 SCC CEACAM5 OPN Ca19-9 Ca72-4 GRP DEFA1 | 9 | 0.89 | 57 | 43 |
| TIMP1 SCC ENO2 TFPI CEACAM5 OPN Ca19-9 Ca72-4 DEFA1 | 9 | 0.89 | 41 | 23 |
| Cyfra TIMP1 SCC ENO2 CEACAM5 OPN Ca19-9 Ca72-4 CA242 DEFA1 | 10 | 0.89 | 41 | 64 |
| Cyfra ENO2 TFPI CEACAM5 OPN Ca72-4 CA242 CAIX GRP DEFA1 | 10 | 0.89 | 39 | 80 |
| TIMP1 SCC ENO2 CEACAM5 OPN Ca19-9 GRP DEFA1 | 8 | 0.89 | 50 | 41 |
| TIMP1 SCC TFPI CEACAM5 OPN Ca72-4 CA242 CAIX GRP | 9 | 0.89 | 55 | 75 |
| SLPI TIMP1 TFPI CEACAM5 OPN Ca19-9 Ca72-4 CAIX GRP DEFA1 | 10 | 0.89 | 64 | 32 |
| Cyfra MIF SLPI TIMP1 TFPI CEACAM5 OPN Ca19-9 Ca72-4 CAIX GRP DEFA1 | 12 | 0.89 | 70 | 20 |
| MIF TIMP1 SCC ENO2 TFPI CEACAM5 OPN Ca19-9 Ca72-4 CA242 GRP DEFA1 | 12 | 0.89 | 61 | 48 |
| TIMP1 SCC TFPI CEACAM5 OPN Ca72-4 CAIX DEFA1 | 8 | 0.89 | 61 | 16 |
| Cyfra TIMP1 SCC ENO2 TFPI CEACAM5 OPN Ca19-9 Ca72-4 CAIX | 10 | 0.89 | 52 | 45 |
| MIF SLPI TIMP1 SCC TFPI CEACAM5 OPN Ca19-9 Ca72-4 GRP DEFA1 | 11 | 0.89 | 70 | 20 |
| MIF SLPI TIMP1 SCC TFPI CEACAM5 OPN Ca19-9 Ca72-4 CAIX GRP DEFA1 | 12 | 0.89 | 70 | 20 |
| Cyfra MIF SLPI TIMP1 SCC ENO2 TFPI CEACAM5 OPN Ca19-9 Ca72-4 CAIX GRP DEFA1 | 14 | 0.89 | 70 | 45 |
| MIF TIMP1 TFPI CEACAM5 OPN Ca72-4 DEFA1 | 7 | 0.89 | 68 | 55 |
| Cyfra SLPI SCC ENO2 TFPI CEACAM5 OPN Ca19-9 Ca72-4 GRP DEFA1 | 11 | 0.89 | 39 | 59 |
| SLPI TIMP1 SCC ENO2 TFPI CEACAM5 OPN Ca19-9 Ca72-4 CAIX DEFA1 | 11 | 0.89 | 55 | 64 |
| Cyfra SLPI TIMP1 SCC ENO2 TFPI CEACAM5 OPN Ca72-4 CA242 GRP DEFA1 | 12 | 0.89 | 75 | 59 |
| Cyfra SLPI TIMP1 ENO2 TFPI CEACAM5 OPN Ca72-4 CA242 CAIX GRP DEFA1 | 12 | 0.89 | 75 | 59 |
| MIF TIMP1 SCC TFPI CEACAM5 OPN Ca19-9 Ca72-4 CAIX GRF | 10 | 0.89 | 70 | 57 |
| SLPI TIMP1 SCC ENO2 CEACAM5 OPN Ca19-9 Ca72-4 GRP DEFA1 | 10 | 0.89 | 43 | 66 |
| SCC ENO2 TFPI CEACAM5 OPN Ca72-4 CAIX DEFA1 | 8 | 0.89 | 25 | 45 |
| Cyfra TIMP1 SCC CEACAM5 OPN Ca72-4 CA242 GRP DEFA1 | 9 | 0.89 | 59 | 57 |
| Cyfra TIMP1 ENO2 CEACAM5 OPN Ca19-9 Ca72-4 CAIX GRF | 9 | 0.89 | 34 | 50 |
| Cyfra TIMP1 ENO2 TFPI CEACAM5 OPN DEFA1 | 7 | 0.89 | 70 | 48 |
| Cyfra SLPI TIMP1 SCC ENO2 CEACAM5 OPN Ca72-4 CA242 GRP DEFA1 | 11 | 0.89 | 68 | 66 |
| Cyfra TIMP1 SCC ENO2 TFPI CEACAM5 OPN Ca72-4 CA242 | 9 | 0.89 | 64 | 50 |
| Cyfra TIMP1 SCC ENO2 CEACAM5 OPN Ca19-9 Ca72-4 CAIX | 9 | 0.89 | 39 | 52 |
| Cyfra SLPI SCC ENO2 CEACAM5 OPN Ca19-9 Ca72-4 GRP DEFA1 | 10 | 0.89 | 23 | 66 |
| Cyfra TIMP1 ENO2 CEACAM5 OPN Ca19-9 Ca72-4 CAIX DEFA1 | 9 | 0.89 | 32 | 27 |
| TIMP1 SCC ENO2 CEACAM5 OPN Ca19-9 Ca72-4 DEFA1 | 8 | 0.89 | 30 | 32 |
| SCC ENO2 TFPI CEACAM5 OPN Ca72-4 CA242 GRP DEFA1 | 9 | 0.89 | 39 | 80 |
| Cyfra MIF TIMP1 ENO2 TFPI CEACAM5 OPN Ca19-9 Ca72-4 CA242 GRP DEFA1 | 12 | 0.89 | 59 | 48 |
| Cyfra MIF TIMP1 ENO2 TFPI CEACAM5 OPN Ca19-9 Ca72-4 CA242 CAIX GRP DEFA1 | 13 | 0.89 | 59 | 48 |
| Cyfra TIMP1 SCC TFPI CEACAM5 OPN Ca72-4 DEFA1 | 8 | 0.89 | 64 | 14 |
| Cyfra TIMP1 SCC ENO2 TFPI CEACAM5 OPN CA242 GRP DEFA1 | 10 | 0.89 | 57 | 30 |
| Cyfra SLPI TIMP1 ENO2 TFPI CEACAM5 OPN Ca19-9 Ca72-4 CAIX DEFA1 | 11 | 0.89 | 55 | 61 |
| Cyfra MIF TIMP1 SCC TFPI CEACAM5 OPN Ca19-9 Ca72-4 DEFA1 | 10 | 0.89 | 61 | 32 |
| MIF SLPI TIMP1 CEACAM5 OPN Ca19-9 Ca72-4 CAIX GRP DEFA1 | 10 | 0.89 | 61 | 34 |
| TIMP1 SCC CEACAM5 OPN Ca72-4 GRP DEFA1 | 7 | 0.89 | 57 | 27 |
| Cyfra SLPI TIMP1 ENO2 TFPI CEACAM5 OPN Ca72-4 CA242 GRP DEFA1 | 11 | 0.89 | 75 | 59 |
| MIF TIMP1 SCC TFPI CEACAM5 OPN Ca19-9 Ca72-4 GRF | 9 | 0.89 | 70 | 57 |
| MIF TIMP1 ENO2 TFPI CEACAM5 OPN Ca72-4 CAIX DEFA1 | 9 | 0.89 | 61 | 73 |
| Cyfra TIMP1 ENO2 CEACAM5 OPN Ca19-9 Ca72-4 GRP | 8 | 0.89 | 34 | 50 |
| TIMP1 SCC ENO2 CEACAM5 OPN Ca72-4 GRP DEFA1 | 8 | 0.89 | 43 | 55 |
| Cyfra MIF SCC ENO2 TFPI CEACAM5 OPN Ca19-9 Ca72-4 CAIX GRP DEFA1 | 12 | 0.89 | 34 | 61 |
| Cyfra ENO2 TFPI CEACAM5 OPN Ca72-4 DEFA1 | 7 | 0.89 | 27 | 41 |
| Cyfra MIF TIMP1 TFPI CEACAMS OPN Ca19-9 Ca72-4 CA242 GRP DEFA1 | 11 | 0.89 | 70 | 68 |
| Cyfra MIF TIMP1 TFPI CEACAM5 OPN Ca19-9 Ca72-4 CA242 CAIX GRP DEFA1 | 12 | 0.89 | 70 | 68 |
| Cyfra MIF TIMP1 ENO2 TFPI CEACAM5 OPN Ca19-9 CAIX GRP DEFA1 | 11 | 0.89 | 68 | 64 |
| MIF TIMP1 SCC ENO2 CEACAM5 OPN Ca19-9 Ca72-4 CAIX GRP DEFA1 | 11 | 0.89 | 41 | 66 |
| Cyfra MIF SLPI TIMP1 SCC ENO2 TFPI CEACAM5 OPN Ca19-9 Ca72-4 CA242 CAIX GRP DEFA1 | 15 | 0.89 | 75 | 34 |
| TIMP1 SCC ENO2 TFPI CEACAM5 OPN CAIX DEFA1 | 8 | 0.89 | 68 | 52 |
| Cyfra SLPI TIMP1 SCC ENO2 CEACAM5 OPN Ca72-4 CA242 CAIX DEFA1 | 11 | 0.89 | 66 | 70 |
| Cyfra TIMP1 TFPI CEACAM5 OPN Ca19-9 Ca72-4 CAIX GRF | 9 | 0.89 | 64 | 73 |
| Cyfra TIMP1 ENO2 TFPI CEACAM5 OPN Ca72-4 CAIX | 8 | 0.88 | 57 | 59 |
| TIMP1 SCC CEACAM5 OPN Ca19-9 Ca72-4 CAIX GRP | 8 | 0.88 | 50 | 75 |
| Cyfra TIMP1 ENO2 TFPI CEACAM5 OPN Ca19-9 Ca72-4 DEFA1 | 9 | 0.88 | 39 | 18 |
| Cyfra ENO2 TFPI CEACAM5 OPN Ca72-4 CA242 GRP DEFA1 | 9 | 0.88 | 39 | 80 |
| SLPI TIMP1 CEACAM5 OPN Ca19-9 Ca72-4 CAIX GRP DEFA1 | 9 | 0.88 | 55 | 43 |
| TIMP1 ENO2 TFPI CEACAM5 OPN Ca19-9 Ca72-4 CAIX DEFA1 | 9 | 0.88 | 39 | 23 |
| TIMP1 TFPI CEACAM5 OPN Ca72-4 CA242 CAIX GRP | 8 | 0.88 | 52 | 75 |
| TIMP1 SCC ENO2 TFPI CEACAM5 OPN Ca19-9 CAIX DEFA1 | 9 | 0.88 | 57 | 32 |
| Cyfra TIMP1 TFPI CEACAM5 OPN Ca72-4 CAIX DEFA1 | 8 | 0.88 | 61 | 14 |
| Cyfra MIF TIMP1 SCC ENO2 CEACAM5 OPN Ca19-9 Ca72-4 DEFA1 | 10 | 0.88 | 39 | 68 |
| Cyfra SCC ENO2 CEACAM5 OPN Ca19-9 Ca72-4 CAIX GRP DEFA1 | 10 | 0.88 | 23 | 82 |
| Cyfra MIF SLPI TIMP1 TFPI CEACAM5 OPN Ca19-9 Ca72-4 GRP DEFA1 | 11 | 0.88 | 70 | 20 |
| Cyfra MIF TIMP1 SCC ENO2 CEACAM5 OPN Ca19-9 Ca72-4 CAIX DEFA1 | 11 | 0.88 | 39 | 68 |
| TIMP1 SCC TFPI CEACAM5 OPN Ca72-4 DEFA1 | 7 | 0.88 | 59 | 16 |
| Cyfra TIMP1 SCC ENO2 TFPI CEACAM5 OPN Ca19-9 Ca72-4 | 9 | 0.88 | 50 | 45 |
| Cyfra TIMP1 ENO2 TFPI CEACAM5 OPN CA242 CAIX GRP DEFA1 | 10 | 0.88 | 55 | 30 |
| Cyfra MIF SLPI TIMP1 SCC ENO2 TFPI CEACAM5 OPN Ca19-9 Ca72-4 GRP DEFA1 | 13 | 0.88 | 70 | 45 |
| TIMP1 SCC ENO2 TFPI CEACAM5 OPN CA242 GRP DEFA1 | 9 | 0.88 | 57 | 34 |
| TIMP1 SCC EN02 TFPI CEACAM5 OPN CA242 CAIX GRP DEFA1 | 10 | 0.88 | 57 | 34 |
| SLPI SCC ENO2 TFPI CEACAM5 OPN Ca19-9 Ca72-4 CAIX GRP DEFA1 | 11 | 0.88 | 39 | 61 |
| MIF TIMP1 EN02 TFPI CEACAM5 OPN Ca72-4 DEFA1 | 8 | 0.88 | 61 | 73 |
| TIMP1 SCC EN02 TFPI CEACAM5 OPN Ca72-4 CAIX GRP | 9 | 0.88 | 64 | 59 |
| Cyfra MIF TIMP1 ENO2 TFPI CEACAM5 OPN Ca19-9 GRP DEFA1 | 10 | 0.88 | 68 | 64 |
| TIMP1 SCC ENO2 TFPI CEACAM5 OPN Ca72-4 CA242 CAIX GRF | 10 | 0.88 | 66 | 50 |
| Cyfra MIF SLPI TIMP1 SCC ENO2 TFPI CEACAM5 OPN Ca19-9 Ca72-4 CA242 GRP DEFA1 | 14 | 0.88 | 75 | 34 |
| Cyfra TIMP1 ENO2 CEACAM5 OPN Ca72-4 CAIX DEFA1 | 8 | 0.88 | 39 | 50 |
| Cyfra MIF TIMP1 CEACAM5 OPN Ca72-4 GRP DEFA1 | 8 | 0.88 | 61 | 59 |
| Cyfra TIMP1 SCC ENO2 CEACAM5 OPN Ca19-9 Ca72-4 | 8 | 0.88 | 36 | 52 |
| Cyfra MIF TIMP1 CEACAM5 OPN Ca72-4 CAIX GRP DEFA1 | 9 | 0.88 | 61 | 59 |
| MIF TIMP1 SCC ENO2 CEACAM5 OPN Ca19-9 Ca72-4 GRP DEFA1 | 10 | 0.88 | 41 | 66 |
| TIMP1 SCC TFPI CEACAM5 OPN Ca19-9 Ca72-4 GRF | 8 | 0.88 | 61 | 73 |
| Cyfra TIMP1 ENO2 CEACAM5 OPN Ca19-9 Ca72-4 DEFA1 | 8 | 0.88 | 30 | 27 |
| SLPI TIMP1 TFPI CEACAM5 OPN Ca19-9 Ca72-4 GRP DEFA1 | 9 | 0.88 | 61 | 32 |
| TIMP1 TFPI CEACAM5 OPN Ca72-4 CAIX DEFA1 | 7 | 0.88 | 59 | 16 |
| Cyfra MIF TIMP1 SCC ENO2 TFPI CEACAMS OPN GRP DEFA1 | 10 | 0.88 | 66 | 32 |
| Cyfra SLPI TIMP1 ENO2 TFPI CEACAM5 OPN Ca19-9 Ca72-4 DEFA1 | 10 | 0.88 | 52 | 61 |
| Cyfra MIF TIMP1 SCC ENO2 TFPI CEACAMS OPN CAIX GRP DEFA1 | 11 | 0.88 | 66 | 32 |
| Cyfra MIF SLPI TIMP1 ENO2 TFPI CEACAMS OPN Ca19-9 Ca72-4 CAIX GRP DEFA1 | 13 | 0.88 | 66 | 45 |
| TIMP1 CEACAMS OPN Ca72-4 CAIX GRP DEFA1 | 7 | 0.88 | 55 | 27 |
| ENO2 TFPI CEACAM5 OPN Ca72-4 CAIX GRP DEFA1 | 8 | 0.88 | 30 | 84 |
| MIF SLPI TIMP1 CEACAM5 OPN Ca19-9 Ca72-4 GRP DEFA1 | 9 | 0.88 | 61 | .34 |
| MIF TIMP1 SCC TFPI CEACAM5 OPN Ca19-9 Ca72-4 CAIX DEFA1 | 10 | 0.88 | 59 | 34 |
| SLPI TIMP1 ENO2 CEACAM5 OPN Ca19-9 Ca72-4 CAIX GRP DEFA1 | 10 | 0.88 | 41 | 66 |
| Cyfra SLPI TIMP1 SCC ENO2 TFPI CEACAM5 OPN Ca72-4 CA242 DEFA1 | 11 | 0.88 | 75 | 64 |
| Cyfra SLPI TIMP1 SCC ENO2 TFPI CEACAM5 OPN Ca72-4 CA242 CAIX DEFA1 | 12 | 0.88 | 75 | 64 |
| Cyfra MIF SCC ENO2 TFPI CEACAM5 OPN Ca19-9 Ca72-4 GRP DEFA1 | 11 | 0.88 | 34 | 61 |
| Cyfra SLPI ENO2 TFPI CEACAM5 OPN Ca19-9 Ca72-4 CAIX GRP DEFA1 | 11 | 0.88 | 36 | 59 |
| Cyfra TIMP1 ENO2 CEACAM5 OPN Ca72-4 DEFA1 | 7 | 0.88 | 39 | 50 |
| SCC ENO2 TFPI CEACAM5 OPN Ca72-4 DEFA1 | 7 | 0.88 | 23 | 45 |
| Cyfra TIMP1 CEACAM5 OPN Ca72-4 CA242 CAIX GRP DEFA1 | 9 | 0.88 | 57 | 57 |
| Cyfra SLPI TIMP1 ENO2 CEACAM5 OPN Ca72-4 CA242 CAIX GRP DEFA1 | 11 | 0.88 | 66 | 66 |
| SLPI TIMP1 ENO2 TFPI CEACAM5 OPN Ca19-9 Ca72-4 CA242 GRP DEFA1 | 11 | 0.88 | 64 | 41 |
| TIMP1 SCC ENO2 TFPI CEACAM5 OPN DEFA1 | 7 | 0.88 | 68 | 52 |
| Cyfra SLPI TIMP1 SCC ENO2 CEACAM5 OPN Ca72-4 CA242 DEFA1 | 10 | 0.88 | 66 | 70 |
| Cyfra TIMP1 TFPI CEACAM5 OPN Ca19-9 Ca72-4 GRF | 8 | 0.88 | 64 | 73 |
| Cyfra TIMP1 ENO2 TFPI CEACAM5 OPN Ca72-4 | 7 | 0.88 | 57 | 59 |
| Cyfra TIMP1 CEACAM5 OPN Ca19-9 Ca72-4 CAIX GRP | 8 | 0.88 | 45 | 75 |
| TIMP1 ENO2 TFPI CEACAM5 OPN Ca19-9 Ca72-4 DEFA1 | 8 | 0.88 | 39 | 23 |
| TIMP1 EN02 CEACAM5 OPN Ca19-9 Ca72-4 CA242 CAIX GRP DEFA1 | 10 | 0.88 | 43 | 59 |
| SLPI TIMP1 CEACAM5 OPN Ca19-9 Ca72-4 GRP DEFA1 | 8 | 0.88 | 52 | 43 |
| Cyfra TIMP1 ENO2 TFPI CEACAM5 OPN Ca19-9 CAIX DEFA1 | 9 | 0.88 | 55 | 27 |
| TIMP1 SCC TFPI CEACAM5 OPN Ca72-4 CA242 GRP | 8 | 0.88 | 50 | 75 |
| Cyfra TIMP1 TFPI CEACAM5 OPN Ca72-4 DEFA1 | 7 | 0.88 | 61 | 14 |
| Cyfra TIMP1 ENO2 TFPI CEACAM5 OPN CA242 GRP DEFA1 | 9 | 0.88 | 55 | 30 |
| Cyfra TIMP1 ENO2 CEACAM5 OPN Ca19-9 GRP DEFA1 | 8 | 0.88 | 50 | 36 |
| Cyfra TIMP1 ENO2 CEACAM5 OPN Ca19-9 CAIX GRP DEFA1 | 9 | 0.88 | 50 | 36 |
| Cyfra MIF SCC TFPI CEACAM5 OPN Ca19-9 Ca72-4 CAIX GRP DEFA1 | 11 | 0.88 | 45 | 34 |
| Cyfra MIF SLPI TIMP1 SCC ENO2 CEACAM5 OPN Ca19-9 Ca72-4 GRP DEFA1 | 12 | 0.88 | 57 | 55 |
| Cyfra SLPI TIMP1 SCC TFPI CEACAM5 OPN Ca72-4 CA242 CAIX GRP DEFA1 | 12 | 0.88 | 59 | 39 |
| Cyfra MIF SLPI TIMP1 SCC ENO2 CEACAM5 OPN Ca19-9 Ca72-4 CAIX GRP DEFA1 | 13 | 0.88 | 57 | 55 |
| MIF TIMP1 SCC TFPI CEACAM5 OPN Ca19-9 Ca72-4 DEFA1 | 9 | 0.88 | 57 | 34 |
| Cyfra MIF TIMP1 SCC TFPI CEACAM5 OPN Ca72-4 CAIX GRP | 10 | 0.88 | 55 | 20 |
| SLPI TIMP1 SCC ENO2 TFPI CEACAM5 OPN Ca19-9 Ca72-4 DEFA1 | 10 | 0.88 | 55 | .64 |
| SLPI TIMP1 ENO2 TFPI CEACAM5 OPN Ca19-9 Ca72-4 CAIX DEFA1 | 10 | 0.88 | 52 | 64 |
| Cyfra TIMP1 SCC ENO2 TFPI CEACAM5 OPN Ca19-9 Ca72-4 CA242 CAIX DEFA1 | 12 | 0.88 | 48 | 61 |
| TIMP1 SCC ENO2 CEACAM5 OPN Ca19-9 CAIX DEFA1 | 8 | 0.88 | 45 | 41 |
| Cyfra MIF SLPI TIMP1 SCC TFPI CEACAM5 OPN Ca19-9 Ca72-4 CA242 CAIX GRP DEFA1 | 14 | 0.88 | 59 | 52 |
| Cyfra TIMP1 SCC TFPI CEACAM5 OPN Ca19-9 Ca72-4 CAIX DEFA1 | 10 | 0.88 | 57 | 50 |
| Cyfra TIMP1 SCC TFPI CEACAM5 OPN Ca19-9 Ca72-4 CA242 CAIX GRP DEFA1 | 12 | 0.88 | 66 | 39 |
| TIMP1 SCC ENO2 TFPI CEACAM5 OPN Ca72-4 GRP | 8 | 0.88 | 64 | 59 |
| Cyfra MIF TIMP1 SCC CEACAM5 OPN Ca19-9 GRP DEFA1 | 9 | 0.88 | 64 | 41 |
| TIMP1 SCC ENO2 CEACAM5 OPN Ca72-4 CA242 CAIX DEFA1 | 9 | 0.88 | 48 | 36 |
| Cyfra MIF TIMP1 SCC CEACAM5 OPN Ca19-9 CAIX GRP DEFA1 | 10 | 0.88 | 64 | 41 |
| Cyfra MIF TIMP1 TFPI CEACAM5 OPN Ca19-9 Ca72-4 CAIX GRF | 10 | 0.88 | 70 | 57 |
| Cyfra SLPI TIMP1 ENO2 CEACAM5 OPN Ca19-9 Ca72-4 CAIX DEFA1 | 10 | 0.88 | 41 | 68 |
| Cyfra SLPI TIMP1 ENO2 CEACAM5 OPN Ca72-4 CA242 GRP DEFA1 | 10 | 0.88 | 66 | 66 |
| MIF TIMP1 ENO2 TFPI CEACAM5 OPN Ca72-4 CA242 GRP DEFA1 | 10 | 0.88 | 73 | 64 |
| SLPI SCC ENO2 TFPI CEACAM5 OPN Ca19-9 Ca72-4 GRP DEFA1 | 10 | 0.88 | 39 | 61 |
| MIF TIMP1 SCC ENO2 TFPI CEACAM5 OPN Ca72-4 CA242 CAIX DEFA1 | 11 | 0.88 | 73 | 66 |
| MIF TIMP1 ENO2 TFPI CEACAM5 OPN Ca72-4 CA242 CAIX GRP DEFA1 | 11 | 0.88 | 73 | 64 |
| MIF TIMP1 SCC CEACAM5 OPN Ca72-4 CAIX GRP DEFA1 | 9 | 0.88 | 64 | 61 |
| TIMP1 SCC ENO2 TFPI CEACAM5 OPN Ca72-4 CA242 GRF | 9 | 0.88 | 66 | 50 |
| Cyfra MIF TIMP1 ENO2 TFPI CEACAM5 OPN Ca72-4 CA242 DEFA1 | 10 | 0.88 | 68 | 66 |
| Cyfra MIF TIMP1 ENO2 TFPI CEACAM5 OPN Ca72-4 CA242 CAIX DEFA1 | 11 | 0.88 | 68 | 66 |
| TIMP1 TFPI CEACAM5 OPN Ca19-9 Ca72-4 CAIX GRP | 8 | 0.88 | 61 | 73 |
| Cyfra MIF TIMP1 SCC CEACAM5 OPN Ca19-9 Ca72-4 CAIX GRF | 10 | 0.88 | 55 | 61 |
| ENO2 TFPI CEACAM5 OPN Ca19-9 Ca72-4 CAIX GRP DEFA1 | 9 | 0.88 | 20 | 77 |
| Cyfra TIMP1 CEACAM5 OPN Ca19-9 Ca72-4 GRP | 7 | 0.88 | 45 | 75 |
| Cyfra TIMP1 ENO2 TFPI CEACAM5 OPN Ca19-9 DEFA1 | 8 | 0.88 | 55 | 27 |
| TIMP1 SCC ENO2 TFPI CEACAM5 OPN Ca19-9 DEFA1 | 8 | 0.88 | 55 | 32 |
| Cyfra SLPI TIMP1 SCC ENO2 TFPI CEACAM5 OPN Ca72-4 CAIX GRP DEFA1 | 12 | 0.88 | 70 | 61 |
| TIMP1 TFPI CEACAM5 OPN Ca72-4 DEFA1 | 6 | 0.88 | 57 | 16 |
| Cyfra SCC ENO2 CEACAM5 OPN Ca19-9 Ca72-4 GRP DEFA1 | 9 | 0.88 | 20 | 82 |
| MIF SLPI TIMP1 TFPI CEACAM5 OPN Ca19-9 Ca72-4 GRP DEFA1 | 10 | 0.88 | 68 | 20 |
| MIF SLPI TIMP1 TFPI CEACAM5 OPN Ca19-9 Ca72-4 CAIX GRP DEFA1 | 11 | 0.88 | 68 | 20 |
| Cyfra MIF SLPI TIMP1 ENO2 TFPI CEACAM5 OPN Ca19-9 Ca72-4 GRP DEFA1 | 12 | 0.88 | 66 | 45 |
| Cyfra SLPI TIMP1 SCC TFPI CEACAM5 OPN Ca72-4 CA242 GRP DEFA1 | 11 | 0.88 | 59 | 39 |
| ENO2 TFPI CEACAM5 OPN Ca72-4 GRP DEFA1 | 7 | 0.88 | 25 | 84 |
| TIMP1 ENO2 TFPI CEACAM5 OPN CA242 GRP DEFA1 | 8 | 0.88 | 55 | 34 |
| TIMP1 SCC ENO2 TFPI CEACAM5 OPN Ca19-9 Ca72-4 CAIX | 9 | 0.88 | 50 | 48 |
| TIMP1 ENO2 TFPI CEACAM5 OPN CA242 CAIX GRP DEFA1 | 9 | 0.88 | 55 | 34 |
| Cyfra TIMP1 SCC ENO2 TFPI CEACAM5 OPN CA242 CAIX DEFA1 | 10 | 0.88 | 52 | 30 |
| TIMP1 CEACAM5 OPN Ca72-4 GRP DEFA1 | 6 | 0.88 | 55 | 27 |
| Cyfra TIMP1 SCC TFPI CEACAM5 OPN Ca19-9 Ca72-4 CA242 GRP DEFA1 | 11 | 0.88 | 66 | 39 |
| Cyfra MIF SLPI TIMP1 SCC TFPI CEACAM5 OPN Ca19-9 Ca72-4 CA242 GRP DEFA1 | 13 | 0.88 | 59 | 52 |
| Cyfra SLPI TIMP1 ENO2 CEACAM5 OPN Ca19-9 Ca72-4 DEFA1 | 9 | 0.88 | 39 | 68 |
| SLPI TIMP1 ENO2 CEACAM5 OPN Ca19-9 Ca72-4 GRP DEFA1 | 9 | 0.88 | 39 | 66 |
| Cyfra TIMP1 ENO2 CEACAM5 OPN Ca19-9 CAIX DEFA1 | 8 | 0.88 | 45 | 36 |
| Cyfra MIF TIMP1 TFPI CEACAM5 OPN Ca19-9 Ca72-4 GRF | 9 | 0.88 | 70 | 57 |
| Cyfra SLPI TIMP1 SCC CEACAM5 OPN Ca19-9 Ca72-4 CA242 CAIX GRP DEFA1 | 12 | 0.88 | 64 | 64 |
| TIMP1 SCC ENO2 CEACAM5 OPN Ca19-9 Ca72-4 CAIX GRF | 9 | 0.88 | 39 | 52 |
| Cyfra TIMP1 SCC TFPI CEACAM5 OPN Ca72-4 CA242 CAIX DEFA1 | 10 | 0.88 | 68 | 57 |
| MIF TIMP1 SCC ENO2 TFPI CEACAM5 OPN Ca72-4 CA242 DEFA1 | 10 | 0.88 | 73 | 66 |
| MIF TIMP1 SCC TFPI CEACAM5 OPN Ca19-9 CAIX GRP DEFA1 | 10 | 0.88 | 77 | 36 |
| TIMP1 ENO2 TFPI CEACAM5 OPN CAIX DEFA1 | 7 | 0.88 | 68 | 52 |
| Cyfra TIMP1 CEACAM5 OPN Ca72-4 CA242 GRP DEFA1 | 8 | 0.88 | 57 | 57 |
| MIF TIMP1 ENO2 TFPI CEACAM5 OPN Ca19-9 CAIX GRP DEFA1 | 10 | 0.88 | 66 | 64 |
| SLPI TIMP1 SCC EN02 CEACAM5 OPN Ca19-9 CAIX GRP DEFA1 | 10 | 0.88 | 61 | 70 |
| Cyfra MIF TIMP1 SCC CEACAM5 OPN Ca19-9 Ca72-4 GRF | 9 | 0.88 | 55 | 61 |
| TIMP1 SCC CEACAM5 OPN Ca19-9 Ca72-4 GRP | 7 | 0.88 | 48 | 75 |
| TIMP1 TFPI CEACAM5 OPN Ca72-4 CA242 GRP | 7 | 0.88 | 48 | 75 |
| Cyfra SLPI TIMP1 ENO2 TFPI CEACAM5 OPN Ca72-4 CAIX GRP DEFA1 | 11 | 0.88 | 70 | 61 |
| Cyfra SLPI TIMP1 SCC CEACAM5 OPN Ca72-4 CA242 CAIX GRP DEFA1 | 11 | 0.88 | 73 | 50 |
| Cyfra MIF TIMP1 SCC ENO2 TFPI CEACAM5 OPN Ca19-9 Ca72-4 CAIX GRF | 12 | 0.88 | 66 | 32 |
| Cyfra MIF SCC TFPI CEACAM5 OPN Ca19-9 Ca72-4 GRP DEFA1 | 10 | 0.88 | 45 | 34 |
| Cyfra MIF SLPI TIMP1 SCC ENO2 TFPI CEACAM5 OPN Ca19-9 Ca72-4 CAIX DEFA1 | 13 | 0.88 | 66 | 48 |
| Cyfra MIF SLPI TIMP1 SCC ENO2 CEACAM5 OPN Ca19-9 Ca72-4 CA242 CAIX GRP DEFA1 | 14 | 0.88 | 64 | 43 |
| Cyfra TIMP1 SCC ENO2 TFPI CEACAM5 OPN CA242 DEFA1 | 9 | 0.88 | 52 | 30 |
| Cyfra MIF TIMP1 TFPI CEACAM5 OPN Ca19-9 Ca72-4 CAIX DEFA1 | 10 | 0.88 | 59 | 32 |
| Cyfra TIMP1 SCC ENO2 TFPI CEACAM5 OPN Ca19-9 Ca72-4 CA242 DEFA1 | 11 | 0.88 | 45 | 61 |
| Cyfra MIF TIMP1 SCC TFPI CEACAM5 OPN Ca72-4 GRP | 9 | 0.88 | 55 | 20 |
| Cyfra SLPI ENO2 TFPI CEACAM5 OPN Ca19-9 Ca72-4 GRP DEFA1 | 10 | 0.88 | 34 | 59 |
| Cyfra SLPI TIMP1 SCC ENO2 TFPI CEACAM5 OPN Ca19-9 CAIX GRP DEFA1 | 12 | 0.88 | 75 | 61 |
| Cyfra TIMP1 SCC TFPI CEACAM5 OPN Ca72-4 CAIX | 8 | 0.88 | 68 | 34 |
| SLPI TIMP1 SCC ENO2 CEACAM5 OPN Ca19-9 Ca72-4 CAIX DEFA1 | 10 | 0.88 | 36 | 70 |
| Cyfra TIMP1 SCC CEACAM5 OPN Ca72-4 GRP | 7 | 0.88 | 64 | 45 |
| Cyfra TIMP1 ENO2 CEACAM5 OPN Ca19-9 DEFA1 | 7 | 0.88 | 45 | 36 |
| Cyfra TIMP1 SCC CEACAM5 OPN Ca72-4 CAIX GRP | 8 | 0.88 | 64 | 45 |
| Cyfra TIMP1 SCC TFPI CEACAM5 OPN Ca19-9 Ca72-4 DEFA1 | 9 | 0.88 | 55 | 50 |
| Cyfra MIF TIMP1 SCC TFPI CEACAM5 OPN Ca19-9 CAIX GRP DEFA1 | 11 | 0.88 | 77 | 34 |
| Cyfra TIMP1 SCC ENO2 TFPI CEACAM5 OPN Ca19-9 Ca72-4 CA242 CAIX GRF | 12 | 0.88 | 61 | 32 |
| Cyfra TIMP1 SCC TFPI CEACAM5 OPN Ca72-4 CA242 DEFA1 | 9 | 0.88 | 68 | 57 |
| MIF TIMP1 SCC TFPI CEACAM5 OPN Ca19-9 GRP DEFA1 | 9 | 0.88 | 77 | 36 |
| TIMP1 ENO2 TFPI CEACAM5 OPN Ca72-4 CA242 CAIX GRF | 9 | 0.88 | 66 | 50 |
| MIF TIMP1 SCC CEACAM5 OPN Ca19-9 CAIX GRP DEFA1 | 9 | 0.88 | 61 | 43 |
| MIF TIMP1 ENO2 TFPI CEACAM5 OPN Ca19-9 GRP DEFA1 | 9 | 0.88 | 66 | 64 |
| Cyfra SLPI TIMP1 ENO2 CEACAM5 OPN Ca19-9 CAIX GRP DEFA1 | 10 | 0.88 | 59 | 68 |
| Cyfra MIF SLPI TIMP1 SCC CEACAM5 OPN Ca19-9 CAIX GRP DEFA1 | 11 | 0.88 | 75 | 34 |
| Cyfra MIF TIMP1 ENO2 CEACAM5 OPN Ca19-9 Ca72-4 CAIX GRP DEFA1 | 11 | 0.88 | 36 | 66 |
| MIF TIMP1 TFPI CEACAM5 OPN Ca19-9 Ca72-4 CA242 CAIX GRP DEFA1 | 11 | 0.88 | 68 | 68 |
| MIF SCC ENO2 TFPI CEACAM5 OPN Ca19-9 Ca72-4 CAIX GRP DEFA1 | 11 | 0.88 | 34 | 61 |
| Cyfra MIF SLPI TIMP1 CEACAM5 OPN Ca19-9 Ca72-4 CA242 CAIX GRP DEFA1 | 12 | 0.88 | 68 | 52 |
| MIF TIMP1 SCC CEACAM5 OPN Ca72-4 GRP DEFA1 | 8 | 0.88 | 64 | 61 |
| Cyfra TIMP1 ENO2 TFPI CEACAM5 OPN Ca72-4 CA242 CAIX | 9 | 0.88 | 61 | 50 |
| SLPI TIMP1 SCC ENO2 CEACAM5 OPN Ca19-9 GRP DEFA1 | 9 | 0.88 | 61 | 70 |
| ENO2 TFPI CEACAM5 OPN Ca19-9 Ca72-4 GRP DEFA1 | 8 | 0.88 | 18 | 77 |
| TIMP1 ENO2 CEACAM5 OPN Ca19-9 Ca72-4 CA242 GRP DEFA1 | 9 | 0.88 | 41 | 59 |
| Cyfra SLPI TIMP1 SCC ENO2 TFPI CEACAM5 OPN Ca72-4 GRP DEFA1 | 11 | 0.88 | 70 | 61 |
| TIMP1 ENO2 CEACAM5 OPN Ca19-9 Ca72-4 CAIX DEFA1 | 8 | 0.88 | 25 | 32 |
| ENO2 TFPI CEACAM5 OPN Ca72-4 CA242 CAIX GRP DEFA1 | 9 | 0.88 | 39 | 80 |
| Cyfra SLPI TIMP1 SCC CEACAM5 OPN Ca72-4 CA242 GRP DEFA1 | 10 | 0.88 | 73 | 50 |
| Cyfra MIF SLPI TIMP1 SCC ENO2 CEACAM5 OPN Ca19-9 Ca72-4 CA242 GRP DEFA1 | 13 | 0.88 | 64 | 43 |
| Cyfra TIMP1 ENO2 TFPI CEACAM5 OPN Ca19-9 Ca72-4 CAIX | 9 | 0.88 | 48 | 45 |
| Cyfra MIF TIMP1 SCC ENO2 TFPI CEACAM5 OPN Ca19-9 Ca72-4 GRF | 11 | 0.88 | 66 | 32 |
| Cyfra SLPI TIMP1 SCC TFPI CEACAM5 OPN Ca72-4 CAIX GRP DEFA1 | 11 | 0.88 | 75 | 48 |
| Cyfra SLPI TIMP1 TFPI CEACAM5 OPN Ca72-4 CA242 CAIX GRP DEFA1 | 11 | 0.88 | 57 | 39 |
| Cyfra MIF SLPI TIMP1 SCC CEACAM5 OPN Ca72-4 CA242 CAIX GRP DEFA1 | 12 | 0.88 | 57 | 41 |
| Cyfra MIF SLPI TIMP1 SCC ENO2 TFPI CEACAM5 OPN Ca19-9 Ca72-4 DEFA1 | 12 | 0.88 | 66 | 48 |
| Cyfra MIF SLPI TIMP1 SCC CEACAM5 OPN Ca19-9 Ca72-4 CAIX DEFA1 | 11 | 0.88 | 64 | 36 |
| Cyfra SLPI TIMP1 SCC ENO2 TFPI CEACAM5 OPN Ca19-9 GRP DEFA1 | 11 | 0.88 | 75 | 61 |
| Cyfra SLPI TIMP1 ENO2 TFPI CEACAM5 OPN Ca19-9 CAIX GRP DEFA1 | 11 | 0.88 | 75 | 61 |
| TIMP1 SCC ENO2 CEACAM5 OPN Ca19-9 DEFA1 | 7 | 0.88 | 43 | 41 |
| MIF TIMP1 TFPI CEACAM5 OPN Ca19-9 Ca72-4 CAIX GRF | 9 | 0.88 | 68 | 57 |
| TIMP1 SCC TFPI CEACAM5 OPN Ca19-9 Ca72-4 CAIX DEFA1 | 9 | 0.88 | 52 | 52 |
| Cyfra TIMP1 SCC ENO2 TFPI CEACAM5 OPN Ca19-9 Ca72-4 CA242 GRF | 11 | 0.88 | 61 | 32 |
| SLPI TIMP1 SCC ENO2 TFPI CEACAM5 OPN Ca19-9 CAIX GRP DEFA1 | 11 | 0.88 | 73 | 64 |
| Cyfra TIMP1 SCC ENO2 CEACAM5 OPN GRP DEFA1 | 8 | 0.88 | 61 | 59 |
| TIMP1 ENO2 CEACAM5 OPN Ca72-4 CAIX GRP DEFA1 | 8 | 0.88 | 41 | 55 |
| Cyfra TIMP1 SCC ENO2 CEACAM5 OPN CAIX GRP DEFA1 | 9 | 0.88 | 61 | 59 |
| Cyfra MIF TIMP1 SCC TFPI CEACAM5 OPN Ca19-9 GRP DEFA1 | 10 | 0.88 | 77 | 34 |
| Cyfra TIMP1 ENO2 TFPI CEACAM5 OPN Ca19-9 Ca72-4 CA242 CAIX DEFA1 | 11 | 0.88 | 48 | 61 |
| TIMP1 SCC ENO2 TFPI CEACAM5 OPN Ca19-9 Ca72-4 CA242 CAIX DEFA1 | 11 | 0.88 | 45 | 61 |
| MIF SLPI TIMP1 SCC TFPI CEACAM5 OPN Ca19-9 Ca72-4 CA242 CAIX GRP DEFA1 | 13 | 0.88 | 59 | 55 |
| TIMP1 SCC ENO2 CEACAM5 OPN Ca72-4 CA242 DEFA1 | 8 | 0.88 | 45 | 36 |
| Cyfra MIF TIMP1 SCC TFPI CEACAM5 OPN Ca72-4 CAIX | 9 | 0.88 | 80 | 23 |
| Cyfra MIF TIMP1 ENO2 CEACAM5 OPN Ca19-9 Ca72-4 GRP DEFA1 | 10 | 0.88 | 36 | 66 |
| TIMP1 ENO2 TFPI CEACAM5 OPN DEFA1 | 6 | 0.88 | 68 | 52 |
| MIF TIMP1 SCC CEACAM5 OPN Ca19-9 GRP DEFA1 | 8 | 0.88 | 61 | 43 |
| MIF TIMP1 TFPI CEACAM5 OPN Ca19-9 Ca72-4 CA242 GRP DEFA1 | 10 | 0.88 | 68 | 68 |
| Cyfra MIF SLPI TIMP1 CEACAM5 OPN Ca19-9 Ca72-4 CA242 GRP DEFA1 | 11 | 0.88 | 68 | 52 |
| Cyfra SLPI TIMP1 SCC CEACAM5 OPN Ca19-9 Ca72-4 CA242 GRP DEFA1 | 11 | 0.88 | 64 | 64 |
| Cyfra TIMP1 ENO2 TFPI CEACAM5 OPN Ca72-4 CA242 | 8 | 0.88 | 61 | 50 |
| Cyfra SLPI TIMP1 ENO2 CEACAM5 OPN Ca19-9 GRP DEFA1 | 9 | 0.88 | 59 | 68 |
| Cyfra MIF SLPI TIMP1 SCC CEACAM5 OPN Ca19-9 GRP DEFA1 | 10 | 0.88 | 75 | 34 |
| Cyfra SLPI TIMP1 ENO2 CEACAM5 OPN Ca72-4 CA242 CAIX DEFA1 | 10 | 0.88 | 61 | 70 |
| TIMP1 SCC ENO2 TFPI CEACAM5 OPN Ca72-4 CAIX | 8 | 0.88 | 61 | 61 |
| TIMP1 ENO2 TFPI CEACAM5 OPN Ca19-9 CAIX DEFA1 | 8 | 0.88 | 52 | 32 |
| Cyfra TIMP1 ENO2 CEACAM5 OPN Ca19-9 Ca72-4 CA242 CAIX DEFA1 | 10 | 0.88 | 43 | 64 |
| Cyfra SLPI TIMP1 ENO2 TFPI CEACAM5 OPN Ca72-4 GRP DEFA1 | 10 | 0.88 | 70 | 61 |
| Cyfra MIF TIMP1 ENO2 TFPI CEACAM5 OPN GRP DEFA1 | 9 | 0.88 | 61 | 32 |
| Cyfra MIF TIMP1 ENO2 TFPI CEACAM5 OPN CAIX GRP DEFA1 | 10 | 0.88 | 61 | 32 |
| MIF SLPI TIMP1 SCC ENO2 TFPI CEACAM5 OPN Ca19-9 Ca72-4 GRP DEFA1 | 12 | 0.88 | 70 | 48 |
| MIF TIMP1 ENO2 TFPI CEACAM5 OPN Ca19-9 Ca72-4 CA242 CAIX GRP DEFA1 | 12 | 0.88 | 59 | 48 |
| MIF SLPI TIMP1 SCC ENO2 TFPI CEACAM5 OPN Ca19-9 Ca72-4 CAIX GRP DEFA1 | 13 | 0.88 | 70 | 48 |
| Cyfra MIF TIMP1 SCC ENO2 CEACAM5 OPN Ca72-4 CAIX DEFA1 | 10 | 0.88 | 55 | 36 |
| Cyfra SLPI TIMP1 TFPI CEACAM5 OPN Ca72-4 CA242 GRP DEFA1 | 10 | 0.88 | 57 | 39 |
| Cyfra MIF SLPI TIMP1 SCC CEACAM5 OPN Ca72-4 CA242 GRP DEFA1 | 11 | 0.88 | 57 | 41 |
| Cyfra MIF SLPI TIMP1 SCC TFPI CEACAM5 OPN Ca19-9 Ca72-4 CAIX DEFA1 | 12 | 0.88 | 68 | 23 |
| SLPI TIMP1 SCC ENO2 CEACAM5 OPN Ca19-9 Ca72-4 CA242 CAIX GRP DEFA1 | 12 | 0.88 | 52 | 48 |
| Cyfra MIF TIMP1 TFPI CEACAM5 OPN Ca19-9 Ca72-4 DEFA1 | 9 | 0.88 | 59 | 32 |
| MIF SCC TFPI CEACAM5 OPN Ca19-9 Ca72-4 CAIX GRP DEFA1 | 10 | 0.88 | 43 | 36 |
| Cyfra SLPI TIMP1 SCC ENO2 CEACAM5 OPN Ca19-9 Ca72-4 CA242 CAIX DEFA1 | 12 | 0.88 | 48 | 50 |
| SLPI TIMP1 ENO2 TFPI CEACAM5 OPN Ca19-9 Ca72-4 DEFA1 | 9 | 0.88 | 52 | 64 |
| Cyfra MIF SLPI TIMP1 SCC CEACAM5 OPN Ca19-9 Ca72-4 DEFA1 | 10 | 0.88 | 64 | 36 |
| Cyfra SLPI TIMP1 ENO2 TFPI CEACAM5 OPN Ca19-9 GRP DEFA1 | 10 | 0.88 | 75 | 61 |
| SLPI TIMP1 SCC ENO2 TFPI CEACAM5 OPN Ca72-4 CA242 CAIX GRP DEFA1 | 12 | 0.88 | 75 | 61 |
| Cyfra TIMP1 SCC TFPI CEACAM5 OPN Ca72-4 | 7 | 0.88 | 68 | 34 |
| MIF TIMP1 TFPI CEACAM5 OPN Ca19-9 Ca72-4 GRF | 8 | 0.88 | 68 | 57 |
| TIMP1 SCC ENO2 TFPI CEACAM5 OPN CA242 CAIX DEFA1 | 9 | 0.88 | 52 | 34 |
| Cyfra SLPI TIMP1 ENO2 TFPI CEACAM5 OPN Ca72-4 CA242 DEFA1 | 10 | 0.88 | 73 | 64 |
| SLPI TIMP1 ENO2 TFPI CEACAM5 OPN Ca19-9 CAIX GRP DEFA1 | 10 | 0.88 | 73 | 64 |
| Cyfra SLPI TIMP1 ENO2 TFPI CEACAM5 OPN Ca72-4 CA242 CAIX DEFA1 | 11 | 0.88 | 73 | 64 |
| TIMP1 ENO2 TFPI CEACAM5 OPN Ca72-4 CAIX GRP | 8 | 0.88 | 61 | 59 |
| SLPI TIMP1 SCC ENO2 TFPI CEACAM5 OPN Ca19-9 GRP DEFA1 | 10 | 0.88 | 73 | 64 |
| SLPI ENO2 TFPI CEACAM5 OPN Ca19-9 Ca72-4 CAIX GRP DEFA1 | 10 | 0.88 | 34 | 61 |
| MIF SLPI TIMP1 SCC TFPI CEACAM5 OPN Ca19-9 Ca72-4 CA242 GRP DEFA1 | 12 | 0.88 | 57 | 55 |
| ENO2 TFPI CEACAM5 OPN Ca72-4 CAIX DEFA1 | 7 | 0.88 | 25 | 45 |
| Cyfra MIF TIMP1 SCC TFPI CEACAM5 OPN Ca72-4 | 8 | 0.88 | 80 | 23 |
| TIMP1 SCC ENO2 CEACAM5 OPN Ca19-9 Ca72-4 GRF | 8 | 0.88 | 36 | 52 |
| TIMP1 EN02 TFPI CEACAM5 OPN Ca72-4 CA242 GRP | 8 | 0.88 | 66 | 50 |
| MIF TIMP1 SCC CEACAM5 OPN Ca72-4 CA242 GRP DEFA1 | 9 | 0.88 | 66 | 45 |
| MIF TIMP1 TFPI CEACAM5 OPN Ca19-9 CAIX GRP DEFA1 | 9 | 0.88 | 77 | 36 |
| MIF TIMP1 SCC CEACAM5 OPN Ca72-4 CA242 CAIX GRP DEFA1 | 10 | 0.88 | 66 | 45 |
| MIF SCC ENO2 TFPI CEACAM5 OPN Ca19-9 Ca72-4 GRP DEFA1 | 10 | 0.88 | 32 | 61 |
| Cyfra TIMP1 ENO2 CEACAM5 OPN Ca19-9 Ca72-4 CAIX | 8 | 0.88 | 34 | 52 |
| Cyfra SLPI SCC ENO2 CEACAM5 OPN Ca19-9 Ca72-4 CA242 CAIX GRP DEFA1 | 12 | 0.88 | 34 | 48 |
| TIMP1 TFPI CEACAM5 OPN Ca19-9 Ca72-4 GRP | 7 | 0.88 | 59 | 73 |
| Cyfra MIF TIMP1 SCC ENO2 CEACAM5 OPN Ca72-4 GRP DEFA1 | 10 | 0.88 | 55 | 36 |
| Cyfra MIF TIMP1 SCC ENO2 CEACAM5 OPN Ca72-4 CAIX GRP DEFA1 | 11 | 0.88 | 55 | 36 |
| TIMP1 ENO2 CEACAM5 OPN Ca19-9 Ca72-4 DEFA1 | 7 | 0.88 | 25 | 32 |
| TIMP1 ENO2 CEACAM5 OPN Ca19-9 CAIX GRP DEFA1 | 8 | 0.88 | 48 | 41 |
| Cyfra MIF TIMP1 SCC CEACAM5 OPN Ca72-4 CAIX DEFA1 | 9 | 0.88 | 64 | 61 |
| MIF TIMP1 ENO2 TFPI CEACAM5 OPN Ca19-9 Ca72-4 CA242 GRP DEFA1 | 11 | 0.88 | 59 | 48 |
| Cyfra TIMP1 ENO2 TFPI CEACAM5 OPN Ca19-9 Ca72-4 | 8 | 0.88 | 45 | 45 |
| Cyfra MIF SLPI TIMP1 SCC TFPI CEACAM5 OPN Cal9-9 Ca72-4 DEFA1 | 11 | 0.88 | 68 | 23 |
| Cyfra SLPI TIMP1 SCC ENO2 TFPI CEACAM5 OPN Ca72-4 CAIX DEFA1 | 11 | 0.88 | 70 | 66 |
| Cyfra MIF TIMP1 SCC ENO2 CEACAM5 OPN Ca72-4 DEFA1 | 9 | 0.88 | 55 | 36 |
| Cyfra SLPI TIMP1 SCC TFPI CEACAM5 OPN Ca72-4 GRP DEFA1 | 10 | 0.88 | 75 | 48 |
| SLPI TIMP1 SCC TFPI CEACAM5 OPN Ca72-4 CA242 CAIX GRP DEFA1 | 11 | 0.88 | 59 | 39 |
| TIMP1 SCC TFPI CEACAM5 OPN Ca19-9 Ca72-4 CA242 CAIX GRP DEFA1 | 11 | 0.88 | 64 | 41 |
| SLPI TIMP1 SCC ENO2 TFPI CEACAM5 OPN Ca72-4 CA242 GRP DEFA1 | 11 | 0.88 | 75 | 61 |
| SLPI TIMP1 ENO2 TFPI CEACAM5 OPN Ca72-4 CA242 CAIX GRP DEFA1 | 11 | 0.88 | 75 | 61 |
| Cyfra MIF SLPI TIMP1 TFPI CEACAM5 OPN Ca19-9 Ca72-4 CA242 CAIX GRP DEFA1 | 13 | 0.88 | 57 | 52 |
| Cyfra MIF TIMP1 SCC ENO2 CEACAMS OPN Cal9-9 Ca72-4 CA242 CAIX GRP DEFA1 | 13 | 0.88 | 50 | 52 |
| Cyfra TIMP1 CEACAM5 OPN Ca72-4 GRP | 6 | 0.88 | 64 | 45 |
| Cyfra TIMP1 CEACAM5 OPN Ca72-4 CAIX GRP | 7 | 0.88 | 64 | 45 |
| TIMP1 ENO2 CEACAM5 OPN Ca72-4 GRP DEFA1 | 7 | 0.88 | 41 | 55 |
| SLPI TIMP1 SCC ENO2 CEACAM5 OPN Ca19-9 Ca72-4 DEFA1 | 9 | 0.88 | 36 | 70 |
| SLPI TIMP1 ENO2 TFPI CEACAM5 OPN Ca19-9 GRP DEFA1 | 9 | 0.88 | 73 | 64 |
| Cyfra MIF TIMP1 TFPI CEACAMS OPN Ca19-9 CAIX GRP DEFA1 | 10 | 0.88 | 77 | 34 |
| Cyfra TIMP1 ENO2 TFPI CEACAM5 OPN Ca19-9 Ca72-4 CA242 DEFA1 | 10 | 0.88 | 45 | 61 |
| TIMP1 SCC ENO2 TFPI CEACAM5 OPN Ca19-9 Ca72-4 CA242 DEFA1 | 10 | 0.88 | 45 | 61 |
| TIMP1 SCC TFPI CEACAM5 OPN Ca72-4 CAIX | 7 | 0.88 | 66 | 39 |
| MIF TIMP1 TFPI CEACAM5 OPN Ca19-9 GRP DEFA1 | 8 | 0.88 | 77 | 36 |
| TIMP1 SCC TFPI CEACAM5 OPN Ca72-4 CA242 CAIX DEFA1 | 9 | 0.88 | 66 | 59 |
| Cyfra SLPI TIMP1 ENO2 CEACAM5 OPN Ca72-4 CA242 DEFA1 | 9 | 0.88 | 61 | 70 |
| Cyfra MIF SCC ENO2 TFPI CEACAM5 OPN Ca72-4 CAIX DEFA1 | 10 | 0.88 | 39 | 75 |
| TIMP1 CEACAM5 OPN Ca19-9 Ca72-4 CAIX GRP | 7 | 0.88 | 45 | 75 |
| TIMP1 ENO2 CEACAM5 OPN Ca19-9 GRP DEFA1 | 7 | 0.88 | 48 | 41 |
| Cyfra MIF TIMP1 SCC CEACAM5 OPN Ca72-4 DEFA1 | 8 | 0.88 | 64 | 61 |
| Cyfra SCC TFPI CEACAM5 OPN Ca72-4 CAIX GRP DEFA1 | 9 | 0.88 | 48 | 66 |
| Cyfra SLPI TIMP1 SCC ENO2 TFPI CEACAM5 OPN Ca72-4 DEFA1 | 10 | 0.88 | 70 | 66 |
| Cyfra SLPI TIMP1 CEACAM5 OPN Ca72-4 CA242 CAIX GRP DEFA1 | 10 | 0.88 | 73 | 50 |
| SLPI TIMP1 SCC ENO2 CEACAM5 OPN Ca19-9 Ca72-4 CA242 GRP DEFA1 | 11 | 0.88 | 50 | 48 |
| Cyfra MIF TIMP1 SCC ENO2 TFPI CEACAM5 OPN Ca19-9 Ca72-4 CA242 CAIX DEFA1 | 13 | 0.88 | 57 | 50 |
| MIF SCC TFPI CEACAM5 OPN Ca19-9 Ca72-4 GRP DEFA1 | 9 | 0.88 | 41 | 36 |
| Cyfra SLPI TIMP1 TFPI CEACAM5 OPN Ca72-4 CAIX GRP DEFA1 | 10 | 0.88 | 75 | 48 |
| TIMP1 SCC ENO2 TFPI CEACAM5 OPN Ca19-9 Ca72-4 | 8 | 0.88 | 50 | 48 |
| Cyfra MIF TIMP1 TFPI CEACAM5 OPN Ca72-4 CAIX GRP | 9 | 0.88 | 50 | 20 |
| Cyfra TIMP1 ENO2 TFPI CEACAM5 OPN CA242 CAIX DEFA1 | 9 | 0.88 | 48 | 30 |
| MIF TIMP1 TFPI CEACAM5 OPN Ca19-9 Ca72-4 CAIX DEFA1 | 9 | 0.88 | 57 | 34 |
| SLPI TIMP1 SCC TFPI CEACAM5 OPN Ca72-4 CAIX GRP DEFA1 | 10 | 0.88 | 73 | 48 |
| Cyfra MIF TIMP1 SCC ENO2 TFPI CEACAM5 OPN Cal9-9 Ca72-4 CAIX | 11 | 0.88 | 59 | 34 |
| SLPI TIMP1 ENO2 TFPI CEACAM5 OPN Ca72-4 CA242 GRP DEFA1 | 10 | 0.88 | 75 | 61 |
| Cyfra MIF SLPI TIMP1 TFPI CEACAM5 OPN Ca19-9 Ca72-4 CA242 GRP DEFA1 | 12 | 0.88 | 57 | 52 |
| Cyfra MIF TIMP1 SCC ENO2 CEACAM5 OPN Ca19-9 Ca72-4 CA242 GRP DEFA1 | 12 | 0.88 | 50 | 52 |
| TIMP1 ENO2 TFPI CEACAM5 OPN Ca72-4 GRP | 7 | 0.88 | 61 | 59 |
| TIMP1 SCC ENO2 TFPI CEACAM5 OPN CA242 DEFA1 | 8 | 0.88 | 50 | 34 |
| Cyfra MIF TIMP1 TFPI CEACAM5 OPN Ca19-9 GRP DEFA1 | 9 | 0.88 | 77 | 34 |
| Cyfra MIF TIMP1 SCC TFPI CEACAM5 OPN Ca72-4 CA242 DEFA1 | 10 | 0.88 | 50 | 39 |
| Cyfra MIF TIMP1 SCC TFPI CEACAM5 OPN Ca72-4 CA242 CAIX DEFA1 | 11 | 0.88 | 50 | 39 |
| Cyfra TIMP1 TFPI CEACAM5 OPN Ca19-9 Ca72-4 CAIX DEFA1 | 9 | 0.88 | 55 | so |
| TIMP1 ENO2 TFPI CEACAM5 OPN Ca19-9 Ca72-4 CA242 CAIX DEFA1 | 10 | 0.88 | 45 | 61 |
| MIF SLPI TIMP1 SCC ENO2 TFPI CEACAM5 OPN Ca19-9 Ca72-4 CA242 GRP DEFA1 | 13 | 0.88 | 75 | 36 |
| MIF SLPI TIMP1 SCC ENO2 TFPI CEACAM5 OPN Ca19-9 Ca72-4 CA242 CAIX GRP DEFA1 | 14 | 0.88 | 75 | 36 |
| TIMP1 SCC ENO2 CEACAM5 OPN Ca72-4 CAIX DEFA1 | 8 | 0.88 | 39 | 55 |
| TIMP1 SCC CEACAM5 OPN Ca72-4 CA242 GRP DEFA1 | 8 | 0.88 | 59 | 59 |
| TIMP1 ENO2 CEACAM5 OPN Ca72-4 CA242 CAIX DEFA1 | 8 | 0.88 | 45 | 36 |
| Cyfra TIMP1 TFPI CEACAM5 OPN Ca72-4 CA242 CAIX DEFA1 | 9 | 0.88 | 66 | 57 |
| SLPI ENO2 TFPI CEACAM5 OPN Cal9-9 Ca72-4 GRP DEFA1 | 9 | 0.88 | 34 | 61 |
| TIMP1 SCC CEACAM5 OPN Ca72-4 CA242 CAIX GRP DEFA1 | 9 | 0.88 | 59 | 59 |
| Cyfra MIF SLPI TIMP1 ENO2 TFPI CEACAM5 OPN Ca19-9 Ca72-4 CA242 CAIX GRP DEFA1 | 14 | 0.88 | 70 | 34 |
| MIF TIMP1 CEACAM5 OPN Ca72-4 CAIX GRP DEFA1 | 8 | 0.88 | 61 | 61 |
| Cyfra TIMP1 ENO2 CEACAM5 OPN Ca19-9 Ca72-4 | 7 | 0.88 | 32 | 52 |
| Cyfra MIF TIMP1 SCC ENO2 CEACAM5 OPN Ca72-4 CA242 CAIX GRP DEFA1 | 12 | 0.88 | 59 | 70 |
| Cyfra TIMP1 SCC ENO2 CEACAM5 OPN CA242 CAIX DEFA1 | 9 | 0.88 | 61 | 39 |
| TIMP1 SCC ENO2 TFPI CEACAM5 OPN Ca72-4 | 7 | 0.88 | 59 | 61 |
| TIMP1 ENO2 TFPI CEACAM5 OPN Ca19-9 DEFA1 | 7 | 0.88 | 50 | 32 |
| MIF SLPI TIMP1 ENO2 TFPI CEACAM5 OPN Ca19-9 Ca72-4 GRP DEFA1 | 11 | 0.88 | 66 | 48 |
| Cyfra MIF SLPI TIMP1 SCC ENO2 CEACAM5 OPN Cal9-9 Ca72-4 CAIX DEFA1 | 12 | 0.88 | 55 | 57 |
| MIF SLPI TIMP1 ENO2 TFPI CEACAM5 OPN Ca19-9 Ca72-4 CAIX GRP DEFA1 | 12 | 0.88 | 66 | 48 |
| ENO2 TFPI CEACAM5 OPN Ca72-4 CA242 GRP DEFA1 | 8 | 0.88 | 34 | 80 |
| Cyfra SLPI TIMP1 CEACAM5 OPN Ca72-4 CA242 GRP DEFA1 | 9 | 0.88 | 73 | 50 |
| MIF SLPI TIMP1 SCC CEACAM5 OPN Ca19-9 GRP DEFA1 | 9 | 0.88 | 73 | 34 |
| MIF SLPI TIMP1 SCC CEACAM5 OPN Ca19-9 CAIX GRP DEFA1 | 10 | 0.88 | 73 | 34 |
| Cyfra MIF TIMP1 SCC EN02 TFPI CEACAM5 OPN Ca19-9 Ca72-4 CA242 DEFA1 | 12 | 0.88 | 57 | 50 |
| MIF SLPI TIMP1 SCC ENO2 TFPI CEACAM5 OPN Cal9-9 Ca72-4 CAIX DEFA1 | 12 | 0.88 | 66 | 50 |
| Cyfra MIF TIMP1 SCC ENO2 TFPI CEACAM5 OPN Ca19-9 DEFA1 | 10 | 0.88 | 68 | 16 |
| SLPI TIMP1 SCC TFPI CEACAM5 OPN Ca72-4 CA242 GRP DEFA1 | 10 | 0.88 | 57 | 39 |
| Cyfra MIF TIMP1 SCC ENO2 TFPI CEACAM5 OPN Ca19-9 CAIX DEFA1 | 11 | 0.88 | 68 | 16 |
| Cyfra MIF SCC ENO2 TFPI CEACAM5 OPN Ca72-4 CAIX GRP DEFA1 | 11 | 0.88 | 43 | 73 |
| Cyfra TIMP1 ENO2 TFPI CEACAM5 OPN CA242 DEFA1 | 8 | 0.88 | 48 | 30 |
| MIF TIMP1 TFPI CEACAM5 OPN Ca19-9 Ca72-4 DEFA1 | 8 | 0.88 | 55 | 34 |
| SLPI TIMP1 SCC TFPI CEACAM5 OPN Ca72-4 GRP DEFA1 | 9 | 0.88 | 73 | 48 |
| MIF TIMP1 SCC TFPI CEACAM5 OPN Ca72-4 CA242 CAIX DEFA1 | 10 | 0.88 | 50 | 41 |
| Cyfra SLPI TIMP1 SCC ENO2 CEACAM5 OPN Ca19-9 Ca72-4 CA242 DEFA1 | 11 | 0.88 | 45 | 50 |
| SLPI TIMP1 SCC TFPI CEACAM5 OPN Ca19-9 Ca72-4 CA242 CAIX GRP DEFA1 | 12 | 0.88 | 45 | 66 |
| TIMP1 SCC TFPI CEACAM5 OPN Cal9-9 Ca72-4 DEFA1 | 8 | 0.88 | 52 | 52 |
| Cyfra TIMP1 TFPI CEACAM5 OPN Ca19-9 Ca72-4 CA242 CAIX GRP DEFA1 | 11 | 0.88 | 61 | 39 |
| SLPI TIMP1 SCC EN02 TFPI CEACAM5 OPN Ca72-4 CA242 CAIX DEFA1 | 11 | 0.88 | 75 | 66 |
| TIMP1 SCC ENO2 TFPI CEACAM5 OPN Ca19-9 Ca72-4 CA242 CAIX GRF | 11 | 0.88 | 61 | 34 |
| TIMP1 SCC TFPI CEACAM5 OPN CAIX DEFA1 | 7 | 0.88 | 77 | 20 |
| TIMP1 SCC TFPI CEACAM5 OPN CAIX GRP DEFA1 | 8 | 0.88 | 77 | 20 |
| TIMP1 SCC CEACAM5 OPN Ca72-4 CAIX GRP | 7 | 0.88 | 64 | 50 |
| Cyfra MIF TIMP1 CEACAM5 OPN Ca19-9 GRP DEFA1 | 8 | 0.88 | 61 | 41 |
| Cyfra TIMP1 TFPI CEACAM5 OPN Ca72-4 CA242 DEFA1 | 8 | 0.88 | 66 | 57 |
| Cyfra MIF TIMP1 CEACAM5 OPN Ca19-9 CAIX GRP DEFA1 | 9 | 0.88 | 61 | 41 |
| Cyfra MIF SLPI TIMP1 CEACAM5 OPN Ca19-9 CAIX GRP DEFA1 | 10 | 0.88 | 75 | 34 |
| MIF SLPI TIMP1 SCC CEACAM5 OPN Ca19-9 Ca72-4 CA242 CAIX GRP DEFA1 | 12 | 0.88 | 73 | 55 |
| Cyfra MIF SLPI TIMP1 ENO2 TFPI CEACAM5 OPN Ca19-9 Ca72-4 CA242 GRP DEFA1 | 13 | 0.88 | 70 | 34 |
| ENO2 TFPI CEACAM5 OPN Ca72-4 DEFA1 | 6 | 0.88 | 23 | 45 |
| Cyfra SLPI SCC ENO2 CEACAM5 OPN Ca19-9 Ca72-4 CA242 GRP DEFA1 | 11 | 0.88 | 32 | 48 |
| Cyfra TIMP1 SCC ENO2 CEACAM5 OPN CA242 DEFA1 | 8 | 0.88 | 61 | 39 |
| Cyfra MIF SCC ENO2 TFPI CEACAM5 OPN Ca72-4 DEFA1 | 9 | 0.88 | 39 | 75 |
| SLPI SCC ENO2 CEACAM5 OPN Ca19-9 Ca72-4 CAIX GRP DEFA1 | 10 | 0.88 | 18 | 68 |
| Cyfra TIMP1 ENO2 CEACAM5 OPN Ca19-9 Ca72-4 CA242 DEFA1 | 9 | 0.88 | 41 | 64 |
| TIMP1 SCC ENO2 CEACAM5 OPN Ca19-9 Ca72-4 CA242 DEFA1 | 9 | 0.88 | 41 | 64 |
| TIMP1 SCC ENO2 CEACAM5 OPN Ca19-9 Ca72-4 CA242 CAIX DEFA1 | 10 | 0.88 | 41 | 64 |
| Cyfra MIF SLPI TIMP1 SCC ENO2 CEACAM5 OPN Ca19-9 Ca72-4 DEFA1 | 11 | 0.88 | 55 | 57 |
| Cyfra SLPI TIMP1 ENO2 TFPI CEACAM5 OPN Ca72-4 CAIX DEFA1 | 10 | 0.88 | 68 | 66 |
| Cyfra MIF SLPI TIMP1 CEACAM5 OPN Ca72-4 CA242 CAIX GRP DEFA1 | 11 | 0.88 | 52 | 41 |
| MIF SLPI TIMP1 SCC ENO2 TFPI CEACAM5 OPN Ca19-9 Ca72-4 DEFA1 | 11 | 0.88 | 64 | 50 |
| Cyfra MIF SLPI TIMP1 SCC TFPI CEACAM5 OPN Ca72-4 CA242 GRP DEFA1 | 12 | 0.88 | 68 | 27 |
| Cyfra SLPI TIMP1 SCC ENO2 TFPI CEACAM5 OPN Ca19-9 Ca72-4 CAIX GRF | 12 | 0.88 | 68 | 34 |
| Cyfra MIF SLPI TIMP1 SCC TFPI CEACAM5 OPN Ca72-4 CA242 CAIX GRP DEFA1 | 13 | 0.88 | 68 | 27 |
| Cyfra SLPI TIMP1 TFPI CEACAM5 OPN Ca72-4 GRP DEFA1 | 9 | 0.88 | 75 | 48 |
| MIF TIMP1 SCC ENO2 TFPI CEACAM5 OPN CAIX GRP DEFA1 | 10 | 0.88 | 64 | 36 |
| SLPI TIMP1 TFPI CEACAM5 OPN Ca72-4 CA242 CAIX GRP DEFA1 | 10 | 0.88 | 57 | 39 |
| Cyfra SLPI TIMP1 SCC ENO2 CEACAM5 OPN Ca72-4 CAIX GRP DEFA1 | 11 | 0.88 | 66 | 41 |
| MIF SLPI TIMP1 SCC TFPI CEACAM5 OPN Ca19-9 Ca72-4 CAIX DEFA1 | 11 | 0.88 | 66 | 23 |
| Cyfra MIF SLPI TIMP1 SCC ENO2 CEACAM5 OPN Ca72-4 CA242 CAIX GRP DEFA1 | 13 | 0.88 | 80 | 59 |
| Cyfra SLPI TIMP1 SCC TFPI CEACAM5 OPN Ca19-9 Ca72-4 CA242 CAIX GRP DEFA1 | 13 | 0.88 | 48 | 64 |
| TIMP1 ENO2 TFPI CEACAM5 OPN Ca19-9 Ca72-4 CAIX | 8 | 0.88 | 45 | 48 |
| Cyfra MIF TIMP1 SCC ENO2 TFPI CEACAM5 OPN Ca19-9 Ca72-4 | 10 | 0.88 | 59 | 34 |
| MIF TIMP1 SCC ENO2 TFPI CEACAM5 OPN Ca19-9 CAIX DEFA1 | 10 | 0.88 | 66 | 20 |
| SLPI TIMP1 SCC CEACAM5 OPN Ca72-4 CA242 CAIX GRP DEFA1 | 10 | 0.88 | 73 | 50 |
| Cyfra MIF TIMP1 TFPI CEACAM5 OPN Ca72-4 GRP | 8 | 0.88 | 50 | 20 |
| Cyfra TIMP1 TFPI CEACAM5 OPN Ca19-9 Ca72-4 DEFA1 | 8 | 0.88 | 52 | 50 |
| SLPI TIMP1 ENO2 CEACAM5 OPN Ca19-9 Ca72-4 CAIX DEFA1 | 9 | 0.88 | 34 | 70 |
| Cyfra TIMP1 TFPI CEACAM5 OPN Ca19-9 Ca72-4 CA242 GRP DEFA1 | 10 | 0.88 | 61 | 39 |
| MIF SLPI TIMP1 TFPI CEACAM5 OPN Ca19-9 Ca72-4 CA242 CAIX GRP DEFA1 | 12 | 0.88 | 57 | 55 |
| Cyfra MIF SLPI TIMP1 SCC ENO2 TFPI CEACAM5 OPN Ca19-9 Ca72-4 CA242 DEFA1 | 13 | 0.88 | 70 | 36 |
| Cyfra MIF SLPI TIMP1 SCC ENO2 TFPI CEACAM5 OPN Ca19-9 Ca72-4 CA242 CAIX DEFA1 | 14 | 0.88 | 70 | 36 |
| Cyfra MIF SLPI TIMP1 CEACAM5 OPN Ca19-9 GRP DEFA1 | 9 | 0.88 | 75 | 34 |
| MIF TIMP1 ENO2 TFPI CEACAM5 OPN Ca72-4 CA242 CAIX DEFA1 | 10 | 0.88 | 68 | 66 |
| MIF TIMP1 CEACAM5 OPN Ca72-4 GRP DEFA1 | 7 | 0.88 | 61 | 61 |
| TIMP1 ENO2 TFPI CEACAM5 OPN Ca19-9 Ca72-4 CA242 DEFA1 | 9 | 0.88 | 45 | 61 |
| Cyfra SLPI TIMP1 CEACAM5 OPN Ca19-9 Ca72-4 CA242 CAIX GRP DEFA1 | 11 | 0.88 | 59 | 64 |
| MIF SLPI TIMP1 SCC CEACAM5 OPN Ca19-9 Ca72-4 CA242 GRP DEFA1 | 11 | 0.88 | 73 | 55 |
| Cyfra TIMP1 SCC ENO2 CEACAM5 OPN CA242 GRP DEFA1 | 9 | 0.88 | 64 | 39 |
| Cyfra TIMP1 SCC ENO2 CEACAM5 OPN CA242 CAIX GRP DEFA1 | 10 | 0.88 | 64 | 39 |
| Cyfra MIF TIMP1 SCC ENO2 CEACAM5 OPN Ca72-4 CA242 GRP DEFA1 | 11 | 0.88 | 59 | 70 |
| Cyfra MIF TIMP1 SCC ENO2 CEACAM5 OPN Ca19-9 GRP DEFA1 | 10 | 0.88 | 57 | 70 |
| Cyfra MIF TIMP1 SCC ENO2 CEACAM5 OPN Ca19-9 CAIX GRP DEFA1 | 11 | 0.88 | 57 | 70 |
| Cyfra MIF TIMP1 SCC ENO2 CEACAM5 OPN Ca72-4 CA242 CAIX DEFA1 | 11 | 0.88 | 59 | 73 |
| Cyfra TFPI CEACAM5 OPN Ca72-4 CAIX GRP DEFA1 | 8 | 0.88 | 41 | 66 |
| SCC TFPI CEACAM5 OPN Ca72-4 CAIX GRP DEFA1 | 8 | 0.88 | 45 | 68 |
| Cyfra SLPI TIMP1 ENO2 TFPI CEACAM5 OPN Ca72-4 DEFA1 | 9 | 0.88 | 68 | 66 |
| Cyfra SCC ENO2 TFPI CEACAM5 OPN Ca72-4 CA242 CAIX DEFA1 | 10 | 0.88 | 41 | 25 |
| Cyfra SLPI TIMP1 SCC ENO2 TFPI CEACAM5 OPN Ca19-9 Ca72-4 GRF | 11 | 0.88 | 68 | 34 |
| Cyfra SLPI TIMP1 SCC TFPI CEACAM5 OPN Ca19-9 Ca72-4 CA242 GRP DEFA1 | 12 | 0.88 | 45 | 64 |
| MIF TIMP1 SCC TFPI CEACAM5 OPN Ca72-4 GRP | 8 | 0.88 | 55 | 25 |
| MIF TIMP1 SCC TFPI CEACAM5 OPN Ca72-4 CAIX GRP | 9 | 0.88 | 55 | 25 |
| Cyfra MIF SLPI TIMP1 CEACAM5 OPN Ca72-4 CA242 GRP DEFA1 | 10 | 0.88 | 52 | 41 |
| Cyfra MIF SCC ENO2 TFPI CEACAM5 OPN Ca72-4 GRP DEFA1 | 10 | 0.88 | 43 | 73 |
| Cyfra MIF SLPI TIMP1 ENO2 CEACAM5 OPN Ca19-9 Ca72-4 GRP DEFA1 | 11 | 0.88 | 52 | 55 |
| SLPI TIMP1 ENO2 CEACAM5 OPN Ca19-9 Ca72-4 CA242 CAIX GRP DEFA1 | 11 | 0.88 | 50 | 48 |
| Cyfra MIF SLPI TIMP1 SCC ENO2 CEACAM5 OPN Ca72-4 CA242 GRP DEFA1 | 12 | 0.88 | 80 | 59 |
| Cyfra MIF SLPI TIMP1 ENO2 CEACAM5 OPN Ca19-9 Ca72-4 CAIX GRP DEFA1 | 12 | 0.88 | 52 | 55 |
| MIF TIMP1 SCC TFP1 CEACAM5 OPN Ca72-4 CA242 DEFA1 | 9 | 0.88 | 48 | 41 |
| SLPI TIMP1 SCC CEACAM5 OPN Ca72-4 CA242 GRP DEFA1 | 9 | 0.88 | 73 | 50 |
| SLPI TIMP1 TFPI CEACAM5 OPN Ca72-4 CAIX GRP DEFA1 | 9 | 0.88 | 73 | 48 |
| Cyfra MIF TIMP1 SCC ENO2 TFPI CEACAM5 OPN CAIX DEFA1 | 10 | 0.88 | 75 | 32 |
| Cyfra SLPI TIMP1 SCC ENO2 CEACAM5 OPN Ca72-4 GRP DEFA1 | 10 | 0.88 | 66 | 41 |
| TIMP1 SCC TFPI CEACAM5 OPN Ca19-9 Ca72-4 CA242 GRP DEFA1 | 10 | 0.88 | 61 | 41 |
| Cyfra MIF SLPI TIMP1 EN02 TFPI CEACAM5 OPN Ca19-9 Ca72-4 CAIX DEFA1 | 12 | 0.88 | 64 | 48 |
| Cyfra MIF SLPI TIMP1 SCC ENO2 TFPI CEACAM5 OPN Ca72-4 CA242 GRP DEFA1 | 13 | 0.88 | 82 | 50 |
| Cyfra MIF SLPI TIMP1 SCC ENO2 TFPI CEACAM5 OPN Ca72-4 CA242 CAIX GRP DEFA1 | 14 | 0.88 | 82 | 50 |
| Cyfra SLPI TIMP1 ENO2 CEACAM5 OPN Ca19-9 Ca72-4 CA242 CAIX DEFA1 | 11 | 0.88 | 48 | 50 |
| Cyfra MIF SLPI TIMP1 SCC ENO2 CEACAM5 OPN Ca19-9 CAIX GRP DEFA1 | 12 | 0.88 | 68 | 59 |
| TIMP1 ENO2 TFPI CEACAM5 OPN CA242 CAIX DEFA1 | 8 | 0.88 | 48 | 34 |
| TIMP1 TFPI CEACAM5 OPN Ca19-9 Ca72-4 CAIX DEFA1 | 8 | 0.88 | 50 | 52 |
| TIMP1 SCC TFPI CEACAM5 OPN GRP DEFA1 | 7 | 0.88 | 77 | 20 |
| Cyfra MIF TIMP1 SCC TFPI CEACAM5 OPN Ca19-9 Ca72-4 CAIX | 10 | 0.88 | 66 | 57 |
| Cyfra TIMP1 SCC TFPI CEACAM5 OPN Ca19-9 CAIX GRP DEFA1 | 10 | 0.88 | 70 | 50 |
| Cyfra SLPI SCC ENO2 TFPI CEACAM5 OPN Ca19-9 Ca72-4 CAIX DEFA1 | 11 | 0.88 | 39 | 64 |
| Cyfra TIMP1 ENO2 TFPI CEACAM5 OPN Ca19-9 Ca72-4 CA242 CAIX GRF | 11 | 0.88 | 57 | 32 |
| MIF SLPI TIMP1 TFPI CEACAM5 OPN Ca19-9 Ca72-4 CA242 GRP DEFA1 | 11 | 0.88 | 55 | 55 |
| SLPI TIMP1 SCC ENO2 CEACAM5 OPN Ca72-4 CA242 CAIX GRP DEFA1 | 11 | 0.88 | 68 | 27 |
| TIMP1 SCC CEACAM5 OPN Ca72-4 GRP | 6 | 0.88 | 64 | 50 |
| TIMP1 SCC ENO2 CEACAM5 OPN GRP DEFA1 | 7 | 0.88 | 59 | 64 |
| TIMP1 ENO2 CEACAM5 OPN Ca72-4 CA242 DEFA1 | 7 | 0.88 | 43 | 36 |
| TIMP1 SCC ENO2 CEACAM5 OPN CAIX GRP DEFA1 | 8 | 0.88 | 59 | 64 |
| TIMP1 SCC TFPI CEACAM5 OPN Ca19-9 CAIX GRP DEFA1 | 9 | 0.88 | 70 | 52 |
| Cyfra MIF TIMP1 SCC TFPI CEACAM5 OPN Ca72-4 CA242 GRF | 10 | 0.88 | 61 | 61 |
| Cyfra MIF TIMP1 SCC TFPI CEACAM5 OPN Ca72-4 CA242 CAIX GRF | 11 | 0.88 | 61 | 61 |
| TIMP1 SCC TFPI CEACAM5 OPN DEFA1 | 6 | 0.88 | 77 | 20 |
| TIMP1 SCC ENO2 CEACAM5 OPN Ca72-4 DEFA1 | 7 | 0.88 | 36 | 55 |
| MIF TIMP1 CEACAM5 OPN Ca19-9 CAIX GRP DEFA1 | 8 | 0.88 | 59 | 43 |
| MIF TIMP1 ENO2 TFPI CEACAM5 OPN Ca72-4 CA242 DEFA1 | 9 | 0.88 | 68 | 66 |
| SLPI TIMP1 SCC CEACAM5 OPN Ca19-9 Ca72-4 CA242 CAIX GRP DEFA1 | 11 | 0.88 | 64 | 66 |
| TIMP1 SCC EN02 TFPI CEACAM5 OPN Ca72-4 CA242 CAIX | 9 | 0.88 | 64 | 52 |
| SLPI TIMP1 ENO2 CEACAM5 OPN Ca19-9 CAIX GRP DEFA1 | 9 | 0.88 | 57 | 70 |
| Cyfra MIF TIMP1 CEACAM5 OPN Ca19-9 Ca72-4 CAIX GRF | 9 | 0.88 | 50 | 61 |
| Cyfra MIF TIMP1 SCC ENO2 CEACAM5 OPN Ca72-4 CA242 DEFA1 | 10 | 0.88 | 59 | 73 |
| Cyfra SLPI ENO2 CEACAM5 OPN Ca19-9 Ca72-4 CAIX GRP DEFA1 | 10 | 0.88 | 23 | 66 |
| TIMP1 CEACAM5 OPN Ca19-9 Ca72-4 GRP | 6 | 0.88 | 43 | 75 |
| Cyfra SLPI TIMP1 SCC ENO2 CEACAM5 OPN Ca72-4 CAIX DEFA1 | 10 | 0.88 | 64 | 41 |
| TIMP1 ENO2 TFPI CEACAM5 OPN Ca72-4 CAIX | 7 | 0.88 | 57 | 61 |
| Cyfra SLPI TIMP1 SCC CEACAM5 OPN Ca19-9 Ca72-4 CAIX DEFA1 | 10 | 0.88 | 55 | 48 |
| Cyfra SCC TFPI CEACAM5 OPN Ca72-4 GRP DEFA1 | 8 | 0.88 | 48 | 66 |
| MIF TIMP1 SCC TFPI CEACAM5 OPN CAIX GRP DEFA1 | 9 | 0.88 | 68 | 57 |
| Cyfra MIF TIMP1 ENO2 TFPI CEACAM5 OPN Ca19-9 Ca72-4 CAIX GRF | 11 | 0.88 | 59 | 32 |
| Cyfra SLPI TIMP1 ENO2 TFPI CEACAM5 OPN Ca19-9 Ca72-4 CAIX GRF | 11 | 0.88 | 66 | 34 |
| MIF TIMP1 SCC ENO2 TFPI CEACAM5 OPN GRP DEFA1 | 9 | 0.88 | 64 | 36 |
| SLPI TIMP1 TFPI CEACAM5 OPN Ca72-4 CA242 GRP DEFA1 | 9 | 0.88 | 55 | 39 |
| MIF SLPI TIMP1 SCC TFPI CEACAM5 OPN Ca19-9 Ca72-4 DEFA1 | 10 | 0.88 | 64 | 23 |
| Cyfra MIF TIMP1 SCC ENO2 TFPI CEACAM5 OPN DEFA1 | 9 | 0.88 | 75 | 32 |
| MIF TIMP1 SCC ENO2 TFPI CEACAM5 OPN Ca19-9 DEFA1 | 9 | 0.88 | 66 | 20 |
| Cyfra SLPI TIMP1 SCC CEACAM5 OPN Ca72-4 CAIX GRP DEFA1 | 10 | 0.88 | 70 | 59 |
| Cyfra MIF SLPI TIMP1 ENO2 TFPI CEACAM5 OPN Ca19-9 Ca72-4 DEFA1 | 11 | 0.88 | 64 | 48 |
| SLPI TIMP1 TFPI CEACAM5 OPN Ca72-4 GRP DEFA1 | 8 | 0.88 | 73 | 48 |
| Cyfra SLPI TIMP1 SCC ENO2 CEACAM5 OPN Ca19-9 Ca72-4 CAIX GRF | 11 | 0.88 | 59 | 39 |
| SLPI TIMP1 SCC TFPI CEACAM5 OPN Ca19-9 Ca72-4 CA242 GRP DEFA1 | 11 | 0.88 | 45 | 66 |
| SLPI TIMP1 TFPI CEACAM5 OPN Ca19-9 Ca72-4 CA242 CAIX GRP DEFA1 | 11 | 0.88 | 43 | 66 |
| Cyfra TIMP1 TFPI CEACAM5 OPN Ca72-4 CAIX | 7 | 0.88 | 66 | 34 |
| Cyfra MIF SLPI TIMP1 SCC ENO2 CEACAM5 OPN Ca19-9 GRP DEFA1 | 11 | 0.88 | 68 | 59 |
| TIMP1 ENO2 CEACAM5 OPN Ca19-9 CAIX DEFA1 | 7 | 0.88 | 41 | 41 |
| TIMP1 TFPI CEACAM5 OPN CAIX GRP DEFA1 | 7 | 0.88 | 77 | 20 |
| Cyfra TIMP1 SCC TFPI CEACAM5 OPN Ca19-9 GRP DEFA1 | 9 | 0.88 | 70 | 50 |
| TIMP1 TFPI CEACAM5 OPN CAIX DEFA1 | 6 | 0.88 | 77 | 20 |
| TIMP1 CEACAM5 OPN Ca72-4 CAIX GRP | 6 | 0.88 | 64 | 50 |
| Cyfra TIMP1 SCC TFPI CEACAM5 OPN CAIX DEFA1 | 8 | 0.88 | 77 | 18 |
| TIMP1 SCC TFPI CEACAM5 OPN Ca72-4 CA242 DEFA1 | 8 | 0.88 | 64 | 59 |
| TIMP1 ENO2 CEACAM5 OPN Ca19-9 Ca72-4 CAIX GRF | 8 | 0.88 | 32 | 52 |
| TIMP1 TFPI CEACAM5 OPN Ca72-4 CA242 CAIX DEFA1 | 8 | 0.88 | 64 | 59 |
| Cyfra TIMP1 ENO2 TFPI CEACAM5 OPN Ca19-9 Ca72-4 CA242 GRF | 10 | 0.88 | 57 | 32 |
| SLPI TIMP1 SCC ENO2 CEACAM5 OPN Ca72-4 CA242 GRP DEFA1 | 10 | 0.88 | 68 | 27 |
| Cyfra SLPI TIMP1 SCC ENO2 TFPI CEACAM5 OPN Ca19-9 Ca72-4 CA242 CAIX DEFA1 | 13 | 0.88 | 64 | 43 |
| MIF TIMP1 CEACAM5 OPN Ca19-9 GRP DEFA1 | 7 | 0.88 | 59 | 43 |
| Cyfra SLPI TIMP1 SCC ENO2 CEACAM5 OPN Ca19-9 CAIX DEFA1 | 10 | 0.88 | 61 | 25 |
| Cyfra SLPI TIMP1 CEACAM5 OPN Ca19-9 Ca72-4 CA242 GRP DEFA1 | 10 | 0.88 | 59 | 64 |
| SLPI TIMP1 ENO2 CEACAM5 OPN Ca19-9 GRP DEFA1 | 8 | 0.88 | 57 | 70 |
| Cyfra MIF TIMP1 CEACAM5 OPN Ca19-9 Ca72-4 GRP | 8 | 0.88 | 50 | 61 |
| Cyfra SLPI ENO2 CEACAM5 OPN Ca19-9 Ca72-4 GRP DEFA1 | 9 | 0.88 | 20 | 66 |
| Cyfra SLPI TIMP1 SCC ENO2 CEACAM5 OPN Ca72-4 DEFA1 | 9 | 0.88 | 64 | 41 |
| Cyfra SLPI TIMP1 SCC CEACAM5 OPN Ca19-9 Ca72-4 DEFA1 | 9 | 0.88 | 52 | 48 |
| SLPI SCC ENO2 CEACAM5 OPN Ca19-9 Ca72-4 GRP DEFA1 | 9 | 0.88 | 18 | 68 |
| MIF SLPI TIMP1 SCC ENO2 CEACAM5 OPN Ca19-9 Ca72-4 CAIX GRP DEFA1 | 12 | 0.88 | 55 | 57 |
| Cyfra SLPI TIMP1 SCC TFPI CEACAM5 OPN Ca19-9 Ca72-4 CAIX DEFA1 | 11 | 0.88 | 61 | 36 |
| SCC TFPI CEACAM5 OPN Ca72-4 GRP DEFA1 | 7 | 0.88 | 41 | 68 |
| Cyfra MIF TIMP1 ENO2 TFPI CEACAM5 OPN Ca19-9 Ca72-4 GRF | 10 | 0.88 | 59 | 32 |
| Cyfra SLPI TIMP1 ENO2 TFPI CEACAM5 OPN Ca19-9 Ca72-4 GRF | 10 | 0.88 | 66 | 34 |
| MIF TIMP1 SCC ENO2 CEACAM5 OPN Ca19-9 Ca72-4 CAIX DEFA1 | 10 | 0.88 | 34 | 68 |
| MIF SLPI TIMP1 SCC TFPI CEACAM5 OPN Ca19-9 CAIX GRP DEFA1 | 11 | 0.88 | 70 | 20 |
| MIF TIMP1 SCC ENO2 TFPI CEACAM5 OPN Ca19-9 Ca72-4 CA242 CAIX DEFA1 | 12 | 0.88 | 57 | 50 |
| Cyfra SLPI TIMP1 SCC CEACAM5 OPN Ca72-4 GRP DEFA1 | 9 | 0.88 | 70 | 59 |
| SLPI TIMP1 ENO2 CEACAM5 OPN Ca19-9 Ca72-4 CA242 GRP DEFA1 | 10 | 0.88 | 48 | 48 |
| Cyfra MIF SLPI TIMP1 ENO2 TFPI CEACAM5 OPN Ca72-4 CA242 GRP DEFA1 | 12 | 0.88 | 82 | 50 |
| Cyfra SLPI TIMP1 TFPI CEACAM5 OPN Ca19-9 Ca72-4 CA242 CAIX GRP DEFA1 | 12 | 0.88 | 45 | 64 |
| Cyfra MIF SLPI TIMP1 ENO2 TFPI CEACAM5 OPN Ca72-4 CA242 CAIX GRP DEFA1 | 13 | 0.88 | 82 | 50 |
| TIMP1 ENO2 TFPI CEACAM5 OPN CA242 DEFA1 | 7 | 0.88 | 45 | 34 |
| Cyfra SLPI TIMP1 SCC ENO2 CEACAM5 OPN Ca19-9 Ca72-4 GRF | 10 | 0.88 | 59 | 39 |
| Cyfra TIMP1 SCC ENO2 TFPI CEACAM5 OPN Ca19-9 CAIX GRP | 10 | 0.88 | 75 | 50 |
| TIMP1 SCC ENO2 TFPI CEACAM5 OPN Ca19-9 Ca72-4 CA242 GRF | 10 | 0.88 | 59 | 34 |
| Cyfra MIF SLPI TIMP1 SCC TFPI CEACAM5 OPN Ca72-4 CAIX GRP DEFA1 | 12 | 0.88 | 82 | 41 |
| Cyfra TIMP1 ENO2 CEACAM5 OPN GRP DEFA1 | 7 | 0.88 | 59 | 59 |
| Cyfra TIMP1 ENO2 CEACAM5 OPN CAIX GRP DEFA1 | 8 | 0.88 | 59 | 59 |
| Cyfra MIF TIMP1 SCC TFPI CEACAM5 OPN Ca19-9 Ca72-4 | 9 | 0.88 | 66 | 57 |
| Cyfra TIMP1 SCC TFPI CEACAM5 OPN DEFA1 | 7 | 0.88 | 77 | 18 |
| SLPI TIMP1 ENO2 CEACAM5 OPN Ca19-9 Ca72-4 DEFA1 | 8 | 0.88 | 34 | 70 |
| TIMP1 CEACAM5 OPN Ca72-4 CA242 GRP DEFA1 | 7 | 0.88 | 57 | 59 |
| MIF T1MP1 CEACAM5 OPN Ca72-4 CA242 GRP DEFA1 | 8 | 0.88 | 64 | 45 |
| TIMP1 SCC TFPI CEACAM5 OPN Ca19-9 GRP DEFA1 | 8 | 0.88 | 70 | 52 |
| TIMP1 CEACAM5 OPN Ca72-4 CA242 CAIX GRP DEFA1 | 8 | 0.88 | 57 | 59 |
| MIF TIMP1 CEACAM5 OPN Ca72-4 CA242 CAIX GRP DEFA1 | 9 | 0.88 | 64 | 45 |
| Cyfra SLPI TIMP1 SCC ENO2 CEACAM5 OPN Ca19-9 DEFA1 | 9 | 0.88 | 61 | 25 |
| MIF TIMP1 SCC CEACAM5 OPN Ca19-9 Ca72-4 CAIX GRF | 9 | 0.88 | 55 | 61 |
| Cyfra MIF SCC TFPI CEACAM5 OPN Ca72-4 CAIX GRP DEFA1 | 10 | 0.88 | 61 | 55 |
| TFPI CEACAM5 OPN Ca72-4 CAIX GRP DEFA1 | 7 | 0.88 | 39 | 68 |
| MIF TIMP1 SCC TFPI CEACAM5 OPN GRP DEFA1 | 8 | 0.88 | 68 | 57 |
| MIF TIMP1 TFPI CEACAM5 OPN CAIX GRP DEFA1 | 8 | 0.88 | 68 | 57 |
| Cyfra SCC ENO2 TFPI CEACAM5 OPN Ca72-4 CA242 DEFA1 | 9 | 0.88 | 39 | 25 |
| Cyfra MIF SLPI TIMP1 TFPI CEACAM5 OPN Ca72-4 CA242 GRP DEFA1 | 11 | 0.88 | 66 | 27 |
| Cyfra MIF SLPI TIMP1 TFPI CEACAM5 OPN Ca72-4 CA242 CAIX GRP DEFA1 | 12 | 0.88 | 66 | 27 |
| MIF SLPI TIMP1 CEACAM5 OPN Ca19-9 GRP DEFA1 | 8 | 0.88 | 73 | 34 |
| MIF SLPI TIMP1 CEACAM5 OPN Ca19-9 CAIX GRP DEFA1 | 9 | 0.88 | 73 | 34 |
| Cyfra SLPI TIMP1 TFPI CEACAM5 OPN Ca19-9 Ca72-4 CA242 GRP DEFA1 | 11 | 0.88 | 43 | 64 |
| SLPI TIMP1 SCC ENO2 TFPI CEACAM5 OPN Ca72-4 CAIX GRP DEFA1 | 11 | 0.88 | 70 | 64 |
| Cyfra MIF SLPI SCC ENO2 TFPI CEACAM5 OPN Ca19-9 Ca72-4 CAIX GRP DEFA1 | 13 | 0.88 | 50 | 48 |
| SLPI TIMP1 CEACAM5 OPN Ca72-4 CA242 CAIX GRP DEFA1 | 9 | 0.88 | 73 | 50 |
| MIF TIMP1 SCC ENO2 TFPI CEACAM5 OPN Ca19-9 Ca72-4 CAIX GRF | 11 | 0.88 | 66 | 34 |
| SLPI TIMP1 SCC EN02 TFPI CEACAM5 OPN Ca72-4 CAIX DEFA1 | 10 | 0.88 | 70 | 68 |
| TIMP1 TFPI CEACAM5 OPN Ca19-9 Ca72-4 CA242 CAIX GRP DEFA1 | 10 | 0.88 | 59 | 41 |
| Cyfra MIF SLPI TIMP1 SCC ENO2 TFPI CEACAM5 OPN Ca19-9 GRP DEFA1 | 12 | 0.88 | 75 | 50 |
| Cyfra MIF SLPI TIMP1 SCC ENO2 TFPI CEACAM5 OPN Ca19-9 CAIX GRP DEFA1 | 13 | 0.88 | 75 | 50 |
| Cyfra TIMP1 TFPI CEACAM5 OPN Ca72-4 | 6 | 0.88 | 66 | 34 |
| Cyfra MIF TIMP1 SCC CEACAM5 OPN Ca19-9 Ca72-4 DEFA1 | 9 | 0.88 | 41 | 39 |
| Cyfra TIMP1 SCC ENO2 TFPI CEACAM5 OPN Ca19-9 GRP | 9 | 0.88 | 75 | 50 |
| Cyfra MIF TIMP1 SCC CEACAM5 OPN Ca19-9 Ca72-4 CAIX DEFA1 | 10 | 0.88 | 41 | 39 |
| Cyfra SLPI TIMP1 SCC CEACAM5 OPN Ca19-9 Ca72-4 CAIX GRF | 10 | 0.88 | 66 | 61 |
| Cyfra SLPI TIMP1 ENO2 CEACAM5 OPN Ca19-9 Ca72-4 CA242 DEFA1 | 10 | 0.88 | 45 | 50 |
| Cyfra MIF SLPI TIMP1 SCC TFPI CEACAM5 OPN Ca72-4 GRP DEFA1 | 11 | 0.88 | 82 | 41 |
| Cyfra SLPI TIMP1 SCC ENO2 CEACAM5 OPN CA242 CAIX GRP DEFA1 | 11 | 0.88 | 59 | 27 |
| Cyfra MIF TIMP1 SCC ENO2 TFPI CEACAM5 OPN CA242 CAIX GRP DEFA1 | 12 | 0.88 | 68 | 20 |
| Cyfra TIMP1 SCC TFPI CEACAM5 OPN GRP DEFA1 | 8 | 0.88 | 77 | 18 |
| Cyfra TIMP1 SCC TFPI CEACAM5 OPN CAIX GRP DEFA1 | 9 | 0.88 | 77 | 18 |
| SLPI TIMP1 SCC ENO2 TFPI CEACAM5 OPN Ca72-4 CA242 DEFA1 | 10 | 0.88 | 73 | 66 |
| SLPI TIMP1 EN02 TFPI CEACAM5 OPN Ca72-4 CA242 CAIX DEFA1 | 10 | 0.88 | 73 | 66 |
| TIMP1 TFPI CEACAM5 OPN DEFA1 | 5 | 0.88 | 77 | 20 |
| TIMP1 SCC TFPI CEACAM5 OPN Ca72-4 | 6 | 0.88 | 64 | 39 |
| TIMP1 TFPI CEACAM5 OPN GRP DEFA1 | 6 | 0.88 | 77 | 20 |
| TIMP1 SCC ENO2 TFPI CEACAM5 OPN Ca19-9 CAIX GRP | 9 | 0.88 | 73 | 52 |
| TIMP1 CEACAM5 OPN Ca72-4 GRP | 5 | 0.88 | 64 | 50 |
| Cyfra TIMP1 SCC ENO2 CEACAM5 OPN DEFA1 | 7 | 0.88 | 59 | 59 |
| Cyfra TIMP1 SCC ENO2 CEACAM5 OPN CAIX DEFA1 | 8 | 0.88 | 59 | 59 |
| SLPI TIMP1 SCC CEACAM5 OPN Ca19-9 Ca72-4 CA242 GRP DEFA1 | 10 | 0.88 | 61 | 66 |
| TIMP1 SCC ENO2 CEACAM5 OPN Ca19-9 Ca72-4 CAIX | 8 | 0.88 | 34 | 55 |
| Cyfra TIMP1 ENO2 CEACAM5 OPN CA242 CAIX DEFA1 | 8 | 0.88 | 59 | 39 |
| MIF TIMP1 SCC CEACAM5 OPN Ca19-9 Ca72-4 GRP | 8 | 0.88 | 55 | 61 |
| Cyfra MIF TIMP1 SCC CEACAM5 OPN Ca19-9 Ca72-4 CA242 CAIX GRP DEFA1 | 12 | 0.88 | 50 | 68 |
| Cyfra TIMP1 SCC ENO2 TFPI CEACAM5 Ca19-9 Ca72-4 GRP DEFA1 | 10 | 0.88 | 27 | 27 |
| Cyfra MIF SCC ENO2 TFPI CEACAM5 OPN Ca19-9 Ca72-4 CAIX DEFA1 | 11 | 0.88 | 34 | 64 |
| Cyfra TIMP1 SCC ENO2 TFPI CEACAM5 Ca19-9 Ca72-4 CAIX GRP DEFA1 | 11 | 0.88 | 27 | 27 |
| TIMP1 ENO2 TFPI CEACAM5 OPN Ca72-4 | 6 | 0.88 | 55 | 61 |
| Cyfra TFPI CEACAM5 OPN Ca72-4 GRP DEFA1 | 7 | 0.88 | 41 | 66 |
| Cyfra MIF TIMP1 CEACAM5 OPN Ca72-4 CAIX DEFA1 | 8 | 0.88 | 59 | 61 |
| Cyfra SLPI TIMP1 SCC TFPI CEACAM5 OPN Ca72-4 CA242 CAIX DEFA1 | 11 | 0.88 | 52 | 43 |
| MIF SLPI TIMP1 SCC ENO2 CEACAM5 OPN Ca19-9 Ca72-4 GRP DEFA1 | 11 | 0.88 | 55 | 57 |
| MIF TIMP1 EN02 TFPI CEACAM5 OPN CAIX GRP DEFA1 | 9 | 0.88 | 59 | 36 |
| MIF SLPI TIMP1 SCC TFPI CEACAM5 OPN Ca19-9 GRP DEFA1 | 10 | 0.88 | 70 | 20 |
| SLPI TIMP1 SCC ENO2 TFPI CEACAM5 OPN Ca72-4 GRP DEFA1 | 10 | 0.88 | 70 | 64 |
| SLPI TIMP1 ENO2 TFPI CEACAM5 OPN Ca72-4 CAIX GRP DEFA1 | 10 | 0.88 | 70 | 64 |
| MIF TIMP1 SCC ENO2 TFPI CEACAM5 OPN Ca19-9 Ca72-4 CA242 DEFA1 | 11 | 0.88 | 55 | 50 |
| Cyfra MIF SLPI SCC ENO2 TFPI CEACAM5 OPN Ca19-9 Ca72-4 GRP DEFA1 | 12 | 0.88 | 50 | 48 |

## Claims

1. A method for detecting lung cancer in an individual, the method comprising detecting the levels of protein markers in the panel comprising TFPI, SCC, OPN, MDK, CEACAM5, and Cyfra in a sample from said individual, wherein elevated levels of at least one of said protein markers indicate that said individual has lung cancer.

2. The method of claim 1, further comprising detecting the level of protein markers SLPI, MMP2, and TIMP1 in a sample from said individual.

3. The method of claim 2, further comprising detecting the level of at least one protein marker selected from ENO2 (NSE), CA125 (MUC16), KRT18, IGFBP4, NCAM, CRP, SERPINA1 (ATT), PKM2, RBP, KLK11, KLK13, SAA, and APOC3 in a sample from said individual.

4. The method of claim 1 further comprising detecting the levels of protein markers ENO2 (NSE), TIMP1, DEFA1 and GRP.

5. The method of any one of claims 1-4, wherein the method is done in conjunction with computed tomography (CT) screening.

6. The method of any one of claims 1-5, wherein the detecting comprises detecting the protein markers by immunoassay, optionally wherein the immunoassay is an ELISA.

7. The method of claim 6, wherein the detecting comprises contacting the sample with antibodies that selectively bind to each of the protein markers and detecting the binding of the antibodies to the protein markers.

8. The method of any one of claims 1-7, wherein the sample is a blood, serum, plasma or bronchial lavage sample.

9. The method of any one of claims 1-8, wherein the method is carried out following lung cancer treatment.

10. The method of any one of claims 1-9, wherein said elevated level is either greater than a predetermined cutoff level, or greater than or equal to a predetermined cutoff level, or wherein the elevated level is at least two standard deviations above the mean level of the marker in individuals who do not have lung cancer.

11. Use of a composition comprising a substrate and detection reagents for detecting a plurality of lung cancer protein markers comprising Cyfra, SCC, TFPI, CEACAM5, OPN, and MDK, wherein the detection reagents are coupled to the substrate for detecting lung cancer in sample from an individual.

12. The use of claim 11, wherein the detection reagents are antibodies that selectively bind to the markers.

13. Use of a kit comprising at least one container and detection reagents for detecting a plurality of lung cancer protein markers comprising Cyfra, SCC, TFPI, CEACAM5, OPN, and MDK, wherein the detection reagents are stored in one or more containers for detecting lung cancer in a sample from an individual.

14. The use of claim 13, wherein the detection reagents are antibodies or antigens for use in an immunoassay, optionally wherein the immunoassay is an ELISA.

15. The use of claim 13, wherein at least one of the detection reagents is labeled with a detectable moiety.

16. The use of claim 13, wherein the detection reagents are antibodies, and wherein (i) the antibodies are at least one of monoclonal, polyclonal, fully human, humanized, chimeric, single-chain, and anti-idiotypic antibodies, or (ii) the antibodies comprise an antibody fragment selected from:
a) an Fab fragment;
b) an F(ab')2 fragment; and
c) an Fv fragment.

17. The use of the composition of claim 11 or 12 or the use of the kit of claims 13 to 16, wherein the composition or the kit further comprise detection reagents for detecting the level of protein markers SLP1, MMP2, or TIMP1.

## Patentansprüche

1. Verfahren zum Nachweis von Lungenkrebs bei einen Individuum, wobei das Verfahren das Nachweisen der Niveaus von Proteinmarkern in der Auswahl umfassend TFPI, SCC, OPN, MDK, CEACAM5 und Cyfra in einer Probe des Individuums umfasst, wobei erhöhte Niveaus von mindestens einem der Proteinmarker anzeigen, dass das Individuum Lungenkrebs hat.

2. Verfahren nach Anspruch 1, weiter umfassend das Nachweisen des Niveaus der Proteinmarker SLPI, MMP2 und TIMP1 in einer Probe des Individuums.

3. Verfahren nach Anspruch 2, weiter umfassend das Nachweisen des Niveaus von mindestens einem Proteinmarker ausgewählt aus EN02 (NSE), CA125 (MUC16), KRT18, IGFBP4, NCAM, CRP, SERPINA1 (ATT), PKM2, RBP, KLK11, KLK13, SAA und APOC3 in einer Probe des Individuums.

4. Verfahren nach Anspruch 1 weiter umfassend das Nachweisen der Niveaus der Proteinmarker EN02 (NSE), TIMP1, DEFA1 und GRP.

5. Verfahren nach einem der Ansprüche 1-4, wobei das Verfahren in Verbindung mit Computertomographie (CT) Screening durchgeführt wird.

6. Verfahren nach einem der Ansprüche 1-5, wobei das Nachweisen das Nachweisen der Proteinmarker durch Immunassay umfasst, wobei optional das Immunassay ein ELISA ist.

7. Verfahren nach Anspruch 6, wobei das Nachweisen das In-Kontakt-bringen der Probe mit Antikörpern, die selektiv an jeden der Proteinmarker binden, und das Nachweisen des Bindens der Antikörper an die Proteinmarker umfasst.

8. Verfahren nach einem der Ansprüche 1-7, wobei die Probe eine Blut-, Serum-, Plasma-oder Bronchiallavageprobe ist

9. Verfahren nach einem der Ansprüche 1-8, wobei das Verfahren im Anschluss an eine Lungenkrebsbehandlung durchgeführt wird.

10. Verfahren nach einem der Ansprüche 1-9, wobei das erhöhte Niveau entweder größer als ein vorherbestimmter Grenzwert, oder größer als oder gleich einem vorherbestimmten Grenzwert ist oder wobei das erhöhte Niveau mindestens zwei Standardabweichungen oberhalb des Durchschnittsniveaus des Markers in Individuen, die keinen Lungenkrebs haben, ist.

11. Verwendung einer Zusammensetzung umfassend ein Substrat und Nachweisreagenzien zum Nachweisen einer Vielzahl von Lungenkrebsproteinmarkern umfassend Cyfra, SCC, TFPI, CEACAM5, OPN und MDK, wobei die Nachweisreagenzien zum Nachweis von Lungenkrebs in einer Probe eines Individuums an das Substrat gekoppelt sind.

12. Verwendung nach Anspruch 11, wobei die Nachweisreagenzien Antikörper sind, die selektiv an die Marker binden.

13. Verwendung eines Kits umfassend mindestens einen Behälter und Nachweisreagenzien zum Nachweisen einer Vielzahl von Lungenkrebsproteinmarkern umfassend Cyfra, SCC, TFPI, CEACAM5, OPN und MDK, wobei die Nachweisreagenzien in einem oder mehreren Behältern zum Nachweis von Lungenkrebs in einer Probe des Individuums gelagert sind.

14. Verwendung nach Anspruch 13, wobei die Nachweisreagenzien Antikörper oder Antigene für die Verwendung in einem Immunassay sind, wobei optional das Immunassay ein ELISA ist.

15. Verwendung nach Anspruch 13, wobei mindestens eines der Nachweisreagenzien mit einer nachweisbaren Einheit markiert ist.

16. Verwendung nach Anspruch 13, wobei die Nachweisreagenzien Antikörper sind und wobei (i) die Antikörper mindestens einer aus monoklonalen, polyklonalen, vollständig menschlichen, humanisierten, chimären, einkettigen und anit-idiotypen Antikörpern sind oder (ii) die Antikörper ein Antikörperfragment umfassen ausgewählt aus:
a) einem Fab Fragment;
b) einem F(ab')2 Fragment; und
c) einem Fv Fragment.

17. Verwendung der Zusammensetzung nach Anspruch 11 oder 12 oder Verwendung des Kits nach den Ansprüchen 13 bis 16, wobei die Zusammensetzung oder das Kit weiter Nachweisreagenzien zum Nachweisen des Niveaus von Proteinmarkern SLP1, MMP2 oder TIMP1 umfassen.

## Revendications

1. Procédé pour détecter un cancer du poumon chez un individu, le procédé comprenant une détection des niveaux de marqueurs protéiques du panel comprenant TFPI, SCC, OPN, MDK, CEACAM5, et Cyfra dans un échantillon provenant dudit individu, dans lequel des niveaux élevés d'au moins un desdits marqueurs protéiques indiquent que ledit individu présente un cancer du poumon.

2. Procédé selon la revendication 1, comprenant en outre une détection du niveau de marqueurs protéiques SLPI, MMP2, et TIMP1 dans un échantillon provenant dudit individu.

3. Procédé selon la revendication 2, comprenant en outre une détection du niveau d'au moins un marqueur protéique sélectionné parmi EN02 (NSE), CA125 (MUC16), KRT18, IGFBP4, NCAM, CRP, SERPINA1 (ATT), PKM2, RBP, KLK11, KLK13, SAA, et APOC3 dans un échantillon provenant dudit individu.

4. Procédé selon la revendication 1 comprenant en outre une détection des niveaux de marqueurs protéiques EN02 (NSE), TIMP1, DEFA1 et GRP.

5. Procédé selon l'une quelconque des revendications 1 à 4, dans lequel le procédé est mis en oeuvre conjointement à un dépistage par tomographie assistée par ordinateur (TAO).

6. Procédé selon l'une quelconque des revendications 1 à 5, dans lequel la détection comprend une détection des marqueurs protéiques par immuno-essai, l'immuno-essai étant éventuellement un ELISA.

7. Procédé selon la revendication 6, dans lequel la détection comprend une mise en contact de l'échantillon avec des anticorps qui se lient de manière sélective à chacun des marqueurs protéiques et une détection de la liaison des anticorps aux marqueurs protéiques.

8. Procédé selon l'une quelconque des revendications 1 à 7, dans lequel l'échantillon est un échantillon de sang, de sérum, de plasma ou de lavage broncho-alvéolaire.

9. Procédé selon l'une quelconque des revendications 1 à 8, dans lequel le procédé est mis en oeuvre suite à un traitement pour le cancer du poumon.

10. Procédé selon l'une quelconque des revendications 1 à 9, dans lequel ledit taux élevé est supérieur à un niveau de seuil de coupure prédéterminé, ou supérieur ou égal à un niveau de seuil de coupure prédéterminé, ou dans lequel le niveau élevé est supérieur d'au moins deux écarts-types au niveau moyen du marqueur chez des individus qui ne présentent pas de cancer du poumon.

11. Utilisation d'une composition comprenant un substrat et des réactifs de détection pour une détection d'une pluralité de marqueurs protéiques du cancer de poumon comprenant Cyfra, SCC, TFPI, CEACAM5, OPN, et MDK, dans laquelle les réactifs de détection sont couplés au substrat pour détecter un cancer du poumon dans un échantillon provenant d'un individu.

12. Utilisation selon la revendication 11, dans laquelle les réactifs de détection sont des anticorps qui se lient de manière sélective aux marqueurs.

13. Utilisation d'une trousse comprenant au moins un conteneur et des réactifs de détection pour une détection d'une pluralité de marqueurs protéiques du cancer du poumon comprenant Cyfra, SCC, TFPI, CEACAM5, OPN, et MDK, dans laquelle les réactifs de détection sont stockés dans un ou plusieurs conteneurs pour une détection du cancer du poumon dans un échantillon provenant d'un individu.

14. Utilisation selon la revendication 13, dans laquelle les réactifs de détection sont des anticorps ou des antigènes destinés à être utilisés dans un immuno-essai, l'immuno-essai étant éventuellement un ELISA.

15. Utilisation selon la revendication 13, dans laquelle au moins un des réactifs de détection est marqué avec un groupement détectable.

16. Utilisation selon la revendication 13, dans laquelle les réactifs de détection sont des anticorps, et dans laquelle (i) les anticorps sont au moins un anticorps parmi des anticorps monoclonaux, polyclonaux, complètement humains, humanisés, chimériques, chaîne simple, et anti-idiotypiques, ou (ii) les anticorps comprennent un fragment d'anticorps sélectionné parmi :
a) un fragment Fab ;
b) un fragment F(ab')2 ; et
c) un fragment Fv.

17. Utilisation de la composition selon la revendication 11 ou 12 ou utilisation de la trousse selon les revendications 13 à 16, dans laquelle la composition ou la trousse comprend en outre des réactifs de détection destinés à détecter le niveau de marqueurs protéiques SLP1, MMP2, ou TIMP1.
